# EUROPEAN PATENT APPLICATION

(11) **EP 2 017 275 A1**
(43) Date of publication of application: **21.01.2009**
(21) Application number: 07742581.7
(22) Date of filing: 27.04.2007
(51) Int. Cl.: C07D 413/06, A61K 31/437, A61K 31/454, A61K 31/4545, A61K 31/497, A61K 31/501, A61K 31/506, A61P 1/14, A61P 25/14, A61P 25/16, A61P 25/22, A61P 25/24, A61P 25/28, A61P 43/00, C07D 413/14, C07D 471/04

(54) **BENZISOXAZOLE COMPOUND**

(30) Priority: 28.04.2006 JP 2006126719
(71) Applicant: Eisai R&D Management Co., Ltd., Tokyo 112-8088 (JP)
(72) Inventor: SASAKI, Atsushi, Tsukuba-shi, Ibaraki 300-2635 (JP); UENO, Kohshi, Tsukuba-shi, Ibaraki 300-2635 (JP); SUZUKI, Yuichi, Tsukuba-shi, Ibaraki 300-2635 (JP); HAMAOKA, Shinichi, Shimbara-shi Nagasaki (JP); SHIMMYO, Daisuke, Tsukuba-shi, Ibaraki 300-2635 (JP); TAKAHASHI, Yoshinori, Tsukuba-shi, Ibaraki 300-2635 (JP); KUROKAWA, Toshiki, Tsukuba-shi, Ibaraki 300-2635 (JP); KAZUTA, Yuji, Tsukuba-shi, Ibaraki 300-2635 (JP); OGURA, Hiroo, Tsukuba-shi, Ibaraki 300-2635 (JP); FUKUSHIMA, Tatsuto, Tsukuba-shi, Ibaraki 3002635 (JP)
(74) Representative: HOFFMANN EITLE
(86) International application number: PCT/JP2007/059146
(87) International publication number: WO 2007/126041

(57) **Abstract**

Disclosed is a compound represented by the general formula (I) or a salt thereof: 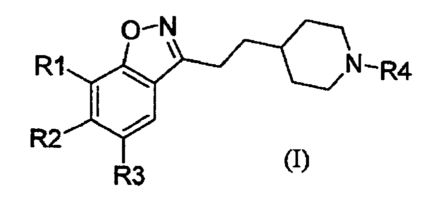 wherein any one of R1, R2 and R3 represents a group represented by the formula: -(CH₂)m-NR11R12 (wherein m is 1 or 2; and R11 and R12 independently represent a hydrogen atom or a C1-6 alkyl group or may, together with a nitrogen atom to which R11 and R12 are bound, form a 4- or 5-membered cyclic group); the remaining two or R1, R2 and R3 independently represent a group represented by the formula: -(O)n-R21 (wherein n is 0 or 1; and R21 represents a hydrogen atom, a C1-6 alkyl group, a C2-6 alkenyl group, a C2-6 alkynyl group, or the like); and R4 represents a C1-6 alkyl group which may have a substituent or the like.

## Description

### TECHNICAL FIELD

The present invention relates to a benzisoxazole compound having acetylcholine esterase inhibitory and serotonin reuptake inhibitory effects.

### BACKGROUND ART

With the increase in the aged population, the number of patients with senile dementia typified by Alzheimer's disease has been steadily increasing. An established method for treating or preventing the senile dementia has been desired not only by patients and their families but also from a socioeconomic point of view. Acetylcholine as a neurotransmitter is involved in learning, memory, motor function control and mood, and patients with Alzheimer's disease are observed to have abnormal intracerebral cholinergic neurons (Non-Patent Document 1). Therefore, therapeutic agents have been researched and developed in order to activate functions of cholinergic neurons. Among them, acetylcholine esterase inhibitors inhibiting degradation of acetylcholine have been found to be effective for impairment of cognitive functions which is a core symptom in patients with Alzheimer's disease from the results of clinical application (Non-Patent Document 2). Acetylcholine esterase inhibitors have also been reported to be useful for cognitive impairment other than Alzheimer's disease (Non-Patent Document 3).

It has been reported that many patients with early-stage Alzheimer's disease exhibit not only a core symptom but also depressive symptoms as peripheral symptoms, and it has been suggested that functions of serotonergic neurons are reduced in patients with Alzheimer's disease (Non-Patent Documents 4 and 5). Depressive symptoms also affect cognitive functions. Therefore, cognitive functions are expected to be improved by alleviation of depressive symptoms, and antidepressants may be used for patients with Alzheimer's disease in order to treat peripheral symptoms. Drugs activating serotonergic neurons have been researched for improvement of depressive symptoms. Serotonin reuptake inhibitors have been prescribed for a wide variety of diseases because of their therapeutic effect and high acceptability. Compounds having an acetylcholine esterase inhibitory effect and a serotonin reuptake inhibitory effect together are expected not only to be effective for improving impairment of cognitive functions and alleviating depressive symptoms by the respective effects but also to achieve improvement of cognitive functions resulting from alleviation of depressive symptoms by a synergistic effect of both effects. Compounds having both effects may be therapeutic agents for Alzheimer's disease which can be expected to be more widely effective than simple acetylcholine esterase inhibitors.

There is disclosed that a benzisoxazole derivative has an acetylcholine esterase inhibitory effect; however, the patent documents do not disclose a serotonin reuptake inhibitory effect or suggest a serotonin reuptake inhibitory effect (Patent Documents 1 and 2).
There is disclosed a benzisoxazole derivative having dopamine and serotonin receptor antagonistic effects; however, the patent document does not describe a serotonin reuptake inhibitory effect or suggest a serotonin reuptake inhibitory effect (Patent Document 3).
There is reported a compound having a different structure but having an acetylcholine esterase inhibitory effect and a serotonin reuptake inhibitory effect together (Patent Document 4).
Non-Patent Document 1 : Lancet, 308, pp. 1403 (1976)
Non-Patent Document 2: Drugs 61, pp. 41-52 (2001)
Non-Patent Document 3: Am. J. Med. Genet. 116, pp. 111-116 (2003), Neurology 63, pp. 1579-1585 (2004)
Non-Patent Document 4: J. Neuropathol. Exp. Neurol. 43, pp. 359-368 (1984)
Non-Patent Document 5: J. Neurosci. Res. 27, pp. 576-586 (1990)
Patent Document 1: WO 92/17475
Patent Document 2: WO 93/04063
Patent Document 3: EP-A-0428437
Patent Document 4: JP-A-2000-219678

### DISCLOSURE OF THE INVENTION

It is assumed that a single drug having an acetylcholine esterase inhibitory effect and a serotonin reuptake inhibitory effect together can exhibit both an effect of improving cognitive functions and an effect of inhibiting abnormal behaviors or the like regarding Alzheimer's disease by an effect of both effects. However, a clinically used drug having the double inhibitory effect has not yet been reported.

As a result of extensive studies in view of the above points, the present inventors have found that a novel benzisoxazole compound has excellent acetylcholine esterase inhibitory and serotonin reuptake inhibitory effects and is useful as a therapeutic agent or a prophylactic agent (particularly a therapeutic agent) for dementia or cognitive impairment, Alzheimer's disease, cerebrovascular dementia, Lewy body dementia, dementia with parkinsonism, mild cognitive impairment, frontotemporal dementia, Huntington's chorea, head injury, Down's syndrome, depression or a disease with depressive symptoms, anxiety, attention deficit hyperactivity disorder or eating disorder. This finding has led to the completion of the present invention. Specifically, the present invention relates to:
<1> A compound represented by the following general formula (I): 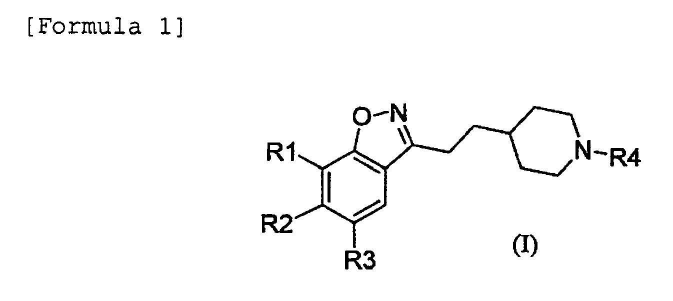
or a salt thereof,
wherein any one of R1, R2 and R3 is a group represented by the formula -(CH₂)m-NR11R12 (wherein m is 1 or 2; and R11 and R12 are the same or different and each represent a hydrogen atom or a C1-6 alkyl group, or R11 and R12, together with a nitrogen atom to which they are bonded, form a 4- or 5-membered cyclic group); the remaining two of R1, R2 and R3 are the same or different and each represent a group represented by the formula -(O)n-R21 [wherein n is 0 or 1; and R21 represents (1) a hydrogen atom, (2) a C1-6 alkyl group, (3) a C2-6 alkenyl group, (4) a C2-6 alkynyl group, (5) an amino group substituted with two C1-6 alkyl groups, (6) a C3-7 cycloalkyl group, (7) a C4-7 cycloalkenyl group, (8) a heterocycloalkyl group selected from the group consisting of a 1-pyrrolidinyl group, a piperidino group, a perhydro-2H-azepin-1-yl group and a morpholino group, (9) a 3,6-dihydro-2H-pyranyl group, (10) a C6-10 aryl group, (11) a heteroaryl group selected from the group consisting of a furyl group, a thienyl group, a thiazolyl group, an oxazolyl group, a pyridyl group and a pyridazinyl group or (12) a group represented by the formula - (CH₂) p-X- (CH₂) q-R22 (wherein X represents -O-, -NHCO- or -CONH-; R22 represents any substituent selected from Group A1 shown below, wherein the substituent may be substituted with 1 to 3 substituents which are the same or different and are selected from Group B1; and p and q are the same or different and each represent 0 to 2, provided that when X is a -O- group or a -NHCO- group and n is 1, p is 2), wherein R21 of (2) to (11) may be substituted with 1 to 3 substituents which are the same or different and are selected from Group A1 and Group A2, provided that when the substituents are selected from the Group A1, the substituents may each be substituted with 1 to 3 substituents which are the same or different and are selected from Group B1], provided that when the remaining two are R2 and R3, the R2 and R3, together with carbon atoms to which they are bonded, may form a benzene ring; and
R4 is (1) a C1-6 alkyl group, (2) a C2-6 alkenyl group, (3) a C2-6 alkynyl group, (4) a heteroaryl group selected from the group consisting of a pyridyl group, a pyridazinyl group, a pyrimidinyl group, a pyrazinyl group, a thiazolyl group, a furyl group, a thienyl group, an isoxazolyl group and an oxazolyl group or (5) a C6-10 aryl group, provided that when R4 is (1) a C1-6 alkyl group, (2) a C2-6 alkenyl group or (3) a C2-6 alkynyl group, the R4 may be substituted with 1 to 3 substituents which are the same or different and are selected from Group T1, provided that when the substituents are other than a formyl group and a hydroxyl group, the substituents may each be further substituted with 1 to 3 substituents which are the same or different and are selected from Group U1

### (Group A1)

<(1) a C3-7 cycloalkyl group, (2) a C6-10 aryl group, (3) a heteroaryl group selected from the group consisting of a pyridyl group, a pyridazinyl group, a pyrimidinyl group, a furyl group, a thiazolyl group and a thienyl group, (4) a 1,2-dihydro-2-oxopyridyl group, (5) a tetrahydrofuryl group and (6) a C3-7 cycloalkenyl group>

### (Group A2)

<(1) a halogen atom, (2) a cyano group and (3) a C1-6 alkoxy group>

### (Group B1)

<(1) a halogen atom, (2) a hydroxyl group, (3) a cyano group, (4) a C1-6 alkoxymethyl group, (5) a C1-6 alkyl group substituted with one hydroxyl group, (6) a C1-6 alkoxy group, (7) a C1-6 alkyl group and (8) a C2-6 alkynyl group which may be substituted with one C1-6 alkoxy group>

### (Group T1)

<(1) a C3-7 cycloalkyl group, (2) a heterocycloalkyl group selected from the group consisting of a 1,3-dioxolanyl group, a 1,3-dioxanyl group, a 1,4-dioxanyl group, a tetrahydrofuryl group, a 3,4,5,6-tetrahydro-2H-pyranyl group, a 2-oxopyrrolidinyl group and a 2-oxopiperidyl group, (3) a C4-7 cycloalkenyl group, (4) a heterocycloalkenyl group selected from the group consisting of a 1,2-dihydro-2-oxopyrazinyl group, a 1,2-dihydro-2-oxopyridyl group and a 1,2-dihydro-2-oxopyrimidinyl group, (5) a C6-10 aryl group, (6) a heteroaryl group selected from the group consisting of a pyridyl group, a pyridazinyl group, a pyrimidinyl group, a pyrazinyl group, a pyrrolyl group, a pyrazolyl group, an imidazolyl group, a thiazolyl group, a furyl group, a thienyl group, an isoxazolyl group, an imidazo[1,2-a]pyridyl group, a pyrazolo[1,5-a]pyridyl group, a benzo[b]thienyl group and an indolyl group, (7) a formyl group, (8) a hydroxyl group and (9) a 4,5,6,7-tetrahydrobenzothienyl group>

### (Group U1)

<(1) a halogen atom, (2) a hydroxyl group, (3) a cyano group, (4) a C1-6 alkyl group, (5) a C3-6 cycloalkyl group, (6) a C1-6 alkoxymethyl group, (7) a C1-6 alkyl group substituted with one hydroxyl group, (8) a C1-6 alkoxyimino group, (9) a C1-6 alkoxy group, (10) a methylenedioxy group, (11) a pyridyl group, (12) a C6-10 aryl group, (13) an N-(C1-6 alkyl)carbamoyl group, (14) a C2-6 alkynyl group, (15) a C2-6 alkenyl group, (16) a C1-6 alkylthio group and (17) an amino group which may be substituted with one or two C1-6 alkyl groups>;

<2> The compound or salt thereof according to <1>, wherein any one of R1, R2 and R3 is a group represented by the formula -(CH₂)m-NR11R12 [wherein m is 1; and R11 and R12 are as defined in <1>];

<3> The compound or salt thereof according to <2>, wherein R1 is a group represented by the formula - (CH₂)m-NR11R12 [wherein m is 1; and R11 and R12 are as defined in <1>]; and R2 and R3 are the same or different and each represent a group represented by the formula -(O)n-R21 [wherein R21 and n are as defined in <1> or <2>], or R2 and R3, together with the carbon atoms to which they are bonded, form a benzene ring;

<4> The compound or salt thereof according to <1>, wherein R1 is a group represented by the formula - (CH₂)m-NR11R12 [wherein m is 1; and R11 and R12 are as defined in <1>]; R2 is a group represented by the formula -(O)n-R21 [wherein R21 and n are as defined in <1>]; and R3 is a hydrogen atom;

<5> The compound or salt thereof according to <4>, wherein R1 is a group represented by the formula - (CH₂)m-NR11R12 [wherein m is 1; and R11 and R12 are the same or different and each represent (1) a hydrogen atom, (2) a methyl group or (3) an ethyl group];

<6> The compound or salt thereof according to <4> or <5>, wherein R2 is a group represented by the formula -(O)n-R21 [wherein n is 1; and R21 represents (1) a C1-6 alkyl group, (2) a C2-6 alkenyl group or (3) a C2-6 alkynyl group, wherein R21 may be substituted with 1 to 3 substituents which are the same or different and are selected from Group A1 and Group A2 according to <1>, provided that when the substituents are selected from the Group A1, the substituents may each be substituted with 1 to 3 substituents which are the same or different and are selected from Group B1 according to <1>];

<7> The compound or salt thereof according to <4> or <5>, wherein R2 is a group represented by the formula -(O)n-R21 [wherein n is 1; and R21 represents a C1-6 alkyl group, wherein R21 may be substituted with 1 to 3 substituents selected from Group A1 and Group A2 according to <1>, provided that when the substituents are selected from the Group A1, the substituents may each be substituted with 1 to 3 substituents which are the same or different and are selected from Group B1 according to <1>];

<8> The compound or salt thereof according to <4> or <5>, wherein R2 is a group represented by the formula -(O)n-R21 [wherein n is 1; and R21 is a group represented by the formula -(CH₂)t-R23 (wherein t is 1 to 2; and R23 represents (1) a C6-10 aryl group, (2) a heteroaryl group selected from the group consisting of a pyridyl group, a pyridazinyl group, a pyrimidinyl group, a furyl group, a thiazolyl group and a thienyl group, (3) a C3-7 cycloalkyl group or (4) a C3-7 cycloalkenyl group, wherein R23 may be substituted with 1 to 3 substituents which are the same or different and are selected from Group B1 according to <1>)];

<9> The compound or salt thereof according to <4> or <5>, wherein R2 is a group represented by the formula -(O)n-R21 [wherein n is 1; and R21 is a group represented by the formula - (CH₂)t-R23 (wherein t is 1 or 2; and R23 represents (1) a C6-10 aryl group, (2) a heteroaryl group selected from the group consisting of a pyridyl group, a pyridazinyl group, a pyrimidinyl group, a furyl group, a thiazolyl group and a thienyl group or (3) a C3-7 cycloalkyl group, wherein R23 may be substituted with 1 to 3 substituents which are the same or different and are selected from Group B1 according to <1>)];

<10> The compound or salt thereof according to <4> or <5>, wherein R2 is a group represented by the formula -(O)n-R21 [wherein n is 1; and R21 is a group represented by the formula -(CH₂)t-R23 (wherein t is 1 or 2; and R23 represents (1) a phenyl group, (2) a pyridyl group, (3) a furyl group, (4) a thienyl group, (5) a cyclopropyl group or (6) a cyclohexyl group, wherein R23 may be substituted with 1 to 3 substituents which are the same or different and are selected from Group B1 according to <1>)];

<11> The compound or salt thereof according to <4> or <5>, wherein R2 is a group represented by the formula -(O)n-R21 [wherein n is 1; and R21 is a group represented by the formula -(CH₂)t-R23 (wherein t is 1 or 2; and R23 represents (1) a phenyl group or (2) a cyclopropyl group, wherein R23 may be substituted with 1 to 3 substituents which are the same or different and are selected from Group B1 according to <1>)];

<12> The compound or salt thereof according to <4> or <5>, wherein R2 is a group represented by the formula -(O)n-R21 [wherein n is 0; and R21 represents (1) a C6-10 aryl group, (2) a heteroaryl group selected from the group consisting of a furyl group, a thienyl group, a thiazolyl group, an oxazolyl group, a pyridyl group and a pyridazinyl group, (3) a heterocycloalkyl group selected from the group consisting of a 1-pyrrolidinyl group, a piperidino group, a perhydro-2H-azepin-1-yl group and a morpholino group or (4) a 3,4,5,6-tetrahydro-2H-pyranyl group, which may be substituted with 1 to 3 cyano groups or/and halogen atoms];

<13> The compound or salt thereof according to <4> or <5>, wherein R2 is a group represented by the formula -(O)n-R21 [wherein n is 0; and R21 represents a phenyl group which may be substituted with 1 to 3 cyano groups or/and halogen atoms];

<14> The compound or salt thereof according to <4> or <5>, wherein R2 is a group represented by the formula -(O)n-R21 [wherein n is 0; and R21 represents (1) a pyridyl group, (2) a thienyl group or (3) a furyl group, which may be substituted with 1 to 3 cyano groups or/and halogen atoms];

<15> The compound or salt thereof according to <4> or <5>, wherein R2 is a group represented by the formula -(O)n-R21 [wherein n is 0; and R21 represents (1) a morpholino group or (2) a piperidino group];

<16> The compound or salt thereof according to <4>, <5>, <6> or <12>, wherein R4 represents (1) a C1-6 alkyl group, (2) a C2-6 alkenyl group or a (3) C2-6 alkynyl group, which is substituted with 1 to 3 substituents which are the same or different and are selected from Group T2 shown below, which substituents selected from Group T2 may be substituted with 1 to 3 substituents which are the same or different and are selected from Group U2 shown below:

### (Group T2)

<(1) a C3-7 cycloalkyl group, (2) a heterocycloalkyl group selected from the group consisting of a 1,3-dioxolanyl group, a 1,3-dioxanyl group, a 1,4-dioxanyl group, a tetrahydrofuryl group, a 3,4,5,6-tetrahydro-2H-pyranyl group, a 2-oxopyrrolidinyl group and a 2-oxopiperidyl group, (3) a C4-7 cycloalkenyl group, (4) a heterocycloalkenyl group selected from the group consisting of a 1,2-dihydro-2-oxopyrazinyl group, a 1,2-dihydro-2-oxopyridyl group and a 1,2-dihydro-2-oxopyrimidinyl group, (5) a C6-10 aryl group, (6) a heteroaryl group selected from the group consisting of a pyridyl group, a pyridazinyl group, a pyrimidinyl group, a pyrazinyl group, a pyrrolyl group, a pyrazolyl group, an imidazolyl group, a thiazolyl group, a furyl group, a thienyl group, an isoxazolyl group, an imidazo[1,2-a]pyridyl group, a pyrazolo[1,5-a]pyridyl group, a benzo[b]thienyl group and an indolyl group and (7) a 4,5,6,7-tetrahydrobenzothienyl group>

### (Group U2)

<(1) a halogen atom, (2) a hydroxyl group, (3) a cyano group, (4) a C1-6 alkyl group, (5) a C3-6 cycloalkyl group, (6) a C1-6 alkoxymethyl group, (7) a C1-6 alkyl group substituted with one hydroxyl group, (8) a C1-6 alkoxyimino group, (9) a C1-6 alkoxy group, (10) a methylenedioxy group, (11) an N-(C1-6 alkyl)carbamoyl group, (12) a C2-6 alkynyl group, (13) a C2-6 alkenyl group, (14) a C1-6 alkylthio group and (15) an amino group which may be substituted with one or two C1-6 alkyl groups>;

<17> The compound or salt thereof according to <4>, <5>, <6> or <12>, wherein R4 represents a C1-6 alkyl group which is substituted with 1 to 3 substituents which are the same or different and are selected from Group T2 according to <16>, which substituents selected from Group T2 may be substituted with 1 to 3 substituents which are the same or different and are selected from Group U2 acocrding to <16>;

<18> The compound or salt thereof according to <4>, <5>, <6> or <12>, wherein R4 is a group represented by the formula -(CH₂)u-R41 [wherein u is 1 to 3; and R41 is a substituent selected from Group T2 according to <16>, wherein the substituent selected from the Group T2 may be substituted with 1 to 3 substituents which are the same or different and are selected from Group U2 according to <16>];

<19> The compound or salt thereof according to <4>, <5>, <6> or <12>, wherein R4 is a group represented by the formula -(CH₂)u-R41 [wherein u is 1; and R41 is a substituent selected from Group T2 according to <16>, wherein the substituent selected from the Group T2 may be substituted with 1 to 3 substituents which are the same or different and are selected from Group U2 according to <16>];

<20> The compound or salt thereof according to <4>, <5>, <6> or <12>, wherein R4 is a group represented by the formula -(CH₂)u-R41 [wherein u is 1; and R41 represents (1) a cyclopentyl group, (2) a 1,3-dioxolanyl group, (3) a phenyl group, (4) a pyridyl group or (5) a thienyl group, which may be substituted with 1 to 3 substituents which are the same or different and are selected from Group U2 according to <16>] ;

<21> The compound or salt thereof according to <4> or <5>, wherein R2 is a group represented by the formula -(O)n-R21 [wherein n is 1; and R21 is a group represented by the formula -(CH₂)t-R23 (wherein t is 1 or 2; and R23 represents (1) a phenyl group or (2) a cyclopropyl group, wherein R23 may be substituted with 1 to 3 substituents which are the same or different and are selected from Group B1 according to <1>)]; and R4 is a group represented by the formula -(CH₂)u-R41 [wherein u is 1; and R41 represents (1) a cyclopentyl group, (2) a 1,3-dioxolanyl group, (3) a phenyl group, (4) a pyridyl group or (5) a thienyl group, which may be substituted with 1 to 3 substituents which are the same or different and are selected from Group U2 according to <16>];

<22> The compound or salt thereof according to <4> or <5>, wherein R2 is a group represented by the formula -(O)n-R21 [wherein n is 0; and R21 represents (1) a C6-10 aryl group, (2) a heteroaryl group selected from the group consisting of a furyl group, a thienyl group, a thiazolyl group, an oxazolyl group, a pyridyl group and a pyridazinyl group, (3) a heterocycloalkyl group selected from the group consisting of a 1-pyrrolidinyl group, a piperidino group, a perhydro-2H-azepin-1-yl group and a morpholino group or (4) a 3,4,5,6-tetrahydro-2H-pyranyl group, which may be substituted with 1 to 3 cyano groups or/and halogen atoms]; and
R4 is a group represented by the formula -(CH₂)u-R41 [wherein u is 1; and R41 represents (1) a cyclopentyl group, (2) a 1,3-dioxolanyl group, (3) a phenyl group, (4) a pyridyl group or (5) a thienyl group, which may be substituted with 1 to 3 substituents which are the same or different and are selected from Group U3 shown below]

### (Group U3)

<(1) a halogen atom, (2) a hydroxyl group, (3) a cyano group, (4) a C1-6 alkyl group substituted with one hydroxyl group and (5) a C1-6 alkoxy group>;

<23> A compound selected from the group consisting of (1) N,N-dimethyl{3-[2-(1-benzylpiperidin-4-yl)ethyl]-7-cyclopropylmethoxybenz[d]isoxazol-6-yl}methylamine, (2) 5-{4-{2-[7-(azetidin-1-ylmethyl)-6-cyclopropylmethoxybenz[d]isoxazol-3-yl]ethyl}piperidinomethyl}thiophene-2-carbonitrile, (3) N,N-dimethyl{6-cyclopropylmethoxy-3-{2-[1-(4,5,6,7-tetrahydrobenzo[b]thiophen-4-ylmethyl)piperidin-4-yl]ethyl}benz[d]isoxazol-7-yl}methylamine, (4) N,N-dimethyl{3-[2-(1-benzylpiperidin-4-yl)ethyl]-6-cyclopropylmethoxybenz[d]isoxazol-5-yl}methylamine, (5) N,N-dimethyl{6-cyclopropylmethoxy-3-[2-(1-benzylpiperidin-4-yl)ethyl]benz[d]isoxazol-7-yl}methylamine, (6) 4-{3-[2-(1-benzylpiperidin-4-yl)ethyl]-7-(N,N-dimethylaminomethyl)benz[d]isoxazol-6-yloxymethyl}benzonitrile, (7) 4-{7-(N,N-dimethylaminomethyl)-3-{2-[1-(1,3-dioxolan-2-ylmethyl)piperidin-4-yl]ethyl}benz[d]isoxazol-6-yloxymethyl}benzonitrile, (8) N,N-dimethyl{3-[2-(1-benzylpiperidin-4-yl)ethyl]-6-phenylbenz[d]isoxazol-7-yl}methylamine, (9) 5-{4-{2-[6-cyclopropylmethoxy-7-(N,N-dimethylaminomethyl)benz[d]isoxazol-3-yl]ethyl}piperidinomethyl}thiophene-2-carbonitrile, (10) {1-{4-{2-[6-cyclopropylmethoxy-7-(N,N-dimethylaminomethyl)benz[d]isoxazol-3-yl]ethyl}piperidinomethyl}cyclopentyl}methanol, (11) N,N-dimethyl{6-cyclopropylmethoxy-3-{2-[1-(5-fluoro-1H-indol-3-ylmethyl)piperidin-4-yl]ethyl}benz[d]isoxazol-7-yl}methylamine, (12) 5-{4-{2-[7-(N,N-dimethylaminomethyl)-6-piperidinobenz[d]isoxazol-3-yl]ethyl}piperidinomethyl}thiophene-2-carbonitrile, (13) 5-{4-{2-[7-(N,N-dimethylaminomethyl)-6-morpholinobenz[d]isoxazol-3-yl]ethyl}piperidinomethyl}thiophene-2-carbonitrile, (14) N,N-dimethyl{3-{2-[1-(1,3-dioxolan-2-ylmethyl)piperidin-4-yl]ethyl}-6-benzyloxybenz[d]isoxazol-7-yl}methylamine, (15) 5-{4-{2-[9-(N,N-dimethylaminomethyl)naphtho[2,3-d]isoxazol-3-yl]ethyl}piperidinomethyl}thiophene-2-carbonitrile, (16) 5-{4-{2-[9-(N-methylaminomethyl)naphtho[2,3-d]isoxazol-3-yl]ethyl}piperidinomethyl}thiophene-2-carbonitrile, (17) N-methyl{3-{2-[1-(1,3-dioxolan-2-ylmethyl)piperidin-4-yl]ethyl}-6-(4-fluorobenzyloxy)benz[d]isoxazol-7-yl}methylamine and (18) N,N-dimethyl{3-{2-[1-(1,3-dioxolan-2-ylmethyl)piperidin-4-yl]ethyl}-6-(4-fluorobenzyloxy)benz[d]isoxazol-7-yl}methylamine, or a salt thereof;

<24> A therapeutic agent for dementia or cognitive impairment, comprising the compound or salt thereof according to any one of <1> to <23> as an active ingredient;

<25> A therapeutic agent for depression or a disease with depressive symptoms, anxiety, attention deficit hyperactivity disorder or eating disorder, comprising the compound or salt thereof according to any one of <1> to <23> as an active ingredient;

<26> The therapeutic agent according to <23>, wherein the dementia or cognitive impairment is cognitive impairment associated with Parkinson's disease, Huntington's chorea, head injury or Down's syndrome, or Alzheimer's disease, cerebrovascular dementia, Lewy body dementia, mild cognitive impairment or frontotemporal dementia;

<27> The therapeutic agent according to <24>, wherein the dementia or cognitive impairment is Alzheimer's disease or cerebrovascular dementia; and

<28> A pharmaceutical composition comprising the compound or salt thereof according to any one of <1> to <23>.

The compound (I) or salt thereof according to the present invention has excellent acetylcholine esterase inhibitory and serotonin reuptake inhibitory effects together, and is therefore useful for dementia, cognitive impairment, depression or a disease with depressive symptoms and particularly useful as a therapeutic agent for Alzheimer's disease.

### BEST MODE FOR CARRYING OUT THE INVENTION

In the present specification, a structural formula of a compound may represent a certain isomer for convenience. However, the present invention includes all isomers and isomer mixtures such as geometric isomers which can be generated from the structure of a compound, optical isomers, stereoisomers and tautomers. The present invention is not limited to the description of a chemical formula for convenience and may include any one of the isomers or mixtures thereof. Accordingly, the compound of the present invention may exist as an optically active compound or a racemate, and the present invention includes each of the optically active compound and the racemate without limitations. Although crystal polymorphs of the compound may be present, the compound is not limited thereto as well and may be present as any of single crystal forms or a mixture of such crystal forms. The compound of the present invention includes an anhydride and a hydrate. The present invention also includes a so-called metabolite generated by degradation of the compound (I) of the present invention in vivo. The present invention further includes a compound generating the compound (I) of the present invention by metabolism in vivo such as oxidation, reduction, hydrolysis or conjugation (so-called prodrug).

Meanings of symbols, terms and the like used in the present specification will be explained and the present invention will be described in detail below.

The "C1-6 alkyl group" herein refers to a linear or branched alkyl group having 1 to 6 carbon atoms. Specific examples of the group include a methyl group, an ethyl group, a 1-propyl group (n-propyl group), a 2-propyl group (isopropyl group), a 2-methyl-1-propyl group (isobutyl group), a 2-methyl-2-propyl group (tert-butyl group), a 1-butyl group (n-butyl group), a 2-butyl group (sec-butyl group), a 1-pentyl group, a 2-pentyl group, a 3-pentyl group, a 2-methyl-1-butyl group, a 3-methyl-1-butyl group, a 2-methyl-2-butyl group, a 3-methyl-2-butyl group, a 2,2-dimethyl-1-propyl group, a 1-hexyl group, a 2-hexyl group, a 3-hexyl group, a 2-methyl-1-pentyl group, a 3-methyl-1-pentyl group, a 4-methyl-1-pentyl group, a 2-methyl-2-pentyl group, a 3-methyl-2-pentyl group, a 4-methyl-2-pentyl group, a 2-methyl-3-pentyl group, a 3-methyl-3-pentyl group, a 2,3-dimethyl-1-butyl group, a 3,3-dimethyl-1-butyl group, a 2,2-dimethyl-1-butyl group, a 2-ethyl-1-butyl group, a 3,3-dimethyl-2-butyl group and a 2,3-dimethyl-2-butyl group.

The "C3-7 cycloalkyl group" herein refers to a monocyclic saturated aliphatic hydrocarbon group having 3 to 7 carbon atoms. Specific examples of the group include a cyclopropyl group, a cyclobutyl group, a cyclopentyl group, a cyclohexyl group and a cycloheptyl group.

The "C3-7 heterocycloalkyl group" herein refers to a monocyclic group which contains 1 to 3 heteroatoms in the ring-forming atoms of the "C3-7 cycloalkyl group" defined above and may contain 1 to 2 carbonyl groups in the ring. Specific examples of the group include a tetrahydrofuryl group, a dioxanyl group, a piperidyl group, a tetrahydropyranyl group, a morpholinyl group and a 2-oxopyrrolidinyl group.

The "C2-6 alkenyl group" herein refers to a linear or branched alkenyl group having 2 to 6 carbon atoms and containing 1 or 2 double bonds. Specific examples of the group include a vinyl group (ethenyl group), a 3-methyl-2-butenyl group, an allyl group (2-propenyl group), a 1-propenyl group, an isopropenyl group (1-methylvinyl group), a 1-butenyl group, a 2-butenyl group, a 3-butenyl group, a 1,3-pentadienyl group, a 1,4-hexadienyl group, a 1-pentenyl group and a 1-hexenyl group.

The "C2-6 alkynyl group" herein refers to a linear or branched alkynyl group having 2 to 6 carbon atoms and having 1 or 2 triple bonds. Specific examples of the group include an ethynyl group, a 1-propynyl group, a 2-propynyl group, a 1-butynyl group, a 1,3-pentanediynyl group, a 1,4-hexadiynyl group, a pentynyl group and a hexynyl group.

The "C4-7 cycloalkenyl group" herein refers to a monocyclic aliphatic hydrocarbon group having 4 to 7 carbon atoms and containing 1 or 2 double bonds in the ring. Specific examples of the group include a cyclopentenyl group, a cyclohexenyl group, a cycloheptenyl group, a cyclopentadienyl group and a cyclohexadienyl group.

The "C4-7 heterocycloalkenyl group" herein refers to a monocyclic group which contains 1 to 3 heteroatoms in the ring-forming atoms of the "C4-7 cycloalkenyl group" defined above and may contain 1 to 3 carbonyl groups in the ring. Specific examples of the group include a 1,2-dihydro-2-oxopyridyl group, a 1,2-dihydro-2-oxopyrazinyl group and a 3,6-dihydro-2H-pyranyl group.

The "C6-10 aryl group" herein refers to an aromatic hydrocarbon cyclic group having 6 to 10 carbon atoms. Specific examples of the group include a phenyl group and a naphthyl group (1-naphthyl group, 2-naphthyl group).

The "5- to 10-membered heteroaryl group" herein refers to an aromatic cyclic group having 5 to 10 ring-forming atoms and containing 1 to 5 heteroatoms in the ring-forming atoms. Specific examples of the group include a furyl group, a thienyl group, a pyrrolyl group, an imidazolyl group, a triazolyl group, a tetrazolyl group, a thiazolyl group, a pyrazolyl group, an oxazolyl group, an isoxazolyl group, an isothiazolyl group, a thiadiazolyl group, an oxadiazolyl group, a pyridyl group, a pyrazinyl group, a pyridazinyl group, a pyrimidinyl group, a triazinyl group, a purinyl group, a pteridinyl group, a quinolyl group, an isoquinolyl group, a naphthyridinyl group, a quinoxalinyl group, a cinnolinyl group, a quinazolinyl group, a phthalazinyl group, an imidazopyridyl group, a pyrazolopyridyl group, an imidazothiazolyl group, an imidazooxazolyl group, a benzothiazolyl group, a benzoxazolyl group, a benzisoxazolyl group, a benzimidazolyl group, an indolyl group, an isoindolyl group, an indazolyl group, a pyrrolopyridyl group, a thienopyridyl group, a furopyridyl group, a benzothiadiazolyl group, a benzisothiazolyl group, a benzoxadiazolyl group, a pyridopyrimidinyl group, a benzofuryl group, a benzothienyl group and a thienofuryl group.

The "C1-6 alkoxy group" herein refers to an oxy group to which the "C1-6 alkyl group" defined above is bonded. Specific examples of the group include a methoxy group, an ethoxy group, a 1-propyloxy group, a 2-propyloxy group, a 2-methyl-1-propyloxy group, a 2-methyl-2-propyloxy group, a 1-butyloxy group, a 2-butyloxy group, a 1-pentyloxy group, a 2-pentyloxy group, a 3-pentyloxy group, a 2-methyl-1-butyloxy group, a 3-methyl-1-butyloxy group, a 2-methyl-2-butyloxy group, a 3-methyl-2-butyloxy group, a 2,2-dimethyl-1-propyloxy group, a 1-hexyloxy group, a 2-hexyloxy group, a 3-hexyloxy group, a 2-methyl-1-pentyloxy group, a 3-methyl-1-pentyloxy group, a 4-methyl-1-pentyloxy group, a 2-methyl-2-pentyloxy group, a 3-methyl-2-pentyloxy group, a 4-methyl-2-pentyloxy group, a 2-methyl-3-pentyloxy group, a 3-methyl-3-pentyloxy group, a 2,3-dimethyl-1-butyloxy group, a 3,3-dimethyl-1-butyloxy group, a 2,2-dimethyl-1-butyloxy group, a 2-ethyl-1-butyloxy group, a 3,3-dimethyl-2-butyloxy group and a 2,3-dimethyl-2-butyloxy group.

The "C1-6 alkoxymethyl group" herein refers to a methyl group to which the "C1-6 alkoxy group" defined above is bonded. Specific examples of the group include a methoxymethyl group and an ethoxymethyl group.

The "C1-6 alkylthio group" herein refers to a thio group to which the "C1-6 alkyl group" defined above is bonded. Specific examples of the group include a methylthio group, an ethylthio group, a 1-propylthio group, a 2-propylthio group, a 1-butylthio group and a 1-pentylthio group.

The "hydroxy C1-6 alkyl group" herein refers to the above-defined "C1-6 alkyl group" substituted with a hydroxyl group. Specific examples of the group include a hydroxymethyl group and a hydroxyethyl group.

The "C1-6 alkoxyimino group" herein refers to an oxyimino group to which the "C1-6 alkyl group" defined above is bonded. Specific examples of the group include a methoxyimino group and an ethoxyimino group.

The "N-(C1-6 alkyl)carbamoyl group" herein refers to a carbamoyl group in which a hydrogen atom on the nitrogen atom of the carbamoyl group is replaced by the "C1-6 alkyl group" defined above. Specific examples of the group include an N-methylcarbamoyl group, an N-ethylcarbamoyl group and an N,N-dimethylcarbamoyl group.

The "halogen atom" herein refers to a fluorine atom, a chlorine atom, a bromine atom or an iodine atom.

The "heteroatom" herein refers to a nitrogen atom, a sulfur atom or an oxygen atom.

The "salt" is not particularly limited herein insofar as it is formed with the compound of the present invention and is pharmacologically acceptable. Examples of the salt include inorganic acid salts, organic acid salts, inorganic base salts, organic base salts and acidic amino acid salts. Preferable examples of the inorganic acid salts include hydrochlorides, hydrobromides, sulfates, nitrates and phosphates. Preferable examples of the organic acid salts include acetates, succinates, fumarates, maleates, tartrates, citrates, lactates, stearates, benzoates, methanesulfonates, ethanesulfonates, p-toluenesulfonates and benzenesulfonates. Preferable examples of the salts with acidic amino acids include salts with aspartic acid and glutamic acid.

Examples of the "dementia" herein include Alzheimer's disease, cerebrovascular dementia, Lewy body dementia, mild cognitive impairment and frontotemporal dementia. Patients with Alzheimer's disease are observed to have cognitive impairment as a core symptom and may also have various psychiatric symptoms and behavior disorders as peripheral symptoms, for example, symptoms such as reduced spontaneity, depressive symptoms, anxiety, restlessness, excitation, aggressive behavior, hallucination, delusion, automatism, delirium, sleep disorder, unclean action and urinary incontinence. A medicine comprising the compound (I) or salt thereof according to the present invention is also a therapeutic agent for these peripheral symptoms.

The "cognitive impairment" is classified into various kinds herein based on its cause. Examples of the cognitive impairment include impairment caused by Alzheimer's disease, Parkinson's disease, Huntington's chorea, Pick's disease, Lewy body dementia, cerebrovascular disorder, stroke, HIV infections, AIDS, epilepsy, brain tumor, brain disorder, multiple sclerosis, Down's syndrome, Rett's syndrome, progressive supranuclear palsy, frontal lobe syndrome, frontotemporal lobar degeneration, schizophrenia, fetal alcohol syndrome, Korsakoff's syndrome, cerebral hypoxia, coronary artery bypass graft surgery, chemotherapy, radiation therapy, radiation exposure, electroshock therapy, cardiopulmonary resuscitation after cardiopulmonary arrest, diabetes, menopause, hypercholesterol, head injury and spinal cord injury. The "cognitive impairment" also includes mild cognitive impairment and age-related cognitive impairment.

The "disease with depressive symptoms" herein includes major depression, bipolar depression, unipolar depression, recurrent depression, dysthymic disorder, neurotic depression, Alzheimer's disease, cerebrovascular dementia, substance-induced mood disorder (induced by alcohols, amphetamine, cocaine, hallucinogens, inhalants, opium-like substances, phencyclidine, sedatives, hypnotics, antianxiety drugs or other substances), schizophrenia and adjustment disorder, and may be psychotic, atypical, catatonic or melancholic or may occur after childbearing, for example. The "anxiety" herein includes anxiety, obsessive compulsive disorder, panic disorder, posttraumatic stress disorder and generalized anxiety disorder.

The carbon-carbon double bond represented by the following formula (A) herein indicates either one of cis-trans isomers or a mixture of both isomers. The carbon-carbon double bond represented by the formula (B) indicates a mixture of cis-trans isomers.

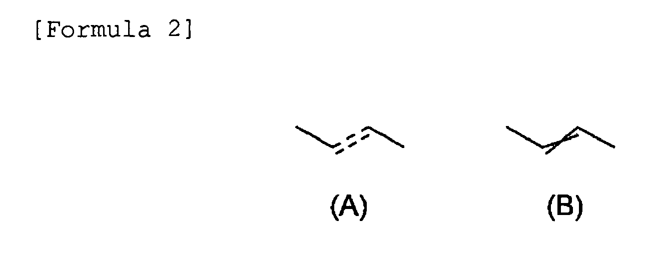

The compound represented by the chemical formula (I) according to the present invention can be prepared according to the method described below. The compound (I) can be prepared by any one of Preparation Method 1 to Preparation Method 18 singly or an appropriate combination of steps in Preparation Method 1 to Preparation Method 18.

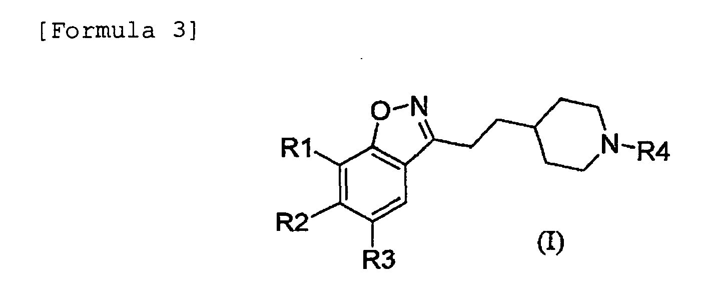

[Preparation Method 1] 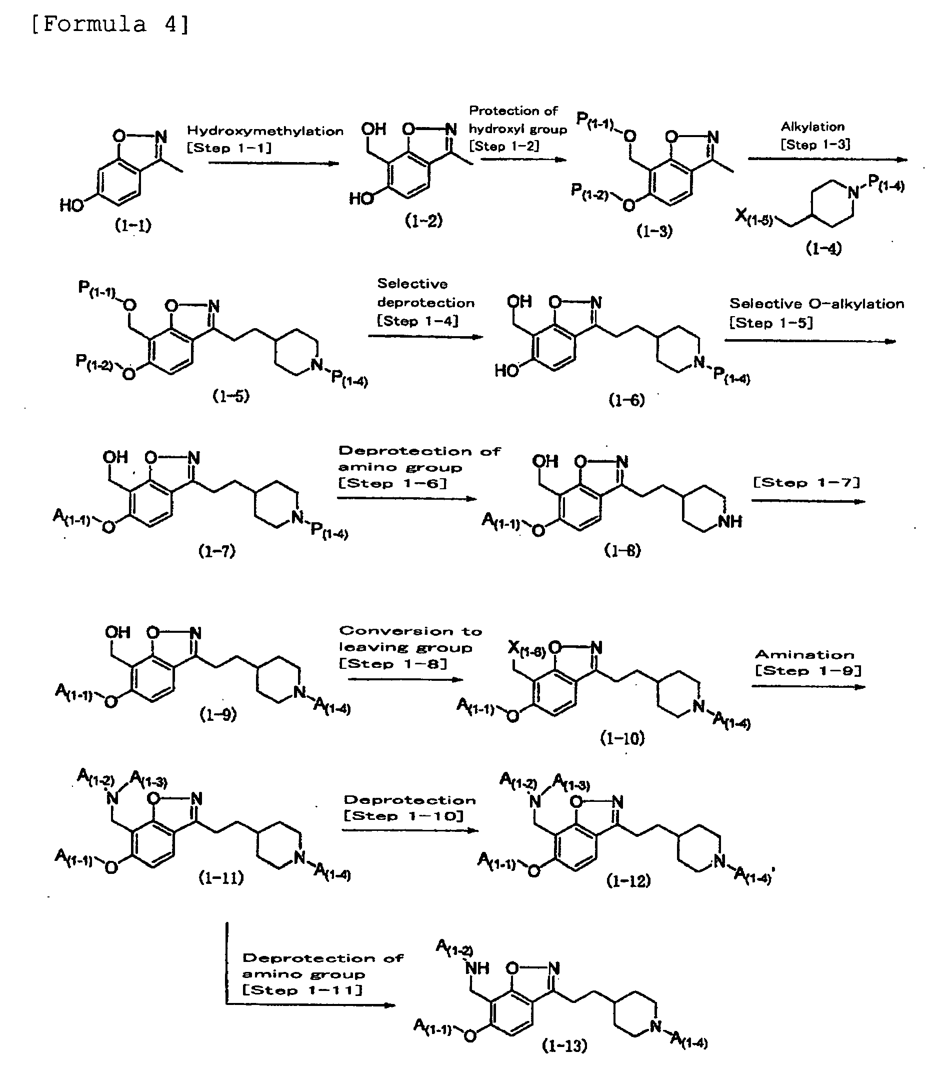 In the formula, the A(1-1) group represents an R21 group; the A(1-4)' group represents an R4 group; the A(1-4) group represents an R4 group when Step 1-10 is not performed; the A(1-4) group represents a functional group converted to an R4 group by deprotection when Step 1-10 is performed; the A(1-2) group represents an R11 group or an R12 group; the A(1-3) group represents an R11 group or an R12 group when Step 1-11 is not performed; the A(1-3) group represents a deprotectable amino protecting group when Step 1-11 is performed; the P(1-1) group represents a deprotectable alcoholic hydroxyl protecting group; the P(1-2) group represents a deprotectable phenolic hydroxyl protecting group; the P(1-4) group represents a deprotectable amino protecting group; the X(1-5) group and the X(1-6) group each represent a leaving group; and the R11 group, the R12 group, the R21 group and the R4 group are as described above.

Preparation Method 1 is a method for synthesizing the compound of the formula (I) according to the present invention from a compound (1-1) as a starting material through multiple steps of Step 1-1 to Step 1-9, or Step 1-1 to Step 1-10, or Step 1-1 to Step 1-9 and Step 1-11. However, the compound represented by the formula (I) which can be obtained by the preparation method is a compound represented by the formula (1-11), the formula (1-12) or the formula (1-13). The compound (1-1) is a known compound and can be synthesized from a commercially available compound by a known method. Examples of the synthesis method include methods described in KUMBHARE, R. M.; RAVINDRA, M.; Ingle V. N.; Asian J. Chem., 2000, 12(4), 1317 and Bhat A. R.; Guruswamy, M. N.; Indian J. Pharm. Sci., 1987, 49(1), 5. The compound (1-1) can also be prepared by a method described in Preparation Examples among Examples.

### [Step 1-1]

Step 1-1 is a step of obtaining a compound (1-2) from the compound (1-1) by hydroxymethylation. The reaction in this step can be performed using a 6-hydroxyisoxazole derivative and formaldehyde under the same conditions as those usually used in hydroxymethylation of phenol. Examples of the hydroxymethylation of phenol or the like include methods described in documents such as DHAWAN, B.; GUTSCHE, C. D.; J. Org. Chem., 1983, 48, P. 1536, MASSY, D. J. R.; MCKILLOP, A.; Synthesis, 1989 and SAIMOTO, H.; YOSHIDA, K.; MURAKAMI, T.; MORIMOTO, M.; SASHIWA, H.; SHIGEMASA, Y.; J. Org. Chem., 1996, 61 (20), 6768. Formalin (formaldehyde solution) is preferably used as the formaldehyde in this step. The solvent in this step is not particularly limited insofar as it does not inhibit the reaction and allows the starting material to be dissolved therein to a certain extent. For example, a mixed solvent of water and an organic solvent such as tetrahydrofuran or N,N-dimethylformamide, or water may be used, and water is preferably used. The reaction temperature in this step is usually 0°C to 100°C, and more preferably room temperature to 100°C. The reaction time in this step is not particularly limited and is usually 0.5 to 24 hours, and preferably 0.5 to 6 hours.

### [Step 1-2]

Step 1-2 is a step of obtaining a compound (1-3) by protecting the two hydroxyl groups of the compound (1-2) with the same or different protecting groups. The protection of the phenolic hydroxyl group in this step can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 246-292). The protection of the alcoholic hydroxyl group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 17-245). Further, the compound can also be prepared by a method described in Preparation Examples among Examples. The hydroxyl protecting group used in this step is not particularly limited. Examples of the protecting group include a tert-butyldimethylsilyl group, a tert-butyldiphenylsilyl group and a triethylsilyl group. A tert-butyldimethylsilyl group or a tert-butyldiphenylsilyl group is preferably used. For example, when the protecting group is a tert-butyldimethylsilyl group, the protecting group is introduced by a combination of a silylating reagent such as tert-butylchlorodimethylsilane and a base such as imidazole. The solvent used in this step is not particularly limited insofar as it does not inhibit the reaction and allows the starting material to be dissolved therein to a certain extent. For example, when the protecting group is a tert-butyldimethylsilyl group, an organic solvent such as N,N-dimethylformamide may be used. For example, when the protecting group is a tert-butyldimethylsilyl group, the reaction temperature in this step is usually 0°C to 50°C, and more preferably 0°C to room temperature. The reaction time in this step is not particularly limited and is usually 0.5 to 24 hours, and preferably 0.5 to 12 hours.

### [Step 1-3]

Step 1-3 is a step of obtaining a compound (1-5) by condensing the compound (1-3) with a compound (1-4) using a base. The compound (1-4) can be prepared from a commercially available compound by a method known to a person skilled in the art, and can also be prepared by a method described in Preparation Examples among Examples. Examples of the known method include a method described in VILLALOBOS, A.; BUTLER, T. W.; CHAPIN, D. S.; CHEN, Y. L.; DEMATTOS, S. B.; IVES, J. L.; JONES, S. B.; LISTON, D. R.; NAGEL, A. A.; NASON, D. M.; NIELSEN, J. A.; RAMIREZ, A. D.; SHALABY, I. A.; WHITE, W. F.; J. Med. Chem., 1995, 38 (15), 2802. Examples of the leaving group (X(1-5)) of the compound (1-4) include sulfonates such as methanesulfonates and p-toluenesulfonates; and halides such as chlorides, bromides and iodides. Iodides are preferable. The condensation reaction of the compound (1-3) with the compound (1-4) using a base can be performed under the same conditions as those usually used and described in the following documents. In this step, preferable results such as an improved yield and a reduced reaction time may be obtained by addition of hexamethylphosphoric acid triamide, N,N,N',N'-tetramethylethylenediamine or the like as an additive. Examples of the known method include conditions described in documents such as OLIVER, J. E.; WATERS, R. M.; LUSBY W. R.; J. Org. Chem., 1989, 54 (20), 4970 and VILLALOBOS, A.; BUTLER, T. W.; CHAPIN, D. S.; CHEN, Y. L.; DEMATTOS, S. B.; IVES, J. L.; JONES, S. B.; LISTON, D. R.; NAGEL, A. A.; NASON, D. M.; NIELSEN, J. A.; RAMIREZ, A. D.; SHALABY, I. A.; WHITE, W. F.; J. Med. Chem., 1995, 38 (15), 2802.

Examples of the base used in this step include strong bases such as lithium diisopropylamide, lithium dicyclohexylamide, lithium hexamethyldisilazide, sodium hexamethyldisilazide, potassium hexamethyldisilazide, n-butyllithium and sec-butyllithium. Lithium diisopropylamide is preferably used. For example, the amino protecting group (P(1-4) group) used in this step may be a carbamate group such as a tert-butoxycarbonyl group and is preferably a tert-butoxycarbonyl group. The solvent used in this step is not particularly limited insofar as it does not inhibit the reaction and allows the starting material to be dissolved therein to a certain extent. Examples of the solvent include diethyl ether, 1,2-dimethoxyethane and tetrahydrofuran. The reaction in this step is preferably performed in a nitrogen or argon atmosphere. The reaction temperature in this step is usually -100°C to room temperature, and preferably -78°C to -60°C. The reaction time in this step is not particularly limited and is usually 0.5 to 24 hours, and preferably 0.5 to 12 hours.

### [Step 1-4]

Step 1-4 is a step of obtaining a compound (1-6) by selective deprotection of the compound (1-5). The deprotection of the phenolic hydroxyl group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 246-292). The deprotection of the alcoholic hydroxyl group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 17-245). However, it is necessary to select deprotection conditions where the amino protecting group (P(1-4) group) is not reacted. Preferably, the amino protecting group is a tert-butoxycarbonyl group and the protecting group for the two hydroxyl groups is a silyl group such as a tert-butyldimethylsilyl group. Examples of the conditions for selectively deprotecting the hydroxyl protecting group in this combination include conditions using a hydrofluoride such as tetrabutylammonium fluoride, potassium fluoride or pyridinium fluoride, or acetic acid. When the above combination of the amino and hydroxyl protecting groups is used, the solvent used in this step is not particularly limited insofar as it does not inhibit the reaction and allows the starting material to be dissolved therein to a certain extent. Examples of the solvent include tetrahydrofuran, diethyl ether and 1,2-dimethoxyethane. When the above combination of the amino and hydroxyl protecting groups is used, the reaction temperature in this step is usually 0°C to 50°C, and preferably 0°C to room temperature. The reaction time in this step is not particularly limited and is usually 0.25 to 24 hours, and preferably 0.5 to 12 hours.

### [Step 1-5]

Step 1-5 is a step of obtaining a compound (1-7) by alkylation of the phenolic hydroxyl group of the compound (1-6). The alkylation of the phenolic hydroxyl group can be performed using a weak base in the presence of the alcoholic hydroxyl group under the same conditions as those usually used (for example, conditions described in documents such as SHEN, X.; VAN HEININGEN, A.; Can. J. Chem., 1992, 70 (6), 1754-1761, MASCI, B.; SACCHEO, S.; Tetrahedron, 1993, 49 (46), 10739-10748, ANELLI, P.-L.; ASAKAWA, M.; ASHTON, P. R.; BROWN, G. R.; HAYES, W.; KOCIAN, O.; RODRIGUEZ PASTOR, S.; STODDART, J. F.; TOLLEY, M. S.; WHITE, A. J. P.; WILLIAMS, D. J.; J. Chem. Soc., Chem. Commun., 1995, (24), 2541-2545 and BRINGMANN, G.; SAEB, W.; RUEBENACKER, M.; Tetrahedron, 1999, 55 (2), 423-432).

The base used in this step is not particularly limited insofar as it cannot be reacted with the alcoholic hydroxyl group. Examples of the base used include alkali metal carbonates such as potassium carbonate, sodium carbonate and cesium carbonate. The solvent used in this step is not particularly limited insofar as it does not inhibit the reaction and allows the starting material to be dissolved therein to a certain extent. Examples of the solvent include acetone, methyl ethyl ketone, acetonitrile, tetrahydrofuran, N,N-dimethylformamide and N-methyl-2-pyrrolidone. The reaction temperature in this step is usually 0°C to 120°C, and preferably 0°C to 80°C. The reaction time in this step is not particularly limited and is usually 0.5 to 48 hours, and preferably 0.5 to 24 hours.

### [Step 1-6]

Step 1-6 is a step of obtaining a compound (1-8) by deprotection of the amino group of the compound (1-7). The deprotection of the amino group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 494-653). For example, when the P(1-4) group is a tert-butoxycarbonyl group, the compound (1-8) can be obtained by deprotection using a hydrogen halide such as hydrogen chloride (gas or its solution), a halogenoacetic acid such as trifluoroacetic acid or a sulfonic acid such as methanesulfonic acid or p-toluenesulfonic acid. The solvent used in this step is not particularly limited insofar as it does not inhibit the reaction and allows the starting material to be dissolved therein to a certain extent. For example, when the protecting group to be deprotected in this step is a tert-butoxycarbonyl group, the solvent may be methanol, ethanol, ethyl acetate, methylene chloride, tetrahydrofuran or a mixture of these solvents. For example, when the protecting group to be deprotected in this step is a tert-butoxycarbonyl group, the reaction temperature in this step is usually 0°C to 40°C, and preferably 0°C to room temperature. The reaction time in this step is not particularly limited and is usually 0.5 to 24 hours, and preferably 0.5 to 12 hours.

### [Step 1-7]

Step 1-7 is a step of obtaining a compound (1-9) by converting the secondary amino group of the compound (1-8) to a tertiary amino group. This step employs a reaction such as alkylation or reductive amination.

When this step is alkylation, the compound (1-8) is reacted with a compound having a leaving group in the presence of a base. The compound having a leaving group may be a commercially available product used directly or may be prepared from a commercially available product by a method known to a person skilled in the art. Further, the reaction in this step can be performed by a method described in Examples. This step can be performed under the same conditions as those usually used and described in the following documents. Examples of the known method include those described in ISHIHARA, Y.; KIYOTA, Y.; GOTO, G.; Chem. Pharm. Bull., 1990, 38 (11), 3024 and DUTTA, A. K.; MELTZER, P. C.; MADRAS, B. K.; Med. Chem. Res., 1993, 3 (4), 209. When this step is alkylation, the base used in this step is not particularly limited. Examples of the base include triethylamine, N,N-diisopropylethylamine, sodium bicarbonate, sodium carbonate, potassium carbonate and cesium carbonate. Examples of the compound having a leaving group used in this step include halides such as chlorides, bromides and iodides; and sulfonates such as methanesulfonates and p-toluenesulfonates. The compound having a leaving group used in this step may be a commercially available product used directly or may be prepared from a commercially available product by a method known to a person skilled in the art. Further, the compound may be prepared by a method described in Examples. The solvent used in this step is not particularly limited insofar as it does not inhibit the reaction and allows the starting material to be dissolved therein to a certain extent. Examples of the solvent include tetrahydrofuran, 1,4-dioxane, acetonitrile, propionitrile, 1-butanol, N,N-dimethylformamide and N-methyl-2-pyrrolidone. The reaction temperature in this step is usually -10°C to solvent reflux temperature, and preferably 0°C to 120°C. The reaction time in this step is not particularly limited and is usually 0.5 to 24 hours, and preferably 0.5 to 12 hours.

When this step is reductive amination, the reaction can be performed using the compound (1-8) and an aldehyde or ketone under the same conditions as those usually used. The aldehyde or ketone may be a commercially available product used directly or may be prepared from a commercially available product by a method known to a person skilled in the art. The reduction reaction in this step is not particularly limited. Examples of the reduction reaction include reductive amination reaction using a reducing agent such as a borane complex (such as a boranetetrahydrofuran complex) or a boron hydride complex compound. Reductive amination reaction using a boron hydride complex compound is preferably used. Examples of the reductive amination reaction using a boron hydride complex compound include methods described in documents such as BAXTER, E. W.; REITZ, A. B.; Organic Reactions, 59, 1 (2002), EMERSON, W. S.; Organic Reactions, 4, 174 (1948), LANE, C. F.; Synthesis, 1975, 135, CTOWELL, J. C.; PEDEGIMAS, S. J.; Synthesis, 1974, 127 and ABDEL-MAGID, A. F.; CARSON, K. G.; HARRIS, B. D.; MARYANOFF, C. A.; SHAH, R. D.; J. Org. Chem., 1996, 61, 1996, 384. Examples of the boron hydride complex compound that may be used include sodium borohydride, sodium cyanoborohydride and sodium triacetoxyborohydride. When the boron hydride complex compound is used as a reducing agent, the solvent is not particularly limited insofar as it does not inhibit the reaction and allows the starting material to be dissolved therein to a certain extent. Examples of the solvent that can be used include methanol, ethanol, tetrahydrofuran, N,N-dimethylformamide, dichloromethane and 1,2-dichloroethane. The reaction temperature in this step is usually 0°C to room temperature. The reaction time in this step is not particularly limited and is usually 0.5 to 24 hours, and preferably 0.5 to 12 hours.

### [Step 1-8]

Step 1-8 is a step of obtaining a compound (1-10) by converting the alcoholic hydroxyl group of the compound (1-9) to a leaving group. Examples of the leaving group (X(1-6) group) of the compound (1-10) include sulfonates such as methanesulfonates and p-toluenesulfonates; and halides such as chlorides, bromides and iodides. This step can be performed under the same conditions as those usually used and described in the following documents. Examples of the known method include those described in ALTAMURA, M.; PERROTTA, E.; J. Org. Chem., 1993, 58 (1), 272 and GMEINER, P.; SOMMER, J.; Arch. Pharm. (Weinheim, Ger.) 1990, 323 (12), 991. For example, when the leaving group used in this step is a methanesulfonate, this step is performed by reaction of the compound (1-9) with methanesulfonyl chloride or the like in the presence of a base. For example, when the leaving group used in this step is a methanesulfonate, the base used in this step is not particularly limited. Examples of the base include sodium hydride, triethylamine, N,N-diisopropylethylamine and pyridine. Triethylamine, N,N-diisopropylethylamine and pyridine are preferably used. For example, when the leaving group used in this step is a methanesulfonate, the solvent used in this step is not particularly limited insofar as it does not inhibit the reaction and allows the starting material to be dissolved therein to a certain extent. Examples of the solvent include tetrahydrofuran, methylene chloride and N,N-dimethylformamide. For example, when the leaving group used in this step is a methanesulfonate, the reaction temperature in this step is usually -10°C to 50°C, and preferably 0°C to room temperature. For example, when the leaving group used in this step is a methanesulfonate, the reaction time in this step is not particularly limited and is usually 0.5 to 12 hours, and preferably 0.5 to 6 hours.

### [Step 1-9]

Step 1-9 is a step of obtaining a compound (1-11) by reacting the leaving group of the compound (1-10) with a primary or secondary amine. The primary or secondary amine used in this step may be a commercially available product used directly or may be prepared from a commercially available product by a method known to a person skilled in the art. In this step, two equivalents or more of the primary or secondary amine is used with respect to the compound (1-10). Alternatively, the reaction may be performed with a slight excess (one equivalent) of the primary or secondary amine used in this step and a base other than the amine used in this step. This step can be performed under the same conditions as those usually used and described in the following documents. Examples of the known method include those described in MELLONI, P.; DELLA TORRE, A.; MERONI, M.; AMBROSINI, A.; ROSSI, A. C.; J. Med. Chem., 1979, 22, 183, Anderson, W. K.; Veysoglu, T.; Synthesis, 1974, 665 and JAROCH, S.; REHWINKEL, H.; HOELSCHER, P.; SUELZLE, D.; BURTON, G.; HILLMANN, M.; MCDONALD, F. M.; MIKLAUTZ, H.; Bioorg. Med. Chem. Lett., 2004, 14 (3), 743.

The base other than the primary or secondary amine which may be used in this step is not particularly limited. Examples of the base include sodium carbonate, potassium carbonate, cesium carbonate, triethylamine, N,N-diisopropylethylamine and pyridine. The solvent used in this step is not particularly limited insofar as it does not inhibit the reaction and allows the starting material to be dissolved therein to a certain extent. Examples of the solvent include tetrahydrofuran, acetonitrile and N,N-dimethylformamide. The reaction temperature in this step is usually 0°C to 120°C, and preferably room temperature to 120°C. The reaction time in this step is not particularly limited and is usually 0.5 to 24 hours, and preferably 0.5 to 12 hours. The reaction in this step can be performed by adding an ammonium salt such as tetrabutylammonium bromide, tetrabutylammonium chloride or tetrabutylammonium iodide. This may provide excellent results such as a reduced reaction time and an improved yield. Use of a sealed pressure vessel may also provide excellent results such as a reduced reaction time and an improved yield.

### [Step 1-10]

Step 1-10 is a step performed as desired and is a step of obtaining a compound (1-12) by converting the A(1-4) group of the compound (1-11) to an A(1-4)' group by deprotection. This step can be performed under the same conditions as those generally used (for example, conditions described in a document such as T. W. GREENE and P. G. M. WUTS, "Protective Groups in Organic Chemistry, Third Edition", John Wiley & Sons, Inc. (1999)).

### [Step 1-11]

Step 1-10 is a step performed as desired when the A(1-3) group of the compound (1-11) is an amino protecting group, and is a step of obtaining a compound (1-13) by deprotecting the compound (1-11). The amino protecting group used in this step is not particularly limited. Examples of the protecting group include a 2,4-dimethoxybenzyl group. This step can be performed under the same conditions as those generally used (for example, conditions described in a document such as NUSSBAUMER, P.; BAUMANN, K.; DECHAT, T.; HARASEK, M.; Tetrahedron Lett., 1991, 47 (26), 4591, when the protecting group is a 2,4-dimethoxybenzyl group). When the protecting group to be deprotected in this step is a 2,4-dimethoxybenzyl group, the solvent used in this step is not particularly limited insofar as it does not inhibit the reaction and allows the starting material to be dissolved therein to a certain extent. Examples of the solvent include methylene chloride. The reaction temperature in this step is usually 0°C to room temperature. The reaction time in this step is not particularly limited and is usually 0.5 to 24 hours, and preferably 0.5 to 12 hours.

[Preparation Method 2] 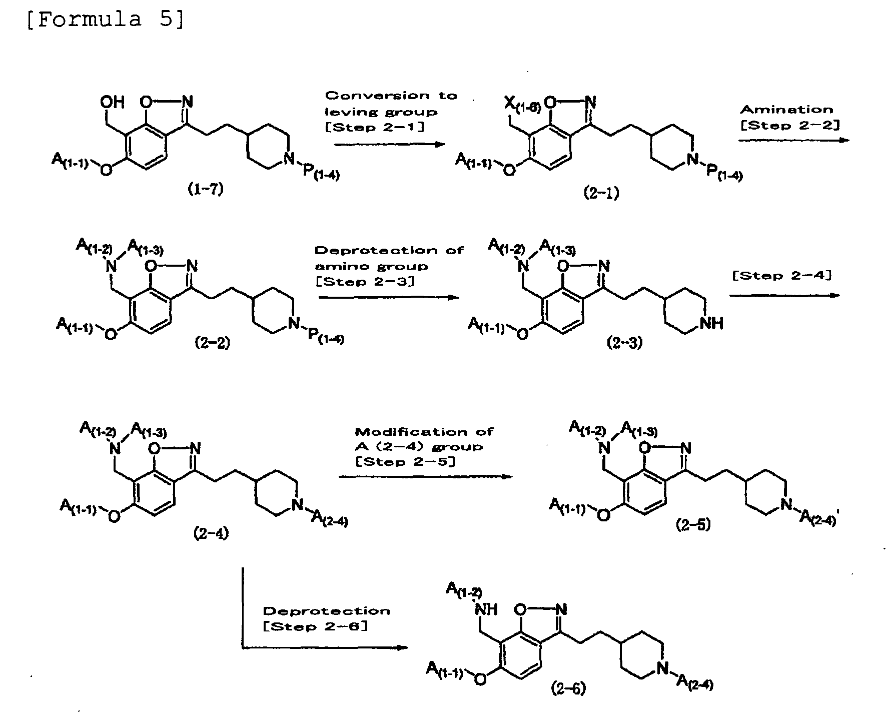 In the formula, the A(1-1) group represents an R21 group; the A(1-2) group represents an R11 group or an R12 group; the A(1-3) group represents an R11 group or an R12 group when Step 2-6 is not performed; the A(1-3) group represents a deprotectable amino protecting group when Step 2-6 is performed; the P(1-4) group represents a deprotectable amino protecting group; the X(1-6) group represents a leaving group; the A(2-4) group represents an R4 group when Step 2-5 is not performed; the A(2-4) group represents a functional group converted to an R4 group by chemical modification when Step 2-5 is performed; the A(2-4)' group represents an R4 group; and the R11 group, the R12 group, the R21 group and the R4 group are as described above.

Preparation Method 2 is a method for synthesizing the compound of the formula (I) according to the present invention from the compound (1-7) obtained by Preparation Method 1 as a starting material through multiple steps of Step 2-1 to Step 2-4, or Step 2-1 to Step 2-5, or Step 2-1 to Step 2-4 and Step 2-6. However, the compound represented by the formula (I) which can be obtained by the preparation method is a compound represented by the formula (2-4), the formula (2-5) or the formula (2-6).

### [Step 2-1]

Step 2-1 is a step of preparing a compound (2-1) from the compound (1-7) by the method described in the above Step 1-8.

### [Step 2-2]

Step 2-2 is a step of preparing a compound (2-2) from the compound (2-1) by the method described in the above Step 1-9.

### [Step 2-3]

Step 2-3 is a step of preparing a compound (2-3) from the compound (2-2) by the method described in the above Step 1-6.

### [Step 2-4]

Step 2-4 is a step of obtaining a compound (2-4) by converting the secondary amino group of the compound (2-3) to a tertiary amino group. This step employs a reaction such as N-alkylation, reductive amination, coupling using a transition metal complex or the like as a catalyst or aromatic nucleophilic substitution (SNAr reaction).

When this step is alkylation or reductive amination, the reaction can be performed by the method described in Step 1-7.

When this step is coupling using a transition metal complex or the like as a catalyst, the reaction can be performed using the compound (2-3) and an aryl halide derivative, a heteroaryl halide derivative, an aryloxy trifluoromethanesulfonate derivative or a heteroaryloxy trifluoromethanesulfonate derivative under the same conditions as those usually used (for example, conditions described in documents such as J. Tsuji, "Palladium Reagents and Catalysts", John Wiley & Sons (1995) and KWONG, F. Y.; KLAPARS, A.; BUCHWALD, S. L.; Org. Lett., 2002, 4 (4), 581). The aryl halide derivative, the heteroaryl halide derivative, the aryloxy trifluoromethanesulfonate derivative or the heteroaryloxy trifluoromethanesulfonate derivative used in this step may be a commercially available product used directly or may be prepared from a commercially available product by a method known to a person skilled in the art. Examples of the transition metal complex used in this step include dichlorobis(triphenylphosphine)palladium (II), tetrakis(triphenylphosphine)palladium (0), tris(dibenzylideneacetone)palladium (0) and a copper-diol ligand complex. In this reaction, a phosphorus ligand (such as perferably triphenylphosphine, tri-o-tolylphosphine, tri-tert-butylphosphine, 2,2'-bis(diphenylphosphino)-1,1'-binaphthyl or 1,1'-bis(diphenylphosphino)ferrocene) may be further added in order to obtain excellent results (such as a reduced reaction temperature, a reduced reaction time and an improved yield). When the transition metal complex used is a palladium complex, the reaction in this step is preferably performed in a nitrogen or argon atmosphere. The solvent used in this step is not particularly limited insofar as it does not inhibit the reaction and allows the starting material to be dissolved therein to a certain extent. For example, when the transition metal complex used is a palladium complex, N,N-dimethylformamide, N-methyl-2-pyrrolidone, 1,4-dioxane, toluene, xylene or the like may be used. When the transition metal complex used is a copper-diol complex, 2-propanol or the like may be used. The reaction temperature in this step is usually room temperature to solvent reflux temperature. The reaction time in this step is not particularly limited and is usually 0.5 to 72 hours, and preferably 0.5 to 24 hours.

When this step is aromatic nucleophilic substitution (SNAr reaction), the reaction can be performed using the compound (2-3) and an aryl halide derivative, a heteroaryl halide derivative, an aryloxy trifluoromethanesulfonate derivative or a heteroaryloxy trifluoromethanesulfonate derivative in the presence of a base under the same conditions as those usually used. The aryl halide derivative, the heteroaryl halide derivative, the aryloxy trifluoromethanesulfonate derivative or the heteroaryloxy trifluoromethanesulfonate derivative used in this step may be a commercially available product used directly or may be prepared from a commercially available product by a method known to a person skilled in the art. The aromatic nucleophilic substitution (SNAr reaction) used in this step can be performed under the same conditions as those generally used (for example, those according to methods described in documents such as MITCHELL, L. H.; BARVIAN, N. C.; Tetrahedron Lett., 2004, 45 (29), 5669 and MARSH, G.; STENUTZ, R.; BERGMAN, A.; Eur. J. Org. Chem., 2003, (14), 2566). The solvent used in this step is not particularly limited insofar as it does not inhibit the reaction and allows the starting material to be dissolved therein to a certain extent. Examples of the solvent that may be used include N,N-dimethylformamide, N-methyl-2-pyrrolidone, dimethyl sulfoxide and acetonitrile. The base used in this step is not particularly limited. Examples of the base include potassium carbonate, sodium carbonate, sodium hydride and tetrabutylammonium fluoride. Potassium carbonate, sodium carbonate and tetrabutylammonium fluoride are preferably used. The reaction temperature in this step is usually room temperature to solvent reflux temperature. The reaction time in this step is not particularly limited and is usually 0.5 to 24 hours, and preferably 0.5 to 12 hours.

### [Step 2-5]

Step 2-5 is a step performed as desired and is a step of obtaining a compound (2-5) by converting the A(2-4) group of the compound (2-4) to an A(2-4)' group by chemical modification using a reaction known to a person skilled in the art. Examples of the reaction used in this step and known to a person skilled in the art include, but are not limited to, (1) oximation of an aldehyde or ketone using a hydroxylamine salt and a base, (2) reaction of converting an oxime into a nitrile by dehydration using 1,1'-carbonyldiimidazole and (3) protection and deprotection of various functional groups described in a document such as T. W. GREENE and P. G. M. WUTS, "Protective Groups in Organic Chemistry, Third Edition", John Wiley & Sons, Inc. (1999).

### [Step 2-6]

Step 2-10 is a step performed as desired and is a step of preparing a compound (2-6) by the method described in the above Step 1-11 when the A(1-3) group of the compound (2-4) is an amino protecting group.

[Preparation Method 3] 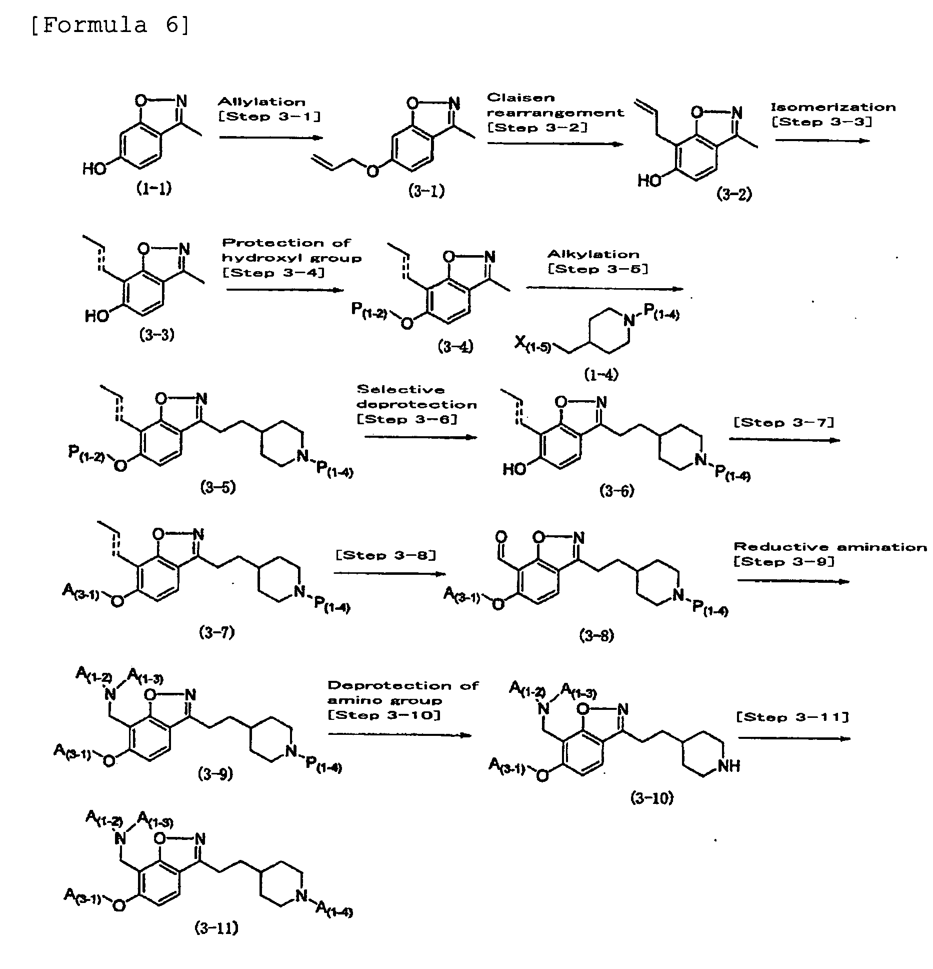 In the formula, the A(3-1) group represents an R21 group; the A(1-2) group and the A(1-3) group each represent an R11 group or an R12 group; the P(1-2) group represents a deprotectable phenolic hydroxyl protecting group; the P(1-4) group represents a deprotectable amino protecting group; the X(1-5) group represents a leaving group; the A(1-4) group represents an R4 group; and the R11 group, the R12 group, the R21 group and the R4 group are as described above.

Preparation Method 3 is a method for synthesizing the compound of the formula (I) according to the present invention from the compound (1-1) as a starting material through multiple steps of Step 3-1 to Step 3-10. However, the compound represented by the formula (I) which can be obtained by the preparation method is a compound represented by the formula (3-10).

### [Step 3-1]

Step 3-1 is a step of obtaining a compound (3-1) by allylating the phenolic hydroxyl group of the compound (1-1). This step is performed by reaction of an allyl derivative having a leaving group such as allyl bromide with the compound (1-1) in the presence of a base. This step can be performed under the same reaction conditions as those usually used and described in the following document. Examples of the reaction conditions include those described in SATO, H.; DAN, T.; ONUMA, E.; TANAKA, H.; KOGA, H.; Chem. Pharm. Bull., 1990, 38 (5), 1266. The allyl derivative used in this step may be a commercially available product used directly or may be prepared from a commercially available product by a method known to a person skilled in the art.

The base used in this step is not particularly limited. Examples of the base include potassium carbonate, sodium carbonate and sodium hydride. Potassium carbonate and sodium carbonate are preferably used. The solvent used in this step is not particularly limited insofar as it does not inhibit the reaction and allows the starting material to be dissolved therein to a certain extent. Examples of the solvent include acetone, methyl ethyl ketone, tetrahydrofuran, acetonitrile and N,N-dimethylformamide. The reaction temperature in this step is usually 0°C to solvent reflux temperature, and preferably room temperature to solvent reflux temperature. The reaction time in this step is not particularly limited and is usually 0.5 to 24 hours, and preferably 0.5 to 12 hours.

### [Step 3-2]

Step 3-2 is a step of obtaining a compound (3-2) from the compound (3-1) as a starting material by Claisen rearrangement. This step can be performed under the same conditions as those usually used. Examples of the conditions include those according to methods described in documents such as NICHOLS, D. E.; SNYDER, S. E.; OBERLENDER, R.; JOHNSON, M, P.; HUANG, X.; J. Med. Chem., 1991, 34 (1), 276 and SATO, H.: DAN, T.; ONUMA, E.; TANAKA, H.; AOKI, B.; KOGA, H.; Chem. Pharm. Bull., 1991, 39 (7), 1760.

Specifically, the compound (3-2) can be obtained by heating a solution of the compound (3-1). The reaction in this step can be performed without a solvent or in an organic solvent such as N,N-dimethylaniline, N,N-diethylaniline, N-methyl-2-pyrrolidone or dichlorobenzene. The reaction temperature in this step is usually 100°C to solvent reflux temperature, and preferably 160°C to 210°C. The reaction in this step is preferably performed in a nitrogen or argon atmosphere. Excellent results such as a reduced reaction time and an improved yield may be obtained by performing the reaction of this step using a microwave reactor.

### [Step 3-3]

Step 3-3 is a step of obtaining a compound (3-3) from the compound (3-2) as a starting material by isomerization of the double bond. This step can be performed under the same conditions as those usually used (for example, conditions described in NICHOLS, D. E.; SNYDER, S. E.; OBERLENDER, R.; JOHNSON, M. P.; HUANG, X.; J. Med. Chem., 1991, 34 (1), 276 and THACH, L. N.; HANH, D.-L.; HIEP, N. B.; RADHAKRISHNA, A. S.; SINGH, B. B.; LOUPY, A.; Synth. Commun., 1993, 23 (10), 1379). The base used in this step is not particularly limited. Examples of the base include potassium hydroxide, sodium hydroxide and potassium tert-butoxide. This step can be performed without a solvent or in an organic solvent such as dimethyl sulfoxide, ethanol or N,N-dimethylformamide or in a mixture of such an organic solvent and water. The solvent used is not particularly limited insofar as it does not inhibit the reaction and allows the starting material to be dissolved therein to a certain extent. The reaction temperature in this step is usually room temperature to 150°C, and preferably room temperature to 120°C. The reaction time in this step is not particularly limited and is usually 0.5 to 30 hours, and preferably 0.5 to 15 hours.

### [Step 3-4]

Step 3-4 is a step of obtaining a compound (3-4) by protecting the phenolic hydroxyl group of the compound (3-3). The reaction in this step can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 246-292). The hydroxyl protecting group used in this step is not particularly limited. Examples of the protecting group include a tert-butyldimethylsilyl group, a tert-butyldiphenylsilyl group and a triethylsilyl group. The solvent used in this step is not particularly limited insofar as it does not inhibit the reaction and allows the starting material to be dissolved therein to a certain extent. For example, when the protecting group used for protection in this step is a tert-butyldimethylsilyl group, an organic solvent such as N,N-dimethylformamide may be used. For example, when the protecting group used for protection in this step is a tert-butyldimethylsilyl group, the reaction temperature in this step is usually 0°C to 100°C, and more preferably 0°C to room temperature. The reaction time in this step is not particularly limited and is usually 0.5 to 24 hours, and preferably 0.5 to 12 hours.

### [Step 3-5]

Step 3-5 is a step of obtaining a compound (3-5) by condensing the compound (3-4) with the compound (1-4) using a base by the method described in Step 1-3.

### [Step 3-6]

Step 3-6 is a step of preparing a compound (3-6) from the compound (3-5) as a starting material by selective deprotection of the phenolic hydroxyl group. The phenolic hydroxyl group can be deprotected by the method described in the above Step 1-4.

### [Step 3-7]

Step 3-7 is a step of preparing a compound (3-7) from the compound (3-6) as a starting material by alkylation or aromatic nucleophilic substitution (SNAr reaction).

When this step is alkylation, the compound (3-6) is reacted with a compound having a leaving group. The reaction can be performed under the same conditions as those generally used (for example, conditions described in documents such as SEHGAL, R. K.; Liebigs. Ann. Chem., 1990, (12), 1269 and STEALEY, M. A.; SHONE, R. L.; MIYANO, M.; Synth. Commun., 1990, 20 (12), 1869). The compound having a leaving group used in this step may be a commercially available product used directly or may be prepared from a commercially available product by a method known to a person skilled in the art. Further, the compound may be prepared by a method described in Examples. Examples of the compound having a leaving group used in this step include halides such as chlorides, bromides and iodides; and sulfonates such as methanesulfonates and p-toluenesulfonates. The base used in this step is not particularly limited. Examples of the base include sodium carbonate, potassium carbonate and cesium carbonate. The solvent used in this step is not particularly limited insofar as it does not inhibit the reaction and allows the starting material to be dissolved therein to a certain extent. Examples of the solvent include tetrahydrofuran, 1,4-dioxane, acetonitrile, propionitrile, acetone, methyl ethyl ketone, N,N-dimethylformamide and N-methyl-2-pyrrolidone. The reaction temperature in this step is usually -10°C to solvent reflux temperature, and preferably 0°C to solvent reflux temperature. The reaction time in this step is not particularly limited and is usually 0.5 to 24 hours, and preferably 0.5 to 12 hours.

When this step is aromatic nucleophilic substitution, the reaction can be performed using the compound (3-7) and an aryl halide derivative, a heteroaryl halide derivative, an aryloxy trifluoromethanesulfonate derivative or a heteroaryloxy trifluoromethanesulfonate derivative in the presence of a base under the same conditions as those usually used. The aryl halide derivative, the heteroaryl halide derivative, the aryloxy trifluoromethanesulfonate derivative or the heteroaryloxy trifluoromethanesulfonate derivative used in this step may be a commercially available product used directly or may be prepared from a commercially available product by a method known to a person skilled in the art. Further, the derivative may be prepared by a method described in Examples. The aromatic nucleophilic substitution (SNAr reaction) used in this step can be performed under the same conditions as those generally used (for example, conditions described in a document such as SAWYER, J. S.; SCHMITTLING, E. A.; PALKOWITZ, J. A.; SMITH, W. J. I.; J. Org. Chem., 1998, 63 (18), 6338). The solvent used in this step is not particularly limited insofar as it does not inhibit the reaction and allows the starting material to be dissolved therein to a certain extent. Dimethyl sulfoxide may be used, for example. The base used in this step is not particularly limited. Examples of the base include a combination of potassium fluoride-alumina and 18-crown-6. The reaction temperature in this step is usually room temperature to 150°C. The reaction time in this step is not particularly limited and is usually 0.5 to 24 hours, and preferably 0.5 to 12 hours.

### [Step 3-8]

Step 3-8 is a step of obtaining a compound (3-8) from the compound (3-7) as a starting material by oxidation cleavage of the double bond. The reaction can be performed under the same reaction conditions as those generally used in oxidation cleavage reaction of obtaining an aldehyde from an olefin. The oxidation cleavage reaction used in this step is not particularly limited. Examples of the reaction include ozone oxidation and oxidation cleavage reaction using osmium tetroxide (which may be used in combination of an oxidizing agent), potassium osmate (VI) (which may be used in combination of an oxidizing agent) or the like. Osmium tetroxide (which may be used in combination of an oxidizing agent) or potassium osmate (VI) (which may be used in combination of an oxidizing agent) is preferably used.

Examples of the oxidation cleavage reaction using osmium tetroxide (which may be used in combination of an oxidizing agent), potassium osmate (VI) (which may be used in combination of an oxidizing agent), AD-mix-α, AD-mix-β or the like include a method described in LAI, G.; ANDERSON, W. K.; Tetrahedron Lett., 1993, 34 (43), 6849. The oxidation cleavage reaction of an olefin using osmium tetroxide or the like can be carried out under the same reaction conditions as those generally used (for example, conditions described in the above document). The oxidizing agent used in combination is not particularly limited. Examples of the oxidizing agent include sodium periodate.

The solvent used in this step is not particularly limited insofar as it does not inhibit the reaction and allows the starting material to be dissolved therein to a certain extent. Examples of the solvent include mixed solvents of water and organic solvents such as diethyl ether, tetrahydrofuran, 1,4-dioxane and acetone. The reaction temperature is usually ice-cold temperature to room temperature. The oxidation cleavage reaction using osmium tetroxide may be performed as two-stage reaction of isolating an olefin as a 1,2-diol using osmium tetroxide (which may be used in combination with an oxidizing agent) and then obtaining an aldehyde using an oxidizing agent such as lead tetraacetate or sodium periodate. The two-stage reaction can be performed under the same reaction conditions as those generally used (for example, conditions described in MASQUELIN, T.; HENGARTNER, U.; STREITH, J; Synthesis, 1995, 780 and BANFIELD, S. C.; ENGLAND, D. B.; KERR, M. A.; Org. Lett., 2001, 3 (21), 3325). Examples of the oxidizing agent used in combination when oxidizing the olefin to the 1,2-diol include N-methylmorpholine N-oxide and potassium hexacyanoferrate (III). The solvent used in this step is not particularly limited insofar as it does not inhibit the reaction and allows the starting material to be dissolved therein to a certain extent. Examples of the solvent include mixed solvents of water and organic solvents such acetonitrile, acetone, tert-butanol and tetrahydrofuran. The reaction temperature is usually ice-cold temperature to room temperature. The reaction time is not particularly limited and is usually 0.2 to 48 hours, and preferably 0.2 to 24 hours.

Examples of the oxidizing agent used in combination when converting the 1,2-diol to the aldehyde include lead tetraacetate and sodium periodate. Examples of the solvent used include organic solvents such as benzene, toluene, dichloromethane, diethyl ether, tetrahydrofuran, 1,4-dioxane and acetone, and mixed solvents of these organic solvents and water. The reaction temperature is usually ice-cold temperature to room temperature. The reaction time is not particularly limited and is usually 5 minutes to 48 hours, and preferably 5 minutes to 24 hours.

### [Step 3-9]

Step 3-9 is a step of obtaining a compound (3-9) by reductive amination of the compound (3-8) with a primary or secondary amine. This step can be performed by the reductive amination method described in Step 1-7.

### [Step 3-10]

Step 3-10 is a step of preparing a compound (3-10) from the compound (3-9) by the method described in the above Step 1-6.

### [Step 3-11]

Step 3-11 is a step of obtaining a compound (3-11) by converting the secondary amino group of the compound (3-10) to a tertiary amino group. This step employs a reaction such as alkylation or reductive amination. The alkylation and reductive amination in this step can be performed by the method described in the above Step 1-7.

[Preparation Method 4] 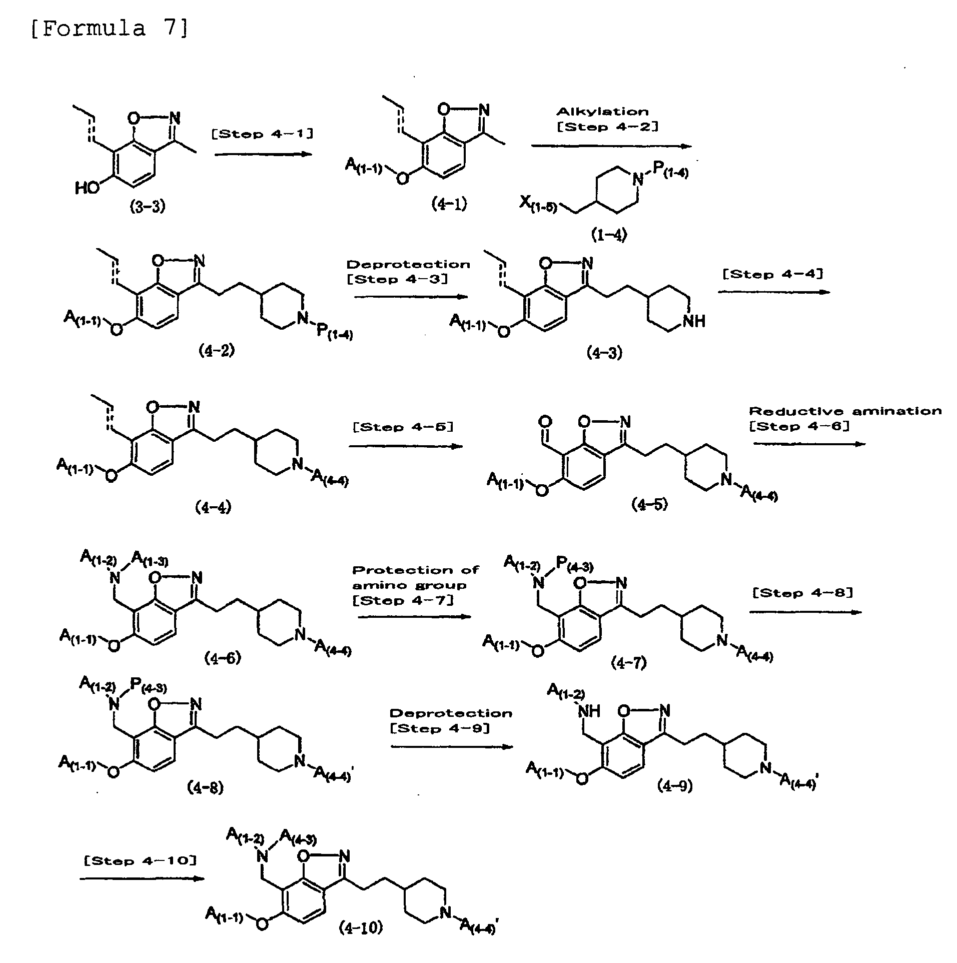 In the formula, the A(1-1) group represents an R21 group; the A(1-2) group and the A(4-3) group each represent an R11 group or an R12 group; the A(1-3) group represents an R11 group or an R12 group when Step 4-7 to Step 4-10 are not performed; the A(1-3) group represents a hydrogen atom when Step 4-7 to Step 4-10 are performed; the P(1-4) group and the P(4-3) group each represent a deprotectable amino protecting group; the X(1-5) group represents a leaving group; the A(4-4)' group represents an R4 group; the A(4-4) group represents an R4 group when Step 4-7 to Step 4-10 are not performed; the A(4-4) group represents an R4 group and the R4 group has a halogen atom removed in Step 4-8 when Step 4-7 to Step 4-10 are performed; and the R11 group, the R12 group, the R21 group and the R4 group are as described above.

Preparation Method 4 is a method for synthesizing the compound of the formula (I) according to the present invention from the compound (3-3) obtained by Preparation Method 3 as a starting material through multiple steps of Step 4-1 to Step 4-8 or Step 4-1 to 4-11. However, the compound represented by the formula (I) which can be obtained by the preparation method is a compound represented by the formula (4-8) or the formula (4-11).

### [Step 4-1]

Step 4-1 is a step of preparing a compound (4-1) from the compound (3-3) by the method described in the above Step 1-5.

### [Step 4-2]

Step 4-2 is a step of obtaining a compound (4-2) by condensing the compound (4-1) with the compound (1-4) using a base by the method described in Step 1-3.

### [Step 4-3]

Step 4-3 is a step of preparing a compound (4-3) from the compound (4-2) by the method described in the above Step 1-6.

### [Step 4-4]

Step 4-4 is a step of preparing a compound (4-4) from the compound (4-3) by a reaction such as coupling using a transition metal complex or the like as a catalyst or aromatic nucleophilic substitution (SNAr reaction). The A(2-4) group to be introduced in this step is an aryl group which may be substituted or a heteroaryl group which may be substituted. When this step employs coupling using a transition metal complex or the like as a catalyst or aromatic nucleophilic substitution (SNAr reaction), the reaction can be performed by the method described in the above Step 2-4.

### [Step 4-5]

Step 4-5 is a step of preparing a compound (4-5) from the compound (4-4) by the method described in the above Step 3-8.

### [Step 4-6]

Step 4-6 is a step of obtaining a compound (4-6) by reductive amination of the compound (4-5) with a primary or secondary amine. This step can be performed by the reductive amination method described in Step 1-7.

Step 4-7 to Step 4-10 are steps performed as desired when the A(4-4) group of the compound (4-6) is a heteroaryl group substituted with a halogen atom or an aryl group substituted with a halogen atom and the A(1-3) group is a hydrogen atom.

### [Step 4-7]

Step 4-7 is a step performed as desired and is a step of preparing a compound (4-7) by protecting the secondary amino group of the compound (4-6) when the A(1-3) group of the compound (4-6) is a hydrogen atom. This step can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 494-653). The amino protecting group used in this step is not particularly limited. Examples of the protecting group include a tert-butoxycarbonyl group and a trifluoroacetyl group. For example, when the protecting group used in this step is a tert-butoxycarbonyl group, it is possible to use a reagent for introducing the protecting group such as a combination of di-tert-butyl dicarbonate and a base. When the protecting group is a tert-butoxycarbonyl group, the base used in this step is not particularly limited. Examples of the base include triethylamine and N,N-diisopropylethylamine. The solvent used in this step is not particularly limited insofar as it does not inhibit the reaction and allows the starting material to be dissolved therein to a certain extent. Examples of the solvent include tetrahydrofuran, 1,4-dioxane, acetonitrile, N,N-dimethylformamide and N-methyl-2-pyrrolidone. The reaction temperature in this step is usually -10°C to room temperature, and preferably 0°C to room temperature. The reaction time in this step is not particularly limited and is usually 0.5 to 24 hours, and preferably 0.5 to 12 hours.

### [Step 4-8]

Step 4-8 is a step performed as desired and is a step of obtaining a compound (4-9) by converting the A(4-4) group of the compound (4-7) to an A(4-4)' group by dehalogenation using a transition metal catalyst and a reducing agent by a method known to a person skilled in the art when the A(4-4) group is a halogenated aryl group or a halogenated heteroaryl group. In this step, the reaction can be performed under conditions generally used (for example, conditions described in a document such as J. Tsuji, "Palladium Reagents and Catalysts", John Wiley & Sons (1995)). Examples of the transition metal catalyst used in this step include palladium acetate and tetrakis(triphenylphosphine)palladium (0). When the catalyst used is a palladium salt such as palladium chloride or palladium acetate, a phosphorus ligand (such as perferably triphenylphosphine, tri-o-tolylphosphine, tri-tert-butylphosphine, 2,2'-bis(diphenylphosphino)-1,1'-binaphthyl or 1,1'-bis(diphenylphosphino)ferrocene) is added. Examples of the reducing agent used in this step include formic acid, formates such as ammonium formate, and formic acid-triethylamine. The solvent used in this step is not particularly limited insofar as it does not inhibit the reaction and allows the starting material to be dissolved therein to a certain extent. Examples of the solvent include N,N-dimethylformamide, N-methyl-2-pyrrolidone and tetrahydrofuran. The reaction temperature in this step is usually room temperature to solvent reflux temperature. The reaction time in this step is not particularly limited and is usually 0.5 to 24 hours, and preferably 0.5 to 12 hours.

### [Step 4-9]

Step 4-9 is a step performed as desired and is a step of preparing a compound (4-9) by deprotecting the P(4-3) group of the compound (4-8). This step can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 494-653). For example, when the protecting group to be deprotected in this step is a tert-butoxycarbonyl group, the compound (4-9) can be obtained by deprotection using a hydrogen halide such as hydrogen chloride (gas or its solution), a halogenoacetic acid such as trifluoroacetic acid or a sulfonic acid such as methanesulfonic acid or p-toluenesulfonic acid. The solvent used in this step is not particularly limited insofar as it does not inhibit the reaction and allows the starting material to be dissolved therein to a certain extent. For example, when the protecting group to be deprotected in this step is a tert-butoxycarbonyl group, the solvent may be methanol, ethanol, methylene chloride, tetrahydrofuran or the like, or a mixture of these solvents. For example, when the protecting group to be deprotected in this step is a tert-butoxycarbonyl group, the reaction temperature in this step is usually 0°C to 40°C, and preferably 0°C to room temperature. The reaction time in this step is not particularly limited and is usually 0.5 to 24 hours, and preferably 0.5 to 12 hours.

### [Step 4-10]

Step 4-10 is a step performed as desired and is a step of preparing a compound (4-10) by converting the secondary amino group of the compound (4-9) to a tertiary amine. This step employs a reaction such as alkylation or reductive amination, and preferably reductive amination. This step can be performed by the alkylation and reductive amination methods described in the above Step 1-7.

[Preparation Method 5] 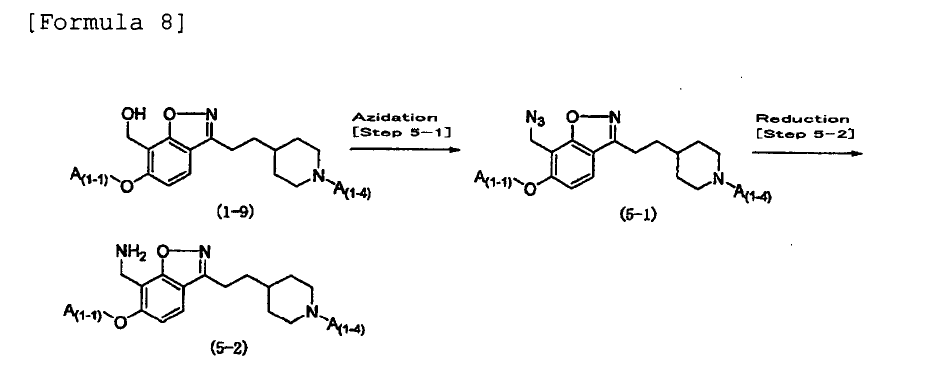 In the formula, the A(1-1) group represents an R21 group; the A(1-4) group represents an R4 group; and the R21 group and the R4 group are as described above.

Preparation Method 5 is a method for synthesizing the compound of the formula (I) according to the present invention from the compound (1-9) obtained by Preparation Method 1 as a starting material through two steps of Step 5-1 to Step 5-2. However, the compound represented by the formula (I) which can be obtained by the preparation method is a compound represented by the formula (5-2).

### [Step 5-1]

Step 5-1 is a step of obtaining a compound (5-1) by converting the alcoholic hydroxyl group of the compound (1-9) to an azide group. This step can be performed under the same conditions as those generally used (for example, conditions described in documents such as SHUTO, S.; ONO, S.; HASE, Y.; KAMIYAMA, N.; MATSUDA, A.; Tetrahedron. Lett., 1996, 37 (5), 641 and DI GIOVANNI, M. C.; MISITI, D.; VILLANI, C.; ZAPPIA, G.; Tetrahedron: Asymmetry, 1996, 7 (8), 2277). The solvent used in this step is not particularly limited insofar as it does not inhibit the reaction and allows the starting material to be dissolved therein to a certain extent. Examples of the solvent include N,N-dimethylformamide and tetrahydrofuran. The reaction temperature is usually ice-cold temperature to room temperature. The reaction time is not particularly limited and is usually 0.5 to 48 hours, and preferably 0.5 to 24 hours.

### [Step 5-2]

Step 5-2 is a step of obtaining a compound (5-2) by reducing the azide group of the compound (5-1). This step can be performed under the same conditions as those generally used (for example, conditions described in documents such as STAUDINGER, H; MEYER, J.; Helv. Chim. Acta, 1919, 2, 635 and LEE, S.; YI, K. Y.; HWANG, S. K.; SUH, J.; LEE, B. H.; YOO, S.-E.; Bull. Korean Chem. Soc., 2004, 25 (7), 1003). The solvent used in this step is not particularly limited insofar as it does not inhibit the reaction and allows the starting material to be dissolved therein to a certain extent. Examples of the solvent include organic solvents such as tetrahydrofuran and pyridine; and mixed solvents of these organic solvents and water. The reaction temperature is usually ice-cold temperature to room temperature. The reaction time is not particularly limited and is usually 0.5 to 48 hours, and preferably 0.5 to 24 hours.

### [Preparation Method 6]

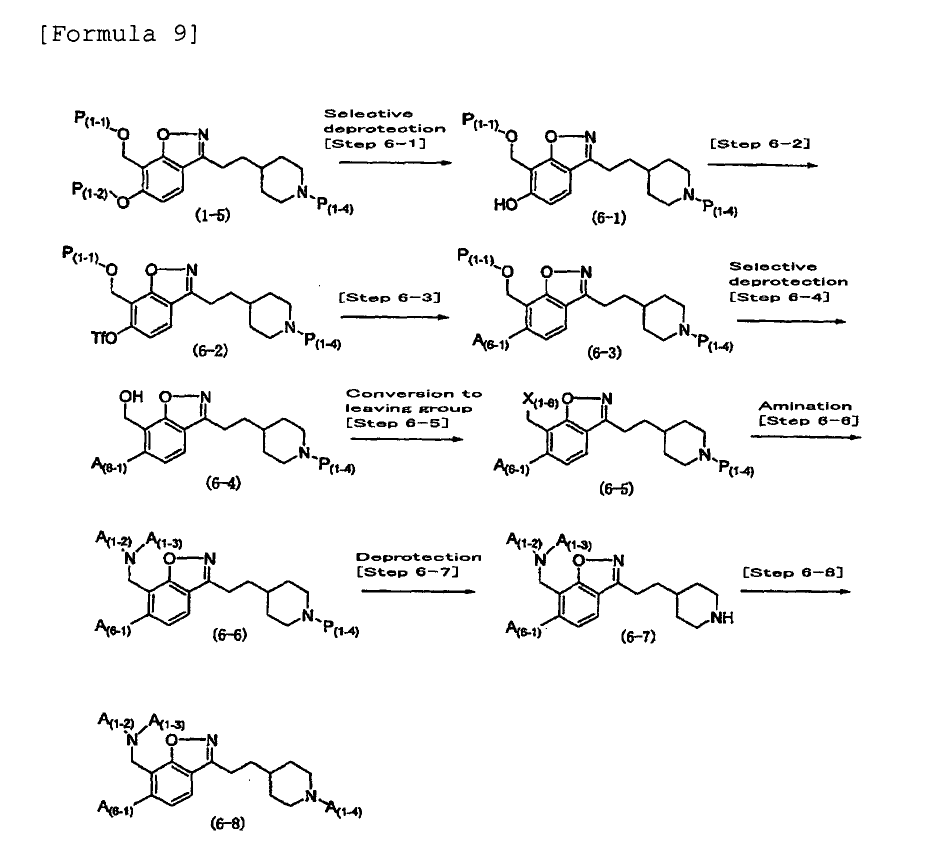

In the formula, the A(6-1) group represents a -(CH₂)n-(O)m-R21 group; the A(1-2) group and the A(1-3) group each represent an R11 group or an R12 group; the P(1-1) group represents a deprotectable alcoholic hydroxyl protecting group; the P(1-2) group represents a deprotectable phenolic hydroxyl protecting group; the P(1-4) group represents a deprotectable amino protecting group; the X(1-6) group represents a leaving group; the A(1-4) group represents an R4 group; and the R11 group, the R12 group, the R21 group, the R4 group, m and n are as described above.

Preparation Method 6 is a method for synthesizing the compound of the formula (I) according to the present invention from the compound (1-5) obtained by Preparation Method 1 as a starting material through multiple steps of Step 6-1 to Step 6-8. However, the compound represented by the formula (I) which can be obtained by the preparation method is a compound represented by the formula (6-8).

### [Step 6-1]

Step 6-1 is a step of obtaining a compound (6-1) by selective deprotection of the phenolic hydroxyl group of the compound (1-5). The deprotection of the phenolic hydroxyl group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 246-292). However, it is necessary to select deprotection conditions where the alcoholic hydroxyl protecting group (P(1-1) group) and the amino protecting group (P(1-4) group) are not reacted. Preferably, the amino protecting group is a tert-butoxycarbonyl group and the protecting group for the two hydroxyl groups is a silyl group such as a tert-butyldimethylsilyl group. Examples of the conditions for selectively deprotecting the phenolic hydroxyl protecting group in this combination include known reaction conditions (for example, conditions described in documents such as ANKALA, S. V.; FENTEANY, G.; Tetrahedron Lett, 2002, 43 (27), 4729 and SCHMITTLING, E. A.; SAWYER, J. S.; Tetrahedron Lett, 1991, 32 (49), 7207).

### [Step 6-2]

Step 6-2 is a step of obtaining a compound (6-2) by converting the phenolic hydroxyl group of the compound (6-1) to a trifluoromethanesulfonate in the presence of a base. This step can be performed under the same conditions as those generally used (for example, conditions described in documents such as ROBL, J. A.; Tetrahedron Lett., 1990, 31 (24), 3421, KOTSUKI, H.; KOBAYASHI, S.; SUENAGA, H.; NISHIZAWA, H.; Synthesis, 1990, (12), 1145 and GILBERTSON, S. R.; STARKEY, G. W.; J. Org. Chem., 1996, 61 (9), 2922). The solvent used in this step is not particularly limited insofar as it does not inhibit the reaction and allows the starting material to be dissolved therein to a certain extent. Examples of the solvent include methylene chloride and tetrahydrofuran. As a trifluoromethanesulfonylating reagent, trifluoromethanesulfonic anhydride, N-phenyltrifluoromethanesulfonimide or the like may be used and trifluoromethanesulfonic anhydride is preferably used. Examples of the base used when trifluoromethanesulfonic anhydride is used include pyridine, triethylamine and N,N-diisopropylethylamine. For example, when trifluoromethanesulfonic anhydride is used, the reaction temperature is usually -20°C to room temperature, and preferably 0°C to room temperature. The reaction time is not particularly limited and is usually 0.5 to 24 hours, and preferably 0.5 to 12 hours.

### [Step 6-3]

Step 6-3 is a step of obtaining a compound (6-3) by reacting the compound (6-2) with a trialkyltin derivative, an acetylene derivative or the like in the presence of a transition metal complex and a chloride such as lithium chloride. The trialkyltin derivative or the acetylene derivative used in this step may be a commercially available product used directly or may be prepared from a commercially available product by a method known to a person skilled in the art. In this step, the reaction can be performed under conditions generally used (for example, conditions described in a document such as J. Tsuji, "Palladium Reagents and Catalysts", John Wiley & Sons (1995)). Examples of the transition metal complex used in this step include dichlorobis(triphenylphosphine)palladium (II), tetrakis(triphenylphosphine)palladium (0) and tris(dibenzylideneacetone)palladium (0). Examples of the chloride used in this step include lithium chloride and tetrabutylammonium chloride. The reaction in this step is preferably performed in a nitrogen or argon atmosphere. The solvent used in this step is not particularly limited insofar as it does not inhibit the reaction and allows the starting material to be dissolved therein to a certain extent. Examples of the solvent include N-methyl-2-pyrrolidone, N,N-dimethylformamide, 1,4-dioxane, toluene and xylene. In this reaction, a phosphorus ligand (such as perferably triphenylphosphine, tri-o-tolylphosphine, tri-tert-butylphosphine, 2,2'-bis(diphenylphosphino)-1,1'-binaphthyl or 1,1'-bis(diphenylphosphino)ferrocene) may be further added in order to obtain excellent results (such as a reduced reaction temperature, a reduced reaction time and an improved yield). The reaction temperature in this step is usually room temperature to 150°C. The reaction time is not particularly limited and is usually 0.5 to 48 hours, and preferably 0.5 to 24 hours.

### [Step 6-4]

Step 6-4 is a step of obtaining a compound (6-4) by selective deprotection of the alcoholic hydroxyl group of the compound (6-3). The deprotection of the alcoholic hydroxyl group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 17-245). However, it is necessary to select deprotection conditions where the amino protecting group (P(1-4) group) is not reacted. Preferably, the amino protecting group is a tert-butoxycarbonyl group and the alcoholic hydroxyl protecting group is a silyl group such as a tert-butyldimethylsilyl group. Examples of the conditions for selectively deprotecting the hydroxyl protecting group in this combination include conditions using a hydrofluoride such as tetrabutylammonium fluoride, potassium fluoride or pyridinium fluoride, or acetic acid. Tetrabutylammonium fluoride is preferably used. When the above combination of the amino and hydroxyl protecting groups is used, the solvent used in this step is not particularly limited insofar as it does not inhibit the reaction and allows the starting material to be dissolved therein to a certain extent. Examples of the solvent include tetrahydrofuran, diethyl ether and 1,2-dimethoxyethane. When the above combination of the amino and hydroxyl protecting groups is used, the reaction temperature is usually 0°C to 50°C, and preferably 0°C to room temperature. The reaction time in this step is not particularly limited and is usually 0.25 to 24 hours, and preferably 0.5 to 12 hours.

### [Step 6-5]

Step 6-5 is a step of preparing a compound (6-5) from the compound (6-4) by the method described in the above Step 1-8.

### [Step 6-6]

Step 6-6 is a step of preparing a compound (6-6) from the compound (6-5) by the method described in the above Step 1-9.

### [Step 6-7]

Step 6-7 is a step of preparing a compound (6-7) from the compound (6-6) by the method described in the above Step 1-6.

### [Step 6-8]

Step 6-8 is a step of obtaining a compound (6-8) by converting the secondary amino group of the compound (6-7) to a tertiary amino group. This step employs a reaction such as alkylation or reductive amination. The alkylation and reductive amination in this step can be performed by the method described in the above Step 1-7.

### [Preparation Method 7]

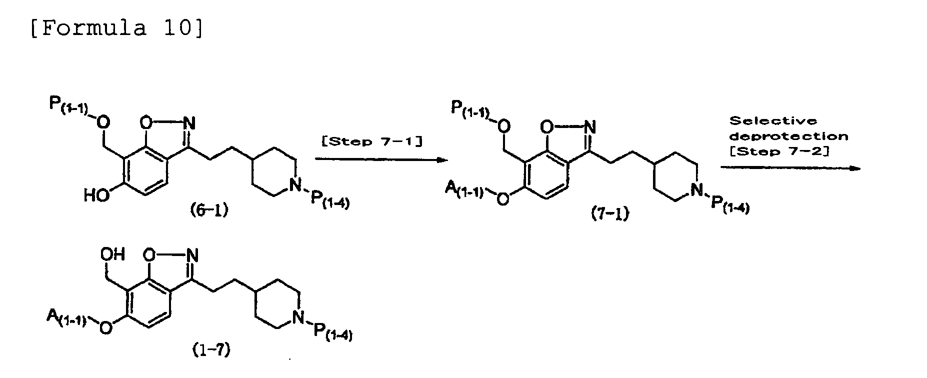

In the formula, the P(1-1) group represents a deprotectable alcoholic hydroxyl protecting group; the P(1-4) group represents a deprotectable amino protecting group; the A(1-1) group represents an R21 group; and the R21 group is as described above.

Preparation Method 7 is a preparation method differing from Preparation Method 1 for obtaining the compound (1-7) obtained by Preparation Method 1 from the compound (6-1) obtained by Preparation Method 6 as a starting material. The compound represented by the formula (1-11), the formula (1-12), the formula (1-13), the formula (2-4), the formula (2-5) or the formula (2-6) can be prepared as the compound represented by the formula (I) by a combination of Preparation Method 7 and Step 1-6 and later of Preparation Method 1 or a combination of Preparation Method 7 and Preparation Method 2.

### [Step 7-1]

Step 7-1 is a step of preparing a compound (7-1) from the compound (6-1) as a starting material by a reaction such as alkylation or Mitsunobu reaction.

When this step is alkylation, the reaction can be performed by the method described in the above Step 3-7.

When this step is Mitsunobu reaction, this step is reaction of condensing the compound (6-1) with an alcohol derivative using an azodicarboxylic acid derivative such as diethyl azodicarboxylate and a triarylphosphine derivative or a trialkylphosphine derivative such as triphenylphosphine. The alcohol derivative used in this step may be a commercially available product used directly or may be prepared from a commercially available product by a method known to a person skilled in the art. This step can be performed under the same conditions as those generally used (for example, conditions described in a document such as PAQUETT, L. A. et al. ed., "Organic Reactions", John Wiley & Sons, Inc. (1992), vol. 42, p. 335-656). Examples of the phosphine derivative used in this step include triphenylphosphine and tributylphosphine. Examples of the azodicarboxylic acid derivative include diisopropyl azodicarboxylate, diethyl azodicarboxylate and 1,1'-(azodicarbonyl)dipiperidine. The solvent used in this step is not particularly limited insofar as it does not inhibit the reaction and allows the starting material to be dissolved therein to a certain extent. Examples of the solvent include tetrahydrofuran, 1,4-dioxane, methylene chloride and toluene. The reaction temperature in this step is usually -10°C to room temperature, and preferably 0°C to room temperature. The reaction time in this step is not particularly limited and is usually 0.5 to 48 hours, and preferably 0.5 to 24 hours.

### [Step 7-2]

Step 7-2 is a step of preparing the compound (1-7) from the compound (7-1) by the method described in the above Step 6-4.

### [Preparation Method 8]

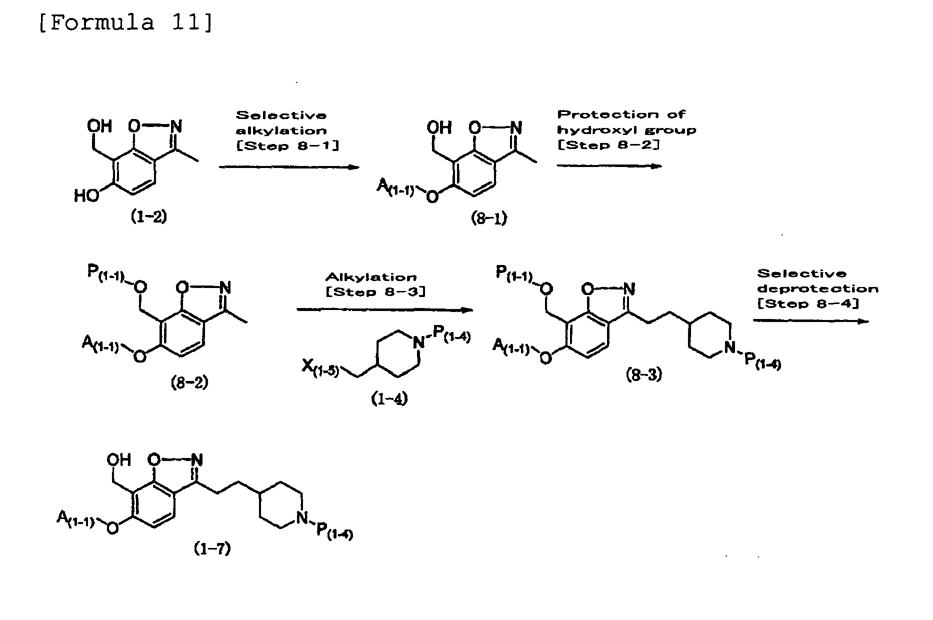

In the formula, the A(1-1) group represents an R21 group; the P(1-1) group represents a deprotectable alcoholic hydroxyl protecting group; the P(1-4) group represents a deprotectable amino protecting group; and the R21 group is as described above.

Preparation Method 8 is a preparation method differing from Preparation Method 1 for obtaining the compound (1-7) obtained by Preparation Method 1 from the compound (1-2) obtained by Preparation Method 1 as a starting material through multiple steps of Step 8-1 to Step 8-4. The compound represented by the formula (1-11), the formula (1-12), the formula (1-13), the formula (2-4), the formula (2-5) or the formula (2-6) can be prepared as the compound represented by the formula (I) by a combination of Preparation Method 8 and Step 1-6 and later of Preparation Method 1 or a combination of Preparation Method 8 and Preparation Method 2.

### [Step 8-1]

Step 8-1 is a step of preparing a compound (8-1) from the compound (1-2) by the method described in the above Step 1-5.

### [Step 8-2]

Step 8-2 is a step of obtaining a compound (8-2) by protecting the alcoholic hydroxyl group of the compound (8-1). This step can be performed by the method of protecting the alcoholic hydroxyl group described in the above Step 1-2.

### [Step 8-3]

Step 8-3 is a step of preparing a compound (8-3) from the compound (8-2) and the compound (1-4) by the method described in the above Step 1-3.

### [Step 8-4]

Step 8-4 is a step of preparing the compound (1-7) from the compound (8-3) by the method described in the above Step 1-4.

### [Preparation Method 9]

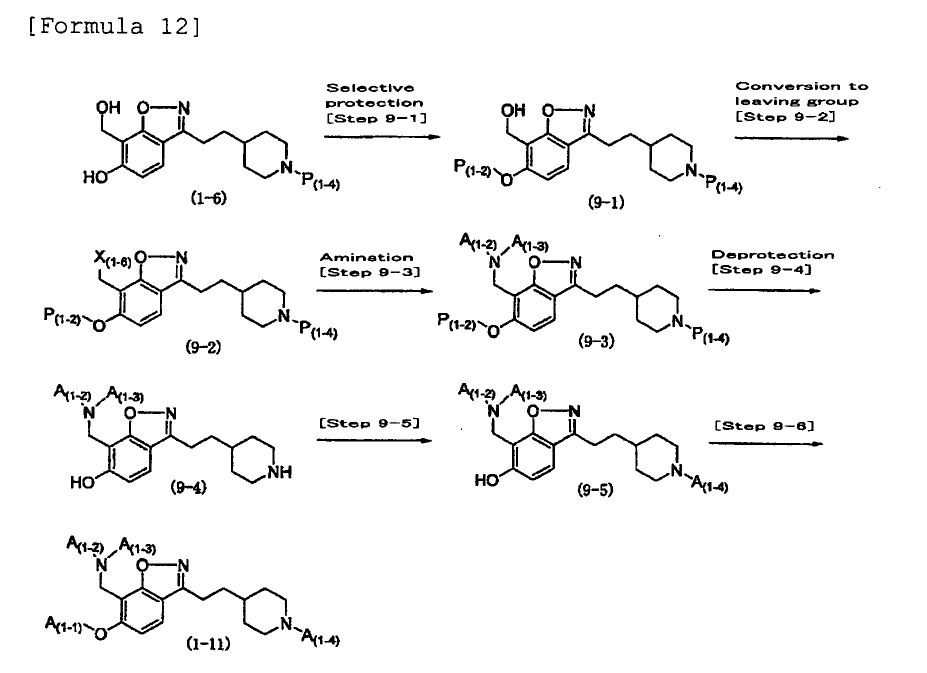

In the formula, the A(1-1) group represents an R21 group; the A(1-2) group and the A(1-3) group each represent an R11 group or an R12 group; the P(1-2) group represents a deprotectable phenolic hydroxyl protecting group; the P(1-4) group represents a deprotectable amino protecting group; the X(1-6) group represents a leaving group; the A(1-4) group represents an R4 group; and the R11 group, the R12 group, the R21 group and the R4 group are as described above.

Preparation Method 9 is a preparation method differing from Preparation Method 1 for obtaining the compound (1-11) obtained by Preparation Method 1 from the compound (1-6) obtained by Preparation Method 1 as a starting material. The compound represented by the formula (1-11), the formula (1-12) or the formula (1-13) can be prepared as the compound represented by the formula (I) by Preparation Method 9 alone or a combination of Preparation Method 9 and Step 1-10 of Preparation Method 1 or a combination of Preparation Method 9 and Step 1-11.

### [Step 9-1]

Step 9-1 is a step of obtaining a compound (9-1) by selective protection of the phenolic hydroxyl group of the compound (1-6). The protection of the phenolic hydroxyl group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 246-292). However, it is necessary to select protection conditions where the alcoholic hydroxyl group is not reacted. Examples of the protecting group to be introduced in this step include allyl-like protecting groups such as a 2-cyclohexen-1-yl group. The solvent used in this step is not particularly limited insofar as it does not inhibit the reaction and allows the starting material to be dissolved therein to a certain extent. Examples of the solvent include tetrahydrofuran, acetonitrile and 1,4-dioxane. The reaction temperature in this step is usually room temperature to solvent reflux temperature. The reaction time in this step is not particularly limited and is usually 0.5 to 48 hours, and preferably 0.5 to 24 hours.

### [Step 9-2]

Step 9-2 is a step of preparing a compound (9-2) from the compound (9-1) by the method described in the above Step 1-8.

### [Step 9-3]

Step 9-3 is a step of preparing a compound (9-3) from the compound (9-2) by the method described in the above Step 1-9.

### [Step 9-4]

Step 9-4 is a step of preparing a compound (9-4) from the compound (9-3) as a starting material by deprotection of the amino group and the phenolic hydroxyl group. The deprotection of the phenolic hydroxyl group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 246-292). The deprotection of the amino group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 494-653). For example, when the P(1-4) group is a tert-butoxycarbonyl group and the P(1-2) group is a 2-cyclohexen-1-yl group, the compound (9-4) can be obtained by deprotection using hydrogen chloride (gas or its solution). The solvent used in this step is not particularly limited insofar as it does not inhibit the reaction and allows the starting material to be dissolved therein to a certain extent. For example, when the protecting groups to be deprotected in this step are a tert-butoxycarbonyl group and a 2-cyclohexen-1-yl group, the solvent may be methanol, ethanol or the like. For example, when the protecting groups to be deprotected in this step are a tert-butoxycarbonyl group and a 2-cyclohexen-1-yl group, the reaction temperature in this step is usually 0°C to 40°C, and preferably 0°C to room temperature. The reaction time in this step is not particularly limited and is usually 0.5 to 48 hours, and preferably 0.5 to 24 hours.

### [Step 9-5]

Step 9-5 is a step of obtaining a compound (9-5) by converting the secondary amino group of the compound (9-4) to a tertiary amino group. This step employs a reaction such as reductive amination. The reductive amination in this step can be performed by the method described in the above Step 1-7.

### [Step 9-6]

Step 9-6 is a step of preparing the compound (1-11) from the compound (9-5) by the method described in the above Step 7-1.

### [Preparation Method 10]

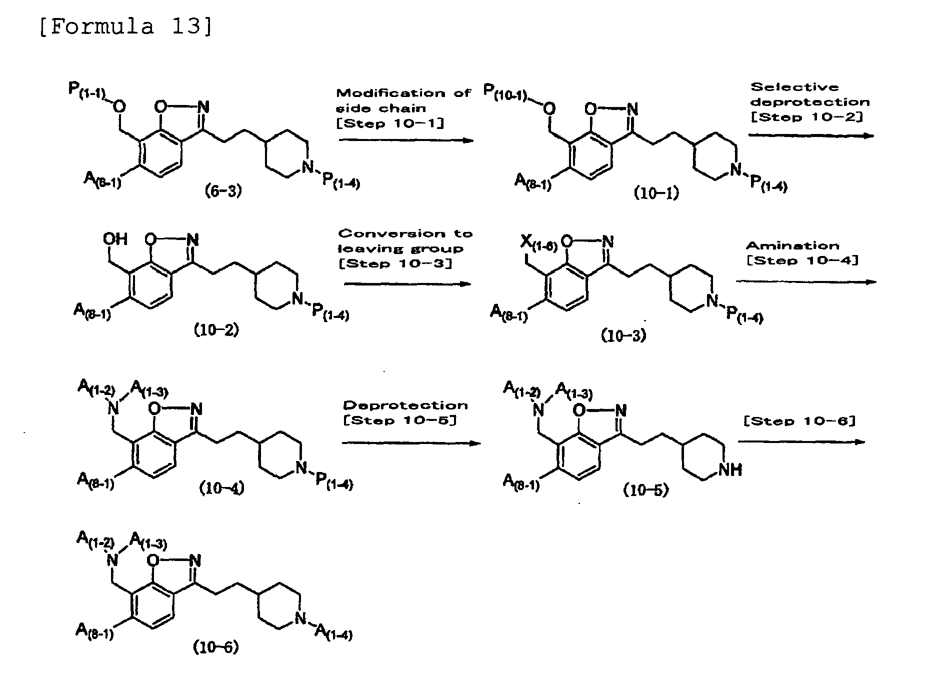

In the formula, the P(1-1) group and the P(10-1) group each represent a deprotectable alcoholic hydroxyl protecting group; the P(1-4) group represents a deprotectable amino protecting group; the A(1-2) group and the A(1-3) group each represent an R11 group or an R12 group; the A(6-1) group and the A(8-1) group each represent a -(CH₂)n-(O)m-R21 group; the X (1-6) group represents a leaving group; the A(1-4) group represents an R4 group; and the R11 group, the R12 group, the R21 group, the R4 group, m and n are as described above.

Preparation Method 10 is a method for synthesizing the compound of the formula (I) according to the present invention from the compound (6-3) obtained by Preparation Method 6 as a starting material through multiple steps of Step 10-1 to Step 10-6. However, the compound represented by the formula (I) which can be obtained by the preparation method is a compound represented by the formula (10-6).

### [Step 10-1]

Step 10-1 is a step of obtaining a compound (10-1) from the compound (6-1) as a starting material. In this step, the A(6-1) group is converted to an A(8-1) group by chemical modification using a reaction known to a person skilled in the art. The P(1-1) group is further converted to a P(10-1) group where necessary. Accordingly, the P(1-1) group and the P(10-1) group may be the same or different. Examples of the reaction used in this step and known to a person skilled in the art include, but are not limited to, (1) cleavage oxidation of a double bond using osmium tetroxide, ozonolysis or the like, (2) alkylation reaction using a compound having a leaving group (such as iodomethane), (3) oxidation reaction of an aromatic aldehyde into a carboxylic acid using a chlorite such as sodium chlorite, (4) amidation by condensation of a carboxylic acid with an amine using a BOP reagent, 1,1'-carbonyldiimidazole or the like, (5) reduction using a reducing agent such as sodium borohydride, (6) nucleophilic substitution using a nucleophilic reagent such as sodium azide, (7) reduction of an azide group using a phosphine reagent such as triphenylphosphine (Staudinger reaction), (8) Wittig reaction, (9) Mitsunobu reaction and (10) protection and deprotection of various functional groups described in a document such as T. W. GREENE and P. G. M. WUTS, "Protective Groups in Organic Chemistry, Third Edition", John Wiley & Sons, Inc. (1999). The reactions known to a person skilled in the art may be used in combination as necessary.

### [Step 10-2]

Step 10-2 is a step of preparing a compound (10-2) by selective deprotection of the alcoholic hydroxyl group of the compound (10-1). The deprotection of the alcoholic hydroxyl group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 17-245). However, it is necessary to select deprotection conditions where the amino protecting group (P(1-4) group) is not reacted. Preferably, the amino protecting group is a tert-butoxycarbonyl group and the hydroxyl protecting group is a ketalic protecting group such as a tetrahydro-2H-pyran-2-yl group, a protecting silyl group such as a tert-butyldimethylsilyl group or the like. Examples of the conditions for selectively deprotecting the hydroxyl protecting group in a combination of a tert-butoxycarbonyl group as an amino protecting group and a tetrahydro-2H-pyran-2-yl group as a hydroxyl protecting group include known reaction conditions in the presence of a weak acid (for example, conditions described in documents such as KLUTCHKO, S.; HAMBY, J. M.; REILY, M.; TAYLOR, M. D.; HODGES, J. C.; Synth. Commun., 1993, 23 (7), 971 and SASAKI, N. A.; PAULY, R.; FONTAINE, C.; CHIARONI, A.; RICHE, C.; POTIER, P.; Tetrahedron Lett., 1994, 35 (2), 241). Examples of the weak acid used in this step when the above combination of protective groups is used include pyridinium p-toluenesulfonate and acetic acid. The solvent used in this step is not particularly limited insofar as it does not inhibit the reaction and allows the starting material to be dissolved therein to a certain extent. Examples of the solvent include methanol and ethanol. The reaction temperature in this step is usually room temperature to 100°C, and preferably room temperature to 70°C. The reaction time in this step is not particularly limited and is usually 0.5 to 48 hours, and preferably 0.5 to 24 hours.

### [Step 10-3]

Step 10-3 is a step of preparing a compound (10-3) from the compound (10-2) by the method described in the above Step 1-8.

### [Step 10-4]

Step 10-4 is a step of preparing a compound (10-4) from the compound (10-3) by the method described in the above Step 1-9.

### [Step 10-5]

Step 10-5 is a step of preparing a compound (10-5) from the compound (10-4) by the method described in the above Step 1-6.

### [Step 10-6]

Step 10-6 is a step of preparing a compound (10-6) from the compound (10-5) by the method described in the above Step 1-7.

### [Preparation Method 11]

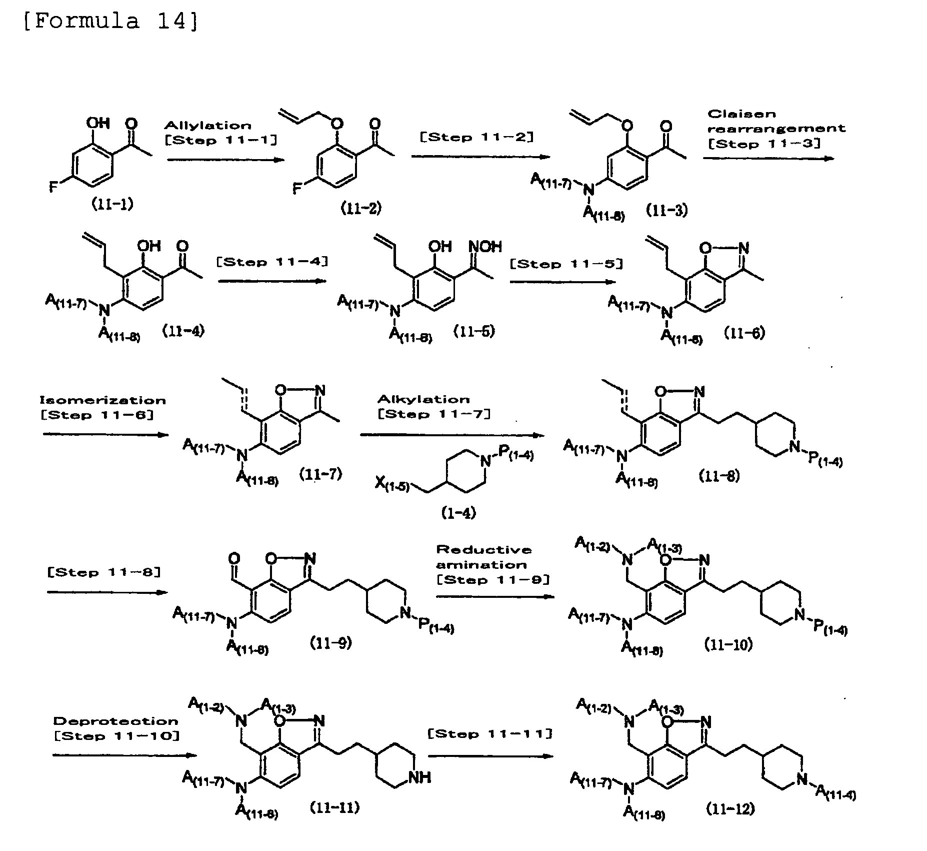

In the formula, the -NA(11-7)A(11-8) group represents an R21 group; the A(1-2) group and the A(1-3) group each represent an R11 group or an R12 group; the P(1-4) group represents a deprotectable amino protecting group; the X(1-5) group represents a leaving group; the A(11-4) group represents an R4 group; and the R11 group, the R12 group, the R21 group and the R4 group are as described above.

Preparation Method 11 is a method for synthesizing the compound of the formula (I) according to the present invention from a commercially available compound (11-1) as a starting material through multiple steps of Step 11-1 to Step 11-11. However, the compound represented by the formula (I) which can be obtained by the preparation method is a compound represented by the formula (11-12).

### [Step 11-1]

Step 11-1 is a step of preparing a compound (11-2) from the compound (11-1) by the method described in the above Step 3-1.

### [Step 11-2]

Step 11-2 is a step of preparing a compound (11-3) from the compound (11-2) by a reaction such as aromatic nucleophilic substitution (SNAr reaction). The aromatic nucleophilic substitution (SNAr reaction) in this step can be performed under the conditions described in the above Step 2-4.

### [Step 11-3]

Step 11-3 is a step of preparing a compound (11-4) from the compound (11-3) by the method described in the above Step 3-2.

### [Step 11-4]

Step 11-4 is a step of preparing a compound (11-5) by reacting the compound (11-4) with a hydroxylamine salt in the presence of a base. This step can be performed under the same conditions as those generally used (for example, conditions described in documents such as BEGER, J.; BINTE, H.-J.; BRUNNE, L.; NEUMANN, R.; J. Prakt. Chem/Chem-Ztg., 1992, 334 (3), 269 and NUHRICH, A.; VARACHE-LEMBEGE, M.; RENARD, P.; DEVAUX, G.; Eur. J. Med. Chem., 1994, 29 (1), 75). Examples of the base used in this step include sodium acetate, sodium hydroxide and pyridine. The solvent used in this step is not particularly limited insofar as it does not inhibit the reaction and allows the starting material to be dissolved therein to a certain extent. Examples of the solvent include mixed solvents of alcohols such as ethanol and water. The reaction temperature in this step is usually room temperature to solvent reflux temperature, and preferably 50°C to solvent reflux temperature. The reaction time in this step is not particularly limited and is usually 0.5 to 12 hours, and preferably 0.5 to 6 hours.

### [Step 11-5]

Step 11-5 is a step of preparing a compound (11-6) from the compound (11-5) as a starting material. This step can be performed by a reaction such as (1) acylation of a hydroxyl group of an oxime and subsequent cyclization or (2) cyclization by Mitsunobu reaction. This step can be performed under the same conditions as those generally used (for example, conditions described in documents such as BORSCHE, W.; HAHN-WEINHEIMER, P.; Justus Liebigs Ann. Chem., 1950, 570, 155 and POISSONNET, G.; Synth. Commun., 1997, 27 (22), 3839). For example, when this step is acylation of a hydroxyl group of an oxime and subsequent cyclization, the acylating reagent used in this step is preferably acetic anhydride-sodium acetate. When this step is acylation of a hydroxyl group of an oxime and subsequent cyclization, the solvent used in this step is not particularly limited insofar as it does not inhibit the reaction and allows the starting material to be dissolved therein to a certain extent. Examples of the solvent include N,N-dimethylformamide. When this step is acylation of a hydroxyl group of an oxime and subsequent cyclization, the reaction temperature in this step is usually room temperature to solvent reflux temperature, and preferably 100°C to solvent reflux temperature. When this step is acylation of a hydroxyl group of an oxime and subsequent cyclization, the reaction time in this step is not particularly limited and is usually 0.5 to 6 hours, and preferably 0.5 to 3 hours. When this step is acylation of a hydroxyl group of an oxime and subsequent cyclization, excellent results such as a reduced reaction time and an improved yield may be obtained by performing the reaction of this step using a microwave reactor.

### [Step 11-6]

Step 11-6 is a step of preparing a compound (11-7) from the compound (11-6) by the method described in the above Step 3-3.

### [Step 11-7]

Step 11-7 is a step of preparing a compound (11-8) from the compound (11-7) and the compound (1-4) by the method described in the above Step 1-3.

### [Step 11-8]

Step 11-8 is a step of preparing a compound (11-9) from the compound (11-8) by the method described in the above Step 3-8.

### [Step 11-9]

Step 11-9 is a step of preparing a compound (11-10) from the compound (11-9) and a primary or secondary amine by the reductive amination method described in the above Step 1-7.

### [Step 11-10]

Step 11-10 is a step of preparing a compound (11-11) from the compound (11-10) by the method described in the above Step 1-6.

### [Step 11-11]

Step 11-11 is a step of preparing a compound (11-12) from the compound (11-11) by a reaction such as reductive amination or aromatic nucleophilic substitution (SNAr reaction). When this step is reductive amination, the reaction can be performed by the method described in the above Step 1-7. When this step is aromatic nucleophilic substitution (SNAr reaction), the reaction can be performed by the method described in the above Step 2-4.

### [Preparation Method 12]

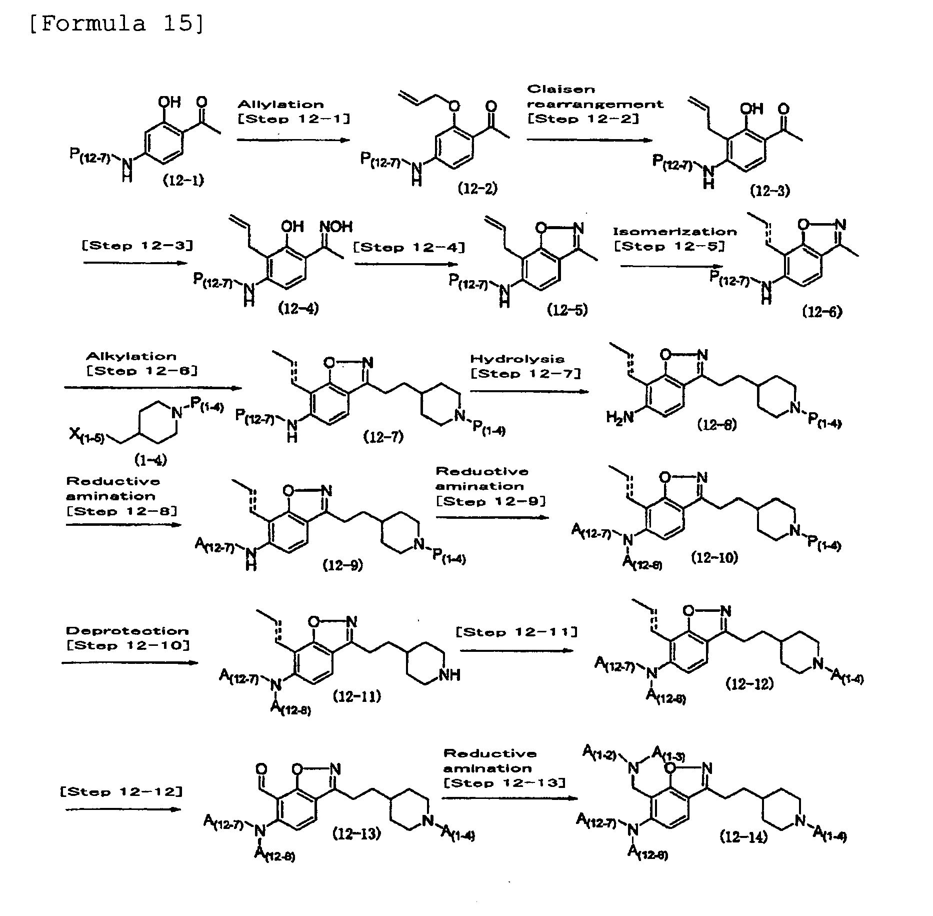

In the formula, the -NA(12-7)A(12-8) group represents an R21 group; the A(1-2) group and the A(1-3) group each represent an R11 group or an R12 group; the P(12-7) group and the P(1-4) group each represent a deprotectable amino protecting group; the X(1-5) group represents a leaving group; the A(1-4) group represents an R4 group; and the R11 group, the R12 group, the R21 group and the R4 group are as described above.

Preparation Method 12 is a method for synthesizing the compound of the formula (I) according to the present invention from a compound (12-1) as a starting material through multiple steps of Step 12-1 to Step 12-13. However, the compound represented by the formula (I) which can be obtained by the preparation method is a compound represented by the formula (12-14). The compound (12-1) may be a commercially available product used directly or may be prepared from a commercially available product by a method known to a person skilled in the art. Further, the compound may be prepared by a method described in Examples. Examples of the P(12-7) group include acyl groups such as an acetyl group. For example, when the P(12-7) group is an acetyl group, the compound (12-1) is a known compound. Examples of the synthesis method for the compound include a method described in NERENBERG, J. B.; ERB, J. L.; BERGMAN, J. M.; O'MALLEY, S.; CHANG, R. S. L.; RAYMOND, S. L.; SCOTT, A. L.; BROTE, T. P.; BOCK, M. G.; Bioorg. Med. Chem. Lett., 1999, 9 (2), 291.

### [Step 12-1]

Step 12-1 is a step of preparing a compound (12-2) from the compound (12-1) by the method described in the above Step 3-1.

### [Step 12-2]

Step 12-2 is a step of preparing a compound (12-3) from the compound (12-2) by the method described in the above Step 3-2.

### [Step 12-3]

Step 12-3 is a step of preparing a compound (12-4) from the compound (12-3) by the method described in the above Step 11-4.

### [Step 12-4]

Step 12-4 is a step of preparing a compound (12-5) from the compound (12-4) by the method described in the above Step 11-5.

### [Step 12-5]

Step 12-5 is a step of obtaining a compound (12-6) from the compound (12-5) as a starting material by isomerization of the double bond using a transition metal catalyst. In this step, the reaction can be carried out under the same conditions as those usually used (for example, conditions described in GREEN, I. R.; HUGO, V. I.; OOSTHUIZEN, F.; GILES, R. G. F.; Synth. Commun., 1996, 26 (5), 867, SINGH, V.; SAMANTA, B.; Synth. Commun., 1997, 27 (24), 4235 and JING, X.; GU, W.; BIE, P.; REN, X.; PAN, X.; Synth. Commun., 2001, 31 (6), 861). The transition metal catalyst used in this step is not particularly limited. Examples of the transition metal catalyst include bis(acetonitrile)dichloropalladium (II) and palladium (II) chloride. This step may be performed in an organic solvent such as methylene chloride or methanol. Methylene chloride is preferably used. The solvent used is not particularly limited insofar as it does not inhibit the reaction and allows the starting material to be dissolved therein to a certain extent. The reaction temperature in this step is usually room temperature to solvent reflux temperature. The reaction time in this step is not particularly limited and is usually 0.5 to 48 hours, and preferably 0.5 to 24 hours.

### [Step 12-6]

Step 12-6 is a step of preparing a compound (12-7) from the compound (12-6) and the compound (1-4) by the method described in the above Step 1-3.

### [Step 12-7]

Step 12-7 is a step of obtaining a compound (12-8) from the compound (12-7) as a starting material by selective deprotection.
The deprotection of the amino group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 246-292). However, it is necessary to select deprotection conditions where the aliphatic amino protecting group (P(1-4) group) is not reacted and only the anilinic amino protecting group (P(12-7) group) is deprotected. Preferably, the P(1-4) group is a tert-butoxycarbonyl group and the P(12-7) group is an acyl group such as an acetyl group. Examples of the conditions for selectively deprotecting the anilinic amino group in this combination include conditions using a base such as sodium hydroxide or potassium hydroxide. When the above combination of the amino protecting groups is used, the solvent used in this step is not particularly limited insofar as it does not inhibit the reaction and allows the starting material to be dissolved therein to a certain extent. Examples of the solvent include mixed solvents of alcohols such as ethanol and water. When the above combination of the amino protecting groups is used, the reaction temperature in this step is usually room temperature to solvent reflux temperature, and preferably 50°C to solvent reflux temperature. The reaction time in this step is not particularly limited and is usually 0.5 to 12 hours, and preferably 0.5 to 6 hours.

### [Step 12-8]

Step 12-8 is a step of preparing a compound (12-9) from the compound (12-8) and a ketone or aldehyde by the reductive amination method described in the above Step 1-7. (1) When the A(12-7) group and the A(12-8) group are the same or (2) when the ketone or aldehyde is divalent (specifically, when R21 is a heterocycloalkyl group), this step may be performed simultaneously with Step 12-9.

### [Step 12-9]

Step 12-9 is a step of preparing a compound (12-10) from the compound (12-9) and a ketone or aldehyde by the reductive amination method described in the above Step 1-7. When the A(12-7) group and the A(12-8) group are the same or (2) when the ketone or aldehyde is divalent (specifically, when R21 is a heterocycloalkyl group), this step may be performed simultaneously with Step 12-8.

### [Step 12-10]

Step 12-10 is a step of preparing a compound (12-11) from the compound (12-10) by the method described in the above Step 1-6.

### [Step 12-11]

Step 12-11 is a step of preparing a compound (12-12) from the compound (12-11) and an aldehyde or ketone by the reductive amination method described in the above Step 1-7.

### [Step 12-12]

Step 12-12 is a step of preparing a compound (12-13) from the compound (12-12) by the method described in the above Step 3-8.

### [Step 12-13]

Step 12-13 is a step of preparing a compound (12-14) from the compound (12-13) and a primary or secondary amine by the reductive amination method described in the above Step 1-7.

### [Preparation Method 13]

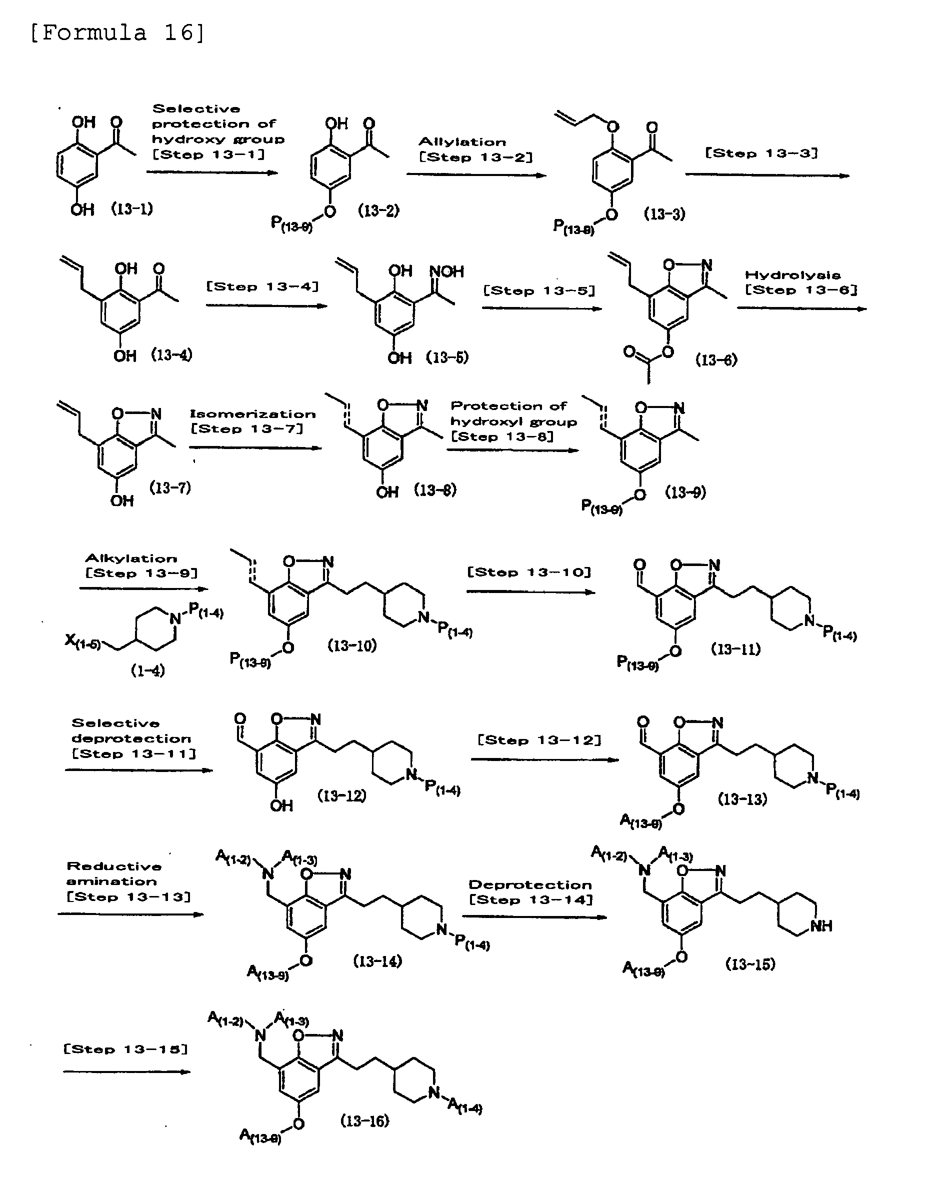

In the formula, the A(13-.9) group represents an R21 group; the A(1-2) group and the A(1-3) group each represent an R11 group or an R12 group; the P(13-9) group represents a deprotectable phenolic hydroxyl protecting group; the P(1-4) group represents a deprotectable amino protecting group; the X(1-5) group represents a leaving group; the A(1-4) group represents an R4 group; and the R11 group, the R12 group, the R21 group and the R4 group are as described above.

Preparation Method 13 is a method for synthesizing the compound of the formula (I) according to the present invention from a compound (13-1) as a starting material through multiple steps of Step 13-1 to Step 13-15. However, the compound represented by the formula (I) which can be obtained by the preparation method is a compound represented by the formula (13-16). A commercially available compound may be used as the compound (13-1). Further, the compound (13-1) is a known compound. Examples of the synthesis method for the compound include methods described in KHANNA, R. N.; SINGH, K. P.; SHARMA, J.; Org. Prep. Proced. Int., 1992, 24 (6), 687 and BOYER, J. L.; KRUM, J. E.; MYERS, M. C.; FAZAL, A. N.; WIGAL, C. T.; J. Org. Chem., 2000, 65 (15), 4712.

### [Step 13-1]

Step 13-1 is a step of obtaining a compound (13-2) by protecting only the hydroxyl group not forming an intramolecular hydrogen bond with an acetyl group among the two hydroxyl groups of the compound (13-1). The protection of the phenolic hydroxyl group in this step can be performed under the same conditions as those generally used (for example, conditions described in documents such as TAKAHASHI, K.; SHIMIZU, S.; OGATA, M.; Heterocycles, 1985, 23 (6), 1483 and SHARMA, A. P.; SAEED, A.; DURANI, S.; KAPIL, R. S.; J. Med. Chem., 1990, 33 (12), 3222). The hydroxyl protecting group used in this step is not particularly limited. A tetrahydro-2H-pyran-2-yl group is preferably used. For example, when the protecting group is a tetrahydro-2H-pyran-2-yl group, the reagent for introducing the protecting group may be a combination of 3,4-dihydro-2H-pyrane and an acid as a catalyst. Examples of the acid used as a catalyst include p-toluenesulfonic acid and pyridinium p-toluenesulfonate. Preferably, p-toluenesulfonic acid is used. For example, when the protecting group is a tetrahydro-2H-pyran-2-yl group, the solvent used in this step is not particularly limited insofar as it does not inhibit the reaction and allows the starting material to be dissolved therein to a certain extent. Organic solvents such as 1,4-dioxane may be used. For example, when the protecting group is a tetrahydro-2H-pyran-2-yl group, the reaction temperature in this step is usually 0°C to 50°C, and more preferably room temperature. The reaction time in this step is not particularly limited and is usually 0.5 to 24 hours, and preferably 0.5 to 12 hours.

### [Step 13-2]

Step 13-2 is a step of preparing a compound (13-3) from the compound (13-2) by the method described in the above Step 3-1.

### [Step 13-3]

Step 13-3 is a step of preparing a compound (13-4) from the compound (13-3) by Claisen rearrangement and deprotection of the phenolic hydroxyl group (P(13-9) group). The Claisen rearrangement can be performed by the method described in the above Step 3-2. The deprotection of the phenolic hydroxyl group can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 246-292). When the P(13-9) group is a tetrahydro-2H-pyran-2-yl group, the deprotection can be performed simultaneously with the Claisen rearrangement.

### [Step 13-4]

Step 13-4 is a step of preparing a compound (13-5) from the compound (13-4) by the method described in the above Step 11-4.

### [Step 13-5]

Step 13-5 is a step of preparing a compound (13-6) from the compound (13-5) by the method described in the above Step 11-5. In this step, the phenolic hydroxyl group of the product is acetylated simultaneously with cyclization of the benzisoxazole ring.

### [Step 13-6]

Step 13-6 is a step of preparing a compound (13-7) by hydrolyzing the acetoxy group of the compound (13-6) with an acid or a base. The hydrolysis in this step can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 276-278). Examples of the acid used in this step include hydrochloric acid. For example, when the acid used in this step is hydrochloric acid, the solvent used in this step is not particularly limited insofar as it does not inhibit the reaction and allows the starting material to be dissolved therein to a certain extent. Examples of the solvent include mixed solvents of alcohols such as methanol and ethanol and water. For example, when the acid used in this step is hydrochloric acid, the reaction temperature in this step is usually room temperature to solvent reflux temperature, and preferably 50°C to solvent reflux temperature. For example, when the acid used in this step is hydrochloric acid, the reaction time in this step is not particularly limited and is usually 0.5 to 24 hours, and preferably 0.5 to 12 hours.

### [Step 13-7]

Step 13-7 is a step of preparing a compound (13-8) from the compound (13-7) by the method described in the above Step 3-3.

### [Step 13-8]

Step 13-8 is a step of preparing a compound (13-9) by protection of the phenolic hydroxyl group of the compound (13-8). This step can be performed by the method described in the above Step 3-4.

### [Step 13-9]

Step 13-9 is a step of preparing a compound (13-10) from the compound (13-9) and the compound (1-4) by the method described in the above Step 1-3.

### [Step 13-10]

Step 13-10 is a step of preparing a compound (13-11) from the compound (13-10) by the method described in the above Step 3-8.

### [Step 13-11]

Step 13-11 is a step of preparing a compound (13-12) from the compound (13-11) as a starting material by selective deprotection of the phenolic hydroxyl group. The phenolic hydroxyl group can be deprotected by the method described in the above Step 1-4.

### [Step 13-12]

Step 13-12 is a step of preparing a compound (13-13) from the compound (13-12) by the method described in the above Step 7-1.

### [Step 13-13]

Step 13-13 is a step of preparing a compound (13-14) from the compound (13-13) and a primary or secondary amine by the reductive amination method described in the above Step 1-7.

### [Step 13-14]

Step 13-14 is a step of preparing a compound (13-15) from the compound (13-14) by the method described in the above Step 1-6.

### [Step 13-15]

Step 13-15 is a step of preparing a compound (13-16) from the compound (13-15) by the method described in the above Step 1-7.

### [Preparation Method 14]

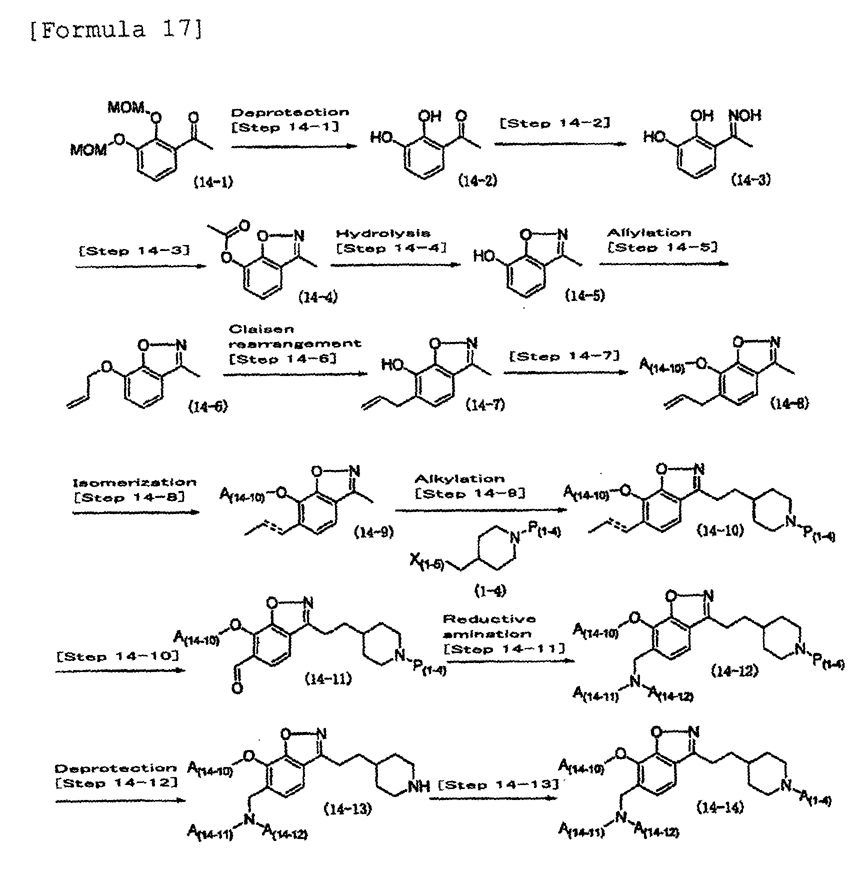

In the formula, the A(14-10) group represents an R21 group; the A(14-11) group and the A(14-12) group each represent an R11 group or an R12 group; the P(1-4) group represents a deprotectable amino protecting group; the X(1-5) group represents a leaving group; the A(1-4) group represents an R4 group; and the R11 group, the R12 group, the R21 group and the R4 group are as described above.

Preparation Method 14 is a method for synthesizing the compound of the formula (I) according to the present invention from a compound (14-1) as a starting material through multiple steps of Step 14-1 to Step 14-13. However, the compound represented by the formula (I) which can be obtained by the preparation method is a compound represented by the formula (14-14). The compound (14-1) and a compound (14-2) are known compounds and can be prepared from a commercially available compound by a method known to a person skilled in the art (for example, a method under conditions described in a document such as KAGAWA, H.; SHIGEMATSU, A.; OHTA, S.; HARIGAYA, Y.; Chem. Pharm. Bull., 2005, 53(5), 547) .

### [Step 14-1]

Step 14-1 is a step of preparing a compound (14-2) by deprotection of the compound (14-1) with an acid. This step can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 289). Examples of the acid used in this step include hydrochloric acid, hydrobromic acid and acetic acid. Hydrochloric acid or hydrobromic acid is preferably used. For example, when hydrobromic acid is used, the solvent used in this step is not particularly limited insofar as it does not inhibit the reaction and allows the starting material to be dissolved therein to a certain extent. Examples of the solvent include water. For example, when hydrobromic acid is used, the reaction temperature in this step is 50°C to solvent reflux temperature, and preferably 70°C to solvent reflux temperature. For example, when hydrobromic acid is used, the reaction time in this step is not particularly limited and is usually 0.5 to 12 hours, and preferably 0.5 to 6 hours.

### [Step 14-2]

Step 14-2 is a step of preparing a compound (14-3) from the compound (14-2) by the method described in the above Step 11-4.

### [Step 14-3]

Step 14-3 is a step of preparing a compound (14-4) from the compound (14-3) by the method described in the above Step 11-5. In this step, the phenolic hydroxyl group of the product is acetylated simultaneously with cyclization of the benzisoxazole ring.

### [Step 14-4]

Step 14-4 is a step of preparing a compound (14-5) by hydrolyzing the acetoxy group of the compound (14-4) with an acid or a base. The hydrolysis in this step can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 276-278). Examples of the base used in this step include sodium hydroxide, potassium hydroxide, sodium carbonate and potassium carbonate. For example, when the base used in this step is sodium hydroxide, the solvent used in this step is not particularly limited insofar as it does not inhibit the reaction and allows the starting material to be dissolved therein to a certain extent. Examples of the solvent include mixed solvents of alcohols such as methanol and ethanol and water. For example, when the base used in this step is sodium hydroxide, the reaction temperature in this step is usually room temperature to solvent reflux temperature. For example, when the base used in this step is sodium hydroxide, the reaction time in this step is not particularly limited and is usually 0.5 to 6 hours, and preferably 0.5 to 2 hours.

### [Step 14-5]

Step 14-5 is a step of preparing a compound (14-6) from the compound (14-5) by the method described in the above Step 3-1.

### [Step 14-6]

Step 14-6 is a step of preparing a compound (14-7) from the compound (14-6) by the method described in the above Step 3-2.

### [Step 14-7]

Step 14-7 is a step of preparing a compound (14-8) from the compound (14-7) by the method described in the above Step 7-1.

### [Step 14-8]

Step 14-8 is a step of preparing a compound (14-9) from the compound (14-8) by the method described in the above Step 3-3.

### [Step 14-9]

Step 14-9 is a step of preparing a compound (14-10) from the compound (14-9) and the compound (1-4) by the method described in the above Step 1-3.

### [Step 14-10]

Step 14-10 is a step of preparing a compound (14-11) from the compound (14-10) by the method described in the above Step 3-8.

### [Step 14-11]

Step 14-11 is a step of preparing a compound (14-12) from the compound (14-11) and a primary or secondary amine by the reductive amination method described in the above Step 1-7.

### [Step 14-12]

Step 14-12 is a step of preparing a compound (14-13) from the compound (14-12) by the method described in the above Step 1-6.

### [Step 14-13]

Step 14-13 is a step of preparing a compound (14-14) from the compound (14-13) by the method described in the above Step 1-7.

### [Preparation Method 15]

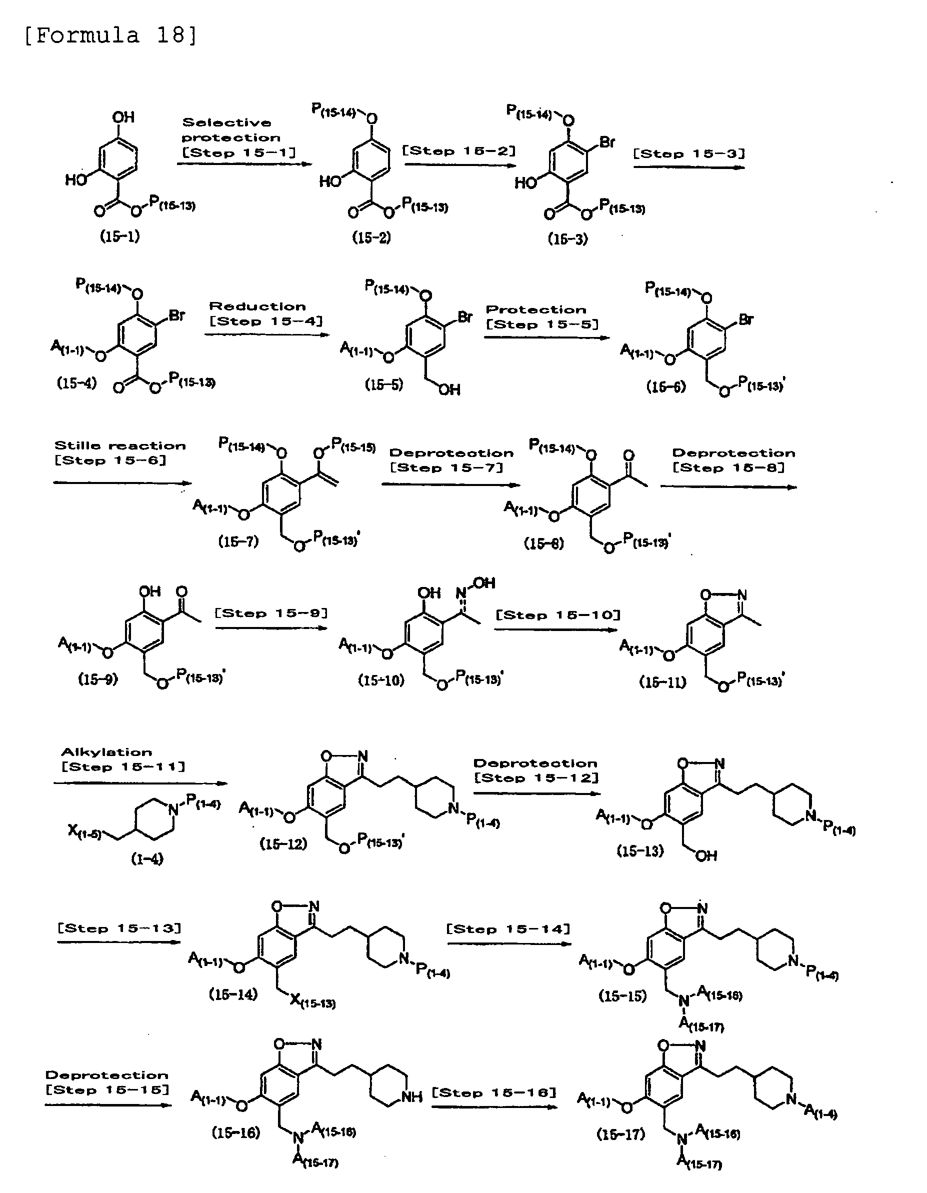

In the formula, the A(1-1) group represents an R21 group; the A(15-16) group and the A(15-17) group each represent an R11 group or an R12 group; the P(1-4) group represents a deprotectable amino protecting group; the P(15-13) group represents a carboxyl protecting group, where the protecting group is deprotected when the ester is reduced to an alcohol; the P(15-14) group represents a deprotectable phenolic hydroxyl protecting group; the P(15-13)' group represents a deprotectable alcoholic hydroxyl protecting group; the X(1-5) group represents a leaving group; the A(1-4) group represents an R4 group; and the R11 group, the R12 group, the R21 group and the R4 group are as described above.

Preparation Method 15 is a method for synthesizing the compound of the formula (I) according to the present invention from a compound (15-1) as a starting material through multiple steps of Step 15-1 to Step 15-16. However, the compound represented by the formula (I) which can be obtained by the preparation method is a compound represented by the formula (15-17). The P(15-13) group is not particularly limited. Preferably, a C1-6 alkyl group is used. For example, when the P(15-13) group is a methyl group, an ethyl group or the like, the compound (15-1) is a commercially available compound and can be prepared from 2,4-dihydroxybenzoic acid by a known method. Examples of the known synthesis method for the compound include methods described in Tatsuta, K.; Tanaka, Y.; Kojima, M.; Ikegami, H.; Chem. Lett., 2002, 262 and GUO, W.; LI, J.; WU, W.; ZHOU, P.; XIA, C.; Synth. Commun., 2005, 35, 145.

### [Step 15-1]

Step 15-1 is a step of obtaining a compound (15-2) by protecting only the hydroxyl group not forming an intramolecular hydrogen bond with an ester group among the two hydroxyl groups of the compound (15-1). The protection of the phenolic hydroxyl group in this step can be performed under the same conditions as those generally used (for example, conditions described in documents such as TAKAHASHI, K.; SHIMIZU, S.; OGATA, M.; Heterocycles, 1985, 23 (6), 1483 and SHARMA, A. P.; SAEED, A.; DURANI, S.; KAPIL, R. S.; J. Med. Chem., 1990, 33 (12), 3222). The hydroxyl protecting group used in this step is not particularly limited. A methoxymethyl group is preferably used. For example, when the protecting group is a methoxymethyl group, the reagent for introducing the protecting group may be a combination of chloromethyl methyl ether and a base. Examples of the base used include sodium carbonate and potassium carbonate. For example, when the protecting group is a methoxymethyl group, the solvent used in this step is not particularly limited insofar as it does not inhibit the reaction and allows the starting material to be dissolved therein to a certain extent. Organic solvents such as acetone and methyl ethyl ketone may be used. For example, when the protecting group is a methoxymethyl group, the reaction temperature in this step is usually room temperature to solvent reflux temperature. The reaction time in this step is not particularly limited and is usually 0.5 to 24 hours, and preferably 0.5 to 12 hours.

### [Step 15-2]

Step 15-2 is a step of preparing a compound (15-3) from the compound (15-2) as a starting material by bromination of the aromatic ring. The bromination in this step can be performed under the same conditions as those generally used (for example, conditions described in documents such as BOX, V. G. S.; YIANNIKOUROS, G. P.; Heterocycles, 1990, 31 (7), 1261 and SIMIG, G.; SCHLOSSER, M.; Acta. Chim. Hung., 1994, 131 (2), 217). Examples of the brominating reagent used in this step include N-bromosuccinimide and bromine. N-bromosuccinimide is preferably used. When N-bromosuccinimide is used for bromination, the solvent used in this step is not particularly limited insofar as it does not inhibit the reaction and allows the starting material to be dissolved therein to a certain extent. Organic solvents such as acetonitrile, methylene chloride and carbon tetrachloride may be used. The reaction temperature in this step is usually room temperature to solvent reflux temperature. The reaction time in this step is not particularly limited and is usually 0.5 to 24 hours, and preferably 0.5 to 12 hours.

### [Step 15-3]

Step 15-3 is a step of preparing a compound (15-4) from the compound (15-3) by the method described in the above Step 7-1.

### [Step 15-4]

Step 15-4 is a step of preparing a compound (15-5) from the compound (15-4) as a starting material by reduction of the ester. The reduction in this step can be performed under the same conditions as those generally used (for example, conditions described in documents such as BOX, V. G. S.; YIANNIKOUROS, G. P.; Heterocycles, 1990, 31 (7), 1261 and SIMIG, G.; SCHLOSSER, M.; Acta. Chim. Hung., 1994, 131 (2), 217). Examples of the reducing agent used in this step include formic acid, formates such as lithium aluminum hydride, lithium borohydride and diisobutylaluminum hydride. Lithium aluminum hydride is preferably used. For example, when lithium aluminum hydride is used for reduction, the solvent used in this step is not particularly limited insofar as it does not inhibit the reaction and allows the starting material to be dissolved therein to a certain extent. Organic solvents such as diethyl ether and tetrahydrofuran may be used. The reaction temperature in this step is usually -50°C to room temperature, and preferably -20°C to 0°C. The reaction time in this step is not particularly limited and is usually 0.25 to 6 hours, and preferably 0.25 to 1 hour.

### [Step 15-5]

Step 15-5 is a step of preparing a compound (15-6) from the compound (15-5) as a starting material by protection of the alcoholic hydroxyl group. This step can be performed by the method of protecting the alcoholic hydroxyl group described in the above Step 1-2.

### [Step 15-6]

Step 15-6 is a step of preparing a compound (15-7) from the compound (15-6) as a starting material by Stille reaction using a transition metal catalyst. The trialkyltin derivative used in this step may be a commercially available product used directly or may be prepared from a commercially available product by a method known to a person skilled in the art. In this step, the reaction can be performed under conditions generally used (for example, conditions described in documents such as J. Tsuji, "Palladium Reagents and Catalysts", John Wiley & Sons (1995), DHAR, T. G. M.; IWANOWICZ, E. J.; ET AL.; Bioorg. Med. Chem. Lett., 2003, 13 (20), 3557 and GAN, T.; VAN ORNUM, S. G.; COOK, J. M.; Tetrahedron Lett., 1997, 38 (49), 8453). Examples of the transition metal catalyst used in this step include dichlorobis(triphenylphosphine)palladium (II), tetrakis(triphenylphosphine)palladium (0) and tris(dibenzylideneacetone)palladium (0). The reaction in this step is preferably performed in a nitrogen or argon atmosphere. The solvent used in this step is not particularly limited insofar as it does not inhibit the reaction and allows the starting material to be dissolved therein to a certain extent. Examples of the solvent include N-methyl-2-pyrrolidone, N,N-dimethylformamide, 1,4-dioxane, toluene and xylene. In this reaction, a phosphorus ligand (such as preferably triphenylphosphine, tri-o-tolylphosphine, tri-tert-butylphosphine, 2,2'-bis(diphenylphosphino)-1,1'-binaphthyl or 1,1'-bis(diphenylphosphino)ferrocene) may be further added in order to obtain excellent results (such as a reduced reaction temperature, a reduced reaction time and an improved yield). The reaction temperature in this step is usually room temperature to solvent reflux temperature. The reaction time is not particularly limited and is usually 0.5 to 48 hours, and preferably 0.5 to 24 hours.

### [Step 15-7]

Step 15-7 is a step of preparing a compound (15-8) from the compound (15-7) as a starting material by deprotection of the ketone using an acid catalyst. In this step, the reaction can be performed under conditions generally used (for example, conditions described in documents such as PENDRAK, I.; CHAMBERS, P. A.; J. Org. Chem., 1995, 60 (10), 3249 and ANDAPPAN, M. M. S.; NILSSON, P.; VON SCHENCK, H.; LARHED, M.; J. Org. Chem., 2004, 69 (16), 5212). The acid used in this step is not particularly limited and is preferably hydrochloric acid. The solvent used in this step is not particularly limited insofar as it does not inhibit the reaction and allows the starting material to be dissolved therein to a certain extent. Examples of the solvent include mixed solvents of alcoholic solvents such as tetrahydrofuran, methanol and ethanol and water. The reaction temperature in this step is usually room temperature to solvent reflux temperature. The reaction time is not particularly limited and is usually 0.5 to 24 hours, and preferably 0.5 to 12 hours.

### [Step 15-8]

Step 15-8 is a step of preparing a compound (15-9) from the compound (15-8) as a starting material by selective deprotection of the phenolic hydroxyl group. In this step, the reaction can be performed under conditions generally used (for example, conditions described in documents such as PENDRAK, I.; CHAMBERS, P. A.; J. Org. Chem., 1995, 60 (10), 3249 and ANDAPPAN, M. M. S.; NILSSON, P.; VON SCHENCK, H.; LARHED, M.; J. Org. Chem., 2004, 69 (16), 5212). However, it is necessary to select a combination of protecting groups and deprotection conditions where the P(15-13) group as the alcoholic hydroxyl protecting group is not reacted. Preferably, the phenolic hydroxyl protecting group is a methoxymethyl group and the alcoholic hydroxyl protecting group is a tert-butyldimethylsilyl group. When the above combination of protecting groups is used, it is possible to perform selective deprotection using an acid catalyst. When the above combination of protecting groups is used, the acid used in this step is not particularly limited and is preferably hydrochloric acid. When the above combination of protecting groups is used, the solvent used in this step is not particularly limited insofar as it does not inhibit the reaction and allows the starting material to be dissolved therein to a certain extent. Examples of the solvent include mixed solvents of alcoholic solvents such as tetrahydrofuran, methanol and ethanol and water. The reaction temperature in this step is usually room temperature to solvent reflux temperature. The reaction time is not particularly limited and is usually 0.5 to 24 hours, and preferably 0.5 to 12 hours.

### [Step 15-9]

Step 15-9 is a step of preparing a compound (15-10) from the compound (15-9) by the method described in the above Step 11-4.

### [Step 15-10]

Step 15-10 is a step of preparing a compound (15-11) from the compound (15-10) by the method described in the above Step 11-5.

### [Step 15-11]

Step 15-11 is a step of preparing a compound (15-12) from the compound (15-11) and the compound (1-4) by the method described in the above Step 1-3.

### [Step 15-12]

Step 15-12 is a step of preparing a compound (15-13) from the compound (15-12) by the method described in the above Step 6-4.

### [Step 15-13]

Step 15-13 is a step of preparing a compound (15-14) from the compound (15-13) by the method described in the above Step 1-8.

### [Step 15-14]

Step 15-14 is a step of preparing a compound (15-15) from the compound (15-14) by the method described in the above Step 1-9.

### [Step 15-15]

Step 15-15 is a step of preparing a compound (15-16) from the compound (15-15) by the method described in the above Step 1-6.

### [Step 15-16]

Step 15-16 is a step of obtaining a compound (15-17) by converting the secondary amino group of the compound (15-16) to a tertiary amino group. This step employs a reaction such as alkylation or reductive amination. The alkylation and reductive amination in this step can be performed by the method described in the above Step 1-7.

### [Preparation Method 16]

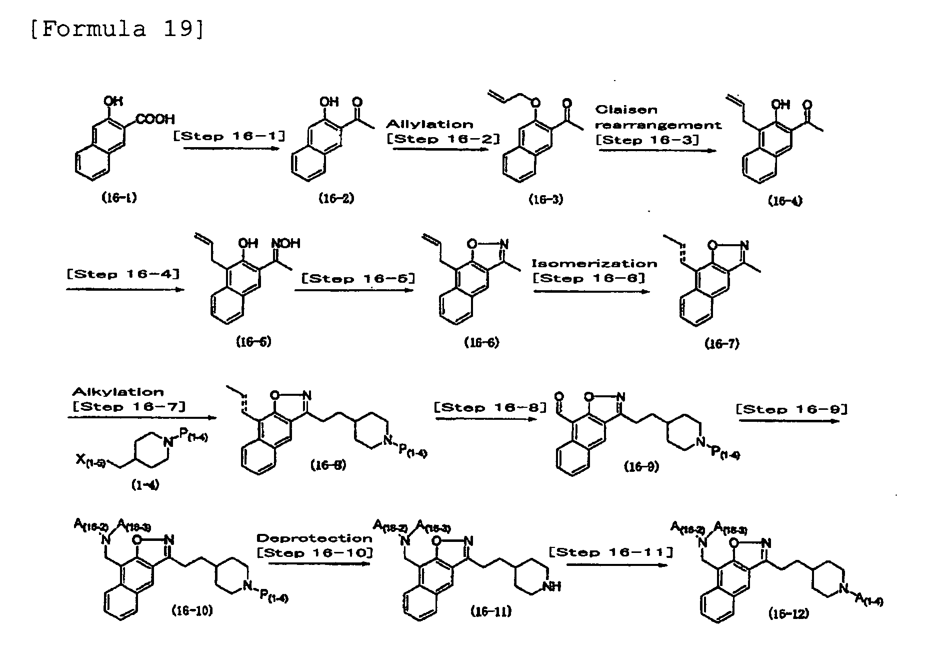

In the formula, the A(16-2) group and the A(16-3) group each represent an R11 group or an R12 group; the P(1-4) group represents a deprotectable amino protecting group; the X(1-5) group represents a leaving group; the A(1-4) group represents an R4 group; and the R11 group, the R12 group and the R4 group are as described above.

Preparation Method 16 is a method for synthesizing the compound of the formula (I) according to the present invention from a known commercially available compound (16-1) as a starting material through multiple steps of Step 16-1 to Step 16-11. However, the compound represented by the formula (I) which can be obtained by the preparation method is a compound represented by the formula (16-12).

### [Step 16-1]

Step 16-1 is a step of obtaining a compound (16-2) by converting the carboxyl group of the compound (16-1) to an acetyl group using methyllithium and chlorotrimethylsilane. This step can be performed under the same conditions as those generally used (for example, conditions described in a document such as RUBOTTOM, G. M.; KIM, C.-W.; J. Org. Chem., 1983, 48 (9), 1550). The solvent used in this step is not particularly limited insofar as it does not inhibit the reaction and allows the starting material to be dissolved therein to a certain extent. The solvent is preferably tetrahydrofuran. The reaction temperature in this step is preferably 0°C. The reaction time in this step is not particularly limited and is usually 0.5 to 24 hours, and preferably 0.5 to 12 hours.

### [Step 16-2]

Step 16-2 is a step of preparing a compound (16-3) from the compound (16-2) by the method described in the above Step 3-1.

### [Step 16-3]

Step 16-3 is a step of preparing a compound (16-4) from the compound (16-3) by the method described in the above Step 3-2.

### [Step 16-4]

Step 16-4 is a step of preparing a compound (16-5) from the compound (16-4) by the method described in the above Step 11-4.

### [Step 16-5]

Step 16-5 is a step of preparing a compound (16-6) from the compound (16-5) by the method described in the above Step 11-5.

### [Step 16-6]

Step 16-6 is a step of preparing a compound (16-7) from the compound (16-6) by the method described in the above Step 3-3.

### [Step 16-7]

Step 16-7 is a step of preparing a compound (16-8) from the compound (16-7) and the compound (1-4) by the method described in the above Step 1-3.

### [Step 16-8]

Step 16-8 is a step of preparing a compound (16-9) from the compound (16-8) by the method described in the above Step 3-8.

### [Step 16-9]

Step 16-9 is a step of preparing a compound (16-10) from the compound (16-9) and a primary or secondary amine by the reductive amination method described in the above Step 1-7.

### [Step 16-10]

Step 16-10 is a step of preparing a compound (16-11) from the compound (16-10) by the method described in the above Step 1-6.

### [Step 16-11]

Step 16-11 is a step of preparing a compound (16-12) from the compound (16-11) by the method described in the above Step 1-7.

### [Preparation Method 17]

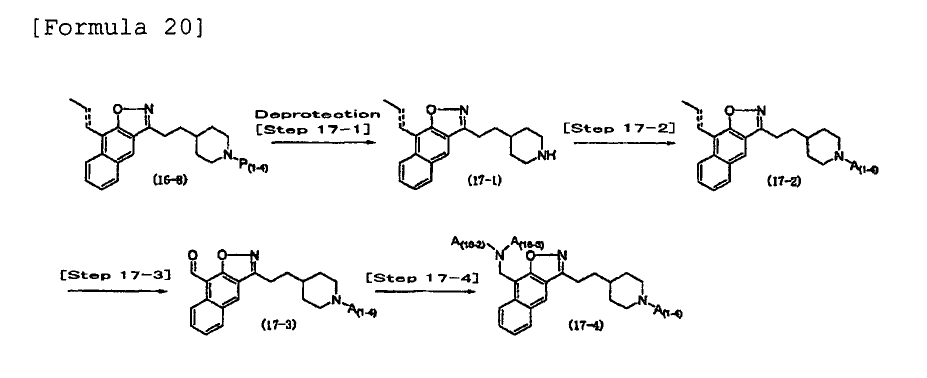

In the formula, the A(16-2) group and the A(16-3) group each represent an R11 group or an R12 group; the P(1-4) group represents a deprotectable amino protecting group; the A(1-4) group represents an R4 group; and the R11 group, the R12 group and the R4 group are as described above.

Preparation Method 17 is a method for synthesizing the compound of the formula (I) according to the present invention from the compound (16-8) obtained by Preparation Method 16 as a starting material through multiple steps of Step 17-1 to Step 17-4. However, the compound represented by the formula (I) which can be obtained by the preparation method is a compound represented by the formula (17-4).

### [Step 17-1]

Step 17-1 is a step of preparing a compound (17-1) from the compound (16-8) by the method described in the above Step 1-6.

### [Step 17-2]

Step 17-2 is a step of preparing a compound (17-2) from the compound (17-1) by the method described in the above Step 1-7.

### [Step 17-3]

Step 17-3 is a step of preparing a compound (17-3) from the compound (17-2) by the method described in the above Step 3-8.

### [Step 17-4]

Step 17-4 is a step of preparing a compound (17-4) from the compound (17-3) and a primary or secondary amine by the reductive amination method described in the above Step 1-7. When the amine used in this step is a primary amine, excellent results such as an improved yield may be obtained by reductive amination using titanium (IV) isopropoxide. The reductive amination using titanium (IV) isopropoxide can be performed under the same conditions as those usually used. Examples of the known method include those described in KUMPATY, H. J.; BHATTACHARYYA, S.; REHR, E. W.; GONZALEZ, A. M.; Synthesis, 2003, (14), 2206 and KUMPATY, H. J.; WILLIAMSON, J. S.; BHATTACHARYYA, S.; Synth. Commun., 2003, 33 (8), 1411.

### [Preparation Method 18]

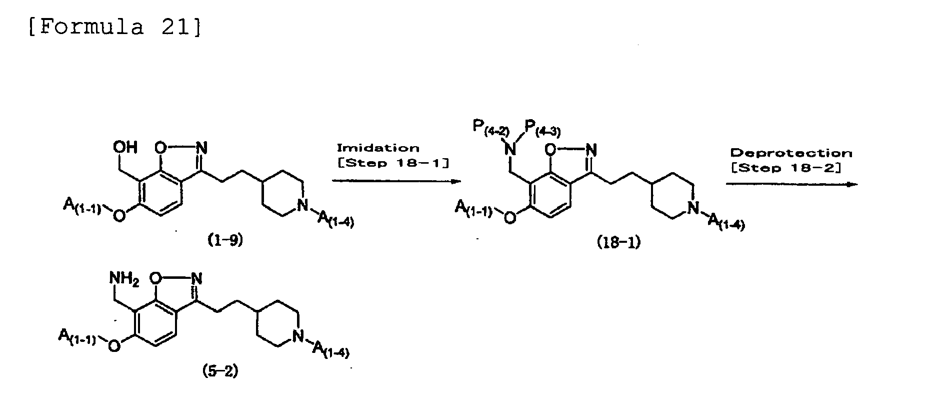

In the formula, the A(1-1) group represents an R21 group; the A(1-4) group represents an R4 group; the P(4-2) group and the P(4-3) group each represent a deprotectable acyl group; and the R21 group and the R4 group are as described above.

Preparation Method 18 is a method for synthesizing the compound of the formula (I) according to the present invention from the compound (1-9) obtained by Preparation Method 1 as a starting material through two steps of Step 18-1 to Step 18-2. However, the compound represented by the formula (I) which can be obtained by the preparation method is the compound represented by the formula (5-2).

### [Step 18-1]

Step 18-1 is a step of obtaining a compound (18-1) by converting the alcoholic hydroxyl group of the compound (1-9) to an imide. The imide group of the compound (18-1) is preferably a phthalimide group. This step can be performed by a reaction such as Mitsunobu reaction. When this step is Mitsunobu reaction, this step is reaction of condensing the compound (1-9) with an imide derivative using an azodicarboxylic acid derivative such as diethyl azodicarboxylate and a triarylphosphine derivative or a trialkylphosphine derivative such as triphenylphosphine. The imide derivative used in this step may be a commercially available product used directly or may be prepared from a commercially available product by a method known to a person skilled in the art. This step can be performed under the same conditions as those generally used (for example, conditions described in a document such as PAQUETT, L. A. et al. ed., "Organic Reactions", John Wiley & Sons, Inc. (1992), vol. 42, p. 335-656). Examples of the phosphine derivative used in this step include triphenylphosphine and tributylphosphine. Examples of the azodicarboxylic acid derivative include diisopropyl azodicarboxylate, diethyl azodicarboxylate and 1,1'-(azodicarbonyl)dipiperidine. The solvent used in this step is not particularly limited insofar as it does not inhibit the reaction and allows the starting material to be dissolved therein to a certain extent. Examples of the solvent include tetrahydrofuran, 1,4-dioxane, methylene chloride and toluene. The reaction temperature in this step is usually -10°C to room temperature, and preferably 0°C to room temperature. The reaction time in this step is not particularly limited and is usually 0.5 to 48 hours, and preferably 0.5 to 24 hours.

### [Step 18-2]

Step 18-2 is a step of obtaining the compound (5-2) by deprotecting the imide group of the compound (18-1). This step can be performed under the same conditions as those generally used (for example, conditions described in a document such as T. W. GREENE and P. G. M. WUTS, "Protective Groups in Organic Chemistry, Third Edition", John Wiley & Sons, Inc. (1999), p. 246-292). Examples of the deprotection reagent used in this step include hydrazine. The solvent used in this step is not particularly limited insofar as it does not inhibit the reaction and allows the starting material to be dissolved therein to a certain extent. Examples of the solvent include organic solvents such as ethanol and 1-propanol; and mixed solvents of organic solvents and water. The reaction temperature is usually room temperature to solvent reflux temperature, and preferably 60°C to solvent reflux temperature. The reaction time is not particularly limited and is usually 0.5 to 48 hours, and preferably 0.5 to 24 hours.

The compound (I) or salt thereof according to the present invention can be formulated by a conventional method. Preferable examples of the dosage form include tablets, powders, fine granules, granules, coated tablets, capsules, syrups, troches, inhalants, suppositories, injections, ointments, eye ointments, eye drops, nasal drops, ear drops, cataplasms and lotions. The compound (I) or salt thereof according to the present invention can be formulated using ingredients typically used such as an expicient, a binder, a disintegrant, a lubricant, a colorant and a corrective and ingredients used where necessary such as a stabilizer, an emulsifier, an absorption promoter, a surfactant, a pH adjuster, a preservative and an antioxidant, and can be formulated by blending ingredients generally used as materials for a pharmaceutical preparation by a conventional method. Examples of such ingredients include (1) animal and vegetable oils such as soybean oil, beef tallow and synthetic glyceride; (2) hydrocarbons such as liquid paraffin, squalane and solid paraffin; (3) ester oils such as octyldodecyl myristate and isopropyl myristate; (4) higher alcohols such as cetostearyl alcohol and behenyl alcohol; (5) a silicone resin; (6) silicone oil; (7) surfactants such as polyoxyethylene fatty acid ester, sorbitan fatty acid ester, glycerol fatty acid ester, polyoxyethylene sorbitan fatty acid ester, polyoxyethylene hydrogenated castor oil and a polyoxyethylene-polyoxypropylene block copolymer; (8) water-soluble polymers such as hydroxyethylcellulose, polyacrytic acid, a carboxyvinyl polymer, polyethylene glycol, polyvinylpyrrolidone and methylcellulose; (9) lower alcohols such as ethanol and isopropanol; (10) polyhydric alcohols such as glycerol, propylene glycol, dipropylene glycol and sorbitol; (11) sugars such as glucose and sucrose; (12) inorganic powders such as silicic anhydride, magnesium aluminum silicate and aluminum silicate; and (13) purified water.

(1) Examples of the expicient used include lactose, corn starch, saccharose, glucose, mannitol, sorbitol, crystalline cellulose and silicon dioxide; (2) examples of the binder used include polyvinyl alcohol, polyvinyl ether, methylcellulose, ethylcellulose, gum arabic, tragacanth, gelatin, shellac, hydroxypropylcellulose, hydroxypropylmethylcellulose, polyvinylpyrrolidone, a polypropylene glycol-polyoxyethylene block polymer, meglumine, calcium citrate, dextrin and pectin; (3) examples of the disintegrant used include starch, agar, gelatin powder, crystalline cellulose, calcium carbonate, sodium bicarbonate, calcium citrate, dextrin, pectin and carboxymethylcellulose calcium; (4) examples of the lubricant used include magnesium stearate, talc, polyethylene glycol, silica and hydrogenated vegetable oil; (5) examples of the colorant used include any of those permitted to be added to pharmaceuticals; (6) examples of the corrective used include cocoa powder, menthol, empasm, mentha oil, borneol and cinnamon powder; and (7) examples of the antioxidant used include those permitted to be added to pharmaceuticals such as ascorbic acid and α-tocopherol.

The administration form of the compound (I) or salt thereof according to the present invention is not particularly limited and may be oral administration or parenteral administration by a method typically used. For example, the compound (I) or salt thereof according to the present invention can be formulated and administered as preparations such as tablets, powders, granules, capsules, syrups, troches, inhalants, suppositories, injections, ointments, eye ointments, tapes, eye drops, nasal drops, ear drops, cataplasms and lotions.

When the compound (I) or salt thereof according to the present invention is administered as a medicine, the dosage may be appropriately selected according to the degree of symptom, the age, the sex, the body weight, the administration form, the type of salt, the specific type of disease, and the like. The dose significantly varies according to the type of disease, the degree of symptom, the age, the sex, the difference in sensitivity to the drug, and the like of the patient. When the medicine is an oral preparation, the dosage is usually 1 to 10000 mg per adult per day, and preferably 10 to 1000 mg per adult per day in a single dose or divided doses and may be appropriately increased and reduced.

### Examples

The present invention will be described in more detail below with reference to Preparation Examples, Examples and Test Examples; however, the scope of the present invention is not limited thereto. The proton nuclear magnetic resonance spectra (¹H-NMR) were measured in the following Preparation Examples and Examples using Mercury 400 manufactured by Varian Inc. The measurement was performed in a deuterated chloroform (CDCl₃) solution, a deuterated methanol (CD₃OD) solution or a deuterated dimethyl sulfoxide (DMSO-d₆) solution. The solvents used for measurement were shown in Examples. The shapes of peaks were described as follows: s = singlet; d = doublet; t = triplet; q = quartet; m = multiplet; br = broad. The coupling constants (J) were indicated in Hertz.

The mass spectra (MS) were measured in the following Preparation Examples and Examples using SSQ7000 manufactured by Thermo Electron Corporation. The ionization method was ESI (electrospray ionization). The measured molecular weights were indicated as values rounded to whole numbers.

In the following Preparation Examples and Examples, Initiator or Emrys Liberator manufactured by Biotage AB was used as a microwave synthesizer. In the following Preparation Examples and Examples, NH PSQ60 or DM 2035 manufactured by Fuji Silysia Chemical Ltd. was used as NH silica gel.

### Preparation Example 1

### Synthesis of tert-butyl 4-iodomethylpiperidine-1-carboxylate

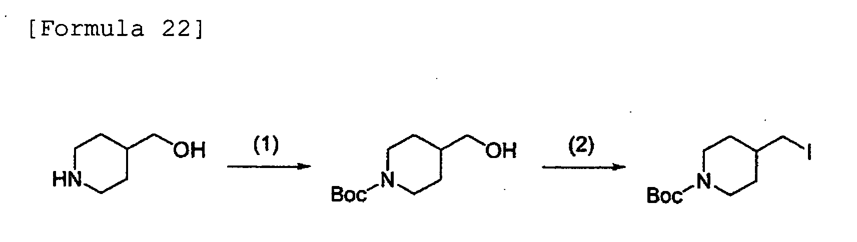

### (1) Synthesis of tert-butyl 4-hydroxymethylpiperidine-1-carboxylate

Piperidin-4-ylmethanol (100 g) was dissolved in chloroform (500 mL). Di-tert-butyl dicarbonate (190 g) was added to the solution under ice-cooling over 10 minutes, and the mixture was stirred at room temperature for two hours. A saturated ammonium chloride solution (100 mL) and water (300 mL) were sequentially added to the reaction mixture, followed by extraction with chloroform (500 mL). The organic layer was washed with a saturated sodium chloride solution. Further, the aqueous layer was extracted with chloroform (300 mL), followed by washing with a saturated sodium chloride solution. The two organic layers were mixed and dried over magnesium sulfate. The drying agent was removed by filtration and then the solvent was evaporated under reduced pressure. Heptane (500 mL) was added to the residue to obtain a suspension. The solid was collected by filtration, washed with heptane (500 mL) and then dried at 50°C for 19 hours to obtain the title compound (179 g). ¹H-NMR (400 MHz, CDCl₃) δ (ppm) : 1.14 (ddd, J=4. 4 Hz, 12.8 Hz, 23.8 Hz, 2H), 1.46 (s, 9H), 1.56-1.75 (m, 3H), 2.70 (dt, J=2.0 Hz, 12.8 Hz, 2H), 3.50 (d, J=6.0 Hz, 2H), 4.12 (br d, J=12.8 Hz, 2H).

### (2) Synthesis of tert-butyl 4-iodomethylpiperidine-1-carboxylate

tert-Butyl 4-hydroxymethylpiperidine-1-carboxylate (88 g) and triethylamine (140 mL) were dissolved in tetrahydrofuran (900 mL). Methanesulfonyl chloride (38 mL) was added to the solution under ice-cooling over 10 minutes, and the mixture was stirred under ice-cooling for 1.5 hours. Water was added to the reaction mixture, followed by extraction with ethyl acetate. The organic layer was washed with a saturated sodium chloride solution and dried over magnesium sulfate. The drying agent was removed by filtration and then the solvent was evaporated under reduced pressure. A crude product of tert-butyl 4-methanesulfonyloxymethylpiperidine-1-carboxylate (123 g) was obtained as a residue. The crude 4-methanesulfonyloxymethylpiperidine-1-carboxylate was dissolved in acetonitrile (900 mL). Sodium iodide (180 g) was added to the solution, and the mixture was stirred at 70°C for 14 hours and 20 minutes. Water was added to the reaction mixture, followed by extraction with ethyl acetate twice. The organic layers were washed with a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (120 g). ¹H-NMR (400 MHz, CDCl₃) δ (ppm): 1.14 (ddd, J=4 .4 Hz, 12.8 Hz, 24.6 Hz, 2H), 1.45 (s, 9H), 1.52-1.68 (m, 1H), 1.83 (br d, J=13.2 Hz, 2H), 2.68 (dt, J=2.0 Hz, 12.8 Hz, 2H), 3.10 (d, J=6.4 Hz, 2H), 4.11 (br d, J=12. 8 Hz, 2H).

### Preparation Example 2

### Synthesis of tert-butyl 4-[2-(6-hydroxy-7-hydroxymethylbenz[d]isoxazol-3-yl)ethyl]piperidine-1-carboxylate

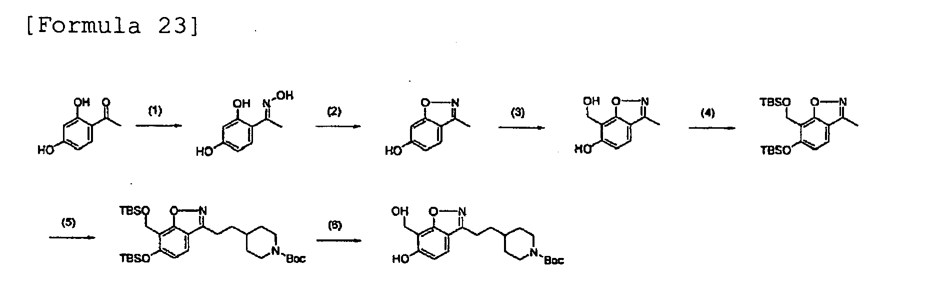

### (1)(2) Synthesis of 3-methylbenz[d]isoxazol-6-ol

2',4'-Dihydroxyacetophenone (39.6 g) was dissolved in ethanol (400 mL) and water (140 mL). Hydroxylamine hydrochloride (45.2 g) and sodium acetate (55.5 g) were added to the solution, and the mixture was heated under reflux for one hour. The reaction mixture was cooled to room temperature and then filtered through celite. The residue was washed with ethanol. The washing liquid and the filtrate were mixed and the mixture was concentrated under reduced pressure. The residue was dissolved in ethyl acetate (1 L) and sequentially washed with water (500 mL) and a saturated sodium chloride solution (300 mL). The organic layer was dried over magnesium sulfate. The drying agent was removed by filtration and then the solvent was evaporated under reduced pressure. Crude 2',4'-dihydroxyacetophenone oxime (43.5 g) was obtained as a residue. The crude 2',4'-dihydroxyacetophenone oxime (43.5 g) was dissolved in N,N-dimethylformamide (200 mL) without further purification. Acetic anhydride (134 mL) and sodium acetate (46.9 g) were added to the solution, and the mixture was stirred at 150°C for one hour. The reaction mixture was cooled to room temperature and then filtered through celite. The celite was washed with ethyl acetate. The filtrate and the washing liquid were mixed and the mixture was concentrated under reduced pressure. The residue was dissolved in methanol (200 mL) and 5 M hydrochloric acid (104 mL), and the solution was stirred at 90°C for one hour. The reaction mixture was cooled to room temperature, followed by extraction with ethyl acetate. The organic layer was dried over magnesium sulfate. The drying agent was removed by filtration and then the solvent was evaporated under reduced pressure. Cold water (300 m) was added to the residue to obtain a suspension. The solid was collected by filtration and then washed with cold water (100 mL) and dried at 60°C overnight to obtain the title compound (33.1 g).
¹H-NMR (400 MHz, CDCl₃) δ (ppm): 2.51 (s, 3H), 6.85 (dd, J=2.0, 8.4 Hz, 1H), 6.92 (d, J=2.0 Hz, 1H), 7.43 (d, J=8.4 Hz, 1H).

### (3) Synthesis of 7-hydroxymethyl-3-methylbenz[d]isoxazol-6-ol

3-methylbenz[d]isoxazol-6-ol (32.8 g) was suspended in water (500 mL). A 2 M sodium hydroxide solution (110 mL) was added to the suspension at room temperature, and the mixture was stirred at 70°C for 35 minutes. A 37% formaldehyde solution (80.4 mL) was added to the mixture, followed by stirring at 70°C for one hour. 5 M hydrochloric acid (66 mL) was added to the reaction mixture under ice-cooling, followed by extraction with ethyl acetate (1 L). The organic layer was washed with a saturated sodium chloride solution (300 mL) and dried over magnesium sulfate. The drying agent was removed by filtration and then the solvent was evaporated under reduced pressure. Ethyl acetate (200 mL) was added to the residue to obtain a suspension. The precipitate was collected by filtration and then washed with ethyl acetate (200 mL) to obtain a solid (29.3 g). The filtrate was concentrated under reduced pressure. The residue after concentration was purified by silica gel column chromatography (heptane-ethyl acetate) to obtain a solid (3.5 g). The solid was combined with the above solid, followed by drying under reduced pressure to obtain the title compound (32.8 g).
¹H-NMR (400 MHz, CD₃OD/CDCl₃, 1:5) δ (ppm) : 2.51 (s, 3H), 5.08 (s, 2H), 6. 87 (d, J=8.4 Hz, 1H), 7.38 (d, J=8.4 Hz, 1H).

### (4) Synthesis of 6-(tert-butyldimethylsilanyloxy)-7-(tert-butyldimethylsilanyloxymethyl)-3-methylbenz[d]isoxazole

7-Hydroxymethyl-3-methylbenz[d]isoxazol-6-ol (60.8 g) and imidazole (60.2 g) were dissolved in N,N-dimethylformamide (400 mL). tert-Butylchlorodimethylsilane (123 g) was added and the mixture was stirred at room temperature for 2.5 hours. A small amount of methanol and water (750 mL) were added to the reaction mixture, followed by extraction with ethyl acetate (750 mL) twice. The organic layers were washed with a saturated sodium chloride solution (500 mL) and dried over magnesium sulfate. The drying agent was removed by filtration and then the solvent was evaporated under reduced pressure. Heptane and ethyl acetate were added to the residue, followed by solidification under ice-cooling. The solid was collected by filtration. The solid was washed with heptane-ethyl acetate to obtain a solid (63.5 g). The filtrate was concentrated under reduced pressure. The residue was purified by silica gel column chromatography (heptane-ethyl acetate) to obtain a solid (61.8 g). The solid was combined with the above solid to obtain the title compound (125.3 g). ¹H-NMR (400 MHz, CDCl₃) δ (ppm): 0.11 (s, 6H), 0.26 (s, 6H), 0.90 (s, 9H), 1.04 (s, 9H), 2.52 (s, 3H), 4.96 (s, 2H), 6. 80 (d, J=8.8 Hz, 1H), 7.35 (d, J=8.8 Hz, 1H).

### (5) Synthesis of tert-butyl 4-{2-[6-(tert-butyldimethylsilanyloxy)-7-(tert-butyldimethylsilanyloxymethyl)benz[d]isoxazol-3-yl]ethyl}piperidine-1-carboxylate

Diisopropylamine (22.4 mL) was dissolved in tetrahydrofuran (400 mL) in a nitrogen atmosphere. n-Butyllithium (2.61 M solution in n-hexane, 56.6 mL) was added dropwise to the solution at -78°C over 10 minutes. The mixture was stirred at -78°C for five minutes and under ice-cooling for 10 minutes to prepare a solution of lithium diisopropylamide in tetrahydrofuran. The solution of lithium diisopropylamide in tetrahydrofuran was cooled to -78°C again and added dropwise to a solution of 6-(tert-butyldimethylsilanyloxy)-7-(tert-butyldimethylsilanyloxymethyl)-3-methylbenz[d]isoxazole (50.2 g) and tert-butyl 4-iodomethylpiperidine-1-carboxylate (43.9 g) in tetrahydrofuran (400 mL) at - 70°C over 30 minutes. Further, the mixture was stirred at -78°C for 45 minutes. A saturated ammonium chloride solution was added to the reaction mixture, followed by heating to room temperature. Water (750 mL) was added to the reaction mixture, followed by extraction with ethyl acetate (750 mL) twice. The organic layers were washed with a saturated sodium chloride solution (500 mL) and dried over magnesium sulfate. The drying agent was removed by filtration and then the solvent was evaporated under reduced pressure. The residue was purified by silica gel column chromatography (heptane-ethyl acetate) to obtain the title compound (74.4 g).
¹H-NMR (400 MHz, CDCl₃) δ (ppm): 0.12 (s, 6H), 0.26 (s, 6H), 0.91 (s, 9H), 1.04 (s, 9H), 1.10-1.20 (m, 2H), 1.46 (s, 9H), 1.47-1.57 (m, 1H), 1.75-1.81 (m, 4H), 2.64-2.71 (m, 2H), 2.92-2.97 (m, 2H), 4.03-4.13 (m, 2H), 4.96 (s, 2H), 6.79 (d, J=8.4 Hz, 1H), 7.36 (d, J=8.4 Hz, 1H).

### (6) Synthesis of tert-butyl 4-[2-(6-hydroxy-7-hydroxymethylbenz[d]isoxazol-3-yl)ethyl]piperidine-1-carboxylate

tert-Butyl 4-{2-[6-(tert-butyldimethylsilanyloxy)-7-(tert-butyldimethylsilanyloxymethyl)benz[d]isoxazol-3-yl]ethylpiperidine-1-carboxylate (74.5 g) was dissolved in tetrahydrofuran (200 mL). Tetrabutylammonium fluoride (1.0 M solution in tetrahydrofuran, 246 mL) was added and the mixture was stirred at room temperature for 1.5 hours. Water (500 mL) was added to the reaction mixture, followed by extraction with ethyl acetate (500 mL) twice. The organic layers were washed with a saturated sodium chloride solution (300 mL) and dried over magnesium sulfate. The drying agent was removed by filtration and then the solvent was evaporated under reduced pressure. The residue was purified by silica gel column chromatography (heptane-ethyl acetate) to obtain the title compound (41.2 g).
¹H-NMR (400 MHz, CDCl₃) δ(ppm): 1.07-1.17 (m, 2H), 1.45 (s, 9H), 1.45-1.55 (m, 1H), 1.70-1.78 (m, 4H), 2.63-2.69 (m, 2H), 2.90-2.94 (m, 2H), 4.06-4.09 (m, 2H), 5.26 (s, 2H), 6.87 (d, J=8.4 Hz, 1H), 7.37 (d, J=8.4 Hz, 1H), 8.43-8.50 (m, 1H).

### Preparation Example 3

### Synthesis of N,N-dimethyl{6-cyclopropylmethoxy-3-[2-(piperidin-4-yl)ethyl]benz[d]isoxazol-7-yl}methylamine

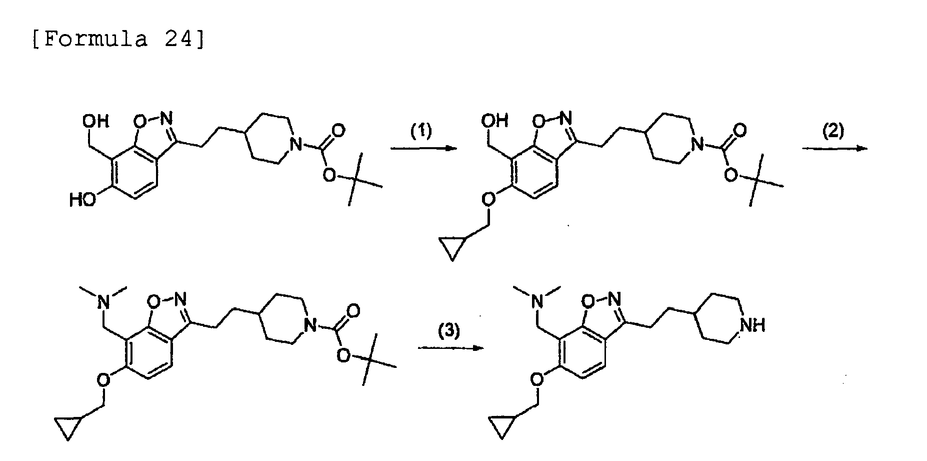

### (1) Synthesis of tert-butyl 4-[2-(6-cyclopropylmethoxy-7-hydroxymethylbenz[d]isoxazol-3-yl)ethyl]piperidine-1-carboxylate

tert-Butyl 4-[2-(6-hydroxy-7-hydroxymethylbenz[d]isoxazol-3-yl)ethyl]piperidine-1-carboxylate (compound synthesized in Preparation Example 2) (5.3 g) and cyclopropylmethyl bromide (2.05 mL) was dissolved in acetonitrile (50 mL). Potassium carbonate (3.51 g) was added and the mixture was heated with stirring at 70°C for 10 hours. A sodium carbonate solution and ethyl acetate were added to the reaction mixture, and the organic layer was separated. The resulting organic layer was washed with a saturated sodium chloride solution and then dried over anhydrous sodium sulfate. The drying agent was removed by filtration and then the organic layer was concentrated under reduced pressure. The residue was purified by silica gel column chromatography (hexane-ethyl acetate) to obtain the title compound (5.8 g).
¹H-NMR (400 MHz, CDCl₃) δ: 0.37-0.41 (m, 2H), 0.65-0.70 (m, 2H), 1.08-1.22 (m, 2H), 1.29-1.39 (m, 1H), 1.45 (s, 9H), 1.44-1.52 (m, 1H), 1.70-1.82 (m, 4H), 2.60-2.72 (m, 2H), 2.97 (t, J=8.0 Hz, 2H), 3.98 (d, J=7.2 Hz, 2H), 4.02-4.18 (m, 2H), 5.04 (d, J=7.2 Hz, 2H), 6.92 (d, J=8.8 Hz, 1H), 7.47 (d, J=8.8 Hz, 1H).

### (2) Synthesis of tert-butyl 4-{2-[6-cyclopropylmethoxy-7-(N,N-dimethylaminomethyl)benz[d]isoxazol-3-yl]ethyl}piperidine-1-carboxylate

tert-Butyl 4-[2-(6-cyclopropylmethoxy-7-hydroxymethylbenz[d]isoxazol-3-yl)ethyl]piperidine-1-carboxylate (5.08 g) was dissolved in tetrahydrofuran (100 mL). Triethylamine (4.93 mL) was added to the solution, and the mixture was stirred under ice-cooling in a nitrogen atmosphere. Methanesulfonyl chloride (1.37 mL) was added to the mixture, followed by stirring for 30 minutes. Triethylamine (1.65 mL) was added and the mixture was stirred under ice-cooling in a nitrogen atmosphere. Methanesulfonyl chloride (0.46 mL) was added to the mixture, followed by stirring for 65 minutes. Then, dimethylamine (2.0 M solution in tetrahydrofuran) (59 mL) was added and the mixture was stirred for 170 minutes. A saturated sodium chloride solution and water were added to the reaction mixture, followed by extraction with ethyl acetate twice. The organic layers were washed with a saturated sodium chloride solution and dried over magnesium sulfate-sodium carbonate. The drying agent was removed by filtration and then the organic layers were concentrated under reduced pressure. The residue was purified by NH silica gel column chromatography (heptane-ethyl acetate) to obtain the title compound (5.30 g).
¹H-NMR (400 MHz, CDCl₃) δ: 0.35-0.39 (m, 2H), 0.62-0.67 (m, 2H), 1.10-1.21 (m, 2H), 1.28-1.43 (m, 1H), 1.46 (s, 9H), 1.46-1.56 (m, 1H), 1.28-1.80 (m, 4H), 2.34 (s, 6H), 2.64-2.73 (m, 2H), 2.96 (m, 2H), 3.82 (s, 2H), 3.94 (d, J=6.8 Hz, 2H), 4.02-4.17 (m, 2H), 6.90 (d, J=8.6 Hz, 1H), 7.44 (d, J=8.6 Hz, 1H).

### (3) Synthesis of N,N-dimethyl{6-cyclopropylmethoxy-3-[2-(piperidin-4-yl)ethyl]benz[d]isoxazol-7-yl}methylamine

tert-Butyl 4-{2-[6-cyclopropylmethoxy-7-(N,N-dimethylaminomethyl)benz[d]isoxazol-3-yl]ethyl}piperidine-1-carboxylate (5.30 g) was dissolved in methanol (120 mL), and the solution was stirred under ice-cooling in a nitrogen atmosphere. Hydrogen chloride (4 M solution in ethyl acetate) (29 mL) was added to the mixture, followed by stirring at room temperature for seven hours and 20 minutes. The solvent was evaporated under reduced pressure. Chloroform, a 5 M sodium hydroxide solution and a saturated sodium chloride solution were added to the residue, followed by stirring. The organic layer was separated and the aqueous layer was extracted with chloroform twice. The combined organic layers were dried over magnesium sulfate. The drying agent was removed by filtration and then the organic layers were concentrated under reduced pressure to obtain the crude title compound (4.56 g).
¹H-NMR (400 MHz, CDCl₃) δ: 0.36-0.39 (m, 2H), 0.62-0.67 (m, 2H), 1.18-1.36 (m, 3H), 1.44-1.56 (m, 1H), 1.75-1.83 (m, 4H), 2.34 (s, 6H), 2.58-2.65 (m, 2H), 2.93-2.97 (m, 2H), 3.10-3.16 (m, 2H), 3.83 (s, 2H), 3.95 (d, J=6.8 Hz, 2H), 6.91 (d, J=8.6 Hz, 1H), 7.45 (d, J=8.6 Hz, 1H).

### Example 1

### {trans-2-{4-{2-[6-Cyclopropylmethoxy-7-(N,N-dimethylaminomethyl)benz[d]isoxazol-3-yl]ethyl}piperidine-1-methyl}cyclopropyl}methanol dihydrochloride

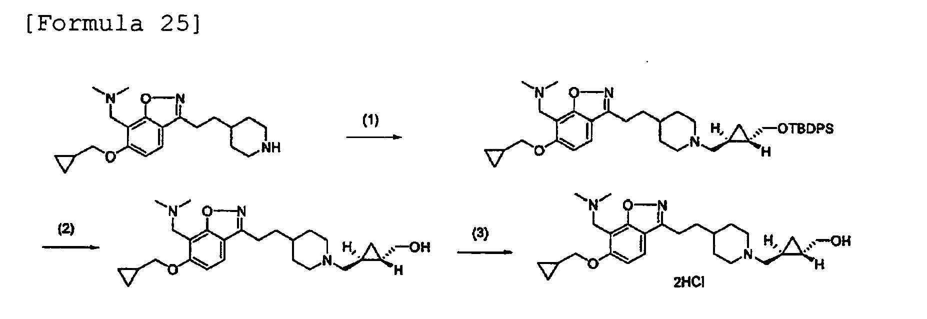

### (1) N,N-Dimethyl{3-{2-{1-[trans-2-(tert-butyldiphenylsilanyloxymethyl)cyclopropylmethyl]piperid in-4-yl}ethyl}-6-cyclopropylmethoxybenz[d]isoxazol-7-yl}methylamine

The title compound (105 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (89.2 mg) and trans-2-(tert-butyldiphenylsilanyloxymethyl)cyclopropanecarbaldehyde (see J. Org. Chem., 2002, 67, 1669-1677) (102 mg) according to Example 21-(3).
¹H-NMR (400 MHz, CDCl₃) δ (ppm): 0.22-0.26 (m, 1H), 0.33-0.38 (m, 3H), 0.62-0.67 (m, 2H), 0.78-0.88 (m, 2H), 1.03 (s, 9H), 1.22-1.34 (m, 4H), 1.74-1.79 (m, 4H), 1.82-1.88 (m, 1H), 1.92-2.04 (m, 2H), 2.33 (s, 6H), 2.49 (dd, J=5.6, 11.6 Hz, 1H), 2.91-2.97 (m, 3H), 3.23-3.26 (m, 1H), 3.38 (dd, J=7.2, 11.6 Hz, 1H), 3.56 (dd, J=5.6, 11.6 Hz, 1H), 3.82 (s, 2H), 3.93 (d, J=6.8 Hz, 2H), 6.88 (d, J=8.8 Hz, 1H), 7.33-7.40 (m, 6H), 7.43 (d, J=8.8 Hz, 1H), 7.63-7.67 (m, 4H).

### (2) {trans-2-{4-{2-[6-Cyclopropylmethoxy-7-(N,N-dimethylaminomethyl)benz[d]isoxazol-3-yl]ethyl}piperidine-1-methyl}cyclopropyl}methanol

The title compound (38 mg) was obtained by synthesis from N,N-dimethyl{3-{2-{1-[trans-2-(tert-butyldiphenylsilanyloxymethyl)cyclopropylmethyl]piperid in-4-yl}ethyl}-6-cyclopropylmethoxybenz[d]isoxazol-7-yl}methylamine (88.4 mg) according to Example 16-(5).
¹H-NMR (400 MHz, CDCl₃) δ (ppm): 0.34-0.38 (m, 3H), 0.43-0.48 (m, 1H), 0.62-0.67 (m, 2H), 0.82-0.87 (m, 1H), 0.90-0.97 (m, 1H), 1.25-1.36 (m, 4H), 1.75-1.79 (m, 4H), 1.92-1.98 (m, 2H), 2.15 (dd, J=7.6, 12.8 Hz, 1H), 2.33 (s, 6H), 2.39 (dd, J=6.0, 12.8 Hz, 1H), 2.91-2.96 (m, 2H), 3.05-3.09 (m, 2H), 3.34 (dd, J=7.2, 11.2 Hz, 1H) , 3.56 (dd, J=6.4, 11.2 Hz, 1H), 3.82 (s, 2H), 3.93 (d, J=6.8 Hz, 2H), 6.89 (d, J=8.8 Hz, 1H), 7.43 (d, J=8.8 Hz, 1H).

### (3) {trans-2-{4-{2-[6-Cyclopropylmethoxy-7-(N,N-dimethylaminomethyl)benz[d]isoxazol-3-yl]ethyl}piperidinomethyl}cyclopropyl}methanol dihydrochloride

The title compound (41 mg) was obtained by synthesis from {trans-2-{4-{2-[6-Cyclopropylmethoxy-7-(N,N-dimethylaminomethyl)benz[d]isoxazol-3-yl]ethyl}piperidine-1-methyl}cyclopropyl}methanol (38 mg) according to Example 21-(4).
¹H-NMR (400 MHz, CD₃OD) δ (ppm): 0.41-0.45 (m, 2H), 0.63-0.73 (m, 4H), 1.03-1.10 (m, 1H), 1.14-1.20 (m, 1H), 1.35-1.46 (m, 1H), 1.56-1.66 (m, 2H), 1.67-1.77 (m, 1H), 1.83-1.90 (m, 2H), 2.07-2.12 (m, 2H), 2.92-3.00 (m, 3H), 2. 96 (s, 6H), 3.05-3.09 (m, 2H), 3.12-3.16 (m, 1H), 3.23-3.29 (m, 1H), 3.66-3.71 (m, 2H), 3.74-3.77 (m, 1H), 4.12 (d, J=6.8 Hz, 2H), 4.64 (s, 2H), 7.23 (d, J=8.8 Hz, 1H), 7.93 (d, J=8.8 Hz, 1H). ESI-MS m/z: 221 [M+2H]²⁺, 442 [M+H]⁺.

### Example 2

### {cis-2={4-{2-[6-Cyclopropylmethoxy-7-(N,N-dimethylaminomethyl)benz[d]isoxazol-3-yl]ethyl}piperidinomethyl}cyclopropyl}methanol dihydrochloride

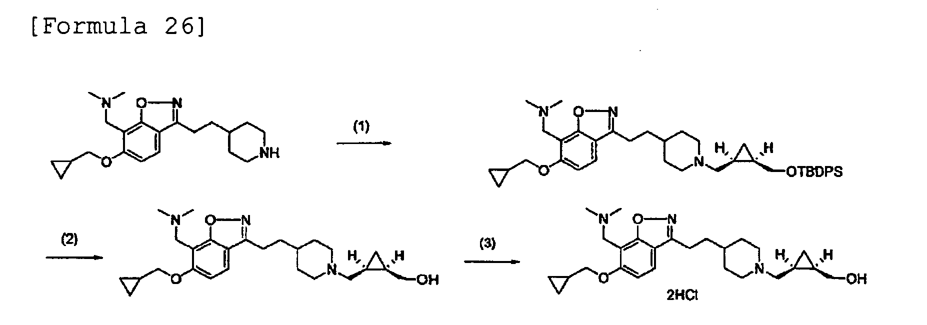

### (1) N,N-Dimethyl{3-{2-{1-[cis-2-(tert-butyldiphenylsilanyloxymethyl)cyclopropylmethyl]piperid in-4-yl}ethyl}-6-cyclopropylmethoxybenz[d]isoxazol-7-yl}methylamine

The title compound (170 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (89.2 mg) and cis-2-(tert-butyldiphenylsilanyloxymethyl)cyclopropanecarbaldehyde (see J. Org. Chem., 2002, 67, 1669-1677) (102 mg) according to Example 21-(3).
¹H-NMR (400 MHz, CDCl₃) δ (ppm): -0.02-0.04 (m, 1H), 0.33-0.38 (m, 2H), 0.62-0.66 (m, 2H), 0.70-0.76 (m, 1H), 0.93-1.00 (m, 1H), 1.05 (s, 9H), 1.10-1.16 (m, 1H), 1.24-1.32 (m, 4H), 1.74-1.79 (m, 4H), 1.78-1.93 (m, 2H), 2.01-2.08 (m, 1H), 2.33 (s, 6H), 2.64-2.70 (m, 1H), 2.91-2.98 (m, 3H), 3.04-3.07 (m, 1H), 3.54 (dd, J=8.0, 11.2 Hz, 1H), 3.75 (dd, J=5.6, 11.2 Hz, 1H), 3.82 (s, 2H), 3.93 (d, J=6.8 Hz, 2H), 6.88 (d, J=8.8 Hz, 1H), 7.34-7.42 (m, 6H), 7.43 (d, J=8.8 Hz, 1H), 7.63-7.71 (m, 4H).

### (2) {cis-2-{4-{2-[6-Cyclopropylmethoxy-7-(N,N-dimethylaminomethyl)benz[d]isoxazol-3-yl]ethyl}piperidine-1-methyl}cyclopropyl}methanol

The title compound (70 mg) was obtained by synthesis from N,N-dimethyl{3-{2-{1-[cis-2-(tert-butyldiphenylsilanyloxymethyl)cyclopropylmethyl]piperid in-4-yl}ethyl}-6-cyclopropylmethoxybenz[d]isoxazol-7-yl}methylamine (170 mg) according to Example 16-(5).
¹H-NMR (400 MHz, CDCl₃) δ (ppm): 0.18-0.24 (m, 1H), 0.34-0.39 (m, 2H), 0.62-0.67 (m, 2H), 0.78-0.84 (m, 1H), 1.13-1.17 (m, 1H), 1.22-1.38 (m, 5H), 1.73-1.84 (m, 5H), 2.04-2.16 (m, 2H), 2.33 (s, 6H), 2.62 (dd, J=5.2, 13.2 Hz, 1H), 2.76-2.78 (m, 1H), 2.91-2.95 (m, 2H), 3.07 (dd, J=11. 6, 12.4 Hz, 1H), 3.30-3.33 (m, 1H), 3.82 (s, 2H), 3.93 (d, J=6.8 Hz, 2H), 3.98 (dd, J=5.2, 12.4 Hz, 1H), 6.88 (d, J=8.8 Hz, 1H), 7.42 (d, J=8.8 Hz, 1H).

### (3) {cis-2-{4-{2-[6-Cyclopropylmethoxy-7-(N,N-dimethylaminomethyl)benz[d]isoxazol-3-yl]ethyl}piperidinomethyl}cyclopropyl}methanol dihydrochloride

The title compound (65 mg) was obtained by synthesis from {cis-2-{4-{2-[6-Cyclopropylmethoxy-7-(N,N-dimethylaminomethyl)benz[d]isoxazol-3-yl]ethyl}piperidine-1-methyl}cyclopropyl}methanol (70 mg) according to Example 21-(4).
¹H-NMR (400 MHz, CD₃OD) δ (ppm): 0.43-0.46 (m, 2H), 0.50-0.56 (m, 1H), 0.67-0.73 (m, 2H), 1.02-1.08 (m, 1H), 1.29-1.47 (m, 3H), 1.50-1.57 (m, 2H), 1.70-1.78 (m, 1H), 1.83-1.89 (m, 2H), 2.10-2.17 (m, 2H), 2.93-3.02 (m, 1H), 2.94-3.10 (m, 3H), 2.96 (s, 6H), 3.19-3.33 (m, 2H), 3.59-3.63 (m, 1H), 3.81-3.85 (m, 1H), 4.01-4.05 (m, 1H), 4.12 (d, J=7.2 Hz, 2H), 4.64 (s, 2H), 7.23 (d, J=8.8 Hz, 1H), 7.93 (d, J=8.8 Hz, 1H). ESI-MS m/z: 221 [M+2H]²⁺, 442 [M+H]⁺.

### Example 3

### {cis-2-{3-[2-(1-Benzylpiperidin-4-yl)ethyl]-7-(N,N-dimethylaminomethyl)benz[d]isoxazol-6-yloxymethyl}cyclopropyl}methanol dihydrochloride

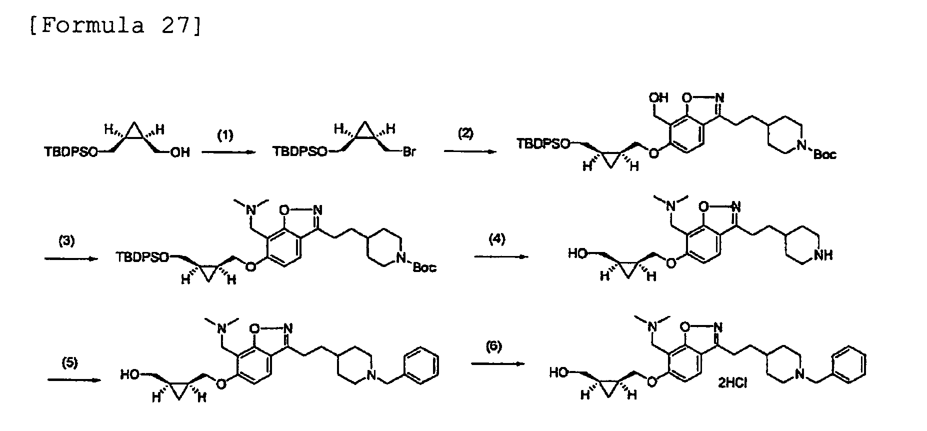

### (1)(2) tert-Butyl 4-{2-{6-[cis-2-(tert-butyldiphenylsilanyloxymethyl)cyclopropylmethoxy]-7-hydroxymethylbenz[d]isoxazol-3-yl}ethyl}piperidine-1-carboxylate

[cis-2-(tert-Butyldiphenylsilanyloxymethyl)cyclopropyl]methanol (see J. Org. Chem. 2002, 67, 1669-1677) (682 mg) was dissolved in dichloromethane (10 mL). Triphenylphosphine (787 mg) and carbon tetrabromide (995 mg) were added under ice-cooling and the mixture was stirred at the same temperature for 30 minutes. Water was added to the reaction mixture, followed by extraction with ethyl acetate. The organic layer was sequentially washed with a saturated sodium chloride solution and a saturated sodium bicarbonate solution, dried over magnesium sulfate and filtered. Then, the solvent was evaporated under reduced pressure. The residue was purified by silica gel column chromatography (heptane-ethyl acetate) to obtain (cis-2-bromomethylcyclopropylmethoxy)-tert-butyldiphenylsilane (806 mg). The title compound (1.13 g) was obtained by synthesis according to Example 16-(1) from (cis-2-bromomethylcyclopropylmethoxy)-tert-butyldiphenylsilane (752 mg) and tert-butyl 4-[2-(6-hydroxy-7-hydroxymethylbenz[d]isoxazol-3-yl)ethyl]piperidine-1-carboxylate obtained in Preparation Example 2-(6).
¹H-NMR (400 MHz, CDCl₃) δ (ppm): 0.37-0.41 (m, 1H), 0.83-0.89 (m, 1H), 1.04-1.61 (m, 5H), 1.04 (s, 9H), 1.45 (s, 9H), 1.72-1.81 (m, 4H), 2.63-2.70 (m, 2H), 2.94-2.98 (m, 2H), 3.61-3.66 (m, 1H), 3.89-3.92 (m, 1H), 4.06-4.12 (m, 3H), 4.21-4.26 (m, 1H), 4.96 (d, J=6.8 Hz, 2H), 6.83 (d, J=8.8 Hz, 1H), 7.31-7.45 (m, 7H), 7.62-7.65 (m, 4H).

### (3) tert-Butyl 4-{2-{6-[cis-2-(tert-butyldiphenylsilanyloxymethyl)cyclopropylmethoxy]-7-(N,N-dimethylaminomethyl)benz[d]isoxazol-3-yl}ethyl}piperidine-1-carboxylate

The title compound (798 mg) was obtained by synthesis from tert-butyl 4-{2-{6-[cis-2-(tert-butyldiphenylsilanyloxymethyl)cyclopropylmethoxy]-7-hydroxymethylbenz[d]isoxazol-3-yl}ethyl}piperidine-1-carboxylate (839 mg) according to Example 21-(1).
¹H-NMR (400 MHz, CDCl₃) δ (ppm): 0.36-0.39 (m, 1H), 0.82-0.88 (m, 1H), 0.88-1.53 (m, 5H), 1.04 (s, 9H), 1.45 (s, 9H), 1.73-1.79 (m, 4H), 2.28 (s, 6H), 2.64-2.70 (m, 2H), 2.86 (s, 2H), 2.93-2.98 (m, 2H), 3.65-3.70 (m, 1H), 3.82-3.87 (m, 1H), 4.01-4.21 (m, 4H), 6.82 (d, J=8.8 Hz, 1H), 7.30-7.42 (m, 7H), 7.62-7.64 (m, 4H).

### (4) {cis-2-{7-(N,N-Dimethylaminomethyl)-3-[2-(piperidin-4-yl)ethyl]benz[d]isoxazol-6-yloxymethyl}cyclopropyl}methanol

The title compound (300 mg) was obtained by synthesis from tert-butyl 4-{2-{6-[cis-2-(tert-butyldiphenylsilanyloxymethyl)cyclopropylmethoxy]-7-(N,N-dimethylaminomethyl)benz[d]isoxazol-3-yl}ethyl}piperidine-1-carboxylate (725 mg) according to Example 21-(2).
¹H-NMR (400 MHz, CDCl₃) δ (ppm): 0.18-0.22 (m, 1H), 0.83-0.89 (m, 1H), 1.13-1.27 (m, 2H), 1.36-1.60 (m, 3H), 1.73-1.79 (m, 4H), 2.23 (s, 6H), 2.55-2.61 (m, 2H), 2.92-2.98 (m, 2H), 3.05-3.08 (m, 2H), 3.14-3.20 (m, 1H), 3.54-3.68 (m, 2H), 3.84-3.93 (m, 2H), 4.58-4.61 (m, 1H), 6.90 (d, J=8.8 Hz, 1H), 7.47 (d, J=8.8 Hz, 1H).

### (5) {cis-2-{3-[2-(1-Benzylpiperidin-4-yl)ethyl]-7-(N,N-dimethylaminomethyl)benz[d]isoxazol-6-yloxymethyl}cyclopropyl}methanol

The title compound (65 mg) was obtained by synthesis from {cis-2-{7-(N,N-dimethylaminomethyl)-3-[2-(piperidin-4-yl)ethyl]benz[d]isoxazol-6-yloxymethyl}cyclopropyl}methanol (97 mg) and benzaldehyde (53.1 µL) according to Example 21-(3).
¹H-NMR (400 MHz, CDCl₃) δ (ppm): 0.19-0.22 (m, 1H), 0.83-0.89 (m, 1H), 1.24-1.44 (m, 4H), 1.51-1.59 (m, 1H), 1.74-1.79 (m, 4H), 1.91-1.97 (m, 2H), 2.23 (s, 6H), 2.86-2.95 (m, 4H), 3.14-3.20 (m, 1H), 3.48 (s, 2H), 3.54-3.67 (m, 2H), 3.83-3.91 (m, 2H), 4.58 (dd, J=5.2, 6.8 Hz, 1H), 6.89 (d, J=8.8 Hz, 1H), 7.21-7.36 (m, 5H), 7.45 (d, J=8.8 Hz, 1H).

### (6) {cis-2-{3- [2-(1-Benzylpiperidin-4-yl) ethyl]-7-(N,N-dimethylaminomethyl)benz[d]isoxazol-6-yloxymethyl}cyclopropyl}methanol dihydrochloride

The title compound (70 mg) was obtained by synthesis from {cis-2-{3-[2-(1-benzylpiperidin-4-yl)ethyl]-7-(N,N-dimethylaminomethyl)benz[d]isoxazol-6-yloxymethyl}cyclopropyl}methanol (65 mg) according to Example 21-(4).
¹H-NMR (400 MHz, CD₃OD) δ (ppm): 0.38-0.43 (m, 1H), 0.92-0.98 (m, 1H), 1.34-1.43 (m, 1H), 1.54-1.65 (m, 3H), 1.69-1.76 (m, 1H), 1.82-1.91 (m, 2H), 2.06-2.09 (m, 2H), 2. 84 (s, 3H), 2.96-3.12 (m, 5H), 3.03 (s, 3H), 3.25-3.32 (m, 1H), 3.49-3.52 (m, 2H), 3.99-4.05 (m, 2H), 4.61 (s, 2H), 4.63-4.70 (m, 2H), 7.27 (d, J=8.8 Hz, 1H), 7.47-7.51 (m, 3H), 7.55-7.59 (m, 2H), 7.93 (d, J=8.8 Hz, 1H).
ESI-MS m/z: 239 [M+2H]²⁺, 478 [M+H]⁺.

### Example 4

### {1-{3-[2-(1-Benzylpiperidin-4-yl)ethyl]-7-(N,N-dimethylaminomethyl)benz[d]isoxazol-6-yloxymethyl}cyclopropyl}methanol dihydrochloride

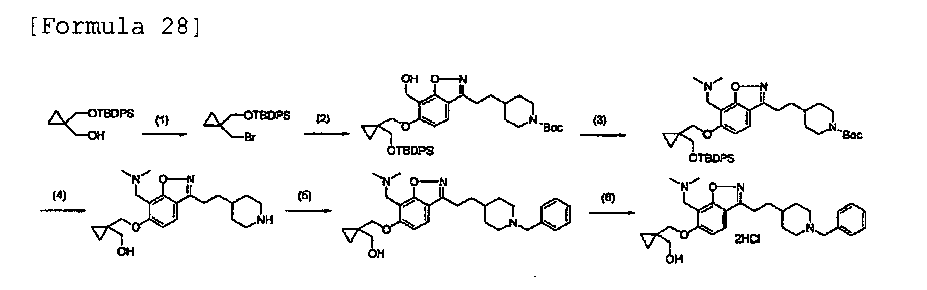

### (1)(2) tert-Butyl 4-{2-{6-[1-(tert-butyldiphenylsilanyloxymethyl)cyclopropylmethoxy]-7-hydroxymethylbenz[d]isoxazol-3-yl}ethyl}piperidine-1-carboxylate

(1-Bromomethylcyclopropylmethoxy)-tert-butyldiphenylsilane (1.11 g) was obtained by synthesis from [1-(tert-butyldiphenylsilanyloxymethyl)cyclopropyl]methanol obtained in Example 45-(1) (1.02 g) according to Example 3-(1). The title compound (1.1 g) was obtained by synthesis according to Example 16-(1) from (1-bromomethylcyclopropylmethoxy)-tert-butyldiphenylsilane (826 mg) and tert-butyl 4-[2-(6-hydroxy-7-hydroxymethylbenz[d]isoxazol-3-yl)ethyl]piperidine-1-carboxylate obtained in Preparation Example 2-(6). ¹H-NMR (400 MHz, CDCl₃) δ (ppm): 0.53-0.61 (m, 4H), 1.03 (s, 9H), 1.11-1.22 (m, 2H), 1.45 (s, 9H), 1.45-1.54 (m, 1H), 1.73-1.82 (m, 4H), 2.39 (t, J=6.4 Hz, 1H), 2.64-2.70 (m, 2H), 2.95-2.99 (m, 2H), 3.68 (s, 2H), 4.03-4.10 (m, 4H), 4.88 (d, J=6.4 Hz, 2H), 6.92 (d, J=8.8 Hz, 1H), 7.25-7.29 (m, 4H), 7.34-7.38 (m, 2H), 7.45 (d, J=8.8 Hz, 1H), 7.57-7.59 (m, 4H).

### (3) tert-Butyl 4-{2-{6-[1-(tert-butyldiphenylsilanyloxymethyl)cyclopropylmethoxy]-7-(N,N-dimethylaminomethyl)benz[d]isoxazol-3-yl}ethyl}piperidine-1-carboxylate

The title compound (650 mg) was obtained by synthesis from tert-butyl 4-{2-{6-[1-(tert-butyldiphenylsilanyloxymethyl)cyclopropylmethoxy]-7-hydroxymethylbenz[d]isoxazol-3-yl}ethyl}piperidine-1-carboxylate (838 mg) according to Example 21-(1). ¹H-NMR (400 MHz, CDCl₃) δ (ppm): 0.53-0.62 (m, 4H), 1.03 (s, 9H), 1.11-1.22 (m, 2H), 1.45 (s, 9H), 1.45-1.55 (m, 1H), 1.73-1.81 (m, 4H), 2.25 (s, 6H), 2.64-2.70 (m, 2H), 2.95-2.98 (m, 2H), 3.68 (s, 2H), 3.70 (s, 2H), 4.03-4.14 (m, 4H), 6.91 (d, J=8.8 Hz, 1H), 7.25-7.30 (m, 4H), 7.34-7.42 (m, 2H), 7.43 (d, J=8.8 Hz, 1H), 7.57-7.60 (m, 4H).

### (4) {1-{7-(N,N-Dimethylaminomethyl)-3-[2-(piperidin-4-yl)ethyl]benz[d]isoxazol-6-yloxymethyl}cyclopropyl}methanol

The title compound (305 mg) was obtained by synthesis from tert-butyl 4-{2-{6-[1-(tert-butyldiphenylsilanyloxymethyl)cyclopropylmethoxy]-7-(N,N-dimethylaminomethyl)benz[d]isoxazol-3-yl}ethyl}piperidine-1-carboxylate (580 mg) according to Example 21- (2) .
¹H-NMR (400 MHz, CDCl₃) δ (ppm): 0.59-0.67 (m, 4H), 1.12-1.23 (m, 2H), 1.45-1.51 (m, 1H), 1.74-1.80 (m, 4H), 2.26 (s, 6H), 2.55-2.62 (m, 2H), 2.92-2.97 (m, 2H), 3.05-3.09 (m, 2H), 3.59 (s, 2H), 3.78 (s, 2H), 4.05 (s, 2H), 6.89 (d, J=8.8 Hz, 1H), 7.47 (d, J=8.8 Hz, 1H).

### (5) {1-{3-[2-(1-Benzylpiperidin-4-yl)ethyl]-7-(N,N-dimethylaminomethyl)benz[d]isoxazol-6-yloxymethyl}cyclopropyl}methanol

The title compound (82 mg) was obtained by synthesis from {1-{7-(N,N-dimethylaminomethyl)-3-[2-(piperidin-4-yl)ethyl]benz[d]isoxazol-6-yloxymethyl}cyclopropyl}methanol (97 mg) and benzaldehyde (53.1 µL) according to Example 21-(3).
¹H-NMR (400 MHz, CDCl₃) δ (ppm): 0.59-0.66 (m, 4H), 1.24-1.35 (m, 3H), 1.73-1.80 (m, 4H), 1.91-1.97 (m, 2H), 2.25 (s, 6H), 2.87-2.95 (m, 4H), 3.48 (s, 2H), 3.58 (s, 2H), 3.77 (s, 2H), 4.04 (s, 2H), 6. 89 (d, J=8.8 Hz, 1H), 7.21-7.34 (m, 5H), 7.45 (d, J=8.8 Hz, 1H).

### (6) {1-{3-[2-(1-Benzylpiperidin-4-yl) ethyl]-7-(N,N-dimethylaminomethyl)benz[d]isoxazol-6-yloxymethyl}cyclopropyl}methanol dihydrochloride

The title compound (88 mg) was obtained by synthesis from {1-{3-[2-(1-benzylpiperidin-4-yl)ethyl]-7-(N,N-dimethylaminomethyl)benz[d]isoxazol-6-yloxymethyl}cyclopropyl}methanol (82 mg) according to Example 21-(4).
¹H-NMR (400 MHz, CD₃OD) δ (ppm): 0.68-0.76 (m, 4H), 1.53-1.63 (m, 2H), 1.67-1.77 (m, 1H), 1.81-1.89 (m, 2H), 2.02-2.08 (m, 2H), 2.93 (s, 6H), 2.97-3.10 (m, 4H), 3.47-3.52 (m, 2H), 3.64 (s, 2H), 4.23 (s, 2H), 4.32 (s, 2H), 4.59 (s, 1H), 4.63 (s, 2H), 7.22 (d, J=8.8 Hz, 1H), 7.46-7.49 (m, 3H), 7.54-7.59 (m, 2SH), 7.91 (d, J=8.8 Hz, 1H).
ESI-MS m/z: 239 [M+2H]²⁺, 478 [M+H]⁺.

### Example 5

### N,N-Dimethyl{3-[2-(1-benzylpiperidin-4-yl)ethyl]-6-(1-methoxymethylcyclopropylmethoxy)benz[d]isoxazol-7-yl}methylamine dihydrochloride

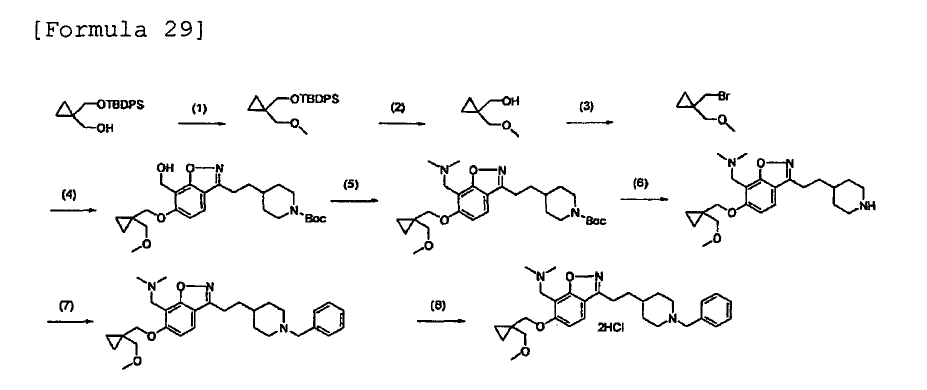

### (1) tert-Butyl-(1-methoxymethylcyclopropylmethoxy)diphenylsilane

The title compound (3.5 g) was obtained by synthesis from [1-(tert-butyldiphenylsilanyloxymethyl)cyclopropyl]methanol obtained in Example 45-(1) (3.4 g) according to Example 14-(4).
¹H-NMR (400 MHz, CDCl₃) δ (ppm): 0.41-0.44 (m, 4H), 1.05 (s, 9H), 3.32 (s, 3H), 3.36 (s, 2H), 3.60 (s, 2H), 7.32-7.42 (m, 6H), 7.62-7.66 (m, 4H).

### (2)(3)(4) tert-Butyl 4-{2-[7-hydroxymethyl-6-(1-methoxymethylcyclopropylmethoxy)benz[d]isoxazol-3-yl]ethyl}piperidine-1-carboxylate

(1-Methoxymethylcyclopropyl)methanol was obtained by synthesis from tert-butyl-(1-methoxymethylcyclopropylmethoxy)diphenylsilane (1.06 g) according to Example 14-(5). 1-Bromomethyl-1-methoxymethylcyclopropane was obtained by synthesis from the (1-methoxymethylcyclopropyl)methanol without further purification according to Example 3-(1). The title compound (900 mg) was obtained by synthesis from the 1-bromomethyl-1-methoxymethylcyclopropane used without further purification and tert-butyl 4-[2-(6-hydroxy-7-hydroxymethylbenz[d]isoxazol-3-yl)ethyl]piperidine-1-carboxylate obtained in Preparation Example 2-(6) (757 mg) according to Example 16-(1) .
¹H-NMR (400 MHz, CDCl₃) δ (ppm): 0.66-0.72 (m, 4H), 1.11-1.19 (m, 2H), 1.42-1.50 (m, 1H), 1.45 (s, 9H), 1.72-1.78 (m, 4H), 2.63-2.69 (m, 2H), 2.93-2.98 (m, 2H), 3.35 (s, 3H), 3.41 (s, 2H), 3.59 (t, J=7.2 Hz, 1H), 3.99-4.16 (m, 2H), 4.03 (s, 2H), 5.00 (d, J=7.2 Hz, 2H), 6.89 (d, J=8.4 Hz, 1H), 7.43 (d, J=8.4 Hz, 1H).

### (5) tert-Butyl 4-{2-[7-(N,N-dimethylaminomethyl)-6-(1-methoxymethylcyclopropylmethoxy)benz[d]isoxazol-3-yl]ethyl}piperidine-1-carboxylate

The title compound (650 mg) was obtained by synthesis from tert-butyl 4-{2-[7-hydroxymethyl-6-(1-methoxymethylcyclopropylmethoxy)benz[d]isoxazol-3-yl]ethyl}piperidine-1-carboxylate (949 mg) according to Example 21-(1).
¹H-NMR (400 MHz, CDCl₃) δ (ppm): 0.62-0.73 (m, 4H), 1.11-1.20 (m, 2H), 1.45 (s, 9H), 1.45-1.54 (m, 1H), 1.72-1.80 (m, 4H), 2.33 (s, 6H), 2.64-2.73 (m, 2H), 2.93-2.98 (m, 2H), 3.33 (s, 3H), 3.41 (s, 2H), 3.87 (s, 2H), 4.00 (s, 2H), 4.02-4.14 (m, 2H), 6.92 (d, J=8.4 Hz, 1H), 7.46 (d, J=8.4 Hz, 1H).

### (6) N,N-Dimethyl{6-(1-methoxymethylcyclopropylmethoxy)-3-[2-(piperidin-4-yl)ethyl]benz[d]isoxazol-7-yl}methylamine

The title compound (100 mg) was obtained by synthesis from tert-butyl 4-{2-[7-(N,N-dimethylaminomethyl)-6-(1-methoxymethylcyclopropylmethoxy)benz[d]isoxazol-3-yl]ethyl}piperidine-1-carboxylate (151 mg) according to Example 21-(2).
¹H-NMR (400 MHz, CDCl₃) δ (ppm): 0.61-0.70 (m, 4H), 1.11-1.21 (m, 2H), 1.43-1.53 (m, 1H), 1.72-1.78 (m, 4H), 2.33 (s, 6H), 2.54-2.61 (m, 2H), 2.92-2.96 (m, 2H), 3.04-3.08 (m, 2H), 3.35 (s, 3H), 3.42 (s, 2H), 3.81 (s, 2H), 4.00 (s, 2H), 6.92 (d, J=8.8 Hz, 1H), 7.44 (d, J=8.8 Hz, 1H).

### (7) N,N-Dimethyl{3-[2-(1-benzylpiperidin-4-yl)ethyl]-6-(1-methoxymethylcyclopropylmethoxy)benz[d]isoxazol-7-yl}methylamine

The title compound (19 mg) was obtained by synthesis from N,N-dimethyl{6-(1-methoxymethylcyclopropylmethoxy)-3-[2-(piperidin-4-yl)ethyl]benz[d]isoxazol-7-yl}methylamine (40.2 mg) and benzaldehyde (21.2 µL) according to Example 21-(3).
¹H-NMR (400 MHz, CDCl₃) δ (ppm): 0.60-0.64 (m, 2H), 0.66-0.70 (m, 2H), 1.25-1.34 (m, 3H), 1.70-1.78 (m, 4H), 1.90-1.96 (m, 2H), 2.33 (s, 6H), 2.86-2.95 (m, 4H), 3.35 (s, 3H), 3.42 (s, 2H), 3.48 (s, 2H), 3.81 (s, 2H), 4.00 (s, 2H), 6.89 (d, J=8.8 Hz, 1H), 7.23 (m, 1H), 7.27-7.30 (m, 4H), 7.43 (d, J=8.8 Hz, 1H).

### (8) {N,N-Dimethyl{3-[2-(1-benzylpiperidin-4-yl)ethyl]-6-(1-methoxymethylcyclopropylmethoxy)benz[d]isoxazol-7-yl}methylamine dihydrochloride

The title compound (13 mg) was obtained by synthesis from {N,N-dimethyl{3-[2-(1-benzylpiperidin-4-yl) ethyl]-6-(1-methoxymethylcyclopropylmethoxy)benz[d]isoxazol-7-yl}methylamine (19 mg) according to Example 21-(4).
¹H-NMR (400 MHz, CD₃OD) δ (ppm): 0.58-0.67 (m, 4H), 1.38-1.48 (m, 2H), 1.57-1.67 (m, 1H), 1.72-1.79 (m, 2H), 1.95-1.99 (m, 2H), 2.87 (s, 6H), 2.87-2.98 (m, 4H), 3.27 (s, 3H), 3.37 (s, 2H), 3.37-3.42 (m, 2H), 4.08 (s, 2H), 4.21 (s, 2H), 4.54 (s, 2H), 7.13 (d, J=8.8 Hz, 1H), 7.38-7.46 (m, 5H), 7.82 (d, J=8.8 Hz, 1H).
ESI-MS m/z: 246 [M+2H]²⁺, 492 [M+H]⁺.

### Example 6

### N,N-Dimethyl{3-[2-(1-benzylpiperidin-4-yl)ethyl]-6-(tetrahydrofuran-3-ylmethoxy)benz[d]isoxazol-7-yl}methylamine dihydrochloride

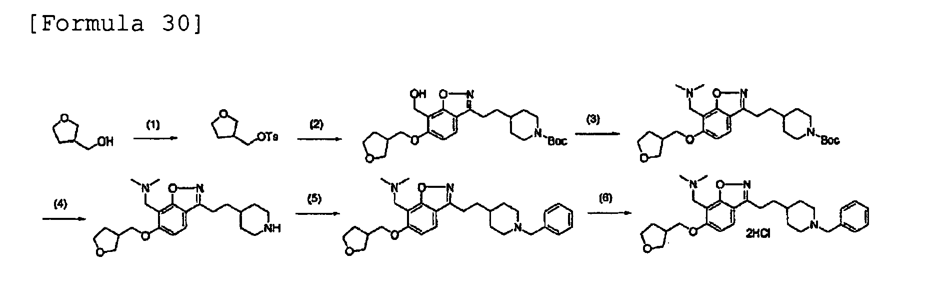

### (1) Tetrahydrofuran-3-ylmethyl 4-methylbenzenesulfonate

Tetrahydro-3-furanmethanol (4.09 g) and triethylamine (7.81 mL) were dissolved in tetrahydrofuran (30 mL). p-Toluenesulfonyl chloride (9.53 g) was added under ice-cooling, followed by stirring for one hour. Then, the mixture was heated to room temperature and further stirred for 18 hours. Water was added to the reaction mixture, followed by extraction with ethyl acetate. Then, the organic layer was sequentially washed with a saturated sodium chloride solution and a saturated sodium bicarbonate solution, dried over magnesium sulfate and filtered. Then, the solvent was evaporated under reduced pressure. The residue was purified by silica gel column chromatography (heptane-ethyl acetate) to obtain the title compound (8.9 g).
¹H-NMR (400 MHz, CDCl₃) δ (ppm): 1.50-1.60 (m, 1H) , 1.96-2.04 (m, 1H), 2.45 (s, 3H), 2.53-2.64 (m, 1H), 3.47-3.51 (m, 1H), 3.65-3.81 (m, 3H), 3.88-3.93 (m, 1H), 3.96-4.00 (m, 1H), 7.32-7.36 (m, 2H), 7.76-7.79 (m, 2H).

### (2) tert-Butyl 4-{2-[7-hydroxymethyl-6-(tetrahydrofuran-3-ylmethoxy)benz[d]isoxazol-3-yl]ethyl}piperidine-1-carboxylate

Tetrahydrofuran-3-ylmethyl 4-methylbenzenesulfonate (1.03 g) and tert-butyl 4-[2-(6-hydroxy-7-hydroxymethylbenz[d]isoxazol-3-yl)ethyl]piperidine-1-carboxylate obtained in Preparation Example 2-(6) (752 mg) were dissolved in N,N-dimethylformamide (8 mL). Potassium carbonate (829 mg) and sodium iodide (600 mg) were added and the mixture was stirred at 70°C for 20 hours. Water was added to the reaction mixture, followed by extraction with ethyl acetate. The organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (616 mg).
¹H-NMR (400 MHz, CDCl₃) δ (ppm): 1.08-1.20 (m, 2H), 1.46 (s, 9H), 1.49 (m, 1H), 1.75-1.82 (m, 5H), 2.14-2.23 (m, 1H), 2.63-2.70 (m, 2H), 2.77-2.83 (m, 1H), 2.92-2.98 (m, 2H), 3.76-4.14 (m, 8H), 5.00 (s, 2H), 6.91 (d, J=8.8 Hz, 1H), 7.47 (d, J=8.8 Hz, 1H).
ESI-MS m/z: 483 [M+Na]⁺.

### (3) tert-Butyl 4-{2-[7-(N,N-dimethylaminomethyl)-6-(tetrahydrofuran-3-ylmethoxy)benz[d]isoxazol-3-yl]ethyl}piperidine-1-carboxylate

The title compound (256 mg) was obtained by synthesis from tert-butyl 4-{2-[7-hydroxymethyl-6-(tetrahydrofuran-3-ylmethoxy)benz[d]isoxazol-3-yl]ethyl}piperidine-1-carboxylate (461 mg) according to Example 21-(1).
¹H-NMR (400 MHz, CDCl₃) δ (ppm): 1.11-1.20 (m, 2H), 1.45 (s, 9H), 1.45-1.55 (m, 1H), 1.68-1.79 (m, 5H), 2.11-2.20 (m, 1H), 2.32 (s, 6H), 2.64-2.70 (m, 2H), 2.77-2.86 (m, 1H), 2.93-2.98 (m, 2H), 3.72-3.83 (m, 4H), 3.90-4.20 (m, 6H), 6.91 (d, J=8.8 Hz, 1H), 7.46 (d, J=8.8 Hz, 1H).

### (4) N,N-Dimethyl{3-[2-(piperidin-4-yl)ethyl]-6-(tetrahydrofuran-3-ylmethoxy)benz[d]isoxazol-7-yl}methylamine

The title compound (154 mg) was obtained by synthesis from tert-butyl 4-{2-[7-(N,N-dimethylaminomethyl)-6-(tetrahydrofuran-3-ylmethoxy)benz[d]isoxazol-3-yl]ethyl}piperidine-1-carboxylate (195 mg) according to Example 21-(2).
¹H-NMR (400 MHz, CDCl₃) δ (ppm): 1.12-1.23 (m, 2H), 1.42-1.51 (m, 1H), 1.73-1.82 (m, 5H), 2.10-2.19 (m, 1H), 2.32 (s, 6H), 2.55-2.61 (m, 2H), 2.77-2.86 (m, 1H), 2.92-2.97 (m, 2H), 3.04-3.08 (m, 2H), 3.70-3.83 (m, 4H), 3.92-4.06 (m, 4H), 6.91 (d, J=8.4 Hz, 1H), 7.46 (d, J=8.4 Hz, 1H).

### (5) N,N-Dimethyl{3-[2-(1-benzylpiperidin-4-yl)ethyl]-6-(tetrahydrofuran-3-ylmethoxy)benz[d]isoxazol-7-yl}methylamine

The title compound (132 mg) was obtained by synthesis from N,N-dimethyl{3-[2-(piperidin-4-yl)ethyl]-6-(tetrahydrofuran-3-ylmethoxy)benz[d]isoxazol-7-yl}methylamine (116 mg) and benzaldehyde (63.7 µL) according to Example 21-(3).
¹H-NMR (400 MHz, CDCl₃) δ (ppm): 1.24-1.34 (m, 3H), 1.75-1.82 (m, 5H), 1.90-1.96 (m, 2H), 2.10-2.19 (m, 1H), 2.31 (s, 6H), 2.78-2.95 (m, 5H), 3.48 (s, 2H), 3.72-3.83 (m, 4H), 3.89-4.07 (m, 4H), 6.90 (d, J=8.8 Hz, 1H), 7.20-7.25 (m, 1H), 7.28-7.30 (m, 4H), 7.44 (d, J=8.8 Hz, 1H).

### (6) N,N-Dimethyl{3-[2-(1-benzylpiperidin-4-yl)ethyl]-6-(tetrahydrofuran-3-ylmethoxy)benz[d]isoxazol-7-yl}methylamine dihydrochloride

The title compound (142 mg) was obtained by synthesis from N,N-dimethyl{3-[2-(1-benzylpiperidin-4-yl)ethyl]-6-(tetrahydrofuran-3-ylmethoxy)benz[d]isoxazol-7-yl}methylamine (132 mg) according to Example 21-(4).
¹H-NMR (400 MHz, CD₃OD) δ (ppm): 1.50-1.59 (m, 2H), 1.67-1.77 (m, 1H), 1.81-1.87 (m, 4H), 2.02-2.06 (m, 2H), 2.18-2.27 (m, 1H), 2.94 (s, 3H), 2.97 (s, 3H), 3.01-3.11 (m, 4H), 3.48-3.52 (m, 2H), 3.77-3.82 (m, 2H), 3.91-4.00 (m, 2H), 4.18-4.28 (m, 2H), 4.31 (s, 2H), 4.61 (s, 2H), 7.27 (d, J=8.8 Hz, 1H), 7.48-7.55 (m, 5H), 7.93 (d, J=8.8 Hz, 1H).
ESI-MS m/z: 239 [M+2H]2⁺, 478 [M+H]⁺.

### Example 7

### N,N-Dimethyl{3-[2-(1-benzylpiperidin-4-yl)ethyl]-6-cyclopentyloxybenz[d]isoxazol-7-yl}methylamine dihydrochloride

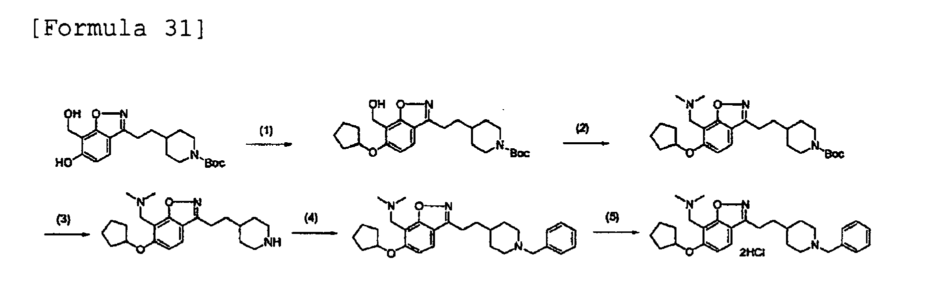

### (1) tert-Butyl 4-[2-(6-cyclopentyloxy-7-hydroxymethylbenz[d]isoxazol-3-yl)ethyl]piperidine-1-carboxylate

tert-Butyl 4-[2-(6-hydroxy-7-hydroxymethylbenz[d]isoxazol-3-yl)ethyl]piperidine-1-carboxylate obtained in Preparation Example 2-(6) (752 mg) and cyclopentyl bromide (447 mg) were dissolved in N,N-dimethylformamide (5 mL). Potassium carbonate (553 mg) was added and the mixture was stirred at 65°C for 22 hours. Water was added to the reaction mixture, followed by extraction with ethyl acetate. The organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (853 mg).
¹H-NMR (400 MHz, CDCl₃) δ (ppm): 1.10-1.20 (m, 2H), 1.45 (s, 9H), 1.45-1.53 (m, 1H), 1.61-1.91 (m, 12H), 2.48 (t, J=6.8 Hz, 1H), 2.64-2.70 (m, 2H), 2.93-2.98 (m, 2H), 4.08-4.12 (m, 2H), 4.92-4.95 (m, 1H), 4.96 (d, J=6.8 Hz, 2H), 6.94 (d, J=8.8 Hz, 1H), 7.45 (d, J=8.8 Hz, 1H).

### (2) tert-Butyl 4-{2-[6-cyclopentyloxy-7-(N,N-dimethylaminomethyl)benz[d]isoxazol-3-yl]ethyl}piperidine-1-carboxylate

The title compound (565 mg) was obtained by synthesis from tert-butyl 4-[2-(6-cyclopentyloxy-7-hydroxymethylbenz[d]isoxazol-3-yl)ethyl]piperidine-1-carboxylate (798 mg) according to Example 21-(1).
¹H-NMR (400 MHz, CDCl₃) δ (ppm): 1.10-1.20 (m, 2H), 1.44-1.54 (m, 1H), 1.45 (s, 9H), 1.63-1.99 (m, 12H), 2.31 (s, 6H), 2.64-2.70 (m, 2H), 2.92-2.97 (m, 2H), 3.76 (s, 2H), 4.03-4.13 (m, 2H), 4.86-4.90 (m, 1H), 6.92 (d, J=8.8 Hz, 1H), 7.42 (d, J=8.8 Hz, 1H).

### (3) N,N-Dimethyl{6-cyclopentyloxy-3-[2-(piperidin-4-yl)ethyl]benz[d]isoxazol-7-yl}methylamine

The title compound (263 mg) was obtained by synthesis from tert-butyl 4-{2-[6-cyclopentyloxy-7-(N,N-dimethylaminomethyl)benz[d]isoxazol-3-yl]ethyl}piperidine-1-carboxylate (378 mg) according to Example 21-(2).
¹H-NMR (400 MHz, CDCl₃) δ (ppm): 1.11-1.22 (m, 2H), 1.43-1.53 (m, 1H), 1.61-1.96 (m, 12H), 2.31 (s, 6H), 2.55-2.60 (m, 2H), 2.91-2.96 (m, 2H), 3.04-3.08 (m, 2H), 3.75 (s, 2H), 4.87-4.91 (m, 1H), 6.92 (d, J=8.8 Hz, 1H), 7.43 (d, J=8.8 Hz, 1H).

### (4) N,N-Dimethyl{3-[2-(1-benzylpiperidin-4-yl)ethyl]-6-cyclopentyloxybenz[d]isoxazol-7-yl}methylamine

The title compound (58 mg) was obtained by synthesis from N,N-dimethyl{6-cyclopentyloxy-3-[2-(piperidin-4-yl)ethyl]benz[d]isoxazol-7-yl}methylamine (112 mg) and benzaldehyde (63.7 mg) according to Example 21-(3).
¹H-NMR (400 MHz, CDCl₃) δ (ppm): 1.25-1.38 (m, 3H), 1.61-1.97 (m, 14H), 2.30 (s, 6H), 2.86-2.94 (m, 4H), 3.48 (s, 2H), 3.75 (s, 2H), 4.86-4.91 (m, 1H), 6.91 (d, J=8.8 Hz, 1H), 7.19-7.24 (m, 1H), 7.26-7.30 (m, 4H), 7.42 (d, J=8.8 Hz, 1H).

### (6) N,N-Dimethyl{3-[2-(1-benzylpiperidin-4-yl)ethyl]-6-cyclopentyloxybenz[d]isoxazol-7-yl}methylamine dihydrochloride

The title compound (142 mg) was obtained by synthesis from N,N-dimethyl{3-[2-(1-benzylpiperidin-4-yl)ethyl]-6-cyclopentyloxybenz[d]isoxazol-7-yl}methylamine (132 mg) according to Example 21-(4).
¹H-NMR (400 MHz, CD₃OD) δ (ppm): 1.48-1.60 (m, 2H), 1.70-2.10 (m, 13H), 2.93 (s, 6H), 2.98-3.10 (m, 4H), 3.48-3.51 (m, 2H), 4.31 (s, 2H), 4.56 (s, 2H), 5.09-5.12 (m, 1H), 7.24 (d, J=8.8 Hz, 1H), 7.48-7.54 (m, 5H), 7.90 (d, J=8.8 Hz, 1H).
ESI-MS m/z: 231 [M+2H]²⁺, 462 [M+H]⁺.

### Example 8

### N,N-Dimethyl{3-[2-(1-benzylpiperidin-4-yl)ethyl]-6-(2,2,2-trifluoroethoxy)benz[d]isoxazol-7-yl}methylamine dihydrochloride

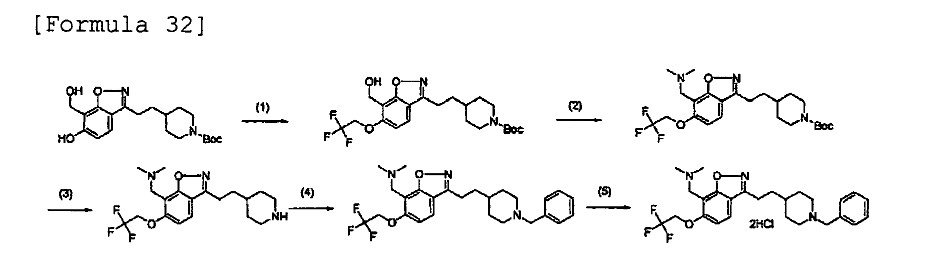

### (1) tert-Butyl 4-{2-[7-hydroxymethyl-6-(2,2,2-trifluoroethoxy)benz[d]isoxazol-3-yl]ethyl}piperidine-1-carboxylate

tert-Butyl 4-[2-(6-hydroxy-7-hydroxymethylbenz[d]isoxazol-3-yl)ethyl]piperidine-1-carboxylate obtained in Preparation Example 2-(6) (752 mg) and 2,2,2-trifluoroethyl trifluoromethanesulfonate (928 mg) were dissolved in N,N-dimethylformamide (5 mL). Potassium carbonate (829 mg) was added and the mixture was stirred at 65°C for 19 hours. Water was added to the reaction mixture, followed by extraction with ethyl acetate. The organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (235 mg).
¹H-NMR (400 MHz, CDCl₃) δ (ppm): 1.11-1.21 (m, 2H), 1.45 (s, 9H), 1.45-1.53 (m, 1H), 1.73-1.81 (m, 4H), 2.27 (t, J=6.8 Hz, 1H), 2.64-2.70 (m, 2H), 2.96-3.00 (m, 2H), 4.05-4.15 (m, 2H), 4.48-4.56 (m, 2H), 5.04 (d, J=6.8 Hz, 2H), 6.91 (d, J=8.4 Hz, 1H), 7.53 (d, J=8.4 Hz, 1H).

### (2) tert-Butyl 4-{2-[7-(N,N-dimethylaminomethyl)-6-(2,2,2-trifluoroethoxy)benz[d]isoxazol-3-yl]ethyl}piperidine-1-carboxylate

The title compound (85 mg) was obtained by synthesis from tert-butyl 4-{2-[7-hydroxymethyl-6-(2,2,2-trifluoroethoxy)benz[d]isoxazol-3-yl]ethyl}piperidine-1-carboxylate (138 mg) according to Example 21-(1).
¹H-NMR (400 MHz, CDCl₃) δ (ppm): 1.11-1.21 (m, 2H), 1.46 (s, 9H), 1.48-1.56 (m, 1H), 1.70-1.81 (m, 4H), 2.32 (s, 6H), 2.64-2.71 (m, 2H), 2.95-2.99 (m, 2H), 3.80 (s, 2H), 4.04-4.14 (m, 2H), 4.46-4.52 (m, 2H), 6.90 (d, J=8.8 Hz, 1H), 7.50 (d, J=8.8 Hz, 1H).

### (3) N,N-Dimethyl{3-[2-(piperidin-4-yl)ethyl]-6-(2,2,2-trifluoroethoxy)benz[d]isoxazol-7-yl}methylamine

The title compound (35 mg) was obtained by synthesis from tert-butyl 4-{2-[7-(N,N-dimethylaminomethyl)-6-(2,2,2-trifluoroethoxy)benz[d]isoxazol-3-yl]ethyl}piperidine-1-carboxylate (77.7 mg) according to Example 21-(2).
¹H-NMR (400 MHz, CDCl₃) δ (ppm): 1.12-1.25 (m, 2H), 1.43-1.52 (m, 1H), 1.73-1.79 (m, 4H), 2.32 (s, 6H), 2.56-2.62 (m, 2H), 2.94-2.99 (m, 2H), 3.04-3.09 (m, 2H), 3.80 (s, 2H), 4.46-4.52 (m, 2H), 6.89 (d, J=8.8 Hz, 1H) , 7.51 (d, J=8.8 Hz, 1H).

### (4) N,N-Dimethyl{3-[2-(1-benzylpiperidin-4-yl)ethyl]-6-(2,2,2-trifluoroethoxy)benz[d]isoxazol-7-yl}methylamine

The title compound (10 mg) was obtained by synthesis from N,N-dimethyl{3-[2-(piperidin-4-yl)ethyl]-6-(2,2,2-trifluoroethoxy)benz[d]isoxazol-7-yl}methylamine (30.8 mg) and benzaldehyde (63.7 mg) according to Example 21-(3).
¹H-NMR (400 MHz, CDCl₃) δ (ppm): 1.25-1.35 (m, 3H), 1.73-1.79 (m, 4H), 1.91-1.97 (m, 2H), 2.31 (s, 6H), 2.87-2.97 (m, 4H), 3.49 (s, 2H), 3.80 (s, 2H), 4.45-4.51 (m, 2H), 6.88 (d, J=8.4 Hz, 1H), 7.23 (m, 1H), 7.28-7.31 (m, 4H), 7.49 (d, J=8.4 Hz, 1H).

### (5) N,N-Dimethyl{3-[2-(1-benzylpiperidin-4-yl)ethyl]-6-(2,2,2-trifluoroethoxy)benz[d]isoxazol-7-yl}methylamine dihydrochloride

The title compound (12 mg) was obtained by synthesis from N,N-dimethyl{3-[2-(1-benzylpiperidin-4-yl)ethyl]-6-(2,2,2-trifluoroethoxy)benz[d]isoxazol-7-yl}methylamine (10 mg) according to Example 21-(4).
¹H-NMR (400 MHz, CD₃OD) δ (ppm): 1.40-1.50 (m, 2H), 1.70-1.80 (m, 1H), 1.83-1.87 (m, 2H), 2.07-2.10 (m, 2H), 2.31 (s, 6H), 3.00-3.10 (m, 4H), 3.48-3.52 (m, 2H), 4.30 (s, 2H), 4.63 (s, 2H), 4.87-4.92 (m, 2H), 7.32 (d, J=8.8 Hz, 1H), 7.47-7.54 (m, 5H), 8.00 (d, J=8.8 Hz, 1H).
ESI-MS m/z: 476 [M+H]⁺.

### Example 9

### N,N-Dimethyl{3-{2-[1-(1,3-benzo[d]dioxol-5-ylmethyl)piperidin-4-yl]ethyl}-6-cyclopropylmethoxybenz[d]isoxazol-7-yl}methylamine dihydrochloride

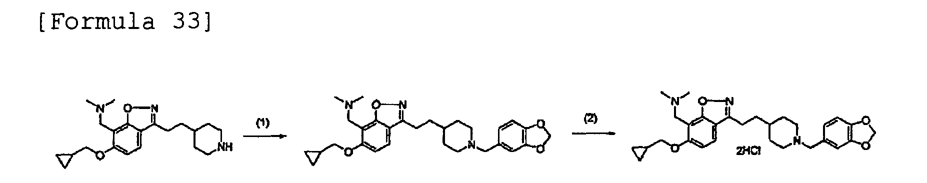

### (1) N,N-Dimethyl(3-{2-[1-(1,3-benzo [d] dioxol-5-ylmethyl)piperidin-4-yl]ethyl}-6-cyclopropylmethoxybenz[d]isoxazol-7-yl}methylamine

The title compound (128 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (107 mg) and 3,4-(methylenedioxy)benzaldehyde (90.1 mg) according to Example 21-(3).
¹H-NMR (400 MHz, CDCl₃) δ (ppm): 0.35-0.39 (m, 2H), 0.62-0.67 (m, 2H), 1.23-1.34 (m, 4H), 1.72-1.79 (m, 4H), 1.91-2.04 (m, 2H), 2.33 (s, 6H), 2.84-2.87 (m, 2H), 2.90-2.95 (m, 2H), 3.38 (s, 2H), 3.82 (s, 2H), 3.93 (d, J=6. 8 Hz, 2H), 5. 92 (s, 2H), 6.71-6.72 (m, 2H), 6.83 (s, 1H), 6.88 (d, J=8.8 Hz, 1H), 7.42 (d, J=8.8 Hz, 1H).

### (2) N,N-Dimethyl{3-{2-[1-(1,3-benzo[d]dioxol-5-ylmethyl)piperidin-4-yl]ethyl}-6-cyclopropylmethoxybenz[d]isoxazol-7-yl}methylamine dihydrochloride

The title compound (135 mg) was obtained by synthesis from N,N-dimethyl{3-{2-[1-(1,3-benzo[d]dioxol-5-ylmethyl)piperidin-4-yl]ethyl}-6-cyclopropylmethoxybenz[d]isoxazol-7-yl}methylamine (128 mg) according to Example 21-(4).
¹H-NMR (400 MHz, CD₃OD) δ (ppm): 0.42-0.46 (m, 2H), 0.67-0.70 (m, 2H), 1.35-1.45 (m, 1H), 1.50-1.59 (m, 2H), 1.66-1.76 (m, 1H), 1.82-1.88 (m, 2H), 2.00-2.09 (m, 2H), 2.95-3.00 (m, 2H), 2.96 (s, 6H), 3.03-3.07 (m, 2H), 3.46-3.50 (m, 2H), 4.12 (d, J=6.8 Hz, 2H), 4.21 (s, 2H), 4.63 (s, 2H), 6.01 (s, 2H), 6. 90 (d, J=8.0 Hz, 1H), 7.00 (dd, J=2.0, 8.0 Hz, 1H), 7.04 (d, J=2.0 Hz, 1H), 7.23 (d, J=8.8 Hz, 1H), 7.91 (d, J=8.8 Hz, 1H).
ESI-MS m/z: 492 [M+H]⁺.

### Example 10

### N,N-Dimethyl{6-cyclopropylmethoxy-3-{2-[1-(2,2-dimethylpropyl)piperidin-4-yl]ethyl}benz[d]isoxazol-7-yl}methylamine dihydrochloride

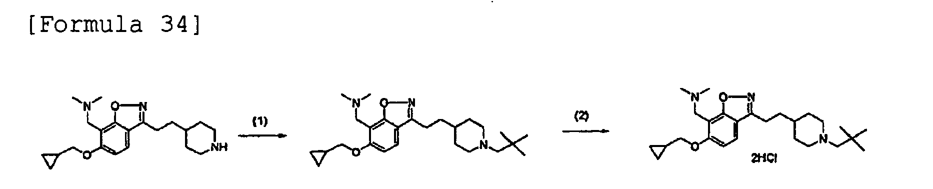

### (1) N,N-Dimethyl{6-cyclopropylmethoxy-3-{2-[1-(2,2-dimethylpropyl)piperidin-4-yl]ethyl}benz[d]isoxazol-7-yl}methylamine

The title compound (10 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (107 mg) and trimethylacetaldehyde (66.3 µL) according to Example 21-(3).
¹H-NMR (400 MHz, CDCl₃) δ (ppm): 0.35-0.39 (m, 2H), 0.62-0.67 (m, 2H), 0.85 (s, 9H), 1.19-1.37 (m, 4H), 1.73-1.77 (m, 4H), 2.01 (s, 2H), 2.12-2.18 (m, 2H), 2.33 (s, 6H), 2.76-2.80 (m, 2H), 2.90-2.95 (m, 2H), 3.82 (s, 2H), 3.93 (d, J=6.8 Hz, 2H), 6.88 (d, J=8.8 Hz, 1H), 7.44 (d, J=8.8 Hz, 1H).

### (2) N,N-Dimethyl{6-cyclopropylmethoxy-3-{2-[1-(2,2-dimethylpropyl)piperidin-4-yl]ethyl}benz[d]isoxazol-7-yl}methylamine dihydrochloride

The title compound (8 mg) was obtained by synthesis from N,N-dimethyl{6-cyclopropylmethoxy-3-{2-[1-(2,2-dimethylpropyl)piperidin-4-yl]ethyl}benz[d]isoxazol-7-yl}methylamine (10 mg) according to Example 21-(4).
¹H-NMR (400 MHz, CD₃OD) δ (ppm): 0.42-0.46 (m, 2H), 0.68-0.72 (m, 2H), 1.12 (s, 9H), 1.29-1.41 (m, 1H), 1.68-1.75 (m, 3H) 1.84-1.93 (m, 2H), 2.03-2.06 (m, 2H), 2.96 (s, 6H), 3.04-3.13 (m, 6H), 3.61-3.66 (m, 2H), 4.11 (d, J=7.2 Hz, 2H), 4.63 (s, 2H), 7.24 (d, J=8.8 Hz, 1H), 7.92 (d, J=8.8 Hz, 1H).
ESI-MS m/z: 214 [M+2H]²⁺, 428 [M+H]⁺.

### Example 11

### N,N-Dimethyl{3-[2-(1-cyclohexylmethylpiperidin-4-yl]ethyl}-6-cyclopropylmethoxybenz[d]isoxazol-7-yl)methylamine dihydrochloride

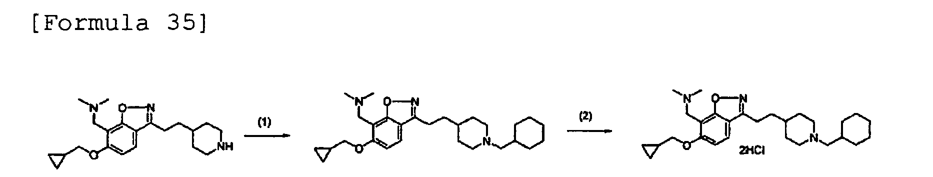

### (1) N,N-Dimethyl{3-[2-(1-cyclohexylmethylpiperidin-4-yl]ethyl}-6-cyclopropylmethoxybenz[d]isoxazol-7-yl)methylamine

The title compound (51 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (107 mg) and cyclohexanecarbaldehyde (72.4 µL) according to Example 21-(3).
¹H-NMR (400 MHz, CDCl₃) δ (ppm): 0.35-0.39 (m, 2H), 0.62-0.67 (m, 2H), 0.82-0.95 (m, 3H), 1.13-1.34 (m, 9H), 1.43-1.50 (m, 2H), 1.64-1.85 (m, 7H), 2.07 (d, J=6.8 Hz, 2H), 2.33 (s, 6H), 2.83-2.86 (m, 2H), 2.91-2.95 (m, 2H), 3.82 (s, 2H), 3.93 (d, J=6.8 Hz, 2H), 6. 88 (d, J=8.8 Hz, 1H), 7.43 (d, J=8.8 Hz, 1H).

### (2) N,N-Dimethyl{3-[2-(1-cyclohexylmethylpiperidin-4-yl]ethyl}-6-cyclopropylmethoxybenz[d]isoxazol-7-yl)methylamine dihydrochloride

The title compound (135 mg) was obtained by synthesis from N,N-dimethyl{3-[2-(1-cyclohexylmethylpiperidin-4-yl)ethyl]-6-cyclopropylmethoxybenz[d]isoxazol-7-yl}methylamine (128 mg) according to Example 21- (4).
¹H-NMR (400 MHz, CD₃OD) δ (ppm): 0.42-0.46 (m, 2H), 0.67-0.73 (m, 2H), 1.02-1.11 (m, 2H), 1.16-1.43 (m, 5H) 1.56-1.89 (m, 10H), 2.05-2.10 (m, 2H), 2.91-2.97 (m, 2H), 2.96 (s, 6H), 3.05-3.09 (m, 2H), 3.29-3.35 (m, 2H), 3.58-3.63 (m, 2H), 4.11 (d, J=7.2 Hz, 2H), 4.63 (s, 2H), 7.23 (d, J=8.8 Hz, 1H), 7.92 (d, J=8.8 Hz, 1H).
ESI-MS m/z: 227 [M+2H]²⁺, 454 [M+H]⁺.

### Example 12

### N,N-Dimethyl{3-{2-[1-(1,3-benzo[d]dioxol-4-ylmethyl)piperidin-4-yl]ethyl}-6-cyclopropylmethoxybenz[d]isoxazol-7-yl}methylamine dihydrochloride

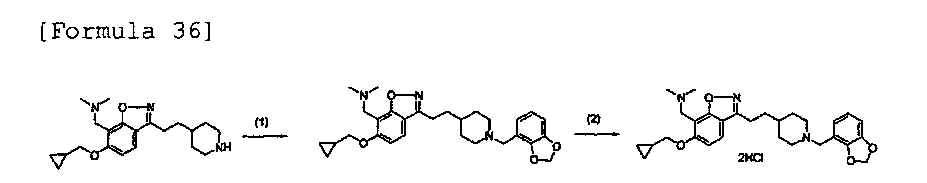

### (1) N,N-Dimethyl{3-[2-{1-(1,3-benzo[d]dioxol-4-ylmethyl)piperidin-4-yl]ethyl}-6-cyclopropylmethoxybenz[d]isoxazol-7-yl}methylamine

The title compound (110 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (107 mg) and 2,3-(methylenedioxy)benzaldehyde (90.1 mg) according to Example 21-(3).
¹H-NMR (400 MHz, CDCl₃) δ (ppm): 0.35-0.39 (m, 2H), 0.62-0.66 (m, 2H), 1.22-1.33 (m, 4H), 1.72-1.79 (m, 4H), 1.96-2.01 (m, 2H), 2.33 (s, 6H), 2.90-2.94 (m, 4H), 3.50 (s, 2H), 3.81 (s, 2H), 3.93 (d, J=6.8 Hz, 2H), 5.93 (s, 2H), 6.71-6.83 (m, 3H), 6.87 (d, J=8.8 Hz, 1H), 7.42 (d, J=8.8 Hz, 1H).

### (2) N,N-Dimethyl{3-{2-[1-(1,3-benzo[d]dioxol-4-ylmethyl)piperidin-4-yl]ethyl}-6-cyclopropylmethoxybenz[d]isoxazol-7-yl}methylamine dihydrochloride

The title compound (135 mg) was obtained by synthesis from N,N-dimethyl{3-{2-[1-(1,3-benzo[d]dioxol-4-ylmethyl)piperidin-4-yl]ethyl}-6-cyclopropylmethoxybenz[d]isoxazol-7-yl}methylamine (128 mg) according to Example 21-(4).
¹H-NMR (400 MHz, CD₃OD) δ (ppm): 0.42-0. 45 (m, 2H), 0.67-0.72 (m, 2H), 1.33-1.45 (m, 1H), 1.50-1.60 (m, 2H), 1.65-1.75 (m, 1H), 1.81-1.86 (m, 2H), 2.07-2.11 (m, 2H), 2.96 (s, 6H), 3.01-3.07 (m, 4H), 3.55-3.59 (m, 2H), 4.12 (d, J=6.8 Hz, 2H), 4.28 (s, 2H), 4.63 (s, 2H), 6.05 (s, 2H), 6.93-7.00 (m, 3H), 7.23 (d, J=8.8 Hz, 1H), 7.91 (d, J=8.8 Hz, 1H).
ESI-MS m/z: 246 [M+2H]²⁺, 492 [M+H]⁺.

### Example 13

### 1-{4-{2-[6-Cyclopropylmethoxy-7-(N,N-dimethylaminomethyl)benz[d]isoxazol-3-yl]ethyl}piperidinomethyl}cyclohexanecarbonitrile dihydrochloride

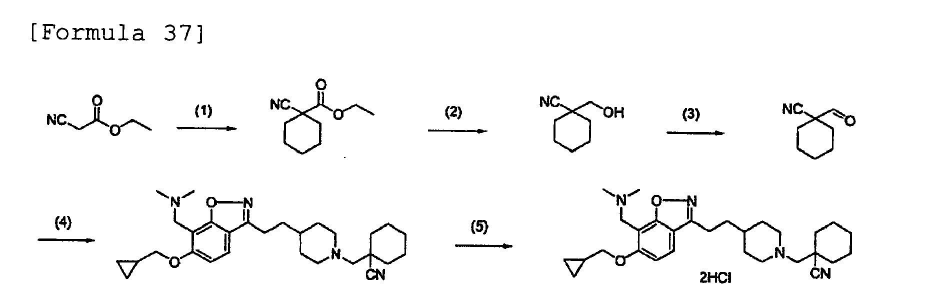

### (1)(2) 1-Hydroxycyclohexanecarbonitrile

Ethyl cyanoacetate (3.39 g) and 1,5-dibromopentane (4.09 mL) were dissolved in acetone (120 mL). Potassium carbonate (12.4 g) was added and the mixture was stirred with heating under reflux for three hours. After cooling to room temperature, water was added to the reaction mixture, followed by extraction with ethyl acetate. The organic layer was washed with a saturated sodium chloride solution, dried over magnesium sulfate and filtered. Then, the solvent was evaporated under reduced pressure to obtain ethyl 1-cyanocyclohexanecarboxylate (5.4 g). The ethyl 1-cyanocyclohexanecarboxylate (5.4 g) was dissolved in tetrahydrofuran (80 mL) and water (10 mL) without further purification. Sodium borohydride (5.64 g) was added and the mixture was stirred at 50°C for 30 minutes. After cooling to room temperature, 5 M hydrochloric acid was added to the reaction mixture, followed by extraction with ethyl acetate. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (3.65 g).
¹H-NMR (400 MHz, CDCl₃) δ (ppm): 1.07-1.31 (m, 4H), 1.56-1.68 (m, 2H), 1.73-1.81 (m, 2H), 1.94 (t, J=4.4 Hz, 1H), 2.00-2.04 (m, 2H), 3.61 (d, J=4.4 Hz, 2H).

### (3)(4) 1-{4-{2-[6-Cyclopropylmethoxy-7-(N,N-dimethylaminomethyl)benz[d]isoxazol-3-yl]ethyl}piperidinomethyl}cyclohexanecarbonitrile

1-Hydroxycyclohexanecarbonitrile (418 mg) and triethylamine (2.51 mL) were dissolved in dimethyl sulfoxide (20 mL). A sulfur trioxide-pyridine complex (1.43 g) was added and the mixture was stirred at room temperature for one hour. 1 M hydrochloric acid was added to the reaction mixture, followed by extraction with ethyl acetate. The organic layer was washed with a saturated sodium chloride solution, dried over magnesium sulfate and filtered. Then, the solvent was evaporated under reduced pressure to obtain 1-formylcyclohexanecarbonitrile (378 mg). The title compound (122 mg) was obtained by synthesis from 1-formylcyclohexanecarbonitrile (82.3 mg) and N,N-dimethyl{6-cyclopropylmethoxy-3-[2-(piperidin-4-yl)ethyl]benz[d]isoxazol-7-yl}methylamine obtained in Preparation Example 3-(3) (107 mg) according to Example 21-(3).
¹H-NMR (400 MHz, CDCl₃) δ (ppm): 0.35-0.39 (m, 2H), 0.62-0.67 (m, 2H), 1.13-1.34 (m, 10H), 1.58-1.79 (m, 4H), 1.97-2.03 (m, 4H), 2.21-2.27 (m, 2H), 2.33 (s, 6H), 2.42 (s, 2H), 2. 91-2. 95 (m, 4H), 3.82 (s, 2H), 3.93 (d, J=6.4 Hz, 2H), 6.88 (d, J=8.8 Hz, 1H), 7.43 (d, J=8.8 Hz, 1H).

### (5) 1-{4-{2-[6-Cyclopropylmethoxy-7-(N,N-dimethylaminomethyl)benz[d]isoxazol-3-yl]ethyl}piperidinomethyl}cyclohexanecarbonitrile dihydrochloride

The title compound (132 mg) was obtained by synthesis from 1-{4-{2-[6-cyclopropylmethoxy-7-(N,N-dimethylaminomethyl)benz[d]isoxazol-3-yl]ethyl}piperidinomethyl}cyclohexanecarbonitrile (122 mg) according to Example 21- (4) .
¹H-NMR (400 MHz, CD₃OD) δ (ppm): 0.42-0.46 (m, 2H), 0.68-0.73 (m, 2H), 1.31-1.99 (m, 16H), 2.09-2.13 (m, 2H), 2.97 (s, 6H), 3.05-3.09 (m, 2H), 3.17-3.21 (m, 2H), 3. 52 (s, 2H), 3.77-3. 81 (m, 2H), 4.12 (d, J=7.2 Hz, 2H), 4.64 (s, 2H), 7.24 (d, J=8.8 Hz, 1H), 7.92 (d, J=8.8 Hz, 1H).
ESI-MS m/z: 479 [M+H]⁺.

### Example 14

### N,N-Dimethyl{6-cyclopropylmethoxy-3-{2-[1-(1-methoxymethylcyclohexylmethyl)piperidin-4-yl]ethyl}benz[d]isoxazol-7-yl}methylamine dihydrochloride

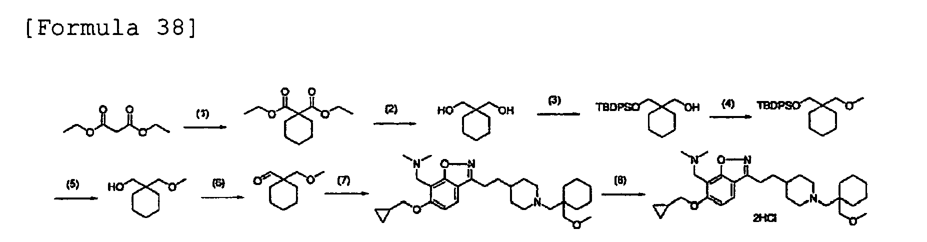

### (1) Diethyl cyclohexane-1,1-dicarboxylate

Diethyl malonate (8.0 g) and 1,5-dibromopentane (4.09 mL) was dissolved in ethanol (100 mL). Sodium ethoxide (21% solution in ethanol, 56 mL) was added and the mixture was stirred at room temperature for 17 hours. Water was added to the reaction mixture, followed by extraction with ethyl acetate. The organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (7.6 g).
¹H-NMR (400 MHz, CDCl₃) δ (ppm): 1.24 (t, J=7.2 Hz, 6H), 1.40-1.46 (m, 2H), 1.49-1.56 (m, 4H), 1.95-1.99 (m, 4H), 4.17 (q, J=7.2 Hz, 4H).

### (2) (3) [1-(tert-Butyldiphenylsilyloxymethyl)cyclohexyl]methanol

Diethyl cyclohexane-1,1-dicarboxylate (2.74 g) was added to a suspension of lithium aluminum hydride (1.02 g) in tetrahydrofuran (10 mL) under ice-cooling. Then, the mixture was heated to room temperature and stirred for one hour. A saturated ammonium chloride solution and 5 M hydrochloric acid were sequentially added to the reaction mixture, followed by extraction with ethyl acetate. The organic layer was washed with a saturated sodium chloride solution, dried over magnesium sulfate and filtered. Then, the solvent was evaporated under reduced pressure to obtain (1-hydroxymethylcyclohexyl)methanol (1.5 g). (1-Hydroxymethylcyclohexyl)methanol (1.5 g) and imidazole (545 mg) were dissolved in N,N-dimethylformamide (20 mL). tert-Butylchlorodiphenylsilane (2.08 mL) was added to the solution at -15°C over 10 minutes, and the mixture was stirred at the same temperature for 35 minutes. Methanol and water were sequentially added to the reaction mixture, followed by extraction with ethyl acetate. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (2.25 g).
¹H-NMR (400 MHz, CDCl₃) δ (ppm): 1.05 (s, 9H), 1.10-1.46 (m, 10H), 2.72 (t, J=5.6 Hz, 1H), 3.55 (s, 2H), 3.64 (d, J=5.6 Hz, 1H), 7.33-7.46 (m, 6H), 7.64-7.67 (m, 4H).

### (4) tert-Butyl-(1-methoxymethylcyclohexylmethoxy)diphenylsilane

[1-(tert-Butyldiphenylsilyloxymethyl)cyclohexyl]methanol (459 mg) was dissolved in tetrahydrofuran (8 mL). 60% sodium hydride (62.4 mg) was added under ice-cooling in a nitrogen atmosphere. The mixture was heated to room temperature and stirred for 30 minutes. Iodomethane (150 µL) was added to the reaction mixture, followed by stirring with heating under reflux for 15 hours. A saturated ammonium chloride solution and 1 M hydrochloric acid were sequantially added to the reaction mixture, followed by extraction with ethyl acetate. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (400 mg). ¹H-NMR (400 MHz, CDCl₃) δ (ppm): 1.05 (s, 9H), 1.34-1.42 (m, 10H), 3.33 (s, 3H), 3.34 (s, 2H), 3.51 (s, 2H), 7.33-7.42 (m, 6H), 7.64-7.68 (m, 4H).

### (5) (1-Methoxymethylcyclohexyl)methanol

tert-Butyl-(1-methoxymethylcyclohexylmethoxy)diphenylsilane (317 mg) was dissolved in tetrahydrofuran (3 mL). Tetrabutylammonium fluoride (1.0 M solution in tetrahydrofuran, 1.6 mL) was added and the mixture was stirred at room temperature for 18 hours. Water was added to the reaction mixture, followed by extraction with ethyl acetate. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (100 mg). ¹H-NMR (400 MHz, CDCl₃) δ (ppm): 1.22-1.43 (m, 10H), 2.83 (t, J=5.6 Hz, 1H), 3.33 (s, 3H), 3.34 (s, 2H), 3.51 (d, J=5.6 Hz, 2H).

### (6)(7) N,N-Dimethyl{6-cyclopropylmethoxy-3-{2-[1-(1-methoxymethylcyclohexylmethyl)piperidin-4-yl]ethyl}benz[d]isoxazol-7-yl}methylamine

1-Methoxymethylcyclohexanecarbaldehyde (90 mg) was obtained by synthesis from (1-methoxymethylcyclohexyl)methanol (94.9 mg) according to Example 13-(3). The title compound (21 mg) was obtained by synthesis from 1-methoxymethylcyclohexanecarbaldehyde (90 mg) and N,N-dimethyl{6-cyclopropylmethoxy-3-[2-(piperidin-4-yl)ethyl]benz[d]isoxazol-7-yl}methylamine obtained in Preparation Example 3-(3) (107 mg) according to Example 21-(3).
¹H-NMR (400 MHz, CDCl₃) δ (ppm): 0.35-0.39 (m, 2H), 0.62-0.66 (m, 2H), 1.20-1.45 (m, 10H), 1.65-1.76 (m, 8H), 2.15-2.22 (m, 4H), 2.33 (s, 6H), 2.71-2.74 (m, 2H), 2.90-2.94 (m, 2H), 3.20 (s, 2H), 3.28 (s, 3H), 3. 82 (s, 2H), 3.93 (d, J=6. 8, 2H), 6.88 (d, J=8. Hz, 1H), 7.43 (d, J=8.8 Hz, 1H).

(8) N,N-Dimethyl{6-cyclopropylmethoxy-3-{2-[1-(1-methoxymethylcyclohexylmethyl)piperidin-4-yl]ethyl}benz[d]isoxazol-7-yl}methylamine dihydrochloride

The title compound (22 mg) was obtained by synthesis from N,N-dimethyl{6-cyclopropylmethoxy-3-{2-[1-(1-methoxymethylcyclohexylmethyl)piperidin-4-yl]ethyl}benz[d]isoxazol-7-yl}methylamine (21 mg) according to Example 21-(4).
¹H-NMR (400 MHz, CD₃OD) δ (ppm): 0.42-0.45 (m, 2H), 0.68-0.71 (m, 2H), 0.88-1.73 (m, 13H), 1.82-2.08 (m, 5H), 2.96 (s, 6H), 3.04-3.21 (m, 4H), 3.26 (s, 2H), 3.44 (s, 2H), 3.53-3.63 (m, 5H), 4.11 (d, J=7.2, 2H), 4.63 (s, 2H), 7.23 (d, J=8.8 Hz, 1H), 7.92 (d, J=8.8 Hz, 1H).
ESI-MS m/z: 498 [M+H]⁺.

### Example 15

### 1-{4-{2-[6-Cyclopropylmethoxy-7-(N,N-dimethylaminomethyl)benz[d]isoxazol-3-yl]ethyl}piperidinomethyl}-N-methylcyclohexanecarboxamide dihydrochloride

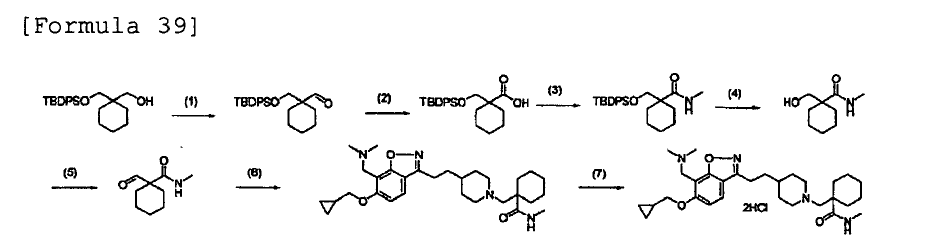

### (1) 1-(tert-Butyldiphenylsilanyloxymethyl)cyclohexanecarbaldehyde

[1-(tert-Butyldiphenylsilyloxymethyl)cyclohexyl]methanol (1.92 g) and 4-methylmorpholine-4-oxide (878 mg) were dissolved in dichloromethane (17 mL).
Tetrapropylammonium perruthenate (87.8 mg) was added at room temperature and the mixture was stirred for 1.5 hours. The reaction mixture was filtered through celite and the filtrate was concentrated under reduced pressure. The residue was purified by silica gel column chromatography (heptane-ethyl acetate) to obtain the title compound (1.73 g).
¹H-NMR (400 MHz, CDCl₃) δ (ppm): 1.03 (s, 9H), 1.18-1.38 (m, 6H), 1.49-1.55 (m, 2H), 1.93-1.97 (m, 2H), 3.60 (s, 2H), 7.35-7.44 (m, 6H), 7.59-7.62 (m, 4H), 9.61 (s, 1H).

(2) 1- (tert-Butyldiphenylsilanyloxymethyl)cyclohexanecarboxylic acid

1-(tert-Butyldiphenylsilanyloxymethyl)cyclohexanecarbaldehyde (1.52 g), 2-methyl-2-butene (212 mL) and sodium dihydrogenphosphate (480 mg) were dissolved in acetone (8 mL) and water (4 mL). Sodium chlorite (1.09 g) was added at room temperature and the mixture was stirred for one hour. A saturated ammonium chloride solution was added to the reaction mixture, followed by extraction with chloroform. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (1.43 g).
¹H-NMR (400 MHz, CDCl₃) δ (ppm): 1.02 (s, 9H), 1.18-1.34 (m, 4H), 1.39-1.57 (m, 4H), 2.04-2.11 (m, 2H), 3.65 (s, 2H), 7.34-7.44 (m, 6H), 7.61-7.65 (m, 4H).

### (3) 1-(tert-Butyldiphenylsilanyloxymethyl)-N-methylcyclohexanecarboxamide

1- (tert-Butyldiphenylsilanyloxymethyl)cyclohexanecarboxylic acid (595 mg), methylamine (2 M solution in tetrahydrofuran, 1.5 mL) and 1-hydroxybenzotriazole (243 mg) were dissolved in dichloromethane (10 mL). 1-Ethyl-3-(3-dimethylaminopropyl)carbodiimide hydrochloride (431 mg) was added at room temperature and the mixture was stirred for 5.5 hours. A saturated ammonium chloride solution was added to the reaction mixture, followed by extraction with ethyl acetate. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (598 mg).
¹H-NMR (400 MHz, CDCl₃) δ (ppm): 1.06 (s, 9H), 1.25-1.35 (m, 3H), 1.40-1.50 (m, 5H), 1.93-1.97 (m, 2H), 2.83 (d, J=4.8 Hz, 3H), 3.57 (s, 2H), 6.36 (q, J=4.8 Hz, 1H), 7.36-7.45 (m, 6H), 7.59-7.62 (m, 4H).

### (4) 1-Hydroxymethyl-N-methylcyclohexanecarboxamide

The title compound (110 mg) was obtained by synthesis from 1-(tert-butyldiphenylsilanyloxymethyl)-N-methylcyclohexanecarboxamide obtained in Example 45-(1) (492 mg) according to Example 14-(5). δ (ppm): 1.42-1.56 (m, 8H), 1.78-1.84 (m, 2H), 2.66 (t, J=4.0 Hz, 1H), 2.83 (d, J=4.8 Hz, 3H), 3.61 (d, J=4.0 Hz, 2H), 6.07 (q, J=4.8 Hz, 1H).

### (5)(6) 1-{4-{2-[6-Cyclopropylmethoxy-7-(N,N-dimethylaminomethyl)benz[d]isoxazol-3-yl]ethyl}piperidinomethyl}-N-methylcyclohexanecarboxamide

1-Formyl-N-methylcyclohexanecarboxamide (83 mg) was obtained by synthesis from 1-hydroxymethyl-N-methylcyclohexanecarboxamide (85.6 mg) according to Example 13-(3). The title compound (73 mg) was obtained by synthesis from 1-formyl-N-methylcyclohexanecarboxamide (76.2 mg) and N,N-dimethyl{6-cyclopropylmethoxy-3-[2-(piperidin-4-yl)ethyl]benz[d]isoxazol-7-yl}methylamine obtained in Preparation Example 3-(3) (107 mg) according to Example 21-(3) .
¹H-NMR (400 MHz, CDCl₃) δ (ppm): 0.35-0.39 (m, 2H), 0.62-0.67 (m, 2H), 1.15-1.32 (m, 8H), 1.43-1.54 (m, 4H), 1.72-1.78 (m, 4H), 1.87-1.96 (m, 2H), 2.18-2.24 (m, 2H), 2.30 (s, 2H), 2.33 (s, 6H), 2.71-2.75 (m, 2H), 2.78 (d, J=4.8 Hz, 3H), 2.91-2.95 (m, 2H), 3.81 (s, 2H), 3.93 (d, J=6.8, 2H), 6.89 (d, J=8.8 Hz, 1H), 7.36 (q, J=4.8 Hz, 1H), 7.43 (d, J=8.8 Hz, 1H).

### (7) 1-{4-{2-[6-Cyclopropylmethoxy-7-(N,N-dimethylaminomethyl)benz[d]isoxazol-3-yl]ethyl}piperidinomethyl}-N-methylcyclohexanecarboxamide dihydrochloride

The title compound (77 mg) was obtained by synthesis from 1-{4-{2-[6-cyclopropylmethoxy-7-(N,N-dimethylaminomethyl)benz[d]isoxazol-3-yl]ethyl}piperidinomethyl}-N-methylcyclohexanecarboxamide (73 mg) according to Example 21-(4).
¹H-NMR (400 MHz, CD₃OD) δ (ppm): 0.42-0.45 (m, 2H), 0.67-0.72 (m, 2H), 1.40-2.12 (m, 18H), 2.79 (s, 3H), 2.96 (s, 6H), 3.00-3.12 (m, 4H), 3.30 (s, 2H), 3.50-3.59 (m, 2H), 4.11 (d, J=7.2 Hz, 2H), 4.63 (s, 2H), 7.23 (d, J=8.8 Hz, 1H), 7.91 (d, J=8.8 Hz, 1H).
ESI-MS m/z: 511 [M+H]⁺, 534 [M+Na]⁺.

### Example 16

### 4-{7-(N,N-Dimethylaminomethyl)-3-{2-[1-(1-hydroxymethylcyclopropylmethyl)piperidin-4-yl]ethyl}benz[d]isoxazol-6-yloxymethyl}benzonitrile dihydrochloride

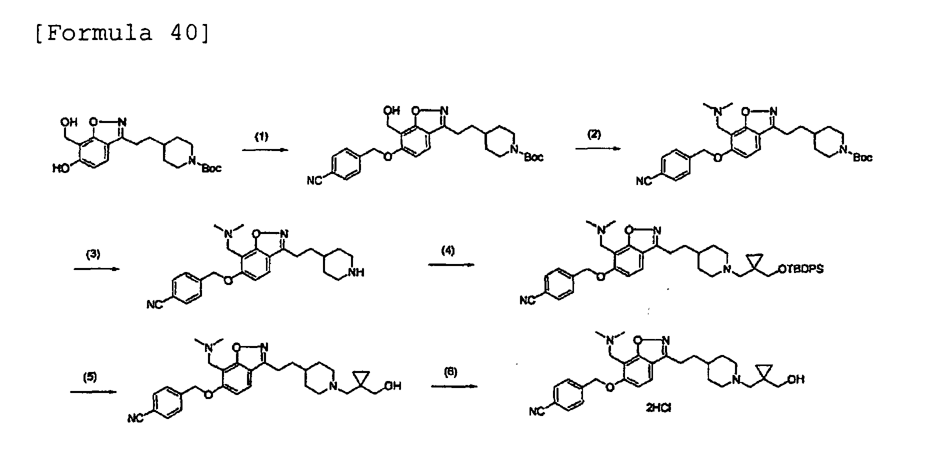

### (1) tert-Butyl 4-[2-(4-cyanobenzyloxy-7-hydroxymethylbenz[d]isoxazol-3-yl)ethyl]piperidine-1-carboxylate

tert-Butyl 4-[2-(6-hydroxy-7-hydroxymethylbenz[d]isoxazol-3-yl)ethyl]piperidine-1-carboxylate obtained in Preparation Example 2-(6) (752 mg) was dissolved in N,N-dimethylformamide (5 mL). 4-Cyanobenzyl bromide (588 mg) and potassium carbonate (553 mg) were added and the mixture was stirred at 50°C for two hours. Water was added to the reaction mixture, followed by extraction with ethyl acetate. The organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (980 mg).
¹H-NMR (400 MHz, CDCl₃) δ (ppm): 1.11-1.20 (m, 2H), 1.41 (s, 9H), 1.50-1.55(m, 1H), 1.72-1.80 (m, 4H), 2.25 (t, J=6.8 Hz, 1H), 2.62-2.72 (m, 2H), 2.94-2.99 (m, 2H), 4.06-4.16 (m, 2H), 5.06 (d, J=6.8 Hz, 2H), 5.29 (s, 2H), 6.92 (d, J=8.8 Hz, 1H), 7.48 (d, J=8.8 Hz, 1H), 7.55 (d, J=8.0 Hz, 2H), 7.69 (d, J=8.0 Hz, 2H).

### (2) tert-Butyl 4-{2-[7-(N,N-dimethylaminomethyl)-6-(4-cyanobenzyloxy)benz[d]isoxazol-3-yl]ethyl}piperidine-1-carboxylate

The title compound (598 mg) was obtained by synthesis from tert-butyl 4-[2-(4-cyanobenzyloxy-7-hydroxymethylbenz[d]isoxazol-3-yl)ethyl]piperidine-1-carboxylate (885 mg) according to Example 21-(1). ¹H-NMR (400 MHz, CDCl₃) δ (ppm): 1.11-1.21 (m, 2H), 1.45 (s, 9H), 1.50-1.55 (m, 1H), 1.72-1.81 (m, 4H), 2.33 (s, 6H), 2.64-2.64 (m, 2H), 2.93-2.98 (m, 2H), 3.82 (s, 2H), 4.06-4.16 (m, 2H), 5.26 (s, 2H), 6.93 (d, J=8.8 Hz, 1H), 7.46 (d, J=8.8 Hz, 1H), 7.58 (d, J=8.0 Hz, 2H), 7.69 (d, J=8.0 Hz, 2H).

### (3) 4-{7-(N,N-Dimethylaminomethyl)-3-[2-(piperidin-4-yl)ethyl]benz[d]isoxazol-6-yloxymethyl}benzonitrile

The title compound (401 mg) was obtained by synthesis from tert-butyl 4-{2-[7-(N,N-dimethylaminomethyl)-6-(4-cyanobenzyloxy)benz[d]isoxazol-3-yl]ethyl}piperidine-1-carboxylate (520 mg) according to Example 21-(2).
¹H-NMR (400 MHz, CDCl₃) δ (ppm): 1.18-1.27 (m, 2H), 1.42-1.32 (m, 1H), 1.71-1.80 (m, 4H), 2.33 (s, 6H), 2.57-2.63 (m, 2H), 2.93-2.97 (m, 2H), 3.09-3.12 (m, 2H), 3.82 (s, 2H), 5.26 (s, 2H), 6.93 (d, J=8.4 Hz, 1H), 7.47 (d, J=8.4 Hz, 1H), 7.58 (d, J=8.0 Hz, 2H), 7.69 (d, J=8.0 Hz, 2H).

### (4) 4-{3-{2-{1-[1-(tert-Butyldiphenylsilanyloxymethyl)cyclopropylmethyl]piperid in-4-yl}ethyl}-7-(N,N-dimethylaminomethyl)benz[d]isoxazol-6-yloxymethyl}benzonitrile

The title compound (154 mg) was obtained by synthesis from 4-[7-dimethylaminomethyl-3-(2-piperidin-4-yl)ethyl]benz[d]isoxazol-6-yloxymethyl]benzonitrile (104 mg) and 1-(tert-butyldiphenylsilanyloxymethyl)cyclopropanecarbaldehyde obtained in Example 45-(2) (127 mg) according to Example 21-(3).
¹H-NMR (400 MHz, CDCl₃) δ (ppm): 0.24-0.26 (m, 2H), 0.45-0.47 (m, 2H), 1.03 (s, 9H), 1.25-1.30 (m, 3H), 1.69-1.84 (m, 6H), 2.31 (s, 2H), 2.33 (s, 6H), 2.92-2.97 (m, 4H), 3.59 (s, 2H), 3.82 (s, 2H), 5.27 (s, 2H), 6.93 (d, J=8.8 Hz, 1H), 7.34-7.41 (m, 6H), 7.47 (d, J=8.8 Hz, 1H), 7.58 (d, J=8.0 Hz, 2H), 7.68-7.71 (m, 6H).

### (5) 4-{7-(N,N-Dimethylaminomethyl)-3-{2-[1-(1-hydroxymethylcyclopropylmethyl)piperidin-4-yl]ethyl}benz[d]isoxazol-6-yloxymethyl}benzonitrile

4-{3-{2-{1-[1-(tert-Butyldiphenylsilanyloxymethyl)cyclopropylmethyl]piperid in-4-yl}ethyl}-7-(N,N-dimethylaminomethyl)benz[d]isoxazol-6-yloxymethyl}benzonitrile (111 mg) was dissolved in tetrahydrofuran (3 mL). Tetrabutylammonium fluoride (1.0 M solution in tetrahydrofuran, 225 µL) was added and the mixture was stirred at room temperature for 15 hours. Water was added to the reaction mixture, followed by extraction with chloroform. Then, the organic layer was washed with a saturated sodium chloride solution, dried over magnesium sulfate and filtered. Then, the solvent was evaporated under reduced pressure. The residue was purified by NH silica gel column chromatography (heptane-ethyl acetate) to obtain the title compound (24 mg).
¹H-NMR (400 MHz, CDCl₃) δ (ppm): 0.33-0.35 (m, 2H), 0.48-0.50 (m, 2H), 1.24-1.35 (m, 3H), 1.73-1.80 (m, 4H), 1.90-2.04 (m, 2H), 2.33 (s, 6H), 2.45 (s, 2H), 2.92-2.96 (m, 2H), 3.17-3.21 (m, 2H), 3.53 (s, 2H), 3.82 (s, 2H), 5.26 (s, 2H), 6.93 (d, J=8.8 Hz, 1H), 7.46 (d, J=8.8 Hz, 1H), 7.57-7.59 (m, 2H), 7.68-7.71 (m, 2H).

### (6) 4-{7-(N,N-Dimethylaminomethyl)-3-{2-[1-(1-hydroxymethylcyclopropylmethyl)piperidin-4-yl]ethyl}benz[d]isoxazol-6-yloxymethyl}benzonitrile dihydrochloride

The title compound (26 mg) was obtained by synthesis from 4-{7-(N,N-dimethylaminomethyl)-3-{2-[1-(1-hydroxymethylcyclopropylmethyl)piperidin-4-yl]ethyl}benz[d]isoxazol-6-yloxymethyl}benzonitrile (24 mg) according to Example 21-(4).
¹H-NMR (400 MHz, CD₃OD) δ (ppm): 0.65-0.73 (m, 4H), 1.49-1.59 (m, 2H), 1.66-1.76 (m, 1H), 1.83-1.89 (m, 2H), 2.08-2.13 (m, 2H), 2.87-2.94 (m, 2H), 2.93 (s, 6H), 3.05-3.10 (m, 2H), 3.18 (s, 2H), 3.56 (s, 2H), 3.81-3.85 (m, 2H), 4.67 (s, 2H), 5.49 (s, 2H), 7.30 (d, J=8.8 Hz, 1H), 7.71 (d, J=8.0 Hz, 2H), 7.79 (d, J=8.0 Hz, 2H), 7.93 (d, J=8.8 Hz, 1H).
ESI-MS m/z: 252 [M+2H]²⁺, 503 [M+H]⁺.

### Example 17

### cis-2-{4-{2-[6-Cyclopropylmethoxy-7-(N,N-dimethylaminomethyl)benz[d]isoxazol-3-yl]ethyl}piperidinomethyl}cyclohexanol dihydrochloride

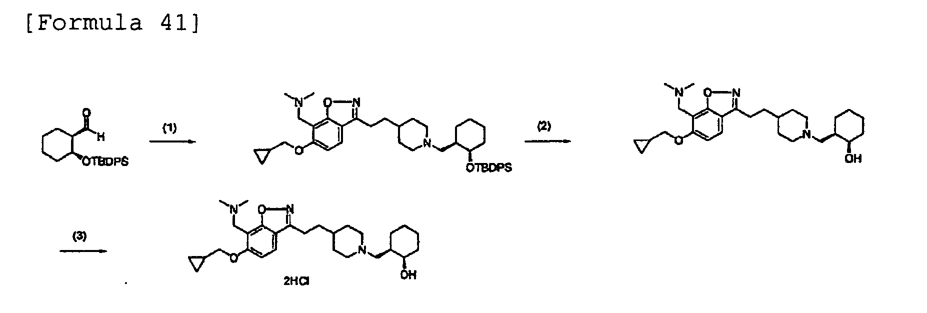

### (1) N,N-Dimethyl{3-{2-{1-[cis-2-tert-butyldiphenylsilanyloxy]cyclohexylmethyl]piperidin-4-yl}ethyl}-6-cyclopropylmethoxybenz[d]isoxazol-7-yl}methylamine

The title compound (212 mg) was obtained by synthesis from (cis-2-tert-butyldiphenylsilanyloxycyclohexyl)carbaldehyde obtained in Example 32-(2) (165 mg) and N,N-dimethyl{6-cyclopropylmethoxy-3-[2-(piperidin-4-yl)ethyl]benz[d]isoxazol-7-yl}methylamine obtained in Preparation Example 3-(3) (107 mg) according to Example 21-(3).
¹H-NMR (400 MHz, CDCl₃) δ (ppm): 0.35-0.39 (m, 2H), 0.61-0.67 (m, 2H), 1.08 (s, 9H), 1.11-1.77 (m, 19H), 2.05 (d, J=6.0, 12.0 Hz, 1H), 2.26 (dd, J=6.0, 12.0 Hz, 1H), 2.33 (s, 6H), 2.53-2.59 (m, 1H), 2.72-2.75 (m, 1H), 2.89-2.94 (m, 2H), 3.82 (s, 2H), 3.93 (d, J=6.8 Hz, 2H), 4.04-4,05 (m, 1H), 6.88 (d, J=8.8 Hz, 1H), 7.31-7.42 (m, 6H), 7.43 (d, J=8.8 Hz, 1H), 7.66-7.71 (m, 4H).
ESI-MS m/z: 708 [M]⁺.

### (2) cis-2-{4-{2-[6-Cyclopropylmethoxy-7-(N,N-dimethylaminomethyl)benz[d]isoxazol-3-yl]ethyl}piperidinomethyl}cyclohexanol

N,N-Dimethyl{3-{2-{1-[cis-2-tert-butyldiphenylsilanyloxy]cyclohexylmethyl}piperidin-4-yl}ethyl}-6-cyclopropylmethoxybenz[d]isoxazol-7-yl}methylamine (141 mg) was dissolved in tetrahydrofuran (3 mL). Hydrofluoric acid (70% solution in pyridine, 1 mL) was added and the mixture was stirred at room temperature for 3.5 hours. Water was added to the reaction mixture, followed by extraction with chloroform. Then, the organic layer was washed with a saturated sodium chloride solution, dried over magnesium sulfate and filtered. Then, the solvent was evaporated under reduced pressure. The residue was purified by NH silica gel column chromatography (heptane-ethyl acetate) to obtain the title compound (70 mg).
¹H-NMR (400 MHz, CDCl₃) δ (ppm): 0.35-0.39 (m, 2H), 0.62-0.67 (m, 2H), 1.26-1.86 (m, 18H), 2.04-2.10 (m, 1H), 2.17-2.25 (m, 2H), 2.33 (s, 6H), 2.85-2.92 (m, 3H), 3.15-3.17 (m, 1H), 3.78-3.81 (m, 3H), 3.93 (d, J=6.8 Hz, 2H), 6.88 (d, J=8.8 Hz, 1H), 7.42 (d, J=8.8 Hz, 1H).

### (3) cis-2-{4-{2-[6-Cyclopropylmethoxy-7-(N,N-dimethylaminomethyl)benz[d]isoxazol-3-yl]ethyl}piperidinomethyl}cyclohexanol dihydrochloride

The title compound (76 mg) was obtained by synthesis from cis-2-{4-{2-[6-cyclopropylmethoxy-7-(N,N-dimethylaminomethyl)benz[d]isoxazol-3-yl]ethyl}piperidinomethyl}cyclohexanol (70 mg) according to Example 21-(4).
¹H-NMR (400 MHz, CD₃OD) δ (ppm): 0.43-0.45 (m, 2H), 0.68-0.71 (m, 2H), 1.40-2.13 (m, 17H), 2.93-3.00 (m, 2H), 2.96 (s, 6H), 3.05-3.09 (m, 2H), 3.26-3.34 (m, 2H), 3.60-3.68 (m, 2H), 3.93-3.94 (m, 1H), 4.11 (d, J=7.2 Hz, 2H), 4.63 (s, 2H), 7.23 (d, J=8.8 Hz, 1H) 7.92 (d, J=8.8 Hz, 1H).
ESI-MS m/z: 235 [M+2H]²⁺, 470 [M+H]⁺.

### Example 18

### 4-{7-(N,N-Dimethylaminomethyl)-3-{2-[1-(furan-2-ylmethyl)piperidin-4-yl]ethyl}benz[d]isoxazol-6-yloxymethyl}benzonitrile dihydrochloride

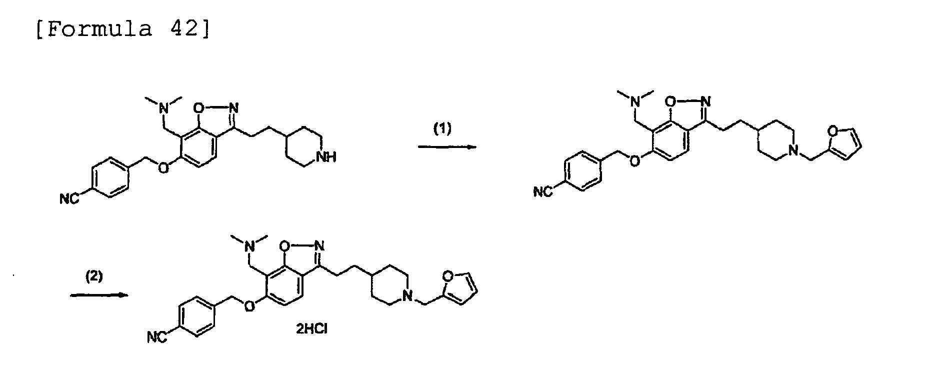

### (1) 4-{7-(N,N-Dimethylaminomethyl)-3-{2-[1-(furan-2-ylmethyl)piperidin-4-yl]ethyl}benz[d]isoxazol-6-yloxymethyl}benzonitrile

The title compound (56 mg) was obtained by synthesis from 4-7-(N,N-dimethylaminomethyl)-3-(2-piperidin-4-yl)ethyl]benz[d]isoxazol-6-yloxymethyl]benzonitrile obtained in Example 16-(3) (83.2 mg) and 2-furancarbaldehyde (33.1 µL) according to Example 21-(3).
¹H-NMR (400 MHz, CDCl₃) δ (ppm): 1.24-1.35 (m, 3H), 1.67-1.75 (m, 4H), 1.94-2.04 (m, 2H), 2.32 (s, 6H), 2.88-2.95 (m, 4H), 3.51 (s, 2H), 3.81 (s, 2H), 5.26 (s, 2H), 6.16-6.18 (m, 1H), 6.29-6.30 (m, 1H), 6.92 (d, J=8.8 Hz, 1H), 7.35-7.36 (m, 1H), 7.45 (d, J=8.8 Hz, 1H), 7.58 (d, J=8.4 Hz, 2H), 7.68 (d, J=8.4 Hz, 2H).

### (2) 4-{7-(N,N-Dimethylaminomethyl)-3-{2-[1-(furan-2-ylmethyl)piperidin-4-yl]ethyl}benz[d]isoxazol-6-yloxymethyl}benzonitrile dihydrochloride

The title compound (62 mg) was obtained by synthesis from 4-{7-(N,N-dimethylaminomethyl)-3-{2-[1-(furan-2-ylmethyl)piperidin-4-yl]ethyl}benz[d]isoxazol-6-yloxymethyl}benzonitrile (56 mg) according to Example 21-(4).
¹H-NMR (400 MHz, CD₃OD) δ (ppm): 1.48-1.58 (m, 2H), 1.65-1.75 (m, 1H), 1.81-1.87 (m, 2H), 2.08-2.11 (m, 2H), 2.93 (s, 6H), 2.96-3.08 (m, 4H), 3.49-3.52 (m, 2H), 4.39 (s, 2H), 4.67 (s, 2H), 5.49 (s, 2H), 6.52-6.54 (m, 1H), 6.72-6.74 (m, 1H), 7.29 (d, J=8.8 Hz, 1H), 7.66-7.68 (m, 1H), 7.73 (d, J=8.0 Hz, 2H), 7.79 (d, J=8.0 Hz, 2H), 7.93 (d, J=8.8 Hz, 1H).
ESI-MS m/z: 250 [M+2H]²⁺, 499 [M+H]⁺.

### Example 19

### 4-{7-(N,N-Dimethylaminomethyl)-3-{2-[1-(pyridin-2-ylmethyl)piperidin-4-yl]ethyl}benz[d]isoxazol-6-yloxymethyl}benzonitrile trihydrochloride

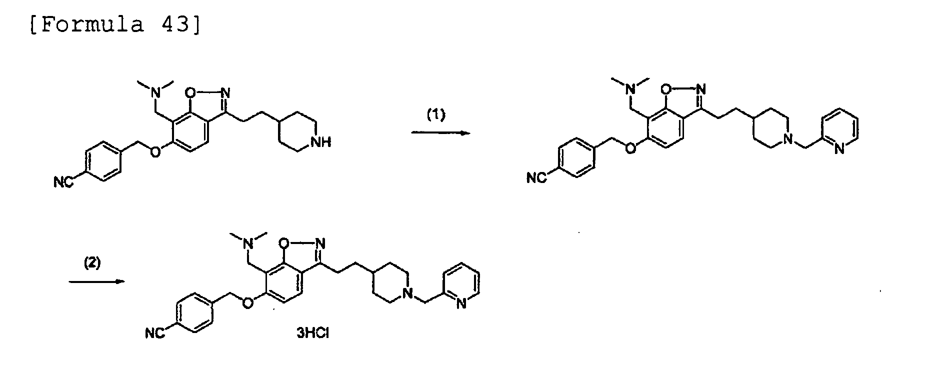

### (1) 4-{7-(N,N-Dimethylaminomethyl)-3-{2-[1-(pyridin-2-ylmethyl)piperidin-4-yl]ethyl}benz[d]isoxazol-6-yloxymethyl}benzonitrile

The title compound (56 mg) was obtained by synthesis from 4-{7-(N,N-dimethylaminomethyl)-3-[2-(piperidin-4-yl)ethyl]benz[d]isoxazol-6-yloxymethyl}benzonitrile obtained in Example 16-(3) (83.2 mg) and 2-pyridinecarbaldehyde (37.9 mg) according to Example 21-(3).
¹H-NMR (400 MHz, CDCl₃) δ (ppm): 1.26-1.37 (m, 3H), 1.68-1.76 (m, 4H), 2.02-2.08 (m, 2H), 2.33 (s, 6H), 2.89-2.96 (m, 4H), 3.63 (s, 2H), 3.82 (s, 2H), 5.26 (s, 2H), 6.92 (d, J=8.8 Hz, 1H), 7.12-7.16 (m, 1H), 7.38 (d, J=7.6 Hz, 1H), 7.46 (d, J=8.8 Hz, 1H), 7.57-7.59 (m, 2H), 7.61-7.65 (m, 1H), 7.63-7.70 (m, 2H), 8.54 (1H, J=8.0 Hz, 1H).

### (2) 4-{7-(N,N-Dimethylaminomethyl)-3-{2-[1-(pyridin-2-ylmethyl)piperidin-4-yl]ethyl}benz[d]isoxazol-6-yloxymethyl}benzonitrile trihydrochloride

The title compound (65 mg) was obtained by synthesis from 4-{7-(N,N-dimethylaminomethyl)-3-{2-[1-(pyridin-2-ylmethyl)piperidin-4-yl]ethyl}benz[d]isoxazol-6-yloxymethyl}benzonitrile (56 mg) according to Example 21-(4).
¹H-NMR (400 MHz, CD₃OD) δ (ppm): 1.59-1.71 (m, 2H), 1.72-1.83 (m, 1H), 1.85-1.93 (m, 2H), 2.05-2.12 (m, 2H), 2.94 (s, 6H), 3.06-3.17 (m, 4H), 3.56-3.59 (m, 2H), 4.48 (s, 2H), 4.68 (s, 2H), 5.49 (s, 2H), 7.30 (d, J=8.8 Hz, 1H), 7.46-7.54 (m, 2H), 7.71 (d, J=8.4 Hz, 2H), 7.80 (d, J=8.4 Hz, 2H), 7.90-7.95 (m, 2H), 8.68-8.75 (m, 1H).
ESI-MS m/z: 255 [M+2H]²⁺, 510 [M+H]⁺.

### Example 20

### trans-2-{4-{2-[6-Cyclopropylmethoxy-7-(N,N-dimethylaminomethyl)benz[d]isoxazol-3-yl]ethyl}piperidinomethyl}cyclohexanol dihydrochloride

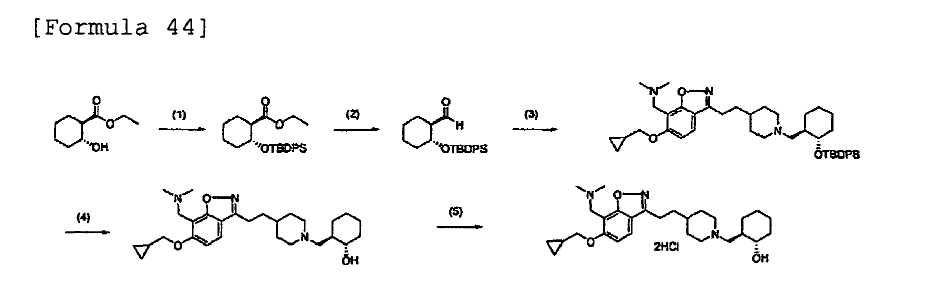

### (1) Ethyl (trans-2-tert-butyldiphenylsilanyloxycyclohexyl)acetate

The title compound (361 mg) was obtained by synthesis from ethyl (trans-2-hydroxycyclohexyl)acetate (172 mg) according to Example 32-(1).
¹H-NMR (400 MHz, CDCl₃) δ (ppm): 1.04 (s, 9H), 1.06-1.30 (m, 3H), 1.22 (t, J=7.2 Hz, 3H), 1.33-1.44 (m, 1H), 1.53-1.65 (m, 3H), 1.84-1.88 (m, 1H), 2.42-2.50 (m, 1H), 3.88-3.95 (m, 1H), 4.00-4.13 (q, J=7.2 Hz, 2H), 7.33-7.42 (m, 6H), 7.65-7.69 (m, 4H).

### (2)(3) N,N-Dimethyl{3-{2-{1-[trans-2-(tert-butyldiphenylsilanyloxy)cyclohexylmethyl]piperidin-4-yl}ethyl}-6-cyclopropylmethoxybenz[d]isoxazol-7-yl}methylamine

(trans-2-tert-Butyldiphenylsilanyloxycyclohexyl)carbaldehyde (210 mg) was obtained by synthesis from ethyl (trans-2-tert-butyldiphenylsilanyloxycyclohexyl)acetate (287 mg) according to Example 32-(2). The title compound (212 mg) was obtained by synthesis from (trans-2-tert-butyldiphenylsilanyloxycyclohexyl)carbaldehyde (110 mg) and N,N-dimethyl{6-cyclopropylmethoxy-3-[2-(piperidin-4-yl)ethyl]benz[d]isoxazol-7-yl}methylamine obtained in Preparation Example 3-(3) (107 mg) according to Example 21-(3).
¹H-NMR (400 MHz, CDCl₃) δ (ppm): 0.35-0.39 (m, 2H), 0.62-0.67 (m, 2H), 1.05 (s, 9H), 1.19-1.76 (m, 17H), 1.83-1.92 (m, 2H), 1.95-1.99 (m, 1H), 2.33 (s, 6H), 2.55-2.62 (m, 2H), 2.78-2.82 (m, 1H), 2.90-2.94 (m, 2H), 3.42-3.46 (m, 1H), 3.82 (s, 2H), 3.94 (d, J=6.8 Hz, 2H), 6.88 (d, J=8.8 Hz, 1H), 7.32-7.42 (m, 6H), 7.43 (d, J=8.8 Hz, 1H), 7.65-7.69 (m, 4H).

### (4) trans-2-{4-{2-[6-Cyclopropylmethoxy-7-(N,N-dimethylaminomethyl)benz[d]isoxazol-3-yl]ethyl}piperidinomethyl}cyclohexanol

The title compound (80 mg) was obtained by synthesis from N,N-dimethyl{3-{2-{1-[trans-2-(tert-butyldiphenylsilanyloxy)cyclohexylmethyl]piperidin-4-yl}ethyl}-6-cyclopropylmethoxybenz[d]isoxazol-7-yl}methylamine (141 mg) according to Example 17-(2).
¹H-NMR (400 MHz, CDCl₃) δ (ppm): 0.35-0.39 (m, 2H), 0.63-0.67 (m, 2H), 1.22-1.86 (m, 17H), 1.89-1.93 (m, 1H), 2.07-2.13 (m, 1H), 2.27-2.38 (m, 2H), 2.33 (s, 6H), 2.84-2.94 (m, 3H), 3.19-3.22 (m, 1H), 3.34-3.39 (m, 1H), 3.81 (s, 2H), 3.93 (d, J=6.8 Hz, 2H), 6.88 (d, J=8.8 Hz, 1H), 7.42 (d, J=8.8 Hz, 1H).

### (5) trans-2-{4-{2-[6-Cyclopropylmethoxy-7-(N,N-dimethylaminomethyl)benz[d]isoxazol-3-yl]ethyl}piperidinomethyl}cyclohexanol dihydrochloride

The title compound (83 mg) was obtained by synthesis from trans-2-{4-{2-[6-cyclopropylmethoxy-7-(N,N-dimethylaminomethyl)benz[d]isoxazol-3-yl]ethyl}piperidinomethyl}cyclohexanol (80 mg) according to Example 21-(4).
¹H-NMR (400 MHz, CD₃OD) δ (ppm): 0.43-0.45 (m, 2H), 0.68-0.72 (m, 2H), 1.02-1.99 (m, 15H), 2.10-2.14 (m, 2H), 2.84-2.91 (m, 1H), 2.96 (s, 6H), 3.02-3.09 (m, 2H), 3.17-3.23 (m, 1H), 3.31-3.47 (m, 3H), 3.57-3.61 (m, 1H), 3.83-3.87 (m, 1H), 4.11 (d, J=7.2 Hz, 2H), 4.63 (s, 2H), 7.23 (d, J=8.8 Hz, 1H) 7.92 (d, J=8.8 Hz, 1H).
ESI-MS m/z: 235 [M+2H]²⁺, 470 [M+H]⁺.

### Example 21

### N,N-Dimethyl{3-[2-(1-benzyl-4-yl)ethyl]-6-(4-fluorobenzyloxy)benz[d]isoxazol-7-yl}methylamine dihydrochloride

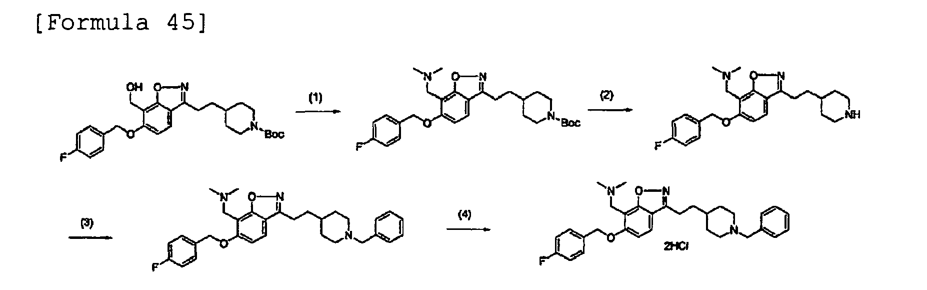

### (1) tert-Butyl 4-{2-[7-(N,N-dimethylaminomethyl)-6-(4-fluorobenzyloxy)benz[d]isoxazol-3-yl]ethyl}piperidine-1-carboxylate

tert-Butyl 4-[2-(4-fluorobenzyloxy-7-hydroxymethylbenz[d]isoxazol-3-yl)ethyl]piperidine-1-carboxylate obtained in Example 22-(4) (339 mg) and triethylamine (140 µL) were dissolved in tetrahydrofuran (5 mL). Methanesulfonyl chloride (81.3 µL) was added under ice-cooling and then the mixture was heated to room temperature and stirred for one hour. Dimethylamine (2.0 M solution in tetrahydrofuran, 1.75 mL) was added to the reaction mixture, followed by stirring for 17.5 hours. A saturated ammonium chloride solution was added to the reaction mixture, and the solvent was evaporated under reduced pressure. Water was added to the residue, followed by extraction with chloroform. Then, the organic layer was washed with a saturated sodium chloride solution, dried over magnesium sulfate and filtered. Then, the solvent was evaporated under reduced pressure. The residue was purified by NH silica gel column chromatography (heptane-ethyl acetate) to obtain the title compound (358 mg).
¹H-NMR (400 MHz, CDCl₃) δ (ppm): 1.11-1.18 (m, 2H), 1.45 (s, 9H), 1.45-1.53 (m, 1H), 1.73-1.81 (m, 4H), 2.31 (s, 6H), 2.64-2.71 (m, 2H), 2.93-2.98 (m, 2H), 3.80 (s, 2H), 4.06-4.16 (m, 2H), 5.16 (s, 2H), 6.97 (d, J=8.4 Hz, 1H), 7.05-7.09 (m, 2H), 7.40-7.47 (m, 3H).

### (2) N,N-Dimethyl{6-(4-fluorobenzyloxy)-3-[2-(piperidin-4-yl)ethyl]benz[d]isoxazol-7-yl}methylamine

tert-Butyl 4-{2-[7-(N,N-dimethylaminomethyl)-6-(4-fluorobenzyloxy)benz[d]isoxazol-3-yl]ethyl}piperidine-1-carboxylate (358 mg) was dissolved in methanol (3 mL). Hydrochloric acid (4 M solution in ethyl acetate, 875 µL) was added and the mixture was stirred at room temperature for 19.5 hours. The reaction mixture was neutralized with a 5 M sodium hydroxide solution, followed by extraction with chloroform. Then, the organic layer was washed with a saturated sodium chloride solution, dried over magnesium sulfate and filtered. Then, the solvent was evaporated under reduced pressure and the residue was dried under reduced pressure to obtain the title compound (280 mg).
¹H-NMR (400 MHz, CDCl₃) δ (ppm): 1.23-1.32 (m, 2H), 1.46-1.56 (m, 1H), 1.69-1.90 (m, 4H), 2.32 (s, 6H), 2.60-2.66 (m, 2H), 2.93-2.97 (m, 2H), 3.13-3.17 (m, 2H), 3.80 (s, 2H), 5.16 (s, 2H), 6.97 (d, J=8.4 Hz, 1H), 7.05-7.09 (m, 2H), 7.40-7.47 (m, 3H).

### (3) N,N-Dimethyl{3-[2-(1-benzylpiperidin-4-yl)ethyl]-6-(4-fluorobenzyloxy)benz[d]isoxazol-7-yl}methylamine

N,N-Dimethyl[6-(4-fluorobenzyloxy)-3-(2-piperidin-4-yl)ethyl]benz[d]isoxazol-7-yl]methylamine (61.7 mg) and benzaldehyde (22.9 µL) were dissolved in tetrahydrofuran (3 mL). Sodium triacetoxyborohydride (47.7 mg) was added and the mixture was stirred at room temperature for 17 hours. A saturated ammonium chloride solution and water were sequentially added to the reaction mixture, followed by extraction with chloroform. Then, the organic layer was sequentially washed with a saturated sodium chloride solution, dried over magnesium sulfate and filtered. Then, the solvent was evaporated under reduced pressure. The residue was purified by NH silica gel chromatography (heptane-ethyl acetate) to obtain the title compound (53 mg).
¹H-NMR (400 MHz, CDCl₃) δ (ppm): 1.25-1.34 (m, 3H), 1.75-1.77 (m, 4H), 1.88-1.97 (m, 2H), 2.31 (s, 6H), 2.86-2.95 (m, 4H), 3.48 (s, 2H), 3.79 (s, 2H), 5.16 (s, 2H), 6.96 (d, J=8.8 Hz, 1H), 7.07 (m, 2H), 7.21-7.24 (m, 1H), 7.29-7.30 (m, 4H), 7.39-7.43 (m, 2H), 7.44 (d, J=8.8 Hz, 1H).

### (4) N,N-Dimethyl{3-[2-(1-benzylpiperidin-4-yl)ethyl]-6-(4-fluorobenzyloxy)benz[d]isoxazol-7-yl}methylamine dihydrochloride

Hydrochloric acid (4 M solution of ethyl acetate, 80 µL) was added to a mixed solution of N,N-dimethyl{3-[2-(1-benzylpiperidin-4-yl)ethyl]-6-(4-fluorobenzyloxy)benz[d]isoxazol-7-yl}methylamine (53 mg) in tetrahydrofuran and methanol at room temperature. After stirring several times, the solvent was evaporated under reduced pressure and the residue was dried under reduced pressure to obtain the title compound (56 mg).
¹H-NMR (400 MHz, CD₃OD) δ (ppm): 1.55-1.62 (m, 2H), 1.66-1.74 (m, 1H), 1.80-1.86 (m, 2H), 2.04-2.08 (m, 2H), 2.31 (s, 6H), 2.98-3.07 (m, 4H), 3.46-3.50 (m, 2H), 4.31 (s, 2H), 4.61 (s, 2H), 5.37 (s, 2H), 7.13-7.18 (m, 2H), 7.34 (d, J=8.8 Hz, 1H), 7.47-7.50 (m, 3H), 7.55-7.61 (m, 4H), 7.93 (d, J=8.8 Hz, 1H).
ESI-MS m/z: 251 [M+2H]²⁺, 502 [M+H]⁺.

### Example 22

### N,N-Dimethyl{3-{2-[1-(1,3-dioxolan-2-ylmethyl)piperidin-4-yl]ethyl}-6-(4-fluorobenzyloxy)benz[d]isoxazol-7-yl}methylamine dihydrobromide

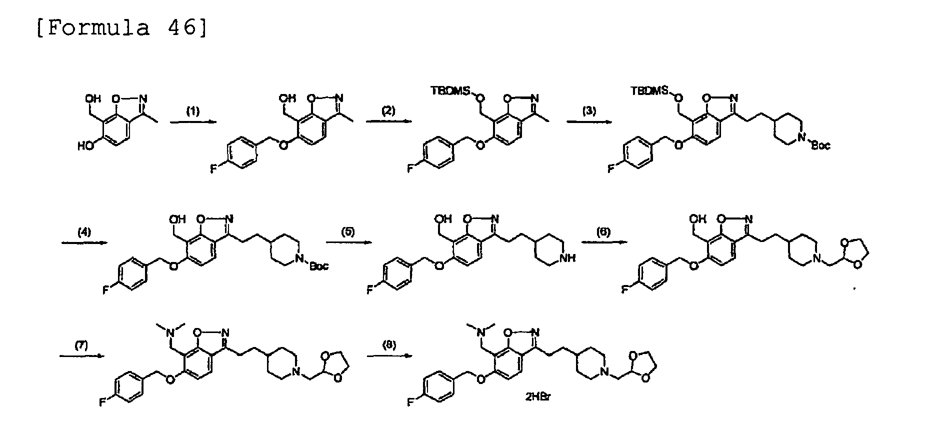

### (1) Synthesis of 6-(4-fluorobenzyloxy)-7-hydroxymethyl-3-methylbenz[d]isoxazole

7-Hydroxymethyl-3-methylbenz[d]isoxazol-6-ol obtained in Preparation Example 2-(3) (2.02 g) was dissolved in acetone (50 mL). Potassium carbonate (2.32 g) and 4-fluorobenzyl bromide (1.63 mL) were added to the solution, and then the mixture was stirred at 60 to 70°C for four hours. The reaction mixture was cooled to room temperature and filtered through celite. The filtrate was concentrated under reduced pressure to obtain a residue. Heptane/tert-butyl methyl ether (3/1) was added to the residue. After solidification, the solid was suspended and collected by filtration. The solid collected by filtration was washed with heptane/t-butyl methyl ether (3/1) and dried under reduced pressure to obtain the title compound (3.05 g).
¹H-NMR (400 MHz, CDCl₃) δ:2.30-2.40 (m, 1H), 2.54 (s, 3H), 5.03 (s, 2H), 5.20 (s, 2H), 7.01 (d, J=8.8 Hz, 1H), 7.06-7.14 (m, 2H), 7.38-7.46 (m, 2H), 7.49 (d, J=8.8 Hz, 1H).

### (2) Synthesis of 6-(4-fluorobenzyloxy)-7-(tert-butyldimethylsilanyloxymethyl)-3-methylbenz[d]isoxazole

6-(4-Fluorobenzyloxy)-7-hydroxymethyl-3-methylbenz[d]isoxazole (2.63 g) and imidazole (3.11 g) were dissolved in N,N-dimethylformamide (30 mL) at room temperature. tert-Butyldimethylchlorosilane (1.58 g) was added to the solution and then the mixture was stirred at room temperature for 19 hours. Water and ethyl acetate were added to the reaction mixture and the organic layer was separated. The organic layer was washed with water and a saturated sodium chloride solution (the turbidity was removed by addition of a small amount of methanol). The organic layer was dried over anhydrous magnesium sulfate. The drying agent was removed by filtration and then the filtrate was concentrated under reduced pressure to obtain a residue. Heptane was added to the residue, followed by cooling. Then, the residue was solidified by rubbing the wall. The solid was collected by filtration and washed with heptane. The filtrate was concentrated and the generated solid was suspended by adding heptane and collected by filtration. The solids collected by filtration were collectively dried under reduced pressure to obtain the title compound (3.26 g).
¹H-NMR (400 MHz, CDCl₃) δ:0.08 (s, 6H), 0.89 (s, 9H), 2.53 (s, 3H), 5.02 (s, 2H), 5.17 (s, 2H), 6.96 (d, J=8.4 Hz, 1H), 7.04-7.11 (m, 2H), 7.40-7.46 (m, 2H), 7.45 (d, J=8.4 Hz, 1H).

### (3) Synthesis of tert-butyl 4-{2-[7-(tert-butyldimethylsilanyloxymethyl)-6-(4-fluorobenzyloxy)benz[d]isoxazol-3-yl]ethyl}piperidine-1-carboxylate

6-(4-Fluorobenzyloxy)-7-(tert-butyldimethylsilanyloxymethyl)-3-methylbenz[d]isoxazole (2 g) and tert-butyl 4-iodomethylpiperidine-1-carboxylate (1.86 g) were dissolved in tetrahydrofuran (20 mL) in a nitrogen atmosphere, and the solution was cooled to an internal temperature of -76°C. A separately prepared 1 M solution of lithium diisopropylamide in tetrahydrofuran (5.98 mL) was added dropwise to the solution over nine minutes. After completion of dropwise addition, the reaction mixture was further stirred at -66 to -62°C for one hour. A saturated ammonium chloride solution was added to the reaction mixture, and then the mixture was heated to room temperature. Ethyl acetate was added and the organic layer was separated. The organic layer was washed with a saturated sodium chloride solution and dried over anhydrous magnesium sulfate. The drying agent was removed by filtration and the filtrate was concentrated under reduced pressure to obtain the title compound (3.58 g) as a crude product. The crude product was used for the next reaction without further purification.

### (Preparation of solution of lithium diisopropylamide in tetrahydrofuran)

Diisopropylamine (2 mL) was added to tetrahydrofuran (6.35 mL) in a nitrogen atmosphere, and the solution was cooled to -20°C. n-Butyllithium (2.55 M solution in hexane, 5.85 mL) was added to the solution at the same temperature. The solution was subsequently stirred at -5°C for 20 minutes and then cooled to -70°C again to prepare a 1 M solution of lithium diisopropylamide in tetrahydrofuran.

### (4) Synthesis of tert-butyl 4-{2-[6-(4-fluorobenzyloxy)-7-hydroxymethylbenz[d]isoxazol-3-yl]ethyl}piperidine-1-carboxylate

tert-Butyl 4-{2-[7-(tert-butyldimethylsilanyloxymethyl)-6-(4-fluorobenzyloxy)benz[d]isoxazol-3-yl]ethyl}piperidine-1-carboxylate (crude product obtained in Example 22-(3)) (3.58 g) was dissolved in tetrahydrofuran (15 mL). A 1 M solution of tetrabutylammonium fluoride in tetrahydrofuran (5.98 mL) was added dropwise to the solution. The reaction mixture was stirred at room temperature for 19 hours. A saturated ammonium chloride solution and ethyl acetate were added to the reaction mixture, and the organic layer was separated. The organic layer was washed with a saturated sodium chloride solution and dried over anhydrous magnesium sulfate. The drying agent was removed by filtration and the filtrate was concentrated under reduced pressure to obtain a residue. Ethyl acetate was added to the residue, followed by solidification. The solid was collected by filtration and washed with ethyl acetate to obtain the title compound. On the other hand, the filtrate was concentrated under reduced pressure to obtain a residue. The residue was subjected to silica gel column chromatography (heptane-ethyl acetate) and then solidified from tert-butyl methyl ether. The solid was collected by filtration to obtain the total compound. In total, 1.80 g of the title compound was obtained.
¹H-NMR (400 MHz, CDCl₃) δ (ppm): 1.08-1.24 (m, 2H), 1.36-1.64 (m, 1H), 1.46 (s, 9H), 1.68-1.82 (m, 4H), 2.34 (br s, 1H), 2.60-2.74 (m, 2H), 2.92-3.01 (m, 2H), 4.00-4.20 (m, 2H), 5.03 (s, 2H), 5.20 (s, 2H), 7.00 (d, J=8.6 Hz, 1H), 7.06-7.14 (m, 2H), 7.38-7.45 (m, 2H), 7.49 (d, J=8.6 Hz, 1H).

### (5) {6-(4-Fluorobenzyloxy)-3-[2-(piperidin-4-yl)ethyl]benz[d]isoxazol-7-yl}methanol

tert-Butyl 4-{2-[6-(4-fluorobenzyloxy)-7-hydroxymethylbenz[d]isoxazol-3-yl]ethyl}piperidine-1-carboxylate (483 mg) was dissolved in methanol (2.5 mL) and tetrahydrofuran (2.5 mL). Methanesulfonic acid (325 µL) was added to the solution, and the mixture was stirred at room temperature for 24 hours. The reaction mixture was concentrated under reduced pressure. A 5 M sodium hydroxide solution (10 mL) was added to the residue, followed by extraction with chloroform (30 mL) twice. The organic layers were washed with a saturated sodium chloride solution and dried over magnesium sulfate. The drying agent was removed by filtration and then the solvent was evaporated under reduced pressure. Ethyl acetate (5 mL) was added to the residue to obtain a suspension. The suspension was filtered and the solid was collected by filtration. The solid collected by filtration was washed with ethyl acetate (5 mL) and dried under reduced pressure to obtain the title compound (364 mg).
¹H-NMR (400 MHz, DMSO-d₆) δ (ppm): 1.00-1.11 (m, 2H), 1.29-1.40 (m, 1H), 1.61-1.70 (m, 4H), 2.38-2.44 (m, 2H), 2.91-2.96 (m, 4H), 4.73 (s, 2H), 5.04 (br s, 1H), 5.26 (s, 2H), 7.20 (d, J=8.8 Hz, 1H), 7.19-7.27 (m, 2H), 7.53-7.58 (m, 2H), 7.73 (d, J=8.8 Hz, 1H).

### (6) (7) N,N-Dimethyl{3-{2-[1-(1,3-dioxolan-2-ylmethyl)piperidin-4-yl]ethyl}-6-(4-fluorobenzyloxy)benz[d]isoxazol-7-yl}methylamine

N,N-Dimethylformamide (4 mL) was added to {6-(4-fluorobenzyloxy)-3-[2-(piperidin-4-yl)ethyl]benz[d]isoxazol-7-yl}methanol (345 mg), followed by dissolution by heating to 80°C. Sodium bicarbonate (227 mg) and 2-bromomethyl-1,3-dioxolane (140 µL) were added to the solution, and the mixture was stirred at 110°C for 3.5 hours. The reaction mixture was cooled to room temperature and then water (20 mL) was added, followed by extraction with ethyl acetate (50 mL). The organic layer was washed with a saturated sodium chloride solution and dried over magnesium sulfate. The drying agent was removed by filtration and then the solvent was evaporated under reduced pressure. {3-{2-[1-(1,3-Dioxolan-2-ylmethyl)piperidin-4-yl]ethyl}-6-(4-fluorobenzyloxy)benz[d]isoxazol-7-yl}methanol (420 mg) was obtained as a residue. The {3-{2-[1-(1,3-dioxolan-2-ylmethyl)piperidin-4-yl]ethyl}-6-(4-fluorobenzyloxy)benz[d]isoxazol-7-yl}methanol (420 mg) was dissolved in tetrahydrofuran (4 mL) without further purification. Triethylamine (251 µL) and methanesulfonyl chloride (104 µL) were sequentially added to the solution under ice-cooling, and the mixture was stirred at the same temperature for one hour. Dimethylamine (2.0 M solution in tetrahydrofuran, 2.25 mL) was added to the reaction mixture under ice-cooling, followed by stirring for 30 minutes. The reaction mixture was heated to room temperature and further stirred for 30 minutes. A saturated ammonium chloride solution was added to the reaction mixture, and the solvent was evaporated under reduced pressure. Water (20 mL) was added to the residue, followed by extraction with chloroform (30 mL) twice. The organic layers were washed with a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (289 mg).
¹H-NMR (400 MHz, CDCl₃) δ (ppm): 1.30-1.44 (m, 3H), 1.67-4.85 (m, 4H), 2.00-2.12 (m, 2H), 2.32 (s, 6H), 2.57 (d, J=4.6 Hz, 2H), 2.90-3.05 (m, 4H), 3.82 (s, 2H), 3.82-3.90 (m, 2H), 3.93-4.01 (m, 2H), 5.01 (t, J=4.6 Hz, 1H) , 5.17 (s, 2H), 6.98 (d, J=8.8 Hz, 1H) , 7.05-7.11 (m, 2H), 7.41-7.47 (m, 2H), 7.47 (d, J=8.8 Hz, 1H).

### (8) N,N-Dimethyl{3-{2-[1-(1,3-dioxolan-2-ylmethyl)piperidin-4-yl]ethyl}-6-(4-fluorobenzyloxy)benz[d]isoxazol-7-yl}methylamine dihydrobromide

N,N-Dimethyl{3-{2-[1-(1,3-dioxolan-2-ylmethyl)piperidin-4-yl]ethyl}-6-(4-fluorobenzyloxy)benz[d]isoxazol-7-yl}methylamine (104 mg) was dissolved in 2-propanol (2 mL) and tetrahydrofuran (3 mL). 48% hydrobromic acid (49.7 µL) was added at room temperature and the mixture was stirred for two hours. The precipitated crystals were collected by filtration, washed with an organic solvent (2-propanol:tetrahydrofuran = 2:3, 1 mL) and then dried at 80°C for two days to obtain crystals of the title compound (125 mg).
¹H-NMR (400 MHz, DMSO-d₆) δ (ppm): 1.40-1.99 (m, 7H), 2.80 (s, 6H), 2.91-3.06 (m, 5H), 3.38-3.62 (m, 3H), 3.85-3.93 (m, 2H), 3.95-4.03 (m, 2H), 4.52 (s, 2H), 5.22-5.30 (m, 1H), 5.36 (s, 2H), 7.23-7.30 (m, 2H), 7.37 (d, J=8.8 Hz, 1H), 7.59-7.65 (m, 2H), 8.00 (d, J=8.8 Hz, 1H) , 9.43 (br s, 1H) , 9.58 (br s, 1H).
ESI-MS m/z: 250 [M+2H ]²⁺, 498 [M+H]⁺.

### Example 23

### 5-{3-[2-(1-Benzylpiperidin-4-yl)ethyl]-7-(N,N-dimethylaminomethyl)benz[d]isoxazol-6-yloxymethyl}thiophene-2-carbonitrile dihydrochloride

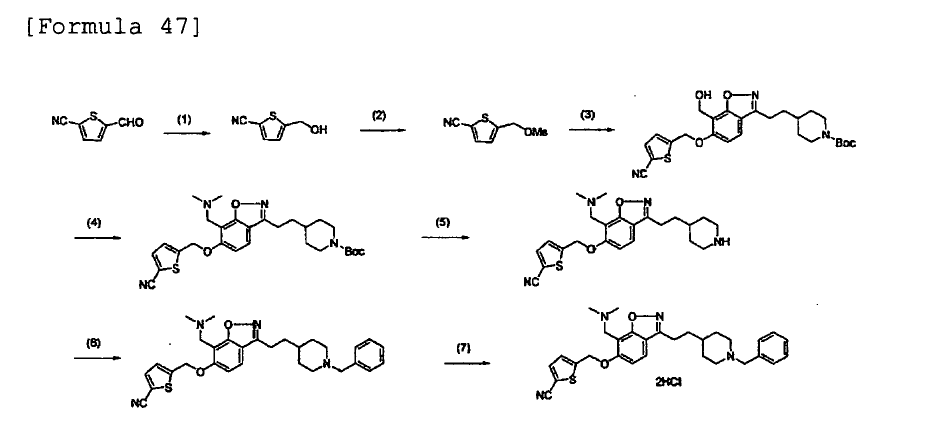

### (1) 5-Hydroxymethylthiophene-2-carbonitrile

5-Formylthiophene-2-carbonitrile obtained in Example 155-(1) (274 mg) was dissolved in tetrahydrofuran (3 mL). Sodium borohydride (151 mg) was added at room temperature and the mixture was stirred for 1.5 hours. 1 M hydrochloric acid and water were sequentially added to the reaction mixture, followed by extraction with ethyl acetate. The organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (277 mg).
¹H-NMR (400 MHz, CDCl₃) δ (ppm): 1.20 (t, J=5.6 Hz, 1H) , 4.88 (d, J=5.6 Hz, 2H), 6.98 (d, J=3.6 Hz, 1H), 7.50 (d, J=3.6 Hz, 1H).

### (2)(3) tert-Butyl 4-{2-[6-(5-cyanothiophen-2-ylmethoxy)-7-hydroxymethylbenz[d]isoxazol-3-yl]ethyl}piperidine-1-carboxylate

5-Hydroxymethylthiophene-2-carbonitrile (278 mg) and triethylamine (558 µL) were dissolved in tetrahydrofuran (5 mL). Methanesulfonyl chloride (232 µL) was added under ice-cooling and the mixture was stirred for 30 minutes. Then, the mixture was heated to room temperature and further stirred for 17 hours. A saturated ammonium chloride solution was added to the reaction mixture, and the solvent was evaporated under reduced pressure. Water was added to the residue, followed by extraction with ethyl acetate. Then, the organic layer was washed with a saturated sodium chloride solution, dried over magnesium sulfate and filtered. Then, the solvent was evaporated under reduced pressure and the residue was dried under reduced pressure to obtain 5-methanesulfonyloxymethylthiophene-2-carbonitrile (435 mg). tert-Butyl 4-[2-(6-hydroxy-7-hydroxymethylbenz[d]isoxazol-3-yl)ethyl]piperidine-1-carboxylate obtained in Preparation Example 2-(6) (301 mg) and 5-methanesulfonyloxymethylthiophene-2-carbonitrile (261 mg) were dissolved in N,N-dimethylformamide (5 mL). Potassium carbonate (166 mg) was added and the mixture was stirred at 60°C for two hours. Water was added to the reaction mixture, followed by extraction with ethyl acetate. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (381 mg).
¹H-NMR (400 MHz, CDCl₃) δ (ppm): 1.12-1.24 (m, 2H), 1.45 (s, 9H), 1.45-1.53 (m, 1H), 1.73-1.81 (m, 4H), 2.64-2.70 (m, 2H), 2.91-3.02 (m, 3H), 4.07-4.14 (m, 2H), 5.39 (d, J=1.2 Hz, 2H), 5.40 (s, 2H), 6.97 (d, J=8.4 Hz, 1H), 7.12 (d, J=3.6 Hz, 1H), 7.51 (d, J=8.4 Hz, 1H), 7.55 (d, J=3.6 Hz, 1H).

### (4) tert-Butyl 4-{2-[6-(5-cyanothiophen-2-ylmethoxy)-7-(N,N-dimethylaminomethyl)benz[d]isoxazol-3-yl]ethyl}piperidine-1-carboxylate

The title compound (110 mg) was obtained by synthesis from tert-butyl 4-{2-[6-(5-cyanothiophen-2-ylmethoxy)-7-hydroxymethylbenz[d]isoxazol-3-yl]ethyl}piperidine-1-carboxylate (381 mg) according to Example 21-(1).
¹H-NMR (400 MHz, CDCl₃) δ (ppm): 1.11-1.20 (m, 2H), 1.45 (s, 9H), 1.48-1.55 (m, 1H), 1.73-1.81 (m, 4H), 2.33 (s, 6H), 2.64-2.71 (m, 2H), 2.94-2.99 (m, 2H), 3.78 (s, 2H), 4.06-4.16 (m, 2H), 5.38 (s, 2H), 6.96 (d, J=8.4 Hz, 1H) , 7.09 (d, J=3.6 Hz, 1H), 7.48 (d, J=8.4 Hz, 1H), 7.54 (d, J=3. 6 Hz, 1H).

### (5) 5-{7-(N,N-Dimethylaminomethyl)-3-[2-(piperidin-4-yl)ethyl]benz[d]isoxazol-6-yloxymethyl}thiophene-2-carbonitrile

The title compound (36 mg) was obtained by synthesis from tert-butyl 4-{2-[6-(5-cyanothiophen-2-ylmethoxy)-7-(N,N-dimethylaminomethyl)benz[d]isoxazol-3-yl]ethyl}piperidine-1-carboxylate (105 mg) according to Example 21-(2).
¹H-NMR (400 MHz, CDCl₃) δ (ppm): 1.12-1.22 (m, 2H), 1.45-1.51 (m, 1H), 1.72-1.80 (m, 4H), 2.33 (s, 6H), 2.56-2.62 (m, 2H), 2.94-2.99 (m, 2H), 3.06-3.09 (m, 2H), 3.79 (s, 2H), 5.39 (s, 2H), 6.97 (d, J=8.8 Hz, 1H), 7.09 (d, J=3.6 Hz, 1H), 7.51 (d, J=8.4 Hz, 1H), 7.56 (d, J=3.6 Hz, 1H).

### (6) 5-{3-[2-(1-Benzylpiperidin-4-yl)ethyl]-7-(N,N-dimethylaminomethyl)benz[d]isoxazol-6-yloxymethyl}thiophene-2-carbonitrile

The title compound (22 mg) was obtained by synthesis from 5-{7-(N,N-dimethylaminomethyl)-3-[2-(piperidin-4-yl)ethyl]benz[d]isoxazol-6-yloxymethyl}thiophene-2-carbonitrile (33.9 mg) and benzaldehyde (12.2 µL) according to Example 21-(3).
¹H-NMR (400 MHz, CDCl₃) δ (ppm): 1.26-1.35 (m, 3H), 1.73-1.80 (m, 4H), 1.91-1.97 (m, 2H), 2.33 (s, 6H), 2.87-2.97 (m, 4H), 3.49 (s, 2H), 3.79 (s, 2H), 5.39 (s, 2H), 6.96 (d, J=8.4 Hz, 1H) , 7.08 (dt, J=3.6, 0.8 Hz, 1H), 7.21-7.27 (m, 1H), 7.29-7.31 (m, 4H), 7.49 (d, J=8.4 Hz, 1H) , 7.55 (d, J=3.6 Hz, 1H).

### (7) 5-{3-[2-(1-Benzylpiperidin-4-yl)ethyl]-7-(N,N-dimethylaminomethyl)benz[d]isoxazol-6-yloxymethyl}thiophene-2-carbonitrile dihydrochloride

The title compound (25 mg) was obtained by synthesis from 5-{3-[2-(1-benzylpiperidin-4-yl)ethyl]-7-(N,N-dimethylaminomethyl)benz[d]isoxazol-6-yloxymethyl}thiophene-2-carbonitrile (22 mg) according to Example 21-(4).
¹H-NMR (400 MHz, CD₃OD) δ (ppm): 1.51-1.60 (m, 2H), 1.67-1.77-1.80 (m, 1H), 1.81-1.90 (m, 2H), 2.04-2.09 (m, 2H), 2.94 (s, 6H), 2.98-3.12 (m, 4H), 3.48-3.52 (m, 2H), 4.31 (s, 2H), 4.63 (s, 2H), 5.65 (s, 2H), 7.36-7.39 (m, 2H), 7.47-7.51 (m, 3H), 7.52-7.57 (m, 2H), 7.72-7.74 (m, 1H), 7.97 (d, J=8.8 Hz, 1H).
m/z: 258 [M+2H]²⁺, 515 [M+H]⁺.

### Example 24

### {cis-2-{4-{2-[6-Cyclopropylmethoxy-7-(N,N-dimethylaminomethyl)benz[d]isoxazol-3-yl]ethyl}piperidinomethyl}cyclohexyl}methanol dihydrochloride

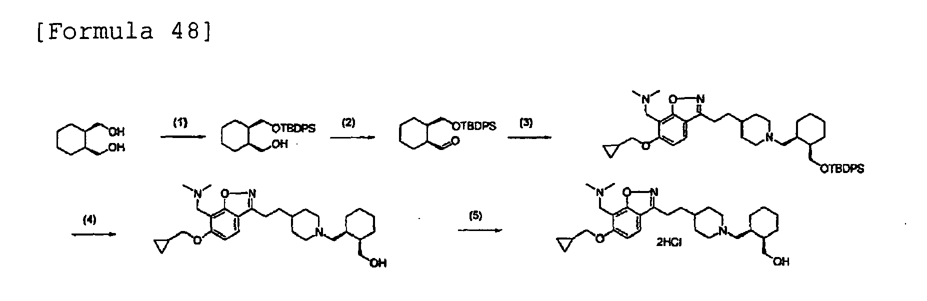

### (1) [cis-2-(tert-Butyldiphenylsilanyloxymethyl)cyclohexyl]methanol

The title compound (1.86 g) was obtained by synthesis from (cis-2-hydroxymethylcyclohexyl)methanol (1.15 g) according to Example 14-(3).
¹H-NMR (400 MHz, CDCl₃) δ (ppm): 1.05 (s, 9H), 1.23-1.38 (m, 4H), 1.41-1.52 (m, 4H), 1.92-2.04 (m, 2H), 2.93-2.97 (m, 1H), 3.52-3.58 (m, 2H), 3.67-3.74 (m, 1H), 3.79-3.84 (m, 1H), 7.36-7.45 (m, 6H), 7.64-7.68 (m, 4H).

### (2)(3) N,N-Dimethyl{3-{2-{1-[cis-2-(tert-butyldiphenylsilanyloxymethyl)cyclohexylmethyl]piperidi n-4-yl}ethyl}-6-cyclopropylmethoxybenz[d]isoxazol-7-yl}methylamine

cis-2-(tert-Butyldiphenylsilanyloxymethyl)cyclohexanecarbaldehyde (989 mg) was obtained by synthesis from [cis-2-(tert-butyldiphenylsilanyloxymethyl)cyclohexyl]methanol (1.15 g) according to Example 15-(1). The title compound (215 mg) was obtained by synthesis from cis-2-(tert-butyldiphenylsilanyloxymethyl)cyclohexanecarbaldehyde (171 mg) and N,N-dimethyl{6-cyclopropylmethoxy-3-[2-(piperidin-4-yl)ethyl]benz[d]isoxazol-7-yl}methylamine obtained in Preparation Example 3-(3) (107 mg) according to Example 21-(3).
¹H-NMR (400 MHz, CDCl₃) δ (ppm) : 0.35-0.39 (m, 2H), 0.63-0.67 (m, 2H), 1.04 (s, 9H), 1.17-1.76 (m, 17H), 1.83 (m, 1H), 1.90-1.94 (m, 2H), 2.02-2.16 (m, 2H), 2.34 (s, 6H), 2.74-2.78 (m, 2H), 2.90-2.94 (m, 2H), 3.56-3.67 (m, 2H), 3.81 (s, 2H), 3.82 (s, 2H), 3.94 (d, J=6.8 Hz, 2H), 6.89 (d, J=8.8 Hz, 1H), 7.35-7.43 (m, 6H), 7.44 (d, J=8.8 Hz, 1H), 7.66-7.70 (m, 4H).

### (4) {cis-2-{4-{2-[6-Cyclopropylmethoxy-7-(N,N-dimethylaminomethyl)benz[d]isoxazol-3-yl]ethyl}piperidinomethyl}cyclohexyl}methanol

The title compound (43 mg) was obtained by synthesis from N,N-dimethyl{3-{2-{1-[cis-2-(tert-butyldiphenylsilanyloxymethyl)cyclohexylmethyl]piperidi n-4-yl}ethyl}-6-cyclopropylmethoxybenz[d]isoxazol-7-yl}methylamine (123 mg) according to Example 16-(5).
¹H-NMR (400 MHz, CDCl₃) δ (ppm): 0.35-0.39 (m, 2H), 0.60-0.67 (m, 2H), 1.22-1.56 (m, 13H), 1.72-1.87 (m, 5H), 1.93-1.97 (m, 1H), 2.04-2.15 (m, 2H), 2.33 (s, 6H), 2.65-2.72 (m, 1H), 2.81-2.85 (m, 1H), 2.91-2.95 (m, 2H), 3.09-3.13 (m, 1H), 3.49-3.51 (m, 2H), 3.81 (s, 2H), 3.94 (d, J=6.8 Hz, 2H), 6.88 (d, J=8.8 Hz, 1H), 7.42 (d, J=8.8 Hz, 1H).

### (5) {cis-2-{4-{2-[6-Cyclopropylmethoxy-7-(N,N-dimethylaminomethyl)benz[d]isoxazol-3-yl]ethyl}piperidinomethyl}cyclohexyl}methanol dihydrochloride

The title compound (45 mg) was obtained by synthesis from {cis-2-{4-{2-[6-cyclopropylmethoxy-7-(N,N-dimethylaminomethyl)benz[d]isoxazol-3-yl]ethyl}piperidinomethyl}cyclohexyl}methanol (43 mg) according to Example 21-(4).
¹H-NMR (400 MHz, CD₃OD) δ (ppm): 0.43-0.45 (m, 2H), 0.66-0.72 (m, 2H), 1.37-2.14 (m, 17H), 2.25-2.35 (m, 1H), 2.87-3.09 (m, 5H), 2.96 (s, 6H), 3.17-3.23 (m, 1H), 3.57-3.61 (m, 2H), 3.67-3.82 (m, 2H), 4.11 (d, J=7.2 Hz, 2H), 4.63 (s, 2H), 7.24 (d, J=8.8 Hz, 1H) 7.93 (d, J=8.8 Hz, 1H).
ESI-MS m/z: 242 [M+2H]²⁺, 484 [M+H]⁺.

### Example 25

### N,N-Dimethyl{3-[2-(1-benzylpiperidin-4-yl)ethyl]-6-(thiophen-2-ylmethoxy)benz[d]isoxazol-7-yl}methylamine dihydrochloride

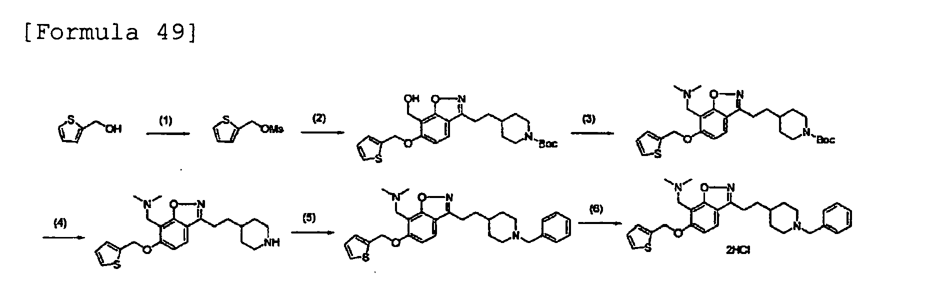

### (1)(2) tert-Butyl 4-{2-[7-hydroxymethyl-6-(thiophen-2-ylmethoxy)benz[d]isoxazol-3-yl]ethyl}piperidine-1-carboxylate

Thiophene-2-methanol (228 mg) and triethylamine (558 µL) were dissolved in tetrahydrofuran (5 mL). Methanesulfonyl chloride (232 µL) was added under ice-cooling and the mixture was stirred for 30 minutes. Then, the mixture was heated to room temperature and further stirred for 1.5 hours. A saturated ammonium chloride solution was added to the reaction mixture, and the solvent was evaporated under reduced pressure. Water was added to the residue, followed by extraction with ethyl acetate. Then, the organic layer was washed with a saturated sodium chloride solution, dried over magnesium sulfate and filtered. Then, the solvent was evaporated under reduced pressure and the residue was dried under reduced pressure to obtain 2-methanesulfonyloxymethylthiophene (385 mg). tert-Butyl 4-[2-(6-hydroxy-7-hydroxymethylbenz[d]isoxazol-3-yl)ethyl]piperidine-1-carboxylate obtained in Preparation Example 2-(6) (301 mg) and 2-methanesulfonyloxymethylthiophene (231 mg) were dissolved in N,N-dimethylformamide (5 mL). Potassium carbonate (166 mg) was added and the mixture was stirred at 60°C for 15 hours. Water was added to the reaction mixture, followed by extraction with ethyl acetate. The organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (172 mg).
¹H-NMR (400 MHz, CDCl₃) δ (ppm): 1.11-1.20 (m, 2H), 1.45 (s, 9H), 1.45-1.55 (m, 1H), 1.72-1.81 (m, 4H), 2.36 (t, J=6.8 Hz, 1H), 2.64-2.70 (m, 2H), 2.93-2.99 (m, 2H), 4.07-4.14 (m, 2H), 4.99 (d, J=6.8 Hz, 2H), 5.38 (s, 2H), 6.99-7.02 (m, 1H), 7.04 (d, J=8.8 Hz, 1H), 7.10-7.12 (m, 1H), 7.32-7.35 (m, 1H), 7.49 (d, J=8.8 Hz, 1H).

### (3) tert-Butyl 4-{2-[7-(N,N-dimethylaminomethyl)-6-(thiophen-2-ylmethoxy)benz[d]isoxazol-3-yl]ethyl}piperidine-1-carboxylate

The title compound (173 mg) was obtained by synthesis from tert-butyl 4-{2-[7-hydroxymethyl-6-(thiophen-2-ylmethoxy)benz[d]isoxazol-3-yl]ethyl}piperidine-1-carboxylate (165 mg) according to Example 21-(1).
¹H-NMR (400 MHz, CDCl₃) δ (ppm): 1.10-1.22 (m, 2H), 1.46 (s, 9H), 1.46-1.56 (m, 1H), 1.73-1.81 (m, 4H), 2.32 (s, 6H), 2.65-2.71 (m, 2H), 2.94-2.98 (m, 2H), 3.36 (s, 2H), 4.03-4.12 (m, 2H), 5.37 (s, 2H), 7.01 (dd, J=3.6, 5.2 Hz, 1H), 7.04 (d, J=8.8 Hz, 1H), 7.10 (dd, J=1.2, 3.6 Hz, 1H), 7.33 (dd, J=1.2, 5.2 Hz, 1H), 7.48 (d, J=8.8 Hz, 1H).

### (4) N,N-Dimethyl{3-[2-(piperidin-4-yl)ethyl]-6-(thiophen-2-ylmethoxy)benz[d]isoxazol-7-yl}methylamine

The title compound (101 mg) was obtained by synthesis from tert-butyl 4-{2-[7-(N,N-dimethylaminomethyl)-6-(thiophen-2-ylmethoxy)benz[d]isoxazol-3-yl]ethyl}piperidine-1-carboxylate (170 mg) according to Example 21-(2).
¹H-NMR (400 MHz, CDCl₃) δ (ppm): 1.12-1.25 (m, 2H), 1.42-1.50 (m, 1H), 1.72-1.79 (m, 4H), 2.32 (s, 6H), 2.54-2.61 (m, 2H), 2.91-2.97 (m, 2H), 3.05-3.09 (m, 2H), 3.79 (s, 2H), 5.36 (s, 2H), 7.00 (dd, J=3.6, 5.2 Hz, 1H), 7.02 (d, J=8.4 Hz, 1H), 7.10 (dd, J=1.2, 3.6 Hz, 1H), 7.32 (dd, J=1.2, 5.2 Hz, 1H), 7.47 (d, J=8.4 Hz, 1H).

### (5) N,N-Dimethyl{3-[2-(1-benzylpiperidin-4-yl)ethyl]-6-(thiophen-2-ylmethoxy)benz[d]isoxazol-7-yl}methylamine

The title compound (55 mg) was obtained by synthesis from N,N-dimethyl{3-[2-(piperidin-4-yl)ethyl]-6-(thiophen-2-ylmethoxy)benz[d]isoxazol-7-yl}methylamine (47.9 mg) and benzaldehyde (18.3 µL) according to Example 21-(3).
¹H-NMR (400 MHz, CDCl₃) δ (ppm): 1.25-1.35 (m, 3H), 1.73-1.79 (m, 4H), 1.91-1.97 (m, 2H), 2.31 (s, 6H), 2.86-2.95 (m, 4H), 3.48 (s, 2H), 3.78 (s, 2H), 5.36 (s, 2H), 6.99 (dd, J=3.6, 5.2 Hz, 1H), 7.01 (d, J=8.4 Hz, 1H), 7.08 (dd, J=1.2, 3.6 Hz, 1H), 7.20-7.26 (m, 1H), 7.28-7.30 (m, 4H), 7.31 (dd, J=1.2, 5.2 Hz, 1H), 7.46 (d, J=8.4 Hz, 1H).

### (6) N,N-Dimethyl{3-[2-(1-benzylpiperidin-4-yl)ethyl]-6-(thiophen-2-ylmethoxy)benz[d]isoxazol-7-yl}methylamine dihydrochloride

The title compound (59 mg) was obtained by synthesis from N,N-dimethyl{3-[2-(1-benzylpiperidin-4-yl)ethyl]-6-(thiophen-2-ylmethoxy)benz[d]isoxazol-7-yl}methylamine (55 mg) according to Example 21-(4).
¹H-NMR (400 MHz, CD₃OD) δ (ppm): 1.45-1.55 (m, 2H), 1.67-1.77 (m, 1H), 1.83-1.88 (m, 2H), 2.06-2.10 (m, 2H), 2.88 (s, 6H), 3.00-3.09 (m, 4H), 3.44-3.49 (m, 2H), 4.31 (s, 2H), 4.58 (s, 2H), 5.58 (s, 2H), 7.03-7.05 (m, 1H), 7.25-7.27 (m, 1H), 7.41(d, J=8.8 Hz, 1H), 7.47-7.52 (m, 6H), 7.94 (d, J=8.8 Hz, 1H).
ESI-MS m/z: 270 [M+2H]²⁺, 490 [M+H]⁺.

### Example 26

### 5-{4-{2-[7-(N,N-Dimethylaminomethyl)-6-(cis-2-hydroxymethylcyclopropylmethoxy)benz[d]isoxazol-3-yl]ethyl}piperidinomethyl]thiophene-2-carbonitrile dihydrochloride

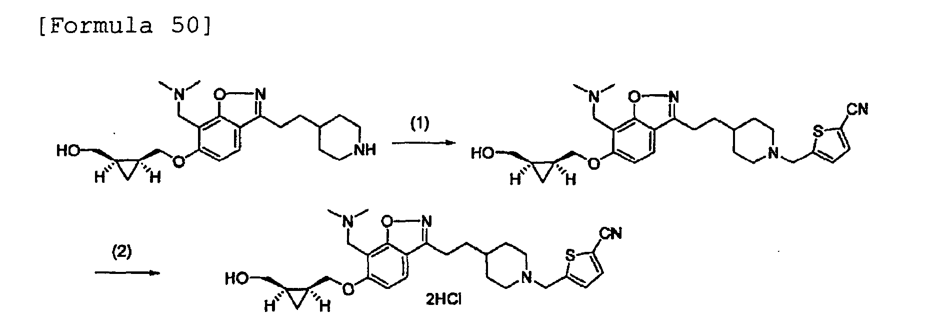

### (1) 5-{4-{2-[7-(N,N-Dimethylaminomethyl)-6-(cis-2-hydroxymethylcyclopropylmethoxy)benz[d]isoxazol-3-yl]ethyl}piperidinomethyl]thiophene-2-carbonitrile

The title compound (68 mg) was obtained by synthesis from {cis-2-{7-(N,N-dimethylaminomethyl)-3-[2-(piperidin-4-yl)ethyl]benz[d]isoxazol-6-yloxymethyl}cyclopropyl}methanol obtained in Example 3-(4) (77.6 mg) and 5-thiophene-2-carbonitrile obtained in Example 155-(1) (41.1 mg) according to Example 21-(3).
¹H-NMR (400 MHz, CDCl₃) δ (ppm): 0.18-0.23 (m, 1H), 0.84-0.90 (m, 1H), 1.25-1.40 (m, 4H), 1.50-1.60 (m, 1H), 1.76-1.81 (m, 4H), 2.01-2.07 (m, 2H), 2.23 (s, 6H), 2.89-2.96 (m, 4H), 3.14-3.21 (m, 1H), 3.54-3.65 (m, 2H), 3.68 (s, 2H), 3.84-3.93 (m, 2H), 4.60 (dd, J=5.2, 10.0 Hz, 1H), 6.86-6.93 (m, 2H), 7.45-7.48 (m, 2H).

(2) 5-{4-{2-[7-(N,N-Dimethylaminomethyl)-6-(cis-2-hydroxymethylcyclopropylmethoxy)benz[d]isoxazol-3-yl]ethyl}piperidinomethyl]thiophene-2-carbonitrile dihydrochloride

The title compound (70 mg) was obtained by synthesis from 5-{4-{2-[7-(N,N-dimethylaminomethyl)-6-(cis-2-hydroxymethylcyclopropylmethoxy)benz[d]isoxazol-3-yl]ethyl}piperidinomethyl]thiophene-2-carbonitrile (68 mg) according to Example 21-(4).
¹H-NMR (400 MHz, CD₃OD) δ (ppm): 0.35-0.39 (m, 1H) , 0.91-0.96 (m, 1H), 1.32-1.40 (m, 1H), 1.51-1.64 (m, 3H), 1.67-1.77 (m, 1H), 1.82-1.90 (m, 2H), 2.09-2.13 (m, 2H), 2.82 (s, 3H), 2.95-3.08 (m, 5H), 3.04 (s, 3H), 3.23-3.34 (m, 1H), 3.54-3.60 (m, 2H), 3.98-4.05 (m, 2H), 4.59-4.68 (m, 4H), 7.26 (d, J=8.8 Hz, 1H), 7.48 (d, J=3.6 Hz, 1H), 7.80 (d, J=3.6 Hz, 1H) , 7.93 (d, J=8.8 Hz, 1H).
ESI-MS m/z: 509 [M+H]⁺.

### Example 27

### 5-{4-{2-[7-(N,N-Dimethylaminomethyl)-6-(1-hydroxymethylcyclopropylmethoxy)benz[d]isoxazol-3-yl]ethyl}piperidinomethyl]thiophene-2-carbonitrile dihydrochloride

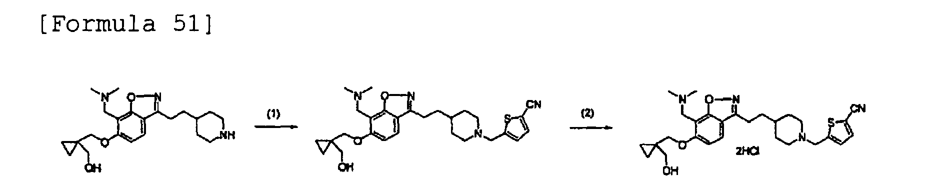

### (1) 5-{4-(2-[7-(N,N-Dimethylaminomethyl)-6-(1-hydroxymethylcyclopropylmethoxy)benz[d]isoxazol-3-yl]ethyl}piperidinomethyl]thiophene-2-carbonitrile

The title compound (102 mg) was obtained by synthesis from {1-{7-(N,N-dimethylaminomethyl)-3-[2-(piperidin-4-yl)ethyl]benz[d]isoxazol-6-yloxymethyl}cyclopropyl}methanol obtained in Example 4-(4) (77.6 mg) and 5-thiophene-2-carbonitrile obtained in Example 155-(1) (41.1 mg) according to Example 21-(3).
¹H-NMR (400 MHz, CDCl₃) δ (ppm): 0.61-0.67 (m, 4H), 1.27-1.37 (m, 3H), 1.77-1.82 (m, 4H), 2.00-2.07 (m, 2H), 2.26 (s, 6H), 2.90-2.97 (m, 4H), 3.59 (s, 2H), 3.69 (s, 2H), 3.79 (s, 2H), 4.06 (s, 2H), 6.88-6.92 (m, 2H), 7.47-7.49 (m, 2H).

### (2) 5-{4-{2-[7-(N,N-Dimethylaminomethyl)-6-(1-hydroxymethylcyclopropylmethoxy)benz[d]isoxazol-3-yl]ethyl}piperidinomethyl]thiophene-2-carbonitrile dihydrochloride

The title compound (116 mg) was obtained by synthesis from 5-{4-{2-[7-(N,N-dimethylaminomethyl)-6-(1-hydroxymethylcyclopropylmethoxy)benz[d]isoxazol-3-yl]ethyl}piperidinomethyl]thiophene-2-carbonitrile (102 mg) according to Example 21-(4).
¹H-NMR (400 MHz, CD₃OD) δ (ppm): 0.67-0.75 (m, 4H), 1.50-1.60 (m, 2H), 1.67-1.77 (m, 1H), 1.81-1.90 (m, 2H), 2.09-2.13 (m, 2H), 2.92 (s, 6H), 3.01-3.08 (m, 4H), 3.54-3.58 (m, 2H), 3.64 (s, 2H), 4.23 (s, 2H), 4.63 (s, 2H), 4.63 (s, 2H), 7.22 (d, J=8.8 Hz, 1H), 7.47 (d, J=3.6 Hz, 1H), 7.80 (d, J=3.6 Hz, 1H), 7.92 (d, J=8.8 Hz, 1H).
ESI-MS m/z: 509 [M+H]⁺.

### Example 28

### 5-{4-{2-[6-(4-Cyanobenzyl)-7-(N,N-dimethylaminomethyl)benz[d]isoxazol-3-yl]ethyl}piperidino}furan-2-carbonitrile dihydrochloride

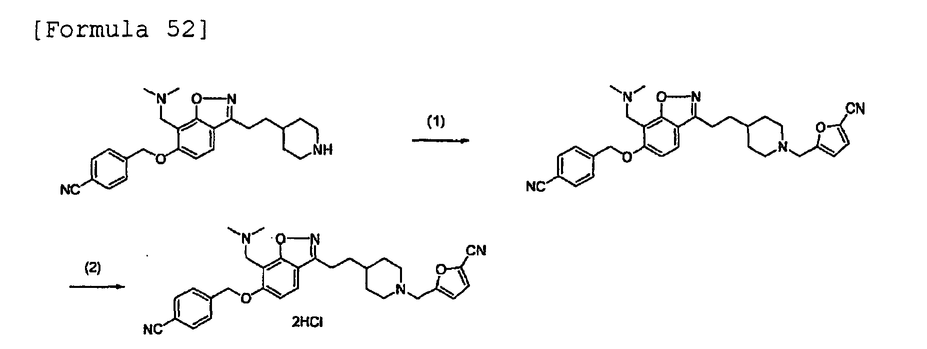

### (1) 5-{4-{2-[6-(4-Cyanobenzyl)-7-(N,N-dimethylaminomethyl)benz[d]isoxazol-3-yl]ethyl}piperidino}furan-2-carbonitrile

The title compound (72 mg) was obtained by synthesis from 4-[7-(N,N-dimethylaminomethyl)-3-(2-piperidin-4-yl)ethyl}benz[d]isoxazol-6-yloxymethyl]benzonitrile obtained in Example 16-(3) (83.2 mg) and 5-formylfuran-2-carbonitrile (48.4 mg) [CAS Registry No. 42061-89-2] according to Example 21-(3).
¹H-NMR (400 MHz, CDCl₃) δ (ppm): 1.24-1.38 (m, 3H), 1.76-1.79 (m, 4H), 1.99-2.04 (m, 2H), 2.33 (s, 6H), 2.86-2.96 (m, 4H), 3.56 (s, 2H), 3.82 (s, 2H), 5.26 (s, 2H), 6.31 (d, J=3,2 Hz, 1H), 6.92 (d, J=8.8 Hz, 1H), 7.02 (d, J=3,2 Hz, 1H), 7.45 (d, J=8.8 Hz, 1H), 7.57-7.59 (m, 2H), 7.68-7.70 (m, 2H).

### (2) 5-{4-{2-[6-(4-Cyanobenzyl)-7-(N,N-dimethylaminomethyl)benz[d]isoxazol-3-yl]ethyl}piperidino}furan-2-carbonitrile dihydrochloride

The title compound (80 mg) was obtained by synthesis from 5-{4-{2-[6-(4-cyanobenzyl)-7-(N,N-dimethylaminomethyl)benz[d]isoxazol-3-yl]ethyl}piperidino}furan-2-carbonitrile (72 mg) according to Example 21-(4).
¹H-NMR (400 MHz, CD₃OD) δ (ppm): 1.49-1.58 (m, 2H), 1.65-1.79 (m, 1H), 1.82-1.87 (m, 2H), 2.10-2.13 (m, 2H), 2.94 (s, 6H), 3.00-3.09 (m, 4H), 3.72-3.74 (m, 2H), 4.50 (s, 2H), 4.68 (s, 2H), 5.49 (s, 2H), 6.31 (d, J=3,2 Hz, 1H), 7.30 (d, J=8.8 Hz, 1H), 7.41 (d, J=3,2 Hz, 1H), 7.72 (d, J=8.0 Hz, 2H), 7.80 (d, J=8.0 Hz, 2H), 7.93 (d, J=8.8 Hz, 1H).
ESI-MS m/z: 524 [M+H]⁺.

### Example 29

### 4-{7-(N,N-Dimethylaminomethyl)-3-{2-{1-[2-(2-oxo-2H-pyridin-1-yl)ethyl]piperidin-4-yl}ethyl}benz[d]isoxazol-6-yloxymethyl}benzonitrile dihydrochloride

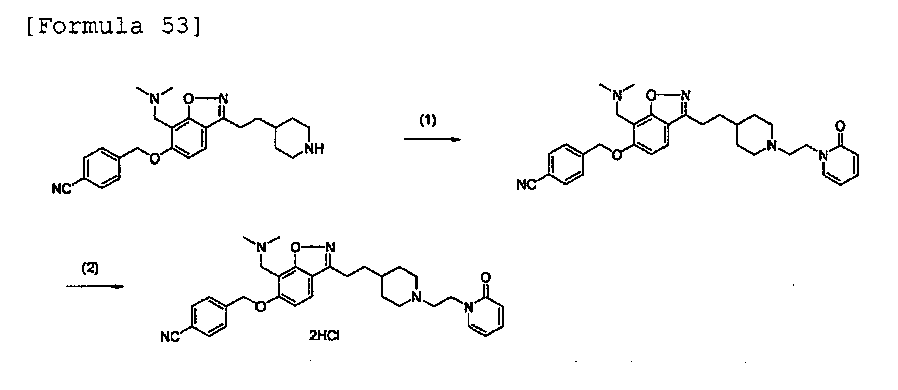

### (1) 4-{7-(N,N-Dimethylaminomethyl)-3-{2-{1-[2-(2-oxo-2H-pyridin-1-yl)ethyl]piperidin-4-yl}ethyl}benz[d]isoxazol-6-yloxymethyl}benzonitrile

The title compound (42 mg) was obtained by synthesis from 4-[7-(N,N-dimethylaminomethyl)-3-(2-piperidin-4-yl)ethyl]benz[d]isoxazol-6-yloxymethyl]benzonitrile obtained in Example 16-(3) (83.2 mg) and (2-oxo-2H-pyridin-1-yl)acetaldehyde [CAS Registry No. 408530-654] (68.6 mg) according to Example 21-(3).
¹H-NMR (400 MHz, CDCl₃) δ (ppm): 1.22-1.37 (m, 3H), 1.71-1.79 (m, 4H), 2.00-2.06 (m, 2H), 2.33 (s, 6H), 2.63-2.67 (m, 2H), 2.88-2.96 (m, 4H), 3.82 (s, 2H), 4.00-4.04 (m, 2H), 5.26 (s, 2H), 6.10-6.14 (m, 1H), 6.52-6.55 (m, 1H), 6.92 (d, J=8.8 Hz, 1H), 7.28-7.32 (m, 2H), 7.46 (d, J=8.8 Hz, 1H), 7.57-7.59 (m, 2H), 7.68-7.70 (m, 2H).

### (2) 4-{7-(N,N-Dimethylaminomethyl)-3-{2-{1-[2-(2-oxo-2H-pyridin-1-yl)ethyl]piperidin-4-yl}ethyl}benz[d]isoxazol-6-yloxymethyl}benzonitrile dihydrochloride

The title compound (46 mg) was obtained by synthesis from 4-{7-(N,N-dimethylaminomethyl)-3-{2-{1-[2-(2-oxo-2H-pyridin-1-yl)ethyl]piperidin-4-yl}ethyl}benz[d]isoxazol-6-yloxymethyl}benzonitrile (42 mg) according to Example 21-(4).
¹H-NMR (400 MHz, CD₃OD) δ (ppm): 1.57-1.66 (m, 2H), 1.71-1.79 (m, 1H), 1.83-1.89 (m, 2H), 2.11-2.15 (m, 2H), 2.94 (s, 6H), 3.06-3.12 (m, 4H), 3.53-3.59 (m, 2H), 3.75-3.78 (m, 2H), 4.51-4.57 (m, 2H), 4.68 (s, 2H), 5.50 (s, 2H), 6.66-6.70 (m, 1H), 6.77 (d, J=8.8 Hz, 1H), 7.31 (d, J=8.8 Hz, 1H), 7.74-7.80 (m, 4H), 7.91-7.96 (m, 3H).
ESI-MS m/z: 270 [M+2H]²⁺, 540 [M+H]⁺.

### Example 30

### 4-{7-(N,N-Dimethylaminomethyl)-3-{2-[1-(1-hydroxymethylcyclopentylmethyl)piperidin-4-yl]ethyl}benz[d]isoxazol-6-yloxymethyl}benzonitrile dihydrochloride

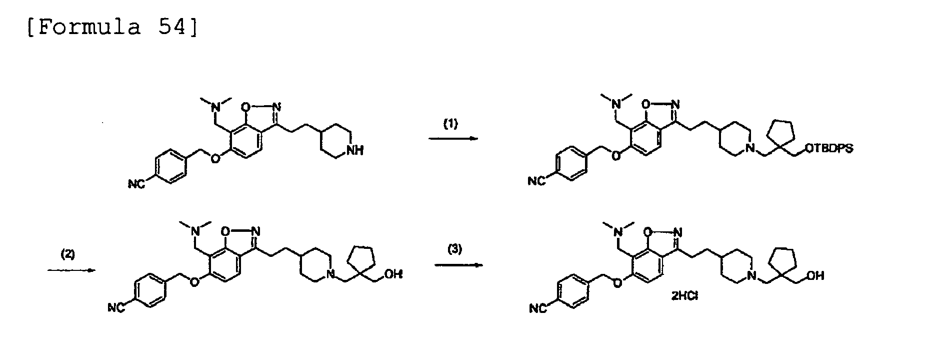

### (1) 4-{3-{2-{1-[1-(tert-Butyldiphenylsilanyloxymethyl)cyclopentylmethyl]piperid in-4-yl}ethyl}-7-(N,N-dimethylaminomethyl)benz[d]isoxazol-6-yloxymethyl}benzonitrile

The title compound (120 mg) was obtained by synthesis from 4-[7-(N,N-dimethylaminomethyl)-3-(2-piperidin-4-yl)ethyl]benz[d]isoxazol-6-yloxymethyl]benzonitrile obtained in Example 16-(3) (83.2 mg) and 1-(tert-butyldiphenylsilanyloxymethyl)cyclopentanecarbaldehyde obtained in Example 51-(3) (147 mg) according to Example 21-(3).
¹H-NMR (400 MHz, CDCl₃) δ (ppm): 1.06 (s, 9H), 1.21-1.59 (m, 13H), 1.62-1.64 (m, 2H), 1.71-1.77 (m, 2H), 2.04-2.11 (m, 2H), 2.33 (s, 6H), 2.81-2.85 (m, 2H), 2.91-2.96 (m, 2H), 3.45 (s, 2H), 3.82 (s, 2H), 5.27 (s, 2H), 6.93 (d, J=8.8 Hz, 1H), 7.33-7.42 (m, 6H), 7.47 (d, J=8.8 Hz, 1H), 7.57-7.59 (m, 2H), 7.65-7.70 (m, 6H).

### (2) 4-{7-(N,N-Dimethylaminomethyl)-3-{2-[1-(1-hydroxymethylcyclopentylmethyl)piperidin-4-yl]ethyl}benz[d]isoxazol-6-yloxymethyl}benzonitrile

The title compound (31 mg) was obtained by synthesis from 4-{3-{2-{1-[1-(tert-butyldiphenylsilanyloxymethyl)cyclopentylmethyl]piperid in-4-yl}ethyl}-7-(N,N-dimethylaminomethyl)benz[d]isoxazol-6-yloxymethyl}benzonitrile (115 mg) according to Example 16-(5).
¹H-NMR (400 MHz, CDCl₃) δ (ppm): 1.21-1.32 (m, 7H), 1.58-1.67 (m, 4H), 1.71-1.77 (m, 4H), 2.03-2.08 (m, 2H), 2.33 (s, 6H), 2.51 (s, 2H), 2.90-2.95 (m, 2H), 2.99-3.03 (m, 2H), 3.50 (s, 2H), 3.82 (s, 2H), 5.26 (s, 2H), 6.93 (d, J=8.4 Hz, 1H), 7.46 (d, J=8.4 Hz, 1H), 7.57-7.59 (m, 2H), 7.68-7.70 (m, 2H).

### (3) 4-{7-(N,N-Dimethylaminomethyl)-3-{2-[1-(1-hydroxymethylcyclopentylmethyl)piperidin-4-yl]ethyl}benz[d]isoxazol-6-yloxymethyl}benzonitrile dihydrochloride

The title compound (34 mg) was obtained by synthesis from 4-{7-(N,N-dimethylaminomethyl)-3-{2-[1-(1-hydroxymethylcyclopentylmethyl)piperidin-4-yl]ethyl}benz[d]isoxazol-6-yloxymethyl}benzonitrile (24 mg) according to Example 21-(4).
¹H-NMR (400 MHz, CD₃OD) δ (ppm): 1.46-1.62 (m, 5H), 1.68-1.75 (m, 5H), 1.81-2.09 (m, 5H), 2.94 (s, 6H), 3.00-3.11 (m, 4H), 3.24 (s, 2H), 3.60 (s, 2H), 3.70-3.73 (m, 2H), 4.68 (s, 2H), 5.49 (s, 2H), 7.30 (d, J=8.8 Hz, 1H), 7.73 (d, J=8.0 Hz, 2H), 7.79 (d, J=8.0 Hz, 2H), 7.94 (d, J=8.8 Hz, 1H).
ESI-MS m/z: 266 [M+2H]²⁺, 531 [M+H]⁺.

### Example 31

### 1-{4-{2-[6-Cyclopropylmethoxy-7-(N,N-dimethylaminomethyl)benz[d]isoxazol-3-yl]ethyl}piperidinomethyl}cyclohexanol dihydrochloride

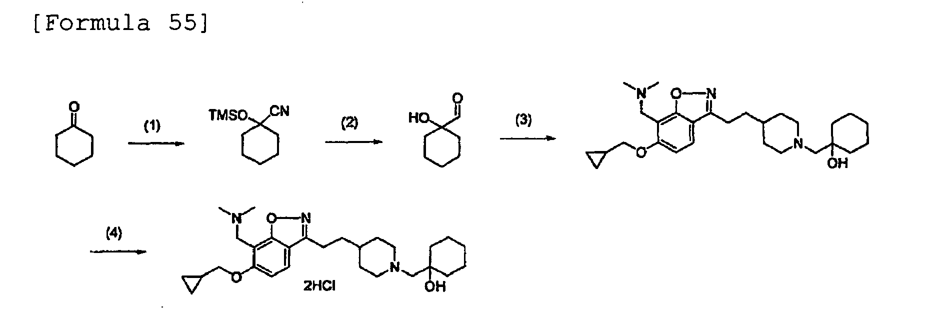

### (1) 1-Trimethylsilanyloxycyclohexanecarbonitrile

Cyclohexanone (2.94 g) and trimethylsilyl cyanide (4.4 mL) were dissolved in dichloromethane (20 mL). Zinc iodide (479 mg) was added at room temperature and then the mixture was stirred with heating under reflux for 22 hours. After cooling to room temperature, the reaction mixture was filtered through Florisil and celite. The solvent was evaporated under reduced pressure to obtain the title compound (5.92 g).
¹H-NMR (400 MHz, CDCl₃) δ (ppm): -0.01 (s, 9H), 0.95-1.05 (m, 1H), 1.26-1.42 (m, 5H), 1.47-1.54 (m, 2H), 1.79-1.83 (m, 2H).

### (2) (3) 1-{4-{2-[6-Cyclopropylmethoxy-7-(N,N-dimethylaminomethyl)benz[d]isoxazol-3-yl]ethyl}piperidinomethyl}cyclohexanol

1-Trimethylsilanyloxycyclohexanecarbonitrile (1.97 g) was dissolved in hexane (10 mL). Diisobutylaluminum hydride (1 M solution in toluene, 15 mL) was added dropwise in a nitrogen atmosphere at - 50°C. The mixture was heated to 0°C and stirred for one hour. The reaction mixture was added to diethyl ether (10 mL). A saturated ammonium chloride solution and a 0.5 M sulfuric acid solution were sequentially added and the mixture was stirred at room temperature for 24 hours. Water was added to the reaction mixture, followed by extraction with diethyl ether. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain 1-hydroxycyclohexanecarbaldehyde (200 mg). The title compound (129 mg) was obtained by synthesis from 1-hydroxycyclohexanecarbaldehyde (82.3 mg) and N,N-dimethyl{6-cyclopropylmethoxy-3-[2-(piperidin-4-yl)ethyl]benz[d]isoxazol-7-yl}methylamine obtained in Preparation Example 3-(3) (107 mg) according to Example 21-(3).
¹H-NMR (400 MHz, CDCl₃) δ (ppm): 0.35-0.39 (m, 2H), 0.61-0.67 (m, 2H), 1.21-1.78 (m, 18H), 2.26-2.32 (m, 2H), 2.28 (s, 2H), 2.33 (s, 6H), 2.83-2.86 (m, 2H), 2.90-2.95 (m, 2H), 3.81 (s, 2H), 3.93 (d, J=6.8 Hz, 2H), 6.88 (d, J=8.8 Hz, 1H), 7.43 (d, J=8.8 Hz, 1H).

### (5) 1-{4-{2-(6-Cyclopropylmethoxy-7-(N,N-dimethylaminomethyl)benz[d]isoxazol-3-yl]ethyl}piperidinomethyl}cyclohexanol dihydrochloride

The title compound (141 mg) was obtained by synthesis from 1-{4-{2-[6-cyclopropylmethoxy-7-(N,N-dimethylaminomethyl)benz[d]isoxazol-3-yl]ethyl}piperidinomethyl}cyclohexanol (129 mg) according to Example 21-(4).
¹H-NMR (400 MHz, CD₃OD) δ (ppm): 0.42-0.45 (m, 2H), 0.68-0.72 (m, 2H), 1.20-2.05 (m, 18H), 2.96 (s, 6H), 3.04-3.12 (m, 6H), 3.70-3.73 (m, 2H), 4.11 (d, J=7.2 Hz, 2H), 4.64 (s, 2H), 7.24 (d, J=8.8 Hz, 1H), 7.91 (d, J=8.8 Hz, 1H).
ESI-MS m/z: 235 [M+2H]²⁺, 470 [M+H]⁺.

### Example 32

### 4-{7-(N,N-Dimethylaminomethyl)-3-{2-[1-(cis-2-hydroxymethylcyclohexylmethyl)piperidin-4-yl]ethyl}benz[d]isoxazol-6-yloxymethyl}benzonitrile dihydrochloride

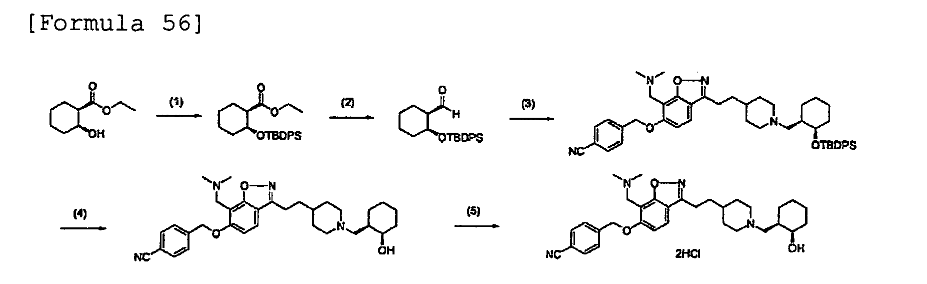

### (1) Ethyl (cis-2-tert-butyldiphenylsilanyloxycyclohexyl)acetate

Ethyl (cis-2-hydroxycyclohexyl)acetate (1.38 g) and imidazole (1.04 g) were dissolved in N,N-dimethylformamide (10 mL). tert-Butylchlorodiphenylsilane (4.16 mL) was added and the mixture was stirred at room temperature for 24 hours. Methanol and water were sequentially added to the reaction mixture, followed by extraction with ethyl acetate. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (3.3 g).
¹H-NMR (400 MHz, CDCl₃) δ (ppm): 1.03 (s, 9H), 1.10 (t, J=7.2 Hz, 3H), 1.15-1.29 (m, 3H), 1.64-1.80 (m, 4H), 1.97-2.06 (m, 1H), 2.30-2.35 (m, 1H), 3.73-3.81 (m, 1H), 3.99-4.06 (m, 1H), 4.39-4.41 (m, 1H), 7.32-7.42 (m, 6H), 7.59-7.61 (m, 2H), 7.66-7.69 (m, 2H).

### (2)(3) 4-{7-(N,N-Dimethylaminomethyl)-3-{2-[1-(cis-2-tert-butyldiphenylsilanyloxymethylcyclohexylmethyl)piperidin -4-yl]ethyl}benz[d]isoxazol-6-yloxymethyl}benzonitrile

Ethyl (cis-2-tert-butyldiphenylsilanyloxycyclohexyl)acetate (3.29 g) was dissolved in dichloromethane (15 mL). Diisobutylaluminum hydride (1.01 M solution in toluene, 8 mL) was added dropwise in a nitrogen atmosphere at - 78°C, and the mixture was stirred at the same temperature for 2.5 hours. Methanol was added to the reaction mixture, followed by heating to room temperature. 1 M hydrochloric acid was added to the reaction mixture, followed by extraction with ethyl acetate. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain (cis-2-tert-butyldiphenylsilanyloxycyclohexyl)carbaldehyde (2.62 g). The title compound (134 mg) was obtained by synthesis from (cis-2-tert-butyldiphenylsilanyloxycyclohexyl)carbaldehyde (110 mg) and 4-{7-(N,N-dimethylaminomethyl)-3-[2-(piperidin-4-yl)ethyl]benz[d]isoxazol-6-yloxymethyl}benzonitrile obtained in Example 16-(3) (83.2 mg) according to Example 21-(3).
¹H-NMR (400 MHz, CDCl₃) δ (ppm): 1.08 (s, 9H), 1.11-1.77 (m, 18H), 2.04-2.08 (m, 1H), 2.24-2.29 (m, 1H), 2.33 (s, 6H), 2.54-2.57 (m, 1H), 2.73-2.76 (m, 1H), 2.91-2.95 (m, 2H), 3.82 (s, 2H), 4.03-4.06 (m, 1H), 5.27 (s, 2H), 6.92 (d, J=8.8 Hz, 1H), 7.31-7.41 (m, 6H), 7.46 (d, J=8.8 Hz, 1H), 7.58 (d, J=8.4 Hz, 2H), 7.66-7.72 (m, 6H).

### (4) 4-{7-(N,N-Dimethylaminomethyl)-3-{2-[1-(cis-2-hydroxymethylcyclohexylmethyl)piperidin-4-yl]ethyl}benz[d]isoxazol-6-yloxymethyl}benzonitrile

The title compound (82 mg) was obtained by synthesis from 4-{7-(N,N-dimethylaminomethyl)-3-{2-[1-(cis-2-tert-butyldiphenylsilanyloxymethylcyclohexylmethyl)piperidin -4-yl]ethyl}benz[d]isoxazol-6-yloxymethyl}benzonitrile (123 mg) according to Example 17-(2).
¹H-NMR (400 MHz, CDCl₃) δ (ppm): 1.22-1.87 (m, 16H), 2.03-2.08 (m, 2H), 2.10-2.18 (m, 1H), 2.22-2.26 (m, 1H), 2.33 (s, 6H), 2.86-2.96 (m, 4H), 3.16-3.19 (m, 1H), 3.79-3.82 (m, 3H), 5.27 (s, 2H), 6.93 (d, J=8.8 Hz, 1H), 7.46 (d, J=8.8 Hz, 1H), 7.58-7.60 (m, 2H), 7.69-7.72 (m, 2H).

### (5) 4-{7-(N,N-Dimethylaminomethyl)-3-{2-[1-(cis-2-hydroxymethylcyclohexylmethyl)piperidin-4-yl]ethyl}benz[d]isoxazol-6-yloxymethyl}benzonitrile dihydrochloride

The title compound (86 mg) was obtained by synthesis from 4-{7-(N,N-dimethylaminomethyl)-3-{2-[1-(cis-2-hydroxymethylcyclohexylmethyl)piperidin-4-yl]ethyl}benz[d]isoxazol-6-yloxymethyl}benzonitrile (82 mg) according to Example 21-(4).
¹H-NMR (400 MHz, CD₃OD) δ (ppm): 1.37-1.43 (m, 2H), 1.56-1.75 (m, 9H), 1.83-1.88 (m, 2H), 2.03-2.01 (m, 3H), 2.90-2.99 (m, 3H), 2.93 (s, 6H), 3.05-3.09 (m, 2H), 3.26-3.29 (m, 1H), 3.59-3.68 (m, 2H), 3.92-3.95 (m, 1H), 4.68 (s, 2H), 5.49 (s, 2H), 7.30 (d, J=8.8 Hz, 1H), 7.73 (d, J=8.4 Hz, 2H), 7.79 (d, J=8.4 Hz, 2H), 7.94 (d, J=8.8 Hz, 1H).
ESI-MS m/z: 266 [M+2H]²⁺, 531 [M+H]⁺.

### Example 33

### cis-2-{4-{2-[6-Cyclopropylmethoxy-7-(N,N-dimethylaminomethyl)benz[d]isoxazol-3-yl]ethyl}piperidinomethyl}cyclopentanol dihydrochloride

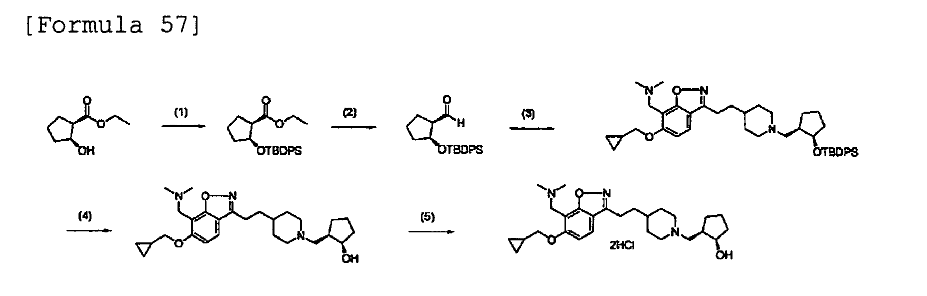

### (1) Ethyl (cis-2-tert-butyldiphenylsilanyloxycyclopentyl)acetate

The title compound (3.97 g) was obtained by synthesis from ethyl (cis-2-hydroxycyclopentyl)acetate (1.58 g) according to Example 32-(1).
¹H-NMR (400 MHz, CDCl₃) δ (ppm): 1.02 (s, 9H), 1.23 (t, J=7.2 Hz, 3H), 1.38-1.50 (m, 2H), 1.57-1.65 (m, 1H), 1.70-1.88 (m, 2H), 2.16-2.26 (m, 1H), 2.72-2.77 (m, 1H), 3.95-4.04 (m, 1H), 4.14-4.24 (m, 1H), 4.50-4.54 (m, 1H), 7. 33-7 . 42 (m, 6H), 7.65-7.69 (m, 4H).

### (2)(3) N,N-Dimethyl{3-{2-{1-[cis-2-tert-butyldiphenylsilanyloxy]cyclopentylmethyl]piperidin-4-yl}ethyl}-6-cyclopropylmethoxybenz[d]isoxazol-7-yl}methylamine

(cis-2-tert-Butyldiphenylsilanyloxycyclopentyl)carbaldehyde (909 mg) was obtained by synthesis from ethyl (cis-2-tert-butyldiphenylsilanyloxycyclopentyl)acetate (2.38 g) according to Example 32-(2). The title compound (201 mg) was obtained by synthesis from (cis-2-tert-butyldiphenylsilanyloxycyclopentyl)carbaldehyde (159 mg) and N,N-dimethyl{6-cyclopropylmethoxy-3-[2-(piperidin-4-yl)ethyl]benz[d]isoxazol-7-yl}methylamine obtained in Preparation Example 3-(3) (107 mg) according to Example 21-(3).
¹H-NMR (400 MHz, CDCl₃) δ (ppm): 0.35-0.39 (m, 2H), 0.62-0.66 (m, 2H), 1.06 (s, 9H), 1.21-1.89 (m, 17H), 2.28-2.33 (m, 1H), 2.33 (s, 6H), 2.55-2.62 (m, 1H), 2.78-2.81 (m, 1H), 2.87-2.96 (m, 3H), 3.83 (s, 2H), 3.94 (d, J=6.8 Hz, 2H), 4.30-4.32 (m, 1H), 6. 90 (d, J=8.8 Hz, 1H), 7.33-7.44 (m, 6H), 7.46 (d, J=8.8 Hz, 1H), 7.65-7.74 (m, 4H).

### (4) cis-2-{4-{2-[6-Cyclopropylmethoxy-7-(N,N-dimethylaminomethyl)benz[d]isoxazol-3-yl]ethyl}piperidinomethyl}cyclopentanol

The title compound (89 mg) was obtained by synthesis from N,N-dimethyl{3-{2-{1-[cis-2-tert-butyldiphenylsilanyloxy]cyclopentylmethyl}piperidin-4-yl}ethyl}-6-cyclopropylmethoxybenz[d]isoxazol-7-yl}methylamine (174 mg) according to Example 17-(2).
¹H-NMR (400 MHz, CDCl₃) δ (ppm): 0.36-0.39 (m, 2H), 0.64-0.67 (m, 2H), 1.22-1.51 (m, 6H), 1.58-1.87 (m, 9H), 1.90-2.05 (m, 2H), 2.18-2.23 (m, 1H), 2.29-2.36 (m, 1H), 2.33 (s, 6H), 2.82-2.85 (m, 1H), 2.91-2.96 (m, 2H), 3.18-3.22 (m, 1H), 3.82 (s, 2H), 3.94 (d, J=6.8 Hz, 2H), 4.25-4.30 (m, 1H), 6.90 (d, J=8.8 Hz, 1H), 7.42 (d, J=8.8 Hz, 1H).

### (5) cis-2-{4-{2-[6-Cyclopropylmethoxy-7-(N,N-dimethylaminomethyl)benz[d]isoxazol-3-yl]ethyl}piperidinomethyl}cyclopentanol dihydrochloride

The title compound (91 mg) was obtained by synthesis from cis-2-{4-{2-[6-cyclopropylmethoxy-7-(N,N-dimethylaminomethyl)benz[d]isoxazol-3-yl]ethyl}piperidinomethyl}cyclopentanol (89 mg) according to Example 21-(4).
¹H-NMR (400 MHz, CD₃OD) δ (ppm): 0.43-0.45 (m, 2H), 0.68-0.71 (m, 2H), 1.34-2.10 (m, 13H), 2.22-2.29 (m, 1H), 2.95-2.99 (m, 2H), 2.96 (s, 6H), 3.04-3.09 (m, 3H), 3.35-3.41 (m, 2H), 3.61-3.69 (m, 2H), 4.11 (d, J=7 .2 Hz, 2H), 4 .25-4 .26 (m, 1H), 4.63 (s, 2H), 7.24 (d, J=8.8 Hz, 1H) 7.92 (d, J=8.8 Hz, 1H).
ESI-MS m/z: 228 [M+2H]2⁺, 456 [M+H]⁺.

### Example 34

### cis-4-{4-{2-[6-Cyclopropylmethoxy-7-(N,N-dimethylaminomethyl)benz[d]isoxazol-3-yl]ethyl}piperidinomethyl}cyclohexanol dihydrochloride

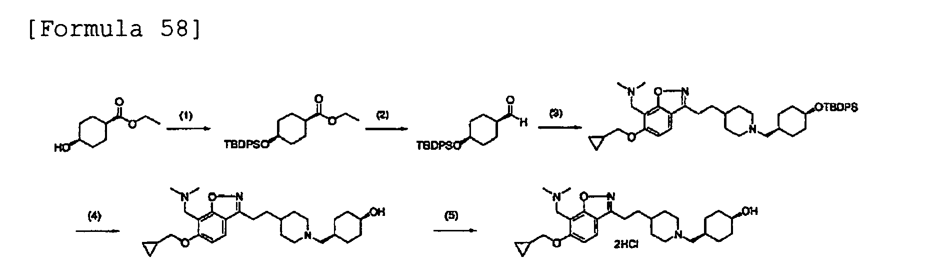

### (1) Ethyl (cis-4-tert-butyldiphenylsilanyloxycyclohexyl)acetate

The title compound (2.07 g) was obtained by synthesis from ethyl (cis-4-hydroxycyclohexyl)acetate (1.12 g) according to Example 32-(1).
¹H-NMR (400 MHz, CDCl₃) δ (ppm): 1.08 (s, 9H), 1.27 (t, J=7.2 Hz, 3H), 1.32-1.40 (m, 2H), 1.62-1.70 (m, 4H), 1.99-2.09 (m, 2H), 2.24-2.30 (m, 1H), 3.93-3.94 (m, 1H), 4.08 (q, J=7.2 Hz, 2H), 7.34-7.44 (m, 6H), 7.65-7.70 (m, 4H).

### (2)(3) N,N-Dimethyl{3-{2-{1-[cis-4-(tert-butyldiphenylsilanyloxy)cyclohexylmethyl]piperidin-4-yl}ethyl}-6-cyclopropylmethoxybenz[d]isoxazol-7-yl}methylamine

(cis-4-tert-Butyldiphenylsilanyloxycyclohexyl)carbaldehyde (1.25 g) was obtained by synthesis from ethyl (cis-4-tert-butyldiphenylsilanyloxycyclohexyl)acetate (1.85 g) according to Example 32-(2). The title compound (191 mg) was obtained by synthesis from (cis-4-tert-butyldiphenylsilanyloxycyclohexyl)carbaldehyde (165 mg) and N,N-dimethyl{6-cyclopropylmethoxy-3-[2-(piperidin-4-yl)ethyl]benz[d]isoxazol-7-yl}methylamine obtained in Preparation Example 3-(3) (107 mg) according to Example 21-(3).
¹H-NMR (400 MHz, CDCl₃) δ (ppm): 0.35-0.39 (m, 2H), 0.62-0.66 (m, 2H), 1.07 (s, 9H), 1.21-1.84 (m, 17H), 2.12-2.22 (m, 2H), 2.34 (s, 6H), 2.87-2.95 (m, 4H), 3. 50-3 . 53 (m, 2H), 3. 84 (s, 2H), 3. 93 (d, J=6.8 Hz, 2H), 3.97-4.01 (m, 1H), 6.88 (d, J=8.8 Hz, 1H), 7.32-7.42 (m, 6H), 7.42 (d, J=8.8 Hz, 1H), 7.63-7.66 (m, 4H).

### (4) cis-4-{4-{2-[6-Cyclopropylmethoxy-7-(N,N-dimethylaminomethyl)benz[d]isoxazol-3-yl]ethyl}piperidinomethyl}cyclohexanol

The title compound (78 mg) was obtained by synthesis from N,N-dimethyl{3-{2-{1-[cis-4-(tert-butyldiphenylsilanyloxy)cyclohexylmethyl]piperidin-4-yl}ethyl}-6-cyclopropylmethoxybenz[d]isoxazol-7-yl}methylamine (177 mg) according to Example 17-(2). ¹H-NMR (400 MHz, CDCl₃) δ (ppm): 0.36-0.39 (m, 2H), 0.62-0.67 (m, 2H), 1.23-1.79 (m, 17H), 1.83-1.91 (m, 2H), 2.15 (d, J=6.4 Hz, 2H), 2.34 (s, 6H), 2.84-2.88 (m, 2H), 2.92-2.96 (m, 2H), 3.83 (s, 2H), 3.94 (d, J=6.8 Hz, 2H), 3.96-3.97 (m, 1H), 6.88 (d, J=8.8 Hz, 1H), 7.42 (d, J=8.8 Hz, 1H).

### (5) cis-4-{4-{2-[6-Cyclopropylmethoxy-7-(N,N-dimethylaminomethyl)benz[d]isoxazol-3-yl]ethyl}piperidinomethyl}cyclohexanol dihydrochloride

The title compound (85 mg) was obtained by synthesis from cis-4-{4-{2-[6-cyclopropylmethoxy-7-(N,N-dimethylaminomethyl)benz[d]isoxazol-3-yl]ethyl}piperidinomethyl}cyclohexanol (78 mg) according to Example 21-(4).
¹H-NMR (400 MHz, CD₃OD) δ (ppm): 0.42-0.44 (m, 2H), 0.68-0.72 (m, 2H), 1.33-2.08 (m, 17H), 2.92-3.00 (m, 4H), 2.96 (s, 6H), 3.04-3.08 (m, 2H), 3.58-3.62 (m, 2H), 3.94-3.95 (m, 1H), 4.11 (d, J=7.2 Hz, 2H), 4.63 (s, 2H), 7.23 (d, J=8.8 Hz, 1H) 7.93 (d, J=8.8 Hz, 1H). ESI-MS m/z: 235 [M+2H]²⁺, 470 [M+H]⁺.

### Example 35

### 5-{4-{2-[7-(N,N-Dimethylaminomethyl)-6-(tetrahydrofuran-3-ylmethoxy)benz[d]isoxazol-3-yl]ethyl}piperidinomethyl]thiophene-2-carbonitrile dihydrochloride

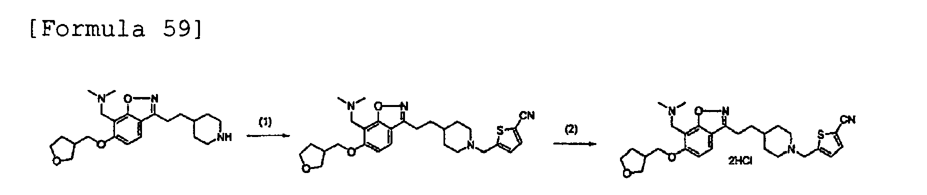

### (1) 5-{4-{2-[7-(N,N-Dimethylaminomethyl)-6-(tetrahydrofuran-3-ylmethoxy)benz[d]isoxazol-3-yl]ethyl}piperidinomethyl]thiophene-2-carbonitrile

The title compound (82 mg) was obtained by synthesis from N,N-dimethyl{3-[2-(piperidin-4-yl)ethyl]-6-(tetrahydrofuran-3-ylmethoxy)benz[d]isoxazol-7-yl}methylamine obtained in Example 6-(4) (116 mg) and 5-thiophene-2-carbonitrile obtained in Example 155-(1) (61.7 mg) according to Example 21-(3).
¹H-NMR (400 MHz, CDCl₃) δ (ppm): 1.24-1.39 (m, 3H), 1.74-1.82 (m, 5H), 2.00-2.07 (m, 2H), 2.11-2.20 (m, 1H), 2.33 (s, 6H), 2.79-2.87 (m, 1H), 2.89-2.97 (m, 4H), 3.69 (s, 2H), 3.74-3.84 (m, 4H), 3.90-4.09 (m, 4H), 6. 87-6. 89 (m, 1H), 6. 92 (d, J=8.8 Hz, 1H), 7. 47-7.49 (m, 2H).

### (2) 5-{4-{2-[7-(N,N-Dimethylaminomethyl)-6-(tetrahydrofuran-3-ylmethoxy)benz[d]isoxazol-3-yl]ethyl}piperidinomethyl]thiophene-2-carbonitrile dihydrochloride

The title compound (90 mg) was obtained by synthesis from 5-{4-{2-[7-(N,N-dimethylaminomethyl)-6-(tetrahydrofuran-3-ylmethoxy)benz[d]isoxazol-3-yl]ethyl}piperidinomethyl]thiophene-2-carbonitrile (82 mg) according to Example 21-(4).
¹H-NMR (400 MHz, CD₃OD) δ (ppm): 1.55-1.65 (m, 2H), 1.69-1.74 (m, 1H), 1.80-1.90 (m, 4H), 2.08-2.12 (m, 2H), 2.17-2.27 (m, 1H), 2.94 (s, 3H), 2.96 (s, 3H), 3.03-3.10 (m, 4H), 3.53-3.57 (m, 2H), 3.76-3.80 (m, 2H), 3.91-4.00 (m, 2H), 4 . 18-4.28 (m, 2H), 4.61 (s, 2H), 4.65 (s, 2H), 7.27 (d, J=8.8 Hz, 1H), 7.51 (d, J=3.6 Hz, 1H), 7 . 79 (d, J=3.6 Hz, 1H), 7 . 94 (d, J=8.8 Hz, 1H).
ESI-MS m/z: 509 [M+H]⁺.

### Example 36

### 5-{4-{2-[7-(N,N-Dimethylaminomethyl)-6-(trans-2-hydroxycyclohexylmethoxy)benz[d]isoxazol-3-yl]ethyl}piperidinomethyl]thiophene-2-carbonitrile dihydrochloride

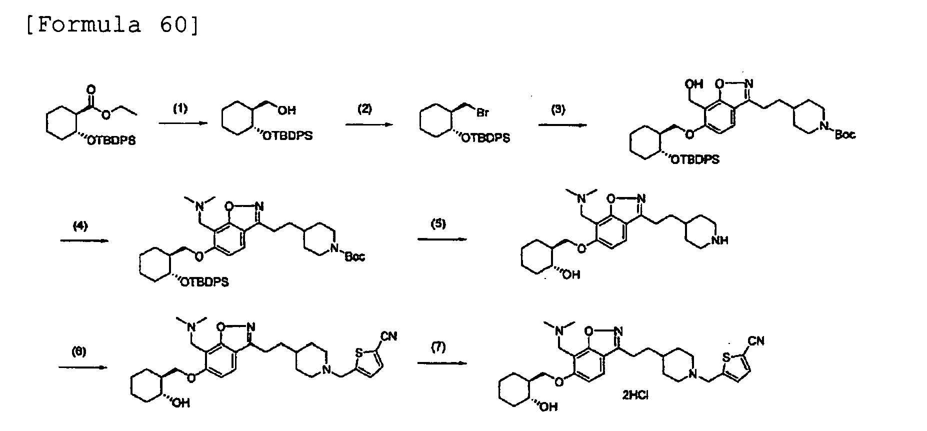

### (1) [trans-2-(tert-Butyldiphenylsilanyloxy)cyclohexyl]methanol

Ethyl (trans-2-tert-butyldiphenylsilanyloxycyclohexyl)acetate obtained in Example 20-(1) (1.64 g) was dissolved in tetrahydrofuran (10 mL). Diisobutylaluminum hydride (1.01 M solution in toluene, 10 mL) was added dropwise in a nitrogen atmosphere at -78°C, and the mixture was stirred at the same temperature for four hours. Methanol was added to the reaction mixture, followed by heating to room temperature. 1 M hydrochloric acid was added to the reaction mixture, followed by extraction with ethyl acetate. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (890 mg).
¹H-NMR (400 MHz, CDCl₃) δ (ppm): 0.84-1.32 (m, 4H), 1.04 (s, 9H), 1.47-1.70 (m, 4H), 2. 15-2. 17 (m, 1H), 3.53-3.61 (m, 2H), 3.68-3.73 (m, 1H), 7 . 36-7 . 47 (m, 6H), 7.69-7.76 (m, 4H).

### (2)(3) tert-Butyl 4-{2-{6-[trans-2-(tert-butyldiphenylsilanyloxy)cyclohexylmethoxy]-7-hydroxymethylbenz[d]isoxazol-3-yl}ethyl}piperidine-1-carboxylate

(trans-2-Bromomethylcyclohexyloxy)-tert-butyldiphenylsilane (693 mg) was obtained by synthesis from [trans-2-(tert-butyldiphenylsilanyloxy)cyclohexyl]methanol (737 mg) according to Example 3-(1). (trans-2-Bromomethylcyclohexyloxy)-tert-butyldiphenylsilane (622 mg) and tert-butyl 4-[2-(6-hydroxy-7-hydroxymethylbenz[d]isoxazol-3-yl)ethyl]piperidine-1-carboxylate obtained in Preparation Example 2-(6) (451 mg) were dissolved in N,N-dimethylformamide (5 mL). Potassium carbonate (332 mg), sodium iodide (216 mg) and hexamethylphosphoric acid triamide (1 mL) were added and the mixture was stirred at 70°C for 8.5 hours. Water was added to the reaction mixture, followed by extraction with ethyl acetate. Then, the organic layer was washed with a saturated sodium chloride solution, dried over magnesium sulfate and filtered. Then, the solvent was evaporated under reduced pressure. The residue was purified by silica gel column chromatography (heptane-ethyl acetate) to obtain the title compound (680 mg).
¹H-NMR (400 MHz, CDCl₃) δ (ppm): 1.04 (s, 9H), 1.11-1.96 (s, 16H), 1.46 (s, 9H), 2.63-2.70 (m, 2H), 2.96-3.00 (m, 2H), 3.70-3.78 (m, 2H), 4.08-4.15 (m, 3H), 4.81-4.88 (m, 2H), 6.79 (d, J=8.8 Hz, 1H), 7.32-7.45 (m, 7H), 7 . 62-7 . 73 (m, 4H).

### (4) tert-Butyl 4-{2-{6-[trans-2-(tert-butyldiphenylsilanyloxy)cyclohexylmethoxy]-7-(N,N-dimethylaminomethyl)benz[d]isoxazol-3-yl}ethyl}piperidine-1-carboxylate

The title compound (389 mg) was obtained by synthesis from tert-butyl 4-{2-{6-[trans-2-(tert-butyldiphenylsilanyloxy)cyclohexylmethoxy]-7-hydroxymethylbenz[d]isoxazol-3-yl}ethyl}piperidine-1-carboxylate (581 mg) according to Example 21-(1).
¹H-NMR (400 MHz, CDCl₃) δ (ppm): 1.01 (s, 9H), 1.03-1.81 (s, 14H), 1.46 (s, 9H), 1.94-2.00 (m, 2H), 2.29 (s, 6H), 2.65-2.72 (m, 2H), 2.95-2.99 (m, 2H), 3.61-3.73 (m, 3H), 4.00-4.25 (m, 4H), 6.80 (d, J=8.8 Hz, 1H), 7.31-7.43 (m, 7H), 7.61-7.67 (m, 4H).

### (5) trans-2-{7-(N,N-Dimethylaminomethyl)-3-[2-(piperidin-4-yl)ethyl]benz[d]isoxazol-6-yloxymethyl}cyclohexanol

tert-Butyl 4-{2-{6-[trans-2-(tert-butyldiphenylsilanyloxy)cyclohexylmethoxy]-7-(N,N-dimethylaminomethyl)benz[d]isoxazol-3-yl}ethyl}piperidine-1-carboxylate (377 mg) was dissolved in methanol (625 µL). Hydrochloric acid (4 M solution in ethyl acetate, 625 µL) was added and the mixture was stirred at room temperature for 23 hours. Tetrahydrofuran (1.9 mL) and hydrofluoric acid (70% solution in pyridine, 625 µL) were sequentially added to the reaction mixture, followed by stirring at room temperature for four hours. Water and a 5 M sodium hydroxide solution were sequentially added to the reaction mixture, followed by extraction with chloroform. Then, the organic layer was washed with a saturated sodium chloride solution, dried over magnesium sulfate and filtered. Then, the solvent was evaporated under reduced pressure. The residue was purified by NH silica gel column chromatography (heptane-ethyl acetate) to obtain the title compound (112 mg).
¹H-NMR (400 MHz, CDCl₃) δ (ppm): 1.13-1.23 (m, 2H), 1.24-1.35 (m, 4H), 1.44-1.53 (m, 1H), 1.74-1.80 (m, 8H), 2.02-2.06 (m, 1H), 2.25 (s, 6H), 2.56-2.62 (m, 2H), 2.93-2.97 (m, 2H),3.06-3.10 (m, 2H), 3.57-3.62 (m, 1H), 3.68 (d, J=12.0 Hz, 1H), 3.79 (d, J=12.0 Hz, 1H), 4.06-4.10 (m, 1H), 4.19-4.22 (m, 1H), 6.99 (d, J=8.8 Hz, 1H), 7.48 (d, J=8.8 Hz, 1H).

### (6) 5-{4-{2-[7-(N,N-Dimethylaminomethyl)-6-(trans-2-hydroxycyclohexylmethoxy)benz[d]isoxazol-3-yl]ethyl}piperidinomethyl]thiophene-2-carbonitrile

The title compound (50 mg) was obtained by synthesis from trans-2-{7-(N,N-dimethylaminomethyl)-3-[2-(piperidin-4-yl)ethyl]benz[d]isoxazol-6-yloxymethyl}cyclohexanol (83.2 mg) and 5-thiophene-2-carbonitrile obtained in Example 155-(1) (41.2 mg) according to Example 21-(3).
¹H-NMR (400 MHz, CDCl₃) δ (ppm): 1.24-1.36 (m, 7H), 1.76-1.82 (m, 8H), 1.99-2.07. (m, 3H), 2.27 (s, 6H), 2.90-2.97 (m, 4H), 3.56-3.63 (m, 1H), 3.69 (s, 2H), 3.70 (d, J=12.0 Hz, 1H), 3.81 (d, J=12.0 Hz, 1H), 4.06-4.11 (m, 1H), 4.19-4.22 (m, 1H), 6.87-6.89 (m, 1H), 6.99 (d, J=8.8 Hz, 1H), 7.47 (d, J=3.6 Hz, 1H), 7.49 (d, J=8.8 Hz, 1H) .

### (7) 5-{4-{2-[7-(N,N-Dimethylaminomethyl)-6-(trans-2-hydroxycyclohexylmethoxy)benz[d]isoxazol-3-yl]ethyl}piperidinomethyl]thiophene-2-carbonitrile dihydrochloride

The title compound (55 mg) was obtained by synthesis from 5-{4-{2-[7-(N,N-dimethylaminomethyl)-6-(trans-2-hydroxycyclohexylmethoxy)benz[d]isoxazol-3-yl]ethyl}piperidinomethyl]thiophene-2-carbonitrile (50 mg) according to Example 21-(4).
¹H-NMR (400 MHz, CD₃OD) δ (ppm): 1.23-2.11 (m, 14H), 2.85 (s, 3H), 2.96 (s, 3H), 3.01-3.08 (m, 4H), 3.47-3.57 (m, 3H), 4.26-4.33 (m, 2H), 4.59-4.63 (m, 4H), 7.27 (d, J=8.8 Hz, 1H), 7.45 (d, J=4.0 Hz, 1H), 7.80 (d, J=4.0 Hz, 1H), 7.93 (d, J=8.8 Hz, 1H).
ESI-MS m/z: 537 [M+H]⁺.

### Example 37

### 5-{4-{2-[7-(N,N-Dimethylaminomethyl)-6-(cis-4-hydroxycyclohexylmethoxy)benz[d]isoxazol-3-yl]ethyl}piperidinomethyl]thiophene-2-carbonitrile dihydrochloride

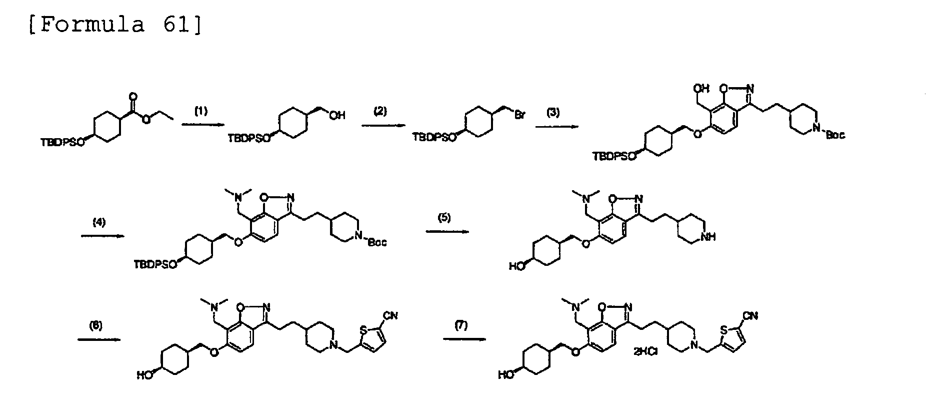

### (1) [cis-4-(tert-Butyldiphenylsilanyloxy)cyclohexyl]methanol

The title compound (4.23 g) was obtained by synthesis from ethyl (cis-2-tert-butyldiphenylsilanyloxycyclohexyl)acetate obtained in Example 34-(1) (4.93 g) according to Example 36-(1).
¹H-NMR (400 MHz, CDCl₃) δ (ppm): 1.07 (s, 9H), 1.24-1.60 (m, 7H), 1.67-1.71 (m, 2H), 3.52 (d, J=5.6 Hz, 2H), 4.01-4.04 (m, 1H), 7.34-7.44 (m, 6H), 7.64-7.69 (m, 4H).

### (2)(3) tert-Butyl 4-{2-{6-[cis-4-(tert-butyldiphenylsilanyloxy)cyclohexylmethoxy]-7-hydroxymethylbenz[d]isoxazol-3-yl}ethyl}piperidine-1-carboxylate

(cis-4-Bromomethylcyclohexyloxy)-tert-butyldiphenylsilane (852 mg) was obtained by synthesis from [cis-4-(tert-butyldiphenylsilanyloxy)cyclohexyl]methanol (737 mg) according to Example 3-(1). The title compound (710 mg) was obtained by synthesis from (cis-4-bromomethylcyclohexyloxy)-tert-butyldiphenylsilane (777 mg) and tert-butyl 4-[2-(6-hydroxy-7-hydroxymethylbenz[d]isoxazol-3-yl)ethyl]piperidine-1-carboxylate obtained in Preparation Example 2-(6) (564 mg) according to Example 36-(3).
¹H-NMR (400 MHz, CDCl₃) δ (ppm): 1.06 (s, 9H), 1.11-2.05 (s, 16H), 1.46 (s, 9H), 2.39-2.47 (m, 2H), 2.93-2.97 (m, 2H), 3.99 (d, J=5.6 Hz, 2H), 4.06-4.15 (m, 3H), 5.03 (s, 2H), 6.97 (d, J=8.8 Hz, 1H), 7.34-7.45 (m, 6H), 7.47 (d, J=8.8 Hz, 1H), 7.65-7.70 (m, 4H).

### (4) tert-Butyl 4-{2-{6-[cis-4-(tert-butyldiphenylsilanyloxy)cyclohexylmethoxy]-7-(N,N-dimethylaminomethyl)benz[d]isoxazol-3-yl}ethyl}piperidine-1-carboxylate

The title compound (420 mg) was obtained by synthesis from tert-butyl 4-{2-{6-[cis-4-(tert-butyldiphenylsilanyloxy)cyclohexylmethoxy]-7-hydroxymethylbenz[d]isoxazol-3-yl}ethyl}piperidine-1-carboxylate (654 mg) according to Example 21-(1).
¹H-NMR (400 MHz, CDCl₃) δ (ppm): 0.95-2.05 (m, 14H), 1.08 (s, 9H), 1.46 (s, 9H), 1.94-2.00 (m, 2H), 2.29 (s, 6H), 2.63-2.72 (m, 2H), 2.94-2.99 (m, 2H), 3.76-3.83 (m, 2H), 3.95 (d, J=5.6 Hz, 2H), 4.05-4.14 (m, 3H), 6.97 (d, J=8.8 Hz, 1H), 7.35-7.44 (m, 6H), 7.47 (d, J=8.8 Hz, 1H), 7.66-7.70 (m, 4H).

### (5) cis-4-{7-(N,N-Dimethylaminomethyl)-3-[2-(piperidin-4-yl)ethyl]benz[d]isoxazol-6-yloxymethyl}cyclohexanol

The title compound (91 mg) was obtained by synthesis from tert-butyl 4-{2-{6-[cis-4-(tert-butyldiphenylsilanyloxy)cyclohexylmethoxy]-7-(N,N-dimethylaminomethyl)benz[d]isoxazol-3-yl}ethyl}piperidine-1-carboxylate (302 mg) according to Example 36- (5) .
¹H-NMR (400 MHz, CDCl₃) δ (ppm): 1.19-1.28 (m, 2H), 1.43-1.53 (m, 1H), 1.57-1.98 (m, 13H), 2.33 (s, 6H), 2.57-2.63 (m, 2H), 2.93-2.97 (m, 2H), 3.08-3.12 (m, 2H), 3.80 (s, 2H), 3.93 (d, J=6.4 Hz, 2H), 4.05-4.07 (m, 1H), 6.93 (d, J=8.8 Hz, 1H), 7.46 (d, J=8.8 Hz, 1H).

### (6) 5-{4-{2-[7-(N,N-Dimethylaminomethyl)-6-(cis-4-hydroxycyclohexylmethoxy)benz[d]isoxazol-3-yl]ethyl}piperidinomethyl]thiophene-2-carbonitrile

The title compound (64 mg) was obtained by synthesis from cis-4-{7-(N,N-dimethylaminomethyl)-3-[2-(piperidin-4-yl)ethyl]benz[d]isoxazol-6-yloxymethyl}cyclohexanol (70.7 mg) and 5-thiophene-2-carbonitrile obtained in Example 155-(1) (35 mg) according to Example 21-(3).
¹H-NMR (400 MHz, CDCl₃) δ (ppm): 1.24-1.36 (m, 3H), 1.60-1.82 (m, 12H), 1.92-1.99 (m, 1H), 2.00-2.05 (m, 2H), 2.33 (s, 6H), 2.89-2.97 (m, 4H), 3.69 (s, 2H), 3.80 (s, 2H), 3.94 (d, J=6.4 Hz, 1H), 3.94 (m, 1H), 4.06-4.08 (m, 1H), 6.87-6.89 (m, 1H), 6.93 (d, J=8.8 Hz, 1H), 7.46 (d, J=8.8 Hz, 1H), 7.48-7.49 (m, 1H).

### (7) 5-{4-{2-[7-(N,N-Dimethylaminomethyl)-6-(cis-4-hydroxycyclohexylmethoxy)benz[d]isoxazol-3-yl]ethyl}piperidinomethyl]thiophene-2-carbonitrile dihydrochloride

The title compound (67 mg) was obtained by synthesis from 5-{4-{2-[7-(N,N-dimethylaminomethyl)-6-(cis-4-hydroxycyclohexylmethoxy)benz[d]isoxazol-3-yl]ethyl}piperidinomethyl]thiophene-2-carbonitrile (64 mg) according to Example 21-(4).
¹H-NMR (400 MHz, CD₃OD) δ (ppm): 1.50-2.00 (m, 13H), 2.10-2.14 (m, 3H), 2.94 (s, 6H), 3.01-3.08 (m, 4H), 3. 64-3. 58 (m, 2H), 3.99-4.00 (m, 1H), 4.12 (d, J=5.6 Hz, 2H), 4.61 (s, 2H), 4.64 (s, 2H), 7.27 (d, J=8.8 Hz, 1H), 7.45 (d, J=4.0 Hz, 1H), 7.80 (d, J=4.0 Hz, 1H), 7.92 (d, J=8.8 Hz, 1H).
ESI-MS m/z: 537 [M+H]⁺.

### Example 38

### N,N-Dimethyl{6-cyclopropylmethoxy-3-{2-[1-(pyridin-2-ylmethyl)piperidin-4-yl]ethyl}benz[d]isoxazol-7-yl}methylamine trihydrochloride

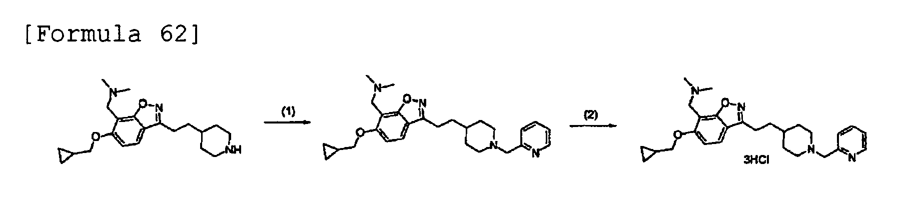

### (1) N,N-Dimethyl{6-cyclopropylmethoxy-3-{2-[1-(pyridin-2-ylmethyl)piperidin-4-yl]ethyl}benz[d]isoxazol-7-yl}methylamine

The title compound was synthesized from 2-pyridinecarbaldehyde and N,N-dimethyl{6-cyclopropylmethoxy-3-[2-(piperidin-4-yl)ethyl]benz[d]isoxazol-7-yl}methylamine according to Example 64-(1).
¹H-NMR(400 MHz, CDCl₃) δ (ppm): 0.34-0.40 (m, 2H), 0.60-0.68 (m, 2H), 1.22-1.46 (m, 4H), 1.67-1.83 (m, 4H), 1.98-2.10 (m, 2H), 2.33 (s, 6H), 2.86-2.98 (m, 4H), 3.63 (s, 2H), 3.81 (s, 2H), 3.93 (d, J=6.8 Hz, 2H), 6.87 (d, J=8.4 Hz, 1H), 7.11-7.16 (m, 1H), 7.39 (d, J=8.0 Hz, 1H) 7.43 (d, J=8.4 Hz, 1H), 7.63 (dt, J=2, 7.6 Hz, 1H), 8.52-8.56 (m, 1H).

### (2) N,N-Dimethyl{6-cyclopropylmethoxy-3-{2-[1-(pyridin-2-ylmethyl)piperidin-4-yl]ethyl}benz[d]isoxazol-7-yl}methylamine trihydrochloride

The title compound was synthesized from N,N-dimethyl{6-cyclopropylmethoxy-3-{2-[1-(pyridin-2-ylmethyl)piperidin-4-yl]ethyl}benz[d]isoxazol-7-yl}methylamine according to Example 55-(2).
¹H-NMR (400 MHz, DMSO-d₆) δ (ppm): 0.37-0.44 (m, 2H), 0.58-0.64 (m, 2H), 1.28-1.40 (m, 1H), 1.50-1.68 (m, 3H), 1.68-1.80 (m, 2H), 1.84-1.98 (m, 2H), 2.74-2.82 (m, 6H), 2.94-3.10 (m, 4H), 3.33-3.45 (m, 2H), 4.07 (d, J=7.2 Hz, 2H), 4.40-4.52 (m, 4H), 7.24 (d, J=8.8 Hz, 1H), 7.45-7.50 (m, 1H), 7.64 (d, J=7.6 Hz, 1H), 7.90-8.00 (m, 2H), 8.64-8.70 (m, 1H), 10.3 (br s, 2H).
ESI-MS m/z: 225 [M+2H ]²⁺, 449 [M+H]⁺.

### Example 39

### N,N-Dimethyl{6-cyclopropylmethoxy-3-[2-(1-benzylpiperidin-4-yl)ethyl]benz[d]isoxazol-7-yl}methylamine dihydrochloride

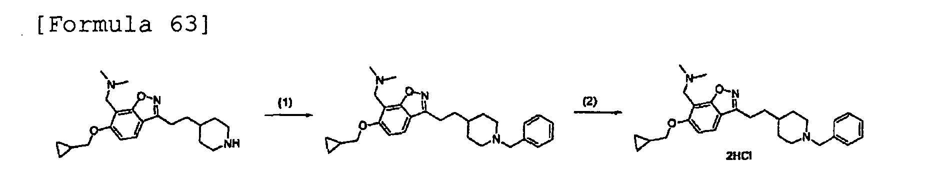

### (1) N,N-Dimethyl{6-cyclopropylmethoxy-3-[2-(1-benzylpiperidin-4-yl)ethyl]benz[d]isoxazol-7-yl}methylamine

The title compound was synthesized from benzaldehyde and N,N-dimethyl{6-cyclopropylmethoxy-3-[2-(piperidin-4-yl)ethyl]benz[d]isoxazol-7-yl}methylamine according to Example 64-(1).
¹H-NMR(400 MHz, CDCl₃) δ (ppm): 0.34-0.40 (m, 2H), 0.60-0.68 (m, 2H), 1.22-1.42 (m, 4H), 1.66-1.81 (m, 4H), 1.88-1.99 (m, 2H), 2.33 (s, 6H), 2.84-2.96 (m, 4H), 3.48 (s, 2H), 3.81 (s, 2H), 3.93 (d, J=6.8 Hz, 2H), 6.88 (d, J=8.4 Hz, 1H), 7.18-7.32 (m, 5H), 7.42 (d, J=8.4 Hz, 1H).

### (2) N,N-Dimethyl{6-cyclopropylmethoxy-3-[2-(1-benzylpiperidin-4-yl)ethyl]benz[d]isoxazol-7-yl}methylamine dihydrochloride

The title compound was synthesized from N,N-dimethyl{6-cyclopropylmethoxy-3-[2-(1-benzylpiperidin-4-yl)ethyl]benz[d]isoxazol-7-yl}methylamine according to Example 55-(2).
¹H-NMR (400 MHz, DMSO-d₆) δ (ppm): 0.36-0.44 (m, 2H), 0.57-0.66 (m, 2H), 1.24-1.40 (m, 1H), 1.44-1.64 (m, 3H), 1.64-1.80 (m, 2H), 1.82-2.00 (m, 2H), 2.68-2.94 (m, 10H), 2.94-3.04 (m, 2H), 4.06 (d, J=7.2 Hz, 2H), 4.18-4.30 (m, 2H), 4.46 (br s, 2H), 7.23 (d, J=8.8 Hz, 1H), 7.35-7.50 (m, 3H), 7.50-7.65 (m, 2H), 7.94 (d, J=8.8 Hz, 1H), 10.1 (br s, 1H), 10.4 (br s, 1H).
ESI-MS m/z: 225 [M+2H]²⁺, 448 [M+H]⁺.

### Example 40

### N,N-Dimethyl{6-cyclopropylmethoxy-3-{2-[1-(furan-2-ylmethyl)piperidin-4-yl]ethyl}benz[d]isoxazol-7-yl}methylamine dihydrochloride

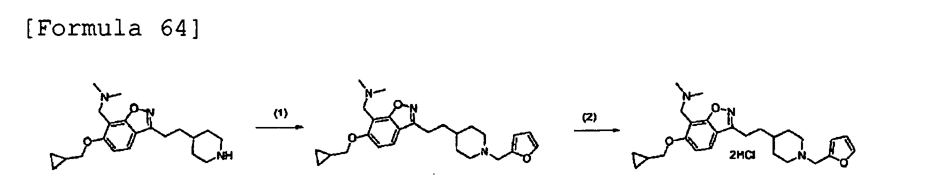

### (1) N,N-Dimethyl{6-cyclopropylmethoxy-3-{2-[1-(furan-2-ylmethyl)piperidin-4-yl]ethyl}benz[d]isoxazol-7-yl}methylamine

The title compound was synthesized from 2-furancarbaldehyde and N,N-dimethyl{6-cyclopropylmethoxy-3-[2-(piperidin-4-yl)ethyl]benz[d]isoxazol-7-yl}methylamine according to Example 64-(1).
¹H-NMR (400 MHz, CDCl₃) δ (ppm): 0.34-0.4 (m, 2H), 0.60-0.68 (m, 2H), 1.22-1.43 (m, 4H), 1.67-1.85 (m, 4H), 1.90-2.04 (m, 2H), 2.33 (s, 6H), 2.86-2.96 (m, 4H), 3.51 (s, 2H), 3.81 (s, 2H), 3.93 (d, J=6.8 Hz, 2H), 6.17 (d, J=3.2 Hz, 1H), 6.30 (dd, J=2.0, 3,2 Hz, 1H), 6.88 (d, J=8.8 Hz, 1H), 7.36 (dd, J=0.8, 2.0 Hz, 1H), 7.42 (d, J=8.6 Hz, 1H).

### (2) N,N-Dimethyl{6-cyclopropylmethoxy-3-{2-[1-(furan-2-ylmethyl)piperidin-4-yl]ethyl}benz[d]isoxazol-7-yl}methylamine dihydrochloride

The title compound was synthesized from N,N-dimethyl{6-cyclopropylmethoxy-3-{2-[1-(furan-2-ylmethyl)piperidin-4-yl]ethyl}benz[d]isoxazol-7-yl}methylamine according to Example 55-(2).
¹H-NMR(400 MHz, DMSO-d₆) δ (ppm): 0.36-0.44 (m, 2H), 0.56-0.64 (m, 2H), 1.27-1.40 (m, 1H), 1.40-1.62 (m, 3H), 1.64-1.82 (m, 2H), 1.82-2.02 (m, 2H), 2.74-2.92 (m, 10H), 2.94-3.04 (m, 2H), 4.07 (d, J=7.2 Hz, 2H), 4.30-4.40 (m, 2H), 4.47 (br s, 2H), 6.56 (dd, J=1.6, 3.2 Hz, 1H), 6.72 (d, J=3.2 Hz, 1H), 7.24 (d, J=8.8 Hz, 1H), 7.81 (s, 1H), 7.94 (d, J=8.8 Hz, 1H), 10.2 (br s, 1H), 10.4 (br s, 1H).
ESI-MS m/z: 220 [M+2H]²⁺, 438 [M+H]⁺.

### Example 41

### N,N-Dimethyl{6-cyclopropylmethoxy-3-{2-[1-(1,3-dioxolan-2-ylmethyl)piperidin-4-yl]ethyl}benz[d]isoxazol-7-yl}methylamine difumarate

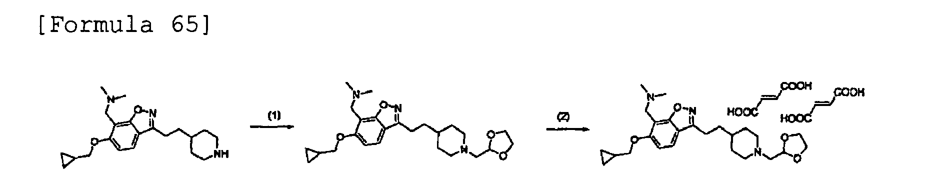

### (1) N,N-Dimethyl{6-cyclopropylmethoxy-3-{2-[1-(1,3-dioxolan-2-ylmethyl)piperidin-4-yl]ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine (100 mg), triethylamine (59 µL) and 2-bromomethyl-1,3-dioxolane (35 µL) were dissolved in acetonitrile (3 mL). The solution was stirred in a nitrogen atmosphere at 65°C for four hours. Sodium iodide (30 mg) was added to the reaction mixture, and the mixture was further stirred with heating under reflux for 13 hours. 2-Bromomethyl-1,3-dioxolane (14 µL) and triethylamine (59 µL) were further added, and the mixture was further stirred with heating under reflux for six hours. The reaction mixture was cooled to room temperature. Then, ethyl acetate and a saturated sodium chloride solution were added to the reaction mixture, and the organic layer was separated. The organic layer was dried over anhydrous magnesium sulfate. The drying agent was removed by filtration and then the filtrate was concentrated under reduced pressure. The residue was subjected to silica gel column chromatography (NH silica gel, heptane-ethyl acetate) to obtain the title compound (46 mg).
¹H-NMR(400 MHz, CDCl₃) δ (ppm): 0.34-0.4 (m, 2H), 0.60-0.68 (m, 2H), 1.22-1.44 (m, 4H), 1.67-1.82 (m, 4H), 1.99-2.12 (m, 2H), 2.33 (s, 6H), 2.57 (d, J=4.4 Hz, 2H), 2.87-3.04 (m, 4H), 3.80-3.88 (m, 2H), 3.81 (s, 2H), 3.91-4.00 (m, 4H), 4.99 (t, J=4.4 Hz, 1H), 6.88 (d, J=8.8 Hz, 1H), 7.43 (d, J=8.8 Hz, 1H).

### (2) N,N-Dimethyl{6-cyclopropylmethoxy-3-{2-[1-(1,3-dioxolan-2-ylmethyl)piperidin-4-yl]ethyl}benz[d]isoxazol-7-yl}methylamine difumarate

The title compound was synthesized from N,N-dimethyl{6-cyclopropylmethoxy-3-{2-[1-(1,3-dioxolan-2-ylmethyl)piperidin-4-yl]ethyl}benz[d]isoxazol-7-yl}methylamine according to Example 64-(2).
¹H-NMR(400 MHz, DMSO-d₆) δ (ppm): 0.32-0.38 (m, 2H), 0.54-0.61 (m, 2H), 1.12-1.33 (m, 4H), 1.61-1.73 (m, 4H), 1.94-2.05 (m, 2H), 2.22 (s, 6H), 2.46 (d, J=4.8 Hz, 2H), 2.86-2.96 (m, 4H), 3.70-3.90 (m, 6H), 3.97 (d, J=6.8 Hz, 2H), 4.87 (t, J=4.4 Hz, 1H), 6.58 (s, 4H),7.10 (d, J=8.8 Hz, 1H), 7.71 (d, J=8.8 Hz, 1H).
ESI-MS m/z: 222 [M+2H]²⁺, 444 [M+H]⁺.

### Example 42

### N,N-Dimethyl{6-cyclopropylmethoxy-3-{2-[1-(tetrahydropyran-4-ylmethyl)piperidin-4-yl]ethyl}benz[d]isoxazol-7-yl}methylamine dihydrochloride

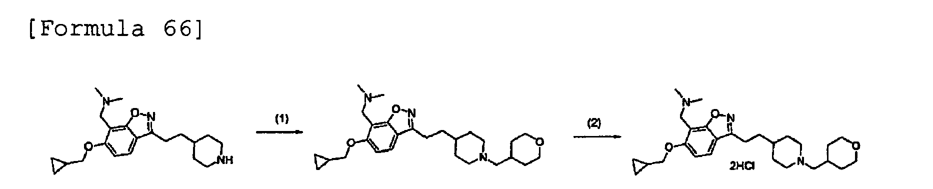

### (1) N,N-Dimethyl{6-cyclopropylmethoxy-3-{2-[1-(tetrahydropyran-4-ylmethyl)piperidin-4-yl]ethyl}benz[d]isoxazol-7-yl}methylamine

The title compound was synthesized from tetrahydropyran-4-carbaldehyde and N,N-dimethyl{6-cyclopropylmethoxy-3-[2-(piperidin-4-yl)ethyl]benz[d]isoxazol-7-yl}methylamine according to Example 64-(1).
¹H-NMR(400 MHz, CDCl₃) δ (ppm): 0.34-0.4 (m, 2H), 0.60-0.68 (m, 2H), 1.18-1.41 (m, 6H), 1.53-1.80 (m, 7H), 1.81-1.92 (m, 2H), 2.14 (d, J=7.2 Hz, 2H), 2.33 (s, 6H), 2.80-2.88 (m, 2H), 2.90-2.98 (m, 2H), 3.33-3.42 (m, 2H), 3.81 (s, 2H), 3.91-3.99 (m, 2H), 3.94 (d, J=6.4 Hz, 2H), 6.88 (d, J=8.8 Hz, 1H), 7.43 (d, J=8.8 Hz, 1H).

### (2) N,N-Dimethyl{6-cyclopropylmethoxy-3-{2-[1-(tetrahydropyran-4-ylmethyl)piperidin-4-yl]ethyl}benz[d]isoxazol-7-yl}methylamine dihydrochloride

The title compound was synthesized from N,N-dimethyl{6-cyclopropylmethoxy-3-{2-[1-(tetrahydropyran-4-ylmethyl)piperidin-4-yl]ethyl}benz[d]isoxazol-7-yl}methylamine according to Example 55-(2).
¹H-NMR (400 MHz, DMSO-d₆) δ (ppm): 0.36-0.44 (m, 2H), 0.58-0.66 (m, 2H), 1.14-2.16 (m, 15H), 2.74-3.08 (m, 12H), 3.44-3.54 (m, 2H), 3.80-3.88 (m, 2H), 4.07 (d, J=7.2 Hz, 2H), 4.47 (br s, 2H), 7.25 (d, J=8.8 Hz, 1H), 7.95 (d, J=8.8 Hz, 1H), 9.49 (br s, 1H), 10.0 (br s, 1H).
ESI-MS m/z: 223 [M+2H]²⁺, 456 [M+H]⁺.

### Example 43

### N,N-Dimethyl{6-cyclopropylmethoxy-3-[2-(1-cyclopropylmethylpiperidin-4-yl)ethyl]benz[d]isoxazol-7-yl}methylamine dihydrochloride

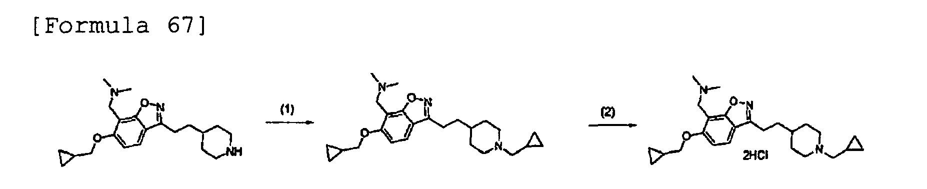

### (1) N,N-Dimethyl{6-cyclopropylmethoxy-3-[2-(1-cyclopropylmethylpiperidin-4-yl)ethyl]benz[d]isoxazol-7-yl}methylamine

The title compound was synthesized from cyclopropanecarbaldehyde and N,N-dimethyl{6-cyclopropylmethoxy-3-[2-(piperidin-4-yl)ethyl]benz[d]isoxazol-7-yl}methylamine according to Example 64-(1).
¹H-NMR (400 MHz, CDCl₃) δ (ppm): 0.06-0.12 (m, 2H), 0.34-0.41 (m, 2H), 0.46-0.54 (m, 2H), 0.60-0.68 (m, 2H), 0.80-0.93 (m, 1H), 1.22-1.43 (m, 4H), 1.70-1.85 (m, 4H), 1.85-1.98 (m, 2H), 2.22 (d, J=6.4 Hz, 2H), 2.33 (s, 6H), 2.89-2.99 (m, 2H), 3.00-3.12 (m, 2H), 3.81 (s, 2H), 3.94 (d, J=6.8 Hz, 2H), 6.89 (d, J=8.6 Hz, 1H), 7.43 (d, J=8.6 Hz, 1H).

### (2) N,N-Dimethyl{6-cyclopropylmethoxy-3-[2-(1-cyclopropylmethylpiperidin-4-yl)ethyl]benz[d]isoxazol-7-yl}methylamine dihydrochloride

The title compound was synthesized from N,N-dimethyl{6-cyclopropylmethoxy-3-[2-(1-cyclopropylmethylpiperidin-4-yl)ethyl]benz[d]isoxazol-7-yl}methylamine according to Example 55-(2).
ESI-MS m/z: 207 [M+2H]²⁺, 412 [M+H]⁺.

### Example 44

### N,N-Dimethyl{6-cyclopropylmethoxy-3-[2-(1-methylpiperidin-4-yl)ethyl]benz[d]isoxazol-7-yl}methylamine dihydrochloride

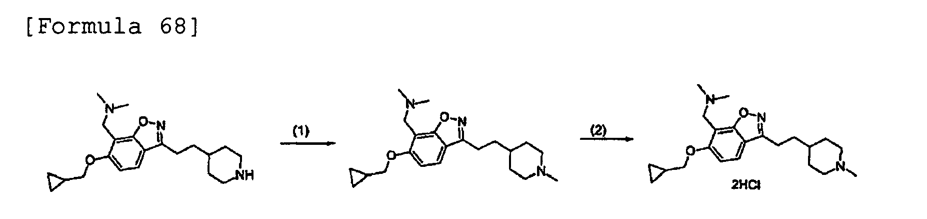

### (1) N,N-Dimethyl{6-cyclopropylmethoxy-3-[2-(1-methylpiperidin-4-yl)ethyl]benz[d]isoxazol-7-yl}methylamine

The title compound was synthesized from N,N-dimethyl{6-cyclopropylmethoxy-3-[2-(piperidin-4-yl)ethyl]benz[d]isoxazol-7-yl}methylamine according to Example 77-(1).
¹H-NMR(400 MHz, CDCl₃) δ (ppm): 0.34-0.40 (m, 2H), 0.60-0.68 (m, 2H), 1.22-1.40 (m, 4H), 1.70-1.83 (m, 4H), 1.83-1.96 (m, 2H), 2.25 (s, 3H), 2.33 (s, 6H), 2.78-2.88 (m, 2H), 2.90-3.00 (m, 2H), 3.82 (s, 2H), 3.94 (d, J=6.8 Hz, 2H), 6.89 (d, J=8.6 Hz, 1H), 7.43 (d, J=8.6 Hz, 1H).

### (2) N,N-Dimethyl{6-cyclopropylmethoxy-3-[2-(1-methylpiperidin-4-yl)ethyl]benz[d]isoxazol-7-yl}methylamine dihydrochloride

The title compound was synthesized from N,N-dimethyl{6-cyclopropylmethoxy-3-[2-(1-methylpiperidin-4-yl)ethyl]benz[d]isoxazol-7-yl}methylamine according to Example 55-(2).
ESI-MS m/z: 187 [M+2H]²⁺, 372 [M+H]²⁺.

### Example 45

### {1-{4-{2-[6-Cyclopropylmethoxy-7-(N,N-dimethylaminomethyl)benz[d]isoxazol-3-yl]ethyl}piperidinomethyl}cyclopropyl}methanol dihydrochloride

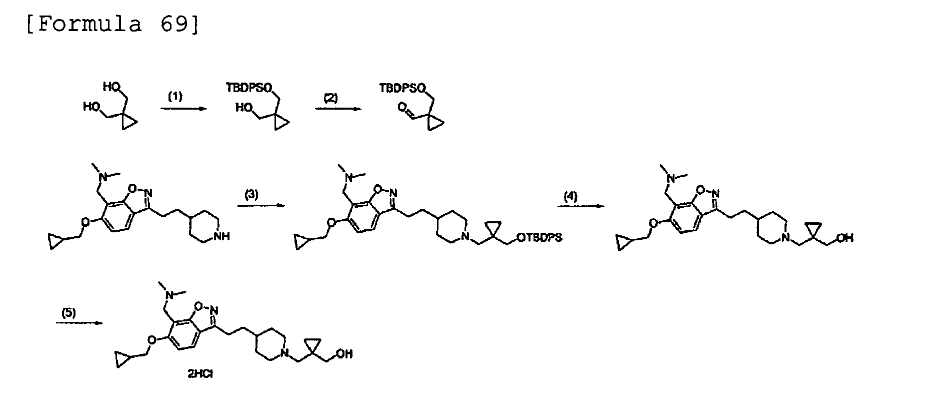

### (1) [1-(tert-Butyldiphenylsilanyloxymethyl)cyclopropyl]methanol

1,1-Bis(hydroxymethyl)cyclopropane (2 g) and imidazole (6.67 g) were dissolved in N,N-dimethylformamide (100 mL) and the solution was ice-cooled. tert-Butyldiphenylchlorosilane (5.39 g) was added dropwise to the solution. Then, the reaction mixture was gradually heated to room temperature and stirred for 18 hours. tert-Butyl methyl ether and a saturated sodium chloride solution were added to the reaction mixture, and the organic layer was separated. The organic layer was dried over anhydrous magnesium sulfate. The drying agent was removed by filtration. The filtrate was concentrated under reduced pressure to obtain a residue. The residue was subjected to silica gel column chromatography (heptane-ethyl acetate) to obtain the title compound (2.62 g).
¹H-NMR (400 MHz, CDCl₃) δ (ppm): 0.30-0.40 (m, 2H), 0.45-0.53 (m, 2H), 1.07 (s, 9H), 2.56 (t, J=5.6 Hz, 1H), 3.61 (d, J=5.6 Hz, 2H), 3.62 (s, 2H), 7.35-7.46 (m, 6H), 7.64-7.70 (m, 4H).

### (2) 1-(tert-Butyldiphenylsilanyloxymethyl)cyclopropanecarbaldehyde

[1-(tert-Butyldiphenylsilanyloxymethyl)cyclopropyl]methanol (341 mg) was dissolved in dimethyl sulfoxide (6 mL) and triethylamine (2 mL). A sulfur trioxide-pyridine complex (477 mg) was added to the solution, and then the mixture was vigorously stirred. After one hour, tert-Butyl methyl ether and a saturated sodium bicarbonate solution were added to the reaction mixture, and the organic layer was separated. The organic layer was washed with a saturated ammonium chloride solution and dried over anhydrous magnesium sulfate. The drying agent was removed by filtration. The filtrate was concentrated under reduced pressure to obtain a residue. The residue was subjected to silica gel column chromatography (heptane-ethyl acetate) to obtain the title compound (300 mg).
¹H-NMR(400 MHz, CDCl₃) δ (ppm): 1.04 (s, 9H), 1.06-1.18 (m, 4H), 3.94 (s, 2H), 7.34-7.46 (m, 6H), 7.60-7.68 (m, 4H), 9.09 (s, 1H).

### (3) N,N-Dimethyl{3-{2-{1-[1-(tert-butyldiphenylsilanyloxymethyl)cyclopropylmethyl]piperid in-4-yl}ethyl}-6-cyclopropylmethoxybenz[d]isoxazol-7-yl}methylamine

The title compound was synthesized from 1-(tert-butyldiphenylsilanyloxymethyl)cyclopropanecarbaldehyde and N,N-dimethyl{6-cyclopropylmethoxy-3-[2-(piperidin-4-yl)ethyl]benz[d]isoxazol-7-yl}methylamine according to Example 64-(1).
¹H-NMR(400 MHz, CDCl₃) δ (ppm): 0.22-0.28 (m, 2H), 0.34-0.40 (m, 2H), 0.42-0.52 (m, 2H), 0.61-0.68 (m, 2H), 1.03 (s, 9H), 1.20-1.38 (m, 4H), 1.64-1.86 (m, 6H), 2.31 (s, 2H), 2.34 (s, 6H), 2.90-2.98 (m, 4H), 3.59 (s, 2H), 3.82 (s, 2H), 3.94 (d, J=6.8 Hz, 2H), 6.89 (d, J=8.8 Hz, 1H), 7.32-7.44 (m, 6H), 7.45 (d, J=8.8 Hz, 1H), 7.62-7.73 (m, 4H).

### (4) {1-{4-{2-[6-Cyclopropylmethoxy-7-(N,N-dimethylaminomethyl)benz[d]isoxazol-3-yl]ethyl}piperidinomethyl}cyclopropyl}methanol

N,N-Dimethyl{3-{2-{1-[1-(tert-butyldiphenylsilanyloxymethyl)cyclopropylmethyl]piperid in-4-yl}ethyl}-6-cyclopropylmethoxybenz[d]isoxazol-7-yl}methylamine (109 mg) was dissolved in tetrahydrofuran (3 mL). A 1 M solution of tetrabutylammonium fluoride in tetrahydrofuran (185 µL) was added to the solution, and the mixture was stirred at room temperature. After 1.5 hours, a 1 M solution of tetrabutylammonium fluoride in tetrahydrofuran (600 µL) was added to the reaction mixture. After six hours, a 1 M solution of tetrabutylammonium fluoride in tetrahydrofuran (400 µL) was further added to the reaction mixture. After 12 hours, ethyl acetate and a saturated ammonium chloride solution were added to the reaction mixture, and the organic layer was separated. The aqueous layer was adjusted to pH 10 to 12 and extracted with chloroform. The organic layer was dried over anhydrous magnesium sulfate. The drying agent was removed by filtration. The filtrate was concentrated under reduced pressure to obtain a residue. The residue was subjected to silica gel column chromatography (NH silica gel, heptane-ethyl acetate) to obtain the title compound (44 mg).
¹H-NMR (400 MHz, CDCl₃) δ (ppm): 0.32-0.42 (m, 4H), 0.46-0.54 (m, 2H), 0.60-0.68 (m, 2H), 1.20-1.44 (m, 4H), 1.70-1.83 (m, 4H), 1.83-1.98 (m, 2H), 2.33 (s, 6H), 2.44 (s, 2H), 2.89-2.98 (m, 2H), 3.13-3.25 (m, 2H), 3.53 (s, 2H), 3.81 (s, 2H), 3.94 (d, J=6.8 Hz, 2H), 6.89 (d, J=8.8 Hz, 1H), 7.43 (d, J=8.8 Hz, 1H).

### (5) {1-{4-{2-[6-Cyclopropylmethoxy-7-(N,N-dimethylaminomethyl)benz[d]isoxazol-3-yl]ethyl}piperidinomethyl}cyclopropyl}methanol dihydrochloride

The title compound was synthesized from {1-{4-{2-[6-cyclopropylmethoxy-7-(N,N-dimethylaminomethyl)benz[d]isoxazol-3-yl]ethyl}piperidinomethyl}cyclopropyl}methanol according to Example 55-(2).
ESI-MS m/z: 222 [M+2H]²⁺, 441 [M+H]⁺,

### Example 46

### {1-{4-{2-[6-Cyclopropylmethoxy-7-(N,N-dimethylaminomethyl)benz[d]isoxazol-3-yl]ethyl}piperidinomethyl}cyclobutyl}methanol dihydrochloride

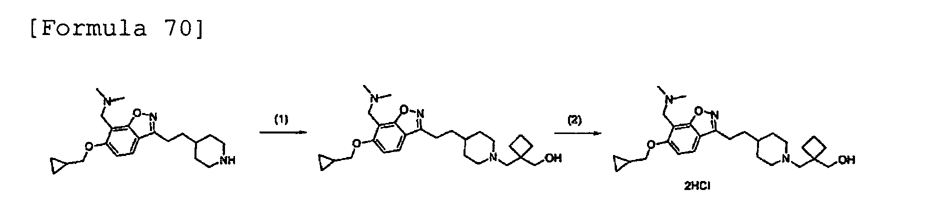

### (1) {1-{4-{2-[6-Cyclopropylmethoxy-7-(N,N-dimethylaminomethyl)benz[d]isoxazol-3-yl]ethyl}piperidinomethyl}cyclobutyl}methanol

The title compound was synthesized from 1-(tert-butyldiphenylsilanyloxymethyl)cyclobutanecarbaldehyde synthesized from (1-hydroxymethylcyclobutyl)methanol [CAS Registry No. 4415-73-0] according to Example 45-(1)(2) and N,N-dimethyl{6-cyclopropylmethoxy-3-[2-(piperidin-4-yl)ethyl]benz[d]isoxazol-7-yl}methylamine according to Example 45-(3)(4).
¹H-NMR (400 MHz, CDCl₃) δ (ppm): 0.34-0.42 (m, 2H), 0.61-0.69 (m, 2H), 1,16-1.40 (m, 4H), 1.42-2.06 (m, 12H), 2.33 (s, 6H), 2.49 (s, 2H), 2.87-2.96 (m, 4H), 3.77 (s, 2H), 3.81 (s, 2H), 3.94 (d, J=6.4 Hz, 2H), 6.89 (d, J=8.6 Hz, 1H), 7.42 (d, J=8.6 Hz, 1H).

### (2) {1-{4-{2-[6-Cyclopropylmethoxy-7-(N,N-dimethylaminomethyl)benz[d]isoxazol-3-yl]ethyl}piperidinomethyl}cyclobutyl}methanol dihydrochloride

The title compound was synthesized from {1-{4-{2-[6-cyclopropylmethoxy-7-(N,N-dimethylaminomethyl)benz[d]isoxazol-3-yl]ethyl}piperidinomethyl}cyclobutyl}methanol according to Example 55-(2).
ESI-MS m/z: 229 [M+2H]²⁺, 456 [M+H]⁺.

### Example 47

### N,N-Dimethyl{3-[2-(1-cyclopentylmethylpiperidin-4-yl)ethyl]-6-cyclopropylmethoxybenz[d]isoxazol-7-yl}methylamine dihydrochloride

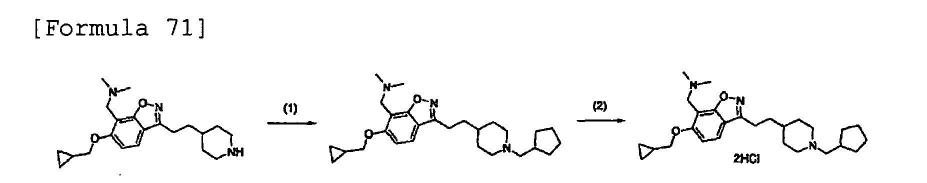

### (1) N,N-Dimethyl{3-[2-(1-cyclopentylmethylpiperidin-4-yl)ethyl]-6-cyclopropylmethoxybenz[d]isoxazol-7-yl}methylamine

The title compound was synthesized from cyclopentanecarbaldehyde and N,N-dimethyl{6-cyclopropylmethoxy-3-[2-(piperidin-4-yl)ethyl]benz[d]isoxazol-7-yl}methylamine according to Example 64-(1).
¹H-NMR(400 MHz, CDCl₃) δ (ppm): 0.34-0.40 (m, 2H), 0.59-0.68 (m, 2H), 1.10-1.22 (m, 2H), 1.23-1.41(m, 4H), 1.42-1.65 (m, 4H), 1.65-1.80 (m, 6H), 1.80-1.93 (m, 2H), 1.96-2.12 (m, 1H), 2.23 (d, J=7.2 Hz, 2H), 2.33 (s, 6H), 2.84-2.98 (m, 4H), 3.81 (s, 2H), 3.94 (d, J=6. 8Hz, 2H), 6.88 (d, J=8.8 Hz, 1H), 7.43 (d, J=8.8 Hz, 1H).

### (2) N,N-Dimethyl{3-[2-(1-cyclopentylmethylpiperidin-4-yl)ethyl]-6-cyclopropylmethoxybenz[d]isoxazol-7-yl}methylamine dihydrochloride

The title compound was synthesized from N,N-dimethyl{3-[2-(1-cyclopentylmethylpiperidin-4-yl)ethyl]-6-cyclopropylmethoxybenz[d]isoxazol-7-yl}methylamine according to Example 55-(2).
ESI-MS m/z: 221 [M+2H]²⁺, 440 [M+H]⁺.

### Example 48

### 2-{4-{2-[6-Cyclopropylmethoxy-7-(N,N-dimethylaminomethyl)benz[d]isoxazol-3-yl]ethyl}piperidinomethyl}benzonitrile dihydrochloride

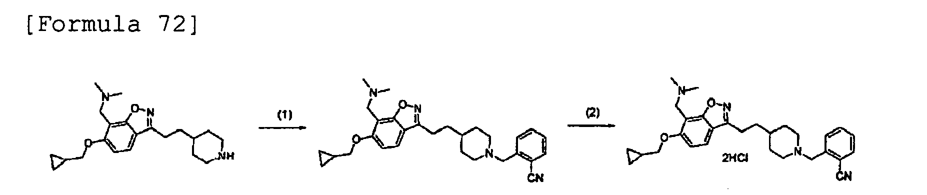

### (1) 2-{4-{2-[6-Cyclopropylmethoxy-7-(N,N-dimethylaminomethyl)benz[d]isoxazol-3-yl]ethyl}piperidinomethyl}benzonitrile

The title compound was synthesized from 2-cyanobenzaldehyde and N,N-dimethyl{6-cyclopropylmethoxy-3-[2-(piperidin-4-yl)ethyl]benz[d]isoxazol-7-yl}methylamine according to Example 64-(1).
¹H-NMR (400 MHz, CDCl₃) δ (ppm): 0.34-0.40 (m, 2H), 0.58-0.68 (m, 2H), 1.22-1.45 (m, 4H), 1.68-1.81 (m, 4H), 2.03-2.12 (m, 2H), 2.33 (s, 6H), 2.82-2.90 (m, 2H), 2. 90-2. 97 (m, 2H), 3.67 (s, 2H), 3.81 (s, 2H), 3.94 (d, J=6.8 Hz, 2H), 6.89 (d, J=8.6 Hz, 1H), 7.29-7.36 (m, 1H), 7.43 (d, J=8.6 Hz, 1H), 7.50-7.57 (m, 2H), 7.60-7.64 (m, 1H).

### (2) 2-{4-{2-[6-Cyclopropylmethoxy-7-(N,N-dimethylaminomethyl)benz[d]isoxazol-3-yl]ethyl}piperidinomethyl}benzonitrile dihydrochloride

The title compound was synthesized from {2-{4-{2-[6-cyclopropylmethoxy-7-(N,N-dimethylaminomethyl)benz[d]isoxazol-3-yl]ethyl}piperidinomethyl}benzonitrile according to Example 55- (2) .
ESI-MS m/z: 237 [M+2H]²⁺, 473 [M+H]⁺.

### Example 49

### N,N-Dimethyl{6-cyclopropylmethoxy-3-{2-[1-(thiophen-2-ylmethyl)piperidin-4-yl]ethyl}benz[d]isoxazol-7-yl}methylamine dihydrochloride

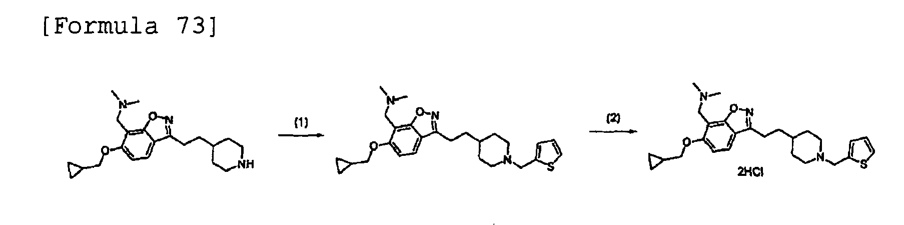

### (1) N,N-Dimethyl{6-cyclopropylmethoxy-3-{2-[1-(thiophen-2-ylmethyl)piperidin-4-yl]ethyl}benz[d]isoxazol-7-yl}methylamine

The title compound was synthesized from thiophen-2-carbaldehyde and N,N-dimethyl{6-cyclopropylmethoxy-3-[2-(piperidin-4-yl)ethyl]benz[d]isoxazol-7-yl}methylamine according to Example 64-(1).
¹H-NMR(400 MHz, CDCl₃) δ (ppm): 0.34-0.40 (m, 2H), 0.60-0.68 (m, 2H), 1.24-1.40 (m, 4H), 1.68-1.82 (m, 4H), 1.92-2.02 (m, 2H), 2.33 (s, 6H), 2.88-2.97 (m, 4H), 3.70 (s, 2H), 3.81 (s, 2H), 3.93 (d, J=6.4 Hz, 2H), 6.86-6.91 (m, 1H), 6.88 (d, J=8.8 Hz, 1H), 6.93 (dd, J=3.6, 5.2 Hz, 1H), 7.20 (dd, J=1.2, 5.2 Hz, 1H), 7.42 (d, J=8.8 Hz, 1H).

### (2) N,N-Dimethyl{6-cyclopropylmethoxy-3-{2-[1-(thiophen-2-ylmethyl)piperidin-4-yl]ethyl}benz[d]isoxazol-7-yl}methylamine dihydrochloride

The title compound was synthesized from N,N-dimethyl{6-cyclopropylmethoxy-3-{2-[1-(thiophen-2-ylmethyl)piperidin-4-yl]ethyl}benz[d]isoxazol-7-yl}methylamine according to Example 55-(2).
ESI-MS m/z: 228 [M+2H]²⁺, 454 [M+H]⁺.

### Example 50

### N,N-Dimethyl{6-cyclopropylmethoxy-3-{2-[1-(3-fluoropyridin-2-ylmethyl)piperidin-4-yl]ethyl}benz[d]isoxazol-7-yl}methylamine trihydrochloride

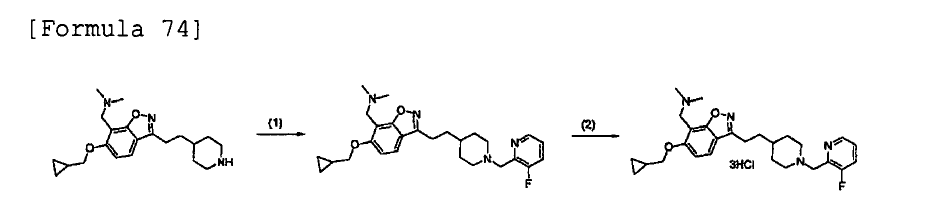

### (1) N,N-Dimethyl{6-cyclopropylmethoxy-3-{2-[1-(3-fluoropyridin-2-ylmethyl)piperidin-4-yl]ethyl}benz[d]isoxazol-7-yl}methylamine

The title compound was synthesized from 3-fluoropyridine-2-carbaldehyde [CAS Registry No. 31224-43-8] and N,N-dimethyl{6-cyclopropylmethoxy-3-[2-(piperidin-4-yl)ethyl]benz[d]isoxazol-7-yl}methylamine according to Example 64-(1).
¹H-NMR (400 MHz, CDCl₃) δ (ppm): 0.34-0.40 (m, 2H), 0.60-0.68 (m, 2H), 1.24-1.44 (m, 4H), 1.66-1.82 (m, 4H), 2.02-2.14 (m, 2H), 2.33 (s, 6H), 2.88-3.00 (m, 4H), 3.73 (d, J=2.8 Hz, 2H), 3.81 (s, 2H), 3.93 (d, J=6.8 Hz, 2H), 6.88 (d, J=8.4 Hz, 1H), 7.17-7.23 (m, 1H), 7.32-7.39 (m, 1H), 7.42 (d, J=8.4 Hz, 1H), 8.38-8.44 (m, 1H).

### (2) N,N-Dimethyl{6-cyclopropylmethoxy-3-{2-[1-(3-fluoropyridin-2-ylmethyl)piperidin-4-yl]ethyl}benz[d]isoxazol-7-yl}methylamine trihydrochloride

The title compound was obtained from N,N-dimethyl{6-cyclopropylmethoxy-3-{2-[1-(3-fluoropyridin-2-ylmethyl)piperidin-4-yl]ethyl}benz[d]isoxazol-7-yl}methylamine according to Example 55-(2).
ESI-MS m/z: 234 [M+2H]²⁺, 467 [M+H]⁺.

### Example 51

### {1-{4-{2-[6-Cyclopropylmethoxy-7-(N,N-dimethylaminomethyl)benz[d]isoxazol-3-yl]ethyl}piperidinomethyl}cyclopentyl}methanol dihydrochloride

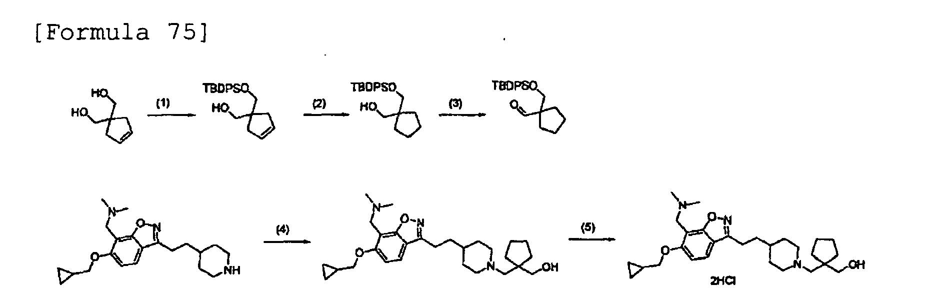

### (1) [1-(tert-Butyldiphenylsilanyloxymethyl)cyclopent-3-enyl]methanol

The title compound was synthesized from (1-hydroxymethylcyclopent-3-enyl)methanol [CAS Registry No. 76910-02-6] according to Example 45-(1).
¹H-NMR(400 MHz, CDCl₃) δ (ppm): 1.07 (s, 9H), 2.10-2.28 (m, 4H), 2.45 (t, J=6.0 Hz, 1H), 3.66 (s, 2H), 3.66 (d, J=6.0 Hz, 2H), 5.56 (s, 2H), 7. 33-7.46 (m, 6H), 7.62-7.72 (m, 4H).

### (2) [1-(tert-Butyldiphenylsilanyloxymethyl)cyclopentyl]methanol

[1-(tert-Butyldiphenylsilanyloxymethyl)cyclopent-3-enyl]methanol (5.29 g) was dissolved in methanol (80 mL). 10% palladium-carbon (50% wet) (500 mg) was added to the solution, and the mixture was vigorously stirred in a hydrogen atmosphere. After 21 hours, the reaction mixture was filtered. The filtrate was concentrated under reduced pressure to obtain a residue. The residue was subjected to silica gel column chromatography (heptane-ethyl acetate) to obtain the title compound (4.63 g). ¹H-NMR(400 MHz, CDCl₃) δ (ppm): 1.06 (s, 9H), 1.32-1.64 (m, 8H), 2.68 (t, J=6.0 Hz, 1H), 3.56 (s, 2H), 3.59 (d, J=6.0 Hz, 2H), 7.35-7.46 (m, 6H), 7.63-7.72 (m, 4H).

### (3) 1-(tert-Butyldiphenylsilanyloxymethyl)cyclopentanecarbaldehyde

The title compound was synthesized from [1-(tert-butyldiphenylsilanyloxymethyl)cyclopentyl]methanol according to Example 45-(2).
¹H-NMR (400 MHz, CDCl₃) δ (ppm): 1.03 (s, 9H), 1.44-1.64 (m, 6H), 1.87-1,99 (m, 2H), 3.72 (s, 2H), 7.34-7.46 (m, 6H), 7.59-7.66 (m, 4H), 9.65 (s, 1H).

### (4) {1-{4-{2-[6-Cyclopropylmethoxy-7-(N,N-dimethylaminomethyl)benz[d]isoxazol-3-yl]ethyl}piperidinomethyl}cyclopentyl}methanol

The title compound was synthesized from 1-(tert-butyldiphenylsilanyloxymethyl)cyclopentanecarbaldehyde and N,N-dimethyl{6-cyclopropylmethoxy-3-[2-(piperidin-4-yl)ethyl]benz[d]isoxazol-7-yl}methylamine according to Example 45-(3)(4).
¹H-NMR ( 400 MHz, CDCl₃) δ (ppm): 0.34-0.40 (m, 2H), 0.60-0.68 (m, 2H), 1,16-1.40 (m, 6H), 1.48-1.80(m, 10H), 1.99-2.10 (m, 2H), 2.33 (s, 6H), 2.49 (s, 2H), 2.88-2.96 (m, 2H), 2.96-3.04 (m, 2H), 3.49 (s, 2H), 3.81 (s, 2H), 3.94 (d, J=6.8 Hz, 2H), 6.89 (d, J=8.8 Hz, 1H), 7.42 (d, J=8.8 Hz, 1H).

### (5) {1-{4-{2-[6-Cyclopropylmethoxy-7-(N,N-dimethylaminomethyl)benz[d]isoxazol-3-yl]ethyl}piperidinomethyl}cyclopentyl}methanol dihydrochloride

The title compound was synthesized from {1-{4-{2-[6-cyclopropylmethoxy-7-(N,N-dimethylaminomethyl)benz[d]isoxazol-3-yl]ethyl}piperidinomethyl}cyclopentyl}methanol according to Example 55-(2).
ESI-MS m/z: 236 [M+2H]²⁺, 470 [M+H]⁺.

### Example 52

### {2-{4-{2-[6-Cyclopropylmethoxy-7-(N,N-dimethylaminomethyl)benz[d]isoxazol-3-yl]ethyl}piperidinomethyl}phenyl}methanol dihydrochloride

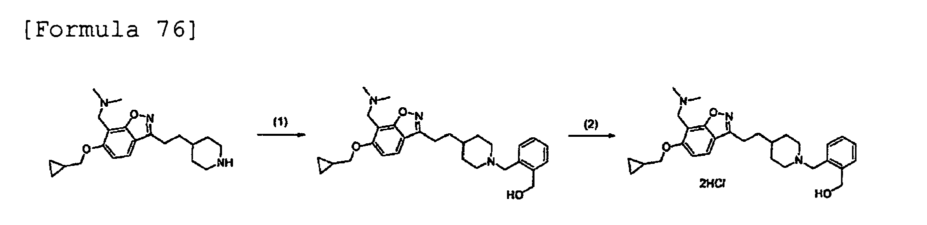

### (1) {2-{4-{2-[6-Cyclopropylmethoxy-7-(N,N-dimethylaminomethyl)benz[d]isoxazol-3-yl]ethyl}piperidinomethyl}phenyl}methanol

The title compound was synthesized from 2-(tert-butyldiphenylsilanyloxymethyl)benzaldehyde synthesized from 1,2-benzenedimethanol according to Example 45-(1)(2) and N,N-dimethyl{6-cyclopropylmethoxy-3-[2-(piperidin-4-yl)ethyl]benz[d]isoxazol-7-yl}methylamine according to Example 45- (3) (4) .
¹H-NMR(400 MHz, CDCl₃) δ (ppm): 0.34-0.40 (m, 2H), 0.60-0.68 (m, 2H), 1.14-1.48 (m, 4H), 1.68-1.80(m, 4H), 1.94-2.09 (m, 2H), 2.33 (s, 6H), 2.86-2.98 (m, 4H), 3.57 (s, 2H), 3.81 (s, 2H), 3.93 (d, J=7.2 Hz, 2H), 4.59 (s, 2H), 6.88 (d, J=8.8 Hz, 1H), 7.16-7.36 (m, 4H), 7.40 (d, J=8.8 Hz, 1H).

### (2) {2-{4-{2-[6-Cyclopropylmethoxy-7-(N,N-dimethylaminomethyl)benz[d]isoxazol-3-yl]ethyl}piperidinomethyl}phenyl}methanol dihydrochloride

The title compound was synthesized from {2-{4-{2-[6-cyclopropylmethoxy-7-(N,N-dimethylaminomethyl)benz[d]isoxazol-3-yl]ethyl}piperidinomethyl}phenyl}methanol according to Example 55-(2).
ESI-MS m/z: 240 [M+2H]²⁺, 478 [M+H]⁺.

### Example 53

### N,N-Dimethyl{3-{2-[1-(1,3-dioxolan-2-ylmethyl)piperidin-4-yl]ethyl}-6-(4-fluorophenoxy)benz[d]isoxazol-7-yl}methylamine fumarate

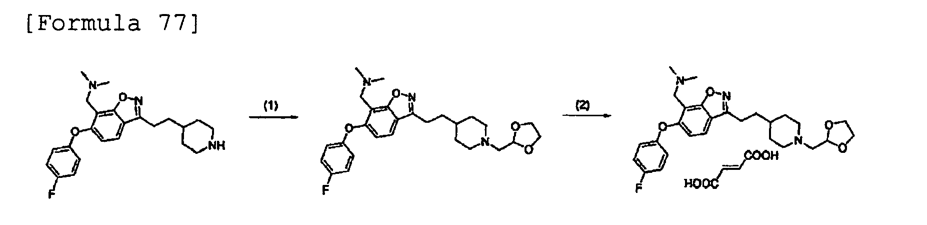

### (1) N,N-Dimethyl{3-{2-[1-(1,3-dioxolan-2-ylmethyl)piperidin-4-yl]ethyl}-6-(4-fluorophenoxy)benz[d]isoxazol-7-yl}methylamine

The title compound was synthesized according to Example 41-(1) from N,N-dimethyl{6-(4-fluorophenoxy)-3-[2-(piperidin-4-yl)ethyl]benz[d]isoxazol-7-ylmethyl}amine synthesized according to Example 153-(1) to (4).
¹H-NMR (400 MHz, CDCl₃) δ (ppm): 1.30-1.44 (m, 3H), 1.68-1.82 (m, 4H), 2.00-2.12 (m, 2H), 2.34 (s, 6H), 2.56 (d, J=4.4 Hz, 2H), 2.90-3.04 (m, 4H), 3.83 (s, 2H), 3.80-4.00 (m, 4H), 4.99 (t, J=4.4 Hz, 1H), 6.80 (d, J=8.6 Hz, 1H), 6.92-7.06 (m, 4H), 7.42 (d, J=8.6 Hz, 1H).

### (2) N,N-Dimethyl{3-{2-[1-(1,3-dioxolan-2-ylmethyl)piperidin-4-yl]ethyl}-6-(4-fluorophenoxy)benz[d]isoxazol-7-yl}methylamine fumarate

The title compound was synthesized according to Example 64-(2).
¹H-NMR (400 MHz, DMSO-d₆) δ (ppm): 1.08-1.35 (m, 3H), 1.62-1.74 (m, 4H), 1.94-2.06 (m, 2H), 2.19 (s, 6H), 2.46 (d, J=4.8 Hz, 2H), 2.86-3.00 (m, 4H), 3.68-3.88 (m, 6H), 4.87 (t, J=4.4 Hz, 1H), 6.59 (s, 2H), 6.85 (d, J=8.6 Hz, 1H), 7.02-7.09 (m, 2H), 7.18-7.26 (m, 2H), 7.75 (d, J=8.6 Hz, 1H).
ESI-MS m/z: 243 [M+2H]²⁺, 484 [M+H]⁺.

### Example 54

### {1-{4-{2-[7-(N,N-Dimethylaminomethyl)-6-(4-fluorophenoxy)benz[d]isoxazol-3-yl]ethyl}piperidinomethyl}cyclopropyl}methanol dihydrochloride

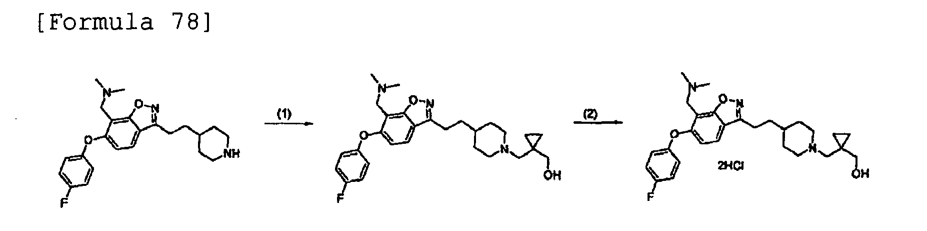

### (1) {1-{4-{2-[7-(N,N-Dimethylaminomethyl)-6-(4-fluorophenoxy)benz[d]isoxazol-3-yl]ethyl}piperidinomethyl}cyclopropyl}methanol

The title compound was synthesized according to Example 45-(3)(4) from N,N-dimethyl{6-(4-fluorophenoxy)-3-[2-(piperidin-4-yl)ethyl]benz[d]isoxazol-7-yl}methylamine synthesized according to Example 153-(1) to (4).
¹H-NMR (400 MHz, CDCl₃) δ (ppm): 0.30-0.37 (m, 2H), 0.44-0.52 (m, 2H), 1.18-1.50 (m, 3H), 1.70-1.83 (m, 4H), 1.88-1.96 (m, 2H), 2.34 (s, 6H), 2.45 (s, 2H), 2.89-3.00 (m, 2H), 3.12-3.26 (m, 2H), 3.53 (s, 2H), 3.82 (s, 2H), 6.80 (d, J=8.6 Hz, 1H), 6.92-7.07 (m, 4H), 7.42 (d, J=8.6 Hz, 1H).

### (2) {1-{4-{2-[7-(N,N-Dimethylaminomethyl)-6-(4-fluorophenoxy)benz[d]isoxazol-3-yl]ethyl}piperidinomethyl}cyclopropyl}methanol dihydrochloride

The title compound was synthesized from {1-{4-{2-[7-(N,N-dimethylaminomethyl)-6-(4-fluorophenoxy)benz[d]isoxazol-3-yl]ethyl}piperidinomethyl}cyclopropyl}methanol according to Example 55-(2).
ESI-MS m/z: 242 [M+2H]²⁺, 482 [M+H]⁺.

### Example 55

### N,N-Dimethyl{3-[2-(1-benzylpiperidin-4-yl)ethyl]-6-(4-fluorophenoxy)benz[d]isoxazol-7-yl}methylamine dihydrochloride

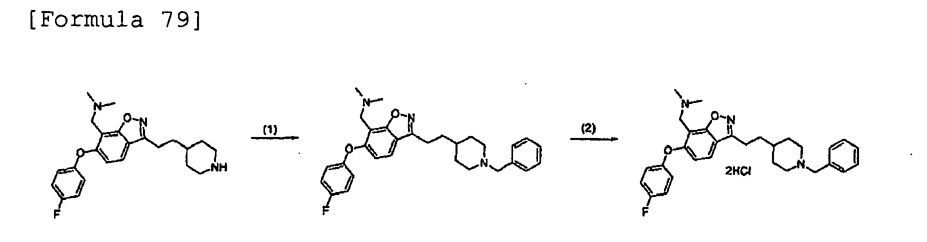

### (1) N,N-Dimethyl{3-[2-(1-benzylpiperidin-4-yl)ethyl]-6-(4-fluorophenoxy)benz[d]isoxazol-7-yl}methylamine

The title compound was synthesized from N,N-dimethyl{6-(4-fluorophenoxy)-3-[2-(piperidin-4-yl)ethyl]benz[d]isoxazol-7-yl}methylamine synthesized according to Example 153-(1) to (4) and benzaldehyde according to Example 64-(1).
¹H-NMR (400 MHz, CDCl₃) δ (ppm): 1.23-1.42 (m, 3H), 1.70-1.81 (m, 4H), 1.88-2.00 (m, 2H), 2.34 (s, 6H), 2.84-3.00 (m, 4H), 3.48 (s, 2H), 3.82 (s, 2H), 6.80 (d, J=8.6 Hz, 1H), 6.92-7.06 (m, 4H), 7.19-7.32 (m, 5H), 7.41 (d, J=8.6 Hz, 1H).

### (2) N,N-Dimethyl{3-[2-(1-benzylpiperidin-4-yl)ethyl]-6-(4-fluorophenoxy)benz[d]isoxazol-7-yl}methylamine dihydrochloride

N,N-Dimethyl{3-[2-(1-benzylpiperidin-4-yl)ethyl]-6-(4-fluorophenoxy)benz[d]isoxazol-7-yl}methylamine (54 mg) was dissolved in methanol (3 mL). A 4 M solution of hydrogen chloride in ethyl acetate (139 µL) was dissolved in the solution, followed by concentration. Diethyl ether (5 mL) was added to the mixture, and the mixture was concentrated again. Diethyl ether (3 mL) was added to the generated solid. The solid was ground and concentrated to dryness again to obtain the title compound (63 mg).
ESI-MS m/z: 245 [M+2H]²⁺, 488 [M+H]⁺.

### Example 56

### N,N-Dimethyl{6-cyclopropylmethoxy-3-{2-[1-(5-methylisoxazol-3-ylmethyl)piperidin-4-yl]ethyl}benz[d]isoxazol-7-yl}methylamine fumarate

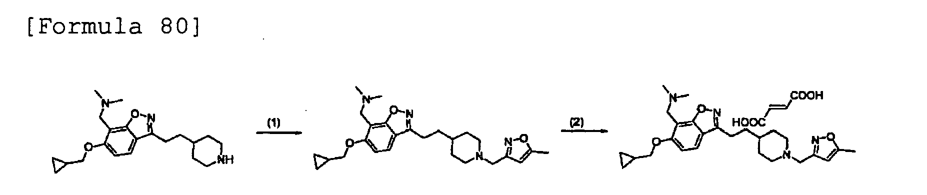

### (1) N,N-Dimethyl{6-cyclopropylmethoxy-3-{2-[1-(5-methylisoxazol-3-ylmethyl)piperidin-4-yl]ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine (120 mg), N,N-diisopropylethylamine (158 µL) and 3-(chloromethyl)-5-methylisoxazole (53 mg) were dissolved in acetonitrile (4 mL) and N,N-dimethylformamide (100 µL), and the solution was stirred at room temperature for nine hours. Ethyl acetate, a 2 M sodium hydroxide solution and a saturated sodium chloride solution were added to the reaction mixture, and the organic layer was separated. The organic layer was dried over anhydrous magnesium sulfate, and then the drying agent was removed by filtration. The filtrate was concentrated under reduced pressure to obtain a residue. The residue was subjected to silica gel column chromatography (NH silica gel, heptane-ethyl acetate) to obtain the title compound (72 mg).
¹H-NMR (400 MHz, CDCl₃) δ (ppm): 0.34-0.40 (m, 2H), 0.60-0.68 (m, 2H), 1.22-1.42 (m, 4H), 1.70-1.80 (m, 4H), 1.94-2.08 (m, 2H), 2.33 (s, 6H), 2.40 (d, J=0.8 Hz, 3H), 2.82-2.98 (m, 4H), 3.53 (s, 2H), 3.81 (s, 2H), 3.93 (d, J=6.8 Hz, 2H), 5.98 (d, J=0.8 Hz, 1H), 6.88 (d, J=8.8 Hz, 1H), 7.42 (d, J=8.8 Hz, 1H).

### (2) N,N-Dimethyl{6-cyclopropylmethoxy-3-{2-[1-(5-methylisoxazol-3-ylmethyl)piperidin-4-yl]ethyl}benz[d]isoxazol-7-yl}methylamine fumarate

The title compound was synthesized from N,N-dimethyl{6-cyclopropylmethoxy-3-{2-[1-(5-methylisoxazol-3-ylmethyl)piperidin-4-yl]ethyl}benz[d]isoxazol-7-yl}methylamine according to Example 64-(2).
¹H-NMR (400 MHz, DMSO-d₆) δ (ppm) : 0.32-0.38 (m, 2H), 0.53-0.61 (m, 2H), 1.10-1.34 (m, 4H), 1.60-1.75 (m, 4H), 1.86-1.98 (m, 2H), 2.21 (s, 6H), 2.37 (s, 3H), 2.73-2.82 (m, 2H), 2.88-2.96 (m, 2H), 3.44 (s, 2H), 3.74 (s, 2H), 3.97 (d, J=6.8 Hz, 2H), 6.14 (s, 1H), 6.59 (s, 2H), 7.10 (d, J=8.6 Hz, 1H), 7.71 (d, J=8.6 Hz, 1H).
ESI-MS m/z: 227 [M+2H]²⁺, 753 [M+H]⁺.

### Example 57

### N,N-Dimethyl{6-isobutoxy-3-{2-[1-(5-methylisoxazol-3-ylmethyl)piperidin-4-yl]ethyl}benz[d]isoxazol-7-yl}methylamine difumarate

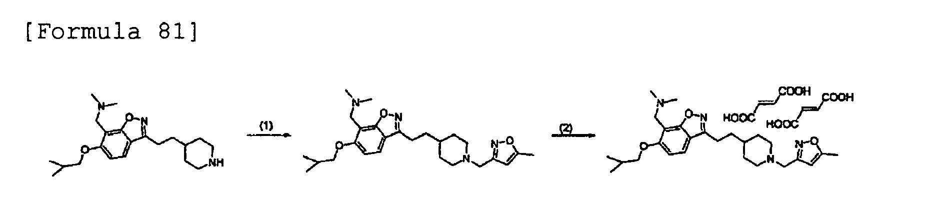

### (1) N,N-Dimethyl{6-isobutoxy-3-{2-[1-(5-methylisoxazol-3-ylmethyl)piperidin-4-yl]ethyl}benz[d]isoxazol-7-yl}methylamine

The title compound was synthesized according to Example 56-(1) from N,N-dimethyl{6-isobutoxy-3-[2-(piperidin-4-yl)ethyl]benz[d]isoxazol-7-yl}methylamine as a starting material which is synthesized according to the synthesis of N,N-dimethyl{6-cyclopropylmethoxy-3-[2-(piperidin-4-yl)ethyl]benz[d]isoxazol-7-yl}methylamine in Preparation Example 3.
¹H-NMR(400 MHz, CDCl₃) δ (ppm): 1.07 (d, J=6.8 Hz, 6H), 1.20-1.41 (m, 3H), 1.70-1.82 (m, 4H), 1.96-2.08 (m, 2H), 2.10-2.22 (m, 1H), 2.32 (s, 6H), 2.40 (d, J=0.8 Hz, 3H), 2.82-2.98 (m, 4H), 3.53 (s, 2H), 3.78 (s, 2H), 3.83 (d, J=6.4 Hz, 2H), 5.99 (d, J=0.8 Hz, 1H), 6.90 (d, J=8.8 Hz, 1H), 7.43 (d, J=8.8 Hz, 1H).

### (2) N,N-Dimethyl{6-isobutoxy-3-{2-[1-(5-methylisoxazol-3-ylmethyl)piperidin-4-yl]ethyl}benz[d]isoxazol-7-yl}methylamine difumarate

The title compound was synthesized from N,N-dimethyl{6-isobutoxy-3-{2-[1-(5-methylisoxazol-3-ylmethyl)piperidin-4-yl]ethyl}benz[d]isoxazol-7-yl}methylamine according to Example 64-(2).
¹H-NMR(400 MHz, DMSO-d₆) δ (ppm): 1.03 (d, J=6.8 Hz, 6H), 1.12-1.34 (m, 3H), 1.63-1.75 (m, 4H), 1.87-1.98 (m, 2H), 2.00-2.12 (m, 1H), 2.20 (s, 6H), 2.37 (d, J=1.0 Hz, 3H), 2.74-2.83 (m, 2H), 2.88-2.97 (m, 2H), 3.44 (s, 2H), 3.71 (s, 2H), 3.88 (d, J=6.0 Hz, 2H), 5.99 (d, J=1.0 Hz, 1H), 6.59 (s, 4H), 7.11 (d, J=8.8 Hz, 1H), 7.72 (d, J=8.8 Hz, 1H).
ESI-MS m/z: 228 [M+2H]²⁺, 455 [M+H]⁺.

### Example 58

### N,N-Dimethyl{3-{2-[1-(1,3-dioxolan-2-ylmethyl)piperidin-4-yl]ethyl}-6-isobutoxybenz[d]isoxazol-7-yl}methylamine difumarate

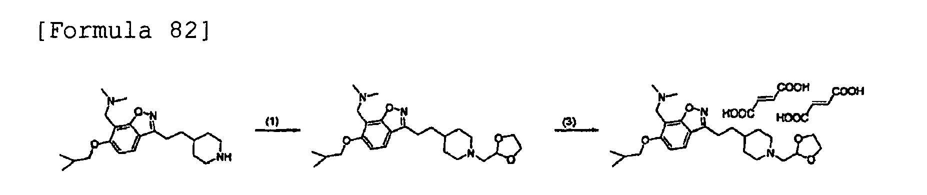

### (1) N,N-Dimethyl{3-{2-[1-(1,3-dioxolan-2-ylmethyl)piperidin-4-yl]ethyl}-6-isobutoxybenz[d]isoxazol-7-yl}methylamine

The title compound was synthesized according to Example 41-(1) from N,N-dimethyl{6-isobutoxy-3-[2-(piperidin-4-yl)ethyl]benz[d]isoxazol-7-yl}methylamine as a starting material which is synthesized according to the synthesis of N,N-dimethyl{6-cyclopropylmethoxy-3-[2-(piperidin-4-yl)ethyl]benz[d]isoxazol-7-yl}methylamine in Preparation Example 3.
¹H-NMR (400 MHz, CDCl₃) δ (ppm): 1.07 (d, J=6.8 Hz, 6H), 1.28-1.44 (m, 3H), 1.64-1.82 (m, 4H), 1.96-2.11 (m, 2H), 2.11-2.23 (m, 1H), 2.32 (s, 6H), 2.56 (d, J=4.4 Hz, 2H), 2.88-3.05 (m, 4H), 3.79 (s, 2H), 3.80-4.02 (m, 6H), 5.00 (t, J=4.4 Hz, 1H), 6.90 (d, J=8.8 Hz, 1H), 7.43 (d, J=8.8 Hz, 1H).

### (2) N,N-Dimethyl{3-{2-[1-(1,3-dioxolan-2-ylmethyl)piperidin-4-yl]ethyl}-6-isobutoxybenz[d]isoxazol-7-yl}methylamine difumarate

The title compound was synthesized from N,N-dimethyl{3-{2-[1-(1,3-dioxolan-2-ylmethyl)piperidin-4-yl]ethyl}-6-isobutoxybenz[d]isoxazol-7-yl}methylamine according to Example 64-(2).
¹H-NMR(400 MHz, DMSO-d₆) δ (ppm): 1.03 (d, J=6.4 Hz, 6H), 1.10-1.33 (m, 3H), 1.60-1.73 (m, 4H), 1.95-2.13 (m, 3H), 2.21 (s, 6H), 2.47 (d, J=4.0 Hz, 2H), 2.88-2.97 (m, 4H), 3.69-3.80 (m, 4H), 3.80-3.92 (m, 4H), 4.87 (t, J=4.0 Hz, 1H), 6.59 (s, 4H), 7.12 (d, J=8.8 Hz, 1H), 7.72 (d, J=8.8 Hz, 1H).
ESI-MS m/z: 224 [M+2H]²⁺, 446 [M+H]⁺.

### Example 59

### N,N-Dimethyl{3-[2-(1-benzylpiperidin-4-yl)ethyl]-6-isobutoxybenz[d]isoxazol-7-yl}methylamine difumarate

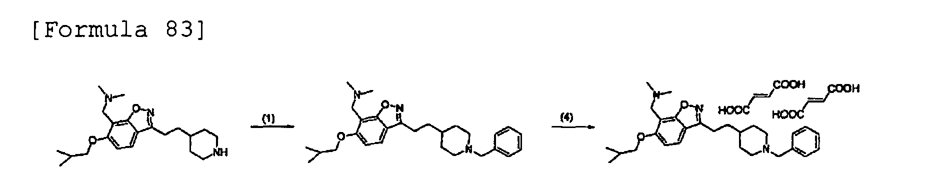

### (1) N,N-Dimethyl{3-[2-(1-benzylpiperidin-4-yl)ethyl]-6-isobutoxybenz[d]isoxazol-7-yl}methylamine

The title compound was synthesized according to Example 64-(1) from N,N-dimethyl{6-isobutoxy-3-[2-(piperidin-4-yl)ethyl]benz[d]isoxazol-7-yl}methylamine synthesized according to the synthesis of N,N-dimethyl{6-cyclopropylmethoxy-3-[2-(piperidin-4-yl)ethyl]benz[d]isoxazol-7-yl}methylamine in
Preparation Example 3 and benzaldehyde as starting materials.
¹H-NMR(400 MHz, CDCl₃) δ (ppm): 1.07 (d, J=6.8 Hz, 6H), 1.20-1.40 (m, 3H), 1.68-1.82 (m, 4H), 1.87-2.00 (m, 2H), 2.10-2.22 (m, 1H), 2.32 (s, 6H), 2.84-2.97 (m, 4H), 3.48 (s, 2H), 3.78 (s, 2H), 3.83 (d, J=6.4 Hz, 2H), 6.90 (d, J=8.6 Hz, 1H), 7.18-7.35 (m, 5H), 7.43 (d, J=8.6 Hz, 1H).

### (2) N,N-Dimethyl{3-[2-(1-benzylpiperidin-4-yl)ethyl]-6-isobutoxybenz[d]isoxazol-7-yl}methylamine difumarate

The title compound was synthesized from N,N-dimethyl{3-[2-(1-benzylpiperidin-4-yl)ethyl]-6-isobutoxybenz[d]isoxazol-7-yl}methylamine according to Example 64-(2).
¹H-NMR (400 MHz, DMSO-d₆) δ (ppm): 1.03 (d, J=6. Hz, 6H), 1.12-1.40 (m, 3H), 1.64-1.76 (m, 4H), 1.89-2.00 (m, 2H), 2.00-2.13 (m, 1H), 2.19 (s, 6H), 2.78-2.86 (m, 2H), 2. 89-2. 97 (m, 2H), 3.49 (s, 2H), 3.70 (s, 2H), 3. 87 (d, J=6.4 Hz, 2H), 6.58 (s, 4H), 7.11 (d, J=8.8 Hz, 1H), 7.20-7.34 (m, 5H), 7.71 (d, J=8.8 Hz, 1H).

### Example 60

### N,N-Dimethyl{3-{2-[1-(3-chloroisoxazol-5-ylmethyl)piperidin-4-yl]ethyl}-6-cyclopropylmethoxybenz[d]isoxazol-7-yl}methylamine difumarate

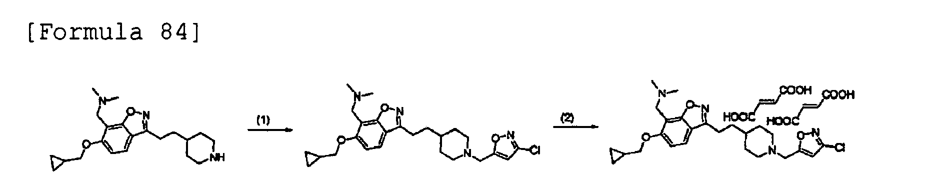

### (1) N,N-Dimethyl{3-{2-[1-(3-chloroisoxazol-5-ylmethyl)piperidin-4-yl]ethyl}-6-cyclopropylmethoxybenz[d]isoxazol-7-yl}methylamine

The title compound was synthesized from N,N-dimethyl{6-cyclopropylmethoxy-3-[2-(piperidin-4-yl)ethyl]benz[d]isoxazol-7-yl}methylamine and 5-bromomethyl-3-chloroisoxazole [CAS Registry No. 118066-90-3] according to Example 56-(1).
¹H-NMR(400 MHz, CDCl₃) δ (ppm): 0.34-0.40 (m, 2H), 0.60-0.68 (m, 2H), 1.22-1.42 (m, 4H), 1.72-1.84 (m, 4H), 2.04-2.13 (m, 2H), 2.33 (s, 6H), 2.83-2.97 (m, 4H), 3.64 (s, 2H), 3.81 (s, 2H), 3.93 (d, J=6.8 Hz, 2H), 6.20 (s, 1H), 6.89 (d, J=8.6 Hz, 1H), 7.42 (d, J=8.6 Hz, 1H).

### (2) N,N-Dimethyl{3-{2-[1-(3-chloroisoxazol-5-ylmethyl)piperidin-4-yl]ethyl}-6-cyclopropylmethoxybenz[d]isoxazol-7-yl}methylamine difumarate

The title compound was synthesized from N,N-dimethyl{3-{2-[1-(3-chloroisoxazol-5-ylmethyl)piperidin-4-yl]ethyl}-6-cyclopropylmethoxybenz[d]isoxazol-7-yl}methylamine according to Example 64-(2).
ESI-MS m/z: 237 [M+2H]²⁺, 473 [M+H]⁺.

### Example 61

### N,N-Dimethyl{6-cyclopropylmethoxy-3-{2-[1-(2-methoxypyridin-3-ylmethyl)piperidin-4-yl]ethyl}benz[d]isoxazol-7-yl}methylamine fumarate

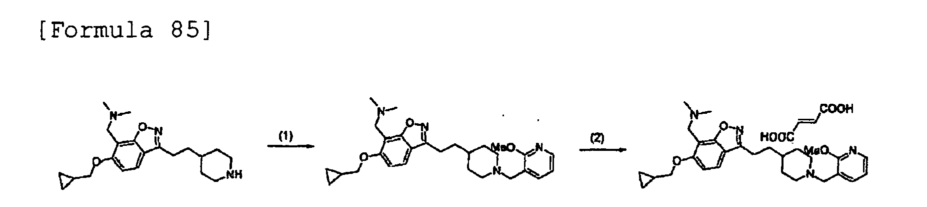

### (1) N,N-Dimethyl{6-cyclopropylmethoxy-3-{2-[1-(2-methoxypyridin-3-ylmethyl)piperidin-4-yl]ethyl}benz[d]isoxazol-7-yl}methylamine

The title compound was synthesized from N,N-dimethyl{6-cyclopropylmethoxy-3-[2-(piperidin-4-yl)ethyl]benz[d]isoxazol-7-yl}methylamine and 3-chloromethyl-2-methoxypyridine [CAS Registry No. 162046-62-0] according to Example 56-(1).
¹H-NMR (400 MHz, CDCl₃) δ (ppm): 0.34-0.40 (m, 2H), 0.60-0.68 (m, 2H), 1.22-1.44 (m, 4H), 1.69-1.84 (m, 4H), 1.96-2.09 (m, 2H), 2.33 (s, 6H), 2.84-2.98 (m, 4H), 3.47 (s, 2H), 3.82 (s, 2H), 3.93 (d, J=6.8 Hz, 2H), 3.94 (s, 3H), 6.85 (dd, J=5.2, 7.2 Hz, 1H), 6.89 (d, J=8.6 Hz, 1H), 7.43 (d, J=8.6 Hz, 1H), 7.60-7.66 (m, 1H), 8.03 (dd, J=2.0, 5.2 Hz, 1H).

### (2) N,N-Dimethyl{6-cyclopropylmethoxy-3-{2-[1-(2-methoxypyridin-3-ylmethyl)piperidin-4-yl]ethyl}benz[d]isoxazol-7-yl}methylamine fumarate

The title compound was synthesized from N,N-dimethyl{6-cyclopropylmethoxy-3-{2-[1-(2-methoxypyridin-3-ylmethyl)piperidin-4-yl]ethyl}benz[d]isoxazol-7-yl}methylamine according to Example 64-(2).
ESI-MS m/z: 240 [M+2H]²⁺, 479 [M+H]⁺.

### Example 62

### N,N-Dimethyl{6-cyclopropylmethoxy-3-{2-[1-(6-fluoropyridin-2-ylmethyl)piperidin-4-yl]ethyl}benz[d]isoxazol-7-yl}methylamine difumarate

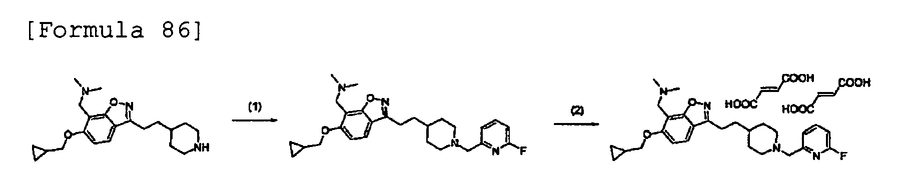

### (1) N,N-Dimethyl{6-cyclopropylmethoxy-3-{2-[1-(6-fluoropyridin-2-ylmethyl)piperidin-4-yl]ethyl}benz[d]isoxazol-7-yl}methylamine

A mixed solution of N,N-dimethyl{6-cyclopropylmethoxy-3-[2-(piperidin-4-yl)ethyl]benz[d]isoxazol-7-yl}methylamine (100 mg), 6-fluoropyridine-2-carbaldehyde [CAS Registry No. 208110-81-0] (53 mg), acetic acid (32 µL) and dichloromethane (6 mL) was stirred at room temperature for 10 minutes. Then, sodium triacetoxyborohydride (89 mg) was added and the mixture was stirred for 3.5 hours. Chloroform (15 mL), a saturated sodium bicarbonate solution (8 mL) and a 2 M sodium hydroxide solution (3 mL) were added to the reaction mixture, and the organic layer was separated. The organic layer was dried over anhydrous magnesium sulfate, and then the drying agent was removed by filtration. The filtrate was concentrated under reduced pressure to obtain a residue. The residue was subjected to silica gel column chromatography (NH silica gel, heptane-ethyl acetate) to obtain the title compound (85 mg).
¹H-NMR(400 MHz, CDCl₃) δ (ppm): 0.34-0.40 (m, 2H), 0.60-0.68 (m, 2H), 1.24-1.46 (m, 4H), 1.70-1.84 (m, 4H), 2.00-2.12 (m, 2H), 2.33 (s, 6H), 2.86-2.98 (m, 4H), 3.57 (s, 2H), 3.82 (s, 2H), 3.93 (d, J=6.8 Hz, 2H), 6.76-6.81 (m, 1H), 6.89 (d, J=8.6 Hz, 1H), 7.28-7.33 (m, 1H), 7.43 (d, J=8.6 Hz, 1H), 7.69-7.77 (m, 1H).

### (2) N,N-Dimethyl{6-cyclopropylmethoxy-3-{2-[1-(6-fluoropyridin-2-ylmethyl)piperidin-4-yl]ethyl}benz[d]isoxazol-7-yl}methylamine difumarate

N,N-Dimethyl{6-cyclopropylmethoxy-3-{2-[1-(6-fluoropyridin-2-ylmethyl)piperidin-4-yl]ethyl}benz[d]isoxazol-7-yl}methylamine (84 mg) and fumaric acid (42 mg) were dissolved in methanol (2 mL), and the solution was concentrated to dryness under reduced pressure. Addition of diethyl ether to the residue and concentration under reduced pressure were repeated to obtain the title compound (118 mg).
¹H-NMR(400 MHz, CD₃OD) δ (ppm): 0.39-0.47 (m, 2H), 0.65-0.73 (m, 2H), 1.30-1.71 (m, 4H), 1.79-1.90 (m, 2H), 1.92-2.03 (m, 2H), 2.68-2.80 (m, 2H), 2.90 (s, 6H), 3.01-3.09 (m, 2H), 3.20-3.40 (m, 2H), 4.10 (d, J=7.2 Hz, 2H), 4.13 (s, 2H), 4.55 (s, 2H), 6.67 (s, 4H), 7.06-7.12 (m, 1H), 7.21 (d, J=8.8 Hz, 1H), 7.39-7.44 (m, 1H), 7.87 (d, J=8.8 Hz, 1H), 7.95-8.04 (m, 1H).
ESI-MS m/z: 234 [M+2H]²⁺, 467 [M+H]⁺.

### Example 63

### N,N-Dimethyl{3-{2-[1-(6-chloropyridin-2-ylmethyl) piperidin-4-yl]ethyl}-6-cyclopropylmethoxybenz[d]isoxazol-7-yl}methylamine difumarate

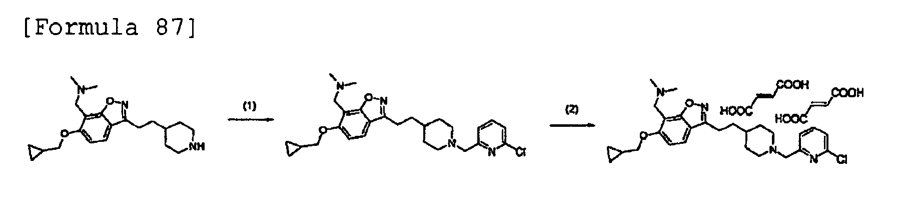

### (1) N,N-Dimethyl{3-{2-[1-(6-chloropridin-2-ylmethyl)piperidin-4-yl]ethyl}-6-cyclopropylmethoxybenz[d]isoxazol-7-yl}methylamine

A mixed solution of N,N-dimethyl{6-cyclopropylmethoxy-3-[2-(piperidin-4-yl)ethyl]benz[d]isoxazol-7-yl}methylamine (100 mg), 6-chloropyridine-2-carbaldehyde [CAS Registry No. 54087-03-5] (60 mg), acetic acid (32 µL) and dichloromethane (6 mL) was stirred at room temperature for 10 minutes. Then, sodium triacetoxyborohydride (89 mg) was added and the mixture was stirred for five hours. Chloroform (15 mL), a saturated sodium bicarbonate solution (8 mL) and a 2 M sodium hydroxide solution (3 mL) were added to the reaction mixture, and the organic layer was separated. The organic layer was dried over anhydrous magnesium sulfate, and then the drying agent was removed by filtration. The filtrate was concentrated under reduced pressure to obtain a residue. The residue was subjected to silica gel column chromatography (NH silica gel, heptane-ethyl acetate) to obtain the title compound (108 mg).
¹H-NMR (400 MHz, CDCl₃) δ (ppm): 0.34-0.40 (m, 2H), 0.60-0.68 (m, 2H), 1.24-1.45 (m, 4H), 1.70-1.83 (m, 4H), 2.00-2.12 (m, 2H), 2.33 (s, 6H), 2.84-2.98 (m, 4H), 3.61 (s, 2H), 3.81 (s, 2H), 3.94 (d, J=6.8 Hz, 2H), 6.89 (d, J=8.6 Hz, 1H), 7.16-7.20 (m, 1H), 7.37-7.41 (m, 1H), 7.43 (d, J=8.6 Hz, 1H), 7.60 (t, J=7.6 Hz, 1H).

### (2) N,N-Dimethyl{3-{2-[1-(6-chloropyridin-2-ylmethyl)piperidin-4-yl]ethyl}-6-cyclopropylmethoxybenz[d]isoxazol-7-yl}methylamine difumarate

N,N-Dimethyl{3-{2-[1-(6-chloropyridin-2-ylmethyl)piperidin-4-yl]ethyl}-6-cyclopropylmethoxybenz[d]isoxazol-7-yl}methylamine (84 mg) and fumaric acid (42 mg) were dissolved in methanol (2 mL), and the solution was concentrated to dryness under reduced pressure. Addition of diethyl ether to the residue and concentration under reduced pressure were repeated to obtain the title compound (100 mg).
ESI-MS m/z: 242 [M+2H]²⁺, 483 [M+H]⁺.

### Example 64

### N,N-Dimethyl{6-cyclopropylmethoxy-3-{2-[1-(6-methylpyridin-2-ylmethyl)piperidin-4-yl]ethyl}benz[d]isoxazol-7-yl}methylamine difumarate

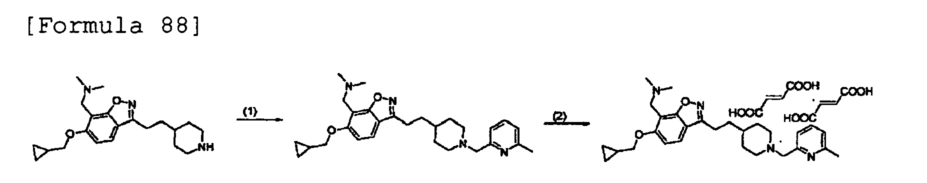

### (1) N,N-Dimethyl{6-cyclopropylmethoxy-3-{2-[1-(6-methylpyridin-2-ylmethyl)piperidin-4-yl]ethyl}benz[d]isoxazol-7-yl}methylamine

A mixed solution of N,N-dimethyl{6-cyclopropylmethoxy-3-[2-(piperidin-4-yl)ethyl]benz[d]isoxazol-7-yl}methylamine (100 mg), 6-methylpyridine-2-carbaldehyde (51 mg), acetic acid (32 µL) and dichloromethane (6 mL) was stirred at room temperature for 10 minutes. Then, sodium triacetoxyborohydride (89 mg) was added and the mixture was stirred for five hours. Chloroform (15 mL), a saturated sodium bicarbonate solution (8 mL) and a 2 M sodium hydroxide solution (3 mL) were added to the reaction mixture, and the organic layer was separated. The organic layer was dried over anhydrous magnesium sulfate, and then the drying agent was removed by filtration. The filtrate was concentrated under reduced pressure to obtain a residue. The residue was subjected to silica gel column chromatography (NH silica gel, heptane-ethyl acetate) to obtain the title compound (91 mg).
¹H-NMR (400 MHz, CDCl₃) δ (ppm): 0.34-0.40 (m, 2H), 0.60-0.68 (m, 2H), 1.24-1.44 (m, 4H), 1.68-1.82 (m, 4H), 1.97-2.00 (m, 2H), 2.33 (s, 6H), 2.54 (s, 3H), 2.87-2.97 (m, 4H), 3.60 (s, 2H), 3.81 (s, 2H), 3.93 (d, J=6.8 Hz, 2H), 6.88 (d, J=8.6 Hz, 1H), 6.97-7.02 (m, 1H), 7.20-7.24 (m, 1H), 7.43 (d, J=8.6 Hz, 1H), 7.49-7.55 (m, 1H).

### (2) N,N-Dimethyl{6-cyclopropylmethoxy-3-{2-[1-(6-methylpyridin-2-ylmethyl)piperidin-4-yl]ethyl}benz[d]isoxazol-7-yl}methylamine difumarate

N,N-Dimethyl{6-cyclopropylmethoxy-3-{2-[1-(6-methylpyridin-2-ylmethyl)piperidin-4-yl]ethyl}benz[d]isoxazol-7-yl}methylamine (84 mg) and fumaric acid (42 mg) were dissolved in methanol (2 mL), and the solution was concentrated to dryness under reduced pressure. Addition of diethyl ether to the residue and concentration under reduced pressure were repeated to obtain the title compound (93 mg).
ESI-MS m/z: 232 [M+2H]²⁺, 463 [M+H]⁺.

### Example 65

### 5-{4-{2-[6-Cyclopropylmethoxy-7-(N,N-dimethylaminomethyl)benz[d]isoxazol-3-yl]ethyl}piperidinomethyl}-1-methyl-1H-pyrrole-2-carbonitrile difumarate

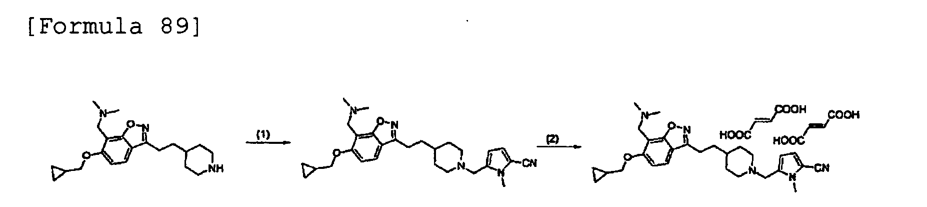

### (1) 5-{4-{2-[6-Cyclopropylmethoxy-7-(N,N-dimethylaminomethyl)benz[d]isoxazol-3-yl]ethyl}piperidinomethyl}-1-methyl-1H-pyrrole-2-carbonitrile

A mixed solution of 5-formyl-1-methyl-1H-pyrrole-2-carbonitrile synthesized according to JP-10-509340 (56 mg), N,N-dimethyl{6-cyclopropylmethoxy-3-[2-(piperidin-4-yl)ethyl]benz[d]isoxazol-7-yl}methylamine (100 mg), acetic acid (32 µL) and dichloromethane (6 mL) was stirred at room temperature for 10 minutes. Then, sodium triacetoxyborohydride (89 mg) was added and the mixture was stirred for 15.5 hours. Chloroform (10 mL), a saturated sodium bicarbonate solution (8 mL) and a 2 M sodium hydroxide solution (3 mL) were added to the reaction mixture, and the organic layer was separated. The organic layer was dried over anhydrous magnesium sulfate, and then the drying agent was removed by filtration. The filtrate was concentrated under reduced pressure to obtain a residue. The residue was subjected to silica gel column chromatography (NH silica gel, heptane-ethyl acetate) to obtain the title compound (47 mg).
¹H-NMR(400 MHz, CDCl₃) δ (ppm): 0.34-0.40 (m, 2H), 0.60-0.68 (m, 2H), 1.17-1.44 (m, 4H), 1.69-1.80 (m, 4H), 1.86-1.97 (m, 2H), 2.33 (s, 6H), 2.74-2.83 (m, 2H), 2.89-2.97 (m, 2H), 3.39 (s, 2H), 3.76 (s, 3H), 3.81 (s, 2H), 3.93 (d, J=6.8 Hz, 2H), 5.99 (d, J=4.0 Hz, 1H), 6.68 (d, J=4.0 Hz, 1H), 6.88 (d, J=8.6 Hz, 1H), 7.43 (d, J=8.6 Hz, 1H).

### (2) 5-{4-{2-[6-Cyclopropylmethoxy-7-(N,N-dimethylaminomethyl)benz[d]isoxazol-3-yl]ethyl}piperidinomethyl}-1-methyl-1H-pyrrole-2-carbonitrile difumarate

The title compound was obtained from 5-{4-{2-[6-cyclopropylmethoxy-7-(N,N-dimethylaminomethyl)benz[d]isoxazol-3-yl]ethyl}piperidinomethyl}-1-methyl-1H-pyrrole-2-carbonitrile according to Example 64-(2).
ESI-MS m/z: 239 [M+2H]²⁺, 476 [M+H]⁺.

### Example 66

### 5-{4-{2-[6-Cyclopropylmethoxy-7-(N,N-dimethylaminomethyl)benz[d]isoxazol-3-yl]ethyl}piperidinomethyl}-1-ethyl-1H-pyrrole-2-carbonitrile difumarate

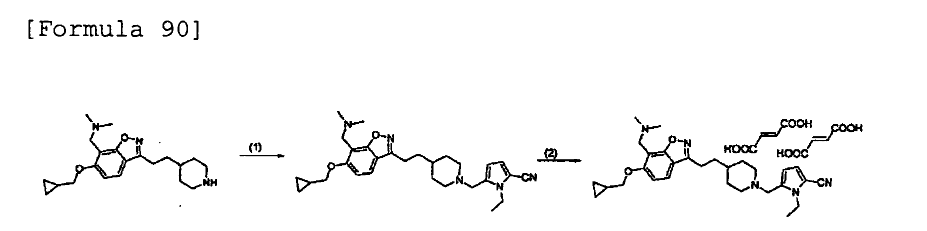

### (1) 5-{4-{2-[6-Cyclopropylmethoxy-7-(N,N-dimethylaminomethyl)benz[d]isoxazol-3-yl]ethyl}piperidinomethyl}-1-ethyl-1H-pyrrole-2-carbonitrile

The title compound was synthesized from 1-ethyl-5-formyl-1H-pyrrole-2-carbonitrile synthesized according to JP-10-509340 and N,N-dimethyl{6-cyclopropylmethoxy-3-[2-(piperidin-4-yl)ethyl]benz[d]isoxazol-7-yl}methylamine according to Example 65-(1).
¹H-NMR (400 MHz, CDCl₃) δ (ppm): 0.34-0.41 (m, 2H), 0.60-0.69 (m, 2H), 1.16-1.46 (m, 4H), 1.40 (t, J=6.8 Hz, 3H), 1.68-1.83 (m, 4H), 1.86-1.96 (m, 2H), 2.33 (s, 6H), 2.75-2.84 (m, 2H), 2.89-2.97 (m, 2H), 3.39 (s, 2H), 3.81 (s, 2H), 3.93 (d, J=6.8 Hz, 2H), 4.16 (q, J=6.8 Hz, 2H), 5.97 (d, J=4.0 Hz, 1H), 6.69 (d, J=4.0 Hz, 1H), 6.88 (d, J=8.6 Hz, 1H), 7.43 (d, J=8.6 Hz, 1H).

### (2) 5-{4-{2-[6-Cyclopropylmethoxy-7-(N,N-dimethylaminomethyl)benz[d]isoxazol-3-yl]ethyl}piperidinomethyl}-1-ethyl-1H-pyrrole-2-carbonitrile difumarate

The title compound was synthesized from 5-{4-{2-[6-cyclopropylmethoxy-7-(N,N-dimethylaminomethyl)benz[d]isoxazol-3-yl]ethyl}piperidinomethyl}-1-ethyl-1H-pyrrole-2-carbonitrile according to Example 64-(2).
ESI-MS m/z: 490 [M+H]⁺.

### Example 67

### N,N-Dimethyl{6-cyclopropylmethoxy-3-{2-[1-(pyridin-2-yl)piperidin-4-yl]ethyl}benz[d]isoxazol-7-yl}methylamine dihydrochloride

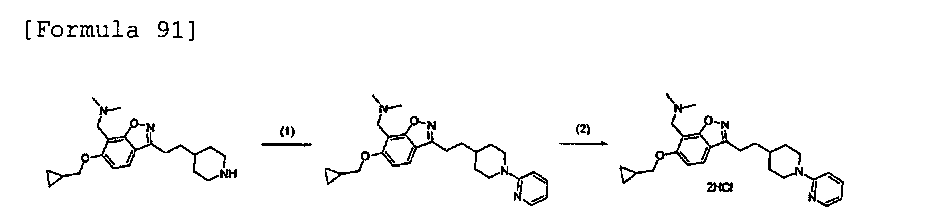

### (1) N,N-Dimethyl{6-cyclopropylmethoxy-3-{2-[1-(pyridin-2-yl)piperidin-4-yl]ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine (132 mg) was dissolved in acetonitrile (1 mL). 2-Fluoropyridine (143 µL) and tetrabutylammonium fluoride hydrate (206 mg) were added to the solution, and then the mixture was heated to 90°C and stirred for 15 hours. The reaction mixture was cooled to room temperature. Water, a 5 M sodium hydroxide solution and ethyl acetate were added to the reaction mixture, and the organic layer was separated. The organic layer was washed with a saturated sodium chloride solution and then dried over anhydrous magnesium sulfate. The drying agent was removed by filtration and then the filtrate was concentrated under reduced pressure to obtain a residue. The residue was subjected to silica gel chromatography (NH silica gel, heptane-ethyl acetate) to obtain the title compound (125 mg).
¹H-NMR(400 MHz, CDCl₃) δ (ppm): 0.34-0.40 (m, 2H), 0.60-0.69 (m, 2H), 1.23-1.38 (m, 3H), 1.50-1.70 (m, 1H), 1.76-1.91 (m, 4H), 2.36 (s, 6H), 2.76-2.86 (m, 2H), 2.95-3.02 (m, 2H), 3.86 (br s, 2H), 3.94 (d, J=6.8 Hz, 2H), 4.25-4.33 (m, 2H), 6.53-6.58 (m, 1H), 6.64 (d, J=8.4 Hz, 1H), 6.90 (d, J=8.4 Hz, 1H), 7.40-7.50 (m, 2H), 8.14-8.19 (m, 1H).

### (2) N,N-Dimethyl{6-cyclopropylmethoxy-3-{2-[1-(pyridin-2-yl)piperidin-4-yl]ethyl}benz[d]isoxazol-7-yl}methylamine dihydrochloride

N,N-Dimethyl{6-cyclopropylmethoxy-3-{2-[1-(pyridin-2-yl)piperidin-4-yl]ethyl}benz[d]isoxazol-7-yl}methylamine (125 mg) was dissolved in methanol (3 mL). A 4 M solution of hydrogen chloride in ethyl acetate (400 µL) was added and then the mixture was concentrated to dryness under reduced pressure. Addition of ethyl acetate and diethyl ether to the residue and concentration under reduced pressure were repeated to obtain the title compound (145 mg).
¹H-NMR (400 MHz, DMSO-d₆) δ (ppm): 0.37-0.44 (m, 2H), 0.58-0.64 (m, 2H), 1.18-1.40 (m, 3H), 1.60-1.94 (m, 5H), 2.81 (s, 6H), 2.96-3.18 (m, 4H), 4.07 (d, J=6.8 Hz, 2H), 4.20-4.31 (m, 2H), 4.45-4.52 (m, 2H), 6.75-6.90 (m, 1H), 7.20-7.50 (m, 1H), 7.24 (d, J=9.0 Hz, 1H), 7.70-8.05 (m, 2H), 7.97 (d, J=9.0 Hz, 1H), 9.90-10.0 (m, 1H).
ESI-MS m/z: 218 [M+2H]²⁺, 435 [M+H]⁺.

### Example 68

### N,N-Dimethyl{6-cyclopropylmethoxy-3-{2-[1-(pyrimidin-2-yl)piperidin-4-yl]ethyl}benz[d]isoxazol-7-yl}methylamine dihydrochloride

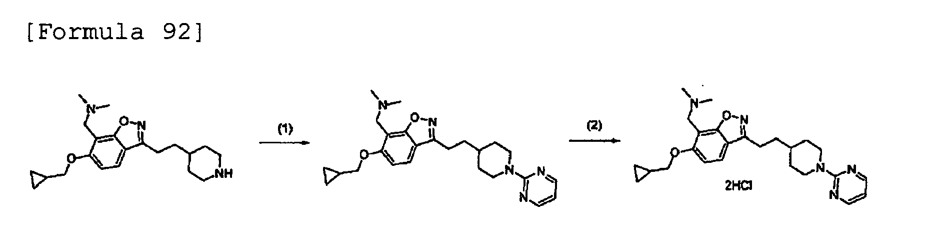

### (1) N,N-Dimethyl{6-cyclopropylmethoxy-3-{2-[1-(pyrimidin-2-yl)piperidin-4-yl]ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine (165 mg) was dissolved in acetonitrile (3 mL). 2-Chloropyrimidine (66.1 mg) and tetrabutylammonium fluoride hydrate (214 mg) were added to the solution, and then the mixture was heated to 90°C and stirred for 15 hours. The reaction mixture was cooled to room temperature. Water, a 5 M sodium hydroxide solution and ethyl acetate were added to the reaction mixture, and the organic layer was separated. The organic layer was washed with a saturated sodium chloride solution and then dried over anhydrous magnesium sulfate. The drying agent was removed by filtration and then the filtrate was concentrated under reduced pressure to obtain a residue. The residue was subjected to silica gel chromatography (NH silica gel, heptane-ethyl acetate) to obtain the title compound (98 mg).
¹H-NMR(400 MHz, CDCl₃) δ (ppm): 0.34-0.41 (m, 2H), 0.60-0.70 (m, 2H), 1.17-1.37 (m, 3H), 1.50-1.74 (m, 1H), 1.76-1.91 (m, 4H), 2.36 (s, 6H), 2.80-2.91 (m, 2H), 2.95-3.03 (m, 2H), 3.85 (br s, 2H), 3.94 (d, J=6.8 Hz, 2H), 4.71-4.80 (m, 2H), 6.42 (t, J=4.8 Hz, 1H), 6.90 (d, J=8.8 Hz, 1H), 7.45 (d, J=8.8 Hz, 1H), 8.27 (d, J=4.8 Hz, 2H).

### (2) N,N-Dimethyl{6-cyclopropylmethoxy-3-{2-[1-(pyrimidin-2-yl)piperidin-4-yl]ethyl}benz[d]isoxazol-7-yl}methylamine dihydrochloride

N,N-Dimethyl{6-cyclopropylmethoxy-3-{2-[1-(pyrimidin-2-yl)piperidin-4-yl]ethyl}benz[d]isoxazol-7-yl}methylamine (98 mg) was dissolved in methanol (3 mL). A 4 M solution of hydrogen chloride in ethyl acetate (300 µL) was added and then the mixture was concentrated to dryness under reduced pressure. Addition of ethyl acetate and diethyl ether to the residue and concentration under reduced pressure were repeated to obtain the title compound (96 mg).
ESI-MS m/z: 436 [M+H]⁺.

### Example 69

### N,N-Dimethyl{6-cyclopropylmethoxy-3-{2-[1-(thiazol-2-yl)piperidin-4-yl]ethyl}benz[d]isoxazol-7-yl}methylamine dihydrochloride

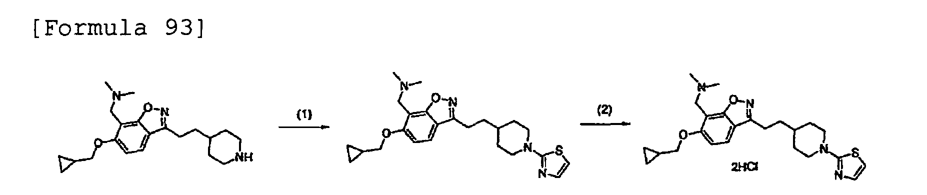

### (1) N,N-Dimethyl{6-cyclopropylmethoxy-3-{2-[1-(thiazol-2-yl)piperidin-4-yl]ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine (145 mg) was dissolved in N,N-dimethylformamide (3 mL). 2-Bromothiazole (45 µL) and potassium carbonate (70 mg) were added to the solution, and then the mixture was heated to 150°C and stirred for six hours. The reaction mixture was cooled to room temperature. Water, a 5 M sodium hydroxide solution and ethyl acetate were added to the reaction mixture, and the organic layer was separated. The organic layer was washed with a saturated sodium chloride solution and then dried over anhydrous magnesium sulfate. The drying agent was removed by filtration and then the filtrate was concentrated under reduced pressure to obtain a residue. The residue was subjected to silica gel chromatography (NH silica gel, heptane-ethyl acetate) to obtain the title compound (69 mg).
¹H-NMR(400 MHz, CDCl+₃) δ (ppm): 0.34-0.41 (m, 2H), 0.60-0.70 (m, 2H), 1.22-1.45 (m, 3H), 1.45-1.74 (m, 1H), 1.77-1.92 (m, 4H), 2.37 (s, 6H), 2.94-3.05 (m, 4H), 3.86 (br s, 2H), 3.94 (d, J=6.4 Hz, 2H), 3.95-4.04 (m, 2H), 6.52 (d, J=3.4 Hz, 1H), 6.90 (d, J=8.6 Hz, 1H), 7.16 (d, J=3.4 Hz, 1H), 7.45 (d, J=8.6 Hz, 1H).

### (2) N,N-Dimethyl{6-cyclopropylmethoxy-3-{2-[1-(thiazol-2-yl)piperidin-4-yl]ethyl}benz[d]isoxazol-7-yl}methylamine dihydrochloride

N,N-Dimethyl{6-cyclopropylmethoxy-3-{2-[1-(thiazol-2-yl)piperidin-4-yl]ethyl}benz[d]isoxazol-7-yl}methylamine (69 mg) was dissolved in methanol (3 mL). A 4 M solution of hydrogen chloride in ethyl acetate (200 µL) was added and then the mixture was concentrated to dryness under reduced pressure. Addition of ethyl acetate and diethyl ether to the residue and concentration under reduced pressure were repeated to obtain the title compound (56 mg).
ESI-MS m/z: 441 [M+H]⁺.

### Example 70

### N,N-Dimethyl{6-cyclopropylmethoxy-3-{2-[1-(2-fluoropyridin-6-yl)piperidin-4-yl]ethyl}benz[d]isoxazol-7-yl}methylamine hydrochloride

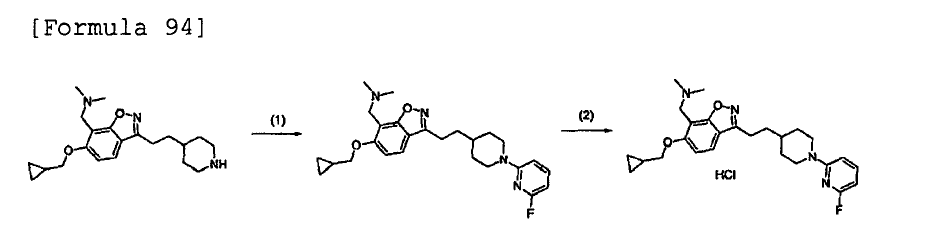

### (1) N,N-Dimethyl{6-cyclopropylmethoxy-3-{2-[1-(2-fluoropyridin-6-yl)piperidin-4-yl]ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine (127 mg) was dissolved in acetonitrile (1.5 mL). 2,6-Difluoropyridine (96.5 µL) and tetrabutylammonium fluoride hydrate (198 mg) were added to the solution, and then the mixture was heated under reflux for three hours. The reaction mixture was cooled to room temperature. Water, a 5 M sodium hydroxide solution and ethyl acetate were added to the reaction mixture, and the organic layer was separated. The organic layer was washed with a saturated sodium chloride solution and then dried over anhydrous magnesium sulfate. The drying agent was removed by filtration and then the filtrate was concentrated under reduced pressure to obtain a residue. The residue was subjected to silica gel chromatography (NH silica gel, heptane-ethyl acetate) to obtain the title compound (122 mg).
¹H-NMR (400 MHz, CDCl₃) δ (ppm): 0.34-0.40 (m, 2H), 0.60-0.68 (m, 2H), 1.20-1.37 (m, 3H), 1.50-1.74 (m, 1H), 1.74-1.90 (m, 4H), 2.35 (s, 6H), 2.76-2.87 (m, 2H), 2.94-3.01 (m, 2H), 3.84 (br s, 2H), 3.94 (d, J=6.8 Hz, 2H), 4.21-4.30 (m, 2H), 6.10 (dd, J=2.4, 8.0 Hz, 1H), 6.40 (dd, J=2.4, 8.0 Hz, 1H), 6.90 (d, J=8.6 Hz, 1H), 7.45 (d, J=8.6 Hz, 1H), 7.42-7.52 (m, 1H).

### (2) N,N-Dimethyl{6-cyclopropylmethoxy-3-{2-[1-(2-fluoropyridin-6-yl)piperidin-4-yl]ethyl}benz[d]isoxazol-7-yl}methylamine hydrochloride

N,N-Dimethyl{6-cyclopropylmethoxy-3-{2-[1-(2-fluoropyridin-6-yl)piperidin-4-yl]ethyl}benz[d]isoxazol-7-yl}methylamine (122 mg) was dissolved in methanol (3 mL). A 4 M solution of hydrogen chloride in ethyl acetate (270 µL) was added and then the mixture was concentrated to dryness under reduced pressure. Addition of acetone and heptane to the residue and concentration under reduced pressure were repeated to obtain the title compound (120 mg).
ESI-MS m/z: 453 [M+H]⁺.

### Example 71

### N,N-Dimethyl{6-cyclopropylmethoxy-3-{2-[1-phenylpiperidin-4-yl]ethyl}benz[d]isoxazol-7-yl}methylamine fumarate

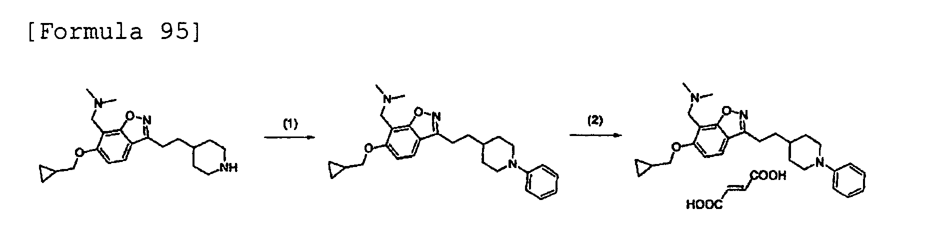

### (1) N,N-Dimethyl{6-cyclopropylmethoxy-3-{2-[1-phenylpiperidin-4-yl]ethyl}benz[d]isoxazol-7-yl}methylamine

A sealed tube was used in the following reaction. N,N-Dimethyl{6-cyclopropylmethoxy-3-[2-(piperidin-4-yl)ethyl]benz[d]isoxazol-7-yl}methylamine (149 mg) was dissolved in 2-propanol (0.5 mL). Iodobenzene (46.5 µL), ethylene glycol (46.5 µL), copper (I) iodide (4 mg) and potassium phosphate (177 mg) were added to the solution, and then the mixture was heated to 80°C and stirred for 12 hours. The reaction mixture was cooled to room temperature. A saturated sodium chloride solution, 28% aqueous ammonia and ethyl acetate were added to the reaction mixture, and the organic layer was separated. The organic layer was washed with a saturated sodium chloride solution and then dried over anhydrous magnesium sulfate. The drying agent was removed by filtration and then the filtrate was concentrated under reduced pressure to obtain a residue. The residue was subjected to silica gel chromatography (NH silica gel, heptane-ethyl acetate) to obtain the title compound (25 mg).
¹H-NMR (400 MHz, CDCl₃)
δ (ppm): 0.34-0.40 (m, 2H), 0.60-0.70 (m, 2H), 1.20-1.93 (m, 8H), 2.36 (s, 6H), 2.63-2.74 (m, 2H), 2.94-3.03 (m, 2H), 3.63-3.72 (m, 2H), 3.85 (br s, 2H), 3.94 (d, J=6.4 Hz, 2H), 6.78-6. 85 (m, 1H), 6.90 (d, J=8.8 Hz, 1H), 6.90-6.97 (m, 2H), 7.20-7.28 (m, 2H), 7.46 (d, J=8.8 Hz, 1H).

### (2) N,N-Dimethyl{6-cyclopropylmethoxy-3-{2-[1-phenylpiperidin-4-yl]ethyl}benz[d]isoxazol-7-yl}methylamine fumarate

N,N-Dimethyl{6-cyclopropylmethoxy-3-{2-[1-phenylpiperidin-4-yl]ethyl}benz[d]isoxazol-7-yl}methylamine (25 mg) and fumaric acid (7 mg) were dissolved in acetone (3 mL), and the solution was concentrated to dryness under reduced pressure. Addition of diethyl ether-heptane to the residue and concentration under reduced pressure were repeated to obtain the title compound (31 mg).
ESI-MS m/z: 434 [M+H]⁺.

### Example 72

### N,N-Dimethyl{6-cyclopropylmethoxy-3-{2-[1-(pyridin-3-yl)piperidin-4-yl]ethyl}benz[d]isoxazol-7-yl}methylamine dihydrochloride

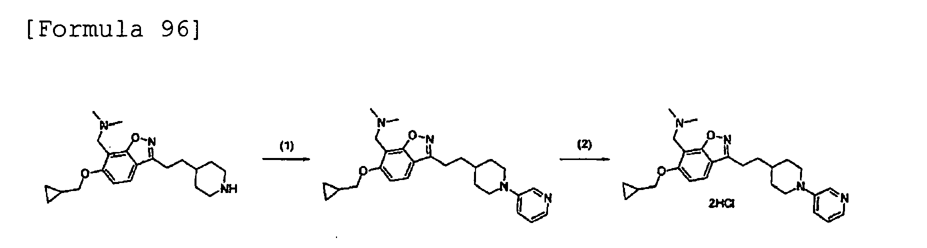

### (1) N,N-Dimethyl{6-cyclopropylmethoxy-3-{2-[1-(pyridin-3-yl)piperidin-4-yl]ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine (157 mg) was dissolved in toluene (4 mL) in a nitrogen atmosphere. 3-Bromopyridine (53.4 µL), sodium tert-butoxide (61.2 mg) and bis(tri-tert-butylphosphine)palladium (0) (22.4 mg) were sequentially added to the solution, and then the mixture was heated under reflux for 40 minutes. The reaction mixture was cooled to room temperature. Water and ethyl acetate were added to the reaction mixture, followed by filtration through celite. Then, the organic layer was separated. The organic layer was washed with a saturated sodium chloride solution and then dried over anhydrous magnesium sulfate. The drying agent was removed by filtration and then the filtrate was concentrated under reduced pressure to obtain a residue. The residue was subjected to silica gel chromatography (NH silica gel, ethyl acetate) to obtain the title compound (147 mg).
¹H-NMR(400 MHz, CDCl₃)
δ (ppm): 0.34-0.41 (m, 2H), 0.61-0.69 (m, 2H), 1.22-1.96 (m, 8H), 2.36 (s, 6H), 2.68-2.79 (m, 2H), 2.95-3.03 (m, 2H), 3.65-3.73 (m, 2H), 3.86 (br s, 2H), 3.95 (d, J=6.8 Hz, 2H), 6.91 (d, J=8.6 Hz, 1H), 7.10-7.20 (m, 2H), 7.46 (d, J=8.6 Hz, 1H), 8.05 (dd, J=1.6, 4.4 Hz, 1H), 8.30 (d, J=2.4 Hz, 1H).

### (2) N,N-Dimethyl{6-cyclopropylmethoxy-3-{2-[1-(pyridin-3-yl)piperidin-4-yl]ethyl}benz[d]isoxazol-7-yl}methylamine dihydrochloride

N,N-Dimethyl{6-cyclopropylmethoxy-3-{2-[1-(pyridin-3-yl)piperidin-4-yl]ethyl}benz[d]isoxazol-7-yl}methylamine (147 mg) was dissolved in methanol (5 mL). A 4 M solution of hydrogen chloride in ethyl acetate (423 µL) was added and then the mixture was concentrated to dryness under reduced pressure. Sequential addition of acetone and diethyl ether to the residue and concentration under reduced pressure were repeated to obtain the title compound (172 mg).
ESI-MS m/z: 218 [M+2H]²⁺, 435 [M+H]⁺.

### Example 73

### N,N-Dimethyl{6-cyclopropylmethoxy-3-{2-[1-(pyridin-4-yl)piperidin-4-yl]ethyl}benz[d]isoxazol-7-yl}methylamine dihydrochloride

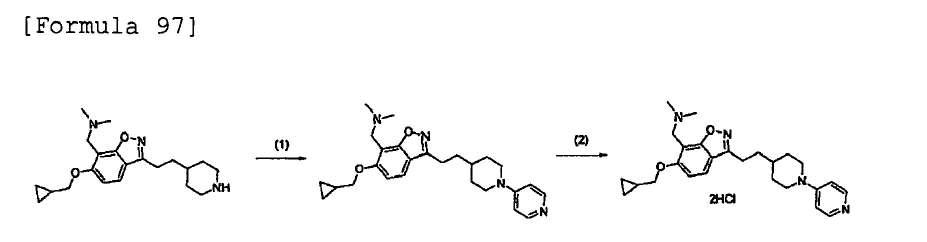

### (1) N,N-Dimethyl{6-cyclopropylmethoxy-3-{2-[1-(pyridin-4-yl)piperidin-4-yl]ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine (160 mg) was dissolved in 2-ethoxyethanol (3 mL) in a nitrogen atmosphere. 4-Chloropyridine hydrochloride (73.9 µL) and N,N-diisopropylethylamine (273 µL) were sequentially added to the solution, and then the mixture was heated under reflux for 17 hours. The reaction mixture was cooled to room temperature. Water, a 5 M sodium hydroxide solution and ethyl acetate were added to the reaction mixture, and the organic layer was separated. The organic layer was washed with a saturated sodium chloride solution and then dried over anhydrous magnesium sulfate. The drying agent was removed by filtration and then the filtrate was concentrated under reduced pressure to obtain a residue. The residue was subjected to silica gel chromatography (NH silica gel, ethyl acetate) to obtain the title compound (55 mg).
¹H-NMR(400 MHz, CDCl₃) δ (ppm): 0.34-0.41 (m, 2H), 0.61-0.70 (m, 2H), 1.24-1.39 (m, 3H), 1.54-1.98 (m, 5H), 2.36 (s, 6H), 2.79-2.89 (m, 2H), 2.94-3.03 (m, 2H), 3.82-3.92 (m, 2H), 3.85 (br s, 2H), 3.94 (d, J=6.8 Hz, 2H), 6.64 (dd, J=1.6, 5.2 Hz, 2H), 6.90 (d, J=8.6 Hz, 1H), 7.45 (d, J=8.6 Hz, 1H), 8.22 (dd, J=1.6, 5.2 Hz, 2H).

### (2) N,N-Dimethyl{6-cyclopropylmethoxy-3-{2-[1-(pyridin-4-yl)piperidin-4-yl]ethyl}benz[d]isoxazol-7-yl}methylamine dihydrochloride

N,N-Dimethyl{6-cyclopropylmethoxy-3-{2-[1-(pyridin-4-yl)piperidin-4-yl]ethyl}benz[d]isoxazol-7-yl}methylamine (55 mg) was dissolved in methanol (3 mL). A 4 M solution of hydrogen chloride in ethyl acetate (159 µL) was added and then the mixture was concentrated to dryness under reduced pressure. Addition of acetone to the residue and concentration under reduced pressure were repeated to obtain the title compound (61 mg).
ESI-MS m/z: 218 [M+2H]²⁺, 435 [M+H]⁺.

### Example 74

### N-Methyl{6-cyclopropylmethoxy-3-{2-[1-(pyrimidin-2-yl)piperidin-4-yl]ethyl}benz[d]isoxazol-7-yl}methylamine fumarate

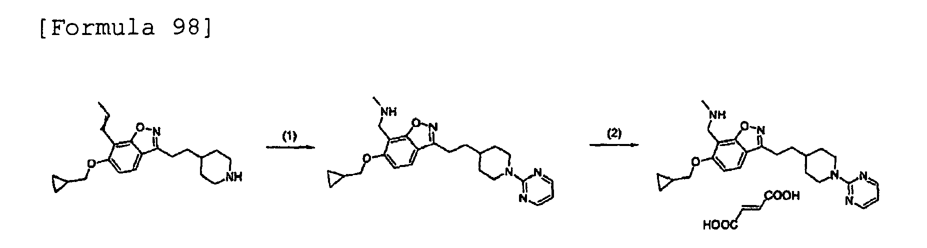

### (1) N-Methyl{6-cyclopropylmethoxy-3-{2-[1-(pyrimidin-2-yl)piperidin-4-yl]ethyl}benz[d]isoxazol-7-yl}methylamine

The title compound was synthesized from 2-chloropyrimidine according to Example 76.
¹H-NMR (400 MHz, CDCl₃) δ (ppm): 0.32-0.42 (m, 2H), 0.60-0.72 (m, 2H), 1.16-1.37 (m, 3H), 1.58-1.73 (m, 1H), 1.74-1.96 (m, 4H), 2.44 (s, 3H), 2.80-2.93 (m, 2H), 2.94-3.02 (m, 2H), 3.95 (d, J=7.2 Hz, 2H), 4.13 (s, 2H), 4.71-4.80 (m, 2H), 6.43 (t, J=4.8 Hz, 1H), 6.90 (d, J=8.4 Hz, 1H), 7.45 (d, J=8.4 Hz, 1H), 8.29 (d, J=4.8 Hz, 2H).

### (2) N-Methyl{6-cyclopropylmethoxy-3-{2-[1-(pyrimidin-2-yl)piperidin-4-yl]ethyl}benz[d]isoxazol-7-yl}methylamine fumarate

N-Methyl{6-cyclopropylmethoxy-3-{2-[1-(pyrimidin-2-yl)piperidin-4-yl]ethyl}benz[d]isoxazol-7-yl}methylamine (107 mg) and fumaric acid (30 mg) were dissolved in methanol (3 mL), and then the solution was concentrated under reduced pressure. Acetone was added to the residue, followed by solidification. The solid was collected by filtration to obtain the title compound (120 mg).
¹H-NMR(400 MHz, CD₃OD) δ (ppm): 0.40-0.46 (m, 2H), 0.66-0.72 (m, 2H), 1.14-1.27 (m, 2H), 1.33-1.44 (m, 1H), 1.60-1.72 (m, 1H), 1.76-1.90 (m, 4H), 2.77 (s, 3H), 2.80-2.90 (m, 2H), 3.02-3.09 (m, 2H), 4.10 (d, J=7.2 Hz, 2H), 4.50 (s, 2H), 4.67-4.76 (m, 2H), 6.53 (t, J=4.8 Hz, 1H), 6.67 (s, 2H), 7.21 (d, J=8.8 Hz, 1H), 7.86 (d, J=8.8 Hz, 1H), 8.28 (d, J=4.8 Hz, 2H).
ESI-MS. m/z: 422 [M+H]⁺.

### Example 75

### N-Methyl{6-cyclopropylmethoxy-3-{2-[1-(pyrimidin-4-yl)piperidin-4-yl]ethyl}benz[d]isoxazol-7-yl}methylamine fumarate

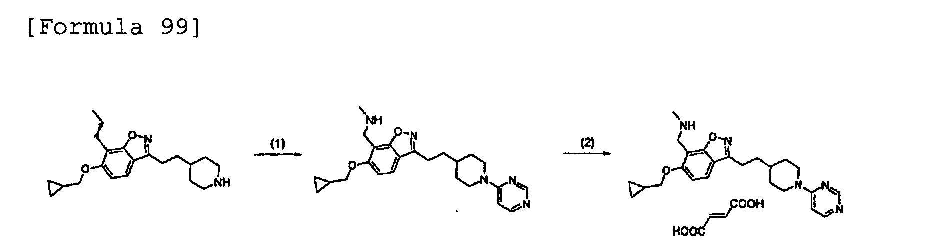

### (1) N-Methyl{6-cyclopropylmethoxy-3-{2-[1-(pyrimidin-4-yl)piperidin-4-yl]ethyl}benz[d]isoxazol-7-yl}methylamine

The title compound was synthesized from 4,6-dichloropyrimidine according to Example 76.
¹H-NMR(400 MHz, CDCl₃) δ (ppm): 0.30-0.40 (m, 2H), 0.58-0.72 (m, 2H), 1.14-1.38 (m, 3H), 1.50-2.16 (m, 5H), 2.46 (d, J=1.2 Hz, 3H), 2.80-2.94 (m, 2H), 2.94-3.04 (m, 2H), 3.96 (d, J=6.8 Hz, 2H), 4.12-4.19 (m, 2H), 4.34-4.49 (m, 2H), 6.49 (dd, J=1.2, 6.4 Hz, 1H), 6.91 (d, J=8.6 Hz, 1H), 7.45 (d, J=8.6 Hz, 1H), 8.16 (dd, J=0.4, 6.4 Hz, 1H), 8.57 (d, J=0.4 Hz, 1H).

### (2) N-Methyl{6-cyclopropylmethoxy-3-{2-[1-(pyrimidin-4-yl)piperidin-4-yl]ethyl}benz[d]isoxazol-7-yl}methylamine fumarate

N-Methyl{6-cyclopropylmethoxy-3-{2-[1-(pyrimidin-4-yl)piperidin-4-yl]ethyl}benz[d]isoxazol-7-yl}methylamine (10 mg) and fumaric acid (3 mg) were dissolved in methanol (0.5 mL), and then the solution was concentrated under reduced pressure. tert-Butyl methyl ether was added to the residue and the mixture was concentrated under reduced pressure to obtain the title compound (12 mg).

### Example 76

### N-Methyl{6-cyclopropylmethoxy-3-{2-[1-(pyridazin-3-yl)piperidin-4-yl]ethyl}benz[d]isoxazol-7-yl}methylamine fumarate

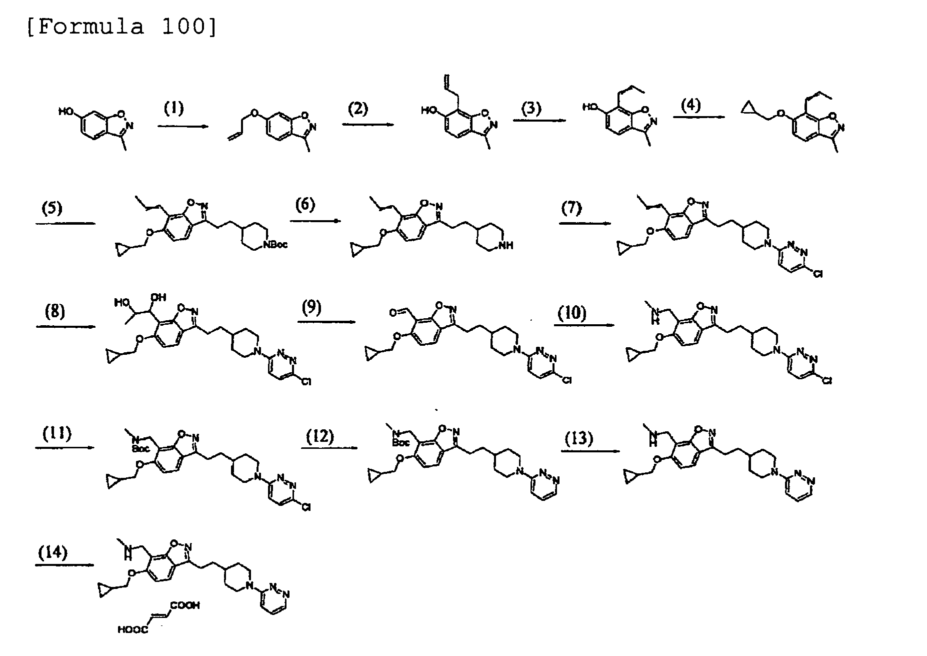

### (1) 6-Allyloxy-3-methylbenz[d]isoxazole

6-Hydroxy-3-methylbenz[d]isoxazole (8.00 g) was dissolved in N,N-dimethylformamide (100 mL) at room temperature. Potassium carbonate (8.89 g) and allyl bromide (5.57 mL) were sequentially added to the solution, and then the mixture was stirred for 15 hours. Water and tert-butyl methyl ether were added to the reaction mixture, and the organic layer was separated. The organic layer was washed with a saturated sodium chloride solution and then dried over anhydrous magnesium sulfate. The drying agent was removed by filtration and then the filtrate was concentrated under reduced pressure to obtain a residue. The residue was subjected to silica gel chromatography (NH silica gel, heptane-ethyl acetate) to obtain the title compound (9.21 g).
¹H-NMR(400 MHz, CDCl₃) δ (ppm): 2.53 (s, 3H), 4.58-4.64 (m, 2H), 5.31-5.37 (m, 1H), 5.42-5.50 (m, 1H), 6.02-6.14 (m, 1H), 6.94 (dd, J=2.0, 8.4 Hz, 1H), 6.98 (d, J=2.0 Hz, 1H), 7.46 (dd, J=0.6, 8.4 Hz, 1H).

### (2) 7-Allyl-6-hydroxy-3-methylbenz[d]isoxazole

6-Allyloxy-3-methylbenz[d]isoxazole (9.21 g) was dissolved in N,N-dimethylaniline (20 mL) in a nitrogen atmosphere, and the solution was heated under reflux for 3.5 hours. The reaction mixture was cooled to room temperature. Then, 5 M hydrochloric acid and ethyl acetate were added to the reaction mixture, and the organic layer was separated. The organic layer was washed with 5 M hydrochloric acid and then dried over anhydrous magnesium sulfate. The drying agent was removed by filtration and then the filtrate was concentrated under reduced pressure. The residue was suspended by adding heptane and then collected by filtration. The solid collected by filtration was dried under reduced pressure with heating to obtain the title compound (6.76 g).
¹H-NMR(400 MHz, CDCl₃) δ (ppm): 2.52 (s, 3H), 3.66-3.74 (m, 2H), 5.17-5.29 (m, 2H), 5.42 (br s, 1H), 6.00-6.12 (m, 1H), 6.85 (d, J=8.6 Hz, 1H), 7.35 (d, J=8.6 Hz, 1H).

### (3) Mixture of 6-hydroxy-3-methyl-7-[(E)-1-propenyl]benz[d]isoxazole and 6-hydroxy-3-methyl-7-[(Z)-1-propenyl]benz[d]isoxazole

Ethanol (50 mL), dimethyl sulfoxide (50 mL) and powdery potassium hydroxide (10.3 g) were added to 7-allyl-6-hydroxy-3-methylbenz[d]isoxazole (6.92 g) at room temperature. Then, the mixture was heated to 110°C and stirred for 14 hours. The reaction mixture was cooled to room temperature. Then, the reaction mixture was adjusted to pH 2 to 3 with 5 M hydrochloric acid, followed by extraction with ethyl acetate and washing with a saturated sodium chloride solution. The organic layer was dried over magnesium sulfate. The drying agent was filtered off. The filtrate was concentrated under reduced pressure. The generated solid was suspended in tert-butyl methyl ether and heptane and collected by filtration. The filtrate was concentrated and the precipitated solid was similarly collected by filtration. The solids were combined and dried under reduced pressure to obtain the title compound (6.35 g). ¹H-NMR(400 MHz, CDCl₃) δ (ppm): 2.01 (d, J=1.6 Hz) and 2.02 (d, J=1.6 Hz) (total 3H), 2.52 (s, 3H), 5.55 (br s, 1H), 6.62-6.85 and 7.23-7.30 (m, 4H).

### (4) Mixture of 6-cyclopropylmethoxy-3-methyl-7-[(E)-1-propenyl]benz[d]isoxazole and 6-cyclopropylmethoxy-3-methyl-7-[(Z)-1-propenyl]benz[d]isoxazole

Acetone (100 mL) was added to the mixture of 6-hydroxy-3-methyl-7-[(E)-1-propenyl]benz[d]isoxazole and 6-hydroxy-3-methyl-7-[(Z)-1-propenyl]benz[d]isoxazole (5 g), followed by dissolution by heating. Potassium carbonate (5.47 g) and cyclopropylmethyl bromide (3.33 mL) were sequentially added to the solution, and then the mixture was heated under reflux at 60°C. After 18 hours, potassium carbonate (3 g), cyclopropylmethyl bromide (1.8 mL) were added and heating with stirring was further continued. After four hours, N,N-dimethylformamide (50 mL) was further added. After heating to 80°C, heating with stirring was further continued for five hours. The reaction mixture was cooled to room temperature. Ethyl acetate (300 mL) and water (200 mL) were added and the organic layer was separated. The organic layer was washed with a saturated sodium chloride solution (250 mL, three times) and dried over anhydrous magnesium sulfate (25 g). The drying agent was removed by filtration and the filtrate was concentrated under reduced pressure to obtain a residue. The residue was subjected to NH silica gel column chromatography (heptane-ethyl acetate) to obtain the title compound (6.04 g).
¹H-NMR (400 MHz, CDCl₃) δ (ppm): 0.32-0.45 (m, 2H), 0.60-0.73 (m, 2H), 1.26-1.39 (m, 1H), 2.01 (d, J=1.6 Hz) and 2.02 (d, J=1.6 Hz) (total 3H), 2.53 (s, 3H), 3.93 (d, J=6.8 Hz, 2H), 6.82-7.05 and 7.24-7.35 (m, 4H).

### (5) Mixture of tert-butyl 4-{2-{6-cyclopropylmethoxy-7-[(E)-1-propenyl]benz[d]isoxazol-3-yl}ethyl}piperidine-1-carboxylate and tert-butyl 4-{2-{6-cyclopropylmethoxy-7-[(Z)-1-propenyl]benz[d]isoxazol-3-yl}ethyl}piperidine-1-carboxylate

The mixture of 6-cyclopropylmethoxy-3-methyl-7-[(E)-1-propenyl]benz[d]isoxazole and 6-cyclopropylmethoxy-3-methyl-7-[(Z)-1-propenyl]benz[d]isoxazole (3 g) and tert-butyl 4-iodomethylpiperidine-1-carboxylate (4.6 g) were dissolved in tetrahydrofuran (35 mL) in a nitrogen atmosphere, and the solution was cooled to an internal temperature of -73°C. A prepared 1 M lithium diisopropylamide solution (14.8 mL) was added dropwise to the solution over 10 minutes. After completion of dropwise addition, the mixture was further stirred at - 63 to -61°C for two hours. A saturated ammonium chloride solution (50 mL) was added to the reaction mixture, and then the mixture was heated to room temperature. Ethyl acetate (200 mL) and a saturated sodium chloride solution (50 mL) were added to the reaction mixture, and the organic layer was separated. The organic layer was further extracted from the aqueous layer with ethyl acetate (50 mL). The organic layers were collectively washed with a saturated sodium chloride solution (100 mL x 2) and dried over anhydrous magnesium sulfate. The drying agent was removed by filtration and the filtrate was concentrated under reduced pressure to obtain a residue. The residue was subjected to silica gel column chromatography (heptane-ethyl acetate) to obtain the title compound (3.57 g).
¹H-NMR (400 MHz, CDCl₃) δ (ppm): 0.32-0.45 (m, 2H), 0.60-0.73 (m, 2H), 1.06-1.23 (m, 2H), 1.23-1.39 (m, 1H), 1.42-1.58 (m, 1H), 1.46 (s, 9H), 1.68-1.84 (m, 4H), 2.00 (d, J=1.6 Hz) and 2.02 (d, J=1.6 Hz)(total 3H), 2.58-2.77 (m, 2H), 2.96 (t, J=7.6 Hz, 2H), 3.93 (d, J=6.8 Hz, 2H), 3.98-4.22 (m, 2H), 6.81-7.06 and 7.24-7.37 (m, 4H).

### (6) Mixture of 6-cyclopropylmethoxy-3-[2-(piperidin-4-yl)ethyl]-7-[(E)-1-propenyl]benz[d]isoxazole and 6-cyclopropylmethoxy-3-[2-(piperidin-4-yl)ethyl]-7-[(Z)-1-propenyl]benz[d]isoxazole

The mixture of tert-butyl 4-{2-{6-cyclopropylmethoxy-7-[(E)-1-propenyl]benz[d]isoxazol-3-yl}ethyl}piperidine-1-carboxylate and tert-butyl 4-{2-{6-cyclopropylmethoxy-7-[(Z)-1-propenyl]benz[d]isoxazol-3-yl}ethyl}piperidine-1-carboxylate (3.57 g) was dissolved in methanol (50 mL), and the solution was ice-cooled. A 4 M solution of hydrogen chloride in ethyl acetate (26 mL) was added to the reaction mixture. Then, the mixture was heated to room temperature and stirred for eight hours. The reaction mixture was concentrated under reduced pressure. Chloroform, a 2 M sodium hydroxide solution and a saturated sodium chloride solution were added to the residue, and the organic layer was separated. The organic layer was dried over anhydrous magnesium sulfate, and the drying agent was removed by filtration. The filtrate was concentrated under reduced pressure to obtain the title compound (2.71 g).
¹H-NMR(400 MHz, CDCl₃) δ (ppm): 0.32-0.45 (m, 2H), 0.60-0.73 (m, 2H), 1.12-1.26 (m, 2H), 1.26-1.39 (m, 1H), 1.41-1.56 (m, 1H), 1.70-1.92 (m, 4H), 2.00 (d, J=1.6 Hz) and 2.02 (d, J=1.6 Hz)(total 3H), 2.34-2.64 (m, 2H), 2.91-2.99 (m, 2H), 3.03-3.13 (m, 2H), 3.93 (d, J=6.8 Hz, 2H), 6.81-7.06 and 7.30-7.38 (m, 4H).

### (7) Mixture of 3-{2-[1-(6-chloropyridazin-3-yl)piperidin-4-yl]ethyl}-6-cyclopropylmethoxy-7-[(E)-1-propenyl]benz[d]isoxazole and 3-{2-[1-(6-chloropyridazin-3-yl)piperidin-4-yl]ethyl}-6-cyclopropylmethoxy-7-[(Z)-1-propenyl]benz[d]isoxazole

The mixture of 6-cyclopropylmethoxy-3-[2-(piperidin-4-yl)ethyl]-7-[(E)-1-propenyl]benz[d]isoxazole and 6-cyclopropylmethoxy-3-(2-piperidin-4-yl)ethyl-7-[(Z)-1-propenyl]benz[d]isoxazole (800 mg) was dissolved in acetonitrile (13 mL) in a nitrogen atmosphere. 3,6-Dichloropyridazine (421 mg) and triethylamine (493 µL) were sequentially added to the solution, and then the mixture was heated to 90°C. After five hours, the reaction mixture was cooled to room temperature. Ethyl acetate, a 1 M sodium hydroxide solution and a saturated sodium chloride solution were added to the reaction mixture, and the organic layer was separated. The organic layer was washed with a saturated sodium chloride solution and then dried over anhydrous magnesium sulfate. The drying agent was removed by filtration and the filtrate was concentrated under reduced pressure to obtain a residue. The residue was subjected to silica gel chromatography (NH silica gel, heptane-ethyl acetate) to obtain the title compound (556 mg).
¹H-NMR (400 MHz, CDCl₃) δ (ppm): 0.33-0.45 (m, 2H), 0.62-0.72 (m, 2H), 1.23-1.40 (m, 3H), 1.60-1.96 (m, 5H), 2.01 (d, J = 1.6 Hz) and 2.02 (d, J = 1.6 Hz) (total 3H), 2.87-3.04 (m, 4H), 3.93 (d, J=7.2 Hz, 2H), 4.29-4.39 (m, 2H), 6.82-7.05, 7.17 (d, J=9.6 Hz) and 7.34 (d, J=8.4 Hz) (total 6H).

### (8)(9)(10) N-Methyl{3-{2-[1-(6-chloropyridazin-3-yl)piperidin-4-yl]ethyl}-6-cyclopropylmethoxybenz[d]isoxazol-7-yl}methylamine

The mixture of 3-{2-[1-(6-chloropyridazin-3-yl)piperidin-4-yl]ethyl}-6-cyclopropylmethoxy-7-[(E)-1-propenyl]benz[d]isoxazole and 3-{2-[1-(6-chloropyridazin-3-yl)piperidin-4-yl]ethyl}-6-cyclopropylmethoxy-7-[(Z)-1-propenyl]benz[d]isoxazole (556 mg) and methanesulfonamide (117 mg) were dissolved in tetrahydrofuran (8 mL) and tert-butanol (8 mL). AD-mix-β (1.59 g) was added to the solution. Then, water (8 mL) was added and the mixture was stirred at room temperature for 58 hours. Sodium sulfite (1.86 g) was added to the reaction mixture, followed by stirring for 30 minutes. Then, ethyl acetate, acetone and a saturated sodium chloride solution were added and the organic layer was separated. The organic layer was washed with a saturated sodium chloride solution and then dried over anhydrous magnesium sulfate. The drying agent was filtered off and the filtrate was concentrated under reduced pressure to obtain 1-{3-{2-[1-(6-chloropyridazin-3-yl)piperidin-4-yl]ethyl}-6-cyclopropylmethoxybenz[d]isoxazol-7-yl}ethane-1,2-diol (crude yield: 604 mg). The diol compound was used for the next reaction without further purification. The diol compound (604 mg) was dissolved in a mixed solvent of tetrahydrofuran (19 mL) and water (6.8 mL). Sodium periodate (973 mg) was added and the mixture was vigorously stirred for one hour. Chloroform, methanol and a saturated sodium chloride solution were added to the reaction mixture, and the organic layer was separated. The organic layer was washed with a saturated sodium chloride solution and then dried over anhydrous magnesium sulfate. The drying agent was filtered off and the filtrate was concentrated under reduced pressure to obtain {3-{2-[1-(6-chloropyridazin-3-yl)piperidin-4-yl]ethyl}-6-cyclopropylmethoxybenz[d]isoxazol-7-carbaldehyde (crude yield: 500 mg). The aldehyde compound was used for the next reaction without further purification. The aldehyde compound (500 mg) was dissolved in dichloromethane (17 mL). Acetic acid (326 µL) and a 2 M solution of methylamine in tetrahydrofuran (3.4 mL) were sequentially added to the solution under ice-cooling, and then the mixture was stirred at room temperature for 15 minutes. Sodium triacetoxyborohydride (500 mg) was added to the reaction mixture, and the mixture was further stirred at room temperature for three hours. Chloroform, a 5 M sodium hydroxide solution and a saturated sodium chloride solution were sequentially added to the reaction mixture, and the organic layer was separated. A small amount of methanol was added to the organic layer to remove the turbidity, followed by drying over anhydrous magnesium sulfate. The drying agent was removed by filtration and then the filtrate was concentrated under reduced pressure to obtain a crude product. The crude product was subjected to silica gel column chromatography (NH silica gel, heptane-ethyl acetate) to obtain the title compound (447 mg).
¹H-NMR (400 MHz, CDCl₃) δ (ppm): 0.32-0.42 (m, 2H), 0.62-0.74 (m, 2H), 1.22-2.02 (m, 8H), 2.51 (s, 3H), 2.87-3.04 (m, 4H), 3.98 (d, J=7.2 Hz, 2H), 4.24 (s, 2H), 4.29-4.40 (m, 2H), 6.90 (d, J=9.4 Hz, 1H), 6.93 (d, J=8.8 Hz, 1H), 7.17 (d, J=9.4 Hz, 1H), 7.49 (d, J=8.8 Hz, 1H).

### (11) 7-[(N-tert-Butoxycarbonyl-N-methylamino)methyl]-3-{2-[1-(6-chloropyridazin-3-yl)piperidin-4-yl]ethyl}-6-cyclopropylmethoxybenz[d]isoxazole

N-Methyl{3-{2-[1-(6-chloropyridazin-3-yl)piperidin-4-yl]ethyl}-6-cyclopropylmethoxybenz[d]isoxazol-7-yl}methylamine (447 mg) and triethylamine (0.21 mL) were dissolved in tetrahydrofuran (10 mL). Di-tert-butyl dicarbonate (300 mg) was added to the solution, and the mixture was stirred at room temperature for 15 hours. The reaction mixture was concentrated to obtain a residue. The residue was subjected to silica gel column chromatography (heptane-ethyl acetate) to obtain the title compound (505 mg).
¹H-NMR(400 MHz, CDCl₃) δ (ppm): 0.33-0.43 (m, 2H), 0.58-0.72 (m, 2H), 1.23-1.74 (m, 13H), 1.76-1.96 (m, 4H), 2.72-3.06 (m, 7H), 3.93 (d, J=7.2 Hz, 2H), 4.28-4.40 (m, 2H), 4.70-4.88 (m, 2H), 6.86-6.93 (m, 2H), 7.17 (d, J=9.6 Hz, 1H), 7.45 (d, J=8.8 Hz, 1H).

### (12)(13) N-Methyl{6-cyclopropylmethoxy-3-{2-[1-(pyridazin-3-yl)piperidin-4-yl]ethyl}benz[d]isoxazol-7-yl}methylamine

7-[(N-tert-Butoxycarbonyl-N-methylamino)methyl]-3-{2-[1-(6-chloropyridazin-3-yl)piperidin-4-yl]ethyl}-6-cyclopropylmethoxybenz[d]isoxazole (382 mg), triethylamine (211 µL) and formic acid (29 µL) were dissolved in N,N-dimethylformamide (6 mL). Triphenylphosphine (12 mg) and palladium acetate (5 mg) were added to the solution, and the mixture was stirred at 120°C. After four hours, triethylamine (211 µL) and formic acid (29 µL) were added. After one hour, the reaction mixture was cooled to room temperature. Ethyl acetate, a saturated sodium chloride solution and aqueous ammonia were added, and the organic layer was separated. The organic layer was washed with a saturated sodium chloride solution and then dried over anhydrous magnesium sulfate. The drying agent was removed by filtration and the filtrate was concentrated to obtain a residue. The residue was subjected to silica gel column chromatography (NH silica gel, heptane-ethyl acetate) to obtain 7-[(N-tert-butoxycarbonyl-N-methylamino)methyl]-6-cyclopropylmethoxy-3-{2-[1-(pyridazin-3-yl)piperidin-4-yl]ethyl}benz[d]isoxazole (106 mg) as a crude product. The crude product was used for the next reaction without further purification. The crude product (106 mg) was dissolved in methanol (2 mL) and the solution was ice-cooled. A 4 M solution of hydrogen chloride in ethyl acetate (1 mL) was added to the solution. Then, the mixture was stirred at room temperature. After one hour and 40 minutes, a 4 M solution of hydrogen chloride in ethyl acetate (1 mL) was added and the mixture was further stirred for one hour. Chloroform, a saturated sodium chloride solution and a 1 M sodium hydroxide solution were added to the reaction mixture, and the organic layer was separated. The organic layer was dried over anhydrous magnesium sulfate. The drying agent was removed by filtration. The filtrate was concentrated to obtain a residue. The residue was subjected to silica gel column chromatography (NH silica gel, ethyl acetate) to obtain the title compound (34 mg).
¹H-NMR (400 MHz, CDCl₃) δ (ppm): 0.32-0.44 (m, 2H), 0.60-0.72 (m, 2H), 1.22-1.40 (m, 3H), 1.60-1.75 (m, 1H), 1.75-1.97 (m, 4H), 2.46 (s, 3H), 2.86-3.05 (m, 4H), 3.96 (d, J=7.2 Hz, 2H), 4.15 (br s, 2H), 4.36-4.46 (m, 2H), 6.86-6.95 (m, 2H), 7.17 (dd, J=4.4, 9.2 Hz, 1H), 7.46 (d, J=8.8 Hz, 1H), 8.54 (dd, J=1.2, 4.4 Hz, 1H).

### (14) N-Methyl{6-cyclopropylmethoxy-3-{2-[1-(pyridazin-3-yl)piperidin-4-yl]ethyl}benz[d]isoxazol-7-yl}methylamine fumarate

N-Methyl{6-cyclopropylmethoxy-3-{2-[1-(pyridazin-3-yl)piperidin-4-yl]ethyl}benz[d]isoxazol-7-yl}methylamine (17 mg) and fumaric acid (5 mg) were dissolved in methanol (0.5 mL), and then the solution was concentrated under reduced pressure. tert-Butyl methyl ether was added to the residue and the mixture was concentrated under reduced pressure to obtain the title compound (21 mg).
ESI-MS m/z: 422 [M+H]⁺, 444 [M+Na]⁺.

### Example 77

### N,N-Dimethyl{6-cyclopropylmethoxy-3-{2-[1-(pyridazin-3-yl)piperidin-4-yl]ethyl}benz[d]isoxazol-7-yl}methylamine fumarate

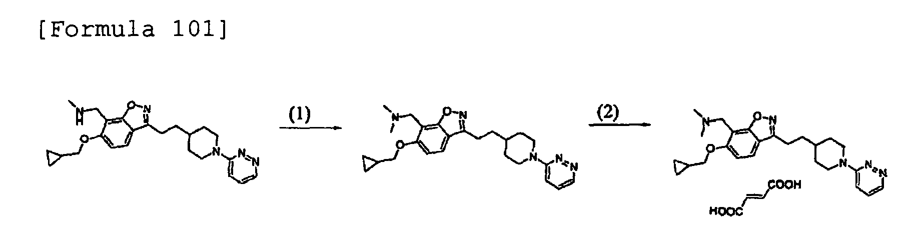

### (1) N,N-Dimethyl{6-cyclopropylmethoxy-3-{2-[1-(pyridazin-3-yl)piperidin-4-yl]ethyl}benz[d]isoxazol-7-yl}methylamine

N-Methyl{6-cyclopropylmethoxy-3-{2-[1-(pyridazin-3-yl)piperidin-4-yl]ethyl}benz[d]isoxazol-7-yl}methylamine (34 mg) was dissolved in methanol (1.5 mL). Acetic acid (10 µL) and formalin (37% solution, 36 µL) were added and the mixture was stirred for five minutes. Then, sodium triacetoxyborohydride (26 mg) was added and the mixture was stirred at room temperature. After one hour and 30 minutes, chloroform, a saturated sodium chloride solution and a 1 M sodium hydroxide solution were added to the reaction mixture, and the organic layer was separated. The organic layer was dried over anhydrous magnesium sulfate. The drying agent was removed by filtration. The filtrate was concentrated to obtain a residue. The residue was subjected to silica gel column chromatography (NH silica gel, heptane-ethyl acetate) to obtain the title compound (34 mg).
¹H-NMR(400 MHz, CDCl₃) δ (ppm): 0.32-0.42 (m, 2H), 0.60-0.72 (m, 2H), 1.23-1.40 (m, 3H), 1.58-1.76 (m, 1H), 1.76-1.96 (m, 4H), 2.40 (br s, 6H), 2.86-3.05 (m, 4H), 3.90 (br s, 2H), 3.96 (d, J=6.8 Hz, 2H), 4.36-4.48 (m, 2H), 6.86-6.96 (m, 2H), 7.17 (dd, J=4.4, 9.2 Hz, 1H), 7.43-7.55 (m, 1H), 8.54 (dd, J=1.2, 4.4 Hz, 1H).

### (2) N,N-Dimethyl{6-cyclopropylmethoxy-3-{2-[1-(pyridazin-3-yl)piperidin-4-yl]ethyl}benz[d]isoxazol-7-yl}methylamine fumarate

N,N-Dimethyl{6-cyclopropylmethoxy-3-{2-[1-(pyridazin-3-yl)piperidin-4-yl]ethyl}benz[d]isoxazol-7-yl}methylamine (34 mg) and fumaric acid (9 mg) were dissolved in methanol (0.5 mL), and then the solution was concentrated under reduced pressure. Acetone was added to the residue and the mixture was concentrated under reduced pressure to obtain the title compound (43 mg).
ESI-MS m/z: 436 [M+H]⁺, 458 [M+Na]⁺.

### Example 78

### N-Methyl{3-{2-[1-(1,3-dioxolan-2-ylmethyl)piperidin-4-yl]ethyl}-6-(4-fluorobenzyloxy)benz[d]isoxazol-7-yl}methylamine dihydrochloride

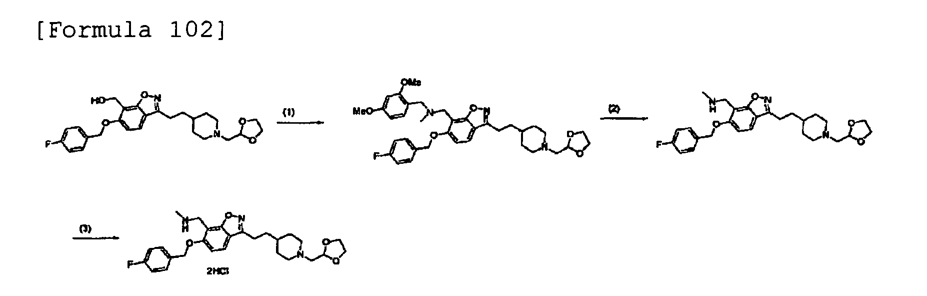

### (1) N-(2,4-Dimethoxybenzyl)-N-methyl{3-{2-[1-(1,3-dioxolan-2-ylmethyl)piperidin-4-yl]ethyl}-6-(4-fluorobenzyloxy)benz[d]isoxazol-7-yl}methylamine

{3-{2-[1-(1,3-Dioxolan-2-ylmethyl)piperidin-4-yl]ethyl}-6-(4-fluorobenzyloxy)benz[d]isoxazol-7-yl}methanol (7.06 g) was dissolved in tetrahydrofuran (50 mL) and N,N-dimethylformamide (5 mL). Triethylamine (6.27 mL) was added and the mixture was ice-cooled. Methanesulfonyl chloride (1.34 mL) was added to the solution, followed by further stirring for 20 minutes. N-Methyl(2,4-dimethoxybenzyl)amine (4.06 g) dissolved in N,N- dimethylformamide (15 mL) was added to the reaction mixture. Then, the mixture was heated to 60°C and further stirred. After five hours, N-methyl(2,4-dimethoxybenzyl)amine (421 mg) was dissolved in N,N-dimethylformamide (1 mL). The solution was added to the reaction mixture, and heating with stirring was further continued for two hours. The reaction mixture was cooled to room temperature and concentrated under reduced pressure. Then, ethyl acetate (200 mL), methanol (10 mL), a 2 M sodium hydroxide solution (20 mL) and water (100 mL) were sequentially added to the residue, and the organic layer was separated. The organic layer was washed with water (100 mL × 2) and a saturated sodium chloride solution (100 mL). The organic layer was dried over anhydrous magnesium sulfate. The drying agent was removed by filtration and the filtrate was concentrated under reduced pressure to obtain a residue. The residue was subjected to silica gel column chromatography (NH silica gel, heptane-ethyl acetate) to obtain the title compound (7.14 g).
¹H-NMR (400 MHz, CDCl₃) δ (ppm) : 1.30-1.44 (m, 3H), 1.66-1.88 (m, 4H), 2.00-2.12 (m, 2H), 2.22 (s, 3H), 2.57 (d, J=4.8 Hz, 2H), 2.88-3.04 (m, 4H), 3.63 (s, 2H), 3.70-4.00 (m, 4H), 3.74 (s, 3H), 3.79 (s, 3H), 3.94 (s, 2H), 5.01 (t, J=4.6 Hz, 1H), 5.15 (s, 2H), 6.36-6.50 (m, 2H), 6.97 (d, J=8.8 Hz, 1H), 7.00-7.10 (m, 2H), 7.25-7.33 (m, 1H), 7.36-7.44 (m, 2H), 7.44 (d, J=8.8 Hz, 1H).

### (2) N-Methyl{3-{2-[1-(1,3-dioxolan-2-ylmethyl)piperidin-4-yl]ethyl}-6-(4-fluorobenzyloxy)benz[d]isoxazol-7-yl}methylamine

N-(2,4-Dimethoxybenzyl)-N-methyl{3-{2-[1-(1,3-dioxolan-2-ylmethyl)piperidin-4-yl]ethyl}-6-(4-fluorobenzyloxy)benz[d]isoxazol-7-yl}methylamine (7.14 g) and triethylamine (2.4 mL) were dissolved in dichloromethane (50 mL), and the solution was ice-cooled. Trifluoroacetic anhydride (2 mL) was added to the solution. The mixture was naturally heated to room temperature while being immersed in an ice bath, and stirred for 12 hours. The reaction mixture was concentrated under reduced pressure to obtain a residue. Methanol (50 mL) and a 5 M sodium hydroxide solution (23 mL) were added to the residue, and the mixture was stirred at room temperature for 30 minutes. The reaction mixture was concentrated under reduced pressure to obtain a residue. A saturated sodium chloride solution (70 mL) and chloroform (150 mL) were added to the residue, and the organic layer was separated. The organic layer was dried over anhydrous magnesium sulfate (20 g). The drying agent was removed by filtration and the filtrate was concentrated under reduced pressure to obtain a residue. The residue was subjected to silica gel column chromatography (NH silica gel, heptane-ethyl acetate and silica gel, ethyl acetate-chloroform-methanol-aqueous ammonia) to obtain the title compound (3.89 g).
¹H-NMR(400 MHz, CDCl₃) δ (ppm): 1.30-1.46 (m, 3H), 1.66-1.90 (m, 4H), 2.02-2.16 (m, 2H), 2.42 (s, 3H), 2.58 (d, J=4.4 Hz, 2H), 2.90-3.08 (m, 4H), 3.81-4.02 (m, 4H), 4.12 (s, 2H), 5.02 (t, J=4.6 Hz, 1H), 5.17 (s, 2H), 6.98 (d, J=8.6 Hz, 1H), 7.05-7.13 (m, 2H), 7.38-7.46 (m, 2H), 7.45 (d, J=8.6 Hz, 1H).

### (3) N-Methyl{3-{2-[1-(1,3-dioxolan-2-ylmethyl)piperidin-4-yl]ethyl}-6-(4-fluorobenzyloxy)benz[d]isoxazol-7-yl}methylamine dihydrochloride

N-Methyl{3-{2-[1-(1,3-dioxolan-2-ylmethyl)piperidin-4-yl]ethyl}-6-(4-fluorobenzyloxy)benz[d]isoxazol-7-yl}methylamine (166 mg) was dissolved in methanol (3 mL), and a 4 M solution of hydrogen chloride in ethyl acetate (300 µL) was added. The solution was concentrated under reduced pressure to obtain a residue. Acetone (6 mL) was added to the residue and the mixture was concentrated under reduced pressure again to obtain the title compound (191 mg).
ESI-MS m/z: 484 [M+H]⁺, 506 [M+Na]⁺.

Example 79

### N,N-Dimethyl{3-[2-(1-benzylpiperidin-4-yl)ethyl]-6-ethoxymethylbenz[d]isoxazol-7-yl}methylamine dihydrochloride

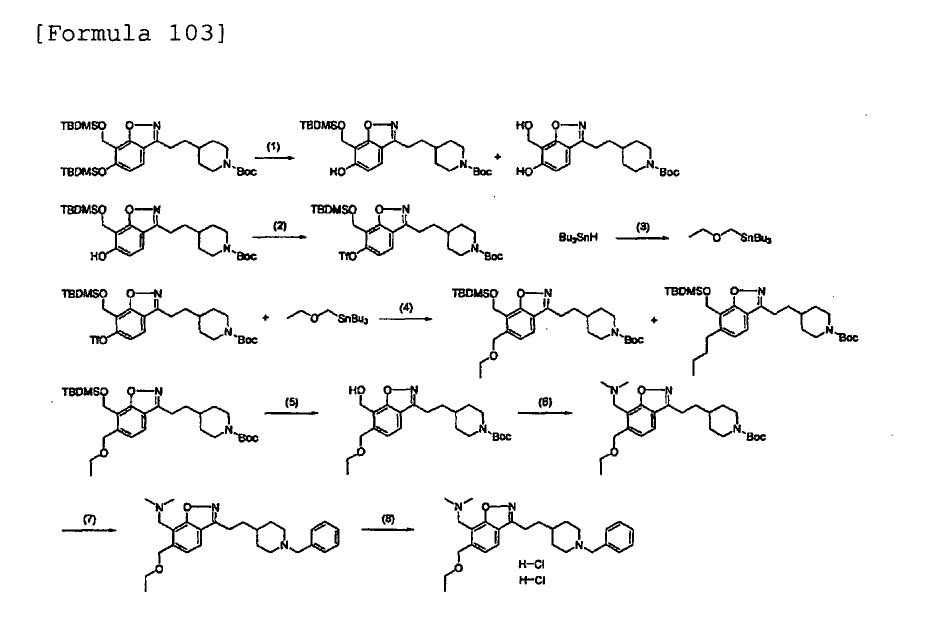

### (1) tert-Butyl 4-{2-[7-(tert-butyldimethylsilanyloxymethyl)-6-hydroxybenz[d]isoxazol-3-yl]ethyl}piperidine-1-carboxylate and tert-butyl 4-[2-(6-hydroxy-7-hydroxymethylbenz[d]isoxazol-3-yl)ethyl]piperidine-1-carboxylate

The title compounds were synthesized with reference to Tetrahedron Lett., 2002, 43, 4729. tert-Butyl 4-{2-[6-(tert-butyldimethylsilanyloxy)-7-(tert-butyldimethylsilanyloxymethyl)benz[d]isoxazol-3-yl]ethyl}piperidine-1-carboxylate shown in Preparation Example 2-(5) (12.4 g) was dissolved in N,N-dimethylformamide (50 mL). Lithium hydroxide (0.6 g) was added and the mixture was stirred at room temperature for 2.5 hours. A saturated ammonium chloride solution and water were sequentially added to the reaction mixture, followed by extraction with ethyl acetate twice. The organic layers were sequentially washed with water and a saturated sodium chloride solution, dried over magnesium sulfate and filtered. Then, the solvent was evaporated under reduced pressure. The residue was purified by silica gel column chromatography (heptane-ethyl acetate) to obtain the title compounds, tert-Butyl 4-{2-[7-(tert-butyldimethylsilanyloxymethyl)-6-hydroxybenz[d]isoxazol-3-yl]ethyl}piperidine-1-carboxylate (5.72 g) and tert-butyl 4-[2-(6-hydroxy-7-hydroxymethylbenz[d]isoxazol-3-yl)ethyl]piperidine-1-carboxylate (2.84 g), respectively.

### tert-Butyl 4-{2-[7-(tert-butyldimethylsilanyloxymethyl)-6-hydroxybenz[d]isoxazol-3-yl]ethyl}piperidine-1-carboxylate

¹H-NMR (400 MHz, CDCl₃) δ (ppm) : 0.20 (s, 6H), 0.96 (s, 9H), 1.15 (ddd, J=4.4 Hz, 12.4 Hz, 24.4 Hz, 2H), 1.44-1.56 (m, 1H), 1.47 (s, 9H), 1.69-1.81 (m, 4H), 2.67 (br t, J=12.4 Hz, 2H), 2.93 (t, J=8.0 Hz, 2H), 3.99-4.20 (m, 2H), 5.26 (s, 2H), 6.83 (d, J=8.4 Hz, 1H), 7.35 (d, J=8.4 Hz, 1H), 8.95 (s, 1H).

### tert-Butyl 4-[2-(6-hydroxy-7-hydroxymethylbenz[d]isoxazol-3-yl)ethyl]piperidine-1-carboxylate

¹H-NMR (400 MHz, CDCl₃) δ(ppm): 1.13 (ddd, J=4.4 Hz, 12.4 Hz, 24.6 Hz, 2H), 1.44-1.52 (m, 1H), 1.45 (s, 9H), 1.68-1.78 (m, 4H), 2.58-2.73 (m, 2H), 2.92 (t, J=8.0 Hz, 2H), 3.97-4.15 (m, 2H), 5.25 (s, 2H), 6.86 (d, J=8.4 Hz, 1H), 7.36 (d, J=8.4 Hz, 1H).

### (2) tert-Butyl 4-{2-[7-(tert-butyldimethylsilanyloxymethyl)-6-trifluoromethanesulfonyloxybenz[d]isoxazol-3-yl]ethyl}piperidine-1-carboxylate

tert-Butyl 4-{2-[7-(tert-butyldimethylsilanyloxymethyl)-6-hydroxybenz[d]isoxazol-3-yl]ethyl}piperidine-1-carboxylate (11.5 g) and pyridine (10 mL) were dissolved in methylene chloride (100 mL) in a nitrogen atmosphere. Trifluoromethanesulfonic anhydride (5 mL) was added dropwise under ice-cooling, and then the mixture was stirred at room temperature for 55 minutes. Concentrated aqueous ammonia was added to the reaction mixture until the pH was 7, followed by extraction with methylene chloride. The organic layer was sequentially washed with water and a saturated sodium chloride solution, dried over magnesium sulfate and filtered. Then, the solvent was evaporated under reduced pressure. The residue was purified by silica gel column chromatography (heptane-ethyl acetate) to obtain the title compound (14.55 g).
¹H-NMR (400 MHz, CDCl₃) δ (ppm) : 0.14 (s, 6H), 0.91 (s, 9H), 1.11-1.25 (m, 2H), 1.44-1.55 (m, 1H), 1.46 (s, 9H), 1.75 (br d, J=14.4 Hz, 2H), 1.81 (dt, J=7.2 Hz, 8.0 Hz, 2H), 2.69 (br t, J=12.4 Hz, 2H), 3.01 (t, J=8.0 Hz, 2H), 4.02-4.18 (m, 2H), 5.06 (s, 2H), 7.25 (d, J=8.4 Hz, 1H), 7.60 (d, J=8.4 Hz, 1H).

### (3) Tributyl(ethoxymethyl)tin

Diisopropylamine (5.8 mL) was dissolved in tetrahydrofuran (100 mL) in a nitrogen atmosphere. n-Butyllithium (2.59 M solution in hexane) (15 mL) was added dropwise at -78°C over six minutes. The mixture was stirred at -78°C for 28 minutes to prepare a solution of lithium diisopropylamide in tetrahydrofuran. Tributyltin hydride (10 mL) was added dropwise to the solution at -78°C over two minutes, and the mixture was stirred at -78°C for eight minutes and under ice-cooling for 30 minutes. Chloromethyl ethyl ether (3.8 mL) was added dropwise at -78°C over five minutes, and the mixture was stirred at -78°C for 10 minutes and at room temperature for 20 hours and 47 minutes. A saturated ammonium chloride solution and water were sequentially added to the reaction mixture, followed by extraction with diethyl ether. The organic layer was washed with a saturated sodium chloride solution, dried over magnesium sulfate and filtered. Then, the solvent was evaporated under reduced pressure. The residue was purified by silica gel column chromatography (heptane-diethyl ether) to obtain the title compound (12.2 g).
¹H-NMR (400 MHz, CDCl₃) δ (ppm) : 0.05-0.95 (m, 15H), 1.16 (t, J=6.8 Hz, 3H), 1.30 (tq, J=7.2 Hz, 7.2 Hz, 6H), 1.42-1.60 (m, 6H), 3.35 (q, J=6.8 Hz, 2H), 3.71-3.75 (m, 2H).

### (4) tert-Butyl 4-{2-[7-(tert-butyldimethylsilanyloxymethyl)-6-ethoxymethylbenz[d]isoxazol-3-yl]ethyl}piperidine-1-carboxylate and tert-butyl 4-{2-[6-butyl-7-(tert-butyldimethylsilanyloxymethyl)benz[d]isoxazol-3-yl]ethyl}piperidine-1-carboxylate

A mixture of tert-butyl 4-{2-[7-(tert-butyldimethylsilanyloxymethyl)-6-trifluoromethanesulfonyloxybenz[d]isoxazol-3-yl]ethyl}piperidine-1-carboxylate shown in Example 79-(2) (1 g), tributyl(ethoxymethyl)tin (0.8 g), dichlorobis(triphenylphosphine)palladium (II) (0.15 g), lithium chloride (0.25 g) and 1-methyl-2-pyrrolidinone (5 mL) was stirred in a nitrogen atmosphere at 80°C for 16 hours and 15 minutes. The reaction mixture was diluted with ethyl acetate. Water and potassium fluoride (0.2 g) were sequentially added and the mixture was stirred at room temperature for five minutes. The precipitate was removed by filtration through celite and washed with ethyl acetate. The filtrate and the washing liquid were mixed and the solvent was evaporated under reduced pressure. The aqueous suspension of the residue was extracted with ethyl acetate. The organic layer was sequentially washed with water (three times) and a saturated sodium chloride solution and dried over magnesium sulfate. The drying agent was removed by filtration and the solvent was evaporated under reduced pressure. The residue was purified by silica gel column chromatography (heptane-ethyl acetate) to obtain tert-butyl 4-{2-[7-(tert-butyldimethylsilanyloxymethyl)-6-ethoxymethylbenz[d]isoxazol-3-yl]ethyl}piperidine-1-carboxylate (207 mg) and tert-butyl 4-{2-[6-butyl-7-(tert-butyldimethylsilanyloxymethyl)benz[d]isoxazol-3-yl)ethyl}piperidine-1-carboxylate (96 mg), respectively.

### tert-Butyl 4-{2-[7-(tert-butyldimethylsilanyloxymethyl)-6-ethoxymethylbenz[d]isoxazol-3-yl]ethyl}piperidine-1-carboxylate

¹H-NMR (400 MHz, CDCl₃) δ(ppm): 0.09 (s, 6H), 0. 89 (s, 9H), 1.09-1.22 (m, 2H), 1.28 (t, J=7.2 Hz, 3H), 1.42-1.52 (m, 1H), 1.45 (s, 9H), 1.68-1.84 (m, 4H), 2.60-2.76 (m, 2H), 3.00 (br t, J=8.0 Hz, 2H), 3.60 (q, J=7.2 Hz, 2H), 4.02-4.17 (m, 2H), 4.79 (s, 2H), 5.06 (s, 2H), 7.44 (d, J=8.4 Hz, 1H) , 7.52 (d, J=8.4 Hz, 1H).

### tert-Butyl 4-{2-[6-butyl-7-(tert-butyldimethylsilanyloxymethyl)benz[d]isoxazol-3-yl]ethyl}piperidine-1-carboxylate

¹H-NMR (400 MHz, CDCl₃) δ(ppm): 0.11 (s, 6H), 0.90 (s, 9H), 0.95 (t, J=7.2 Hz, 3H), 1.09-1.22 (m, 2H), 1.24-1.40 (m, 4H), 1.39-1.48 (m, 1H), 1.45 (s, 9H), 1.69-1.83 (m, 4H), 2.61-2.74 (m, 2H), 2.86 (br t, J=8.0 Hz, 2H), 2.97 (t, J=8.0 Hz, 2H), 4.01-4.16 (m, 2H), 5.01 (s, 2H), 7.14 (d, J=8.0 Hz, 1H), 7.44 (d, J=8.0 Hz, 1H) .

### (5) tert-Butyl 4-[2-(6-ethoxymethyl-7-hydroxymethylbenz[d]isoxazol-3-yl)ethyl]piperidine-1-carboxylate

tert-Butyl 4-{2-[7-(tert-butyldimethylsilanyloxymethyl)-6-ethoxymethylbenz[d]isoxazol-3-yl]ethyl}piperidine-1-carboxylate (392 mg) was dissolved in tetrahydrofuran (5 mL). Tetrabutylammonium fluoride (1 M solution in tetrahydrofuran) (1.1 mL) was added and the mixture was stirred at room temperature for 50.5 hours. Water was added to the reaction mixture, followed by extraction with ethyl acetate. The organic layer was sequentially washed with water (three times) and a saturated sodium chloride solution, dried over magnesium sulfate and filtered. Then, the solvent was evaporated under reduced pressure. The residue was purified by silica gel column chromatography (heptane-ethyl acetate) to obtain the title compound (220 mg).
¹H-NMR (400 MHz, CDCl₃) δ(ppm): 1. 16 (ddd, J=4.0 Hz, 12. 4 Hz, 24.4 Hz, 2H), 1.27 (t, J=7.2 Hz, 3H), 1.43-1.54 (m, 1H), 1.45 (s, 9H), 1.69-1.84 (m, 4H), 2.60-2.76 (m, 2H), 3.01 (t, J=7.6 Hz, 2H), 3.22 (t, J=6.4 Hz, 1H), 3.64 (q, J=7.2 Hz, 2H), 4.01-4.18 (m, 2H), 4.75 (s, 2H) , 5.03 (d, J=6. 4 Hz, 2H), 7.29 (d, J=8.0 Hz, 1H) , 7.53 (d, J=8.0 Hz, 1H) .

### (6) tert-Butyl 4-{2-[7-(N,N-dimethylaminomethyl)-6-ethoxymethylbenz[d]isoxazol-3-yl]ethyl}piperidine-1-carboxylate

tert-Butyl 4-[2-(6-ethoxymethyl-7-hydroxymethylbenz[d]isoxazol-3-yl)ethyl]piperidine-1-carboxylate (218 mg) and triethylamine (0.145 mL) were dissolved in tetrahydrofuran (4 mL) in a nitrogen atmosphere. Methanesulfonyl chloride (60 µL) was added and the mixture was stirred at room temperature for 40 minutes. Dimethylamine (2 M solution in tetrahydrofuran) (2 mL) was added to the reaction mixture, followed by stirring at room temperature for two hours. Water was added to the reaction mixture, followed by extraction with ethyl acetate. The organic layer was sequentially washed with water and a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (165 mg).
¹H-NMR (400 MHz, CDCl₃) δ (ppm) : 1.16 (ddd, J=4.0 Hz, 12.4 Hz, 24.0 Hz, 2H), 1.27 (t, J=7.2 Hz, 3H), 1.45 (s, 9H), 1.48-1.56 (m, 1H), 1.71-1.84 (m, 4H), 2.26 (s, 6H), 2.67 (br t, J=12.4 Hz, 2H), 2.99 (t, J=8.0 Hz, 2H), 3.60 (q, J=7.2 Hz, 2H), 3.77 (s, 2H), 4.02-4.17 (m, 2H), 4.78 (s, 2H), 7.44 (d, J=8.0 Hz, 1H), 7.51 (d, J=8.0 Hz, 1H).

### (7) N,N-Dimethyl{3-[2-(1-benzylpiperidin-4-yl)ethyl]-6-ethoxymethylbenz[d]isoxazol-7-yl}methylamine

tert-Butyl 4-{2-[7-(N,N-dimethylaminomethyl)-6-ethoxymethylbenz[d]isoxazol-3-yl]ethyl}piperidine-1-carboxylate (163 mg) was dissolved in methanol (1 mL). Hydrogen chloride (4 M solution in ethyl acetate) (3 mL) was added and the mixture was stirred at room temperature for one hour and 40 minutes. The solvent was evaporated under reduced pressure. Concentrated aqueous ammonia was added until the pH was 7, followed by extraction with methylene chloride. The organic layer was dried over magnesium sulfate and filtered. The solvent was evaporated under reduced pressure to obtain a crude product of N,N-dimethyl{6-ethoxymethyl-3-[2-(piperidin-4-yl)ethyl]benz[d]isoxazol-7-yl}methylamine. The crude product and benzaldehyde (80 mg) were dissolved in methylene chloride (4 mL). Acetic acid (0.11 mL) and sodium triacetoxyborohydride (160 mg) were sequentially added and the mixture was stirred at room temperature for 15 hours. Ethyl acetate and 2 M hydrochloric acid were sequentially added to the reaction mixture, and the organic layer was extracted with 2 M hydrochloric acid. The resulting aqueous layer was washed with ethyl acetate. A 5 M sodium hydroxide solution was added until the pH was 7, followed by extraction with ethyl acetate. The organic layer was sequentially washed with water and a saturated sodium chloride solution, dried over magnesium sulfate and filtered. Then, the solvent was evaporated under reduced pressure. The residue was purified by NH silica gel column chromatography (heptane-ethyl acetate) to obtain the title compound (137 mg).
¹H-NMR (400 MHz, CDCl₃) δ(ppm): 1.27 (t, J=7.2 Hz, 3H), 1.28-1.37 (m, 3H), 1.69-1.82 (m, 4H), 1.94 (br t, J=11.2 Hz, 2H), 2.25 (s, 6H), 2. 88 (br d, J=11.6 Hz, 2H), 2.97 (t, J=8.0 Hz, 2H), 3.48 (s, 2H), 3.60 (q, J=7.2 Hz, 2H), 3.76 (s, 2H), 4.77 (s, 2H), 7.22-7.32 (m, 5H), 7.42 (d, J=8.0 Hz, 1H), 7.50 (d. J=8.0 Hz, 1H) .

### (8) N,N-Dimethyl{3-[2-(1-benzylpiperidin-4-yl)ethyl]-6-ethoxymethylbenz[d]isoxazol-7-yl}methylamine dihydrochloride

Hydrogen chloride (4 M solution in ethyl acetate) (0.3 mL) was added to a solution of N,N-dimethyl{3-[2-(1-benzylpiperidin-4-yl)ethyl]-6-ethoxymethylbenz[d]isoxazol-7-yl}methylamine (135 mg) in methanol. The solvent was evaporated under reduced pressure and then the residue was dried under reduced pressure to obtain the title compound (147 mg).
¹H-NMR (400 MHz, CD₃OD) δ(ppm): 1.32 (t, J=7.2 Hz, 3H), 1.53 (ddd, J=4.0 Hz, 13.6 Hz, 25.6 Hz, 2H), 1.67-1.79 (m, 1H), 1.86 (dt, J=7.2 Hz, 8.0 Hz, 2H), 2.09 (br d, J=14.4 Hz, 2H), 2.94-3.06 (m, 2H), 2.99 (s, 6H), 3.10 (t, J=8.0 Hz, 2H), 3.50 (br d, J=12.4 Hz, 2H), 3.77 (t, J=7.2 Hz, 2H), 4.30 (s, 2H), 4.76 (s, 2H), 4.86 (s, 2H), 7.47-7.56 (m, 6H), 7.95 (d, J=8.0 Hz, 1H).
ESI-MS m/z: 219 [M+2H]²⁺, 436 [M+H]⁺.

### Example 80

### N,N-Dimethyl{3-[2-(1-benzylpiperidin-4-yl)ethyl]-6-butylbenz[d]isoxazol-7-yl}methylamine dihydrochloride

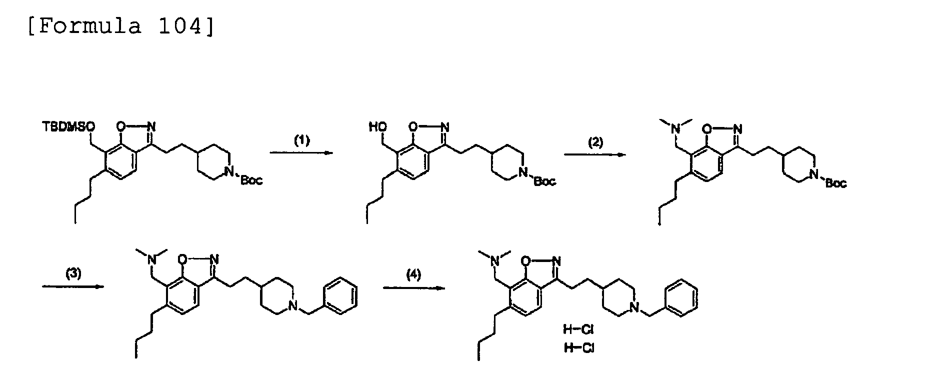

### (1) tert-Butyl 4-[2-(6-butyl-7-hydroxymethylbenz[d]isoxazol-3-yl)ethyl]piperidine-1-carboxylate

The title compound (165 mg) was obtained by synthesis from tert-butyl 4-{2-[6-butyl-7-(tert-butyldimethylsilanyloxymethyl)benz[d]isoxazol-3-yl]ethyl}piperidine-1-carboxylate shown in Example 79-(4) (298 mg) according to Example 79-(5).
¹H-NMR (400 MHz, CDCl₃) δ (ppm) : 0.95 (t, J=7.2 Hz, 3H), 1.10-1.24 (m, 2H), 1.25-1.42 (m, 4H), 1.45 (s, 9H), 1.48-1.56 (m, 1H) , 1.70-1.83 (m, 4H), 2.00 (t, J=6. 4 Hz, 1H) , 2 . 61-2. 74 (m, 2H), 2.86 (t, J=8.0 Hz, 2H), 2.99 (t, J=7.6 Hz, 2H), 4.01-4.17 (m, 2H), 5.03 (d, J=6.4 Hz, 2H), 7.15 (d, J=8. 4 Hz, 1H) , 7.47 (d, J=8. Hz, 1H) .

### (2) tert-Butyl 4-{2-[6-butyl-7-(N,N-dimethylaminomethyl)benz[d]isoxazol-3-yl]ethyl}piperidine-1-carboxylate

The title compound (120 mg) was obtained by synthesis from tert-butyl 4-[2-(6-butyl-7-hydroxymethylbenz[d]isoxazol-3-yl)ethyl]piperidine-1-carboxylate (163 mg) according to Example 79-(6).
¹H-NMR (400 MHz, CDCl₃) δ(ppm): 0.96 (t, J=7.2 Hz, 3H) , 1.16 (ddd, J=4.4 Hz, 12.4 Hz, 24.4 Hz, 2H), 1.36-1.47 (m, 2H), 1.45 (s, 9H), 1.47-1.57 (m, 1H), 1.54-1.65 (m, 2H), 1.70-1.83 (m, 4H), 2.28 (s, 6H), 2.68 (br t, J=12. 8 Hz, 2H), 2.87 (t, J=8.0 Hz, 2H), 2.97 (t, J=8.0 Hz, 2H), 3.71 (s, 2H), 4.04-4.16 (m, 2H), 7.14 (d, J=8.0 Hz, 1H), 7.43 (d, J=8.0 Hz, 1H).

### (3) N,N-Dimethyl{3-[2-(1-benzylpiperidin-4-yl)ethyl]-6-butylbenz[d]isoxazol-7-yl}methylamine

The title compound (96 mg) was obtained by synthesis from tert-butyl 4-{2-[6-butyl-7-(N,N-dimethylaminomethyl)benz[d]isoxazol-3-yl]ethyl}piperidine-1-carboxylate (118 mg) according to Example 79- (7) .
¹H-NMR (400 MHz, CDCl₃) δ(ppm): 0.96 (t, J=7.2 Hz, 3H), 1.25-1.47 (m, 5H), 1.55-1.65 (m, 2H), 1.70-1.82 (m, 4H), 1.94 (br t, J=11.2 Hz, 2H), 2,27 (s, 6H), 2.83-2.91 (m, 4H), 2.95 (br t, J=8.0 Hz, 2H), 3.48 (s, 2H), 3.71 (s, 2H), 7.13 (d, J=8.0 Hz, 1H), 7.20-7.33 (m, 5H), 7.42 (d, J=8.0 Hz, 1H).

### (4) N,N-Dimethyl{3-[2-(1-benzylpiperidin-4-yl)ethyl]-6-butylbenz[d]isoxazol-7-yl}methylamine dihydrochloride

The title compound (98 mg) was obtained by synthesis from N,N-dimethyl{3-[2-(1-benzylpiperidin-4-yl)ethyl]-6-butylbenz[d]isoxazol-7-yl}methylamine (94 mg) according to Example 79-(8).
¹H-NMR (400 MHz, CD₃OD) δ(ppm): 0. 98 (t, J=7.2 Hz, 3H), 1.40-1.78 (m, 7H), 1.85 (dt, J=7.2 Hz, 8.0 Hz, 2H), 2.08 (br d, J=14.0 Hz, 2H), 2.89-3.15 (m, 12H), 3.50 (br d, J=12.4 Hz, 2H), 4.30 (s, 2H), 4.72 (s, 2H), 7.42 (d, J=8.0 Hz, 1H), 7. 47-7. 57 (m, 5H), 7. 88 (d, J=8.0 Hz, 1H) .
ESI-MS m/z: 218 [M+2H]²⁺, 434 [M+H]⁺.

### Example 81

### N,N-Dimethyl{3-[2-(1-benzylpiperidin-4-yl)ethyl]-6-(3-methyl-2-butenyl)benz[d]isoxazol-7-yl}methylamine fumarate

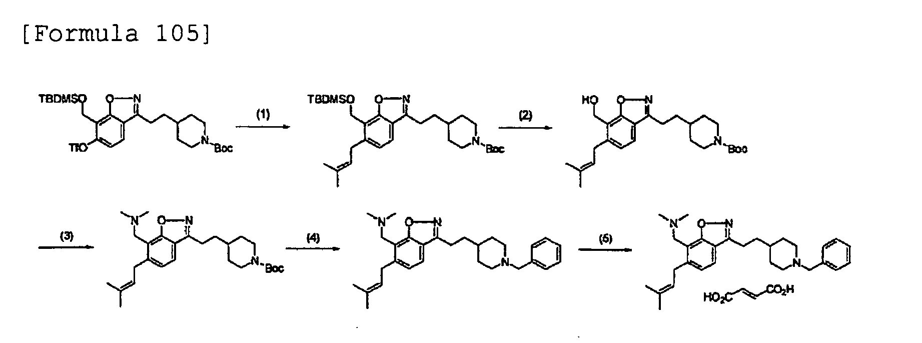

### (1) tert-Butyl 4-{2-[7-(tert-butyldimethylsilanyloxymethyl)-6-(3-methyl-2-butenyl)benz[d]isoxazol-3-yl]ethyl}piperidine-1-carboxylate

The title compound (373 mg) was obtained by synthesis from tert-butyl 4-{2-[7-(tert-butyldimethylsilanyloxymethyl)-6-trifluoromethanesulfonyloxybenz[d]isoxazol-3-yl]ethyl}piperidine-1-carboxylate shown in Example 79-(2) (1 g) and tributyl(3-methyl-2-butenyl)tin (0.8 g) according to Example 79-(4).
¹H-NMR (400 MHz, CDCl₃) δ(ppm): 0.10 (s, 6H), 0.90 (s, 9H), 1.08-1.23 (m, 2H), 1.43-1.54 (m, 1H) , 1 . 45 (s, 9H), 1.69-1.84 (m, 10H), 2.59-2.74 (m, 2H), 2.97 (t, J=8 . 0 Hz, 2H), 3.60 (d, J=6.8 Hz, 2H), 4.01-4.17 (m, 2H), 5.02 (s, 2H), 5.24-5.32 (m, 1H) , 7. 14 (d, J=8.4 Hz, 1H) , 7.44 (d, J=8 . 4 Hz, 1H).

### (2) tert-Butyl 4-{2-[7-hydroxymethyl-6-(3-methyl-2-butenyl)benz[d]isoxazol-3-yl]ethyl}piperidine-1-carboxylate

The title compound (263 mg) was obtained by synthesis from tert-butyl 4-{2-[7-(tert-butyldimethylsilanyloxymethyl)-6-(3-methyl-2-butenyl)benz[d]isoxazol-3-yl]ethyl}piperidine-1-carboxylate (370 mg) according to Example 79-(5).
¹H-NMR (400 MHz, CDCl₃) δ (ppm): 1.16 (ddd, J=4.4 Hz, 12.4 Hz, 24.4 Hz, 2H), 1.45 (s, 9H), 1.46-1.54 (m, 1H), 1.69-1.82 (m, 10H), 1.98 (t, J=6.4 Hz, 1H), 2.60-2.74 (m, 2H), 2.99 (t, J=7.6 Hz, 2H), 3.59 (d, J=6.8 Hz, 2H), 4.02-4.17 (m, 2H), 5.03 (d, J=6.4 Hz, 2H), 5.22-5.29 (m, 1H) , 7.17 (d, J=8.0 Hz, 1H), 7.47 (d, J=8.0 Hz, 1H).

### (3) tert-Butyl 4-{2-[7-(N,N-dimethylaminomethyl)-6-(3-methyl-2-butenyl)benz[d]isoxazol-3-yl]ethyl}piperidine-1-carboxylate

The title compound (247 mg) was obtained by synthesis from tert-butyl 4-{2-[7-hydroxymethyl-6-(3-methyl-2-butenyl)benz[d]isoxazol-3-yl]ethyl}piperidine-1-carboxylate (261 mg) according to Example 79-(6).
¹H-NMR (400 MHz, CDCl₃) δ(ppm): 1.16 (ddd, J=4.0 Hz, 12.0 Hz, 24.0 Hz, 2H), 1.45 (s, 9H), 1.46-1.56 (m, 1H), 1.70-1.83 (m, 10H), 2.28 (s, 6H), 2.68 (br t, J=11.6 Hz, 2H), 2.97 (t, J=8.0 Hz, 2H), 3.62 (d, J=7.2 Hz, 2H), 3.72 (s, 2H), 4.01-4.17 (m, 2H), 5.19-5.26 (m, 1H), 7.14 (d, J=8.4 Hz, 1H), 7.43 (d, J=8.4 Hz, 1H).

### (4) N,N-Dimethyl{3-[2-(1-benzylpiperidin-4-yl)ethyl]-6-(3-methyl-2-butenyl)benz[d]isoxazol-7-yl}methylamine

The title compound (46 mg) was obtained by synthesis from tert-butyl 4-{2-[7-(N,N-dimethylaminomethyl)-6-(3-methyl-2-butenyl)benz[d]isoxazol-3-yl]ethyl}piperidine-1-carboxylate (244 mg) according to Example 79-(7).
¹H-NMR (400 MHz, CDCl₃) δ(ppm): 1.26-1.40 (m, 3H), 1.70-1.82 (m, 4H), 1.73 (s, 3H), 1.77 (s, 3H), 1.94 (br d, J=11.2 Hz, 2H), 2.27 (s, 6H), 2.88 (br d, J=12.0 Hz, 2H), 2. 95 (t, J=8.0 Hz, 2H), 3. 48 (s, 2H), 3.62 (d, J=7.2 Hz, 2H), 3.71 (s, 2H), 5,18-5.25 (m, 1H), 7.13 (d, J=8.0 Hz, 1H), 7.19-7.32 (m, 5H), 7.42 (d, J=8.0 Hz, 1H).

### (5) N,N-Dimethyl{3-[2-(1-benzylpiperidin-4-yl)ethyl]-6-(3-methyl-2-butenyl)benz[d]isoxazol-7-yl}methylamine fumarate

Fumaric acid (11.5 mg) was added to a solution of N,N-dimethyl{3-[2-(1-benzylpiperidin-4-yl)ethyl]-6-(3-methyl-2-butenyl)benz[d]isoxazol-7-yl}methylamine (44 mg) in methanol. The solvent was evaporated under reduced pressure and then the residue was dried under reduced pressure to obtain the title compound (47 mg) .
¹H-NMR (400 MHz, CD₃OD) δ (ppm) : 1.42-1.56 (m, 2H), 1.57-1.68 (m, 1H), 1.75 (s, 3H), 1.78-1.88 (m, 2H), 1.79 (s, 3H), 2.01 (br d, J=13.2 Hz, 2H), 2.51 (s, 6H), 2.85 (br t, J=12.0 Hz, 2H), 3.04 (t, J=7.2 Hz, 2H), 3.39 (br d, J=12.4 Hz, 2H), 3.64 (br d, J=7.2 Hz, 2H), 4.10 (s, 2H), 4.18 (s, 2H), 5.20-5.26 (m, 1H) , 6.67 (s, 2H), 7.27 (d, J=8.0 Hz, 1H), 7.42-7.50 (m, 5H), 7.70 (d, J=8.0 Hz, 1H).
ESI-MS m/z: 224 [M+2H]2^{+,} 446 [M+H]⁺.

### Example 82

### N,N-Dimethyl{3-[2-(1-benzylpiperidin-4-yl)ethyl]-6-(2-methoxyethyl)benz[d]isoxazol-7-yl}methylamine dihydrochloride

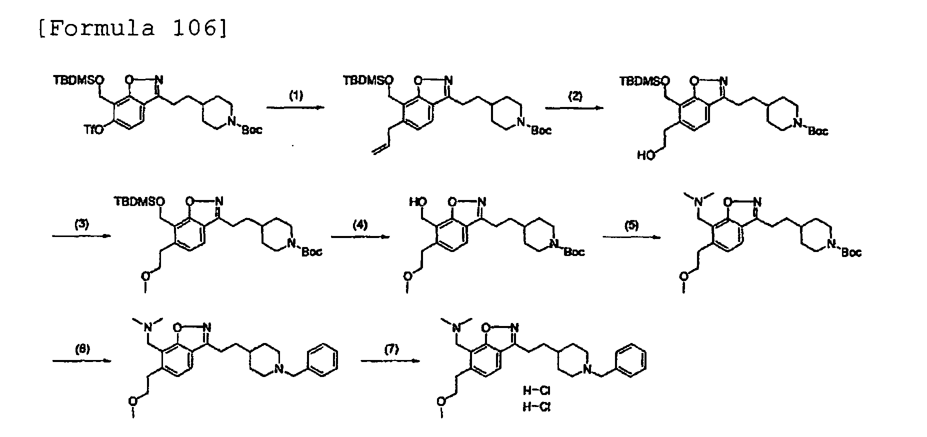

### (1) tert-Butyl 4-{2-[6-allyl-7-(tert-butyldimethylsilanyloxymethyl)benz[d]isoxazol-3-yl]ethyl}piperidine-1-carboxylate

The title compound (2.9 g) was obtained by synthesis from tert-butyl 4-{2-[7-(tert-butyldimethylsilanyloxymethyl)-6-trifluoromethanesulfonyloxybenz[d]isoxazol-3-yl]ethyl}piperidine-1-carboxylate shown in Example 79-(2) (6 g) and allyltributyltin (10 g) according to Example 79-(4).
¹H-NMR (400 MHz, CDCl₃) δ(ppm): 0.10 (s, 6H), 0.90 (s, 9H), 1.10-1.21 (m, 2H), 1.43-1.55 (m, 1H) , 1.45 (s, 9H), 1.70-1.83 (m, 4H), 2.67 (br t, J=11.6 Hz, 2H), 2.98 (t, J=7.6 Hz, 2H), 3.67 (ddd, J=1.6 Hz, 1.6 Hz, 6.4 Hz, 2H), 4.01-4.16 (m, 2H), 5.02 (s, 2H), 5.04 (ddt, J=1.6 Hz, 1.6 Hz, 17.2 Hz, 1H), 5.07 (ddt, J=1.6 Hz, 1.6 Hz, 10.4 Hz, 1H), 5.99 (ddt, J=6.4 Hz, 10.4 Hz, 17.0 Hz, 1H), 7.15 (d, J=8.0 Hz, 1H), 7.46 (d, J=8.0 Hz, 1H).

### (2) tert-Butyl 4-{2-[7-(tert-butyldimethylsilanyloxymethyl)-6-(2-hydroxyethyl)benz[d]isoxazol-3-yl]ethyl}piperidine-1-carboxylate

tert-Butyl 4-{2-[6-allyl-7-(tert-butyldimethylsilanyloxymethyl)benz[d]isoxazol-3-yl]ethyl}piperidine-1-carboxylate (265 mg) was dissolved in tetrahydrofuran (6 mL). Water (1.5 mL) and osmium tetroxide (2.5% aqueous solution) (0.16 mL) were sequentially added to the solution, and the mixture was stirred at room temperature for five minutes. Then, sodium metaperiodate (280 mg) was added at room temperature and the mixture was stirred at room temperature for 3.5 hours. The organic layer was extracted with ethyl acetate. The organic layer was sequentially washed with water and a saturated sodium chloride solution, dried over magnesium sulfate and filtered. Then, the solvent was evaporated under reduced pressure to obtain a crude product of tert-butyl 4-{2-[7-(tert-butyldimethylsilanyloxymethyl)-6-(2-formylmethyl)benz[d]isoxazol-3-yl]ethyl}piperidine-1-carboxylate (263 mg). The crude product (261 mg) was dissolved in methanol (5 mL). Sodium borohydride (30 mg) was added under ice-cooling, and the mixture was stirred under ice-cooling for 30 minutes. A saturated ammonium chloride solution and water were sequentially added to the reaction mixture, followed by extraction with ethyl acetate. The organic layer was sequentially washed with water and a saturated sodium chloride solution, dried over magnesium sulfate and filtered. Then, the solvent was evaporated under reduced pressure. The residue was purified by NH silica gel column chromatography (heptane-ethyl acetate) to obtain the title compound (133 mg). ¹H-NMR (400 MHz, CDCl₃) δ(ppm): 0. 18 (s, 6H), 0. 93 (s, 9H), 1.10-1.23 (m, 2H), 1.43-1.54 (m, 1H), 1.45 (s, 9H), 1.70-1.84 (m, 4H), 2.62-2.74 (m, 2H), 2.72 (t, J=5.6 Hz, 1H), 2.99 (t, J=8.0 Hz, 2H), 3.13 (t, J=6.0 Hz, 2H), 3.93 (dt, J=5.6 Hz, 6.0 Hz, 2H), 4.02-4.17 (m, 2H), 5.06 (s, 2H), 7.21 (d, J=8.0 Hz, 1H), 7.52 (d, J=8.0 Hz, 1H).

### (3) tert-Butyl 4-{2-[7-(tert-butyldimethylsilanyloxymethyl)-6-(2-methoxyethyl)benz[d]isoxazol-3-yl]ethyl}piperidine-l-carboxylate

tert-Butyl 4-{2-[7-(tert-butyldimethylsilanyloxymethyl)-6-(2-hydroxyethyl)benz[d]isoxazol-3-yl]ethyl}piperidine-1-carboxylate (130 mg) and methyl iodide (47 µL) were dissolved in N,N-dimethylformamide (2.5 mL) in a nitrogen atmosphere. 60% sodium hydride (15 mg) was added under ice-cooling, and the mixture was stirred at room temperature for one hour and 25 minutes. A saturated ammonium chloride solution was added to the reaction mixture, followed by extraction with ethyl acetate. The organic layer was sequentially washed with water (three times) and a saturated sodium chloride solution, dried over magnesium sulfate and filtered. Then, the solvent was evaporated under reduced pressure. The residue was purified by NH silica gel column chromatography (heptane-ethyl acetate) to obtain the title compound (128 mg). ¹H-NMR (400 MHz, CDCl₃) δ(ppm): 0.11 (s, 6H), 0.90 (s, 9H), 1.10-1.23 (m, 2H), 1.43-1.55 (m, 1H), 1.45 (s, 9H), 1.70-1.84 (m, 4H), 2.61-2.73 (m, 2H), 2.98 (t, J=8.0 Hz, 2H), 3.16 (t, J=7.2 Hz, 2H), 3.35 (s, 3H), 3.65 (t, J=7.2 Hz, 2H), 4.02-4.16 (m, 2H), 5.04 (s, 2H), 7.19 (d, J=8.4 Hz, 1H), 7.46 (d, J=8.4 Hz, 1H).

### (4) tert-Butyl 4-{2-[7-hydroxymethyl-6-(2-methoxyethyl)benz[d]isoxazol-3-yl]ethyl}piperidine-1-carboxylate

The title compound (97 mg) was obtained by synthesis from tert-butyl 4-{2-[7-(tert-butyldimethylsilanyloxymethyl)-6-(2-methoxyethyl)benz[d]isoxazol-3-yl]ethyl}piperidine-1-carboxylate (127 mg) according to Example 79-(5).
¹H-NMR (400 MHz, CDCl₃) δ(ppm): 1.16 (ddd, J=4.0 Hz, 12.4 Hz, 24.4 Hz, 2H), 1.45 (s, 9H), 1.47-1.56 (m, 1H), 1.70-1.83 (m, 4H), 2.67 (br t, J=12.0 Hz, 2H), 2.99 (t, J=7.6 Hz, 2H), 3.12 (t, J=5.6 Hz, 2H), 3.30 (s, 3H), 3.63 (t, J=6.4 Hz, 1H) , 3.68 (t, J=5.6 Hz, 2H), 4.02-4.16 (m, 2H), 4.97 (d, J=6.4 Hz, 2H), 7.16 (d, J=8.0 Hz, 1H) , 7.51 (d, J=8.0 Hz, 1H).

### (5) tert-Butyl 4-{2-[7-(N,N-dimethylaminomethyl)-6-(2-methoxyethyl)benz[d]isoxazol-3-yl]ethyl}piperidine-1-carboxylate

The title compound (92 mg) was obtained by synthesis from tert-butyl 4-{2-[7-hydroxymethyl-6-(2-methoxyethyl)benz[d]isoxazol-3-yl]ethyl}piperidine-1-carboxylate (96 mg) according to Example 79-(6).
¹H-NMR (400 MHz, CDCl₃) δ(ppm): 1.16 (ddd, J=4. 4 Hz, 12.4 Hz, 24.4 Hz, 2H), 1.45 (s, 9H), 1.47-1.56 (m, 1H), 1.71-1.83 (m, 4H), 2.27 (s, 6H), 2.68 (br t, J=11.6 Hz, 2H), 2.97 (t, J=8.0 Hz, 2H), 3.17 (t, J=7.2 Hz, 2H), 3.35 (s, 3H), 3.64 (t, J=7.2 Hz, 2H), 3.74 (s, 2H), 4.02-4.16 (m, 2H), 7.19 (d, J=8.0 Hz, 1H), 7.45 (d, J=8.0 Hz, 1H).

### (6) N,N-Dimethyl{3-[2-(1-benzylpiperidin-4-yl)ethyl]-6-(2-methoxyethyl)benz[d]isoxazol-7-yl}methylamine

The title compound (62 mg) was obtained by synthesis from tert-butyl 4-{2-[7-(N,N-dimethylaminomethyl)-6-(2-methoxyethyl)benz[d]isoxazol-3-yl]ethyl}piperidine-1-carboxylate (91 mg) according to Example 79-(7).
¹H-NMR (400 MHz, CDCl₃) δ(ppm): 1.24-1.40 (m, 3H), 1.70-1.82 (m, 4H), 1.94 (br d, J=11 .2 Hz, 2H), 2.26 (s, 6H), 2.88 (br d, J=11.2 Hz, 2H), 2.95 (t, J=8.0 Hz, 2H), 3.16 (t, J=7.2 Hz, 2H), 3.35 (s, 3H), 3.48 (s, 2H), 3.64 (t, J=7.2 Hz, 2H), 3.74 (s, 2H), 7.18 (d, J=8.4 Hz, 1H) , 7.19-7.33 (m, 5H), 7. 44 (d, J=8.4 Hz, 1H).

### (7) N,N-Dimethyl{3-[2-(1-benzylpiperidin-4-yl)ethyl]-6-(2-methoxyethyl)benz[d]isoxazol-7-yl}methylamine dihycrochloride

The title compound (68 mg) was obtained by synthesis from N,N-dimethyl{3-[2-(1-benzylpiperidin-4-yl)ethyl]-6-(2-methoxyethyl)benz[d]isoxazol-7-yl}methylamine (60 mg) according to Example 79-(8).
¹H-NMR (400 MHz, CD₃OD) δ(ppm): 1.53 (ddd, J=3.6 Hz, 14.0 Hz, 24.8 Hz, 2H), 1.66-1.79 (m, 1H), 1.86 (dt, J=7.2 Hz, 8.0 Hz, 2H), 2.10 (br d, J=14.4 Hz, 2H), 2.96-3.06 (m, 2H), 2.99 (s, 6H), 3.09 (t, J=8.0 Hz, 2H), 3.21 (t, J=5.6 Hz, 2H), 3.34 (s, 3H), 3.51 (br d, J=12.4 Hz, 2H), 3.76 (t, J=5.6 Hz, 2H), 4.31 (s, 2H), 4.74 (s, 2H), 7.44-7.56 (m, 6H), 7.92 (d, 1H).
ESI-MS m/z: 219 [M+2H ]²⁺ , 436 [M+H]⁺.

### Example 83

### N-(4-Fluorophenyl)-2-{3-[2-(1-benzylpiperidin-4-yl)ethyl]-7-(N,N-dimethylaminomethyl)benz[d]isoxazol-6-yl}acetamide dihydrochloride

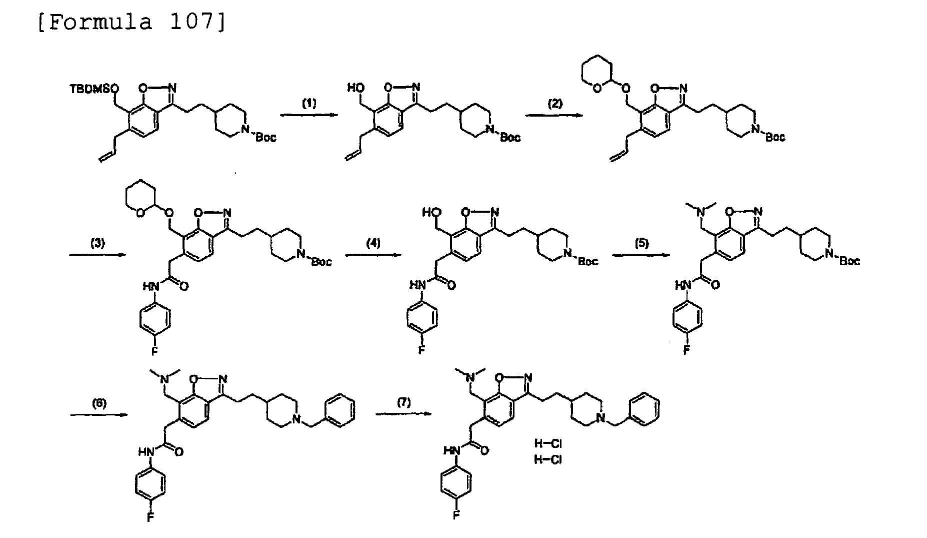

### (1) tert-Butyl 4-[2-(6-allyl-7-hydroxymethylbenz[d]isoxazol-3-yl)ethyl]piperidine-1-carboxylate

The title compound (1.43 g) was obtained by synthesis from tert-butyl 4-{2-[6-allyl-7-(tert-butyldimethylsilanyloxymethyl)benz[d]isoxazol-3-yl]ethyl}piperidine-1-carboxylate shown in Example 82-(1) (2.9 g) according to Example 79- (5) .
¹H-NMR (400 MHz, CDCl₃) δ(ppm): 1.16 (ddd, J=4.0 Hz, 12.4 Hz, 24.4 Hz, 2H), 1.43-1.56 (m, 1H), 1.45 (s, 9H), 1.69-1.83 (m, 4H), 2.00 (t, J=6.4 Hz, 1H), 2.67 (br t, J=12.4 Hz, 2H), 3.00 (t, J=8.0 Hz, 2H), 3.66 (ddd, J=1.6 Hz, 1.6 Hz, 6.0 Hz, 2H), 4.02-4.18 (m, 2H), 5.01 (ddt, J=1.6 Hz, 1.6 Hz, 17.2 Hz, 1H), 5.03 (d, J=6.4 Hz, 2H), 5.10 (ddt, J=1.6 Hz, 1.6 Hz, 10.0 Hz, 1H) , 6.02 (ddt, J=6.0 Hz, 10.0 Hz, 17.2 Hz, 1H) , 7.17 (d, J=8.0 Hz, 1H), 7.50 (d, J=8.0 Hz, 1H).

### (2) tert-Butyl 4-{2-[6-allyl-7-(tetrahydro-2H-pyran-2-yloxymethyl)benz[d]isoxazol-3-yl]ethyl}piperidine-1-carboxylate

tert-Butyl 4-[2-(6-allyl-7-hydroxymethylbenz[d]isoxazol-3-yl)ethyl]piperidine-1-carboxylate (1.43 g) and 3,4-dihydro-2H-pyrane (1 mL) were dissolved in methylene chloride (15 mL). (1S)-(+)-10-Camphorsulfonic acid (70 mg) was added and the mixture was stirred at room temperature for six hours and 35 minutes. A saturated sodium bicarbonate solution and water were sequentially added to the reaction mixture, followed by extraction with chloroform. The organic layer was washed with a saturated sodium chloride solution, dried over magnesium sulfate and filtered. Then, the solvent was evaporated under reduced pressure. The residue was purified by NH silica gel column chromatography (heptane-ethyl acetate) to obtain the title compound (1.64 g).
¹H-NMR (400 MHz, CDCl₃) δ (ppm) : 1.09-1.21 (m, 2H), 1.45 (s, 9H), 1.46-1.66 (m, 3H), 1.57-1.66 (m, 2H), 1.68-1.84 (m, 6H), 2.67 (br t, J=12.8 Hz, 2H), 2.98 (t, J=8.0 Hz, 2H), 3.58-3.68 (m, 3H), 4.00 (ddd, J=3.2 Hz, 8.0 Hz, 11.2 Hz, 1H), 4.02 (m, 2H), 4.80 (d, J=11.2 Hz, 1H), 4.82 (t, J=3.2 Hz, 1H), 5.03 (ddt, J=1.6 Hz, 1.6 Hz, 16.8 Hz, 1H), 5.08 (ddt, J=1.6 Hz, 1.6 Hz, 10.0 Hz, 1H), 5.10 (d, J=11.2 Hz, 1H), 5.99 (ddt, J=6.4 Hz, 10.0 Hz, 16.8 Hz, 1H), 7.16 (d, J=8.0 Hz, 1H), 7.49 (d, J=8.0 Hz, 1H).

### (3) tert-Butyl 4-{2-{6-[(4-fluorophenylcarbamoyl)methyl]-7-(tetrahydro-2H-pyran-2-yloxymethyl)benz[d]isoxazol-3-yl}ethyl}piperidine-1-carboxylate

tert-Butyl 4-{2-[6-allyl-7-(tetrahydro-2H-pyran-2-yloxymethyl)benz[d]isoxazol-3-yl]ethyl}piperidine-1-carboxylate (600 mg) was dissolved in tetrahydrofuran (16 mL). Water (4 mL) and osmium tetroxide (2.5% aqueous solution) (0.4 mL) were sequentially added to the solution, and the mixture was stirred at room temperature for 14 minutes. Then, sodium metaperiodate (280 mg) was added at room temperature and the mixture was stirred at room temperature for two hours and 40 minutes. Water was added to the reaction mixture, followed by extraction with diethyl ether. The organic layer was sequentially washed with water and a saturated sodium chloride solution, dried over magnesium sulfate and filtered. Then, the solvent was evaporated under reduced pressure to obtain a crude product of tert-butyl 4-{2-[6-(2-formylmethyl)-7-(tetrahydro-2H-pyran-2-yloxymethyl)benz[d]isoxazol-3-yl]ethyl}piperidine-1-carboxylate (608 mg). The crude product (606 mg) was dissolved in acetone (12 mL). Water (6 mL), 2-methyl-2-butene (1.5 mL), sodium dihydrogenphosphate (160 mg) and 80% sodium chlorite (280 mg) were sequentially added and the mixture was stirred at room temperature for 19 hours and 50 minutes. A saturated ammonium chloride solution was added to the reaction mixture, followed by extraction with chloroform. The organic layer was washed with a saturated sodium chloride solution, dried over magnesium sulfate and filtered. Then, the solvent was evaporated under reduced pressure to obtain a crude product of tert-butyl 4-{2-[6-carboxymethyl-7-(tetrahydro-2H-pyran-2-yloxymethyl)benz[d]isoxazol-3-yl]ethyl}piperidine-1-carboxylate. The crude product, 4-fluoroaniline (180 mg) and triethylamine (0.8 mL) were dissolved in methylene chloride (10 mL). (Benzotriazol-1-yloxy)tris(dimethylamino)phosphonium hexafluorophosphate (600 mg) was added and the mixture was stirred at room temperature for 67 hours and 50 minutes. Water was added to the reaction mixture, followed by extraction with ethyl acetate. The organic layer was sequentially washed with water and a saturated sodium chloride solution, dried over magnesium sulfate and filtered. Then, the solvent was evaporated under reduced pressure. The residue was purified by NH silica gel column chromatography (heptane-ethyl acetate) to obtain the title compound (116 mg) .
¹H-NMR (400 MHz, CDCl₃) δ (ppm): 1.10-1.23 (m, 2H), 1.45 (s, 9H), 1.46-1.63 (m, 5H), 1.69-1.84 (m, 6H), 2.59-2.75 (m, 2H), 3.00 (t, J=8.0 Hz, 2H), 3.60-3.69 (m, 1H), 3.89 (d, J=13.6 Hz, 1H), 3.96 (d, J=13.6 Hz, 1H), 3.97-4.17 (m, 3H), 4.81-4.87 (m, 1H), 5.00 (d, J=11.2 Hz, 1H), 5.27 (d, J=11.2 Hz, 1H) , 6. 93 (dd, J=8. 8 Hz, 8.8 Hz, 2H), 7.37 (dd, J=5.2 Hz, 8.8 Hz, 2H), 7.45 (d, J=8.4 Hz, 1H), 7.57 (d, J=8. Hz, 1H), 8. 32 (s, 1H).

### (4) tert-Butyl 4-{2-{6-[(4-fluorophenylcarbamoyl)methyl]-7-hydroxymethylbenz[d]isoxazol-3-yl}ethyl}piperidine-1-carboxylate

tert-Butyl 4-{2-{6-[(4-fluorophenylcarbamoyl)methyl]-7-(tetrahydro-2H-pyran-2-yloxymethyl)benz[d]isoxazol-3-yl}ethyl}piperidine-1-carboxylate (115 mg) and pyridinium p-toluenesulfonate (12 mg) were dissolved in ethanol (5 mL), and the solution was stirred at 60°C for 50 minutes. A saturated sodium bicarbonate solution and water were sequentially added to the reaction mixture, followed by extraction with ethyl acetate. The organic layer was sequentially washed with water and a saturated sodium chloride solution, dried over magnesium sulfate and filtered. Then, the solvent was evaporated under reduced pressure. The residue was purified by NH silica gel column chromatography (heptane-ethyl acetate) to obtain the title compound (71 mg).
¹H-NMR (400 MHz, CDCl₃) δ(ppm): 1.08-1.21 (m, 2H), 1.45 (s, 9H), 1.46-1.53 (m, 1H), 1.69-1.81 (m, 4H), 2.59-2.73 (m, 2H), 2.99 (t, J=8.0 Hz, 2H), 3.13 (t, J=5.2 Hz, 1H), 3.92 (s, 2H), 4.00-4.16 (m, 2H), 5.19 (d, J=5.2 Hz, 2H), 6. 93 (dd, J=8. 8 Hz, 8.8 Hz, 2H), 7.39 (dd, J=4.8 Hz, 8.8 Hz, 2H), 7.40 (d, J=8.0 Hz, 1H) , 7.55 (d, J=8.0 Hz, 1H) , 8.62 (s, 1H) .

### (5) tert-Butyl 4-{2-{7-(N,N-dimethylaminomethyl)-6-[(4-fluorophenylcarbamoyl)methyl]benz[d]isoxazol-3-yl}ethyl}piperidine-1-carboxylate

The title compound (76 mg) was obtained by synthesis from tert-butyl 4-{2-{6-[(4-fluorophenylcarbamoyl)methyl]-7-hydroxymethylbenz[d]isoxazol-3-yl}ethyl}piperidine-1-carboxylate (70 mg) according to Example 79-(6).
¹H-NMR (400 MHz, CDCl₃) δ(ppm): 1. 16 (ddd, J=4.0 Hz, 12.0 Hz, 24.0 Hz, 2H), 1.45 (s, 9H), 1.46-1.57 (m, 1H), 1.70-1.82 (m, 4H), 2.44 (s, 6H), 2.61-2.73 (m, 2H), 2.98 (t, J=8.0 Hz, 2H), 3.79 (s, 2H), 3.92 (s, 2H), 4.01-4.15 (m, 2H), 6.92 (dd, J=8.8 Hz, 8.8 Hz, 2H), 7.28-7.38 (m, 2H), 7.50 (d, J=8.0 Hz, 1H), 7.55 (d, J=8.0 Hz, 1H), 11.06 (s, 1H).

### (6) N-(4-Fluorophenyl)-2-{3-[2-(1-benzylpiperidin-4-yl)ethyl]-7-(N,N-dimethylaminomethyl)benz[d]isoxazol-6-yl}acetamide

The title compound (28 mg) was obtained by synthesis from tert-butyl 4-{2-{7-(N,N-dimethylaminomethyl)-6-[(4-fluorophenylcarbamoyl)methyl]benz[d]isoxazol-3-yl}ethyl}piperidine-1-carboxylate (74 mg) according to Example 79- (7) .
¹H-NMR (400 MHz, CDCl₃) δ(ppm): 1.24-1.40 (m, 3H), 1.68-1.82 (m, 4H), 1.94 (br t, J=11.2 Hz, 2H), 2.43 (s, 6H), 2.88 (br d, J=11.6 Hz, 2H), 2.96 (t, J=8.0 Hz, 2H), 3.48 (s, 2H), 3.77 (s, 2H), 3.90 (s, 2H), 6.92 (dd, J=8.8 Hz, 8.8 Hz, 2H), 7.19-7.37 (m, 5H), 7.32 (dd, J=5.2 Hz, 8.8 Hz, 2H), 7.47 (d, J=8.4 Hz, 1H), 7.53 (d, J=8.4 Hz, 1H), 11.08 (s, 1H).

### (7) N-(4-Fluorophenyl)-2-{3-[2-(1-benzylpiperidin-4-yl)ethyl]-7-(N,N-dimethylaminomethyl)benz[d]isoxazol-6-yl}acetamide dihydrochloride

The title compound (31 mg) was obtained by synthesis from N-(4-fluorophenyl)-2-{3-[2-(1-benzylpiperidin-4-yl)ethyl]-7-dimethylaminomethylbenz[d]isoxazol-6-yl}acetamide (28 mg) according to Example 79-(8).
¹H-NMR (400 MHz, CD₃OD) δ(ppm): 1.45-1.58 (m, 2H), 1.66-1.78 (m, 1H), 1.87 (dt, J=7.6 Hz, 7.6 Hz, 2H), 2.09 (br d, J=14.8 Hz, 2H), 2.94-3.06 (m, 2H), 3.03 (s, 6H), 3.11 (t, J=7.6 Hz, 2H), 3.50 (br d, J=12.4 Hz, 2H), 4.15 (s, 2H), 4.30 (s, 2H), 4.80 (s, 2H), 7.07 (dd, J=8.8 Hz, 8.8 Hz, 2H), 7.46-7.54 (m, 5H), 7.54 (d, J=8.0 Hz, 1H), 7.59 (dd, J=4.8 Hz, 8.8 Hz, 2H), 7.97 (d, J=8.0 Hz, 1H).
ESI-MS m/z: 265 [M+2H]²⁺, 529 [M+H]⁺.

### Example 84

### N-(4-Fluorobenzyl)-3-[2-(1-benzylpiperidin-4-yl)ethyl]-7-(N,N-dimethylaminomethyl)benz[d]isoxazole-6-carboxamide dihydrochloride

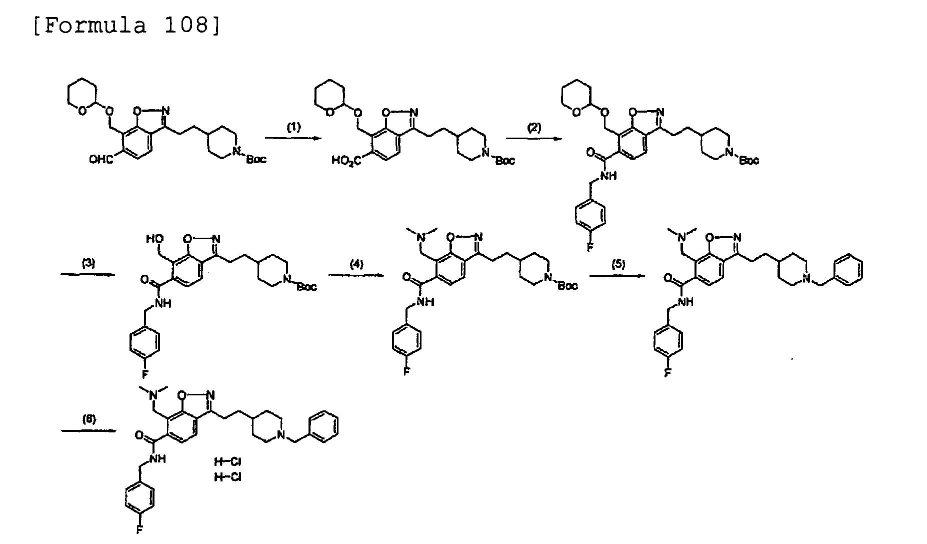

### (1) 3-[2-(1-tert-Butoxycarbonylpiperidin-4-yl)ethyl]-7-(tetrahydro-2H-pyran-2-yloxymethyl)benz[d]isoxazole-6-carboxylic acid

tert-Butyl 4-{2-[6-formyl-7-(tetrahydro-2H-pyran-2-yloxymethyl)benz[d]isoxazol-3-yl]ethyl}piperidine-1-carboxylate shown in Example 764-(4) (472 mg) was dissolved in acetone (10 mL). Water (5 mL), 2-methyl-2-butene (1 mL), sodium dihydrogenphosphate (140 mg) and 80% sodium chlorite (230 mg) were sequentially added and the mixture was stirred at room temperature for 50 minutes. A saturated ammonium chloride solution was added to the reaction mixture, followed by extraction with chloroform. The organic layer was washed with a saturated sodium chloride solution, dried over magnesium sulfate and filtered. Then, the solvent was evaporated under reduced pressure to obtain the title compound (500 mg).
¹H-NMR (400 MHz, CDCl₃) δ(ppm): 1.18 (ddd, J=4.4 Hz, 12.4 Hz, 24.4 Hz, 2H), 1.46 (s, 9H), 1.48-1.68 (m, 5H), 1.67-1.86 (m, 6H), 2.69 (br t, J=12.4 Hz, 2H), 3.04 (t, J=8.0 Hz, 2H), 3.54-3.63 (m, 1H), 3.88-3.97 (m, 1H), 4.02-4.20 (m, 2H), 4.91 (dd, J=2. 8 Hz, 2.8 Hz, 1H), 5.15 (d, J=11.6 Hz, 1H), 5.41 (d, J=11.6 Hz, 1H), 7.64 (d, J=8.4 Hz, 1H), 7.90 (d, J=8.4 Hz, 1H).

### (2) tert-Butyl 4-{2-[6-(4-fluorobenzylcarbamoyl)-7-(tetrahydro-2H-pyran-2-yloxymethyl)benz[d]isoxazol-3-yl]ethyl}piperidine-1-carboxylate

3-[2-(1-tert-Butoxycarbonylpiperidin-4-yl)ethyl]-7-(tetrahydro-2H-pyran-2-yloxymethyl)benz[d]isoxazole-6-carboxylic acid (227 mg) was dissolved in methylene chloride (5 mL). 4-Fluorobenzylamine (80 µL), triethylamine (0.2 mL) and (benzotriazol-1-yloxy)tris(dimethylamino)phosphonium hexafluorophosphate (250 mg) were sequentially added and the mixture was stirred at room temperature for 120 hours. Water was added to the reaction mixture, followed by extraction with methylene chloride twice. The organic layers were sequentially washed with water and a saturated sodium chloride solution, dried over magnesium sulfate and filtered. Then, the solvent was evaporated under reduced pressure. The residue was purified by NH silica gel column chromatography (heptane-ethyl acetate) to obtain the title compound (195 mg).
¹H-NMR (400 MHz, CDCl₃) δ(ppm): 1.10-1.32 (m, 4H), 1.37-1.64 (m, 5H), 1.45 (s, 9H), 1.74 (br d, J=13.2 Hz, 2H), 1.80 (dt, J=7.2 Hz, 8.0 Hz, 2H), 2.67 (br t, J=12.0 Hz, 2H), 3.02 (t, J=8.0 Hz, 2H), 3.47-3.56 (m, 1H), 3.76-3.84 (m, 1H), 4.02-4.20 (m, 2H), 4.51 (dd, J=5.2 Hz, 10.4 Hz, 1H), 4.59 (dd, J=2.4 Hz, 2.4 Hz, 1H), 4.74 (dd, J=6.4 Hz, 10.4 Hz, 1H), 4.84 (d, J=11.2 Hz, 1H), 5.15 (d, J=11 .2 Hz, 1H), 7.03 (dd, J=8. 8 Hz, 8.8 Hz, 2H), 7.36 (dd, J=5.2 Hz, 8.8 Hz, 2H), 7.65 (d, J=8.0 Hz, 1H), 7.76 (d, J=8.0 Hz, 1H), 7.85 (br t, J=5.2 Hz, 1H) .

### (3) tert-Butyl 4-{2-[6-(4-fluorobenzylcarbamoyl)-7-hydroxymethylbenz[d]isoxazol-3-yl]ethyl}piperidine-1-carboxylate

The title compound (124 mg) was obtained by synthesis from tert-butyl 4-{2-[6-(4-fluorobenzylcarbamoyl)-7-(tetrahydro-2H-pyran-2-yloxymethyl)benz[d]isoxazol-3-yl]ethyl}piperidine-1-carboxylate (193 mg) according to Example 83-(4).
¹H-NMR (400 MHz, CDCl₃) δ(ppm): 1.16 (ddd, J=4.0 Hz, 12.0 Hz, 24.0 Hz, 2H), 1.42-1.55 (m, 1H), 1.45 (s, 9H), 1.73 (br d, J=12.0 Hz, 2H), 1.79 (dt, J=7.2 Hz, 8.0 Hz, 2H), 2.66 (br t, J=12.0 Hz, 2H), 3.02 (t, J=8.0 Hz, 2H), 3.88 (t, J=6.0 Hz, 1H), 4.00-4.18 (m, 2H), 4.65 (d, J=6.0 Hz, 2H), 5.02 (d, J=6.0 Hz, 2H), 7.04-7.10 (m, 1H), 7.05 (dd, J=8.8 Hz, 8.8 Hz, 2H), 7.35 (dd, J=5.2 Hz, 8.8 Hz, 2H), 7.40 (d, J=8.4 Hz, 1H), 7.61 (d, J=8.4 Hz, 1H).

### (4) tert-Butyl 4-{2-[7-(N,N-dimethylaminomethyl)-6-(4-fluorobenzylcarbamoyl)benz[d]isoxazol-3-yl]ethyl}piperidine-1-carboxylate

The title compound (115 mg) was obtained by synthesis from tert-butyl 4-{2-[6-(4-fluorobenzylcarbamoyl)-7-hydroxymethylbenz[d]isoxazol-3-yl]ethyl}piperidine-1-carboxylate (122 mg) according to Example 79-(6). ¹H-NMR (400 MHz, CDCl₃) δ(ppm): 1.18 (ddd, J=4.0 Hz, 12.4 Hz, 24.4 Hz, 2H), 1.45-1.57 (m, 1H), 1.46 (s, 9H), 1.75 (br d, J=12.8 Hz, 2H), 1.81 (dt, J=7.2 Hz, 8.4 Hz, 2H), 2.04 (s, 6H), 2.68 (br t, J=12.0 Hz, 2H), 3.01 (t, J=8.0 Hz, 2H), 3.72 (s, 2H), 4.02-4.17 (m, 2H), 4.60 (d, J=4.8 Hz, 2H), 7.03 (dd, J=8.4 Hz, 8.4 Hz, 2H), 7.35 (dd, J=5.2 Hz, 8.4 Hz, 2H), 7.63 (d, J=8.4 Hz, 1H), 7.97 (d, J=8.4 Hz, 1H), 10.93 (t, J=4.8 Hz, 1H).

### (5) N-(4-Fluorobenzyl)-3-[2-(1-benzylpiperidin-4-yl)ethyl]-7-(N,N-dimethylaminomethyl)benz[d]isoxazole-6-carboxamide

The title compound (89 mg) was obtained by synthesis from tert-butyl 4-{2-[7-(N,N-dimethylaminomethyl)-6-(4-fluorobenzylcarbamoyl)benz[d]isoxazol-3-yl]ethyl}piperidine-1-carboxylate (113 mg) according to Example 79- (7) .
¹H-NMR (400 MHz, CDCl₃) δ(ppm): 1.28-1.39 (m, 3H), 1.71-1.83 (m, 4H), 1.94 (br t, J=10.4 Hz, 2H), 2.03 (s, 6H), 2.89 (br d, J=11.6 Hz, 2H), 2.99 (t, J=8.0 Hz, 2H), 3.48 (s, 2H), 3.71 (s, 2H), 4.60 (d, J=5.2 Hz, 2H), 7.03 (dd, J=8.8 Hz, 8.8 Hz, 2H), 7.20-7.40 (m, 5H), 7. 35 (dd, J=5.2 Hz, 8. 8 Hz, 2H), 7.62 (d, J=8.4 Hz, 1H) , 7.96 (d, J=8.4 Hz, 1H), 10.94 (t, J=5.2 Hz, 1H).

### (6) N-(4-Fluorobenzyl)-3-[2-(1-benzylpiperidin-4-yl)ethyl]-7-(N,N-dimethylaminomethyl)benz[d]isoxazole-6-carboxamide dihydrochloride

The title compound (102 mg) was obtained by synthesis from N-(4-fluorobenzyl)-3-[2-(1-benzylpiperidin-4-yl)ethyl]-7-(N,N-dimethylaminomethyl)benz[d]isoxazole-6-carboxamide (87 mg) according to Example 79-(8).
¹H-NMR (400 MHz, CD₃OD) δ(ppm): 1.49-1.63 (m, 2H), 1.69-1.82 (m, 1H), 1.88 (dt, J=7.2 Hz, 8.0 Hz, 2H), 2.10 (br d, J=14.0 Hz, 2H), 2.94 (s, 6H), 2.96-3.07 (m, 2H), 3.14 (t, J=8.0 Hz, 2H), 3.51 (br d, J=11.6 Hz, 2H), 4.31 (s, 2H), 4.62 (s, 2H), 4.67 (s, 2H), 7.08 (dd, J=8.8 Hz, 8.8 Hz, 2H), 7.44 (dd, J=5.2 Hz, 8.8 Hz, 2H), 7.45-7.57 (m, 5H), 7.80 (d, J=8.0 Hz, 1H), 8.09 (d, J=8.0 Hz, 1H).
ESI-MS m/z: 265 [M+2H]²⁺, 529 [M+H]⁺.

### Example 85

### N-{3-[2-(1-Benzylpiperidin-4-yl)ethyl]-7-(N,N-dimethylaminomethyl)benz[d]isoxazol-6-ylmethyl}-4-fluorobenzamide dihydrochloride

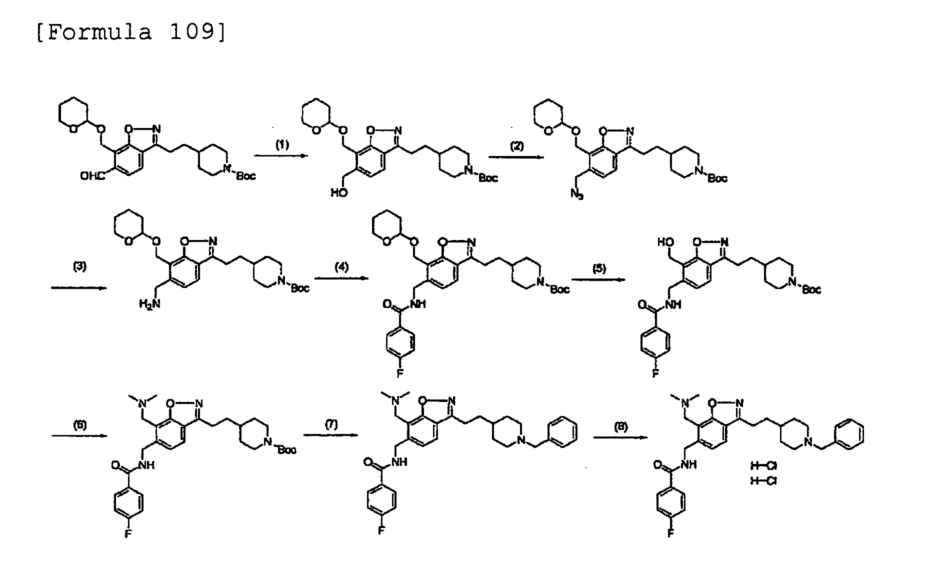

### (1) tert-Butyl 4-{2-[6-hydroxymethyl-7-(tetrahydro-2H-pyran-2-yloxymethyl)benz[d]isoxazol-3-yl]ethyl}piperidine-1-carboxylate

tert-Butyl 4-{2-[6-formyl-7-(tetrahydro-2H-pyran-2-yloxymethyl)benz[d]isoxazol-3-yl]ethyl}piperidine-1-carboxylate shown in Example 764-(4) (500 mg) was dissolved in methanol (10 mL). Sodium borohydride (60 mg) was added under ice-cooling, and the mixture was stirred under ice-cooling for one hour and 20 minutes. A saturated ammonium chloride solution and water were sequentially added to the reaction mixture, followed by extraction with ethyl acetate. The organic layer was sequentially washed with water and a saturated sodium chloride solution, dried over magnesium sulfate and filtered. Then, the solvent was evaporated under reduced pressure. The residue was purified by NH silica gel column chromatography (heptane-ethyl acetate) to obtain the title compound (480 mg).
¹H-NMR (400 MHz, CDCl₃) δ (ppm) : 1.17 (ddd, J=4.0 Hz, 12.4 Hz, 24.8 Hz, 2H), 1.42-1.67 (m, 5H), 1.45 (s, 9H), 1.70-1.85 (m, 6H), 2.68 (br d, J=12.0 Hz, 2H), 3.01 (t, J=8.0 Hz, 2H), 3.27 (t, J=6.8 Hz, 1H), 3.58-3.65 (m, 1H), 3.89-3.96 (m, 1H), 4.02-4.18 (m, 2H), 4.80-4.84 (m, 1H), 4 . 84 (d, J=6. 8 Hz, 2H), 4 . 98 (d, J=11.6 Hz, 1H), 5.23 (d, J=11.6 Hz, 1H), 7.38 (d, J=8.0 Hz, 1H) , 7.58 (d, J=8.0 Hz, 1H).

### (2) tert-Butyl 4-{2-[6-azidomethyl-7-(tetrahydro-2H-pyran-2-yloxymethyl)benz[d]isoxazol-3-yl]ethyl}piperidine-1-carboxylate

tert-Butyl 4-{2-[6-hydroxymethyl-7-(tetrahydro-2H-pyran-2-yloxymethyl)benz[d]isoxazol-3-yl]ethyl}piperidine-1-carboxylate (477 mg) and triphenylphosphine (530 mg) were dissolved in N,N-dimethylformamide (5 mL). Carbon tetrabromide (670 mg) and sodium azide (330 mg) were sequentially added under ice-cooling, and the mixture was stirred at room temperature for 116 hours. Water was added to the reaction mixture, followed by extraction with ethyl acetate. The organic layer was sequentially washed with a saturated sodium bicarbonate solution, water (three times) and a saturated sodium chloride solution, dried over magnesium sulfate and filtered. Then, the solvent was evaporated under reduced pressure. The residue was purified by silica gel column chromatography (heptane-ethyl acetate) to obtain the title compound (429 mg).
¹H-NMR (400 MHz, CDCl₃) δ(ppm): 1.11-1.23 (m, 2H), 1.46 (s, 9H), 1.47-1.67 (m, 5H), 1.68-1.85 (m, 6H), 2.68 (br t, J=11.6 Hz, 2H), 3.01 (t, J=8.0 Hz, 2H), 3.61 (ddd, J=2.0 Hz, 5.2 Hz, 11.2 Hz, 1H), 3.96 (ddd, J=3.2 Hz, 8.0 Hz, 11.2 Hz, 1H), 4.02-4.19 (m, 2H), 4.65 (s, 2H), 4.80 (dd, J=2.8 Hz, 4.0 Hz, 1H), 4.88 (d, J=11.2 Hz, 1H), 5.15 (d, J=11.2 Hz, 1H), 7.34 (d, J=8.0 Hz, 1H), 7.60 (d, J=8.0 Hz, 1H).

### (3) tert-Butyl 4-{2-[6-aminomethyl-7-(tetrahydro-2H-pyran-2-yloxymethyl)benz[d]isoxazol-3-yl]ethyl}piperidine-1-carboxylate

tert-Butyl 4-{2-[6-azidomethyl-7-(tetrahydro-2H-pyran-2-yloxymethyl)benz[d]isoxazol-3-yl]ethyl}piperidine-1-carboxylate (397 mg) was dissolved in tetrahydrofuran (6 mL). Triphenylphosphine (210 mg) was added and the mixture was stirred at room temperature for 18 hours. Concentrated aqueous ammonia was added to the reaction mixture, followed by extraction with ethyl acetate. The organic layer was sequentially washed with water and a saturated sodium chloride solution, dried over magnesium sulfate and filtered. Then, the solvent was evaporated under reduced pressure. The residue was purified by silica gel column chromatography (chloroform-methanol) to obtain the title compound (335 mg).
¹H-NMR (400 MHz, CDCl₃) δ(pom): 1.16 (ddd, J=4.0 Hz, 12.4 Hz, 24.8 Hz, 2H), 1.45 (s, 9H), 1.46-1.68 (m, 5H), 1.70-1.83 (m, 6H), 2.67 (br t, J=11.2 Hz, 2H), 3.00 (t, J=8.0 Hz, 2H), 3.58-3.66 (m, 1H), 3.94-4.02 (m, 1H), 4.01-4.16 (m, 4H), 4.84 (dd, J=2.8 Hz, 4.0 Hz, 1H), 4.87 (d, J=11.6 Hz, 1H), 5.18 (d, J=11.6 Hz, 1H), 7.33 (d, J=8.4 Hz, 1H), 7.55 (d, J=8.4 Hz, 1H) .

### (4) tert-Butyl 4-{2-{6-[(4-fluorobenzoylamino)methyl]-7-(tetrahydro-2H-pyran-2-yloxymethyl)benz[d]isoxazol-3-yl}ethyl}piperidine-1-carboxylate

The title compound (156 mg) was obtained by synthesis from tert-butyl 4-{2-[6-aminomethyl-7-(tetrahydro-2H-pyran-2-yloxymethyl)benz[d]isoxazol-3-yl]ethyl}piperidine-1-carboxylate (162 mg) and 4-fluorobenzoic acid (70 mg) according to Example 84-(2). ¹H-NMR (400 MHz, CDCl₃) δ(ppm): 1.16 (ddd, J=4.0 Hz, 12.4 Hz, 24.8 Hz, 2H), 1.42-1.68 (m, 5H), 1.45 (s, 9H), 1.70-1.84 (m, 6H), 2.60-2.76 (m, 2H), 2.99 (t, J=8.0 Hz, 2H), 3.56-3.64 (m, 1H), 3.92-4.00 (m, 1H), 3.99-4.19 (m, 2H), 4.75-4.80 (m, 1H), 4.79 (dd, J=6.0 Hz, 10.0 Hz, 1H), 4.88 (dd, J=6.0 Hz, 10.0 Hz, 1H), 5.05 (d, J=11.2 Hz, 1H), 5.26 (d, J=11.2 Hz, 1H), 7.05 (dd, J=8.8 Hz, 8.8 Hz, 2H), 7.40 (br t, J=6.0 Hz, 1H), 7.48 (d, J=8.0 Hz, 1H), 7.56 (d, J=8.0 Hz, 1H), 7.79 (dd, J=5.2 Hz, 8.8 Hz, 2H) .

### (5) tert-Butyl 4-{2-{6-[(4-fluorobenzoylamino)methyl]-7-hydroxymethylbenz[d]isoxazol-3-yl}ethyl}piperidine-1-carboxylate

The title compound (102 mg) was obtained by synthesis from tert-butyl 4-{2-{6-[(4-fluorobenzoylamino)methyl]-7-(tetrahydro-2H-pyran-2-yloxymethyl)benz[d]isoxazol-3-yl}ethyl}piperidine-1-carboxylate (154 mg) according to Example 83-(4).
¹H-NMR (400 MHz, CDCl₃) δ(ppm): 1.14 (ddd, J=4.4 Hz, 12.4 Hz, 24.4 Hz, 2H), 1.42-1.53 (m, 1H), 1.45 (s, 9H), 1.68-1.82 (m, 4H), 2.58-2.73 (m, 2H), 2.99 (t, J=8.0 Hz, 2H), 3.15 (t, J=5.6 Hz, 1H), 4.00-4.16 (m, 2H), 4. 85 (d, J=6.0 Hz, 2H), 5.18 (d, J=5.6 Hz, 2H), 7.05 (dd, J=8.8 Hz, 8.8 Hz, 2H), 7.29 (t, J=6.0 Hz, 1H), 7.42 (d, J=8.0 Hz, 1H), 7.55 (d, J=8.0 Hz, 1H), 7.76 (dd, J=5.2 Hz, 8.8 Hz, 2H).

### (6) tert-Butyl 4-{2-{7-(N,N-dimethylaminomethyl)-6-[(4-fluorobenzoylamino)methyl]benz[d]isoxazol-3-yl}ethyl}piperidine-1-carboxylate

The title compound (102 mg) was obtained by synthesis from tert-butyl 4-{2-{6-[(4-fluorobenzoylamino)methyl]-7-hydroxymethylbenz[d]isoxazol-3-yl}ethyl}piperidine-1-carboxylate (100 mg) according to Example 79-(6).
¹H-NMR (400 MHz, CDCl₃) δ(ppm): 1.17 (ddd, J=4.0 Hz, 12.0 Hz, 24.4 Hz, 2H), 1.45 (s, 9H), 1.47-1.56 (m, 1H), 1.71-1.84 (m, 4H), 2.33 (s, 6H), 2.62-2.75 (m, 2H), 3.00 (t, J=8.0 Hz, 2H), 3.87 (s, 2H), 4.02-4.16 (m, 2H), 4.77 (d, J=5.2 Hz, 2H), 7.05 (dd, J=8.8 Hz, 8.8 Hz, 2H), 7.44 (d, J=8.0 Hz, 1H), 7.54 (d, J=8.0 Hz, 1H) , 7.74 (dd, J=5.2 Hz, 8.8 Hz, 2H), 9.76 (t, J=5.2 Hz, 1H).

### (7) N-{3-[2-(1-Benzylpiperidin-4-yl)ethyl]-7-(N,N-dimethylaminomethyl)benz[d]isoxazol-6-ylmethyl}-4-fluorobenzamide

The title compound (67 mg) was obtained by synthesis from tert-butyl 4-{2-{7-(N,N-dimethylaminomethyl)-6-[(4-fluorobenzoylamino)methyl]benz[d]isoxazol-3-yl}ethyl}piperidine-1-carboxylate (100 mg) according to Example 79-(7).
¹H-NMR (400 MHz, CDCl₃) δ(ppm) : 1.28-1.38 (m, 3H), 1.70-1.82 (m, 4H), 1.90-1.98 (m, 2H), 2.32 (s, 6H), 2.88 (br d, J=11.6 Hz, 2H), 2.98 (t, J=8.0 Hz, 2H), 3.48 (s, 2H), 3.86 (s; 2H), 4.77 (d, J=5.2 Hz, 2H), 7.05 (dd, J=8.8 Hz, 8.8 Hz, 2H), 7.20-7.31 (m, 5H), 7.43 (d, J=8.0 Hz, 1H) , 7.53 (d, J=8.0 Hz, 1H), 7.74 (dd, J=5.2 Hz, 8.8 Hz, 2H), 9.77 (t, J=5.2 Hz, 1H).

### (8) N-{3-[2-(1-Benzylpiperidin-4-yl)ethyl]-7-(N,N-dimethylaminomethyl)benz[d]isoxazol-6-ylmethyl}-4-fluorobenzamide dihydrochloride

The title compound (70 mg) was obtained by synthesis from N-{3-[2-(1-benzylpiperidin-4-yl)ethyl]-7-(N,N-dimethylaminomethyl)benz[d]isoxazol-6-ylmethyl}-4-fluorobenzamide (66 mg) according to Example 79-(8). ¹H-NMR (400 MHz, CD₃OD) δ(ppm): 1.46-1.59 (m, 2H), 1.65-1.78 (m, 1H), 1.85 (dt, J=7.2 Hz, 8.0 Hz, 2H), 2.08 (br d, J=14.0 Hz, 2H), 2.95-3.14 (m, 4H), 3.06 (s, 6H), 3.49 (br d, J=12.4 Hz, 2H), 4.29 (s, 2H), 4.74 (s, 2H), 4.94 (s, 2H), 7.21 (dd, J=8.8 Hz, 8.8 Hz, 2H), 7.46-7.55 (m, 5H), 7.60 (d, J=8.4 Hz, 1H), 7.93 (dd, J=5.2 Hz, 8.8 Hz, 2H), 7.96 (d, J=8.4 Hz, 1H).
ESI-MS m/z: 265 [M+2H]²⁺, 529 [M+H]⁺.

### Example 86

### N,N-Dimethyl{6-cyclopropylmethoxy-3-{2-[1-(1H-indol-5-ylmethyl)piperidin-4-yl]ethyl}benz[d]isoxazol-7-yl}methylamine dihydrochloride

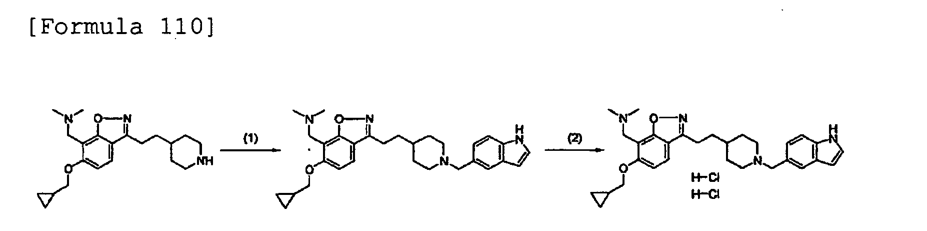

### (1) N,N-Dimethyl{6-cyclopropylmethoxy-3-{2-[1-(1H-indol-5-ylmethyl)piperidin-4-yl]ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine shown in Preparation Example 3-(3) (100 mg) and 1H-indole-5-carbaldehyde (80 mg) were dissolved in tetrahydrofuran (3 mL). Sodium triacetoxyborohydride (130 mg) was added and the mixture was stirred at room temperature for four hours. Ethyl acetate and 2 M hydrochloric acid were sequentially added to the reaction mixture, and the organic layer was extracted with 2 M hydrochloric acid. The resulting aqueous layer was washed with ethyl acetate. A 5 M sodium hydroxide solution was added until the pH was 7, followed by extraction with ethyl acetate. The organic layer was sequentially washed with water and a saturated sodium chloride solution, dried over magnesium sulfate and filtered. Then, the solvent was evaporated under reduced pressure. The residue was purified by NH silica gel column chromatography (heptane-ethyl acetate) to obtain the title compound (108 mg).
¹H-NMR (400 MHz, CDCl₃) δ (ppm) : 0.34-0.39 (m, 2H), 0.60-0.67 (m, 2H), 1.21-1.39 (m, 4H), 1.67-1.80 (m, 4H), 1.95 (br t,J=10.8 Hz, 2H), 2.33 (s, 6H), 2.88-2.97 (m, 4H), 3.58 (s, 2H), 3.82 (s, 2H), 3.93 (d, J=6. 8 Hz, 2H), 6.48-6.52 (m, 1H), 6.88 (d, J=8.8 Hz, 1H), 7.15 (d, J=8.4 Hz, 1H), 7.18 (dd, J=2.8 Hz, 2.8 Hz, 1H), 7.32 (d, J=8.4 Hz, 1H), 7.42 (d, J=8.8 Hz, 1H), 7.53 (s, 1H), 8.18-8.24 (m, 1H).

### (2) N,N-Dimethyl{6-cyclopropylmethoxy-3-{2-[1-(1H-indol-5-ylmethyl)piperidin-4-yl]ethyl}benz[d]isoxazol-7-yl}methylamine dihydrochloride

The title compound (108 mg) was obtained by synthesis from N,N-dimethyl{6-cyclopropylmethoxy-3-{2-[1-(1H-indol-5-ylmethyl)piperidin-4-yl]ethyl}benz[d]isoxazol-7-yl}methylamine (105 mg) according to Example 79-(8).
¹H-NMR (400 MHz, CD₃OD) δ (ppm) : 0.41-0.46 (m, 2H), 0.66-0.73 (m, 2H), 1.34-1.42 (m, 1H), 1.42-1.57 (m, 2H), 1.64-1.76 (m, 1H), 1.83 (dt, J=7.2 Hz, 8.0 Hz, 2H), 2.07 (br d, J=14.0 Hz, 2H), 2.93-3.08 (m, 4H), 2.96 (s, 6H), 3.52 (br d, J=12. 4 Hz, 2H), 4 .11 (d, J=6. 8 Hz, 2H), 4.36 (s, 2H), 4.63 (s, 2H), 6.51 (d, J=3.2 Hz, 1H), 7.22 (dd, J=1 .2 Hz, 8 . 0 Hz, 1H), 7.22 (d, J=8. 8 Hz, 1H), 7 . 33 (d, J=3.2 Hz, 1H), 7 . 48 (d, J=8.0 Hz, 1H), 7.71 (d, J=1 .2 Hz, 1H), 7. 90 (d, J=8.8 Hz, 1H) .
ESI-MS m/z: 487 [M+H]⁺.

### Example 87

### N,N-Dimethyl{6-cyclopropylmethoxy-3-{2-[1-(1H-indol-3-ylmethyl)piperidin-4-yl]ethyl}benz[d]isoxazol-7-yl}methylamine dihydrochloride

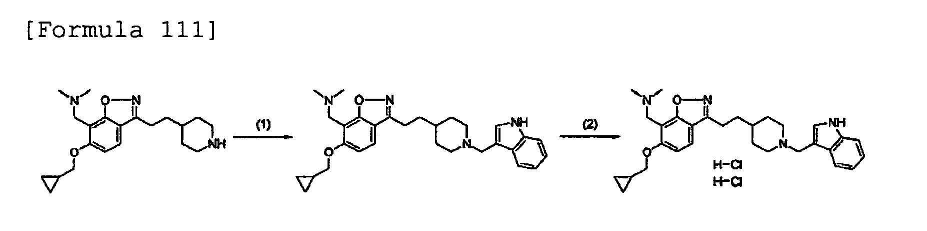

### (1) N,N-Dimethyl{6-cyclopropylmethoxy-3-{2-[1-(1H-indol-3-ylmethyl)piperidin-4-yl]ethyl}benz[d]isoxazol-7-yl}methylamine

The title compound (81 mg) was obtained by synthesis from N,N-dimethyl{6-cyclopropylmethoxy-3-[2-(piperidin-4-yl)ethyl]benz[d]isoxazol-7-yl}methylamine shown in Preparation Example 3-(3) (100 mg) and 1H-indole-3-carbaldehyde (80 mg) according to Example 86-(1) .
¹H-NMR (400 MHz, CDCl₃) δ(ppm): 0.33-0.39 (m, 2H), 0.61-0.67 (m, 2H), 1.24-1.36 (m, 4H), 1.58-1.78 (m, 4H), 2.00 (br t, J=11 .2 Hz, 2H), 2.32 (s, 6H), 2.91 (t, J=8.0 Hz, 2H), 2.98 (br d, J=11.2 Hz, 2H), 3.71 (s, 2H), 3.81 (s, 2H), 3.92 (d, J=6.8 Hz, 2H), 6.87 (d, J=8.8 Hz, 1H), 7.11 (dd, J=8.0 Hz, 8.0 Hz, 1H), 7.13 (d, J=2.4 Hz, 1H), 7. 18 (dd, J=8.0 Hz, 8.0 Hz, 1H) , 7.35 (d, J=8.0 Hz, 1H), 7.42 (d, J=8.0 Hz, 1H), 7.70 (d, J=8.0 Hz, 1H), 8.13-8.17 (m, 1H) .

### (2) N,N-Dimethyl{6-cyclopropylmethoxy-3-{2-[1-(1H-indol-3-ylmethyl)piperidin-4-yl]ethyl}benz[d]isoxazol-7-yl}methylamine dihydrochloride

The title compound (83 mg) was obtained by synthesis from N,N-dimethyl{6-cyclopropylmethoxy-3-{2-[1-(1H-indol-3-ylmethyl)piperidin-4-yl]ethyl}benz[d]isoxazol-7-yl}methylamine (79 mg) according to Example 79-(8).
¹H-NMR (400 MHz, CD₃OD) δ(ppm): 0.41-0.47 (m, 2H), 0.67-0.73 (m, 2H), 1.34-1.44 (m, 1H), 1.44-1.56 (m, 2H), 1.62-1.74 (m, 1H), 1.82 (dt, J=7.2 Hz, 7.6 Hz, 2H), 2.07 (br d, J=14.4 Hz, 2H), 2.95 (s, 6H), 2.98-3.08 (m, 4H), 3.54-3.62 (m, 2H), 4.10 (d, J=6.8 Hz, 2H), 4.50 (s, 2H), 4.62 (s, 2H), 7.15 (dd, J=8.0 Hz, 8.0 Hz, 1H), 7.20 (dd, J=8.0 Hz, 8.0 Hz, 1H), 7.22 (d, J=8.8 Hz, 1H), 7.44 (d, J=8.0 Hz, 1H), 7.55 (s, 1H), 7.71 (d, J=8.0 Hz, 1H), 7.89 (d, J=8.8 Hz, 1H).
ESI-MS m/z: 358 [M-C₉H₈N⁺H]⁺, 487 [M+H]⁺, 509 [M+Na]⁺.

### Example 88

### N,N-Dimethyl{6-cyclopropylmethoxy-3-{2-[1-(5-fluoro-1H-indol-3-ylmethyl)piperidin-4-yl]ethyl}benz[d]isoxazol-7-yl}methylamine dihydrochloride

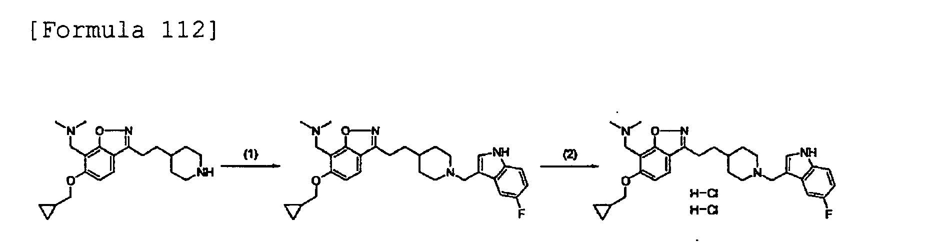

### (1) N,N-Dimethyl{6-cyclopropylmethoxy-3-{2-[1-(5-fluoro-1H-indol-3-ylmethyl)piperidin-4-yl]ethyl}benz[d]isoxazol-7-yl}methylamine

The title compound (81 mg) was obtained by synthesis from N,N-dimethyl{6-cyclopropylmethoxy-3-[2-(piperidin-4-yl)ethyl]benz[d]isoxazol-7-yl}methylamine shown in Preparation Example 3-(3) (100 mg) and 5-fluoro-1H-indole-3-carbaldehyde (90 mg) according to Example 86- (1) .
¹H-NMR (400 MHz, CDCl₃) δ (ppm) : 0.34-0.39 (m, 2H), 0.61-0.67 (m, 2H), 1.24-1.36 (m, 4H), 1.71-1.79 (m, 4H), 1.98 (br t, J=10.8 Hz, 2H), 2.33 (s, 6H), 2.89-2.98 (m, 4H), 3.65 (s, 2H), 3. 81 (s, 2H), 3.93 (d, J=6. 8 Hz, 2H), 6.88 (d, J=8.8 Hz, 1H), 6.92 (ddd, J=2.8 Hz, 8.8 Hz, 8. 8 Hz, 1H), 7.17 (d, J=2.4 Hz, 1H), 7.23-7.27 (m, 1H), 7.36 (d, J=2.8 Hz, 9.6 Hz, 1H), 7.43 (d, J=8.8 Hz, 1H), 8.08-8.13 (m, 1H) .

### (2) N,N-Dimethyl{6-cyclopropylmethoxy-3-{2-[1-(5-fluoro-1H-indol-3-ylmethyl)piperidin-4-yl]ethyl}benz[d]isoxazol-7-yl}methylamine dihydrochloride

The title compound (82 mg) was obtained by synthesis from N,N-dimethyl{6-cyclopropylmethoxy-3-{2-[1-(5-fluoro-1H-indol-3-ylmethyl)piperidin-4-yl]ethyl}benz[d]isoxazol-7-yl}methylamine (79 mg) according to Example 79-(8).
¹H-NMR (400 MHz, CD₃OD) δ(ppm): 0.42-0.47 (m, 2H), 0.67-0. 73 (m, 2H), 1.34-1.44 (m, 1H), 1.44-1.56 (m, 2H), 1.63-1.75 (m, 1H), 1.82 (dt, J=7.2 Hz, 8.0 Hz, 2H), 2.08 (br d, J=14.4 Hz, 2H), 2.95 (s, 6H), 2.98-3.08 (m, 4H), 3.53-3.60 (m, 2H), 4.11 (d, J=6.8 Hz, 2H), 4.46 (s, 2H), 4.62 (s, 2H), 6.98 (ddd, J=2.0 Hz, 8. 8 Hz, 8. 8 Hz, 1H), 7.22 (d, J=8.8 Hz, 1H), 7.42 (dd, J=4.4 Hz, 8. Hz, 1H), 7.44 (dd, J=2.0 Hz, 9.6 Hz, 1H), 7.61 (s, 1H), 7.90 (d, J=8.8 Hz, 1H).
ESI-MS m/z: 358 [M-C₉H₇FN⁺H]⁺, 527 [M+Na]⁺.

### Example 89

### N,N-Dimethyl{6-cyclopropylmethoxy-3-{2-[1-(naphthalen-1-ylmethyl)piperidin-4-yl]ethyl}benz[d]isoxazol-7-yl}methylamine dihydrochloride

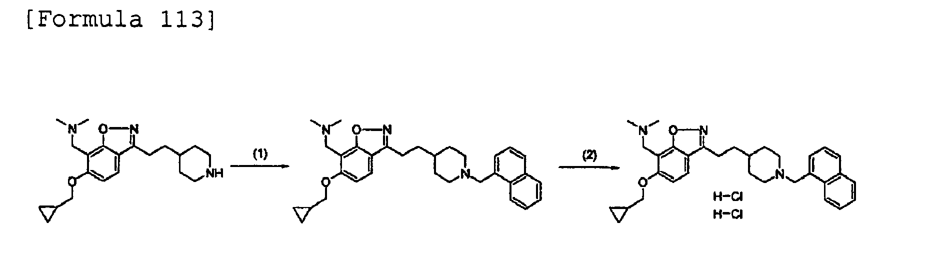

### (1) N,N-Dimethyl{6-cyclopropylmethoxy-3-{2-[1-(naphthalen-1-ylmethyl)piperidin-4-yl]ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine shown in Preparation Example 3-(3) (100 mg) and naphthalene-1-carbaldehyde (80 mg) were dissolved in methylene chloride (3 mL). Acetic acid (0.1 mL) and sodium triacetoxyborohydride (130 mg) were sequentially added and the mixture was stirred at room temperature for 143.5 hours. Ethyl acetate and 2 M hydrochloric acid were sequentially added to the reaction mixture, and the organic layer was extracted 2 M hydrochloric acid. The resulting aqueous layer was washed with ethyl acetate. A 5 M sodium hydroxide solution was added until the pH was 7, followed by extraction with ethyl acetate. The organic layer was sequentially washed with water and a saturated sodium chloride solution, dried over magnesium sulfate and filtered. Then, the solvent was evaporated under reduced pressure. The residue was purified by NH silica gel column chromatography (heptane-ethyl acetate) to obtain the title compound (70 mg) .
¹H-NMR (400 MHz, CDCl₃) δ(ppm): 0.34-0.39 (m, 2H), 0.61-0.67 (m, 2H), 1.23-1.44 (m, 4H), 1.68-1.80 (m, 4H), 1.98-2.07 (m, 2H), 2.33 (s, 6H), 2.89-2.98 (m, 2H), 2.93 (t, J=8.0 Hz, 2H), 3.81 (s, 2H), 3.92 (s, 2H), 3.94 (d, J=7.2 Hz, 2H), 6. 88 (d, J=8 . 0 Hz, 1H) , 7 . 36-7. 52 (m, 5H), 7. 75 (d, J=7 .2 Hz, 1H) , 7.83 (d, J=8.0 Hz, 1H) , 8.28 (d, J=8.0 Hz, 1H) .

### (2) N,N-Dimethyl{6-cyclopropylmethoxy-3-{2-[1-(naphthalen-1-ylmethyl)piperidin-4-yl]ethyl}benz[d]isoxazol-7-yl}methylamine dihydrochloride

The title compound (67 mg) was obtained by synthesis from N,N-dimethyl{6-cyclopropylmethoxy-3-{2-[1-(naphthalen-5-ylmethyl)piperidin-4-yl]ethyl}benz[d]isoxazol-7-yl}methylamine (68 mg) according to Example 79-(8).
¹H-NMR (400 MHz, CD₃OD) δ(ppm): 0.42-0.46 (m, 2H), 0.67-0.73 (m, 2H), 1.34-1.45 (m, 1H) , 1.44-1.60 (m, 2H), 1.71-1.87 (m, 3H), 2.06 (br d, J=14.0 Hz, 2H), 2.96 (s, 6H), 3.05 (br t, J=7.6 Hz, 2H), 3.21 (br t, J=12.0 Hz, 2H), 3.56 (br d, J=12.0 Hz, 2H), 4.11 (d, J=7.2 Hz, 2H), 4.63 (s, 2H), 4.83 (s, 2H), 7.23 (d, J=8.8 Hz, 1H), 7.56-7.64 (m, 2H), 7.69 (dd, J=8.0 Hz, 8.0 Hz, 1H), 7.78 (d, J=6.4 Hz, 1H) , 7.90 (d, J=8.8 Hz, 1H) , 8.00 (d, J=8.0 Hz, 1H) , 8.05 (d, J=8.8 Hz, 1H) , 8.25 (d, J=8.0 Hz, 1H) .
ESI-MS m/z: 250 [M+2H]2⁺, 498 [M+H]⁺.

### Example 90

### N,N-Dimethyl{3-{2-[1-(benzo[b]thiophen-3-ylmethyl)piperidin-4-yl]ethyl}-6-cyclopropylmethoxybenz[d]isoxazol-7-yl}methylamine dihydrochloride

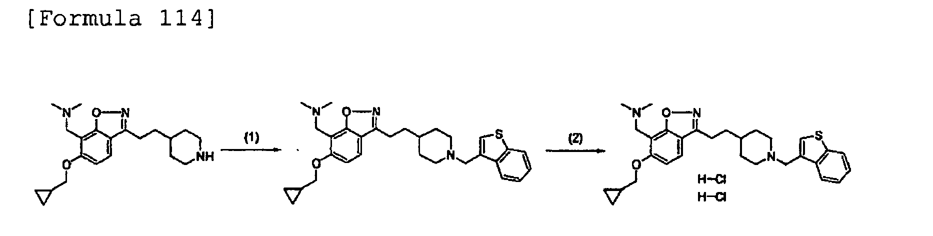

### (1) N,N-Dimethyl{3-{2-[1-(benzo[b]thiophen-3-ylmethyl)piperidin-4-yl]ethyl}-6-cyclopropylmethoxybenz[d]isoxazol-7-yl}methylamine

The title compound (58 mg) was obtained by synthesis from N,N-dimethyl{6-cyclopropylmethoxy-3-[2-(piperidin-4-yl)ethyl]benz[d]isoxazol-7-yl}methylamine shown in Preparation Example 3-(3) (100 mg) and benzo[b]thiophene-3-carbaldehyde (80 mg) according to Example 89-(1) .
¹H-NMR (400 MHz, CDCl₃) δ (ppm) : 0.34-0.39 (m, 2H), 0.61-0.66 (m, 2H), 1.24-1.40 (m, 4H), 1.71-1.79 (m, 4H), 1.95-2.03 (m, 2H), 2.33 (s, 6H), 2.90-2.98 (m, 2H), 2.93 (t, J=8.0 Hz, 2H), 3.71 (s, 2H), 3.81 (s, 2H), 3.93 (d, J=6.8 Hz, 2H), 6.88 (d, J=8.8 Hz, 1H), 7.24-7.39 (m, 3H), 7.42 (d, J=8.8 Hz, 1H), 7.83 (d, J=7.2 Hz, 1H), 7.95 (d, J=7.2 Hz, 1H) .

### (2) N,N-Dimethyl{3-{2-[1-(benzo[b]thiophen-3-ylmethyl)piperidin-4-yl]ethyl}-6-cyclopropylmethoxybenz[d]isoxazol-7-yl}methylamine dihydrochloride

The title compound (52 mg) was obtained by synthesis from N,N-dimethyl{3-{2-[1-(benzo[b]thiophen-3-ylmethyl)piperidin-4-yl]ethyl}-6-cyclopropylmethoxybenz[d]isoxazol-7-yl}methylamine (56 mg) according to Example 79-(8).
¹H-NMR (400 MHz, CD₃OD) δ(ppm): 0.41-0.46 (m, 2H), 0.66-0.73 (m, 2H), 1.33-1.44 (m, 1H) , 1.48-1.61 (m, 2H), 1.67-1.78 (m, 1H), 1.83 (dt, J=7.6 Hz, 8.0 Hz, 2H), 2.08 (br d, J=14.4 Hz, 2H), 2.96 (s, 6H), 3.04 (t, J=7.6 Hz, 2H), 3.13 (br t, J=12.4 Hz, 2H), 3.58 (br d, J=12.4 Hz, 2H), 4.11 (d, J=7.2 Hz, 2H), 4.62 (s, 2H), 4.63 (s, 2H), 7.22 (d, J=8.8 Hz, 1H), 7.45 (dd, J=8.0 Hz, 8.0 Hz, 1H), 7.52 (dd, J=8.0 Hz, 8.0 Hz, 1H), 7.90 (d, J=8.8 Hz, 1H), 7.98 (d, J=8.0 Hz, 1H), 8.03 (s, 1H), 8.05 (d, J=8.0 Hz, 1H).
ESI-MS m/z: 253 [M+2H]²⁺, 504 [M+H]⁺.

### Example 91

### N,N-Dimethyl{3-{2-[1-(benzo[b]thiophen-4-ylmethyl)piperidin-4-yl]ethyl}-6-cyclopropylmethoxybenz[d]isoxazol-7-yl}methylamine dihydrochloride

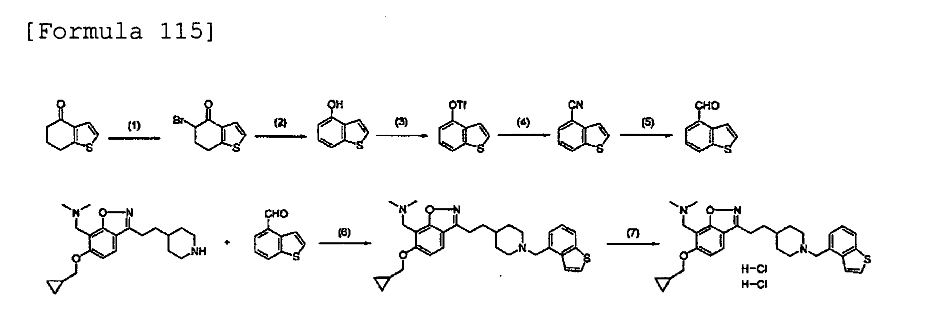

### (1) 5-Bromo-6,7-dihydro-5H-benzo[b]thiophen-4-one

The title compound was synthesized with reference to J. Chem. Soc. Perkin Trans. I, 1995, 197. A mixture of 6,7-dihydro-5H-benzo[b]thiophen-4-one (5 g), copper (II) bromide (15 g), ethyl acetate (80 mL) and chloroform (80 mL) was stirred at 80°C for one hour and 20 minutes. The reaction mixture was diluted with ethyl acetate (200 mL) and then filtered through celite and washed with ethyl acetate. Water was added, followed by extraction with ethyl acetate. The organic layer was sequentially washed with a saturated sodium bicarbonate solution, water and a saturated sodium chloride solution, dried over magnesium sulfate and filtered. Then, the solvent was evaporated under reduced pressure to obtain the title compound (7.63 g).
¹H-NMR (400 MHz, CDCl₃) δ (ppm): 2.50-2.63 (m, 2H), 3.06 (ddd, J=4.0 Hz, 4.0 Hz, 17.2 Hz, 1H), 3.29 (ddd, J=5.2 Hz, 9.2 Hz, 17.2 Hz, 1H), 4.62 (dd, J=4.0 Hz, 4.0 Hz, 1H), 7.13 (d, J=5.2 Hz, 1H), 7.43 (d, J=5.2 Hz, 1H).

### (2) Benzo[b]thiophen-4-ol

The title compound was synthesized with reference to Synth. Commun., 1995, 25, 507. A mixture of 5-bromo-6,7-dihydro-5H-benzo[b]thiophen-4-one (7.62 g), lithium carbonate (2.7 g), lithium bromide (3.2 g) and N,N-dimethylformamide (200 mL) was stirred at 150°C for one hour and 20 minutes. The reaction mixture was cooled to room temperature and then filtered through celite and washed with ethyl acetate. Then, the solvent was evaporated under reduced pressure. A saturated ammonium chloride solution was added, followed by extraction with ethyl acetate. The organic layer was washed with water and a saturated sodium chloride solution, dried over magnesium sulfate and filtered. Then, the solvent was evaporated under reduced pressure. The residue was purified by silica gel column chromatography (heptane-ethyl acetate). The resulting solid was suspended in a 20:1 mixed solution of heptane and diethyl ether and then filtered and washed with a 20:1 mixed solution of heptane and diethyl ether to obtain the title compound (4.16 g).
¹H-NMR (400 MHz, CDCl₃) δ (ppm): 5.31 (s, 1H), 6.71 (d, J=8.0 Hz, 1H), 7.19 (dd, J=8.0 Hz, 8.0 Hz, 1H), 7.35 (d, J=5.2 Hz, 1H), 7.45 (d, J=5.2 Hz, 1H), 7.46 (d, J=8.0 Hz, 1H).

### (3) Benzo [b] thiophen-4-yl trifluoromethanesulfonate

The title compound (3.61 g) was obtained by synthesis from benzo[b]thiophen-4-ol (2 g) according to Example 79-(2).
¹H-NMR (400 MHz, CDCl₃) δ(ppm): 7.30 (d, J=8.0 Hz, 1H), 7.38 (dd, J=8.0 Hz, 8.0 Hz, 1H), 7.45 (d, J=5.6 Hz, 1H), 7.58 (d, J=5.6 Hz, 1H), 7.88 (d, J=8.0 Hz, 1H) .

### (4) Benzo[b]thiophene-4-carbonitrile

A mixture of benzo[b]thiophen-4-yl trifluoromethanesulfonate (3.6 g), tetrakis (triphenylphosphine) palladium (0) (740 mg), zinc cyanide (1.5 g) and 1-methyl-2-pyrrolidinone (40 mL) was stirred in a nitrogen atmosphere at 100°C for one hour. The reaction mixture was diluted with ethyl acetate and then filtered through celite and washed with ethyl acetate. Then, water was added, followed by extraction with ethyl acetate. The organic layer was sequentially washed with water (three times) and a saturated sodium chloride solution, dried over magnesium sulfate and filtered. Then, the solvent was evaporated under reduced pressure. The residue was purified by silica gel column chromatography (heptane-ethyl acetate) to obtain the title compound (1.85 g).
¹H-NMR (400 MHz, CDCl₃) δ(ppm): 7.40 (dd, J=8.0 Hz, 8.0 Hz, 1H), 7.60 (d, J=5.6 Hz, 1H), 7.69 (d, J=5.6 Hz, 1H) , 7.72 (d, J=8.0 Hz, 1H) , 8.09 (d, J=8.0 Hz, 1H) .

### (5) Benzo[b]thiophene-4-carbaldehyde

The title compound was synthesized with reference to Chem. Pharm. Bull., 1991, 39, 1440. Benzo[b]thiophene-4-carbonitrile (1 g) was dissolved in formic acid (20 mL). Raney nickel (50% aqueous suspension) (3 mL) was added and the mixture was stirred at 80°C for 2.5 hours. The reaction mixture was cooled to room temperature and then filtered through celite and washed with methanol. Then, the solvent was evaporated under reduced pressure. A saturated sodium bicarbonate solution was added, followed by extraction with ethyl acetate. The organic layer was sequentially washed with a saturated sodium bicarbonate solution, water and a saturated sodium chloride solution, dried over magnesium sulfate and filtered. Then, the solvent was evaporated under reduced pressure. The residue was purified by silica gel column chromatography (heptane-ethyl acetate) to obtain the title compound (462 mg). ¹H-NMR (400 MHz, CDCl₃) δ (ppm): 7.53 (dd, J=8.0 Hz, 8.0 Hz, 1H), 7.72 (d, J=5.6 Hz, 1H), 7.86 (d, J=8.0 Hz, 1H), 8.14 (d, J=8.0 Hz, 1H) , 8.38 (d, J=5.6 Hz, 1H) , 10.24 (s, 1H).

### (6) N,N-Dimethyl{3-{2-[1-(benzo[b]thiophen-4-ylmethyl)piperidin-4-yl]ethyl}-6-cyclopropylmethoxybenz[d]isoxazol-7-yl}methylamine

The title compound (84 mg) was obtained by synthesis from N,N-dimethyl{6-cyclopropylmethoxy-3-[2-(piperidin-4-yl)ethyl]benz[d]isoxazol-7-yl}methylamine shown in Preparation Example 3-(3) (100 mg) and benzo[b]thiophene-4-carbaldehyde (90 mg) according to Example 89-(1).
¹H-NMR (400 MHz, CDCl₃) δ (ppm) : 0.33-0.39 (m, 2H), 0.61-0.66 (m, 2H), 1.23-1.42 (m, 4H), 1.70-1.79 (m, 4H), 1.95-2.04 (m, 2H), 2.33 (s, 6H), 2.88-2.95 (m, 2H), 2.93 (t, J=8.0 Hz, 2H), 3.77 (s, 2H), 3.81 (s, 2H), 3.93 (d, J=6.8 Hz, 2H), 6.88 (d, J=8.8 Hz, 1H), 7.20-7.31 (m, 2H), 7.42 (d, J=5.6 Hz, 1H), 7.42 (d, J=8.8 Hz, 1H), 7.64 (dd, J=0.8 Hz, 5.6 Hz, 1H), 7.76-7.79 (m, 1H).

### (7) N,N-Dimethyl{3-{2-[1-(benzo[b]thiophen-4-ylmethyl)piperidin-4-yl]ethyl}-6-cyclopropylmethoxybenz[d]isoxazol-7-yl}methylamine dihydrochloride

The title compound (83 mg) was obtained by synthesis from N,N-dimethyl{3-{2-[1-(benzo[b]thiophen-4-ylmethyl)piperidin-4-yl]ethyl}-6-cyclopropylmethoxybenz[d]isoxazol-7-yl}methylamine (82 mg) according to Example 79-(8).
¹H-NMR (400 MHz, CD₃OD) δ (ppm): 0.41-0.46 (m, 2H), 0.65-0.72 (m, 2H), 1.33-1.42 (m, 1H), 1.43-1.56 (m, 2H), 1.59-1.69 (m, 1H), 1.82 (dt, J=6.8 Hz, 8.0 Hz, 2H), 1.94-2.02 (m, 2H), 2.83 (s, 6H), 2.88 (br t, J=12.0 Hz, 2H), 3.03 (t, J=8.0 Hz, 2H), 3. 38 (br d, J=12.0 Hz, 2H), 4.09 (d, J=7.2 Hz, 2H), 4.48 (s, 2H), 4.88 (s, 2H), 7.20 (d, J=8.8 Hz, 1H), 7.43 (dd, J=7.2 Hz, 8.0 Hz, 1H), 7.52 (d, J=7.2 Hz, 1H), 7.72 (d, J=5.6 Hz, 1H), 7.74 (d, J=5.6 Hz, 1H), 7.85 (d, J=8.8 Hz, 1H), 8.01 (d, J=8.0 Hz, 1H).
ESI-MS m/z: 253 [M+2H]²⁺, 504 [M+H]⁺.

### Example 92

### N,N-Dimethyl{3-{2-[1-(benzo[b]thiophen-7-ylmethyl)piperidin-4-yl]ethyl}-6-cyclopropylmethoxybenz[d]isoxazol-7-yl}methylamine dihydrochloride

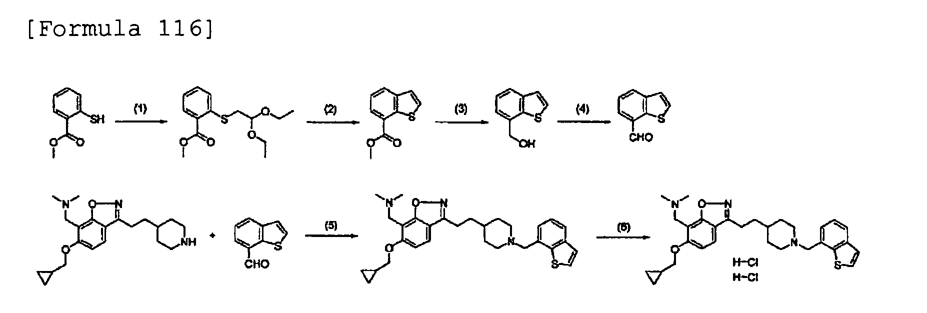

### (1) Methyl 2-(2,2-diethoxyethylsulfanyl)benzoate

Methyl thiosalicylate (7.5 g) was dissolved in tetrahydrofuran (50 mL). Sodium methoxide (28% solution in methanol) (10 mL) was added and the mixture was stirred at room temperature for 10 minutes. 2-Bromo-1,1-diethoxyethane (7.3 mL) was added and the mixture was stirred at 70°C for 19 hours. The reaction mixture was cooled to room temperature. Then, a saturated ammonium chloride solution was added, followed by extraction with ethyl acetate. The organic layer was sequentially washed with water and a saturated sodium chloride solution, dried over magnesium sulfate and filtered. Then, the solvent was evaporated under reduced pressure. The residue was purified by silica gel column chromatography (heptane-ethyl acetate) to obtain the title compound (10.1 g).
¹H-NMR (400 MHz, CDCl₃) δ(ppm): 1.21 (t, J=7.2 Hz, 6H), 3.17 (d, J=5.6 Hz, 2H), 3.57 (dq, J=7.2 Hz, 7.2 Hz, 2H), 3.71 (dq, J=7.2 Hz, 7.2 Hz, 2H), 3.91 (s, 3H), 4.73 (t, J=5.6 Hz, 1H), 7.13-7.18 (m, 1H), 7.38-7.45 (m, 2H), 7.92 (d, J=8.0 Hz, 1H) .

### (2) Methyl benzo[b]thiophene-7-carboxylate

Polyphosphoric acid (20 mL) was stirred at 130°C. A solution of methyl 2-(2,2-diethoxyethylsulfanyl)benzoate (10.1 g) in chlorobenzene (30 mL) was added and the mixture was stirred at 130°C for two hours. The reaction mixture was cooled to room temperature and then poured into ice water, followed by extraction with ethyl acetate. The organic layer was sequentially washed with a saturated sodium bicarbonate solution, water and a saturated sodium chloride solution, dried over magnesium sulfate and filtered. Then, the solvent was evaporated under reduced pressure. The residue was purified by silica gel column chromatography (heptane-ethyl acetate) to obtain the title compound (2.9 g).
¹H-NMR (400 MHz, CDCl₃) δ (ppm): 4.02 (s, 3H), 7.39 (d, J=5.6 Hz, 1H), 7.44 (dd, J=7.6 Hz, 7.6 Hz, 1H), 7.57 (d, J=5.6 Hz, 1H) , 8.02 (d, J=7.6 Hz, 1H), 8.11 (d, J=7.6 Hz, 1H).

### (3) Benzo[b]thiophen-7-ylmethanol

Lithium aluminum hydride (300 mg) was suspended in tetrahydrofuran (20 mL) in a nitrogen atmosphere. A solution of methyl benzo[b]thiophene-7-carboxylate (1.5 g) in tetrahydrofuran (10 mL) was added under ice-cooling, and the mixture was stirred under ice-cooling for 40 minutes. 1 M hydrochloric acid was added dropwise to the reaction mixture under ice-cooling, followed by extraction with ethyl acetate. The organic layer was sequentially washed with water and a saturated sodium chloride solution, dried over magnesium sulfate and filtered. Then, the solvent was evaporated under reduced pressure. The residue was purified by silica gel column chromatography (heptane-ethyl acetate) to obtain the title compound (1.22 g). ¹H-NMR (400 MHz, CDCl₃) δ (ppm) : 1.89 (t, J=6.0 Hz, 1H), 4.98 (t, J=6.0 Hz, 2H), 7.33-7.39 (m, 3H), 7.46 (d, J=5.6 Hz, 1H), 7.75-7.80 (m, 1H).

### (4) Benzo[b]thiophene-7-carbaldehyde

Benzo[b]thiophen-7-ylmethanol (1.22 g) was dissolved in acetone (30 mL). 85% manganese dioxide (6 g) was added and the mixture was stirred at room temperature for 39 hours and 40 minutes. The reaction mixture was filtered through celite and washed with acetone. Then, the solvent was evaporated under reduced pressure. The residue was purified by silica gel column chromatography (heptane-ethyl acetate) to obtain the title compound (943 mg).
¹H-NMR (400 MHz, CDCl₃) δ (ppm) : 7.44 (d, J=5.6 Hz, 1H) , 7.58 (dd, J=7.2 Hz, 8.0 Hz, 1H), 7.66 (d, J=5.6 Hz, 1H), 7.89 (d, J=7.2 Hz, 1H), 8.11 (d, J=8.0 Hz, 1H), 10.24 (s, 1H).

### (5) N,N-Dimethyl{3-{2-[1-(benzo[b]thiophen-7-ylmethyl)piperidin-4-yl]ethyl}-6-cyclopropylmethoxybenz[d]isoxazol-7-yl}methylamine

The title compound (72 mg) was obtained by synthesis from N,N-dimethyl{6-cyclopropylmethoxy-3-[2-(piperidin-4-yl)ethyl]benz[d]isoxazol-7-yl}methylamine shown in Preparation Example 3-(3) (100 mg) and benzo[b]thiophene-7-carbaldehyde (90 mg) according to Example 89-(1).
¹H-NMR (400 MHz, CDCl₃) δ(ppm): 0.35-0.40 (m, 2H), 0.61-0.67 (m, 2H), 1.25-1.48 (m, 4H), 1.60-1.72 (m, 4H), 2.00-2.09 (m, 2H), 2.33 (s, 6H), 2.90 (br d, J=11.6 Hz, 2H), 2.94 (t, J=8.0 Hz, 2H), 3.74 (s, 2H), 3.82 (s, 2H), 3.93 (d, J=6.8 Hz, 2H), 6.88 (d, J=8.8 Hz, 1H), 7.23 (d, J=8.0 Hz, 1H), 7.30 (dd, J=8.0 Hz, 8.0 Hz, 1H), 7.33 (d, J=5.6 Hz, 1H), 7.42 (d, J=5.6 Hz, 1H), 7.43 (d, J=8.8 Hz, 1H), 7.72 (d, J=8.0 Hz, 1H).

### (6) N,N-Dimethyl{3-{2-[1-(benzo[b]thiophen-7-ylmethyl)piperidin-4-yl]ethyl}-6-cyclopropylmethoxybenz[d]isoxazol-7-yl}methylamine dihydrochloride

The title compound (77 mg) was obtained by synthesis from N,N-dimethyl{3-{2-[1-(benzo[b]thiophen-7-ylmethyl)piperidin-4-yl]ethyl}-6-cyclopropylmethoxybenz[d]isoxazol-7-yl}methylamine (70 mg) according to Example 79-(8).
¹H-NMR (400 MHz, CD₃OD) δ(ppm): 0.41-0.47 (m, 2H), 0.66-0.73 (m, 2H), 1.35-1.44 (m, 1H), 1.48-1.62 (m, 2H), 1.62-1.74 (m, 1H), 1.84 (dt, J=7.2 Hz, 8.0 Hz, 2H), 1.96-2.05 (m, 2H), 2.90-3.03 (m, 2H), 2.94 (s, 6H), 3.04 (t, J=8.0 Hz, 2H), 3.43 (br d, J=12.0 Hz, 2H), 4.11 (d, J=7.2 Hz, 2H), 4.43 (s, 2H), 4.61 (s, 2H), 7.22 (d, J=8.8 Hz, 1H) , 7.48 (dd, J=6. Hz, 8.0 Hz, 1H), 7.48 (d, J=5.6 Hz, 1H), 7.55 (d, J=6.8 Hz, 1H), 7.67 (d, J=5.6 Hz, 1H), 7.90 (d, J=8.8 Hz, 1H), 7.94 (d, J=8.0 Hz, 1H) .
ESI-MS m/z: 253 [M+2H]²⁺, 504 [M+H]⁺.

### Example 93

### N,N-Dimethyl{6-cyclopropylmethoxy-3-{2-[1-(4,5,6,7-tetrahydrobenzo[b]thiophen-4-ylmethyl)piperidin-4-yl]ethyl}benz[d]isoxazol-7-yl}methylamine dihydrochloride

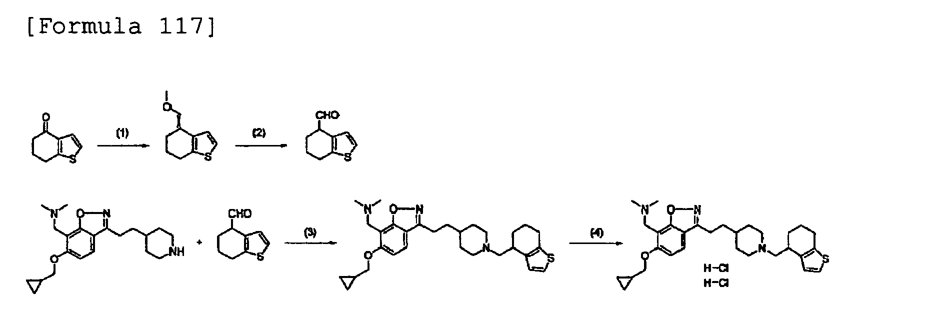

### (1) Mixture of (E)-4-methoxymethylene-4,5,6,7-tetrahydrobenzo[b]thiophene and (Z)-4-methoxymethylene-4, 5, 6, 7-tetrahydrobenzo [b] thiophene

(Methoxymethyl)triphenylphosphonium chloride (3.7 g) was dissolved in tetrahydrofuran (30 mL) in a nitrogen atmosphere. tert-Butoxypotassium (1.3 g) was added under ice-cooling and the mixture was stirred under ice-cooling for 12 minutes. 6,7-Dihydro-5H-benzo[b]thiophen-4-one (1.5 g) was added under ice-cooling, and the mixture was stirred at room temperature for two hours and 40 minutes. A saturated ammonium chloride solution was added to the reaction mixture, followed by extraction with ethyl acetate. The organic layer was sequentially washed with water and a saturated sodium chloride solution, dried over magnesium sulfate and filtered. Then, the solvent was evaporated under reduced pressure. The residue was purified by silica gel column chromatography (heptane-ethyl acetate) to obtain the title compound (1.32 g). ¹H-NMR (400 MHz, CDCl₃) δ(ppm): 1.82-1.93 (m, 2H), 2.25-2.31 (m, 0.5H), 2.44-2.50 (m, 1.5H), 2.79 (t, J=6.0 Hz, 1 .5H) , 2.86 (t, J=6.0 Hz, 0.5H), 3.68 (s, 3H), 5.90 (s, 0.25H), 6.46 (s, 0.75H), 6.95 (d, J=5.2 Hz, 0.75H), 7.01 (d, J=5.2 Hz, 0.25H), 7.04 (d, J=5.2 Hz, 0.75H), 7.57 (d, J=5.2 Hz, 0.25H).

### (2) 4,5,6,7-Tetrahydrobenzo[b]thiophene-4-carbaldehyde

The mixture of (E)-4-methoxymethylene-4,5,6,7-tetrahydrobenzo[b]thiophene and (Z)-4-methoxymethylene-4,5,6,7-tetrahydrobenzo[b]thiophene (1.32 g) was dissolved in acetone (15 mL). Water (5 mL) was added and concentrated sulfuric acid (1.5 mL) was added dropwise. The mixture was stirred at room temperature for 30 minutes and then heated under reflux for one hour and 55 minutes. The reaction mixture was cooled to room temperature and then water was added, followed by extraction with ethyl acetate. The organic layer was sequentially washed with a saturated sodium bicarbonate solution, water and a saturated sodium chloride solution, dried over magnesium sulfate and filtered. Then, the solvent was evaporated under reduced pressure. The residue was purified by silica gel column chromatography (heptane-ethyl acetate) to obtain the title compound (724 mg).
¹H-NMR (400 MHz, CDCl₃) δ(ppm): 1.84-2.00 (m, 3H), 2.08-2.17 (m, 1H), 2.78-2.84 (m, 2H), 3.52-3.57 (m, 1H), 6.87 (d, J=5.2 Hz, 1H) , 7.14 (d, J=5.2 Hz, 1H) , 9.66 (d, J=2.4 Hz, 1H).

### (3) N,N-Dimethyl{6-cyclopropylmethoxy-3-{2-[1-(4,5,6,7-tetrahydrobenzo[b]thiophen-4-ylmethyl)piperidin-4-yl]ethyl}benz[d]isoxazol-7-yl}methylamine

The title compound (99 mg) was obtained by synthesis from N,N-dimethyl{6-cyclopropylmethoxy-3-[2-(piperidin-4-yl)ethyl]benz[d]isoxazol-7-yl}methylamine shown in Preparation Example 3-(3) (100 mg) and 4,5,6,7-tetrahydrobenzo[b]thiophene-4-carbaldehyde (90 mg) according to Example 89-(1).
¹H-NMR (400 MHz, CDCl₃) δ(ppm): 0.34-0.40 (m, 2H), 0.61-0.67 (m, 2H), 1.21-1.39 (m, 4H), 1.57-1.68 (m, 2H), 1.70-1.82 (m, 4H), 1.82-2.03 (m, 4H), 2.29-2.37 (m, 1H), 2.34 (s, 6H), 2.43-2.49 (m, 1H), 2.72-2.77 (m, 2H), 2.85-3.00 (m, 3H), 2.95 (t, J=8.0 Hz, 2H), 3.82 (s, 2H), 3.94 (d, J=6.4 Hz, 2H), 6.89 (d, J=8.8 Hz, 1H), 7.02 (s, 2H), 7.45 (d, J=8.8 Hz, 1H).

### (4) N,N-Dimethyl{6-cyclopropylmethoxy-3-{2-[1-(4,5,6,7-tetrahydrobenzo[b]thiophen-4-ylmethyl)piperidin-4-yl]ethyl}benz[d]isoxazol-7-yl}methylamine dihydrochloride

The title compound (109 mg) was obtained by synthesis from N,N-dimethyl{6-cyclopropylmethoxy-3-{2-[1-(4,5,6,7-tetrahydrobenzo[b]thiophen-4-ylmethyl)piperidin-4-yl]ethyl}benz[d]isoxazol-7-yl}methylamine (97 mg) according to Example 79-(8).
¹H-NMR (400 MHz, CD₃OD) δ (ppm) : 0.41-0.46 (m, 2H), 0.66-0.72 (m, 2H), 1.36-1.44 (m, 1H) , 1.52-1.93 (m, 7H), 1.94-2.10 (m, 4H), 2.76-2.95 (m, 4H), 2.77 (s, 6H), 3.01-3.10 (m, 1H), 3.07 (t, J=7.6 Hz, 2H), 3.21-3.30 (m, 2H), 3. 44 (br d, J=12.0 Hz, 1H), 3.62 (br d, J=12.0 Hz, 1H) , 4.08 (d, J=7.2 Hz, 2H), 4.41 (s, 2H), 6.93 (d, J=5.2 Hz, 1H) , 7.16 (d, J=5.2 Hz, 1H) , 7.20 (d, J=8.8H, 1H), 7.86 (d, J=8.8 Hz, 1H).
ESI-MS m/z: 255 [M+2H]²⁺, 508 [M+H]⁺.

### Example 94

### N,N-Dimethyl{6-cyclopropylmethoxy-3-{2-[1-(imidazo[1,2-a]pyridin-5-ylmethyl)piperidin-4-yl]ethyl}benz[d]isoxazol-7-yl}methylamine trihydrochloride

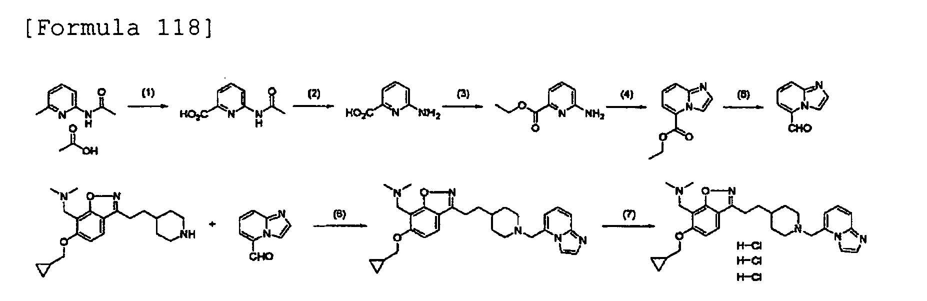

### (1) 6-Acetylaminopyridine-2-carboxylic acid

The title compound was synthesized with reference to J. Carbohydr. Chem., 1994, 13, 715. N-(6-Methylpyridin-2-yl)acetamide acetate (34 g) was neutralized by sequentially adding a 5 M sodium hydroxide solution and a saturated sodium bicarbonate solution, followed by extraction with ethyl acetate. The organic layer was sequentially washed with water and a saturated sodium chloride solution, dried over magnesium sulfate and filtered. Then, the solvent was evaporated under reduced pressure and the resulting N-(6-methylpyridin-2-yl)acetamide (24 g) was suspended in water (400 mL). Potassium permanganate (28 g) was added at 80°C and the mixture was stirred at 80°C for one hour. Then, potassium permanganate (28 g) was further added at 80°C and the mixture was stirred at 80°C for 10.5 hours. The reaction mixture was filtered and the filtrate was washed with chloroform twice. Then, the aqueous layer was evaporated under reduced pressure to 100 mL. Concentrated hydrochloric acid was added until the pH was 4, and the resulting suspension was filtered. The resulting solid was washed with ice water and then dried at 60°C for 45 hours to obtain the title compound (11.9 g). ¹H-NMR (400 MHz, DMSO-d₆) δ (ppm) : 2.11 (s, 3H), 7.72 (d, J=8.4 Hz, 1H) , 7.92 (dd, J=8.4 Hz, 8.4 Hz, 1H) , 8.26 (d, J=8.4 Hz, 1H) , 10.78 (s, 1H), 13.19 (br s, 1H) .

### (2) 6-Aminopyridine-2-carboxylic acid

A mixture of 6-acetylaminopyridine-2-carboxylic acid (13 g) and a 2 M sodium hydroxide solution (100 mL) was heated under reflux for two hours and 40 minutes. The reaction mixture was ice-cooled and concentrated hydrochloric acid was added until the pH was 5 to 6. The resulting suspension was filtered. The precipitate collected by filtration was washed with ice water and then dried at 60°C for 17 hours to obtain the title compound (7.5 g).
¹H-NMR (400 MHz, DMSO-d₆) δ(ppm): 6.68 (dd, J=0.8 Hz, 8.4 Hz, 1H), 6.74 (br s, 2H), 7.15 (dd, J=0.8 Hz, 7.2 Hz, 1H), 7.58 (dd, J=7.2 Hz, 8.4 Hz, 1H).

### (3) Ethyl 6-aminopyridine-2-carboxylate

6-Aminopyridine-2-carboxylic acid (5.1 g) was dissolved in ethanol (100 mL). Concentrated sulfuric acid (4 mL) was added and the mixture was heated under reflux for 23 hours. The reaction mixture was cooled to room temperature, and then the solvent was evaporated under reduced pressure. Water was added to the residue, followed by neutralization with a saturated sodium bicarbonate solution under ice-cooling. The reaction mixture was extracted with chloroform twice. The organic layers were washed with a saturated sodium chloride solution and dried over magnesium sulfate. The drying agent was removed by filtration and then the solvent was evaporated under reduced pressure. The title compound (4.75 g) was obtained as a residue.
¹H-NMR (400 MHz, CDCl₃) δ(ppm): 1.41 (t, J=7.2 Hz, 3H), 4.43 (q, J=7.2 Hz, 2H), 4.68 (br s, 2H), 6.65 (dd, J=1.2 Hz, 8.0 Hz, 1H), 7.47 (dd, J=1.2 Hz, 7.2 Hz, 1H), 7.53 (dd, J=7.2 Hz, 8.0 Hz, 1H).

### (4) Ethyl imidazo[1,2-a]pyridine-5-carboxylate

Ethyl 6-aminopyridine-2-carboxylate (2.6 g) was dissolved in ethanol (100 mL). Sodium carbonate (2.5 g) and chloroacetaldehyde (40% aqueous solution) (15 mL) were sequentially added and the mixture was heated under reflux for seven hours. The reaction mixture was cooled to room temperature, and then the solvent was evaporated under reduced pressure. Water was added to the residue, followed by extraction with ethyl acetate. The organic layer was washed with a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (2.39 g).
¹H-NMR (400 MHz, CDCl₃) δ(ppm): 1.46 (t, J=7.2 Hz, 3H), 4.48 (q, J=7.2 Hz, 2H), 7.23 (dd, J=7.2 Hz, 8.8 Hz, 1H), 7.78 (d, J=1.2 Hz, 1H), 7.80 (d, J=7.2 Hz, 1H), 7.89 (d, J=8.8 Hz, 1H) , 8.82-8.85 (m, 1H).

### (5) Imidazo[1,2-a]pyridine-5-carbaldehyde

Ethyl imidazo[1,2-a]pyridine-5-carboxylate (824 mg) was dissolved in methylene chloride (40 mL). Diisobutylaluminum hydride (1 M solution in toluene) (5 mL) was added at -78°C and the mixture was stirred at - 78°C for two hours. Diisobutylaluminum hydride (1 M solution in toluene) (10 mL) was further added to the mixture, followed by stirring at -78°C for two hours. Methanol (1 mL) was added to the reaction mixture at - 78°C, followed by addition of a 20% (+)-potassium sodium tartrate solution. After stirring at room temperature, the reaction mixture was filtered through celite and washed with methylene chloride, followed by extraction with methylene chloride twice. The organic layers were combined, washed with a saturated sodium chloride solution and then dried over magnesium sulfate. The drying agent was removed by filtration and the solvent was evaporated under reduced pressure. The resulting residue was dissolved in acetone (15 mL). 80% manganese dioxide (3 g) was added and the mixture was stirred at room temperature for 11 hours and 20 minutes. The reaction mixture was filtered and washed with acetone. The solvent of the filtrate was evaporated under reduced pressure. The residue was purified by silica gel column chromatography (ethyl acetate-methanol) to obtain the title compound (96 mg).
¹H-NMR (400 MHz, CDCl₃) δ(ppm): 7.35-7.44 (m, 1H), 7.53 (d, J=6.8 Hz, 1H), 7.88 (s, 1H), 7.99 (d, J=8.8 Hz, 1H) , 9.04 (s, 1H) , 9.92 (s, 1H) .

### (6) N,N-Dimethyl{6-cyclopropylmethoxy-3-{2-[1-(imidazo[1,2-a]pyridin-5-ylmethyl)piperidin-4-yl]ethyl}benz[d]isoxazol-7-yl}methylamine

The title compound (64 mg) was obtained by synthesis from N,N-dimethyl{6-cyclopropylmethoxy-3-{2-[1-(imidazo[1,2-a]pyridin-5-ylmethyl)piperidin-4-yl]ethyl}benz[d]isoxazol-7-yl}methylamine (compound obtained in Preparation Example 3-(3)) (100 mg) and imidazo[1,2-a]pyridine-5-carbaldehyde (70 mg) according to Example 89-(1).
¹H-NMR (400 MHz, CDCl₃) δ(ppm):0.35-0.39 (m, 2H), 0.61-0.67 (m, 2H), 1.23-1.45 (m, 4H), 1.72-1.79 (m, 4H), 2.07 (br t, J=11.6 Hz, 2H), 2.34 (s, 6H), 2.85 (br d, J=11.6 Hz, 2H), 2.93 (t, J=7.6 Hz, 2H), 3.69 (s, 2H), 3.82 (s, 2H), 3.93 (d, J=6.8 Hz, 2H), 6.70 (d, J=6.8 Hz, 1H), 6.88 (d, J=8.8 Hz, 1H), 7.13 (dd, J=6.8 Hz, 9.2 Hz, 1H), 7.43 (d, J=8.8 Hz, 1H) , 7.57 (d, J=9.2 Hz, 1H) , 7.64 (d, J=1.6 Hz, 1H) , 7.88 (d, J=1.6 Hz, 1H) .

### (7) N,N-Dimethyl{6-cyclopropylmethoxy-3-{2-[1-(imidazo[1,2-a]pyridin-5-ylmethyl)piperidin-4-yl]ethyl}benz[d]isoxazol-7-yl}methylamine trihydrochloride

The title compound (70 mg) was obtained by synthesis from N,N-dimethyl{6-cyclopropylmethoxy-3-{2-[1-(imidazo[1,2-a]pyridin-5-ylmethyl)piperidin-4-yl]ethyl}benz[d]isoxazol-7-yl}methylamine (62 mg) according to Example 79-(8).
¹H-NMR (400 MHz, CD₃OD) δ (ppm) : 0.42-0.47 (m, 2H), 0.66-0.73 (m, 2H), 1.35-1.45 (m, 1H), 1.64-1.90 (m, 5H), 2.10 (br d, J=13.2 Hz, 2H), 2.96 (s, 6H), 3.04 (t, J=7.6 Hz, 2H), 3.31-3.41 (m, 2H), 3.69 (br d, J=12.4 Hz, 2H), 4.11 (d, J=7.2 Hz, 2H), 4.63 (s, 2H), 5.02 (s, 2H), 7.23 (d, J=8.8 Hz, 1H), 7.89-7.95 (m, 2H), 8.03-8.12 (m, 2H), 8.22 (d, J=2.0 Hz, 1H), 8.80 (d, J=2.0 Hz, 1H) .
ESI-MS m/z: 488 [M+H]⁺.

### Example 95

### N,N-Dimethyl{6-cyclopropylmethoxy-3-{2-[1-(imidazo[1,2-a]pyridin-8-ylmethyl)piperidin-4-yl]ethyl}benz[d]isoxazol-7-yl}methylamine trihydrochloride

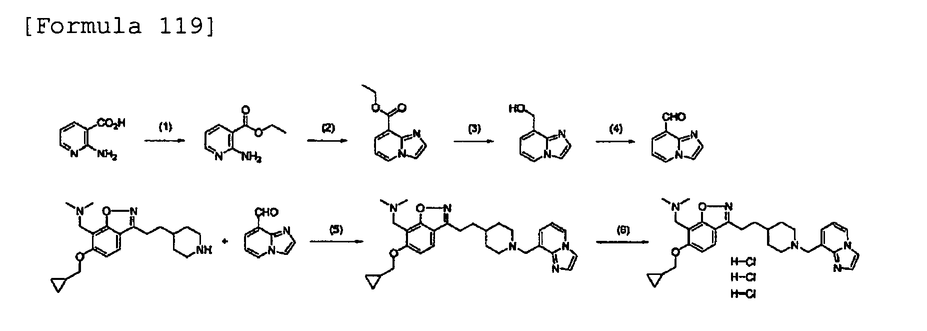

### (1) Ethyl 2-aminopyridine-3-carboxylate

The title compound (3.16 g) was obtained by synthesis from 2-aminonicotinic acid (5.1 g) according to Example 94-(3).
¹H-NMR (400 MHz, CDCl₃) δ(ppm): 1.38 (t, J=7.2 Hz, 3H), 4.34 (q, J=7.2 Hz, 2H), 6.40 (br s, 2H), 6.61 (dd, J=4.8 Hz, 8.0 Hz, 1H), 8.13 (dd, J=2.0 Hz, 8.0 Hz, 1H), 8.20 (dd, J=2.0 Hz, 4.8 Hz, 1H).

### (2) Ethyl imidazo[1,2-a]pyridine-8-carboxylate

The title compound (1.54 g) was obtained by synthesis from ethyl 2-aminonicotinate (2 g) according to Example 94-(4).
¹H-NMR (400 MHz, CDCl₃) δ(ppm): 1.46 (t, J=7.2 Hz, 3H), 4.52 (q, J=7.2 Hz, 2H), 6.87 (dd, J=6.8 Hz, 7.2 Hz, 1H) , 7.65 (d, J=1.2 Hz, 1H) , 7.78 (d, J=1.2 Hz, 1H) , 7.95 (dd, J=1.2 Hz, 7.2 Hz, 1H) , 8.30 (dd, J=1.2 Hz, 6.8 Hz, 1H).

### (3) Imidazo[1,2-a]pyridin-8-ylmethanol

The title compound (422 mg) was obtained by synthesis from ethyl imidazo[1,2-a]pyridine-8-carboxylate (1.54 g) according to Example 92-(3). ¹H-NMR (400 MHz, CDCl₃) δ(ppm): 4.10 (br s, 1H) , 5.03 (s, 2H), 6.77 (dd, J=6.8 Hz, 6.8 Hz, 1H) , 7.09 (dd, J=0.8 Hz, 6.8 Hz, 1H), 7.58 (d, J=1.2 Hz, 1H), 7.60 (d, J=1.2 Hz, 1H), 8.06 (dd, J=0.8 Hz, 6.8 Hz, 1H).

### (4) Imidazo[1,2-a]pyridine-8-carbaldehyde

The title compound (146 mg) was obtained by synthesis from imidazo[1,2-a]pyridin-8-ylmethanol (420 mg) according to Example 92-(4).
¹H-NMR (400 MHz, CDCl₃) δ(ppm): 6.97 (dd, J=6.8 Hz, 7.2 Hz, 1H), 7.71 (d, J=1.2 Hz, 1H), 7.79 (d, J=1.2H, 1H), 7.82 (dd, J=1.2 Hz, 7.2 Hz, 1H), 8.37 (dd, J=1.2 Hz, 6.8 Hz, 1H) , 10.74 (s, 1H) .

### (5) N,N-Dimethyl{6-cyclopropylmethoxy-3-{2-[1-(imidazo[1,2-a]pyridin-8-ylmethyl)piperidin-4-yl]ethyl}benz[d]isoxazol-7-yl}methylamine

The title compound (86 mg) was obtained by synthesis from N,N-dimethyl{6-cyclopropylmethoxy-3-[2-(piperidin-4-yl)ethyl]benz[d]isoxazol-7-yl}methylamine (compound obtained in Preparation Example 3-(3)) (100 mg) and imidazo[1,2-a]pyridine-8-carbaldehyde (70 mg) according to Example 89-(1).
¹H-NMR (400 MHz, CDCl₃) δ (ppm) : 0.35-0.40 (m, 2H), 0.61-0.67 (m, 2H), 1.25-1.45 (m, 4H), 1.73-1.82 (m, 4H), 2.14 (br t, J=10.8 Hz, 2H), 2.33 (s, 6H), 2.94 (t, J=8.0 Hz, 2H), 3.00 (br d, J=10.8 Hz, 2H), 3.82 (s, 2H), 3.93 (d, J=6.4 Hz, 2H), 3.94 (s, 2H), 6.78 (dd, J=6.8 Hz, 6.8 Hz, 1H), 6.89 (d, J=8.8 Hz, 1H), 7.23-7.27 (m, 1H) , 7.43 (d, J=8.8 Hz, 1H) , 7.57 (d, J=1.6 Hz, 1H), 7.60 (d, J=1.6 Hz, 1H), 8.03 (d, J=6.8 Hz, 1H) .

### (6) N,N-Dimethyl{6-cyclopropylmethoxy-3-{2-[1-(imidazo[1,2-a]pyridin-8-ylmethyl)piperidin-4-yl]ethyl}benz[d]isoxazol-7-yl}methylamine trihydrochloride

The title compound (94 mg) was obtained by synthesis from N,N-dimethyl{6-cyclopropylmethoxy-3-{2-[1-(imidazo[1,2-a]pyridin-8-ylmethyl)piperidin-4-yl]ethyl}benz[d]isoxazol-7-yl}methylamine (84 mg) according to Example 79-(8).
¹H-NMR (400 MHz, CD₃OD) δ(ppm): 0.42-0.47 (m, 2H), 0.67-0.73 (m, 2H), 1.35-1.45 (m, 1H) , 1.60-1.82 (m, 5H), 2.10 (br d, J=13.2 Hz, 2H), 2.96 (s, 6H), 3.07 (t, J=8.0 Hz, 2H), 3.24-3.36 (m, 2H), 3.68 (br d, J=10.8 Hz, 2H), 4.11 (d, J=7.2 Hz, 2H), 4.63 (s, 2H), 4.84 (s, 2H), 7.23 (d, J=8.8 Hz, 1H), 7.60 (dd, J=6.8 Hz, 7.2 Hz, 1H), 7.92 (d, J=8.8 Hz, 1H) , 8.18 (d, J=2.0 Hz, 1H), 8.29 (d, J=7.2 Hz, 1H) , 8.35 (d, J=2.0 Hz, 1H) , 8.95 (d, J=6.8 Hz, 1H).
ESI-MS m/z: 488 [M+H]⁺, 510 [M+Na]⁺.

### Example 96

### 6-{4-{2-[6-Cyclopropylmethoxy-7-(N,N-dimethylaminomethyl)benz[d]isoxazol-3-yl]ethyl} piperidinomethyl}pyridin-2-ylamine trihydrochloride

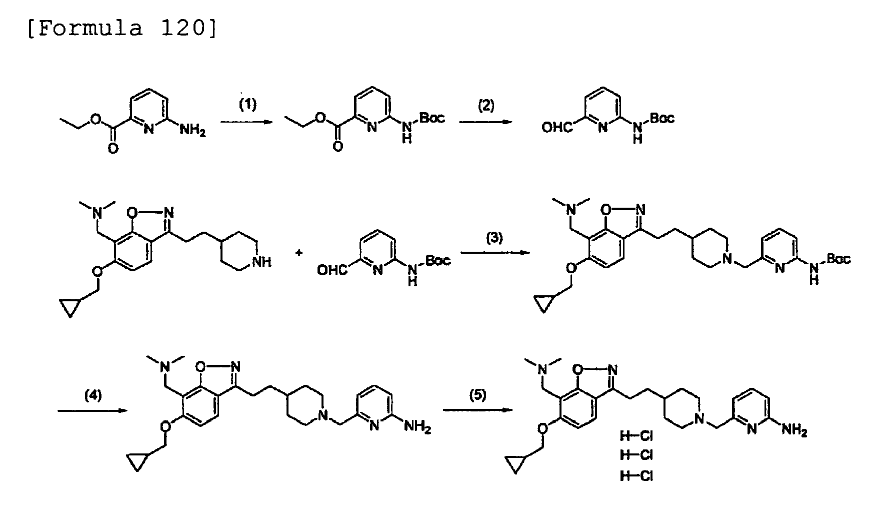

### (1) Ethyl 6-tert-butoxycarbonylaminopyridine-2-carboxylate

A mixture of ethyl 6-aminopyridine-2-carboxylate shown in Example 94-(3) (2 g), di-tert-butyl dicarbonate (3 g), triethylamine (5 mL) and tetrahydrofuran (40 mL) was stirred at 60°C for six hours. Di-tert-butyl dicarbonate (15 g) was further added to the mixture, followed by heating under reflux for three hours. The reaction mixture was cooled to room temperature and then water was added, followed by extraction with ethyl acetate. The organic layer was sequentially washed with water and a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (1.9 g).
¹H-NMR (400 MHz, DMSO-d₆) δ(ppm): 1.31 (t, J=7.2 Hz, 3H), 1.46 (s, 9H), 4.32 (q, J=7.2 Hz, 2H), 7.68 (d, J=7.2 Hz, 1H) , 7.90 (dd, J=7.2 Hz, 8.4 Hz, 1H) , 8.01 (d, J=8.4 Hz, 1H) , 10.10 (s, 1H) .

### (2) tert-Butyl (6-formylpyridin-2-yl)carbamate

Ethyl 6-tert-butoxycarbonylaminopyridine-2-carboxylate (1.5 g) was dissolved in methylene chloride (30 mL). Diisobutylaluminum hydride (1 M solution in toluene) (5 mL) was added dropwise at -78°C over 10 minutes, and the mixture was stirred at -78°C for three hours. Methanol (1 mL) was added to the reaction mixture at -78°C, followed by addition of a 20% (+)-potassium sodium tartrate solution. After stirring at room temperature, the reaction mixture was filtered through celite and washed with methylene chloride, followed by extraction with methylene chloride twice. The organic layers were washed with a saturated sodium chloride solution and then dried over magnesium sulfate. The drying agent was removed by filtration and the solvent was evaporated under reduced pressure. The residue was purified by silica gel column chromatography (heptane-ethyl acetate) to obtain the title compound (604 mg).
¹H-NMR (400 MHz, CDCl₃) δ (ppm) : 1.54 (s, 9H), 7.40 (br s, 1H) , 7.60 (d, J=7.6 Hz, 1H) , 7.83 (dd, J=7.6 Hz, 8.4 Hz, 1H) , 8.19 (d, J=8.4 Hz, 1H), 9.88 (s, 1H) .

### (3) tert-Butyl {6-{4-{2-[6-cyclopropylmethoxy-7-(N,N-dimethylaminomethyl)benz[d]isoxazol-3-yl]ethyl}piperidinomethyl}pyridin-2-yl}carbamate

The title compound (168 mg) was obtained by synthesis from N,N-dimethyl{6-cyclopropylmethoxy-3-[2-(piperidin-4-yl)ethyl]benz[d]isoxazol-7-yl}methylamine shown in Preparation Example 3-(3) (150 mg) and tert-butyl (6-formylpyridin-2-yl)carbamate (150 mg) according to Example 89-(1).
¹H-NMR (400 MHz, CDCl₃) δ(ppm): 0.35-0.40 (m, 2H), 0.61-0.67 (m, 2H), 1.27-1.42 (m, 4H), 1.51 (s, 9H), 1.70-1.81 (m, 4H), 1.95-2.04 (m, 2H), 2.34 (s, 6H), 2.88 (br d, J=10.8 Hz, 2H), 2.93 (t, J=8.0 Hz, 2H), 3.48 (s, 2H), 3.83 (s, 2H), 3.94 (d, J=6.8 Hz, 2H), 6.89 (d, J=8.8 Hz, 1H), 7.00 (d, J=7.6 Hz, 1H), 7.43 (d, J=8.8 Hz, 1H), 7.59 (dd, J=7.6 Hz, 8.0 Hz, 1H), 7.76 (d, J=8.0 Hz, 1H).

### (4) 6-{4-{2-[6-Cyclopropylmethoxy-7-(N,N-dimethylaminomethyl)benz[d]isoxazol-3-yl]ethyl}piperidinomethyl}pyridin-2-ylamine

tert-Butyl {6-{4-{2-[6-cyclopropylmethoxy-7-(N,N-dimethylaminomethyl)benz[d]isoxazol-3-yl]ethyl}piperidinomethyl}pyridin-2-yl}carbamate (166 mg) was dissolved in methanol (2 mL). Hydrogen chloride (4 M solution in ethyl acetate) (2 mL) was added to the solution, and the mixture was stirred at room temperature for 16 hours and 40 minutes. Concentrated aqueous ammonia was added to the reaction mixture until the pH was 7, followed by extraction with ethyl acetate. The organic layer was sequentially washed with water and a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (93 mg).
¹H-NMR (400 MHz, CDCl₃) δ(ppm): 0.35-0.40 (m, 2H), 0.61-0.67 (m, 2H), 1.27-1.43 (m, 4H), 1.70-1.81 (m, 4H), 1.95-2.03 (m, 2H), 2.33 (s, 6H), 2.88-2.95 (m, 2H), 2.93 (t, J=8.0 Hz, 2H), 3.44 (s, 2H), 3.82 (s, 2H), 3.93 (d, J=6.4 Hz, 2H), 4.40 (br s, 2H), 6.36 (d, J=8.4 Hz, 1H), 6.71 (d, J=7.6 Hz, 1H), 6.88 (d, J=8.8 Hz, 1H), 7.37 (dd, J=7.6 Hz, 8.4 Hz, 1H) , 7.43 (d, J=8.8 Hz, 1H).

### (5) 6-{4-{2-[6-Cyclopropylmethoxy-7-(N,N-dimethylaminomethyl)benz[d]isoxazol-3-yl]ethyl}piperidinomethyl}pyridin-2-ylamine trihydrochloride

The title compound (102 mg) was obtained by synthesis from 6-{4-{2-[6-cyclopropylmethoxy-7-(N,N-dimethylaminomethyl)benz[d]isoxazol-3-yl]ethyl}piperidinomethyl}pyridin-2-ylamine (91 mg) according to Example 79-(8).
¹H-NMR (400 MHz, CD₃OD) δ (ppm) : 0.42-0.47 (m, 2H), 0.67-0.73 (m, 2H), 1.35-1.45 (m, 1H), 1.64-1.84 (m, 3H), 1.88 (dt, J=7.6 Hz, 7.6 Hz, 2H), 2.11 (br d, J=14.0 Hz, 2H), 2.96 (s, 6H), 3.07 (t, J=7.6 Hz, 2H), 3.19 (br t, J=12.0 Hz, 2H), 3.61 (br d, J=12.0 Hz, 2H), 4.12 (d, J=7.2 Hz, 2H), 4.49 (s, 2H), 4.63 (s, 2H), 7.10 (d, J=8.8 Hz, 1H), 7.13 (d, J=7.2 Hz, 1H), 7.24 (d, J=9.2 Hz, 1H) , 7 . 93 (d, J=9.2 Hz, 1H), 7.94 (dd, J=7.2 Hz, 8.8 Hz, 1H).
ESI-MS m/z: 233 [M+2H]²⁺, 464 [M+H]⁺.

### Example 97

### N,N-Dimethyl{3-[2-(1-benzylpiperidin-4-yl)ethyl]-6-(1-propynyl)benz[d]isoxazol-7-yl}methylamine dihydrochloride

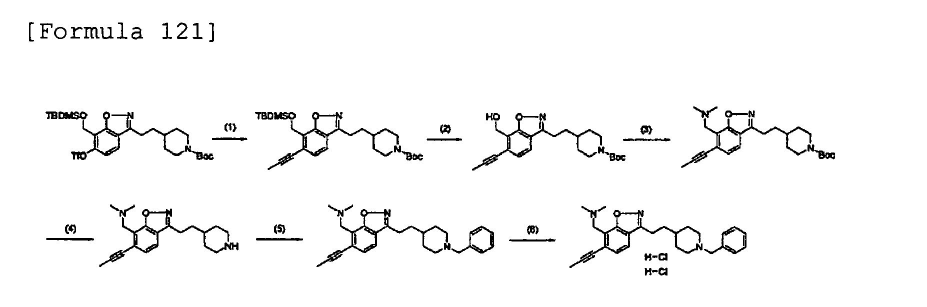

### (1) tert-Butyl 4-{2-[7-(tert-butyldimethylsilanyloxymethyl)-6-(1-propynyl)benz[d]isoxazol-3-yl]ethyl}piperidine-1-carboxylate

The title compound (385 mg) was obtained by synthesis from tert-butyl 4-{2-[7-(tert-butyldimethylsilanyloxymethyl)-6-trifluoromethanesulfonyloxybenz[d]isoxazol-3-yl]ethyl}piperidine-1-carboxylate (compound obtained in Example 79-(2)) (1.8 g) and tributyl(1-propynyl)tin (1.46 g) according to Example 79-(4).
¹H-NMR (400 MHz, CDCl₃) δ (ppm) : 0.13 (s, 6H), 0.93 (s, 9H), 1.10-1.21 (m, 2H), 1.45 (s, 9H), 1.46-1.55 (m, 1H), 1.69-1.82 (m, 4H), 2.12 (s, 3H), 2.61-2.73 (m, 2H), 2.98 (t, J=8.0 Hz, 2H), 4.03-4.16 (m, 2H), 5.11 (s, 2H), 7.30 (d, J=8.4 Hz, 1H), 7.43 (d, J=8.4 Hz, 1H) .

### (2) tert-Butyl 4-{2-[7-hydroxymethyl-6-(1-propynyl)benz[d]isoxazol-3-yl]ethyl}piperidine-1-carboxylate

The title compound (228 mg) was obtained by synthesis from tert-butyl 4-{2-[7-(tert-butyldimethylsilanyloxymethyl)-6-(1-propynyl)benz[d]isoxazol-3-yl]ethyl}piperidine-1-carboxylate (382 mg) according to Example 79-(5). ¹H-NMR (400 MHz, CDCl₃) δ(ppm): 1.16 (ddd, J=4.4 Hz, 14.4 Hz, 24.4 Hz, 2H), 1.45 (s, 9H), 1.47-1.56 (m, 1H), 1.71-1.80 (m, 2H), 1.78 (dt, J=7.2 Hz, 7.6 Hz, 2H), 2.15 (s, 3H), 2.38 (t, J=6.8 Hz, 1H), 2.67 (br d, J=12.0 Hz, 2H), 2.99 (t, J=7.6 Hz, 2H), 4.02-4.18 (m, 2H), 5.12 (d, J=6.8 Hz, 2H), 7.33 (d, J=8.0 Hz, 1H), 7.46 (d, J=8.0 Hz, 1H) .

### (3) tert-Butyl 4-{2-[7-(N,N-dimethylaminomethyl)-6-(1-propynyl)benz[d]isoxazol-3-yl]ethyl}piperidine-1-carboxylate

The title compound (210 mg) was obtained by synthesis from tert-butyl 4-{2-[7-hydroxymethyl-6-(1-propynyl)benz[d]isoxazol-3-yl]ethyl}piperidine-1-carboxylate (226 mg) according to Example 79-(6).
¹H-NMR (400 MHz, CDCl₃) δ(ppm): 1.16 (ddd, J=4.0 Hz, 12.0 Hz, 24.0 Hz, 2H), 1.45 (s, 9H), 1.47-1.55 (m, 1H), 1.72-1.80 (m, 2H), 1.78 (dt, J=7.2 Hz, 8.0 Hz, 2H), 2.14 (s, 3H), 2.35 (s, 6H), 2.67 (br d, J=11.2 Hz, 2H), 2.98 (d, J=8.0 Hz, 2H), 3.89 (s, 2H), 4.03-4.15 (m, 2H), 7.33 (d, J=8.0 Hz, 1H) , 7.43 (d, J=8.0 Hz, 1H).

### (4) N,N-Dimethyl{3-[2-(piperidin-4-yl)ethyl]-6-(1-propynyl)benz[d]isoxazol-7-yl}methylamine

The title compound (126 mg) was obtained by synthesis from tert-butyl 4-{2-[7-(N,N-dimethylaminomethyl)-6-(1-propynyl)benz[d]isoxazol-3-yl]ethyl}piperidine-1-carboxylate (166 mg) according to Example 96-(4).
¹H-NMR (400 MHz, CDCl₃) δ (ppm) : 1.12-1.23 (m, 2H), 1.42-1.53 (m, 1H), 1.72-1.81 (m, 4H), 2.14 (s, 3H), 2.35 (s, 6H), 2.58 (br t, J=12.0 Hz, 2H), 2.97 (t, J=8.0 Hz, 2H), 3.07 (br d, J=12.0 Hz, 2H), 3.89 (s, 2H), 7.32 (d, J=8.4 Hz, 1H) , 7.44 (d, J=8.4 Hz, 1H) .

### (5) N,N-Dimethyl{3-[2-(1-benzylpiperidin-4-yl)ethyl]-6-(1-propynyl)benz[d]isoxazol-7-yl}methylamine

The title compound (56 mg) was obtained by synthesis from N,N-dimethyl{3-[2-(piperidin-4-yl)ethyl]-6-(1-propynyl)benz[d]isoxazol-7-yl}methylamine (51 mg) and benzaldehyde (30 mg) according to Example 89-(1).
¹H-NMR (400 MHz, CDCl₃) δ (ppm) : 1.25-1.37 (m, 3H), 1.71-1.79 (m, 4H), 1.90-1.97 (m, 2H), 2.14 (s, 3H), 2.35 (s, 6H), 2.84-2.91 (m, 2H), 2.95 (t, J=7.6 Hz, 2H), 3.48 (s, 2H), 3.88 (s, 2H), 7.21-7.30 (m, 5H), 7.31 (d, J=8.4 Hz, 1H) , 7.42 (d, J=8.4 Hz, 1H) .

### (6) N,N-Dimethyl{3-[2-(1-benzylpiperidin-4-yl)ethyl]-6-(1-propynyl)benz[d]isoxazol-7-yl}methylamine dihydrochloride

The title compound (58 mg) was obtained by synthesis from N,N-dimethyl{3-[2-(1-benzylpiperidin-4-yl)ethyl]-6-(1-propynyl)benz[d]isoxazol-7-yl}methylamine (54 mg) according to Example 79-(8).
¹H-NMR (400 MHz, CD₃OD) δ (ppm) : 1.49-1.61 (m, 2H), 1.67-1.77 (m, 1H), 1.86 (dt, J=7.2 Hz, 8.0 Hz, 2H), 2.08 (br d, J=14.4 Hz, 2H), 2.22 (s, 3H), 2.97-3.06 (m, 2H), 3.00 (s, 6H), 3.10 (t, J=8.0 Hz, 2H), 3.50 (br d, J=12.4 Hz, 2H), 4.31 (s, 2H), 4.79 (s, 2H), 7.46-7.57 (m, 6H), 7.93 (d, J=9.2 Hz, 1H).
ESI-MS m/z: 209 [M+2H]²⁺, 416 [M+H]⁺.

### Example 98

### N,N-Dimethyl{3-{2-[1-(2-butynyl)piperidin-4-yl]ethyl}-6-cyclopropylmethoxybenz[d]isoxazol-7-yl}methylamine dihydrochloride

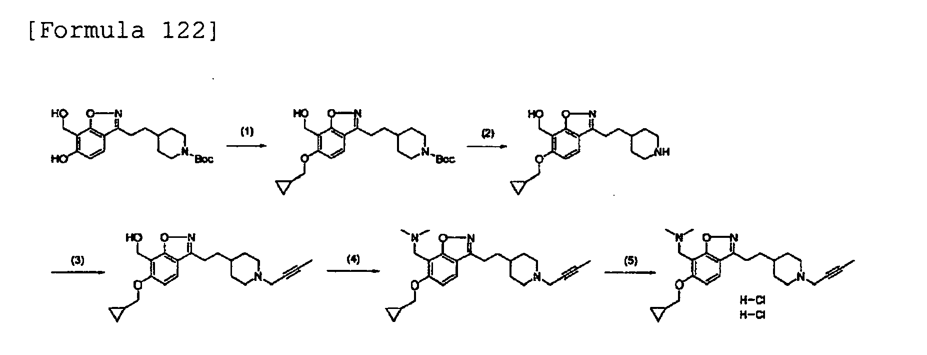

### (1) tert-Butyl 4-[2-(6-cyclopropylmethoxy-7-hydroxymethylbenz[d]isoxazol-3-yl)ethyl]piperidine-1-carboxylate

The title compound (2.55 g) was obtained by synthesis from tert-butyl 4-[2-(6-hydroxy-7-hydroxymethylbenz[d]isoxazol-3-yl)ethyl]piperidine-1-carboxylate (compound obtained in Preparation Example 2-(6)) (2.66 g) and cyclopropylmethyl bromide (1 mL) according to Example 874-(1).
¹H-NMR (400 MHz, CDCl₃) δ (ppm) : 0.36-0.41 (m, 2H), 0.65-0.72 (m, 2H), 1.15 (ddd, J=4.0 Hz, 12.4 Hz, 24.0 Hz, 2H), 1.28-1.38 (m, 1H) , 1.42-1.53 (m, 1H) , 1.45 (s, 9H), 1.70-1.82 (m, 4H), 2.62-2.73 (m, 2H), 2.63 (t, J=6.8 Hz, 1H), 2.96 (t, J=8.0 Hz, 2H), 3.97 (d, J=7.2 Hz, 2H), 4.02-4.15 (m, 2H), 5.03 (d, J=6.8 Hz, 2H), 6.90 (d, J=8.8 Hz, 1H), 7.46 (d, J=8.8 Hz, 1H).

### (2) {6-Cyclopropylmethoxy-3-[2-(piperidin-4-yl)ethyl]benz[d]isoxazol-7-yl}methanol

tert-Butyl 4-[2-(6-cyclopropylmethoxy-7-hydroxymethylbenz[d]isoxazol-3-yl)ethyl]piperidine-1-carboxylate (2.34 g) was dissolved in methylene chloride (30 mL). Trifluoroacetic acid (10 mL) was added to the solution, and the mixture was stirred at room temperature for 15 minutes. Concentrated aqueous ammonia was added to the reaction mixture until the pH was 7, followed by extraction with ethyl acetate. The organic layer was sequentially washed with water and a saturated sodium chloride solution and dried over magnesium sulfate. The drying agent was removed by filtration and then the solvent was evaporated under reduced pressure. The residue was purified by NH silica gel column chromatography (chloroform-methanol) to obtain the title compound (1.55 g).
¹H-NMR (400 MHz, CDCl₃) δ(ppm): 0.36-0.41 (m, 2H), 0.65-0.71 (m, 2H), 1.17 (ddd, J=4.0 Hz, 12.4 Hz, 24.4 Hz, 2H), 1.28-1.36 (m, 1H), 1.39-1.54 (m, 1H), 1.70-1.80 (m, 4H), 2.57 (dt, J=2.8 Hz, 12.0 Hz, 2H), 2.95 (t, J=8.0 Hz, 2H), 3.06 (br d, J=12.0 Hz, 2H), 3.97 (d, J=7.2 Hz, 2H), 5.03 (s, 2H), 6.90 (d, J=8.4 Hz, 1H), 7.47 (d, J=8.4 Hz, 1H).

### (3) {3-{2-[1-(2-Butynyl)piperidin-4-yl]ethyl}-6-cyclopropylmethoxybenz[d]isoxazol-7-yl}methanol

A mixture of {6-cyclopropylmethoxy-3-[2-(piperidin-4-yl)ethyl]benz[d]isoxazol-7-yl}methanol (100 mg), 1-bromo-2-butyne (32 µL), triethylamine (0.2 mL) and N,N-dimethylformamide (3 mL) was stirred at 50°C for four hours. Water was added to the reaction mixture, followed by extraction with ethyl acetate. The organic layer was sequentially washed with water and a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (68 mg).
¹H-NMR (400 MHz, CDCl₃) δ(ppm): 0.36-0.41 (m, 2H), 0.65-0.71 (m, 2H), 1.28-1.40 (m, 4H), 1.73-1.82 (m, 4H), 1.82 (t, J=2.4 Hz, 3H), 2.08 (br d, J=11.2 Hz, 2H), 2.59-2.72 (m, 1H), 2.88-2.98 (m, 4H), 3.18 (q, J=2.4 Hz, 2H), 3.97 (d, J=6.8 Hz, 2H), 5.03 (s, 2H), 6.90 (d, J=8.8 Hz, 1H), 7.46 (d, J=8.8 Hz, 1H).

### (4) N,N-Dimethyl{3-{2-[1-(2-butynyl)piperidin-4-yl]ethyl}-6-cyclopropylmethoxybenz[d]isoxazol-7-yl}methylamine

The title compound (44 mg) was obtained by synthesis from {3-{2-[1-(2-butynyl)piperidin-4-yl]ethyl}-6-cyclopropylmethoxybenz[d]isoxazol-7-yl}methanol (66 mg) according to Example 79-(6).
¹H-NMR (400 MHz, CDCl₃) δ (ppm) : 0.35-0.40 (m, 2H), 0.62-0.67 (m, 2H), 1.26-1.40 (m, 4H), 1.74-1.84 (m, 4H), 1.82 (t, J=2.4 Hz, 3H), 2.09 (br t, J=10.8 Hz, 2H), 2.33 (s, 6H), 2.88-2.97 (m, 4H), 3.18 (q, J=2.4 Hz, 2H), 3.82 (s, 2H), 3.94 (d, J=6.8 Hz, 2H), 6.89 (d, J=8.8 Hz, 1H), 7.44 (d, J=8.8 Hz, 1H) .

### (5) N,N-Dimethyl{3-{2-[1-(2-butynyl)piperidin-4-yl]ethyl}-6-cyclopropylmethoxybenz[d]isoxazol-7-yl}methylamine dihydrochloride

The title compound (44 mg) was obtained by synthesis from N,N-dimethyl{3-{2-[1-(2-butynyl)piperidin-4-yl]ethyl}-6-cyclopropylmethoxybenz[d]isoxazol-7-yl}methylamine (42 mg) according to Example 79-(8).
¹H-NMR (400 MHz, CD₃OD) δ(ppm): 0.42-0.47 (m, 2H), 0.67-0.73 (m, 2H), 1.33-1.45 (m, 1H), 1.46-1.59 (m, 2H), 1.66-1.78 (m, 1H), 1.86 (dt, J=7.2 Hz, 8.0 Hz, 2H), 1.93 (t, J=2.4 Hz, 3H), 2.10-2.18 (m, 2H), 2.96 (s, 6H), 2.98-3.10 (m, 4H), 3.65 (br d, J=12.4 Hz, 2H), 3.97 (q, J=2.4 Hz, 2H), 4.12 (d, J=7.2 Hz, 2H), 4.64 (s, 2H), 7.24 (d, J=8.8 Hz, 1H) , 7.92 (d, J=8.8 Hz, 1H) .
ESI-MS m/z: 206 [M+2H ]²⁺ , 410 [M+H]⁺.

### Example 99

### N,N-Dimethyl{6-cyclopropylmethoxy-3-{2-[1-(2-propynyl)piperidin-4-yl]ethyl}benz[d]isoxazol-7-yl}methylamine dihydrochloride

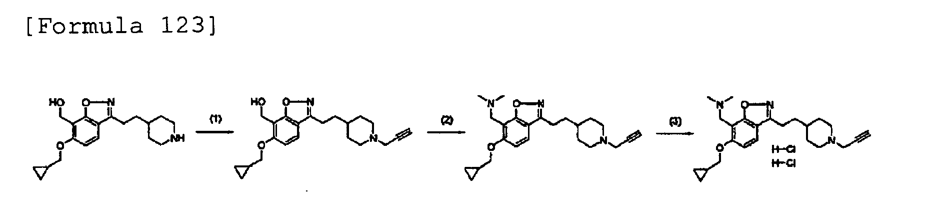

### (1) {6-Cyclopropylmethoxy-3-{2-[1-(2-propynyl)piperidin-4-yl]ethyl}benz[d]isoxazol-7-yl}methanol

The title compound (81 mg) was obtained by synthesis from {6-cyclopropylmethoxy-3-[2-(piperidin-4-yl)ethyl]benz[d]isoxazol-7-yl}methanol (compound obtained in Example 98-(2)) (100 mg) and propargyl bromide (35 µL) according to Example 98-(3).
¹H-NMR (400 MHz, CDCl₃) δ(ppm): 0.36-0.41 (m, 2H), 0.65-0.71 (m, 2H), 1.26-1.38 (m, 4H), 1.76-1.84 (m, 4H), 2.18 (br t, J=11.2 Hz, 2H), 2.22 (t, J=2.8 Hz, 1H), 2.60-2.67 (m, 1H), 2.89 (br d, J=11.2 Hz, 2H), 2.95 (t, J=8.0 Hz, 2H), 3.28 (d, J=2.4 Hz, 2H), 3.97 (d, J=7.2 Hz, 2H), 5.02-5.06 (m, 2H), 6.90 (d, J=8.8 Hz, 1H), 7.46 (d, J=8.8 Hz, 1H) .

### (2) N,N-Dimethyl{6-cyclopropylmethoxy-3-{2-[1-(2-propynyl)piperidin-4-yl]ethyl}benz[d]isoxazol-7-yl}methylamine

The title compound (43 mg) was obtained by synthesis from {6-cyclopropylmethoxy-3-{2-[1-(2-propynyl)piperidin-4-yl]ethyl}benz[d]isoxazol-7-yl}methanol (79 mg) according to Example 79-(6).
¹H-NMR (400 MHz, CDCl₃) δ(ppm): 0.35-0.40 (m, 2H), 0.62-0.67 (m, 2H), 1.26-1.39 (m, 4H), 1.74-1.84 (m, 4H), 2.16-2.23 (m, 2H), 2.23 (t, J=2.4 Hz, 1H), 2.33 (s, 6H), 2.89 (br d, J=11.6 Hz, 2H), 2.94 (t, J=8.0 Hz, 2H), 3.28 (d, J=2.4 Hz, 2H), 3. 82 (s, 2H), 3.94 (d, J=6.8 Hz, 2H), 6.89 (d, J=8.8 Hz, 1H) , 7.44 (d, J=8.8 Hz, 1H) .

### (3) N,N-Dimethyl{6-cyclopropylmethoxy-3-{2-[1-(2-propynyl)piperidin-4-yl]ethyl}benz[d]isoxazol-7-yl}methylamine dihydrochloride

The title compound (34 mg) was obtained by synthesis from N,N-dimethyl{6-cyclopropylmethoxy-3-{2-[1-(2-propynyl)piperidin-4-yl]ethyl}benz[d]isoxazol-7-yl}methylamine (27 mg) according to Example 79-(8).
¹H-NMR (400 MHz, CD₃OD) δ (ppm) : 0.42-0.47 (m, 2H), 0.67-0.74 (m, 2H), 1.35-1.46 (m, 1H), 1.48-1.61 (m, 2H), 1.67-1.80 (m, 1H), 1.87 (dt, J=7.2 Hz, 8.0 Hz, 2H), 2.11-2.19 (m, 2H), 2.96 (s, 6H), 3.04-3.13 (m, 4H), 3.40 (t, J=2.4 Hz, 1H), 3.69 (br d, J=12.0 Hz, 2H), 4.08 (d, J=2.4 Hz, 2H), 4.12 (d, J=7.2 Hz, 2H), 4.64 (s, 2H), 7.24 (d, J=8.8 Hz, 1H), 7.92 (d, J=8.8 Hz, 1H) .
ESI-MS m/z: 199 [M+2H]²⁺, 396 [M+H]⁺,

### Example 100

### N,N-Dimethyl{6-cyclopropylmethoxy-3-{2-[1-(3-methyl-2-butenyl)piperidin-4-yl]ethyl}benz[d]isoxazol-7-yl}methylamine dihydrochloride

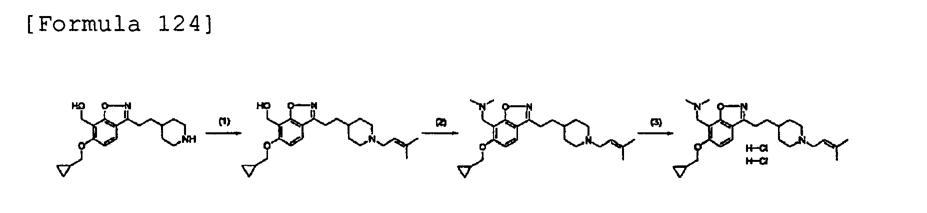

### (1) {6-Cyclopropylmethoxy-3-{2-[1-(3-methyl-2-butenyl)piperidin-4-yl]ethyl}benz[d]isoxazol-7-yl}methanol

The title compound (58 mg) was obtained by synthesis from {6-cyclopropylmethoxy-3-[2-(piperidin-4-yl)ethyl]benz[d]isoxazol-7-yl}methanol (compound obtained in Example 98-(2)) (100 mg) and 1-bromo-3-methyl-2-butene (45 µL) according to Example 98-(3).
¹H-NMR (400 MHz, CDCl₃) δ(ppm): 0.36-0.41 (m, 2H), 0.65-0.71 (m, 2H), 1.25-1.37 (m, 4H), 1.61 (s, 3H), 1.63 (s, 3H), 1.71-1.80 (m, 4H), 1.88 (br t, J=11.2 Hz, 2H), 2,60-2.71 (m, 1H), 2.88-2.97 (m, 6H), 3.97 (d, J=2.8 Hz, 2H), 5.03 (s, 2H), 5.22-5.28 (m, 1H) , 6.90 (d, J=8.8 Hz, 1H) , 7.46 (d, J=8.8 Hz, 1H) .

### (2) N,N-Dimethyl{6-cyclopropylmethoxy-3-{2-[1-(3-methyl-2-butenyl)piperidin-4-yl]ethyl}benz[d]isoxazol-7-yl}methylamine

The title compound (38 mg) was obtained by synthesis from {6-cyclopropylmethoxy-3-{2-[1-(3-methyl-2-butenyl)piperidin-4-yl]ethyl}benz[d]isoxazol-7-yl}methanol (56 mg) according to Example 79-(6).
¹H-NMR (400 MHz, CDCl₃) δ(ppm): 0.35-0.40 (m, 2H), 0.62-0.67 (m, 2H), 1.25-1.37 (m, 4H), 1.63 (s, 3H), 1.73 (s, 3H), 1.73-1.80 (m, 4H), 1.89 (br t, J=11.6 Hz, 2H), 2.33 (s, 6H), 2.88-2.97 (m, 6H), 3.82 (s, 2H), 3.94 (d, J=7.2 Hz, 2H), 5.23-5.29 (m, 1H) , 6.89 (d, J=8.8 Hz, 1H) , 7.43 (d, J=8.8 Hz, 1H) .

### (3) N,N-Dimethyl{6-cyclopropylmethoxy-3-{2-[1-(3-methyl-2-butenyl)piperidin-4-yl]ethyl}benz[d]isoxazol-7-yl}methylamine dihydrochloride

The title compound (39 mg) was obtained by synthesis from N,N-dimethyl{6-cyclopropylmethoxy-3-{2-[1-(3-methyl-2-butenyl)piperidin-4-yl]ethyl}benz[d]isoxazol-7-yl}methylamine (36 mg) according to Example 79-(8).
¹H-NMR (400 MHz, CD₃OD) δ(ppm): 0.42-0.47 (m, 2H), 0.67-0.73 (m, 2H), 1.34-1.44 (m, 1H), 1.45-1.59 (m, 2H), 1.66-1.79 (m, 1H), 1.80 (s, 3H), 1.80-1.89 (m, 2H), 1.87 (s, 3H), 2.06-2.14 (m, 2H), 2.90-2.98 (m, 2H), 2.96 (s, 6H), 3.07 (t, J=8.0 Hz, 2H), 3.54 (br d, J=12.8 Hz, 2H), 3.71 (d, J=8.0 Hz, 2H), 4.12 (d, J=6.8 Hz, 2H), 4.63 (s, 2H), 5.31-5.37 (m, 1H), 7.24 (d, J=8.8 Hz, 1H), 7.92 (d, J=8.8 Hz, 1H).
ESI-MS m/z: 214 [M+2H]²⁺, 426 [M+H]⁺.

### Example 101

### 3-{3-[2-(1-Benzylpiperidin-4-yl)ethyl]-7-(N,N-dimethylaminomethyl)benz[d]isoxazol-6-yloxymethyl}benzonitrile dihydrochloride

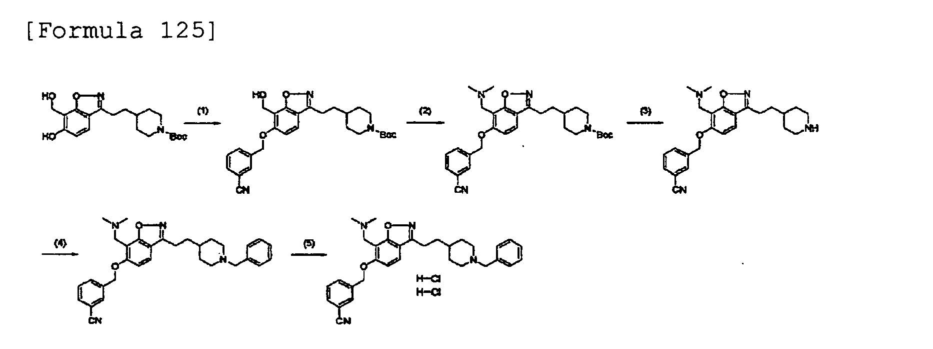

### (1) tert-Butyl 4-{2-[6-(3-cyanobenzyloxy)-7-hydroxymethylbenz[d]isoxazol-3-yl]ethyl}piperidine-1-carboxylate

The title compound (260 mg) was obtained by synthesis from tert-butyl 4-[2-(6-hydroxy-7-hydroxymethylbenz[d]isoxazol-3-yl)ethyl]piperidine-1-carboxylate (compound obtained in Preparation Example 2-(6)) (200 mg) and 3-(bromomethyl)benzonitrile (150 mg) according to Example 874- (1) .
¹H-NMR (400 MHz, CDCl₃) δ(ppm): 1.16 (ddd, J=4.0 Hz, 12.0 Hz, 24.4 Hz, 2H), 1.44-1.53 (m, 1H), 1.45 (s, 9H), 1.71-1.79 (m, 4H), 2.25 (t, J=6.4 Hz, 1H), 2.61-2.74 (m, 2H), 2.97 (t, J=8.0 Hz, 2H), 4.02-4.16 (m, 2H), 5.06 (d, J=6.4 Hz, 2H), 5.25 (s, 2H), 6.94 (d, J=8.8 Hz, 1H), 7.49 (d, J=8.8 Hz, 1H) , 7.53 (dd, J=7.6 Hz, 8.0 Hz, 1H), 7.65 (d, J=8.0 Hz, 1H), 7.70 (d, J=7.6 Hz, 1H), 7.73 (s, 1H).

### (2) tert-Butyl 4-{2-[6-(3-cyanobenzyloxy)-7-(N,N-dimethylaminomethyl)benz[d]isoxazol-3-yl]ethyl}piperidine-1-carboxylate

The title compound (207 mg) was obtained by synthesis from tert-butyl 4-{2-[6-(3-cyanobenzyloxy)-7-hydroxymethylbenz[d]isoxazol-3-yl]ethyl}piperidine-1-carboxylate (258 mg) according to Example 79-(6).
¹H-NMR (400 MHz, CDCl₃) δ (ppm) : 1.16 (ddd, J=4.0 Hz, 12.0 Hz, 24.0 Hz, 2H), 1.45 (s, 9H), 1.48-1.55 (m, 1H), 1.71-1.82 (m, 4H), 2.33 (s, 6H), 2.62-2.74 (m, 2H), 2.96 (t, J=7.6 Hz, 2H), 3.81 (s, 2H), 4.03-4.16 (m, 2H), 5.23 (s, 2H), 6.95 (d, J=8.4 Hz, 1H), 7.47 (d, J=8.4 Hz, 1H), 7.51 (dd, J=7.6 Hz, 7.6 Hz, 1H), 7.63 (d, J=7.6 Hz, 1H), 7.69 (d, J=7.6 Hz, 1H), 7.81 (s, 1H) .

### (3) 3-{7-(N,N-Dimethylaminomethyl)-3-[2-(piperidin-4-yl)ethyl]benz[d]isoxazol-6-yloxymethyl}benzonitrile

The title compound (139 mg) was obtained by synthesis from tert-butyl 4-{2-[6-(3-cyanobenzyloxy)-7-(N,N-dimethylaminomethyl)benz[d]isoxazol-3-yl]ethyl}piperidine-1-carboxylate (204 mg) according to Example 96-(4).
¹H-NMR (400 MHz, CDCl₃) δ(ppm): 1.17 (ddd, J=4.0 Hz, 12.0 Hz, 24.0 Hz, 2H), 1.42-1.54 (m, 1H), 1.72-1.81 (m, 4H), 2.33 (s, 6H), 2.58 (dt, J=2.8 Hz, 12.0 Hz, 2H), 2.95 (t, J=8.0 Hz, 2H), 3.07 (br d, J=12.0 Hz, 2H), 3.81 (s, 2H), 5.23 (s, 2H), 6.94 (d, J=8.8 Hz, 1H), 7.48 (d, J=8.8 Hz, 1H), 7.51 (dd, J=8.0 Hz, 8.0 Hz, 1H) , 7.63 (d, J=8.0 Hz, 1H) , 7.70 (d, J=8.0 Hz, 1H) , 7.82 (s, 1H) .

### (4) 3-{3-[2-(1-Benzylpiperidin-4-yl)ethyl]-7-(N,N-dimethylaminomethyl)benz[d]isoxazol-6-yloxymethyl}benzonitrile

The title compound (36 mg) was obtained by synthesis from 3-{7-(N,N-dimethylaminomethyl)-3-[2-(piperidin-4-yl)ethyl]benz[d]isoxazol-6-yloxymethyl}benzonitrile (68 mg) and benzaldehyde (50 mg) according to Example 89-(1).
¹H-NMR (400 MHz, CDCl₃) δ(ppm): 1.25-1.38 (m, 3H), 1.71-1.80 (m, 4H), 1.94 (br t, J=11.6 Hz, 2H), 2.33 (s, 6H), 2.88 (br d, J=11.6 Hz, 2H), 2.94 (t, J=8.0 Hz, 2H), 3.48 (s, 2H), 3.80 (s, 2H), 5.23 (s, 2H), 6.93 (d, J=8.8 Hz, 1H), 7.21-7.31 (m, 5H), 7.46 (d, J=8.8 Hz, 1H) , 7.50 (dd, J=7.6 Hz, 8.0 Hz, 1H), 7.62 (d, J=8.0 Hz, 1H), 7.69 (d, J=7.6 Hz, 1H), 7.81 (s, 1H).

### (5) 3-{3-[2-(1-Benzylpiperidin-4-yl)ethyl]-7-(N,N-dimethylaminomethyl)benz[d]isoxazol-6-yloxymethyl}benzonitrile dihydrochloride

The title compound (38 mg) was obtained by synthesis from 3-{3-[2-(1-benzylpiperidin-4-yl)ethyl]-7-(N,N-dimethylaminomethyl)benz[d]isoxazol-6-yloxymethyl}benzonitrile (36 mg) according to Example 79-(8).
¹H-NMR (400 MHz, CD₃OD) δ (ppm) : 1.46-1.58 (m, 2H), 1.65-1.76 (m, 1H), 1.78-1.86 (m, 2H), 2.01-2.11 (m, 2H), 2.92 (s, 6H), 2.94-3.08 (m, 4H), 3.44-3.53 (m, 2H), 4.30 (s, 2H), 4.66 (s, 2H), 5.45 (s, 2H), 7.32 (d, J=8.8 Hz, 1H) , 7.45-7.55 (m, 5H), 7.63 (dd, J=7.6 Hz, 8.0 Hz, 1H), 7.75 (d, J=7.6 Hz, 1H), 7.87 (d, J=8.0 Hz, 1H), 7.92-7.96 (m, 2H).
ESI-MS m/z: 255 [M+2H]²⁺, 509 [M+H]⁺.

### Example 102

### 2-{3-[2-(1-Benzylpiperidin-4-yl)ethyl]-7-(N,N-dimethylaminomethyl)benz[d]isoxazol-6-yloxymethyl}benzonitrile dihydrochloride

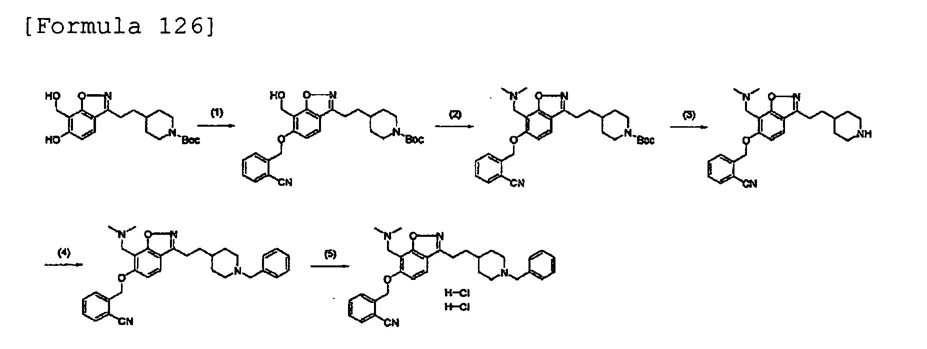

### (1) tert-Butyl 4-{2-[6-(2-cyanobenzyloxy)-7-hydroxymethylbenz[d]isoxazol-3-yl]ethyl}piperidine-1-carboxylate

The title compound (246 mg) was obtained by synthesis from tert-butyl 4-[2-(6-hydroxy-7-hydroxymethylbenz[d]isoxazol-3-yl)ethyl]piperidine-1-carboxylate (compound obtained in Preparation Example 2-(6)) (200 mg) and 2-(bromomethyl)benzonitrile (150 mg) according to Example 874-(1).
¹H-NMR (400 MHz, CDCl₃) δ (ppm) : 1.10-1.23 (m, 2H), 1.45 (s, 9H), 1.46-1.55 (m, 1H), 1.71-1.82 (m, 4H), 2.37 (t, J=6.4 Hz, 1H), 2.61-2.74 (m, 2H), 2.98 (t, J=8.0 Hz, 2H), 4.03-4.17 (m, 2H), 5.06 (d, J=6.4 Hz, 2H), 5.40 (s, 2H), 7.03 (d, J=8.8 Hz, 1H), 7.45-7.51 (m, 1H), 7.52 (d, J=8.8 Hz, 1H), 7.63-7.71 (m, 2H), 7.74 (d, J=8.4 Hz, 1H).

### (2) tert-Butyl 4-{2-[6-(2-cyanobenzyloxy)-7-(N,N-dimethylaminomethyl)benz[d]isoxazol-3-yl]ethyl}piperidine-1-carboxylate

The title compound (203 mg) was obtained by synthesis from tert-butyl 4-{2-[6-(2-cyanobenzyloxy)-7-hydroxymethylbenz[d]isoxazol-3-yl]ethyl}piperidine-1-carboxylate (244 mg) according to Example 79-(6).
¹H-NMR (400 MHz, CDCl₃) δ(ppm): 1.11-1.23 (m, 2H), 1.46 (s, 9H), 1.48-1.56 (m, 1H), 1.71-1.83 (m, 4H), 2.32 (s, 6H), 2.62-2.75 (m, 2H), 2.97 (t, J=8.0 Hz, 2H), 3.84 (s, 2H), 4.02-4.16 (m, 2H), 5.40 (s, 2H), 7.04 (d, J=8.4 Hz, 1H), 7.45 (dd, J=7.6 Hz, 7.6 Hz, 1H), 7.50 (d, J=8.4 Hz, 1H), 7.65 (ddd, J=0.8 Hz, 7.6 Hz, 7.6 Hz, 1H), 7.72 (dd, J=0.8 Hz, 7.6 Hz, 1H), 7.77 (d, J=7.6 Hz, 1H) .

### (3) 2-{7-(N,N-Dimethylaminomethyl)-3-[2-(piperidin-4-yl)ethyl]benz[d]isoxazol-6-yloxymethyl}benzonitrile

The title compound (126 mg) was obtained by synthesis from tert-butyl 4-{2-[6-(2-cyanobenzyloxy)-7-(N,N-dimethylaminomethyl)benz[d]isoxazol-3-yl]ethyl}piperidine-1-carboxylate (200 mg) according to Example 96-(4).
¹H-NMR (400 MHz, CDCl₃) δ(ppm): 1.17 (ddd, J=4.0 Hz, 12.0 Hz, 24.4 Hz, 2H), 1.43-1.53 (m, 1H), 1.74-1.81 (m, 4H), 2.32 (s, 6H), 2.59 (dt, J=2.4 Hz, 12.0 Hz, 2H), 2.96 (t, J=7.6 Hz, 2H), 3.07 (br d, J=12.0 Hz, 2H), 3.84 (s, 2H), 5.40 (s, 2H), 7.04 (d, J=8.8 Hz, 1H) , 7.45 (ddd, J=0.8 Hz, 8.0 Hz, 8.0 Hz, 1H), 7.50 (d, J=8.8 Hz, 1H), 7.65 (dd, J=1.2 Hz, 8.0 Hz, 1H), 7.71 (dd, J=1.2 Hz, 8.0 Hz, 1H), 7.77 (dd, J=0.8 Hz, 8.0 Hz, 1H) .

### (4) 2-{3-[2-(1-Benzylpiperidin-4-yl)ethyl]-7-(N,N-dimethylaminomethyl)benz[d]isoxazol-6-yloxymethyl}benzonitrile

The title compound (44 mg) was obtained by synthesis from 2-{7-(N,N-dimethylaminomethyl)-3-[2-(piperidin-4-yl)ethyl]benz[d]isoxazol-6-yloxymethyl}benzonitrile (62 mg) and benzaldehyde (50 mg) according to Example 89-(1).
¹H-NMR (400 MHz, CDCl₃) δ(ppm): 1.25-1.39 (m, 3H), 1.71-1.81 (m, 4H), 1.94 (br t, J=11.2 Hz, 2H), 2.32 (s, 6H), 2.88 (br d, J=11.2 Hz, 2H), 2.95 (t, J=8.0 Hz, 2H), 3.48 (s, 2H), 3.83 (s, 2H), 5.39 (s, 2H), 7.03 (d, J=8.8 Hz, 1H), 7.20-7.32 (m, 5H), 7.45 (dd, J=7.6 Hz, 7.6 Hz, 1H) , 7.49 (d, J=8.8 Hz, 1H), 7.65 (ddd, J=1.2 Hz, 7.6 Hz, 7.6 Hz, 1H) , 7.71 (dd, J=1.2 Hz, 7.6 Hz, 1H), 7.77 (d, J=7.6 Hz, 1H).

### (5) 2-{3-[2-(1-Benzylpiperidin-4-yl)ethyl]-7-(N,N-dimethylaminomethyl)benz[d]isoxazol-6-yloxymethyl}benzonitrile dihydrochloride

The title compound (48 mg) was obtained by synthesis from 2-{3-[2-(1-benzylpiperidin-4-yl)ethyl]-7-(N,N-dimethylaminomethyl)benz[d]isoxazol-6-yloxymethyl}benzonitrile (44 mg) according to Example 79-(8).
¹H-NMR (400 MHz, CD₃OD) δ(ppm): 1.44-1.59 (m, 2H), 1.64-1.76 (m, 1H), 1.84 (dt, J=7.6 Hz, 8.4 Hz, 2H), 2.04-2.12 (m, 2H), 2.91 (s, 6H), 2.94-3.07 (m, 2H), 3.06 (t, J=7.6 Hz, 2H), 3.50 (br d, J=12.8 Hz, 2H), 4.30 (s, 2H), 4.66 (s, 2H), 5.56 (s, 2H), 7.42 (d, J=8.8 Hz, 1H), 7.47-7.55 (m, 5H), 7.59 (dd, J=7.6 Hz, 7.6 Hz, 1H), 7.76 (dd, J=7.6 Hz, 7.6 Hz, 1H), 7.80 (d, J=7.6 Hz, 1H), 7 . 87 (d, J=7.6 Hz, 1H) , 7.97 (d, J=8.8 Hz, 1H) .
ESI-MS m/z: 255 [M+2H]²⁺, 509 [M+H]⁺.

### Example 103

### N,N-Dimethyl{3-[2-(1-benzylpiperidin-4-yl)ethyl]-6-(pyridin-2-ylmethoxy)benz[d]isoxazol-7-yl}methylamine trihydrochloride

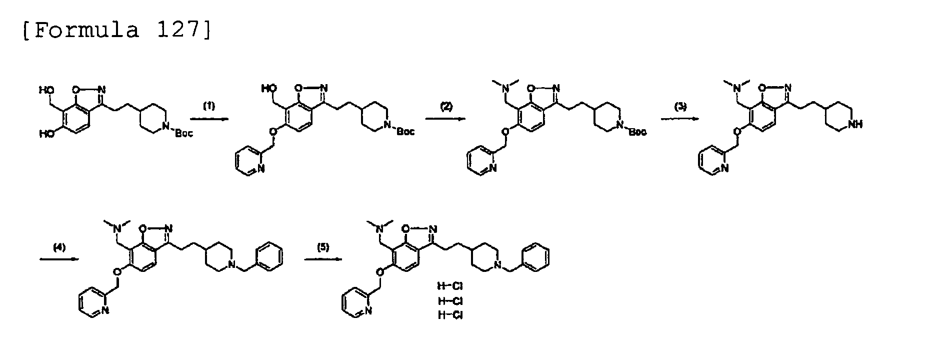

### (1) tert-Butyl 4-{2-[7-hydroxymethyl-6-(pyridin-2-ylmethoxy)benz[d]isoxazol-3-yl]ethyl}piperidine-1-carboxylate

2-Picolyl chloride hydrochloride (170 mg) was neutralized with a 5 M sodium hydroxide solution. Diethyl ether (0.5 mL) was added and the organic layer was separated. The organic layer was added to a solution of tert-butyl 4-[2-(6-hydroxy-7-hydroxymethylbenz[d]isoxazol-3-yl)ethyl]piperidine-1-carboxylate (compound obtained in Preparation Example 2-(6)) (296 mg) and potassium carbonate (270 mg) in N,N-dimethylformamide (5 mL), and the mixture was stirred at 60°C for nine hours. The reaction mixture was cooled to room temperature and then water was added, followed by extraction with ethyl acetate. The organic layer was sequentially washed with water (twice) and a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (263 mg).
¹H-NMR (400 MHz, CDCl₃) δ(ppm): 1.15 (ddd, J=4.0 Hz, 12.4 Hz, 24.4 Hz, 2H), 1.45 (s, 9H), 1.46-1.53 (m, 1H), 1.69-1.80 (m, 4H), 2.61-2.73 (m, 2H), 2.96 (t, J=8.0 Hz, 2H), 4.02-4.16 (m, 2H), 4.15 (t, J=6.0 Hz, 1H), 5.08 (d, J=6.0 Hz, 2H), 5.36 (s, 2H), 7.01 (d, J=8.8 Hz, 1H), 7.24-7.29 (m, 1H), 7.43 (d, J=8.0 Hz, 1H), 7.47 (d, J=8.8 Hz, 1H), 7.73 (ddd, J=2.0 Hz, 8.0 Hz, 8.0 Hz, 1H), 8.61 (d, J=4.8 Hz, 1H).

### (2) tert-Butyl 4-{2-[7-(N,N-dimethylaminomethyl)-6-(pyridin-2-ylmethoxy)benz[d]isoxazol-3-yl]ethyl}piperidine-1-carboxylate

The title compound (251 mg) was obtained by synthesis from tert-butyl 4-{2-[7-hydroxymethyl-6-(pyridin-2-ylmethoxy)benz[d]isoxazol-3-yl]ethyl}piperidine-1-carboxylate (261 mg) according to Example 79-(6).
¹H-NMR (400 MHz, CDCl₃) δ (ppm) : 1.15 (ddd, J=4.0 Hz, 12.4 Hz, 24.4 Hz, 2H), 1.45 (s, 9H), 1.46-1.56 (m, 1H), 1.70-1.81 (m, 4H), 2.36 (s, 6H), 2.61-2.73 (m, 2H), 2.95 (t, J=8.0 Hz, 2H), 3.88 (s, 2H), 4.01-4.15 (m, 2H), 5.34 (s, 2H), 6.99 (d, J=8.8 Hz, 1H), 7.22-7.26 (m, 1H), 7.45 (d, J=8.8 Hz, 1H), 7.58 (d, J=8.0 Hz, 1H), 7.73 (ddd, J=2.0 Hz, 8.0 Hz, 8.0 Hz, 1H), 8.59 (d, J=4.8 Hz, 1H).

### (3) N,N-Dimethyl{3-[2-(piperidin-4-yl)ethyl]-6-(pyridin-2-ylmethoxy)benz[d]isoxazol-7-yl}methylamine

The title compound (107 mg) was obtained by synthesis from tert-butyl 4-{2-[7-(N,N-dimethylaminomethyl)-6-(pyridin-2-ylmethoxy)benz[d]isoxazol-3-yl]ethyl}piperidine-1-carboxylate (191 mg) according to Example 96-(4).
¹H-NMR (400 MHz, CDCl₃) δ (ppm) : 1.01-1.23 (m, 2H), 1.41-1.53 (m, 1H), 1.72-1.81 (m, 4H), 2.37 (s, 6H), 2.54-2.61 (m, 2H), 2.91-2.98 (m, 2H), 3.03-3.09 (m, 2H), 3.85 (s, 2H), 5.35 (s, 2H), 6.99 (d, J=8.8 Hz, 1H), 7.22-7.26 (m, 1H), 7.46 (d, J=8.8 Hz, 1H), 7.59 (d, J=7.6 Hz, 1H), 7.73 (ddd, J=2.0 Hz, 7.6 Hz, 7.6 Hz, 1H), 8.59 (d, J=4.8 Hz, 1H).

### (4) N,N-Dimethyl{3-[2-(1-benzylpiperidin-4-yl)ethyl]-6-(pyridin-2-ylmethoxy)benz[d]isoxazol-7-yl}methylamine

The title compound (38 mg) was obtained by synthesis from N,N-dimethyl{3-[2-(piperidin-4-yl)ethyl]-6-(pyridin-2-ylmethoxy)benz[d]isoxazol-7-yl}methylamine (52 mg) and benzaldehyde (40 mg) according to Example 89-(1).
¹H-NMR (400 MHz, CDCl₃) δ (ppm) : 1.24-1.36 (m, 3H), 1.70-1.79 (m, 4H), 1.93 (br t, J=11.6 Hz, 2H), 2.35 (s, 6H), 2.87 (br d, J=11.6 Hz, 2H), 2.93 (t, J=7.6 Hz, 2H), 3.48 (s, 2H), 3.87 (s, 2H), 5.33 (s, 2H), 6.98 (d, J=8.8 Hz, 1H), 7.20-7.32 (m, 6H), 7.45 (d, J=8.8 Hz, 1H), 7.58 (d, J=8.0 Hz, 1H), 7.73 (ddd, J=2.0 Hz, 8.0 Hz, 8.0 Hz, 1H), 8.59 (d, J=4.8 Hz, 1H).

### (5) N,N-Dimethyl{3-[2-(1-benzylpiperidin-4-yl)ethyl]-6-(pyridin-2-ylmethoxy)benz[d]isoxazol-7-yl}methylamine trihydrochloride

The title compound (44 mg) was obtained by synthesis from N,N-dimethyl{3-[2-(1-benzylpiperidin-4-yl)ethyl]-6-(pyridin-2-ylmethoxy)benz[d]isoxazol-7-yl}methylamine (38 mg) according to Example 79-(8).
¹H-NMR (400 MHz, CD₃OD) δ(ppm): 1.46-1.59 (m, 2H), 1.66-1.78 (m, 1H), 1.85 (dd, J=7.2 Hz, 8.0 Hz, 2H), 2.04-2.12 (m, 2H), 2.95 (s, 6H), 2.96-3.08 (m, 2H), 3.07 (t, J=8.0 Hz, 2H), 3.50 (br d, J=12.0 Hz, 2H), 4.30 (s, 2H), 4.75 (s, 2H), 5.78 (s, 2H), 7.37 (d, J=8.8 Hz, 1H), 7.47-7.56 (m, 5H), 8.00 (d, J=8.8 Hz, 1H), 8.06 (dd, J=5.2 Hz, 8.0 Hz, 1H), 8.24 (d, J=8.0 Hz, 1H), 8.63 (dd, J=8.0 Hz, 8.0 Hz, 1H), 8.92 (d, J=5.2 Hz, 1H).
ESI-MS m/z: 243 [M+2H]²⁺, 485 [M+H]⁺.

### Example 104

### 6-{3-[2-(1-Benzylpiperidin-4-yl)ethyl]-7-(N,N-dimethylaminomethyl)benz[d]isoxazol-6-yloxymethyl}pyridine-3-carbonitrile fumarate

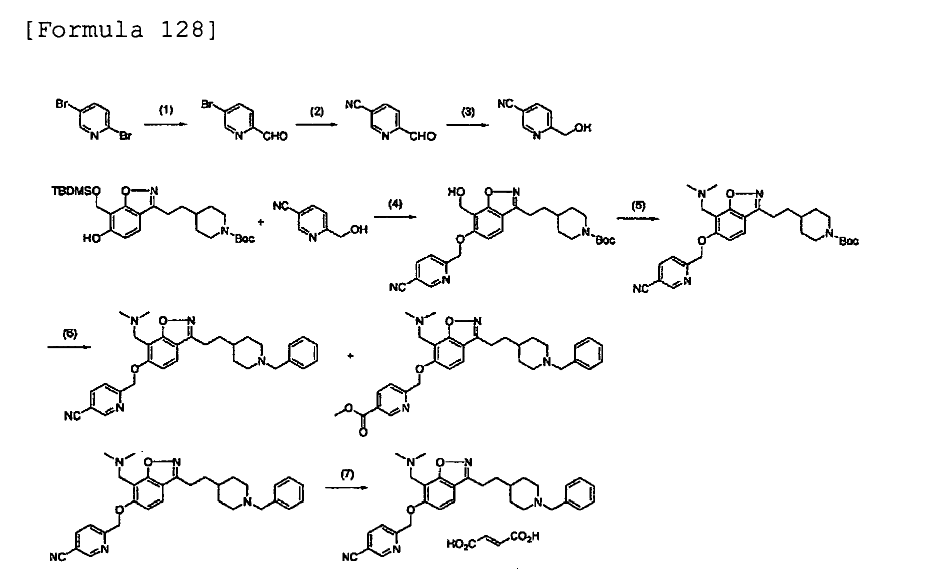

### (1) 5-Bromopyridine-2-carbaldehyde

The title compound was synthesized with reference to Tetrahedron Lett., 2000, 41, 4335. 2,5-Dibromopyridine (10 g) was dissolved in toluene (450 mL) in a nitrogen atmosphere. After cooling the solution to -78°C, n-butyllithium (2.59 M solution in hexane) (19 mL) was added dropwise at -78°C over 13 minutes, and the mixture was stirred at -78°C for 2.5 hours. N,N-Dimethylformamide (10 mL) was added at -78°C and the mixture was stirred at -78°C for 50 minutes. A saturated ammonium chloride solution was added at -78°C and then water was added at room temperature, followed by extraction with ethyl acetate. The organic layer was sequentially washed with water and a saturated sodium chloride solution and dried over magnesium sulfate. The drying agent was removed by filtration and then the solvent was evaporated under reduced pressure. The resulting solid was suspended in a 20:1 mixed solution of heptane and diethyl ether and then collected by filtration to obtain the title compound (4.1 g).
¹H-NMR (400 MHz, CDCl₃) δ (ppm) : 7.85 (d, J=8.4 Hz, 1H), 8.02 (dd, J=2.0 Hz, 8.4 Hz, 1H), 8.84 (d, J=2.0 Hz, 1H), 10.02 (s, 1H).

### (2) 6-Formylpyridine-3-carbonitrile

The title compound (298 mg) was obtained by synthesis from 5-bromopyridine-2-carbaldehyde (2 g) according to Example 91-(4).
¹H-NMR (400 MHz, CDCl₃) δ(ppm) : 8.06 (d, J=8.0 Hz, 1H), 8.16 (dd, J=2.0 Hz, 8.0 Hz, 1H), 9.04 (d, J=2.0 Hz, 1H), 10.11 (s, 1H).

### (3) 6-Hydroxymethylpyridine-3-carbonitrile

The title compound (200 mg) was obtained by synthesis from 6-formylpyridine-2-carbonitrile (296 mg) according to Example 85-(1).
¹H-NMR (400 MHz, CDCl₃) δ(ppm): 3.23 (t, J=5.2 Hz, 1H), 4.85 (d, J=5.2 Hz, 2H), 7.45 (d, J=8.0 Hz, 1H), 7.96 (dd, J=2.0 Hz, 8.0 Hz, 1H), 8.84 (d, J=2.0 Hz, 1H).

### (4) tert-Butyl 4-{2-[6-(5-cyanopyridin-2-ylmethoxy)-7-hydroxymethylbenz[d]isoxazol-3-yl]ethyl}piperidine-1-carboxylate

tert-Butyl 4-{2-[7-(tert-butyldimethylsilanyloxymethyl)-6-hydroxybenz[d]isoxazol-3-yl]ethylpiperidine-1-carboxylate (compound obtained in Example 79-(1)) (300 mg), 6-hydroxymethylpyridine-3-carbonitrile (115 mg) and triphenylphosphine (225 mg) were dissolved in tetrahydrofuran (7 mL) in a nitrogen atmosphere, and then the solution was ice-cooled. A solution of diisopropyl azodicarboxylate (175 mg) in tetrahydrofuran (1 mL) was added dropwise to the solution under ice-cooling over 20 minutes, and the mixture was stirred at room temperature for 20.5 hours. Water was added to the reaction mixture, followed by extraction with ethyl acetate. The organic layer was sequentially washed with water and a saturated sodium chloride solution and dried over magnesium sulfate. The drying agent was removed by filtration and then the solvent was evaporated under reduced pressure. The residue was purified by NH silica gel column chromatography (heptane-ethyl acetate) and silica gel column chromatography (heptane-ethyl acetate) to obtain tert-butyl 4-{2-[7-(tert-butyldimethylsilanyloxymethyl)-6-(5-cyanopyridin-2-ylmethoxy)benz[d]isoxazol-3-yl]ethyl}piperidine-1-carboxylate (210 mg). The compound (208 mg) was dissolved in tetrahydrofuran (3 mL).
Tetrabutylammonium fluoride (1 M solution in tetrahydrofuran) (0.5 mL) was added to the solution, and the mixture was stirred at room temperature for two hours. Water was added to the reaction mixture, followed by extraction with ethyl acetate. The organic layer was sequentially washed with water (twice) and a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (112 mg).
¹H-NMR (400 MHz, CDCl₃) δ (ppm) : 1.15 (ddd, J=3.6 Hz, 12.0 Hz, 24.0 Hz, 2H), 1.44-1.53 (m, 1H), 1.45 (s, 9H), 1.70-1.81 (m, 4H), 2.61-2.74 (m, 2H), 2.88 (t, J=6.8 Hz, 1H), 2.97 (t, J=8.0 Hz, 2H), 4.00-4.17 (m, 2H), 5.10 (d, J=6.8 Hz, 2H), 5.42 (s, 2H), 6.94 (d, J=8.8 Hz, 1H), 7.49 (d, J=8.8 Hz, 1H), 7.67 (dd, J=0.8 Hz, 8.4 Hz, 1H), 8.01 (dd, J=2.0 Hz, 8.4 Hz, 1H), 8.88 (dd, J=0.8 Hz, 2.0 Hz, 1H).

### (5) tert-Butyl 4-{2-[6-(5-cyanopyridin-2-ylmethoxy)-7-(N,N-dimethylaminomethyl)benz[d]isoxazol-3-yl]ethyl}piperidine-1-carboxylate

The title compound (76 mg) was obtained by synthesis from tert-butyl 4-{2-[6-(5-cyanopyridin-2-ylmethoxy)-7-hydroxymethylbenz[d]isoxazol-3-yl]ethyl}piperidine-1-carboxylate (110 mg) according to Example 79-(6).
¹H-NMR (400 MHz, CDCl₃) δ (ppm) : 1. 16 (ddd, J=4.0 Hz, 12.4 Hz, 24.4 Hz, 2H), 1.45 (s, 9H), 1.46-1.55 (m, 1H), 1.71-1.82 (m, 4H), 2.35 (s, 6H), 2.62-2.74 (m, 2H), 2.96 (t, J=8.0 Hz, 2H), 3.86 (s, 2H), 4.02-4.17 (m, 2H), 5.38 (s, 2H), 6.94 (d, J=8.8 Hz, 1H), 7.47 (d, J=8.8 Hz, 1H), 7.81 (d, J=8.4 Hz, 1H), 8.02 (dd, J=2.0 Hz, 8.4 Hz, 1H), 8.86 (d, J=2.0 Hz, 1H).

### (6) 6-{3-[2-(1-Benzylpiperidin-4-yl)ethyl]-7-(N,N-dimethylaminomethyl)benz[d]isoxazol-6-yloxymethyl}pyridine-3-carbonitrile and methyl 6-{3-[2-(1-benzylpiperidin-4-yl)ethyl]-7-(N,N-dimethylaminomethyl)benz[d]isoxazol-6-yloxymethyl}pyridine-3-carboxylate

The title compounds, 6-{3-[2-(1-benzylpiperidin-4-yl)ethyl]-7-(N,N-dimethylaminomethyl)benz[d]isoxazol-6-yloxymethyl}pyridine-3-carbonitrile (28 mg) and methyl 6-{3-[2-(1-benzylpiperidin-4-yl)ethyl]-7-(N,N-dimethylaminomethyl)benz[d]isoxazol-6-yloxymethyl}pyridine-3-carboxylate (7 mg), were obtained from tert-butyl 4-{2-[6-(5-cyanopyridin-2-ylmethoxy)-7-(N,N-dimethylaminomethyl)benz[d]isoxazol-3-yl]ethyl}piperidine-1-carboxylate (74 mg) and benzaldehyde (40 mg) according to Example 79-(7), respectively.

### 6-{3-[2-(1-Benzylpiperidin-4-yl)ethyl]-7-(N,N-dimethylaminomethyl)benz[d]isoxazol-6-yloxymethyl}pyridine-3-carbonitrile

¹H-NMR (400 MHz, CDCl₃) δ (ppm) : 1.25-1.38 (m, 3H), 1.70-1.81 (m, 4H), 1.91-1.98 (m, 2H), 2.35 (s, 6H), 2.89 (br d, J=12.0 Hz, 2H), 2.94 (t, J=8.0 Hz, 2H), 3.49 (s, 2H), 3.86 (s, 2H), 5.39 (s, 2H), 6.94 (d, J=8.8 Hz, 1H), 7.21-7.34 (m, 5H), 7.48 (d, J=8.8 Hz, 1H), 7.82 (d, J=8.0 Hz, 1H), 8.03 (dd, J=2.0 Hz, 8.0 Hz, 1H), 8.88 (d, J=2.0 Hz, 1H) .

### Methyl 6-{3-[2-(1-benzylpiperidin-4-yl)ethyl]-7-(N,N-dimethylaminomethyl)benz[d]isoxazol-6-yloxymethyl}pyridine-3-carboxylate

¹H-NMR (400 MHz, CDCl₃) δ(ppm): 1.25-1.38 (m, 3H), 1.71-1.81 (m, 4H), 1.94 (br t, J=11.2 Hz, 2H), 2.36 (s, 6H), 2.88 (br d, J=11.2 Hz, 2H), 2.94 (t, J=8.0 Hz, 2H), 3.49 (s, 2H), 3.88 (s, 2H), 3.97 (s, 3H), 5.39 (s, 2H), 6.96 (d, J=8.8 Hz, 1H), 7.12-7.23 (m, 5H), 7.47 (d, J=8.8 Hz, 1H), 7.73 (d, J=8.4 Hz, 1H), 8.35 (dd, J=2.4 Hz, 8.4 Hz, 1H), 9.20 (d, J=2.4 Hz, 1H).

### (7) 6-{3-[2-(1-Benzylpiperidin-4-yl)ethyl]-7-(N,N-dimethylaminomethyl)benz[d]isoxazol-6-yloxymethyl}pyridine-3-carbonitrile fumarate

The title compound (30 mg) was obtained by synthesis from 6-{3-[2-(1-benzylpiperidin-4-yl)ethyl]-7-(N,N-dimethylaminomethyl)benz[d]isoxazol-6-yloxymethyl}pyridine-3-carbonitrile (28 mg) according to Example 81-(5).
¹H-NMR (400 MHz, CD₃OD) δ(ppm): 1.42-1.53 (m, 2H), 1.54-1.65 (m, 1H), 1.81 (dt, J=7.6 Hz, 7.6 Hz, 2H), 1.92-2.00 (m, 2H), 2.66-2.75 (m, 2H), 2.72 (s, 6H), 3.02 (t, J=7.6 Hz, 2H), 3.26-3.36 (m, 2H), 4.07 (s, 2H), 4.39 (s, 2H), 5.53 (s, 2H), 6.65 (s, 2H), 7.23 (d, J=8.8 Hz, 1H), 7.40-7.48 (m, 5H), 7.81 (d, J=8.8 Hz, 1H), 7.81 (d, J=8.4 Hz, 1H), 8.25 (dd, J=2.0 Hz, 8.4 Hz, 1H), 8.95 (d, J=2.0 Hz, 1H).
ESI-MS m/z: 256 [M+2H]²⁺, 510 [M+H]⁺.

### Example 105

### 6-{3-[2-(1-Benzylpiperidin-4-yl)ethyl]-7-(N,N-dimethylaminomethyl)benz[d]isoxazol-6-yloxymethyl}pyridine-2-carbonitrile difumarate

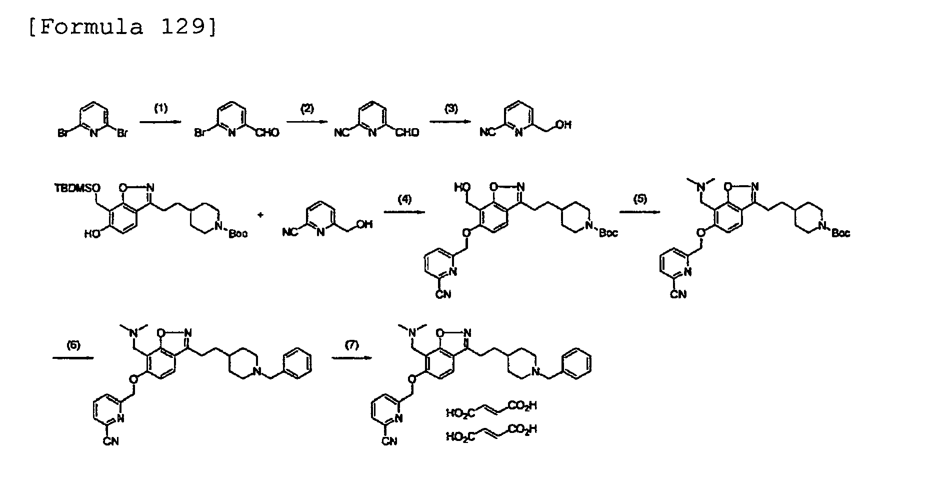

### (1) 6-Bromopyridine-2-carbaldehyde

The title compound was synthesized with reference to Tetrahedron Lett., 1996, 37, 2537. A solution of 2,6-dibromopyridine (10 g) in tetrahydrofuran (50 mL) was added dropwise to a solution of n-butyllithium (2.59 M solution in hexane) (16 mL) in tetrahydrofuran (50 mL) in a nitrogen atmosphere at -78°C over 25 minutes, and the mixture was stirred at -78°C for 20 minutes. N,N-Dimethylformamide (7 mL) was added at -78°C and the mixture was stirred at -78°C for 30 minutes. A saturated ammonium chloride solution was added at -78°C and then water was added at room temperature, followed by extraction with ethyl acetate. The organic layer was sequentially washed with water and a saturated sodium chloride solution and dried over magnesium sulfate. The drying agent was removed by filtration and then the solvent was evaporated under reduced pressure. The resulting solid was suspended in a 20:1 mixed solution of heptane and diethyl ether and then collected by filtration to obtain the title compound (5.4 g).
¹H-NMR (400 MHz, CDCl₃) δ(ppm): 7.70-7.77 (m, 2H), 7.92 (dd, J=1.6 Hz, 6.8 Hz, 1H), 9.99 (s, 1H).

### (2) 6-Formylpyridine-2-carbonitrile

The title compound (1.02 g) was obtained by synthesis from 6-bromopyridine-2-carbaldehyde (3 g) according to Example 91-(4).
¹H-NMR (400 MHz, CDCl₃) δ(ppm) : 7.92 (dd, J=1.2 Hz, 8.0 Hz, 1H), 8.06 (dd, J=8.0 Hz, 8.0 Hz, 1H), 8.15 (dd, J=1.2 Hz, 8.0 Hz, 1H), 10.05 (s, 1H).

### (3) 6-Hydroxymethylpyridine-2-carbonitrile

The title compound (374 mg) was obtained by synthesis from 6-formylpyridine-2-carbonitrile (500 mg) according to Example 85-(1).
¹H-NMR (400 MHz, CDCl₃) δ(ppm): 3.15 (t, J=5.2 Hz, 1H), 4.82 (d, J=5.2 Hz, 2H), 7.54 (d, J=8.0 Hz, 1H), 7.62 (d, J=8.0 Hz, 1H), 7.84 (dd, J=8.0 Hz, 8.0 Hz, 1H).

### (4) tert-Butyl-4-{2-[6-(6-cyanopyridin-2-ylmethoxy)-7-hydroxymethylbenz[d]isoxazol-3-yl]ethyl}piperidine-1-carboxylate

The title compound (231 mg) was obtained by synthesis from tert-butyl 4-{2-[7-(tert-butyldimethylsilanyloxymethyl)-6-hydroxybenz[d]isoxazol-3-yl]ethylpiperidine-1-carboxylate (compound obtained in Example 79-(1)) (300 mg) and 6-hydroxymethylpyridine-2-carbonitrile (115 mg). according to Example 104-(4).
¹H-NMR (400 MHz, CDCl₃) δ(ppm): 1.16 (ddd, J=4.4 Hz, 12.0 Hz, 24.0 Hz, 2H), 1.44-1.55 (m, 1H), 1.45 (s, 9H), 1.71-1.81 (m, 4H), 2.49 (t, J=6.8 Hz, 1H), 2.67 (br t, J=12.0 Hz, 2H), 2.97 (t, J=8.0 Hz, 2H), 4.03-4.17 (m, 2H), 5.10 (d, J=6.8 Hz, 2H), 5.39 (s, 2H), 6.96 (d, J=8.8 Hz, 1H), 7.50 (d, J=8.8 Hz, 1H), 7.67 (dd, J=0.8 Hz, 7.6 Hz, 1H), 7.79 (dd, J=0.8 Hz, 8.0 Hz, 1H), 7.90 (dd, J=7.6 Hz, 8.0 Hz, 1H).

### (5) tert-Butyl 4-{2-[6-(6-cyanopyridin-2-ylmethoxy)-7-(N,N-dimethylaminomethyl)benz[d]isoxazol-3-yl]ethyl}piperidine-1-carboxylate

The title compound (147 mg) was obtained by synthesis from tert-butyl 4-{2-[6-(6-cyanopyridin-2-ylmethoxy)-7-hydroxymethylbenz[d]isoxazol-3-yl]ethyl}piperidine-1-carboxylate (229 mg) according to Example 79-(6).
¹H-NMR (400 MHz, CDCl₃) δ(ppm): 1.16 (ddd, J=4.4 Hz, 12.4 Hz, 24.4 Hz, 2H), 1.45 (s, 9H), 1.46-1.56 (m, 1H), 1.71-1.82 (m, 4H), 2.35 (s, 6H), 2.61-2.73 (m, 2H), 2.96 (t, J=8.0 Hz, 2H), 3.85 (s, 2H), 4.02-4.16 (m, 2H), 5.36 (s, 2H), 6.96 (d, J=8.4 Hz, 1H), 7.48 (d, J=8.4 Hz, 1H), 7.65 (dd, J=4.0 Hz, 5.2 Hz, 1H), 7.89 (d, J=4.0 Hz, 1H), 7.89 (d, J=5.2 Hz, 1H).

### (6) 6-{3-[2-(1-Benzylpiperidin-4-yl)ethyl]-7-(N,N-dimethylaminomethyl)benz[d]isoxazol-6-yloxymethyl}pyridine-2-carbonitrile

The title compound (8 mg) was obtained by synthesis from tert-butyl 4-{2-[6-(6-cyanopyridin-2-ylmethoxy)-7-(N,N-dimethylaminomethyl)benz[d]isoxazol-3-yl]ethyl}piperidine-1-carboxylate (145 mg) and benzaldehyde (40 mg) according to Example 79-(7).
¹H-NMR (400 MHz, CDCl₃) δ(ppm): 1.25-1.38 (m, 3H), 1.70-1.82 (m, 4H), 1.95 (br t, J=11.6 Hz, 2H), 2.35 (s, 6H), 2.89 (br d, J=11.6 Hz, 2H), 2.94 (t, J=8.0 Hz, 2H), 3.49 (s, 2H), 3.86 (s, 2H), 5.36 (s, 2H), 6.96 (d, J=8.8 Hz, 1H), 7.22-7.33 (m, 5H), 7.49 (d, J=8.8 Hz, 1H), 7.66 (dd, J=4.4 Hz, 4.8 Hz, 1H), 7.90 (d, J=4.4 Hz, 1H), 7.90 (d, J=4.8 Hz, 1H).

### (7) 6-{3-[2-(1-Benzylpiperidin-4-yl)ethyl]-7-(N,N-dimethylaminomethyl)benz[d]isoxazol-6-yloxymethyl}pyridine-2-carbonitrile difumarate

The title compound (9 mg) was obtained by synthesis from 6-{3-[2-(1-benzylpiperidin-4-yl)ethyl]-7-(N,N-dimethylaminomethyl)benz[d]isoxazol-6-yloxymethyl}pyridine-2-carbonitrile (8 mg) according to Example 81- (5) .
¹H-NMR (400 MHz, CD₃OD) δ (ppm) : 1.45-1.62 (m, 2H), 1.64-1.76 (m, 1H), 1.84 (dt, J=7.6 Hz, 7.6 Hz, 2H), 2.01-2.08 (m, 2H), 2.91-3.01 (m, 2H), 2.96 (s, 6H), 3.05 (t, J=7.6 Hz, 2H), 3.45 (br d, J=13.6 Hz, 2H), 4.27 (s, 2H), 4.66 (s, 2H), 5.54 (s, 2H), 6.70 (s, 4H), 7.28 (d, J=8.8 Hz, 1H), 7.46-7.52 (m, 5H), 7.88 (d, J=7.6 Hz, 1H), 7.89 (d, J=8.0 Hz, 1H), 7.90 (d, J=8.8 Hz, 1H), 8.08 (dd, J=7.6 Hz, 8.0 Hz, 1H) .
ESI-MS m/z: 256 [M+2H]²⁺, 510 [M+H]⁺.

### Example 106

### 3-{7-(N,N-Dimethylaminomethyl)-3-{2-[1-(1,3-dioxolan-2-ylmethyl)piperidin-4-yl]ethyl}benz[d]isoxazol-6-yloxymethyl}benzonitrile fumarate

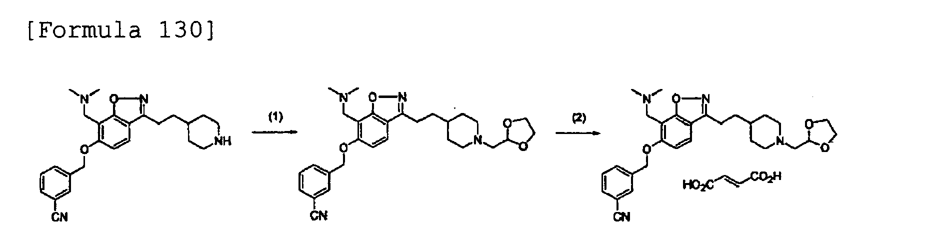

### (1) 3-{7-(N,N-Dimethylaminomethyl)-3-{2-[1-(1,3-dioxolan-2-ylmethyl)piperidin-4-yl]ethyl}benz[d]isoxazol-6-yloxymethyl}benzonitrile

A mixture of 3-{7-(N,N-dimethylaminomethyl)-3-[2-(piperidin-4-yl)ethyl]benz[d]isoxazol-6-yloxymethyl}benzonitrile (compound obtained in Example 101-(3)) (68 mg), 2-bromomethyl-1, 3-dioxolane (50 µL), N,N-diisopropylethylamine (0.18 mL), sodium iodide (30 mg) and acetonitrile (2.5 mL) was stirred at 70°C for 34.5 hours. A saturated sodium bicarbonate solution was added to the reaction mixture, followed by extraction with ethyl acetate. The organic layer was sequentially washed with water and a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (34 mg).
¹H-NMR (400 MHz, CDCl₃) δ(ppm): 1.32-1.43 (m, 3H), 1.71-1.81 (m, 4H), 2.02-2.11 (m, 2H), 2.34 (s, 6H), 2.57 (d, J=4.4 Hz, 2H), 2.95 (t, J=8.0 Hz, 2H), 3.00 (br d, J=11.6 Hz, 2H), 3.81 (s, 2H), 3.83-3.90 (m, 2H), 3.93-4.01 (m, 2H), 5.01 (t, J=4.4 Hz, 1H), 5.24 (s, 2H), 6.95 (d, J=8.8 Hz, 1H), 7.48 (d, J=8.8 Hz, 1H), 7.52 (dd, J=7.6 Hz, 8.0 Hz, 1H), 7.64 (d, J=8.0 Hz, 1H), 7.71 (d, J=7.6 Hz, 1H), 7.83 (s, 1H).

### (2) 3-{7-(N,N-Dimethylaminomethyl)-3-{2-[1-(1,3-dioxolan-2-ylmethyl)piperidin-4-yl]ethyl}benz[d]isoxazol-6-yloxymethyl}benzonitrile fumarate

The title compound (40 mg) was obtained by synthesis from 3-{7-(N,N-dimethylaminomethyl)-3-{2-[1-(1,3-dioxolan-2-ylmethyl)piperidin-4-yl]ethyl}benz[d]isoxazol-6-yloxymethyl}benzonitrile (34 mg) according to Example 81-(5).
¹H-NMR (400 MHz, CD₃OD) δ (ppm) : 1.44-1.62 (m, 3H), 1.82 (dt, J=6.8 Hz, 8.0 Hz, 2H), 1.90-1.98 (m, 2H), 2.67-2.76 (m, 2H), 2.73 (s, 6H), 3.04 (t, J=8.0 Hz, 2H), 3.06 (d, J=4.0 Hz, 2H), 3.43 (br d, J=12.8 Hz, 2H), 3.88-3.94 (m, 2H), 3.97-4.04 (m, 2H), 4.41 (s, 2H), 5.14 (t, J=4.0 Hz, 1H), 5.40 (s, 2H), 6.65 (s, 2H), 7.28 (d, J=8.8 Hz, 1H), 7.62 (dd, J=7.6 Hz, 7.6 Hz, 1H), 7.74 (d, J=7.6 Hz, 1H), 7.85 (d, J=7.6 Hz, 1H), 7.86 (d, J=8.8 Hz, 1H), 7.93 (s, 1H).
ESI-MS m/z: 253 [M+2H]²⁺, 505 [M+H]⁺.

### Example 107

### 2-{7-(N,N-Dimethylaminomethyl)-3-{2-[1-(1,3-dioxolan-2-ylmethyl)piperidin-4-yl]ethyl}benz[d]isoxazol-6-yloxymethyl}benzonitrile fumarate

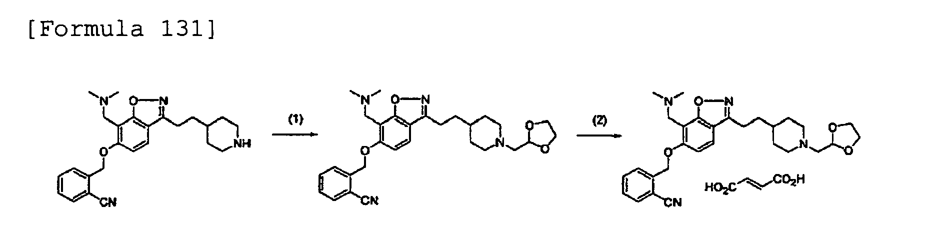

### (1) 2-{7-(N,N-Dimethylaminomethyl)-3-{2-[1-(1,3-dioxolan-2-ylmethyl)piperidin-4-yl]ethyl}benz[d]isoxazol-6-yloxymethyl}benzonitrile

The title compound (26 mg) was obtained by synthesis from 2-{7-(N,N-dimethylaminomethyl)-3-[2-(piperidin-4-yl)ethyl]benz[d]isoxazol-6-yloxymethyl}benzonitrile shown in Example 102-(3) (62 mg) according to Example 106-(1).
¹H-NMR (400 MHz, CDCl₃) δ(ppm): 1.32-1.43 (m, 3H), 1.72-1.82 (m, 4H), 2.02-2.11 (m, 2H), 2.32 (s, 6H), 2.57 (d, J=4.4 Hz, 2H), 2.95 (t, J=8.0 Hz, 2H), 3.01 (br d, J=11.6 Hz, 2H), 3.82-3.90 (m, 2H), 3.85 (s, 2H), 3.93-4.02 (m, 2H), 5.01 (t, J=4.4 Hz, 1H), 5.41 (s, 2H), 7.05 (d, J=8.4 Hz, 1H), 7.46 (dd, J=7.6 Hz, 8.0 Hz, 1H), 7.51 (d, J=8.4 Hz, 1H), 7.66 (dd, J=7.6 Hz, 8.0 Hz, 1H), 7.73 (d, J=8.0 Hz, 1H), 7.78 (d, J=7.6 Hz, 1H).

### (2) 2-{7-(N,N-Dimethylaminomethyl)-3-{2-[1-(1,3-dioxolan-2-ylmethyl)piperidin-4-yl]ethyl}benz[d]isoxazol-6-yloxymethyl}benzonitrile fumarate

The title compound (28 mg) was obtained by synthesis from 2-{7-(N,N-dimethylaminomethyl)-3-{2-[1-(1,3-dioxolan-2-ylmethyl)piperidin-4-yl]ethyl}benz[d]isoxazol-6-yloxymethyl}benzonitrile (26 mg) according to Example 81-(5).
¹H-NMR (400 MHz, CD₃OD) δ (ppm) : 1.44-1.64 (m, 3H), 1.83 (dt, J=7.6 Hz, 7.6 Hz, 2H), 1.90-1.98 (m, 2H), 2.68-2.78 (m, 2H), 2.74 (s, 6H), 3.05 (t, J=7.6 Hz, 2H), 3.07 (d, J=4.0 Hz, 2H), 3.44 (br d, J=12.4 Hz, 2H), 3.88-3.94 (m, 2H), 3.97-4.04 (m, 2H), 4.46 (s, 2H), 5.14 (t, J=4.0 Hz, 1H), 5.52 (s, 2H), 6.56 (s, 2H), 7.38 (d, J=8.8 Hz, 1H), 7.58 (ddd, J=1.2 Hz, 7.6 Hz, 7.6 Hz, 1H), 7.75 (ddd, J=1.2 Hz, 7.6 Hz, 7.6 Hz, 1H), 7.79 (dd, J=0.8 Hz, 7.6 Hz, 1H), 7.85 (dd, J=0.8 Hz, 7.6 Hz, 1H), 7.91 (d, J=8.8 Hz, 1H).
ESI-MS m/z: 253 [M+2H]²⁺, 505 [M+H]⁺.

### Example 108

### N,N-Dimethyl{3-(2-[1-(1,3-dioxolan-2-ylmethyl)piperidin-4-yl]ethyl}-6-(pyridin-2-ylmethoxy)benz[d]isoxazol-7-yl}methylamine fumarate

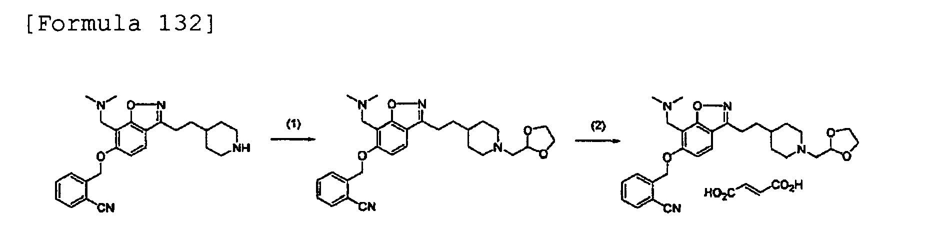

### (1) N,N-Dimethyl{3-{2-[1-(1,3-dioxolan-2-ylmethyl)piperidin-4-yl]ethyl}-6-(pyridin-2-ylmethoxy)benz[d]isoxazol-7-yl}methylamine

The title compound (23 mg) was obtained by synthesis from N,N-dimethyl{3-[2-(piperidin-4-yl)ethyl]-6-(pyridin-2-ylmethoxy)benz[d]isoxazol-7-yl}methylamine (compound obtained in Example 103-(3)) (52 mg) according to Example 106-(1).
¹H-NMR (400 MHz, CDCl₃) δ (ppm) : 1.31-1.41 (m, 3H), 1.70-1.79 (m, 4H), 2.02-2.11 (m, 2H), 2.36 (s, 6H), 2.56 (d, J=4.4 Hz, 2H), 2.94 (t, J=8.0 Hz, 2H), 3.00 (br d, J=11.2 Hz, 2H), 3.83-3.90 (m, 2H), 3.88 (s, 2H), 3.92-3.99 (m, 2H), 5.00 (t, J=4.4 Hz, 1H), 5.35 (s, 2H), 7.00 (d, J=8.8 Hz, 1H), 7.21-7.28 (m, 1H), 7.46 (d, J=8.8 Hz, 1H), 7.60 (d, J=8.0 Hz, 1H), 7.74 (dd, J=8.0 Hz, 8.0 Hz, 1H), 8.60 (d, J=4.8 Hz, 1H).

### (2) N,N-Dimethyl{3-{2-[1-(1,3-dioxolan-2-ylmethyl)piperidin-4-yl]ethyl}-6-(pyridin-2-ylmethoxy)benz[d]isoxazol-7-yl}methylamine fumarate

The title compound (26 mg) was obtained by synthesis from N,N-dimethyl{3-{2-[1-(1,3-dioxolan-2-ylmethyl)piperidin-4-yl]ethyl}-6-(pyridin-2-ylmethoxy)benz[d]isoxazol-7-yl}methylamine (23 mg) according to Example 81-(5).
¹H-NMR (400 MHz, CD₃OD) δ(ppm): 1.42-1.61 (m, 3H), 1.81 (dt, J=7.6 Hz, 7.6 Hz, 2H), 1.91 (br d, J=12.0 Hz, 2H), 2.64 (br t, J=12.4 Hz, 2H), 2.87 (s, 6H), 2.99 (d, J=4.4 Hz, 2H), 3.03 (t, J=7.6 Hz, 2H), 3.38 (br d, J=12.4 Hz, 2H), 3.86-3.93 (m, 2H), 3.95-4.02 (m, 2H), 4.55 (s, 2H), 5.12 (t, J=4. Hz, 1H), 5.50 (s, 2H), 6.66 (s, 2H), 7.29 (d, J=8.8 Hz, 1H), 7.43 (ddd, J=1.2 Hz, 4.8 Hz, 7.6 Hz, 1H), 7.64 (d, J=7.6 Hz, 1H), 7.87 (d, J=8.8 Hz, 1H), 7.93 (ddd, J=2.0 Hz, 7.6 Hz, 7.6 Hz, 1H), 8.61 (ddd, J=1.2 Hz, 2.0 Hz, 4.8 Hz, 1H).
ESI-MS m/z: 241 [M+2H]²⁺, 481 [M+H]⁺.

### Example 109

### N,N-Dimethyl{6-cyclopropylmethoxy-3-{2-[1-(1-ethynylcyclopropylmethyl)piperidin-4-yl]ethyl}benz[d]isoxazol-7-yl}methylamine dihydrochloride

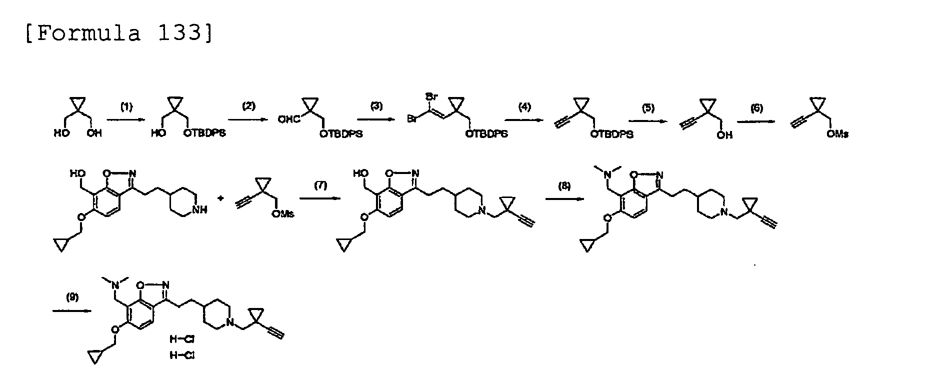

### (1) [1-(tert-Butyldiphenylsilanyloxymethyl)cyclopropyl]methanol

1,1-Bis(hydroxymethyl)cyclopropane (3.6 g) and imidazole (2.4 g) were dissolved in N,N-dimethylformamide (35 mL) in a nitrogen atmosphere. tert-Butylchlorodiphenylsilane (9.2 mL) was added to the solution at -15°C, and the mixture was stirred at - 15°C for one hour. Methanol and water were sequentially added to the reaction mixture, followed by extraction with ethyl acetate twice. The organic layers were sequentially washed with water and a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (5.58 g).
¹H-NMR (400 MHz, CDCl₃) δ(ppm): 0.34-0.38 (m, 2H), 0.47-0.51 (m, 2H), 1.06 (s, 9H), 2.57 (t, J=5.6 Hz, 1H), 3.61 (d, J=5.6 Hz, 2H), 3.62 (s, 2H), 7.35-7.45 (m, 6H), 7.64-7.68 (m, 4H).

### (2) 1-(tert-Butyldiphenylsilanyloxymethyl)cyclopropanecarbaldehyde

The title compound (4.91 g) was obtained by synthesis from [1-(tert-butyldiphenylsilanyloxymethyl)cyclopropyl]methanol (5.57 g) according to Example 874-(2).
¹H-NMR (400 MHz, CDCl₃) δ(ppm) : 1.04 (s, 9H), 1.07-1.12 (m, 2H), 1.13-1.17 (m, 2H), 3.94 (s, 2H), 7.34-7.45 (m, 6H), 7.62-7.66 (m, 4H), 9.09 (s, 1H).

### (3) tert-Butyl[1-(2,2-dibromovinyl)cyclopropylmethoxy]diphenylsilane

Carbon tetrabromide (4 g) was dissolved in methylene chloride (70 mL) in a nitrogen atmosphere. Triphenylphosphine (7.5 g) was added to the solution at -10°C, and the mixture was stirred under ice-cooling for 15 minutes. A solution of 1-(tert-butyldiphenylsilanyloxymethyl)cyclopropanecarbaldehyde (2.4 g) in methylene chloride (10 mL) was added dropwise under ice-cooling over five minutes, and the mixture was stirred under ice-cooling for 20 minutes. Triethylamine (3 mL) and heptane (200 mL) were added under ice-cooling. The resulting suspension was filtered through celite, and the solvent of the filtrate was evaporated under reduced pressure. The residue was suspended in diethyl ether, followed by filtration through celite. The solvent of the filtrate was evaporated under reduced pressure. The residue was purified by silica gel column chromatography (heptane) to obtain the title compound (3.26 g).
¹H-NMR (400 MHz, CDCl₃) δ (ppm): 0.72-0.78 (m, 4H), 1.05 (s, 9H), 3.60 (s, 2H), 6.67 (s, 1H), 7. 34-7.45 (m, 6H), 7.62-7.66 (m, 4H).

### (4) tert-Butyl(1-ethynylcyclopropylmethoxy)diphenylsilane

tert-Butyl[1-(2,2-dibromovinyl)cyclopropylmethoxy]diphenylsilane (1.6 g) was dissolved in tetrahydrofuran (20 mL) in a nitrogen atmosphere. n-Butyllithium (2.71 M solution in hexane) (3 mL) was added dropwise to the solution at -78°C over three minutes, and the mixture was stirred at -78°C for 30 minutes and under ice-cooling for 30 minutes. A saturated ammonium chloride solution was added to the reaction mixture, followed by stirring at room temperature for 30 minutes. A saturated ammonium chloride solution and water were sequentially added, followed by extraction with ethyl acetate. The organic layer was sequentially washed with water and a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (1.07 g).
¹H-NMR (400 MHz, CDCl₃) δ(ppm): 0.79-0.83 (m, 2H), 0.85-0.89 (m, 2H), 1.05 (s, 9H), 1.88 (s, 1H), 3.65 (s, 2H), 7.34-7.44 (m, 6H), 7.63-7.67 (m, 4H).

### (5) (1-Ethynylcyclopropyl)methanol

The title compound (162 mg) was obtained by synthesis from tert-butyl(1-ethynylcyclopropylmethoxy)diphenylsilane (1.07 g) according to Example 79-(5). ¹H-NMR (400 MHz, CDCl₃) δ (ppm) : 0.75-0.80 (m, 2H), 0.97-1.02 (m, 2H), 1.74 (t, J=6.4 Hz, 1H), 1.96 (s, 1H), 3.50 (d, J=6.4 Hz, 2H).

### (6) 1-Ethynylcyclopropylmethyl methanesulfonate

(1-Ethynylcyclopropyl)methanol (160 mL) and triethylamine (0.7 mL) were dissolved in methylene chloride (8 mL). Methanesulfonyl chloride (0.2 mL) was added to the solution under ice-cooling, and the mixture was stirred at room temperature for 30 minutes. A saturated sodium bicarbonate solution was added to the reaction mixture, followed by extraction with methylene chloride. The organic layer was washed with a saturated sodium chloride solution and dried over magnesium sulfate. The drying agent was removed by filtration and then the solvent was evaporated under reduced pressure. The residue was purified by silica gel column chromatography (hexane-diethyl ether) to obtain the title compound (207 mg).
¹H-NMR (400 MHz, CDCl₃) δ(ppm): 0.93-0.97 (m, 2H), 1.12-1.16 (m, 2H), 1.98 (s, 1H), 3.10 (s, 3H), 4.12 (s, 2H).

### (7) {6-Cyclopropylmethoxy-3-{2-[1-(1-ethynylcyclopropylmethyl)piperidin-4-yl]ethyl}benz[d]isoxazol-7-yl}methanol

The title compound (130 mg) was obtained by synthesis from {6-cyclopropylmethoxy-3-[2-(piperidin-4-yl)ethyl]benz[d]isoxazol-7-yl} methanol shown in Example 98-(2) (150 mg) and 1-ethynylcyclopropylmethyl methanesulfonate (100 mg) according to Example 106-(1). ¹H-NMR (400 MHz, CDCl₃) δ(ppm): 0.17-0.22 (m, 2H), 0.23-0.30 (m, 4H), 0.95-0.99 (m, 2H), 1.29-1.42 (m, 4H), 1.70-1.81 (m, 4H), 1.88 (s, 1H), 1.94-2.02 (m, 2H), 2.34 (s, 2H), 2.54-2.72 (br s, 1H), 2.95 (t, J=8.0 Hz, 2H), 3.08 (br d, J=11.6 Hz, 2H), 3.98 (d, J=6.8 Hz, 2H), 5.04 (s, 2H), 6.91 (d, J=8.8 Hz, 1H), 7.48 (d, J=8.8 Hz, 1H).

### (8) N,N-Dimethyl{6-cyclopropylmethoxy-3-{2-[1-(1-ethynylcyclopropylmethyl)piperidin-4-yl]ethyl}benz[d]isoxazol-7-yl}methylamine

The title compound (110 mg) was obtained by synthesis from {6-cyclopropylmethoxy-3-{2-[1-(1-ethynylcyclopropylmethyl)piperidin-4-yl]ethyl}benz[d]isoxazol-7-yl}methanol (127 mg) according to Example 79-(6).
¹H-NMR (400 MHz, CDCl₃) δ(ppm): 0.35-0.40 (m, 2H), 0.61-0.69 (m, 4H), 0.94-0.98 (m, 2H), 1.24-1.43 (m, 4H), 1.71-1.81 (m, 4H), 1.88 (s, 1H), 1.97 (br t, J=11.6 Hz, 2H), 2.33 (s, 6H), 2.33 (s, 2H), 2.94 (t, J=8.0 Hz, 2H), 3.07 (br d, J=11.6 Hz, 2H), 3.82 (s, 2H), 3.94 (d, J=6.8 Hz, 2H), 6.89 (d, J=8.8 Hz, 1H), 7.44 (d, J=8.8 Hz, 1H).

### (9) N,N-Dimethyl{6-cyclopropylmethoxy-3-{2-[1-(1-ethynylcyclopropylmethyl)piperidin-4-yl]ethyl}benz[d]isoxazol-7-yl}methylamine dihydrochloride

The title compound (127 mg) was obtained by synthesis from N,N-dimethyl{6-cyclopropylmethoxy-3-{2-[1-(1-ethynylcyclopropylmethyl)piperidin-4-yl]ethyl}benz[d]isoxazol-7-yl}methylamine (108 mg) according to Example 79-(8).
¹H-NMR (400 MHz, CD₃OD) δ(ppm): 0.42-0.47 (m, 2H), 0.68-0.73 (m, 2H), 1.07-1.12 (m, 2H), 1.18-1.22 (m, 2H), 1.36-1.45 (m, 1H), 1.56-1.69 (m, 2H), 1.67-1.82 (m, 1H), 1.88 (dt, J=7.2 Hz, 8.0 Hz, 2H), 2.08-2.16 (m, 2H), 2.61 (s, 1H), 2.97 (s, 6H), 3.03-3.12 (m, 4H), 3.19 (s, 2H), 3.81 (br d, J=12.4 Hz, 2H), 4.12 (d, J=7.2 Hz, 2H), 4.64 (s, 2H), 7.24 (d, J=8.8 Hz, 1H), 7.93 (d, J=8.8 Hz, 1H).
ESI-MS m/z: 219 [M+2H]²⁺, 436 [M+H]⁺.

### Example 110

### N,N-Dimethyl{6-cyclopropylmethoxy-3-{2-{1-[1-(1-propynyl)cyclopropylmethyl]piperidin-4-yl}ethyl}benz[d]isoxazol-7-yl}methylamine dihydrochloride

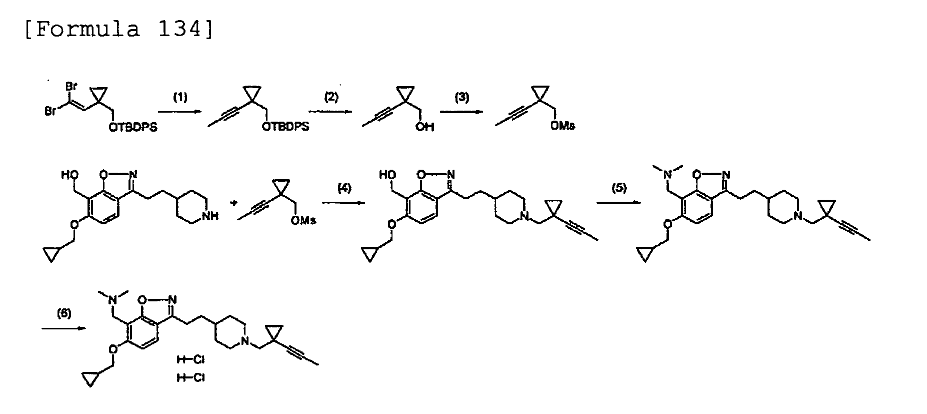

### (1) tert-Butyldiphenyl[1-(1-propynyl)cyclopropylmethoxy]silane

The title compound (1.1 g) was obtained by synthesis from tert-butyl[1-(2,2-dibromovinyl)cyclopropylmethoxy]diphenylsilane (compound obtained in Example 109-(3)) (1.62 g) and methyl iodide (0.6 mL) according to Example 109-(4).
¹H-NMR (400 MHz, CDCl₃) δ(ppm): 0.73-0.80 (m, 4H), 1.05 (s, 9H), 1.76 (s, 3H), 3.63 (s, 2H), 7.33-7.43 (m, 6H), 7.64-7.68 (m, 4H).

### (2) [1-(1-Propynyl)cyclopropyl]methanol

The title compound (306 mg) was obtained by synthesis from tert-butyldiphenyl[1-(1-propynyl)cyclopropylmethoxy]silane (1.07 g) according to Example 79-(5).
¹H-NMR (400 MHz, CDCl₃) δ(ppm) : 0.67-0.71 (m, 2H), 0.87-0.91 (m, 2H), 1.74 (t, J=6.4 Hz, 1H), 1.80 (s, 3H), 3.44 (d, J=6.4 Hz, 2H).

### (3) 1-(1-Propynyl)cyclopropylmethyl methanesulfonate

The title compound (237 mg) was obtained by synthesis from [1-(1-propynyl)cyclopropyl]methanol (180 mg) according to Example 109-(6).
¹H-NMR (400 MHz, CDCl₃) δ(ppm): 0.85-0.89 (m, 2H), 1.00-1.04 (m, 2H), 1.78 (s, 3H), 3.10 (s, 3H), 4.08 (s, 2H).

### (4) {6-Cyclopropylmethoxy-3-{2-{1-[1-(1-propynyl)cyclopropylmethyl]piperidin-4-yl}ethyl}benz[d]isoxazol-7-yl}methanol

The title compound (138 mg) was obtained by synthesis from {6-cyclopropylmethoxy-3-[2-(piperidin-4-yl)ethyl]benz[d]isoxazol-7-yl}methanol (compound obtained in Example 98-(2)) (160 mg) and 1-(1-propynyl)cyclopropylmethyl methanesulfonate (117 mg) according to Example 106-(1).
¹H-NMR (400 MHz, CDCl₃) δ (ppm) : 0.36-0.41 (m, 2H), 0.57-0.61 (m, 2H), 0.65-0.71 (m, 2H), 0.83-0.86 (m, 2H), 1.24-1.42 (m, 4H), 1.68-1.81 (m, 4H), 1.76 (s, 3H), 1.94-2.03 (m, 2H), 2.30 (s, 2H), 2.63 (br t, J=4.4 Hz, 1H), 2.95 (t, J=8.0 Hz, 2H), 3.06 (br d, J=11.6 Hz, 2H), 3.97 (d, J=6.8 Hz, 2H), 5.03 (d, J=4.4 Hz, 2H), 6.90 (d, J=8.8 Hz, 1H), 7.47 (d, J=8.8 Hz, 1H).

### (5) N,N-Dimethyl{6-cyclopropylmethoxy-3-{2-{1-[1-(1-propynyl)cyclopropylmethyl]piperidin-4-yl}ethyl}benz[d]isoxazol-7-yl}methylamine

The title compound (116 mg) was obtained by synthesis from {6-cyclopropylmethoxy-3-{2-{1-[1-(1-propynyl)cyclopropylmethyl]piperidin-4-yl}ethyl}benz[d]isoxazol-7-yl}methanol (136 mg) according to Example 79-(6).
¹H-NMR (400 MHz, CDCl₃) δ(ppm): 0.35-0.40 (m, 2H), 0.57-0.61 (m, 2H), 0.61-0.67 (m, 2H), 0.83-0.87 (m, 2H), 1.25-1.42 (m, 4H), 1.71-1.81 (m, 4H), 1.77 (s, 3H), 1.94-2.03 (m, 2H), 2.30 (s, 2H), 2.33 (s, 6H), 2.94 (t, J=7.6 Hz, 2H), 3.02-3.09 (m, 2H), 3.82 (s, 2H), 3.94 (d, J=6.8 Hz, 2H), 6.89 (d, J=8.8 Hz, 1H), 7.44 (d, J=8.8 Hz, 1H).

### (6) N,N-Dimethyl{6-cyclopropylmethoxy-3-{2-{1-[1-(1-propynyl)cyclopropylmethyl]piperidin-4-yl}ethyl}benz[d]isoxazol-7-yl}methylamine dihydrochloride

The title compound (134 mg) was obtained by synthesis from N,N-dimethyl{6-cyclopropylmethoxy-3-{2-{1-[1-(1-propynyl)cyclopropylmethyl]piperidin-4-yl}ethyl}benz[d]isoxazol-7-yl}methylamine (114 mg) according to Example 79-(8).
¹H-NMR (400 MHz, CD₃OD) δ(ppm) : 0.42-0.47 (m, 2H), 0.68-0.73 (m, 2H), 0.96-1.02 (m, 2H), 1.07-1.11 (m, 2H), 1.36-1.44 (m, 1H), 1.56-1.68 (m, 2H), 1.68-1.78 (m, 1H), 1.80 (s, 3H), 1.89 (dt, J=7.2 Hz, 8.0 Hz, 2H), 2.08-2.16 (m, 2H), 2.97 (s, 6H), 3.01-3.12 (m, 4H), 3.12 (s, 2H), 3.79 (br d, J=12.8 Hz, 2H), 4.12 (d, J=7.2 Hz, 2H), 4.64 (s, 2H), 7.24 (d, J=8.8 Hz, 1H), 7.93 (d, J=8.8 Hz, 1H).
ESI-MS m/z: 226 [M+2H]²⁺, 450 [M+H]⁺.

### Example 111

### N,N-Dimethyl{3-[2-(1-benzylpiperidin-4-yl)ethyl]-6-(1-ethynylcyclopropylmethoxy)benz[d]isoxazol-7-yl}methylamine dihydrochloride

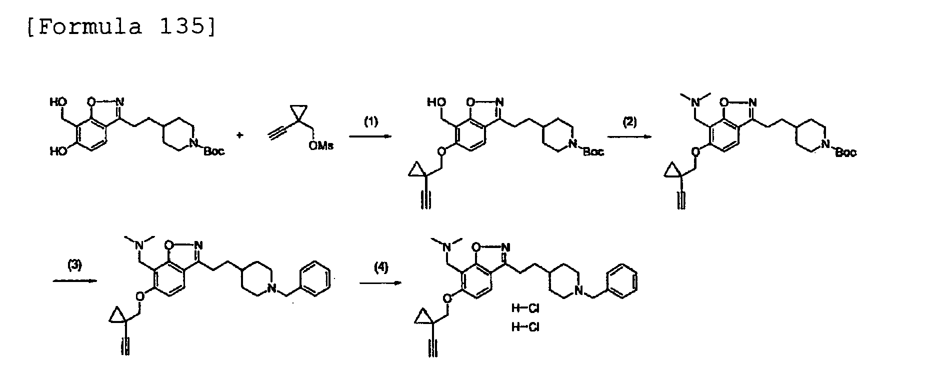

### (1) tert-Butyl 4-{2-[6-(1-ethynylcyclopropylmethoxy)-7-hydroxymethylbenz[d]isoxazol-3-yl]ethyl}piperidine-1-carboxylate

A mixture of tert-butyl 4-[2-(6-hydroxy-7-hydroxymethylbenz[d]isoxazol-3-yl)ethyl]piperidine-1-carboxylate (compound obtained in Preparation Example 2-(6)) (150 mg), 1-ethynylcyclopropylmethyl methanesulfonate (compound obtained in Example 109-(6)) (100 mg), potassium carbonate (110 mg) and N,N-dimethylformamide (4 mL) was stirred at 60°C over one hour and 10 minutes. Sodium iodide (80 mg) and hexamethylphosphoric acid triamide (0.4 mL) were added to the mixture, followed by stirring at 80°C for 2.5 hours. The reaction mixture was cooled to room temperature and then water was added, followed by extraction with ethyl acetate. The organic layer was sequentially washed with water (twice) and a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (142 mg).
¹H-NMR (400 MHz, CDCl₃) δ(ppm): 0.92-0.96 (m, 2H), 1.09-1.23 (m, 4H), 1.45 (s, 9H), 1.46-1.53 (m, 1H), 1.70-1.81 (m, 4H), 2.00 (s, 1H), 2.67 (br t, J=12.0 Hz, 2H), 2.88 (t, J=7.2 Hz, 1H), 2.97 (t, J=8.0 Hz, 2H), 4.02 (s, 2H), 4.02-4.16 (m, 2H), 5.06 (d, J=7.2 Hz, 2H), 6.86 (d, J=8.8 Hz, 1H), 7.47 (d, J=8.8 Hz, 1H).

### (2) tert-Butyl 4-{2-[7-(N,N-dimethylaminomethyl)-6-(1-ethynylcyclopropylmethoxy)benz[d]isoxazol-3-yl]ethyl}piperidine-1-carboxylate

The title compound (127 mg) was obtained by synthesis from tert-butyl 4-{2-[6-(1-ethynylcyclopropylmethoxy)-7-hydroxymethylbenz[d]isoxazol-3-yl]ethyl}piperidine-1-carboxylate (140 mg) according to Example 79-(6).
¹H-NMR (400 MHz, CDCl₃) δ(ppm): 0.95-1.00 (m, 2H), 1.08-1.23 (m, 4H), 1.45 (s, 9H), 1.47-1.54 (m, 1H), 1.69-1.82 (m, 4H), 1.94 (s, 1H), 2.34 (s, 6H), 2.62-2.73 (m, 2H), 2.95 (t, J=8.0 Hz, 2H), 3.84 (s, 2H), 3.98-4.15 (m, 2H), 4.03 (s, 2H), 6.88 (d, J=8.8 Hz, 1H), 7.44 (d, J=8.8 Hz, 1H).

### (3) N,N-Dimethyl{3-[2-(1-benzylpiperidin-4-yl)ethyl]-6-(1-ethynylcyclopropylmethoxy)benz[d]isoxazol-7-yl}methylamine

The title compound (76 mg) was obtained by synthesis from tert-butyl 4-{2-[7-(N,N-dimethylaminomethyl)-6-(1-ethynylcyclopropylmethoxy)benz[d]isoxazol-3-yl]ethyl}piperidine-1-carboxylate (125 mg) according to Example 79- (7) .
¹H-NMR (400 MHz, CDCl₃) δ(ppm): 0.94-0.99 (m, 2H), 1.08-1.12 (m, 2H), 1.25-1.38 (m, 3H), 1.70-1.80 (m, 4H), 1.90-1.97 (m, 2H), 1.94 (s, 1H), 2.34 (s, 6H), 2.88 (br d, J=12.0 Hz, 2H), 2.93 (t, J=8.0 Hz, 2H), 3.48 (s, 2H), 3.84 (s, 2H), 4.03 (s, 2H), 6.87 (d, J=8.4 Hz, 1H), 7.21-7.31 (m, 5H), 7.43 (d, J=8.4 Hz, 1H).

### (4) N,N-Dimethyl{3-[2-(1-benzylpiperidin-4-yl)ethyl]-6-(1-ethynylcyclopropylmethoxy)benz[d]isoxazol-7-yl}methylamine dihydrochloride

The title compound (82 mg) was obtained by synthesis from N,N-dimethyl{3-[2-(1-benzylpiperidin-4-yl)ethyl]-6-(1-ethynylcyclopropylmethoxy)benz[d]isoxazol-7-yl}methylamine (74 mg) according to Example 79-(8).
¹H-NMR (400 MHz, CD₃OD) δ(ppm): 1.00-1.06 (m, 2H), 1.08-1.16 (m, 2H), 1.48-1.60 (m, 2H), 1.67-1.78 (m, 1H), 1.85 (dt, J=7.6 Hz, 7.6 Hz, 2H), 2.04-2.12 (m, 2H), 2.42 (s, 1H), 2.96-3.03 (m, 2H), 3.00 (s, 6H), 3.06 (t, J=7.6 Hz, 2H), 3.50 (br d, J=12.8 Hz, 2H), 4.17 (s, 2H), 4.31 (s, 2H), 4.69 (s, 2H), 7.20-7.26 (m, 1H), 7.47-7.53 (m, 5H), 7.91-7.95 (m, 1H).
ESI-MS m/z: 237 [M+2H]²⁺, 472 [M+H]⁺.

### Example 112

### N,N-Dimethyl{3-[2-(1-benzylpiperidin-4-yl)ethyl]-6-[1-(1-propynyl)cyclopropylmethoxy]benz[d]isoxazol-7-yl}methylamine dihydrochloride

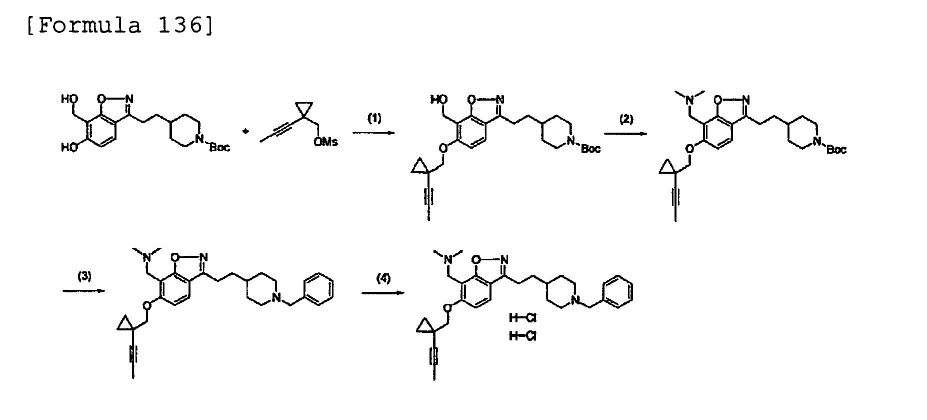

### (1) tert-Butyl 4-(2-{7-hydroxymethyl-6-[1-(1-propynyl)cyclopropylmethoxy]benz[d]isoxazol-3-yl}ethyl}piperidine-1-carboxylate

The title compound (97 mg) was obtained by synthesis from tert-butyl 4-[2-(6-hydroxy-7-hydroxymethylbenz[d]isoxazol-3-yl)ethyl]piperidine-1-carboxylate (compound obtained in Preparation Example 2-(6)) (160 mg) and 1-(1-propynyl)cyclopropylmethyl methanesulfonate (compound obtained in Example 110-(3)) (117 mg) according to Example 111-(1).
¹H-NMR (400 MHz, CDCl₃) δ(ppm) : 0.84-0.89 (m, 2H), 1.03-1.07 (m, 2H), 1.15 (ddd, J=4.0 Hz, 12.4 Hz, 24.4 Hz, 2H), 1.44-1.54 (m, 1H), 1.45 (s, 9H), 1.70-1.81 (m, 4H), 1.78 (s, 3H), 2.66 (br, t, J=12.8 Hz, 2H), 2.96 (t, J=7.6 Hz, 2H), 3.16 (t, J=7.2 Hz, 1H), 3.97 (s, 2H), 4.01-4.17 (m, 2H), 5.06 (d, J=7.2 Hz, 2H), 6.85 (d, J=8.8 Hz, 1H), 7.46 (d, J=8.8 Hz, 1H).

### (2) tert-Butyl 4-{2-{7-(N,N-dimethylaminomethyl)-6-[1-(1-propynyl)cyclopropylmethoxy]benz[d]isoxazol-3-yl}ethyl}piperidine-1-carboxylate

The title compound (95 mg) was obtained by synthesis from tert-butyl 4-{2-{7-hydroxymethyl-6-[1-(1-propynyl)cyclopropylmethoxy]benz[d]isoxazol-3-yl}ethyl}piperidine-1-carboxylate (95 mg) according to Example 79-(6).
¹H-NMR (400 MHz, CDCl₃) δ(ppm): 0.86-0.91 (m, 2H), 0.96-1.01 (m, 2H), 1.15 (ddd, J=4.0 Hz, 12.0 Hz, 24.0 Hz, 2H), 1.45 (s, 9H), 1.47-1.54 (m, 1H), 1.70-1.82 (m, 4H), 1.77 (s, 3H), 2.35 (s, 6H), 2.62-2.73 (m, 2H), 2.95 (t, J=7.6 Hz, 2H), 3.85 (s, 2H), 3.99 (s, 2H), 4.03-4.17 (m, 2H), 6.88 (d, J=8.8 Hz, 1H), 7.43 (d, J=8.8 Hz, 1H).

### (3) N,N-Dimethyl{3-[2-(1-benzylpiperidin-4-yl)ethyl]-6-[1-(1-propynyl)cyclopropylmethoxy]benz[d]isoxazol-7-yl}methylamine

The title compound (67 mg) was obtained by synthesis from tert-butyl 4-{2-{7-(N,N-dimethylaminomethyl)-6-[1-(1-propynyl)cyclopropylmethoxy]benz[d]isoxazol-3-yl}ethyl}piperidine-1-carboxylate (93 mg) according to Example 79-(7).
¹H-NMR (400 MHz, CDCl₃) δ(ppm) : 0.86-0.90 (m, 2H), 0.96-1.00 (m, 2H), 1.24-1.36 (m, 3H), 1.70-1.79 (m, 4H), 1.77 (s, 3H), 1.93 (br d, J=11.2 Hz, 2H), 2.34 (s, 6H), 2.87 (br d, J=11.2 Hz, 2H), 2.93 (t, J=8.0 Hz, 2H), 3.48 (s, 2H), 3.84 (s, 2H), 3.98 (s, 2H), 6.87 (d, J=8.8 Hz, 1H), 7.20-7.31 (m, 5H), 7.42 (d, J=8.8 Hz, 1H).

### (4) N,N-Dimethyl{3-[2-(1-benzylpiperidin-4-yl)ethyl]-6-[1-(1-propynyl)cyclopropylmethoxy]benz[d]isoxazol-7-yl}methylamine dihydrochloride

The title compound (77 mg) was obtained by synthesis from N,N-dimethyl{3-[2-(1-benzylpiperidin-4-yl)ethyl]-6-[1-(1-propynyl)cyclopropylmethoxy]benz[d]isoxazol-7-yl}methylamine (65 mg) according to Example 79-(8).
¹H-NMR (400 MHz, CD₃OD) δ(ppm) : 0.91-0.96 (m, 2H), 0.97-1.02 (m, 2H), 1.47-1.69 (m, 2H), 1.67-1.76 (m, 1H), 1.75 (s, 3H), 1.86 (dt, J=7.6 Hz, 7.6 Hz, 2H), 2.04-2.12 (m, 2H), 2.94-3.02 (m, 2H), 3.00 (s, 6H), 3.06 (t, J=7.6 Hz, 2H), 3.50 (br d, J=13.2 Hz, 2H), 4.11 (s, 2H), 4.31 (s, 2H), 4.68 (s, 2H), 7.21 (d, J=8.8 Hz, 1H), 7.47-7.55 (m, 5H), 7.92 (d, J=8.8 Hz, 1H).
ESI-MS m/z: 244 [M+2H]²⁺, 486 [M+H]⁺.

### Example 113

### N,N-Dimethyl{3-[2-(1-benzylpiperidin-4-yl)ethyl]-6-[1-(3-methoxy-1-propynyl) cyclopropylmethoxy]benz[d]isoxazol-7-yl}methylamine dihydrochloride

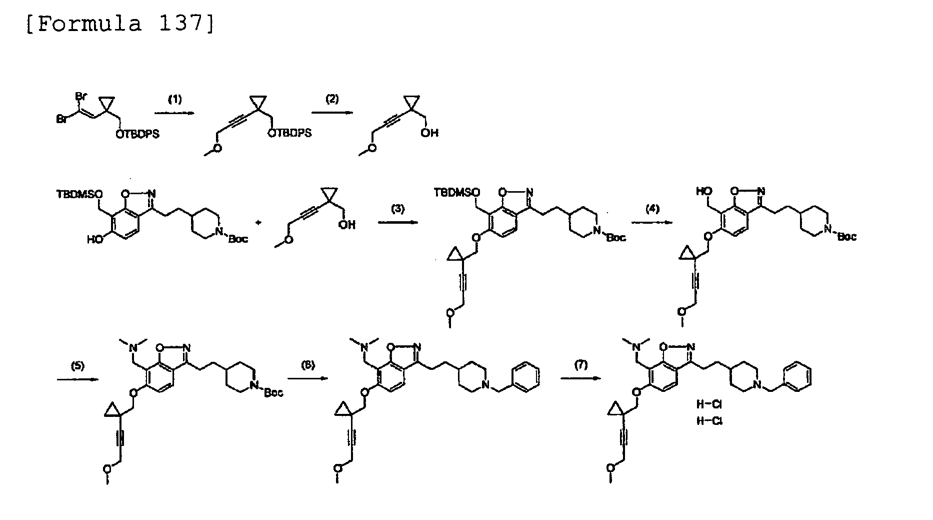

### (1) tert-Butyl[1-(3-methoxy-1-propynyl)cyclopropylmethoxy]diphenylsilane

The title compound (1.2 g) was obtained by synthesis from tert-butyl[1-(2,2-dibromovinyl)cyclopropylmethoxy]diphenylsilane (compound obtained in Example 109-(3)) (1.62 g) and chloromethyl methyl ether (0.75 mL) according to Example 109-(4).
¹H-NMR (400 MHz, CDCl₃) δ(ppm): 0.82-0.87 (m, 4H), 1.05 (s, 9H), 3.34 (s, 3H), 3.65 (s, 2H), 4.05 (s, 2H), 7.34-7.44 (m, 6H), 7.63-7.68 (m, 4H).

### (2) [1-(3-Methoxy-1-propynyl)cyclopropyl]methanol

The title compound (353 mg) was obtained by synthesis from tert-butyl[1-(3-methoxy-1-propynyl)cyclopropylmethoxy]diphenylsilane (1.07 g) according to Example 79-(5).
¹H-NMR (400 MHz, CDCl₃) δ(ppm): 0.76-0.80 (m, 2H), 0.96-1.01 (m, 2H), 1.77 (br t, J=5.2 Hz, 1H), 3.36 (s, 3H), 3.49 (d, J=5.2 Hz, 2H), 4.08 (s, 2H).

### (3) tert-Butyl 4-{2-{7-(tert-butyldimethylsilanyloxymethyl)-6-[1-(3-methoxy-1-propynyl)cyclopropylmethoxy]benz[d]isoxazol-3-yl}ethyl}piperidine-1-carboxylate

The title compound was synthesized with reference to Tetrahedron Lett., 1993, 34, 1639. tert-Butyl 4-{2-[7-(tert-butyldimethylsilanyloxymethyl)-6-hydroxybenz[d]isoxazol-3-yl]ethylpiperidine-1-carboxylate (compound obtained in Example 79-(1)) (350 mg) and 1,1'-(azodicarbonyl)dipiperidine (270 mg) were dissolved in tetrahydrofuran (3 mL) in a nitrogen atmosphere. A solution of [1-(3-methoxy-1-propynyl)cyclopropyl]methanol (120 mg) in tetrahydrofuran (4 mL) was added to the solution, and then a solution of tributylphosphine (216 mg) in tetrahydrofuran (1 mL) was added dropwise under ice-cooling over four minutes. The mixture was stirred under ice-cooling for 45 minutes and at room temperature for 8.5 hours. The reaction mixture was diluted with diethyl ether and then filtered through celite. The solvent was evaporated under reduced pressure. Diethyl ether was added to the residue. The resulting suspension was filtered through celite, and the solvent was evaporated under reduced pressure. The residue was purified by NH silica gel column chromatography (heptane-ethyl acetate) and silica gel column chromatography (heptane-ethyl acetate) to obtain the title compound (110 mg).
¹H-NMR (400 MHz, CDCl₃) δ(ppm) : 0.14 (s, 6H), 0.92 (s, 9H), 0.96-1.00 (m, 2H), 1.05-1.09 (m, 2H), 1.08-1.22 (m, 2H), 1.43-1.53 (m, 1H), 1.45 (s, 9H), 1.70-1.78 (m, 2H), 1.78 (dt, J=7.6 Hz, 7.6 Hz, 2H), 2.62-2.73 (m, 2H), 2.95 (t, J=7.6 Hz, 2H), 3.34 (s, 3H), 4.02-4.12 (m, 2H), 4.04 (s, 2H), 4.06 (s, 2H), 5.02 (s, 2H), 6.89 (d, J=8.4 Hz, 1H), 7.44 (d, J=8.4 Hz, 1H).

### (4) tert-Butyl 4-{2-{7-hydroxymethyl-6-[1-(3-methoxy-1-propynyl)cyclopropylmethoxy]benz[d]isoxazol-3-yl}ethyl}piperidine-1-carboxylate

The title compound (87 mg) was obtained by synthesis from tert-butyl 4-{2-{7-(tert-butyldimethylsilanyloxymethyl)-6-[1-(3-methoxy-1-propynyl)cyclopropylmethoxy]benz[d]isoxazol-3-yl}ethyl}piperidine-1-carboxylate (108 mg) according to Example 79-(5).
¹H-NMR (400 MHz, CDCl₃) δ(ppm): 0.93-0.97 (m, 2H), 1.09-1.21 (m, 4H), 1.44-1.52 (m, 1H), 1.45 (s, 9H), 1.70-1.81 (m, 4H), 2.62-2.73 (m, 2H), 2.93 (t, J=6.8 Hz, 1H), 2.96 (t, J=8.0 Hz, 2H), 3.33 (s, 3H), 4.02 (s, 2H), 4.04-4.14 (m, 2H), 4.05 (s, 2H), 5.05 (d, J=6.8 Hz, 2H), 6.86 (d, J=8.8 Hz, 1H), 7.46 (d, J=8.8 Hz, 1H).

### (5) tert-Butyl 4-{2-{7-(N,N-dimethylaminomethyl)-6-[1-(3-methoxy-1-propynyl)cyclopropylmethoxy]benz[d]isoxazol-3-yl}ethyl}piperidine-1-carboxylate

The title compound (77 mg) was obtained by synthesis from tert-butyl 4-{2-{7-hydroxymethyl-6-[1-(3-methoxy-1-propynyl)cyclopropylmethoxy]benz[d]isoxazol-3-yl}ethyl}piperidine-1-carboxylate (85 mg) according to Example 79-(6).
¹H-NMR (400 MHz, CDCl₃) δ(ppm): 0.96-1.00 (m, 2H), 1.06-1.11 (m, 2H), 1.15 (ddd, J=4.0 Hz, 12.4 Hz, 24.4 Hz, 2H), 1.45 (s, 9H), 1.47-1.53 (m, 1H), 1.71-1.81 (m, 4H), 2.34 (s, 6H), 2.67 (br d, J=12.4 Hz, 2H), 2.95 (t, J=8.0 Hz, 2H), 3.33 (s, 2H), 3.83 (s, 3H), 4.03 (s, 2H), 4.03-4.15 (m, 2H), 4.05 (s, 2H), 6.88 (d, J=8.4 Hz, 1H), 7.44 (d, J=8.4 Hz, 1H).

### (6) N,N-Dimethyl{3-[2-(1-benzylpiperidin-4-yl)ethyl]-6-[1-(3-methoxy-1-propynyl)cyclopropylmethoxy]benz[d]isoxazol-7-yl}methylamine

The title compound (57 mg) was obtained by synthesis from tert-butyl 4-{2-{7-(N,N-dimethylaminomethyl)-6-[1-(3-methoxy-1-propynyl)cyclopropylmethoxy]benz[d]isoxazol-3-yl}ethyl}piperidine-1-carboxylate (75 mg) according to Example 79- (7) .
¹H-NMR (400 MHz, CDCl₃) δ(ppm): 0.96-1.00 (m, 2H), 1.06-1.10 (m, 2H), 1.24-1.38 (m, 3H), 1.70-1.80 (m, 4H), 1.93 (br t, J=11.2 Hz, 2H), 2.33 (s, 6H), 2.88 (br d, J=11.2 Hz, 2H), 2.93 (t, J=8.0 Hz, 2H), 3.33 (s, 3H), 3.48 (s, 2H), 3.83 (s, 2H), 4.03 (s, 2H), 4.05 (s, 2H), 6.87 (d, J=8.8 Hz, 1H), 7.21-7.31 (m, 5H), 7.43 (d, J=8.8 Hz, 1H).

### (7) N,N-Dimethyl{3-[2-(1-benzylpiperidin-4-yl)ethyl]-6-[1-(3-methoxy-1-propynyl)cyclopropylmethoxy]benz[d]isoxazol-7-yl}methylamine dihydrochloride

The title compound (64 mg) was obtained by synthesis from N,N-dimethyl{3-[2-(1-benzylpiperidin-4-yl)ethyl]-6-[1-(3-methoxy-1-propynyl)cyclopropylmethoxy]benz[d]isoxazol-7-yl}methylamine (55 mg) according to Example 79-(8).
¹H-NMR (400 MHz, CD₃OD) 5(ppm): 1.02-1.07 (m, 2H), 1.08-1.13 (m, 2H), 1.46-1.58 (m, 2H), 1.66-1.77 (m, 1H), 1.85 (dt, J=7.6 Hz, 8.0 Hz, 2H), 2.05-2.13 (m, 2H), 2.96-3.02 (m, 2H), 3.00 (s, 6H), 3.06 (t, J=8.0 Hz, 2H), 3.30 (s, 3H), 3.50 (br d, J=12.4 Hz, 2H), 4.05 (s, 2H), 4.17 (s, 2H), 4.30 (s, 2H), 4.68 (s, 2H), 7.23 (d, J=8.8 Hz, 1H), 7.47-7.54 (m, 5H), 7.92 (d, J=8.8 Hz, 1H).
ESI-MS m/z: 259 [M+2H]²⁺, 516 [M+H]⁺.

### Example 114

### 5-{4-{2-[7-(N,N-Dimethylaminomethyl)-6-(pyridin-2-ylmethoxy)benz[d]isoxazol-3-yl]ethyl}piperidinomethyl}thiophene-2-carbonitrile trihydrochloride

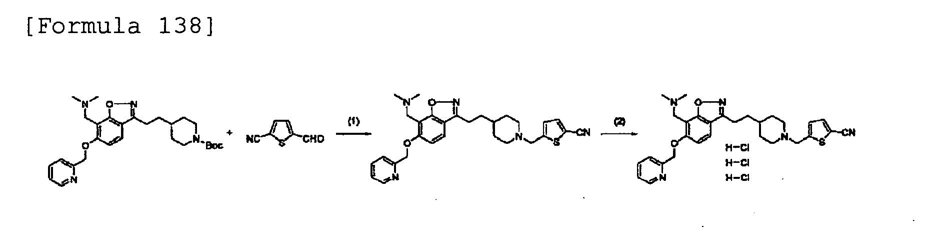

### (1) 5-{4-{2-[7-(N,N-Dimethylaminomethyl)-6-(pyridin-2-ylmethoxy)benz[d]isoxazol-3-yl]ethyl}piperidinomethyl}thiophene-2-carbonitrile

N,N-Dimethyl{3-[2-(piperidin-4-yl)ethyl]-6-(pyridin-2-ylmethoxy)benz[d]isoxazol-7-yl}methylamine was synthesized from tert-butyl 4-{2-[7-(N,N-dimethylaminomethyl)-6-(pyridin-2-ylmethoxy)benz[d]isoxazol-3-yl]ethyl}piperidine-1-carboxylate (compound obtained in Example 103-(2)) (130 mg) according to Example 96-(4). Then, the title compound (84 mg) was obtained by synthesis from the compound and 5-formylthiophene-2-carbonitrile (compound obtained in Example 155-(1)) (70 mg) according to Example 86-(1).
¹H-NMR (400 MHz, CDCl₃) δ(ppm) : 1.25-1.38 (m, 3H), 1.72-1.81 (m, 4H), 1.99-2.07 (m, 2H), 2.35 (s, 6H), 2.90 (br d, J=11.6 Hz, 2H), 2.94 (t, J=8.0 Hz, 2H), 3.68 (s, 2H), 3.87 (s, 2H), 5.33 (s, 2H), 6.87 (d, J=3.6 Hz, 1H), 6.99 (d, J=8.8 Hz, 1H), 7.21-7.27 (m, 1H), 7.46
(d, J=3.6 Hz, 1H), 7.46 (d, J=8.8 Hz, 1H), 7.58 (d, J=8.0 Hz, 1H), 7.74 (ddd, J=1.6 Hz, 8.0 Hz, 8.0 Hz, 1H), 8.59 (dd, J=1.6 Hz, 4.8 Hz, 1H).

### (2) 5-{4-{2-[7-(N,N-Dimethylaminomethyl)-6-(pyridin-2-ylmethoxy)benz[d]isoxazol-3-yl]ethyl}piperidinomethyl}thiophene-2-carbonitrile trihydrochloride

The title compound (98 mg) was obtained by synthesis from 5-{4-{2-[7-(N,N-dimethylaminomethyl)-6-(pyridin-2-ylmethoxy)benz[d]isoxazol-3-yl]ethyl}piperidinomethyl}thiophene-2-carbonitrile (82 mg) according to Example 79-(8).
¹H-NMR (400 MHz, CD₃OD) δ(ppm) : 1.53-1.65 (m, 2H), 1.68-1.79 (m, 1H), 1.85 (dt, J=7.6 Hz, 7.6 Hz, 2H), 2.08-2.16 (m, 2H), 2.95 (s, 6H), 3.02-3.13 (m, 4H), 3.56 (br d, J=12.0 Hz, 2H), 4.65 (s, 2H), 4.76 (s, 2H), 5.80 (s, 2H), 7.38 (d, J=8.8 Hz, 1H), 7.49 (d, J=4.0 Hz, 1H), 7. 80 (d, J=4.0 Hz, 1H), 8.01 (d, J=8.8 Hz, 1H), 8.11 (dd, J=4.8 Hz, 7.6 Hz, 1H), 8.30 (d, J=7.6 Hz, 1H), 8.69 (ddd, J=1.2 Hz, 7.6 Hz, 7.6 Hz, 1H), 8.94 (dd, J=1.2 Hz, 4.8 Hz, 1H).
ESI-MS m/z: 516 [M+H]⁺.

### Example 115

### 5-{4-{2-[6-Cyclopropylmethoxy-7-ethylaminomethylbenz[d]isoxazol-3-yl)ethyl]piperidinomethyl}thiophene-2-carbonitrile dihydrochloride

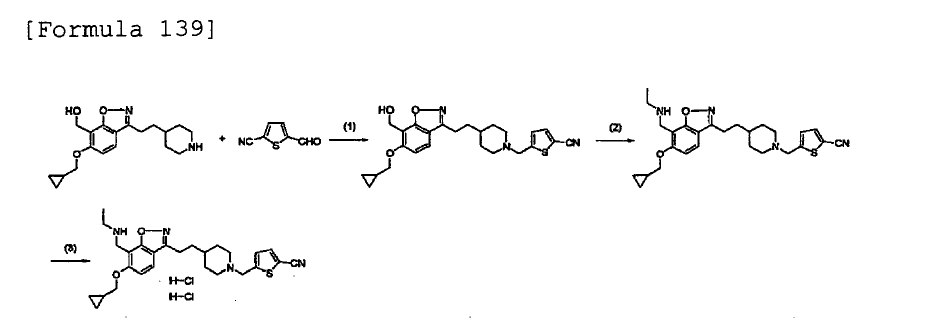

### (1) 5-{4-{2-[6-Cyclopropylmethoxy-7-hydroxymethylbenz[d]isoxazol-3-yl)ethyl]piperidinomethyl}thiophene-2-carbonitrile

The title compound (635 mg) was obtained by synthesis from {6-cyclopropylmethoxy-3-[2-(piperidin-4-yl)ethyl]benz[d]isoxazol-7-yl}methanol (compound obtained in Example 98-(2)) (500 mg) and 5-formylthiophene-2-carbonitrile (compound obtained in Example 155-(1)) (300 mg) according to Example 86-(1).
¹H-NMR (400 MHz, CDCl₃) δ(ppm): 0.36-0.41 (m, 2H), 0.66-0.71 (m, 2H), 1.26-1.37 (m, 4H), 1.72-1.81 (m, 4H), 1.98-2.06 (m, 2H), 2.64 (t, J=6.4 Hz, 1H), 2.90 (br d, J=11.6 Hz, 2H), 2.95 (t, J=8.0 Hz, 2H), 3.68 (s, 2H), 3.97 (d, J=7.2 Hz, 2H), 5.03 (d, J=6.4 Hz, 2H), 6.87 (d, J=4.0 Hz, 1H), 6.90 (d, J=8.8 Hz, 1H), 7.46 (d, J=4.0 Hz, 1H), 7.46 (d, J=8.8 Hz, 1H).

### (2) 5-{4-{2-[6-Cyclopropylmethoxy-7-ethylaminomethylbenz[d]isoxazol-3-yl)ethyl]piperidinomethyl}thiophene-2-carbonitrile

The title compound (72 mg) was obtained by synthesis from 5-{4-{2-[6-cyclopropylmethoxy-7-hydroxymethylbenz[d]isoxazol-3-yl]ethyl}piperidinomethyl}thiophene-2-carbonitrile (100 mg) and ethylamine (2 M solution in tetrahydrofuran) (1.5 mL) according to Example 79-(6).
¹H-NMR (400 MHz, CDCl₃) δ(ppm) : 0.35-0.40 (m, 2H), 0.63-0.69 (m, 2H), 1.17 (t, J=7.2 Hz, 3H), 1.24-1.37 (m, 4H), 1.71-1.83 (m, 4H), 1.99-2.06 (m, 2H), 2.71 (q, J=7.2 Hz, 2H), 2.90 (br d, J=11.6 Hz, 2H), 2.94 (t, J=8.0 Hz, 2H), 3.68 (s, 2H), 3.95 (d, J=7.2 Hz, 2H), 4.19 (s, 2H), 6.87 (d, J=4.0 Hz, 1H), 6.88 (d, J=8.4 Hz, 1H), 7.43 (d, J=8.4 Hz, 1H), 7.46 (d, J=4.0 Hz, 1H).

### (3) 5-{4-{2-[6-Cyclopropylmethoxy-7-ethylaminomethylbenz[d]isoxazol-3-yl)ethyl]piperidinomethyl}thiophene-2-carbonitrile dihydrochloride

The title compound (72 mg) was obtained by synthesis from 5-{4-{2-[6-cyclopropylmethoxy-7-ethylaminomethylbenz[d]isoxazol-3-yl]ethyl}piperidinomethyl}thiophene-2-carbonitrile (70 mg) according to Example 79-(8).
¹H-NMR (400 MHz, CD₃OD) δ(ppm) : 0.43-0.47 (m, 2H), 0.67-0.72 (m, 2H), 1.36-1.43 (m, 1H), 1.40 (t, J=7.2 Hz, 3H), 1.51-1.63 (m, 2H), 1.66-1.79 (m, 1H), 1.85 (dt, J=7.2 Hz, 8.0 Hz, 2H), 2.07-2.16 (m, 2H), 3.00-3.09 (m, 4H), 3.20 (t, J=7.2 Hz, 2H), 3.56 (br d, J=12.0 Hz, 2H), 4.10 (d, J=7.2 Hz, 2H), 4.50 (s, 2H), 4.64 (s, 2H), 7.21 (d, J=8.8 Hz, 1H), 7.47 (d, J=4.0 Hz, 1H), 7.80 (d, J=4.0 Hz, 1H), 7.87 (d, J=8.8 Hz, 1H).
ESI-MS m/z: 479 [M+H]⁺.

### Example 116

### 5-{4-{2-{6-Cyclopropylmethoxy-7-[(N-methylethylamino)methyl]benz[d]isoxazol-3-yl}ethyl}piperidinomethyl}thiophene-2-carbonitrile dihydrochloride

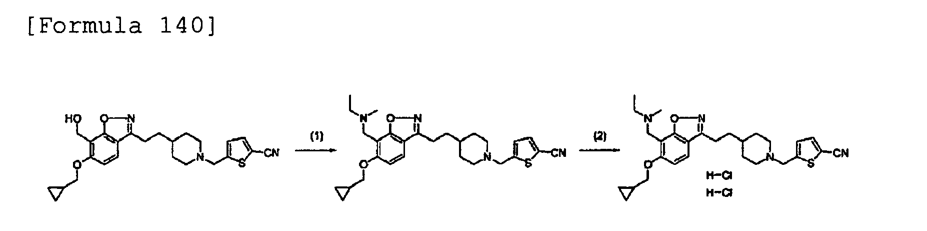

### (1) 5-{4-{2-{6-Cyclopropylmethoxy-7-[(N-methylethylamino)methyl]benz[d]isoxazol-3-yl}ethyl}piperidinomethyl}thiophene-2-carbonitrile

The title compound (95 mg) was obtained by synthesis from 5-{4-{2-[6-cyclopropylmethoxy-7-hydroxymethylbenz[d]isoxazol-3-yl]ethyl}piperidinomethyl}thiophene-2-carbonitrile (compound obtained in Example 115-(1)) (100 mg) and ethylmethylamine (0.5 mL) according to Example 79-(6).
¹H-NMR (400 MHz, CDCl₃) δ(ppm): 0.35-0.40 (m, 2H), 0.62-0.67 (m, 2H), 1.20 (t, J=7.2 Hz, 3H), 1.24-1.38 (m, 4H), 1.72-1.82 (m, 4H), 2.03 (br t, J=11.2 Hz, 2H), 2.30 (s, 3H), 2.60 (br q, J=7.2 Hz, 2H), 2.90 (br d, J=11.2 Hz, 2H), 2.94 (d, J=8.0 Hz, 2H), 3.68 (s, 2H), 3.89 (s, 2H), 3.93 (d, J=6.8 Hz, 2H), 6.87 (d, J=4.0 Hz, 1H), 6.88 (d, J=8.8 Hz, 1H), 7.43 (d, J=8.8 Hz, 1H), 7.46 (d, J=4.0 Hz, 1H).

### (2) 5-{4-{2-{6-Cyclopropylmethoxy-7-[(N-methylethylamino)methyl]benz[d]isoxazol-3-yl}ethyl}piperidinomethyl}thiophene-2-carbonitrile dihydrochloride

The title compound (106 mg) was obtained by synthesis from 5-{4-{2-{6-cyclopropylmethoxy-7-[(N-methylethylamino)methyl]benz[d]isoxazol-3-yl}ethyl}piperidinomethyl}thiophene-2-carbonitrile (93 mg) according to Example 79-(8).
¹H-NMR (400 MHz, CD₃OD) δ (ppm) : 0.43-0.47 (m, 2H), 0.68-0.73 (m, 2H), 1.35-1.44 (m, 1H), 1.50 (dd, J=7.2 Hz, 7.2 Hz, 3H), 1.51-1.64 (m, 2H), 1.66-1.79 (m, 1H), 1.85 (dt, J=7.2 Hz, 7.2 Hz, 2H), 2.11 (br d, J=12.4 Hz, 2H), 2.89 (s, 3H), 3.01-3.11 (m, 4H), 3.29 (dq, J=7.2 Hz, 12.8 Hz, 1H), 3.45 (dq, J=7.2 Hz, 12.8 Hz, 1H), 3.56 (br d, J=12.4 Hz, 2H), 4.08 (dd, J=7.2 Hz, 10.0 Hz, 1H), 4.14 (dd, J=7.2 Hz, 10.0 Hz, 1H), 4.52 (d, J=13.2 Hz, 1H), 4.64 (s, 2H), 4.73 (d, J=13.2 Hz, 1H), 7.22 (d, J=8.8 Hz, 1H), 7.48 (d, J=3.6 Hz, 1H), 7.80 (d, J=3.6 Hz, 1H), 7.91 (d, J=8.8 Hz, 1H).
ESI-MS m/z: 493 [M+H]⁺.

### Example 117

### 5-{4-[2-(6-Cyclopropylmethoxy-7-diethylaminomethylbenz[d]isoxazol-3-yl)ethyl]piperidinomethyl}thiophene-2-carbonitrile dihydrochloride

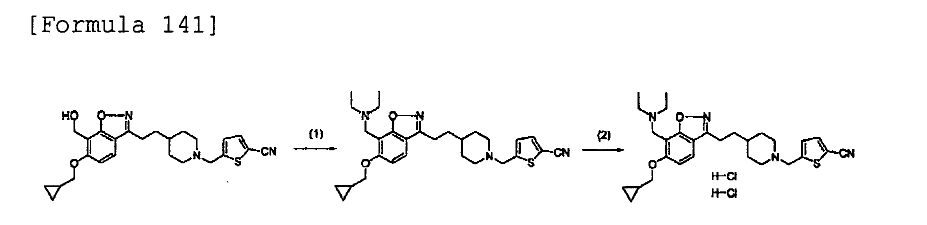

### (1) 5-{4-[2-(6-Cyclopropylmethoxy-7-diethylaminomethylbenz[d]isoxazol-3-yl)ethyl]piperidinomethyl}thiophene-2-carbonitrile

The title compound (106 mg) was obtained by synthesis from 5-{4-{2-[6-cyclopropylmethoxy-7-hydroxymethylbenz[d]isoxazol-3-yl]ethyl}piperidinomethyl}thiophene-2-carbonitrile (compound obtained in Example 115-(1)) (100 mg) and diethylamine (0.5 mL) according to Example 79-(6).
¹H-NMR (400 MHz, CDCl₃) δ(ppm): 0.34-0.39 (m, 2H), 0.62-0.70 (m, 2H), 1.11-1.22 (m, 6H), 1.25-1.39 (m, 4H), 1.72-1.83 (m, 4H), 1.99-2.08 (m, 2H), 2.56-2.74 (m, 4H), 2.86-2.97 (m, 4H), 3.68 (s, 2H), 3.92 (d, J=6.8 Hz, 2H), 3.92-3.99 (m, 2H), 6.87 (d, J=3.6 Hz, 1H), 6.87 (d, J=8.4 Hz, 1H), 7.42 (d, J=8.4 Hz, 1H), 7.45 (d, J=3.6 Hz, 1H).

### (2) 5-{4-[2-(6-Cyclopropylmethoxy-7-diethylaminomethylbenz[d]isoxazol-3-yl)ethyl]piperidinomethyl}thiophene-2-carbonitrile dihydrochloride

The title compound (110 mg) was obtained by synthesis from 5-{4-[2-(6-cyclopropylmethoxy-7-diethylaminomethylbenz[d]isoxazol-3-yl)ethyl]piperidinomethyl}thiophene-2-carbonitrile (104 mg) according to Example 79-(8).
¹H-NMR (400 MHz, CD₃OD) δ (ppm) : 0.43-0.48 (m, 2H), 0.68-0.74 (m, 2H), 1.34-1.45 (m, 1H), 1.49 (t, J=7.2 Hz, 6H), 1.50-1.64 (m, 2H), 1.67-1.80 (m, 1H), 1.85 (dt, J=7.2 Hz, 8.0 Hz, 2H), 2.07-2.15 (m, 2H), 3.01-3.11 (m, 4H), 3.24-3.40 (m, 4H), 3.56 (br d, J=12.8 Hz, 2H), 4.10 (d, J=7.2 Hz, 2H), 4.62 (s, 2H), 4.64 (s, 2H), 7.22 (d, J=8.8 Hz, 1H), 7.48 (d, J=4.0 Hz, 1H), 7.80 (d, J=4.0 Hz, 1H), 7.91 (d, J=8.8 Hz, 1H).
ESI-MS m/z: 507 [M+H]⁺.

### Example 118

### 5-{4-{2-[7-(Azetidin-1-ylmethyl)-6-cyclopropylmethoxybenz[d]isoxazol-3-yl]ethyl}piperidinomethyl}thiophene-2-carbonitrile fumarate

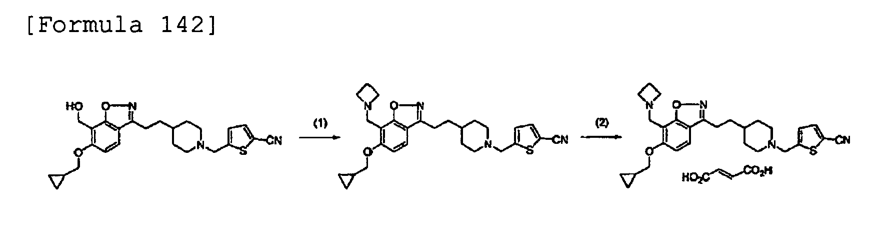

### (1) 5-{4-{2-[7-(Azetidin-1-ylmethyl)-6-cyclopropylmethoxybenz[d]isoxazol-3-yl]ethyl}piperidinomethyl}thiophene-2-carbonitrile

The title compound (28 mg) was obtained by synthesis from 5-{4-{2-[6-cyclopropylmethoxy-7-hydroxymethylbenz[d]isoxazol-3-yl]ethyl}piperidinomethyl}thiophene-2-carbonitrile (compound obtained in Example 115-(1)) (100 mg) and azetidine hydrochloride (1.2 g) according to Example 79-(6).
¹H-NMR (400 MHz, CDCl₃) δ (ppm) : 0.35-0.40 (m, 2H), 0.62-0.68 (m, 2H), 1.23-1.38 (m, 4H), 1.70-1.83 (m, 4H), 1.98-2.08 (m, 4H), 2.87-2.96 (m, 4H), 3.40 (br t, J=6.8 Hz, 4H), 3.68 (s, 2H), 3.92 (s, 2H), 3.93 (d, J=7.2 Hz, 2H), 6.87 (d, J=4.0 Hz, 1H), 6.88 (d, J=8.8 Hz, 1H), 7.43 (d, J=8.8 Hz, 1H), 7.46 (d, J=4.0 Hz, 1H).

### (2) 5-{4-{2-[7-(Azetidin-1-ylmethyl)-6-cyclopropylmethoxybenz[d]isoxazol-3-yl]ethyl}piperidinomethyl}thiophene-2-carbonitrile fumarate

The title compound (28 mg) was obtained by synthesis from 5-{4-{2-[7-(azetidin-1-ylmethyl)-6-cyclopropylmethoxybenz[d]isoxazol-3-yl]ethyl}piperidinomethyl}thiophene-2-carbonitrile (28 mg) according to Example 81-(5).
¹H-NMR (400 MHz, CD₃OD) δ(ppm): 0.42-0.46 (m, 2H), 0.67-0.73 (m, 2H), 1.25-1.44 (m, 4H), 1.75-1.87 (m, 4H), 2.15-2.23 (m, 2H), 2.50 (quintet, J=8.0 Hz, 2H), 2.98-3.05 (m, 4H), 3.87 (s, 2H), 4.10 (d, J=7.2 Hz, 2H), 4.26 (t, J=8.0 Hz, 4H), 4.67 (s, 2H), 6.69 (s, 2H), 7.09 (d, J=3.6 Hz, 1H), 7.20 (d, J=8.8 Hz, 1H), 7.63 (d, J=3.6 Hz, 1H), 7.85 (d, J=8.8 Hz, 1H).
ESI-MS m/z: 491 [M+H]⁺.

### Example 119

### 4-{3-[2-(1-Benzylpiperidin-4-yl)ethyl]-7-(N,N-dimethylaminomethyl)benz[d]isoxazol-5-yloxymethyl}benzonitrile dihydrochloride

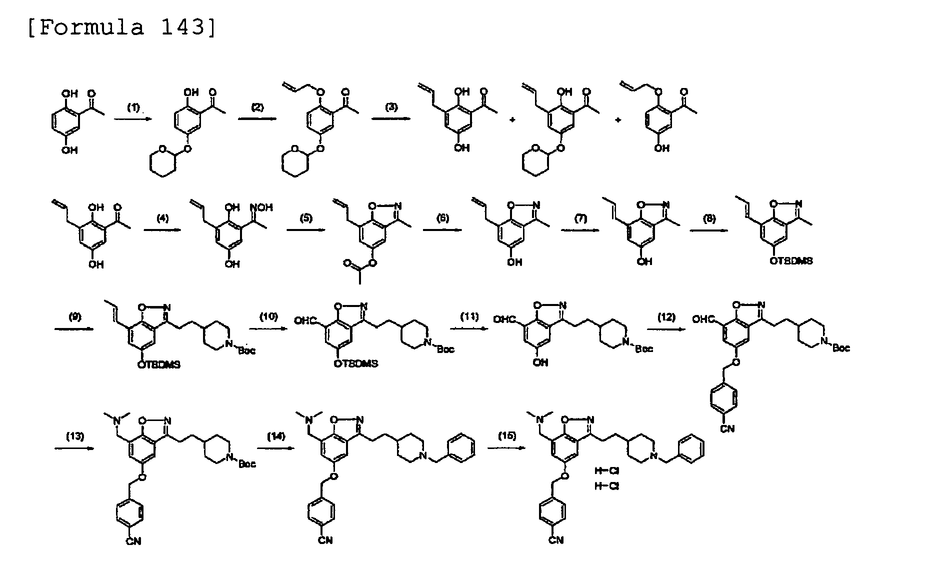

### (1) 1-[2-Hydroxy-5-(tetrahydro-2H-pyran-2-yloxy)phenyl]ethanone

2',5'-Dihydroxyacetophenone (5 g) was dissolved in 1,4-dioxane (100 mL) in a nitrogen atmosphere. p-Toluenesulfonic acid monohydrate (70 mg)
and 3,4-dihydro-2H-pyrane (6 mL) were sequentially added to the solution, and the mixture was stirred at room temperature for 15 hours. p-Toluenesulfonic acid monohydrate (70 mg) and 3,4-dihydro-2H-pyrane (1 mL) were further added to the mixture, followed by stirring at room temperature for four hours. Concentrated aqueous ammonia was added to the reaction mixture under ice-cooling and then water was added, followed by extraction with ethyl acetate. The organic layer was washed with water and a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (7.47 g). ¹H-NMR (400 MHz, CDCl₃) δ(ppm): 1.52-1.76 (m, 3H), 1.82-2.05 (m, 3H), 2.61 (s, 3H), 3.58-3.65 (m, 1H), 3.88-3.96 (m, 1H), 5.30 (dd, J=3.2 Hz, 3.2 Hz, 1H), 6.90 (d, J=8.8 Hz, 1H), 7.23 (dd, J=2.8 Hz, 8.8 Hz, 1H), 7.40 (d, J=2. Hz, 1H), 11.88 (s, 1H).

### (2) 1-[2-Allyloxy-5-(tetrahydro-2H-pyran-2-yloxy)phenyl]ethanone

The title compound (9.83 g) was obtained by synthesis from 1-[2-hydroxy-5-(tetrahydro-2H-pyran-2-yloxy)phenyl]ethanone (9.3 g) and allyl bromide (3.8 mL) according to Example 82-(3).
¹H-NMR (400 MHz, CDCl₃) δ(ppm): 1.54-1.72 (m, 3H), 1.80-2.03 (m, 3H), 2.63 (s, 3H), 3.56-3.62 (m, 1H), 3.86-3.94 (m, 1H), 4.58 (ddd, J=1.6 Hz, 1.6 Hz, 5.2 Hz, 2H), 5.30 (ddt, J=1.6 Hz, 1.6 Hz, 10.4 Hz, 1H), 5.33 (dd, J=3.2 Hz, 3.2 Hz, 1H), 5.41 (ddt, J=1.6 Hz, 1.6 Hz, 17.2 Hz, 1H), 6.06 (ddt, J=5.2 Hz, 10.4 Hz, 17.2 Hz, 1H), 6.86 (d, J=8.8 Hz, 1H), 7.14 (dd, J=3.2 Hz, 8.8 Hz, 1H), 7.42 (d, J=3.2 Hz, 1H).

### (3) 1-(3-Allyl-2,5-dihydroxyphenyl)ethanone, 1-[3-allyl-2-hydroxy-5-(tetrahydro-2H-pyran-2-yloxy)phenyl]ethanone and 1-(2-allyloxy-5-hydroxyphenyl)ethanone

A solution of 1-[2-allyloxy-5-(tetrahydro-2H-pyran-2-yloxy)phenyl]ethanone (9.8 g) in 1-methyl-2-pyrrolidinone (20 mL) was heated under microwave (220 W) irradiation at 180°C for 30 minutes. The reaction mixture was cooled to room temperature and then water was added, followed by extraction with ethyl acetate. The organic layer was sequentially washed with water (twice) and a saturated sodium chloride solution and dried over magnesium sulfate. The drying agent was removed by filtration and then the solvent was evaporated under reduced pressure. The residue was purified by silica gel column chromatography (heptane-ethyl acetate) and silica gel column chromatography (heptane-acetone) to obtain the title compounds, 1-(3-allyl-2,5-dihydroxyphenyl)ethanone (4 g), 1-[3-allyl-2-hydroxy-5-(tetrahydro-2H-pyran-2-yloxy)phenyl]ethanone (230 mg) and 1-(2-allyloxy-5-hydroxyphenyl)ethanone (680 mg), respectively.

### 1-(3-Allyl-2,5-dihydroxyphenyl)ethanone

¹H-NMR (400 MHz, CDCl₃) δ(ppm): 2.59 (s, 3H), 3.39 (d, J=6.4 Hz, 2H), 4.48 (s, 1H), 5.06-5.13 (m, 2H), 5.97 (ddt, J=6.4 Hz, 9.6 Hz, 17.6 Hz, 1H), 6.92 (d, J=3.2 Hz, 1H), 7.06 (d, J=3.2 Hz, 1H), 12.15 (s, 1H).

### 1-[3-Allyl-2-hydroxy-5-(tetrahydro-2H-pyran-2-yloxy)phenyl]ethanone

¹H-NMR (400 MHz, CDCl₃) δ (ppm) : 1.56-1.74 (m, 3H), 1.82-2.03 (m, 3H), 2.60 (s, 3H), 3.40 (d, J=6.8 Hz, 2H), 3.58-3.64 (m, 1H), 3.88-3.96 (m, 1H), 5.08 (d, J=10.4 Hz, 1H), 5.09 (d, J=16.4 Hz, 1H), 5.30 (dd, J=3.2 Hz, 3.2 Hz, 1H), 5.98 (ddd, J=6.8 Hz, 10.4 Hz, 16.4 Hz, 1H), 7.12 (d, J=3.2 Hz, 1H), 7.29 (d, J=3.2 Hz, 1H), 12.26 (s, 1H).

### 1-(2-Allyloxy-5-hydroxyphenyl)ethanone

¹H-NMR (400 MHz, CDCl₃) δ (ppm) : 2.64 (s, 3H), 4.58 (ddd, J=1.6 Hz, 1.6 Hz, 5.2 Hz, 2H), 5.03 (br s, 1H), 5.30 (ddt, J=1.6 Hz, 1.6 Hz, 10.4 Hz, 1H), 5.41 (ddt, J=1.6 Hz, 1.6 Hz, 17.2 Hz, 1H), 6.06 (ddd, J=5.2 Hz, 10.4 Hz, 17.2 Hz, 1H), 6.85 (d, J=8.8 Hz, 1H), 6.96 (dd, J=2.8 Hz, 8.8 Hz, 1H), 7.26 (d, J=2.8 Hz, 1H).

### (4) 1-(3-Allyl-2,5-dihydroxyphenyl)ethanone oxime

1-(3-Allyl-2,5-dihydroxyphenyl)ethanone (4.68 g) was dissolved in ethanol (50 mL). A solution of hydroxyammonium chloride (4 g) and sodium acetate (5 g) in water (15 mL) was added to the solution, and the mixture was heated under reflux for 1.5 hours. The reaction mixture was cooled to room temperature and then filtered through celite. The residue was washed with ethanol. The solvent of the filtrate was evaporated under reduced pressure. The residue was dissolved in ethyl acetate, sequentially washed with a saturated sodium bicarbonate solution, water and a saturated sodium chloride solution and dried over magnesium sulfate. The drying agent was removed by filtration and then the solvent was evaporated under reduced pressure to obtain the title compound (5.08 g). ¹H-NMR (400 MHz, CDCl₃) δ(ppm): 2.32 (s, 3H), 3.39 (d, J=6. Hz, 2H), 5.07 (dd, J=1.6 Hz, 16.8 Hz, 1H), 5.09 (dd, J=1.6 Hz, 10.0 Hz, 1H), 5.99 (ddd, J=6.8 Hz, 10.0 Hz, 16.8 Hz, 1H), 6.68 (d, J=2.8 Hz, 1H), 6.81 (d, J=2.8 Hz, 1H).

### (5) 7-Allyl-3-methylbenz[d]isoxazol-5-yl acetate

1-(3-Allyl-2,5-dihydroxyphenyl)ethanone oxime (5.08 g) and sodium acetate (5 g) were dissolved in N,N-dimethylformamide (50 mL). Acetic anhydride (6 mL) was added to the solution, and the mixture was stirred at 140°C for 1.5 hours. The reaction mixture was cooled to room temperature and then filtered through celite. The residue was washed with ethyl acetate. The solvent of the filtrate was evaporated under reduced pressure. The residue was dissolved in ethyl acetate, sequentially washed with water (twice) and a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (4.7 g).
¹H-NMR (400 MHz, CDCl₃) δ (ppm) : 2.33 (s, 3H), 2.55 (s, 3H), 3.67 (d, J=6.8 Hz, 2H), 5.16 (dd, J=1.6 Hz, 10.0 Hz, 1H), 5.19 (dd, J=1.6 Hz, 17.2 Hz, 1H), 6.02 (ddd, J=6.8 Hz, 10.0 Hz, 17.2 Hz, 1H), 7.05 (d, J=2.0 Hz, 1H), 7.20 (d, J=2.0 Hz, 1H).

### (6) 7-Allyl-3-methylbenz[d]isoxazol-5-ol

A mixture of 7-allyl-3-methylbenz[d]isoxazol-5-yl acetate (4.7 g), 5 M hydrochloric acid (8 mL) and methanol (50 mL) was heated under reflux for seven hours. The reaction mixture was cooled to room temperature and then water was added, followed by extraction with ethyl acetate. The organic layer was sequentially washed with water (three times) and a saturated sodium chloride solution and dried over magnesium sulfate. The organic layer was filtered through silica gel, and the solvent of the filtrate was evaporated under reduced pressure. The residue was dried under reduced pressure to obtain the title compound (3.58 g).
¹H-NMR (400 MHz, CDCl₃) δ(ppm): 2.52 (s, 3H), 3.63 (d, J=6.4 Hz, 2H), 4.88 (s, 1H), 5.14 (dd, J=1.6 Hz, 10.4 Hz, 1H), 5.17 (dd, J=1.6 Hz, 16.8 Hz, 1H), 6.03 (ddd, J=6.4 Hz, 10.4 Hz, 16.8 Hz, 1H), 6.83 (d, J=2.4 Hz, 1H), 6.90 (d, J=2.4 Hz, 1H).

### (7) 3-Methyl-7-[(E)-1-propenyl]benz[d]isoxazol-5-ol

A mixture of 7-allyl-3-methylbenz[d]isoxazol-5-ol (1.14 g), tert-butoxypotassium (2.1 g), tetrahydrofuran (15 mL) and N,N-dimethylformamide (5 mL) was stirred in a nitrogen atmosphere at 60°C for one hour. Ice was added to the reaction mixture, and then 5 M hydrochloric acid was added until the pH was 2.
The organic layer was extracted with ethyl acetate, sequentially washed with water (twice) and a saturated sodium chloride solution and dried over magnesium sulfate. The drying agent was removed by filtration and then the solvent was evaporated under reduced pressure. The resulting solid was suspended in a 3:2 mixed solution of heptane and diethyl ether and then collected by filtration to obtain the title compound (1.17 g).
¹H-NMR (400 MHz, CDCl₃) δ(ppm): 1.98 (d, J=6.8 Hz, 3H), 2.52 (s, 3H), 4.87 (br s, 1H), 6.50 (d, J=15.6 Hz, 1H), 6.79 (d, J=2.0 Hz, 1H), 6.82 (dq, J=6.8 Hz, 15.6 Hz, 1H), 6.93 (d, J=2.0 Hz, 1H).

### (8) 5-(tert-Butyldimethylsilanyloxy)-3-methyl-7-[(E)-1-propenyl]benz[d]isoxazole

3-Methyl-7-[(E)-1-propenyl]benz[d]isoxazol-5-ol (2.25 g) and imidazole (2 g) were dissolved in N,N-dimethylformamide (25 mL). tert-Butylchlorodimethylsilane (2.1 g) was added to the solution under ice-cooling, and the mixture was stirred at room temperature for one hour and 45 minutes. Water was added to the reaction mixture, followed by extraction with ethyl acetate. The organic layer was sequentially washed with water (three times) and a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (3.31 g).
¹H-NMR (400 MHz, CDCl₃) δ(ppm): 0.21 (s, 6H), 1.01 (s, 9H), 1.97 (dd, J=1.6 Hz, 6.8 Hz, 3H), 2.52 (s, 3H), 6.50 (dq, J=1.6 Hz, 16.0 Hz, 1H), 6.77 (d, J=2.4 Hz, 1H), 6.81 (dq, J=6.8 Hz, 16.0 Hz, 1H), 6.90 (d, J=2.4 Hz, 1H).

### (9) tert-Butyl 4-{2-[5-(tert-butyldimethylsilanyloxy)-7-[(E)-1-propenyl]benz[d]isoxazol-3-yl]ethyl}piperidine-1-carboxylate

Diisopropylamine (2 mL) was dissolved in tetrahydrofuran (15 mL) in a nitrogen atmosphere. n-Butyllithium (2.59 M solution in hexane) (5 mL) was added dropwise to the solution at -78°C. The mixture was stirred at -78°C for 27 minutes to prepare a solution of lithium diisopropylamide in tetrahydrofuran. The lithium diisopropylamide solution was added dropwise to a solution of 5-(tert-butyldimethylsilanyloxy)-3-methyl-7-[(E)-1-propenyl]benz[d]isoxazole (3.31 g) and tert-butyl 4-iodomethylpiperidine-1-carboxylate (compound obtained in Preparation Example 1-(2)) (3.9 g) in tetrahydrofuran (50 mL) at -70°C over 10 minutes. The mixture was stirred at -70°C for 1.5 hours. A saturated ammonium chloride solution was added to the reaction mixture, followed by heating to room temperature. The organic layer was extracted with ethyl acetate. The organic layer was sequentially washed with water (twice) and a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (4.73 g).
¹H-NMR (400 MHz, CDCl₃) δ(ppm): 0.21 (s, 6H), 1.01 (s, 9H), 1.08-1.22 (m, 2H), 1.44-1.54 (m, 1H), 1.45 (s, 9H), 1.71-1.81 (m, 4H), 1.97 (dd, J=1.6 Hz, 6.4 Hz, 3H), 2.67 (br d, J=12.0 Hz, 2H), 2.95 (t, J=8.0 Hz, 2H), 4.00-4.15 (m, 2H), 6.50 (dq, J=1.6 Hz, 16.0 Hz, 1H), 6.77 (d, J=2.4 Hz, 1H), 6.81 (dq, J=6.8 Hz, 16.0 Hz, 1H), 6.90 (d, J=2.4 Hz, 1H).

### (10) tert-Butyl 4-{2-[5-(tert-butyldimethylsilanyloxy)-7-formylbenz[d]isoxazol-3-yl]ethyl}piperidine-1-carboxylate

The title compound was synthesized with reference to Org. Lett., 2004, 6, 3217. tert-Butyl 4-{2-[5-(tert-butyldimethylsilanyloxy)-7-[(E)-1-propenyl]benz[d]isoxazol-3-yl]ethyl}piperidine-1-carboxylate (4.72 g) was dissolved in 1,4-dioxane (80 mL). Water (20 mL), 2,6-lutidine (2.2 mL), osmium tetroxide (2.5% solution in tert-butanol) (3.3 mL) and sodium metaperiodate (5.3 g) were sequentially added to the solution at room temperature, and the mixture was stirred at room temperature for two hours and 50 minutes. Water and 1 M hydrochloric acid were sequentially added to the reaction mixture, followed by extraction with ethyl acetate. The organic layer was sequentially washed with water (twice) and a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (4.38 g).
¹H-NMR (400 MHz, CDCl₃) δ(ppm): 0.24 (s, 6H), 1.02 (s, 9H), 1.18 (ddd, J=4.4 Hz, 12.4 Hz, 24.8 Hz, 2H), 1.45-1.55 (m, 1H), 1.46 (s, 9H), 1.72-1.83 (m, 4H), 2.68 (br d, J=12.4 Hz, 2H), 3.01 (t, J=8.0 Hz, 2H), 4.04-4.17 (m, 2H), 7.24 (d, J=2.0 Hz, 1H), 7.53 (d, J=2.0 Hz, 1H), 10.42 (s, 1H).

### (11) tert-Butyl 4-[2-(7-formyl-5-hydroxybenz[d]isoxazol-3-yl)ethyl]piperidine-1-carboxylate

The title compound (2.36 g) was obtained by synthesis from tert-butyl 4-{2-[5-(tert-butyldimethylsilanyloxy)-7-formylbenz[d]isoxazol-3-yl]ethyl}piperidine-1-carboxylate (3.3 g) according to Example 79-(5).
¹H-NMR (400 MHz, CDCl₃) δ(ppm): 1.17 (ddd, J=4.0 Hz, 12.0 Hz, 24.0 Hz, 2H), 1.46-1.54 (m, 1H), 1.48 (s, 9H), 1.72-1.83 (m, 4H), 2.71 (br t, J=11.6 Hz, 2H), 3.00 (t, J=8.0 Hz, 2H), 4.04-4.16 (m, 2H), 7.08 (s, 1H), 7.33 (d, J=2. 4 Hz, 1H), 7.61 (d, J=2.4 Hz, 1H), 10.42 (s, 1H).

### (12) tert-Butyl 4-{2-[5-(4-cyanobenzyloxy)-7-formylbenz[d]isoxazol-3-yl]ethyl}piperidine-1-carboxylate

The title compound (318 mg) was obtained by synthesis from tert-butyl 4-[2-(7-formyl-5-hydroxybenz[d]isoxazol-3-yl)ethyl]piperidine-1-carboxylate (250 mg) according to Example 874-(1).
¹H-NMR (400 MHz, CDCl₃) δ (ppm) : 1.18 (ddd, J=4.4 Hz, 12.4 Hz, 24.4 Hz, 2H), 1.45-1.53 (m, 1H), 1.46 (s, 9H), 1.71-1.83 (m, 4H), 2.62-2.74 (m, 2H), 3.02 (t, J=8.0 Hz, 2H), 4.05-4.16 (m, 2H), 5.22 (s, 2H), 7.37 (d, J=2.4 Hz, 1H), 7.58 (d, J=8.4 Hz, 2H), 7.71 (d, J=8.4 Hz, 2H), 7.72 (d, J=2.4 Hz, 1H), 10.47 (s, 1H) .

### (13) tert-Butyl 4-{2-[5-(4-cyanobenzyloxy)-7-(N,N-dimethylaminomethyl)benz[d]isoxazol-3-yl]ethyl}piperidine-1-carboxylate

The title compound (311 mg) was obtained by synthesis from tert-butyl 4-{2-[5-(4-cyanobenzyloxy)-7-formylbenz[d]isoxazol-3-yl]ethyl}piperidine-1-carboxylate (316 mg) and dimethylamine (2 M solution in tetrahydrofuran) (0.8 mL) according to Example 89-(1). ¹H-NMR (400 MHz, CDCl₃) δ(ppm): 1.16 (ddd, J=4.4 Hz, 12.4 Hz, 24.4 Hz, 2H), 1.46 (s, 9H), 1.49-1.56 (m, 1H), 1.70-1.82 (m, 4H), 2.37 (s, 6H), 2.62-2.75 (m, 2H), 2.96 (t, J=8.0 Hz, 2H), 3.80 (s, 2H), 4.04-4.16 (m, 2H), 5.18 (s, 2H), 6.95 (s, 1H), 7.28-7.42 (br s, 1H), 7.58 (d, J=8.4 Hz, 2H), 7.69 (d, J=8.4 Hz, 2H).

### (14) 4-{3-[2-(1-Benzylpiperidin-4-yl)ethyl]-7-(N,N-dimethylaminomethyl)benz[d]isoxazol-5-yloxymethyl}benzonitrile

4-{7-(N,N-Dimethylaminomethyl)-3-[2-(piperidin-4-yl)ethyl]benz[d]isoxazol-5-yloxymethyl}benzonitrile was synthesized from tert-butyl 4-{2-[5-(4-cyanobenzyloxy)-7-(N,N-dimethylaminomethyl)benz[d]isoxazol-3-yl]ethyl}piperidine-1-carboxylate (154 mg) according to Example 98-(2). The title compound (37 mg) was obtained by synthesis from the compound and benzaldehyde (80 mg) according to Example 89-(1).
¹H-NMR (400 MHz, CDCl₃) δ(ppm): 1.25-1.46 (m, 3H), 1.70-2.12 (m, 6H), 2.33 (s, 6H), 2.90-3.06 (m, 2H), 2.95 (t, J=7.6 Hz, 2H), 3.55 (s, 2H), 3.75 (s, 2H), 5.17 (s, 2H), 6.93 (d, J=2.0 Hz, 1H), 7.24-7.40 (m, 6H), 7.59 (d, J=8.8 Hz, 2H), 7.70 (d, J=8.8 Hz, 2H).

### (15) 4-{3-[2-(1-Benzylpiperidin-4-yl)ethyl]-7-(N,N-dimethylaminomethyl)benz[d]isoxazol-5-yloxymethyl}benzonitrile dihydrochloride

The title compound (45 mg) was obtained by synthesis from 4-{3-[2-(1-benzylpiperidin-4-yl)ethyl]-7-(N,N-dimethylaminomethyl)benz[d]isoxazol-5-yloxymethyl}benzonitrile (35 mg) according to Example 79-(8).
¹H-NMR (400 MHz, CD₃OD) δ(ppm): 1.52-1.64 (m, 2H), 1.70-1.82 (m, 1H), 1.88 (dt, J=7.2 Hz, 8.0 Hz, 2H), 2.08-2.16 (m, 2H), 2.98 (s, 6H), 3.05 (br t, J=12.0 Hz, 2H), 3.12 (t, J=8.0 Hz, 2H), 3.55 (br d, J=12.0 Hz, 2H), 4.35 (s, 2H), 4.68 (s, 2H), 5.36 (s, 2H), 7.51-7.62 (m, 7H), 7.73 (d, J=8.8 Hz, 2H), 7.81 (d, J=8.8 Hz, 2H).
ESI-MS m/z: 255 [M+2H]²⁺, 509 [M+H]⁺.

### Example 120

### N,N-Dimethyl{3-[2-(1-benzylpiperidin-4-yl)ethyl]-6-phenylbenz[d]isoxazol-7-yl}methylamine dihydrochloride

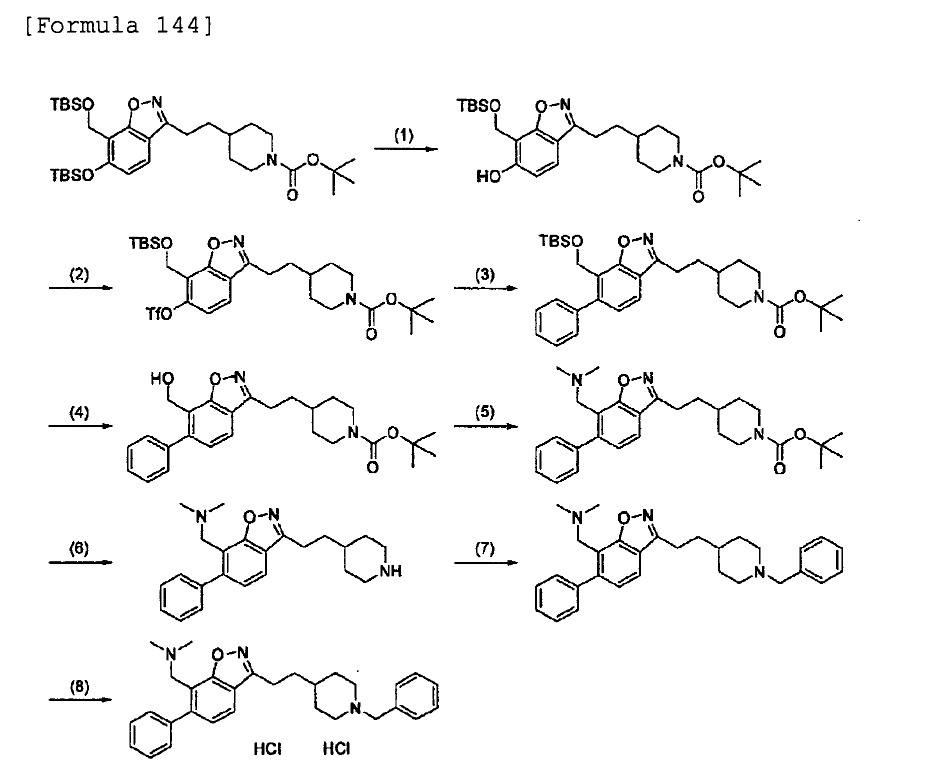

### (1) tert-Butyl 4-{2-[7-(tert-butyldimethylsilanyloxymethyl)-6-hydroxybenz[d]isoxazol-3-yl]ethyl}piperidine-1-carboxylate

tert-Butyl 4-{2-[6-(tert-butyldimethylsilanyloxy)-7-(tert-butyldimethylsilanyloxymethyl)benz[d]isoxazol-3-yl]ethyl}piperidine-1-carboxylate (compound synthesized in Preparation Example 2-(5)) (12.4 g) was dissolved in N,N-dimethylformamide (50 mL). Lithium hydroxide (0.6 g) was added to the solution, and the mixture was stirred at room temperature for 2.5 hours. A saturated ammonium chloride solution and water were sequentially added to the reaction mixture, followed by extraction with ethyl acetate twice. The organic layers were sequentially washed with water and a saturated sodium chloride solution and dried over magnesium sulfate. The drying agent was removed by filtration and then the solvent was evaporated under reduced pressure. The residue was purified by silica gel chromatography (heptane-ethyl acetate) to obtain the title compound (5.72 g) and tert-butyl 4-[2-(6-hydroxy-7-hydroxymethylbenz[d]isoxazol-3-yl)ethyl]piperidine-1-carboxylate (2.84 g), respectively.
¹H-NMR (400 MHz, CDCl₃) δ (ppm) : 0.20 (s, 6H), 0.96 (s, 9H), 1.15 (ddd, J=4.4 Hz, 12.4 Hz, 24.4 Hz, 2H), 1.44-1.56 (m, 1H), 1.47 (s, 9H), 1.69-1.81 (m, 4H), 2.67 (br t, J=12.4 Hz, 2H), 2.93 (t, J=8.0 Hz, 2H), 3.99-4.20 (m, 2H), 5.26 (s, 2H), 6.83 (d, J=8. Hz, 1H), 7.35 (d, J=8.4 Hz, 1H), 8.95 (s, 1H).

### (2) tert-Butyl 4-{2-[7-(tert-butyldimethylsilanyloxymethyl)-6-trifluoromethanesulfonyloxybenz[d]isoxazol-3-yl]ethyl}piperidine-1-carboxylate

tert-Butyl 4-{2-[7-(tert-butyldimethylsilanyloxymethyl)-6-hydroxybenz[d]isoxazol-3-yl]ethyl}piperidine-1-carboxylate (11.5 g) and pyridine (10 mL) were dissolved in dichloromethane (100 mL) in a nitrogen atmosphere. Trifluoromethanesulfonic anhydride (5 mL) was added dropwise to the solution under ice-cooling, and then the mixture was stirred at room temperature for 55 minutes. Concentrated aqueous ammonia was added to the reaction mixture until the pH was 7, followed by extraction with dichloromethane. The organic layer was sequentially washed with water and a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (14.55 g).
¹H-NMR (400 MHz, CDCl₃) δ (ppm) : 0.14 (s, 6H), 0.91 (s, 9H), 1.11-1.25 (m, 2H), 1.44-1.55 (m, 1H), 1.46 (s, 9H), 1.75 (br d, J=14.4 Hz, 2H), 1.81 (dt, J=7.2 Hz, 8.0 Hz, 2H), 2.69 (br t, J=12.4 Hz, 2H), 3.01 (t, J=8.0 Hz, 2H), 4.02-4.18 (m, 2H), 5.06 (s, 2H), 7.25 (d, J=8. 4 Hz, 1H), 7.60 (d, J=8.4 Hz, 1H).

### (3) tert-Butyl 4-{2-[7-(tert-butyldimethylsilanyloxymethyl)-6-phenylbenz[d]isoxazol-3-yl]ethyl}piperidine-1-carboxylate

tert-Butyl 4-{2-[7-(tert-butyldimethylsilanyloxymethyl)-6-trifluoromethanesulfonyloxybenz[d]isoxazol-3-yl]ethyl}piperidine-1-carboxylate (2.4 g), phenyltributyltin (1.84 g) and lithium chloride (245 mg) were dissolved in N-methyl-2-pyrrolidone (30 mL). Dichlorobis(triphenylphosphine)palladium (II) (135 mg) was added and the mixture was heated with stirring at 80°C for 2.5 hours. Water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure. The residue was purified by silica gel column chromatography (heptane-ethyl acetate) to obtain the title compound (2.3 g).
¹H-NMR (400 MHz, CDCl₃) δ(ppm): 0.10 (s, 6H), 0.90 (s, 9H), 1.10-1.24 (m, 2H), 1.46 (s, 9H), 1.48-1.60 (m, 1H), 1.72-1.88 (m, 4H), 2.63-2.77 (m, 2H), 3.04 (t, J=7.6 Hz, 2H), 4.03-4.19 (m, 2H), 4.84 (s, 2H), 7.30 (d, J=8.0 Hz, 1H), 7.40-7.48 (m, 3H), 7.53-7.61 (m, 3H).

### (4) tert-Butyl 4-[2-(7-hydroxymethyl-6-phenylbenz[d]isoxazol-3-yl)ethyl]piperidine-1-carboxylate

tert-Butyl 4-{2-[7-(tert-butyldimethylsilanyloxymethyl)-6-phenylbenz[d]isoxazol-3-yl]ethyl}piperidine-1-carboxylate (2.3 g) was dissolved in tetrahydrofuran (30 mL). Tetrabutylammonium fluoride (1.0 M solution in tetrahydrofuran) (5.5 mL) was added at room temperature, and the mixture was stirred at room temperature overnight. A saturated ammonium chloride solution, water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure. The residue was purified by silica gel column chromatography (heptane-ethyl acetate) to obtain the title compound (1.6 g).
¹H-NMR (400 MHz, CDCl₃) δ(ppm): 1.16-1.26 (m, 2H), 1.46 (s, 9H), 1.48-1.58 (m, 1H), 1.74-1.86 (m, 4H), 2.17 (t, J=7.6 Hz, 1H), 2.64-2.74 (m, 2H), 3.06 (t, J=7.6 Hz, 2H), 4.06-4.18 (m, 2H), 4.93 (d, J=6.4 Hz, 2H), 7.31 (d, J=8.2 Hz, 1H), 7.41-7.49 (m, 5H), 7.61 (d, J=8.2 Hz, 1H).

### (5) tert-Butyl 4-{2-[7-(N,N-dimethylaminomethyl)-6-phenylbenz[d]isoxazol-3-yl]ethyl}piperidine-1-carboxylate

tert-Butyl 4-[2-(7-hydroxymethyl-6-phenylbenz[d]isoxazol-3-yl)ethyl]piperidine-1-carboxylate (1.6 g) and triethylamine (0.41 mL) were dissolved in tetrahydrofuran (30 mL). Methanesulfonyl chloride (1 mL) was added to the solution under ice-cooling, and the mixture was stirred for one hour. Dimethylamine (2 M solution in tetrahydrofuran) (18 mL) and sodium iodide (53 mg) were added to the mixture, followed by further stirring at room temperature for three hours. A saturated sodium bicarbonate solution, water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure. The residue was purified by NH silica gel column chromatography (heptane-ethyl acetate) to obtain the title compound (1.7 g).
¹H-NMR (400 MHz, CDCl₃) δ(ppm): 1.14-1.26 (m, 2H), 1.46 (s, 9H), 1.48-1.62 (m, 1H), 1.74-1.86 (m, 4H), 2.18 (s, 6H), 2.65-2.75 (m, 2H), 3.04 (t, J=7.6 Hz, 2H), 3.65 (s, 2H), 4.05-4.18 (m, 2H), 7.28 (d, J=8.4 Hz, 1H), 7.38-7.46 (m, 3H), 7.50-7.60 (m, 3H).
ESI-MS m/z: 464 [M+H]⁺, 486 [M+Na]⁺.

### (6) N,N-Dimethyl{6-phenyl-3-[2-(piperidin-4-yl)ethyl]benz[d]isoxazol-7-yl}methylamine

tert-Butyl 4-{2-[7-(N,N-dimethylaminomethyl)-6-phenylbenz[d]isoxazol-3-yl]ethyl}-piperidine-1-carboxylate (1.7 g) was dissolved in methanol (40 mL). Hydrogen chloride (4 M solution in ethyl acetate) (9.2 mL) was added to the solution, and the mixture was stirred at room temperature overnight. A saturated sodium carbonate solution, water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain the title compound (1.3 g).
ESI-MS m/z: 364 [M+H]⁺.

### (7) N,N-Dimethyl{3-[2-(1-benzylpiperidin-4-yl)ethyl]-6-phenylbenz[d]isoxazol-7-yl}methylamine

N,N-Dimethyl{6-phenyl-3-[2-(piperidin-4-yl)ethyl]benz[d]isoxazol-7-yl}methylamine (120 mg) and benzaldehyde (40 µL) were dissolved in dichloromethane (5 mL). Acetic acid (38 µL) and sodium triacetoxyborohydride (105 mg) were sequentially added to the solution, and the mixture was stirred at room temperature overnight. A saturated sodium carbonate solution, water and dichloromethane were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure. The residue was purified by NH silica gel column chromatography (heptane-ethyl acetate) to obtain the title compound (120 mg).
¹H-NMR (400 MHz, CDCl₃)
δ(ppm): 1.27-1.48 (m, 3H), 1.76-1.86 (m, 4H), 1.92-2.00 (m, 2H), 2.18 (s, 6H), 2.86-2.94 (m, 2H), 2.99-3.03 (m, 2H), 3.49 (s, 2H), 3.64 (s, 2H), 7.22-7.28 (m, 3H), 7.28-7.36 (m, 3H), 7.38-7.48 (m, 3H), 7.52-7.60 (m, 3H).
ESI-MS m/z: 454 [M+H]⁺.

### (8) N,N-Dimethyl{3-[2-(1-benzylpiperidin-4-yl)ethyl]-6-phenylbenz[d]isoxazol-7-yl}methylamine dihydrochloride

N,N-Dimethyl{3-[2-(1-benzylpiperidin-4-yl)ethyl]-6-phenylbenz[d]isoxazol-7-yl}methylamine (120 mg) was dissolved in methanol (3 mL). Hydrogen chloride (4 M solution in ethyl acetate) (0.5 mL) was added to the solution, and the mixture was stirred at room temperature. The solvent was evaporated under reduced pressure, and then the residue was dried under reduced pressure to obtain the title compound (130 mg). ¹H-NMR (400 MHz, DMSO-d₆) δ(ppm) : 1.58-1.69 (m, 3H), 1.71-1.83 (m, 2H), 1.88-2.00 (m, 2H), 2.50-2.55 (m, 6H), 2.83-2.95 (m, 2H), 3.09 (t, J=7.6 Hz, 2H), 3.27-3.33 (m, 2H), 4.23-4.25 (m, 2H), 4.54-4.57 (m, 2H), 7.39 (d, J=8.0 Hz, 1H), 7.40-7.57 (m, 8H), 7.60-7.66 (m, 2H), 8.07 (d, J=8.0 Hz, 1H), 10.29 (br s, 1H), 10.85 (br, 1H).
ESI-MS m/z: 454 [M+H]⁺.

### Example 121

### N,N-Dimethyl{3-[2-(1-benzylpiperidin-4-yl)ethyl]-6-(pyridin-3-yl)benz[d]isoxazol-7-yl}methylamine trihydrochloride

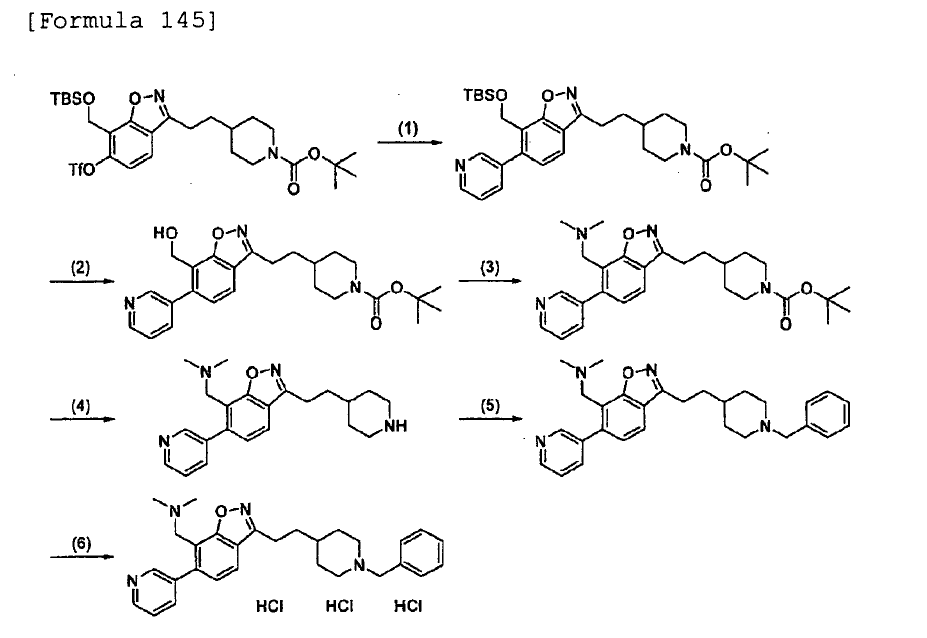

### (1) tert-Butyl 4-{2-[7-(tert-butyldimethylsilanyloxymethyl)-6-(pyridin-3-yl)benz[d]isoxazol-3-yl]ethyl}piperidine-1-carboxylate

The title compound was synthesized from tert-butyl 4-{2-[7-(tert-butyldimethylsilanyloxymethyl)-6-trifluoromethanesulfonyloxybenz[d]isoxazol-3-yl]ethyl}piperidine-1-carboxylate (compound synthesized in Example 120-(2)) and (3-pyridyl)tributyltin according to Example 120-(3).
¹H-NMR (400 MHz, CDCl₃) δ(ppm): 0.10 (s, 6H), 0.90 (s, 9H), 1.16-1.30 (m, 2H), 1.46 (s, 9H), 1.48-1.62 (m, 1H), 1.74-1.88 (m, 4H), 2.64-2.76 (m, 2H), 3.05 (t, J=8.0 Hz, 2H), 4.07-4.17 (m, 2H), 4.84 (s, 2H), 7.28 (d, J=8.2 Hz, 2H), 7.39 (ddd, J=0.8, 4.8, 8.0 Hz, 1H), 7.64 (d, J=8.2 Hz, 1H), 7.95 (ddd, J=2.0, 2.0, 8.0 Hz, 1H), 8.67 (dd, J=2.0, 4.8 Hz, 1H), 8.78 (dd, J=0.8, 2.0 Hz, 1H).

### (2) tert-Butyl 4-{2-[7-hydroxymethyl-6-(pyridin-3-yl)benz[d]isoxazol-3-yl]ethyl}piperidine-1-carboxylate

The title compound was synthesized from tert-butyl 4-{2-[7-(tert-butyldimethylsilanyloxymethyl)-6-(pyridin-3-yl)benz[d]isoxazol-3-yl]ethyl}piperidine-1-carboxylate according to Example 120-(4).
¹H-NMR (400 MHz, CDCl₃) δ(ppm) : 1.16-1.26 (m, 2H), 1.46 (s, 9H), 1.48-1.60 (m, 1H), 1.74-1.86 (m, 4H), 2.38 (t, J=6.0 Hz, 1H), 2.65-2.75 (m, 2H), 3.07 (t, J=7.8 Hz, 2H), 4.08-4.18 (m, 2H), 4.91 (d, J=6.0 Hz, 2H), 7.30 (d, J=8.0 Hz, 1H), 7.43 (ddd, J=0.8, 4.8, 8.0 Hz, 1H), 7.67 (d, J=8.0 Hz, 1H), 7.89 (ddd, J=2.0, 2.0, 8.0 Hz, 1H), 8.69 (dd, J=2.0, 4.8 Hz, 1H), 8.74 (dd, J=0.8, 2.0 Hz, 1H).

### (3) tert-Butyl 4-{2-[7-(N,N-dimethylaminomethyl)-6-(pyridin-3-yl)benz[d]isoxazol-3-yl]ethyl}piperidine-1-carboxylate

The title compound was synthesized from tert-butyl 4-{2-[7-hydroxymethyl-6-(pyridin-3-yl)benz[d]isoxazol-3-yl]ethyl}piperidine-1-carboxylate according to Example 120-(5).
¹H-NMR (400 MHz, CDCl₃) δ(ppm): 1.14-1.28 (m, 2H), 1.46 (s, 9H), 1.30-1.45 (m, 1H), 1.74-1.86 (m, 4H), 2.20 (s, 6H), 2.67-2.77 (m, 2H), 3.05 (t, J=8.0 Hz, 2H), 3.56 (s, 2H), 4.06-4.18 (m, 2H), 7.27 (d, J=8.4 Hz, 1H), 7.39 (ddd, J=0.8, 4.8, 8.0 Hz, 1H), 7.62 (d, J=8.4 Hz, 1H), 8.02 (ddd, J=1.6, 2.0, 8.0 Hz, 1H), 8.65 (dd, J=2.4, 4.8 Hz, 1H), 8.77 (dd, J=0.8, 2.4 Hz, 1H).
ESI-MS m/z: 465 [M+H]⁺, 487 [M+Na]⁺.

### (4) N,N-Dimethyl{3-[2-(piperidin-4-yl)ethyl]-6-(pyridin-3-yl)benz[d]isoxazol-7-yl}methylamine

The title compound was synthesized from tert-butyl 4-{2-[7-(N,N-dimethylaminomethyl)-6-(pyridin-3-yl)benz[d]isoxazol-3-yl]ethyl}piperidine-1-carboxylate according to Example 120-(6).
ESI-MS m/z: 183 [M+2H]²⁺, 365 [M+H]⁺.

### (5) N,N-Dimethyl{3-[2-(1-benzylpiperidin-4-yl)ethyl]-6-(pyridin-3-yl)benz[d]isoxazol-7-yl}methylamine

The title compound was synthesized from N,N-dimethyl{3-[2-(piperidin-4-yl)ethyl]-6-(pyridin-3-yl)benz[d]isoxazol-7-yl}methylamine according to Example 120-(7).
¹H-NMR (400 MHz, CDCl₃) δ(ppm): 1.28-1.44 (m, 3H), 1.74-1.86 (m, 4H), 1.92-2.00 (m, 2H), 2.19 (s, 6H), 2.86-2.94 (m, 2H), 3.00-3.06 (m, 2H), 3.50 (s, 2H), 3.58 (s, 2H), 7.22-7.28 (m, 3H), 7.28-7.34 (m, 3H), 7.36-7.42 (m, 1H), 7.62 (d, 1H, J=8.0 Hz), 8.01-8.03 (m, 1H), 8.64-8.66 (m, 1H), 8.76-8.77 (m, 1H).
ESI-MS m/z: 455 [M+H]⁺.

### (6) N,N-Dimethyl {3-[2-(1-benzylpiperidin-4-yl)ethyl]-6-(pyridin-3-yl)benz[d]isoxazol-7-yl}methylamine trihydrochloride

The title compound was synthesized from N,N-dimethyl{3-[2-(1-benzylpiperidin-4-yl)ethyl]-6-(pyridin-3-yl)benz[d]isoxazol-7-yl}methylamine according to Example 120-(8).
¹H-NMR (400 MHz, DMSO-d₆) δ (ppm) : 1.61-1.73 (m, 3H), 1.75-1.83 (m, 2H), 1.89-2.00 (m, 2H), 2.60-2.61 (m, 6H), 2.82-2.94 (m, 2H), 3.06-3.15 (m, 2H), 3.23-3.35 (m, 2H), 4.25 (d, J=5.2 Hz, 2H), 4.57-4.60 (m, 2H), 7.44-7.48 (m, 3H), 7.46 (d, J=8.2 Hz, 1H), 7.63-7.69 (m, 2H), 7.98-8.03 (m, 1H), 8.18 (d, J=8.2 Hz, 1H), 8.52-8.58 (m, 1H), 8.96 (d, J=5.6 Hz, 1H), 9.04 (s, 1H), 10.57 (br s, 1H), 10.98 (br s, 1H).
ESI-MS m/z: 228 [M+2H]²⁺, 455 [M+H]⁺.

### Example 122

### N,N-Dimethyl{6-phenyl-3-{2-[1-(pyridin-2-ylmethyl)piperidin-4-yl]ethyl}benz[d]isoxazol-7-yl}methylamine trihydrochloride

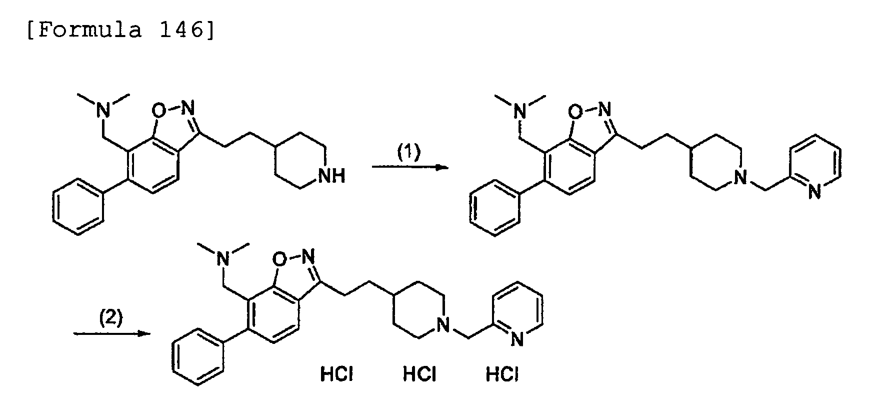

### (1) N,N-Dimethyl{6-phenyl-3-{2-[1-(pyridin-2-ylmethyl)piperidin-4-yl]ethyl}benz[d]isoxazol-7-yl}methylamine

The title compound was synthesized from N,N-dimethyl{6-phenyl-3-[2-(piperidin-4-yl)ethyl]benz[d]isoxazol-7-yl}methylamine (compound synthesized in Example 120-(6)) and 2-pyridinecarbaldehyde according to Example 120-(7).
¹H-NMR (400 MHz, CDCl₃) δ(ppm): 1.35-1.48 (m, 3H), 1.74-1.88 (m, 4H), 2.02-2.14 (m, 2H), 2.18 (s, 6H), 2.86-2.98 (m, 2H), 3.00-3.04 (m, 2H), 3.64 (s, 2H), 3.64 (s, 2H), 7.12-7.20 (m, 1H), 7.24-7.28 (m, 2H), 7.37-7.47 (m, 3H), 7.50-7.58 (m, 3H), 7.63-7.67 (m, 1H), 8.55-8.57 (m, 1H).
ESI-MS m/z: 455 [M+H]⁺, 477 [M+Na]⁺.

### (2) N,N-Dimethyl {6-phenyl-3-{2-[1-(pyridin-2-ylmethyl)piperidin-4-yl]ethyl}benz[d]isoxazol-7-yl}methylamine trihydrochloride

The title compound was synthesized from N,N-dimethyl{6-phenyl-3-{2-[1-(pyridin-2-ylmethyl)piperidin-4-yl]ethyl}benz[d]isoxazol-7-yl}methylamine according to Example 120-(8).
¹H-NMR (400 MHz, DMSO-d₆) δ(ppm): 1.60-1.75 (m, 3H), 1.77-1.84 (m, 2H), 1.89-2.00 (m, 2H), 2.51-2.53 (m, 6H), 3.00-3.13 (m, 4H), 3.37-3.45 (m, 2H), 4.46 (s, 2H), 4.56 (d, J=4.4 Hz, 2H), 7.41 (d, J=8.0 Hz, 1H), 7.51-7.56 (m, 6H), 7.79 (d, J=8.0 Hz, 1H), 7.96 (ddd, J=1.6, 7.6, 8.0 Hz, 1H), 8.09 (d, J=8.0 Hz, 1H), 8.69 (ddd, J=0.8, 1.6, 4.8 Hz, 1H), 10.58 (br s, 1H), 10.83 (br s, 1H).

### Example 123

### N,N-Dimethyl{6-phenyl-3-{2-[1-(thiophen-2-ylmethyl)piperidin-4-yl]ethyl}benz[d]isoxazol-7-yl}methylamine dihydrochloride

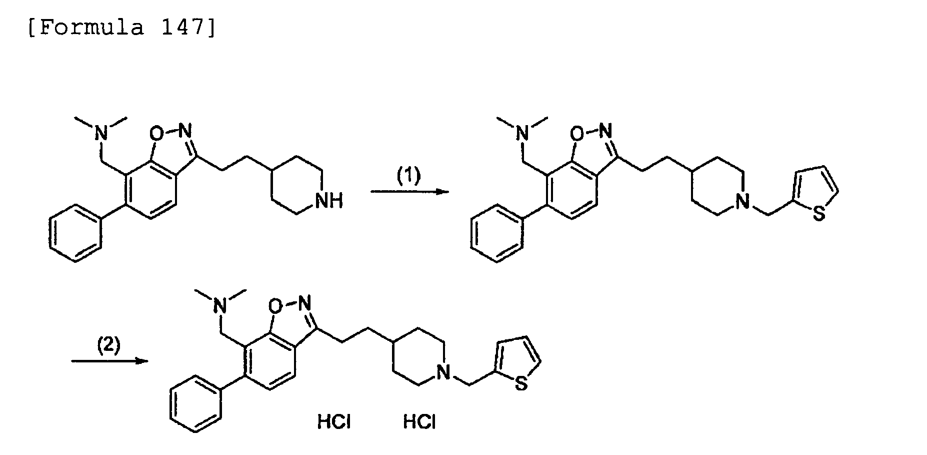

### (1) N,N-Dimethyl{6-phenyl-3-{2-[1-(thiophen-2-ylmethyl)piperidin-4-yl]ethyl}benz[d]isoxazol-7-yl}methylamine

The title compound was synthesized from N,N-dimethyl{6-phenyl-3-[2-(piperidin-4-yl)ethyl]benz[d]isoxazol-7-yl}methylamine (compound synthesized in Example 120-(6)) and 2-thiophenecarbaldehyde according to Example 120-(7).
¹H-NMR (400 MHz, CDCl₃) δ(ppm): 1.28-1.42 (m, 3H), 1.76-1.86 (m, 4H), 1.96-2.06 (m, 2H), 2.18 (s, 6H), 2.92-3.00 (m, 2H), 3.00-3.04 (m, 2H), 3.64 (s, 2H), 3.72 (s, 2H), 6.86-6.90 (m, 1H), 6.93-6.96 (m, 1H), 7.21-7.23 (m, 1H), 7.25-7.28 (m, 1H), 7.38-7.48 (m, 3H), 7.51-7.57 (m, 3H).
ESI-MS m/z: 460 [M+H]⁺, 482 [M+Na]⁺.

### (2) N,N-Dimethyl{6-phenyl-3-{2-[1-(thiophen-2-ylmethyl)piperidin-4-yl]ethyl}benz[d]isoxazol-7-yl}methylamine dihydrochloride

The title compound was synthesized from N,N-dimethyl{6-phenyl-3-{2-[1-(thiophen-2-yl)methylpiperidin-4-yl]ethyl}benz[d]isoxazol-7-yl}methylamine according to Example 120-(8).
¹H-NMR (400 MHz, DMSO-d₆) δ(ppm): 1.56-1.64 (m, 3H), 1.70-1.84 (m, 2H), 1.88-2.02 (m, 2H), 2.53-2.56 (m, 6H), 2.80-2.97 (m, 2H), 3.06-3.16 (m, 2H), 3.34-3.38 (m, 2H), 4.49 (d, J=5.2 Hz, 2H), 4.55-4.58 (m, 2H), 7.14 (dd, J=3.6, 4.8 Hz, 1H), 7.38-7.46 (m, 2H), 7.48-7.60 (m, 5H), 7.70 (d, J=4.8 Hz, 1H), 8.08 (d, J=8.0 Hz, 1H), 10.30 (br s, 1H), 10.92 (br s, 1H).
ESI-MS m/z: 460 [M+H]⁺.

### Example 124

### N,N-Dimethyl{3-{2-[1-(1,3-dioxolan-2-ylmethyl)piperidin-4-yl]ethyl}-6-phenylbenz[d]isoxazol-7-yl}methylamine fumarate

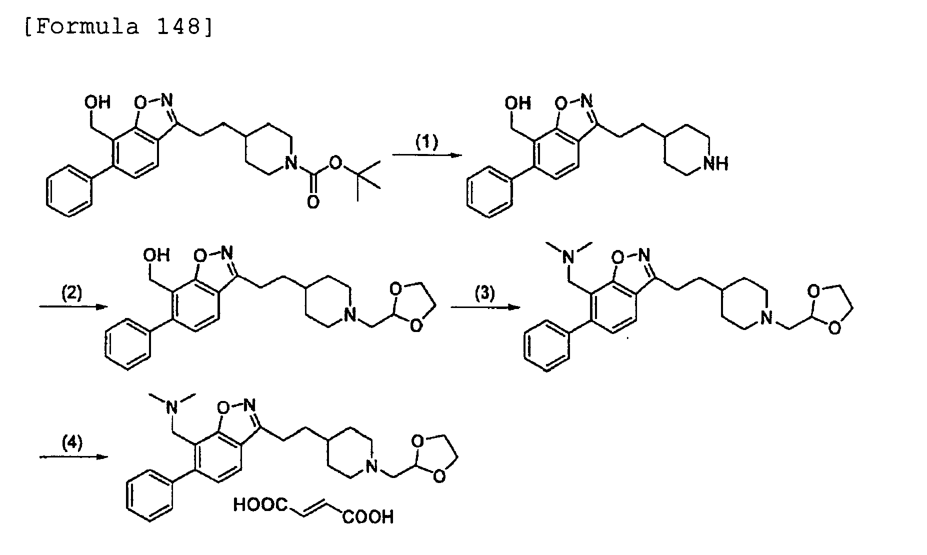

### (1) {6-Phenyl-3-[2-(piperidin-4-yl)ethyl]benz[d]isoxazol-7-yl}methanol

The title compound was synthesized from tert-butyl 4-[2-(7-hydroxymethyl-6-phenylbenz[d]isoxazol-3-yl)ethyl]piperidine-1-carboxylate (compound synthesized in Example 120-(4)) according to Example 120-(6).
¹H-NMR (400 MHz, DMSO-d₆) δ(ppm): 1.20-1.32 (m, 2H), 1.34-1.42 (m, 1H), 1.65-1.78 (m, 4H), 2.34-2.46 (m, 2H), 2.88-2.94 (m, 2H), 3.03 (t, J=8.0 Hz, 2H), 4.62 (d, J=8.0 Hz, 2H), 5.36 (t, J=8.0 Hz, 1H), 7.32 (d, J=8.0 Hz, 1H), 7.32-7.59 (m, 5H), 7.85 (d, J=8.0 Hz, 1H).

### (2) {3-{2-[1-(1,3-Dioxolan-2-ylmethyl)piperidin-4-yl]ethyl}-6-phenylbenz[d]isoxazol-7-yl}methanol

{6-Phenyl-3-[2-(piperidin-4-yl)ethyl]benz[d]isoxazol-7-yl}methanol (216 mg) and 2-bromomethyl-1,3-dioxolane (0.2 mL) were dissolved in N,N-dimethylformamide (10 mL). Potassium carbonate (266 mg) was added to the solution, and the mixture was heated with stirring in a nitrogen atmosphere at 90°C overnight. Water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure. The residue was purified by NH silica gel column chromatography (heptane-ethyl acetate) to obtain the title compound (228 mg).
¹H-NMR (400 MHz, CDCl₃) δ(ppm) : 1.33-1.46 (m, 3H), 1.73-1.85 (m, 4H), 2.03-2.11 (m, 2H), 2.57 (d, J=4.8 Hz, 2H), 3.00-3.08 (m, 4H), 3.83-3.88 (m, 2H), 3.94-4.00 (m, 2H), 4.92 (m, 2H), 5.02 (t, J=4.8 Hz, 1H), 7.30 (d, =8.0 Hz, 1H), 7.40-7.50 (m, 5H), 7.60 (d, J=8.0 Hz, 1H).

### (3) N,N-Dimethyl{3-{2-[1-(1,3-dioxolan-2-ylmethyl)piperidin-4-yl]ethyl}-6-phenylbenz[d]isoxazol-7-yl}methylamine

The title compound was synthesized from 3-{2-[1-(1,3-dioxolan-2-ylmethyl)piperidin-4-yl]ethyl}-6-phenylbenz[d]isoxazol-7-yl}methanol according to Example 120- (5) .
¹H-NMR (400 MHz, CDCl₃) δ (ppm) 1.34-1.45 (m, 3H), 1.72-1.84 (m, 4H), 2.04-2.12 (m, 2H), 2.18 (s, 6H), 2.57 (d, d=4.4 Hz, 2H), 2.98-3.04 (m, 4H), 3.64 (s, 2H), 3.82-3.88 (m, 2H), 3.92-3.98 (m, 2H), 5.01 (t, J=4.4 Hz, 1H), 7.24-7.28 (m, 1H), 7.38-7.46 (m, 3H), 7.50-7.58 (m, 3H).
ESI-MS m/z: 450 [M+H]⁺, 472 [M+Na]⁺.

### (4) N,N-Dimethyl{3-[2-(1,3-dioxolan-2-ylmethyl)piperidin-4-yl)ethyl]-6-phenylbenz[d]isoxazol-7-yl}methylamine fumarate

N,N-Dimethyl{3-{2-[1-(1,3-dioxolan-2-ylmethyl)piperidin-4-yl]ethyl}-6-phenylbenz[d]isoxazol-7-yl}methylamine (250 mg) was dissolved in methanol (2 mL). A solution of fumaric acid (65 mg) in methanol (1 mL) was added to the solution, and the mixture was stirred at room temperature. The solvent was evaporated under reduced pressure, and then the residue was dried under reduced pressure to obtain the title compound (230 mg).
¹H-NMR (400 MHz, DMSO-d₆) δ(ppm): 1.19-1.36 (m, 3H), 1.69-1.75 (m, 4H), 2.03-2.12 (m, 2H), 2.50-2.54 (m, 6H), 2.94-3.05 (m, 4H), 3.17 (s, 2H), 3.56 (s, 2H), 3.73-3.80 (m, 2H), 3.82-3.90 (m, 2H), 4.91 (t, J=7.2 Hz, 1H), 6.61 (s, 2H), 7.31 (d, J=8.6 Hz, 1H), 7.42-7.52 (m, 3H), 7.54-7.58 (m, 2H), 7.85 (d, J=8.6 Hz, 1H).
ESI-MS m/z: 225 [M+2H]²⁺, 450 [M+H]⁺.

### Example 125

### N,N-Dimethyl{3-{2-[1-(1,3-dioxolan-2-ylmethyl)piperidin-4-yl]ethyl}-6-(pyridin-3-yl)benz[d]isoxazol-7-yl}methylamine fumarate

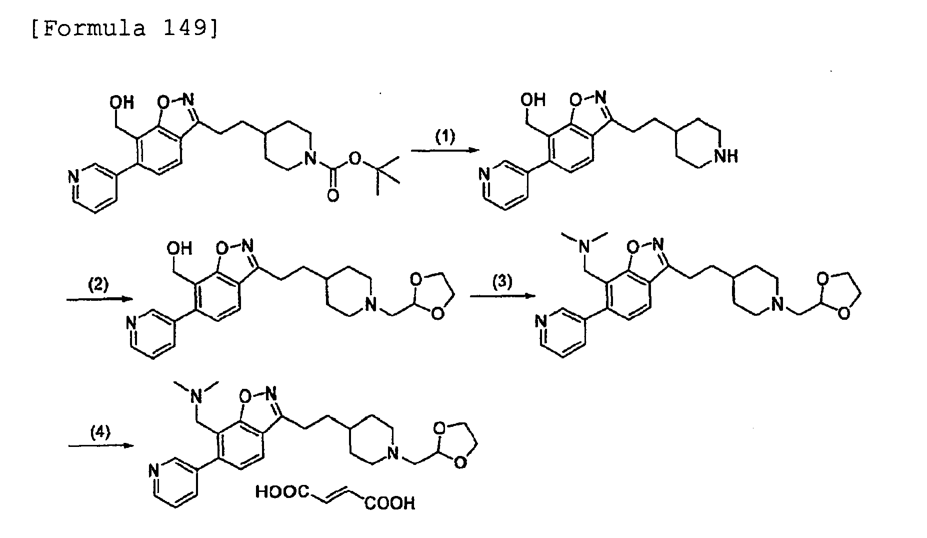

### (1) {3-[2-(Piperidin-4-yl)ethyl]-6-(pyridin-3-yl)benz[d]isoxazol-7-yl}methanol

The title compound was synthesized from tert-butyl 4-{2-[7-hydroxymethyl-6-(pyridin-3-yl)benz[d]isoxazol-3-yl]ethyl}piperidine-1-carboxylate (compound synthesized in Example 121-(2)) according to Example 120-(6).
¹H-NMR (400 MHz, DMSO-d₆) δ(ppm) : 1.00-1.12 (m, 2H), 1.32-1.42 (m, 1H), 1.64-1.76 (m, 4H), 2.39 (dt, J=2.0, 12 Hz, 2H), 2.86-2.95 (m, 2H), 3.04 (t, J=8.0 Hz, 2H), 4.62 (d, J=4.8 Hz, 2H), 5.45 (t, J=4.8 Hz, 1H), 7.38 (d, J=8.0 Hz, 1H), 7.54 (dd, J=5.2, 8.0 Hz, 1H), 7.91 (d, J=8.0 Hz, 1H), 8.00 (d, J=8.0 Hz, 1H), 8.66 (dd, J=2.0, 5.2 Hz, 1H), 8.76 (d, J=2.0 Hz, 1H).

### (2) {3-{2-[1-(1,3-Dioxolan-2-ylmethyl)piperidin-4-yl]ethyl}-6-(pyridin-3-yl)benz[d]isoxazol-7-yl}methanol

The title compound was synthesized from {3-[2-(piperidin-4-yl)ethyl]-6-(pyridin-3-yl)benz[d]isoxazol-7-yl}methanol according to Example 124-(2).
¹H-NMR (400 MHz, CDCl₃) δ (ppm) : 1.32-1.46 (m, 3H), 1.72-1.86 (m, 4H), 2.04-2.10 (m, 2H), 2.57 (d, J=4.4 Hz, 2H), 2.98-3.08 (m, 4H), 3.84-3.88 (m, 2H), 3.94-4.00 (m, 2H), 4.90 (s, 2H), 5.01 (t, J=4.4 Hz, 1H), 7.29 (d, J=8.4 Hz, 1H), 7.43 (ddd, J=0.8, 4.8, 8.0 Hz, 1H), 7.66 (d, J=8.4 Hz, 1H), 8.90 (ddd, J=1.6, 2.4, 8.0 Hz, 1H), 8.68 (dd, J=1.6, 4.8 Hz, 1H), 8.74 (dd, J=0.8, 2.4 Hz, 1H).
ESI-MS m/z: 424 [M+H]⁺.

### (3) N,N-Dimethyl{3-{2-[1-(1,3-dioxolan-2-ylmethyl)piperidin-4-yl]ethyl}-6-(pyridin-3-yl)benz[d]isoxazol-7-yl}methylamine

The title compound was synthesized from {3-{2-[1-(1,3-dioxolan-2-ylmethyl)piperidin-4-yl]ethyl}-6-(pyridin-3-yl)benz[d]isoxazol-7-yl}methanol according to Example 124-(3).
¹H-NMR (400 MHz, CDCl₃) δ (ppm) : 1.36-1.46 (m, 3H), 1.74-1.86 (m, 4H), 2.03-2.13 (m, 2H), 2.19 (s, 6H), 2.58 (d, J=4.4 Hz, 2H), 3.00-3.08 (m, 4H), 3.58(s, 2H), 3.83-3.88 (m, 2H), 3.95-4.00 (m, 2H), 5.01 (t, J=4.4 Hz, 1H), 8.38 (ddd, J=0.8, 4.8, 8.0 Hz, 1H), 8.02 (dd, J=2.0, 8.0 Hz, 1H), 8.65 (dd, J=1.2, 4.8 Hz, 1H), 8.77 (ddd, J=0.8, 1.2, 2.0 Hz, 1H).
ESI-MS m/z: 451 [M+H]⁺, 473 [M+Na]⁺.

### (4) N,N-Dimethyl{3-{2-[1-(1,3-dioxolan-2-ylmethyl)piperidin-4-yl]ethyl}-6-(pyridin-3-yl)benz[d]isoxazol-7-yl}methylamine fumarate

The title compound was synthesized from N,N-dimethyl{3-{2-[1-(1,3-dioxolan-2-ylmethyl)piperidin-4-yl]ethyl}-6-(pyridin-3-yl)benz[d]isoxazol-7-yl}methylamine according to Example 124-(4).
¹H-NMR (400 MHz, DMSO-d₆) δ (ppm) : 1.20-1.39 (m, 3H), 1.68-1.78 (m, 4H), 2.03-2.15 (m, 2H), 2.50-2.55 (m, 6H), 2.95-3.07 (m, 4H), 3.17 (s, 2H), 3.55 (s, 2H), 3.74-3.81 (m, 2H), 3.83-3.93 (m, 2H), 4.91-4.93 (m, 1H), 6.61 (s, 2H), 7.36 (d, J=8.0 Hz, 1H), 7.52 (dd, J=4.4, 8.0 Hz, 1H), 7.91 (d, J=8.0 Hz, 1H), 8.03 (d, J=8.0 Hz, 1H), 8.64 (d, J=4.4 Hz, 1H), 8.75 (s, 1H). ESI-MS m/z: 226 [M+2H]²⁺, 451 [M+H]⁺.

### Example 126

### N,N-Dimethyl{3-[2-(1-benzylpiperidin-4-yl)ethyl]-6-(furan-2-yl)benz[d]isoxazol-7-yl}methylamine dihydrochloride

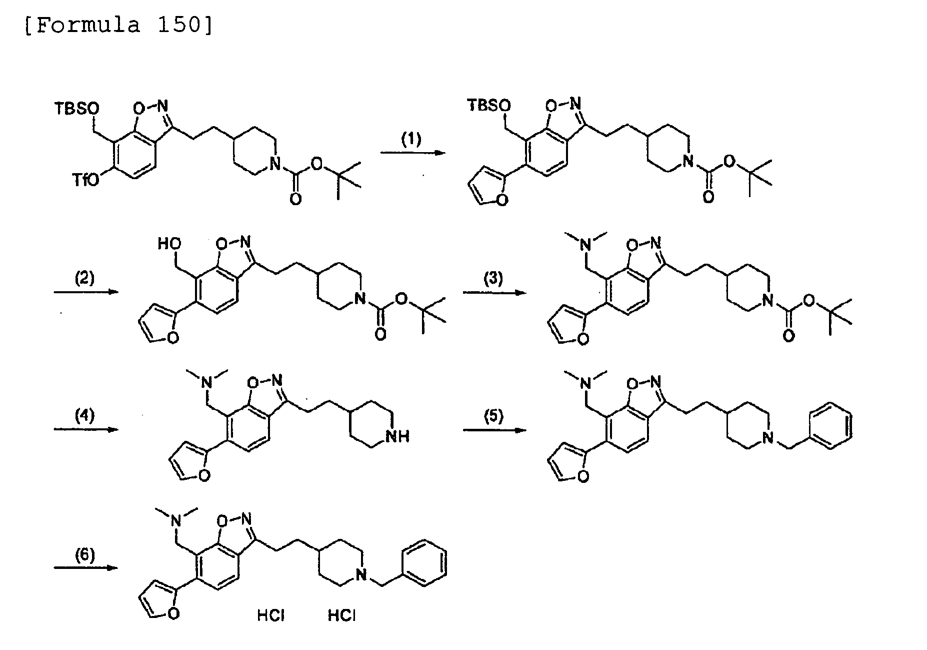

### (1) tert-Butyl 4-{2-[7-(tert-butyldimethylsilanyloxymethyl)-6-(furan-2-yl)benz[d]isoxazol-3-yl]ethyl}piperidine-1-carboxylate

The title compound was synthesized from tert-butyl 4-{2-[7-(tert-butyldimethylsilanyloxymethyl)-6-trifluoromethanesulfonyloxybenz[d]isoxazol-3-yl]ethyl}piperidine-1-carboxylate (compound synthesized in Example 120-(2)) and 2-(tributylstannyl)furan according to Example 120-(3).
¹H-NMR (400 MHz, CDCl₃) δ(ppm): 0.12 (s, 6H), 0.90 (s, 9H), 1.12-1.26 (m, 2H), 1.46 (s, 9H), 1.46-1.56 (m, 1H), 1.72-1.86 (m, 4H), 2.60-2.74 (m, 2H), 3.02 (t, J=8.0 Hz, 2H), 4.04-4.18 (m, 2H), 5.09 (s, 2H) 6.57 (dd, J=2.0, 3. 4 Hz, 1H), 7 . 14 (d, J=3.4 Hz, 1H), 7.58-7.61 (m, 2H), 7.74 (d, J=8.4 Hz, 1H).

### (2) tert-Butyl 4-{2-[6-(furan-2-yl)-7-hydroxymethylbenz[d]isoxazol-3-yl]ethyl}piperidine-1-carboxylate

The title compound was synthesized from tert-butyl 4-{2-[7-(tert-butyldimethylsilanyloxymethyl)-6-(furan-2-yl)benz[d]isoxazol-3-yl]ethyl}piperidine-1-carboxylate according to Example 120-(4).
¹H-NMR (400 MHz, CDCl₃) δ (ppm) : 1.14-1.24 (m, 2H), 1.46 (s, 9H), 1.46-1.58 (m, 1H), 1.72-1.84 (m, 4H), 2.37 (t, J=6.4 Hz, 1H), 2.64-2.74 (m, 2H), 3.03 (t, J=7.8 Hz, 2H), 4.06-4.18 (m, 2H), 5.15 (d, J=6.4 Hz, 2H), 6.59 (dd, J=2.0, 3.6 Hz, 1H), 6.94 (dd, J=0.8, 3.6 Hz, 1H), 7.59 (d, J=8.2 Hz, 1H), 7.62 (dd, J=0.8, 2.0 Hz, 1H), 7.65 (d, J=8.2 Hz, 1H).

### (3) tert-Butyl 4-{2-[7-(N,N-dimethylaminomethyl)-6-(furan-2-yl)benz[d]isoxazol-3-yl]ethyl}piperidine-1-carboxylate

The title compound was synthesized from tert-butyl 4-{2-[6-(furan-2-yl)-7-hydroxymethylbenz[d]isoxazol-3-yl]ethyl}piperidine-1-carboxylate according to Example 120-(5).
¹H-NMR (400 MHz, CDCl₃) δ(ppm): 1.16-1.26 (m, 2H), 1.46 (s, 9H), 1.47-1.62 (m, 1H), 1.74-1.86 (m, 4H), 2.32 (s, 6H), 2.64-2.76 (m, 2H), 3.02 (t, J=8.0 Hz, 2H), 3.81 (s, 2H), 4.04⁺4.20 (m, 2H), 6.57 (dd, J=2.0, 3.2 Hz, 1H), 7.12 (dd, J=0.8, 3.2 Hz, 1H), 7.55-7.60 (m, 2H), 7.75 (d, J=8.0 Hz, 1H) .

### (4) N,N-Dimethyl{6-(furan-2-yl)-3-[2-(piperidin-4-yl)ethyl]benz[d]isoxazol-7-yl}methylamine

The title compound was synthesized from tert-butyl 4-{2-[7-(N,N-dimethylaminomethyl)-6-(furan-2-yl)benz[d]isoxazol-3-yl]ethyl}piperidine-1-carboxylate according to Example 120-(6). The resulting crude product was used for the next reaction without further purification.

### (5) N,N-Dimethyl{3-[2-(1-benzylpiperidin-4-yl)ethyl]-6-(furan-2-yl)benz[d]isoxazol-7-yl}methylamine

The title compound was synthesized from N,N-dimethyl{6-(furan-2-yl)-3-[2-(piperidin-4-yl)ethyl]benz[d]isoxazol-7-yl}methylamine according to Example 120-(7).
¹H-NMR (400 MHz, CDCl₃) δ (ppm) : 1.32-1.42 (m, 3H), 1.72-1.84 (m, 4H), 1.92-1.98 (m, 2H), 2.32 (s, 6H), 2.86-2.94 (m, 2H), 2.97-3.02 (m, 2H), 3.49 (s, 2H), 3.81 (s, 2H), 6.56 (dd, J=2.0, 3.2 Hz, 1H), 7.12 (dd, J=0.8, 3.2 Hz, 1H), 7.22-7.34 (m, 5H), 7.54-7.58 (m, 2H), 7.40 (d, J=8.4 Hz, 1H).
ESI-MS m/z: 444 [M+H]⁺, 466 [M+Na]⁺.

### (6) N,N-Dimethyl{3-[2-(1-benzylpiperidin-4-yl)ethyl]-6-(furan-2-yl)benz[d]isoxazol-7-yl}methylamine dihydrochloride

The title compound was synthesized from N,N-dimethyl{3-[2-(1-benzylpiperidin-4-yl)ethyl]-6-(furan-2-yl)benz[d]isoxazol-7-yl}methylamine according to Example 120-(8).
¹H-NMR (400 MHz, DMSO-d₆) δ(ppm): 1.58-1.70 (m, 3H), 1.70-1.82 (m, 2H), 1.88-1.98 (m, 2H), 2.49-2.51 (m, 6H), 2.81-2.93 (m, 2H), 3.02-3.13 (m, 2H), 3.26-3.35 (m, 2H), 4.23-4.26 (m, 2H), 4.82-4.86 (m, 2H), 6.75-6.48 (m, 1H), 7.35-7.37 (m, 1H), 7.43-7.48 (m, 3H), 7.60-7.67 (m, 2H), 7.90 (d, J=8.4 Hz, 1H), 8.03-8.06 (m, 2H), 9.76 (br s, 1H), 10.92 (br s, 1H).
ESI-MS m/z: 444 [M+H]⁺.

### Example 127

### N,N-Dimethyl{3-[2-(1-benzylpiperidin-4-yl)ethyl]-6-(thiophen-2-yl)benz[d]isoxazol-7-yl}methylamine dihydrochloride

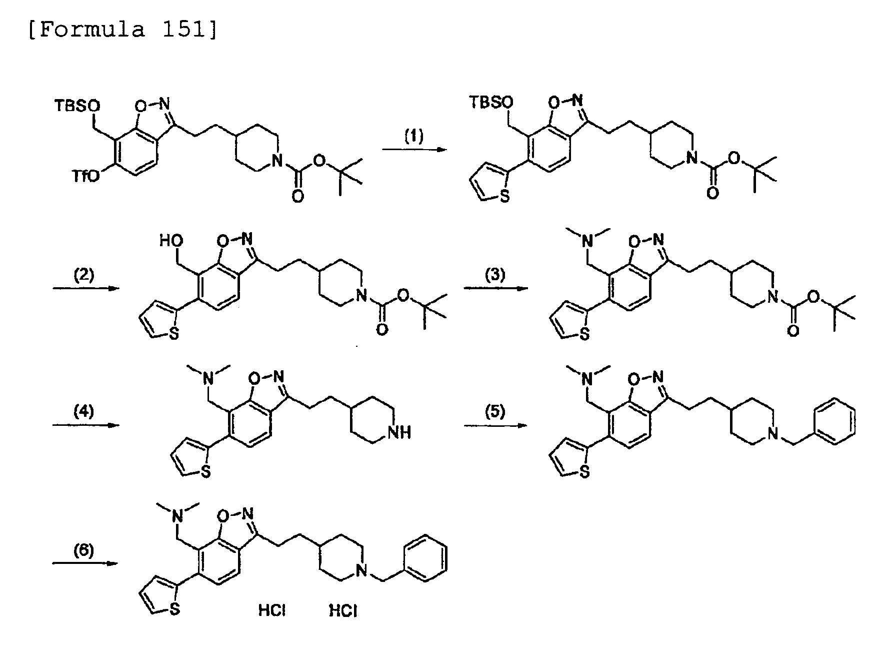

### (1) tert-Butyl 4-{2-[7-(tert-butyldimethylsilanyloxymethyl)-6-(thiophen-2-yl)benz[d]isoxazol-3-yl]ethyl}piperidine-1-carboxylate

The title compound was synthesized from tert-butyl 4-{2-[7-(tert-butyldimethylsilanyloxymethyl)-6-trifluoromethanesulfonyloxybenz[d]isoxazol-3-yl]ethyl}piperidine-1-carboxylate (compound synthesized in Example 120-(2)) and 2-(tributylstannyl)thiophene according to Example 120-(3).
¹H-NMR (400 MHz, CDCl₃) δ(ppm): 0.14 (s, 6H), 0.92 (s, 9H), 1.14-1.26 (m, 2H), 1.46 (s, 9H), 1.43-1.60 (m, 1H), 1.74-1.84 (m, 4H), 2.64-2.72 (m, 2H), 3.03 (t, J=7.8 Hz, 2H), 4.03-4.19 (m, 2H), 4.98 (s, 2H), 7.15 (dd, J=3.6, 5.2 Hz, 1H), 7.44 (dd, J=1.2, 5.2 Hz, 1H), 7.46 (d, J=8.2 Hz, 1H), 7.53 (dd, J=1.2, 3.6 Hz, 1H), 7.56 (d, J=8.2 Hz, 1H).

### (2) tert-Butyl 4-{2-[7-hydroxymethyl-6-(thiophen-2-yl)benz[d]isoxazol-3-yl]ethyl}piperidine-1-carboxylate

The title compound was synthesized from tert-butyl 4-{2-[7-(tert-butyldimethylsilanyloxymethyl)-6-(thiophen-2-yl)benz[d]isoxazol-3-yl]ethyl}piperidine-1-carboxylate according to Example 120-(4).
¹H-NMR (400 MHz, CDCl₃) δ(ppm): 1.14-1.24 (m, 2H), 1.46 (s, 9H), 1.46-1.60 (m, 1H), 1.74-1.84 (m, 4H), 2.20 (t, J=6.4 Hz, 1H), 2.63-2.73 (m, 2H), 3.04 (t, J=8.0 Hz, 2H), 4.02-4.18 (m, 2H), 5.06 (d, J=6.4 Hz, 2H), 7.16 (dd, J=3.6, 5.2 Hz, 1H), 7.38 (dd, J=1.2, 3.6 Hz, 1H), 7.44-7.48 (m, 2H), 7.59 (d, J=8.0 Hz, 1H).

### (3) tert-Butyl 4-{2-[7-(N,N-dimethylaminomethyl)-6-(thiophen-2-yl)benz[d]isoxazol-3-yl]ethyl}piperidine-1-carboxylate

The title compound was synthesized from tert-butyl 4-{2-[7-hydroxymethyl-6-(thiophen-2-yl)benz[d]isoxazol-3-yl]ethyl}piperidine-1-carboxylate according to Example 120-(5).
¹H-NMR (400 MHz, CDCl₃) δ(ppm): 1.16-1.24 (m, 2H), 1.46 (s, 9H), 1.47-1.62 (m, 1H), 1.74-1.86 (m, 4H), 2.29 (s, 6H), 2.66-2.74 (m, 2H), 3.02 (t, J=7.8 Hz, 2H), 3.72 (s, 2H), 4.05-4.19 (m, 2H), 7.15 (dd, J=3.6, 5.2 Hz, 1H), 7.43 (dd, J=1.2, 5.2 Hz, 1H), 7.49 (d, J=8.2 Hz, 1H), 7.55 (d, J=8.2 Hz, 1H), 7.61 (dd, J=1.2, 3.6 Hz, 1H).

### (4) N,N-Dimethyl{3-[2-(piperidin-4-yl)ethyl]-6-(thiophen-2-yl)benz[d]isoxazol-7-yl}methylamine

The title compound was synthesized from tert-butyl 4-{2-[7-(N,N-dimethylaminomethyl)-6-(thiophen-2-yl)benz[d]isoxazol-3-yl]ethyl}piperidine-1-carboxylate according to Example 120-(6). The resulting crude product was used for the next reaction without further purification.

### (5) N,N-Dimethyl{3-[2-(1-benzylpiperidin-4-yl)ethyl]-6-(thiophen-2-yl)benz[d]isoxazol-7-yl}methylamine

The title compound was synthesized from N,N-dimethyl{3-[2-(piperidin-4-yl)ethyl]-6-(thiophen-2-yl)benz[d]isoxazol-7-yl}methylamine according to Example 120-(7).
¹H-NMR (400 MHz, CDCl₃) δ(ppm): 1.30-1.42 (m, 3H), 1.72-1.86 (m, 4H), 1.90-2.00 (m, 2H), 2.89 (s, 6H), 2.86-2.92 (m, 2H), 2.98-3.02 (m, 2H), 3.49 (s, 2H), 3.72 (s, 2H), 7.15 (dd, J=4.4, 5.1 Hz, 1H), 7.24-7.34 (m, 5H), 7.43 (dd, J=1.2, 5.1 Hz, 1H), 7.48 (d, J=8.0 Hz, 1H), 7.54 (d, J=8.0 Hz, 1H), 7.61 (dd, J=1.2, 4.0 Hz, 1H). ESI-MS m/z: 460 [M+H]⁺, 482 [M+Na]⁺.

### (6) N,N-Dimethyl{3-[2-(1-benzylpiperidin-4-yl)ethyl]-6-(thiophen-2-yl)benz[d]isoxazol-7-yl}methylamine dihydrochloride

The title compound was synthesized from N,N-dimethyl{3-[2-(1-benzylpiperidin-4-yl)ethyl]-6-(thiophen-2-yl)benz[d]isoxazol-7-yl}methylamine according to Example 120-(8).
¹H-NMR (400 MHz, DMSO-d₆) δ(ppm): 1.57-1.69 (m, 3H), 1.70-1.83 (m, 2H), 1.90-1.98 (m, 2H), 2.48-2.51 (m, 6H), 2.81-2.92 (m, 2H), 3.06-3.12 (m, 2H), 3.27-3.34 (m, 2H), 4.23-4.26 (m, 2H), 4.70-4.43 (m, 2H), 7.27 (dd, J=3.6, 5.2 Hz, 1H), 7.43-7.47 (m, 3H), 7.48-7.53 (m, 1H), 7.54 (d, J=8.0 Hz, 1H), 7.60-7.67 (m, 2H), 7.83 (dd, J=1.2, 5.2 Hz, 1H), 8.08 (d, J=8.0 Hz, 1H), 10.35 (br s, 1H), 10.80 (br s, 1H).
ESI-MS m/z: 460 [M+H]⁺.

### Example 128

### N,N-Dimethyl{3-[2-(piperidin-4-yl)ethyl]-6-(pyridin-4-yl)benz[d]isoxazol-7-yl}methylamine trihydrochloride

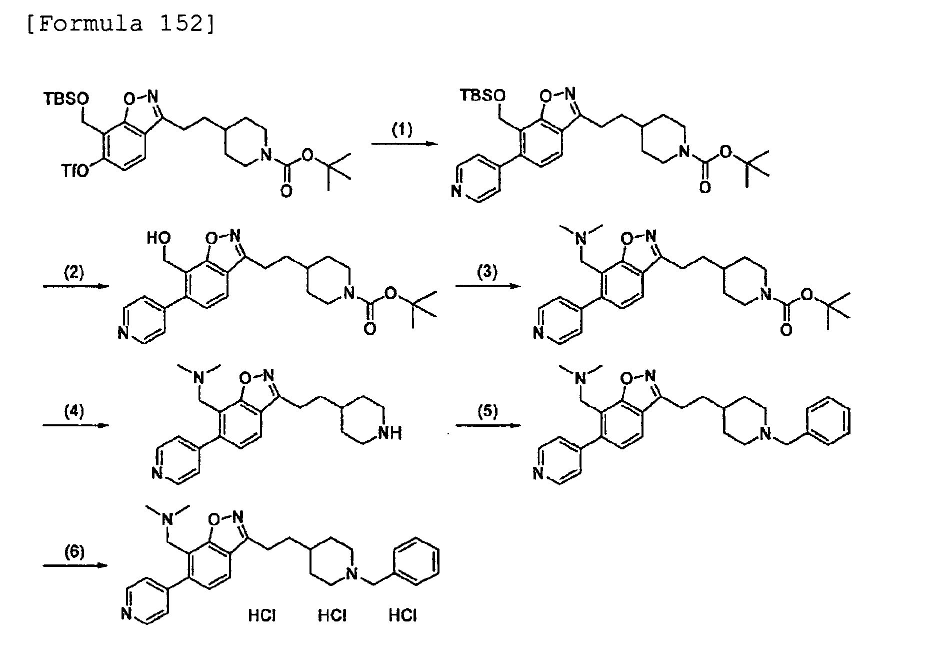

### (1) tert-Butyl 4-{2-[7-(tert-butyldimethylsilanyloxymethyl)-6-(pyridin-4-yl)benz[d]isoxazol-3-yl]ethyl}piperidine-1-carboxylate

The title compound was synthesized from tert-butyl 4-{2-[7-(tert-butyldimethylsilanyloxyxymethyl)-6-trifluoromethanesulfonyloxybenz[d]isoxazol-3-yl]ethyl}piperidine-1-carboxylate (compound synthesized in Example 120-(2)) and (4-pyridyl)tributyltin according to Example 120-(3).
¹H-NMR (400 MHz, CDCl₃) δ(ppm): 0.11 (s, 6H), 0.90 (s, 9H), 1.14-1.28 (m, 2H), 1.46 (s, 9H), 1.48-1.60 (m, 1H), 1.75-1.88 (m, 4H), 2.66-2.78 (m, 2H), 3.05 (t, J=7.8 Hz, 2H), 4.05-4.20 (m, 2H), 4.83 (s, 2H), 7.28 (d, J=8.4 Hz, 1H), 7.52 (dd, J=1.6, 4.4 Hz, 2H), 7.65 (d, J=8.4 Hz, 1H), 8.70 (dd, J=1.6, 4.4 Hz, 2H).

### (2) tert-Butyl 4-{2-[7-hydroxymethyl-6-(pyridin-4-yl)benz[d]isoxazol-3-yl]ethyl}piperidine-1-carboxylate

The title compound was synthesized from tert-butyl 4-{2-[7-(tert-butyldimethylsilanyloxymethyl)-6-(pyridin-4-yl)benz[d]isoxazol-3-yl]ethyl}piperidine-1-carboxylate according to Example 120-(4).
¹H-NMR (400 MHz, CDCl₃) δ(ppm): 1.15-1.30 (m, 2H), 1.46 (s, 9H), 1.48-1.62 (m, 1H), 1.76-1.86 (m, 4H), 2.30 (t, J=6.4 Hz, 1H), 2.64-2.76 (m, 2H), 3.07 (t, J=7.6 Hz, 2H), 4.06-4.18 (m, 2H), 4.91 (d, J=6.4 Hz, 2H), 7.29 (d, J=8.0 Hz, 1H), 7.46 (dd, J=2.0, 4.4 Hz, 2H), 7.67 (d, J=8.0 Hz, 1H), 8.73 (dd, J=2. 0, 4.4 Hz, 2H).

### (3) tert-Butyl 4-{2-[7-(N,N-dimethylaminomethyl)-6-(pyridin-4-yl)benz[d]isoxazol-3-yl]ethyl}piperidine-1-carboxylate

The title compound was synthesized from tert-butyl 4-{2-[7-hydroxymethyl-6-(pyridin-4-yl)benz[d]isoxazol-3-yl]ethyl}piperidine-1-carboxylate according to Example 120-(5).
¹H-NMR (400 MHz, CDCl₃) δ(ppm): 1.15-1.27 (m, 2H), 1.46 (s, 9H), 1.50-1.64 (m, 1H), 1.77-1.89 (m, 4H), 2.02 (s, 6H), 2.66-2.78 (m, 2H), 3.05 (t, J=7.8 Hz, 2H), 3.60 (s, 2H), 4.08-4.20 (m, 2H), 7.26 (d, J=8.2 Hz, 1H), 7.56 (dd, J=1.6, 4.4 Hz, 2H), 7.62 (d, J=8.2 Hz, 1H), 8.69 (dd, J=1.6, 4.4 Hz, 2H).
ESI-MS m/z: 465 [M+H]⁺, 487 [M+Na]⁺.

### (4) N,N-Dimethyl{3-[2-(piperidin-4-yl)ethyl]-6-(pyridin-4-yl)benz[d]isoxazol-7-yl}methylamine

The title compound was synthesized from tert-butyl 4-{2-[7-(N,N-dimethylaminomethyl)-6-(pyridin-4-yl)benz[d]isoxazol-3-yl]ethyl}piperidine-1-carboxylate according to Example 120-(6).
¹H-NMR (400 MHz, CDCl3) δ(ppm): 1.16-1.30 (m, 2H), 1.50-1.62 (m, 1H), 1.76-1.86 (m, 4H), 2.20 (s, 6H), 2.61 (dt, J=2. 4, 12.0 Hz, 2H), 3.04 (t, J=7.8 Hz, 2H), 3.06-3.13 (m, 2H), 3.60 (s, 2H), 7.26 (d, J=8.0 Hz, 1H), 7.56 (dd, J=1.4, 4.4 Hz, 2H), 7.63 (d, J=8.0 Hz, 1H), 8.69 (dd, J=1.4, 4.4 Hz, 2H).

### (5) N,N-Dimethyl{3-[2-(1-benzylpiperidin-4-yl)ethyl]-6-(pyridin-4-yl)benz[d]isoxazol-7-yl}methylamine

The title compound was synthesized from N,N-dimethyl{3-[2-(piperidin-4-yl)ethyl]-6-(pyridin-4-yl)benz[d]isoxazol-7-yl}methylamine according to Example 120-(7).
¹H-NMR (400 MHz, CDCl₃) δ(ppm) : 1.30-1.44 (m, 3H), 1.74-1.84 (m, 4H), 1.92-2.00 (m, 2H), 2.20 (s, 6H), 2.84-2.94 (m, 2H), 3.20 (t, J=8.0 Hz, 2H), 3.50 (s, 2H), 3.59 (s, 2H), 7.22-7.33 (m, 6H), 7.55 (dd, J=1.6, 4.4 Hz, 2H), 7.62 (d, J=8.0 Hz, 1H), 8.69 (dd, J=2.0, 4.4 Hz, 2H).
ESI-MS m/z: 455 [M+H]⁺, 477 [M+Na]⁺.

### (6) N,N-Dimethyl{3-[2-(1-benzylpiperidin-4-yl)ethyl]-6-(pyridin-4-yl)benz[d]isoxazol-7-yl}methylamine trihydrochloride

The title compound was synthesized from N,N-dimethyl{3-[2-(1-benzylpiperidin-4-yl)ethyl]-6-(pyridin-4-yl)benz[d]isoxazol-7-yl}methylamine according to Example 120-(8).
¹H-NMR (400 MHz, DMSO-d₆) δ(ppm): 1.58-1.68 (m, 3H), 1.70-1.82 (m, 2H), 1.90-2.00 (m, 2H), 2.59-2.62 (m, 6H), 2.83-2.95 (m, 2H), 3.07-3.18 (m, 2H), 3.28-3.34 (m, 2H), 4.23-4.27 (m, 2H), 4.55-4.61 (m, 2H), 7.44-7.48 (m, 4H), 7.61-7.68 (m, 2H), 7.97-8.04 (m, 2H), 8.20 (d, J=8.4 Hz, 1H), 8.96 (d, J=6.0 Hz, 2H), 10.40 (br s, 1H), 10.82 (br s, 1H).
ESI-MS m/z: 455 [M+H]⁺.

### Example 129

### {1-{4-{2-[7-(N,N-Dimethylaminomethyl)-6-phenylbenz[d]isoxazol-3-yl]ethyl}piperidinomethyl}cyclopentyl}methanol dihydrochloride

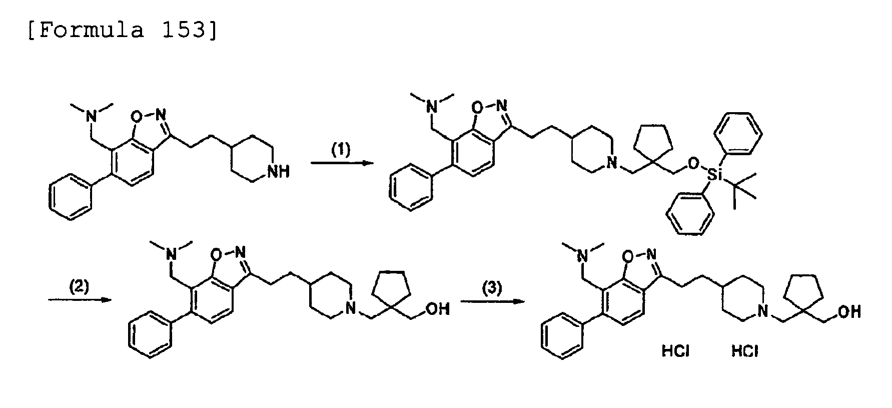

### (1) N,N-Dimethyl{3-{2-{1-[1-(tert-butyldiphenylsilanyloxymethyl)cyclopentylmethyl]piperid in-4-yl}ethyl}-6-phenylbenz[d]isoxazol-7-yl}methylamine

The title compound was synthesized from N,N-dimethyl{6-phenyl-3-[2-(piperidin-4-yl)ethyl]benz[d]isoxazol-7-yl}methylamine (compound synthesized in Example 120-(6)) and 1-(tert-butyldiphenylsilanyloxymethyl)cyclopentanecarbaldehyde (compound synthesized in Example 51-(3)) according to Example 120- (7) .
¹H-NMR (400 MHz, CDCl₃) δ(ppm): 1.05 (s, 9H), 1.20-1.38 (m, 6H), 1.40-1.60 (m, 5H), 1.63-1.70 (m, 2H), 1.76-1.84 (m, 2H), 2.06-2.16 (m, 2H), 2.19 (s, 6H), 2.35 (s, 2H), 2.83-2.89 (m, 2H), 3.01 (t, J=8.0 Hz, 2H), 3.47 (s, 2H), 3.65 (s, 2H), 7.28 (d, J=8.0 Hz, 1H), 7.33-7.46 (m, 9H), 7.52-7.55 (m, 2H), 7.58 (d, J=8.0 Hz, 1H), 7.68-7.72 (m, 4H).
ESI-MS m/z: 715 [M+H]⁺.

### (2) {1-{4-{2-[7-(N,N-Dimethylaminomethyl)-6-phenylbenz[d]isoxazol-3-yl)]ethyl}piperidinomethyl}cyclopentyl}methanol

The title compound was synthesized from N,N-dimethyl{3-{2-{1-[1-(tert-butyldiphenylsilanyloxymethyl)cyclopentylmethyl]piperid in-4-yl}ethyl}-6-phenylbenz[d]isoxazol-7-yl}methylamine according to Example 120-(4).
¹H-NMR (400 MHz, CDCl₃) δ (ppm) : 1.23-1.38 (m, 6H), 1.58-1.68 (m, 5H), 1.74-1.86 (m, 4H), 2.03-2.12 (m, 2H), 2.18 (s, 6H), 2.51 (s, 2H), 2.98-3.06 (m, 4H), 3.51 (s, 2H), 3.65 (s, 2H), 7.27 (d, J=8.0 Hz, 1H), 7.34-7.44 (m, 3H), 7.50-7.54 (m, 2H), 7. 56 (d, J=8.0 Hz, 1H) .
ESI-MS m/z: 476 [M+H]⁺.

### (3) {1-{4-{2-[7-(N,N-Dimethylaminomethyl)-6-phenylbenz[d]isoxazol-3-yl)]ethyl}piperidinomethyl}cyclopentyl}methanol dihydrochloride

The title compound was synthesized from {1-{4-{2-[7-(N,N-dimethylaminomethyl)-6-phenylbenz[d]isoxazol-3-yl]ethyl}piperidinomethyl}cyclopentyl}methanol according to Example 120-(8).
ESI-MS m/z: 238 [M+2H]²⁺, 476 [M+H]⁺.

### Example 130

### {1-{4-{2-[7-(N,N-Dimethylaminomethyl)-6-phenylbenz[d]isoxazol-3-yl]ethyl}piperidinomethyl}cyclobutyl}methanol dihydrochloride

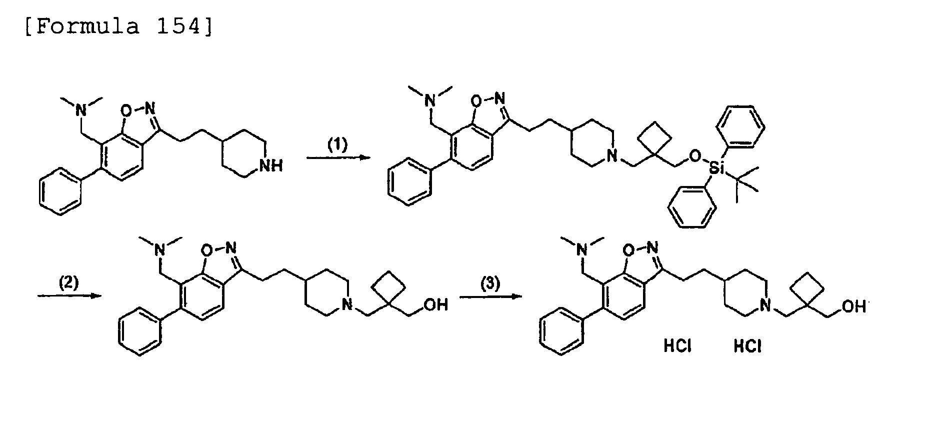

### (1) N,N-Dimethyl{3-{2-{1-[1-(tert-butyldiphenylsilanyloxymethyl)cyclobutylmethyl]piperidi n-4-yl}ethyl}-6-phenylbenz[d]isoxazol-7-yl}methylamine

The title compound was synthesized from N,N-dimethyl{6-phenyl-3-[2-(piperidin-4-yl)ethyl]benz[d]isoxazol-7-yl}methylamine (compound synthesized in Example 120-(6)) and 1-(tert-butyldiphenylsilanyloxymethyl)cyclobutanecarbaldehyde (synthesized from (1-hydroxymethylcyclobutyl)methanol [CAS Registry No. 4415-73-0] according to Example 45-(1)(2)) according to Example 120-(7).
¹H-NMR (400 MHz, CDCl₃) δ (ppm) : 1.04 (s, 9H), 1.22-1.38 (m, 2H), 1.66-1.74 (m, 2H), 1.76-1.88 (m, 9H), 1.90-2.00 (m, 2H), 2.19 (s, 6H), 2.43 (s, 2H), 2.70-2.76 (m, 2H), 3.02 (t, J=7.6 Hz, 2H), 3.65 (s, 2H), 3.73 (s, 2H), 7.28 (d, J=8.0 Hz, 1H), 7.36-7.48 (m, 9H), 7.52-7.56 (m, 2H), 7.58 (d, J=8.0 Hz, 1H), 7.75-7.79 (m, 4H).
ESI-MS m/z: 701 [M+H]⁺.

### (2) {1-{4-{2-[7-(N,N-Dimethylaminomethyl)-6-phenylbenz[d]isoxazol-3-yl)]ethyl}piperidinomethyl}cyclobutyl}methanol

The title compound was synthesized from N,N-dimethyl{3-{2-{1-[1-(tert-butyldiphenylsilanyloxymethyl)cyclobutylmethyl]piperidi n-4-yl}ethyl}-6-phenylbenz[d]isoxazol-7-yl}methylamine according to Example 120-(4).
¹H-NMR (400 MHz, CDCl₃) δ(ppm): 1.20-1.32 (m, 2H), 1.73-1.86 (m, 9H), 1.93-2.02 (m, 4H), 2.18 (s, 6H), 2.51 (s, 2H), 2.90-2.97 (m, 2H), 3.00 (t, J=8.0 Hz, 2H), 3.65 (s, 2H), 3.78 (s, 2H), 7.27 (d, J=8.0 Hz, 1H), 7.34-7.47 (m, 3H), 7.51-7.54 (m, 2H), 7.56 (d, J=8.0 Hz, 1H) .
ESI-MS m/z: 462 [M+H]⁺.

### (3) {1-{4-{2-[7-(N,N-Dimethylaminomethyl)-6-phenylbenz[d]isoxazol-3-yl)]ethyl}piperidinomethyl}cyclobutyl}methanol dihydrochloride

The title compound was synthesized from {1-{4-{2-[7-(N,N-dimethylaminomethyl)-6-phenylbenz[d]isoxazol-3-yl]ethyl}piperidinomethyl}cyclobutyl}methanol according to Example 120-(8).
ESI-MS m/z: 231 [M+2H]²⁺, 462 [M+H]⁺.

### Example 131

### {1-{4-{2-[7-(N,N-Dimethylaminomethyl)-6-phenylbenz[d]isoxazol-3-yl]ethyl}piperidinomethyl}cyclopropyl}methanol dihydrochloride

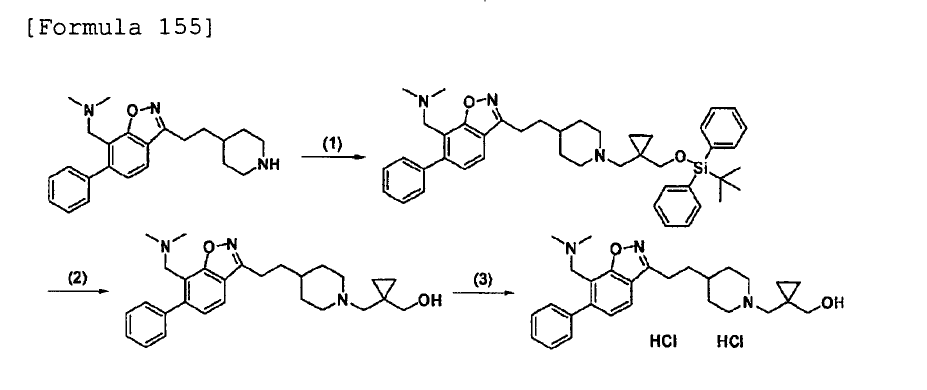

### (1) N,N-Dimethyl{3-{2-{1-[1-(tert-butyldiphenylsilanyloxymethyl)cyclopropylmethyl]piperid in-4-yl}ethyl}-6-phenylbenz[d]isoxazol-7-yl}methylamine

The title compound was synthesized from N,N-dimethyl{6-phenyl-3-[2-(piperidin-4-yl)ethyl]benz[d]isoxazol-7-yl}methylamine (compound synthesized in Example 120-(6)) and 1-(tert-butyldiphenylsilanyloxymethyl)cyclopropanecarbaldehyde (compound synthesized in Example 45-(2)) according to Example 120- (7) .
¹H-NMR (400 MHz, CDCl₃) δ(ppm): 0.23-0.27 (m, 2H), 0.42-0.46 (m, 2H), 1.04 (s, 9H), 1.25-1.37 (m, 3H), 1.69-1.79 (m, 2H), 1.80-1.87 (m, 4H), 2.19 (s, 6H), 2.33 (s, 2H), 2.93-3.00 (m, 2H), 3.02 (t, J=8.0 Hz, 2H), 3.60 (s, 2H), 3.65 (s, 2H), 7.28 (d, J=8.0 Hz, 1H), 7.35-7.48 (m, 9H), 7.52-7.55 (m, 2H), 7.58 (d, J=8.0 Hz, 1H), 7.71-7.74 (m, 4H).

### (2) {1-{4-{2-[7-(N,N-Dimethylaminomethyl)-6-phenylbenz[d]isoxazol-3-yl)]ethyl}piperidinomethyl}cyclopropyl}methanol

The title compound was synthesized from N,N-dimethyl{3-{2-{1-[1-(tert-butyldiphenylsilanyloxymethyl)cyclopropylmethyl]piperid in-4-yl}ethyl}-6-phenylbenz[d]isoxazol-7-yl}methylamine according to Example 120-(4).
¹H-NMR (400 MHz, CDCl₃) δ(ppm): 0.34-0.38 (m, 2H), 0.48-0.52 (m, 2H), 1.24-1.44 (m, 4H), 1.78-1.86 (m, 4H), 1.86-1.96 (m, 2H), 2.18 (s, 6H), 2.46 (s, 2H), 3.02 (t, J=7.6 Hz, 2H), 3.18-3.24 (m, 2H), 3.54 (s, 2H), 3.65 (s, 2H), 7.26-7.29 (m, 1H), 7.40-7.47 (m, 3H), 7.51-7.54 (m, 2H), 7.57 (d, J=8.0 Hz, 1H).
ESI-MS m/z: 448 [M+H]⁺.

### (3) {1-{4-{2-[7-(N,N-Dimethylaminomethyl)-6-phenylbenz[d]isoxazol-3-yl)]ethyl}piperidinomethyl}cyclopropyl}methanol dihydrochloride

The title compound was synthesized from {1-{4-{2-[7-(N,N-dimethylaminomethyl)-6-phenylbenz[d]isoxazol-3-yl]ethyl}piperidinomethyl}cyclopropyl}methanol according to Example 120-(8).
ESI-MS m/z: 224 [M+2H]²⁺, 448 [M+H]⁺.

### Example 132

### 3-{3-[2-(1-Benzylpiperidin-4-yl)ethyl]-7-(N,N-dimethylaminomethyl)benz[d]isoxazol-6-yl}benzonitrile dihydrochloride

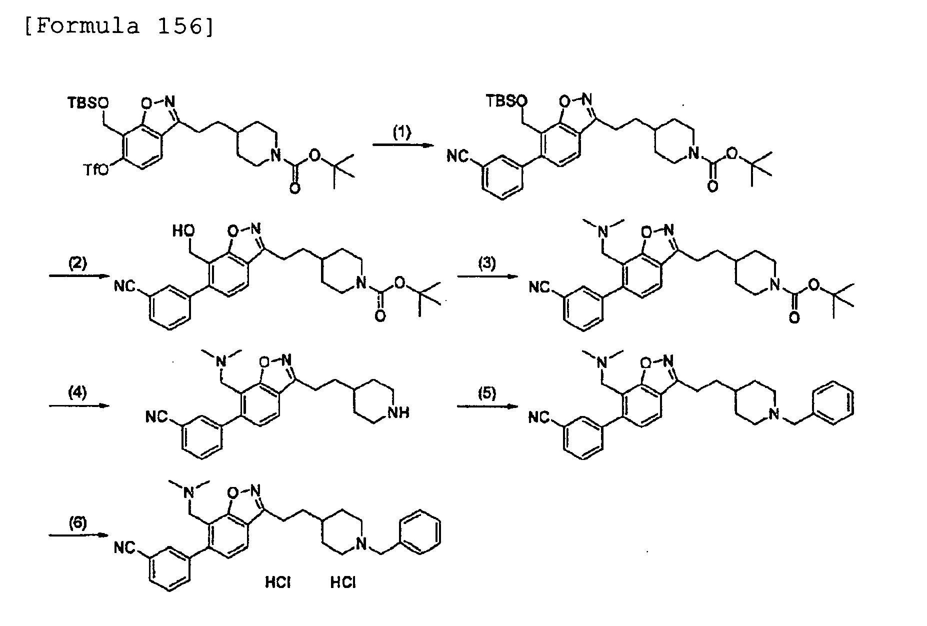

### (1) tert-Butyl 4-{2-[7-(tert-butyldimethylsilanyloxymethyl)-6-(3-cyanophenyl)benz[d]isoxazol-3-yl]ethyl}piperidine-1-carboxylate

The title compound was synthesized from tert-butyl 4-{2-[7-(tert-butyldimethylsilanyloxymethyl)-6-trifluoromethanesulfonyloxybenz[d]isoxazol-3-yl]ethyl}piperidine-1-carboxylate (compound synthesized in Example 120-(2)) and 3-tributyltin-benzonitrile [CAS Registry No. 79062-29-6] according to Example 120-(3).
¹H-NMR (400 MHz, CDCl₃) δ(ppm): 0.12 (s, 6H), 0.91 (s, 9H), 1.16-1.26 (m, 2H), 1.46 (s, 9H), 1.50-1.62 (m, 1H), 1.74-1.88 (m, 4H), 2.64-2.78 (m, 2H), 3.05 (t, J=7.8 Hz, 2H), 4.80 (s, 2H), 7.26 (d, J=8.0 Hz, 1H), 7.56-7.85 (m, 4H), 7.92 (s, 1H).
ESI-MS m/z: 598 [M+Na]⁺.

### (2) tert-Butyl 4-{2-[6-(3-cyanophenyl)-7-hydroxymethylbenz[d]isoxazol-3-yl]ethyl}piperidine-1-carboxylate

The title compound was synthesized from tert-butyl 4-{2-[7-(tert-butyldimethylsilanyloxymethyl)-6-(3-cyanophenyl)benz[d]isoxazol-3-yl]ethyl}piperidine-1-carboxylate according to Example 120-(4).
¹H-NMR (400 MHz, CDCl₃) δ(ppm): 1.16-1.26 (m, 2H), 1.46 (s, 9H), 1.50-1.62 (m, 1H), 1.45-1.89 (m, 4H), 2.64-2.76 (m, 2H), 3.07 (t, J=7.6 Hz, 2H), 4.06-4.22 (m, 2H), 4.87 (s, 2H), 7.27 (d, J=8.0 Hz, 1H), 7.60 (ddd, J=0.8, 7.6, 8.4 Hz, 1H), 7.66 (d, J=8.0 Hz, 1H), 7.72-7.82 (m, 3H).

### (3) tert-Butyl 4-{2-[6-(3-cyanophenyl)-7-(N,N-dimethylaminomethyl)benz[d]isoxazol-3-yl]ethyl}piperidine-1-carboxylate

The title compound was synthesized from tert-butyl 4-{2-[6-(3-cyanophenyl)-7-hydroxymethylbenz[d]isoxazol-3-yl]ethyl}piperidine-1-carboxylate according to Example 120-(5).
¹H-NMR (400 MHz, CDCl₃) δ(ppm): 1.15-1.28 (m, 2H), 1.46 (s, 9H), 1.52-1.66 (m, 1H), 1.74-1.88 (m, 4H), 2.22 (s, 6H), 2.66-2.78 (m, 2H), 3.05 (t, J=7.8 Hz, 2H), 3.54 (s, 2H), 4.04-4.20 (m, 2H), 7.25 (d, J=8.0 Hz, 1H), 7.56 (dd, J=7.2, 7.6 Hz, 1H), 7.62 (d, J=8.0 Hz, 1H), 7.68-7.71 (m, 1H), 7.84-7.87 (m, 1H), 8.04-8.06 (m, 1H).

### (4) 3-{7-(N,N-Dimethylaminomethyl)-3-[2-(piperidin-4-yl)ethyl]benz[d]isoxazol-6-yl}benzonitrile

The title compound was synthesized from tert-butyl 4-{2-[6-(3-cyanophenyl)-7-(N,N-dimethylaminomethyl)benz[d]isoxazol-3-yl]ethyl}piperidine-1-carboxylate according to Example 120-(6).
ESI-MS m/z: 389 [M+H]⁺.

### (5) 3-{3-[2-(1-Benzylpiperidin-4-yl)ethyl]-7-(N,N-dimethylaminomethyl)benz[d]isoxazol-6-yl}benzonitrile

The title compound was synthesized from 3-{7-(N,N-dimethylaminomethyl)-3-[2-(piperidin-4-yl)ethyl]benz[d]isoxazol-6-yl}benzonitrile according to Example 120- (7).
¹H-NMR (400 MHz, CDCl₃) δ(ppm): 1.30-1.44 (m, 3H), 1.76-1.86 (m, 4H), 1.94-2.02 (m, 2H), 2.21 (s, 6H), 2.87-2.93 (m, 2H), 3.02 (dd, J=7.2, 8.4 Hz, 2H), 3.50 (s, 2H), 3.54 (s, 2H), 7.22-7.27 (m, 2H), 7.29-7.33 (m, 4H), 7.55 (dd, J=7.2, 8.0 Hz, 1H), 7.61 (d, J=8.0 Hz, 1H), 7.70 (dd, J=1.6, 8.0 Hz, 1H), 7.86 (dd, J=1.2, 7.2 Hz, 1H), 8.05 (dd, J=1.2, 1.6 Hz, 1H).
ESI-MS m/z: 479 [M+H]⁺, 501 [M+Na]⁺.

### (6) 3-{3-[2-(1-Benzylpiperidin-4-yl)ethyl]-7-(N,N-dimethylaminomethyl)benz[d]isoxazol-6-yl}benzonitrile dihydrochloride

The title compound was synthesized from 3-{3-[2-(1-benzylpiperidin-4-yl)ethyl]-7-(N,N-dimethylaminomethyl)benz[d]isoxazol-6-yl}benzonitrile according to Example 120-(8).
¹H-NMR (400 MHz, DMSO-d₆) δ(ppm): 1.54-1.66 (m, 3H), 1.72-1.83 (m, 2H), 1.90-1.99 (m, 2H), 2.55-2.58 (m, 6H), 2.84-2.92 (m, 2H), 3.10 (t, J=7.6 Hz, 2H), 3.31-3.38 (m, 2H), 4.23-4.27 (m, 2H), 4.54-4.57 (m, 2H), 7.41 (d, J=8.0 Hz, 1H), 7.43-7.46 (m, 3H), 7.59-7.65 (m, 2H), 7.75 (dd, J=2.4, 7.6 Hz, 1H), 7.84 (dd, J=1.6, 6.6 Hz, 1H), 7.97-8.01 (m, 2H), 8.10 (d, J=8.0 Hz, 1H), 10.03 (br s, 1H), 10.64 (br s, 1H).
ESI-MS m/z: 479 [M+H]⁺.

### Example 133

### 3-{7-(N,N-Dimethylaminomethyl)-3-{2-[1-(1,3-dioxolan-2-ylmethyl)piperidin-4-yl]ethyl}benz[d]isoxazol-6-yl}benzonitrile fumarate

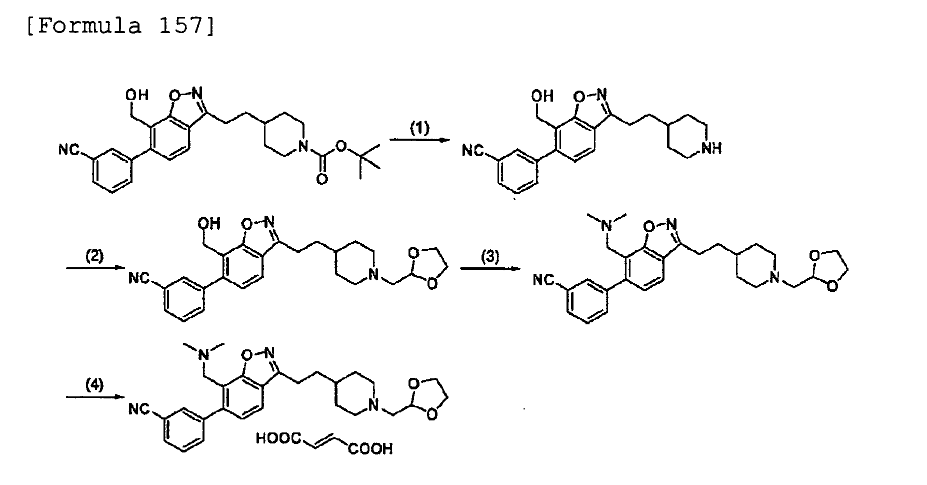

### (1) 3-{7-Hydroxymethyl-3-[2-(piperidin-4-yl)ethyl]benz[d]isoxazol-6-yl}benzonitrile

The title compound was synthesized from tert-butyl 4-{2-[6-(3-cyanophenyl)-7-hydroxymethylbenz[d]isoxazol-3-yl]ethyl}piperidine-1-carboxylate (compound synthesized in Example 132-(2)) according to Example 120-(6).
¹H-NMR (400 MHz, CDCl₃) δ(ppm): 1.18-1.28 (m, 1H), 1.48-1.58 (m, 1H), 1.74-1.88 (m, 4H), 2.56-2.65 (m, 2H), 3.02-3.14 (m, 4H), 4.86 (s, 2H), 7.26 (d, J=8.4 Hz, 1H), 7.61 (ddd, J=0.8, 7.2, 8.0 Hz, 1H), 7.66 (d, J=8.4 Hz, 1H), 7.73 (ddd, J=1.2, 1.6, 8.0 Hz, 1H), 7.80 (ddd, J=0.8, 1.2, 7.2 Hz, 1H), 7.82 (ddd, J=0.8, 0.8, 1.6 Hz, 1H) .

### (2) 3-{3-{2-[1-(1,3-Dioxolan-2-ylmethyl)piperidin-4-yl]ethyl}-7-hydroxymethylbenz[d]isoxazol-6-yl}benzonitrile

The title compound was synthesized from 3-{7-hydroxymethyl-3-[2-(piperidin-4-yl)ethyl]benz[d]isoxazol-6-yl}benzonitrile according to Example 124-(2).
¹H-NMR (400 MHz, CDCl₃) δ(ppm) : 1.37-1.47 (m, 3H), 1.75-1.87 (m, 4H), 2.03-2.13 (m, 2H), 2.58 (d, J=4.8 Hz, 2H), 3.00-3.08 (m, 4H), 3.83-3.89 (m, 2H), 3.94-4.00 (m, 2H), 4.86 (s, 2H), 5.01 (t, J=4.8 Hz, 1H), 7.26 (d, J=8.0 Hz, 1H), 7.60 (dd, J=7.2, 8.0 Hz, 1H), 7.65 (d, J=8.0 Hz, 1H), 7.72-7.82 (m, 3H).

### (3) 3-{7-(N,N-Dimethylaminomethyl)-3-{2-[1-(1,3-dioxolan-2-ylmethyl)piperidin-4-yl]ethyl}benz[d]isoxazol-6-yl}benzonitrile

The title compound was synthesized from 3-{3-{2-[1-(1,3-dioxolan-2-ylmethyl)piperidin-4-yl]ethyl}-7-hydroxymethylbenz[d]isoxazol-6-yl}benzonitrile according to Example 124-(3).
¹H-NMR (400 MHz, CDCl₃) δ (ppm) : 1.36-1.46 (m, 3H), 1.74-1.86 (m, 4H), 2.03-2.13 (m, 2H), 2.21 (s, 6H), 2.58 (d, J=4.4 Hz, 2H), 3.00-3.06 (m, 4H), 3.54 (s, 2H), 3.83-3.89 (m, 2H), 3.94-4.00 (m, 2H), 5.02 (t, J=4.4 Hz, 1H), 7.25 (d, J=8.0 Hz, 1H), 7.56 (dd, J=7.6, 8.0 Hz, 1H), 7.61 (d, J=8.0 Hz, 1H), 7.70 (dd, J=1.6, 7.6 Hz, 1H), 7.86 (dd, J=1.6, 8.0 Hz, 1H), 8.06 (dd, J=1.6, 1.6 Hz, 1H) .
ESI-MS m/z: 475 [M+H]⁺, 497 [M+Na]⁺.

### (4) 3-{7-(N,N-Dimethylaminomethyl)-3-{2-[1-(1,3-dioxolan-2-ylmethyl)piperidin-4-yl]ethyl}benz[d]isoxazol-6-yl}benzonitrile fumarate

The title compound was synthesized from 3-{7-(N,N-dimethylaminomethyl)-3-{2-[1-(1,3-dioxolan-2-ylmethyl)piperidin-4-yl]ethyl}benz[d]isoxazol-6-yl}benzonitrile according to Example 124-(4).
ESI-MS m/z: 238 [M+2H]²⁺, 475 [M+H]⁺.

### Example 134

### 4-{4-{2-[6-Cyclopropylmethoxy-7-(N,N-dimethylaminomethyl)benz[d]isoxazol-3-yl]ethyl}piperidinomethyl}phenol dihydrochloride

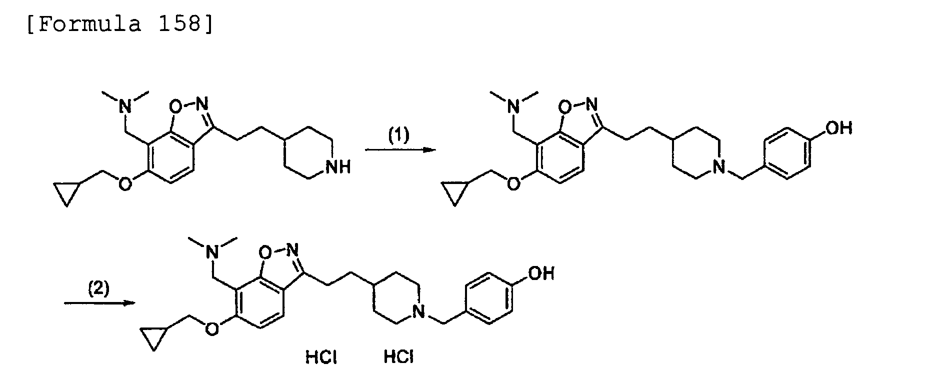

### (1) 4-{4-{2-[6-Cyclopropylmethoxy-7-(N,N-dimethylaminomethyl)benz[d]isoxazol-3-yl]ethyl}piperidinomethyl}phenol

N,N-Dimethyl{6-cyclopropylmethoxy-3-[2-(piperidin-4-yl)ethyl]benz[d]isoxazol-7-yl}methylamine (compound synthesized in Preparation Example 3) (120 mg) and 4-hydroxybenzaldehyde (82 mg) were dissolved in dichloromethane (5 mL). Acetic acid (39 µL) and sodium triacetoxyborohydride (107 mg) were sequentially added to the solution, and the mixture was stirred at room temperature for two hours. A 5 M sodium hydroxide solution, water and dichloromethane were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain a residue. The residue was purified by NH silica gel column chromatography (heptane-ethyl acetate -> chloroform-methanol-aqueous ammonia) to obtain the title compound (117 mg).
¹H-NMR (400 MHz, CDCl₃) δ(ppm): 0.34-0.38 (m, 2H), 0.61-0.65 (m, 2H), 1.22-1.40 (m, 4H), 1.72-1.77 (m, 4H), 1.90-2.00 (m, 2H), 2.34 (s, 6H), 2.89-2.96 (m, 4H), 3.41 (s, 2H), 3.85 (s, 2H), 3.91 (d, J=6.4 Hz, 2H), 6.64 (d, J=8.4 Hz, 2H), 6.89 (d, J=8.8 Hz, 1H), 7.08 (d, J=8.4 Hz, 2H), 7.44 (d, J=8.8 Hz, 1H).
ESI-MS m/z: 486 [M+Na]⁺.

### (2) 4-{4-{2-[6-Cyclopropylmethoxy-7-(N,N-dimethylaminomethyl)benz[d]isoxazol-3-yl]ethyl}piperidinomethyl}phenol dihydrochloride

The title compound was synthesized from 4-{4-{2-[6-cyclopropylmethoxy-7-(N,N-dimethylaminomethyl)benz[d]isoxazol-3-yl]ethyl}piperidinomethyl}phenol according to Example 120-(8).

### Example 135

### N,N-Dimethyl{6-cyclopropylmethoxy-3-{2-[1-(6-methoxypyridin-3-ylmethyl)piperidin-4-yl]ethyl}benz[d]isoxazol-7-yl}methylamine dihydrochloride

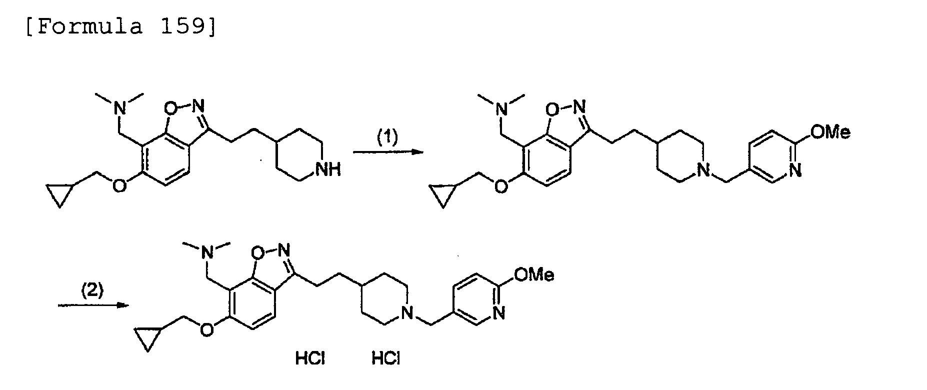

### (1) N,N-Dimethyl{6-cyclopropylmethoxy-3-{2-[1-(6-methoxypyridin-3-ylmethyl)piperidin-4-yl]ethyl}benz[d]isoxazol-7-yl}methylamine

The title compound was synthesized from N,N-dimethyl{6-cyclopropylmethoxy-3-[2-(piperidin-4-yl)ethyl]benz[d]isoxazol-7-yl}methylamine (compound synthesized in Preparation Example 3) and 6-methoxypyridine-3-carbaldehyde according to Example 134- (1) .
¹H-NMR (400 MHz, CDCl₃) δ(ppm): 0.35-0.40 (m, 2H), 0.62-0.66 (m, 2H), 1.22-1.38 (m, 4H), 1.68-1.80 (m, 4H), 1.88-1.98 (m, 2H), 2.05 (s, 6H), 2.82-2.88 (m, 2H), 2.93 (t, J=8.0 Hz, 2H), 3.40 (s, 2H), 3.82 (s, 2H), 3.93 (s, 3H), 3.94 (d, J=6.8 Hz, 2H), 6.71 (d, J=8.4 Hz, 1H), 6.90 (d, J=8.4 Hz, 1H), 7.44 (d, J=8.4 Hz, 1H), 7.56 (dd, J=2.0, 8.4 Hz, 1H), 8.02 (d, J=2.0 Hz, 1H) .
ESI-MS m/z: 479 [M+H]⁺.

### (2) N,N-Dimethyl{6-cyclopropylmethoxy-3-{2-[1-(6-methoxypyridin-3-ylmethyl)piperidin-4-yl]ethyl}benz[d]isoxazol-7-yl}methylamine trihydrochloride

The title compound was synthesized from N,N-dimethyl{6-cyclopropylmethoxy-3-{2-[1-(6-methoxypyridin-3-ylmethyl)piperidin-4-yl]ethyl}benz[d]isoxazol-7-yl}methylamine according to Example 120- (8) .

### Example 136

### 5-{4-{2-[6-Cyclopropylmethoxy-7-(N,N-dimethylaminomethyl)benz[d]isoxazol-3-yl]ethyl}piperidinomethyl}-2(1H)-pyridone dihydrochloride

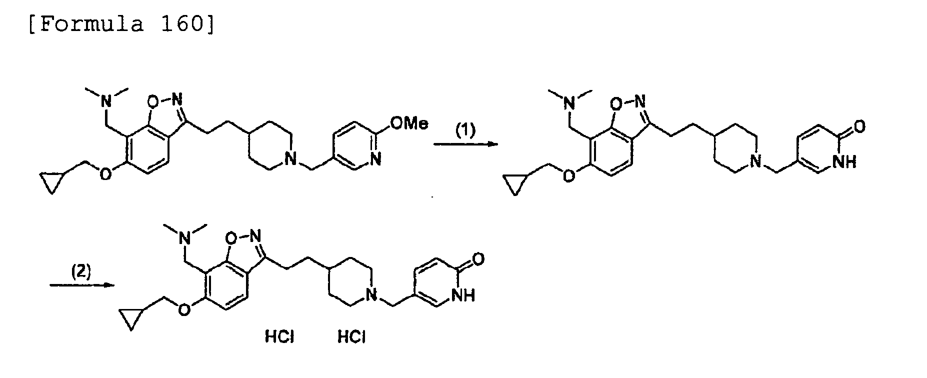

### (1) 5-{4-{2-[6-Cyclopropylmethoxy-7-(N,N-dimethylaminomethyl)benz[d]isoxazol-3-yl]ethyl}piperidinomethyl}-2(1H)-pyridone

N,N-Dimethyl{6-cyclopropylmethoxy-3-{2-[1-(6-methoxypyridin-3-ylmethyl)piperidin-4-yl]ethyl}benz[d]isoxazol-7-yl}methylamine (compound synthesized in Example 135-(1)) (100 mg) was dissolved in ethanol (5 mL). Hydrogen chloride (4 M solution in ethyl acetate) (2 mL) was added to the solution, and the mixture was heated under reflux for five hours. After cooling to room temperature, a saturated sodium carbonate solution, water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain the title compound (104 mg).
¹H-NMR (400 MHz, CDCl₃) δ(ppm): 0.35-0.39 (m, 2H), 0.62-0.66 (m, 2H), 1.22-1.40 (m, 4H), 1.62-1.82 (m, 4H), 1.86-1.96 (m, 2H), 2.33 (s, 6H), 2.80-2.88 (m, 2H), 2.90-2.98 (m, 2H), 3.22 (s, 2H), 3.82 (s, 2H), 3.94 (d, J=6.8 Hz, 2H), 6.56 (d, J=8.8 Hz, 1H), 6.90 (d, J=8.8 Hz, 1H), 7.23 (d, J=2.0 Hz, 1H), 7.45 (d, J=8.8 Hz, 1H), 7.49 (dd, J=2.0, 8.8 Hz, 1H) .
ESI-MS m/z: 465 [M+H]⁺, 487 [M+Na]⁺.

### (2) 5-{4-{2-[6-Cyclopropylmethoxy-7-(N,N-dimethylaminomethyl)benz[d]isoxazol-3-yl]ethyl}piperidinomethyl}-2(1H)-pyridone dihydrochloride

The title compound was synthesized from 5-{4-{2-[6-cyclopropylmethoxy-7-(N,N-dimethylaminomethyl)benz[d]isoxazol-3-yl]ethyl}piperidinomethyl}-2(1H)-pyridone according to Example 120-(8).
ESI-MS m/z: 233 [M+2H]²⁺, 465 [M+H]⁺.

### Example 137

### N,N-Dimethyl{6-cyclopropylmethoxy-3-[2-(1-phenethylpiperidin-4-yl)ethyl]benz[d]isoxazol-7-yl}methylamine dihydrochloride

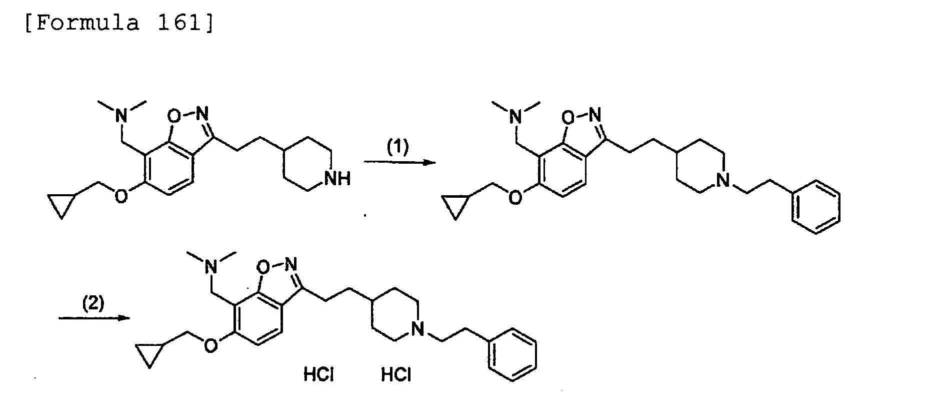

### (1) N,N-Dimethyl{6-cyclopropylmethoxy-3-[2-(1-phenethylpiperidin-4-yl)ethyl]benz[d]isoxazol-7-yl}methylamine

The title compound was synthesized from N,N-dimethyl{6-cyclopropylmethoxy-3-[2-(piperidin-4-yl)ethyl]benz[d]isoxazol-7-yl}methylamine (compound synthesized in Preparation Example 3) and phenylacetaldehyde according to Example 134-(1).
¹H-NMR (400 MHz, CDCl₃) δ(ppm): 0.35-0.39 (m, 2H), 0.62-0.67 (m, 2H), 1.28-1.42 (m, 4H), 1.75-1.85 (m, 4H), 1.96-2.04 (m, 2H), 2.34 (s, 6H), 2.55-2.62 (m, 2H), 2.78-2.84 (m, 2H), 2.93-3.05 (m, 4H), 3.83 (s, 2H), 3.95 (d, J=7.2 Hz, 2H), 6.91 (d, J=8.4 Hz, 1H), 7.18-7.22 (m, 3H), 7.26-7.72 (m, 2H), 7.46 (d, J=8.4 Hz, 1H) .
ESI-MS m/z: 462 [M+H]⁺.

### (2) N,N-Dimethyl{6-cyclopropylmethoxy-3-[2-(1-phenethylpiperidin-4-yl)ethyl]benz[d]isoxazol-7-yl}methylamine dihydrochloride

The title compound was synthesized from N,N-dimethyl{6-cyclopropylmethoxy-3-[2-(1-phenethylpiperidin-4-yl)ethyl]benz[d]isoxazol-7-yl}methylamine according to Example 120-(8).
ESI-MS m/z: 231 [M+2H]²⁺, 462 [M+H]⁺.

### Example 138

### N,N-Dimethyl{6-cyclopropylmethoxy-3-[2-(1-pyrazin-2-ylmethylpiperidin-4-yl)ethyl]benz[d]isoxazol-7-yl}methylamine trihydrochloride

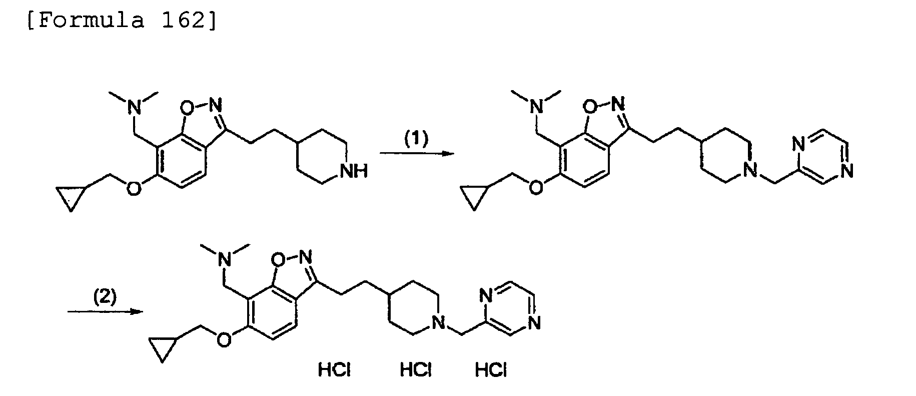

### (1) N,N-Dimethyl{6-cyclopropylmethoxy-3-[2-(1-pyrazin-2-ylmethylpiperidin-4-yl)ethyl]benz[d]isoxazol-7-yl}methylamine

The title compound was synthesized from N,N-dimethyl{6-cyclopropylmethoxy-3-[2-(piperidin-4-yl)ethyl]benz[d]isoxazol-7-yl}methylamine (compound synthesized in Preparation Example 3) and 2-pyrazinecarbaldehyde according to Example 134-(1).
¹H-NMR (400 MHz, CDCl₃) δ (ppm) : 0.35-0.39 (m, 2H), 0.62-0.67 (m, 2H), 1.24-1.42 (m, 4H), 1.74-1.82 (m, 4H), 2.02-2.12 (m, 2H), 2.33 (s, 6H), 2.86-2.95 (m, 2H), 2.94 (t, J=8.0 Hz, 2H), 3.67 (s, 2H), 3.82 (s, 2H), 3.94 (d, J=6.4 Hz, 2H), 6.89 (d, J=8.8 Hz, 1H), 8.43 (d, J=8.8 Hz, 1H), 8.44 (d, J=2.4 Hz, 1H), 8.51 (dd, J=1.6, 2.4 Hz, 1H), 8.66 (d, J=1.6 Hz, 1H).
ESI-MS m/z: 450 [M+H]⁺, 472 [M+Na]⁺.

### (2) N,N-Dimethyl{6-cyclopropylmethoxy-3-[2-(1-pyrazin-2-ylmethylpiperidin-4-yl)ethyl]benz[d]isoxazol-7-yl}methylamine trihydrochloride

The title compound was synthesized from N,N-dimethyl{6-cyclopropylmethoxy-3-[2-(1-pyrazin-2-ylmethylpiperidin-4-yl)ethyl]benz[d]isoxazol-7-yl}methylamine according to Example 120-(8).
ESI-MS m/z: 225 [M+2H]²⁺, 450 [M+H]⁺.

### Example 139

### 3-{4-{2-[6-Cyclopropylmethoxy-7-(N,N-dimethylaminomethyl)benz[d]isoxazol-3-yl]ethyl}piperidinomethyl}-2(1H)-pyridone dihydrochloride

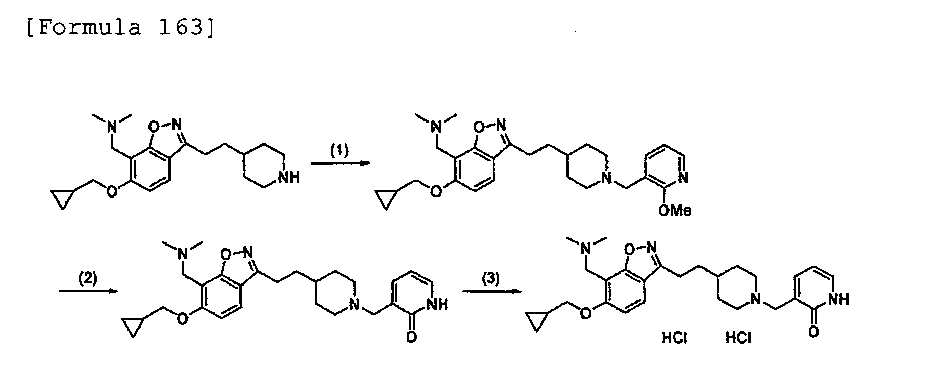

### (1) N,N-Dimethyl{6-cyclopropylmethoxy-3-{2-[1-(2-methoxypyridin-3-ylmethyl)piperidin-4-yl]ethyl}benz[d]isoxazol-7-yl}methylamine

The title compound was synthesized from N,N-dimethyl{6-cyclopropylmethoxy-3-[2-(piperidin-4-yl)ethyl]benz[d]isoxazol-7-yl}methylamine (compound synthesized in Preparation Example 3) and 2-methoxypyridine-3-carbaldehyde according to Example 134-(1).
¹H-NMR (400 MHz, CDCl₃) δ(ppm): 0.35-0.40 (m, 2H), 0.62-0.66 (m, 2H), 1.26-1.40 (m, 4H), 1.68-1.80 (m, 4H), 2.00-2.09 (m, 2H), 2.04 (s, 6H), 2.85-2.93 (m, 2H), 2.94 (t, J=7.8 Hz, 2H), 3.47 (s, 2H), 3.82 (s, 2H), 3.935 (d, J=6.4 Hz, 2H), 3.937 (s, 3H), 6.85 (dd, J=5.2, 7.2 Hz, 1H), 6.89 (d, J=8.6 Hz, 1H), 7.43 (d, J=8.6 Hz, 1H), 7.63 (dd, J=2.0, 7.2 Hz, 1H), 8.04 (dd, J=2.0, 5.2 Hz, 1H).
ESI-MS m/z: 479 [M+H]⁺.

### (2) 3-{4-{2-[6-Cyclopropylmethoxy-7-(N,N-dimethylaminomethyl)benz[d]isoxazol-3-yl]ethyl}piperidinomethyl}-2(1H)-pyridone

The title compound was synthesized from N,N-dimethyl{6-cyclopropylmethoxy-3-{2-[1-(2-methoxypyridin-3-ylmethyl)piperidin-4-yl]ethyl}benz[d]isoxazol-7-yl}methylamine according to Example 136- (1) .
¹H-NMR (400 MHz, CDCl₃) δ(ppm): 0.36-0.40 (m, 2H), 0.62-0.67 (m, 2H), 1.23-1.43 (m, 4H), 1.76-1.82 (m, 4H), 2.03-2.12 (m, 2H), 2.34 (s, 6H), 2.90-3.00 (m, 4H), 3.47 (s, 2H), 3.83 (s, 2H), 3.94 (d, J=6.4 Hz, 2H), 6.33 (dd, J=6.4, 6.8 Hz, 1H), 6.90 (d, J=8.8 Hz, 1H), 7.36 (d, J=6.4 Hz, 1H), 7.45 (d, J=8.8 Hz, 1H), 7.54 (dd, J=2.0, 6.8 Hz, 1H) .
ESI-MS m/z: 233 [M+2H]²⁺, 465 [M+H]⁺.

### (3) 3-{4-{2-[6-Cyclopropylmethoxy-7-(N,N-dimethylaminomethyl)benz[d]isoxazol-3-yl]ethyl}piperidinomethyl}-2(1H)-pyridone dihydrochloride

The title compound was synthesized from 3-{4-{2-[6-cyclopropylmethoxy-7-(N,N-dimethylaminomethyl)benz[d]isoxazol-3-yl]ethyl}piperidinomethyl}-2(1H)-pyridone according to Example 120-(8).
ESI-MS m/z: 233 [M+2H]²⁺, 465 [M+H]⁺.

### Example 140

### 3-{2-{4-{2-[6-Cyclopropylmethoxy-7-(N,N-dimethylaminomethyl)benz[d]isoxazol-3-yl]ethyl}piperidino}ethyl}benzonitrile dihydrochloride

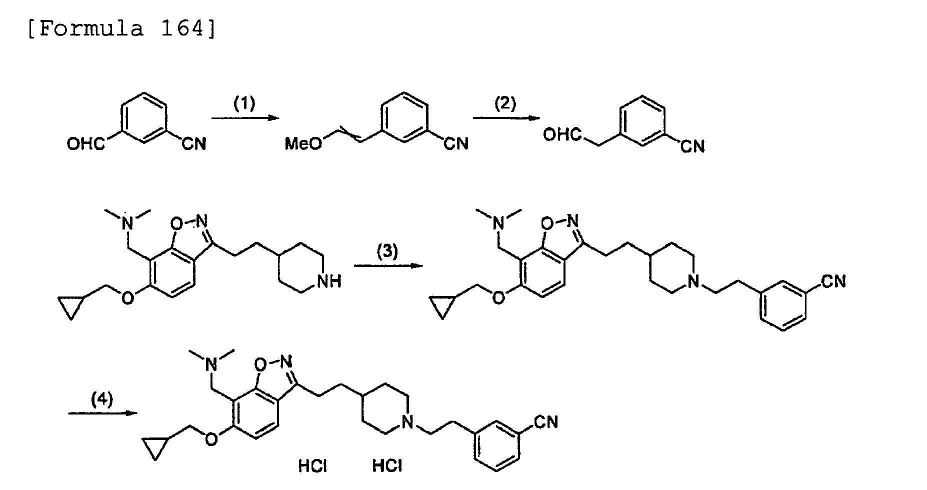

### (1) Mixture of (E)-3-(2-methoxyvinyl)benzonitrile and (Z)-3-(2-methoxyvinyl)benzonitrile

(Methoxymethyl)triphenylphosphonium chloride (2 g) was suspended in tetrahydrofuran (20 mL), and Potassium tert-butoxide (641 mg) was added under ice-cooling. The reaction mixture was stirred under ice-cooling for 20 minutes, and a solution of 3-formylbenzonitrile (500 mg) in tetrahydrofuran (10 mL) was further added. After dropwise addition, the mixture was stirred at room temperature for 1.5 hours. Water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain a residue. The residue was purified by NH silica gel column chromatography (heptane-ethyl acetate) to obtain the title compound (495 mg) .

### (2) 3-(2-Formylmethyl)benzonitrile

The mixture of (E)-3-(2-methoxyvinyl)benzonitrile and (Z)-3-(2-methoxyvinyl)benzonitrile (496 mg) was dissolved in tetrahydrofuran (10 mL). 5 M hydrochloric acid (3.1 mL) was added and the mixture was heated with stirring at 80°C for 1.5 hours. After cooling to room temperature, a saturated sodium carbonate solution, water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain a crude product (551 mg). The resulting crude product was used for the next reaction without further purification.

### (3) 3-{2-{4-{2-[6-Cyclopropylmethoxy-7-(N,N-dimethylaminomethyl)benz[d]isoxazol-3-yl]ethyl}piperidino}ethyl}benzonitrile

The title compound was synthesized from N,N-dimethyl{6-cyclopropylmethoxy-3-[2-(piperidin-4-yl)ethyl]benz[d]isoxazol-7-yl}methylamine (compound synthesized in Preparation Example 3) and 3-(2-formylmethyl)benzonitrile according to Example 134-(1). ¹H-NMR (400 MHz, CDCl₃) δ (ppm) : 0.35-0.40 (m, 2H), 0.62-0.67 (m, 2H), 1.24-1.42 (m, 4H), 1.76-1.84 (m, 4H), 1.95-2.05 (m, 2H), 2.34 (s, 6H), 2.52-2.59 (m, 2H), 2.80-2.86 (m, 2H), 2.93-3.01 (m, 4H), 3.83 (s, 2H), 3.95 (d, J=6.8 Hz, 2H), 6.91 (d, J=8.8 Hz, 1H), 7.36-7.51 (m, 5H).
ESI-MS m/z: 244 [M+2H]²⁺, 487 [M+H]⁺.

### (4) 3-{2-{4-{2-[6-Cyclopropylmethoxy-7-(N,N-dimethylaminomethyl)benz[d]isoxazol-3-yl]ethyl}piperidino}ethyl}benzonitrile dihydrochloride

The title compound was synthesized from 3-{2-{4-{2-[6-cyclopropylmethoxy-7-(N,N-dimethylaminomethyl)benz[d]isoxazol-3-yl]ethyl}piperidino}ethyl}benzonitrile according to Example 120-(8).
ESI-MS m/z: 244 [M+2H]²⁺, 487 [M+H]⁺.

### Example 141

### N,N-Dimethyl{6-cyclopropylmethoxy-3-{2-{1-[2-(pyridin-2-yl)ethyl]piperidin-4-yl}ethyl}benz[d]isoxazol-7-yl}methylamine trihydrochloride

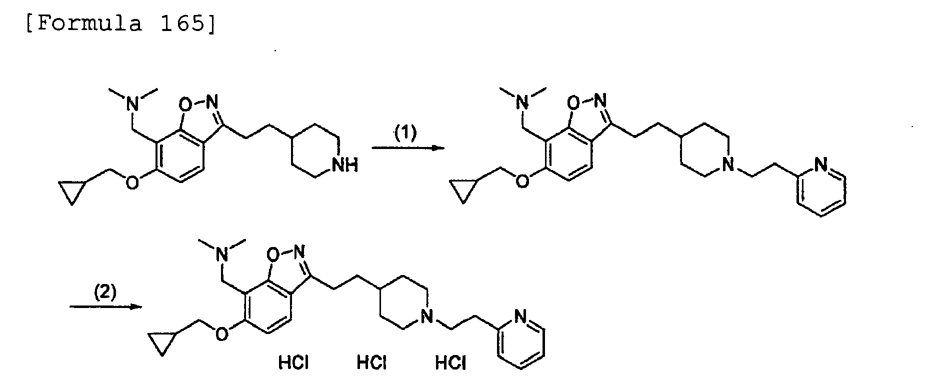

### (1) N,N-Dimethyl{6-cyclopropylmethoxy-3-{2-{1-[2-(pyridin-2-yl)ethyl]piperidin-4-yl}ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine (compound synthesized in Preparation Example 3) (120 mg) and potassium carbonate (36 mg) were dissolved in ethanol (5 mL), and the mixture was heated under reflux for six hours. Sodium acetate (50 mg) and water (1 mL) were further added, and the mixture was further heated under reflux overnight. After cooling to room temperature, a saturated sodium carbonate solution, water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain a residue. The residue was purified by NH silica gel column chromatography (heptane-ethyl acetate) to obtain the title compound (47 mg).
¹H-NMR (400 MHz, CDCl₃) δ(ppm): 0.35-0.41 (m, 2H), 0.62-0.68 (m, 2H), 1.24-1.42 (m, 4H), 1.76-1.84 (m, 4H), 2.00-2.07 (m, 2H), 2.34 (s, 6H), 2.72-2.78 (m, 2H), 2.92-3.03 (m, 6H), 3.83 (s, 2H), 3.95 (d, J=6.8 Hz, 2H), 6.90 (d, J=8.4 Hz, 1H), 7.12 (dd, J=4.8, 5.6 Hz, 1H), 7.19 (d, J=7.6 Hz, 1H), 7.45 (d, J=8.4 Hz, 1H), 7.59 (dd, J=5.6, 7.6 Hz, 1H), 8.53 (d, J=4.8 Hz, 1H) . ESI-MS m/z: 232 [M+2H]²⁺, 463 [M+H]⁺.

### (2) N,N-Dimethyl{6-cyclopropylmethoxy-3-{2-{1-[2-(pyridin-2-yl)ethyl]piperidin-4-yl}ethyl}benz[d]isoxazol-7-yl}methylamine trihydrochloride

The title compound was synthesized from N,N-dimethyl{6-cyclopropylmethoxy-3-{2-{1-[2-(pyridin-2-yl)ethyl]piperidin-4-yl}ethyl}benz[d]isoxazol-7-yl}methylamine according to Example 120-(8).
ESI-MS m/z: 232 [M+2H]²⁺, 463 [M+H]⁺.

### Example 142

### N,N-Dimethyl{6-cyclopropylmethoxy-3-{2-[1-(2-methylthiazol-4-ylmethyl)piperidin-4-yl]ethyl}benz[d]isoxazol-7-yl}methylamine dihydrochloride

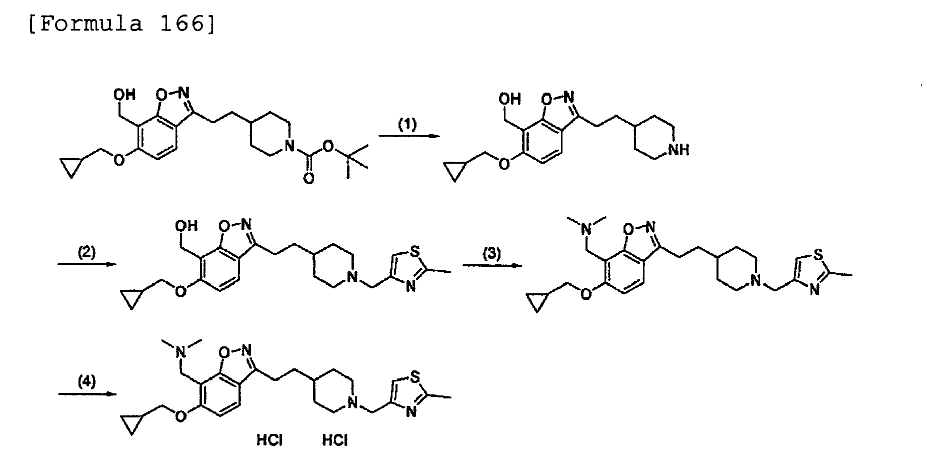

### (1) {6-Cyclopropylmethoxy-3-[2-(piperidin-4-yl)ethyl]benz[d]isoxazol-7-yl}methanol

tert-Butyl 4-[2-(6-cyclopropylmethoxy-7-hydroxymethylbenz[d]isoxazol-3-yl)ethyl]piperidine-1-carboxylate (compound synthesized in Preparation Example 3-(1)) (5 g) was dissolved in methanol (50 mL). Hydrogen chloride (4 M solution in ethyl acetate) (14.5 mL) was added to the solution, and the mixture was stirred at room temperature for 24 hours. Methanol was evaporated under reduced pressure to obtain a residue. A saturated sodium carbonate solution, water and chloroform were added to the residue, and the organic layer was separated. The organic layer was washed with a saturated sodium chloride solution and then dried over anhydrous sodium sulfate. The drying agent was removed by filtration and then the organic layer was concentrated under reduced pressure to obtain a residue. The residue was purified by NH silica gel column chromatography (chloroform-methanol-aqueous ammonia) to obtain the title compound (2.58 g).
¹H-NMR (400 MHz, CDCl₃) δ (ppm) : 0.36-0.41 (m, 2H), 0.64-0.70 (m, 2H), 1.10-1.23 (m, 2H), 1.25-1.38 (m, 1H), 1.40-1.53 (m, 1H), 1.71-1.81 (m, 4H), 2.58 (dt, J=2.4, 12.2 Hz, 2H), 2.96 (t, J=8.0 Hz, 2H), 3.03-3.10 (m, 2H), 3.98 (d, J=6.4 Hz, 2H), 5.04 (s, 2H), 6.91 (d, J=8.6 Hz, 1H), 7.48 (d, J=8.6 Hz, 1H).
ESI-MS m/z: 331 [M+H]⁺.

### (2) {6-Cyclopropylmethoxy-3-{2-[1-(2-methylthiazol-4-ylmethyl)piperidin-4-yl]ethyl}benz[d]isoxazol-7-yl}methanol

{6-Cyclopropylmethoxy-3-[2-(piperidin-4-yl)ethyl]benz[d]isoxazol-7-yl}methanol (200 mg) and 4-chloromethyl-2-methylthiazole (500 mg) were dissolved in N,N-dimethylformamide (7 mL). Potassium carbonate (168 mg) was added to the solution, and the mixture was heated with stirring in a nitrogen atmosphere at 90°C for one hour. After cooling to room temperature, water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain a residue. The residue was purified by NH silica gel column chromatography (heptane-ethyl acetate) to obtain the title compound (96 mg).
¹H-NMR (400 MHz, CDCl₃) δ(ppm): 0.37-0.40 (m, 2H), 0.66-0.70 (m, 2H), 1.28-1.43 (m, 5H), 1.63-1.83 (m, 4H), 1.96-2.03 (m, 2H), 2.70 (s, 3H), 2.81-2.98 (m, 4H), 3.59 (s, 2H), 3.97 (d, J=6.8 Hz, 2H), 5.04 (s, 2H), 6.91 (d, J=8.8 Hz, 1H), 6.93 (s, 1H), 7.47 (d, J=8.8 Hz, 1H) .
ESI-MS m/z: 442 [M+H]⁺.

### (3) N,N-Dimethyl{6-cyclopropylmethoxy-3-{2-[1-(2-methylthiazol-4-ylmethyl)piperidin-4-yl]ethyl}benz[d]isoxazol-7-yl}methylamine

The title compound was synthesized from {6-cyclopropylmethoxy-3-{2-[1-(2-methylthiazol-4-ylmethyl)piperidin-4-yl]ethyl}benz[d]isoxazol-7-yl}methanol according to Example 120-(5).
¹H-NMR (400 MHz, CDCl₃) δ (ppm) : 0.36-0.40 (m, 2H), 0.62-0.66 (m, 2H), 1.24-1.44 (m, 4H), 1.70-1.82 (m, 4H), 1.96-2.04 (m, 2H), 2.33 (s, 6H), 2.70 (s, 3H), 2.90-2.98 (m, 4H), 3.59 (s, 2H), 3.82 (s, 2H), 3.94 (d, J=6.4 Hz, 2H), 6.90 (d, J=8.4 Hz, 1H), 6.93 (s, 1H), 7.44 (d, J=8.4 Hz, 1H) .
ESI-MS m/z: 235 [M+2H]²⁺, 469 [M+H]⁺.

### (4) N,N-Dimethyl{6-cyclopropylmethoxy-3-{2-[1-(2-methylthiazol-4-ylmethyl)piperidin-4-yl]ethyl}benz[d]isoxazol-7-yl}methylamine dihydrochloride

The title compound was synthesized from N,N-dimethyl{6-cyclopropylmethoxy-3-{2-[1-(2-methylthiazol-4-ylmethyl)piperidin-4-yl]ethyl}benz[d]isoxazol-7-yl}methylamine according to Example 120-(8).
ESI-MS m/z: 235 [M+2H]²⁺, 469 [M+H]⁺.

### Example 143

### N,N-Dimethyl{6-cyclopropylmethoxy-3-[2-(1-thiophen-3-ylmethylpiperidin-4-yl)ethyl]benz[d]isoxazol-7-yl}methylamine dihydrochloride

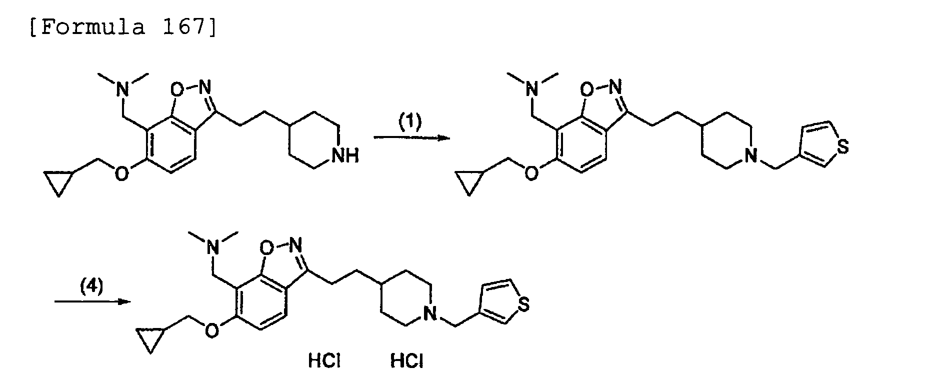

### (1) N,N-Dimethyl{6-cyclopropylmethoxy-3-[2-(1-thiophen-3-ylmethylpiperidin-4-yl)ethyl]benz[d]isoxazol-7-yl}methylamine

The title compound was synthesized from N,N-dimethyl{6-cyclopropylmethoxy-3-[2-(piperidin-4-yl)ethyl]benz[d]isoxazol-7-yl}methylamine (compound synthesized in Preparation Example 3) and thiophene-3-carbaldehyde according to Example 134-(1).
¹H-NMR (400 MHz, CDCl₃) δ(ppm): 0.35-0.39 (m, 2H), 0.62-0.66 (m, 2H), 1.24-1.38 (m, 4H), 1.71-1.80 (m, 4H), 1.88-1.98 (m, 2H), 2.33 (s, 6H), 2.85-2.97 (m, 4H), 3.52 (s, 2H), 3.82 (s, 2H), 3.94 (d, J=6.8 Hz, 2H), 6.89 (d, J=8.4 Hz, 1H), 7.05 (d, J=4.8 Hz, 1H), 7.10 (s, 1H), 7.22-7.28 (m, 1H), 7.44 (d, J=8.4 Hz, 1H).
ESI-MS m/z: 227 [M+2H]²⁺, 454 [M+H]⁺.

### (2) N,N-Dimethyl{6-cyclopropylmethoxy-3-[2-(1-thiophen-3-ylmethylpiperidin-4-yl)ethyl]benz[d]isoxazol-7-yl}methylamine dihydrochloride

The title compound was synthesized from N,N-dimethyl{6-cyclopropylmethoxy-3-[2-(1-thiophen-3-ylmethylpiperidin-4-yl)ethyl]benz[d]isoxazol-7-yl}methylamine according to Example 120-(8).
ESI-MS m/z: 227 [M+2H]²⁺, 454 [M+H]⁺.

### Example 144

### N,N-Dimethyl{6-cyclopropylmethoxy-3-[2-(1-furan-3-ylmethylpiperidin-4-yl)ethyl]benz[d]isoxazol-7-yl}methylamine dihydrochloride

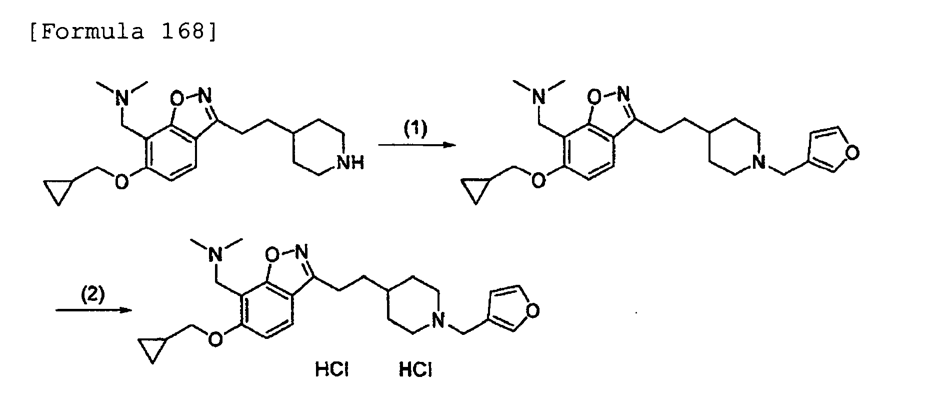

### (1) N,N-Dimethyl{6-cyclopropylmethoxy-3-[2-(1-furan-3-ylmethylpiperidin-4-yl)ethyl]benz[d]isoxazol-7-yl}methylamine

The title compound was synthesized from N,N-dimethyl{6-cyclopropylmethoxy-3-[2-(piperidin-4-yl)ethyl]benz[d]isoxazol-7-yl}methylamine (compound synthesized in Preparation Example 3) and furan-3-carbaldehyde according to Example 134-(1).
¹H-NMR (400 MHz, CDCl₃) δ (ppm) : 0.35-0.39 (m, 2H), 0.62-0.66 (m, 2H), 1.25-1.40 (m, 4H), 1.72-1.85 (m, 4H), 1.87-1.97 (m, 2H), 2.33 (s, 6H), 2.88-2.96 (m, 4H), 3.36 (s, 2H), 3.82 (s, 2H), 3.94 (d, J=6.8 Hz, 2H), 6.38 (d, J=1.2 Hz, 1H), 6.90 (d, J=8.8 Hz, 1H), 7.32 (s, 1H), 7.37-7.38 (m, 1H), 7.44 (d, J=8.8 Hz, 1H).
ESI-MS m/z: 219 [M+2H]²⁺, 438 [M+H]⁺.

### (2) N,N-Dimethyl{6-cyclopropylmethoxy-3-[2-(1-furan-3-ylmethylpiperidin-4-yl)ethyl]benz[d]isoxazol-7-yl}methylamine dihydrochloride

The title compound was synthesized from N,N-dimethyl{6-cyclopropylmethoxy-3-[2-(1-furan-3-ylmethylpiperidin-4-yl)ethyl]benz[d]isoxazol-7-yl}methylamine according to Example 120-(8).
ESI-MS m/z: 219 [M+2H]²⁺, 438 [M+H]⁺.

### Example 145

### N,N-Dimethyl{3-{2-[1-(4-chlorobenzyl)piperidin-4-yl]ethyl}-6-cyclopropylmethoxybenz[d]isoxazol-7-yl}methylamine dihydrochloride

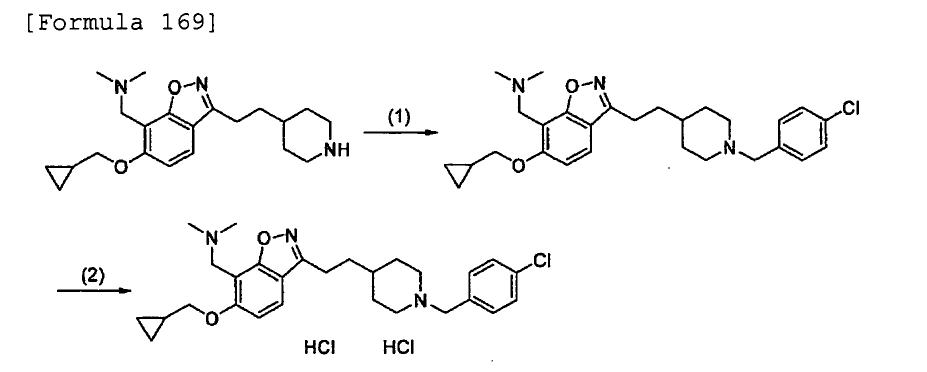

### (1) N,N-Dimethyl{3-{2-[1-(4-chlorobenzyl)piperidin-4-yl]ethyl}-6-cyclopropylmethoxybenz[d]isoxazol-7-yl}methylamine

The title compound was synthesized from N,N-dimethyl{6-cyclopropylmethoxy-3-[2-(piperidin-4-yl)ethyl]benz[d]isoxazol-7-yl}methylamine (compound synthesized in Preparation Example 3) and 4-chlorobenzaldehyde according to Example 134-(1).
¹H-NMR (400 MHz, CDCl₃) δ(ppm): 0.35-0.40 (m, 2H), 0.62-0.67 (m, 2H), 1.25-1.40 (m, 4H), 1.70-1.81 (m, 4H), 1.88-1.96 (m, 2H), 2.33 (s, 6H), 2.81-2.87 (m, 2H), 3.93 (t, J=8.0 Hz, 2H), 3.43 (s, 2H), 3.82 (s, 2H), 3.94 (d, J=6.8 Hz, 2H), 6.89 (d, J=8.8 Hz, 1H), 7.22-7.34 (m, 4H), 7.44 (d, J=8.8 Hz, 1H) .
ESI-MS m/z: 241 [M+2H]²⁺, 482 [M+H]⁺.

### (2) N,N-Dimethyl{3-{2-[1-(4-chlorobenzyl)piperidin-4-yl]ethyl}-6-cyclopropylmethoxybenz[d]isoxazol-7-yl}methylamine dihydrochloride

The title compound was synthesized from N,N-dimethyl{3-{2-[1-(4-chlorobenzyl)piperidin-4-yl]ethyl}-6-cyclopropylmethoxybenz[d]isoxazol-7-yl}methylamine according to Example 120-(8).
ESI-MS m/z: 241 [M+2H]²⁺, 482 [M+H]⁺.

### Example 146

### 5-{4-{2-[6-Cyclopropylmethoxy-7-(N,N-dimethylaminomethyl)benz[d]isoxazol-3-yl]ethyl}piperidinomethyl}-2-fluorobenzonitrile dihydrochloride

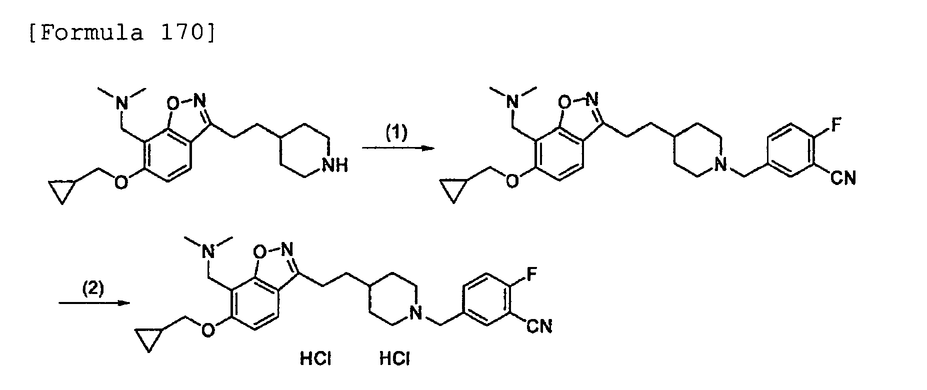

### (1) 5-{4-{2-[6-Cyclopropylmethoxy-7-(N,N-dimethylaminomethyl)benz[d]isoxazol-3-yl]ethyl}piperidinomethyl}-2-fluorobenzonitrile

The title compound was synthesized from N,N-dimethyl{6-cyclopropylmethoxy-3-[2-(piperidin-4-yl)ethyl]benz[d]isoxazol-7-yl}methylamine (compound synthesized in Preparation Example 3) and 2-fluoro-5-formylbenzonitrile according to Example 134-(1).
¹H-NMR (400 MHz, CDCl₃) δ(ppm): 0.36-0.40 (m, 2H), 0.62-0.66 (m, 2H), 1.23-1.43 (m, 4H), 1.69-1.81 (m, 4H), 1.92-2.00 (m, 2H), 2.34 (s, 6H), 2.77-2.83 (m, 2H), 2.95 (t, J=7.8 Hz, 2H), 3.44 (s, 2H), 3.82 (s, 2H), 3.94 (d, J=6.8 Hz, 2H), 6.90 (d, J=8.8 Hz, 1H), 7.15 (t, J=8.8 Hz, 1H), 7.45 (d, J=8.8 Hz, 1H), 7.51-7.57 (m, 1H), 7.59-7.62 (m, 1H) .
ESI-MS m/z: 246 [M+2H]²⁺, 491 [M+H]⁺.

### (2) 5-{4-{2-[6-Cyclopropylmethoxy-7-(N,N-dimethylaminomethyl)benz[d]isoxazol-3-yl]ethyl}piperidinomethyl}-2-fluorobenzonitrile dihydrochloride

The title compound was synthesized from 5-{4-{2-[6-cyclopropylmethoxy-7-(N,N-dimethylaminomethyl)benz[d]isoxazol-3-yl]ethyl}piperidinomethyl}-2-fluorobenzonitrile according to Example 120-(8).
¹H-NMR (400 MHz, DMSO-d₆) δ(ppm): 0.39-0.42 (m, 2H), 0.58-0.63 (m, 2H), 1.16-1.24 (m, 1H), 1.50-1.80 (m, 5H), 1.84-1.95 (m, 2H), 2.76-2.90 (m, 8H), 2.99 (t, J=7.6 Hz, 2H), 3.28-3.35 (m, 2H), 4.07 (d, J=7.2 Hz, 2H), 4.29-4.31 (m, 2H), 4.43-4.47 (m, 2H), 7.23 (d, J=8.8 Hz, 1H), 7.63 (dd, J=9.2, 9.2 Hz, 1H), 7.95 (d, J=8.8 Hz, 1H), 8.04-8.09 (m, 1H), 8.21-8.24 (m, 1H), 10.45 (br s, 1H), 11.20 (br s, 1H).
ESI-MS m/z: 246 [M+2H]²⁺, 491 [M+H]⁺.

### Example 147

### 6-{4-{2-[6-Cyclopropylmethoxy-7-(N,N-dimethylaminomethyl)benz[d]isoxazol-3-yl]ethyl}piperidinomethyl}-2-methylsulfanylpyridine-3-carbonitrile dihydrochloride

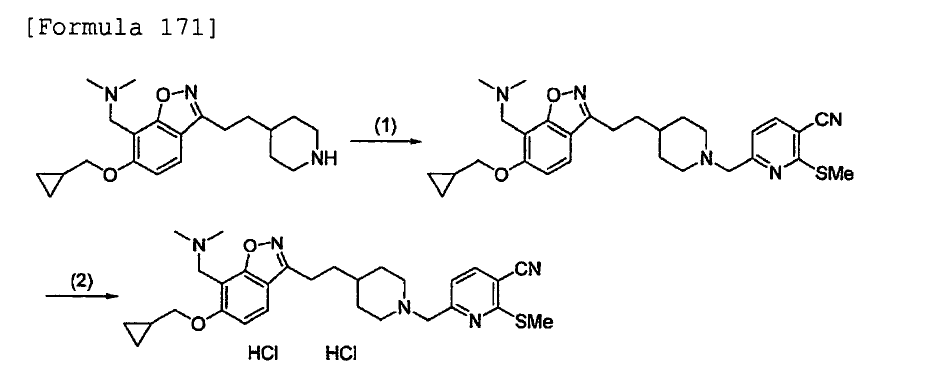

### (1) 6-{4-{2-[6-Cyclopropylmethoxy-7-(N,N-dimethylaminomethyl)benz[d]isoxazol-3-yl]ethyl}piperidinomethyl}-2-methylsulfanylpyridine-3-carbonitrile

The title compound was synthesized from N,N-dimethyl{6-cyclopropylmethoxy-3-[2-(piperidin-4-yl)ethyl]benz[d]isoxazol-7-yl}methylamine (compound synthesized in Preparation Example 3) and 6-formyl-2-(methylsulfanyl)pyridine-3-carbonitrile according to Example 134-(1).
¹H-NMR (400 MHz, CDCl₃) δ(ppm): 0.36-0.42 (m, 2H), 0.62-0.67 (m, 2H), 1.25-1.42 (m, 4H), 1.74-1.82 (m, 4H), 2.04-2.14 (m, 2H), 2.35 (s, 6H), 2.62 (s, 3H), 2.84-2.92 (m, 2H), 2.95 (t, J=7.8 Hz, 2H), 3.66 (s, 2H), 3.84 (s, 2H), 3.95 (d, J=7.2 Hz, 2H), 6.91 (d, J=8.8 Hz, 1H), 7.25 (d, J=7.6 Hz, 1H), 7.46 (d, J=8.8 Hz, 1H) 7.74 (d, J=7.6 Hz, 1H).
ESI-MS m/z: 260 [M+2H]²⁺, 520 [M+H]⁺, 542 [M+Na]⁺.

### (2) 6-{4-{2-[6-Cyclopropylmethoxy-7-(N,N-dimethylaminomethyl)benz[d]isoxazol-3-yl]ethyl}piperidinomethyl}-2-methylsulfanylpyridine-3-carbonitrile trihydrochloride

The title compound was synthesized from 6-{4-{2-[6-cyclopropylmethoxy-7-(N,N-dimethylaminomethyl)benz[d]isoxazol-3-yl]ethyl}piperidinomethyl}-2-methylsulfanylpyridine-3-carbonitrile according to Example 120-(8).
¹H-NMR (400 MHz, DMSO-d₆) δ(ppm): 0.38-0.43 (m, 2H), 0.58-0.63 (m, 2H), 1.30-1.36 (m, 1H), 1.57-1.80 (m, 5H), 1.82-2.00 (m, 2H), 2.68 (s, 3H), 2.77-2.80 (m, 6H), 2.97-3.09 (m, 4H), 3.40-3.65 (m, 2H), 4.70 (d, J=7.2 Hz, 2H), 4.45-4.53 (m, 4H), 7.24 (d, J=8.8 Hz, 1H), 7.53 (d, J=8.0 Hz, 1H), 7.96 (d, J=8.8 Hz, 1H), 8.30 (d, J=8.0 Hz, 1H), 10.31 (br s, 1H), 10.67 (br s, 1H) .
ESI-MS m/z: 260 [M+2H]²⁺, 520 [M+H]⁺.

### Example 148

### 3-{4-{2-[6-Cyclopropylmethoxy-7-(N,N-dimethylaminomethyl)benz[d]isoxazol-3-yl]ethyl}piperidinomethyl}-6-dimethylamino-2-fluorobenzonitrile trihydrochloride

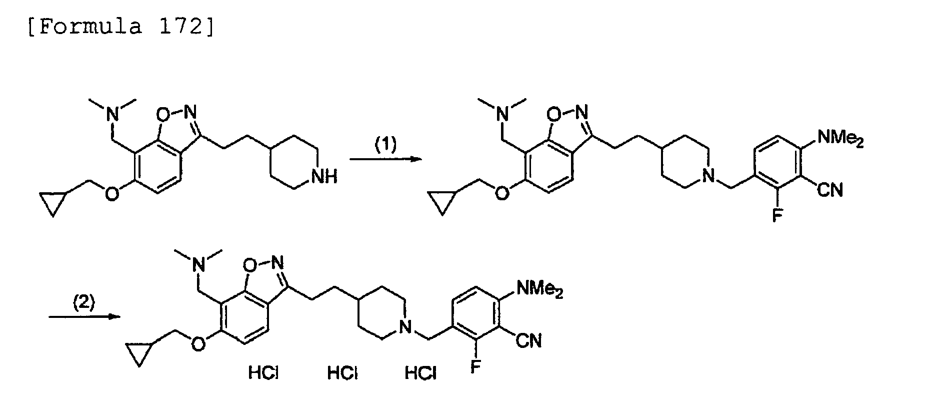

### (1) 3-{4-{2-[6-Cyclopropylmethoxy-7-(N,N-dimethylaminomethyl)benz[d]isoxazol-3-yl]ethyl}piperidinomethyl}-6-dimethylamino-2-fluorobenzonitrile

The title compound was synthesized from N,N-dimethyl{6-cyclopropylmethoxy-3-[2-(piperidin-4-yl)ethyl]benz[d]isoxazol-7-yl}methylamine (compound synthesized in Preparation Example 3) and 6-dimethylamino-2-fluoro-3-formylbenzonitrile according to Example 134-(1).
¹H-NMR (400 MHz, CDCl₃) δ(ppm): 0.36-0.40 (m, 2H), 0.62-0.66 (m, 2H), 1.23-1.40 (m, 4H), 1.70-1.82 (m, 4H), 1.94-2.02 (m, 2H), 2.34 (s, 6H), 2.83-2.89 (m, 2H), 2.93 (t, J=7.8 Hz, 2H), 3.09 (s, 6H), 3.46 (d, J=0.8 Hz, 2H), 3.83 (s, 2H), 3.94 (d, J=6.8 Hz, 2H), 6.58 (d, J=8.8 Hz, 1H), 6.90 (d, J=8.4 Hz, 1H), 7.36 (t, J=8.4 Hz, 1H), 7.44 (d, J=8.8 Hz, 1H) .
ESI-MS m/z: 534 [M+H]⁺, 556 [M+Na]⁺.

### (2) 3-{4-{2-[6-Cyclopropylmethoxy-7-(N,N-dimethylaminomethyl)benz[d]isoxazol-3-yl]ethyl}piperidinomethyl}-6-dimethylamino-2-fluorobenzonitrile trihydrochloride

The title compound was synthesized from 3-{4-{2-[6-cyclopropylmethoxy-7-(N,N-dimethylaminomethyl)benz[d]isoxazol-3-yl]ethyl}piperidinomethyl}-6-dimethylamino-2-fluorobenzonitrile according to Example 120-(8).
ESI-MS m/z: 534 [M+H]⁺.

### Example 149

### 5-{4-{2-[7-(N,N-Dimethylaminomethyl)-6-(pyridin-2-yl)benz[d]isoxazol-3-yl]ethyl}piperidinomethyl}thiophene-2-carbonitrile trihydrochloride

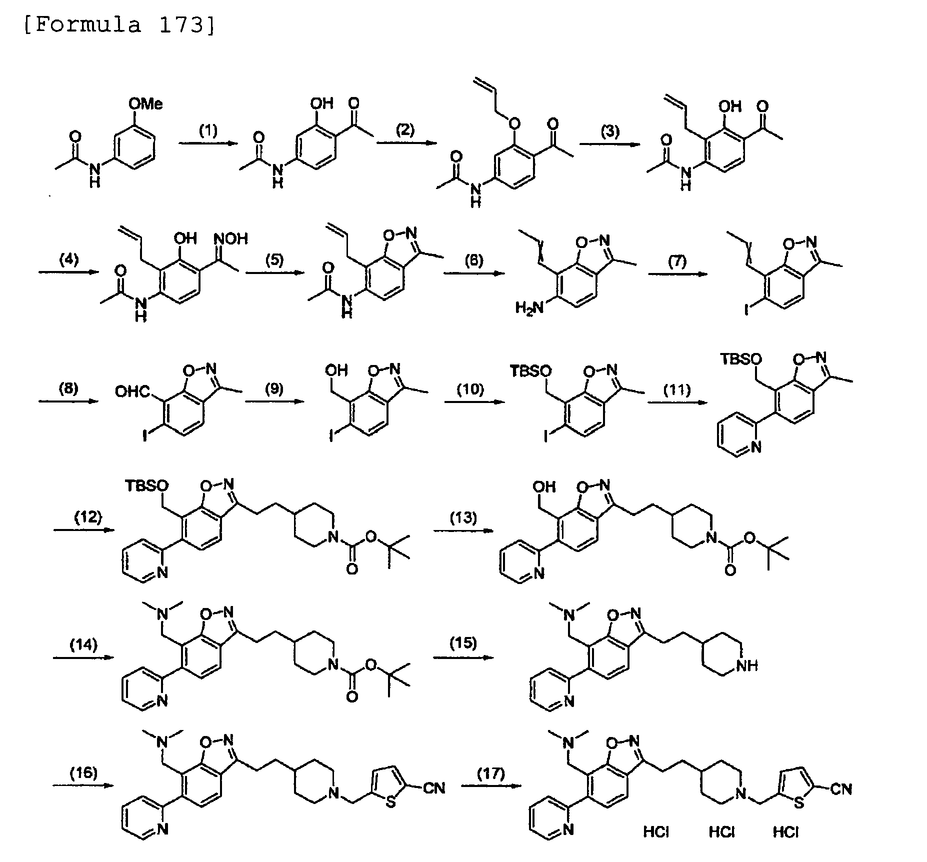

### (1) N-(4-Acetyl-3-hydroxyphenyl)acetamide

Aluminum chloride (48.4 g) was suspended in dichloromethane (400 mL). Acetyl Chloride (25.8 mL) was added under ice-cooling, and the mixture was stirred for 20 minutes. Thereafter, a solution of N-(3-methoxyphenyl)acetamide [CAS Registry No. 588-16-9] (20 g) in dichloromethane (100 mL) was added dropwise under ice-cooling over 10 minutes. The reaction mixture was stirred under ice-cooling for 1.5 hours and further heated under reflux for seven hours. After cooling to room temperature, the reaction mixture was poured into ice water. The precipitate was collected by filtration and dried under reduced pressure to obtain the title compound (26.4 g). The organic layer was separated from the filtrate by adding 10% methanol-dichloromethane and dried over anhydrous sodium sulfate. The drying agent was removed by filtration and then the organic layer was concentrated under reduced pressure to obtain a residue. The residue was further purified by silica gel column chromatography (heptane-ethyl acetate) to obtain the title compound (3.6 g).
¹H-NMR (400 MHz, DMSO-d₆) δ(ppm): 2.08 (s, 3H), 2.56 (s, 3H), 7.05 (dd, J=2.0, 8.8 Hz, 1H), 7.35 (d, J=2.0 Hz, 1H), 7.84 (d, J=8.8 Hz, 1H), 10.29 (br s, 1H), 12.32 (s, 1H).

### (2) N-(4-Acetyl-3-allyloxyphenyl)acetamide

N-(4-Acetyl-3-hydroxyphenyl)acetamide (3.5 g) and allyl bromide (3.2 mL) were dissolved in N,N-dimethylformamide (30 mL). Potassium carbonate (5 g) was added and the mixture was heated with stirring at 80°C for 20 hours. Water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain a residue. The residue was purified by silica gel column chromatography (heptane-ethyl acetate) to obtain the title compound (2.9 g).
¹H-NMR (400 MHz, CDCl₃) δ(ppm): 2.21 (s, 3H), 2.62 (s, 3H), 4.65-4.68 (m, 2H), 5.32-5.36 (m, 1H), 5.43-5.50 (m, 1H), 6.06-6.15 (m, 1H), 6.71 (dd, J=2.0, 8.8 Hz, 1H), 7.35 (br s, 1H), 7.76 (d, J=2.0 Hz, 1H), 7.77 (d, J=8.8 Hz, 1H).

### (3) N-(4-Acetyl-2-allyl-3-hydroxyphenyl)acetamide

N-(4-Acetyl-3-allyloxyphenyl)acetamide (2.7 g) was dissolved in N-methyl-2-pyrrolidone (25 mL), and the solution was heated with stirring using a microwave reactor at 180°C for 10 minutes. After cooling to room temperature, water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain a residue. The residue was purified by silica gel column chromatography (heptane-ethyl acetate) to obtain a crude product (3.9 g). The resulting crude product was used for the next reaction without further purification.
¹H-NMR (400 MHz, CDCl₃) δ(ppm): 2.18 (s, 3H), 2.61 (s, 3H), 3.49-3.53 (m, 2H), 5.10-5.24 (m, 2H), 5.90-6.00 (m, 1H), 7.48 (br s, 1H), 7.63-7.68 (m, 1H), 7.70-7.80 (m, 1H), 12.92 (s, 1H) .

### (4) N-[2-Allyl-3-hydroxy-4-(1-hydroxyiminoethyl)phenyl]acetamide

The crude product of N-(4-acetyl-2-allyl-3-hydroxyphenyl)acetamide (3.9 g) was dissolved in ethanol (100 mL). A solution of hydroxylamine hydrochloride (2.1 g) and sodium acetate (2.5 g) in water (20 mL) was added to the reaction mixture, followed by heating under reflux for two hours. After cooling to room temperature, the reaction mixture was concentrated under reduced pressure. Water and chloroform were added to the residue, and the organic layer was separated. The organic layer was dried over anhydrous sodium sulfate, and the drying agent was removed by filtration. The organic layer was concentrated under reduced pressure to obtain a crude product (2.9 g). The resulting crude product was used for the next reaction without further purification.

### (5) N-(7-Allyl-3-methylbenz[d]isoxazol-6-yl)acetamide

The crude product of N-[2-allyl-3-hydroxy-4-(1-hydroxyiminoethyl)phenyl]acetamide (2.9 g), sodium acetate (2.3 g) and acetic anhydride (2.5 mL) were dissolved in N,N-dimethylformamide (30 mL), and the solution was heated with stirring at 150°C for one hour. After cooling to room temperature, a saturated sodium carbonate solution, water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain a residue. The residue was purified by silica gel column chromatography (heptane-ethyl acetate) to obtain the title compound (2 g). ¹H-NMR (400 MHz, CDCl₃) δ (ppm) : 2.20 (s, 3H), 2.56 (s, 3H), 3.73 (dt, J=2.0, 6.0 Hz, 2H), 5.12-5.27 (m, 2H), 5.95-6.08 (m, 1H), 7.41 (br s, 1H), 7.50 (d, J=8.4 Hz, 1H), 7.98 (d, J=8.4 Hz, 1H) .

### (6) Mixture of (E)-3-methyl-7-propenylbenz[d]isoxazol-6-ylamine and (Z)-3-methyl-7-propenylbenz[d]isoxazol-6-ylamine

N-(7-Allyl-3-methylbenz[d]isoxazol-6-yl)acetamide (2 g) was dissolved in N,N-dimethyl sulfoxide (40 mL). A solution of potassium hydroxide powder (1.9 g) in ethanol (40 mL) was added to the solution, and the mixture was heated with stirring at 110°C overnight. After cooling to room temperature, water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure. The residue was purified by silica gel column chromatography (heptane-ethyl acetate) to obtain the title compound (1.5 g).

### (7) Mixture of (E)-6-iodo-3-methyl-7-propenylbenz[d]isoxazole and (Z)-6-iodo-3-methyl-7-propenylbenz[d]isoxazole

The mixture of (E)-3-methyl-7-propenylbenz[d]isoxazol-6-ylamine and (Z)-3-methyl-7-propenylbenz[d]isoxazol-6-ylamine (1.5 g), copper (I) iodide (1 g) and diiodomethane (2.1 mL) were dissolved in tetrahydrofuran (30 mL). tert-Butyl nitrite (1.9 mL) was added and the mixture was heated with stirring at 60°C for 50 minutes. After cooling to room temperature, ethyl acetate was added to the reaction mixture, followed by filtration through celite. The filtrate was washed with aqueous ammonia and a saturated sodium chloride solution and then concentrated under reduced pressure to obtain a residue. The residue was purified by silica gel column chromatography (heptane-ethyl acetate) to obtain the title compound (690 mg).

### (8) 6-Iodo-3-methylbenz[d]isoxazole-7-carbaldehyde

The mixture of (E)-6-iodo-3-methyl-7-propenylbenz[d]isoxazole and (Z)-6-iodo-3-methyl-7-propenylbenz[d]isoxazole (690 mg) was dissolved in a mixed solvent of tetrahydrofuran (24 mL) and water (6 mL). Osmium tetroxide (2.5% aqueous solution) (1.2 mL) and sodium periodate (980 mg) were added and the mixture was stirred at room temperature for two hours. A sodium sulfite solution was added to the reaction mixture, followed by stirring for a while. Then, chloroform was added and the organic layer was separated. The organic layer was dried over anhydrous sodium sulfate, and the drying agent was removed by filtration. Then, the organic layer was concentrated under reduced pressure to obtain a crude product (739 mg). The resulting crude product was used for the next reaction without further purification.

### (9) (6-Iodo-3-methylbenz[d]isoxazol-7-yl)methanol

6-Iodo-3-methylbenz[d]isoxazole-7-carbaldehyde (730 mg) was dissolved in methanol (20 mL). Sodium borohydride (96 mg) was added under ice-cooling, and the mixture was stirred for 30 minutes. Water and chloroform were added to the reaction mixture, and the organic layer was separated. The organic layer was dried over anhydrous sodium sulfate, and the drying agent was removed by filtration. Then, the organic layer was concentrated under reduced pressure to obtain a crude product (716 mg). The resulting crude product was used for the next reaction without further purification.

### (10) 7-(tert-Butyldimethylsilanyloxymethyl)-6-iodo-3-methylbenz[d]isoxazole

(6-Iodo-3-methylbenz[d]isoxazol-7-yl)methanol (716 mg) and imidazole (405 mg) were dissolved in N,N-dimethylformamide (20 mL). A solution of tert-butyldimethylchlorosilane (450 mg) in N,N-dimethylformamide (20 mL) was added to the solution, and the mixture was stirred at room temperature for three hours. Water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain a residue. The residue was purified by silica gel column chromatography (heptane-ethyl acetate) to obtain the title compound (552 mg). ¹H-NMR (400 MHz, CDCl₃) δ(ppm): 0.15 (s, 6H), 0.93 (s, 9H), 2.57 (s, 3H), 5.05 (s, 2H), 7.24 (d, J=8.4 Hz, 1H), 7.75 (d, J=8.4 Hz, 1H) .

### (11) 7-(tert-Butyldimethylsilanyloxymethyl)-3-methyl-6-(pyridin-2-yl)benz[d]isoxazole

The title compound was synthesized from 7-(tert-butyldimethylsilanyloxymethyl)-6-iodo-3-methylbenz[d]isoxazole and tributyl(2-pyridyl)tin according to Example 120-(3).
¹H-NMR (400 MHz, CDCl₃) δ (ppm) : 0.08 (s, 6H), 0.87 (s, 9H), 2.62 (s, 3H), 5.00 (s, 2H), 7.30-7.35 (m, 1H), 7.56 (d, J=8.0 Hz, 1H), 7.64 (d, J=8.0 Hz, 1H), 7.77-7.84 (m, 2H), 8.73-8.75 (m, 1H).

### (12) tert-Butyl 4-{2-[7-(tert-butyldimethylsilanyloxymethyl)-6-(pyridin-2-yl)benz[d]isoxazol-3-yl]ethyl}piperidine-1-carboxylate

Diisopropylamine (1 mL) was dissolved in tetrahydrofuran (7.5 mL) in a nitrogen atmosphere. n-Butyllithium (2.71 M solution in hexane) (2.4 mL) was added dropwise at -78°C. The mixture was stirred at - 78°C for 10 minutes, under ice-cooling for 10 minutes and further at -78°C for 10 minutes to prepare a solution of lithium diisopropylamide in tetrahydrofuran. The lithium diisopropylamide solution was added dropwise to a solution of 7-(tert-butyldimethylsilanyloxymethyl)-3-methyl-6-(pyridin-2-yl)benz[d]isoxazole (394 mg) and tert-butyl 4-iodomethylpiperidine-1-carboxylate (compound synthesized in Preparation Example 1) (433 mg) in tetrahydrofuran (20 mL) at -70°C, and the mixture was stirred at -70°C for one hour. A saturated ammonium chloride solution, water and ethyl acetate were added to the reaction mixture, and the organic layer was separated. The organic layer was washed with a saturated sodium chloride solution and then dried over anhydrous sodium sulfate. The drying agent was removed by filtration and then the organic layer was concentrated under reduced pressure to obtain a residue. The residue was purified by NH silica gel column chromatography (heptane-ethyl acetate) to obtain the title compound (317 mg).
¹H-NMR (400 MHz, CDCl₃) δ(ppm): 0.09 (s, 6H), 0.87 (s, 9H), 1.14-1.24 (m, 2H), 1.46 (s, 9H), 1.46-1.58 (m, 1H), 1.74-1.86 (m, 4H), 2.62-2.76 (m, 2H), 3.05 (t, J=8.0 Hz, 2H), 4.02-4.18 (m, 2H), 4.99 (s, 2H), 7.31-7.35 (m, 1H), 7.56 (d, J=8.0 Hz, 1H), 7.64 (d, J=8.0 Hz, 1H), 7.78-7.85 (m, 2H), 8.73-8.75 (m, 1H).
ESI-MS m/z: 574 [M+Na]⁺.

### (13) tert-Butyl 4-{2-[7-hydroxymethyl-6-(pyridin-2-yl)benz[d]isoxazol-3-yl]ethyl}piperidine-1-carboxylate

The title compound was synthesized from tert-butyl 4-{2-[7-(tert-butyldimethylsilanyloxymethyl)-6-(pyridin-2-yl)benz[d]isoxazol-3-yl]ethyl}piperidine-1-carboxylate according to Example 120-(4).
¹H-NMR (400 MHz, CDCl₃) δ(ppm): 1.13-1.26 (m, 2H), 1.46 (s, 9H), 1.46-1.60 (m, 1H), 1.73-1.88 (m, 4H), 2.64-2.74 (m, 2H), 3.06 (t, J=7.8 Hz, 2H), 7.02-4.20 (m, 2H), 4.87 (d, J=6.8 Hz, 2H), 6.08 (t, J=6.8 Hz, 1H), 7.40 (ddd, J=1.0, 5.2, 7.6 Hz, 1H), 7.52 (d, J=8.2 Hz, 1H), 7.65 (d, J=8.2 Hz, 1H), 7.71 (dd, J=1.0, 8.0 Hz, 1H), 7.91 (ddd, J=1.6, 7.6, 8.0 Hz, 1H), 8.71 (dd, J=1.6, 5.2 Hz, 1H) .

### (14) tert-Butyl 4-{2-[7-(N,N-dimethylaminomethyl)-6-(pyridin-2-yl)benz[d]isoxazol-3-yl]ethyl}piperidine-1-carboxylate

The title compound was synthesized from tert-butyl 4-{2-[7-hydroxymethyl-6-(pyridin-2-yl)benz[d]isoxazol-3-yl]ethyl}piperidine-1-carboxylate according to Example 120-(5).
¹H-NMR (400 MHz, CDCl₃) δ(ppm): 1.14-1.24 (m, 2H), 1.46 (s, 9H), 1.46-1.60 (m, 1H), 1.73-1.86 (m, 4H), 2.17 (s, 6H), 2.60-2.76 (m, 2H), 3.05 (t, J=7.8 Hz, 2H), 3.80 (s, 2H), 4.04-4.22 (m, 2H), 7.32 (dd, J=4.0, 7.6 Hz, 1H), 7.51 (d, J=8.6 Hz, 1H), 7.62 (d, J=8.6 Hz, 1H), 7.80 (ddd, J=1.6, 6.8, 7.6 Hz, 1H), 7.88 (dd, J=0.8, 6.8 Hz, 1H), 8.73 (ddd, J=0.8, 1.6, 4.0 Hz, 1H) .

### (15) N,N-Dimethyl{3-[2-(piperidin-4-yl)ethyl]-6-(pyridin-2-yl)benz[d]isoxazol-7-yl}methylamine

The title compound was synthesized from tert-butyl 4-{2-[7-(N,N-dimethylaminomethyl)-6-(pyridin-2-yl)benz[d]isoxazol-3-yl]ethyl}piperidine-1-carboxylate according to Example 120-(6). The resulting crude product was used for the next reaction without further purification.

### (16) 5-{4-{2-[7-(N,N-Dimethylaminomethyl)-6-(pyridin-2-yl)benz[d]isoxazol-3-yl]ethyl}piperidinomethyl}thiophene-2-carbonitrile

The title compound was synthesized from N,N-dimethyl{3-[2-(piperidin-4-yl)ethyl]-6-(pyridin-2-yl)benz[d]isoxazol-7-yl}methylamine and 5-formylthiophene-2-carbonitrile [CAS Registry No. 21512-16-3] according to Example 120-(7).
¹H-NMR (400 MHz, CDCl₃) δ(ppm): 1.30-1.43 (m, 3H), 1.74-1.86 (m, 4H), 2.00-2.10 (m, 2H), 2.17 (s, 6H), 2.86-2.94 (m, 2H), 3.04 (t, J=7.6 Hz, 2H), 3.69 (s, 2H), 3.80 (br s, 2H), 6.89 (d, J=3.6 Hz, 1H), 7.32 (dd, J=4.4, 7.6 Hz, 1H), 7.47 (d, J=4.4 Hz, 1H), 7.51 (d, J=8.0 Hz, 1H), 7.63 (d, J=8.0 Hz, 1H), 7.81 (dd, J=7.6, 7.6 Hz, 1H), 7.88 (d, J=7.6 Hz, 1H), 7.82 (d, J=3.6 Hz, 1H) .
ESI-MS m/z: 486 [M+H]⁺.

### (17) 5-{4-{2-[7-(N,N-Dimethylaminomethyl)-6-(pyridin-2-yl)benz[d]isoxazol-3-yl]ethyl}piperidinomethyl}thiophene-2-carbonitrile trihydrochloride

The title compound was synthesized from 5-{4-{2-[7-(N,N-dimethylaminomethyl)-6-(pyridin-2-yl)benz[d]isoxazol-3-yl]ethyl}piperidinomethyl}thiophene-2-carbonitrile according to Example 120-(8).
¹H-NMR (400 MHz, DMSO-d₆) 5(ppm): 1.58-1.67 (m, 3H), 1.65-1.83 (m, 2H), 1.92-2.00 (m, 2H), 2.91-2.97 (m, 8H), 3.07-3.16 (m, 2H), 3.33-3.40 (m, 2H), 4.53-4.60 (m, 4H), 7.58 (d, J=3.6 Hz, 1H), 7.61-7.66 (m, 1H), 7.88 (d, J=7.6 Hz, 1H), 7.98 (d, J=3.6 Hz, 1H), 8.03 (d, J=8.0 Hz, 1H), 8.11-8.15 (m, 1H), 8.18 (d, J=8.0 Hz, 1H), 8.82 (d, J=4.0 Hz, 1H), 10.09 (br s, 1H), 11.38 (br s, 1H).
ESI-MS m/z: 486 [M+H]⁺.

### Example 150

### N,N-Dimethyl{3-{2-[1-(2-pyridyl)piperidin-4-yl]ethyl}-6-phenylbenz[d]isoxazol-7-yl}methylamine dihydrochloride

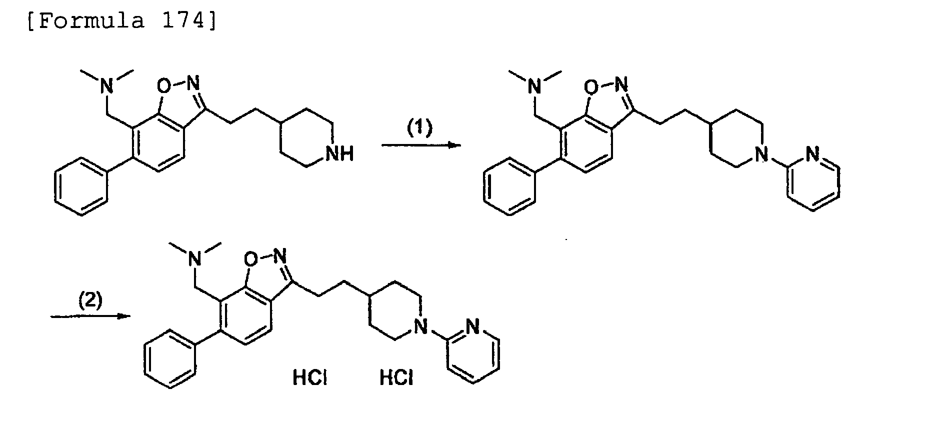

### (1) N,N-Dimethyl{3-{2-[1-(2-pyridyl)piperidin-4-yl]ethyl}-6-phenylbenz[d]isoxazol-7-yl}methylamine

N,N-Dimethyl{6-phenyl-3-[2-(piperidin-4-yl)ethyl]benz[d]isoxazol-7-yl}methylamine (compound synthesized in Example 120-(6)) (150 mg) and 2-fluoropyridine (71 µL) were dissolved in acetonitrile (3 mL). Tetrabutylammonium fluoride hydrate (519 mg) was added and the mixture was heated with stirring at 90°C overnight. After cooling to room temperature, water and ethyl acetate were added to the reaction mixture, and the organic layer was separated. The organic layer was dried over anhydrous sodium sulfate. The drying agent was removed by filtration and then the organic layer was concentrated under reduced pressure to obtain a residue. The residue was purified by NH silica gel column chromatography (heptane-ethyl acetate) to obtain the title compound (48 mg).
¹H-NMR (400 MHz, CDCl₃) δ (ppm): 1.28-1.40 (m, 2H), 1.60-1.72 (m, 1H), 1.83-1.94 (m, 4H), 2.18 (s, 6H), 2.80-2.86 (m, 2H), 3.07 (t, J=7.8 Hz, 2H), 3.65 (s, 2H), 4.26-4.36 (m, 2H), 6.58 (dd, J=5.2, 6.8 Hz, 1H), 6.67 (d, J=8.8 Hz, 1H), 7.27 (d, J=8.8 Hz, 1H), 7.38-7.48 (m, 4H), 7.50-7.55 (m, 2H), 7.58 (d, J=8.0 Hz, 1H), 8.16-8.20 (m, 1H).
ESI-MS m/z: 441 [M+H]⁺.

### (2) N,N-Dimethyl{3-{2-[1-(2-pyridyl)piperidin-4-yl]ethyl}-6-phenylbenz[d]isoxazol-7-yl}methylamine dihydrochloride

The title compound was synthesized from N,N-dimethyl{3-{2-[1-(2-pyridyl)piperidin-4-yl]ethyl}-6-phenylbenz[d]isoxazol-7-yl}methylamine according to Example 120-(8).
ESI-MS m/z: 221 [M+2H]²⁺, 441 [M+H]⁺.

### Example 151

### 5-{4-{2-[9-(N,N-Dimethylaminomethyl)naphtho[2,3-d]isoxazol-3-yl]ethyl}piperidinomethyl}thiophene-2-carbonitrile dihydrochloride

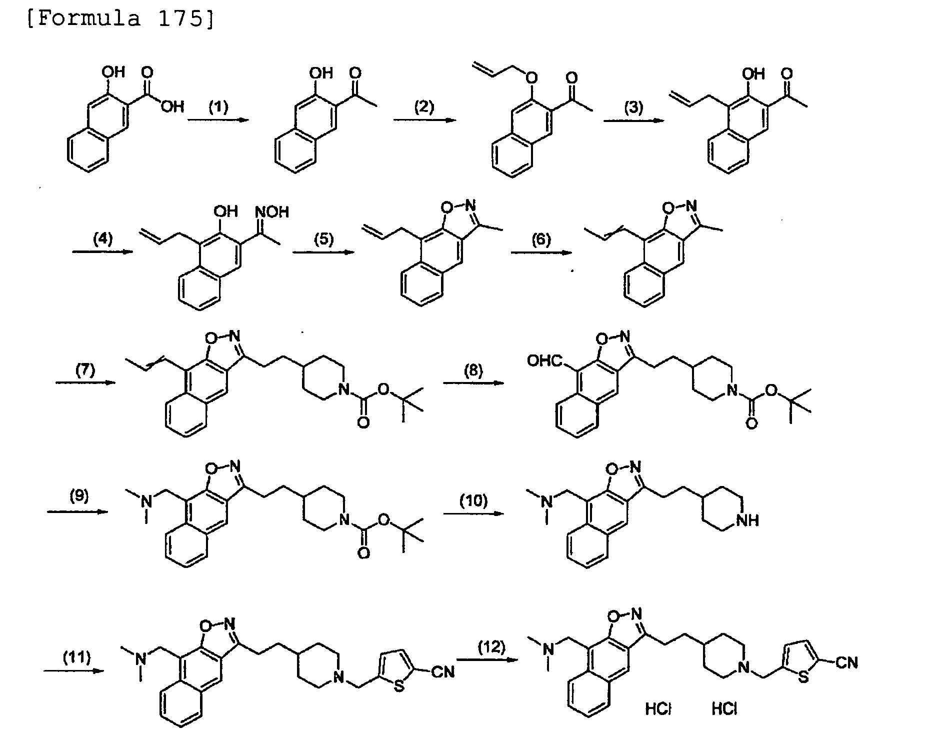

### (1) 1-(3-Hydroxynaphthalen-2-yl)ethanone

3-Hydroxynaphthalene-2-carboxylic acid (1 g) was dissolved in tetrahydrofuran (40 mL). The mixture was ice-cooled in a nitrogen atmosphere, and methyllithium (1.14 M solution in diethyl ether) (21.2 mL) was added. The reaction mixture was stirred under ice-cooling for 30 minutes. Chlorotrimethylsilane (13.6 mL) was added under ice-cooling and the mixture was further stirred at room temperature for 50 minutes. 2 M hydrochloric acid (15 mL) was added to the reaction mixture, followed by stirring for 30 minutes. Water and diethyl ether were added to the reaction mixture, and the organic layer was separated. The resulting organic layer was washed with a saturated sodium chloride solution and then dried over anhydrous sodium sulfate. The drying agent was removed by filtration and then the organic layer was concentrated under reduced pressure to obtain the title compound (1.07 g). ¹H-NMR (400 MHz, CDCl₃) δ(ppm): 2.81 (s, 3H), 7.29 (s, 1H), 7.34 (ddd, J=1.2, 6.8, 8.4 Hz, 1H), 7.53 (ddd, J=1.2, 6. 8, 8.4 Hz, 1H) , 7.68 (d, J=8.4 Hz, 1H) , 7.83 (d, J=8.4 Hz, 1H) , 8.37 (s, 1H), 11.56 (s, 1H).

### (2) 1-(3-Allyloxynaphthalen-2-yl)ethanone

The title compound was synthesized from 1-(3-hydroxynaphthalen-2-yl)ethanone according to Example 149-(2).
¹H-NMR (400 MHz, CDCl₃) δ(ppm) : 2.71 (s, 3H), 4.73-4.76 (m, 2H), 5.36 (dd, J=2.0, 10.6 Hz, 1H), 5.50 (dd, J=2.0, 17.2 Hz, 1H), 6.10-6.21 (m, 1H), 7.18 (s, 1H), 7.37 (ddd, J=0.8, 6.8, 8.0 Hz, 1H), 7.51 (ddd, J=1.2, 6.8, 8.0 Hz, 1H), 7.72 (d, J=8.0 Hz, 1H), 7.84 (d, J=8.0 Hz, 1H), 8.18 (s, 1H).

### (3) 1-(4-Allyl-3-hydroxynaphthalen-2-yl)ethanone

The title compound was synthesized from 1-(3-allyloxynaphthalen-2-yl)ethanone according to Example 149-(3).
¹H-NMR (400 MHz, CDCl₃) δ (ppm) : 2.81 (s, 3H), 3.85-3.88 (m, 2H), 4.98-5.04 (m, 2H), 6.00-6.10 (m, 1H), 7.35 (ddd, J=1.0, 6.8, 8.0 Hz, 1H) , 7.57 (ddd, J=1.2, 6.8, 8.6 Hz, 1H) , 8.84 (dd, J=1.2, 8.0 Hz, 1H), 8.89 (dd, J=1.0, 8.6 Hz, 1H), 8.31 (s, 1H), 11.93 (s, 1H).

### (4) 1-(4-Allyl-3-hydroxynaphthalen-2-yl)ethanone oxime

The title compound was synthesized from 1-(4-allyl-3-hydroxynaphthalen-2-yl)ethanone according to Example 149-(4). The crude product was used for the next reaction.

### (5) 9-Allyl-3-methylnaphtho[2,3-d]isoxazole

The title compound was synthesized from 1-(4-allyl-3-hydroxynaphthalen-2-yl)ethanone oxime according to Example 149-(5). ¹H-NMR (400 MHz, CDCl₃) δ(ppm): 2.69 (s, 3H), 4.12-4.16 (m, 2H), 5.05-5.11 (m, 2H), 6.06-6.17 (m, 1H), 7.46 (ddd, J=1.2, 6.8, 8.4 Hz, 1H) , 7.59 (ddd, J=0.8, 6.8, 8.8 Hz, 1H) , 8.02 (dd, J=0.8, 8.4 Hz, 1H) , 8.07 (s, 1H), 8.13 (dd, J=1.2, 8.8 Hz, 1H).

### (6) Mixture of 3-methyl-9-[(E)-1-propenyl]naphtho[2,3-d]isoxazole and 3-methyl-9-[(Z)-1-propenyl]naphtho[2,3-d]isoxazole

The title compound was synthesized from 9-allyl-3-methylnaphtho[2,3-d]isoxazole according to Example 149-(6).
¹H-NMR (400 MHz, CDCl₃) δ(ppm) : 2.13 and 2.15 (d, J=1.6 Hz, total 3H), 2.70 (s, 3H), 6.98-7.06 (m, total 1H), 7.14-7.18 (m, total 1H), 7.44-7.49 (m, total 1H), 7.56-7.61 (m, total 1H), 7.98-8.01 (m, total 1H), 8.03 (s, 1H), 8.32 (dd, J=0.8, 8.8 Hz, 1H).

### (7) Mixture of tert-butyl 4-{2-{9-[(E)-1-propenyl]naphtho[2,3-d]isoxazol-3-yl}ethyl}piperidine-1-carboxylate and tert-butyl 4-{2-{9-[(Z)-1-propenyl]naphtho[2,3-d]isoxazol-3-yl}ethyl}piperidine-1-carboxylate

The title compound was synthesized from the mixture of 3-methyl-9-[(E)-1-propenyl]naphtho[2,3-d]isoxazole and 3-methyl-9-[(Z)-1-propenyl]naphtho[2,3-d]isoxazole according to Example 149-(12).
¹H-NMR (400 MHz, CDCl₃) δ (ppm) : 1.18-1.26 (m, 2H), 1.46 (s, 9H), 1.55-1.62 (m, 1H), 1.77-1.84 (m, 2H), 1.86-1.93 (m, 2H), 2.13 and 2.15 (d, J=1.6 Hz, total 3H), 2.63-2.75 (m, 2H), 3.14 (t, J=7.8 Hz, 2H), 4.06-4.20 (m, 2H), 7.00-7.09 (m, total 1H), 7.15-7.19 (m, total 1H), 7.44-7.49 (m, total 1H), 7.57-7.62 (m, total 1H), 7.99 (dd, J=0. 6, 7.8 Hz, 1H) , 8.03 (s, 1H) , 8.32 (d, J=8.4 Hz, 1H).

### (8) tert-Butyl 4-[2-(9-formylnaphtho[2,3-d]isoxazol-3-yl)ethyl]piperidine-1-carboxylate

The title compound was synthesized from the mixture of tert-butyl 4-{2-{9-[(E)-1-propenyl]naphtho[2,3-d]isoxazol-3-yl}ethyl}piperidine-1-carboxylate and tert-butyl 4-{2-{9-[(Z)-1-propenyl]naphtho[2,3-d]isoxazol-3-yl}ethyl}piperidine-1-carboxylate according to Example 149-(8).

### (9) tert-Butyl 4-{2-[9-(N,N-dimethylaminomethyl)naphtho[2,3-d]isoxazol-3-yl]ethyl}piperidine-1-carboxylate

tert-Butyl 4-[2-(9-formylnaphtho[2,3-d]isoxazol-3-yl)ethyl]piperidine-1-carboxylate (533 mg) and dimethylamine (2 M solution in tetrahydrofuran) (975 µL) were dissolved in dichloromethane (20 mL). Acetic acid (149 µL) and sodium triacetoxyborohydride (468 mg) were sequentially added and the mixture was stirred at room temperature for three hours. A 5 M sodium hydroxide solution, water and dichloromethane were added to the reaction mixture, and the organic layer was separated. The resulting organic layer was washed with a saturated sodium chloride solution and then dried over anhydrous sodium sulfate. The drying agent was removed by filtration and then the organic layer was concentrated under reduced pressure. The residue was purified by NH silica gel column chromatography (heptane-ethyl acetate) to obtain the title compound (287 mg).
¹H-NMR (400 MHz, CDCl₃) δ (ppm): 1.17-1.26 (m, 2H), 1.46 (s, 9H), 1.56-1.62 (m, 1H), 1.76-1.84 (m, 2H), 1.87-1.94 (m, 2H), 2.37 (br s, 6H), 2.66-2.76 (m, 2H), 3.13 (t, J=7.8 Hz, 2H), 4.06-4.23 (m, 4H), 7.48 (ddd, J=1.2, 6.8, 8.4 Hz, 1H), 7.64 (dd, J=6.8, 7.8 Hz, 1H), 8.01 (d, J=7.8 Hz, 1H), 8.13 (s, 1H) , 8.39 (d, J=8.4 Hz, 1H).
ESI-MS m/z: 438 [M+H]⁺, 460 [M+Na]⁺.

### (10) N,N-Dimethyl{3-[2-(piperidin-4-yl)ethyl]naphtho[2,3-d]isoxazol-9-ylmethyl}amine

The title compound was synthesized from tert-butyl 4-{2-[9-(N,N-dimethylaminomethyl)naphtho[2,3-d]isoxazol-3-yl]ethyl}piperidine-1-carboxylate according to Example 120-(6).

### (11) 5-{4-{2-[9-(N,N-Dimethylaminomethyl)naphtho[2,3-d]isoxazol-3-yl]ethyl}piperidinomethyl}thiophene-2-carbonitrile

The title compound was synthesized from N,N-dimethyl{3-[2-(piperidin-4-yl)ethyl]naphtho[2,3-d]isoxazol-9-ylmethyl}amine and 5-formylthiophene-2-carbonitrile [CAS Registry No. 21512-16-3] according to Example 120-(7).
¹H-NMR (400 MHz, CDCl₃) δ(ppm): 1.32-1.48 (m, 3H), 1.78-1.85 (m, 2H), 1.86-1.96 (m, 2H), 2.00-2.10 (m, 2H), 2.36 (s, 6H), 2.94 (br d, J=11.2 Hz, 2H), 3.12 (t, J=7.8 Hz, 2H), 3.70 (s, 2H), 4.17 (s, 2H), 6.89 (d, J=3.6 Hz, 1H), 7.46-7.51 (m, 2H), 7.60-7.66 (m, 1H), 8.01 (d, J=8.4 Hz, 1H), 8.13 (s, 1H), 8.39 (d, J=8.4 Hz, 1H).
ESI-MS m/z: 459 [M+H]⁺, 481 [M+Na]⁺.

### (12) 5-{4-{2-[9-(N,N-Dimethylaminomethyl)naphtho[2,3-d]isoxazol-3-yl]ethyl}piperidinomethyl}thiophene-2-carbonitrile dihydrochloride

The title compound was synthesized from 5-{4-{2-[9-(N,N-dimethylaminomethyl)naphtho[2,3-d]isoxazol-3-yl]ethyl}piperidinomethyl}thiophene-2-carbonitrile according to Example 120-(8).
ESI-MS m/z: 459 [M+H]⁺.

### Example 152

### 5-{4-{2-[9-(N-Methylaminomethyl)naphtho[2,3-d]isoxazol-3-yl]ethyl}piperidinomethyl}thiophene-2-carbonitrile dihydrochloride

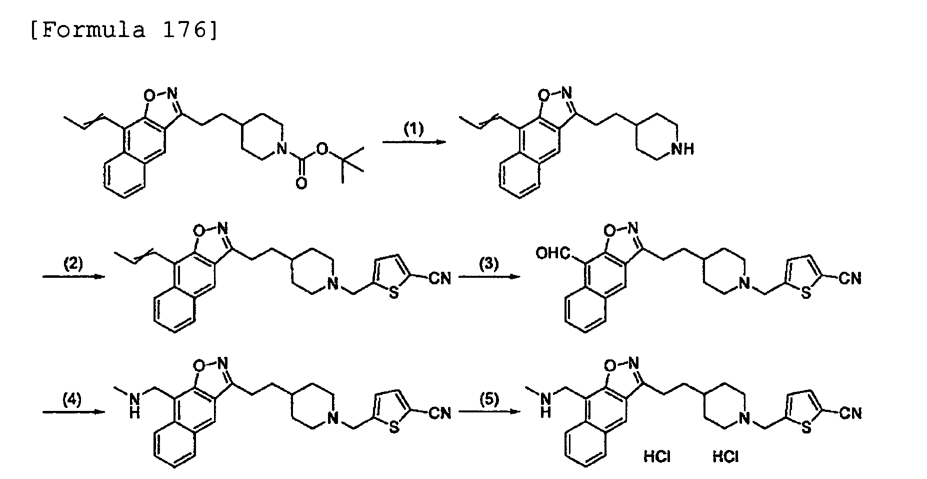

### (1) Mixture of 3-[2-(piperidin-4-yl)ethyl]-9-[(E)-1-propenyl]naphtho[2,3-d]isoxazole and 3-[2-(piperidin-4-yl)ethyl]-9-[(Z)-1-propenyl]naphtho[2,3-d]isoxazole

The title compound was synthesized from the mixture of tert-butyl 4-{2-{9-[(E)-1-propenyl]naphtho[2,3-d]isoxazol-3-yl}ethyl}piperidine-1-carboxylate and tert-butyl 4-{2-{9-[(Z)-1-propenyl]naphtho[2,3-d]isoxazol-3-yl}ethyl}piperidine-1-carboxylate (compound synthesized in Example 151-(7)) according to Example 120-(6).

### (2) Mixture of 5-{4-{2-{9-[(E)-1-propenyl]naphtho[2,3-d]isoxazol-3-yl}ethyl}piperidinomethyl}thiophene-2-carbonitrile and 5-{4-{2-{9-[(Z)-1-propenyl]naphtho[2,3-d]isoxazol-3-yl}ethyl}piperidinomethyl}thiophene-2-carbonitrile

The title compound was synthesized from the mixture of 3-[2-(piperidin-4-yl)ethyl]-9-[(E)-1-propenyl]naphtho[2,3-d]isoxazole and 3-[2-(piperidin-4-yl)ethyl]-9-[(Z)-1-propenyl]naphtho[2,3-d]isoxazole and 5-formylthiophene-2-carbonitrile [CAS Registry No. 21512-16-3] according to Example 120-(7).
¹H-NMR (400 MHz, CDCl₃) δ (ppm) : 1.29-1.45 (m, 3H), 1.79-1.83 (m, 2H), 1.86-1.95 (m, 2H), 2.00-2.08 (m, 2H), 2.13 and 2.15 (d, J=1.2 Hz, total 3H), 2.93 (br d, J=11.6 Hz, 2H), 3.13 (t, J=8.0 Hz, 2H), 3.69 (s, 2H), 6.88 (d, J=3.6 Hz, 1H), 6.99-7.06 (m, total 1H), 7.14-7.20 (m, total 1H), 7.46-7.50 (m, 2H), 7.59 (ddd, J=1.6, 7.2, 8.4 Hz, 1H), 7.99 (dd, J=0.8, 8.0 Hz, 1H), 8.03 (s, 1H), 8.32 (d, J=8.4 Hz, 1H).

### (3) 5-{4-[2-(9-Formylnaphtho[2,3-d]isoxazol-3-yl)ethyl]piperidinomethyl}thiophene-2-carbonitrile

The title compound was synthesized from the mixture of 5-{4-{2-{9-[(E)-1-propenyl]naphtho[2,3-d]isoxazol-3-yl}ethyl}piperidinomethyl}thiophene-2-carbonitrile and 5-{4-{2-{9-[(Z)-1-propenyl]naphtho[2,3-d]isoxazol-3-yl}ethyl}piperidinomethyl}thiophene-2-carbonitrile according to Example 149-(8).
¹H-NMR (400 MHz, CDCl₃) δ (ppm) : 1.29-1.49 (m, 3H), 1.70-1.86 (m, 2H), 1.86-1.99 (m, 2H), 2.00-2.14 (m, 2H), 2.88-3.02 (m 2H), 3.10-3.20 (m, 2H), 3.71 (br s, 2H), 6. 83-6. 95 (m, 1H) , 7.48 (d, J=7. 4 Hz, 1H) , 7.60 (dd, J=7.2, 7.4 Hz, 1H), 7.82 (ddd, J=1.2, 7.2, 8.4 Hz, 1H), 8.06 (d, J=8.0 Hz, 1H) , 8.45 (s, 1H) , 9.44 (d, J=8.4 Hz, 1H) , 11.13 (s, 1H).

### (4) 5-{4-{2-[9-(N-Methylaminomethyl)naphtho[2,3-d]isoxazol-3-yl]ethyl}piperidinomethyl}thiophene-2-carbonitrile

5-{4-[2-(9-Formylnaphtho[2,3-d]isoxazol-3-yl)ethyl]piperidinomethyl}thiophene-2-carbonitrile (158 mg) and methylamine (2 M solution in tetrahydrofuran) (552 mL) were dissolved in methanol (5 mL). Titanium (IV) isopropoxide (141 µL) was added to the solution in a nitrogen atmosphere at room temperature, and the mixture was stirred for eight hours. Magnesium sulfate (58 mg) was further added, and the mixture was stirred at room temperature for 1.25 hours. The reaction mixture was ice-cooled. Sodium borohydride (15.3 mg) was added and the mixture was further stirred at room temperature for one hour. Aqueous ammonia, water and ethyl acetate were added to the reaction mixture, followed by filtration through celite. The organic layer was separated. The organic layer was washed with a saturated sodium chloride solution and then dried over anhydrous sodium sulfate. The drying agent was removed by filtration and then the organic layer was concentrated under reduced pressure. The residue was purified by NH silica gel column chromatography (heptane-ethyl acetate) to obtain the title compound (102 mg) .
¹H-NMR (400 MHz, CDCl₃) δ(ppm): 1.32-1.48 (m, 3H), 1.77-1.94 (m, 4H), 2.00-2.57 (m, 2H), 2.56 (s, 3H), 2.93 (br d, J=12.0 Hz, 2H), 3.13 (t, J=7.8 Hz, 2H), 3.70 (s, 2H), 4.53 (s, 2H), 6.89 (d, J=3.6 Hz, 1H), 7.46-7.67 (m, 2H), 7.64 (dd, J=7.2, 8.4 Hz, 1H), 8.02 (d, J=8.4 Hz, 1H) , 8.13 (s, 1H), 8.29 (d, J=8.8 Hz, 1H).
ESI-MS m/z: 445 [M+H]⁺, 467 [M+Na]⁺.

### (5) 5-{4-{2-[9-(N-Methylaminomethyl)naphtho[2,3-d]isoxazol-3-yl]ethyl}piperidinomethyl}thiophene-2-carbonitrile dihydrochloride

The title compound was synthesized from 5-{4-{2-[9-(N-methylaminomethyl)naphtho[2,3-d]isoxazol-3-yl]ethyl}piperidinomethyl}thiophene-2-carbonitrile according to Example 120-(8).
ESI-MS m/z: 445 [M+H]⁺.

### Example 153

### 4-{3-[2-(1-Benzylpiperidin-4-yl)ethyl]-7-(N,N-dimethylaminomethyl)benz[d]isoxazol-6-yloxymethyl}benzonitrile dihydrochloride

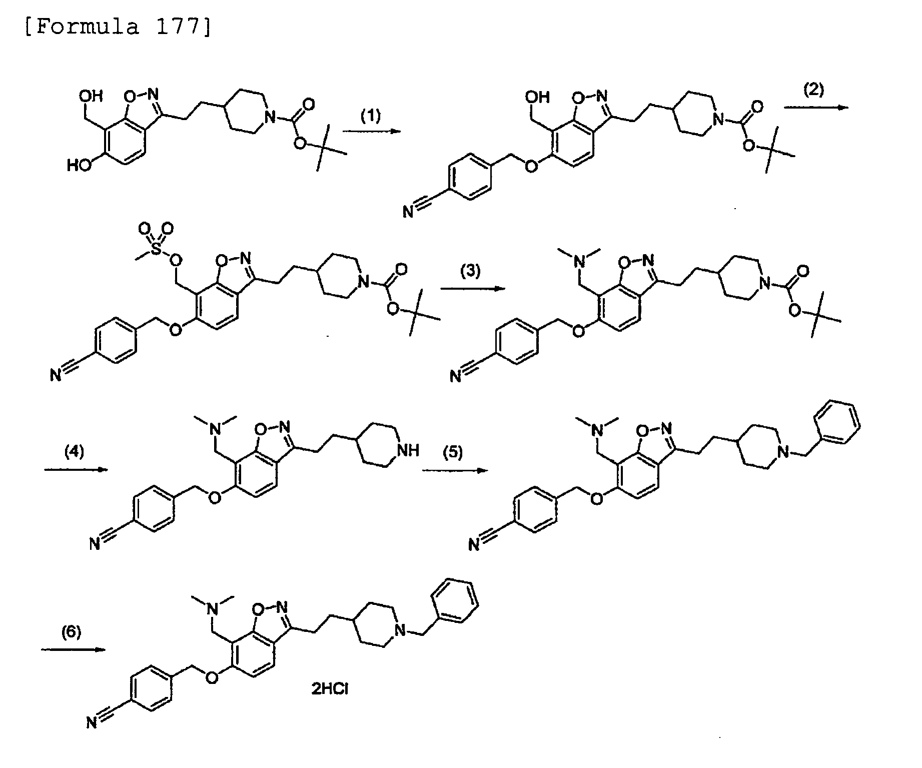

### (1) tert-Butyl 4-{2-[6-(4-cyanobenzyloxy)-7-hydroxymethylbenz[d]isoxazol-3-yl]ethyl}piperidine-1-carboxylate

tert-Butyl 4-[2-(6-hydroxy-7-hydroxymethylbenz[d]isoxazol-3-yl)ethyl]piperidine-1-carboxylate (0.29 g) was dissolved in acetone (15 mL). Potassium carbonate (160 mg) and 4-cyanobenzyl bromide (190 mg) were added to the mixture, followed by heating under reflux in a nitrogen atmosphere for nine hours. The insoluble matter was removed by filtration and the solvent was evaporated under reduced pressure. The residue was reprecipitated from ethyl acetate-n-hexane. The precipitate was collected by filtration to obtain the title compound (370 mg).
¹H-NMR (400 MHz, CDCl₃) δ (ppm): 1.11-1.21 (m, 2H), 1.46 (s, 9H), 1.45-1.55 (m, 1H), 1.72-1.81 (m, 4H), 1.24 (t, J=6.7 Hz, 1H), 2.63-2.71 (m, 2H), 2.97 (t, J=7.6 Hz, 2H), 4.05-4.18 (m, 2H), 5.08 (d, J=6.7 Hz, 2H), 5.30 (s, 2H), 6.94 (d, J=8.8 Hz, 1H), 7.50 (d, J=8.8 Hz, 1H), 7.57 (d, J=8.4 Hz, 2H), 7.71 (d, J=8.4 Hz, 2H).

### (2) tert-Butyl 4-{2-[6-(4-cyanobenzyloxy)-7-methanesulfonyloxymethylbenz[d]isoxazol-3-yl]ethyl}piperidine-1-carboxylate

tert-Butyl 4-{2-[6-(4-cyanobenzyloxy)-7-hydroxymethylbenz[d]isoxazol-3-yl]ethyl}piperidine-1-carboxylate (305 mg) was dissolved in tetrahydrofuran (10 mL). Triethylamine (259 µL) was added to the solution, and the mixture was stirred under ice-cooling in a nitrogen atmosphere. Methanesulfonyl chloride (72 µL) was added to the mixture, followed by stirring under ice-cooling for 1.5 hours. Ethyl acetate, a saturated ammonium chloride solution and a saturated sodium bicarbonate solution were added to the reaction mixture, and the organic layer was separated. The organic layer was sequentially washed with a saturated sodium bicarbonate solution, water (four times) and a saturated sodium chloride solution and dried over magnesium sulfate. The drying agent was removed by filtration and then the organic layer was concentrated under reduced pressure to obtain 445 mg of the title compound. The title compound was used for the next reaction without further purification.
¹H-NMR (400 MHz, CDCl₃) δ (ppm): 1.11-1.21 (m, 2H), 1.43 (s, 9H), 1.43-1.56 (m, 1H), 1.73-1.81 (m, 4H), 2.66-2.72 (m, 2H), 2.98 (t, J=8.0 Hz, 2H), 3.07 (s, 3H), 4.05-4.18 (m, 2H), 5.31 (s, 2H), 5.63 (s, 2H), 6.96 (d, J=8.8 Hz, 1H), 7.60-7.62 (m, 3H), 7.72 (d, J=8.4 Hz, 2H).

### (3) tert-Butyl 4-{2-[6-(4-cyanobenzyloxy)-7-(N,N-dimethylaminomethyl)benz[d]isoxazol-3-yl]ethyl}piperidine-1-carboxylate

Dimethylamine (2 M solution in tetrahydrofuran) (6.2 mL) was added to a mixture of tert-butyl 4-{2-[6-(4-cyanobenzyloxy)-7-methanesulfonyloxymethylbenz[d]isoxazol-3-yl]ethyl}piperidine-1-carboxylate (350 mg), sodium iodide (19 mg) and acetonitrile (10 mL). The mixture was stirred in a sealed tube at 50°C for 17 hours. The reaction mixture was cooled to room temperature. Ethyl acetate and a 10% sodium carbonate solution were added and the organic layer was separated. The organic layer was sequentially washed with a 10% sodium thiosulfate solution and a saturated sodium chloride solution and dried over magnesium sulfate. The drying agent was removed by filtration and then the organic layer was concentrated under reduced pressure. The residue was purified by NH silica gel column chromatography (heptane-ethyl acetate) to obtain the title compound (280 mg) .
¹H-NMR (400 MHz, CDCl₃) δ (ppm): 1.11-1.21 (m, 2H), 1.46 (s, 9H), 1.46-1.56 (m, 1H), 1.73-1.71 (m, 4H), 2.33 (s, 6H), 2.63-2.73 (m, 2H), 2.94-2.98 (m, 2H), 3.82 (s, 2H), 4.05-4.16 (m, 2H), 5.27 (s, 2H), 6.94 (d, J=8.6 Hz, 1H), 7.47 (d, J=8.6 Hz, 1H), 7.59 (d, J=8.2 Hz, 2H), 7.70 (d, J=8 .2 Hz, 2H).

### (4) 4-{7-(N,N-Dimethylaminomethyl)-3-[2-(piperidin-4-yl)ethyl]benz[d]isoxazol-6-yloxymethyl}benzonitrile

Methanol (5 mL) was added to tert-butyl 4-{2-[6-(4-cyanobenzyloxy)-7-(N,N-dimethylaminomethyl)benz[d]isoxazol-3-yl]ethyl}piperidine-1-carboxylate (280 mg), and the solution was stirred under ice-cooling in a nitrogen atmosphere. Hydrogen chloride (4 M solution in ethyl acetate) (1.35 mL) was added to the mixture, followed by stirring at room temperature for 11 hours and 30 minutes. Hydrogen chloride (4 M solution in ethyl acetate) (1.35 mL) was further added and the mixture was stirred at room temperature for 10 hours and 30 minutes. The solvent was evaporated under reduced pressure. A 5 M sodium hydroxide solution was added to the residue, followed by extraction with chloroform four times. The organic layers were washed with a saturated sodium chloride solution and dried over magnesium sulfate. The drying agent was removed by filtration and then the organic layers were concentrated under reduced pressure to obtain the crude title compound (260 mg). The title compound was used for the next reaction without further purification.
¹H-NMR (400 MHz, CDCl₃) δ (ppm): 1.17-1.26 (m, 2H), 1.44-1.56 (m, 1H) , 1.75-1.80 (m, 4H), 2.33 (s, 6H), 2.58-2.64 (m, 2H), 2.94-2.98 (m, 2H), 3.08-3.15 (m, 2H), 3.83 (s, 2H), 5.27 (s, 2H), 6.94 (d, J=8.8 Hz, 1H), 7.48 (d, J=8.8 Hz, 1H), 6.59 (d, J=8.4 Hz, 2H), 7.70 (d, J=8.4 Hz, 2H).

### (5) 4-{3-[2-(1-Benzylpiperidin-4-yl)ethyl]-7-(N,N-dimethylaminomethyl)benz[d]isoxazol-6-yloxymethyl}benzonitrile

4-{7-(N,N-Dimethylaminomethyl)-3-[2-(piperidin-4-yl)ethyl]benz[d]isoxazol-6-yloxymethyl}benzonitrile (90 mg) and benzaldehyde (66 µL) were dissolved in methylene chloride (3 mL), and the solution was stirred in a nitrogen atmosphere at room temperature. Acetic acid (37 µL) was added to the mixture, followed by stirring for 10 minutes. Then, sodium triacetoxyborohydride (68 mg) was added and the mixture was stirred for nine hours and 30 minutes. A 5 M sodium hydroxide solution and a saturated sodium chloride solution were added to the reaction mixture, followed by extraction with chloroform three times. The combined organic layers were dried over magnesium sulfate. The drying agent was removed by filtration and then the organic layers were concentrated under reduced pressure. The residue was purified by NH silica gel column chromatography (heptane-ethyl acetate) to obtain the title compound (79 mg).
¹H-NMR (400 MHz, CDCl₃) δ (ppm): 1.26-1.35 (m, 3H), 1.73-1.79 (m, 4H), 1.91-1.97 (m, 2H), 2.33 (s, 6H), 2.86-2.91 (m, 2H), 2.91-2.96 (m, 2H), 3.49 (s, 2H), 3.82 (s, 2H), 5.27 (s, 2H), 6.93 (d, J=8.6Hz, 1H), 7.25-7.31 (m, 5H), 7.47 (d, J = 8.6 Hz, 1H), 7.59 (d, J = 8.0 Hz, 2H), 7.70 (d, J = 8.0 Hz, 2H).

### (6) 4-{3-[2-(1-Benzylpiperidin-4-yl)ethyl]-7-(N,N-dimethylaminomethyl)benz[d]isoxazol-6-yloxymethyl}benzonitrile dihydrochloride

4-{3-[2-(1-Benzylpiperidin-4-yl)ethyl]-7-(N,N-dimethylaminomethyl)benz[d]isoxazol-6-yloxymethyl}benzonitrile (79 mg) was dissolved in ethyl acetate (1 mL). Excess hydrogen chloride (4 M solution in ethyl acetate) was added and the solvent was evaporated under reduced pressure. The resulting precipitate was collected by filtration and dried under reduced pressure to obtain the title compound (73 mg). ¹H-NMR (400 MHz, DMSO-d₆) δ (ppm): 1.56-3.48 (m, 19H), 4.23-4.25 (m, 2H), 4.54 (s, 2H), 5.48 (s, 2H), 7.30-8.00 (m, 11H), 10.41 (br s, 1H) , 10.79 (br s, 1H).
ESI-MS m/z: 255 [M+2H]²⁺, 509 [M+H]⁺.

### Example 154

### 4-{7-(N,N-Dimethylaminomethyl)-3-{2-[1-(1,3-dioxolan-2-ylmethyl)piperidin-4-yl]ethyl}benz[d]isoxazol-6-yloxymethyl}benzonitrile fumarate

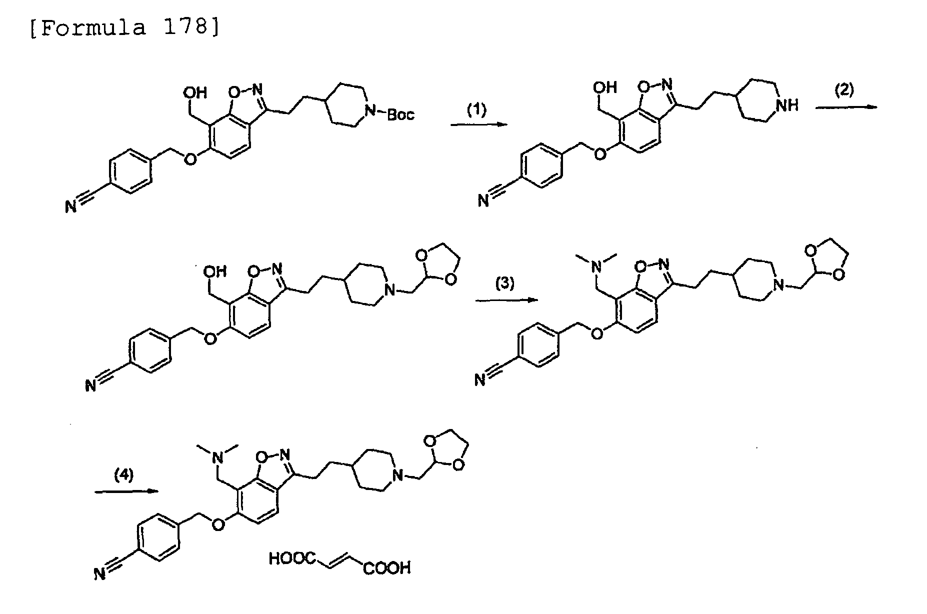

### (1) 4-{7-Hydroxymethyl-3-[2-(piperidin-4-yl)ethyl]benz[d]isoxazol-6-yloxymethyl}benzonitrile

The title compound was obtained by synthesis according to Example 153-(4) from tert-butyl 4-{2-[6-(4-cyanobenzyloxy)-7-hydroxymethylbenz[d]isoxazol-3-yl]ethyl}piperidine-1-carboxylate obtained in Example 153-(1).
¹H-NMR (400 MHz, CDCl₃) δ (ppm): 1.13-1.26 (m, 2H), 1.41-1.53 (m, 1H), 1.73-1.79 (m, 4H), 2.55-2.62 (m, 2H), 2.94-2.98 (m, 2H), 3.05-3.11 (m, 2H), 5.08 (s, 2H), 5.30 (s, 2H), 6.93 (d, J=8.8 Hz, 1H), 7.51 (d, J=8.8 Hz, 1H), 7.57 (d, J=8.0 Hz, 2H), 7.71 (d, J=8.0 Hz, 2H).

### (2) 4-(3-{2-[1-(1,3-Dioxolan-2-ylmethyl)piperidin-4-yl]ethyl}-7-hydroxymethylbenz[d]isoxazol-6-yloxymethyl}benzonitrile

N,N-Dimethylformamide (8 mL), propionitrile (4 mL), potassium carbonate (285 mg) and 2-bromomethyl-1,3-dioxolane (106 µL) were added to 4-{7-hydroxymethyl-3-[2-(piperidin-4-yl)ethyl]benz[d]isoxazol-6-yloxymethyl}benzonitrile (250 mg), and the mixture was stirred in a nitrogen atmosphere at 90°C for three hours. 2-Bromomethyl-1,3-dioxolane (106 µL) was further added and the mixture was stirred for 16 hours. The solvent was evaporated under reduced pressure. A 1 M sodium hydroxide solution and a saturated sodium chloride solution were added to the residue, followed by extraction with chloroform three times. The combined organic layers were dried over magnesium sulfate-sodium carbonate. The drying agent was removed by filtration and then the solvent was evaporated under reduced pressure. The residue was purified by NH silica gel column chromatography (heptane-ethyl acetate) to obtain the title compound (210 mg).
¹H-NMR (400 MHz, CDCl₃) δ (ppm): 1.26-1.41 (m, 2H), 1.73-1.79 (m, 4H), 2.03-2.08 (m, 2H), 2.22-2.28 (m, 1H), 2.56 (d, J=4.20 Hz, 2H), 2.93-3.02 m, 4H), 3.82-4.01 (m, 4H), 5.00 (t, J=4.2 Hz, 1H), 5.06-5.08 (m, 2H), 5.30 (s, 2H), 6.93 (d, J=8.6 Hz, 1H) , 7.49 (d, J=8.6 Hz, 1H) , 7.57 (d, J=8. 4 Hz, 2H), 7.71 (d, J=8.4 Hz, 2H).

### (3) 4-{7-(N,N-Dimethylaminomethyl)-3-{2-[1-(1,3-dioxolan-2-ylmethyl)piperidin-4-yl]ethyl}benz[d]isoxazol-6-yloxymethyl}benzonitrile

4-{3-{2-[1-(1,3-Dioxolan-2-ylmethyl)piperidin-4-yl]ethyl}-7-hydroxymethylbenz[d]isoxazol-6-yloxymethyl}benzonitrile (210 mg) was dissolved in tetrahydrofuran (10 mL), and triethylamine (186 µL) was added. The solution was stirred under ice-cooling in a nitrogen atmosphere. Methanesulfonyl chloride (186 µL) was added to the mixture, followed by stirring for 25 minutes. Methanesulfonyl chloride (10 µL) was further added and the mixture was stirred for 20 minutes. The precipitate was removed by filtration and the solvent was evaporated under reduced pressure. The residue was dissolved by adding acetonitrile (10 mL). Dimethylamine (2 M solution in tetrahydrofuran) (6.2 mL) was added and the mixture was stirred in a sealed tube at 50°C. The mixture was cooled to room temperature and the solvent was evaporated under reduced pressure. Ethyl acetate and a 10% sodium carbonate solution were added to the residue, and the organic layer was separated. The organic layer was washed with a saturated sodium chloride solution and dried over magnesium sulfate-sodium carbonate. The drying agent was removed by filtration and then the organic layer was concentrated under reduced pressure. The residue was purified by NH silica gel column chromatography (heptane-ethyl acetate) to obtain the title compound (150 mg).
¹H-NMR (400 MHz, CDCl₃) δ (ppm): 1.32-1.41 (m, 3H), 1.63-1.79 (m, 4H), 2.03-2.10 (m, 2H), 2.33 (s, 6H), 2.56 (d, J=4.4 Hz, 2H), 2.92-3.02 (m, 4H), 3.82 (s, 2H), 3.82-3.99 (m, 4H), 5.00 (t, J=4.4 Hz, 1H), 5.27 (s, 2H), 6.94 (d, J=8.6 Hz, 1H), 7.47 (d, J=8.6 Hz, 1H), 7.59 (d, J=8.0 Hz, 2H), 7.70 (d, J=8.0 Hz, 2H).

### (4) 4-{7-(N,N-Dimethylaminomethyl)-3-{2-[1-(1,3-dioxolan-2-ylmethyl)piperidin-4-yl]ethyl}benz[d]isoxazol-6-yloxymethyl}benzonitrile fumarate

4-{7-(N,N-Dimethylaminomethyl)-3-{2-[1-(1,3-dioxolan-2-ylmethyl)piperidin-4-yl]ethyl}benz[d]isoxazol-6-yloxymethyl}benzonitrile (150 mg) was dissolved in ethyl acetate (1 mL). A solution of fumaric acid (34 mg) in methanol (1 mL) was added to the solution, and the solvent was evaporated under reduced pressure. Diethyl ether was added to the residue, and the solvent was evaporated under reduced pressure. The residue was dried under reduced pressure to obtain the title compound (147 mg).
¹H-NMR (400 MHz, DMSO-d₆) δ (ppm): 1.17-1.33 (m, 3H), 1.65-1.72 (m, 4H), 2.06-2.12 (m, 2H), 2.28 (s, 6H), 2.54 (d, J=4.4 Hz, 2H), 2.92-2.99 (m, 4H), 3.74-3.89 (m, 4H), 3.86 (s, 2H), 4.92 (t, J=4.4 Hz, 1H), 5.40 (s, 2H), 6.58 (s, 2H), 7.22 (d, J=4.4 Hz, 1H) , 7.71 (d, J=8.2 Hz, 2H), 7.80 (d, J=4.4 Hz, 1H), 7.90 (d, J=8.2 Hz, 2H).
ESI-MS m/z: 253 [M+2H]²⁺, 505 [M+H]⁺.

### Example 155

### 5-{4-{2-[6-Cyclopropylmethoxy-7-(N,N-dimethylaminomethyl)benz[d]isoxazol-3-yl]ethyl}piperidinomethyl}thiophene-2-carbonitrile dihydrochloride

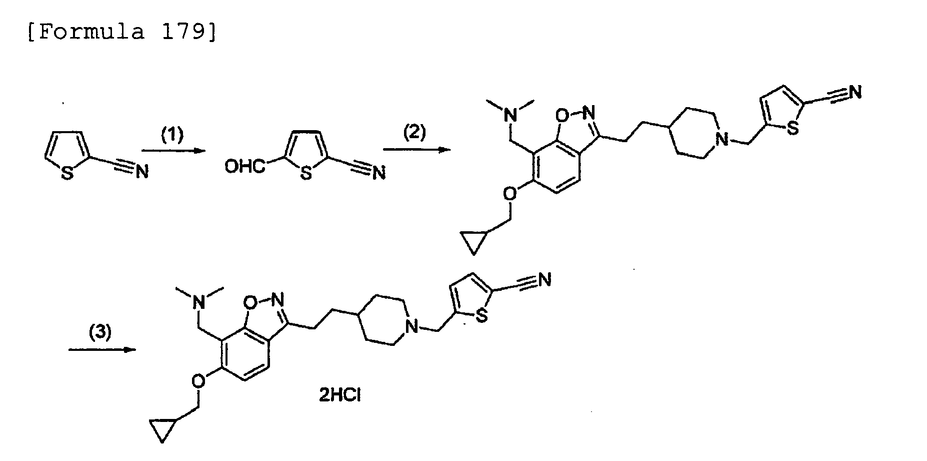

### (1) 5-Formylthiophene-2-carbonitrile

Diisopropylamine (5.75 mL) was dissolved in tetrahydrofuran (15 mL), and the solution was stirred in a dry ice-acetone bath in a nitrogen atmosphere. n-Butyllithium (2.5 M solution in hexane) (16 mL) was added dropwise to the mixture. The reaction mixture was stirred under ice-cooling for five minutes and stirred in the dry ice-acetone bath again to prepare a solution of lithium diisopropylamide in tetrahydrofuran. A solution of thiophene-2-carbonitrile (4.0 g) in tetrahydrofuran (10 mL) was added dropwise to the lithium diisopropylamide solution. Thereafter, the mixture was stirred at the same temperature for 45 minutes. N,N-Dimethylformamide (10 mL) was added and the reaction mixture was stirred at the same temperature for 10 minutes. A saturated ammonium chloride solution and water were added and then the dry ice-acetone bath was removed, followed by extraction with ethyl acetate. The organic layer was washed with a saturated sodium chloride solution and dried over magnesium sulfate. The drying agent was removed by filtration and then the organic layer was concentrated under reduced pressure. The residue was purified by silica gel column chromatography (heptane-ethyl acetate) to obtain the title compound (4.05 g). ¹H-NMR (400 MHz, CDCl₃) δ (ppm): 7.70 (d, J=4.0 Hz, 1H) , 7.76 (d, J=4.0 Hz, 1H), 9.99 (s, 1H).

### (2) 5-{4-{2-[6-Cyclopropylmethoxy-7-(N,N-dimethylaminomethyl)benz[d]isoxazol-3-yl]ethyl}piperidinomethyl}thiophene-2-carbonitrile

N,N-Dimethyl{6-cyclopropylmethoxy-3-[2-(piperidin-4-yl)ethyl]benz[d]isoxazol-7-yl}methylamine (84 mg) and 5-formylthiophene-2-carbonitrile (65 mg) were dissolved in methylene chloride (2.2 mL). Acetic acid (27 µL) was added and the reaction mixture was stirred in a nitrogen atmosphere at room temperature. Sodium triacetoxyborohydride (76 mg) was added and the reaction mixture was stirred for 45 minutes. A 1 M sodium hydroxide solution and a saturated sodium chloride solution were added, followed by extraction with chloroform three times. The combined organic layers were dried over magnesium sulfate-sodium carbonate. The drying agent was removed by filtration and then the organic layers were concentrated under reduced pressure. The residue was purified by NH silica gel column chromatography (heptane-ethyl acetate) to obtain the title compound (98 mg).
¹H-NMR (400 MHz, CDCl₃) δ (ppm): 0.35-0.39 (m, 2H), 0.62-0.67 (m, 2H), 1.29-1.38 (m, 4H), 1.74-1.81 (m, 4H), 2.00-2.06 (m, 2H), 2.34 (s, 6H), 2.88-2.97 (m, 4H), 3.69 (s. 2H), 3.82 (s, 2H), 3.94 (d, J=6.4 Hz, 2H), 6.88 (d, J=3.8 Hz, 1H) , 6.90 (d, J=8.6 Hz, 1H), 7.45 (d, J=8.6 Hz, 1H) , 7.47 (d, J=3.8 Hz, 1H).

### (3) 5-{4-{2-[6-Cyclopropylmethoxy-7-(N,N-dimethylaminomethyl)benz[d]isoxazol-3-yl]ethyl}piperidinomethyl}thiophene-2-carbonitrile dihydrochloride

The title compound was obtained by converting 5-{4-{2-[6-cyclopropylmethoxy-7-(N,N-dimethylaminomethyl)benz[d]isoxazol-3-yl]ethyl}piperidinomethyl}thiophene-2-carbonitrile to a hydrochloride according to Example 153-6.
¹H-NMR (400 MHz, DMSO-d₆) δ (ppm): 0.39-0.43 (m, 2H), 0.58-0.63 (m, 2H), 1.29-1.39 (m, 1H), 1.54-3.39 (m, 6H), 2.78 (s, 6H), 2.78-3.50 (m, 7H), 4.07 (d, J=7.6 Hz, 2H), 4.46-4.47 (m, 2H), 4.58-4.60 (m, 2H), 7.25 (J=9.0 Hz, 1H), 7.60 (d, J=3.8 Hz, 1H), 7.96 (d, J=9.0 Hz, 1H), 7.99 (d, J=3.8 Hz, 1H), 10.52 (br s, 1H), 11.52 (br s, 1H).
ESI-MS m/z: 479 [M+H]⁺, 501 [M+Na]⁺.

### Example 156

### 4-{3-[2-(1-Benzylpiperidin-4-yl)ethyl]-7-(N,N-dimethylaminomethyl)benz[d]isoxazol-6-yl}benzonitrile dihydrochloride

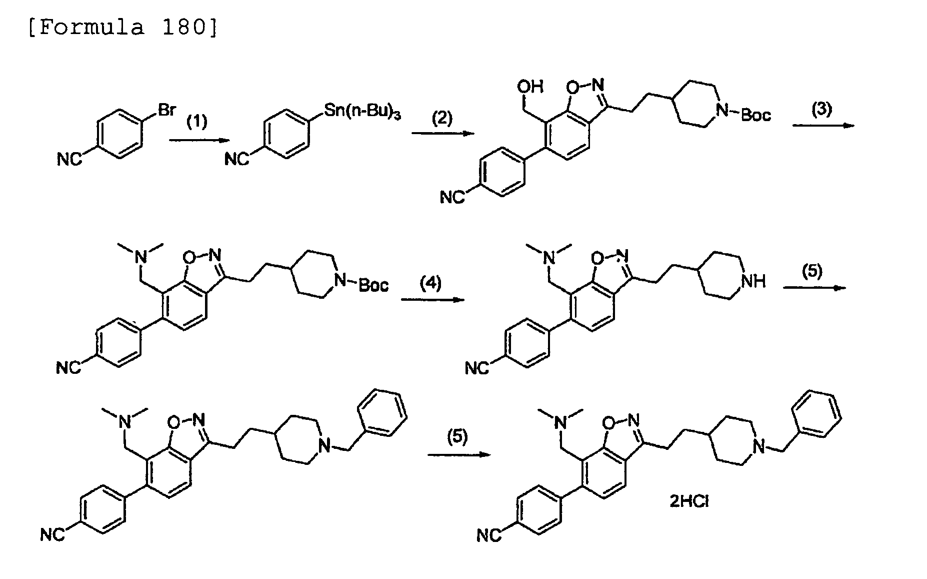

### (1) 4-Tributylstannylbenzonitrile

4-Bromobenzonitrile (5.0 g) was dissolved by adding tetrahydrofuran (30 mL) and diethyl ether (5 mL), and the solution was stirred in a liquid nitrogen bath in a nitrogen atmosphere. The internal temperature was adjusted to -110°C and diethyl ether (10 mL) was added. n-BuLi (2.5 M solution in hexane) (12 mL) was added dropwise to the mixture. The mixture was heated to an internal temperature of -100°C and stirred for five minutes. Then, tributyltin chloride (7.9 mL) was added dropwise. During the dropwise addition, the internal temperature was maintained at -95°C or less. The reaction mixture was stirred at the same temperature for five minutes and heated to an internal temperature of about -15°C over 40 minutes. The solvent was evaporated under reduced pressure at about 40°C. Ethyl acetate, n-hexane and water were added to the residue, and the organic layer was separated. The organic layer was sequentially washed with water and a saturated sodium chloride solution and dried over magnesium sulfate. The drying agent was removed by filtration and then the organic layer was concentrated under reduced pressure. The residue was purified by silica gel column chromatography (n-hexane-ethyl acetate) to obtain the title compound (8.49 g).
¹H-NMR (400 MHz, CDCl₃) δ (ppm): 0.88 (t, J=7.2 Hz, 9H), 1.00-1.18 (m, 6H), 1.28-1.37 (m, 6H), 1.48-1.56 (m, 6H), 7.56 (s, 4H).

### (2) tert-Butyl 4-{2-[6-(4-cyanophenyl)-7-hydroxymethylbenz[d]isoxazol-3-yl]ethyl}piperidine-1-carboxylate

A mixture of tert-butyl 4-{2-[7-(tert-butyldimethylsilanyloxymethyl)-6-trifluoromethanesulfonyloxybenz[d]isoxazol-3-yl]ethyl}piperidine-1-carboxylate (990 mg), 4-tributylstannylbenzonitrile (1.06 g), lithium chloride (200 mg), bis(triphenylphosphine)palladium (II) dichloride (56 mg) and N-methyl-2-pyrrolidone (30 mL) was stirred in a nitrogen atmosphere at 120°C for one hour. The reaction mixture was cooled to room temperature. Ethyl acetate, water and potassium fluoride were added, followed by stirring. The precipitate was removed by filtration. The organic layer was separated and sequentially washed with water (four times) and a saturated sodium chloride solution. The solvent was evaporated under reduced pressure. Ethyl acetate and n-hexane were added and the precipitate was removed by filtration. The filtrate was concentrated under reduced pressure. The resulting residue (590 mg) was dissolved in tetrahydrofuran (5 mL). Tetrabutylammonium fluoride (1 M solution in tetrahydrofuran) (1.39 mL) was added and the mixture was stirred in a nitrogen atmosphere at room temperature for one hour. The solvent was evaporated under reduced pressure. The residue was dissolved in ethyl acetate, sequentially washed with water and a saturated sodium chloride solution and dried over magnesium sulfate. The drying agent was removed by filtration and then the organic layer was concentrated under reduced pressure. The residue was purified by silica gel column chromatography (heptane-ethyl acetate) to obtain the title compound (290 mg).
¹H-NMR (400 MHz, CDCl₃) δ (ppm): 1.14-1.24 (m, 2H), 1.46 (s, 9H), 1.46-1.56 (m, 1H), 1.75-1.86 (m, 4H), 2.26 (t, J=6.4 Hz, 1H), 2.55-2.75 (m, 2H), 3.04-3.08 (m, 2H), 4.07-4.18 (m, 2H), 4.87 (d, J=6.4 Hz, 2H), 7.26-7.29 (m, 1H) , 7.63-7.67 (m, 3H), 7.78 (d, J=8.4 Hz, 2H).

### (3) tert-Butyl 4-{2-[6-(4-cyanophenyl)-7-(N,N-dimethylaminomethyl)benz[d]isoxazol-3-yl]ethyl}piperidine-1-carboxylate

tert-Butyl 4-{2-[6-(4-cyanophenyl)-7-hydroxymethylbenz[d]isoxazol-3-yl]ethyl}piperidine-1-carboxylate (290 mg) was dissolved in tetrahydrofuran (10 mL). Triethylamine (260 µL) was added to the mixture, followed by stirring under ice-cooling in a nitrogen atmosphere. Methanesulfonyl chloride (72 µL) was added to the mixture, followed by stirring for 30 minutes. The reaction mixture was further stirred at room temperature for 15 minutes. The reaction mixture was stirred under ice-cooling. Methanesulfonyl chloride (14 µL) was added and the mixture was further stirred for 10 minutes. The precipitate was removed by filtration and the residue was washed with tetrahydrofuran (5 mL). The filtrate and the washing liquid were mixed and dimethylamine (2 M solution in tetrahydrofuran) (4.7 mL) was added. The mixture was stirred in a sealed tube at 50°C for 11 hours and 20 minutes. The reaction mixture was cooled to room temperature, and the solvent was evaporated under reduced pressure. Ethyl acetate and a 10% sodium carbonate solution were added to the residue, and the organic layer was separated. The organic layer was washed with a saturated sodium chloride solution and dried over magnesium sulfate-sodium carbonate. The drying agent was removed by filtration and then the organic layer was concentrated under reduced pressure. The residue was purified by NH silica gel column chromatography (heptane-ethyl acetate) to obtain the title compound (250 mg).
¹H-NMR (400 MHz, CDCl₃) δ (ppm): 1.14-1.24 (m, 2H), 1.46 (s, 9H), 1.48-1.60 (m, 1H), 1.75-1.86 (m, 4H), 2.19 (s, 6H), 2.67-2.75 (m, 2H), 3.02-3.06 (m, 2H), 3.56 (s 2H), 4.08-4.16 (m, 2H), 7.24 (d, J=8.0 Hz, 1H), 7.61 (d, J=8.0 Hz, 1H), 7.74 (s, 4H).

### (4) 4-{7-(N,N-Dimethylaminomethyl)-3-[2-(piperidin-4-yl)ethyl]benz[d]isoxazol-6-yl}benzonitrile

The title compound was obtained by synthesis from tert-butyl 4-{2-[6-(4-cyanophenyl)-7-(N,N-dimethylaminomethyl)benz[d]isoxazol-3-yl]ethyl}piperidine-1-carboxylate according to Example 153- (4) .
¹H-NMR (400 MHz, CDCl₃) δ (ppm): 1.20-1.30 (m, 2H), 1.48-1.56 (m, 1H) , 1 . 80-1 .86 (m, 4H), 2.19 (s, 6H), 2.59-2.66 (m, 2H), 3.02-3.06 (m, 2H), 3.11-3.16 (m, 2H), 3.56 (s, 2H), 7.24 (d, J=8.2 Hz, 1H), 7.62 (d, J=8.2 Hz, 1H) , 7.72-7.77 (m, 4H).

### (5) 4-{3-[2-(1-Benzylpiperidin-4-yl)ethyl]-7-(N,N-dimethylaminomethyl)benz[d]isoxazol-6-yl}benzonitrile

The title compound was obtained by synthesis from 4-{7-(N,N-dimethylaminomethyl)-3-[2-(piperidin-4-yl)ethyl]benz[d]isoxazol-6-yl}benzonitrile according to Example 153-(5).
¹H-NMR (400 MHz, CDCl₃) δ (ppm): 1.29-1.38 (m, 3H), 1.75-1.85 (m, 4H), 1.93-1.99 (m, 2H), 2.19 (s, 6H), 2.88-2.93 (m, 2H), 3.00-3.04 (m, 2H), 3.50 (s, 2H), 3.55 (s, 2H), 7.22-7.32 (6H, m), 7.61 (d, J=8.0 Hz, 1H), 7.72-7.76 (m, 4H).

### (6) 4-{3-[2-(1-Benzylpiperidin-4-yl)ethyl]-7-(N,N-dimethylaminomethyl)benz[d]isoxazol-6-yl}benzonitrile dihydrochloride

The title compound was obtained by converting 4-{3-[2-(1-benzylpiperidin-4-yl)ethyl]-7-(N,N-dimethylaminomethyl)benz[d]isoxazol-6-yl}benzonitrile was to a hydrochloride according to Example 153-(6).
¹H-NMR (400 MHz, DMSO-d₆) δ (ppm): 1.52-3.31 (m, 19H) , 4.24-4.35 (m, 4H), 7.40-8.14 (m, 11H), 10.55 (br s, 1H), 11.12 (br s, 1H).
ESI-MS m/z: 240 [M+2H]²⁺, 479 [M+H]⁺.

### Example 157

### N,N-Dimethyl{6-benzyl-3-[2-(1-benzylpiperidin-4-yl)ethyl]benz[d]isoxazol-7-yl}methylamine dihydrochloride

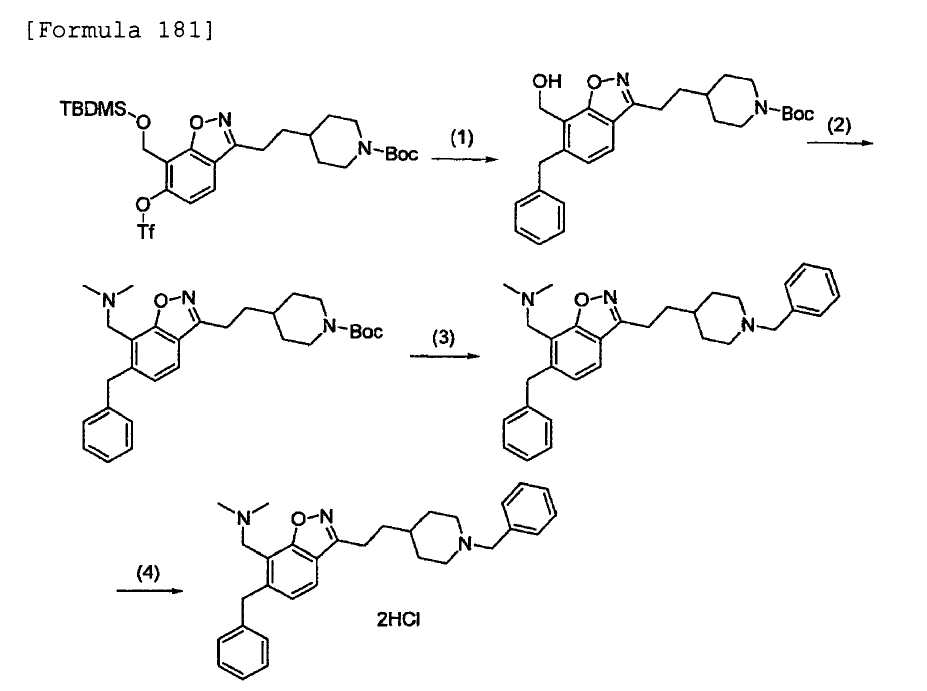

### (1) tert-Butyl 4-[2-(6-benzyl-7-hydroxymethylbenz[d]isoxazol-3-yl)ethyl]piperidine-l-carboxylate

A mixture of tert-butyl 4-{2-[7-(tert-butyldimethylsilanyloxymethyl)-6-trifluoromethanesulfonyloxybenz[d]isoxazol-3-yl]ethyl}piperidine-1-carboxylate (1.01 g), benzyltributylstannane (1.05 g), lithium chloride (210 mg), bis(triphenylphosphine)palladium (II) dichloride (110 mg) and N-methyl-2-pyrrolidone (30 mL) was stirred in a nitrogen atmosphere at 120°C for 18 hours. The reaction mixture was cooled to room temperature. Ethyl acetate, water and potassium fluoride were added to the reaction mixture, followed by stirring. The precipitate was removed by filtration. The organic layer was separated and sequentially washed with water (four times) and a saturated sodium chloride solution. The solvent was evaporated under reduced pressure. Ethyl acetate and n-hexane were added and the precipitate was removed by filtration. The filtrate was concentrated under reduced pressure. The residue was purified by silica gel column chromatography (heptane-ethyl acetate) to obtain the title compound (150 mg).
¹H-NMR (400 MHz, CDCl₃) δ (ppm): 1.09-1.74 (m, 7H), 1.46 (s, 9H), 2.39 (t, J=6.8 Hz, 1H), 2.63-2.72 (m, 2H), 2.97-3.01 (m, 2H), 4.02-4.18 (m, 2H), 4.13 (s, 2H), 4.76 (d, J=6.8 Hz, 2H), 6.73 (d, J=8.0 Hz, 1H), 7.11-7.29 (m, 5H), 7.63 (d, J=8.0 Hz, 1H).

### (2) tert-Butyl 4-{2-[6-benzyl-7-(N,N-dimethylaminomethyl)benz[d]isoxazol-3-yl}ethyl}piperidine-1-carboxylate

The title compound was obtained by synthesis from tert-butyl 4-[2-(6-benzyl-7-hydroxymethylbenz[d]isoxazol-3-yl)ethyl]piperidine-1-carboxylate according to Example 153-(2)(3).
¹H-NMR (400 MHz, CDCl₃) δ (ppm): 1.08-1.18 (m, 2H), 1. 45-1 . 71 (m, 5H), 1.45 (s, 9H), 2.23 (s, 6H), 2.64-2.73 (m, 2H), 3.01-3.05 (m, 2H), 3. 54 (s, 2H), 4.03-4.16 (m, 2H), 4.11 (s, 2H), 6.69 (d, J=8.0 Hz, 1H), 7.09-7.29 (m, 5H), 7.62 (d, J=8.0 Hz, 1H).

### (3) N,N-Dimethyl{6-benzyl-3-[2-(1-benzylpiperidin-4-yl)ethyl]benz[d]isoxazol-7-yl}methylamine dihydrochloride

The title compound was obtained by synthesis from tert-butyl 4-{2-[6-benzyl-7-(N,N-dimethylaminomethyl)benz[d]isoxazol-3-yl]ethyl}piperidine-1-carboxylate according to Example 153- (3) (4) .
¹H-NMR (400 MHz, CDCl₃) δ (ppm): 1.25-1.34 (m, 3H), 1.64-1.72 (m, 4H), 1.91-1.96 (m, 2H), 2.23 (s, 6H), 2.86-2.90 (m, 2H), 2.98-3.02 (m, 2H), 3.48 (s, 2H), 3.52 (s, 2H), 4.13 (s, 2H), 7.67 (d, J=8.0 Hz, 1H), 7.09-7.33 (m, 10H), 7.61 (d, J=8.0 Hz, 1H).

### (4) N,N-Dimethyl{6-benzyl-3-[2-(1-benzylpiperidin-4-yl)ethyl]benz[d]isoxazol-7-yl}methylamine dihydrochloride

The title compound was obtained by synthesis from tert-butyl 4-{2-[6-benzyl-7-(N,N-dimethylaminomethyl)benz[d]isoxazol-3-yl]ethyl}piperidine-1-carboxylate according to Example 153-(6).
ESI-MS m/z: 234 [M+2H]²⁺, 468 [M+H]⁺.

### Example 158

### 4-{3-[2-(1-Benzylpiperidin-4-yl)ethyl]-7-(N,N-dimethylaminomethyl)benz[d]isoxazol-6-yloxy}benzonitrile dihydrochloride

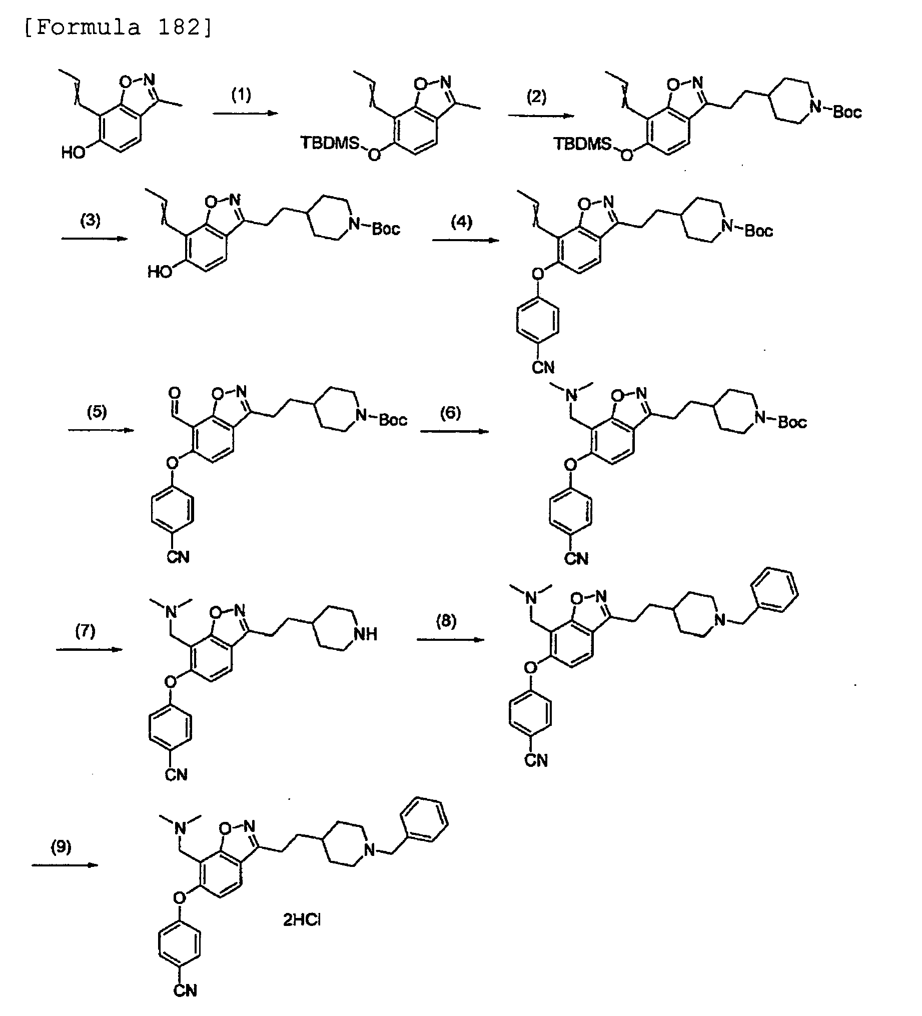

### (1) Mixture of 6-(tert-butyldimethylsilanyloxy)-3-methyl-7-[(E)-1-propenyl]benz[d]isoxazole and 6-(tert-butyldimethylsilanyloxy)-3-methyl-7-[(Z)-1-propenyl]benz[d]isoxazole

The mixture of 6-hydroxy-3-methyl-7-[(E)-1-propenyl]benz[d]isoxazole and 6-hydroxy-3-methyl-7-[(Z)-1-propenyl]benz[d]isoxazole (compound obtained in Example 76-(3)) (2.48 g) and tert-butyldimethylchlorosilane (2.37 g) were dissolved in a N,N-dimethylformamide solution (40 mL). Imidazole (2.23 g) was added to the solution, and the mixture was stirred in a nitrogen atmosphere at room temperature for 18 hours and 10 minutes. The solvent was evaporated under reduced pressure. Ethyl acetate and water were added to the residue, and the organic layer was separated. The organic layer was sequentially washed with water (three times) and a saturated sodium chloride solution and dried over magnesium sulfate. The drying agent was removed by filtration and then the organic layer was concentrated under reduced pressure. The residue was purified by silica gel column chromatography (heptane-ethyl acetate) to obtain the title compound (250 mg).
¹H-NMR (400 MHz, CDCl₃) δ (ppm): 0.24 (s, 6H), 1.05 (s, 9H), 1.98-2.00 (m, 3H), 2.52 (s, 3H), 6.71-6.76 (m, total 1H), 6.80 (d, J=8.6 Hz, 1H), 6.89-6.98 (m, total 1H), 7.25 (d, J=8.6 Hz, 1H).

### (2) Mixture of tert-butyl 4-{2-{6-(tert-butyldimethylsilanyloxy)-7-[(E)-1-propenyl]benz[d]isoxazol-3-yl}ethyl}piperidine-1-carboxylate and tert-butyl 4-{2-{6-(tert-butyldimethylsilanyloxy)-7-[(Z)-1-propenyl]benz[d]isoxazol-3-yl}ethyl}piperidine-1-carboxylate

N,N-Diisopropylamine (4.9 mL) was dissolved in tetrahydrofuran (26 mL), and the solution was stirred in a dry ice-acetone bath in a nitrogen atmosphere. n-Butyllithium (2.5 M solution in hexane) (9.3 mL) was added dropwise to the mixture. The reaction mixture was stirred under ice-cooling for five minutes and stirred in the dry ice-acetone bath again to prepare a solution of lithium diisopropylamide in tetrahydrofuran. The mixture of 6-(tert-butyldimethylsilanyloxy)-3-methyl-7-[(E)-1-propenyl]benz[d]isoxazole and 6-(tert-butyldimethylsilanyloxy)-3-methyl-7-[(Z)-1-propenyl]benz[d]isoxazole (3.64 g) and a solution of tert-butyl 4-iodomethylpiperidine-1-carboxylate (4.47 g) in tetrahydrofuran (80 mL) were added dropwise to the lithium diisopropylamide solution. The mixture was gradually heated with stirring for 35 minutes. A saturated ammonium chloride solution, water and ethyl acetate were added and the organic layer was separated. The organic layer was washed with a saturated sodium chloride solution and dried over sodium sulfate. The drying agent was removed by filtration and then the organic layer was concentrated under reduced pressure. The residue was purified by silica gel column chromatography (heptane-ethyl acetate) to obtain the title compound (5.94 g).
¹H-NMR (400 MHz, CDCl₃) δ (ppm): 0.24 (s, 6H), 1.05 and 1.06 (s, total 9H), 1.46 (s, 9H), 1.11-1.21 (m, 2H), 1.46-1.56 (m, 1H), 1.71-1.81 (m, 4H), 1.98-1.99 (m, total 3H), 2.65-2.73 (m, 2H), 2.93-2.97 (m, 2H), 4.05-4.15 (m, 2H), 6.71-6.76 (m, total 1H), 6.79 (d, J=1H), 6.89-6.98 (m, total 1H), 7.25 (d, J=8.4 Hz, 1H).

### (3) Mixture of tert-butyl 4-{2-{6-hydroxy-7-[(E)-1-propenyl]benz[d]isoxazol-3-yl}ethyl}piperidine-1-carboxylate and tert-butyl 4-{2-{6-hydroxy-7-[(Z)-1-propenyl]benz[d]isoxazol-3-yl}ethyl}piperidine-1-carboxylate

The mixture of tert-butyl 4-{2-{6-(tert-butyldimethylsilanyloxy)-7-[(E)-1-propenyl]benz[d]isoxazol-3-yl}ethyl}piperidine-1-carboxylate and tert-butyl 4-{2-{6-(tert-butyldimethylsilanyloxy)-7-[(Z)-1-propenyl]benz[d]isoxazol-3-yl}ethyl}piperidine-1-carboxylate (5.94 g) was dissolved in tetrahydrofuran (80 mL). Tetrabutylammonium fluoride (1 M solution in tetrahydrofuran) (14.3 mL) was added to the solution, and the mixture was stirred in a nitrogen atmosphere at room temperature for 45 minutes. The solvent was evaporated under reduced pressure. The residue was dissolved in ethyl acetate, sequentially washed with a 5% sodium bisulfate solution, water (three times) and a saturated sodium chloride solution and dried over magnesium sulfate. The drying agent was removed by filtration and then the organic layer was concentrated under reduced pressure. Ethyl acetate and n-hexane were added to the residue, and the solid was collected by filtration. The resulting solid was dried under reduced pressure to obtain the title compound (3.96 g). ¹H-NMR (400 MHz, CDCl₃) δ (ppm): 1.10-1.21 m, 2H), 1.46 (s, 9H), 1.46-1.55 (m, 1H), 1.73-1.80 (m, 4H), 2.00-2.02 (m, 3H), 2.64-2.72 (m, 2H), 2.93-2.97 (m, 2H), 4.05-4.14 (m, 2H), 5.98 (s, 1H) , 6.06-6.71 (m, total 1H) , 6.78-6.87 (m, total 2H), 7.27 (d, J=8.0 Hz, 1H).

### (4) Mixture of tert-butyl 4-{2-{6-(4-cyanophenoxy)-7-[(E)-1-propenyl]benz[d]isoxazol-3-yl}ethyl}piperidine-1-carboxylate and tert-butyl 4-{2-16-(4-cyanophenoxy)-7-[(Z)-1-propenyl]benz[d]isoxazol-3-yl}ethyl}piperidine-1-carboxylate

The following reaction was performed according to the method described in J. Org. Chem., 1998, 63, 6338. A mixture of the mixture of tert-butyl 4-{2-{6-hydroxy-7-[(E)-1-propenyl]benz[d]isoxazol-3-yl}ethyl}piperidine-1-carboxylate and tert-butyl 4-{2-{6-hydroxy-7-[(Z)-1-propenyl]benz[d]isoxazol-3-yl}ethyl}piperidine-1-carboxylate (110 mg), 4-fluorobenzonitrile (340 mg), 18-crown-6 (7 mg), potassium fluoride-alumina (110 mg) and dimethyl sulfoxide (980 µL) was stirred in a nitrogen atmosphere at 100°C for 9.5 hours. The reaction mixture was cooled to room temperature. Ethyl acetate was added to the reaction mixture, and the precipitate was removed by filtration. The filtrate was sequentially washed with water (three times) and a saturated sodium chloride solution and dried over magnesium sulfate. The drying agent was removed by filtration and then the organic layer was concentrated under reduced pressure. The residue was purified by silica gel column chromatography (n-hexane-ethyl acetate) to obtain the title compound (110 mg). ¹H-NMR (400 MHz, CDCl₃) δ (ppm): 1.13-1.24 (m, 2H), 1.46 (s, 9H), 1.49-1.57 (m, 1H), 1.73-1.84 (m, 4H), 1.94-1.96 (m, total 3H), 2.66-2.74 (m, 2H), 3.00-3.04 (m, 2H), 4.06-4.16 (m, 2H), 6.53-6.58 (m, total 1H), 6.94 (d, J=8.4 Hz, 1H), 6.97 (d, J=9.0 Hz, 2H), 7.03-7.12 (m, total 1H), 7.43 (d, J=8.4 Hz, 1H), 7.62 (d, J=9.0 Hz, 2H).

### (5) tert-Butyl 4-{2-[6-(4-cyanophenoxy)-7-formylbenz[d]isoxazol-3-yl]ethyl}piperidine-1-carboxylate

The mixture of tert-butyl 4-{2-{6-(4-cyanophenoxy)-7-[(E)-1-propenyl]benz[d]isoxazol-3-yl}ethyl}piperidine-1-carboxylate and tert-butyl 4-{2-{6-(4-cyanophenoxy)-7-[(Z)-1-propenyl]benz[d]isoxazol-3-yl}ethyl}piperidine-1-carboxylate (106 mg) was dissolved in tetrahydrofuran (3 mL) and water (0.75 mL). Osmium tetroxide (2.5% aqueous solution) (66 µL) was added to the solution, and the reaction mixture was stirred at room temperature for five minutes. Sodium metaperiodate (116 mg) was added to the solution, and the reaction mixture was stirred for six hours and 30 minutes. Ethyl acetate and water were added to the reaction mixture, and the organic layer was separated. The organic layer was washed with a saturated sodium chloride solution and dried over magnesium sulfate. The drying agent was removed by filtration and then the organic layer was concentrated under reduced pressure. The residue was purified by silica gel column chromatography (n-hexane-ethyl acetate) to obtain the title compound (83 mg).
¹H-NMR (400 MHz, CDCl₃) δ (ppm): 1.12-1.22 (m, 2H), 1.46 (s, 9H), 1.49-1.56 (m, 1H), 1.72-1.83 (m, 4H), 2.64-2.73 (m, 2H), 3.01-3.05 (m, 2H), 4.07-4.16 (m, 2H), 6.96 (d, J=8.4 Hz, 1H) , 7.16 (d, J=9.2 Hz, 2H), 7.72 (d, J=9.2 Hz, 2H), 7.82 (d, J=8.4 Hz, 1H), 10.57 (s, 1H).

### (6) tert-Butyl 4-{2-[6-(4-cyanophenoxy)-7-(N,N-dimethylaminomethyl)benz[d]isoxazol-3-yl]ethyl}piperidine-1-carboxylate

tert-Butyl 4-{2-[6-(4-cyanophenoxy)-7-formylbenz[d]isoxazol-3-yl]ethyl}piperidine-1-carboxylate (83 mg) was dissolved in methylene chloride (2 mL). Dimethylamine (2 M solution in tetrahydrofuran) (350 µL) and acetic acid (40 µL) were added to the mixture, and the reaction mixture was stirred in a nitrogen atmosphere at room temperature for 15 minutes. Sodium triacetoxyborohydride (78 mg) was added and the reaction mixture was stirred for 12 hours and 15 minutes. A 10% sodium carbonate solution was added, followed by extraction with chloroform three times. The combined organic layers were dried over magnesium sulfate. The drying agent was removed by filtration and then the organic layers were concentrated under reduced pressure. The residue was purified by NH silica gel column chromatography (n-hexane-ethyl acetate) to obtain the title compound (66 mg).
¹H-NMR (400 MHz, CDCl₃) δ (ppm): 1.13-1.23 (m, 2H), 1.46 (s, 9H), 1.51-1.58 (m, 1H), 1.74-1.84 (m, 4H), 2.29 (s, 6H), 2.65-2.75 (m, 2H), 2.99-3.03 (m, 2H), 3.73 (s, 2H), 4.06-4.16 (m, 2H), 6.95 (d, J=8.6 Hz, 1H), 7.01 (d, J=8.6 Hz, 2H), 7.55 (d, J=8.6 Hz, 1H), 7.62 (d, J=8.6 Hz, 2H).

### (7) 4-{7-(N,N-Dimethylaminomethyl)-3-[2-(piperidin-4-yl)ethyl]benz[d]isoxazol-6-yloxy}benzonitrile

The title compound was obtained by synthesis from tert-butyl 4-{2-[6-(4-cyanophenoxy)-7-(N,N-dimethylaminomethyl)benz[d]isoxazol-3-yl]ethyl}piperidine-1-carboxylate according to Example 153-(4).
¹H-NMR (400 MHz, CDCl₃) δ (ppm): 1.20-1.31 (m. 2H), 1.48-1.59 (m, 1H), 1.78-1.83 (m, 4H), 2.29 (s, 6H), 2.60-2.67 (m, 2H), 2.98-3.02 (m, 2H), 3.11-3.17 (m, 2H), 3.73 (s, 2H), 6.95 (d, J=8.6 Hz, 1H), 7.01 (d, J=9.0 Hz, 2H), 7.55 (d, J=8.6 Hz, 1H), 7.62 (d, J=9.0 Hz, 2H).

### (8) 4-{3-[2-(1-Benzylpiperidin-4-yl)ethyl]-7-(N,N-dimethylaminomethyl)benz[d]isoxazol-6-yloxy}benzonitrile

The title compound was obtained by synthesis from 4-{7-(N,N-dimethylaminomethyl)-3-[2-(piperidin-4-yl)ethyl]benz[d]isoxazol-6-yloxy}benzonitrile according to Example 153-(5).
¹H-NMR (400 MHz, CDCl₃) δ (ppm): 1.31-1.43 (m, 3H), 1.73-1.82 (m, 4H), 1.92-1.99 (m, 2H), 2.28 (s, 6H), 2.87-2.92 (m, 2H), 2.97-3.01 (m, 2H), 3.49 (s, 2H), 3.72 (s, 2H), 6.94 (d, J=8.4 Hz, 1H), 7.00 (d, J=8.8 Hz, 2H), 7.22-7.32 (m, 5H), 7.54 (d, J=8.4 Hz, 1H), 7.62 (d, J=8.8 Hz, 2H).

### (9) 4-{3-[2-(1-Benzylpiperidin-4-yl)ethyl]-7-(N,N-dimethylaminomethyl)benz[d]isoxazol-6-yloxy}benzonitrile dihydrochloride

The title compound was obtained by synthesis from 4-(3-[2-(1-benzylpiperidin-4-yl)ethyl]-7-(N,N-dimethylaminomethyl)benz[d]isoxazol-6-yloxy}benzonitrile according to Example 153-(6).
¹H-NMR (400 MHz, DMSO-d₆) δ (ppm): 1.47-1.96 (m, 7H), 2.82-2.92 (m, 8H), 3.00-3.19 (m, 2H), 3.27-3.32 (m, 2H), 4.22-4.58 (m, 4H), 7.04 (d, J=8.8 Hz, 1H), 7.39 (d, J=8.8 Hz, 2H), 7.43-7.47 (m, 3H), 7.62-7.67 (m, 2H), 7.96 (d, J=8.8 Hz, 2H), 8.02 (d, J=8.8 Hz, 1H), 10.79 (br s, 1H), 10.92 (br s, 1H).
ESI-MS m/z: 248 [M+2H]²⁺, 495 [M+H]⁺.

### Example 159

### 6-{3-[2-(1-Benzylpiperidin-4-yl)ethyl]-7-(N,N-dimethylaminomethyl)benz[d]isoxazol-6-yloxy}pyridine-3-carbonitrile fumarate

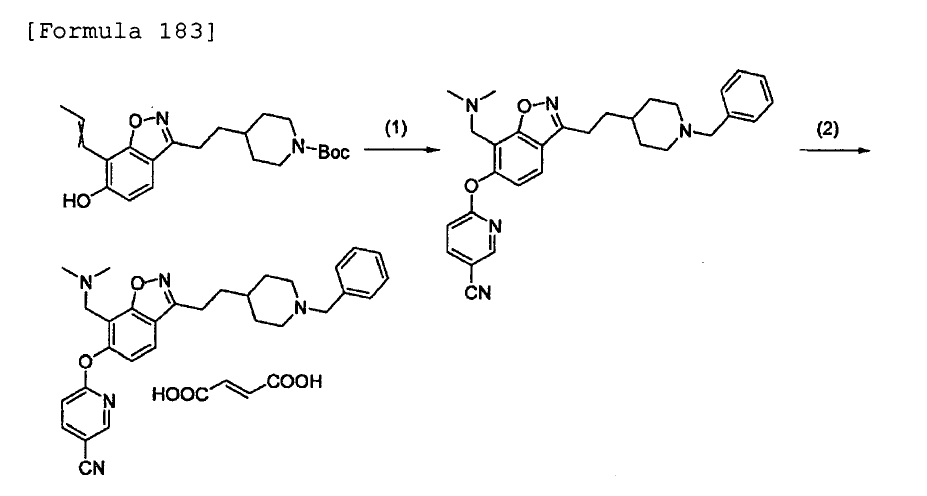

### (1) 6-{3-[2-(1-Benzylpiperidin-4-yl)ethyl]-7-(N,N-dimethylaminomethyl)benz[d]isoxazol-6-yloxy}pyridine-3-carbonitrile

The title compound was obtained by synthesis according to Example 158-(4) to (8) from the mixture of tert-butyl 4-{2-{6-hydroxy-7-[(E)-1-propenyl]benz[d]isoxazol-3-yl}ethyl}piperidine-1-carboxylate and tert-butyl 4-{2-{6-hydroxy-7-[(Z)-1-propenyl]benz[d]isoxazol-3-yl}ethyl}piperidine-1-carboxylate obtained in Example 158-(3).
¹H-NMR (400 MHz, CDCl₃) δ (ppm): 1.23-1.46 (m, 3H), 1.74-1.83 (m, 4H), 1.93-1.99 (m, 2H), 2.22 (s, 6H), 2.87-2.92 (m, 2H), 2.97-3.01 (m, 2H), 3.50 (s, 2H), 3.66 (s, 2H), 7.06 (d, J=8.4 Hz, 1H), 7.14 (dd, J=0.8, 8.8 Hz, 1H), 7.25-7.32 (m, 5H), 7.58 (d, J=8.4 Hz, 1H), 7.96 (dd, J=2.2, 8.6 Hz, 1H), 8.41-8.42 (m, 1H).

### (2) 6-{3-[2-(1-Benzylpiperidin-4-yl)ethyl]-7-(N,N-dimethylaminomethyl)benz[d]isoxazol-6-yloxy}pyridine-3-carbonitrile fumarate

The title compound was obtained by synthesis from 6-{3-[2-(1-benzylpiperidin-4-yl)ethyl]-7-(N,N-dimethylaminomethyl)benz[d]isoxazol-6-yloxy}pyridine-3-carbonitrile according to Example 154-(4).
¹H-NMR (400 MHz, DMSO-d₆) δ (ppm): 1.23-1.42 (m, 3H), 1.66-1.78 (m, 4H), 2.08 (s, 6H), 2.05-2.14 (m, 2H), 2.86-2.93 (m, 2H), 2.99-3.05 (m, 2H), 3.58 (s, 2H), 3.60 (s, 2H), 6.59 (s, 2H), 7.21 (d, J=8.8 Hz, 1H) , 7.26-7.36 (m, 6H), 7.86 (d, J=8.8 Hz, 1H), 8.36 (dd, J=2.2, 8.8 Hz, 1H) , 8.61 (d, J=2.2 Hz, 1H).
ESI-MS m/z: 248 [M+2H]²⁺, 496 [M+H]⁺.

### Example 160

### 4-{7-(N,N-Dimethylaminomethyl)-3-{2-[1-(pyridin-2-ylmethyl)piperidin-4-yl]ethyl}benz[d]isoxazol-6-yloxy}benzonitrile trihydrochloride

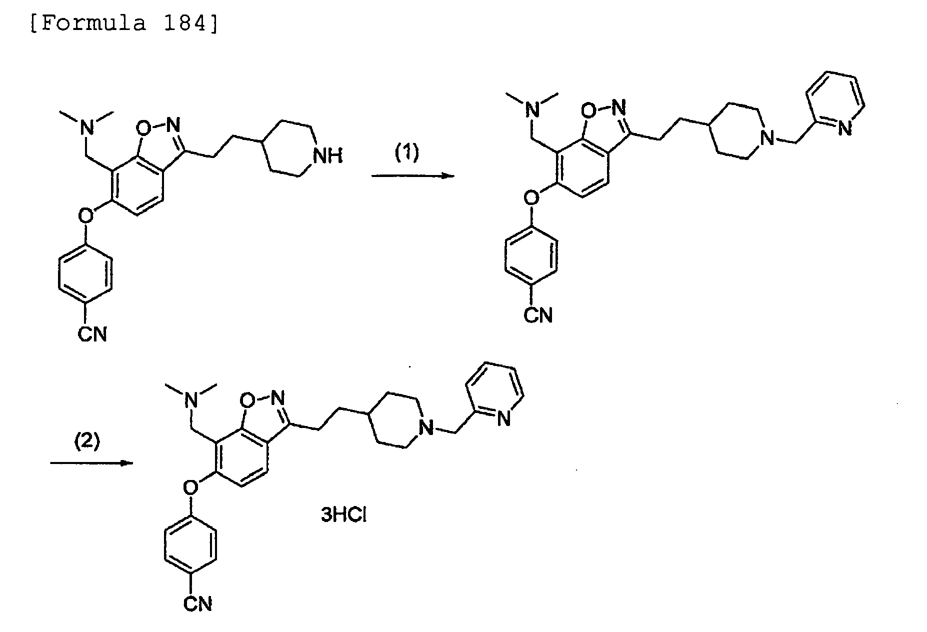

### (1) 4-{7-(N,N-Dimethylaminomethyl)-3-{2-[1-(pyridin-2-ylmethyl)piperidin-4-yl]ethyl}benz[d]isoxazol-6-yloxy}benzonitrile

The title compound was obtained by synthesis according to Example 153-(5) from 2-formylpyridine and 4-{7-(N,N-dimethylaminomethyl)-3-[2-(piperidin-4-yl)ethyl]benz[d]isoxazol-6-yloxy}benzonitrile obtained in Example 53-(4).
¹H-NMR (400 MHz, CDCl₃) δ (ppm): 1.33-1.43 (m, 3H), 1.74-1.83 (m, 4H), 2.04-2.11 (m, 2H), 2.28 (s, 6H), 2.89-2.94 (m, 2H), 2.97-3.01 (m, 2H), 3.64 (s, 2H), 3.72 (s, 2H), 6.95 (d, J=8.6 Hz, 1H), 7.00 (d, J=8.8 Hz, 2H), 7.14-7.17 (m, 1H), 7.40 (d, J=8.6 Hz, 1H), 7.54 (d, J=8.6 Hz, 1H), 7.62 (d, J=8.8 Hz, 2H), 7.63-7.67 (m, 1H), 8.55-8.57 (m, 1H).

### (2) 4-{7-(N,N-Dimethylaminomethyl)-3-{2-[1-(pyridin-2-ylmethyl)piperidin-4-yl]ethyl}benz[d]isoxazol-6-yloxy}benzonitrile trihydrochloride

The title compound was obtained by synthesis from 4-{7-(N,N-dimethylaminomethyl)-3-{2-[1-(pyridin-2-ylmethyl)piperidin-4-yl]ethyl}benz[d]isoxazol-6-yloxy}benzonitrile according to Example 153-(6).
¹H-NMR (400 MHz, DMSO-d₆) δ (ppm): 1.58-1.96 (m, 7H), 2.81-2.85 (m, 6H), 3.00-3.08 (m, 4H), 3.34-3.44 (m, 2H), 4.45 (s, 2H), 4.57-4.58 (m, 2H), 7.04 (d, J=8.6 Hz, 1H), 7.39 (d, J=8.6 Hz, 2H), 7.49-7.52 (m, 1H), 7.73-7.75 (m, 1H), 7.94-7.97 (m, 1H), 7.96 (d, J=8.6 Hz, 2H), 8.03 (d, J=8.6 Hz, 1H), 8.67-8.69 (m, 1H), 10.68 (br s, 1H) , 10.90 (br s, 1H).
ESI-MS m/z: 249 [M+2H]²⁺, 496 [M+H]⁺.

### Example 161

### 4-{7-(N,N-Dimethylaminomethyl)-3-{2-[1-(1,3-dioxolan-2-ylmethyl)piperidin-4-yl]ethyl}benz[d]isoxazol-6-yloxy}benzonitrile dihydrochloride

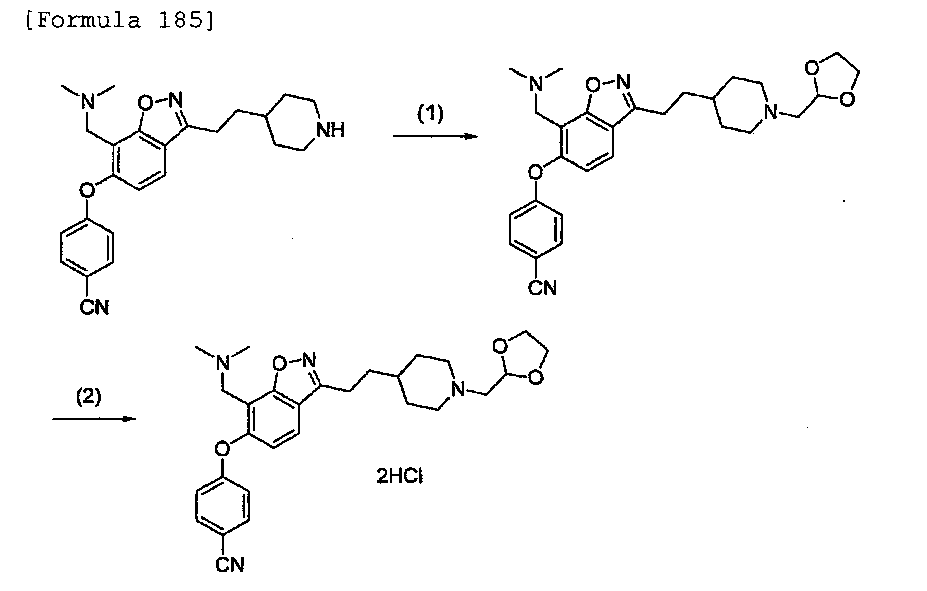

### (1) 4-{7-(N,N-Dimethylaminomethyl)-3-{2-[1-(1,3-dioxolan-2-ylmethyl)piperidin-4-yl]ethyl}benz[d]isoxazol-6-yloxy}benzonitrile

A mixture of 4-{7-(N,N-dimethylaminomethyl)-3-[2-(piperidin-4-yl)ethyl]benz[d]isoxazol-6-yloxy}benzonitrile obtained in Example 53-(4) (157 mg), sodium iodide (62 mg), 2-bromomethyl-1,3-dioxolane (100 µL), N,N-diisopropylethylamine (338 µL) and acetonitrile (10 mL) was heated under reflux in a nitrogen atmosphere for six hours. 2-Bromomethyl-1,3-dioxolane (20 µL) and N,N-diisopropylethylamine (68 µL) were added and the mixture was heated under reflux for 14 hours and 10 minutes. The solvent was evaporated under reduced pressure. A saturated sodium chloride solution and a 10% sodium carbonate solution were added to the residue, followed by extraction with ethyl acetate three times. The combined organic layers were sequentially washed with a 10% sodium thiosulfate solution and a saturated sodium chloride solution and dried over magnesium sulfate-sodium carbonate. The drying agent was removed by filtration and then the organic layers were concentrated under reduced pressure. The residue was purified by NH silica gel column chromatography (n-hexane-ethyl acetate) to obtain the title compound (80 mg).
¹H-NMR (400 MHz, CDCl₃) δ (ppm): 1.35-1.42 (m, 3H), 1.73-1.82 (m, 4H), 2.05-2.11 (m, 2H), 2.28 (s, 6H), 2.57 (d, J=4.4 Hz, 2H), 2.97-3.03 (m, 4H), 3.72 (s, 2H), 3.82-3.90 (m, 2H), 3.93-4.01 (m, 2H), 5.01 (t, J=4.4 Hz, 1H), 6.95 (d, J=8.4 Hz, 1H), 8.00 (d, J=8.8 Hz, 2H), 7.54 (d, J=8.4 Hz, 1H), 7.62 (d, J=8.8 Hz, 2H).

### (2) 4-{7-(N,N-Dimethylaminomethyl)-3-{2-[1-(1,3-dioxolan-2-ylmethyl)piperidin-4-yl]ethyl}benz[d]isoxazol-6-yloxy}benzonitrile fumarate

The title compound was obtained by synthesis from 4-{7-(N,N-dimethylaminomethyl)-3-{2-[1-(1,3-dioxolan-2-ylmethyl)piperidin-4-yl]ethyl}benz[d]isoxazol-6-yloxy}benzonitrile according to Example 154-(4).
¹H-NMR (400 MHz, DMSO-d₆) δ (ppm): 1.16-1.38 (m, 3H), 1.68-1.74 (m,4H), 2.09-2.16 (m, 2H), 2.16 (s, 6H), 2.55 (d, J=4.0 Hz, 2H), 2.97-3.05 (m, 4H), 3.68 (s, 2H), 3.74-3.81 (m, 2H), 3.83-3.90 (m, 2H), 4.93 (t, J=4.0 Hz, 1H) , 6.59 (s, 2H), 7.09 (d, J=8.8 Hz, 1H), 7.13 (d, J=8.6 Hz, 2H), 7.85 (d, J=8.6 Hz, 2H), 7.88 (d, J=8.8 Hz, 1H).
ESI-MS m/z: 246 [M+2H]²⁺, 491 [M+H]⁺, 513 [M+Na]⁺.

### Example 162

### 6-{4-{2-[6-Cyclopropylmethoxy-7-(N,N-dimethylaminomethyl)benz[d]isoxazol-3-yl]ethyl}piperidinomethyl}pyridine-2-carbonitrile fumarate

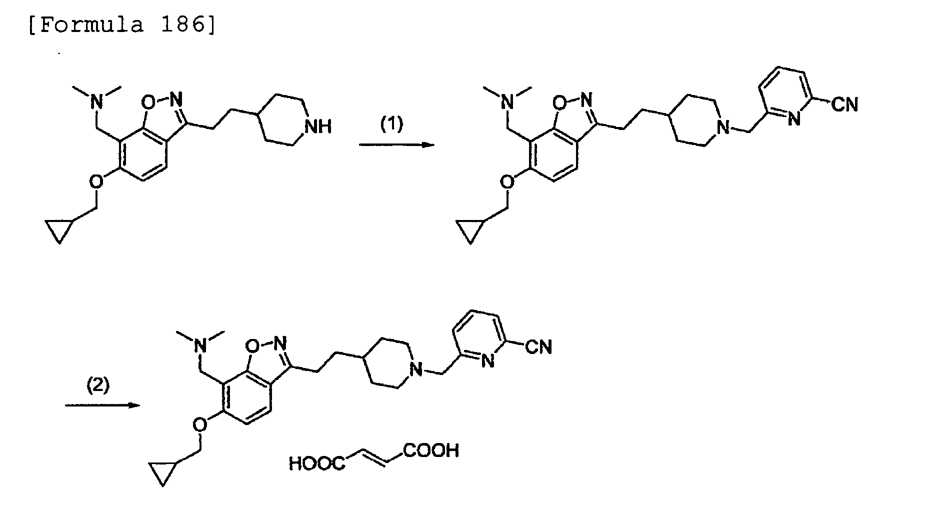

### (1) 6-{4-(2-[6-Cyclopropylmethoxy-7-(N,N-dimethylaminomethyl)benz[d]isoxazol-3-yl]ethyl}piperidinomethyl}pyridine-2-carbonitrile

6-Formylpyridine-2-carbonitrile was synthesized by the method described in Chem. Pharm. Bull., 1990, 38, 2446. The title compound was obtained by synthesis from N,N-dimethyl{6-cyclopropylmethoxy-3-[2-(piperidin-4-yl)ethyl]benz[d]isoxazol-7-yl}methylamine and 6-formylpyridine-2-carbonitrile according to Example 153-(5).
¹H-NMR (400 MHz, CDCl₃) δ (ppm): 0.34-0.39 (m, 2H), 0.62-0.67 (m, 2H), 1.28-1.46 (m, 4H), 1.73-1.81 (m, 4H), 2.07-2.13 (m, 2H), 2.34 (s, 6H), 2.83-2.87 (m, 2H), 2.93-2.97 (m, 2H), 3.67 (s, 2H), 3.82 (s, 2H), 3.94 (d, J=6.4 Hz, 2H), 6.90 (d, J=8.8 Hz, 1H), 7.45 (d, J=8.8 Hz, 1H), 7.57 (dd, J=1.0, 7.4 Hz, 1H), 7.72 (dd, J=1.0, 8.2 Hz, 1H), 7.77-7.81 (m, 1H).

### (2) 6-{4-{2-[6-Cyclopropylmethoxy-7-(N,N-dimethylaminomethyl)benz[d]isoxazol-3-yl]ethyl}piperidinomethyl}pyridine-2-carbonitrile fumarate

The title compound was obtained by synthesis from 6-{4-{2-[6-cyclopropylmethoxy-7-(N,N-dimethylaminomethyl)benz[d]isoxazol-3-yl]ethyl}piperidinomethyl}pyridine-2-carbonitrile according to Example 154-(4).
¹H-NMR (400 MHz, DMSO-d₆) δ (ppm): 0.34-0.37 (m, 2H), 0.56-0.60 (m, 2H), 1.15-1.36 (m, 4H), 1.66-1.74 (m, 4H), 1.99-2.04 (m, 2H), 2.22 (s, 6H), 2.77-2.81 (m, 2H), 2.92-2.96 (m, 2H), 3.62 (s, 2H), 3.75 (s, 2H), 3.98 (d, J=7.2 Hz, 2H), 6.60 (s, 2H), 7.12 (d, J=8.8 Hz, 1H), 7.73 (d, J=8.8 Hz, 1H), 7.77 (d, J=7.6 Hz, 1H), 7.92 (d, J=7.2 Hz, 1H), 8.01-8.05 (m, 1H).
ESI-MS m/z: 238 [M+2H]²⁺, 474 [M+H]⁺, 496 [M+Na]⁺.

### Example 163

### 5-{4-{2-[6-Cyclopropylmethoxy-7-(N,N-dimethylaminomethyl)benz[d]isoxazol-3-yl]ethyl}piperidinomethyl}thiophene-3-carbonitrile fumarate

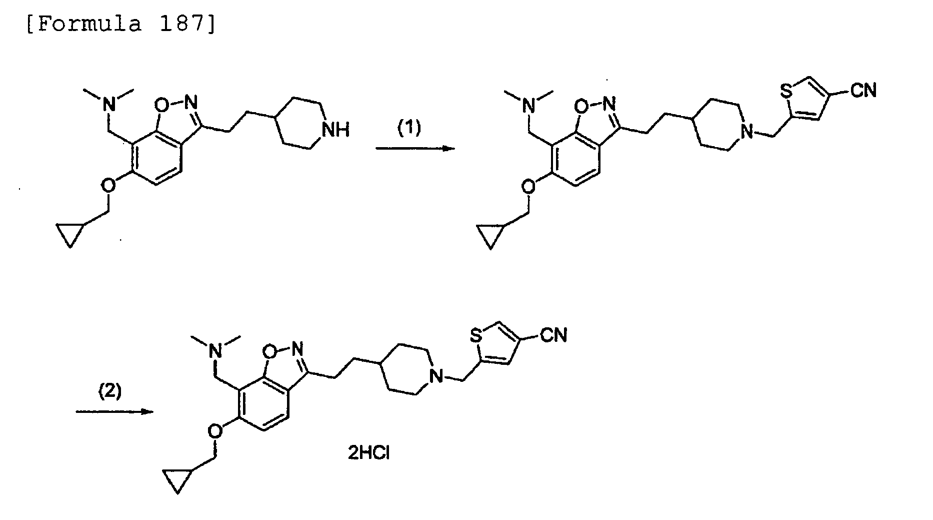

### (1) 5-{4-{2-[6-Cyclopropylmethoxy-7-(N,N-dimethylaminomethyl)benz[d]isoxazol-3-yl]ethyl}piperidinomethyl}thiophene-3-carbonitrile

5-Formylthiophene-3-carbonitrile was synthesized by the method described in Chem. Pharm. and JP-A-2000-516598. The title compound was obtained by synthesis from N,N-dimethyl{6-cyclopropylmethoxy-3-[2-(piperidin-4-yl)ethyl]benz[d]isoxazol-7-yl}methylamine and 5-formylthiophene-3-carbonitrile according to Example 153-(5).
¹H-NMR (400 MHz, CDCl₃) δ (ppm): 0.35-0.39 (m, 2H), 0.62-0.67 (m, 2H), 1.27-1.43 (m, 4H), 1.75-1.80 (m, 4H), 1.98-2.05 (m, 2H), 2.34 (s, 6H), 2.86-2.96 (m, 4H), 3.65 (s, 2H), 3.82 (s, 2H), 3.94 (d, J=6.8 Hz, 2H), 6.90 (d, J=8.6 Hz, 1H), 7.06 (d, J=0.6 Hz, 1H), 7.45 (d, J=8.6 Hz, 1H), 7.82 (d, J=0.6 Hz, 1H).

### (2) 5-{4-{2-[6-Cyclopropylmethoxy-7-(N,N-dimethylaminomethyl)benz[d]isoxazol-3-yl]ethyl}piperidinomethyl}thiophene-3-carbonitrile dihydrochloride

The title compound was obtained by synthesis from 5-{4-{2-[6-cyclopropylmethoxy-7-(N,N-dimethylaminomethyl)benz[d]isoxazol-3-yl]ethyl}piperidinomethyl}thiophene-3-carbonitrile according to Example 153-(6).
¹H-NMR (400 MHz, DMSO-d₆) δ (ppm): 0.39-0.42 (m, 2H), 0.58-0.63 (m, 2H), 1.30-1.96 (m, 10H), 2.80 (s, 6H), 2.80-3.16 (m, 4H), 4.08 (d, J=7.2 Hz, 2H), 4.46-4.55 (m, 4H), 7.25 (d, J=8.8 Hz, 1H), 7.78 (s, 1H), 7.97 (d, J=8.8 Hz, 1H), 8.69 (s, 1H), 10.33 (br s, 1H), 11.18 (br s, 1H).
ESI-MS m/z: 240 [M+2H]²⁺, 479 [M+H]⁺.

### Example 164

### 4-{4-{2-[6-Cyclopropylmethoxy-7-(N,N-dimethylaminomethyl)benz[d]isoxazol-3-yl]ethyl}piperidinomethyl}thiophene-2-carbonitrile fumarate

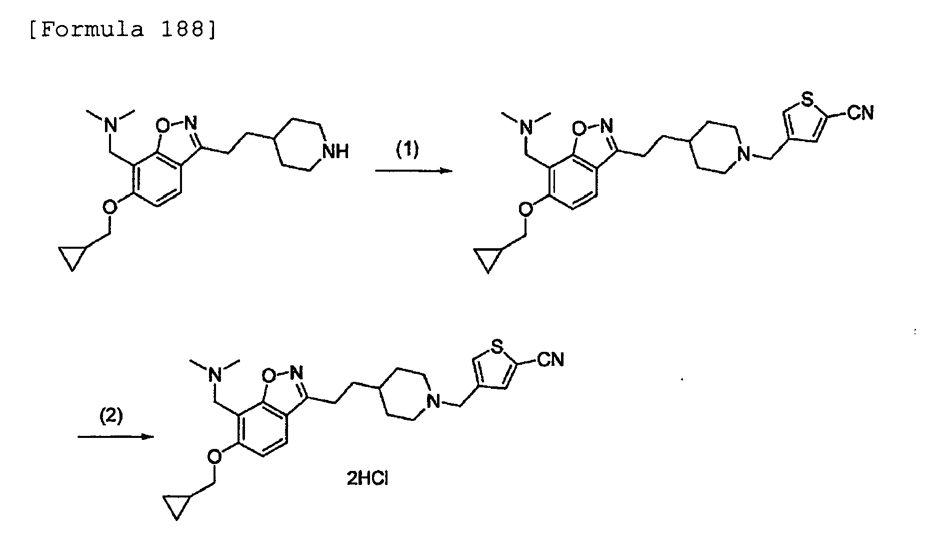

### (1) 4-{4-{2-[6-Cyclopropylmethoxy-7-(N,N-dimethylaminomethyl)benz[d]isoxazol-3-yl]ethyl}piperidinomethyl}thiophene-2-carbonitrile

4-Formylthiophene-2-carbonitrile was synthesized by the method described in JP-A-2000-516598. The title compound was obtained by synthesis from N,N-dimethyl(6-cyclopropylmethoxy-3-[2-(piperidin-4-yl)ethyl]benz[d]isoxazol-7-yl}methylamine and 4-formylthiophene-2-carbonitrile according to Example 153-(5).
¹H-NMR (400 MHz, CDCl₃) δ (ppm): 0.35-0.39 (m, 2H), 0.62-0.67 (m, 2H), 1.26-1.34 (m. 4H), 1.74-1.80 (m, 4H), 1.91-1.97 (m, 2H), 2.34 (s, 6H), 2.81-2.85 (m, 2H), 2.92-2.96 (m, 2H), 3.47 (s, 2H), 3.82 (s, 2H), 3.94 (d, J=6.8 Hz, 2H), 6.90 (d, J=8.6 Hz, 1H), 7.37 (d, J=1.2 Hz, 1H), 7.44 (d, J=8.6 Hz, 1H), 7.58 (d, J=1.2 Hz, 1H).

### (2) 4-{4-{2-[6-Cyclopropylmethoxy-7-(N,N-dimethylaminomethyl)benz[d]isoxazol-3-yl]ethyl}piperidinomethyl}thiophene-2-carbonitrile dihydrochloride

The title compound was obtained by synthesis from 4-{4-{2-[6-cyclopropylmethoxy-7-(N,N-dimethylaminomethyl)benz[d]isoxazol-3-yl]ethyl}piperidinomethyl}thiophene-2-carbonitrile according to Example 153-(6).
¹H-NMR (400 MHz, DMSO-d₆) δ (ppm): 0.40-0.46 (m, 2H), 0.58-0.63 (m, 2H), 1.29-1.96 (m, 8H), 2.78-3.34 (m, 12H), 4.07 (d, J=7.2 Hz, 2H), 4.28-4.47 (m, 4H), 7.25 (d, J=8.8 Hz, 1H), 7.96 (d, J=8.8 Hz, 1H), 8.21-8.22 (m, 1H), 8.29 (s, 1H), 10.42 (br s, 1H), 11.20 (br s 1H).
ESI-MS m/z: 240 [M+2H]²⁺, 479 [M+H]⁺.

### Example 165

### 4-{4-(2-[6-Cyclopropylmethoxy-7-(N,N-dimethylaminomethyl)benz[d]isoxazol-3-yl]ethyl}piperidinomethyl}pyridine-2-carbonitrile fumarate

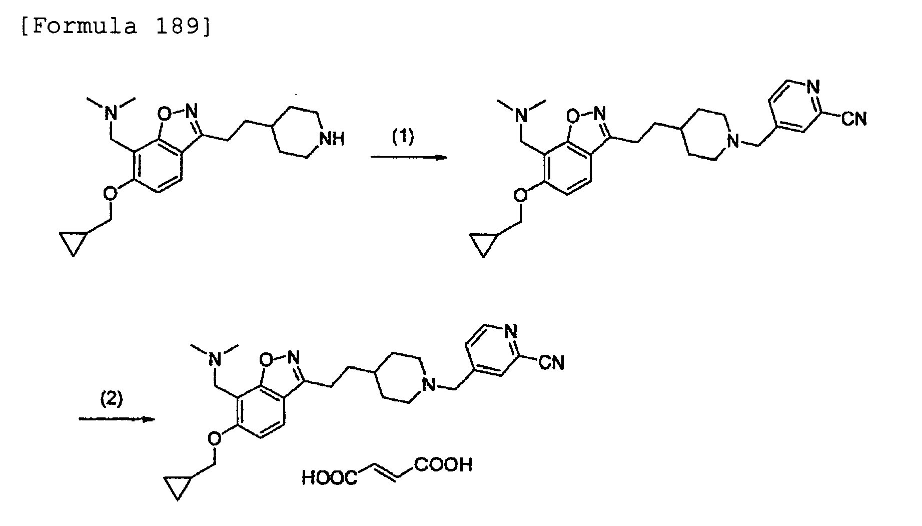

### (1) 4-{4-{2-[6-Cyclopropylmethoxy-7-(N,N-dimethylaminomethyl)benz[d]isoxazol-3-yl]ethyl}piperidinomethyl}pyridine-2-carbonitrile

4-Formylpyridine-2-carbonitrile was synthesized by the method described in Chem. Pharm. Bull., 1990, 38, 2446. The title compound was obtained by synthesis from N,N-dimethyl{6-cyclopropylmethoxy-3-[2-(piperidin-4-yl)ethyl]benz[d]isoxazol-7-yl}methylamine and 4-formylpyridine-2-carbonitrile according to Example 153-(5).
¹H-NMR (400 MHz, CDCl₃) δ (ppm): 0.36-0.39 (m, 2H), 0.62-0.67 (m, 2H), 1.30-1.42 (m, 4H), 1.76-1.82 (m, 4H), 2.00-2.05 (m, 2H), 2.34 (s, 6H), 2.77-2.81 (m, 2H), 2.94-2.97 (m, 2H), 3.52 (s, 2H), 3.82 (s, 2H), 3.94 (d, J=6.8 Hz, 2H), 6.91 (d, J=8.6 Hz, 1H), 7.46 (d, J=8.6 Hz, 1H), 7.47-7.48 (m, 1H), 7.74 (s, 1H), 8.62 (d, J=5.2 Hz, 1H).

### (2) 4-{4-{2-[6-Cyclopropylmethoxy-7-(N,N-dimethylaminomethyl)benz[d]isoxazol-3-yl]ethyl}piperidinomethyl}pyridine-2-carbonitrile fumarate

The title compound was obtained by synthesis from 6-{4-{2-[6-cyclopropylmethoxy-7-(N,N-dimethylaminomethyl)benz[d]isoxazol-3-yl]ethyl}piperidinomethyl}pyridine-2-carbonitrile according to Example 154-(4).
¹H-NMR (400 MHz, DMSO-d₆) δ (ppm) : 0.34-0.37 (m, 2H), 0.56-0.60 (m, 2H), 1.21-1.34 (m, 4H), 1.67-1.74 (m, 4H), 1.94-1.99 (m, 2H), 2.22 (s, 6H), 2.74-2.79 (m, 2H), 2.92-2.96 (m, 2H), 3.56 (s, 2H), 3.75 (s, 2H), 3. 98 (d, J=7.2 Hz, 2H), 6.60 (s, 2H), 7.12 (d, J=8.6 Hz, 1H), 7.66-7.67 (m, 1H), 7.73 (d, J=8.6 Hz, 1H), 7.93 (s, 1H), 8.68 (d, J=4.4 Hz, 1H).
ESI-MS m/z: 474 [M+H]⁺.

### Example 166

### 2-{4-{2-[6-Cyclopropylmethoxy-7-(N,N-dimethylaminomethyl)benz[d]isoxazol-3-yl]ethyl}piperidinomethyl}pyridine-4-carbonitrile fumarate

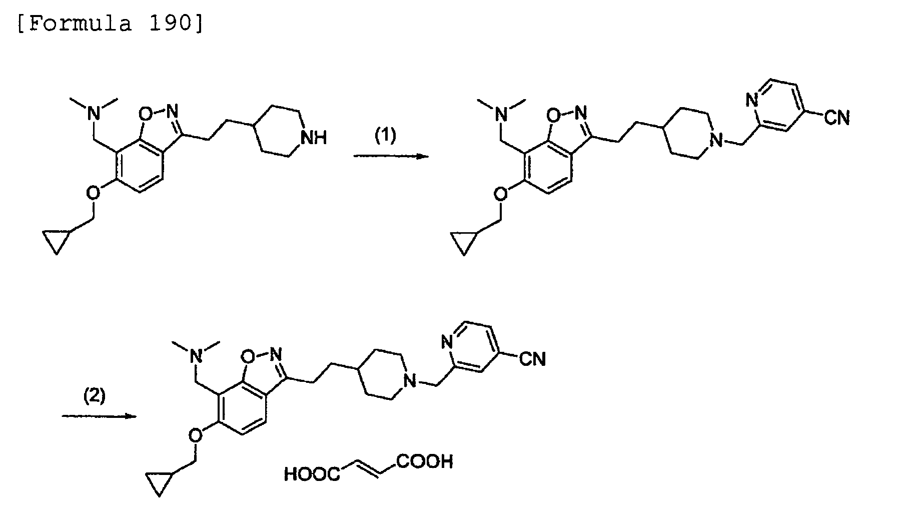

### (1) 2-{4-{2-[6-Cyclopropylmethoxy-7-(N,N-dimethylaminomethyl)benz[d]isoxazol-3-yl]ethyl}piperidinomethyl}pyridine-4-carbonitrile

2-Formylpyridine-4-carbonitrile was synthesized by the method described in Chem. Pharm. Bull., 1990, 38, 2446. The title compound was obtained by synthesis from N,N-dimethyl{6-cyclopropylmethoxy-3-[2-(piperidin-4-yl)ethyl]benz[d]isoxazol-7-yl}methylamine and 2-formylpyridine-4-carbonitrile according to Example 153-(5).
¹H-NMR (400 MHz, CDCl₃) δ (ppm): 0.34-0.41 (m, 2H), 0.63-0.68 (m, 2H), 1.28-1.43 (m, 4H), 1.46-1.84 (m, 4H), 2.06-2.15 (m, 2H), 2.22 (s, 6H), 2.83-2.88 (m, 2H), 2.93-2.97 (m, 2H), 3.68 (s, 2H), 3.83 (s, 2H), 3.94 (d, J=6.4 Hz, 2H), 6.91 (d, J=8.8 Hz, 1H), 7.38 (dd, J=1.6, 5.0 Hz, 1H), 7.46 (d, J=8.8 Hz, 1H), 7.71-7.72 (m, 1H), 8.71 (d, J=5.0 Hz, 1H).

### (2) 2-{4-{2-[6-Cyclopropylmethoxy-7-(N,N-dimethylaminomethyl)benz[d]isoxazol-3-yl]ethyl}piperidinomethyl}pyridine-4-carbonitrile fumarate

The title compound was obtained by synthesis from 2-{4-{2-[6-cyclopropylmethoxy-7-(N,N-dimethylaminomethyl)benz[d]isoxazol-3-yl]ethyl}piperidinomethyl}pyridine-4-carbonitrile according to Example 154-(4).
¹H-NMR (400 MHz, DMSO-d₆) δ (ppm): 0.35-0.39 (m, 2H), 0.62-0.67 (m, 2H), 1.26-1.44 (m, 4H), 1.75-1.83 (m, 4H), 2.08-2.13 (m, 2H), 2.23 (s, 6H), 2.79-2.84 (m, 2H), 2.92-2.96 (m, 2H), 3.65 (s, 2H), 3.76 (s, 2H), 3.97 (d, J=6.4 Hz, 2H), 6.60 (s, 2H), 7.12 (d, J=8.8 Hz, 1H), 7.72-7.74 (m, 2H), 7.80 (s, 1H), 8.75 (d, J=5.2 Hz, 1H).
ESI-MS m/z: 238 [M+2H]²⁺, 474 [M+H]⁺.

### Example 167

### N,N-Dimethyl{6-cyclopropylmethoxy-3-{2-[1-(2-thiophen-2-ylethyl)piperidin-4-yl]ethyl}benz[d]isoxazol-7-yl}methylamine fumarate

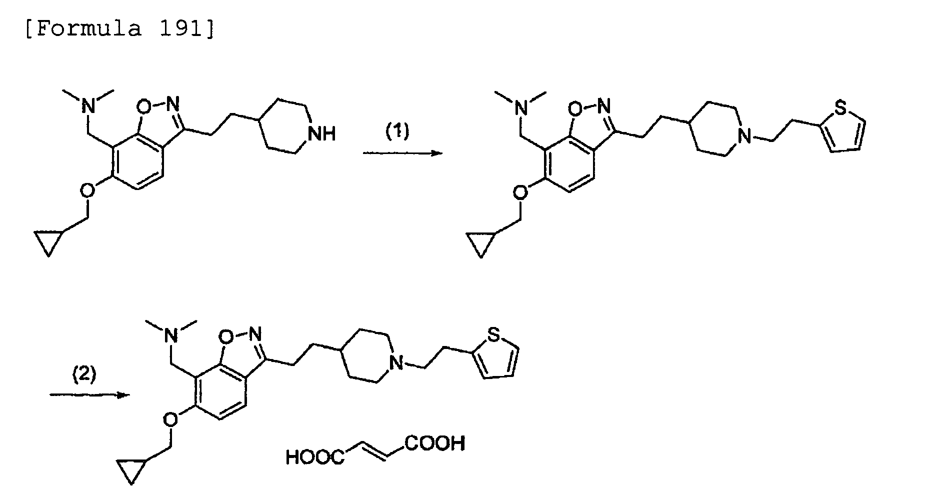

### (1) N,N-Dimethyl{6-cyclopropylmethoxy-3-{2-[1-(2-thiophen-2-ylethyl)piperidin-4-yl]ethyl}benz[d]isoxazol-7-yl}methylamine

The title compound was obtained by synthesis from N,N-dimethyl{6-cyclopropylmethoxy-3-[2-(piperidin-4-yl)ethyl]benz[d]isoxazol-7-yl}methylamine and thiophen-2-ylacetaldehyde [CAS Registry No. 15022-15-8] according to Example 153-(5).
¹H-NMR (400 MHz, CDCl₃) δ (ppm): 0.36-0.39 (m, 2H), 0.62-0.67 (m, 2H), 1.27-1.39 (m, 4H), 1.76-1.81 (m, 4H), 1.99-2.05 (m, 2H), 2.34 (s, 6H), 2.62-2.66 (m, 2H), 2.93-3.04 (m, 6H), 3.83 (s, 2H), 4.12 (d, J=6.8 Hz, 2H), 6.81-6.82 (m, 1H), 6.91 (d, J=8.8 Hz, 1H), 6.91-6.93 (m, 1H), 7.12 (dd, J=1.6, 5.2 Hz, 1H), 7.46 (d, J=8.8 Hz, 1H).

### (2) N,N-Dimethyl{6-cyclopropylmethoxy-3-{2-[1-(2-thiophen-2-ylethyl)piperidin-4-yl]ethyl}benz[d]isoxazol-7-yl}methylamine fumarate

The title compound was obtained by synthesis from N,N-dimethyl{6-cyclopropylmethoxy-3-{2-[1-(2-thiophen-2-ylethyl)piperidin-4-yl]ethyl}benz[d]isoxazol-7-yl}methylamine according to Example 154-(4).
¹H-NMR (400 MHz, DMSO-d₆)
δ (ppm): 0.34-0.37 (m, 2H), 0.56-0.61 (m, 2H), 1.22-1.36 (m, 4H), 1.67-1.76 (m, 4H), 2.00-2.07 (m, 2H), 2.21 (s, 6H), 2.55-2.62 (m, 2H), 2.93-2.99 (m, 6H), 3.74 (s, 2H), 3.98 (d, J=6.8 Hz, 2H), 6.59 (s, 2H), 6.86-6.95 (m, 2H), 7.11-7.13 (m, 1H), 7.30-7.32 (m, 1H), 7.72-7.74 (m, 1H).
0.35-0.39 (m, 2H), 0.62-0.67 (m, 2H), 1.26-1.44 (m, 4H), 1.75-1.83 (m, 4H), 2.08-2.13 (m, 2H), 2.23 (s, 6H), 2.79-2.84 (m, 2H), 2.92-2.96 (m, 2H), 3.65 (s, 3H), 3.76 (s, 3H), 3.97 (d, J=6.4 Hz, 2H), 6.60 (s, 2H), 7.12 (d, J=8.8 Hz, 1H), 7.72-7.74 (m, 2H), 7.80 (s, 1H), 8.75 (d, J=5.2 Hz, 1H).
ESI-MS m/z: 235 [M+2H]²⁺, 468 [M+H]⁺.

### Example 168

### 4-{4-{2-[6-Cyclopropylmethoxy-7-(N,N-dimethylaminomethyl)benz[d]isoxazol-3-yl]ethyl}piperidinomethyl}benzonitrile dihydrochloride

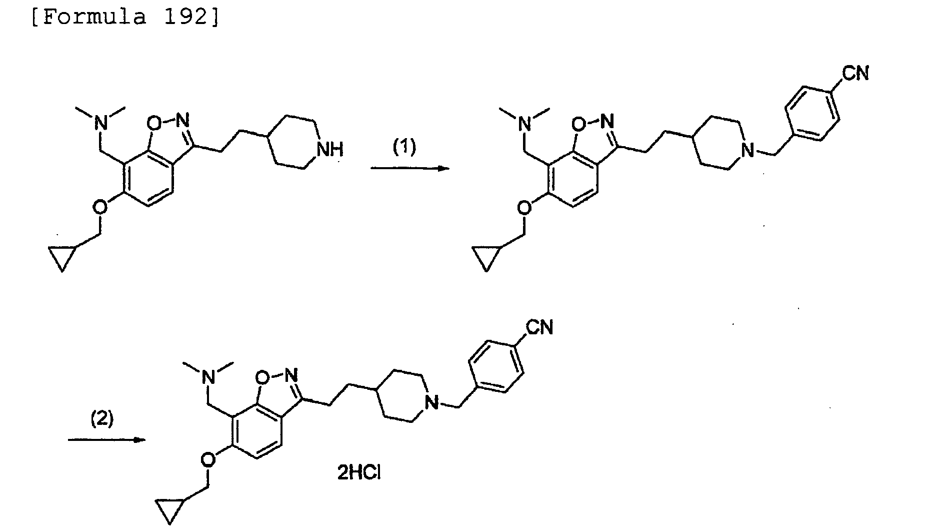

### (1) 4-{4-{2-[6-Cyclopropylmethoxy-7-(N,N-dimethylaminomethyl)benz[d]isoxazol-3-yl]ethyl}piperidinomethyl}benzonitrile

The title compound was obtained by synthesis from N,N-dimethyl{6-cyclopropylmethoxy-3-[2-(piperidin-4-yl)ethyl]benz[d]isoxazol-7-yl}methylamine and 4-formylbenzonitrile according to Example 153-(5).
¹H-NMR (400 MHz, CDCl₃) δ (ppm): 0.35-0.39 (m 2H), 0.62-0.67 (m, 2H), 1.26-1.40 (m, 4H), 1.73-1.80 (m, 4H), 1.94-2.00 (m, 2H), 2.34 (s, 6H), 2.80-2.84 (m, 2H), 2.92-2.96 (m, 2H), 3.51 (s, 2H), 3.82 (s, 2H), 3.94 (d, J=6.4 Hz, 2H), 6.90 (d, J=8.6 Hz, 1H), 7.44 (d, J=8.4 Hz, 2H), 7.44 (d, J=8.6 Hz, 1H), 7.60 (d, J=8.4 Hz, 2H).

### (2) 4-{4-{2-[6-Cyclopropylmethoxy-7-(N,N-dimethylaminomethyl)benz[d]isoxazol-3-yl]ethyl}piperidinomethyl}benzonitrile dihydrochloride

The title compound was obtained by synthesis from 2-{4-{2-[6-cyclopropylmethoxy-7-(N,N-dimethylaminomethyl)benz[d]isoxazol-3-yl]ethyl}piperidinomethyl}pyridine-4-carbonitrile according to Example 154-(4).
¹H-NMR (400 MHz, DMSO-d₆) δ (ppm): 0.38-0.43 (m, 2H), 0.59-0.64 (m, 2H), 1.28-1.96 (m, 8H), 2.82 (s, 6H), 2.88-3.01 (m, 6H), 4.08 (d, J=7.2 Hz, 2H), 4.37 (s, 2H), 4.50 (s, 2H), 7.25 (d, J=9.2 Hz, 1H), 7.77-7.81 (m, 2H), 7.65-7.98 (m, 3H), 9.84 (br s, 1H), 10.27 (br s, 1H).
ESI-MS m/z: 237 [M+2H]²⁺, 473 [M+H]⁺.

### Example 169

### 3-{4-{2-[6-Cyclopropylmethoxy-7-(N,N-dimethylaminomethyl)benz[d]isoxazol-3-yl]ethyl}piperidinomethyl}benzonitrile dihydrochloride

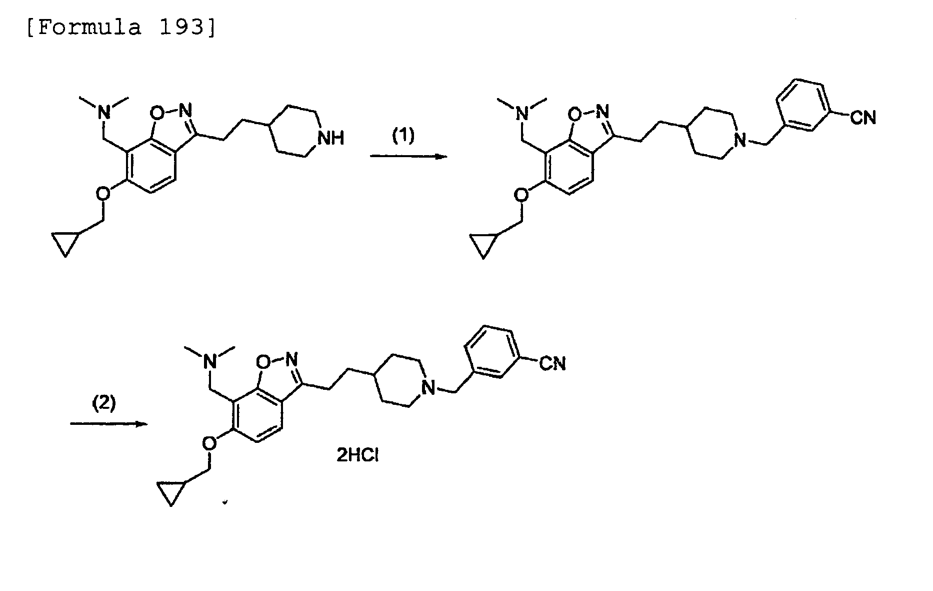

### (1) 3-{4-{2-[6-Cyclopropylmethoxy-7-(N,N-dimethylaminomethyl)benz[d]isoxazol-3-yl]ethyl}piperidinomethyl)benzonitrile

The title compound was obtained by synthesis from N,N-dimethyl{6-cyclopropylmethoxy-3-[2-(piperidin-4-yl)ethyl]benz[d]isoxazol-7-yl}methylamine and 3-formylbenzonitrile according to Example 153-(5).
¹H-NMR (400 MHz, CDCl₃) δ (ppm): 0.35-0.39 (m, 2H), 0.62-0.67 (m, 2H), 1.26-1.42 (m, 4H), 1.74-1.80 (m, 4H), 1.94-2.00 (m, 2H), 2.34 (s, 6H), 2.80-2.84 (m, 2H), 2.92-2.96 (m, 2H), 3.49 (s, 2H), 3..82 (s, 2H), 3.94 (d, J=6.8 Hz, 2H), 6.90 (d, J=8.8 Hz, 1H), 7.39-7.42 (m, 1H), 7.45 (d, J=8.8 Hz, 1H), 7.52-7.56 (m, 2H), 7.64-7.65 (m, 1H).

### (2) 3-{4-{2-[6-Cyclopropylmethoxy-7-(N,N-dimethylaminomethyl)benz[d]isoxazol-3-yl]ethyl}piperidinomethyl}benzonitrile dihydrochloride

The title compound was obtained by synthesis from 3-{4-{2-[6-cyclopropylmethoxy-7-(N,N-dimethylaminomethyl)benz[d]isoxazol-3-yl]ethyl}piperidinomethyl}benzonitrile according to Example 154-(4).
¹H-NMR (400 MHz, DMSO-d₆) δ (ppm): 0.38-0.42 (m, 2H), 0.59-0.63 (m, 2H), 1.30-1.96 (m, 8H), 2.80 (s, 6H), 2.87-3.01 (m, 6H), 4.08 (d, J=7.2 Hz, 2H), 4.34 (s, 2H), 4.49 (s, 2H), 7.25 (d, J=8.8 Hz, 1H), 7.67-7.71 (m, 1H), 7.91-8.00 (m, 3H), 8.08 (s, 1H), 9.95 (br s, 1H), 10.42 (br s, 1H).
ESI-MS m/z: 237 [M+2H]²⁺, 473 [M+H]⁺.

### Example 170

### N,N-Dimethyl{6-cyclopropylmethoxy-3-{2-[1-(1-methyl-1H-imidazol-2-ylmethyl)piperidin-4-yl]ethyl}benz[d]isoxazol-7-yl}methylamine trihydrochloride

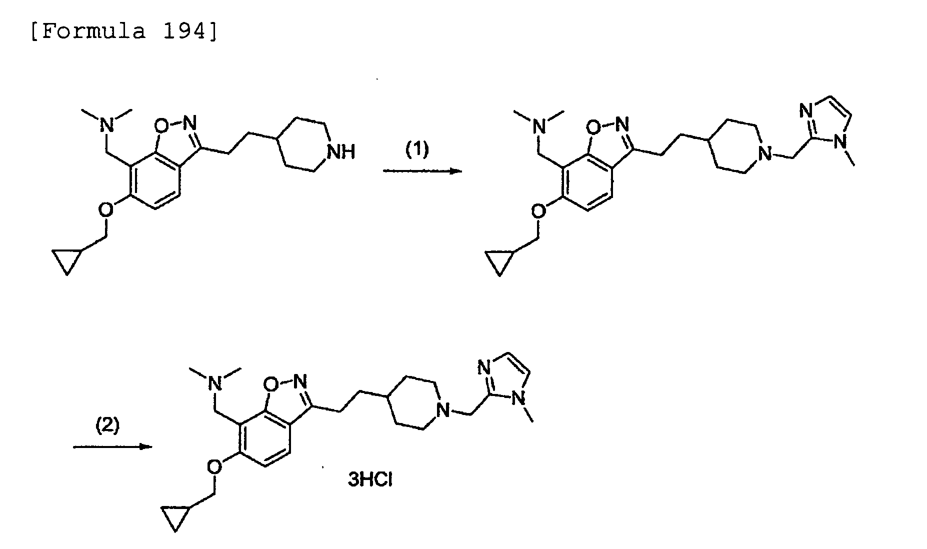

### (1) N,N-Dimethyl{6-cyclopropylmethoxy-3-{2-[1-(1-methyl-1H-imidazol-2-ylmethyl)piperidin-4-yl]ethyl}benz[d]isoxazol-7-yl}methylamine

1-Methyl-1H-imidazole-2-carbaldehyde was synthesized by the method described in J. Med. Chem., 1991, 34, 1363. The title compound was obtained by synthesis from N,N-dimethyl{6-cyclopropylmethoxy-3-[2-(piperidin-4-yl)ethyl]benz[d]isoxazol-7-yl}methylamine and 1-methyl-1H-imidazole-2-carbaldehyde according to Example 153-(5).
¹H-NMR (400 MHz, CDCl₃) δ (ppm): 0.35-0.39 (m, 2H), 0.62-0.66 (m, 2H), 1.19-1.42 (m, 4H), 1.72-1.78 (m, 4H), 2.00-2.06 (m, 2H), 2.33 (s, 6H), 2.78-2.82 (m, 2H), 2.91-2.95 (m, 2H), 3.56 (s, 2H), 3.70 (s, 3H), 3.82 (s, 2H), 3.94 (d, J=6.4 Hz, 2H), 6.83-6.84 (m, 1H), 6.89 (d, J=8.6 Hz, 1H), 6.91-6.92 (m, 1H), 7.44 (d, J=8.6 Hz, 1H).

### (2) N,N-Dimethyl{6-cyclopropylmethoxy-3-{2-[1-(1-methyl-1H-imidazol-2-ylmethyl)piperidin-4-yl]ethyl}benz[d]isoxazol-7-yl}methylamine trihydrochloride

The title compound was obtained by synthesis from N,N-dimethyl{6-cyclopropylmethoxy-3-{2-[1-(1-methyl-1H-imidazol-2-ylmethyl)piperidin-4-yl]ethyl}benz[d]isoxazol-7-yl}methylamine according to Example 154-(4).
¹H-NMR (400 MHz, DMSO-d₆) δ (ppm): 0.39-0.43 (m, 2H), 0.58-0.63 (m, 2H), 1.30-1.97 (m, 8H), 2.77-2.79 (m, 6H), 2.99-3.03 (m, 2H), 3.13-3.21 (m, 2H), 3.51-3.58 (m, 2H), 4.02-4.09 (m, 5H), 4.46-4.47 (m, 2H), 4.66-4.68 (m, 2H), 7.25 (d, J=8.6 Hz, 1H), 7.79 (d, J=1.8 Hz, 1H), 7.83 (d, J=1.8 Hz, 1H), 7.97 (d, J=8.6 Hz, 1H), 10.65 (br s, 1H), 12.01 (br s, 1H).
ESI-MS m/z: 227 [M+2H]²⁺, 452 [M+H]⁺, 474 [M+Na]⁺.

### Example 171

### N,N-Dimethyl{6-cyclopropylmethoxy-3-{2-{1-[2-(1H-pyrazol-1-yl)ethyl]piperidin-4-yl}ethyl}benz[d]isoxazol-7-yl}methylamine trihydrochloride

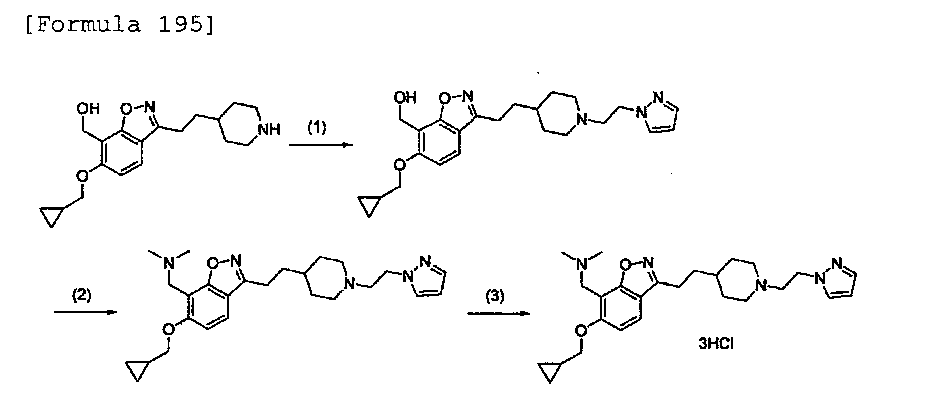

### (1) {6-Cyclopropylmethoxy-3-{2-{1-[2-(1H-pyrazol-1-yl)ethyl]piperidin-4-yl}ethyl}benz[d]isoxazol-7-yl}methanol

1-(2-Bromoethyl)-1H-pyrazole was synthesized by the method described in Bioorg. Med. Chem., 1999, 7, 517. A mixture of [6-cyclopropylmethoxy-3-(2-piperidin-4-yl)ethyl]benz[d]isoxazol-7-yl]methanol (103 mg), 1-(2-bromoethyl)-1H-pyrazole (66 mg), N,N-diisopropylethylamine (136 µL) and acetonitrile (5 mL) was stirred in a nitrogen atmosphere at room temperature for 1.5 hours and subsequently at 40°C for 25 hours. The solvent was evaporated under reduced pressure. A 5 M sodium hydroxide solution and a saturated sodium chloride solution were added to the residue, followed by extraction with chloroform twice. The organic layers were dried over magnesium sulfate-sodium carbonate. The drying agent was removed by filtration and then the organic layers were concentrated under reduced pressure. The residue was purified by NH silica gel column chromatography (n-hexane-ethyl acetate) to obtain the title compound (69 mg).
¹H-NMR (400 MHz, CDCl₃) δ (ppm): 0.36-0.40 (m, 2H), 0.66-0.71 (m, 2H), 1.24-1.36 (m, 4H), 1.74-1.79 (m, 4H), 2.01-2.07 (m, 2H), 2.63 (br s, 1H), 2.79 (t, J=7.0 Hz, 2H), 2.85-2.89 (m, 2H), 2.93-2.97 (m, 2H), 3.98 (d, J=6.8 Hz, 2H), 4.26 (t, J=7.0 Hz, 2H), 5.04 (s, 2H), 6.23-6.24 (m, 1H), 6.91 (d, J=8.8 Hz, 1H), 7.45-7.50 (m, 3H).

### (2) N,N-Dimethyl{6-cyclopropylmethoxy-3-{2-{1-[2-(1H-pyrazol-1-yl)ethyl]piperidin-4-yl}ethyl}benz[d]isoxazol-7-yl}methylamine

(6-Cyclopropylmethoxy-3-{2-{1-[2-(1H-pyrazol-1-yl)ethyl]piperidin-4-yl}ethyl}benz[d]isoxazol-7-yl}methanol (69 mg) was dissolved in tetrahydrofuran (3 mL). Triethylamine (68 µL) was added to the solution, and the mixture was stirred under ice-cooling in a nitrogen atmosphere. Methanesulfonyl chloride (19 µL) was added to the mixture, followed by stirring for 45 minutes. Triethylamine (68 µL) and methanesulfonyl chloride (19 µL) were added to the reaction mixture, followed by stirring for 65 minutes. Dimethylamine (2 M solution in tetrahydrofuran) (1.22 mL) was added to the reaction mixture, followed by stirring for two hours and 50 minutes. A saturated sodium chloride solution was added to the reaction mixture, followed by extraction with ethyl acetate twice. The organic layers were washed with a saturated sodium chloride solution and dried over magnesium sulfate. The drying agent was removed by filtration and then the organic layers were concentrated under reduced pressure. The residue was purified by NH silica gel column chromatography (n-hexane-ethyl acetate) to obtain the title compound (77 mg).
¹H-NMR (400 MHz, CDCl₃) δ (ppm): 0.35-0.39 (m, 2H), 0.62-0.67 (m, 2H), 1.25-1.42 (m, 4H), 1.74-1.79 (m, 4H), 2.02-2.07 (m, 2H), 2.34 (s, 6H), 2.79 (t, J=6.8 Hz, 2H), 2.85-2.89 (m, 2H), 2.92-2.96 (m, 2H), 3.82 (s, 2H), 3.94 (d, J=6.4 Hz, 2H), 4.26 (t, J=6.8 Hz, 2H), 6.23-6.24 (m, 1H), 6.90 (d, J=8.8 Hz, 1H), 7.43-7.50 (m, 3H).

### (3) N,N-Dimethyl{6-cyclopropylmethoxy-3-{2-{1-[2-(1H-pyrazol-1-yl)ethyl]piperidin-4-yl}ethyl}benz[d]isoxazol-7-yl}methylamine trihydrochloride

The title compound was obtained by synthesis from N,N-dimethyl{6-cyclopropylmethoxy-3-{2-{1-[2-(1H-pyrazol-1-yl)ethyl]piperidin-4-yl}ethyl}benz[d]isoxazol-7-yl}methylamine according to Example 154-(4).
¹H-NMR (400 MHz, DMSO-d₆) δ (ppm): 0.38-0.43 (m, 2H), 0.60-0.65 (m, 2H), 1.28-1.96 (m, 8H), 2.78-3.16 (m, 6H), 3.37-3.45 (m, 8H), 4.06-4.08 (m, 2H), 4.48 (s, 2H), 4.60-4.63 (m, 2H), 6.30 (s, 1H), 7.24 (d, J=8.8 Hz, 1H), 7.52 (s, 1H), 7.83 (s, 1H), 7.96 (d, J=8.8 Hz, 1H), 10.14 (br s, 1H), 10.45 (br s, 1H).
ESI-MS m/z: 227 [M+2H]²⁺, 452 [M+H]⁺, 474 [M+Na]⁺.

### Example 172

### 5-{4-{2-[6-Cyclopropylmethoxy-7-(N-methylaminomethyl)benz[d]isoxazol-3-yl]ethyl}piperidinomethyl}thiophene-2-carbonitrile dihydrochloride

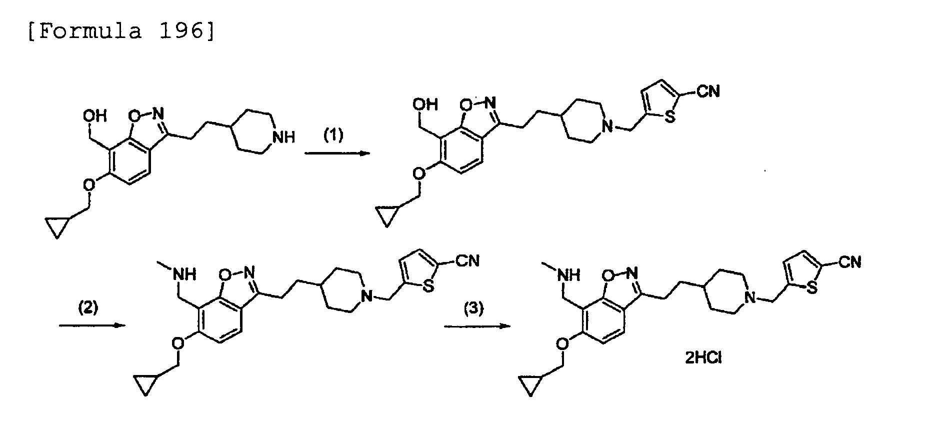

### (1) 5-{4-[2-(6-Cyclopropylmethoxy-7-hydroxymethylbenz[d]isoxazol-3-yl)ethyl]piperidinomethyl}thiophene-2-carbonitrile

The title compound was obtained by synthesis from [6-cyclopropylmethoxy-3-(2-piperidin-4-yl)ethyl]benz[d]isoxazol-7-yl]methanol according to Example 155-(2).
¹H-NMR (400 MHz, CDCl₃) δ (ppm): 0.37-0.40 (m, 2H), 0.66-0.71 (m, 2H), 1.26-1.36 (m, 4H), 1.73-1.81 (m, 4H), 2.00-2.05 (m, 2H), 2.63 (t, J=6.8 Hz 1H), 2.89-2.98 (m, 4H), 3.68 (s, 2H), 3.98 (d, J=6.8 Hz, 2H), 5.04 (d, J=6.8 Hz, 2H), 6.88 (d, J=3.6 Hz, 1H), 6.91 (d, J=8.8 Hz, 1H), 7.46-7.49 (m, 2H).

### (2) 5-{4-{2-[6-Cyclopropylmethoxy-7-(N-methylaminomethyl)benz[d]isoxazol-3-yl]ethyl}piperidinomethyl}thiophene-2-carbonitrile

5-{4-[2-(6-Cyclopropylmethoxy-7-hydroxymethylbenz[d]isoxazol-3-yl)ethyl]piperidinomethyl}thiophene-2-carbonitrile (144 mg) was dissolved in tetrahydrofuran (5 mL). Triethylamine (133 µL) was added to the mixture, followed by stirring under ice-cooling in a nitrogen atmosphere. Methanesulfonyl chloride (37 µL) was added to the mixture, followed by stirring for 40 minutes. Triethylamine (133 µL) and methanesulfonyl chloride (37 µL) were added to the reaction mixture, followed by stirring for 70 minutes. The reaction mixture was cooled in a dry ice-acetone bath. Methylamine (2 M solution in tetrahydrofuran) (3.19 mL) was added to the reaction mixture. The reaction mixture was stirred under ice-cooling for 10 minutes and then stirred at room temperature for 12 hours and 20 minutes. A saturated sodium chloride solution was added to the reaction mixture, followed by extraction with ethyl acetate. The organic layer was washed with a saturated sodium chloride solution and dried over magnesium sulfate. The drying agent was removed by filtration and then the organic layer was concentrated under reduced pressure. The residue was purified by NH silica gel column chromatography (n-hexane-ethyl acetate) twice. The resulting crude product was purified by preparative TLC (NH silica gel) (n-hexane-ethyl acetate) to obtain the title compound (69 mg).
¹H-NMR (400 MHz, CDCl₃) δ (ppm): 0.35-0.39 (m, 2H), 0.63-0.68 (m, 2H), 1.26-1.35 (m, 4H), 1.76-1.81 (m, 4H), 2.00-2.06 (m, 2H), 2.43 (s, 3H), 2.89-2.97 (m, 4H), 3.69 (s, 2H), 3.94 (d, J=6.4 Hz, 2H), 4.10 (s, 2H), 6.88-6.91 (m, 2H), 7.43 (d, J=8.8 Hz, 1H), 7.47 (d, J=3.6 Hz, 1H).

### (3) 5-{4-{2-[6-Cyclopropylmethoxy-7-(N-methylaminomethyl)benz[d]isoxazol-3-yl]ethyl}piperidinomethyl}thiophene-2-carbonitrile dihydrochloride

The title compound was obtained by synthesis from 5-{4-{2-[6-cyclopropylmethoxy-7-(N-methylaminomethyl)benz[d]isoxazol-3-yl]ethyl}piperidinomethyl}thiophene-2-carbonitrile according to Example 154-(4).
¹H-NMR (400 MHz, DMSO-d₆) δ (ppm): 0.39-0.43 (m, 2H), 0.59-0.63 (m, 2H), 1.28-1.98 (m, 8H), 2.61 (s, 3H), 2.80-3.10 (m, 6H), 4.06 (d, J=7.2 Hz, 2H), 4.33 (s, 2H), 4.60 (s, 2H), 7.22 (d, J=8.6 Hz, 1H), 7.53 (s, 1H), 7.91 (d, J=8.6 Hz, 1H), 7.99 (s, 1H), 8.87-8.98 (m, 2H), 10.84 (br s, 1H).
ESI-MS m/z: 465 [M+H]⁺.

### Example 173

### 5-{4-[2-(7-Aminomethyl-6-cyclopropylmethoxybenz[d]isoxazol-3-yl)ethyl]piperidinomethyl}thiophene-2-carbonitrile dihydrochloride

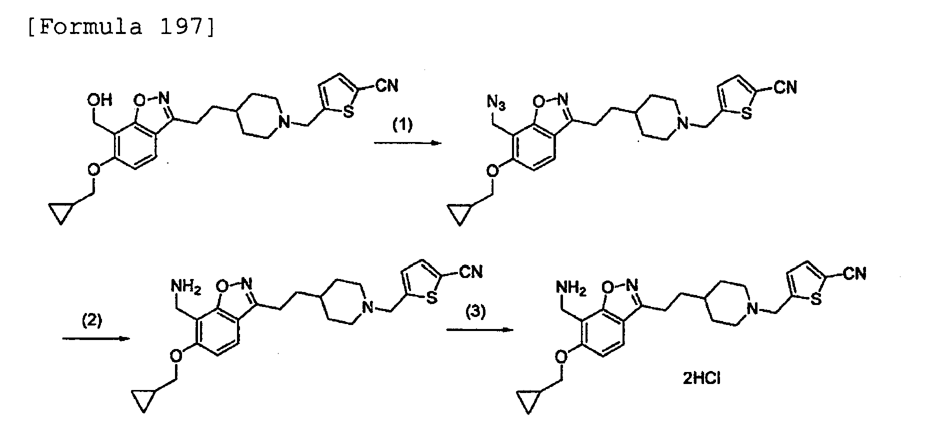

### (1) 5-{4-[2-(7-Azidomethyl-6-cyclopropylmethoxybenz[d]isoxazol-3-yl)ethyl]piperidinomethyl}thiophene-2-carbonitrile

5-{4-[2-(6-Cyclopropylmethoxy-7-hydroxymethylbenz[d]isoxazol-3-yl)ethyl]piperidinomethyl}thiophene-2-carbonitrile (compound obtained in Example 172-(1)) (183 mg), triphenylphosphine (159 mg) and sodium azide (132 mg) were dissolved in N,N-dimethylformamide (5 mL), and the solution was stirred under ice-cooling in a nitrogen atmosphere. Carbon tetrabromide (201 mg) was added to the reaction mixture, followed by stirring at room temperature for 18 hours and 40 minutes. The reaction mixture was diluted with ethyl acetate, sequentially washed with water (five times) and a saturated sodium chloride solution and dried over magnesium sulfate. The drying agent was removed by filtration and then the organic layer was concentrated under reduced pressure. The residue was purified by NH silica gel column chromatography (n-hexane-ethyl acetate) to obtain the title compound (164 mg).
¹H-NMR (400 MHz, CDCl₃) δ (ppm): 0.38-0.41 (m, 2H), 0.66-0.70 (m, 2H), 1.28-1.38 (m, 4H), 1.64-1.82 (m, 4H), 2.01-2.06 (m, 2H), 2.89-2.98 (m, 4H), 3.69 (s, 2H), 3.97 (d, J=7.2 Hz, 2H), 4.68 (s, 2H), 6.88 (d, J=4.0 Hz, 1H), 6.93 (d, J=8.8 Hz, 1H), 7.47 (d, J=4.0 Hz, 1H), 7.52 (d, J=8.8 Hz, 1H).

### (2) 5-{4-[2-(7-Aminomethyl-6-cyclopropylmethoxybenz[d]isoxazol-3-yl)ethyl]piperidinomethyl}thiophene-2-carbonitrile

5-{4-[2-(7-Azidomethyl-6-cyclopropylmethoxybenz[d]isoxazol-3-yl)ethyl]piperidinomethyl}thiophene-2-carbonitrile (164 mg) was dissolved in tetrahydrofuran (4 mL). The solution was stirred in a nitrogen atmosphere at room temperature. Triphenylphosphine (99 mg) was added to the reaction mixture, followed by stirring at room temperature for 15 hours and 30 minutes. Triphenylphosphine (18 mg) was added to the reaction mixture, and then the mixture was stirred for five hours and 30 minutes. 28% aqueous ammonia (1 mL) was added to the reaction mixture, and then the mixture was stirred for 15 minutes. A saturated sodium chloride solution was added to the reaction mixture, followed by extraction with chloroform three times. The organic layers were dried over magnesium sulfate. The drying agent was removed by filtration and then the organic layers were concentrated under reduced pressure. The residue was purified by NH silica gel column chromatography (chloroform-methanol) twice to obtain the title compound (93 mg).
¹H-NMR (400 MHz, CDCl₃) δ (ppm): 0.36-0.39 (m, 2H), 0.64-0.69 (m, 2H), 1.24-1.36 (m, 4H), 1.75-1.81 (m, 4H), 2.01-2.06 (m, 2H), 2.89-2.97 (m, 4H), 3.68 (s, 2H), 3.95 (d, J=7.2 Hz, 2H), 4.15 (s, 2H), 6.87-6.91 (m, 2H), 7.42 (d, J=8.8 Hz, 1H), 7.47 (d, J=3.6 Hz, 1H).

### (3) 5-{4-[2-(7-Aminomethyl-6-cyclopropylmethoxybenz[d]isoxazol-3-yl)ethyl]piperidinomethyl}thiophene-2-carbonitrile dihydrochloride

The title compound was obtained by synthesis from 5-{4-[2-(7-aminomethyl-6-cyclopropylmethoxybenz[d]isoxazol-3-yl)ethyl]piperidinomethyl}thiophene-2-carbonitrile according to Example 154-(4).
¹H-NMR (400 MHz, DMSO-d₆) δ (ppm): 0.40-0.46 (m, 2H), 0.60-0.65 (m, 2H), 1.25-2.00 (m, 8H), 2.82-3.06 (m, 6H), 4.06 (d, J=6.8 Hz, 2H), 4.23 (s, 2H), 4.62 (s, 2H), 7.22 (d, J=8.6 Hz, 1H), 7.48-7.50 (m, 1H), 7.89 (d, J=8.6 Hz, 1H), 8.00-8.01 (m, 1H), 8.17-8.28 (m, 3H), 10.21 (br s, 1H).
m/z: 451 [M+H]⁺.

### Example 174

### N,N-Dimethyl{6-cyclopropylmethoxy-3-{2-[1-(3-pyrazol-1-ylpropyl)piperidin-4-yl]ethyl}benz[d]isoxazol-7-yl}methylamine trihydrochloride

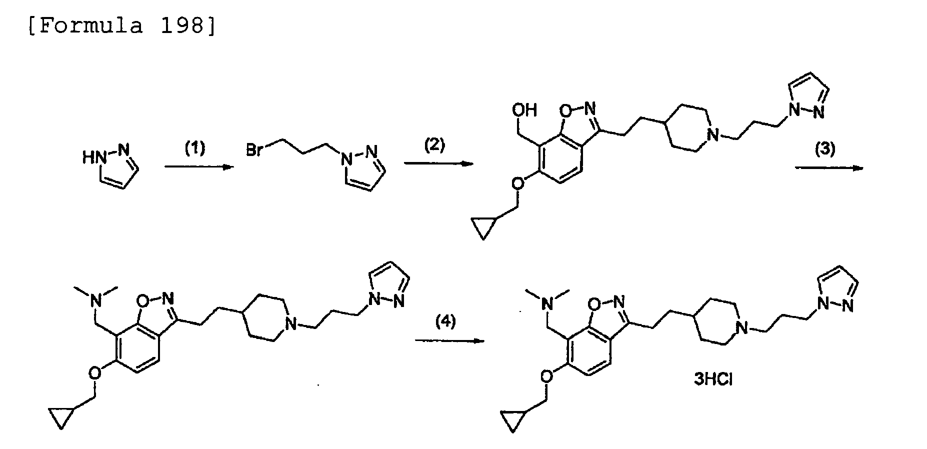

### (1) 1-(3-Bromopropyl)-1H-pyrazole

1H-Pyrazole (1.00 g), sodium hydroxide (1.86 g) and tetrabutylammonium bromide (118 mg) were dissolved in water (5 mL). 1,3-Dibromopropane (10.4 mL) was added to the solution, and the mixture was stirred at room temperature for 15 hours and 50 minutes. Chloroform was added and the organic layer was separated. The aqueous layer was extracted with chloroform. The combined organic layers were washed with a saturated sodium chloride solution and dried over magnesium sulfate. The drying agent was removed by filtration and then the organic layers were concentrated under reduced pressure. The residue was purified by silica gel column chromatography (n-hexane-ethyl acetate) to obtain the title compound (1.67 g). ¹H-NMR (400 MHz, CDCl₃) δ (ppm): 2.40 (tt, J=6.3, 6.3 Hz, 2H), 3.31 (t, J=6.3 Hz, 2H), 4.32 (t, J=6.3 Hz, 2H), 6.24-6.26 (m, 1H), 7.45 (d, J=2.4 Hz, 1H), 7.53 (d, J=1.6 Hz, 1H).

### (2) {6-Cyclopropylmethoxy-3-{2-{1-[3-(1H-pyrazol-1-yl)propyl]piperidin-4-yl}ethyl}benz[d]isoxazol-7-yl}methanol

A mixture of [6-cyclopropylmethoxy-3-(2-piperidin-4-yl)ethyl]benz[d]isoxazol-7-yl]methanol (104 mg), 1-(3-bromopropyl)-1H-pyrazole (75 mg), N,N-diisopropylethylamine (137 µL) and acetonitrile (5 mL) was stirred in a nitrogen atmosphere at 40°C for 19 hours and 10 minutes. 1-(3-Bromopropyl)-1H-pyrazole (80 mg) was dissolved in acetonitrile (0.5 mL), and the solution was added to the reaction mixture. The reaction mixture was stirred at 40°C for five hours and 30 minutes. The solvent was evaporated under reduced pressure. A 5 M sodium hydroxide solution and a saturated sodium chloride solution were added to the residue, followed by extraction with chloroform twice. The organic layers were dried over magnesium sulfate-sodium carbonate. The drying agent was removed by filtration and then the organic layers were concentrated under reduced pressure. The residue was purified by NH silica gel column chromatography (n-hexane-ethyl acetate-methanol) to obtain the title compound (123 mg).
¹H-NMR (400 MHz, CDCl₃) δ (ppm): 0.37-0.40 (m, 2H), 0.66-0.71 (m, 2H), 1.24-1.38 (m, 4H), 1.73-1.79 (m, 4H), 1.85-1.91 (m, 2H), 2.03 (t, J=7.0 Hz, 2H), 2.27 (t, J=7.0 Hz, 2H), 2.84-2.88 (m, 2H), 2.93-2.97 (m, 2H), 3.98 (d, J=7.2 Hz, 2H), 4.18 (t. J=7.0 Hz, 2H), 5.04 (s, 2H), 6.22-6.23 (m, 1H), 6.91 (d, J=8.8 Hz, 1H), 7.38-7.39 (m, 1H), 7.47 (d, J=8.8 Hz, 1H), 7.49-7.50 (m, 1H).

### (3) N,N-Dimethyl{6-cyclopropylmethoxy-3-{2-{1-[3-(1H-pyrazol-1-yl)propyl]piperidin-4-yl}ethyl}benz[d]isoxazol-7-yl}methylamine

The title compound was obtained by synthesis from {6-cyclopropylmethoxy-3-{2-{1-[3-(1H-pyrazol-1-yl)propyl]piperidin-4-yl}ethyl}benz[d]isoxazol-7-yl}methanol according to Example 171-(2).
¹H-NMR (400 MHz, CDCl₃) δ (ppm): 0.35-0.39 (m, 2H), 0.62-0.67 (m, 2H), 1.25-1.37 (m, 4H), 1.74-1.81(m, 4H), 1.86-1.91 (m, 2H), 2.03 (tt, J=7.0, 7.0 Hz, 2H), 2.27 (t, J=7.0 Hz, 2H), 2.34 (s, 6H), 2.84-2.88 (m, 2H), 2.92-2.96 (m, 2H), 3.82 (s, 2H), 3.94 (d, J=6.8H, 2H), 4.18 (t, J=7.0 Hz, 2H), 6.22-6.23 (m, 1H), 6.90 (d, J=8.8 Hz, 1H), 7.38-7.39 (m, 1H), 7.45 (d, J=8.8 Hz, 1H), 7.49-7.50 (m, 1H).

### (4) N,N-Dimethyl{6-cyclopropylmethoxy-3-{2-{1-[3-(1H-pyrazol-1-yl)propyl]piperidin-4-yl}ethyl}benz[d]isoxazol-7-yl}methylamine trihydrochloride

The title compound was obtained by synthesis from N,N-dimethyl{6-cyclopropylmethoxy-3-{2-{1-(3-(1H-pyrazol-1-yl)propyl]piperidin-4-yl}ethyl}benz[d]isoxazol-7-yl}methylamine according to Example 154-(4).
¹H-NMR (400 MHz, DMSO-d₆) δ (ppm): 0.39-0.42 (m, 2H), 0.58-0.63 (m, 2H), 1.25-1.38 (m, 1H), 1.52-1.94 (m, 7H), 2.18-2.2.28 (m, 2H), 2.80 (s, 6H), 2.80-3.04 (m, 6H), 3.41-3.47 (m, 2H), 4.08 (d, J=7.2 Hz, 2H), 4.22 (t, J=6.6 Hz, 2H), 4.47-4.48 (m, 2H), 6.25-6.26 (m, 1H), 7.25 (d, J=8.8 Hz, 1H), 7.47-7.47 (m, 1H), 7.77-7.78 (m, 1H), 7.97 (d, J=8.8 Hz, 1H), 10.32 (br s, 1H), 10.43 (br s, 1H).
m/z: 234 [M+2H]²⁺, 466 [M+H]⁺.

### Example 175

### N,N-Dimethyl{3-{2-[1-(5-chlorothiophen-2-ylmethyl)piperidin-4-yl]ethyl}-6-cyclopropylmethoxybenz[d]isoxazol-7-yl}methylamine difumarate

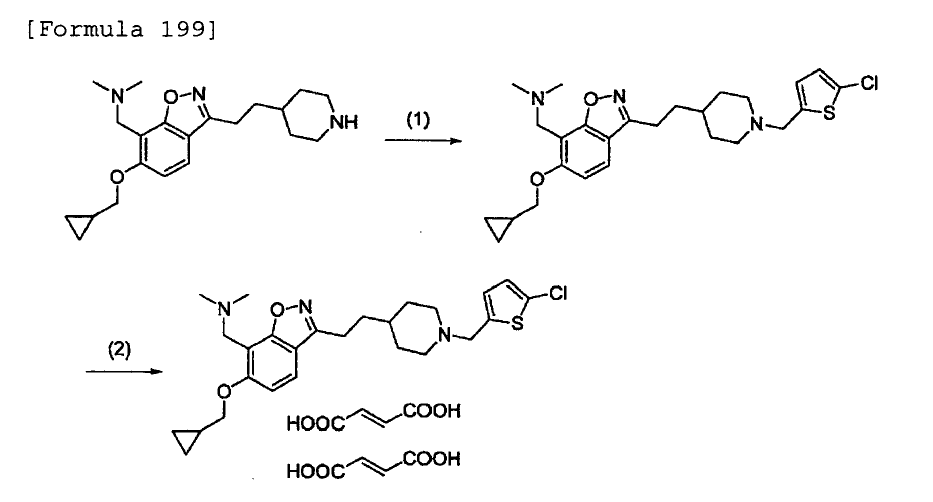

### (1) N,N-Dimethyl{3-{2-[1-(5-chlorothiophen-2-ylmethyl)piperidin-4-yl]ethyl}-6-cyclopropylmethoxybenz[d]isoxazol-7-yl}methylamine

The title compound was obtained by synthesis from N,N-dimethyl{6-cyclopropylmethoxy-3-[2-(piperidin-4-yl)ethyl]benz[d]isoxazol-7-yl}methylamine and 5-chlorothiophene-2-carbaldehyde according to Example 153-(5).
¹H-NMR (400 MHz, CDCl₃) δ (ppm): 0.35-0.39 (m, 2H), 0.62-0.67 (m, 2H), 1.25-1.37 (m, 4H), 1.73-1.80 (m, 4H), 1.94-2.00 (m, 2H), 2.33 (s, 6H), 2.90-2.96 (m, 4H), 3.59 (d, J=1.0 Hz, 2H), 3.82 (s, 2H), 3.94 (d, J=6.8 Hz, 2H), 6.64 (td, J=1.0, 3.6 Hz, 1H), 6.72 (d, J=3.6 Hz, 1H), 6.90 (d, J=8.8 Hz, 1H), 7.44 (d, J=8.8 Hz, 1H).

### (2) N,N-Dimethyl{3-{2-[1-(5-chlorothiophen-2-ylmethyl)piperidin-4-yl]ethyl}-6-cyclopropylmethoxybenz[d]isoxazol-7-yl}methylamine difumarate

The title compound was obtained by synthesis from N,N-dimethyl{3-{2-[1-(5-chlorothiophen-2-ylmethyl)piperidin-4-yl]ethyl}-6-cyclopropylmethoxybenz[d]isoxazol-7-yl}methylamine according to Example 154-(4).
¹H-NMR (400 MHz, DMSO-d₆) δ (ppm): 0.34-0.38 (m, 2H), 0.56-0.61 (m, 2H), 1.16-1.35 (m, 4H), 1.66-1.74 (m, 4H), 1.91-1.97 (m, 2H), 2.30 (s, 6H), 2.84-2.88 (m, 2H), 2.92-2.96 (m, 2H), 3.60 (s, 2H), 3.86 (s, 2H), 3.99 (d, J=6.8 Hz, 2H), 6.61 (s, 4H), 6.82 (d, J=3.6 Hz, 1H), 6.94 (d, J=3.6 Hz, 1H), 7.13 (d, J=8.8 Hz, 1H), 7.76 (d, J=8.8 Hz, 1H).
ESI-MS m/z: 245 [M+2H]²⁺, 358 [M-C5H4ClS⁺2H]⁺, 488, 489 [M+H]⁺.

### Example 176

### N-Methyl{6-cyclopropylmethoxy-3-{2-{1-[2-(1H-pyrazol-1-yl)ethyl]piperidin-4-yl}ethyl}benz[d]isoxazol-7-yl}methylamine trihydrochloride

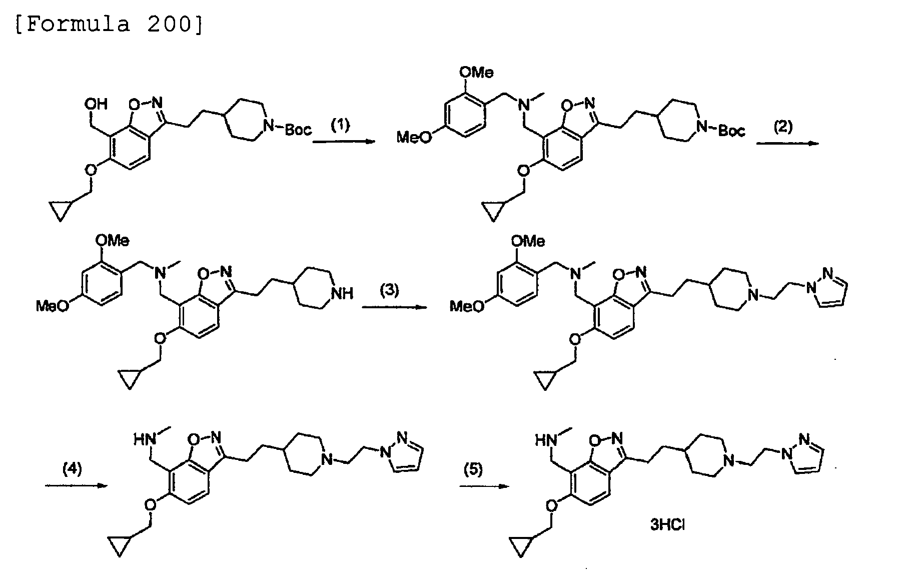

### (1) tert-Butyl 4-{2-{6-cyclopropylmethoxy-7-{[(2,4-dimethoxybenzyl)methylamino]methyl}benz[d]isoxazol-3-yl}ethyl}piperidine-1-carboxylate

tert-Butyl 4-[2-(6-cyclopropylmethoxy-7-hydroxymethylbenz[d]isoxazol-3-yl)ethyl]piperidine-1-carboxylate (609 mg) was dissolved in tetrahydrofuran (6 mL), and triethylamine (591 µL) was added. The reaction mixture was stirred under ice-cooling in a nitrogen atmosphere. Methanesulfonyl chloride (131 µL) was added to the reaction mixture, and the reaction mixture was stirred for 35 minutes. A solution of N-methyl-2,4-dimethoxybenzylamine [CAS Registry No.
102503-23-1] (564 mg) in N,N-dimethylformamide (6 mL) was added to the mixture. The mixture was stirred at 60°C for 11 hours and 50 minutes. The reaction mixture was cooled to room temperature, and then the solvent was evaporated under reduced pressure. A 10% sodium carbonate solution was added to the residue, followed by extraction with ethyl acetate. The organic layer was washed with a saturated sodium chloride solution and dried over magnesium sulfate. The drying agent was removed by filtration and then the organic layer was concentrated under reduced pressure. The residue was purified by NH silica gel column chromatography (heptane-ethyl acetate-methanol) to obtain the title compound (701 mg).
¹H-NMR (400 MHz, CDCl₃) δ (ppm): 0.32-0.37 (m, 2H), 0.58-0.63 (m, 2H), 1.11-1.21 (m, 2H), 1.24-1.30 (m, 1H), 1.46 (s, 9H), 1.46-1.56 (m, 1H), 1.72-1.81 (m, 4H), 2.24 (s, 3H), 2.64-2.72 (m, 2H), 2.93-2.97 (m, 2H), 3.64 (s, 2H), 3.78 (s, 3H), 3.80 (s, 3H), 3.92 (d, J=6.8 Hz, 2H), 3.95 (s, 2H), 4.03-4.15 (m, 2H), 6.44-6.47 (m, 2H), 6.89 (d, J=8.4 Hz, 1H), 7.34 (d, J=8.4 Hz, 1H), 7.43 (d, J=8.8 Hz, 1H).

### (2) N-(2,4-Dimethoxybenzyl)-N-methyl{6-cyclopropylmethoxy-3-[2-(piperidin-4-yl)ethyl]benz[d]isoxazol-7-yl}methylamine

tert-Butyl 4-{2-{6-cyclopropylmethoxy-7-{[(2,4-dimethoxybenzyl)methylamino]methyl}benz[d]isoxazol-3-yl}ethyl}piperidine-1-carboxylate (701 mg) was dissolved in tetrahydrofuran (7 mL) and methanol (7 mL). The solution was stirred in an ice bath in a nitrogen atmosphere. Hydrogen chloride (4 M solution in ethyl acetate) (2.95 mL) was added to the reaction mixture. The mixture was stirred at room temperature for six hours. The solvent was evaporated under reduced pressure. A saturated sodium bicarbonate solution and a saturated sodium chloride solution were added to the residue, followed by extraction with chloroform three times. The combined organic layers were washed with a saturated sodium chloride solution and dried over magnesium sulfate. The drying agent was removed by filtration and then the organic layers were concentrated under reduced pressure to obtain the crude title compound (667 mg). The title compound was used for the next reaction without further purification.
¹H-NMR (400 MHz, CDCl₃) δ (ppm): 0.32-0.36 (m, 2H), 0.59-0.63 (, 2H), 1.23-1.31 (m, 1H), 1.40-1.58 (m, 3H), 1.79-1.89 (m, 4H), 2.24 (s, 3H), 2.68-2.75 (m, 2H), 2.94-2.97 (m, 2H), 3.27-3.32 (m, 2H), 3.65 (s, 2H), 3.79 (s, 3H), 3.80 (s, 3H), 3.92 (d, J=6.8 Hz, 2H), 3.96 (s, 2H), 6.44-6.48 (m, 2H), 6.94 (d, J=8.8 Hz, 1H), 7.35 (d, J=8.0 Hz, 1H), 7.43 (d, J=8.8 Hz, 1H).

### (3) N-(2,4-Dimethoxybenzyl)-N-methyl{6-cyclopropylmethoxy-3-{2-{1-[2-(1H-pyrazol-1-yl)ethyl]piperidin-4-yl}ethyl}benz[d]isoxazol-7-yl}methylamine

1-(2-Bromoethyl)-1H-pyrazole was synthesized by the method described in Bioorg. Med. Chem., 1999, 7, 517. A mixture of N-(2,4-dimethoxybenzyl)-N-methyl{6-cyclopropylmethoxy-3-[2-(piperidin-4-yl)ethyl]benz[d]isoxazol-7-yl}methylamine (583 mg), 1-(2-bromoethyl)-1H-pyrazole (248 mg), N,N-diisopropylethylamine (514 µL) and acetonitrile (20 mL) was stirred in a nitrogen atmosphere at 40°C for nine hours and 30 minutes. Here, 1-(2-bromoethyl)-1H-pyrazole (124 mg) and N,N-diisopropylethylamine (247 µL) were added and the mixture was further stirred for eight hours and 20 minutes. The mixture was cooled to room temperature and the solvent was evaporated under reduced pressure. A 10% sodium carbonate solution was added to the residue, followed by extraction with ethyl acetate twice. The combined organic layers were washed with a saturated sodium chloride solution and dried over magnesium sulfate. The drying agent was removed by filtration and then the organic layers were concentrated under reduced pressure. The residue was purified by NH silica gel column chromatography (heptane-ethyl acetate) to obtain the title compound (584 mg).
¹H-NMR (400 MHz, CDCl₃) δ (ppm): 0.32-0.38 (m, 2H), 0.58-0.63 (m, 2H), 1.25-1.43 (m, 4H), 1.74-1.80 (m, 4H), 2.02-2.08 (m, 2H), 2.24 (s, 3H), 2.79 (t, J=6.9 Hz, 2H), 2.85-2.89 (m, 2H), 2.92-2.95 (m, 2H), 3.64 (s, 2H), 3.78 (s, 3H), 3.81 (s, 3H), 3.92 (d, J=6.8 Hz, 2H), 3.95 (s, 2H), 4.26 (t, J=6.8 Hz, 2H), 6.23-6.24 (m, 1H), 6.44-6.48 (m, 2H), 6.89 (d, J=8.6 Hz, 1H), 7.34 (d, J=8.4 Hz, 1H), 7.43 (d, J=8.6 Hz, 1H), 7.45-7.46 (m, 1H), 7.49-7.50 (m, 1H).

### (4) N-Methyl{6-cyclopropylmethoxy-3-{2-{1-[2-(1H-pyrazol-1-yl)ethyl]piperidin-4-yl}ethyl}benz[d]isoxazol-7-yl}methylamine

N-(2,4-Dimethoxybenzyl)-N-methyl{6-cyclopropylmethoxy-3-{2-{1-[2-(1H-pyrazol-1-yl)ethyl]piperidin-4-yl}ethyl}benz[d]isoxazol-7-yl}methylamine (584 mg) was dissolved in methylene chloride (5 mL). The solution was stirred under ice-cooling in a nitrogen atmosphere. Trifluoroacetic anhydride (180 µL) was added to the solution, and the mixture was stirred at the same temperature for three hours and 10 minutes. The mixture was diluted with chloroform under ice-cooling. A saturated sodium bicarbonate solution was added, followed by stirring. The organic layer was separated and the aqueous layer was extracted with chloroform. The combined organic layers were washed with a saturated sodium chloride solution and dried over magnesium sulfate. The drying agent was removed by filtration and then the organic layers were concentrated under reduced pressure. The residue was dissolved in methanol (6 mL). A 5 M sodium hydroxide solution (1.99 mL) was added and the mixture was stirred for 30 minutes. A saturated sodium chloride solution was added to the reaction mixture, followed by extraction with chloroform. The organic layer was dried over magnesium sulfate. The drying agent was removed by filtration and then the organic layer was concentrated under reduced pressure. The residue was purified by NH silica gel column chromatography (heptane-ethyl acetate) to obtain the title compound (302 mg).
¹H-NMR (400 MHz, DMSO-d₆) δ (ppm): 0.39-0.43 (m, 2H), 0.57-0.63 (m, 2H), 1.28-1.39 (m, 1H), 1.44-1.95 (m, 7H), 2.58-2.61 (m, 3H), 2.83-3.18 (m, 4H), 3.36-3.42 (m, 2H), 3.47-3.52 (m, 2H), 4.07 (d, J=6.8 Hz, 2H), 4.31-4.34 (m, 2H), 4.61-4.65 (m, 2H), 6.31-6.32 (m, 1H), 7.24 (d, J=8.8 Hz, 1H), 7.53-7.53 (m, 1H), 7.84-7.85 (m, 1H), 7.92 (d, J=8.8 Hz, 1H), 9.00-9.01 (m, 2H), 10.56 (br s, 1H).
ESI-MS m/z: 407 [M-CH4NH⁺H]⁺, 438 [M+H]⁺, 460 [M+Na]⁺.

### Example 177

### N-Methyl{6-benzyloxy-3-{2-[1-(1,3-dioxolan-2-ylmethyl)piperidin-4-yl]ethyl}benz[d]isoxazol-7-yl}methylamine fumarate

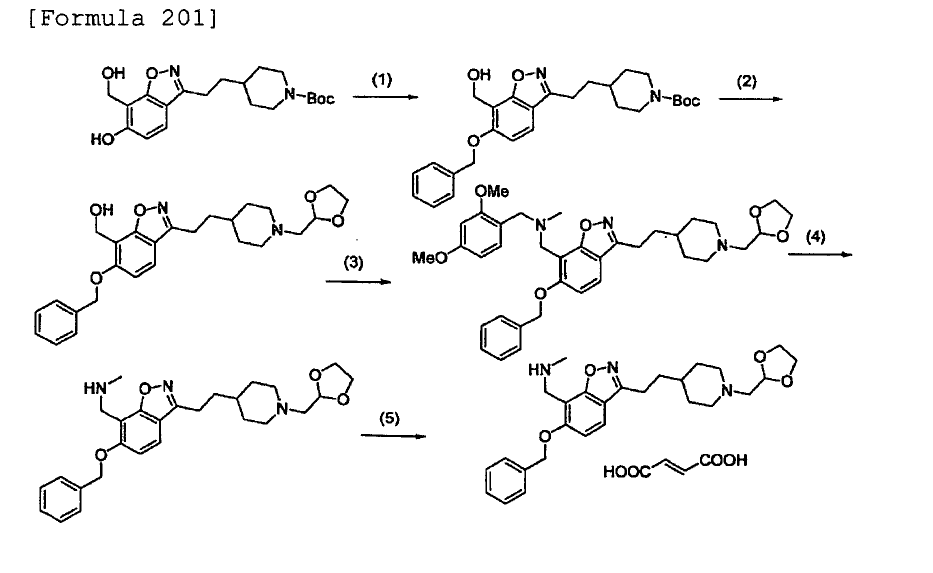

### (1) tert-Butyl 4-[2-(6-benzyloxy-7-hydroxymethylbenz[d]isoxazol-3-yl)ethyl]piperidine-1-carboxylate

tert-Butyl 4-[2-(6-hydroxy-7-hydroxymethylbenz[d]isoxazol-3-yl)ethyl]piperidine-1-carboxylate (2.93 g) was dissolved in acetone (30 mL). Potassium carbonate (1.61 g) and benzyl bromide (1.20 mL) were added to the solution. The reaction mixture was heated under reflux in a nitrogen atmosphere for two hours. The reaction mixture was cooled to room temperature. Water and a saturated sodium chloride solution were added to the reaction mixture, followed by extraction with ethyl acetate. The organic layer was washed with a saturated sodium chloride solution and dried over magnesium sulfate. The drying agent was removed by filtration and then the organic layer was concentrated under reduced pressure. Ethyl acetate was added to the residue, and the precipitate was removed by filtration. The filtrate was concentrated under reduced pressure. A mixture of hexane and diethyl ether (10:1) was added to the residue. The precipitate was collected by filtration and washed with the same solvent. The precipitate was dried under reduced pressure to obtain the title compound (3.38 g).
¹H-NMR (400 MHz, CDCl₃) δ (ppm): 1.10-1.21 (m, 2H), 1.46 (s, 9H), 1.46-1.54 (m, 1H), 1.71-1.80 (m, 4H), 2.64-2.71 (m, 2H), 2.95-2.99 (m, 2H), 4.06-4.15 (m, 2H), 5.04 (s, 2H), 5.24 (s, 2H), 7.02 (d, J=8.8 Hz, 1H), 7.34-7.45 (m, 5H), 7.49 (d, J=8.8 Hz, 1H).

### (2) {6-Benzyloxy-3-{2-[1-(1,3-dioxolan-2-ylmethyl)piperidin-4-yl]ethyl}benz[d]isoxazol-7-yl}methanol

tert-Butyl 4-[2-(6-benzyloxy-7-hydroxymethylbenz[d]isoxazol-3-yl)ethyl]piperidine-1-carboxylate (3.38 g) was dissolved by adding methanol (35 mL) and tetrahydrofuran (35 mL). The solution was stirred in an ice bath in a nitrogen atmosphere. Hydrogen chloride (4 M solution in ethyl acetate) (18.1 mL) was added to the solution, and the mixture was stirred at room temperature for 17 hours and 40 minutes. The solvent was evaporated under reduced pressure. A saturated sodium bicarbonate solution, a saturated sodium chloride solution and a 5 M sodium hydroxide solution were added to the residue, followed by extraction with chloroform. The organic layer was washed with a saturated sodium chloride solution and dried over magnesium sulfate. The drying agent was removed by filtration and then the organic layer was concentrated under reduced pressure.
The resulting residue was dissolved in N,N-dimethylformamide (20 mL). Potassium carbonate (4.00 g) and bromomethyl-1,3-dioxolane (1.5 mL) were added to the reaction mixture, and the mixture was stirred in a nitrogen atmosphere at 110°C for one hour and 30 minutes. Bromomethyl-1,3-dioxolane (0.75 mL) was added and the mixture was further stirred at 110°C for one hour and 20 minutes. The reaction mixture was cooled to room temperature and the precipitate was removed by filtration. The solvent was evaporated under reduced pressure. A saturated sodium bicarbonate solution and a saturated sodium chloride solution were added to the residue, followed by stirring and extraction with ethyl acetate. The organic layer was washed with a saturated sodium chloride solution and dried over magnesium sulfate. The drying agent was removed by filtration and then the organic layer was concentrated under reduced pressure. The residue was purified by NH silica gel column chromatography (heptane-ethyl acetate) to obtain the title compound (1.44 g).
¹H-NMR (400 MHz, CDCl₃) δ (ppm): 1.32-1.44 (m, 3H), 1.73-1.79 (m, 4H), 2.03-2.12 (m, 2H), 2.39 (br s, 1H), 2.56-2.59 (m, 2H), 2.93-2.97 (m, 2H), 2.97-3.05 (m, 2H), 3.82-3.90 (m, 2H), 3.93-4.02 (m, 2H), 5.01-5.05 (m, 3H), 5.24 (s, 2H), 7.01 (d, J=8.8 Hz, 1H), 7.36-7.45 (m, 5H), 7.48 (d, J=8.8 Hz, 1H).

### (3) N-(2,4-Dimethoxybenzyl)-N-methyl{6-benzyloxy-3-{2-[1-(1,3-dioxolan-2-ylmethyl)piperidin-4-yl]ethyl}benz[d]isoxazol-7-yl}methylamine

The title compound was obtained by synthesis from {6-benzyloxy-3-{2-[1-(1,3-dioxolan-2-ylmethyl)piperidin-4-yl]ethyl}benz[d]isoxazol-7-yl}methanol according to Example 176-(1).
¹H-NMR (400 MHz, CDCl₃) δ (ppm): 1.34-1.42 (m, 3H), 1.73-1.80 (m, 4H), 2.04-2.09 (m, 2H), 2.24 (s, 3H), 2.57 (d, J=4.4 Hz, 2H), 2.91-2.95 (m, 2H), 2.96-3.03 (m, 2H), 3.65 (s, 2H), 3.74 (s, 3H), 3.78 (s, 3H), 3.78-4.00 (m, 6H), 5.01 (t, J=4.4 Hz, 1H), 5.20 (s, 2H), 6.38-6.46 (m, 3H), 6.98 (d, J=8.8 Hz, 1H), 7.30-7.45 (m, 6H).

### (4) N-Methyl{6-benzyloxy-3-{2-[1-(1,3-dioxolan-2-ylmethyl)piperidin-4-yl]ethyl}benz[d]isoxazol-7-yl}methylamine

The title compound was obtained by synthesis from N-(2,4-dimethoxybenzyl)-N-methyl{6-benzyloxy-3-{2-[1-(1,3-dioxolan-2-ylmethyl)piperidin-4-yl]ethyl}benz[d]isoxazol-7-yl}methylamine according to Example 176-(4).
¹H-NMR (400 MHz, CDCl₃) δ (ppm): 1.37-1.46 (m, 3H), 1.73-1.80 (m, 4H), 2.07-2.14 (m, 2H), 2.43 (s, 3H), 2.60 (d, J=4.4 Hz, 2H), 2.92-2.96 (m, 2H), 3.01-3.06 (m, 2H), 3.82-3.90 (m, 2H), 3.93-4.01 (m, 2H), 4.17 (s, 2H), 5.04 (t, J=4.4 Hz, 1H), 5.23 (s, 2H), 7.00 (d, J=8.8 Hz, 1H), 7.33-7.47 (m, 6H).

### (5) N-Methyl{6-benzyloxy-3-{2-[1-(1,3-dioxolan-2-ylmethyl)piperidin-4-yl]ethyl}benz[d]isoxazol-7-yl}methylamine fumarate

The title compound was obtained by synthesis from N-methyl{6-benzyloxy-3-{2-[1-(1,3-dioxolan-2-ylmethyl)piperidin-4-yl]ethyl}benz[d]isoxazol-7-yl}methylamine according to Example 154-(4).
¹H-NMR (400 MHz, DMSO-d₆) δ (ppm): 1.12-1.27 (m, 3H), 1.64-1.70 (m, 4H), 1.23-1.99 (m, 2H), 2.42 (s, 3H), 2.44 (d, J=4.4 Hz, 2H), 2.88-2.97 (m, 4H), 3.70-3.78 (m, 2H), 3.81-3.90 (m, 2H), 4.14 (s, 2H), 4.86 (t, J=4.4 Hz, 1H), 5.32 (s, 2H), 6.54 (s, 2H), 7.27 (d, J=8.8 Hz, 1H), 7.33-7.37 (m, 1H), 7.39-7.44 (m, 2H), 7.50-7.54 (m, 2H), 7.82 (d, J=8.8 Hz, 1H).
ESI-MS m/z: 234 [M+2H]²⁺, 345 [M-C8H11N⁺H]⁺, 466 [M+H]⁺.

### Example 178

### N,N-Dimethyl{3-[2-(1-benzylpiperidin-4-yl)ethyl]-7-cyclopropylmethoxybenz[d]isoxazol-6-yl}methylamine dihydrochloride

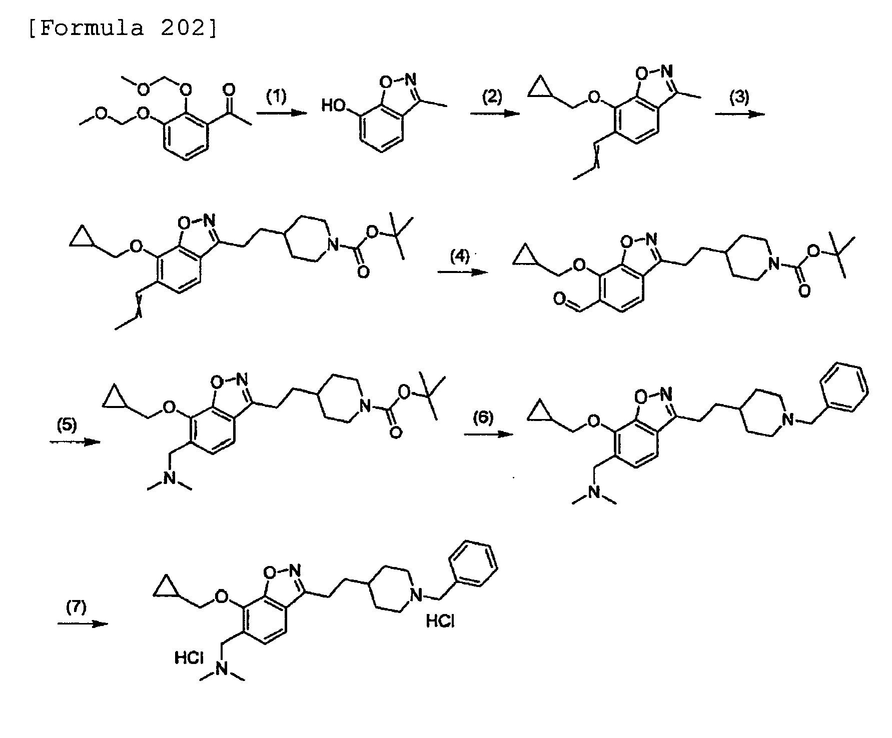

### (1) 7-Hydroxy-3-methylbenz[d]isoxazole

1-(2,3-Bismethoxymethoxyphenyl)ethanone (1.6 g) [CAS Registry No. 863191-11-1] was heated in 48% hydrobromic acid (10 mL) at 80°C for two hours. The mixture was cooled to room temperature and diluted with water, followed by extraction with ethyl acetate. The organic layer was washed with water, a saturated sodium bicarbonate solution and a saturated sodium chloride solution and dried over magnesium sulfate. The drying agent was removed by filtration and the filtrate was concentrated under reduced pressure to obtain a crude product of 1-(2,3-dihydroxyphenyl)ethanone (1.0 g). The resulting crude product of 1-(2,3-dihydroxyphenyl)ethanone (1.0 g), hydroxylamine hydrochloride (603 mg) and sodium acetate (735 mg) were dissolved in methanol (20 mL). The solution was stirred at 60°C for two hours. The mixture was cooled to room temperature. Water was added to the reaction mixture, followed by extraction with ethyl acetate. The organic layer was separated, sequentially washed with water and a saturated sodium chloride solution and dried over magnesium sulfate. The drying agent was removed by filtration and the filtrate was concentrated under reduced pressure to obtain a residue. A solution of the resulting residue, acetic anhydride (1.4 mL) and sodium acetate (1.19 g) in N,N-dimethylformamide (10 mL) was heated in a microwave synthesizer at 150°C for one hour. The mixture was diluted with water, followed by extraction with ethyl acetate. The ethyl acetate layer was sequentially washed with water and a saturated sodium chloride solution and dried over magnesium sulfate. The drying agent was removed by filtration. The solvent was evaporated under reduced pressure. A 2 M sodium hydroxide solution was added to a solution of the residue in methanol, and the mixture was stirred at 45°C for 0.5 hour. Water was added to the reaction mixture, followed by extraction with ethyl acetate. The organic layer was separated, sequentially washed with water and a saturated sodium chloride solution and dried over magnesium sulfate. The drying agent was removed by filtration. The filtrate was concentrated under reduced pressure. The residue was purified by silica gel column chromatography (heptane-ethyl acetate) to obtain the title compound (530 mg). ¹H-NMR (400 MHz, DMSO-d₆) δ (ppm): 2.52 (s, 3H), 6.99 (dd, J=1.2, 7.6 Hz, 1H), 7.17 (t, J=7.6 Hz, 1H), 7.22 (dd, J=1.2, 7.6 Hz, 1H).

### (2) Mixture of 7-cyclopropylmethoxy-3-methyl-6-[(E)-1-propenyl]benz[d]isoxazole and 7-cyclopropylmethoxy-3-methyl-6-[(Z)-1-propenyl]benz[d]isoxazole

A mixture of 7-hydroxy-3-methylbenz[d]isoxazole (530 mg), allyl bromide (338 mg), potassium carbonate (981 mg) and N,N-dimethylformamide (5 mL) was stirred at room temperature for three hours. The mixture was diluted with water, followed by extraction with ethyl acetate. The ethyl acetate layer was sequentially washed with water and a saturated sodium chloride solution and dried over magnesium sulfate. The drying agent was removed by filtration and the filtrate was concentrated under reduced pressure. The residue was purified by silica gel chromatography to obtain 7-allyloxy-3-methylbenz[d]isoxazole (654 mg). A mixture of 7-allyloxy-3-methylbenz[d]isoxazole (654 mg) and N,N-dimethylaniline (3.5 mL) was heated in a microwave synthesizer at 220°C for one hour. The reaction mixture was dissolved in ethyl acetate and washed with 2 M hydrochloric acid. The ethyl acetate layer was further sequentially washed with water and a saturated sodium chloride solution and dried over magnesium sulfate. The drying agent was removed by filtration and the filtrate was concentrated under reduced pressure. A crude product of 6-allyl-7-hydroxy-3-methylbenz[d]isoxazole (640 mg) was obtained as a residue. A mixture of the resulting 6-allyl-7-hydroxy-3-methylbenz[d]isoxazole crude product (400 mg), cyclopropylmethyl bromide (0.27 mL), potassium carbonate (583 mg) and N,N-dimethylformamide (5 mL) was stirred at room temperature for three hours. The reaction mixture was diluted with water, followed by extraction with ethyl acetate. The organic layer was separated, sequentially washed with water and a saturated sodium chloride solution and dried over magnesium sulfate. The drying agent was removed by filtration and the solvent was evaporated under reduced pressure. The residue was purified by silica gel chromatography to obtain 6-allyl-7-cyclopropylmethoxy-3-methylbenz[d]isoxazole (350 mg). A solution of 6-allyl-7-cyclopropylmethoxy-3-methylbenz[d]isoxazole (350 mg) and potassium hydroxide (202 mg) in ethanol (3 mL) was heated in a microwave synthesizer at 120°C for three hours. The mixture was concentrated under reduced pressure. Water was added to the residue, followed by extraction with ethyl acetate. The organic layer was separated, sequentially washed with water and a saturated sodium chloride solution and dried over magnesium sulfate. The drying agent was removed by filtration and the solvent was evaporated under reduced pressure. The residue was purified by silica gel column chromatography (heptane-ethyl acetate) to obtain the title compound (320 mg).
¹H-NMR (400 MHz, CDCl₃) δ (ppm): 0.33-0.38 (m, 2H), 0.57-0.63 (m, 2H), 1.22-1.34 (m, 1H), 1.96 (dd, J=1.6, 7.2 Hz, 3H), 2.54 (s, 3H), 4.25 (d, J=7.2 Hz, 2H), 6.38 (dq, J=6.8, 16.0 Hz, 1H), 6.90 (dq, J=1.6, 16.0 Hz, 1H), 7.17 (d, J=8.0 Hz, 1H), 7.38 (d, J=8.0 Hz, 1H).

### (3) Mixture of tert-butyl 4-{2-{7-cyclopropylmethoxy-6-[(E)-1-propenyl]benz[d]isoxazol-3-yl}ethyl}piperidinecarboxylate and tert-butyl 4-{2-{7-cyclopropylmethoxy-6-[(Z)-1-propenyl]benz[d]isoxazol-3-yl}ethyl}piperidinecarboxylate

A solution of the mixture of 7-cyclopropylmethoxy-3-methyl-6-[(E)-1-propenyl]benz[d]isoxazole and 7-cyclopropylmethoxy-3-methyl-6-[(Z)-1-propenyl]benz[d]isoxazole (310 mg) and tert-butyl 4-iodomethylpiperidine-1-carboxylate (458 mg) in tetrahydrofuran (15 mL) was cooled to -70°C. A 1.5 M solution of lithium N,N-diisopropylamide in cyclohexane (1.7 mL) was added dropwise to the mixture. The mixture was stirred at -60°C for two hours and then gradually returned to room temperature. Water was added to the reaction mixture, followed by extraction with ethyl acetate. The organic layer was separated, sequentially washed with water and a saturated sodium chloride solution and dried over magnesium sulfate. The drying agent was removed by filtration and the solvent was evaporated under reduced pressure. The residue was purified by silica gel column chromatography (heptane-ethyl acetate) to obtain the title compound (440 mg).
¹H-NMR (400 MHz, CDCl₃) δ (ppm): 0.33-0.38 (m, 2H), 0.58-0.64 (m, 2H), 1.10-1.24 (m, 2H), 1.24-1.36 (m, 1H), 1.40-1.60 (m, 1H), 1.45 (s, 9H), 1.71-1.87 (m, 4H), 1.96 (dd, J=1.6, 6.8 Hz, 3H), 2.61-2.76 (m, 2H), 2.97 (t, J=8.0 Hz, 2H), 4.02-4.20 (m, 2H), 4.25 (d, J=6.8 Hz, 2H), 6.37 (dq, J=6.8, 16.0 Hz, 1H), 6.90 (dq, J=1.6, 16.0 Hz, 1H), 7.17 (d, J=8.0 Hz, 1H), 7.37 (d, J=8.0 Hz, 1H).

### (4) tert-Butyl 4-[2-(7-cyclopropylmethoxy-6-formylbenz[d]isoxazol-3-yl)ethyl]piperidine-1-carboxylate

The mixture of tert-butyl 4-{2-{7-cyclopropylmethoxy-6-[(E)-1-propenyl]benz[d]isoxazol-3-yl}ethyl}piperidinecarboxylate and tert-butyl 4-{2-{7-cyclopropylmethoxy-6-[(Z)-1-propenyl]benz[d]isoxazol-3-yl}ethyl}piperidinecarboxylate (440 mg) was dissolved in tetrahydrofuran (10 mL)-water (5 mL). A 2.5% osmium tetroxide solution (508 mg) was added to the solution at room temperature, followed by stirring. After five minutes, sodium metaperiodate (535 mg) was added to the mixture, and the reaction mixture was stirred at room temperature for one hour. Water was added to the reaction mixture, followed by extraction with ethyl acetate. The organic layer was separated and sequentially washed with water and a saturated sodium chloride solution. The drying agent was removed by filtration. The filtrate was concentrated under reduced pressure to obtain the title compound (330 mg).
¹H-NMR (400 MHz, CDCl₃) δ (ppm): 0.37-0.42 (m, 2H), 0.62-0.68 (m, 2H), 1.10-1.40 (m, 3H), 1.45 (s, 9H), 1.48-1.62 (m, 1H), 1.70-1.84 (m, 4H), 2.63-2.73 (m, 2H), 3.00 (t, J=8.0 Hz, 2H), 4.06-4.16 (m, 2H), 4.53 (d, J=7.6 Hz, 2H), 7.24 (d, J=8.4 Hz, 1H), 7.75 (d, J=8.0 Hz, 1H), 10.63 (s, 1H).

### (5) tert-Butyl 4-{2-[7-cyclopropylmethoxy-6-(N,N-dimethylaminomethyl)benz[d]isoxazol-3-yl]ethyl}piperidine-1-carboxylate

tert-Butyl 4-[2-(7-cyclopropylmethoxy-6-formylbenz[d]isoxazol-3-yl)ethyl]piperidine-1-carboxylate (330 mg), a 2 M solution of dimethylamine in tetrahydrofuran (0.77 mL) and acetic acid (0.07 mL) were stirred in ethyl acetate (10 mL) at room temperature for 15 minutes. Then, sodium triacetoxyborohydride (326 mg) was added to the mixture, followed by stirring at room temperature for one hour. The mixture was diluted with water and sodium carbonate was added, followed by extraction with ethyl acetate. The organic layer was separated, sequentially washed with water and a saturated sodium chloride solution and dried over magnesium sulfate. The drying agent was removed by filtration and the solvent was evaporated under reduced pressure. The residue was purified by NH silica gel column chromatography (heptane-ethyl acetate) to obtain the title compound (218 mg).
¹H-NMR (400 MHz, CDCl₃) δ (ppm): 0.33-0.38 (m, 2H), 0.57-0.62 (m, 2H), 1.10-1.24 (m, 2H), 1.24-1.35 (m, 1H), 1.45 (s, 9H), 1.48-1.57 (m, 1H), 1.71-1.84 (m, 4H), 2.30 (s, 6H), 2.61-2.75 (m, 2H), 2.98 (t, J=8.0 Hz, 2H), 3.65 (s, 2H), 4.03-4.19 (m, 2H), 4.30 (d, J=7.2 Hz, 2H), 7.22 (d, J=8.0 Hz, 1H), 7.32 (d, J=8.0 Hz, 1H).

### (6) N,N-Dimethyl{3-[2-(1-benzylpiperidin-4-yl)ethyl]-7-cyclopropylmethoxybenz[d]isoxazol-6-yl}methylamine

A solution of tert-butyl 4-{2-[7-cyclopropylmethoxy-6-(N,N-dimethylaminomethyl)benz[d]isoxazol-3-yl]ethyl}piperidine-1-carboxylate (218 mg) in dichloromethane (10 mL) was ice-cooled and trifluoroacetic acid (0.73 mL) was added. The mixture was stirred at room temperature for one hour and then concentrated under reduced pressure. Water was added to the residue. The mixture was made alkaline with potassium carbonate, followed by extraction with chloroform. The organic layer was dried over magnesium sulfate. The drying agent was removed by filtration and the solvent was evaporated under reduced pressure. The resulting crude product was used for the next reaction without purification. A mixture of the resulting crude product (160 mg), benzaldehyde (0.05 mL) and acetic acid (0.03 mL) was stirred in ethyl acetate (10 mL) at room temperature for 15 minutes. Sodium triacetoxyborohydride (202 mg) was added to the reaction mixture, followed by stirring at room temperature for one hour. The mixture was diluted with water and potassium carbonate was added, followed by extraction with ethyl acetate. The organic layer was separated, washed with water and a saturated sodium chloride solution and dried over magnesium sulfate. The drying agent was removed by filtration and the solvent was evaporated under reduced pressure. The residue was purified by silica gel column chromatography (chloroform-methanol) to obtain the title compound (83 mg).
¹H-NMR (400 MHz, CDCl₃) δ (ppm): 0.33-0.38 (m, 2H), 0.57-0.62 (m, 2H), 1.24-1.40 (m, 4H), 1.72-1.83 (m, 4H), 1.92-2.02 (m, 2H), 2.31 (s, 6H), 2.68-2.90 (m, 4H), 3.51 (s, 2H), 3.67 (s, 2H), 4.29 (d, J=7.2 Hz, 2H), 7.21 (d, J=8.0 Hz, 1H), 7.30-7.36 (m, 6H).

### (7) N,N-Dimethyl{3-[2-(1-benzylpiperidin-4-yl)ethyl]-7-cyclopropylmethoxybenz[d]isoxazol-6-yl}methylamine dihydrochloride

N,N-Dimethyl{3-[2-(1-benzylpiperidin-4-yl)ethyl]-7-cyclopropylmethoxybenz[d]isoxazol-6-yl}methylamine (83 mg) was dissolved in methanol, and a 4 M solution of hydrogen chloride in ethyl acetate was added. The mixture was concentrated and dried under reduced pressure to obtain the title compound (92 mg). ¹H-NMR (400 MHz, CD₃OD) δ (ppm): 0.36-0.41 (m, 2H), 0.59-0.65 (m, 2H), 1.25-1.38 (m, 1H), 1.51-1.65 (m, 2H), 1.65-1.79 (m, 1H), 1.79-1.86 (m, 2H), 2.02-2.19 (m, 2H), 2.91 (s, 6H), 2.96-3.10 (m, 4H), 3.44-3.50 (m, 2H), 4.29 (s, 2H), 4.47 (d, J=7.6 Hz, 2H), 4.51 (s, 2H), 7.43-7.58 (m, 7H).
ESI-MS m/z: 448 [M+H]⁺.

### Example 179

### N,N-Dimethyl{3-[2-(1-benzylpiperidin-4-yl)ethyl]-6-propoxybenz[d]isoxazol-7-yl}methylamine dihydrochloride

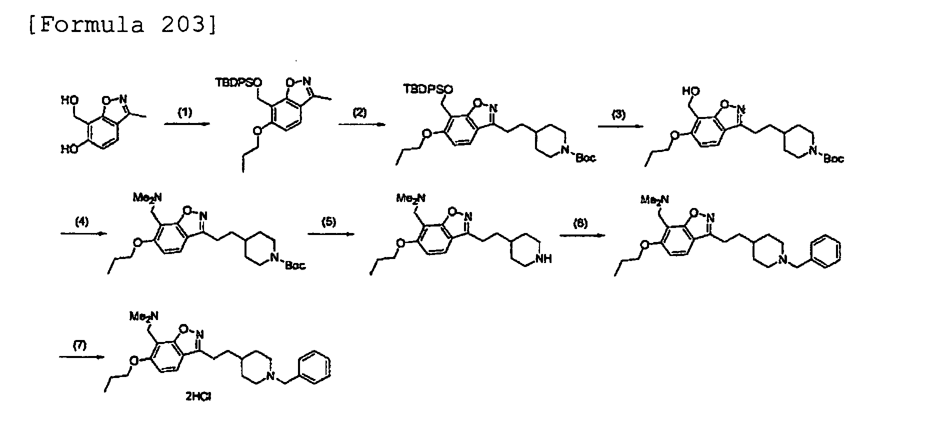

### (1) 7-(tert-Butyldiphenylsilanyloxymethyl)-3-methyl-6-propoxybenz[d]isoxazole

7-Hydroxymethyl-3-methylbenz[d]isoxazol-6-ol (compound synthesized in Preparation Example 2-(3)) (1.5 g) was dissolved in methyl ethyl ketone (80 mL), and potassium carbonate (1.74 g) and 1-bromopropane (1 mL) were sequentially added. The mixture was heated with stirring at 70°C for 21 hours. The reaction mixture was cooled to room temperature and then water was added, followed by extraction with ethyl acetate three times. The organic layers were washed with a mixed solution of a saturated sodium bicarbonate solution and a saturated sodium chloride solution and then dried over magnesium sulfate. The drying agent was removed by filtration and then the solvent was evaporated under reduced pressure to obtain a crude product.
The total amount of the resulting crude product was dissolved in N,N-dimethylformamide (20 mL). Imidazole (0.84 g) and tert-butyldiphenylchlorosilane (2.4 mL) were sequentially added to the solution, and the mixture was stirred at room temperature for 20 hours. Water was added to the reaction mixture, followed by extraction with ethyl acetate twice. The organic layers were sequentially washed with water and a saturated sodium chloride solution. The organic layers were dried over magnesium sulfate. The drying agent was removed by filtration and then the solvent was evaporated under reduced pressure. The residue was purified by silica gel chromatography (heptane-ethyl acetate) to obtain the title compound (2.98 g).
¹H-NMR (400 MHz, CDCl₃) δ (ppm) : 0.97 (t, J=7.4 Hz, 3H), 1.03 (s, 9H), 1.72-1.82 (m, 2H), 2.54 (s, 3H), 3.92 (t, J=6.6 Hz, 2H), 5.03 (s, 2H), 6.89 (d, J=8.4 Hz, 2H), 7.33-7.41 (m, 6H), 7.44 (d, J=8.4 Hz, 2H), 7.74-7.79 (m, 4H).

### (2) tert-Butyl 4-{2-[7-(tert-butyldiphenylsilanyloxymethyl)-6-propoxybenz[d]isoxazol-3-yl]ethyl}piperidine-1-carboxylate

The title compound was obtained by synthesis from 7-(tert-butyldiphenylsilanyloxymethyl)-3-methyl-6-propoxybenz[d]isoxazole according to Preparation Example 2-(5).
¹H-NMR (400 MHz, CDCl₃) δ (ppm) : 0.99 (t, J=7.6 Hz, 3H), 1.03 (s, 9H), 1.10-1.23 (m, 2H), 1.48 (s, 9H), 1.48-1.58 (m, 1H), 1.69-1.85 (m, 6H), 2.58-2.78 (m, 2H), 2.97 (t, J=7.8 Hz, 2H), 3.97 (t, J=6.4 Hz, 2H), 4.00-4.21 (m, 2H), 5.02 (s, 2H), 6.88 (d, J=8.8 Hz, 1H), 7.32-7.41 (m, 6H), 7.45 (d, J=8.8 Hz, 1H), 7.72-7.80 (m, 4H).

### (3) tert-Butyl 4-[2-(7-hydroxymethyl-6-propoxybenz[d]isoxazol-3-yl)ethyl]piperidine-1-carboxylate

tert-Butyl 4-{2-[7-(tert-butyldiphenylsilanyloxymethyl)-6-propoxybenz[d]isoxazol-3-yl]ethyl}piperidine-1-carboxylate (3.77 g) was dissolved in tetrahydrofuran (30 mL). Tetrabutylammonium fluoride (1 M solution in tetrahydrofuran, 9 mL) was added to the solution, and the mixture was stirred at room temperature for nine hours. The reaction mixture was concentrated under reduced pressure. The residue was purified by silica gel chromatography (heptane-ethyl acetate) to obtain the title compound (2.61 g).
¹H-NMR (400 MHz, CDCl₃) δ (ppm): 1.04-1.33 (m, 6H), 1.44 (s, 9H), 1.68-1.82 (m, 2H), 1.84-1.94 (m, 2H), 2.36-2.49 (m, 1H), 2.58-2.75 (m, 2H), 2.96 (t, J=7.6 Hz, 2H), 3.95-4.19 (m, 4H), 5.01 (d, J=6.4 Hz, 2H), 6.93 (d, J=8.8 Hz, 1H), 7.47 (d, J=8.8 Hz, 1H).

### (4) tert-Butyl 4-{2-[7-(N,N-dimethylaminomethyl)-6-propoxybenz[d]isoxazol-3-yl]ethyl}piperidine-1-carboxylate

tert-Butyl 4-[2-(7-hydroxymethyl-6-propoxybenz[d]isoxazol-3-yl)ethyl]piperidine-1-carboxylate (1.06 g) was dissolved in tetrahydrofuran (25 mL). Triethylamine (1.1 mL) was added to the solution, and the reaction mixture was ice-cooled. Methanesulfonyl chloride (300 µL) was added dropwise, followed by stirring. Dimethylamine (2 M solution in tetrahydrofuran, 15 mL) was added to the reaction mixture. Sodium iodide (40 mg) was added to the reaction mixture at room temperature, followed by stirring for 30 minutes. Water and a saturated sodium chloride solution were added to the reaction mixture. After extraction with ethyl acetate three times, the organic layers were washed with water and a mixed solution of a saturated sodium bicarbonate solution and a saturated sodium chloride solution. The organic layers were dried over magnesium sulfate. The drying agent was filtered off and then the filtrate was evaporated under reduced pressure. The residue was purified by NH silica gel chromatography (heptane-ethyl acetate) to obtain the title compound (0.95 g).
¹H-NMR (400 MHz, CDCl₃) δ (ppm) : 1.04-1.22 (m, 5H), 1.45 (s, 9H), 1.47-1.55 (m, 1H), 1.68-1.82 (m, 4H), 1.82-1. 92 (m, 2H), 2.32 (s, 9H), 2.60-2.75 (m, 2H), 2.95 (t, J=7.8 Hz, 2H), 3.78 (s, 2H), 3.97-4.18 (m, 4H), 6.92 (d, J=8.8 Hz, 1H), 7.44 (d, J=8.8 Hz, 1H).

### (5) N,N-Dimethyl{3-[2-(piperidin-4-yl)ethyl]-6-propoxybenz[d]isoxazol-7-yl}methylamine

tert-Butyl 4-{2-[7-(N,N-dimethylaminomethyl)-6-propoxybenz[d]isoxazol-3-yl]ethyl}-piperidine-1-carboxylate (0.95 g) was dissolved in methanol (25 mL). The solution was ice-cooled and then hydrogen chloride (4 M solution in ethyl acetate, 7 mL) was added to the reaction mixture. The reaction mixture was gradually heated to room temperature. After 25 hours, toluene (25 mL) was added to the reaction mixture, and the solvent was evaporated under reduced pressure. The residue was diluted with chloroform and neutralized with a 2 N sodium hydroxide solution. Sodium chloride was added to the mixture, followed by extraction with chloroform three times. The organic layers were dried over magnesium sulfate. The drying agent was filtered off and then the filtrate was evaporated under reduced pressure to obtain the title compound (800 mg).
¹H-NMR (400 MHz, CDCl₃) δ(ppm): 1.08 (t, J=7.4 Hz, 3H), 1.13-1.27 (m, 2H), 1.42-1.55 (m, 1H), 1.70-1.82 (m, 4H), 1.82-2.08 (m, 3H), 2.32 (s, 6H), 2.60 (dt, J=2.8 Hz, J=12.0 Hz, 2H), 2.95 (t, J=8.0 Hz, 2H), 3.04-3.14 (m, 2H), 3.78 (s, 2H), 4.04 (t, J=6.4 Hz, 2H), 6.92 (d, J=8.6 Hz, 1H), 7.45 (d, J=8.6 Hz, 1H).

### (6) N,N-Dimethyl{3-[2-(1-benzylpiperidin-4-yl)ethyl]-6-propoxybenz[d]isoxazol-7-yl}methylamine

N,N-Dimethyl{3-[2-(piperidin-4-yl)ethyl]-6-propoxybenz[d]isoxazol-7-yl}methylamine (71 mg) was dissolved in dichloromethane (5 mL). Benzaldehyde (28 µL), acetic acid (25 µL) and sodium triacetoxyborohydride (92 mg) were sequentially added to the solution, and the mixture was stirred at room temperature overnight. A saturated sodium bicarbonate solution was added to the reaction mixture, followed by extraction with chloroform three times. The organic layers were washed with a mixed solution of a saturated sodium bicarbonate solution and a saturated sodium chloride solution and then dried over magnesium sulfate. The drying agent was filtered off and then the filtrate was evaporated under reduced pressure. The residue was purified by NH silica gel chromatography (heptane-ethyl acetate) to obtain the title compound (61 mg).
¹H-NMR (400 MHz, CDCl₃) δ(ppm): 1.08 (t, J=7.6 Hz, 3H), 1.22-1.38 (m, 3H), 1.70-1.79 (m, 4H), 1.83-1.97 (m, 4H), 2.32 (s, 6H), 2.86-2.95 (m, 4H), 3.48 (s, 2H), 3.78 (s, 2H), 4.03 (t, J=6.4 Hz, 2H), 6.91 (d, J=8.4 Hz, 1H), 7.21-7.30 (m, 5H), 7.44 (d, J=8.4 Hz, 1H).

### (7) N,N-Dimethyl{3-[2-(1-benzylpiperidin-4-yl)ethyl]-6-propoxybenz[d]isoxazol-7-yl}methylamine dihydrochloride

N,N-Dimethyl{3-[2-(1-benzylpiperidin-4-yl)ethyl]-6-propoxybenz[d]isoxazol-7-yl}methylamine (61 mg) was dissolved in methanol. 2 N hydrochloric acid (146 µL) was added to the solution. The solvent was evaporated under reduced pressure and then the residue was dried under reduced pressure to obtain the title compound (41 mg).
¹H-NMR (400 MHz, CD₃OD) δ (ppm) : 1.12 (t, J=7.4 Hz, 3H), 1.48-1.64 (m, 2H), 1.66-1.79 (m, 1H), 1.79-1.89 (m, 2H), 1.89-2.00 (m, 2H), 2.03-2.13 (m, 2H), 2.94 (s, 6H), 2.97-3.13 (m, 4H), 3.45-3.54 (m, 2H), 4.22 (t, J=6.8 Hz, 2H), 4.31 (s, 2H), 4.61 (s, 2H), 7.26 (d, J=8.8 Hz, 1H), 7.46-7.51 (m, 3H), 7.51-7.58 (m, 2H), 7.93 (d, J=8.8 Hz, 1H).
ESI-MS m/z: 219 [M+2H]²⁺, 436 [M+H]⁺.

### Example 180

### 2-{4-{2-[7-(N,N-Dimethylaminomethyl)-6-propoxybenz[d]isoxazol-3-yl]ethyl}piperidinomethyl}benzonitrile dihydrochloride

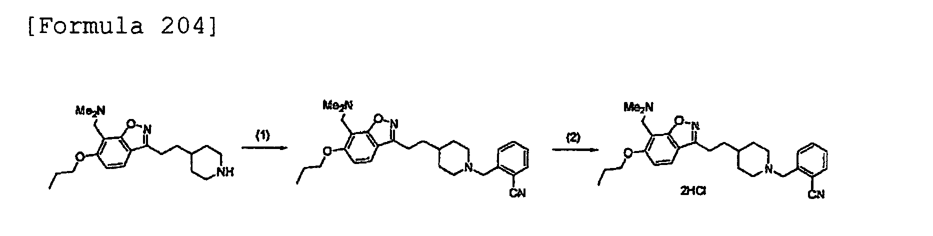

### (1) 2-{4-{2-[7-(N,N-Dimethylaminomethyl)-6-propoxybenz[d]isoxazol-3-yl]ethyl}piperidinomethyl}benzonitrile

The title compound was obtained by synthesis from N,N-dimethyl{3-[2-(piperidin-4-yl)ethyl]-6-propoxybenz[d]isoxazol-7-yl}methylamine (compound synthesized in Example 179-(5)) and 2-cyanobenzaldehyde according to Example 179-(6).
¹H-NMR (400 MHz, CDCl₃) δ (ppm) : 1.08 (t, J=7.2 Hz, 3H), 1.24-1.44 (m, 3H), 1.71-1.80 (m, 2H), 1.83-1.92 (m, 2H), 2.03-2.12 (m; 2H), 2.32 (s, 6H), 2.82-2.90 (m, 2H), 2.93 (t, J=7.8 Hz, 2H), 3.67 (s, 2H), 3.79 (s, 2H), 4.04 (t, J=6.4 Hz, 2H), 6.92 (d, J=8.8 Hz, 1H), 7.30-7.34 (m, 1H), 7.44 (d, J=8.8 Hz, 1H), 7.50-7.66 (m, 3H).

### (2) 2-{4-{2-[7-(N,N-Dimethylaminomethyl)-6-propoxybenz[d]isoxazol-3-yl]ethyl}piperidinomethyl}benzonitrile dihydrochloride

The title compound was obtained by synthesis from 2-{4-{2-[7-(N,N-dimethylaminomethyl)-6-propoxybenz[d]isoxazol-3-yl]ethyl}piperidinomethyl}benzonitrile according to Example 179-(7).
¹H-NMR (400 MHz, CD₃OD) δ(ppm): 1.12 (t, J=7.4 Hz, 3H), 1.54-1.68 (m, 2H), 1.70-1.89 (m, 3H), 1.89-2.00 (m, 2H), 2.06-2.16 (m, 2H), 2.94 (s, 6H), 3.06 (t, J=7.8 Hz, 2H), 3.11-3.24 (m, 2H), 3.55-3.68 (m, 2H), 4.23 (t, J=6.6 Hz, 2H), 4.55 (s, 2H), 4.61 (s, 2H), 7.26 (d, J=8.8 Hz, 1H), 7.64-7.73 (m, 1H), 7.80-7.86 (m, 1H), 7.88-7.96 (m, 3H).
ESI-MS m/z: 231 [M+2H]²⁺, 461 [M+H]⁺.

### Example 181

### 3-{4-{2-[7-(N,N-Dimethylaminomethyl)-6-propoxybenz[d]isoxazol-3-yl]ethyl}piperidino}-2,2-dimethylpropan-1-ol dihydrochloride

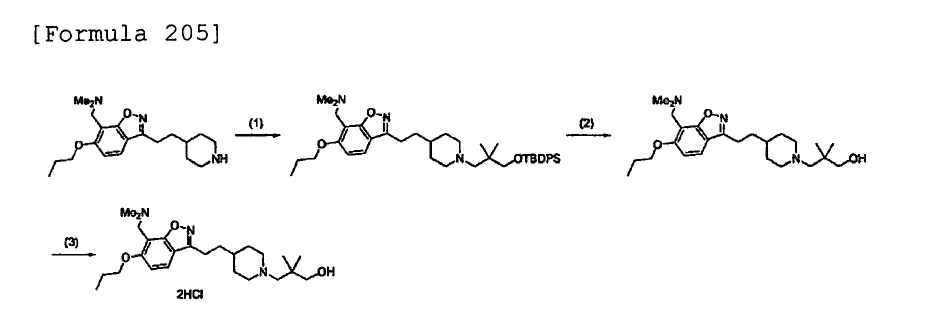

### (1) N,N-Dimethyl{3-{2-{1-[3-(tert-butyldiphenylsilanyloxy)-2,2-dimethylpropyl]piperidin-4-yl}ethyl}-6-propoxybenz[d]isoxazol-7-yl}methylamine

The title compound was obtained by synthesis from N,N-dimethyl{3-[2-(piperidin-4-yl)ethyl]-6-propoxybenz[d]isoxazol-7-yl}methylamine (compound synthesized in Example 179-(5)) and 3-(tert-butyldiphenylsilanyloxy)-2,2-dimethylpropionaldehyde [CAS Registry No. 136022-55-4] according to Example 179-(6).
¹H-NMR (400 MHz, CDCl₃) δ(ppm): 0.82 (s, 6H), 1.02-1.12 (m, 9H), 1.12-1.34 (m, 3H), 1.57-1.66 (m, 2H), 1.68-1.78 (m, 2H), 1.82-1.93 (m, 2H), 2.08-2.22 (m, 4H), 2.33 (s, 6H), 2.74-2.82 (m, 2H), 2.92 (t, J=8.0 Hz, 2H), 3.36 (s, 2H), 3.80 (s, 2H), 4.04 (t, J=6.4 Hz, 2H), 6.92 (d, J=8.8 Hz, 1H), 7.32-7.43 (m, 6H), 7.45 (d, J=8.8 Hz, 1H), 7.62-7.68 (m, 4H).

### (2) 3-{4-{2-[7-(N,N-Dimethylaminomethyl)-6-propoxybenz[d]isoxazol-3-yl]ethyl}piperidino}-2,2-dimethylpropan-1-ol

N,N-Dimethyl{3-{2-{1-[3-(tert-butyldiphenylsilanyloxy)-2,2-dimethylpropyl]piperidin-4-yl}ethyl}-6-propoxybenz[d]isoxazol-7-yl}methylamine (122 mg) was dissolved in tetrahydrofuran (1 mL). Tetrabutylammonium fluoride (1 M solution in tetrahydrofuran, 1.5 mL) was added to the solution, and the mixture was stirred at room temperature overnight. Ethyl acetate was added to the reaction mixture, followed by extraction with 2 N hydrochloric acid twice. The aqueous layer was made basic with a 5 N sodium hydroxide solution, followed by extraction with ethyl acetate three times. The organic layers were dried over sodium sulfate. Then, the drying agent was removed by filtration and the filtrate was evaporated under reduced pressure. The residue was purified by NH preparative TLC (heptane-ethyl acetate) to obtain the title compound (35 mg).
¹H-NMR (400 MHz, CDCl₃) δ(ppm): 0.92 (s, 6H), 1.08 (t, J=7.2 Hz, 3H), 1.23-1.38 (m, 3H), 1.61-1.78 (m, 5H), 1.83-1.92 (m, 2H), 2.08-2.17 (m, 2H), 2.32 (s, 6H), 2.36 (s, 2H), 2.90-3.00 (m, 4H), 3.49 (s, 2H), 3.78 (s, 2H), 4.04 (t, J=6.4 Hz, 2H), 6.92 (d, J=8.6 Hz, 1H), 7.44 (d, J=8.6 Hz, 1H).

### (3) 3-{4-{2-[7-(N,N-Dimethylaminomethyl)-6-propoxybenz[d]isoxazol-3-yl]ethyl}piperidino}-2,2-dimethylpropan-1-ol dihydrochloride

The title compound was obtained by synthesis from 3-{4-{2-[7-(N,N-dimethylaminomethyl)-6-propoxybenz[d]isoxazol-3-yl]ethyl}piperidino}-2,2-dimethylpropan-1-ol according to Example 179-(7).
¹H-NMR (400 MHz, CD₃OD) δ(ppm): 1.06 (s, 6H), 1.12 (t, J=7.4 Hz, 3H), 1.50-1.73 (m, 3H), 1.78-2.06 (m, 6H), 2.76 (s, 6H), 2.92-3.10 (m, 6H), 3.42-3.57 (m, 4H), 4.19 (t, J=6.4 Hz, 2H), 4.39 (s, 2H), 7.22 (d, J=8.8 Hz, 1H), 7.87 (d, J=8.8 Hz, 1H).
ESI-MS m/z: 217 [M+2H]²⁺, 432 [M+H]⁺.

### Example 182

### N,N-Dimethyl{3-[2-(1-benzylpiperidin-4-yl)ethyl]-6-(cyclohex-2-enyloxy)benz[d]isoxazol-7-yl}methylamine difumarate

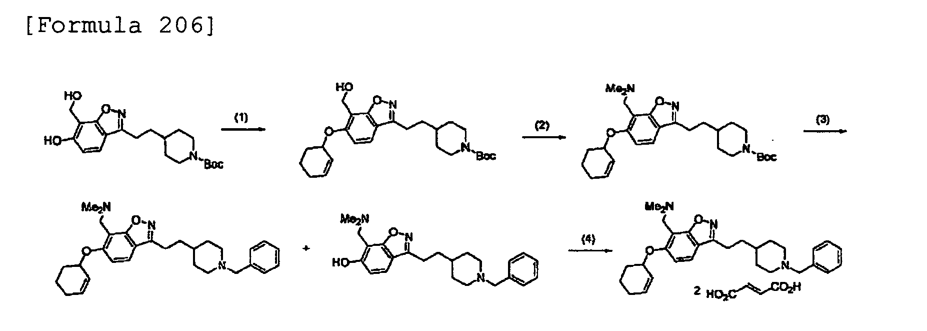

### (1) tert-Butyl 4-{2-[6-(cyclohex-2-enyloxy)-7-hydroxymethylbenz[d]isoxazol-3-yl]ethyl}piperidine-1-carboxylate

tert-Butyl 4-[2-(6-hydroxy-7-hydroxymethylbenz[d]isoxazol-3-yl)ethyl]piperidine-1-carboxylate (compound synthesized in Preparation Example 2) (1.05 g) was dissolved in propionitrile (50 mL). Potassium carbonate (1.2 g) and 3-bromocyclohexene (800 µL) were sequentially added to the solution, and the mixture was heated with stirring at 70°C for 15 hours. The reaction mixture was cooled to room temperature and then water was added, followed by extraction with ethyl acetate twice. The organic layers were washed with water and a saturated sodium chloride solution and then dried over sodium sulfate. The drying agent was filtered off and then the filtrate was evaporated under reduced pressure. The residue was purified by NH silica gel chromatography (heptane-ethyl acetate) to obtain the title compound (866 mg).
¹H-NMR (400 MHz, CDCl₃) δ(ppm): 1.08-1.22 (m, 2H), 1.33-2.24 (m, 20H), 2.46-2.78 (m, 3H), 2.96 (t, J=7.6 Hz, 2H), 3.82-4.26 (m, 2H), 4.88-5.02 (m, 3H), 5.83-5.89 (m, 1H), 5.98-6.04 (m, 1H), 6.99 (d, J=8.8 Hz, 1H), 7.46 (d, J=8.8 Hz, 1H).

### (2) tert-Butyl 4-{2-[6-(cyclohex-2-enyloxy)-7-(N,N-dimethylaminomethyl)benz[d]isoxazol-3-yl]ethyl}piperidine-1-carboxylate

The title compound was obtained by synthesis from tert-butyl 4-{2-[6-(cyclohex-2-enyloxy)-7-hydroxymethylbenz[d]isoxazol-3-yl]ethyl}piperidine-1-carboxylate according to Example 179-(4).
¹H-NMR (400 MHz, CDCl₃) δ(ppm): 1.08-1.22 (m, 2H), 1.37-2.21 (m, 20H), 2.31 (s, 6H), 2.58-2.76 (m, 2H), 2.95 (t, J=8.0 Hz, 2H), 3.78 (s, 2H), 3.95-4.20 (m, 2H), 4.83-4.91 (m, 1H), 5.82-5.89 (m, 1H), 5.91-5.99 (m, 1H), 6.97 (d, J=8.6 Hz, 1H), 7.43 (d, J=8.6 Hz, 1H).

### (3) N,N-Dimethyl{3-[2-(1-benzylpiperidin-4-yl)ethyl]-6-(cyclohex-2-enyloxy)benz[d]isoxazol-7-yl}methylamine

tert-Butyl 4-{2-[6-(cyclohex-2-enyloxy)-7-(N,N-dimethylaminomethyl)benz[d]isoxazol-3-yl]ethyl}piperidine-1-carboxylate (982 mg) was dissolved in methanol (25 mL). The solution was ice-cooled and hydrogen chloride (4 M solution in ethyl acetate, 7 mL) was added. The reaction mixture was gradually heated to room temperature. After 23 hours, toluene (25 mL) was added to the reaction mixture, and then the solvent was evaporated under reduced pressure. The residue was diluted with chloroform and then neutralized with a 2 N sodium hydroxide solution. Sodium chloride was added, followed by extraction with chloroform four times. The organic layers were dried over magnesium sulfate, and the drying agent was removed by filtration. The solvent was evaporated under reduced pressure. The resulting residue was dissolved in dichloromethane (20 mL). Benzaldehyde (300 µL), acetic acid (160 µL) and sodium triacetoxyborohydride (840 mg) were sequentially added to the solution, and the mixture was stirred at room temperature overnight. A saturated sodium bicarbonate solution was added to the reaction mixture, followed by extraction with chloroform three times. The organic layers were washed with a saturated sodium chloride solution and then dried over magnesium sulfate. The drying agent was removed by filtration and then the solvent was evaporated under reduced pressure. The residue was sequentially purified by NH silica gel chromatography (heptane-ethyl acetate) and silica gel chromatography (heptane-ethyl acetate-chloroform-methanol) to obtain the title compound (93 mg) and 3-[2-(1-benzylpiperidin-4-yl)ethyl]-7-(N,N-dimethylaminomethyl)benz[d]isoxazol-6-ol (150 mg). Title compound: N,N-Dimethyl{3-[2-(1-benzylpiperidin-4-yl)ethyl]-6-(cyclohex-2-enyloxy)benz[d]isoxazol-7-yl}methylamine
¹H-NMR (400 MHz, CDCl₃) δ(ppm): 1.23-1.43 (m, 3H), 1.62-1.81 (m, 5H), 1.82-2.21 (m, 7H), 2.32 (s, 6H), 2.85-2.96 (m, 4H), 3.49 (s, 2H), 3.80 (s, 2H), 4.84-4.92 (m, 1H), 5.82-5.88 (m, 1H), 5.96(dt, J=4.0 Hz, 10.4 Hz, 1H), 6.96 (d, J=8.6 Hz, 1H), 7.20-7.32 (m, 5H), 7.43 (d, J=8.6 Hz, 1H).
By-product: 3-[2-(1-Benzylpiperidin-4-yl)ethyl]-7-(N,N-dimethylaminomethyl)benz[d]isoxazol-6-ol
¹H-NMR (400 MHz, CDCl₃) δ(ppm) : 1.22-1.40 (m, 3H), 1.66-1.81 (m, 4H), 1.88-1.99 (m, 2H), 2.40 (s, 6H), 2.82-2.94 (m, 4H), 3.48 (s, 2H), 3.96 (m, 2H), 6.79 (d, J=8.4 Hz, 1H), 7.18-7.31 (m, 5H), 7.33 (d, J=8.4 Hz, 1H).

### (4) N,N-Dimethyl{3-[2-(1-benzylpiperidin-4-yl)ethyl]-6-(cyclohex-2-enyloxy)benz[d]isoxazol-7-yl}methylamine difumarate

N,N-Dimethyl{3-[2-(1-benzylpiperidin-4-yl)ethyl]-6-(cyclohex-2-enyloxy)benz[d]isoxazol-7-yl}methylamine (93 mg) was dissolved in methanol, and fumaric acid (46 mg) was added. The solvent was evaporated under reduced pressure, followed by drying under reduced pressure. The title compound (89 mg) was obtained as a residue.
¹H-NMR (400 MHz, CD₃OD) δ(ppm) : 1.46-2.25 (m, 13H), 2.84-3.09 (m, 10H), 3.35 (s, 2H), 4.28 (s, 2H), 4.54 (s, 2H), 5.13-5.20 (m, 1H), 5.88-5.94 (m, 1H), 6.02-6.10 (m, 1H), 6.70 (s, 4H), 7.30 (d, J=8.8 Hz, 1H), 7.44-7.53 (m, 5H), 7.88 (d, J=8.8 Hz, 1H).
ESI-MS m/z: 238 [M+2H]²⁺, 474 [M+H]⁺.

### Example 183

### N,N-Dimethyl{3-[2-(1-benzylpiperidin-4-yl)ethyl]-6-(3-methyl-2-butenyloxy)benz[d]isoxazol-7-yl}methylamine dihydrochloride

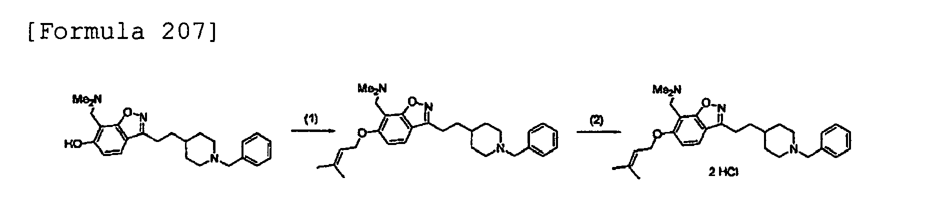

### (1) N,N-Dimethyl{3-[2-(1-benzylpiperidin-4-yl)ethyl]-6-(3-methyl-2-butenyloxy)benz[d]isoxazol-7-yl}methylamine

3-[2-(1-Benzylpiperidin-4-yl)ethyl]-7-(N,N-dimethylaminomethyl)benz[d]isoxazol-6-ol (compound synthesized in Example 182-(3)) (150 mg) was dissolved in tetrahydrofuran (10 mL). 3-Methyl-2-buten-1-ol (45 µL) and triphenylphosphine (200 mg) were added to the solution. Diisopropyl azodicarboxylate (150 µL) was added dropwise to the solution. The reaction mixture was stirred at room temperature for two hours, and then the solvent was evaporated under reduced pressure. The residue was purified by NH silica chromatography (heptane-ethyl acetate) and preparative TLC (chloroform-methanol) to obtain the title compound (25 mg).
¹H-NMR (400 MHz, CDCl₃) δ(ppm): 1.22-1.41 (m, 3H), 1.69-1.88 (m, 10H), 1.89-1.98 (m, 2H), 2.31 (s, 6H), 2.84-2.96 (m, 4H), 3.48 (s, 2H), 3.77 (s, 2H), 4.64 (d, J=6.4 Hz, 2H), 5.45-5.50 (m, 1H), 6.92 (d, J=8.6 Hz, 1H), 7.20-7.31 (m, 5H), 7.43 (d, J=8.6 Hz, 1H).

### (2) N,N-Dimethyl{3-[2-(1-benzylpiperidin-4-yl)ethyl]-6-(3-methyl-2-butenyloxy)benz[d]isoxazol-7-yl}methylamine dihydrochloride

The title compound was obtained by synthesis from N,N-dimethyl{3-[2-(1-benzylpiperidin-4-yl)ethyl]-6-(3-methyl-2-butenyloxy)benz[d]isoxazol-7-yl}methylamine according to Example 179-(7).
¹H-NMR (400 MHz, CD₃OD) δ(ppm): 1.45-1.60 (m, 2H), 1.60-1.72 (m, 1H), 1.77-1.89 (m, 8H), 1.96-2.07 (m, 2H), 2.75-2.90 (m, 8H), 3.04 (t, J=7.8 Hz, 2H), 3.33-3.42 (m, 2H), 4.17 (s, 2H), 4.42 (s, 2H), 4.81 (d, J=6.8 Hz, 2H), 5.52-5.58 (m, 1H), 7.23 (d, J=8.8 Hz, 1H), 7.42-7.53 (m, 5H), 7.87 (d, J=8.8 Hz, 1H).
ESI-MS m/z: 462 [M+H]⁺.

### Example 184

### N,N-Dimethyl{3-[2-(1-benzylpiperidin-4-yl)ethyl]-6-(3,4-dichlorobenzyloxy)benz[d]isoxazol-7-yl}methylamine dihydrochloride

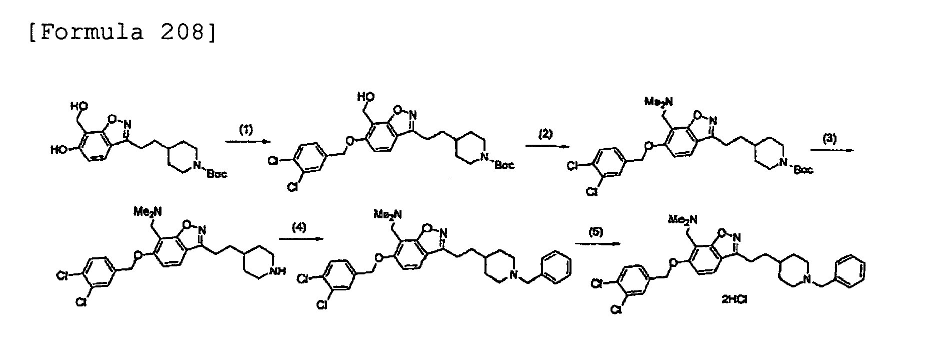

### (1) tert-Butyl 4-{2-[6-(3,4-dichlorobenzyloxy)-7-hydroxymethylbenz[d]isoxazol-3-yl]ethyl}piperidine-1-carboxylate

tert-Butyl 4-[2-(6-hydroxy-7-hydroxymethylbenz[d]isoxazol-3-yl)ethyl]piperidine-1-carboxylate (compound synthesized in Preparation Example 2) (854 mg) was dissolved in propionitrile (25 mL). Potassium carbonate (0.5 g) and 3,4-dichlorobenzyl chloride (410 µL) were sequentially added to the solution, and the mixture was heated with stirring at 70°C for 13 hours. The reaction mixture was cooled to room temperature and then water was added, followed by extraction with ethyl acetate twice. The organic layers were washed with water and a saturated sodium chloride solution and dried over sodium sulfate. The drying agent was removed by filtration and then the solvent was evaporated under reduced pressure. The residue was purified by NH silica gel chromatography (heptane-ethyl acetate) to obtain the title compound (884 mg).
¹H-NMR (400 MHz, CDCl₃) δ(ppm): 1.08-1.22 (m, 2H), 1.44-1.57 (m, 10H), 1.68-1.83 (m, 4H), 2.25 (t, J=6.8 Hz, 1H), 2.66-2.76 (m, 2H), 2.97 (t, J=7.8 Hz, 2H), 3.97-4.20 (m, 2H), 5.05 (d, J=6.8 Hz, 2H), 5.18 (s, 2H), 6.93 (d, J=8.8 Hz, 1H), 7.22-7.30 (m, 1H), 7.47 (d, J=8.4 Hz, 1H), 7.48 (d, J=8.8 Hz, 1H), 7.53 (d, J=2.0 Hz, 1H).

### (2) tert-Butyl 4-{2-[6-(3,4-dichlorobenzyloxy)-7-(N,N-dimethylaminomethyl)benz[d]isoxazol-3-yl]ethyl}piperidine-1-carboxylate

The title compound was obtained by synthesis from tert-butyl 4-{2-[6-(3,4-dichlorobenzyloxy)-7-hydroxymethylbenz[d]isoxazol-3-yl]ethyl}piperidine-1-carboxylate according to Example 179-(4).
¹H-NMR (400 MHz, CDCl₃) δ(ppm): 1.08-1.22 (m, 2H), 1.42-1.58 (m, 10H), 1.68-1.82 (m, 4H), 2.33 (s, 6H), 2.55-2.75 (m, 2H), 2.96 (t, J=7.6 Hz, 2H), 3.80 (s, 2H), 3.91-4.22 (m, 2H), 5.16 (s, 2H), 6.93 (d, J=8.8 Hz, 1H), 7.24-7.30 (m, 1H), 7.45 (d, J=8.4 Hz, 1H), 7.46 (d, J=8.8 Hz, 1H), 7.60 (d, J=2.0 Hz, 1H).

### (3) N,N-Dimethyl{6-(3,4-dichlorobenzyloxy)-3-[2-(piperidin-4-yl)ethyl]benz[d]isoxazol-7-yl}methylamine

The title compound was obtained by synthesis from tert-butyl 4-{2-[6-(3,4-dichlorobenzyloxy)-7-(N,N-dimethylaminomethyl)benz[d]isoxazol-3-yl]ethyl}piperidine-1-carboxylate according to Example 179-(5).
¹H-NMR (400 MHz, CDCl₃) δ(ppm): 1.12-1.27 (m, 2H), 1.42-1.55 (m, 1H), 1.63-2.00 (m, 4H), 2.33 (s, 6H), 2.53-2.65 (m, 2H), 2.95 (t, J=7.8 Hz, 2H), 3.02-3.13 (m, 2H), 3.80 (s, 2H), 5.16 (s, 2H), 6.93 (d, J=8.8 Hz, 1H), 7.23-7.30 (m, 1H), 7.45 (d, J=8.4 Hz, 1H), 7.47 (d, J=8.8 Hz, 1H), 7.60 (d, J=2.0 Hz, 1H).

### (4) N,N-Dimethyl{3-[2-(1-benzylpiperidin-4-yl)ethyl]-6-(3,4-dichlorobenzyloxy)benz[d]isoxazol-7-yl}methylamine

The title compound was obtained by synthesis from N,N-dimethyl{6-(3,4-dichlorobenzyloxy)-3-[2-(piperidin-4-yl)ethyl]benz[d]isoxazol-7-yl}methylamine (158 mg) and benzaldehyde according to Example 179-(6). ¹H-NMR (400 MHz, CDCl₃) δ(ppm) : 1.23-1.42 (m, 3H), 1.69-1.81 (m, 4H), 1.89-1.99 (m, 2H), 2.33 (s, 6H), 2.86-2.96 (m, 4H), 3.49 (s, 2H), 3.81 (s, 2H), 5.15 (s, 2H), 6.92 (d, J=8.6 Hz, 1H), 7.21-7.33 (m, 6H), 7.45 (d, J=8.4 Hz, 1H), 7.45 (d, J=8.6 Hz, 1H), 7.60 (d, J=2.0 Hz, 1H).

### (5) N,N-Dimethyl{3-[2-(1-benzylpiperidin-4-yl)ethyl]-6-(3,4-dichlorobenzyloxy)benz[d]isoxazol-7-yl}methylamine dihydrochloride

The title compound was obtained by synthesis from N,N-dimethyl{3-[2-(1-benzylpiperidin-4-yl)ethyl]-6-(3,4-dichlorobenzyloxy)benz[d]isoxazol-7-yl}methylamine according to Example 179-(7).
¹H-NMR (400 MHz, CD₃OD) δ(ppm): 1.46-1.63 (m, 2H), 1.66-1.79 (m, 1H), 1.79-1.94 (m, 2H), 1.97-2.14 (m, 2H), 2.86-3.14 (m, 10H), 3.43-3.56 (m, 2H), 4.31 (s, 2H), 4.65 (s, 2H), 5.38 (s, 2H), 7.31 (d, J=8.6 Hz, 1H), 7.42-7.62 (m, 7H), 7.71-7.77 (m, 1H), 7.94 (d, J=8.6 Hz, 1H).
ESI-MS m/z: 277 [M+2H]²⁺, 552 [M+H]⁺.

### Example 185

### N,N-Dimethyl{3-[2-(1-benzylpiperidin-4-yl)ethyl]-6-(3,6-dihydro-2H-pyran-4-yl)benz[d]isoxazol-7-yl}methylamine dihydrochloride

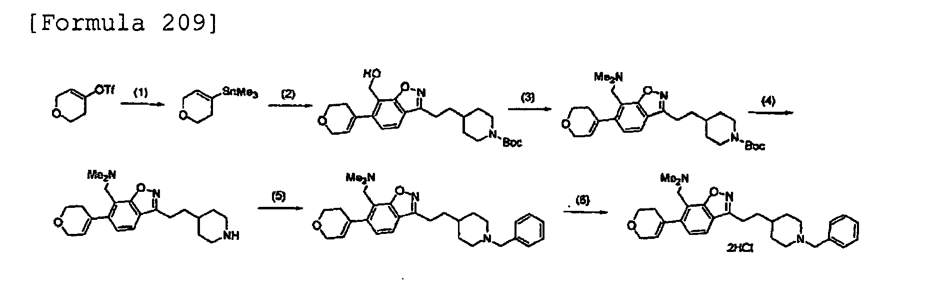

### (1) (3,6-Dihydro-2H-pyran-4-yl)trimethylstannane

3,6-Dihydro-2H-pyran-4-yl trifluoromethanesulfonate [CAS Registry No. 188975-30-6] (5.1 g) was dissolved in tetrahydrofuran (135 mL). Hexamethylditin (7.92 g), lithium chloride (4.68 g) and tetrakistriphenylphosphinepalladium (0) (760 mg) were sequentially added to the solution, and the mixture was heated with stirring at 60°C for 10 hours. The reaction mixture was cooled to room temperature, and then the solvent was evaporated under reduced pressure to reduce the amount of the solvent. The remaining reaction mixture was filtered through celite (washed with hexane). The resulting filtrate was concentrated under reduced pressure to obtain a residue. The residue was filtered through celite and washed with hexane. The filtrate was evaporated under reduced pressure again. This operation was repeated again to obtain the title compound (3.27 g).
¹H-NMR (400 MHz, CDCl₃) δ(ppm): 0.20 (s, 9H), 2.22-2.33 (m, 2H), 3.75-3.82 (m, 2H), 4.10-4.18 (m, 2H), 5.82-5.86 (m, 1H).

### (2) tert-Butyl 4-{2-[6-(3,6-dihydro-2H-pyran-4-yl)-7-hydroxymethylbenz[d]isoxazol-3-yl]ethyl}piperidine-1-carboxylate

tert-Butyl 4-{2-[7-(tert-butyldimethylsilanyloxymethyl)-6-trifluoromethanesulfonyloxybenz[d]isoxazol-3-yl]ethyl}piperidine-1-carboxylate (compound synthesized in Example 79-(2)) (1 g) and (3,6-dihydro-2H-pyran-4-yl)trimethylstannane (1.4 g) were dissolved in xylene (30 mL). Tetrabutylammonium chloride (1.8 g) and tetrakistriphenylphosphinepalladium (0) (190 mg) were sequentially added to the solution, and the mixture was heated with stirring at 140°C for 20 minutes. The reaction mixture was cooled to room temperature. Then, diethyl ether was added and the mixture was filtered through celite to obtain a filtrate. The insoluble matter was dissolved in ethyl acetate. Diethyl ether was added to the solution, and the mixture was filtered through celite to obtain a filtrate. The insoluble matter was dissolved in ethyl acetate again. Diethyl ether was added to the solution, and the mixture was filtered through celite to obtain a filtrate. The above three filtrates were mixed and the solvent was evaporated under reduced pressure. The residue was purified by NH silica gel chromatography (heptane-ethyl acetate) and silica gel chromatography (heptane-ethyl acetate) to obtain a crude product (393 mg).
The crude product synthesized according to the above reaction (407 mg) was dissolved in tetrahydrofuran (10 mL). Tetrabutylammonium fluoride (1 M solution in tetrahydrofuran, 1 mL) was added to the solution, and the mixture was stirred at room temperature for eight hours. The reaction mixture was evaporated under reduced pressure. The residue was purified by silica gel chromatography (heptane-ethyl acetate) to obtain the title compound (331 mg).
¹H-NMR (400 MHz, CDCl₃) δ (ppm) : 1.08-1.23 (m, 2H), 1.40-1.57 (m, 10H), 1.68-1.84 (m, 4H), 2.42-2.50 (m, 2H), 2.58-2.76 (m, 2H), 3.01 (t, J=7.8 Hz, 2H), 3.96 (t, J=5.6 Hz, 2H), 4.00-4.21 (m, 2H), 4.33 (dd, J=2.8 Hz, 5.2 Hz, 2H), 4.99 (s, 2H), 5.83-5.87 (m, 1H), 7.14 (d, J=8.2 Hz, 1H), 7.52 (d, J=8.2 Hz, 1H).

### (3) tert-Butyl 4-{2-[6-(3,6-dihydro-2H-pyran-4-yl)-7-(N,N-dimethylaminomethyl)benz[d]isoxazol-3-yl]ethyl}piperidine-1-carboxylate

The title compound was obtained by synthesis from tert-butyl 4-{2-[6-(3,6-dihydro-2H-pyran-4-yl)-7-hydroxymethylbenz[d]isoxazol-3-yl]ethyl}piperidine-1-carboxylate according to Example 179-(4).
¹H-NMR (400 MHz, CDCl₃) δ(ppm): 1.09-1.23 (m, 2H), 1.41-1.65 (m, 10H), 1.71-1.84 (m, 4H), 2.26 (s, 6H), 2.41-2.48 (m, 2H), 2.60-2.76 (m, 2H), 2.99 (t, J=7.8 Hz, 2H), 3.70 (s, 2H), 3.94 (t, J=5.4 Hz, 2H), 3.98-4.20 (m, 2H), 4.33 (dd, J=2.6 Hz, 5.4 Hz, 2H), 5.83-5.89 (m, 1H), 7.11 (d, J=8.0 Hz, 1H), 7.48 (d, J=8.0 Hz, 1H).

### (4) N,N-Dimethyl[6-(3,6-dihydro-2H-pyran-4-yl)-3-(2-piperidin-4-ylethyl)benz[d]isoxazol-7-yl]methylamine

The title compound was obtained by synthesis from tert-butyl 4-{2-[6-(3,6-dihydro-2H-pyran-4-yl)-7-(N,N-dimethylaminomethyl)benz[d]isoxazol-3-yl]ethyl}piperidine-1-carboxylate according to Example 179-(5).
¹H-NMR (400 MHz, CDCl₃) δ(ppm): 1.13-1.34 (m, 2H), 1.43-1.56 (m, 1H), 1.66-1.91 (m, 4H), 2.26 (s, 6H), 2.42-2.48 (m, 2H), 2.55-2.65 (m, 2H), 2.98 (t, J=7.8 Hz, 2H), 3.05-3.13 (m, 2H), 3.70 (s, 2H), 3.94 (t, J=5.4 Hz, 2H), 4.33 (dd, J=2.6 Hz, 5.4 Hz, 2H), 5.84-5.88 (m, 1H), 7.11 (d, J=8.0 Hz, 1H), 7.49 (d, J=8.0 Hz, 1H).

### (5) N,N-Dimethyl{3-[2-(1-benzylpiperidin-4-yl)ethyl]-6-(3,6-dihydro-2H-pyran-4-yl)benz[d]isoxazol-7-yl}methylamine

The title compound was obtained by synthesis from N,N-dimethyl[6-(3,6-dihydro-2H-pyran-4-yl)-3-(2-piperidin-4-ylethyl)benz[d]isoxazol-7-yl]methylamine and benzaldehyde according to Example 179-(6).
¹H-NMR (400 MHz, CDCl₃) δ(ppm): 1.23-1.42 (m, 3H), 1.69-1.89 (m, 4H), 1.89-1.99 (m, 2H), 2.26 (s, 6H), 2.41-2.48 (m, 2H), 2.85-2.92 (m, 2H), 2.93-3.00 (m, 2H), 3.49 (s, 2H), 3.69 (s, 2H), 3.94 (t, J=5.4 Hz, 2H), 4.32 (dd, J=2.8 Hz, 5.6 Hz, 2H), 5.84-5.87 (m, 1H), 7.10 (d, J=8.0 Hz, 1H), 7.20-7.32 (m, 5H), 7.47 (d, J=8.0 Hz, 1H).

### (6) N,N-Dimethyl{3-[2-(1-benzylpiperidin-4-yl)ethyl]-6-(3,6-dihydro-2H-pyran-4-yl)benz[d]isoxazol-7-yl}methylamine dihydrochloride

The title compound was obtained by synthesis from N,N-dimethyl{3-[2-(1-benzylpiperidin-4-yl)ethyl]-6-(3,6-dihydro-2H-pyran-4-yl)benz[d]isoxazol-7-yl}methylamine according to Example 179-(7).
¹H-NMR (400 MHz, CD₃OD) δ(ppm): 1.48-1.63 (m, 2H), 1.66-1.80 (m, 1H), 1.82-1.94 (m, 2H), 2.06-2.14 (m, 2H), 2.41-2.50 (m, 2H), 2.94 (s, 6H), 2.97-3.08 (m, 2H), 3.12 (t, J=7.8 Hz, 2H), 3.45-3.55 (m, 2H), 3.99 (t, J=5.4 Hz, 2H), 4.26-4.38 (m, 4H), 4.77 (s, 2H), 5.80-5.93 (m, 1H), 7.37 (d, J=8.0 Hz, 1H), 7.46-7.60 (m, 5H), 7.94 (d, J=8.0 Hz, 1H).
ESI-MS m/z: 231 [M+2H]²⁺, 460 [M+H]⁺.

### Example 186

### N,N-Dimethyl(6-cyclopropylmethoxy-3-{2-[1-(3-fluorobenzyl)piperidin-4-yl]ethyl}benz[d]isoxazol-7-yl)methylamine dihydrochloride

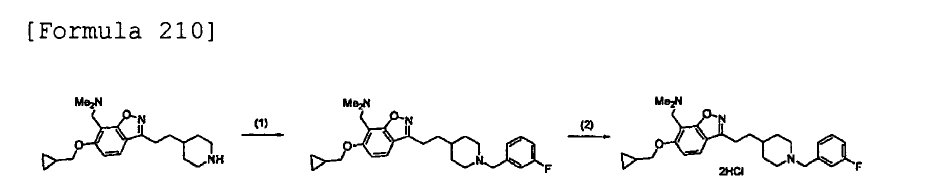

### (1) N,N-Dimethyl{6-cyclopropylmethoxy-3-{2-[1-(3-fluorobenzyl)piperidin-4-yl]ethyl}benz[d]isoxazol-7-yl}methylamine

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3-(3)) and 3-fluorobenzaldehyde according to Example 179-(6).
¹H-NMR (400 MHz, CDCl₃) δ (ppm) : 0.35-0.40 (m, 2H), 0.61-0.68 (m, 2H), 1.28-1.44 (m, 4H), 1.70-1.89 (m, 4H), 1.89-2.00 (m, 2H), 2.33 (s, 6H), 2.92-2.89 (m, 2H), 2.93 (t, J=7.8 Hz, 2H), 3.46 (s, 2H), 3.82 (s, 2H), 3.93 (d, J=6.8 Hz, 2H), 6.88 (d, J=8.8 Hz, 1H), 6.88-6.94 (m, 1H), 7.02-7.08 (m, 2H), 7.21-7.26 (m, 1H), 7.43 (d, J=8.8 Hz, 1H).

### (2) N,N-Dimethyl{6-cyclopropylmethoxy-3-{2-[1-(3-fluorobenzyl)piperidin-4-yl]ethyl}benz[d]isoxazol-7-yl}methylamine dihydrochloride

The title compound was obtained by synthesis from N,N-dimethyl(6-cyclopropylmethoxy-3-{2-[1-(3-fluorobenzyl)piperidin-4-yl]ethyl}benz[d]isoxazol-7-yl)methylamine according to Example 179-(7).
¹H-NMR (400 MHz, CD₃OD) δ (ppm) : 0.38-0.51 (m, 2H), 0.62-0.76 (m, 2H), 1.32-1.47 (m, 1H), 1.48-1.64 (m, 2H), 1.64-1.80 (m, 1H), 1.80-1.96 (m, 2H), 1.96-2.13 (m, 2H), 2.88-3.13 (m, 10H), 3.44-3.55 (m, 2H), 4.12 (d, J=7.2 Hz, 2H), 4.24 (s, 2H), 4.63 (s, 2H), 7.19-7.28 (m, 2H), 7.38-7.44 (m, 2H), 7.46-7.56 (m, 1H), 7.92 (d, J=8.8 Hz, 1H).
ESI-MS m/z: 234 [M+2H]²⁺, 466 [M+H]⁺.

### Example 187

### N,N-Dimethyl{6-(3,4-dichlorobenzyloxy)-3-{2-[1-(1,3-dioxolan-2-ylmethyl)piperidin-4-yl]ethyl}benz[d]isoxazol-7-yl}methylamine difumarate

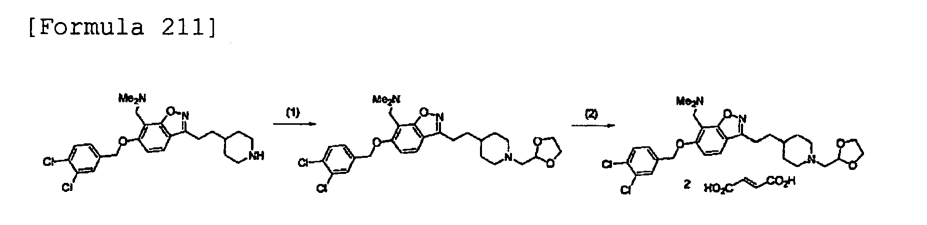

### (1) N,N-Dimethyl{6-(3,4-dichlorobenzyloxy)-3-{2-[1-(1,3-dioxolan-2-ylmethyl)piperidin-4-yl]ethyl}benz[d]isoxazol-7-yl}methylamine

N,N-Dimethyl{6-(3,4-dichlorobenzyloxy)-3-[2-(piperidin-4-yl)ethyl]benz[d]isoxazol-7-yl}methylamine (compound synthesized in Example 184-(3)) (222 mg) was dissolved in propionitrile (5 mL). N,N-Diisopropylethylamine (510 µL), 2-bromomethyl-1,3-dioxolane (150 µL) and sodium iodide (72 mg) were added to the solution, and the mixture was heated with stirring at 90°C for 1.5 days. The reaction mixture was cooled to room temperature. Then, a saturated sodium chloride solution was added, followed by extraction with ethyl acetate twice. The organic layers were washed with water and a saturated sodium chloride solution and dried over magnesium sulfate. The drying agent was removed by filtration and then the solvent was evaporated under reduced pressure. The residue was purified by NH silica gel chromatography (heptane-ethyl acetate) to obtain the title compound (115 mg).
¹H-NMR (400 MHz, CDCl₃) δ (ppm) : 1.23-1.43 (m, 2H), 1.69-1.84 (m, 4H), 2.00-2.12 (m, 2H), 2.33 (s, 6H), 2.56 (d, J=4.4 Hz, 2H), 2.89-3.04 (m, 4H), 3.80 (s, 2H), 3.82-3.89 (m, 2H), 3.91-4.00 (m, 2H), 5.00 (t, J=4.4 Hz, 1H), 5.15 (s, 2H), 6.93 (d, J=8.6 Hz, 1H), 7.24-7.30 (m, 1H), 7.45 (d, J=8.4 Hz, 1H), 7.46 (d, J=8.6 Hz, 1H), 7.58-7.62 (m, 1H).

### (2) N,N-Dimethyl{6-(3,4-dichlorobenzyloxy)-3-{2-[1-(1,3-dioxolan-2-ylmethyl)piperidin-4-yl]ethyl}benz[d]isoxazol-7-yl}methylamine difumarate

The title compound was obtained by synthesis from N,N-dimethyl{6-(3,4-dichlorobenzyloxy)-3-{2-[1-(1,3-dioxolan-2-ylmethyl)piperidin-4-yl]ethyl}benz[d]isoxazol-7-yl}methylamine according to Example 182-(4).
¹H-NMR (400 MHz, CD₃OD) δ (ppm) : 1.44-1.65 (m, 3H), 1.76-1.88 (m, 2H), 1.88-1.99 (m, 2H), 2.63-2.79 (m, 8H), 2.98-3.09 (m, 4H), 3.36-3.48 (m, 2H), 3.87-3.95 (m, 2H), 3.96-4.04 (m, 2H), 4.35-4.45 (m, 2H), 5.11-5.17 (m, 1H), 5.32 (s, 2H), 6.65 (s, 2H), 7.26 (d, J=8.8 Hz, 1H), 7.46 (dd, J=2.0 Hz, 8.0 Hz, 1H), 7.57 (d, J=8.0 Hz, 1H), 7.72 (d, J=2.0 Hz, 1H), 7.85 (d, J=8.8 Hz, 1H).
ESI-MS m/z: 275 [M+2H]²⁺, 548 [M+H]⁺.

### Example 188

### 4-{(Z)-2-{3-[2-(1-Benzylpiperidin-4-yl)ethyl]-7-(N,N-dimethylaminomethyl)benz[d]isoxazol-6-yl}vinyl}benzonitrile dihydrochloride

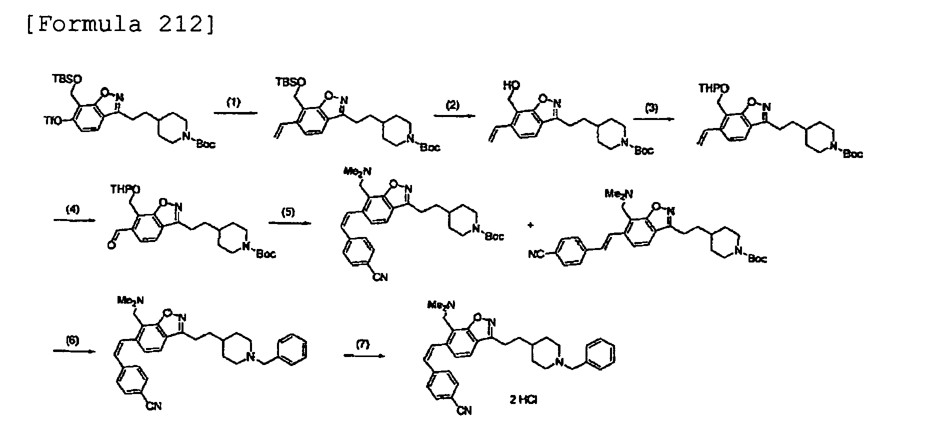

### (1) tert-Butyl 4-[2-(7-tert-butyldimethylsilanyloxymethyl-6-vinylbenz[d]isoxazol-3-yl)ethyl]piperidine-1-carboxylate

tert-Butyl 4-{2-[7-(tert-butyldimethylsilanyloxymethyl)-6-trifluoromethanesulfonyloxybenz[d]isoxazol-3-yl]ethyl}piperidine-1-carboxylate (compound synthesized in Preparation Example 79-(2)) (4 g) was dissolved in N-methylpyrrolidin-2-one (100 mL). Lithium chloride (800 mg), 2,6-di-tert-butyl-4-methylphenol (134 mg), vinyltributyltin (3.8 mL) and bis(triphenylphosphine)palladium (II) dichloride (460 mg) were sequentially added to the solution, and the mixture was heated with stirring at 100°C for 30 minutes. The reaction mixture was cooled to room temperature and then water was added, followed by extraction with ethyl acetate twice. The organic layers were washed with water and a saturated sodium chloride solution and then dried over magnesium sulfate. The drying agent was removed by filtration and then the solvent was evaporated under reduced pressure. The residue was purified by NH silica gel chromatography (heptane-ethyl acetate) and silica gel chromatography (heptane-ethyl acetate) to obtain the title compound (2.86 g).
¹H-NMR (400 MHz, CDCl₃) δ (ppm) : 0.09 (s, 6H), 0.89 (s, 9H), 1.09-1.23 (m, 2H), 1.44-1.59 (m, 10H), 1.69-1.86 (m, 4H), 2.58-2.78 (m, 2H), 2.99 (t, J=7.8 Hz, 2H), 3.93-4.24 (m, 2H), 5.06 (s, 2H), 5.47 (dd, J=1.2 Hz, 11.2 Hz, 1H), 5.83 (dd, J=1.2 Hz, 17.6 Hz, 1H), 7.21-7.30 (m, 1H), 7.48-7.50 (m, 2H).

### (2) tert-Butyl 4-[2-(7-hydroxymethyl-6-vinylbenz[d]isoxazol-3-yl)ethyl]piperidine-1-carboxylate

tert-Butyl 4-[2-(7-tert-butyldimethylsilanyloxymethyl-6-vinylbenz[d]isoxazol-3-yl)ethyl]piperidine-1-carboxylate (2.86 g) was dissolved in tetrahydrofuran (20 mL). Tetrabutylammonium fluoride (1 M solution in tetrahydrofuran, 9 mL) was added to the solution, and the mixture was stirred at room temperature overnight. The solvent was evaporated under reduced pressure. The residue was purified by silica gel chromatography (heptane-ethyl acetate) to obtain the title compound (2.22 g).
¹H-NMR (400 MHz, CDCl₃) δ (ppm): 1.09-1.23 (m, 2H), 1.42-1.58 (m, 10H), 1.69-1.83 (m, 4H), 1.88-1.98 (m, 1H), 2.68-2.77 (m, 2H), 3.00 (t, J=7.8 Hz, 2H), 3.96-4.23 (m, 2H), 5.09 (d, J=4.8 Hz, 2H), 5.53(dd, J=0.8 Hz, 11.2 Hz, 1H), 5.85 (dd, J=0.8 Hz, 17.4 Hz, 1H), 7.17-7.26 (m, 1H), 7.47-7.54 (m, 2H).

### (3) tert-Butyl 4-{2-[7-(tetrahydro-2H-pyran-2-yloxymethyl)-6-vinylbenz[d]isoxazol-3-yl]ethyl}piperidine-1-carboxylate

tert-Butyl 4-[2-(7-hydroxymethyl-6-vinylbenz[d]isoxazol-3-yl)ethyl]piperidine-1-carboxylate (2.22 g) was dissolved in dichloromethane (50 mL), and 3,4-dihydro-2H-pyrane (790 µL) and pyridinium p-toluenesulfonate (145 mg) were sequentially added. The reaction mixture was stirred at room temperature for 19 hours. Then, 3,4-dihydro-2H-pyrane (800 µL) and (1R)-(-)-10-camphorsulfonic acid (70 mg) were added and the mixture was further stirred at room temperature for one hour. A saturated sodium bicarbonate solution was added to the reaction mixture, followed by extraction with chloroform twice. The organic layers were washed with a saturated sodium chloride solution and then dried over magnesium sulfate. The drying agent was removed by filtration and then the solvent was evaporated under reduced pressure. The residue was purified by NH silica gel chromatography (heptane-ethyl acetate) and silica gel chromatography (heptane-ethyl acetate) to obtain the title compound (2.76 g).
¹H-NMR (400 MHz, CDCl₃) δ(ppm): 1.08-1.23 (m, 2H), 1.42-1.90 (m, 20H), 2.57-2.77 (m, 2H), 2.99 (t, J=7.8 Hz, 2H), 3.46-3.65 (m, 1H), 3.83-4.25 (m, 3H), 4.82 (t, J=3.4 Hz, 1H), 4.86 (d, J=11.4 Hz, 1H), 5.13 (d, J=11.4 Hz, 1H), 5.50 (dd, J=0.8 Hz, 11.2 Hz, 1H), 5.84 (dd, J=0.8 Hz, 17.6 Hz, 1H), 7.18-7.26 (m, 1H), 7.48-7.53 (m, 2H).

### (4) tert-Butyl 4-{2-[6-formyl-7-(tetrahydro-2H-pyran-2-yloxymethyl)benz[d]isoxazol-3-yl]ethyl}piperidine-1-carboxylate

tert-Butyl 4-{2-[7-(tetrahydro-2H-pyran-2-yloxymethyl)-6-vinylbenz[d]isoxazol-3-yl]ethyl}piperidine-1-carboxylate (2.76 g) was dissolved in tetrahydrofuran (50 mL) and water (12.5 mL). Osmium tetroxide (2.5% aqueous solution, 2 mL) and sodium periodate (3.2 g) were sequentially added to the solution, and the mixture was stirred at room temperature for one hour. Ethyl acetate and water were added to the reaction mixture, followed by extraction with ethyl acetate three times. The organic layers were washed with water and a saturated sodium chloride solution and then dried over magnesium sulfate. The drying agent was removed by filtration and then the solvent was evaporated under reduced pressure. The residue was purified by silica gel chromatography (heptane-ethyl acetate) to obtain the title compound (2.48 g).
¹H-NMR (400 MHz, CDCl₃) δ (ppm) : 1.10-1.24 (m, 2H), 1.38-1.86 (m, 20H), 2.58-2.76 (m, 2H), 3.04 (t, J=7.8 Hz, 2H), 3.56-3.64 (m, 1H), 3.87-3.96 (m, 1H), 3.98-4.23 (m, 2H), 4.86 (t, J=3.2 Hz, 1H), 5.17 (d, J=11.8 Hz, 1H), 5.45 (d, J=11.8 Hz, 1H), 7.68 (d, J=8.2 Hz, 1H), 7.89 (d, J=8.2 Hz, 1H), 10.59 (s, 1H).

### (5) tert-Butyl 4-{2-{6-[(Z)-2-(4-cyanophenyl)vinyl]-7-(N,N-dimethylaminomethyl)benz[d]isoxazol-3-yl}ethyl}piperidine-1-carboxylate

4-Cyanobenzyltriphenylphosphonium chloride (450 mg) was dissolved in tetrahydrofuran (4 mL), and the reaction mixture was ice-cooled. Potassium tert-butoxide (130 mg) was added and the mixture was stirred for 25 minutes. A solution of tert-butyl 4-{2-[6-formyl-7-(tetrahydro-2H-pyran-2-yloxymethyl)benz[d]isoxazol-3-yl]ethyl}piperidine-1-carboxylate (336 mg) in tetrahydrofuran (2 mL) was added to the reaction mixture. After stirring for 1.5 hours, a saturated ammonium chloride solution was added and the mixture was returned to room temperature. The reaction mixture was extracted with ethyl acetate twice. The organic layers were washed with a saturated sodium chloride solution and then dried over magnesium sulfate. The drying agent was removed by filtration and then the solvent was evaporated under reduced pressure. The residue was subjected to NH silica gel chromatography (heptane-ethyl acetate) and silica gel chromatography (heptane-ethyl acetate) to obtain a crude product of tert-butyl 4-{2-{6-[(Z)-2-(4-cyanophenyl)vinyl]-7-(tetrahydro-2H-pyran-2-yloxymethyl)benz[d]isoxazol-3-yl}ethyl}piperidine-1-carboxylate. The total amount of the crude product was dissolved in methanol (20 mL). Pyridinium p-toluenesulfonate (15 mg) was added to the solution, and the mixture was heated with stirring at 60°C for two hours. The reaction mixture was cooled to room temperature. Then, a saturated sodium bicarbonate solution was added, followed by extraction with ethyl acetate three times. The organic layers were washed with a saturated sodium chloride solution and then dried over magnesium sulfate. The drying agent was removed by filtration and then the solvent was evaporated under reduced pressure. The residue was purified by silica gel chromatography (heptane-ethyl acetate) to obtain a crude product of tert-butyl 4-{2-{6-[(Z)-2-(4-cyanophenyl)vinyl]-7-hydroxymethylbenz[d]isoxazol-3-yl}ethyl}piperidine-1-carboxylate. The total amount of the crude product was dissolved in tetrahydrofuran (8 mL). Triethylamine (250 µL) was added to the solution, and the reaction mixture was ice-cooled. Methanesulfonyl chloride (65 µL) was added dropwise to the reaction mixture, and the mixture was stirred at the same temperature for five minutes. Then, dimethylamine (2 M solution in tetrahydrofuran, 3 mL) and sodium iodide (7 mg) were added. The reaction mixture was stirred at room temperature for 30 minutes. A saturated sodium chloride solution was added to the reaction mixture, followed by extraction with ethyl acetate three times. The organic layers were washed with a mixed solution of a saturated sodium bicarbonate solution and a saturated sodium chloride solution and then dried over magnesium sulfate. The drying agent was removed by filtration and then the solvent was evaporated under reduced pressure. The residue was purified by NH silica gel chromatography (heptane-ethyl acetate) and silica gel chromatography (heptane-ethyl acetate-chloroform-methanol) to obtain the title compound (121 mg) and tert-butyl 4-{2-{6-[(E)-2-(4-cyanophenyl)vinyl]-7-(N,N-dimethylaminomethyl)benz[d]isoxazol-3-yl}ethyl}piperidine-1-carboxylate (93 mg). Title compound: tert-Butyl 4-{2-{6-[(Z)-2-(4-cyanophenyl)vinyl]-7-(N,N-dimethylaminomethyl)benz[d]isoxazol-3-yl}ethyl}piperidine-1-carboxylate
¹H-NMR (400 MHz, CDCl₃) δ (ppm): 1.10-1.23 (m, 2H), 1.40-1.62 (m, 10H), 1.70-1.85 (m, 4H), 2.30 (s, 6H), 2.58-2.78 (m, 2H), 2.98 (t, J=7.8 Hz, 2H), 3.75 (s, 2H), 3.96-4.24 (m, 2H), 6.74 (d, J=12.4 Hz, 1H), 7.00 (d, J=8.0 Hz, 1H), 7.13-7.19 (m, 3H), 7.33 (d, J=8.0 Hz, 1H), 7.40-7.44 (m, 2H).
By-product: tert-Butyl 4-{2-{6-[(E)-2-(4-cyanophenyl)vinyl]-7-(N,N-dimethylaminomethyl)benz[d]isoxazol-3-yl}ethyl}piperidine-1-carboxylate
¹H-NMR (400 MHz, CDCl₃) δ(ppm): 1.10-1.29 (m, 2H), 1.40-1.62 (m, 10H), 1.71-1.97 (m, 4H), 2.33 (s, 6H), 2.60-2.76 (m, 2H), 3.01 (t, J=7.8 Hz, 2H), 3.85 (s, 2H), 3.98-4.24 (m, 2H), 7.16 (d, J=16.4 Hz, 1H), 7.53 (d, J=8.0 Hz, 1H), 7.58-7.69 (m, 5H), 7.89 (d, J=16.4 Hz, 1H).

### (6) 4-{(Z)-2-{3-[2-(1-Benzylpiperidin-4-yl)ethyl]-7-(N,N-dimethylaminomethyl)benz[d]isoxazol-6-yl}vinyl}benzonitrile

tert-Butyl 4-{2-{6-[(Z)-2-(4-cyanophenyl)vinyl]-7-(N,N-dimethylaminomethyl)benz[d]isoxazol-3-yl}ethyl}piperidine-1-carboxylate (121 mg) was dissolved in methanol (5 mL), and the solution was ice-cooled. Hydrogen chloride (4 M solution in ethyl acetate, 1.4 mL) was added to the solution, and then the mixture was gradually heated to room temperature. After nine hours, toluene (5 mL) was added to the reaction mixture, and then the solvent was evaporated under reduced pressure. The residue was diluted with chloroform and neutralized with a 2 N sodium hydroxide solution. Sodium chloride was added to the mixture, followed by extraction with chloroform three times. The organic layers were dried over magnesium sulfate. The drying agent was removed by filtration and then the solvent was evaporated under reduced pressure. The residue was dissolved in dichloromethane (5 mL). Benzaldehyde (36 µL), acetic acid (20 µL) and sodium triacetoxyborohydride (110 mg) were sequentially added to the solution, and the mixture was stirred at room temperature overnight. Ethyl acetate and a mixed solution of 2 N hydrochloric acid and a saturated sodium chloride solution were sequentially added to the reaction mixture, followed by extraction with 2 N hydrochloric acid twice. The extracted aqueous layer was made basic with a 5 N sodium hydroxide solution, followed by extraction with ethyl acetate three times. The organic layers were washed with a saturated sodium chloride solution and then dried over magnesium sulfate. The drying agent was removed by filtration and then the solvent was evaporated under reduced pressure. The residue was purified by NH silica gel chromatography (heptane-ethyl acetate) to obtain the title compound (79 mg; containing 4-{(E)-2-{3-[2-(1-benzylpiperidin-4-yl)ethyl]-7-(N,N-dimethylaminomethyl)benz[d]isoxazol-6-yl}vinyl}benzonitrile as an impurity).
¹H-NMR (400 MHz, CDCl₃) δ (ppm) : 1.23-1.44 (m, 3H), 1.70-1.83 (m, 4H), 1.88-2.07 (m, 2H), 2.29 (s, 6H), 2.86-3.00 (m, 4H), 3.49 (s, 2H), 3.74 (s, 2H), 6.73 (d, J=12.4 Hz, 1H), 6.98 (d, J=8.0 Hz, 1H), 7.16 (d, J=8.0 Hz, 1H), 7.16 (d, J=12.4 Hz, 1H), 7.20-7.34 (m, 6H), 7.40-7.44 (m, 2H).

### (7) 4-{(Z)-2-{3-[2-(1-Benzylpiperidin-4-yl)ethyl]-7-(N,N-dimethylaminomethyl)benz[d]isoxazol-6-yl}vinyl}benzonitrile dihydrochloride

The title compound (containing 4-{(E)-2-{3-[2-(1-benzylpiperidin-4-yl)ethyl]-7-(N,N-dimethylaminomethyl)benz[d]isoxazol-6-yl}vinyl}benzonitrile dihydrochloride as an impurity) was obtained by synthesis from 4-{(Z)-2-{3-[2-(1-benzylpiperidin-4-yl)ethyl]-7-(N,N-dimethylaminomethyl)benz[d]isoxazol-6-yl}vinyl}benzonitrile according to Example 179-(7).
¹H-NMR (400 MHz, CD₃OD) δ (ppm): 1.46-1.64 (m, 2H), 1.68-1.94 (m, 3H), 2.02-2.14 (m, 2H), 2.94 (s, 6H), 2.97-3.17 (m, 4H), 3.40-3.57 (m, 2H), 4.32 (s, 2H), 4.62 (s, 2H), 7.05 (d, J=12.4 Hz, 1H), 7.18 (d, J=12.4 Hz, 1H), 7.47-7.57 (m, 7H), 7.83 (d, J=8.0 Hz, 1H).
ESI-MS m/z: 505 [M+H]⁺.

### Example 189

### 4-{(E)-2-{3-[2-(1-Benzylpiperidin-4-yl)ethyl]-7-(N,N-dimethylaminomethyl)benz[d]isoxazol-6-yl}vinyl}benzonitrile dihydrochloride

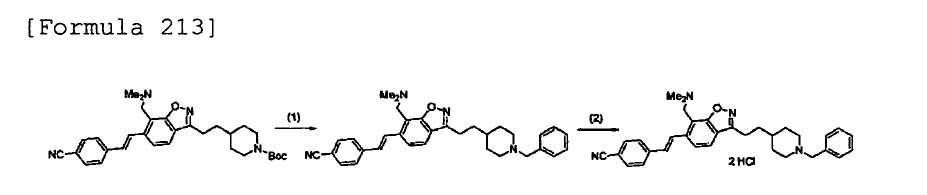

### (1) 4-{(E)-2-{3-[2-(1-Benzylpiperidin-4-yl)ethyl]-7-(N,N-dimethylaminomethyl)benz[d]isoxazol-6-yl}vinyl}benzonitrile

The title compound (containing 4-{(Z)-2-{3-[2-(1-benzylpiperidin-4-yl)ethyl]-7-(N,N-dimethylaminomethyl)benz[d]isoxazol-6-yl}vinyl}benzonitrile as an impurity) was obtained by synthesis from tert-butyl 4-{2-{6-[(E)-2-(4-cyanophenyl)vinyl]-7-(N,N-dimethylaminomethyl)benz[d]isoxazol-3-yl}ethyl}piperidine-1-carboxylate (compound synthesized in Example 188-(5)) according to Example 182-(3).
¹H-NMR (400 MHz, CDCl₃) δ (ppm) : 1.24-1.44 (m, 3H), 1.71-1.91 (m, 4H), 1.91-2.01 (m, 2H), 2.32 (s, 6H), 2.86-3.02 (m, 4H), 3.49 (s, 2H), 3.84 (s, 2H), 7.15 (d, J=16.4 Hz, 1H), 7.22-7.32 (m, 5H), 7.52 (d, J=8.4 Hz, 1H), 7.58-7.68 (m, 5H), 7.88 (d, J=16.4 Hz, 1H).

### (2) 4-{(E)-2-{3-[2-(1-Benzylpiperidin-4-yl)ethyl]-7-(N,N-dimethylaminomethyl)benz[d]isoxazol-6-yl}vinyl}benzonitrile dihydrochloride

The title compound (containing 4-{(Z)-2-{3-[2-(1-benzylpiperidin-4-yl)ethyl]-7-(N,N-dimethylaminomethyl)benz[d]isoxazol-6-yl}vinyl}benzonitrile dihydrochloride as an impurity) was obtained by synthesis from 4-{(E)-2-{3-[2-(1-benzylpiperidin-4-yl)ethyl]-7-(N,N-dimethylaminomethyl)benz[d]isoxazol-6-yl}vinyl}benzonitrile according to Example 179-(7).
¹H-NMR (400 MHz, CD₃OD) δ (ppm) : 1.46-1.64 (m, 2H), 1.66-1.96 (m, 3H), 2.03-2.16 (m, 2H), 2.86 (s, 6H), 2.94-3.18 (m, 4H), 3.40-3.58 (m, 2H), 4.31 (s, 2H), 4.76 (s, 2H), 7.46-7.56 (m, 6H), 7.74-7.79 (m, 2H), 7.83 (d, J=16.4 Hz, 1H), 7.87-7.97 (m, 4H).
ESI-MS m/z: 505 [M+H]⁺,

### Example 190

### N,N-Dimethyl{3-[2-(1-benzylpiperidin-4-yl)ethyl]-6-[(E)-2-cyclopropylvinyl]benz[d]isoxazol-7-yl}methylamine dihydrochloride

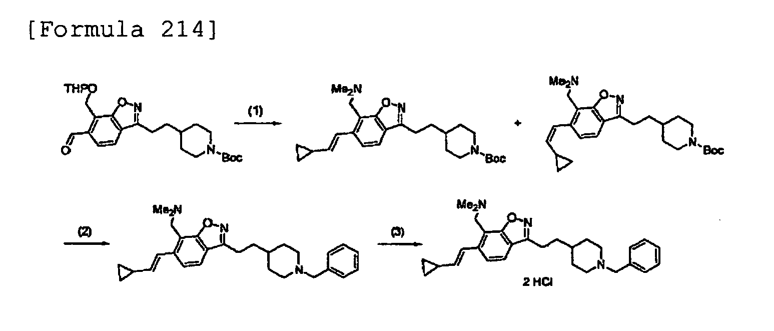

### (1) tert-Butyl 4-{2-{6-[(E)-2-cyclopropylvinyl]-7-(N,N-dimethylaminomethyl)benz[d]isoxazol-3-yl}ethyl}piperidine-1-carboxylate

The title compound and tert-butyl 4-{2-{6-[(Z)-2-cyclopropylvinyl]-7-(N,N-dimethylaminomethyl)benz[d]isoxazol-3-yl}ethyl}piperidine-1-carboxylate (containing tert-butyl 4-{2-{6-[(E)-2-cyclopropylvinyl]-7-(N,N-dimethylaminomethyl)benz[d]isoxazol-3-yl}ethyl}piperidine-1-carboxylate as an impurity) were obtained by synthesis from tert-butyl 4-{2-[6-formyl-7-(tetrahydropyran-2-yloxymethyl)benz[d]isoxazol-3-yl]ethyl}piperidine-1-carboxylate (compound synthesized in Example 188-(4)) and cyclopropylmethyltriphenylphosphonium bromide [CAS Registry No. 14799-82-7] according to Example 188-(5). Title compound: tert-Butyl 4-{2-{6-[(E)-2-cyclopropylvinyl]-7-(N,N-dimethylaminomethyl)benz[d]isoxazol-3-yl}ethyl}piperidine-1-carboxylate
¹H-NMR (400 MHz, CDCl₃) δ (ppm) : 0.50-0.63 (m, 2H), 0.81-0.94 (m, 2H), 1.08-1.23 (m, 2H), 1.39-1.57 (m, 10H), 1.60-1.94 (m, 5H), 2.30 (s, 6H), 2.56-2.77 (m, 2H), 2.96 (t, J=7.8 Hz, 2H), 3.75 (s, 2H), 3.94-4.22 (m, 2H), 5.80 (dd, J=9.2 Hz, 15.6 Hz, 1H), 7.01 (d, J=15.6 Hz, 1H), 7.37-7.44 (m, 2H).
By-product: tert-Butyl 4-{2-{6-[(Z)-2-cyclopropylvinyl]-7-(N,N-dimethylaminomethyl)benz[d]isoxazol-3-yl}ethyl}piperidine-1-carboxylate
¹H-NMR (400 MHz, CDCl₃) δ (ppm): 0.44-0.53 (m, 2H), 0.72-0.83 (m, 2H), 1.05-1.31 (m, 2H), 1.36-2.03 (m, 15H), 2.29 (s, 6H), 2.58-2.76 (m, 2H), 2.99 (t, J=7.8 Hz, 2H), 3.76 (s, 2H), 3.94-4.23 (m, 2H), 5.20 (dd, J=10.0 Hz, 11.2 Hz, 1H), 6.68 (d, J=11.2 Hz, 1H), 7.42-7.50 (m, 2H).

### (2) N,N-Dimethyl{3-[2-(1-benzylpiperidin-4-yl)ethyl]-6-[(E)-2-cyclopropylvinyl]benz[d]isoxazol-7-yl}methylamine

The title compound was obtained by synthesis from tert-butyl 4-{2-{6-[(E)-2-cyclopropylvinyl]-7-(N,N-dimethylaminomethyl)benz[d]isoxazol-3-yl}ethyl}piperidine-1-carboxylate according to Example 182-(3).
¹H-NMR (400 MHz, CDCl₃) δ (ppm): 0.52-0.60 (m, 2H), 0.81-0.92 (m, 2H), 1.20-1.43 (m, 3H), 1.62-1.83 (m, 5H), 1.86-2.10 (m, 2H), 2.30 (s, 6H), 2.84-2.98 (m, 4H), 3.49 (s, 2H), 3.75 (s, 2H), 5.80 (dd, J=9.2 Hz, 15.8 Hz, 1H), 7.00 (d, J=15.8 Hz, 1H), 7.19-7.32 (m, 5H), 7.37-7.42 (m, 2H).

### (3) N,N-Dimethyl{3-[2-(1-benzylpiperidin-4-yl)ethyl]-6-[(E)-2-cyclopropylvinyl]benz[d]isoxazol-7-yl}methylamine dihydrochloride

The title compound was obtained by synthesis from N,N-dimethyl{3-[2-(1-benzylpiperidin-4-yl)ethyl]-6-[(E)-2-cyclopropylvinyl]benz[d]isoxazol-7-yl}methylamine according to Example 179-(7).
¹H-NMR (400 MHz, CD₃OD) δ (ppm): 0.62-0.71 (m, 2H), 0.90-0.99 (m, 2H), 1.47-1.66 (m, 2H), 1.66-1.93 (m, 4H), 2.01-2.13 (m, 2H), 2.89 (s, 6H), 2.94-3.23 (m, 4H), 3.40-3.55 (m, 2H), 4.30 (s, 2H), 4.69 (s, 2H), 6.06 (dd, J=9.2 Hz, 15.2 Hz, 1H), 6.99 (d, J=15.2 Hz, 1H), 7.45-7.57 (m, 5H), 7.65 (d, J=8.4 Hz, 1H), 7.82 (d, J=8.4 Hz, 1H).
ESI-MS m/z: 223 [M+2H]²⁺, 444 [M+H]⁺.

### Example 191

### N,N-Dimethyl{3-[2-(1-benzylpiperidin-4-yl)ethyl]-6-[(Z)-2-cyclopropylvinyl]benz[d]isoxazol-7-yl}methylamine dihydrochloride

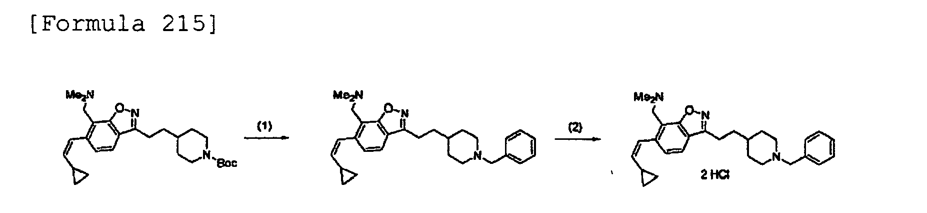

### (1) N,N-Dimethyl{3-[2-(1-benzylpiperidin-4-yl)ethyl]-6-[(Z)-2-cyclopropylvinyl]benz[d]isoxazol-7-yl}methylamine

The title compound (containing N,N-dimethyl{3-[2-(1-benzylpiperidin-4-yl)ethyl]-6-[(E)-2-cyclopropylvinyl]benz[d]isoxazol-7-yl}methylamine as an impurity) was obtained by synthesis from tert-butyl 4-{2-{6-[(Z)-2-cyclopropylvinyl]-7-(N,N-dimethylaminomethyl)benz[d]isoxazol-3-yl}piperidine-1-carboxylate (compound synthesized in Example 190-(1)) according to Example 182-(3).
¹H-NMR (400 MHz, CDCl₃) δ(ppm): 0.46-0.52 (m, 2H), 0.75-0.82 (m, 2H), 1.22-1.44 (m, 3H), 1.57-1.82 (m, 5H), 1.82-2.00 (m, 2H), 2.28 (s, 6H), 2.84-3.00 (m, 4H), 3.48 (s, 2H), 3.75 (s, 2H), 5.19 (dd, J=10.0 Hz, 11.2 Hz, 1H), 6.68 (d, J=11.2 Hz, 1H), 7.20-7.32 (m, 5H), 7.43-7.48 (m, 2H).

### (2) N,N-Dimethyl{3-[2-(1-benzylpiperidin-4-yl)ethyl]-6-[(Z)-2-cyclopropylvinyl]benz[d]isoxazol-7-yl}methylamine dihydrochloride

The title compound (containing N,N-dimethyl{3-[2-(1-benzylpiperidin-4-yl)ethyl]-6-[(E)-2-cyclopropylvinyl]benz[d]isoxazol-7-yl}methylamine dihydrochloride as an impurity) was obtained by synthesis from N,N-dimethyl{3-[2-(1-benzylpiperidin-4-yl)ethyl]-6-[(Z)-2-cyclopropylvinyl]-benz[d]isoxazol-7-yl}methylamine according to Example 179-(7).
¹H-NMR (400 MHz, CD₃OD) δ (ppm) : 0.53-0.61 (m, 2H), 0.80-0.88 (m, 2H), 1.44-1.95 (m, 6H), 2.03-2.14 (m, 2H), 2.90 (s, 6H), 2.94-3.15 (m, 4H), 3.38-3.57 (m, 2H), 4.31 (s, 2H), 4.68 (s, 2H), 5.44 (dd, J=10.6 Hz, 11.2 Hz, 1H), 6.66 (d, J=11.2 Hz, 1H), 7.46-7.60 (m, 6H), 7.93 (d, J=8.4 Hz, 1H).
ESI-MS m/z: 223 [M+2H]²⁺, 444 [M+H]⁺.

### Example 192

### 2-{4-{2-[6-Cyclopropylmethoxy-7-(N,N-dimethylaminomethyl)benz[d]isoxazol-3-yl]ethyl}piperidinomethyl}thiophene-3-carbonitrile dihydrochloride

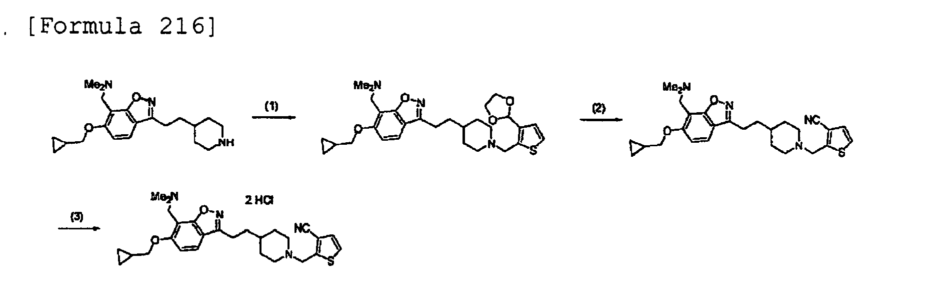

### (1) N,N-Dimethyl{6-cyclopropylmethoxy-3-{2-{1-[3-(1,3-dioxolan-2-yl)thiophen-2-ylmethyl]piperidin-4-yl}ethyl}benz[d]isoxazol-7-yl}methylamine

The title compound was obtained by synthesis from N,N-dimethyl{6-cyclopropylmethoxy-3-[2-(piperidin-4-ylethyl)benz[d]isoxazol-7-yl]methylamine (compound synthesized in Preparation Example 3) and 3-(1,3-dioxolan-2-yl)thiophene-2-carbaldehyde [CAS Registry No. 13250-83-4] according to Example 179-(6).
¹H-NMR (400 MHz, CDCl₃) δ (ppm) : 0.32-0.41 (m, 2H), 0.59-0.69 (m, 2H), 0.84-0.95 (m, 1H), 1.20-1.44 (m, 3H), 1.54-2.13 (m, 6H), 2.34 (s, 6H), 2.88-3.00 (m, 4H), 3.73 (s, 2H), 3.83 (s, 2H), 3.93 (d, J=6.8 Hz, 2H), 3.96-4.06 (m, 2H), 4.06-4.17 (m, 2H), 5.93 (s, 2H), 6.88 (d, J=8.4 Hz, 1H), 7.04 (d, J=5.4 Hz, 1H), 7.15 (d, J=5.4 Hz, 1H), 7.43 (d, J=8.4 Hz, 1H).

### (2) 2-{4-{2-[6-Cyclopropylmethoxy-7-(N,N-dimethylaminomethyl)benz[d]isoxazol-3-yl]ethyl}piperidinomethyl}thiophene-3-carbonitrile

N,N-Dimethyl{6-cyclopropylmethoxy-3-{2-{1-[3-(1,3-dioxolan-2-yl)thiophen-2-ylmethyl]piperidin-4-yl}ethyl}benz[d]isoxazol-7-yl}methylamine (54 mg) was dissolved in acetone (4 mL). 5 N hydrochloric acid (2 mL) was added to the solution, and the mixture was heated with stirring at 70°C for 20 minutes. The reaction mixture was cooled to room temperature and then neutralized with a 5 N sodium hydroxide solution and water, followed by extraction with chloroform three times. The organic layers were washed with a saturated sodium chloride solution and then dried over magnesium sulfate. The drying agent was removed by filtration and then the solvent was evaporated under reduced pressure. The resulting residue was dissolved in ethanol (4 mL). Hydroxylamine hydrochloride (26 mg), sodium acetate (16 mg) and water (1 mL) were sequentially added to the solution, and the mixture was heated with stirring at 70°C for 15 minutes. Hydroxylamine hydrochloride (29 mg) was added to the reaction mixture, and the mixture was further heated with stirring at 70°C for 10 minutes. The reaction mixture was cooled to room temperature. Then, a saturated sodium bicarbonate solution was added, followed by extraction with chloroform three times. The organic layers were washed with a saturated sodium chloride solution and then dried over sodium sulfate. The drying agent was removed by filtration and then the solvent was evaporated under reduced pressure. The resulting residue was dissolved in N,N-dimethylformamide (4 mL). 1,1'-Carbonyldiimidazole (80 mg) was added to the solution, and the mixture was heated with stirring at 60°C overnight. The reaction mixture was cooled to room temperature and then water was added, followed by extraction with ethyl acetate twice. The organic layers were sequentially washed with a saturated sodium chloride solution, water and a saturated sodium chloride solution and then dried over magnesium sulfate. The drying agent was removed by filtration and then the solvent was evaporated under reduced pressure. The residue was purified by NH preparative TLC (heptane-ethyl acetate) to synthesize the title compound (17 mg).
¹H-NMR (400 MHz, CDCl₃) δ (ppm): 0.32-0.44 (m, 2H), 0.58-0.71 (m, 2H), 1.18-1.46 (m, 4H), 1.66-1.84 (m, 4H), 2.06-2.16 (m, 2H), 2.33 (s, 6H), 2.88-2.98 (m, 4H), 3.82 (s, 2H), 3.85 (s, 2H), 3.94 (d, J=6.8 Hz, 2H), 6.89 (d, J=8.6 Hz, 1H), 7.11-7.13 (m, 1H), 7.25-7.27 (m, 1H), 7.43 (d, J=8.6 Hz, 1H).

### (3) 2-{4-{2-[6-Cyclopropylmethoxy-7-(N,N-dimethylaminomethyl)benz[d]isoxazol-3-yl]ethyl}piperidinomethyl}thiophene-3-carbonitrile dihydrochloride

The title compound was obtained by synthesis from 2-{4-{2-[6-cyclopropylmethoxy-7-(N,N-dimethylaminomethyl)benz[d]isoxazol-3-yl]ethyl}piperidinomethyl}thiophene-3-carbonitrile according to Example 179-(7).
¹H-NMR (400 MHz, CD₃OD) δ (ppm) : 0.39-0.50 (m, 2H), 0.63-0.75 (m, 2H), 1.27-1.47 (m, 1H), 1.47-1.73 (m, 3H), 1.73-1.94 (m, 2H), 1.94-2.16 (m, 2H), 2.78-2.99 (m, 8H), 3.06 (t, J=7.8 Hz, 2H), 3.36-3.48 (m, 2H), 4.12 (d, J=7.2 Hz, 2H), 4.50 (s, 2H), 4.63 (s, 2H), 7.23 (d, J=8.8 Hz, 1H), 7.39 (d, J=5.2 Hz, 1H), 7.79 (d, J=5.2 Hz, 1H), 7.92 (d, J=8.8 Hz, 1H).
ESI-MS m/z: 479 [M+H]⁺.

### Example 193

### 2-{4-{2-[6-Cyclopropylmethoxy-7-(N,N-dimethylaminomethyl)benz[d]isoxazol-3-yl]ethyl}piperidinomethyl}thiophene-3-carbaldehyde O-methyloxime dihydrochloride

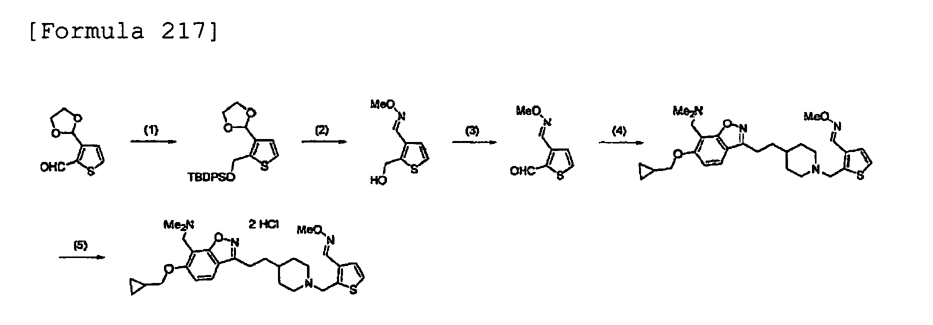

### (1) tert-Butyl-[3-(1,3-dioxolan-2-yl)thiophen-2-ylmethoxy]diphenylsilane

3-(1,3-Dioxolan-2-yl)thiophene-2-carbaldehyde [CAS Registry No. 13250-83-4] (4.23 g) was dissolved in methanol (70 mL). The solution was ice-cooled. Then, sodium borohydride (1.31 g) was added and the mixture was stirred at the same temperature for 25 minutes. Acetone was added to the reaction mixture, followed by heating to room temperature. The reaction mixture was concentrated by evaporation. Ethyl acetate and water were added to the concentrated reaction mixture, followed by extraction with ethyl acetate three times. The organic layers were sequentially washed with water and a saturated sodium chloride solution and then dried over sodium sulfate. The drying agent was removed by filtration and then the solvent was evaporated under reduced pressure.
The residue was dissolved in N,N-dimethylformamide (50 mL). Imidazole (2.4 g) and tert-butyldiphenylchlorosilane (7.1 mL) were sequentially added to the solution, and the mixture was stirred at room temperature overnight. Water was added to the reaction mixture, followed by extraction with ethyl acetate three times. The organic layers were washed with water and a saturated sodium chloride solution and then dried over magnesium sulfate. The drying agent was removed by filtration and then the solvent was evaporated under reduced pressure. The residue was purified by silica gel chromatography (heptane-ethyl acetate) to obtain the title compound (9.3 g).
¹H-NMR (400 MHz, CDCl₃) δ (ppm): 1.07 (s, 9H), 3.83-3.91 (m, 2H), 3.92-4.00 (m, 2H), 4.97 (s, 2H), 5.68 (s, 1H), 7.03 (d, J=5.0 Hz, 1H), 7.17 (d, J=5.0 Hz, 1H), 7.34-7.45 (m, 6H), 7.68-7.72 (m, 4H).

### (2) 2-Hydroxymethylthiophene-3-carbaldehyde O-methyloxime

tert-Butyl-[3-(1,3-dioxolan-2-yl)thiophen-2-ylmethoxy]diphenylsilane (6.7 g) was dissolved in tetrahydrofuran (62 mL). 5 N hydrochloric acid (31 mL) was added to the solution, and the mixture was stirred at room temperature for six hours. The reaction mixture was neutralized with 5 N sodium hydroxide and a saturated sodium bicarbonate solution, followed by extraction with ethyl acetate twice. The organic layers were washed with water and a saturated sodium chloride solution and then dried over magnesium sulfate. The drying agent was removed by filtration and then the solvent was evaporated under reduced pressure. The residue was subjected to silica gel chromatography (heptane-ethyl acetate) to obtain a crude product of 2-(tert-butyldiphenylsilanyloxymethyl). The total amount of the crude product was dissolved in ethanol (100 mL). Methoxylamine hydrochloride (1.89 g) and a mixed solution of sodium acetate (2.47 g) and water (24 mL) were sequentially added to the solution, and the mixture was heated with stirring at 70°C for 20 minutes. The reaction mixture was cooled to room temperature. Then, a saturated sodium bicarbonate solution was added, followed by extraction with ethyl acetate twice. The organic layers were washed with a saturated sodium chloride solution and then dried over magnesium sulfate. The drying agent was removed by filtration and then the solvent was evaporated under reduced pressure. The residue was subjected to NH silica gel chromatography (heptane-ethyl acetate) and silica gel chromatography (heptane-ethyl acetate) to obtain a crude product of 2-(tert-butyldiphenylsilanyloxy)thiophene-3-carbaldehyde O-methyloxime. The total amount of the resulting crude product was dissolved in tetrahydrofuran (60 mL). 5 N hydrochloric acid (30 mL) was added to the solution, and the mixture was heated with stirring at 60°C for one hour. The reaction mixture was cooled to room temperature and then neutralized with a 5 N sodium hydroxide solution and a saturated sodium bicarbonate solution, followed by extraction with ethyl acetate twice. The organic layers were washed with water and a saturated sodium chloride solution and then dried over magnesium sulfate. The drying agent was removed by filtration and then the solvent was evaporated under reduced pressure. The residue was subjected to silica gel chromatography (heptane-ethyl acetate) to obtain a crude product of 2-(tert-butyldiphenylsilanyloxymethyl)thiophene-3-carbaldehyde O-methyloxime and a crude product of 2-(tert-butyldiphenylsilanyloxymethyl)thiophene-3-carbaldehyde.
The total amount of the crude product of 2-(tert-butyldiphenylsilanyloxymethyl)thiophene-3-carbaldehyde obtained here was converted to a crude product of 2-(tert-butyldiphenylsilanyloxymethyl)thiophene-3-carbaldehyde O-methyloxime by the following operation.
The total amount of the crude product of 2-(tert-butyldiphenylsilanyloxymethyl)thiophene-3-carbaldehyde was dissolved in ethanol (42 mL). Methoxylamine hydrochloride (790 mg) and a mixed solution of sodium acetate (1.05 g) and water (10 mL) were sequentially added to the solution, and the mixture was heated with stirring at 70°C for 10 minutes. The reaction mixture was cooled to room temperature. Then, a saturated sodium bicarbonate solution was added, followed by extraction with ethyl acetate twice. The organic layers were washed with a saturated sodium chloride solution and then dried over magnesium sulfate. The drying agent was removed by filtration and then the solvent was evaporated under reduced pressure. The residue was dissolved in tetrahydrofuran (40 mL). 5 N hydrochloric acid (20 mL) was added to the solution, and the mixture was heated with stirring at 60°C for 30 minutes. The reaction mixture was cooled to room temperature and then neutralized with a 5 N sodium hydroxide solution and a saturated sodium bicarbonate solution, followed by extraction with ethyl acetate twice. The organic layers were washed with water and a saturated sodium chloride solution and then dried over magnesium sulfate. The drying agent was removed by filtration and then the solvent was evaporated under reduced pressure. The residue was subjected to NH silica gel chromatography (heptane-ethyl acetate) to obtain a crude product of 2-(tert-butyldiphenylsilanyloxymethyl)thiophene-3-carbaldehyde O-methyloxime.
The total amount of the crude product of 2-(tert-butyldiphenylsilanyloxymethyl)thiophene-3-carbaldehyde O-methyloxime obtained by the above operation was dissolved in tetrahydrofuran (50 mL). Tetrabutylammonium fluoride (1 M solution in tetrahydrofuran, 15 mL) was added to the solution, and the mixture was stirred at room temperature overnight. Water was added to the reaction mixture, followed by extraction with ethyl acetate three times. The organic layers were washed with a saturated sodium chloride solution and then dried over sodium sulfate. The drying agent was removed by filtration and then the solvent was evaporated under reduced pressure. The residue was purified by silica gel chromatography (heptane-ethyl acetate) to obtain the title compound (1.29 g).
¹H-NMR (400 MHz, CDCl₃) δ(ppm): 3.88 (t, J=7.2 Hz, 1H), 3. 97 (s, 3H), 4. 76 (d, J=7.2 Hz, 2H), 7. 11 (d, J=5.2 Hz, 1H), 7.17 (d, J=5.2 Hz, 1H), 8.18 (s, 1H) .

### (3) Thiophene-2,3-dicarbaldehyde 3-(O-methyloxime)

2-Hydroxymethylthiophene-3-carbaldehyde O-methyloxime (200 mg) was dissolved in dichloromethane (15 mL), and pyridine (500 µL) was added. The solution was ice-cooled. Then, Dess-Martin periodinane (650 mg) was added and the mixture was stirred for 30 minutes. Further, pyridine (170 µL) and Dess-Martin periodinane (650 mg) were sequentially added and the mixture was stirred for 40 minutes. A saturated sodium bicarbonate solution and sodium thiosulfate were added to the reaction mixture. The reaction mixture was extracted with ethyl acetate three times at room temperature. The organic layers were washed with water and a saturated sodium chloride solution and then dried over magnesium sulfate. The drying agent was removed by filtration and then the solvent was evaporated under reduced pressure. The residue was purified by silica gel chromatography (heptane-ethyl acetate) to obtain the title compound (153 mg).
¹H-NMR (400 MHz, CDCl₃) δ (ppm): 4.01 (s, 3H), 7.45 (d, J=5.0 Hz, 1H), 7.65 (dd, J=0.6 Hz, 5.0 Hz, 1H), 8.55 (s, 1H), 10. 17 (d, J=0.6 Hz, 1H) .

### (4) 2-{4-{2-[6-Cyclopropylmethoxy-7-(N,N-dimethylaminomethyl)benz[d]isoxazol-3-yl]ethyl}piperidinomethyl}thiophene-3-carbaldehyde O-methyloxime

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3) and thiophene-2,3-dicarbaldehyde 3-(O-methyloxime) according to Example 179-(6).
¹H-NMR (400 MHz, CDCl₃) δ(ppm): 0.31-0.43 (m, 2H), 0.57-0.71 (m, 2H), 1.23-1.42 (m, 4H), 1.65-1.86 (m, 4H), 1.93-2.04 (m, 2H), 2.33 (s, 6H), 2.87-2.97 (m, 4H), 3.70 (s, 2H), 3. 82 (s, 2H), 3. 93 (s, 3H), 3.93 (d, J=6.8 Hz, 2H), 6.88 (d, J=8.4 Hz, 1H), 7.13-7.15 (m, 1H), 7.28-7.29 (m, 1H), 7.43 (d, J=8.4 Hz, 1H), 8.24 (s, 1H) .

### (5) 2-{4-{2-[6-Cyclopropylmethoxy-7-(N,N-dimethylaminomethyl)benz[d]isoxazol-3-yl]ethyl}piperidinomethyl}thiophene-3-carbaldehyde O-methyloxime dihydrochloride

The title compound was obtained by synthesis from 2-{4-{2-[6-cyclopropylmethoxy-7-(N,N-dimethylaminomethyl)benz[d]isoxazol-3-yl]ethyl}piperidinomethyl}thiophene-3-carbaldehyde O-methyloxime according to Example 179-(7).
¹H-NMR (400 MHz, CD₃OD) δ(ppm): 0.40-0.50 (m, 2H), 0.65-0.75 (m, 2H), 1.33-1.47 (m, 1H), 1.49-1.64 (m, 2H), 1.68-1.96 (m, 3H), 2.06-2.16 (m, 2H) 2.96 (s, 6H) 3.02-3.19 (m, 4H), 3.58-3.68 (m, 2H), 3.99 (s, 3H), 4.12 (d, J=6.8 Hz, 2H), 4.63 (s, 2H), 4 . 69 (s, 2H), 7.23 (d, J=8.8 Hz, 1H), 7 . 39 (d, J=5.4 Hz, 1H), 7.66 (d, J=5. 4 Hz, 1H), 7.92 (d, J=8.8 Hz, 1H), 8.37 (s, 1H) .
ESI-MS m/z: 511 [M+H]⁺.

Example 194

### N,N-Dimethyl{6-cyclopropylmethoxy-3-[2-(1-thiazol-2-ylmethylpiperidin-4-yl)ethyl]benz[d]isoxazol-7-yl}methylamine dihydrochloride

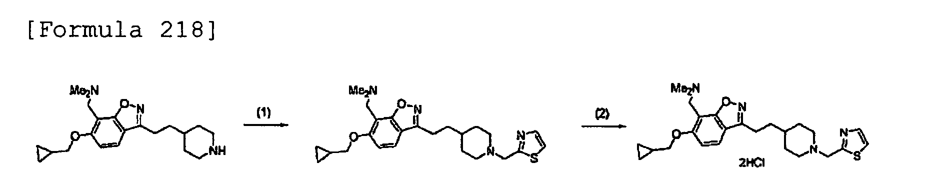

### (1) N,N-Dimethyl{6-cyclopropylmethoxy-3-[2-(1-thiazol-2-ylmethylpiperidin-4-yl)ethyl]benz[d]isoxazol-7-yl}methylamine

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3) and 2-formylthiazole according to Example 179-(6).
¹H-NMR (400 MHz, CDCl₃) δ(ppm): 0.32-0.44 (m, 2H), 0.57-0.70 (m, 2H), 1.23-1.46 (m, 4H), 1.71-2.20 (m, 6H), 2.33 (s, 6H), 2. 91-3.00 (m, 4H), 3.82 (s, 2H), 3. 84 (s, 2H), 3. 94 (d, J=6.8 Hz, 2H), 6. 89 (d, J=8.6 Hz, 1H), 7.25-7.28 (m, 1H), 7.44 (d, J=8.6 Hz, 1H), 7.68-7.70 (m, 1H) .

### (2) N,N-Dimethyl{6-cyclopropylmethoxy-3-[2-(1-thiazol-2-ylmethylpiperidin-4-yl)ethyl]benz[d]isoxazol-7-yl}methylamine dihydrochloride

The title compound was obtained by synthesis from N,N-dimethyl{6-cyclopropylmethoxy-3-[2-(1-thiazol-2-ylmethylpiperidin-4-yl)ethyl]benz[d]isoxazol-7-yl}methylamine according to Example 179-(7).
¹H-NMR (400 MHz, CD₃OD) δ (ppm) : 0.40-0.50 (m, 2H), 0.65-0.76 (m, 2H), 1.36-1.47 (m, 1H), 1.50-1.96 (m, 5H), 2.04-2.19 (m, 2H), 2.96 (s, 6H), 3.02-3.28 (m, 4H), 3.57-3.77 (m, 2H), 4.12 (d, J=7.2 Hz, 2H), 4.63 (s, 2H), 4.74 (s, 2H), 7.24 (d, J=8.8 Hz, 1H), 7.79 (d, J=3.4 Hz, 1H), 7.93 (d, J=8.8 Hz, 1H), 7.96 (d, J=3.4 Hz, 1H).
ESI-MS m/z: 455 [M+H]⁺.

### Example 195

### N,N-Dimethyl{6-cyclopropylmethoxy-3-{2-[1-(2-methoxybenzyl)piperidin-4-yl]ethyl}benz[d]isoxazol-7-yl}methylamine dihydrochloride

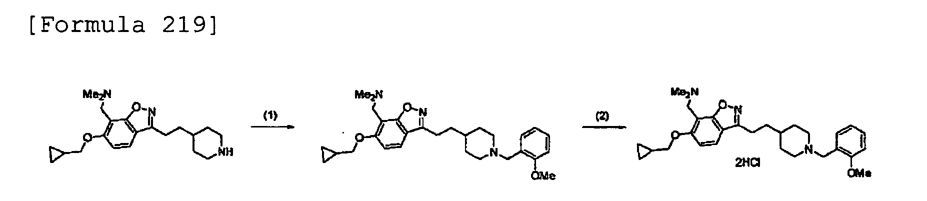

### (1) N,N-Dimethyl{6-cyclopropylmethoxy-3-{2-[1-(2-methoxybenzyl)piperidin-4-yl]ethyl}benz[d]isoxazol-7-yl}methylamine

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3) and 2-methoxybenzaldehyde according to Example 179-(6).
¹H-NMR (400 MHz, CDCl₃) δ(ppm): 0.31-0.44 (m, 2H), 0.57-0.71 (m, 2H), 1.22-1.43 (m, 4H), 1.66-2.08 (m, 6H), 2.33 (s, 6H), 2.87-2.96 (m, 4H), 3.54 (s, 2H), 3.81 (s, 3H), 3.81 (s, 2H), 3.93 (d, J=6.8 Hz, 2H), 6.82-6.94 (m, 3H), 7.18-7.23 (m, 1H), 7.33 (dd, J=1.8 Hz, 7.4 Hz, 1H), 7.43 (d, J=8.6 Hz, 1H).

### (2) N,N-Dimethyl(6-cyclopropylmethoxy-3-{2-[1-(2-methoxybenzyl)piperidin-4-yl]ethyl}benz[d]isoxazol-7-yl)methylamine dihydrochloride

The title compound was obtained by synthesis from N,N-dimethyl(6-cyclopropylmethoxy-3-{2-[1-(2-methoxybenzyl)piperidin-4-yl]ethyl}benz[d]isoxazol-7-yl)methylamine according to Example 179-(7).
¹H-NMR (400 MHz, CD₃OD) δ(ppm): 0.40-0.50 (m, 2H), 0.65-0.75 (m, 2H), 1.35-1.46 (m, 1H), 1.48-1.93 (m, 5H), 1.96-2.12 (m, 2H), 2.96 (s, 6H), 2.99-3.15 (m, 4H), 3.38-3.59 (m, 2H), 3.93 (s, 3H), 4.12 (d, J=7.2 Hz, 2H), 4.32 (s, 2H), 4.63 (s, 2H), 7.04 (dt, J=0.8 Hz, 7.6 Hz, 1H), 7.12 (d, J=7.6 Hz, 1H), 7.23 (d, J=8.8 Hz, 1H), 7.43-7.51 (m, 2H), 7. 92 (d, J=8.8 Hz, 1H).
ESI-MS m/z: 240 [M+2H]²⁺, 478 [M+H]⁺.

### Example 196

### N,N-Dimethyl(6-cyclopropylmethoxy-3-{2-[1-(3-methoxybenzyl)piperidin-4-yl]ethyl}benz[d]isoxazol-7-yl)methylamine dihydrochloride

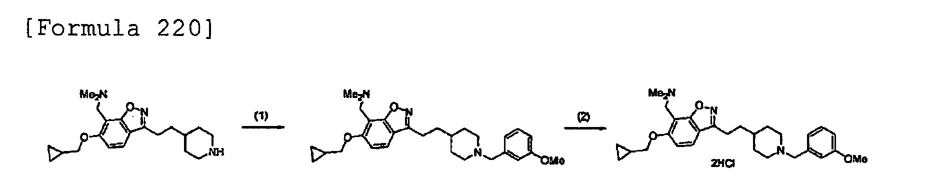

### (1) N,N-Dimethyl{6-cyclopropylmethoxy-3-{2-[1-(3-methoxybenzyl)piperidin-4-yl]ethyl}benz[d]isoxazol-7-yl}methylamine

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3) and 3-methoxybenzaldehyde according to Example 179-(6).
¹H-NMR (400 MHz, CDCl₃) δ (ppm) : 0.31-0. 44 (m, 2H), 0.57-0.71 (m, 2H), 1.22-1.44 (m, 4H), 1.68-2.05 (m, 6H), 2.33 (s, 6H), 2.83-2.97 (m, 4H), 3.45 (s, 2H), 3.80 (s, 3H), 3.81 (s, 2H), 3.93 (d, J=6.8 Hz, 2H), 6.75-6.80 (m, 1H), 6.85-6.91 (m, 3H), 7.17-7.23 (m, 1H), 7.43 (d, J=8.8 Hz, 1H).

### (2) N,N-Dimethyl(6-cyclopropylmethoxy-3-{2-[1-(3-methoxybenzyl)piperidin-4-yl]ethyl}benz[d]isoxazol-7-yl)methylamine dihydrochloride

The title compound was obtained by synthesis from N,N-dimethyl(6-cyclopropylmethoxy-3-{2-[1-(3-methoxybenzyl)piperidin-4-yl]ethyl}benz[d]isoxazol-7-yl)methylamine according to Example 179-(7).
¹H-NMR (400 MHz, CD₃OD) δ (ppm) : 0.39-0.49 (m, 2H), 0.64-0.74 (m, 2H), 1.27-1.46 (m, 1H), 1.51-1.77 (m, 3H), 1.79-1.91 (m, 2H), 1.97-2.10 (m, 2H), 2.83-2.99 (m, 8H), 3.04 (t, J=7.8 Hz, 2H), 3.35-3.46 (m, 2H), 3.83 (s, 3H), 4. 10 (d, J=7.2 Hz, 2H), 4.20 (s, 2H), 4. 51 (s, 2H), 7.01 (ddd, J=0.8 Hz, 2 . 4 Hz, 8.0 Hz, 1H), 7.05-7.10 (m, 1H), 7.12-7.16 (m, 1H), 7.21 (d, J=8.8 Hz, 1H), 7.36 (dd, J=7.6 Hz, 8.0 Hz, 1H), 7.88 (d, J=8.8 Hz, 1H).
ESI-MS m/z: 240 [M+2H]²⁺, 478 [M+H]⁺.

### Example 197

### N,N-Dimethyl(6-cyclopropylmethoxy-3-{2-[1-(4-methoxybenzyl)piperidin-4-yl]ethyl}benz[d]isoxazol-7-yl)methylamine dihydrochloride

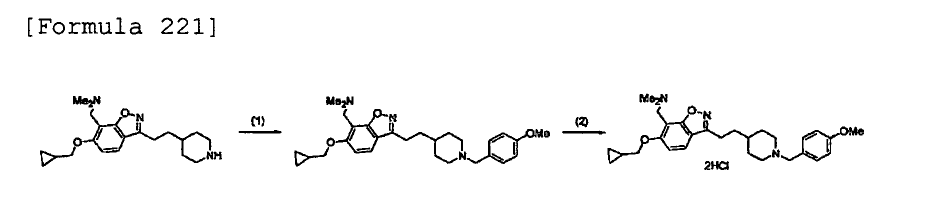

### (1) N,N-Dimethyl(6-cyclopropylmethoxy-3-{2-[1-(4-methoxybenzyl)piperidin-4-yl]ethyl}benz[d]isoxazol-7-yl)methylamine

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3) and 4-methoxybenzaldehyde according to Example 179-(6).
¹H-NMR (400 MHz, CDCl₃) δ(ppm): 0.31-0.44 (m, 2H), 0.57-0.70 (m, 2H), 1.21-1.42 (m, 4H), 1.60-1.81 (m, 4H), 1.84-1.95 (m, 2H), 2.33 (s, 6H), 2.82-2.97 (m, 4H), 3.42 (s, 2H), 3.79 (s, 3H), 3. 81 (s, 2H), 3.93 (d, J=6.8 Hz, 2H), 6.80-6.86 (m, 2H), 6.88 (d, J=8.6 Hz, 1H), 7.18-7.22 (m, 2H), 7.42 (d, J=8.6 Hz, 1H).

### (2) N,N-Dimethyl(6-cyclopropylmethoxy-3-{2-[1-(4-methoxybenzyl)piperidin-4-yl]ethyl}benz[d]isoxazol-7-yl)methylamine dihydrochloride

The title compound was obtained by synthesis from N,N-dimethyl(6-cyclopropylmethoxy-3-{2-[1-(4-methoxybenzyl)piperidin-4-yl]ethyl}benz[d]isoxazol-7-yl)methylamine according to Example 179-(7).
¹H-NMR (400 MHz, CD₃OD) δ(ppm): 0.48-0.58 (m, 2H), 0.64-0.76 (m, 2H), 1.28-1.44 (m, 1H), 1.46-1.62 (m, 2H), 1.62-1.76 (m, 1H), 1.78-1.91 (m, 2H), 1.98-2.09 (m, 2H), 2.81 (s, 6H), 2.84-2.96 (m, 2H), 3.04 (t, J=7.8 Hz, 2H), 3.35-3.47 (m, 2H), 3.82 (s, 3H), 4.08 (d, J=6.8 Hz, 2H), 4.17 (s, 2H), 4.45 (s, 2H), 6.96-7.03 (m, 2H), 7.20 (d, J=8.8 Hz, 1H), 7.39-7.46 (m, 2H), 7.86 (d, J=8.8 Hz, 1H).
ESI-MS m/z: 478 [M+H]⁺.

### Example 198

### N,N-Dimethyl(6-cyclopropylmethoxy-3-{2-[1-(6-cyclopropylpyridin-2-ylmethyl)piperidin-4-yl]ethyl}benz[d]isoxazol-7-yl)methylamine dihydrochloride

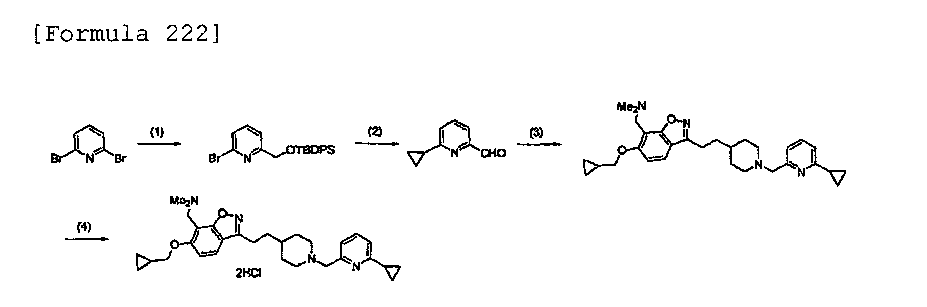

### (1) 2-Bromo-6-(tert-butyldiphenylsilanyloxymethyl)pyridine

The title compound was synthesized from 2,6-dibromopyridine with reference to Tetrahedron Lett., 37 (15)-2537, (1996) .
Tetrahydrofuran (30 mL) was cooled in a dry ice-acetone bath in a nitrogen atmosphere, and n-butyllithium (2.71 M solution in hexane, 18.5 mL) was added. A solution of 2,6-dibromopyridine (11.85 g) in tetrahydrofuran (70 mL) was added while maintaining the internal temperature of the solution at -70°C or less. The reaction mixture was stirred at the same temperature for 15 minutes. Then, N,N-dimethylformamide (6 mL) was added and the mixture was stirred for 15 minutes. Methanol (50 mL), acetic acid (3.2 mL) and sodium borohydride (1.9 g) were sequentially added to the reaction mixture. Then, the dry ice-acetone bath was removed and the mixture was gradually heated to room temperature. After stirring for 25 minutes, a saturated ammonium chloride solution was added to the reaction mixture, followed by extraction with ethyl acetate twice. The organic layers were washed with a saturated sodium chloride solution and then dried over sodium sulfate. The drying agent was removed by filtration and then the solvent was evaporated under reduced pressure. The residue was dissolved in N,N-dimethylformamide (50 mL). Imidazole (4.08 g) and tert-butyldiphenylchlorosilane (14 mL) were sequentially added to the solution, and the mixture was stirred at room temperature overnight. Water and a saturated sodium bicarbonate solution were added to the reaction mixture, followed by extraction with ethyl acetate twice. The organic layers were washed with water and a saturated sodium chloride solution and then dried over magnesium sulfate. The drying agent was removed by filtration and then the solvent was evaporated under reduced pressure. The residue was purified by silica gel chromatography (heptane-ethyl acetate) to obtain the title compound (18.01 g).
¹H-NMR (400 MHz, CDCl₃) δ (ppm): 1.13 (s, 9H), 4.83 (s, 2H), 7.30-7.46 (m, 7H), 7.55-7.68 (m, 6H).

### (2) 6-Cyclopropylpyridine-2-carbaldehyde

2-Bromo-6-(tert-butyldiphenylsilanyloxymethyl)pyridine azeotropically distilled with toluene and dried under reduced pressure in a nitrogen atmosphere (3 g) was dissolved in tetrahydrofuran (14 mL). [1,1'-Bis(diphenylphosphino)ferrocene]dichloronickel (II) (250 mg) was added to the solution, and the reaction mixture was cooled in an ice bath. Cyclopropylmagnesium bromide (0.5 M solution in tetrahydrofuran, 35 mL) was added to the reaction mixture, and the mixture was removed from the ice bath. After stirring at room temperature for 3.5 hours, cyclopropylmagnesium bromide (0.5 M solution in tetrahydrofuran, 15 mL) was added. After stirring for 30 minutes, the reaction mixture was cooled in an ice bath and [1,1'-bis(diphenylphosphino)ferrocene]dichloronickel (II) (250 mg) was added. The reaction mixture was stirred at room temperature for 1.5 hours. A saturated ammonium chloride solution was added to the reaction mixture, followed by extraction with ethyl acetate twice. The organic layers were sequentially washed with water and a saturated sodium chloride solution and then dried over magnesium sulfate. The drying agent was removed by filtration and then the solvent was evaporated under reduced pressure. The residue was subjected to silica gel chromatography (heptane-ethyl acetate) to obtain a crude product of 2-(tert-butyldiphenylsilanyloxymethyl)-6-cyclopropylpyridine.
The total amount of the crude product was dissolved in tetrahydrofuran (15 mL). Tetrabutylammonium fluoride (1 M solution in tetrahydrofuran, 10 mL) was added to the solution, and the mixture was stirred at room temperature overnight. Water was added to the reaction mixture, followed by extraction with ethyl acetate three times. The organic layers were sequentially washed with water and a saturated sodium chloride solution and then dried over sodium sulfate. The drying agent was removed by filtration and then the solvent was evaporated under reduced pressure. The residue was subjected to NH silica gel chromatography (heptane-ethyl acetate) to obtain a crude product of (6-cyclopropylpyridin-2-yl)methanol (290 mg). The crude product was used for the next reaction without further purification. The crude product of (6-cyclopropylpyridin-2-yl)methanol (180 mg) was dissolved in dichloromethane (15 mL). Pyridine (500 µL) was added to the solution, and the reaction mixture was cooled in an ice bath. Dess-Martin periodinane (770 mg) was added to the reaction mixture, followed by stirring for 35 minutes. A saturated sodium bicarbonate solution and sodium thiosulfate were added to the reaction mixture. The reaction mixture was extracted with chloroform twice at room temperature. The organic layers were washed with a mixed solution of a saturated sodium bicarbonate solution and a saturated sodium chloride solution and then dried over magnesium sulfate. The drying agent was removed by filtration and then the solvent was evaporated under reduced pressure. The residue was purified by silica gel chromatography (heptane-ethyl acetate) to obtain the title compound (151 mg).
¹H-NMR (400 MHz, CDCl₃) δ (ppm) : 1.02-1.15 (m, 4H), 2.07-2.16 (m, 1H), 7.32-7.39 (m, 1H), 7.66-7.70 (m, 2H), 9.96 (d, J=0.4 Hz, 1H) .

### (3) N,N-Dimethyl(6-cyclopropylmethoxy-3-{2-[1-(6-cyclopropylpyridin-2-ylmethyl)piperidin-4-yl]ethyl}benz[d]isoxazol-7-yl)methylamine

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3) and 6-cyclopropylpyridine-2-carbaldehyde according to Example 179-(6).
¹H-NMR (400 MHz, CDCl₃) δ(ppm): 0.34-0.40 (m, 2H), 0.64-0.68 (m, 2H), 0.92-0.99 (m, 4H), 1.21-1.44 (m, 4H), 1.55-1.82 (m, 4H), 1.97-2.10 (m, 3H), 2.33 (s, 2H), 2. 86-2. 97 (m, 4H), 3.58 (s, 2H), 3. 82 (s, 2H), 3. 93 (d, J=6.8 Hz, 2H), 6.88 (dd, J=0.8 Hz, 7.6 Hz, 1H), 6.88 (d, J=8.6 Hz, 1H), 7.15 (dd, J=0.8 Hz, 7 . 6 Hz, 1H), 7.43 (d, J=8.6 Hz, 1H), 7.47 (d, J=7.6 Hz, 1H) .

### (4) N,N-Dimethyl(6-cyclopropylmethoxy-3-{2-[1-(6-cyclopropylpyridin-2-ylmethyl)piperidin-4-yl]ethyl}benz[d]isoxazol-7-yl)methylamine dihydrochloride

N,N-Dimethyl(6-cyclopropylmethoxy-3-{2-[1-(6-cyclopropylpyridin-2-ylmethyl)piperidin-4-yl]ethyl}benz[d]isoxazol-7-yl)methylamine (200 mg) was dissolved in methanol. 5 N hydrochloric acid (166 µL) was added and the solvent was evaporated under reduced pressure. The residue was dried under reduced pressure to obtain the title compound (186 mg).
¹H-NMR (400 MHz, CD₃OD) δ(ppm): 0.40-0.50 (m, 2H), 0.65-0.75 (m, 2H), 1.04-1.16 (m, 4H), 1.34-1.50 (m, 1H), 1.54-1.94 (m, 5H), 2.04-2.16 (m, 2H), 2.16-2.25 (m, 1H), 2.97 (s, 6H), 3.02-3.25 (m, 4H), 3.51-3.67 (m, 2H), 4.12 (d, J=7.2 Hz, 2H), 4.44 (s, 2H), 4.64 (s, 2H), 7.24 (d, J=8.8 Hz, 1H), 7.30-7.37 (m, 2H), 7.79 (t, J=7. Hz, 1H), 7.93 (d, J=8.8 Hz, 1H) .
ESI-MS m/z: 245 [M+2H]²⁺, 489 [M+H]⁺.

### Example 199

### N,N-Dimethyl{6-cyclopropylmethoxy-3-{2-{[1-(2-pyridyl)cyclopropyl]methyl}piperidin-4-yl}ethyl}benz[d]isoxazol-7-yl}methylamine dihydrochloride

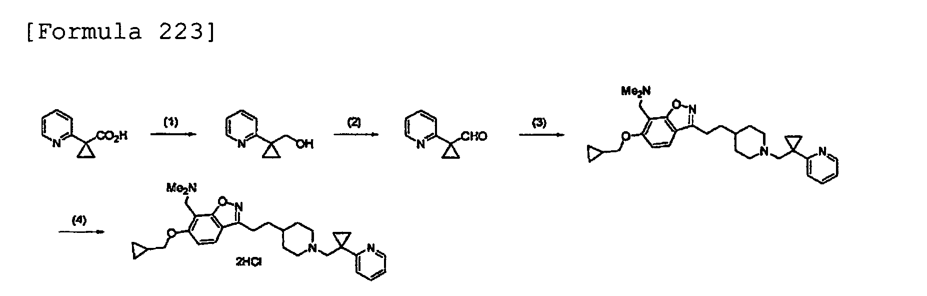

### (1) [1-(2-Pyridyl)cyclopropyl]methanol

1-(2-Pyridyl)cyclopropanecarboxylic acid [CAS Registry No. 162960-26-1] (5.40 g) was dissolved in tetrahydrofuran (100 mL). Triethylamine (6.1 mL) was added to the solution, and the reaction mixture was cooled to -30°C or less. Ethyl chloroformate (3.8 mL) was added to the reaction mixture, followed by stirring for one hour. A solution of sodium borohydride (3.2 g) in water (27 mL) was added dropwise to the reaction mixture. Then, the reaction mixture was removed from the cooling bath and stirred at room temperature for 30 minutes. Water was added to the reaction mixture, followed by extraction with ethyl acetate three times. The organic layers were sequentially washed with water and a mixed solution of a saturated sodium bicarbonate solution and a saturated sodium chloride solution and then dried over sodium sulfate. The drying agent was removed by filtration and then the solvent was evaporated under reduced pressure. The residue was purified by NH silica gel chromatography (heptane-ethyl acetate) and silica gel chromatography (heptane-ethyl acetate) to obtain the title compound (2.38 g).
¹H-NMR (400 MHz, CDCl₃) δ(ppm): 1.40-1.44 (m, 4H), 3.83 (s, 2H), 4.49 (br s, 1H), 6.86-6.92 (m, 1H), 7.06-7.11 (m, 1H), 7.54-7.61 (m, 1H), 8.41-8.47 (m, 1H).

### (2) 1-(2-Pyridyl)cyclopropanecarbaldehyde

The title compound was obtained by synthesis from [1-(2-pyridyl)cyclopropyl]methanol according to Example 193-(3).
¹H-NMR (400 MHz, CDCl₃) δ (ppm) : 1.63-1.75 (m, 4H), 7.15-7.19 (m, 1H), 7.52-7.56 (m, 1H), 7.64-7.70 (m, 1H), 8.52-8.57 (m, 1H).

### (3) N,N-Dimethyl{6-cyclopropylmethoxy-3-{2-{[1-(2-pyridyl)cyclopropyl]methyl}piperidin-4-yl}ethyl}benz[d]isoxazol-7-yl}methylamine

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3) and 1-(2-pyridyl)cyclopropanecarbaldehyde according to Example 179-(6).
¹H-NMR (400 MHz, CDCl₃) δ (ppm) : 0.31-0.43 (m, 2H), 0.58-0.68 (m, 2H), 0.75-0.83 (m, 2H), 1.02-1.39 (m, 6H), 1.60-1.96 (m, 6H), 2.33 (s, 6H), 2.68 (s, 2H), 2.90 (t, J=7.8 Hz, 2H), 2.95-3.05 (m, 2H), 3.81 (s, 2H), 3.93 (d, J=6.8 Hz, 2H), 6. 87 (d, J=8.6 Hz, 1H), 6.98-7.04 (m, 1H), 7 . 42 (d, J=8 . 6 Hz, 1H), 7 . 50-7. 57 (m, 1H), 7 . 61-7. 67 (m, 1H), 8.40-8.46 (m, 1H) .

### (4) N,N-Dimethyl{6-cyclopropylmethoxy-3-{2-{[1-(2-pyridyl)cyclopropyl]methyl}piperidin-4-yl}ethyl}benz[d]isoxazol-7-yl}methylamine dihydrochloride

The title compound was obtained by synthesis from N,N-dimethyl{6-cyclopropylmethoxy-3-{2-{[1-(2-pyridyl)cyclopropyl]methyl}piperidin-4-yl}ethyl}benz[d]isoxazol-7-yl}methylamine according to Example 198-(4).
¹H-NMR (400 MHz, CD₃OD) δ (ppm) : 0.39-0.51 (m, 2H), 0.64-0.76 (m, 2H), 1.28-1.47 (m, 5H), 1.50-1.96 (m, 5H), 1.98-2.19 (m, 2H), 2.88-3.18 (m, 10H), 3.49-3.86 (m, 4H), 4. 12 (d, J=7.2 Hz, 2H), 4.63 (s, 2H), 7.04-7.12 (m, 1H), 7.24 (d, J=9.0 Hz, 1H), 7.26-7.34 (m, 1H), 7.73-7.84 (m, 1H), 7.94 (d, J=9.0 Hz, 1H), 8.49-8.56 (m, 1H).
ESI-MS m/z: 245 [M+2H]²⁺, 489 [M+H]⁺.

### Example 200

### N,N-Dimethyl{6-cyclopropylmethoxy-3-{2-[1-(pyrazolo[1,5-a]pyridin-7-ylmethyl)piperidin-4-yl]ethyl}benz[d]isoxazol-7-yl}methylamine dihydrochloride

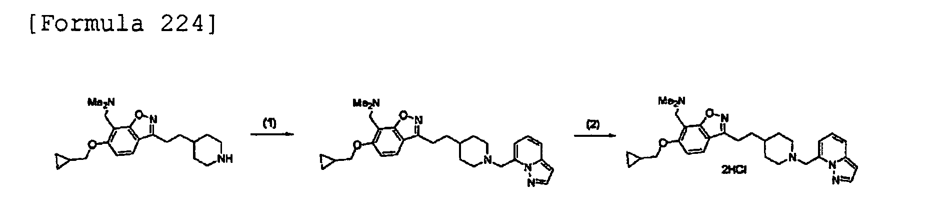

### (1) N,N-Dimethyl{6-cyclopropylmethoxy-3-{2-[1-(pyrazolo[1,5-a]pyridin-7-ylmethyl)piperidin-4-yl]ethyl}benz[d]isoxazol-7-yl}methylamine

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3) and pyrazolo[1,5-a]pyridine-7-carbaldehyde [CAS Registry No. 362661-83-4] according to Example 179-(6).
¹H-NMR (400 MHz, CDCl₃) δ(ppm): 0.31-0.44 (m, 2H), 0.58-0.72 (m, 2H), 1.21-1.36 (m, 1H), 1.36-1.52 (m, 2H), 1.76-2.30 (m, 6H), 2.34 (s, 6H), 2.96 (t, J=7.8 Hz, 2H), 3.01-3.11 (m, 2H), 3.83 (s, 2H), 3.94 (d, J=6.8 Hz, 2H), 4.04 (s, 2H), 6.54 (d, J=2.2 Hz, 1H), 6.89 (d, J=8.6 Hz, 1H), 6.95 (dd, J=1.2 Hz, 6.8 Hz, 1H), 7.13 (dd, J=6.8 Hz, 8.8 Hz, 1H), 7.45 (d, J=8.8 Hz, 1H), 7.47 (d, J=8.6 Hz, 1H), 7.96 (d, J=2.2 Hz, 1H).

### (2) N,N-Dimethyl{6-cyclopropylmethoxy-3-{2-[1-(pyrazolo[1,5-a]pyridin-7-ylmethyl)piperidin-4-yl]ethyl}benz[d]isoxazol-7-yl}methylamine dihydrochloride

The title compound was obtained by synthesis from N,N-dimethyl{6-cyclopropylmethoxy-3-{2-[1-(pyrazolo[1,5-a]pyridin-7-ylmethyl)piperidin-4-yl]ethyl}benz[d]isoxazol-7-yl}methylamine according to Example 198-(4).
¹H-NMR (400 MHz, CD₃OD) δ(ppm): 0.37-0.50 (m, 2H), 0.62-0.75 (m, 2H), 1.34-1.46 (m, 1H), 1.50-1.91 (m, 5H), 1.96-2.11 (m, 2H), 2.96 (s, 6H), 3.06 (t, J=7.8 Hz, 1H), 3. 14-3. 26 (m, 2H), 3. 46-3. 58 (m, 2H), 4 . 12 (d, J=7.2 Hz, 2H), 4.63 (s, 2H), 4.84 (s, 2H), 6.76 (d, J=2.4 Hz, 1H), 7.16-7.31 (m, 3H), 7.81 (dd, J=1.2 Hz, 8.8 Hz, 1H), 7.92 (d, J=8.8 Hz, 1H), 8.08 (d, J=2.4 Hz, 1H) .
ESI-MS m/z: 245 [M+2H]²⁺, 488 [M+H]⁺.

### Example 201

### N,N-Dimethyl{6-cyclopropylmethoxy-3-{2-[1-(pyrazolo[1,5-a]pyridin-2-ylmethyl)piperidin-4-yl]ethyl}benz[d]isoxazol-7-yl}methylamine dihydrochloride

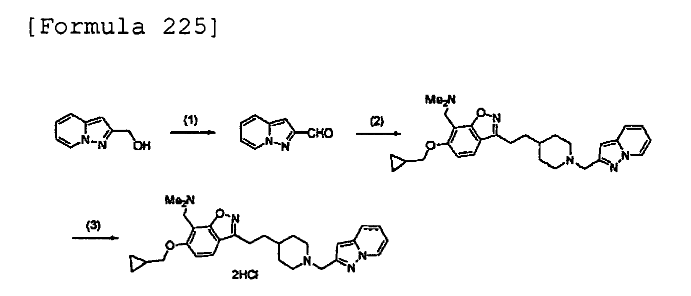

### (1) Pyrazolo[1,5-a]pyridine-2-carbaldehyde

The title compound was obtained by synthesis from pyrazolo[1,5-a]pyridin-2-ylmethanol [CAS Registry No. 76943-47-0] according to Example 193-(3).
¹H-NMR (400 MHz, CDCl₃) δ (ppm) : 6.93 (dt, J=1.2 Hz, 7.0 Hz, 1H), 7.03 (s, 1H), 7.14-7.22 (m, 1H), 7.59-7.64 (m, 1H), 8.46-8.51 (m, 1H), 10.20 (s, 1H).

### (2) N,N-Dimethyl{6-cyclopropylmethoxy-3-{2-[1-(pyrazolo[1,5-a]pyridin-2-ylmethyl)piperidin-4-yl]ethyl}benz[d]isoxazol-7-yl}methylamine

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3) and pyrazolo[1,5-a]pyridine-2-carbaldehyde according to Example 179-(6).
¹H-NMR (400 MHz, CDCl₃) δ (ppm) : 0.33-0.43 (m, 2H), 0.58-0.71 (m, 2H), 1.21-1.45 (m, 4H), 1.68-1.82 (m, 4H), 1.97-2.13 (m, 2H), 2.33 (s, 6H), 2.88-3.05 (m, 4H), 3.73 (s, 2H), 3.83 (s, 2H), 3.93 (t, J=6.8 Hz, 2H), 6.41-6.45 (m, 1H), 6.65-6.71 (m, 1H), 6.88 (d, J=8.6 Hz, 1H), 7.02-7.08 (m, 1H), 7.40-7.47 (m, 2H), 8.37-8.42 (m, 1H).

### (3) N,N-Dimethyl{6-cyclopropylmethoxy-3-{2-[1-(pyrazolo[1,5-a]pyridin-2-ylmethyl)piperidin-4-yl]ethyl}benz[d]isoxazol-7-yl}methylamine dihydrochloride

The title compound was obtained by synthesis from N,N-dimethyl{6-cyclopropylmethoxy-3-{2-[1-(pyrazolo[1,5-a]pyridin-2-ylmethyl)piperidin-4-yl]ethyl}benz[d]isoxazol-7-yl}methylamine according to Example 198-(4).
¹H-NMR (400 MHz, CD₃OD) δ (ppm) : 0.39-0.49 (m, 2H), 0.64-0.75 (m, 2H), 1.34-1.46 (m, 1H), 1.48-1.64 (m, 2H), 1.64-1.79 (m, 1H), 1.79-1.96 (m, 2H), 2.04-2.15 (m, 2H), 2.96 (s, 6H), 3.02-3.15 (m, 4H), 3.60-3.68 (m, 2H), 4.11 (d, J=7.2 Hz, 2H), 4.51 (s, 2H), 4.63 (s, 2H), 6.78-6.82 (m, 1H), 6.93-6.99 (m, 1H), 7.20-7.31 (m, 2H), 7.66-7.71 (m, 1H), 7.91 (d, J=8.8 Hz, 1H), 8.55-8.59 (m, 1H).
ESI-MS m/z: 245 [M+2H]²⁺, 488 [M+H]⁺.

### Example 202

### 2-{4-{2-[6-Cyclopropylmethoxy-7-(N,N-dimethylaminomethyl)benz[d]isoxazol-3-yl]ethyl}piperidinomethyl}thiazole-4-carbonitrile difumarate

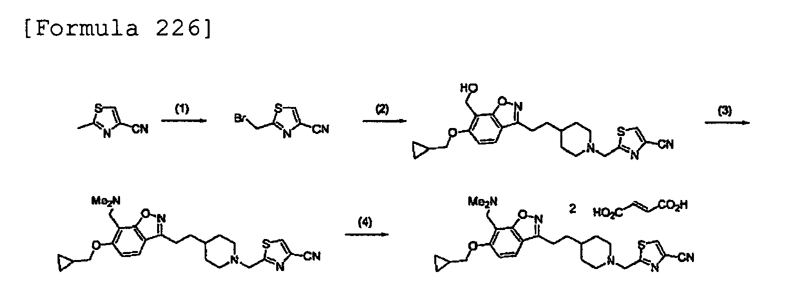

### (1) 2-Bromomethylthiazole-4-carbonitrile

2-Methylthiazole-4-carbonitrile [CAS Registry No. 21917-76-0] (300 mg) was dissolved in carbon tetrachloride (15 mL). N-Bromosuccinimide (431 mg) and 2,2'-azobisisobutylonitrile (5 mg) were added and the mixture was heated with stirring at 95°C for 5.5 hours. The reaction mixture was cooled to room temperature. Then, a saturated sodium bicarbonate solution was added, followed by extraction with ethyl acetate twice. The organic layers were washed with a saturated sodium chloride solution and then dried over magnesium sulfate. The drying agent was removed by filtration and then the solvent was evaporated under reduced pressure. The residue was purified by silica gel chromatography (toluene-ethyl acetate) to obtain the title compound (112 mg).
¹H-NMR (400 MHz, CDCl₃) δ(ppm): 4.71 (s, 2H), 8.04 (s, 1H) .

### (2) 2-{4-[2-(6-Cyclopropylmethoxy-7-hydroxymethylbenz[d]isoxazol-3-yl)ethyl]piperidinomethyl}thiazole-4-carbonitrile

Acetonitrile (5 mL) and triethylamine (192 µL) were sequentially added to {6-cyclopropylmethoxy-3-[2-(piperidin-4-yl)ethyl]benz[d]isoxazol-7-yl}methanol (compound synthesized in Preparation Example 3-(1)) (160 mg). A solution of 2-bromomethylthiazole-4-carbonitrile (93 mg) in acetonitrile (2.5 mL) was added to the mixture, and the mixture was stirred at room temperature for three days. A saturated sodium bicarbonate solution was added to the reaction mixture, followed by extraction with ethyl acetate three times. The organic layers were washed with a saturated sodium chloride solution and then dried over magnesium sulfate. The drying agent was removed by filtration and then the solvent was evaporated under reduced pressure. The residue was purified by NH silica gel chromatography (heptane-ethyl acetate) to obtain the title compound (168 mg).
¹H-NMR (400 MHz, CDCl₃) δ(ppm): 0.34-0.43 (m, 2H), 0.63-0.73 (m, 2H), 1.26-1.45 (m, 4H), 1.74-1.84 (m, 4H), 2.15-2.26 (m, 2H), 2.88-3.00 (m, 4H), 3.76 (s, 2H), 3. 97 (d, J=7 .2 Hz, 2H), 5.03 (s, 2H), 6. 90 (d, J=8 . 4 Hz, 1H), 7.46 (d, J=8.4 Hz, 1H), 7.93 (s, 1H).

### (3) 2-{4-{2-[6-Cyclopropylmethoxy-7-(N,N-dimethylaminomethyl)benz[d]isoxazol-3-yl]ethyl}piperidinomethyl}thiazole-4-carbonitrile

The title compound was obtained by synthesis from 2-{4-[2-(6-cyclopropylmethoxy-7-hydroxymethylbenz[d]isoxazol-3-yl)ethyl]piperidinomethyl}thiazole-4-carbonitrile according to Example 179-(4).
¹H-NMR (400 MHz, CDCl₃) δ(ppm): 0.31-0.44 (m, 2H), 0.57-0.72 (m, 2H), 1.22-1.45 (m, 4H), 1.71-1.87 (m, 4H), 2.14-2.27 (m, 2H), 2.34 (s, 6H), 2.88-3.01 (m, 4H), 3.80 (s, 2H), 3. 82 (s, 2H), 3. 94 (d, J=6.8 Hz, 2H), 6.89 (d, J=8.6 Hz, 1H), 7.44 (d, J=8.6 Hz, 1H), 7.93 (s, 1H).

### (4) 2-{4-{2-[6-Cyclopropylmethoxy-7-(N,N-dimethylaminomethyl)benz[d]isoxazol-3-yl]ethyl}piperidinomethyl}thiazole-4-carbonitrile difumarate

The title compound was obtained by synthesis from 2-{4-{2-[6-cyclopropylmethoxy-7-(N,N-dimethylaminomethyl)benz[d]isoxazol-3-yl]ethyl}piperidinomethyl}thiazole-4-carbonitrile according to Example 182-(4).
¹H-NMR (400 MHz, CD₃OD) δ(ppm): 0.40-0.46 (m, 2H), 0.67-0.76 (m, 2H), 1.28-1.46 (m, 4H), 1.76-1.88 (m, 4H), 2.18-2.30 (m, 2H), 2.92-3.07 (m, 10H), 3.84-3.91 (m, 2H), 4.11 (d, J=7.2 Hz, 2H), 4.61 (s, 2H), 6.71 (s, 4H), 7.22 (d, J=9.0 Hz, 1H), 7.89 (d, J=9.0 Hz, 1H), 8.42 (s, 1H).
ESI-MS m/z: 480 [M+H]⁺.

### Example 203

### N,N-Dimethyl(3-{2-[1-(5-chloropyridin-2-ylmethyl)piperidin-4-yl]ethyl}-6-cyclopropylmethoxybenz[d]isoxazol-7-yl)methylamine trihydrochloride

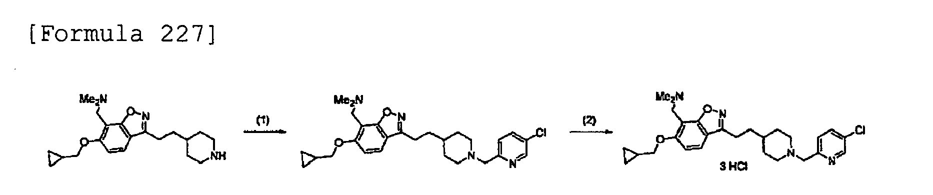

### (1) N,N-Dimethyl(3-{2-[1-(5-chloropyridin-2-ylmethyl)piperidin-4-yl]ethyl}-6-cyclopropylmethoxybenz[d]isoxazol-7-yl)methylamine

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3) and 5-chloropyridine-2-carbaldehyde [CAS Registry No. 31181-89-2] according to Example 179-(6).
¹H-NMR (400 MHz, CDCl₃) δ (ppm) : 0.31-0.44 (m, 2H), 0.57-0.71 (m, 2H), 1.24-1.45 (m, 4H), 1.66-1.85 (m, 4H), 1.97-2.10 (m, 2H), 2.33 (s, 6H), 2.81-2.90 (m, 2H), 2.93 (t, J=8.0 Hz, 2H), 3.59 (s, 2H), 3.81 (s, 2H), 3.93 (d, J=6.8 Hz, 2H), 6.88 (d, J=8.8 Hz, 1H), 7.36 (d, J=8.4 Hz, 1H), 7.43 (d, J=8.8 Hz, 1H), 7.61 (dd, J=2.4 Hz, 8.4 Hz, 1H), 8.49 (d, J=2.4 Hz, 1H).

### (2) N,N-Dimethyl(3-{2-[1-(5-chloropyridin-2-ylmethyl)piperidin-4-yl]ethyl}-6-cyclopropylmethoxybenz[d]isoxazol-7-yl)methylamine trihydrochloride

The title compound was obtained by synthesis from N,N-dimethyl(3-{2-[1-(5-chloropyridin-2-ylmethyl)piperidin-4-yl]ethyl}-6-cyclopropylmethoxybenz[d]isoxazol-7-yl)methylamine according to Example 198-(4).
¹H-NMR (400 MHz, CD₃OD) δ (ppm) : 0.40-0.50 (m, 2H), 0.65-0.74 (m, 2H), 1.35-1.46 (m, 1H), 1.54-1.93 (m, 5H), 2.04-2.14 (m, 2H), 2.97 (s, 6H), 3.03-3.19 (m, 4H), 3.55-3.65 (m, 2H), 4.12 (d, J=7.2 Hz, 2H), 4.48 (s, 2H), 4.64 (s, 2H), 7.24 (d, J=8.8 Hz, 1H), 7.53-7.61 (m, 1H), 7.90-7.99 (m, 2H), 8.68 (d, J=2.6 Hz, 1H).
ESI-MS m/z: 242 [M+2H]²⁺, 483 [M+H]⁺.

### Example 204

### 1-{4-{2-[6-Cyclopropylmethoxy-7-(N,N-dimethylaminomethyl)benz[d]isoxazol-3-yl]ethyl}piperidinomethyl}cyclopropanecarbonitrile dihydrochloride

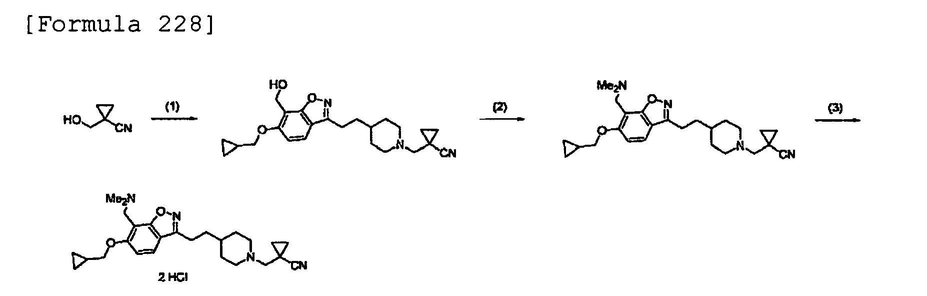

### (1) 1-{4-[2-(6-Cyclopropylmethoxy-7-hydroxymethylbenz[d]isoxazol-3-yl)ethyl]piperidinomethyl}cyclopropanecarbonitrile

1-Hydroxymethylcyclopropanecarbonitrile [CAS Registry No. 98730-77-9] (90 mg) was dissolved in dichloromethane (4 mL), and triethylamine (390 µL) was added. The reaction mixture was ice-cooled and methanesulfonyl chloride (110 µL) was added dropwise. The reaction mixture was stirred for five minutes and then a saturated ammonium chloride solution was added. The reaction mixture was extracted with chloroform three times at room temperature. The organic layers were washed with a mixed solvent of a saturated sodium bicarbonate solution and a saturated sodium chloride solution and then dried over magnesium sulfate. The drying agent was removed by filtration and then the solvent was evaporated under reduced pressure. Propionitrile (10 mL), N,N-diisopropylethylamine (320 µL), sodium iodide (90 mg) and {6-cyclopropylmethoxy-3-[2-(piperidin-4-yl)ethyl]benz[d]isoxazol-7-yl}methanol (compound synthesized in Preparation Example 3-(1)) (200 mg) were sequentially added to the residue, and the mixture was heated with stirring at 45°C for one day. The reaction mixture was cooled to room temperature and water was added, followed by extraction with chloroform three times. The organic layers were washed with a saturated sodium chloride solution and then dried over sodium sulfate. The drying agent was removed by filtration and then the solvent was evaporated under reduced pressure. The residue was purified by NH silica gel chromatography (heptane-ethyl acetate) to obtain the title compound (191 mg).
¹H-NMR (400 MHz, CDCl₃) δ (ppm) : 0.34-0.44 (m, 2H), 0.62-0.72 (m, 2H), 0.81-0.89 (m, 2H), 1.15-1.49 (m, 6H), 1.78-1.82 (m, 4H), 1.96-2.07 (m, 2H), 2.41 (s, 2H), 2.90-3.04 (m, 4H), 3.97 (d, J=7.2 Hz, 2H), 5.03 (s, 2H), 6.90 (d, J=8.4 Hz, 1H), 7.47 (d, J=8.4 Hz, 1H).

### (2) 1-{4-{2-[6-Cyclopropylmethoxy-7-(N,N-dimethylaminomethyl)benz[d]isoxazol-3-yl]ethyl}piperidinomethyl}cyclopropanecarbonitrile

The title compound was obtained by synthesis from 1-{4-[2-(6-cyclopropylmethoxy-7-hydroxymethylbenz[d]isoxazol-3-yl)ethyl]piperidinomethyl}cyclopropanecarbonitrile according to Example 179-(4).
¹H-NMR (400 MHz, CDCl₃) δ(ppm): 0.32-0.43 (m, 2H), 0.58-0.71 (m, 2H), 0.78-0.92 (m, 2H), 1.22-1.44 (m, 6H), 1.68-1.84 (m, 4H), 1.96-2.11 (m, 2H), 2.33 (s, 6H), 2.41 (s, 2H), 2.90-3.06 (m, 4H), 3.81 (s, 2H), 3.93 (d, J=6.8 Hz, 2H), 6.89 (d, J=8.8 Hz, 1H), 7.41-7.46 (m, 1H) .

### (3) 1-{4-{2-[6-Cyclopropylmethoxy-7-(N,N-dimethylaminomethyl)benz[d]isoxazol-3-yl]ethyl}piperidinomethyl}cyclopropanecarbonitrile dihydrochloride

The title compound was obtained by synthesis from 1-{4-{2-[6-cyclopropylmethoxy-7-(N,N-dimethylaminomethyl)benz[d]isoxazol-3-yl]ethyl}piperidinomethyl}cyclopropanecarbonitrile according to Example 198-(4).
¹H-NMR (400 MHz, CD₃OD) δ(ppm): 0.40-0.50 (m, 2H), 0.65-0.75 (m, 2H), 1.34-1.46 (m, 3H), 1.53-1.94 (m, 7H), 2.09-2.19 (m, 2H), 2.97 (s, 6H), 3.03-3.19 (m, 4H), 3.40 (s, 2H), 3.72-3.81 (m, 2H), 4.12 (d, J=7.2 Hz, 2H), 4.64 (s, 2H), 7.24 (d, J=8.8 Hz, 1H), 7.93 (d, J=8.8 Hz, 1H).
ESI-MS m/z: 437 [M+H]⁺.

### Example 205

### N,N-Dimethyl(3-{2-[1-(4-chloropyridin-2-ylmethyl)piperidin-4-yl]ethyl}-6-cyclopropylmethoxybenz[d]isoxazol-7-yl)methylamine trihydrochloride

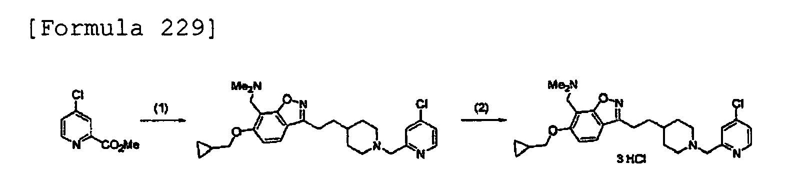

### (1) N,N-Dimethyl(3-{2-[1-(4-chloropyridin-2-ylmethyl)piperidin-4-yl]ethyl}-6-cyclopropylmethoxybenz[d]isoxazol-7-yl)methylamine

Methyl 4-chloropyridine-2-carboxylate [CAS Registry No. 24484-93-3] (200 mg) was dissolved in dichloromethane (10 mL) in a nitrogen atmosphere. The reaction mixture was cooled in a dry ice-acetone bath. Diisobutylaluminum hydride (1.01 M solution in toluene, 580 µL) was added dropwise to the reaction mixture, followed by stirring for 15 minutes. The temperature of the cooling bath was adjusted to about -45°C, and the mixture was further stirred for 15 minutes. Sodium sulfate decahydrate was carefully added to the reaction mixture until the unreacted diisobutylaluminum hydride was consumed. The reaction mixture was filtered through celite. The residue was washed with chloroform and the solvent was evaporated under reduced pressure. The residue was dissolved in dichloromethane (8 mL). N,N-Dimethyl{6-cyclopropylmethoxy-3-[2-(piperidin-4-yl)ethyl]benz[d]isoxazol-7-yl}methylamine (compound synthesized in Preparation Example 3) (100 mg), acetic acid (29 µL) and sodium triacetoxyborohydride (180 mg) were sequentially added to the solution, and the mixture was stirred at room temperature for two hours. A saturated sodium bicarbonate solution was added to the reaction mixture, followed by extraction with chloroform three times. The organic layers were washed with a saturated sodium chloride solution and then dried over magnesium sulfate. The drying agent was removed by filtration and then the solvent was evaporated under reduced pressure. The residue was purified by NH silica gel chromatography (heptane-ethyl acetate) and NH preparative TLC (heptane-ethyl acetate) to obtain the title compound (27 mg).
¹H-NMR (400 MHz, CDCl₃) δ (ppm) : 0.31-0.44 (m, 2H), 0.57-0.72 (m, 2H), 1.23-1.48 (m, 4H), 1.77-2.13 (m, 6H), 2.33 (s, 6H), 2.82-2.91 (m, 2H), 2.94 (t, J=7.8 Hz, 2H), 3.61 (s, 2H), 3.82 (s, 2H), 3.94 (d, J=6.8 Hz, 2H), 6.89 (d, J=8.6 Hz, 1H), 7.16 (dd, J=2.2 Hz, 5.4 Hz, 1H), 7.44 (d, J=8.6 Hz, 1H), 7.45 (d, J=2.2 Hz, 1H), 8.42 (d, J=5. 4 Hz, 1H) .

### (2) N,N-Dimethyl(3-{2-[1-(4-chloropyridin-2-ylmethyl)piperidin-4-yl]ethyl}-6-cyclopropylmethoxybenz[d]isoxazol-7-yl)methylamine trihydrochloride

The title compound was obtained by synthesis from N,N-dimethyl(3-{2-[1-(4-chloropyridin-2-ylmethyl)piperidin-4-yl]ethyl}-6-cyclopropylmethoxybenz[d]isoxazol-7-yl)methylamine according to Example 198-(4).
¹H-NMR (400 MHz, CD₃OD) δ(ppm): 0.40-0.50 (m, 2H), 0.65-0.75 (m, 2H), 1. 34-1 .47 (m, 1H), 1.55-1.97 (m, 5H), 2.03-2.16 (m, 2H), 2.97 (s, 6H), 3.23-3.26 (m, 4H), 3.51-3.69 (m, 2H), 4.12 (d, J=7.2 Hz, 2H), 4.52 (s, 2H), 4.64 (s, 2H), 7.24 (d, J=8.8 Hz, 1H), 7.56 (dd, J=2.0 Hz, 5.2 Hz, 1H), 7.70-7.73 (m, 1H), 7.94 (d, J=8.8 Hz, 1H), 8.61-8.65 (m, 1H).
ESI-MS m/z: 242 [M+2H]²⁺, 483 [M+H]⁺.

### Example 206

### N,N-Dimethyl{6-cyclopropylmethoxy-3-{2-[1-(1,3-dioxan-2-ylmethyl)piperidin-4-yl]ethyl}benz[d]isoxazol-7-yl}methylamine difumarate

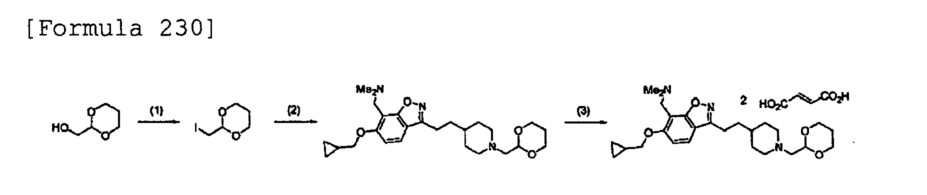

### (1) 2-Iodomethyl-1,3-dioxane

(1,3-Dioxan-2-yl)methanol [CAS Registry No. 39239-93-5] (500 mg) was dissolved in dichloromethane (30 mL). Triethylamine (1.8 mL) was added and the mixture was ice-cooled. Methanesulfonyl chloride (500 µL) was added to the reaction mixture, and the mixture was stirred at the same temperature for 10 minutes. A saturated sodium bicarbonate solution was added to the reaction mixture, followed by extraction with chloroform twice. The organic layers were washed with a saturated sodium chloride solution and then dried over magnesium sulfate. The drying agent was removed by filtration and then the solvent was evaporated under reduced pressure.
The total amount of the residue was dissolved in acetonitrile (40 mL), and sodium iodide (3.2 g) was added. The mixture was heated with stirring at 70°C overnight. The reaction mixture was cooled to room temperature. Then, a saturated sodium bicarbonate solution was added, followed by extraction with ethyl acetate three times. The organic layers were washed with a saturated sodium chloride solution and then dried over magnesium sulfate. The drying agent was removed by filtration and then the solvent was evaporated under reduced pressure. The residue was subjected to silica gel chromatography (heptane-ethyl acetate) to obtain a crude product of 1,3-dioxan-2-ylmethyl methanesulfonate. The total amount of the resulting crude product was dissolved in N,N-dimethylformamide (20 mL). Sodium iodide (2.6 g) was added and the mixture was heated with stirring at 80°C for 18 hours. Thereafter, N,N-dimethylformamide (10 mL) was added and the mixture was heated with stirring at 100°C overnight. After cooling to room temperature, water was added to the reaction mixture, followed by extraction with ethyl acetate twice. The organic layers were sequentially washed with water and a saturated sodium chloride solution and then dried over magnesium sulfate. The drying agent was removed by filtration and then the solvent was evaporated under reduced pressure. The residue was purified by silica gel chromatography (heptane-ethyl acetate) to obtain the title compound (497 mg).
¹H-NMR (400 MHz, CDCl₃) δ (ppm) : 1 .29-1 .36 (m, 1H), 2.04-2.18 (m, 1H), 3.19 (d, J=4.4 Hz, 2H), 3.79-3.88 (m, 2H), 4. 12-4. 19 (m, 2H), 4.51 (t, J=4.4 Hz, 1H).

### (2) N,N-Dimethyl{6-cyclopropylmethoxy-3-{2-[1-(1,3-dioxan-2-ylmethyl)piperidin-4-yl]ethyl}benz[d]isoxazol-7-yl}methylamine

N,N-Dimethyl{6-cyclopropylmethoxy-3-[2-(piperidin-4-yl)ethyl]benz[d]isoxazol-7-yl}methylamine (compound synthesized in Preparation Example 3) (100 mg) was dissolved in N,N-dimethylformamide (5 mL). 2-Iodomethyl-1,3-dioxane (223 mg) and N,N-diisopropylethylamine (250 µL) were added to the solution, and the mixture was heated with stirring at 100°C overnight. The reaction mixture was cooled to room temperature and then water was added, followed by extraction with ethyl acetate three times. The organic layers were sequentially washed with water and a saturated sodium chloride solution and then dried over magnesium sulfate. The drying agent was removed by filtration and then the solvent was evaporated under reduced pressure. The residue was purified by NH preparative TLC (heptane-ethyl acetate) to obtain the title compound (34 mg).
¹H-NMR (400 MHz, CDCl₃) δ(ppm): 0.30-0.44 (m, 2H), 0.57-0.72 (m, 2H), 1.21-1.44 (m, 4H), 1.64-2.19 (m, 6H), 2.38 (s, 6H), 2. 50 (d, J=4.4 Hz, 2H), 2.98-3.01 (m, 4H), 3. 71-3. 84 (m, 4H), 3.93 (d, J=6.4 Hz, 2H), 4.11 (dd, J=5.0 Hz, 11.8 Hz, 2H), 4.70 (t, J=4.4 Hz, 1H), 6.88 (d, J=8.6 Hz, 1H), 7.38-7.46 (m, 1H).

### (3) N,N-Dimethyl{6-cyclopropylmethoxy-3-{2-[1-(1,3-dioxan-2-ylmethyl)piperidin-4-yl]ethyl}benz[d]isoxazol-7-yl}methylamine difumarate

The title compound was obtained by synthesis from N,N-dimethyl{6-cyclopropylmethoxy-3-{2-[1-(1,3-dioxan-2-ylmethyl)piperidin-4-yl]ethyl}benz[d]isoxazol-7-yl}methylamine according to Example 182-(4).
¹H-NMR (400 MHz, CD₃OD) δ (ppm) : 0.38-0.48 (m, 2H), 0.66-0.74 (m, 2H), 1.28-1.47 (m, 2H), 1.53-1.74 (m, 3H), 1.79-1.89 (m, 2H), 1.95-2.14 (m, 3H), 2.90 (s, 6H), 2.93-3.09 (m, 4H), 3.19 (d, J=4.4 Hz, 2H), 3.56-3.66 (m, 2H), 3.81-3.92 (m, 2H), 4.04-4.17 (m, 4H), 4.56 (s, 2H), 5.01 (t, J=4.4 Hz, 1H), 6.64 (s, 4H), 7.20 (d, J=8.8 Hz, 1H), 7.87 (d, J=8.8 Hz, 1H).
ESI-MS m/z: 230 [M+2H]²⁺, 458 [M+H]⁺.

### Example 207

### 2-{4-{2-[6-Cyclopropylmethoxy-7-(N,N-dimethylaminomethyl)benz[d]isoxazol-3-yl]ethyl}piperidinomethyl}thiazole-5-carbonitrile difumarate

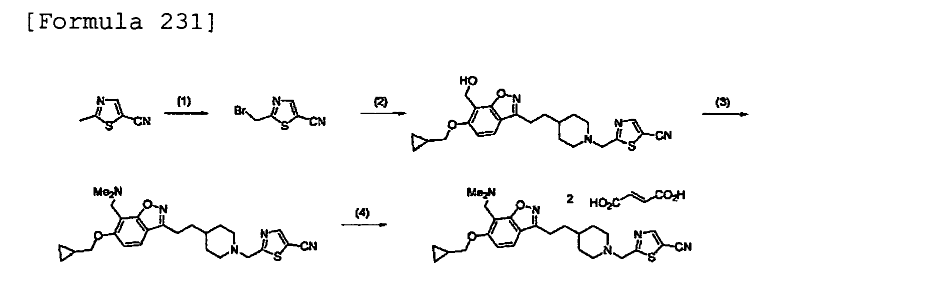

### (1) 2-Bromomethylthiazole-5-carbonitrile

The title compound was obtained by synthesis from 2-methylthiazole-5-carbonitrile [CAS Registry No. 65735-10-6] according to Example 202-(1).
¹H-NMR (400 MHz, CDCl₃) δ (ppm): 4.73 (s, 2H), 8.22 (s, 1H) .

### (2) 2-{4-[2-(6-Cyclopropylmethoxy-7-hydroxymethylbenz[d]isoxazol-3-yl)ethyl]piperidinomethyl}thiazole-5-carbonitrile

{6-Cyclopropylmethoxy-3-[2-(piperidin-4-yl)ethyl]benz[d]isoxazol-7-yl}methanol (compound synthesized in Preparation Example 3-(1)) (110 mg) was dissolved in acetonitrile (6 mL). N,N-Diisopropylethylamine (170 µL) and a solution of 2-bromomethylthiazole-5-carbonitrile (65 mg) in acetonitrile (2 mL) were sequentially added to the solution, and the mixture was stirred at room temperature overnight. Water was added to the reaction mixture, followed by extraction with ethyl acetate three times. The organic layers were washed with water and a saturated sodium chloride solution and then dried over magnesium sulfate. The drying agent was removed by filtration and then the solvent was evaporated under reduced pressure. The residue was purified by NH silica gel chromatography (heptane-ethyl acetate) to obtain the title compound (104 mg).
¹H-NMR (400 MHz, CDCl₃) δ(ppm): 0.36-0.44 (m, 2H), 0.63-0.73 (m, 2H), 1.28-1.45 (m, 4H), 1.74-1.87 (m, 4H), 2.18-2.32 (m, 2H), 2.67 (br s, 1H), 2.88-3.02 (m, 4H), 3.82 (s, 2H), 3. 97 (d, J=7.2 Hz, 2H), 5.03 (s, 2H), 6.91 (d, J=8.8 Hz, 1H), 7. 47 (d, J=8.8 Hz, 1H), 8.14 (s, 1H).

### (3) 2-{4-{2-[6-Cyclopropylmethoxy-7-(N,N-dimethylaminomethyl)benz[d]isoxazol-3-yl]ethyl}piperidinomethyl}thiazole-5-carbonitrile

The title compound was obtained by synthesis from 2-{4-[2-(6-cyclopropylmethoxy-7-hydroxymethylbenz[d]isoxazol-3-yl)ethyl]piperidinomethyl}thiazole-5-carbonitrile according to Example 179-(4).
¹H-NMR (400 MHz, CDCl₃) δ(ppm): 0.31-0.44 (m, 2H), 0.58-0.71 (m, 2H), 1.22-1.48 (m, 4H), 1.64-1.98 (m, 4H), 2.20-2.31 (m, 2H), 2.35 (s, 6H), 2.89-3.01 (m, 4H), 3.83 (s, 2H), 3.83 (s, 2H), 3.95 (d, J=6.8 Hz, 2H), 6.91 (d, J=8.8 Hz, 1H), 7.46 (d, J=8.8 Hz, 1H), 8.16 (s, 1H).

### (4) 2-{4-{2-[6-Cyclopropylmethoxy-7-(N,N-dimethylaminomethyl)benz[d]isoxazol-3-yl]ethyl}piperidinomethyl}thiazole-5-carbonitrile difumarate

The title compound was obtained by synthesis from 2-{4-{2-[6-cyclopropylmethoxy-7-(N,N-dimethylaminomethyl)benz[d]isoxazol-3-yl]ethyl}piperidinomethyl}thiazole-5-carbonitrile according to Example 182-(4).
¹H-NMR (400 MHz, CD₃OD) δ(ppm): 0.38-0.47 (m, 2H), 0.63-0.73 (m, 2H), 1.32-1.53 (m, 4H), 1.74-1.82 (m, 4H), 2.36-2.48 (m, 2H), 2.94 (s, 6H), 3.02 (t, J=7.6 Hz, 2H), 3.06-3.17 (m, 2H), 3.99-4.06 (m, 2H), 4.09 (d, J=7.2 Hz, 2H), 4.61 (s, 2H), 6.68 (s, 4H), 7.20 (d, J=8.8 Hz, 1H), 7.88 (d, J=8.8 Hz, 1H), 8.34 (s, 1H).
ESI-MS m/z: 230 [M+2H]²⁺.

### Example 208

### 5-(4-{2-[6-(3,6-Dihydro-2H-pyran-4-yl)-7-(N,N-dimethylaminomethyl)benz[d]isoxazol-3-yl]ethyl}piperidinomethyl)thiophene-2-carbonitrile dihydrochloride

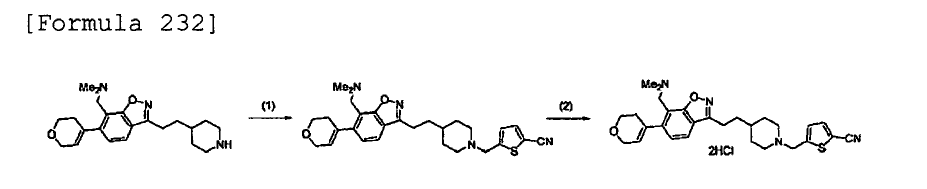

### (1) 5-(4-{2-[6-(3,6-Dihydro-2H-pyran-4-yl)-7-(N,N-dimethylaminomethyl)benz[d]isoxazol-3-yl]ethyl}piperidinomethyl)thiophene-2-carbonitrile

N,N-Dimethyl[6-(3,6-dihydro-2H-pyran-4-yl)-3-(2-piperidin-4-ylethyl)benz[d]isoxazol-7-yl]methylamine (compound synthesized in Example 185-(4)) (131 mg) was dissolved in dichloromethane (8 mL). 5-Formylthiophene-2-carbonitrile [CAS Registry No. 21512-16-3] (83 mg) and sodium triacetoxyborohydride (120 mg) were sequentially added to the solution, and the mixture was stirred at room temperature for three hours. A saturated sodium bicarbonate solution was added to the reaction mixture, followed by extraction with ethyl acetate three times. The organic layers were washed with a saturated sodium chloride solution and then dried over magnesium sulfate. The drying agent was removed by filtration and then the solvent was evaporated under reduced pressure. The residue was purified by NH silica gel chromatography (heptane-ethyl acetate) to obtain the title compound (129 mg).
¹H-NMR (400 MHz, CDCl₃) δ(ppm): 1.20-1.46 (m, 3H), 1.71-1.85 (m, 4H), 1.97-2.10 (m, 2H), 2.28 (br s, 6H), 2.41-2.49 (m, 2H), 2.86-2.95 (m, 2H), 2.98 (t, J=7.8 Hz, 2H), 3.62-3.80 (m, 4H), 3.94 (t, J=5.4 Hz, 2H), 4.33 (q, J=2.8 Hz, 2H), 5.81-5.93 (m, 1H), 6.87 (d, J=3.4 Hz, 1H), 7.11 (d, J=8.2 Hz, 1H), 7.46 (d, J=3.4 Hz, 1H), 7.49 (d, J=3.4 Hz, 1H) .

### (2) 5-(4-{2-[6-(3,6-Dihydro-2H-pyran-4-yl)-7-(N,N-dimethylaminomethyl)benz[d]isoxazol-3-yl]ethyl}piperidinomethyl)thiophene-2-carbonitrile dihydrochloride

The title compound was obtained by synthesis from 5-(4-{2-[6-(3,6-dihydro-2H-pyran-4-yl)-7-(N,N-dimethylaminomethyl)benz[d]isoxazol-3-yl]ethyl}piperidinomethyl)thiophene-2-carbonitrile according to Example 198-(4).
¹H-NMR (400 MHz, CD₃OD) δ(ppm): 1.52-1.67 (m, 2H), 1.67-1.81 (m, 1H), 1.82-1.94 (m, 2H), 2.07-2.17 (m, 2H), 2.42-2.50 (m, 2H), 2.94 (s, 6H), 3.00-3.18 (m, 4H), 3.50-3.60 (m, 2H), 3.99 (t, J=5.4 Hz, 2H), 4.35 (dd, J=2.8 Hz, 5.4 Hz, 2H), 4.65 (s, 2H), 4.77 (s, 2H), 5.88-5.92 (m, 1H), 7.37 (d, J=8.4 Hz, 1H), 7.49 (d, J=3.8 Hz, 1H), 7.80 (d, J=3.8 Hz, 1H), 7.95 (d, J=8.4 Hz, 1H) .
ESI-MS m/z: 491 [M+H]⁺.

### Example 209

### N,N-Dimethyl{3-[2-(1-benzylpiperidin-4-yl)ethyl]-6-piperidinobenz[d]isoxazol-7-yl}methylamine dihydrochloride

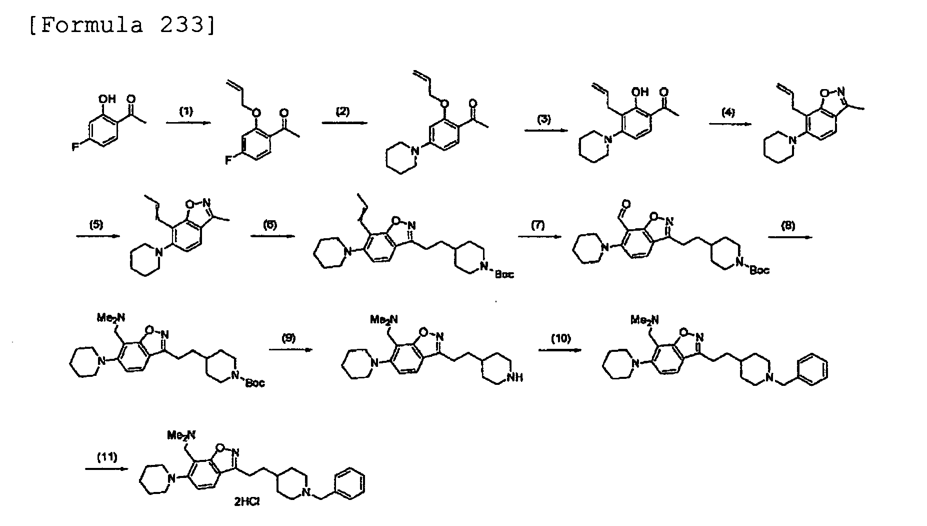

### (1) 1-(2-Allyloxy-4-fluorophenyl)ethanone

4'-Fluoro-2'-hydroxyacetophenone (5 g) was dissolved in N,N-dimethylformamide (70 mL). Potassium carbonate (4.48 g) and allyl bromide (3.5 mL) were sequentially added to the solution, and the mixture was stirred at room temperature overnight. Water was added to the reaction mixture, followed by extraction with ethyl acetate twice. The organic layers were washed with water and a saturated sodium chloride solution and then dried over magnesium sulfate. The drying agent was removed by filtration and then the solvent was evaporated under reduced pressure. The resulting solid was washed with a mixed solvent of hexane and diethyl ether to obtain the title compound (5.21 g).
¹H-NMR (400 MHz, CDCl₃) δ(ppm): 2.62 (s, 3H), 4.62 (d, J=5.2 Hz, 2H), 5. 36 (d, J=10.8 Hz, 1H), 5. 45 (d, J=17.2 Hz, 1H), 6.00-6.14 (m, 1H), 6.59-6.65 (m, 2H), 7.76-7.85 (m, 1H) .

### (2) 1-(2-Allyloxy-4-piperidinophenyl)ethanone

1-(2-Allyloxy-4-fluorophenyl)ethanone (5.16 g) was dissolved in piperidine (50 mL), and the mixture was heated with stirring at 105°C for one day. After cooling to room temperature, the solvent was evaporated under reduced pressure. The residue was purified by silica gel chromatography (heptane-ethyl acetate) to obtain the title compound (6.30 g).
¹H-NMR (400 MHz, CDCl₃) δ (ppm) : 1.58-1.77 (m, 6H), 2.58 (s, 3H), 3.26-3.37 (m, 4H), 4.63 (dt, J=1.6 Hz, 5.2 Hz, 2H), 5. 32 (dq, J=1.6 Hz, 10.4 Hz, 1H), 5. 45 (dq, J=1.6 Hz, 17.2 Hz, 1H), 6.05-6.16 (m, 1H), 6.25-6.36 (m, 1H), 6.45-6.54 (m, 1H), 7.80 (d, J=8.8 Hz, 1H).

### (3) 1-(3-Allyl-2-hydroxy-4-piperidinophenyl)ethanone

1-(2-Allyloxy-4-piperidinophenyl)ethanone (6.3 g) was dissolved in N-methylpyrrolidin-2-one (30 mL), and the mixture was heated with stirring under microwave irradiation at 180°C for 20 minutes. The reaction mixture was cooled to room temperature and then water was added, followed by extraction with ethyl acetate three times. The organic layers were sequentially washed with water and a saturated sodium chloride solution and then dried over sodium sulfate. The drying agent was removed by filtration and then the solvent was evaporated under reduced pressure. The residue was purified by NH silica gel chromatography (heptane-ethyl acetate) and silica gel chromatography (heptane-ethyl acetate) to obtain the title compound (6.16 g).
¹H-NMR (400 MHz, CDCl₃) δ(ppm): 1.54-1.65 (m, 2H), 1.65-1.76 (m, 4H), 2.56 (s, 3H), 2.88-3.00 (m, 4H), 3.43 (dt, J=1.8 Hz, 6.0 Hz, 2H), 4.97-5.07 (m, 2H), 6.05-6.16 (m, 1H), 6.54 (d, J=8.8 Hz, 1H), 7.56 (d, J=8.8 Hz, 1H), 12.78 (s, 1H) .

### (4) 7-Allyl-3-methyl-6-piperidinobenz[d]isoxazole

1-(3-Allyl-2-hydroxy-4-piperidinophenyl)ethanone (6.16 g) was dissolved by adding ethanol (60 mL) and water (25 mL) and heating. Hydroxylamine hydrochloride (4.13 g) and sodium acetate (5.08 g) were sequentially added to the solution, and the mixture was heated with stirring at 100°C for two hours. Hydroxylamine hydrochloride (2 g) and sodium acetate (2.5 g) were sequentially added and the mixture was further heated with stirring at 100°C for three hours. The reaction mixture was cooled to room temperature, and then the solvent was evaporated under reduced pressure to reduce the amount of the solvent. A saturated sodium bicarbonate solution was added to the concentrated reaction mixture, followed by sequential extraction with ethyl acetate once and chloroform three times. The organic layers were washed with a saturated sodium chloride solution and then dried over magnesium sulfate. The drying agent was removed by filtration and then the solvent was evaporated under reduced pressure. The residue was dissolved in N,N-dimethylformamide (20 mL). Acetic anhydride (5 mL) and sodium acetate (4.13 g) were sequentially added to the solution, and the mixture was heated with stirring at 145°C for 40 minutes. The reaction mixture was cooled to room temperature and then filtered through celite. The insoluble matter was washed with N,N-dimethylformamide. The filtrate was concentrated under reduced pressure. The residue was purified by NH silica gel chromatography (heptane-ethyl acetate) to obtain the title compound (4.23 g).
¹H-NMR (400 MHz, CDCl₃) δ(ppm): 1.56-1.63 (m, 2H), 1.69-1.79 (m, 4H), 2.52 (s, 3H), 2.91 (t, J=5.2 Hz, 4H), 3.68-3.75 (m, 2H), 5.04 (dq, J=1.6 Hz, 10.0 Hz, 1H), 5.10 (dq, J=1.6 Hz, 17.2 Hz, 1H), 6.07-6.18 (m, 1H), 7.07 (d, J=8.4 Hz, 1H), 7.39 (d, J=8.4 Hz, 1H) .

### (5) Mixture of 3-methyl-6-piperidino-7-[(E)-1-propenyl]benz[d]isoxazole and 3-methyl-6-piperidino-7-[(Z)-1-propenyl]benz[d]isoxazole

7-Allyl-3-methyl-6-piperidinobenz[d]isoxazole (4.13 g) was dissolved in ethanol (20 mL) and dimethyl sulfoxide (20 mL). Powdery potassium hydroxide (3.63 g) was added to the solution, and the mixture was heated with stirring at 110°C overnight. The reaction mixture was cooled to room temperature and then water was added, followed by extraction with ethyl acetate twice. The organic layers were washed with water and a saturated sodium chloride solution and then dried over magnesium sulfate. The drying agent was removed by filtration and then the solvent was evaporated under reduced pressure. The residue was purified by NH silica gel chromatography (heptane-ethyl acetate) and silica gel chromatography (heptane-ethyl acetate) to obtain the title compound (3.78 g).
¹H-NMR (400 MHz, CDCl₃) δ(ppm): 1.56-1.66 (m, 2H), 1.66-1.80 (m, 4H), 1.99-2.05 (m, 3H), 2.52 (s, 3H), 2.90-3.04 (m, 4H), 6.61-6.69 (m, 1H), 6.88-7.03 (m, 2H), 7.32 (d, J=8.4 Hz, 1H).

### (6) Mixture of tert-butyl 4-{2-[6-piperidino-7-[(E)-1-propenyl]benz[d]isoxazol-3-yl]ethyl}piperidine-1-carboxylate and tert-butyl 4-{2-[6-piperidino-7-[(Z)-1-propenyl]benz[d]isoxazol-3-yl]ethyl}piperidine-1-carboxylate

The title compound was obtained by synthesis from the mixture of 3-methyl-6-piperidino-7-[(E)-1-propenyl]benz[d]isoxazole and 3-methyl-6-piperidino-7-[(Z)-1-propenyl]benz[d]isoxazole according to Preparation Example 2-(5).
¹H-NMR (400 MHz, CDCl₃) δ (ppm) : 1.08-1.22 (m, 2H), 1.39-1.64 (m, 12H), 1.64-1.82 (m, 8H), 1.98-2.05 (m, 3H), 2.58-2.75 (m, 2H), 2.87-3.05 (m, 6H), 3.95-4.22 (m, 2H), 6.60-6.68 (m, 1H), 6.87-7.01 (m, 2H), 7.33 (d, J=8.4 Hz, 1H).

### (7) tert-Butyl 4-[2-(7-formyl-6-piperidinobenz[d]isoxazol-3-yl)ethyl]piperidine-1-carboxylate

The mixture of tert-butyl 4-{2-[6-piperidino-7-[(E)-1-propenyl]benz[d]isoxazol-3-yl]ethyl}piperidine-1-carboxylate and tert-butyl 4-{2-[6-piperidino-7-[(Z)-1-propenyl]benz[d]isoxazol-3-yl]ethyl}piperidine-1-carboxylate (6.13 g) was dissolved in tetrahydrofuran (20 mL), tert-butanol (60 mL) and water (60 mL). Methanesulfonamide (1.28 g) and AD-MIX-β (17.4 g) were sequentially added to the solution, and the mixture was stirred at room temperature for two days. Sodium sulfite (20 g) was added to the reaction mixture, and the mixture was further stirred for 30 minutes. A saturated sodium chloride solution was added to the reaction mixture, followed by extraction with ethyl acetate three times. The organic layers were washed with a saturated sodium chloride solution and then dried over sodium sulfate. The drying agent was removed by filtration and then the solvent was evaporated under reduced pressure. The residue was dissolved in tetrahydrofuran (180 mL) and water (60 mL). Sodium periodate (8.66 g) was added to the solution, and the mixture was stirred at room temperature for one hour. A saturated sodium chloride solution was added to the reaction mixture, followed by extraction with ethyl acetate three times. The organic layers were washed with a saturated sodium chloride solution and then dried over magnesium sulfate. The drying agent was removed by filtration and then the solvent was evaporated under reduced pressure. The residue was purified by silica gel chromatography (heptane-ethyl acetate) to obtain the title compound (4.85 g).
¹H-NMR (400 MHz, CDCl₃) δ (ppm) : 1.08-1.22 (m, 2H), 1.42-1.60 (m, 12H), 1.62-1.85 (m, 8H), 2.59-2.74 (m, 2H), 2.96 (t, J=7.8 Hz, 2H), 3.27 (t, J=5.4 Hz, 4H), 3.98-4.18 (m, 2H), 7.01 (d, J=8.6 Hz, 1H), 7.63 (d, J=8.6 Hz, 1H), 10.33 (s, 1H).

### (8) tert-Butyl 4-{2-[7-(N,N-dimethylaminomethyl)-6-piperidinobenz[d]isoxazol-3-yl]ethyl}piperidine-1-carboxylate

The title compound was obtained by synthesis from tert-butyl 4-[2-(7-formyl-6-piperidinobenz[d]isoxazol-3-yl)ethyl]piperidine-1-carboxylate and dimethylamine (2 M solution in tetrahydrofuran) according to Example 179-(6).
¹H-NMR (400 MHz, CDCl₃) δ(ppm): 1.09-1.24 (m, 2H), 1.42-1.67 (m, 12H), 1.69-1.83 (m, 8H), 2.33 (s, 6H), 2.59-2.76 (m, 2H), 2.95 (t, J=7.8 Hz, 2H), 3.04 (t, J=5.2 Hz, 4H), 3.75 (s, 2H), 3.94-4.20 (m, 2H), 7.06 (d, J=8.4 Hz, 1H), 7.44 (d, J=8.4 Hz, 1H) .

### (9) N,N-Dimethyl[6-piperidino-3-(2-piperidin-4-ylethyl)benz[d]isoxazol-7-yl]methylamine

tert-Butyl 4-{2-[7-(N,N-dimethylaminomethyl)-6-piperidinobenz[d]isoxazol-3-yl]ethyl}-piperidine-1-carboxylate (415 mg) was dissolved in dichloromethane (4.5 mL). Trifluoroacetic acid (1.5 mL) was added to the solution, and the mixture was stirred at room temperature for 15 minutes. Chloroform was added to the reaction mixture, followed by ice-cooling. Then, aqueous ammonia, a saturated sodium bicarbonate solution and a saturated sodium chloride solution were sequentially added. The reaction mixture was extracted with chloroform three times at room temperature. The organic layers were dried over magnesium sulfate. The drying agent was removed by filtration and then the solvent was evaporated under reduced pressure. The title compound (374 mg) was obtained as a residue.
¹H-NMR (400 MHz, CDCl₃) δ (ppm) : 1.12-1.25 (m, 2H), 1.45-1.69 (m, 3H), 1.70-1.82 (m, 8H), 1.90-2.04 (m, 1H), 2.33 (s, 6H), 2.55-2.65 (m, 2H), 2.91-2.99 (m, 2H), 3.00-3.14 (m, 6H), 3.75 (s, 2H), 7.07 (d, J=8 .4 Hz, 1H), 7.46 (d, J=8.4 Hz, 1H).

### (10) N,N-Dimethyl{3-[2-(1-benzylpiperidin-4-yl)ethyl]-6-piperidinobenz[d]isoxazol-7-yl}methylamine

The title compound was obtained by synthesis from N,N-dimethyl{6-piperidino-3-[2-(piperidin-4-yl)ethyl]benz[d]isoxazol-7-yl}methylamine (compound synthesized in Example 209-(9)) and benzaldehyde according to Example 179-(6).
¹H-NMR (400 MHz, CDCl₃) δ(ppm): 1.24-1.43 (m, 3H), 1.54-1.64 (m, 2H), 1.67-1.81 (m, 8H), 1.86-2.01 (m, 2H), 2.32 (s, 6H), 2.83-2.97 (m, 4H), 3.03 (t, J=5.2 Hz, 4H), 3.48 (s, 2H), 3.74 (s, 2H), 7.04 (d, J=8.4 Hz, 1H), 7.19-7.32 (m, 5H), 7.43 (d, J=8.4 Hz, 1H).

### (11) N,N-Dimethyl{3-[2-(1-benzylpiperidin-4-yl)ethyl]-6-piperidinobenz[d]isoxazol-7-yl}methylamine dihydrochloride

The title compound was obtained by synthesis from N,N-dimethyl{3-[2-(1-benzylpiperidin-4-yl)ethyl]-6-piperidinobenz[d]isoxazol-7-yl}methylamine according to Example 198-(4).
¹H-NMR (400 MHz, CD₃OD) δ (ppm) : 1.47-1.61 (m, 2H), 1.61-1.78 (m, 3H), 1.78-1.94 (m, 6H), 1.94-2.14 (m, 2H), 2. 92-3. 14 (m, 14H), 3.45-3.54 (m, 2H), 4.31 (s, 2H), 4.71 (s, 2H), 7.43 (d, J=8.6 Hz, 1H), 7.46-7.57 (m, 5H), 7.92 (d, J=8.6 Hz, 1H).
ESI-MS m/z: 209 [M+H-NMe₂]²⁺, 416 [M-NMe₂]⁺, 461 [M+H]⁺.

### Example 210

### 5-{4-{2-[7-(N,N-Dimethylaminomethyl)-6-piperidinobenz[d]isoxazol-3-yl]ethyl}piperidinomethyl}thiophene-2-carbonitrile dihydrochloride

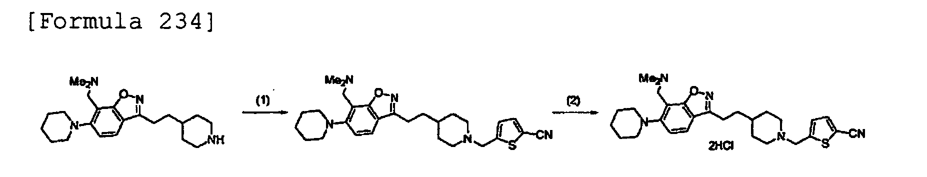

### (1) 5-{4-{2-[7-(N,N-Dimethylaminomethyl)-6-piperidinobenz[d]isoxazol-3-yl]ethyl}piperidinomethyl}thiophene-2-carbonitrile

The title compound was obtained by synthesis from N,N-dimethyl{6-piperidino-3-[2-(piperidin-4-yl)ethyl]benz[d]isoxazol-7-yl}methylamine (compound synthesized in Example 209-(9)) and 5-formylthiophene-2-carbonitrile [CAS Registry No. 21512-16-3] according to Example 179-(6).
¹H-NMR (400 MHz, CDCl₃) δ(ppm): 1.23-1.44 (m, 3H), 1.67-1.89 (m, 8H), 1.97-2.09 (m, 2H), 2.33 (s, 6H), 2.84-2.98 (m, 4H), 3.03 (t, J=5.0 Hz, 4H), 3.68 (s, 2H), 3. 75 (s, 2H), 6. 87 (d, J=3.4 Hz, 1H), 7.05 (d, J=8.2 Hz, 1H), 7.44 (d, J=8.2 Hz, 1H), 7.45 (d, J=3.4 Hz, 1H) .

### (2) 5-{4-{2-[7-(N,N-Dimethylaminomethyl)-6-piperidinobenz[d]isoxazol-3-yl]ethyl}piperidinomethyl}thiophene-2-carbonitrile dihydrochloride

The title compound was obtained by synthesis from 5-{4-{2-[7-(N,N-dimethylaminomethyl)-6-piperidinobenz[d]isoxazol-3-yl]ethyl}piperidinomethyl}thiophene-2-carbonitrile according to Example 198-(4).
¹H-NMR (400 MHz, CD₃OD) δ(ppm): 1.48-1.94 (m, 11H), 1.94-2.15 (m, 2H), 2.88-3.14 (m, 14H), 3.50-3.60 (m, 2H), 4.64 (s, 2H), 4.71 (s, 2H), 7.43 (d, J=8.6 Hz, 1H), 7.49 (d, J=3.8 Hz, 1H), 7.80 (d, J=3.8 Hz, 1H), 7.93 (d, J=8.6 Hz, 1H).
ESI-MS m/z: 492 [M+H]⁺.

### Example 211

### N,N-Dimethyl{3-{2-[1-(1,3-dioxolan-2-ylmethyl)piperidin-4-yl]ethyl}-6-piperidinobenz[d]isoxazol-7-yl}methylamine fumarate

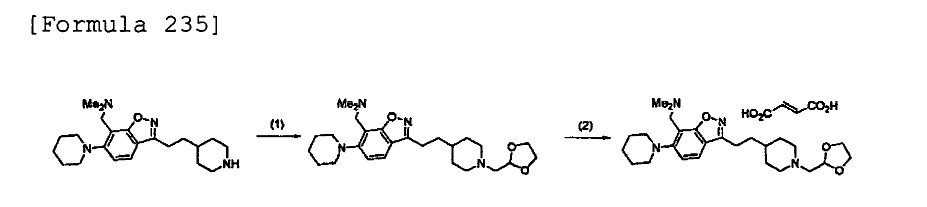

### (1) N,N-Dimethyl{3-{2-[1-(1,3-dioxolan-2-ylmethyl)piperidin-4-yl]ethyl}-6-piperidinobenz[d]isoxazol-7-yl}methylamine

N,N-Dimethyl[6-piperidino-3-(2-piperidine-4-ylethyl)benz[d]isoxazol-7-yl]methylamine (compound synthesized in Example 209-(9)) (156 mg) was dissolved in propionitrile (5 mL). N,N-Diisopropylethylamine (450 µL), 2-bromomethyl-1,3-dioxolane (130 µL) and sodium iodide (63 mg) were sequentially added and the mixture was heated with stirring at 90°C overnight. The reaction mixture was cooled to room temperature. Then, a saturated sodium chloride solution was added, followed by extraction with ethyl acetate twice. The organic layers were washed with water and a saturated sodium chloride solution and then dried over magnesium sulfate. The drying agent was removed by filtration and then the solvent was evaporated under reduced pressure. The residue was purified by NH silica gel chromatography (heptane-ethyl acetate) to obtain the title compound (80 mg).
¹H-NMR (400 MHz, CDCl₃) δ(ppm) : 1.29-1.44 (m, 3H), 1.54-1.64 (m, 2H), 1.78-1.82 (m, 8H), 1.87-2.01 (m, 2H), 2.33 (s, 6H), 2.56 (d, J=4.4 Hz, 2H), 2.84-3.13 (m, 8H), 3.75 (s, 2H), 3.81-3.90 (m, 2H), 3.91-3.99 (m, 2H), 5.00 (t, J=4.4 Hz, 1H), 7.05 (d, J=8.4 Hz, 1H), 7.43 (d, J=8.4 Hz, 1H) .

### (2) N,N-Dimethyl{3-{2-[1-(1,3-dioxolan-2-ylmethyl)piperidin-4-yl]ethyl}-6-piperidinobenz[d]isoxazol-7-yl}methylamine fumarate

The title compound was obtained by synthesis from N,N-dimethyl{3-{2-[1-(1,3-dioxolan-2-ylmethyl)piperidin-4-yl]ethyl}-6-piperidinobenz[d]isoxazol-7-yl}methylamine according to Example 182-(4).
¹H-NMR (400 MHz, CD₃OD) δ(ppm): 1.44-1.61 (m, 3H), 1.61-1.71 (m, 2H), 1.71-1.88 (m, 6H), 1.88-1.99 (m, 2H), 2.61-2.71 (m, 2H), 2.76 (s, 6H), 2.92-3.08 (m, 8H), 3.36-3.46 (m, 2H), 3.85-3.93 (m, 2H), 3.96-4.04 (m, 2H), 4.46 (s, 2H), 5.14 (t, J=4.4 Hz, 1H), 6.64 (s, 2H), 7.36 (d, J=8.4 Hz, 1H), 7.83 (d, J=8.4 Hz, 1H).
ESI-MS m/z: 207 [M+H-NMe₂]²⁺, 457 [M+H]⁺.

### Example 212

### N,N-Dimethyl{3-[2-(1-benzylpiperidin-4-yl)ethyl]-6-morpholinobenz[d]isoxazol-7-yl}methylamine dihydrochloride

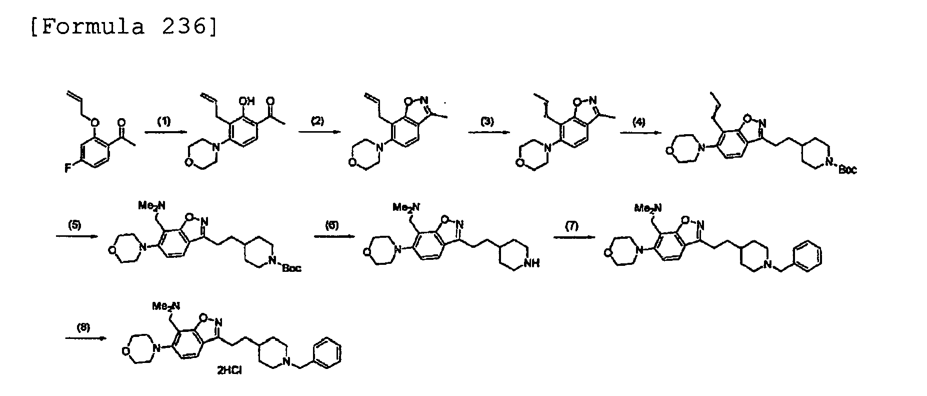

### (1) 1-(3-Allyl-2-hydroxy-4-morpholinophenyl)ethanone

1-(2-Allyloxy-4-fluorophenyl)ethanone (7 g) was dissolved in morpholine (70 mL), and the mixture was heated with stirring at 125°C for one day. The reaction mixture was cooled to room temperature, and then the solvent was evaporated under reduced pressure. The residue was subjected to silica gel chromatography (heptane-ethyl acetate) to obtain a crude product of 1-(2-allyloxy-4-morpholinophenyl)ethanone. The crude product was dissolved in N-methylpyrrolidin-2-one (33 mL). The solution was heated with stirring under microwave irradiation at 180°C for 20 minutes. The reaction mixture was cooled to room temperature and then water was added, followed by extraction with ethyl acetate three times. The organic layers were sequentially washed with water and a saturated sodium chloride solution and then dried over sodium sulfate. The drying agent was removed by filtration and then the solvent was evaporated under reduced pressure. The residue was purified by NH silica gel chromatography (heptane-ethyl acetate) and silica gel chromatography (heptane-ethyl acetate) to obtain the title compound (4.13 g).
¹H-NMR (400 MHz, CDCl₃) δ (ppm): 2.59 (s, 3H), 3.02 (t, J=4.6 Hz, 4H), 3.48 (dt, J=1.8 Hz, 5.6 Hz, 2H), 3.85 (t, J=4.6 Hz, 4H), 4.94-5.07 (m, 2H), 6.03-6.15 (m, 1H), 6.59 (d, J=8.8 Hz, 1H), 7.63 (d, J=8.8 Hz, 1H), 12.80 (s, 1H).

### (2) 7-Allyl-3-methyl-6-morpholinobenz[d]isoxazole

The title compound was synthesized from 1-(3-allyl-2-hydroxy-4-morpholinophenyl)ethanone according to Example 209-(4).
¹H-NMR (400 MHz, CDCl₃) δ (ppm) : 2.54 (s, 3H), 2.99 (t, J=4.6 Hz, 4H), 3.76 (dt, J=1.6 Hz, J=6.0 Hz, 2H), 3.88 (t, J=4.6 Hz, 4H), 5.00-5.08 (m, 2H), 6.06-6.16 (m, 1H), 7.10 (d, J=8.4 Hz, 1H), 7.44 (d, J=8.4 Hz, 1H).

### (3) Mixture of 3-methyl-6-morpholino-7-[(E)-1-propenyl]benz[d]isoxazole and 3-methyl-6-morpholino-7-[(Z)-1-propenyl]benz[d]isoxazole

The title compound was synthesized from 7-allyl-3-methyl-6-morpholinobenz[d]isoxazole according to Example 209-(5).
¹H-NMR (400 MHz, CDCl₃) δ(ppm): 2.02 (dd, J=1.6 Hz, 6.4 Hz, 3H), 2.54 (s, 3H), 3.04 (t, J=4.6 Hz, 4H), 3.90 (t, J=4.6 Hz, 4H), 6.65-6.72 (m, 1H), 6.92-7.06 (m, 2H), 7.38 (d, J=8.4 Hz, 1H).

### (4) Mixture of tert-butyl 4-{2-[6-morpholino-7-[(E)-1-propenyl]benz[d]isoxazol-3-yl]ethyl}piperidine-1-carboxylate and tert-butyl 4-{2-[6-morpholino-7-[(Z)-1-propenyl]benz[d]isoxazol-3-yl]ethyl}piperidine-1-carboxylate

The title compound was obtained by synthesis from the mixture of 3-methyl-6-morpholino-7-[(E)-1-propenyl]benz[d]isoxazole and 3-methyl-6-morpholino-7-[(Z)-1-propenyl]benz[d]isoxazole according to Preparation Example 2-(5).
¹H-NMR (400 MHz, CDCl₃) δ (ppm) : 1.09-1.23 (m, 2H), 1.40-1.67 (m, 10H), 1.70-1.83 (m, 4H), 2.02 (dd, J=1.6 Hz, 6.8 Hz, 3H), 2.61-2.74 (m, 2H), 2.98 (t, J=7.6 Hz, 2H), 3.05 (t, J=4.6 Hz, 4H), 3.91 (t, J=4.6 Hz, 4H), 4.02-4.16 (m, 2H), 6.65-6.72 (m, 1H), 6.91-7.05 (m, 2H), 7.39 (d, J=8.4 Hz, 1H) .

### (5) tert-Butyl 4-{2-[7-(N,N-dimethylaminomethyl)-6-morpholinobenz[d]isoxazol-3-yl]ethyl}piperidine-1-carboxylate

The title compound was synthesized from tert-butyl 4-[2-(6-morpholino-7-propenylbenz[d]isoxazol-3-yl)ethyl]piperidine-1-carboxylate with reference to Org. Lett., 6(19)-3217, (2004).
The mixture of tert-butyl 4-{2-[6-morpholino-7-[(E)-1-propenyl]benz[d]isoxazol-3-yl]ethyl}piperidine-1-carboxylate and tert-butyl 4-{2-[6-morpholino-7-[(Z)-1-propenyl]benz[d]isoxazol-3-yl]ethyl}piperidine-1-carboxylate (3.62 g) was dissolved in 1,4-dioxane (75 mL) and water (25 mL). 2,6-Lutidine (1.9 mL), osmium tetroxide (2.5% solution in tert-butyl alcohol, 3 mL) and sodium periodate (4.38 g) were sequentially added to the solution, and the mixture was stirred at room temperature for 1.5 hours. Water was added to the reaction mixture, followed by extraction with ethyl acetate three times. The organic layers were washed with a saturated sodium chloride solution and then dried over magnesium sulfate. The drying agent was removed by filtration and then the solvent was evaporated under reduced pressure. The residue was dissolved in dichloromethane (90 mL). Dimethylamine (2 M solution in tetrahydrofuran, 20 mL), acetic acid (2.3 mL) and sodium triacetoxyborohydride (3.37 g) were sequentially added to the solution, and the mixture was stirred at room temperature overnight. A saturated sodium bicarbonate solution was added to the reaction mixture, followed by extraction with chloroform three times. The organic layers were washed with a saturated sodium chloride solution and then dried over magnesium sulfate. The drying agent was removed by filtration and then the solvent was evaporated under reduced pressure. The residue was purified by NH silica gel chromatography (heptane-ethyl acetate) to obtain the title compound (2.71 g).
¹H-NMR (400 MHz, CDCl₃) δ (ppm) : 1.09-1.22 (m, 2H), 1.39-1.66 (m, 10H), 1.69-1.83 (m, 4H), 2.32 (s, 6H), 2.57-2. 77 (m, 2H), 2.96 (t, J=7.8 Hz, 2H), 3.19 (t, J=4.6 Hz, 4H), 3.76 (s, 2H), 3.88 (t, J=4.6 Hz, 4H), 3.95-4.22 (m, 2H), 7.07 (d, J=8.4 Hz, 1H), 7.49 (d, J=8.4 Hz, 1H).

### (6) N,N-Dimethyl{6-morpholino-3-[2-(piperidin-4-yl)ethyl]benz[d]isoxazol-7-yl}methylamine

The title compound was obtained by synthesis from tert-butyl 4-{2-[7-(N,N-dimethylaminomethyl)-6-morpholinobenz[d]isoxazol-3-yl]ethyl}piperidine-1-carboxylate according to Example 209-(9).
¹H-NMR (400 MHz, CDCl₃) δ (ppm) : 1.12-1.24 (m, 2H), 1.42-1.55 (m, 1H), 1.65-1.86 (m, 4H), 2.31 (s, 6H), 2.54-2.65 (m, 2H), 2.96 (t, J=7.8 Hz, 2H), 3.04-3.12 (m, 2H), 3.20 (t, J=4.4 Hz, 4H), 3.75 (s, 2H), 3.88 (t, J=4.4 Hz, 4H), 7.06 (d, J=8.6 Hz, 1H), 7.49 (d, J=8.6 Hz, 1H).

### (7) N,N-Dimethyl{3-[2-(1-benzylpiperidin-4-yl)ethyl]-6-morpholinobenz[d]isoxazol-7-yl}methylamine

The title compound was obtained by synthesis from N,N-dimethyl{6-morpholino-3-[2-(piperidin-4-yl)ethyl]benz[d]isoxazol-7-yl}methylamine and benzaldehyde according to Example 179-(6).
¹H-NMR (400 MHz, CDCl₃) δ (ppm): 1.23-1.44 (m, 3H), 1.67-1.85 (m, 4H), 1.88-2.00 (m, 2H), 2.31 (s, 6H), 2.83-2.98 (m, 4H), 3.19 (t, J=4.6 Hz, 4H), 3.48 (s, 2H), 3.74 (s, 2H), 3.87 (t, J=4.6 Hz, 4H), 7.05 (d, J=8.4 Hz, 1H), 7.19-7.32 (m, 5H), 7.48 (d, J=8.4 Hz, 1H).

### (8) N,N-Dimethyl{3-[2-(1-benzylpiperidin-4-yl)ethyl]-6-morpholinobenz[d]isoxazol-7-yl}methylamine dihydrochloride

The title compound was obtained by synthesis from N,N-dimethyl{3-[2-(1-benzylpiperidin-4-yl)ethyl]-6-morpholinobenz[d]isoxazol-7-yl}methylamine according to Example 198-(4).
¹H-NMR (400 MHz, CD₃OD) δ (ppm): 1.48-1.63 (m, 2H), 1.65-1.80 (m, 1H), 1.80-1.94 (m, 2H), 1.99-2.14 (m, 2H), 2.93-3.14 (m, 14H), 3.45-3.54 (m, 2H), 3.91 (t, J=4.4 Hz, 4H), 4.31 (s, 2H), 4.73 (s, 2H), 7.44-7.58 (m, 6H), 7.96 (d, J=8.4 Hz, 1H).
ESI-MS m/z: 232 [M+2H]²⁺, 463 [M+H]⁺.

### Example 213

### 5-{4-{2-[7-(N,N-Dimethylaminomethyl)-6-morpholinobenz[d]isoxazol-3-yl]ethyl}piperidinomethyl}thiophene-2-carbonitrile dihydrochloride

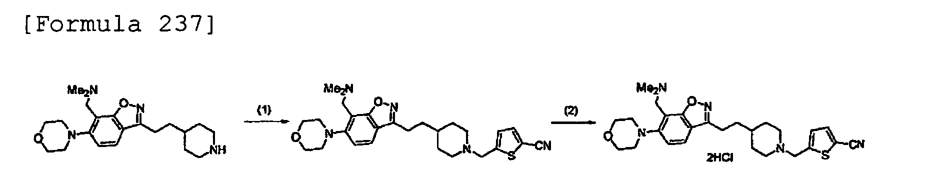

### (1) 5-{4-{2-[7-(N,N-Dimethylaminomethyl)-6-morpholinobenz[d]isoxazol-3-yl]ethyl}piperidinomethyl}thiophene-2-carbonitrile

The title compound was obtained by synthesis from N,N-dimethyl{6-morpholino-3-[2-(piperidin-4-yl)ethyl]benz[d]isoxazol-7-yl}methylamine (compound synthesized in Example 212-(6)) and 5-formylthiophene-2-carbonitrile [CAS Registry No. 21512-16-3] according to Example 179-(6).
¹H-NMR (400 MHz, CDCl₃) δ (ppm): 1.23-1.44 (m, 3H), 1.57-1.84 (m, 4H), 1.97-2.10 (m, 2H), 2.32 (s, 6H), 2.84-3.00 (m, 4H), 3.19 (t, J=4.4 Hz, 4H), 3.68 (s, 2H), 3.75 (br s, 2H), 3.88 (t, J=4.4 Hz, 4H), 6.87 (d, J=3.6 Hz, 1H), 7.06 (d, J=8.4 Hz, 1H), 7.46 (d, J=3.6 Hz, 1H), 7.49 (d, J=8.4 Hz, 1H).

### (2) 5-{4-{2-[7-(N,N-Dimethylaminomethyl)-6-morpholinobenz[d]isoxazol-3-yl]ethyl}piperidinomethyl}thiophene-2-carbonitrile dihydrochloride

The title compound was obtained by synthesis from 5-{4-{2-[7-(N,N-dimethylaminomethyl)-6-morpholinobenz[d]isoxazol-3-yl]ethyl}piperidinomethyl}thiophene-2-carbonitrile according to Example 198-(4).
¹H-NMR (400 MHz, CD₃OD) δ (ppm): 1.48-1.78 (m, 3H), 1.81-1.93 (m, 2H), 2.00-2.14 (m, 2H), 2.86-3.13 (m, 14H), 3.40-3.57 (m, 2H), 3.91 (t, J=4.6 Hz, 4H), 4.57 (br s, 2H), 4.73 (s, 2H), 7.42-7.49 (m, 2H), 7.78 (d, J=4.0 Hz, 1H), 7.97 (d, J=8.4 Hz, 1H).
ESI-MS m/z: 449 [M-NMe₂]⁺, 494 [M+H]⁺.

### Example 214

### N,N-Dimethyl(3-{2-[1-(4-chlorobenzyl)piperidin-4-yl]ethyl}-6-morpholinobenz[d]isoxazol-7-yl)methylamine dihydrochloride

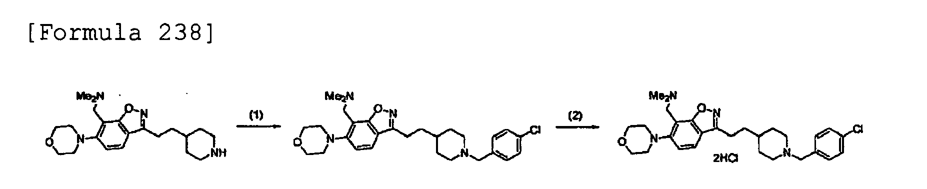

### (1) N,N-Dimethyl(3-{2-[1-(4-chlorobenzyl)piperidin-4-yl]ethyl}-6-morpholinobenz[d]isoxazol-7-yl)methylamine

The title compound was obtained by synthesis from N,N-dimethyl{6-morpholino-3-[2-(piperidin-4-yl)ethyl]benz[d]isoxazol-7-yl}methylamine (compound synthesized in Example 212-(6)) and 4-chlorobenzaldehyde according to Example 179-(6).
¹H-NMR (400 MHz, CDCl₃) δ (ppm) : 1.23-1.44 (m, 3H), 1.68-1.82 (m, 4H), 1.87-2.00 (m, 2H), 2.31 (s, 6H), 2.79-2.90 (m, 2H), 2.94 (t, J=8.0 Hz, 2H), 3.19 (t, J=4.6
Hz, 4H), 3.44 (s, 2H), 3.74 (s, 2H), 3.87 (t, J=4.6 Hz, 4H), 7.05 (d, J=8.4 Hz, 1H), 7.19-7.29 (m, 4H), 7.48 (d, J=8.4 Hz, 1H).

### (2) N,N-Dimethyl(3-{2-[1-(4-chlorobenzyl)piperidin-4-yl]ethyl}-6-morpholinobenz[d]isoxazol-7-yl)methylamine dihydrochloride

The title compound was obtained by synthesis from N,N-dimethyl(3-{2-[1-(4-chlorobenzyl)piperidin-4-yl]ethyl}-6-morpholinobenz[d]isoxazol-7-yl)methylamine according to Example 198-(4).
¹H-NMR (400 MHz, CD₃OD) δ (ppm): 1.48-1.62 (m, 2H), 1.65-1.79 (m, 1H), 1.80-1.94 (m, 2H), 2.04-2.13 (m, 2H), 2.93-3.15 (m, 14H), 3.44-3.54 (m, 2H), 3.91 (t, J=4.4 Hz, 4H), 4.31 (s, 2H), 4.73 (s, 2H), 7.46 (d, J=8.4 Hz, 1H), 7.48-7.58 (m, 4H), 7.96 (d, J=8.4 Hz, 1H).
ESI-MS m/z: 249 [M+2H]²⁺, 452 [M-NMe₂]⁺, 497 [M+H]⁺.

### Example 215

### N,N-Dimethyl(3-{2-[1-(4-chlorobenzyl)piperidin-4-yl]ethyl}-6-piperidinobenz[d]isoxazol-7-yl)methylamine dihydrochloride

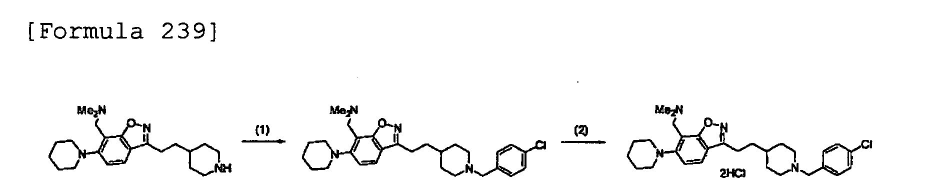

### (1) N,N-Dimethyl{3-{2-[1-(4-chlorobenzyl)piperidin-4-yl]ethyl}-6-piperidinobenz[d]isoxazol-7-yl}methylamine

The title compound was obtained by synthesis from N,N-dimethyl{6-piperidino-3-[2-(piperidin-4-yl)ethyl]benz[d]isoxazol-7-yl}methylamine (compound synthesized in Example 209-(9)) and 4-chlorobenzaldehyde according to Example 179-(6).
¹H-NMR (400 MHz, CDCl₃) δ (ppm): 1.23-1.44 (m, 3H), 1.54-1.64 (m, 2H), 1.69-1.81 (m, 8H), 1.86-1.99 (m, 2H), 2.33 (s, 6H), 2.80-2.89 (m, 2H), 2.93 (t, J=7.8 Hz, 2H), 3.03 (t, J=5.2 Hz, 4H), 3.43 (s, 2H), 3.75 (s, 2H), 7.04 (d, J=8.4 Hz, 1H), 7.20-7.28 (m, 4H), 7.43 (d, J=8.4 Hz, 1H).

### (2) N,N-Dimethyl{3-{2-[1-(4-chlorobenzyl)piperidin-4-yl]ethyl}-6-piperidinobenz[d]isoxazol-7-yl}methylamine dihydrochloride

The title compound was obtained by synthesis from N,N-dimethyl{3-{2-[1-(4-chlorobenzyl)piperidin-4-yl]ethyl}-6-piperidinobenz[d]isoxazol-7-yl}methylamine according to Example 198-(4).
¹H-NMR (400 MHz, CD₃OD) δ (ppm): 1.51-1.79 (m, 5H), 1.79-1.96 (m, 6H), 1.96-2.12 (m, 2H), 2.92-3.15 (m, 14H), 3.44-3.54 (m, 2H), 4.31 (s, 2H), 4.72 (s, 2H), 7.44 (d, J=8.6 Hz, 1H), 7.46-7.53 (m, 2H), 7.54-7.61 (m, 2H), 7.93 (d, J=8.6 Hz, 1H).
ESI-MS m/z: 248 [M+2H]²⁺, 495 [M+H]⁺.

### Example 216

### N,N-Dimethyl{6-piperidino-3-{2-[1-(pyridin-2-yl)piperidin-4-yl]ethyl}benz[d]isoxazol-7-yl}methylamine dihydrochloride

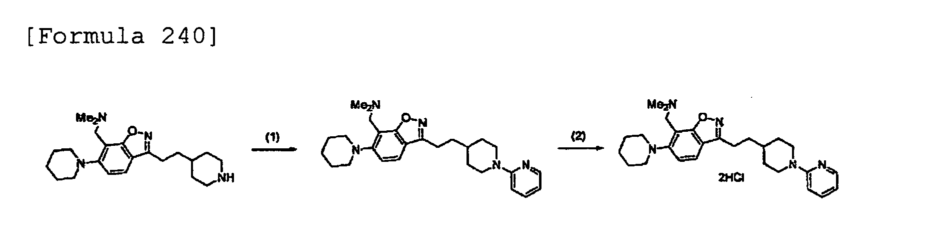

### (1) N,N-Dimethyl{6-piperidino-3-{2-[1-(pyridin-2-yl)piperidin-4-yl]ethyl}benz[d]isoxazol-7-yl}methylamine

N,N-Dimethyl{6-piperidino-3-[2-(piperidin-4-yl)ethyl]benz[d]isoxazol-7-yl}methylamine (compound synthesized in Example 209-(9)) (134 mg) was dissolved in propionitrile (1 mL). 2-Fluoropyridine (140 µL) and tetrabutylammonium fluoride hydrate (202 mg) were sequentially added to the solution, and the mixture was heated with stirring at 90°C overnight. The reaction mixture was cooled to room temperature. Then, a 5 N sodium hydroxide solution was added, followed by extraction with ethyl acetate three times. The organic layers were washed with a saturated sodium chloride solution and then dried over magnesium sulfate. The drying agent was removed by filtration and then the solvent was evaporated under reduced pressure. The residue was purified by NH silica gel chromatography (heptane-ethyl acetate) and NH preparative TLC (heptane-ethyl acetate) to obtain the title compound (50 mg).
¹H-NMR (400 MHz, CDCl₃) δ (ppm) : 1.23-1.38 (m, 3H), 1.53-1.69 (m, 3H), 1.69-1.93 (m, 8H), 2.33 (s, 6H), 2.75-2.84 (m, 2H), 2.98 (t, J=7.6 Hz, 2H), 3.04 (t, J=5.2 Hz, 4H), 3. 75 (s, 2H), 4.24-4.33 (m, 2H), 6.55 (dd, J=2.0 Hz, 5.2 Hz, 1H), 6.64 (d, J=8.8 Hz, 1H), 7.05 (d, J=8.4 Hz, 1H), 7.39-7.47 (m, 2H), 8.16 (ddd, J=0.8 Hz, 2.0 Hz, 5.2 Hz, 1H).

### (2) N,N-Dimethyl{6-piperidino-3-{2-[1-(pyridin-2-yl)piperidin-4-yl]ethyl}benz[d]isoxazol-7-yl}methylamine dihydrochloride

The title compound was obtained by synthesis from N,N-dimethyl{6-piperidino-3-{2-[1-(pyridin-2-yl)piperidin-4-yl]ethyl}benz[d]isoxazol-7-yl}methylamine according to Example 198-(4).
¹H-NMR (400 MHz, CD₃OD) δ (ppm): 1.36-1.49 (m, 2H), 1.63-1.73 (m, 2H), 1.76-1.94 (m, 7H), 2.00-2.10 (m, 2H), 2.92-3.07 (m, 10H), 3.12 (t, J=7.6 Hz, 2H), 3.22-3.33 (m, 2H), 4.18-4.28 (m, 2H), 4.73 (s, 2H), 6.91-6.97 (m, 1H), 7.40 (d, J=9.2 Hz, 1H), 7.45 (d, J=8.4 Hz, 1H), 7.88-7.92 (m, 1H), 7.95 (d, J=8.4 Hz, 1H), 7.97-8.03 (m, 1H).
ESI-MS m/z: 403 [M-NMe₂], 448 [M+H]⁺.

### Example 217

### 4-{{{7-(N,N-Dimethylaminomethyl)-3-{2-[1-(1,3-dioxolan-2-ylmethyl)piperidin-4-yl]ethyl}benz[d]isoxazol-6-yl}methylamino}methyl}benzonitrile difumarate

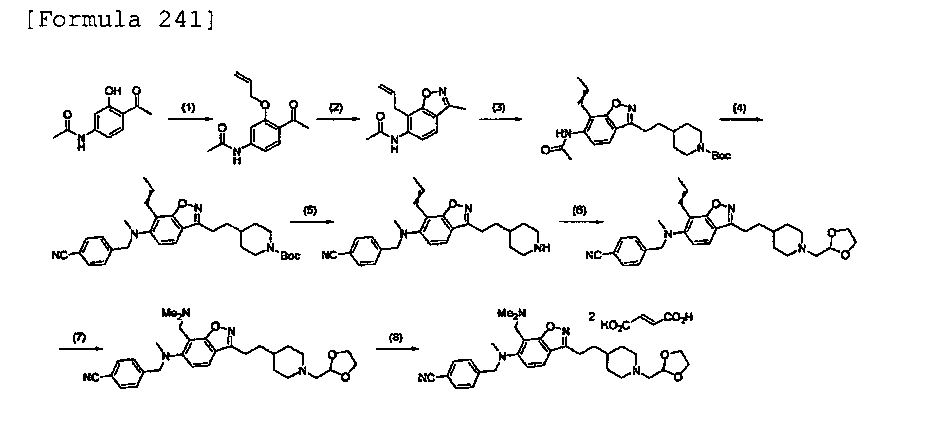

### (1) N-(4-Acetyl-3-allyloxyphenyl)acetamide

The title compound was obtained by synthesis from N-(4-acetyl-3-hydroxyphenyl)acetamide [CAS Registry No. 40547-58-8] according to Example 209-(1). ¹H-NMR (400 MHz, CDCl₃) δ (ppm) : 2.20 (s, 3H), 2.62 (s, 3H), 4.61-4.68 (m, 2H), 5.31-5.48 (m, 2H), 6.03-6.13 (m, 1H), 6.70 (dd, J=2.0 Hz, 8.4 Hz, 1H), 7.28-7.42 (m, 1H), 7.69-7.80 (m, 2H).

### (2) N-(7-Allyl-3-methylbenz[d]isoxazol-6-yl)acetamide

N-(4-Acetyl-3-allyloxyphenyl)acetamide (6.36 g) was dissolved in N-methylpyrrolidin-2-one (36 mL). The solution was heated with stirring under microwave irradiation at 180°C for 35 minutes. The reaction mixture was cooled to room temperature and then water was added, followed by extraction with ethyl acetate three times. The organic layers were sequentially washed with water and a saturated sodium chloride solution and then dried over sodium sulfate. The drying agent was removed by filtration and then the solvent was evaporated under reduced pressure. The residue was dissolved in ethanol (120 mL). Hydroxylamine hydrochloride (4.23 g) and a mixed solution of sodium acetate (5.28 g) and water (50 mL) were sequentially added, and the mixture was heated with stirring at 100°C for 2.5 hours. The reaction mixture was cooled to room temperature. Then, a saturated sodium bicarbonate solution was added, followed by extraction with chloroform three times. The organic layers were washed with a saturated sodium chloride solution and then dried over sodium sulfate. The drying agent was removed by filtration and then the solvent was evaporated under reduced pressure. The residue was dissolved in N,N-dimethylformamide (80 mL). Acetic anhydride (5.7 mL) and sodium acetate (4.6 g) were sequentially added and the mixture was heated with stirring at 145°C for two hours. The reaction mixture was cooled to room temperature and then water was added, followed by extraction with ethyl acetate three times. The organic layers were washed with water and a saturated sodium chloride solution and then dried over magnesium sulfate. The drying agent was removed by filtration and then the solvent was evaporated under reduced pressure. The residue was purified by NH silica gel chromatography (heptane-ethyl acetate) to obtain the title compound (4.20 g).
¹H-NMR (400 MHz, CDCl₃) δ (ppm) : 2.20 (s, 3H), 2.56 (s, 3H), 3.71 (d, J=6.0 Hz, 2H), 5.14 (d, J=17.2 Hz, 1H), 5.22 (d, J=10.0 Hz, 1H), 5.91-6.06 (m, 1H), 7.37-7.56 (m, 2H), 7.86-7.98 (m, 1H).

### (3) Mixture of tert-butyl 4-{2-[6-acetylamino-7-[(E)-1-propenyl]benz[d]isoxazol-3-yl]ethyl}piperidine-1-carboxylate and tert-butyl 4-{2-[6-acetylamino-7-[(Z)-1-propenyl]benz[d]isoxazol-3-yl]ethyl}piperidine-1-carboxylate

N-(7-Allyl-3-methylbenz[d]isoxazol-6-yl)acetamide (4.2 g) was dissolved in dichloromethane (100 mL). Bis(acetonitrile)dichloropalladium (II) (840 mg) was added to the solution, and the mixture was heated with stirring at 40°C for 22 hours. The reaction mixture was cooled to room temperature and then filtered through celite. The insoluble matter was washed with ethyl acetate and chloroform. Chloroform, water and potassium sodium tartrate were added to the residue, followed by stirring. The insoluble matter was removed by filtration, followed by extraction with chloroform twice. The filtrate was dried over magnesium sulfate, and the drying agent was removed by filtration. The filtrate was mixed with the filtrate after filtration through celite. The solvent was evaporated under reduced pressure. The residue was purified by NH silica gel chromatography to obtain a crude mixture of N-{7-[(E)-1-propenyl]-3-methylbenz[d]isoxazol-6-yl}acetamide and N-{7-[(Z)-1-propenyl]-3-methylbenz[d]isoxazol-6-yl}acetamide. The crude mixture was used for the next reaction without further purification.
Diisopropylamine (900 µL) was dissolved in tetrahydrofuran (4.5 mL) in a nitrogen atmosphere, and n-butyllithium (2.71 M solution in hexane, 2.1 mL) was added dropwise at -78°C. The solution was stirred at - 78°C for five minutes, further stirred under ice-cooling for five minutes and then cooled to -78°C again to prepare a solution of lithium diisopropylamide in tetrahydrofuran. The solution of lithium diisopropylamide in tetrahydrofuran (5.1 mL) was added dropwise to a solution of N-{7-[(E)-1-propenyl]-3-methylbenz[d]isoxazol-6-yl}acetamide and N-{7-[(Z)-1-propenyl]-3-methylbenz[d]isoxazol-6-yl}acetamide obtained by the above operation (300 mg) and tert-butyl 4-iodomethylpiperidine-1-carboxylate (compound synthesized in Preparation Example 1) (0.48 g) in tetrahydrofuran (13 mL) at -70°C over five minutes. Then, the mixture was stirred for one hour while maintaining the internal temperature at -70°C to -54°C. Further, the reaction mixture was heated with stirring for 30 minutes. A saturated ammonium chloride solution was added to the reaction mixture, followed by heating to room temperature. Water was added to the reaction mixture, followed by extraction with ethyl acetate three times. The organic layers were sequentially washed with water and a saturated sodium chloride solution and then dried over magnesium sulfate. The drying agent was removed by filtration and then the solvent was evaporated under reduced pressure. The residue was purified by NH silica gel column chromatography (heptane-ethyl acetate) to obtain the title compound (450 mg). The NMR data are shown only for the main product isomer.
¹H-NMR (400 MHz, CDCl₃) δ (ppm): 1.02-1.22 (m, 2H), 1.40-1.67 (m, 10H), 1.67-1.85 (m, 4H), 2.00-2.08 (m, 3H), 2.27 (s, 3H), 2.59-2.78 (m, 2H), 2.97 (t, J=7.6 Hz, 2H), 3.87-4.25 (m, 2H), 6.35-6.49 (m, 1H), 6.75-6.92 (m, 1H), 7.26-7.35 (m, 1H), 7.42 (d, J=8.4 Hz, 1H), 7.82 (d, J=8.4 Hz, 1H).

### (4) Mixture of tert-butyl 4-{2-{6-[(4-cyanobenzyl)methylamino]-7-[(E)-1-propenyl]benz[d]isoxazol-3-yl}ethyl}piperidine-1-carboxylate and tert-butyl 4-{2-{6-[(4-cyanobenzyl)methylamino]-7-[(Z)-1-propenyl]benz[d]isoxazol-3-yl}ethyl}piperidine-1-carboxylate

The mixture of tert-butyl 4-{2-[6-acetylamino-7-[(E)-1-propenyl]benz[d]isoxazol-3-yl]ethyl}piperidine-1-carboxylate and tert-butyl 4-{2-[6-acetylamino-7-[(Z)-1-propenyl]benz[d]isoxazol-3-yl]ethyl}piperidine-1-carboxylate (1 g) was dissolved in ethanol (20 mL). A 5 N sodium hydroxide solution (20 mL) was dissolved in the solution, and the mixture was heated with stirring at 80°C for three hours. The reaction mixture was cooled to room temperature and then water was added, followed by extraction with ethyl acetate three times. The organic layers were washed with a saturated sodium chloride solution and then dried over magnesium sulfate. The drying agent was removed by filtration. Then, the solvent was evaporated under reduced pressure to obtain a crude mixture of tert-butyl 4-{2-{6-amino-[7-(E)-1-propenyl]benz[d]isoxazol-3-yl}ethyl}piperidine-1-carboxylate and tert-butyl 4-{2-{6-amino-[7-(Z)-1-propenyl]benz[d]isoxazol-3-yl}ethyl}piperidine-1-carboxylate (949 mg). The crude mixture was used for the next reaction without further purification.
The crude mixture of tert-butyl 4-{2-{6-amino-[7-(E)-1-propenyl]benz[d]isoxazol-3-yl}ethyl}piperidine-1-carboxylate and tert-butyl 4-{2-{6-amino-[7-(Z)-1-propenyl]benz[d]isoxazol-3-yl}ethyl}piperidine-1-carboxylate (949 mg) was dissolved in dichloromethane (30 mL). 4-Cyanobenzaldehyde (390 mg), acetic acid (150 µL), and sodium triacetoxyborohydride (800 mg) were sequentially added to the solution, and the mixture was stirred at room temperature overnight. Water was added to the reaction mixture, followed by extraction with ethyl acetate twice. The organic layers were sequentially washed with a saturated sodium bicarbonate solution and a saturated sodium chloride solution and then dried over magnesium sulfate. The drying agent was removed by filtration and the solvent was evaporated under reduced pressure. The residue was purified by NH silica gel chromatography (heptane-ethyl acetate) to obtain a crude mixture of tert-butyl 4-{2-{6-[(4-cyanobenzylidene)amino]-7-[(E)-1-propenyl]benz[d]isoxazol-3-yl}ethyl}piperidine-1-carboxylate and tert-butyl 4-{2-{6-[(4-cyanobenzylidene)amino]-7-[(Z)-1-propenyl]benz[d]isoxazol-3-yl}ethyl}piperidine-1-carboxylate (200 mg) and a crude mixture of tert-butyl 4-{2-{6-amino-7-[(E)-1-propenyl]benz[d]isoxazol-3-yl}ethyl}piperidine-1-carboxylate and tert-butyl 4-{2-{6-amino-7-[(Z)-1-propenyl]benz[d]isoxazol-3-yl}ethyl}piperidine-1-carboxylate (594 mg).
The respective mixtures were reacted as follows.
The crude mixture of tert-butyl 4-{2-{6-[(4-cyanobenzylidene)amino]-7-[(E)-1-propenyl]benz[d]isoxazol-3-yl}ethyl}piperidine-1-carboxylate and tert-butyl 4-{2-{6-[(4-cyanobenzylidene)amino]-7-[(Z)-1-propenyl]benz[d]isoxazol-3-yl}ethyl}piperidine-1-carboxylate (200 mg) was dissolved in methanol. The reaction mixture was ice-cooled, and sodium borohydride was added until elimination of the raw material was confirmed. A saturated sodium bicarbonate solution was added to the reaction mixture and then the mixture was removed from the ice bath, followed by extraction with ethyl acetate three times. The organic layers were washed with a saturated sodium chloride solution and then dried over magnesium sulfate. The drying agent was removed by filtration and then the solvent was evaporated under reduced pressure. A crude mixture of tert-butyl 4-{2-{6-[(4-cyanobenzyl)amino]-7-[(E)-1-propenyl]benz[d]isoxazol-3-yl}ethyl}piperidine-1-carboxylate and tert-butyl 4-{2-{6-[(4-cyanobenzyl)amino]-7-[(E)-1-propenyl]benz[d]isoxazol-3-yl}ethyl}piperidine-1-carboxylate was obtained as a residue.
The crude mixture of tert-butyl 4-{2-{6-amino-7-[(E)-1-propenyl]benz[d]isoxazol-3-yl}ethyl}piperidine-1-carboxylate and tert-butyl 4-{2-{6-amino-7-[(Z)-1-propenyl]benz[d]isoxazol-3-yl}ethyl}piperidine-1-carboxylate (594 mg) was dissolved in methanol (10 mL) and acetic acid (1 mL). 4-Cyanobenzaldehyde (220 mg) and α-picolineborane (190 mg) were sequentially added to the solution, and the mixture was stirred at room temperature for three hours. 1 N aqueous sodium hydroxide and a saturated sodium bicarbonate solution were added to the reaction mixture, followed by extraction with ethyl acetate twice. The organic layers were washed with a saturated sodium chloride solution and then dried over magnesium sulfate. The drying agent was removed by filtration and then the solvent was evaporated under reduced pressure. The residue was purified by NH silica gel chromatography (heptane-ethyl acetate). A mixture of tert-butyl 4-{2-{6-[(4-cyanobenzyl)amino]-7-[(E)-1-propenyl]benz[d]isoxazol-3-yl}ethyl}piperidine-1-carboxylate and tert-butyl 4-{2-{6-[(4-cyanobenzyl)amino]-7-[(E)-1-propenyl]benz[d]isoxazol-3-yl}ethyl}piperidine-1-carboxylate was obtained.
The crude mixtures of tert-butyl 4-{2-{6-[(4-cyanobenzyl)amino]-7-[(E)-1-propenyl]benz[d]isoxazol-3-yl}ethyl}piperidine-1-carboxylate and tert-butyl 4-{2-{6-[(4-cyanobenzyl)amino]-7-[(E)-1-propenyl]benz[d]isoxazol-3-yl}ethyl}piperidine-1-carboxylate obtained by the above two methods were mixed and used for the next reaction.
The mixed crude mixtures were dissolved in methanol (20 mL) and acetic acid (2 mL). Formaldehyde (37% aqueous solution, 1.5 mL) and α-picolineborane (340 mg) were added to the solution, and the mixture was stirred at room temperature overnight. 1 N aqueous sodium hydroxide and a saturated sodium bicarbonate solution were added to the reaction mixture, followed by extraction with ethyl acetate twice. The organic layers were washed with a saturated sodium chloride solution and then dried over magnesium sulfate. The drying agent was removed by filtration and then the solvent was evaporated under reduced pressure. The residue was purified by NH silica gel chromatography (heptane-ethyl acetate) to obtain the title compound (690 mg). The NMR data are shown only for the main product isomer.
¹H-NMR (400 MHz, CDCl₃) δ (ppm) : 1.09-1.22 (m, 2H), 1.42-1.59 (m, 10H), 1.70-1.84 (m, 4H), 1.99 (dd, J=1.6 Hz, 6.8 Hz, 3H), 2.59-2.78 (m, 5H), 2.96 (t, J=7.6 Hz, 2H), 3.99-4.19 (m, 2H), 4.28 (s, 2H), 6.70-6.77 (m, 1H), 6.89-6.99 (m, 2H), 7.32 (d, J=8.6 Hz, 1H), 7.41 (d, J=8.4 Hz, 2H), 7.58-7.63 (m, 2H).

### (5) Mixture of 4-{{methyl{3-(2-piperidin-4-ylethyl)-7-[(E)-1-propenyl]benz[d]isoxazol-6-yl}amino}methyl}benzonitrile and 4-{{methyl-{3-(2-piperidin-4-ylethyl)-7-[(Z)-1-propenyl]benz[d]isoxazol-6-yl}amino}methyl}benzonitrile

The title compound was obtained by synthesis from the mixture of tert-butyl 4-{2-{6-[(4-cyanobenzyl)methylamino]-7-[(E)-1-propenyl]benz[d]isoxazol-3-yl}ethyl}piperidine-1-carboxylate and tert-butyl 4-{2-{6-[(4-cyanobenzyl)methylamino]-7-[(Z)-1-propenyl]benz[d]isoxazol-3-yl}ethyl}piperidine-1-carboxylate according to Example 209-(9). The NMR data are shown only for the main product isomer.
¹H-NMR (400 MHz, CDCl₃) δ(ppm): 1.11-1.27 (m, 2H), 1.41-1.55 (m, 1H), 1.68-1.84 (m, 4H), 1.99 (dd, J=1.6 Hz, 6.8 Hz, 3H), 2.53-2.64 (m, 2H), 2.74 (s, 3H), 2.95 (t, J=8.0 Hz, 2H), 3.03-3.13 (m, 2H), 4.28 (s, 2H), 6.71-6.78 (m, 1H), 6.89-7.00 (m, 2H), 7.33 (d, J=8.4 Hz, 1H), 7.41 (d, J=8.4 Hz, 2H), 7.58-7.63 (m, 2H).

### (6) Mixture of 4-{{{3-{2-[1-(1,3-dioxolan-2-ylmethyl)piperidin-4-yl]ethyl}-7-[(E)-1-propenyl]benz[d]isoxazol-6-yl}methylamino}methyl}benzonitrile and 4-{{{3-{2-[1-(1,3-dioxolan-2-ylmethyl)piperidin-4-yl]ethyl}-7-[(Z)-1-propenyl]benz[d]isoxazol-6-yl}methylamino}methyl}benzonitrile

The mixture of 4-{{methyl-{3-(2-piperidin-4-ylethyl)-7-[(E)-1-propenyl]benz[d]isoxazol-6-yl}amino}methyl}benzonitrile and 4-{{methyl-{3-(2-piperidin-4-ylethyl)-7-[(Z)-1-propenyl]benz[d]isoxazol-6-yl}amino}methyl}benzonitrile (608 mg) was dissolved in N,N-dimethylformamide (10 mL). N,N-Diisopropylethylamine (820 µL), 2-bromomethyl-1,3-dioxolane (230 µL) and sodium iodide (220 mg) were sequentially added to the solution, and the mixture was heated with stirring at 80°C for one day. The reaction mixture was cooled to room temperature. Then, a saturated sodium bicarbonate solution was added, followed by extraction with ethyl acetate three times. The organic layers were sequentially washed with water and a saturated sodium chloride solution and then dried over magnesium sulfate. The drying agent was removed by filtration and then the solvent was evaporated under reduced pressure. The residue was purified by NH silica gel chromatography (heptane-ethyl acetate) to obtain the title compound (297 mg). The NMR data are shown only for the main product isomer.
¹H-NMR (400 MHz, CDCl₃) δ (ppm): 1.30-1.44 (m, 3H), 1.63-1.83 (m, 4H), 1.98 (dd, J=1.6 Hz, 6.4 Hz, 3H), 2.01-2.12 (m, 2H), 2.57 (d, J=4.4 Hz, 2H), 2.73 (s, 3H), 2.94 (t, J=8.0 Hz, 2H), 2.97-3.05 (m, 2H), 3.80-3.89 (m, 2H), 3.92-4.01 (m, 2H), 4.28 (s, 2H), 5.00 (t, J=4.4 Hz, 1H), 6.70-6.78 (m, 1H), 6.89-6.99 (m, 2H), 7.32 (d, J=8.4 Hz, 1H), 7.41 (d, J=8.8 Hz, 2H), 7.58-7.63 (m, 2H).

### (7) 4-{{{7-(N,N-Dimethylaminomethyl)-3-{2-[1-(1,3-dioxolan-2-ylmethyl)piperidin-4-yl]ethyl}benz[d]isoxazol-6-yl}methylamino}methyl}benzonitrile

The following reaction was performed with reference to the method described in Org. Lett., 6(19)-3217, (2004).
The mixture of 4-{{{3-{2-[1-(1,3-dioxolan-2-ylmethyl)piperidin-4-yl]ethyl}-7-[(E)-1-propenyl]benz[d]isoxazol-6-yl}methylamino}methyl}benzonitrile and 4-{{{3-{2-[1-(1,3-dioxolan-2-ylmethyl)piperidin-4-yl]ethyl}-7-[(Z)-1-propenyl]benz[d]isoxazol-6-yl}methylamino}methyl}benzonitrile (297 mg) was dissolved in 1,4-dioxane (6 mL) and water (2 mL). 2,6-Lutidine (150 µL), osmium tetroxide (2.5% aqueous solution, 250 µL) and sodium periodate (330 mg) were sequentially added to the solution, and the mixture was stirred at room temperature for 1.5 hours. Water was added to the reaction mixture, followed by extraction with chloroform three times. The organic layers were washed with a saturated sodium chloride solution and then dried over magnesium sulfate. The drying agent was removed by filtration and then the solvent was evaporated under reduced pressure. A crude product of 4-{{{3-{2-[1-(1,3-dioxolan-2-ylmethyl)piperidin-4-yl]ethyl}-7-formylbenz[d]isoxazol-6-yl}methylamino}methyl}benzonitrile (443 mg) was obtained as a residue. The crude product was used for the next reaction without further purification.
The crude product of 4-{{{3-{2-[1-(1,3-dioxolan-2-ylmethyl)piperidin-4-yl]ethyl}-7-formylbenz[d]isoxazol-6-yl}methylamino}methyl}benzonitrile (254 mg) was dissolved in dichloromethane (5 mL). Dimethylamine (2 M solution in tetrahydrofuran, 850 µL), acetic acid (96 µL) and sodium triacetoxyborohydride (145 mg) were sequentially added to the solution, and the mixture was stirred at room temperature overnight. A saturated sodium bicarbonate solution was added to the reaction mixture, followed by extraction with ethyl acetate three times. The organic layers were washed with a saturated sodium chloride solution and then dried over magnesium sulfate. The drying agent was removed by filtration and then the solvent was evaporated under reduced pressure. The residue was purified by NH silica gel chromatography (heptane-ethyl acetate) and preparative TLC (chloroform-methanol) to obtain the title compound (45 mg).
¹H-NMR (400 MHz, CDCl₃) δ (ppm) : 1.23-1.44 (m, 3H), 1.51-1.83 (m, 4H), 2.00-2,12 (m, 2H), 2.23 (s, 6H), 2.57 (d, J=4.4 Hz, 2H), 2.78 (s, 3H), 2.94 (t, J=8.0 Hz, 2H), 2.97-3.06 (m, 2H), 3.74 (s, 2H), 3.82-3.90 (m, 2H), 3.92-4.00 (m, 2H), 4.63 (s, 2H), 5.00 (t, J=4.4 Hz, 1H), 7.03 (d, J=8.4 Hz, 1H), 7.44 (d, J=8.4 Hz, 1H), 7.48 (d, J=8.2 Hz, 2H), 7.62 (d, J=8.2 Hz, 2H).

### (8) 4-{{{7-(N,N-Dimethylaminomethyl)-3-{2-[1-(1,3-dioxolan-2-ylmethyl)piperidin-4-yl]ethyl}benz[d]isoxazol-6-yl}methylamino}methyl}benzonitrile difumarate

The title compound was obtained by synthesis from 4-{{{7-(N,N-dimethylaminomethyl)-3-{2-[1-(1,3-dioxolan-2-ylmethyl)piperidin-4-yl]ethyl}benz[d]isoxazol-6-yl}methylamino}methyl}benzonitrile according to Example 182-(4).
¹H-NMR (400 MHz, CD₃OD) δ (ppm) : 1.42-1.66 (m, 3H), 1.69-1.80 (m, 2H), 1.85-1.98 (m, 2H), 2.66 (s, 3H), 2.75 (s, 6H), 2.84-3.00 (m, 4H), 3.14-3.22 (m, 2H), 3.44-3.56 (m, 2H), 3.78-3.88 (m, 2H), 3.89-3.99 (m, 2H), 4.20 (s, 2H), 4.54 (s, 2H), 5.09-5.15 (m, 1H), 6.56 (s, 4H), 7.28 (d, J=8.6 Hz, 1H), 7.39 (d, J=8.0 Hz, 2H), 7.60 (d, J=8.0 Hz, 2H), 7.76 (d, J=8.6 Hz, 1H).
ESI-MS m/z: 237 [M+H-NMe₂]²⁺, 260 [M+2H]²⁺, 518 [M+H]⁺.

### Example 218

### 4-{{{3-{2-[1-(1,3-Dioxolan-2-ylmethyl)piperidin-4-yl]ethyl}-7-(N-methylaminomethyl)benz[d]isoxazol-6-yl}methylamino}methyl}benzonitrile difumarate

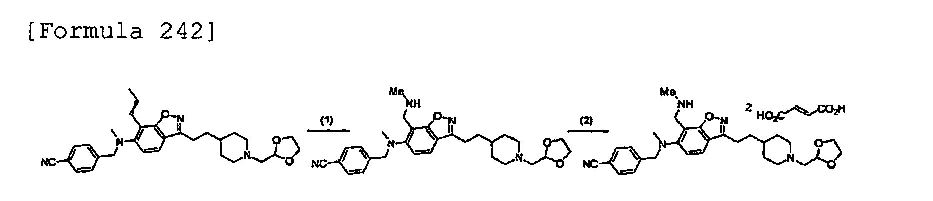

### (1) 4-{{{3-{2-[1-(1,3-Dioxolan-2-ylmethyl)piperidin-4-yl]ethyl}-7-(N-methylaminomethyl)benz[d]isoxazol-6-yl}methylamino}methyl}benzonitrile

The following reaction was performed with reference to the method described in Org. Lett., 6(19)-3217, (2004).
A mixture of 4-{{{3-{2-[1-(1,3-dioxolan-2-ylmethyl)piperidin-4-yl]ethyl}-7-[(E)-1-propenyl]benz[d]isoxazol-6-yl}methylamino}methyl}benzonitrile and 4-{{{3-{2-[1-(1,3-dioxolan-2-ylmethyl)piperidin-4-yl]ethyl}-7-[(Z)-1-propenyl]benz[d]isoxazol-6-yl}methylamino}methyl}benzonitrile (297 mg) was dissolved in 1,4-dioxane (6 mL) and water (2 mL). 2,6-Lutidine (150 µL), osmium tetroxide (2.5% aqueous solution, 250 µL) and sodium periodate (330 mg) were sequentially added to the solution, and the mixture was stirred at room temperature for 1.5 hours. Water was added to the reaction mixture, followed by extraction with chloroform three times. The organic layers were washed with a saturated sodium chloride solution and then dried over magnesium sulfate. The drying agent was removed by filtration and then the solvent was evaporated under reduced pressure. A crude product of 4-{{{3-{2-[1-(1,3-dioxolan-2-ylmethyl)piperidin-4-yl]ethyl}-7-formylbenz[d]isoxazol-6-yl}methylamino}methyl}benzonitrile (443 mg) was obtained as a residue. The crude product was used for the next reaction without further purification.
Methylamine (2 M solution in tetrahydrofuran, 2 mL) and magnesium sulfate (160 mg) were sequentially added to the crude product of 4-{{{3-{2-[1-(1,3-dioxolan-2-ylmethyl)piperidin-4-yl]ethyl}-7-formylbenz[d]isoxazol-6-yl}methylamino}methyl}benzonitrile (189 mg), and the mixture was stirred at room temperature for 30 minutes. Further, methanol (2 mL) and sodium borohydride (15 mg) were added to the reaction mixture, and the mixture was stirred at room temperature overnight. A saturated sodium bicarbonate solution was added to the reaction mixture, followed by extraction with chloroform three times. The organic layers were washed with a saturated sodium chloride solution and then dried over magnesium sulfate. The drying agent was removed by filtration and then the solvent was evaporated under reduced pressure. The residue was purified by NH preparative TLC (heptane-ethyl acetate) and preparative TLC (chloroform-methanol) to obtain the title compound (18 mg).
¹H-NMR (400 MHz, CDCl₃) δ (ppm): 1.28-1.44 (m, 3H), 1.66-1.86 (m, 4H), 2.00-2.13 (m, 2H), 2.42 (s, 3H), 2.57 (d, J=4.4 Hz, 2H), 2.75 (s, 3H), 2.90-3.05 (m, 4H), 3.82-3.90 (m, 2H), 3.93-4.01 (m, 2H), 4.09 (s, 2H), 4.38 (s, 2H), 5.01 (t, J=4.4 Hz, 1H), 7.06 (d, J=8.6 Hz, 1H), 7.44 (d, J=8.6 Hz, 1H), 7.47 (d, J=8.8 Hz, 2H), 7.61-7.64 (m, 2H).

### (2) 4-{{{3-{2-[1-(1,3-Dioxolan-2-ylmethyl)piperidin-4-yl]ethyl}-7-(N-methylaminomethyl)benz[d]isoxazol-6-yl}methylamino}methyl}benzonitrile difumarate

The title compound was obtained by synthesis from 4-{{{3-{2-[1-(1,3-dioxolan-2-ylmethyl)piperidin-4-yl]ethyl}-7-(N-methylaminomethyl)benz[d]isoxazol-6-yl}methylamino}methyl}benzonitrile according to Example 182-(4).
¹H-NMR (400 MHz, CD₃OD) δ (ppm) : 1.59-1.75 (m, 3H), 1.78-1.92 (m, 2H), 1.96-2.07 (m, 2H), 2.77 (s, 3H), 2.78 (s, 3H), 2.92-3.11 (m, 4H), 3.24-3.29 (m, 2H), 3.55-3.66 (m, 2H), 3.88-3.98 (m, 2H), 4.00-4.08 (m, 2H), 4.30 (s, 2H), 4.60 (s, 2H), 5.22 (t, J=4.0 Hz, 1H), 6.63 (s, 4H), 7.36 (d, J=8.4 Hz, 1H), 7.51 (d, J=8.4 Hz, 2H), 7.66-7.72 (m, 2H), 7.82 (d, J=8.4 Hz, 1H).
ESI-MS m/z: 237 [M+H-NHMe]²⁺, 504 [M+H]⁺.

### Example 219

### 3-{4-{2-[6-Cyclopropylmethoxy-7-(N,N-dimethylaminomethyl)benz[d]isoxazol-3-yl]ethyl}piperidinomethyl}-1H-pyrazin-2-one dihydrochloride

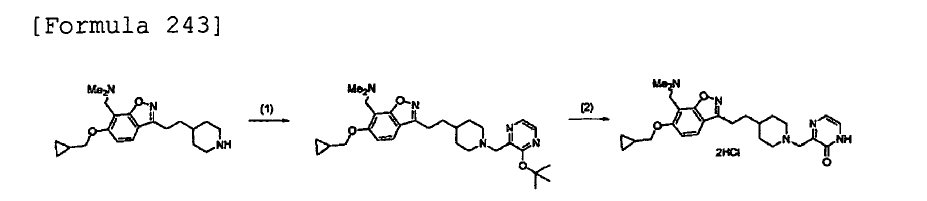

### (1) N,N-Dimethyl(3-{2-[1-(3-tert-butyloxypyrazin-2-ylmethyl)piperidin-4-yl]ethyl}-6-cyclopropylmethoxybenz[d]isoxazol-7-yl)methylamine

The title compound was obtained by synthesis from N,N-dimethyl{6-cyclopropylmethoxy-3-[2-(piperidin-4-yl)ethyl]benz[d]isoxazol-7-yl}methylamine (compound synthesized in Preparation Example 3) and 3-tert-butyloxypyrazine-2-carbaldehyde [CAS Registry No. 614729-22-5] according to Example 179-(6).
¹H-NMR (400 MHz, CDCl₃) δ (ppm): 0.32-0.42 (m, 2H), 0.60-0.69 (m, 2H), 1.24-1.45 (m, 4H), 1.56-1.82 (m, 13H), 2.04-2.14 (m, 2H), 2.34 (s, 6H), 2.89-3.03 (m, 4H), 3.63 (s, 2H), 3.82 (s, 2H), 3.94 (d, J=6.4 Hz, 2H), 6.89 (d, J=8.8 Hz, 1H), 7.44 (d, J=8.8 Hz, 1H), 7.91 (d, J=2.8 Hz, 1H), 8.04 (d, J=2.8 Hz, 1H).

### (2) 3-{4-{2-[6-Cyclopropylmethoxy-7-(N,N-dimethylaminomethyl)-benz[d]isoxazol-3-yl]ethyl}piperidinomethyl}-1H-pyrazin-2-one dihydrochloride

N,N-Dimethyl(3-{2-[1-(3-tert-butyloxypyrazin-2-ylmethyl)piperidin-4-yl]ethyl}-6-cyclopropylmethoxybenz[d]isoxazol-7-yl)methylamine (36 mg) was dissolved in ethyl acetate (2 mL), and the reaction mixture was ice-cooled. Hydrogen chloride (4 M solution in ethyl acetate) was added to the reaction mixture until the raw material was eliminated. The solvent was evaporated under reduced pressure. The residue was dried under reduced pressure to obtain the title compound (45 mg).
¹H-NMR (400 MHz, CD₃OD) δ (ppm) : 0.44-0.51 (m, 2H), 0.70-0.78 (m, 2H), 1.37-1.50 (m, 1H), 1.60-1.77 (m, 2H), 1.77-1.98 (m, 3H), 2.11-2.21 (m, 2H), 3.01 (s, 6H), 3.07-3.26 (m, 4H), 3.72-3.83 (m, 2H), 4.16 (d, J=6.8 Hz, 2H), 4.45 (s, 2H), 4.68 (s, 2H), 7.29 (d, J=8.8 Hz, 1H), 7.50 (d, J=4.0 Hz, 1H), 7.55 (d, J=4.0 Hz, 1H), 7.97 (d, J=8.8 Hz, 1H).
ESI-MS m/z: 234 [M+2H]²⁺, 466 [M+H]⁺.

### Example 220

### N,N-Dimethyl{3-[2-(1-benzylpiperidin-4-yl)ethyl]-6-cyclopropylmethoxybenz[d]isoxazol-5-yl}methylamine dihydrochloride

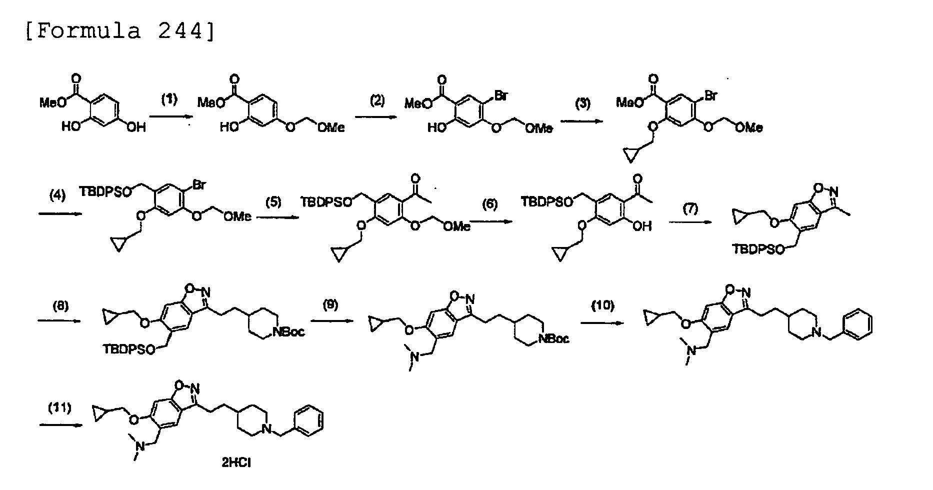

### (1) Methyl 2-hydroxy-4-methoxymethoxybenzoate

Methyl 2,4-dihydroxybenzoate (10 g) was dissolved in acetone (200 mL). Potassium carbonate (20.6 g) and chloromethyl methyl ether (5 mL) were sequentially added to the solution at room temperature, and then the mixture was stirred at 50°C for 2.5 hours. The reaction mixture was cooled to room temperature and then filtered through celite. The residue was washed with acetone. The filtrate was concentrated under reduced pressure. The precipitated insoluble matter was suspended in a mixed solvent of heptane and ethyl acetate (1:1), and the suspension was filtered through celite. The residue was washed with the same solvent. The filtrate was concentrated under reduced pressure. The residue was purified by silica gel column chromatography (heptane-ethyl acetate) to obtain the title compound (10.4 g).
1H-NMR (400 MHz, CDCl₃) δ (ppm): 3.48 (s, 3H), 3.92 (s, 3H), 5.20 (s, 2H), 6.54 (d, J=8.8 Hz, 1H), 6.62 (s, 1H), 7.75 (d, J=8.8 Hz, 1H), 10.90 (s, 1H).

### (2) Methyl 5-bromo-2-hydroxy-4-methoxymethoxybenzoate

Methyl 2-hydroxy-4-methoxymethoxybenzoate (10.4 g) was dissolved in acetonitrile (122 mL). N-Bromosuccinimide (9.6 g) was added to the solution under ice-cooling, and then the mixture was stirred at room temperature for 1.5 hours. Water was added to the reaction mixture, followed by extraction with ethyl acetate and then washing with a saturated sodium chloride solution. The organic layer was separated and dried over magnesium sulfate. The drying agent was removed by filtration and then the solvent was evaporated under reduced pressure. The residue was purified by NH silica gel column chromatography (heptane-ethyl acetate) to obtain the title compound (10.9 g).
1H-NMR (400 MHz, CDCl₃)
δ (ppm): 3.51 (s, 3H), 3.93 (s, 3H), 5.27 (s, 2H), 6.75 (s, 1H), 8.01 (s, 1H), 10.84 (s, 1H).

### (3) Methyl 5-bromo-2-cyclopropylmethoxy-4-methoxymethoxybenzoate

Methyl 5-bromo-2-hydroxy-4-methoxymethoxybenzoate (3.1 g) was dissolved in N,N-dimethylformamide (27 mL). Potassium carbonate (2.9 g) and bromomethylcyclopropane (1.2 mL) were sequentially added to the solution at room temperature, and then the mixture was stirred at 70°C for one hour. The reaction mixture was cooled to room temperature and then water was added, followed by extraction with ethyl acetate. The organic layer was washed with a saturated sodium chloride solution, dried over magnesium sulfate and filtered. Then, the solvent was evaporated under reduced pressure. The residue was purified by silica gel column chromatography (heptane-ethyl acetate-methylene chloride) to obtain the title compound (3.5 g).
1H-NMR (400 MHz, CDCl₃)
δ (ppm): 0.38-0.44 (m, 2H), 0.60-0.67 (m, 2H), 1.24-1.36 (m, 1H), 3.52 (s, 3H), 3.87 (s, 3H), 3.91 (d, J=6.8 Hz, 2H), 5.28 (s, 2H), 6.77 (s, 1H), 8.05 (s, 1H).

### (4) (5-Bromo-2-cyclopropylmethoxy-4-methoxymethoxybenzyloxy)tert-butyldiphenylsilane

Methyl 5-bromo-2-cyclopropylmethoxy-4-methoxymethoxybenzoate (3.5 g) was dissolved in tetrahydrofuran (34 mL). Lithium aluminum hydride (380 mg) was slowly added to the solution under ice-cooling, and then the mixture was stirred at the same temperature for 30 minutes. Water (0.38 mL), a 15% sodium hydroxide solution (0.38 mL) and water (1.1 mL) were sequentially added and the mixture was stirred at room temperature for 20 minutes. The mixture was filtered through celite. The filtrate was washed with ethyl acetate and then dried over magnesium sulfate. The drying agent was removed by filtration and then the solvent was evaporated under reduced pressure. The residue was purified by silica gel column chromatography (heptane-ethyl acetate-methylene chloride) to obtain (5-bromo-2-cyclopropylmethoxy-4-methoxymethoxyphenyl)methanol (2.1 g).
The resulting (5-bromo-2-cyclopropylmethoxy-4-methoxymethoxyphenyl)methanol (2.1 g) and imidazole (660 mg) were dissolved in N,N-dimethylformamide (16 mL). tert-Butylchlorodiphenylsilane (2 mL) was added to the solution at room temperature, and then the mixture was stirred for three hours. Water was added to the reaction mixture, followed by extraction with ethyl acetate. The organic layer was washed with a saturated sodium chloride solution. The organic layer was dried over magnesium sulfate, and the drying agent was removed by filtration. The solvent was evaporated under reduced pressure. The residue was purified by silica gel column chromatography (heptane-ethyl acetate) to obtain the title compound (3.6 g).
1H-NMR (400 MHz, CDCl₃)
δ (ppm): 0.19-0.26 (m, 2H), 0.47-0.55 (m, 2H), 1.04-1.16 (m, 1H), 1.10 (s, 9H), 3.53 (s, 3H), 3.70 (d, J=6.8 Hz, 2H), 4.73 (s, 2H), 5.21 (s, 2H), 6.65 (s, 1H), 7.34-7.50 (m, 6H), 7.65-7.80 (m, 5H).

### (5) 1-[5-(tert-Butyldiphenylsilanyloxymethyl)-4-cyclopropylmethoxy-2-methoxymethoxyphenyl]ethanone

A mixture of (5-bromo-2-cyclopropylmethoxy-4-methoxymethoxybenzyloxy)tert-butyldiphenylsilane (3.6 g), tributyl(1-ethoxyvinyl)tin (3.5 g), dichlorobis(triphenylphosphine)palladium (II) (0.46 g) and 1-methyl-2-pyrrolidinone (16 mL) was stirred in a nitrogen atmosphere at 140°C for one hour. The reaction mixture was cooled to room temperature and then diluted with water and ethyl acetate and stirred at room temperature for five minutes. The mixture was filtered through celite, and then the filtration residue was washed with ethyl acetate. The filtrate was extracted with ethyl acetate. The organic layer was washed with a saturated sodium chloride solution three times and dried over magnesium sulfate. The drying agent was removed by filtration and then the solvent was evaporated under reduced pressure. The residue was purified by NH silica gel column chromatography (heptane-ethyl acetate) to obtain tert-butyl[2-cyclopropylmethoxy-5-(1-ethoxyvinyl)-4-methoxymethoxybenzyloxy]diphenylsilane (1.4 g).
The resulting tert-butyl[2-cyclopropylmethoxy-5-(1-ethoxyvinyl)-4-methoxymethoxybenzyloxy]diphenylsilane (1.4 g) was dissolved in tetrahydrofuran (13 mL). 2 M hydrochloric acid (1.4 mL) was added at room temperature and the mixture was stirred for 10 minutes. The reaction mixture was made alkaline with a saturated sodium bicarbonate solution, followed by extraction with ethyl acetate. The organic layer was washed with a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (1.2 g).
1H-NMR (400 MHz, CDCl₃) δ(ppm): 0.23-0.31 (m, 2H), 0.52-0.60 (m, 2H), 1.08-1.22 (m, 1H), 1.09 (s, 9H), 2.61 (s, 3H), 3.53 (s, 3H), 3.79 (d, J=7.2 Hz, 2H), 4.73 (s, 2H), 5.26 (s, 2H), 6.60 (s, 1H), 7.34-7.46 (m, 6H), 7.69-7.76 (m, 4H), 8.05 (s, 1H).

### (6) 1-[5-(tert-Butyldiphenylsilanyloxymethyl)-4-cyclopropylmethoxy-2-hydroxyphenyl]ethanone

1-[5-(tert-Butyldiphenylsilanyloxymethyl)-4-cyclopropylmethoxy-2-methoxymethoxyphenyl]ethanone (1.2 g) was dissolved in tetrahydrofuran (23 mL). 5 M hydrochloric acid (1.4 mL) was added at room temperature and then the mixture was stirred at 50°C for 30 minutes. The reaction mixture was cooled to room temperature and then extracted with water, followed by extraction with ethyl acetate. The organic layer was washed with a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (1.0 g).
1H-NMR (400 MHz, CDCl₃) δ (ppm): 0.22-0.29 (m, 2H), 0.51-0.59 (m, 2H), 1.06-1.22 (m, 1H), 1.12 (s, 9H), 2.54 (S, 3H), 3.77 (d, J=6. Hz, 2H), 4.76 (s, 2H), 6.31 (s, 1H), 7.34-7.48 (m, 6H), 7.68-7.75 (m, 4H), 7.89 (s, 1H).

### (7) 5-(tert-Butyldiphenylsilanyloxymethyl)-6-cyclopropylmethoxy-3-methylbenz[d]isoxazole

1-[5-(tert-Butyldiphenylsilanyloxymethyl)-4-cyclopropylmethoxy-2-hydroxyphenyl]ethanone (0.99 g) was dissolved in ethanol (6 mL) and water (2 mL). Hydroxylammonium chloride (0.36 g) and sodium acetate (0.45 g) were added to the solution, and the mixture was heated under reflux for two hours. The reaction mixture was cooled to room temperature, and then the solvent was evaporated under reduced pressure. A saturated sodium bicarbonate solution was added to the residue, followed by extraction with ethyl acetate. The organic layer was washed with a saturated sodium chloride solution and dried over magnesium sulfate.
The drying agent was removed by filtration and then the solvent was evaporated under reduced pressure to obtain a crude product of 1-[5-(tert-butyldiphenylsilanyloxymethyl)-4-cyclopropylmethoxy-2-hydroxyphenyl]ethanone oxime (0.99 g).
The crude product (0.99 g) was dissolved in N,N-dimethylformamide (4 mL). Sodium acetate (0.36 g) and acetic anhydride (0.44 mL) were added to the solution, and the mixture was stirred at 160°C for one hour. The reaction mixture was cooled to room temperature. Then, a saturated sodium bicarbonate solution was added, followed by extraction with ethyl acetate. The organic layer was washed with a 2 N sodium hydroxide solution and a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (0.78 g):
1H-NMR (400 MHz, CDCl₃)
δ (ppm): 0.23-0.30 (m, 2H), 0.51-0.59 (m, 2H), 1.10-1.25 (m, 1H), 1.13 (s, 9H), 2.54 (s, 3H), 3.81 (d, J=7.2 Hz, 2H), 4.86 (s, 2H), 6.86 (s, 1H), 7.35-7.47 (m, 6H), 7.69-7.76 (m, 5H).

### (8) tert-Butyl 4-{2-[5-(tert-butyldiphenylsilanyloxymethyl)-6-cyclopropylmethoxybenz[d]isoxazol-3-yl]ethyl}piperidine-1-carboxylate

n-Butyllithium (2.59 M solution in n-hexane, 1.4 mL) was added to a solution of diisopropylamine (0.56 mL) in tetrahydrofuran (3 mL) in a nitrogen atmosphere at -78°C. Then, the mixture was stirred under ice-cooling for 15 minutes to prepare a solution of lithium diisopropylamide in tetrahydrofuran. The lithium diisopropylamide solution was cooled to -78°C and added dropwise to a solution of 5-(tert-butyldiphenylsilanyloxymethyl)-6-cyclopropylmethoxy-3-methylbenz[d]isoxazole (0.3 g) and tert-butyl 4-iodomethylpiperidine-1-carboxylate obtained in Preparation Example 1-(2) (0.24 g) in tetrahydrofuran (5 mL) at -70°C over five minutes. Then, the mixture was stirred at the same temperature for one hour. A saturated ammonium chloride solution was added to the reaction mixture, followed by heating to room temperature. Water was added to the reaction mixture, followed by extraction with ethyl acetate twice. The organic layers were washed with a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (0.25 g).
1H-NMR (400 MHz, CDCl₃)
δ(ppm): 0.22-0.30 (m, 2H), 0.50-0.60 (m, 2H), 1.06-1.35 (m, 4H), 1.13 (s, 9H), 1.45 (s, 9H), 1.69-1.76 (m, 4H), 2.59-2.77 (m, 2H), 2.92-3.02 (m, 2H), 3.81 (d, J=7.2 Hz, 2H), 4.00-4.20 (m, 2H), 4.88 (s, 2H), 6.87 (s, 1H), 7.33-7.47 (m, 6H), 7.68-7.75 (m 4H), 7.81 (s, 1H).

### (9) tert-Butyl 4-{2-[6-cyclopropylmethoxy-5-(N,N-dimethylaminomethyl)benz[d]isoxazol-3-yl]ethyl}piperidine-1-carboxylate

tert-Butyl 4-{2-[5-(tert-butyldiphenylsilanyloxymethyl)-6-cyclopropylmethoxybenz[d]isoxazol-3-yl]ethyl}piperidine-1-carboxylate (250 mg) was dissolved in tetrahydrofuran (4 mL). Tetrabutylammonium fluoride (1 M solution in tetrahydrofuran, 0.49 mL) was added to the solution, and the mixture was stirred at room temperature for 30 minutes. A saturated ammonium chloride solution was added to the reaction mixture, followed by extraction with ethyl acetate. The organic layer was washed with a saturated sodium chloride solution and dried over magnesium sulfate. The drying agent was removed by filtration and then the solvent was evaporated under reduced pressure. The residue was purified by silica gel column chromatography (heptane-ethyl acetate) to obtain tert-butyl 4-[2-(6-cyclopropylmethoxy-5-hydroxymethylbenz[d]isoxazol-3-yl)ethyl]piperidine-1-carboxylate (161 mg).
The tert-butyl 4-[2-(6-cyclopropylmethoxy-5-hydroxymethylbenz[d]isoxazol-3-yl)ethyl]piperidine-1-carboxylate (161 mg) and triethylamine (0.156 mL) were dissolved in tetrahydrofuran (2 mL). Methanesulfonyl chloride (43 µL) was added to the solution under ice-cooling, and the mixture was stirred for one hour. Dimethylamine (2 M solution in tetrahydrofuran, 1.9 mL) and sodium iodide (6 mg) were added to the reaction mixture, and the mixture was stirred at room temperature for one hour. A saturated sodium chloride solution was added to the reaction mixture, followed by extraction with ethyl acetate. The organic layer was washed with a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (145 mg).
1H-NMR (400 MHz, CDCl₃)
δ (ppm): 0.37-0.44 (m, 2H), 0.62-0.71 (m, 2H), 1.09-1.23 (m, 2H), 1.27-1.39 (m, 1H), 1.44-1.57 (m, 1H), 1.46 (s, 9H), 1.69-1.84 (m, 4H), 2.32 (s, 6H), 2.60-2.76 (m, 2H), 2.91-3.00 (m, 2H), 3.55 (s, 2H), 3.90 (d, J=6.8 Hz, 2H), 4.01-4.20 (m, 2H), 6.92 (s, 1H), 7.53 (s, 1H).

### (10) N,N-Dimethyl{3-[2-(1-benzylpiperidin-4-yl)ethyl]-6-cyclopropylmethoxybenz[d]isoxazol-5-yl}methylamine

tert-Butyl 4-{2-[6-cyclopropylmethoxy-5-(N,N-dimethylaminomethyl)benz[d]isoxazol-3-yl]ethyl}piperidine-1-carboxylate (145 mg) was dissolved in methanol (3.8 mL). Hydrogen chloride (4 M solution in ethyl acetate, 0.95 mL) was added to the solution under ice-cooling, and the mixture was stirred at room temperature for 12 hours. The solvent was evaporated under reduced pressure. The residue was diluted with chloroform and then made alkaline with a 1 N sodium hydroxide solution, followed by extraction with chloroform. The organic layer was dried over magnesium sulfate. The drying agent was removed by filtration and the solvent was evaporated under reduced pressure to obtain a crude product of N,N-dimethyl[6-cyclopropylmethoxy-3-(2-piperidin-4-ylethyl)benz[d]isoxazol-5-yl]methylamine (105 mg).
The crude N,N-dimethyl[6-cyclopropylmethoxy-3-(2-piperidin-4-ylethyl)benz[d]isoxazol-5-yl]methylamine (55 mg) and benzaldehyde (19 µL) were dissolved in tetrahydrofuran (2 mL). Acetic acid (18 µL) and sodium triacetoxyborohydride (49 mg) were sequentially added to the solution, and the mixture was stirred at room temperature for five hours. The reaction mixture was made alkaline with a saturated sodium bicarbonate aqueous solution and sodium carbonate, followed by extraction with chloroform. The organic layer was washed with a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (50 mg).
1H-NMR (400 MHz, CDCl₃)
δ(ppm): 0.35-0.45 (m, 2H), 0.61-0.72 (m, 2H), 1.22-1.40 (m, 4H), 1.68-1.83 (m, 4H), 1.87-2.00 (m, 2H), 2.31 (s, 6H), 2.82-3.00 (m, 4H), 3.48 (s, 2H), 3.54 (s, 2H), 3.89 (d, J=6.8 Hz, 2H), 6.91 (s, 1H), 7.20-7.35 (m, 5H), 7.52 (s, 1H).

### (11) N,N-Dimethyl{3-[2-(1-benzylpiperidin-4-yl)ethyl]-6-cyclopropylmethoxybenz[d]isoxazol-5-yl}methylamine dihydrochloride

N,N-Dimethyl{3-[2-(1-benzylpiperidin-4-yl)ethyl]-6-cyclopropylmethoxybenz[d]isoxazol-5-yl}methylamine dihydrochloride (50 mg) was dissolved in methanol. 2 M hydrochloric acid (112 µL) was added and the solvent was evaporated under reduced pressure. The residue was dried under reduced pressure to obtain the title compound (28 mg).
1H-NMR (400 MHz, DMSO-d₆)
δ(ppm): 0.37-0.43 (m, 2H), 0.57-0.67 (m, 2H), 1.29-1.42 (m, 1H), 1.47-1.67 (m, 3H), 1.70-1.85 (m, 2H), 1.87-2.00 (m, 2H), 2.74 (s, 6H), 2.80-3.00 (m, 4H), 3.25-3.39 (m, 2H), 4.02 (d, J=6.8 Hz, 2H), 4.23 (s, 2H), 4.32 (s, 2H), 7.39 (s, 1H), 7.42-7.52 (m, 3H), 7.56-7.65 (m, 2H), 8.10 (s, 1H), 10.45 (bs, 1H), 10.62 (bs, 1H).
ESI-MS m/z: 224 [M+2H]²⁺, 448 [M+H]⁺.

### Example 221

### 4-{3-[2-(1-Benzylpiperidin-4-yl)ethyl]-7-(N,N-dimethylaminomethyl)benz[d]isoxazol-6-ylmethoxy}benzonitrile dihydrochloride

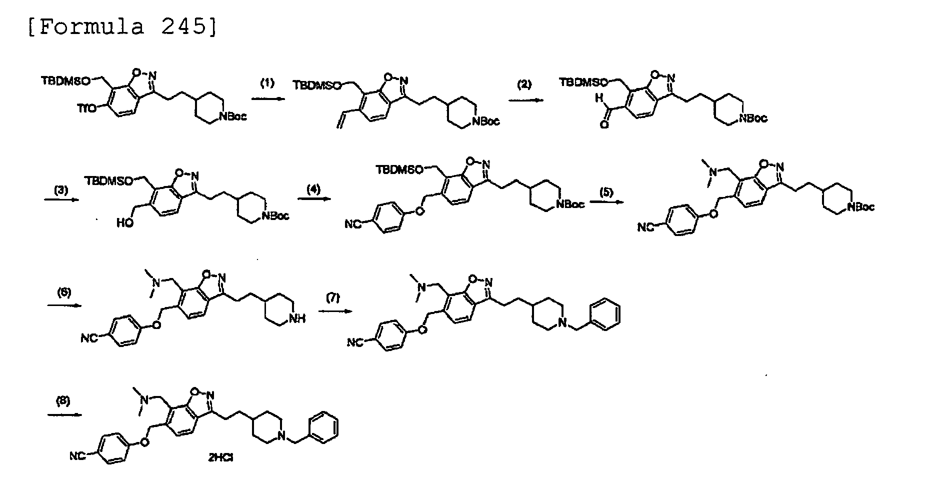

### (1) tert-Butyl 4-{2-[7-(tert-butyldimethylsilanyloxymethyl)-6-vinylbenz[d]isoxazol-3-yl]ethyl}piperidine-1-carboxylate

A mixture of tert-butyl 4-{2-[7-(tert-butyldimethylsilanyloxymethyl)-6-trifluoromethanesulfonyloxybenz[d]isoxazol-3-yl]ethyl}piperidine-1-carboxylate (compound synthesized in Example 79-(2)) (3.0 g), tri-n-butylvinyltin (3.1 g), dichlorobis(triphenylphosphine)palladium (II) (0.34 g), lithium chloride (0.59 g) and 1-methyl-2-pyrrolidinone (60 mL) was stirred at 110°C for 30 minutes. The reaction mixture was cooled to room temperature and then diluted with water and ethyl acetate. The diluted mixture was stirred at room temperature for five minutes, followed by extraction with ethyl acetate. The organic layer was washed with a saturated sodium chloride solution three times and dried over magnesium sulfate. The drying agent was removed by filtration and then the solvent was evaporated under reduced pressure. The residue was purified by silica gel column chromatography (heptane-ethyl acetate) to obtain the title compound (1.1 g).
1H-NMR (400 MHz, CDCl₃) δ (ppm): 0.086 (s, 6H), 0.89 (s, 9H), 1.09-1.23 (m, 2H), 1.41-1.52 (m, 1H), 1.45 (s, 9H), 1.70-1.84 (m, 4H), 2.58-2.75 (m, 2H), 2.95-3.03 (m, 2H), 3.99-4.20 (m, 2H), 5.06 (s, 2H), 5.47 (d, J=11.0 Hz, 1H), 5.83 (d, J=17.6 Hz, 1H), 7.26 (dd, J=11.0, 17.6 Hz, 1H), 7.49 (s, 2H).

### (2) tert-Butyl 4-{2-[7-(tert-butyldimethylsilanyloxymethyl)-6-formylbenz[d]isoxazol-3-yl]ethyl}piperidine-1-carboxylate

tert-Butyl 4-{2-[7-(tert-butyldimethylsilanyloxymethyl)-6-vinylbenz[d]isoxazol-3-yl]ethyl}piperidine-1-carboxylate (1.1 g) was dissolved in tetrahydrofuran (10 mL) and water (2 mL). Osmium tetroxide (2.5% aqueous solution, 1.1 mL) was added to the solution, and the mixture was stirred at room temperature for two minutes. Then, sodium metaperiodate (0.95 g) was added and the mixture was stirred at room temperature for 30 minutes. Water was added to the reaction mixture, followed by extraction with ethyl acetate. The organic layer was washed with a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (0.76 g).
1H-NMR (400 MHz, CDCl₃) δ(ppm): 0.10 (s, 6H), 0.88 (s, 9H), 1.11-1.25 (m, 2H), 1.45-1.58 (m, 1H), 1.46 (s, 9H), 1.50-1.86 (m, 4H), 2.61-2.75 (m, 2H), 3.00-3.08 (m, 2H), 4.01-4.20 (m, 2H), 5.37 (s, 2H), 7.64 (d, J=8.0 Hz, 1H), 7.88 (d, J=8.0 Hz, 1H), 10.65 (s, 1H).

### (3)(4) tert-Butyl 4-{2-[7-(tert-butyldimethylsilanyloxymethyl)-6-(4-cyanophenoxymethyl)benz[d]isoxazol-3-yl]ethyl}piperidine-1-carboxylate

tert-Butyl 4-{2-[7-(tert-butyldimethylsilanyloxymethyl)-6-formylbenz[d]isoxazol-3-yl]ethyl}piperidine-1-carboxylate (800 mg) was dissolved in methanol (8 mL). Sodium borohydride (60 mg) was added to the solution under ice-cooling, and the mixture was stirred at room temperature for 10 minutes. Water was added to the reaction mixture, followed by extraction with ethyl acetate. The organic layer was washed with a saturated sodium chloride solution and dried over magnesium sulfate. The drying agent was removed by filtration and then the solvent was evaporated under reduced pressure. The residue was purified by silica gel column chromatography (heptane-ethyl acetate) to obtain tert-butyl 4-{2-[7-(tert-butyldimethylsilanyloxymethyl)-6-hydroxymethylbenz[d]isoxazol-3-yl]ethyl}piperidine-1-carboxylate (840 mg).
The resulting tert-butyl 4-{2-[7-(tert-butyldimethylsilanyloxymethyl)-6-hydroxymethylbenz[d]isoxazol-3-yl]ethyl}piperidine-1-carboxylate (410 mg), 4-cyanophenol (97 mg) and triphenylphosphine (234 mg) were dissolved in tetrahydrofuran (4 mL). Diisopropyl azodicarboxylate (184 µL) was added dropwise to the solution under ice-cooling over 10 minutes, and then the mixture was stirred at room temperature for 14 hours. The solvent was evaporated under reduced pressure. The residue was purified by NH silica gel column chromatography (heptane-ethyl acetate) to obtain the title compound (285 mg).
1H-NMR (400 MHz, CDCl₃)
δ (ppm): 0.09 (s, 6H), 0.88 (s, 9H), 1.09-1.25 (m, 2H), 1.41-1.58 (m, 1H), 1.46 (s, 9H), 1.69-1.87 (m, 4H), 2.59-2.77 (m, 2H), 2.96-3.06 (m, 2H), 4.00-4.20 (m, 2H), 5.13 (s, 2H), 5.46 (s, 2H), 7.04 (d, J=8.8 Hz, 2H), 7.45 (d, J=8.0 Hz, 1H), 7.56 (d, J=8.0 Hz, 1H), 7.59 (d, J=8.8 Hz, 2H).

### (5) tert-Butyl 4-{2-[6-(4-cyanophenoxymethyl)-7-(N,N-dimethylaminomethyl)benz[d]isoxazol-3-yl]ethyl}piperidine-1-carboxylate

tert-Butyl 4-{2-[7-(tert-butyldimethylsilanyloxymethyl)-6-(4-cyanophenoxymethyl)benz[d]isoxazol-3-yl]ethyl}piperidine-1-carboxylate (285 mg) was dissolved in tetrahydrofuran (5 mL). Tetra-n-butylammonium fluoride (1 M solution in tetrahydrofuran, 0.61 mL) was added to the solution at room temperature, and the mixture was stirred at room temperature for 30 minutes. A saturated ammonium chloride solution was added to the reaction mixture, followed by extraction with ethyl acetate. The organic layer was washed with a saturated sodium chloride solution and dried over magnesium sulfate. The drying agent was removed by filtration and then the solvent was evaporated under reduced pressure. The residue was purified by silica gel column chromatography (heptane-ethyl acetate) to obtain tert-butyl 4-{2-[6-(4-cyanophenoxymethyl)-7-hydroxymethylbenz[d]isoxazol-3-yl]ethyl}piperidine-1-carboxylate (230 mg).
tert-Butyl 4-{2-[6-(4-cyanophenoxymethyl)-7-hydroxymethylbenz[d]isoxazol-3-yl]ethyl}piperidine-1-carboxylate (230 mg) and triethylamine (0.20 mL) were dissolved in tetrahydrofuran (5 mL). Methanesulfonyl chloride (54 µL) was added to the solution under ice-cooling, and the mixture was stirred for 30 minutes. Dimethylamine (2 M solution in tetrahydrofuran, 2.3 mL) and sodium iodide (7 mg) were added to the reaction mixture, and the mixture was stirred at room temperature for 3.5 hours. A saturated sodium chloride solution was added to the reaction mixture, followed by extraction with ethyl acetate. The organic layer was washed with a saturated sodium chloride solution and dried over magnesium sulfate. The drying agent was removed by filtration and then the solvent was evaporated under reduced pressure. The residue was purified by NH silica gel column chromatography (heptane-ethyl acetate-methylene chloride) to obtain the title compound (215 mg).
1H-NMR (400 MHz, CDCl₃)
δ (ppm): 1.10-1.24 (m, 2H), 1.42-1.60 (m, 1H), 1.46 (s, 9H), 1.70-1.86 (m, 4H), 2.25 (s, 6H), 2.60-2.77 (m, 2H), 2.95-3.05 (m, 2H), 3.81 (s, 2H), 4.00-4.20 (m, 2H), 5.48 (s, 2H), 7.08 (d, J=8.8 Hz, 2H), 7.44 (d, J=8.0 Hz, 1H), 7.55 (d, J=8.0 Hz, 1H), 7.59 (d, J=8.8 Hz, 2H).

### (6)(7) 4-{3-[2-(1-Benzylpiperidin-4-yl)ethyl]-7-(N,N-dimethylaminomethyl)benz[d]isoxazol-6-ylmethoxy}benzonitrile

tert-Butyl 4-{2-[6-(4-cyanophenoxymethyl)-7-(N,N-dimethylaminomethyl)benz[d]isoxazol-3-yl]ethyl}piperidine-1-carboxylate (215 mg) was dissolved in methanol (6 mL). Hydrogen chloride (4 M solution in ethyl acetate, 1.25 mL) was added to the solution under ice-cooling, and the mixture was stirred at room temperature for 14.5 hours. The solvent was evaporated under reduced pressure. The residue was diluted with chloroform and then made alkaline with a 2 M sodium carbonate solution, followed by extraction with chloroform. The organic layer was dried over magnesium sulfate. The drying agent was removed by filtration and the solvent was evaporated under reduced pressure to obtain a crude product of 4-{7-(N,N-dimethylaminomethyl)-3-[2-(piperidin-4-yl)ethyl]benz[d]isoxazol-6-ylmethoxy}benzonitrile (157 mg).

The resulting crude product of 4-{7-(N,N-dimethylaminomethyl)-3-[2-(piperidin-4-yl)ethyl]benz[d]isoxazol-6-ylmethoxy}benzonitrile (79 mg) and benzaldehyde (23 µL) were dissolved in methylene chloride (2 mL). Acetic acid (22 µL) and sodium triacetoxyborohydride (60 mg) were sequentially added to the solution, and the mixture was stirred at room temperature for five hours. The reaction mixture was made alkaline with a saturated sodium bicarbonate aqueous solution and sodium carbonate, followed by extraction with chloroform. The organic layer was washed with a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (68 mg).
1H-NMR (400 MHz, CDCl₃)
δ (ppm): 1.25-1.45 (m, 3H), 1.70-1.83 (m, 4H), 1.88-2.00 (m, 2H), 2.25 (s, 6H), 2.84-3.03 (m, 4H), 3.49 (s, 2H), 3.81 (s, 2H), 5.48 (s, 2H), 7.08 (d, J=8.8 Hz, 2H), 7.20-7.36 (m, 5H), 7.43 (d, J=8.0 Hz, 1H), 7.54 (d, J=8.0 Hz, 1H), 7.59 (d, J=8.8 Hz, 2H).

### (8) 4-{3-[2-(1-Benzylpiperidin-4-yl)ethyl]-7-dimethylaminomethylbenz[d]isoxazol-6-ylmethoxy}benzonitrile dihydrochloride

The title compound (64 mg) was obtained by synthesis from 4-{3-[2-(1-benzylpiperidin-4-yl)ethyl]-7-dimethylaminomethylbenz[d]isoxazol-6-ylmethoxy}benzonitrile (68 mg) according to Example 220-(11).
1H-NMR (400 MHz, DMSO-d₆) δ (ppm): 1.49-2.01 (m, 7H), 2.73-2.95 (m, 2H), 2.82 (s, 6H), 2.99-3.14 (m, 2H), 3.21-3.46 (m, 2H), 4.24 (s, 2H), 4.73 (s, 2H), 5.73 (s, 2H), 7.31 (d, J=8.4 Hz, 2H), 7.39-7.50 (m, 3H), 7.56-7.67 (m, 2H), 7.64 (d, J=8.0 Hz, 1H), 7.84 (d, J=8.4 Hz, 2H), 8.06 (d, J=8.0 Hz, 1H), 10.78 (bs, 1H), 10.93 (bs, 1H).
ESI-MS
m/z: 255 [M+2H]²⁺, 509 [M+H]⁺.

### Example 222

### N,N-Dimethyl{3-[2-(1-benzylpiperidin-4-yl)ethyl]-6-(pyridin-2-yloxy)benz[d]isoxazol-7-yl}methylamine dihydrochloride

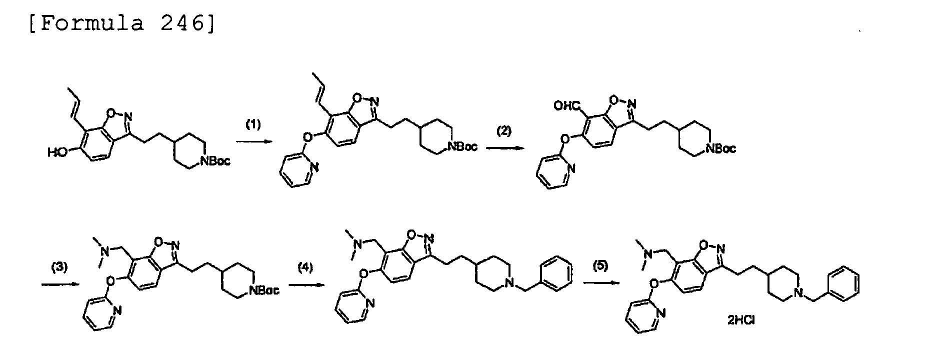

### (1) Mixture of tert-butyl 4-{2-{7-[(E)-propenyl]-6-(pyridin-2-yloxy)benz[d]isoxazol-3-yl}ethyl}piperidine-1-carboxylate and tert-butyl 4-{2-{7-[(Z)-propenyl]-6-(pyridin-2-yloxy)benz[d]isoxazol-3-yl}ethyl}piperidine-1-carboxylate

The title compound (207 mg) was obtained by synthesis according to Example 158-(4) from the mixture of tert-butyl 4-{2-{6-hydroxy-7-[(E)-propenyl]benz[d]isoxazol-3-yl}ethyl}piperidine-1-carboxylate and tert-butyl 4-{2-{6-hydroxy-7-[(Z)-propenyl]benz[d]isoxazol-3-yl}ethyl}piperidine-1-carboxylate obtained in Example 158-(3) (300 mg) and 2-fluoropyridine (5 mL).
¹H-NMR (400 MHz, CDCl₃)
δ(ppm): 1.10-1.25 (m, 2H), 1.43-1.60 (m, 1H), 1.46 (s, 9H), 1. 70-1. 86 (m, 4H), 1. 93 (d, J=6.8 Hz, 3H), 2.61-2.78 (m, 2H), 2.95-3.05 (m, 2H), 4.00-4.21 (m, 2H), 6.62 (d, J=16.0 Hz, 1H) , 6.97-7.05 (m, 2H), 7.03 (d, J=8.0 Hz, 1H) , 7.04 (dq, J=6.8, 16.0 Hz, 1H) , 7.42 (d, J=8 . 0 Hz, 1H) , 7.70-7.78 (m, 1H) , 8 . 15-8 .21 (m, 1H) .

### (2) tert-Butyl 4-{2-[7-formyl-6-(pyridin-2-yloxy)benz[d]isoxazol-3-yl]ethyl}piperidine-1-carboxylate

The title compound (263 mg) was obtained by synthesis from the mixture of tert-butyl 4-{2-{7-[(E)-propenyl]-6-(pyridin-2-yloxy)benz[d]isoxazol-3-yl}ethyl}piperidine-1-carboxylate and tert-butyl 4-{2-{7-[(Z)-propenyl]-6-(pyridin-2-yloxy)benz[d]isoxazol-3-yl}ethyl}piperidine-1-carboxylate (357 mg) according to Example 158-(2).
1H-NMR (400 MHz, CDCl₃)
δ(ppm): 1.10-1.25 (m, 2H), 1.46 (s, 9H), 1.45-1.60 (m, 1H), 1.70-1.86 (m, 4H), 2.60-2.78 (m, 2H), 2.98-3.08 (m, 2H), 4.00-4.20 (m, 2H), 7.12 (dd, J=5.2, 7.2 Hz, 1H), 7.14 (d, J=8.4 Hz, 1H), 7.15 (d, J=8.4 Hz, 1H), 7.82 (dd, J=7.2, 8.4 Hz, 1H) , 7.83 (d, J=8.4 Hz, 1H) , 8.16 (d, J=5.2 Hz, 1H), 10.50 (s, 1H).

### (3)(4) N,N-Dimethyl{3-[2-(1-benzylpiperidin-4-yl)ethyl]-6-(pyridin-2-yloxy)benz[d]isoxazol-7-yl}methylamine

tert-Butyl 4-{2-[7-formyl-6-(pyridin-2-yloxy)benz[d]isoxazol-3-yl]ethyl}piperidine-1-carboxylate (263 mg) and dimethylamine (2 M solution in tetrahydrofuran, 1.2 mL) were dissolved in methylene chloride (3.9 mL). Acetic acid (0.13 mL) and sodium triacetoxyborohydride (259 mg) were sequentially added to the solution, and the mixture was stirred at room temperature for one hour. The reaction mixture was made alkaline with a saturated sodium bicarbonate aqueous solution and sodium carbonate, followed by extraction with chloroform. The organic layer was washed with a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain tert-butyl 4-{2-[7-dimethylaminomethyl-6-(pyridin-2-yloxy)benz[d]isoxazol-3-yl]ethyl}piperidine-1-carboxylate (265 mg).
A crude product of N,N-dimethyl{3-[2-(piperidin-4-yl)ethyl]-6-(pyridin-2-yloxy)benz[d]isoxazol-7-yl}methylamine (157 mg) was obtained by synthesis from the compound according to Example 221-(6).
The title compound (73 mg) was obtained by synthesis from the crude product of N,N-dimethyl{3-[2-(piperidin-4-yl)ethyl]-6-(pyridin-2-yloxy)benz[d]isoxazol-7-yl}methylamine (75 mg) according to Example 221-(7).
1H-NMR (400 MHz, CDCl₃)
δ(ppm): 1.25-1.45 (m, 3H), 1.68-1.85 (m, 4H), 1.88-2.02 (m, 2H), 2.30 (s, 6H), 2.82-3.03 (m, 4H), 3.49 (s, 2H), 3.74 (s, 2H), 6.99-7.08 (m, 2H), 7.05 (d, J=8.8 Hz, 1H), 7.20-7.36 (m, 5H), 7.53 (d, J=8.4 Hz, 1H), 7.70-7.77 (m, 1H) , 8.12-8.17 (m, 1H).

### (5) N,N-Dimethyl{3-[2-(1-benzylpiperidin-4-yl)ethyl]-6-(pyridin-2-yloxy)benz[d]isoxazol-7-yl}methylamine dihydrochloride

The title compound (65 mg) was obtained by synthesis from N,N-dimethyl{3-[2-(1-benzylpiperidin-4-yl)ethyl]-6-(pyridin-2-yloxy)benz[d]isoxazol-7-yl}methylamine (73 mg) according to Example 220-(11).
1H-NMR (400 MHz, DMSO-d₆)
δ(ppm): 1.50-1.98 (m, 7H), 2.80 (s, 6H), 2.30-2.95 (m, 2H), 3.00-3.12 (m, 2H), 3.22-3.34 (m, 2H), 4.24 (s, 2H), 4.54 (s, 2H), 7.18 (d, J=8.4 Hz, 1H), 7.27 (dd, J=5.2, 7.2 Hz, 1H), 7.33 (d, J=8.0 Hz, 1H), 7.40-7.48
(m, 3H), 7.62-7.69 (m, 2H), 7.99 (dd, J=7.2, 8.0 Hz, 1H), 8.05 (d, J=8.4 Hz, 1H), 8.19 (d, J=5.2 Hz, 1H), 10.92 (bs, 1H), 11.14 (bs, 1H).
ESI-MS
m/z: 236 [M+2H] ²⁺, 471 [M+H]⁺.

### Example 223

### 4-{7-(N,N-Dimethylaminomethyl)-3-{2-[1-(pyridin-2-ylmethyl)piperidin-4-yl]ethyl}benz[d]isoxazol-6-ylmethoxy}benzonitrile trihydrochloride

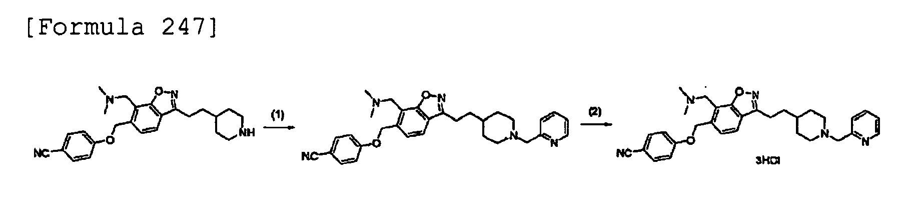

### (1) 4-{7-(N,N-Dimethylaminomethyl)-3-{2-[1-(pyridin-2-ylmethyl)piperidin-4-yl]ethyl}benz[d]isoxazol-6-ylmethoxy}benzonitrile

The crude product of 4-{7-(N,N-dimethylaminomethyl)-3-[2-(piperidin-4-yl)ethyl]benz[d]isoxazol-6-ylmethoxy}benzonitrile obtained in Example 221-(6) (72 mg) and 2-formylpyridine (23 µL) were dissolved in methylene chloride (3 mL). Acetic acid (35 µL) and sodium triacetoxyborohydride (55 mg) were sequentially added to the solution, and the mixture was stirred at room temperature for 16 hours. The reaction mixture was made alkaline with a saturated sodium bicarbonate aqueous solution and sodium carbonate, followed by extraction with chloroform. The organic layer was washed with a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (58 mg) .
1H-NMR (400 MHz, CDCl₃)
δ(ppm): 1.31-1.48 (m, 3H), 1.70-1.86 (m, 4H), 2.00-2.12 (m, 2H), 2.25 (s, 6H), 2.86-3.04 (m, 4H), 3.64 (s, 2H), 3.81 (s, 2H), 5.48 (s, 2H), 7.08 (d, J=8.4 Hz, 2H), 7.16 (dd, J=4.8, 7.2 Hz, 1H), 7.40 (d, J=7.6 Hz, 1H), 7.43 (d, J=8.0 Hz, 1H), 7.55 (d, J=8.0 Hz, 1H), 7.59 (d, J=8.4 Hz, 2H), 7.65 (dd, J=7.2, 7.6 Hz, 1H), 8.56 (d, J=4.8 Hz, 1H) .

### (2) 4-{7-(N,N-Dimethylaminomethyl)-3-{2-[1-(pyridin-2-ylmethyl)piperidin-4-yl]ethyl}benz[d]isoxazol-6-ylmethoxy}benzonitrile trihydrochloride

The title compound (55 mg) was obtained by synthesis from 4-{7-(N,N-dimethylaminomethyl)-3-{2-[1-(pyridin-2-ylmethyl)piperidin-4-yl]ethyl}benz[d]isoxazol-6-ylmethoxy}benzonitrile (58 mg) according to Example 220-(11).
1H-NMR (400 MHz, DMSO-d₆)
δ(ppm): 1.55-2.01 (m, 7H), 2.82 (s, 6H), 2.97-3.16 (m, 4H), 3.22-3.46 (m, 2H), 4.46 (s, 2H), 4.74 (s, 2H), 5.78 (s, 2H), 7.32 (d, J=8.8 Hz, 2H), 7.54 (dd, J=5.0, 8.0 Hz, 1H), 7.64 (d, J=8.4 Hz, 1H), 7.81 (d, J=8.0 Hz, 1H), 7.84 (d, J=8.8 Hz, 2H), 8.00 (dd, J=8.0, 8.0 Hz, 1H), 8.07 (d, J=8.4 Hz, 1H), 8.70 (d, J=5.0 Hz, 1H), 10. 86 (bs, 1H) , 11.25 (bs, 1H) .
ESI-MS
m/z: 255 [M+2H]²⁺, 510 [M+H]⁺.

### Example 224

### 1-{3-[2-(1-Benzylpiperidin-4-yl)ethyl]-7-(N,N-dimethylaminomethyl)benz[d]isoxazol-6-ylmethyl}-1H-pyridin-2-one dihydrochloride

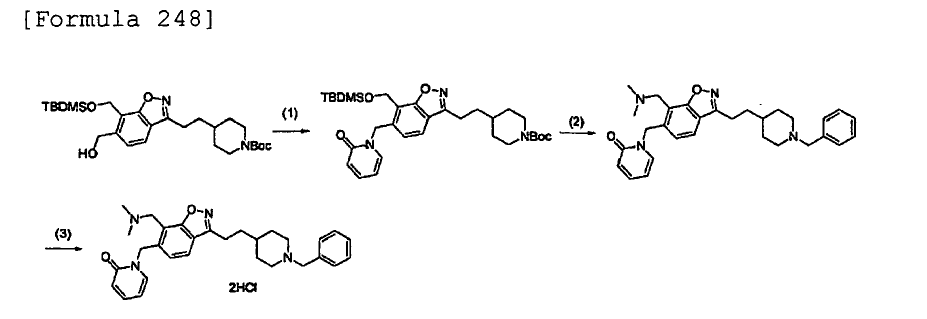

### (1) tert-Butyl 4-{2-[7-(tert-butyldimethylsilanyloxymethyl)-6-(2-oxo-2H-pyridin-1-ylmethyl)benz[d]isoxazol-3-yl]ethyl}piperidine-1-carboxylate

The title compound (200 mg) was obtained by synthesis from tert-butyl 4-{2-[7-(tert-butyldimethylsilanyloxymethyl)-6-hydroxymethylbenz[d]isoxazol-3-yl]ethyl}piperidine-1-carboxylate obtained in Example 221-(3) (0.30 g) and 2-hydroxypyridine (51 mg) according to Example 221-(4).
1H-NMR (400 MHz, CDCl₃)
δ(ppm): 0.13 (s, 6H), 0.91 (s, 9H), 1.08-1.22 (m, 2H), 1.41-1.56 (m, 1H), 1.45 (s, 9H), 1.68-1.72 (m, 4H), 2.59-2.65 (m, 2H), 2.94-3.04 (m, 2H), 4.00-4.20 (m, 2H), 5.18 (s, 2H), 5.46 (s, 2H), 6.13 (dd, J=6.4, 6.8 Hz, 1H), 6.64 (d, J=9.2 Hz, 1H), 7.16 (d, J=8.2 Hz, 1H), 7.34 (dd, J=6.4, 9.2 Hz, 1H), 7.43 (d, J=6.8 Hz, 1H), 7.49 (d, J=8.2 Hz, 1H).

### (2) 1-{3-[2-(1-Benzylpiperidin-4-yl)ethyl]-7-(N,N-dimethylaminomethyl)benz[d]isoxazol-6-ylmethyl}-1H-pyridin-2-one

The title compound (55 mg) was obtained by synthesis from tert-butyl 4-{2-[7-(tert-butyldimethylsilanyloxymethyl)-6-(2-oxo-2H-pyridin-1-ylmethyl)benz[d]isoxazol-3-yl]ethyl}piperidine-1-carboxylate (200 mg) according to Example 221-(5) (6) (7).
1H-NMR (400 MHz, CDCl₃)
δ(ppm): 1.24-1.40 (m, 3H), 1.68-1.81 (m, 4H), 1.88-1.99 (m, 2H), 2.28 (s, 6H), 2.83-2.99 (m, 4H), 3.48 (s, 2H), 3.85 (s, 2H), 5.48 (s, 2H), 6.14 (dd, J=6.8, 6.8 Hz, 1H), 6.64 (d, J=8.8 Hz, 1H), 7.16 (d, J=8.0 Hz, 1H), 7.20-7.33 (m, 5H), 7.33 (dd, J=8.8, 6.8 Hz, 1H), 7.46 (d, J=8.0 Hz, 1H), 7.52 (d, J=6.8 Hz, 1H).

### (3) 1-{3-[2-(1-Benzylpiperidin-4-yl)ethyl]-7-(N,N-dimethylaminomethyl)benz[d]isoxazol-6-ylmethyl}-1H-pyridin-2-one dihydrochloride

The title compound (50 mg) was obtained by synthesis from 1-{3-[2-(1-benzylpiperidin-4-yl)ethyl]-7-(N,N-dimethylaminomethyl)benz[d]isoxazol-6-ylmethyl}-1H-pyridin-2-one (55 mg) according to Example 220-(11).
1H-NMR (400 MHz, DMSO-d₆)
δ(ppm): 1.44-1.81 (m, 5H), 1.83-1.98 (m, 2H), 2.78-3.11 (m, 4H), 2.89 (s, 6H), 3.21-3.32 (m, 2H), 4.22 (s, 2H), 4.87 (s, 2H), 5.57 (s, 2H), 6.43 (dd, J=6.8, 6.8 Hz, 1H) , 6.48 (d, J=8.8 Hz, 1H), 7.08 (d, J=8.0 Hz, 1H), 7.37-7.50 (m, 3H), 7.57 (dd, J=6.8, 8.8 Hz, 1H), 7.60-7.70 (m, 2H), 7.97 (d, J=8.0 Hz, 1H), 8.18 (d, J=6.8 Hz, 1H), 11.00 (bs, 1H), 11.09 (bs, 1H).
ESI-MS
m/z: 243 [M+2H]²⁺, 485 [M+H]⁺.

### Example 225

### N,N-Dimethyl{3-[2-(1-benzylpiperidin-4-yl)ethyl]-6-(4-fluorophenoxymethyl)benz[d]isoxazol-7-yl}methylamine dihydrochloride

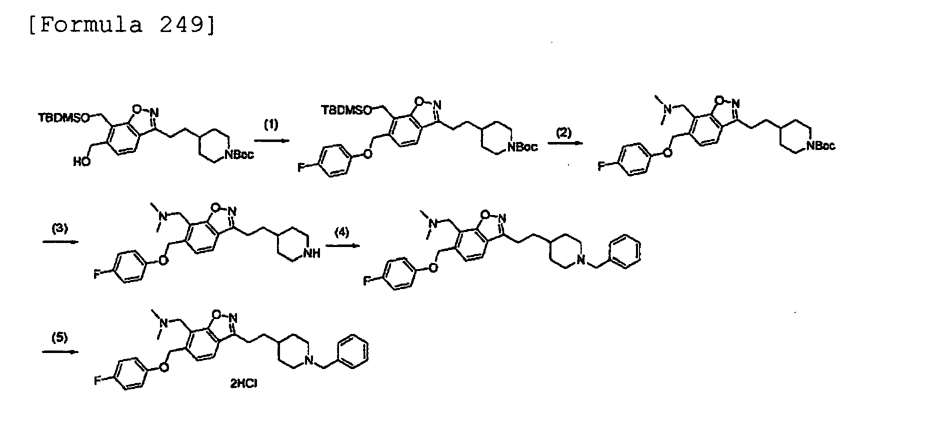

### (1) tert-Butyl 4-{2-[7-(tert-butyldimethylsilanyloxymethyl)-6-(4-fluorophenoxymethyl)benz[d]isoxazol-3-yl]ethyl}piperidine-1-carboxylate

The title compound (571 mg) was obtained by synthesis from tert-butyl 4-{2-[7-(tert-butyldimethylsilanyloxymethyl)-6-hydroxymethylbenz[d]isoxazol-3-yl]ethyl}piperidine-1-carboxylate obtained in Example 221-(3) (614 mg) and 4-fluorophenol (123 mg) according to Example 221-(4).
1H-NMR (400 MHz, CDCl₃) δ(ppm): 0.092 (s, 6H), 0.89 (s, 9H), 1.10-1.25 (m, 2H), 1.41-1.60 (m, 1H), 1.46 (s, 9H), 1.70-1.88 (m, 4H), 2.58-2.78 (m, 2H), 2.95-3.09 (m, 2H), 4.00-4.20 (m, 2H), 5.12 (s, 2H), 5.36 (s, 2H), 6.88-7.02 (m, 4H), 7.49 (d, J=8.0 Hz, 1H), 7.56 (d, J=8.0 Hz, 1H).

### (2)(3)(4) N,N-Dimethyl{3-[2-(1-benzylpiperidin-4-yl)ethyl]-6-(4-fluorophenoxymethyl)benz[d]isoxazol-7-yl}methylamine

The title compound (91 mg) was obtained by synthesis from tert-butyl 4-{2-[7-(tert-butyldimethylsilanyloxymethyl)-6-(4-fluorophenoxymethyl)benz[d]isoxazol-3-yl]ethyl}piperidine-1-carboxylate (686 mg) according to Example 221- (5) (6) (7).
1H-NMR (400 MHz, CDCl₃)
δ(ppm): 1.27-1.42 (m, 3H), 1.70-1.84 (m, 4H), 1.89-2.00 (m, 2H), 2.25 (s, 6H), 2.84-3.02 (m, 4H), 3.49 (s, 2H), 3.80 (s, 2H), 5.36 (s, 2H), 6.91-7.02 (m, 4H), 7.20-7.35 (m, 5H), 7.47 (d, J=8.0 Hz, 1H), 7.54 (d, J=8.0 Hz, 1H) .

### (5) N,N-Dimethyl{3-[2-(1-benzylpiperidin-4-yl)ethyl]-6-(4-fluorophenoxymethyl)benz[d]isoxazol-7-yl}methylamine dihydrochloride

The title compound (74 mg) was obtained by synthesis from N,N-dimethyl{3-[2-(1-benzylpiperidin-4-yl)ethyl]-6-(4-fluorophenoxymethyl)benz[d]isoxazol-7-yl}methylamine (91 mg) according to Example 220-(11).
1H-NMR (400 MHz, DMSO-d₆)
δ(ppm): 1.50-1.99 (m, 7H), 2.73-2.95 (m, 2H), 2.83 (s, 6H), 2.99-3.12 (m, 2H), 3.22-3.42 (m, 2H), 4.24 (s, 2H), 4.72 (s, 2H), 5.57 (s, 2H), 7.11-7.24 (m, 4H), 7.41-7.52 (m, 3H), 7.58-7.71 (m, 2H), 7.65 (d, J=8.4 Hz, 1H), 8.05 (d, J=8.4 Hz, 1H), 10.64-10.89 (m, 2H).
ESI-MS
m/z: 251 [M+2H]²⁺, 502 [M+H]⁺.

### Example 226

### N,N-Dimethyl{6-(4-fluorophenoxymethyl)-3-{2-[1-(pyridin-2-ylmethyl)piperidin-4-yl]ethyl}benz[d]isoxazol-7-yl}methylamine trihydrochloride

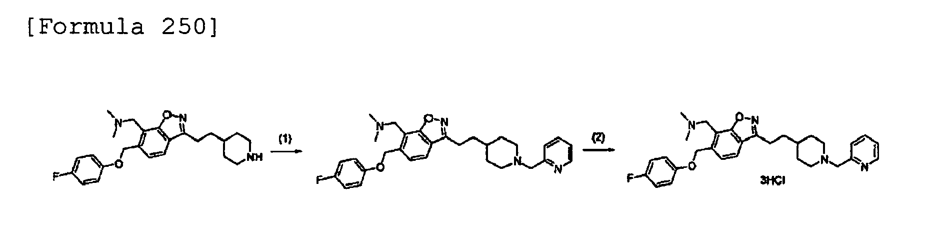

### (1) N,N-Dimethyl{6-(4-fluorophenoxymethyl)-3-{2-[1-(pyridin-2-ylmethyl)piperidin-4-yl]ethyl}benz[d]isoxazol-7-yl}methylamine

The title compound (95 mg) was obtained by synthesis from the crude product of N,N-dimethyl{6-(4-fluorophenoxymethyl)-3-[2-(piperidin-4-yl)ethyl]-benz[d]isoxazol-7-yl}methylamine obtained in Example 225-(3) (92 mg) according to Example 223-(1).
1H-NMR (400 MHz, CDCl₃)
δ(ppm) : 1.30-1.48 (m, 3H), 1.70-1.87 (m, 4H), 1.99-2.12 (m, 2H), 2.25 (s, 6H), 2.85-3.05 (m, 4H), 3.64 (s, 2H), 3.80 (s, 2H), 5.36 (s, 2H), 6.90-7.03 (m, 4H), 7.15 (dd, J=4.8, 7.6 Hz, 1H), 7.41 (d, J=7.6 Hz, 1H), 7.48 (d, J=8.0 Hz, 1H) , 7.54 (d, J=8.0 Hz, 1H) , 7.64 (dd, J=7.6, 7. 6 Hz, 1H), 8.56 (d, J=4.8 Hz, 1H) .

### (2) N,N-Dimethyl{6-(4-fluorophenoxymethyl)-3-{2-[1-(pyridin-2-ylmethyl)piperidin-4-yl]ethyl}benz[d]isoxazol-7-yl}methylamine trihydrochloride

The title compound (90 mg) was obtained by synthesis from N,N-dimethyl{6-(4-fluorophenoxymethyl)-3-{2-[1-(pyridin-2-ylmethyl)piperidin-4-yl]ethyl}benz[d]isoxazol-7-yl}methylamine (95 mg) according to Example 220-(11).
1H-NMR (400 MHz, DMSO-d₆) δ (ppm): 1.52-1.69 (m, 3H), 1.70-1.84 (m, 2H), 1.86-2.00 (m, 2H), 2.83 (s, 6H), 2.95-3.12 (m, 4H), 3.32-3.46 (m, 2H), 4.44 (s, 2H), 4.72 (s, 2H), 5.58 (s, 2H), 7.11-7.22 (m, 4H), 7.49 (dd, J=5.2, 7.6 Hz, 1H), 7.65 (d, J=8.0 Hz, 1H), 7.67 (d, J=7.6 Hz, 1H), 7.94 (dd, J=7.6, 7.6 Hz, 1H), 8.06 (d, J=8.0 Hz, 1H), 8.68 (d, J=5.2 Hz, 1H), 10.41 (bs, 1H), 10.80 (bs, 1H).
ESI-MS
m/z: 252 [M+2H]²⁺, 503 [M+H]⁺.

### Example 227

### N,N-Dimethyl{6-(4-fluorophenoxymethyl)-3-{2-[1-(tetrahydrofuran-2-ylmethyl)piperidin-4-yl]ethyl}benz[d]isoxazol-7-yl}methylamine dihydrochloride

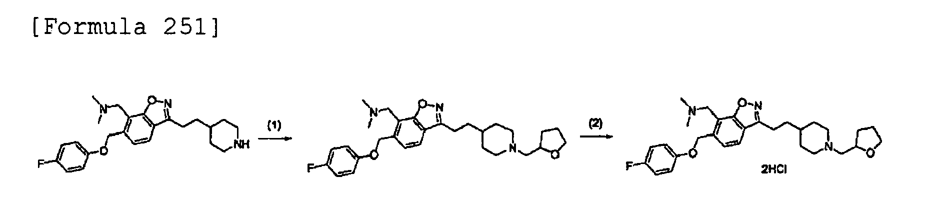

### (1) N,N-Dimethyl(6-(4-fluorophenoxymethyl)-3-{2-[1-(tetrahydrofuran-2-ylmethyl)piperidin-4-yl]ethyl}benz[d]isoxazol-7-yl)methylamine

A mixture of the crude product of N,N-dimethyl[6-(4-fluorophenoxymethyl)-3-(2-piperidin-4-ylethyl)benz[d]isoxazol-7-yl]methylamine obtained in Example 225-(3) (144 mg), tetrahydrofurfuryl bromide (99 µL), N,N-diisopropylethylamine (307 µL), sodium iodide (53 mg) and acetonitrile (5 mL) was heated under reflux for 21 hours. The reaction mixture was cooled to room temperature. The mixture was made alkaline with a saturated sodium bicarbonate solution, followed by extraction with chloroform. The organic layer was washed with a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (123 mg).
1H-NMR (400 MHz, CDCl₃)
δ(ppm): 1.30-1.55 (m, 4H), 1.68-2.05 (m, 9H), 2.25 (s, 6H), 2.34-2.52 (m, 2H), 2.93-3.07 (m, 4H), 3.70-3.78 (m, 1H), 3.80 (s, 2H), 3.83-3.92 (m, 1H), 3.98-4.08 (m, 1H) , 5.36 (s, 2H), 6.90-7.03 (m, 4H), 7.48 (d, J=8.0 Hz, 1H), 7.54 (d, J=8.0 Hz, 1H).

### (2) N,N-Dimethyl{6-(4-fluorophenoxymethyl)-3-{2-[1-(tetrahydrofuran-2-ylmethyl)piperidin-4-yl]ethyl}benz[d]isoxazol-7-yl}methylamine dihydrochloride

The title compound (120 mg) was obtained by synthesis from N,N-dimethyl{6-(4-fluorophenoxymethyl)-3-{2-[1-(tetrahydrofuran-2-ylmethyl)piperidin-4-yl]ethyl}benz[d]isoxazol-7-yl}methylamine (126 mg) according to Example 220-(11).
1H-NMR (400 MHz, DMSO-d₆)
δ(ppm): 1.43-2.10 (m, 11H), 2.82 (s, 6H), 2.83-3.12 (m, 4H), 3.14-3.30 (m, 2H), 3.34-3.50 (m, 1H), 3.55-3.66 (m, 1H), 3.67-3.87 (m, 2H), 4.27-4.40 (m, 1H), 4.73 (s, 2H), 5.62 (s, 2H), 7.09-7.23 (m, 4H), 7.65 (d, J=8.4 Hz, 1H), 8.06 (d, J=8.4 Hz, 1H), 10.43 (bs, 1H), 11.06 (bs, 1H) .
ESI-MS
m/z: 248 [M+2H]²⁺, 496 [M+H]⁺.

### Example 228

### 3-{4-{2-[6-Cyclopropylmethoxy-7-(N,N-dimethylaminomethyl)benz[d]isoxazol-3-yl]ethyl}piperidin-1-ylmethyl}thiophene-2-carbonitrile dihydrochloride

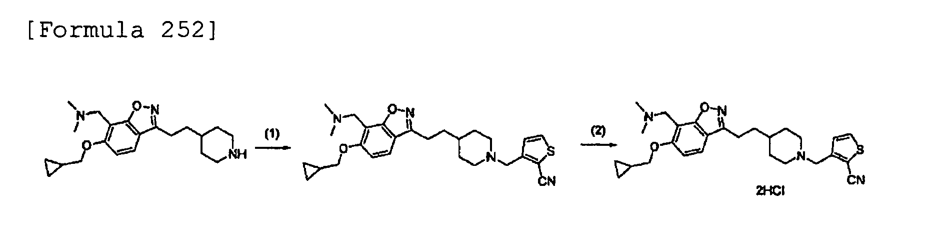

### (1) 3-{4-{2-[6-Cyclopropylmethoxy-7-(N,N-dimethylaminomethyl)benz[d]isoxazol-3-yl]ethyl}piperidin-1-ylmethyl}thiophene-2-carbonitrile

The title compound (77 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (100 mg) and 3-formylthiophene-2-carbonitrile [CAS Registry No. 41056-99-9] (50 mg) according to Example 223-(1).
1H-NMR (400 MHz, CDCl₃) δ(ppm): 0.33-0.41 (m, 2H), 0.60-0.69 (m, 2H), 1.25-1.43 (m, 4H), 1.70-1.82 (m, 4H), 1.98-2.10 (m, 2H), 2.34 (s, 6H), 2.80-2.98 (m, 4H), 3.65 (s, 2H), 3.83 (s, 2H), 3.91-3.98 (m, 2H), 6.90 (d, J=8.8 Hz, 1H) , 7.15 (d, J=5.2 Hz, 1H), 7.44 (d, J=8.8 Hz, 1H), 7.50 (d, J=5.2 Hz, 1H) .

### (2) 3-{4-{2-[6-Cyclopropylmethoxy-7-(N,N-dimethylaminomethyl)benz[d]isoxazol-3-yl]ethyl}piperidin-1-ylmethyl}thiophene-2-carbonitrile dihydrochloride

The title compound (76 mg) was obtained by synthesis from 3-{4-{2-[6-cyclopropylmethoxy-7-(N,N-dimethylaminomethyl)benz[d]isoxazol-3-yl]ethyl}piperidin-1-ylmethyl}thiophene-2-carbonitrile (77 mg) according to Example 220- (11) .
1H-NMR (400 MHz, DMSO-d₆) δ(ppm): 0.36-0.46 (m, 2H), 0.55-0.67 (m, 2H), 1.28-1.42 (m, 1H), 1.53-1.80 (m, 5H), 1.86-1.98 (m, 2H), 2.77 (s, 6H), 2.91-3.10 (m, 4H), 3.27-3.40 (m, 2H), 4.08 (d, J=7.2 Hz, 2H), 4.37 (s, 2H), 4.46 (s, 2H), 7.25 (d, J=8.8 Hz, 1H), 7.87 (d, J=5.2 Hz, 1H), 7.97 (d, J=8.8 Hz, 1H) , 8.17 (d, J=5.2 Hz, 1H) , 10.63 (bs, 1H) , 11.57 (bs, 1H) .
ESI-MS
m/z: 239 [M+2H]²⁺, 479 [M+H]⁺.

### Example 229

### N,N-Dimethyl{6-cyclopropylmethoxy-3-{2-[1-(2,3-difluorobenzyl)piperidin-4-yl]ethyl}benz[d]isoxazol-7-yl}methylamine dihydrochloride

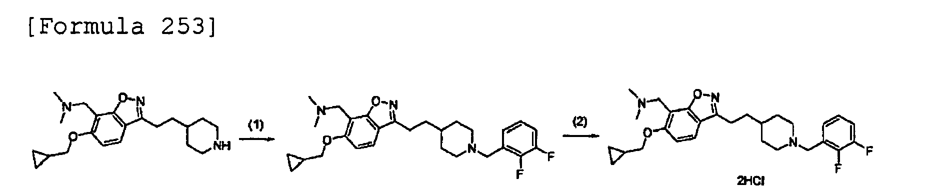

### (1) N,N-Dimethyl{6-cyclopropylmethoxy-3-{2-[1-(2,3-difluorobenzyl)piperidin-4-yl]ethyl}benz[d]isoxazol-7-yl}methylamine

The title compound (88 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (100 mg) and 2,3-difluorobenzaldehyde (40 µL) according to Example 223-(1) .
1H-NMR (400 MHz, CDCl₃)
δ(ppm): 0.33-0.41 (m, 2H), 0.60-0.69 (m, 2H), 1.24-1.40 (m, 4H), 1.70-1.82 (m, 4H), 1.97-2.08 (m, 2H), 2.34 (s, 6H), 2. 84-2. 98 (m, 4H), 3.58 (s, 2H), 3. 83 (s, 2H), 3.91-3.96 (m, 2H), 6.90 (d, J=8.8 Hz, 1H), 6.99-7.16 (m, 3H), 7.44 (d, J=8.8Hz, 1H).

### (2) N,N-Dimethyl{6-cyclopropylmethoxy-3-{2-[1-(2,3-difluorobenzyl)piperidin-4-yl]ethyl}benz[d]isoxazol-7-ylmethyl}methylamine dihydrochloride

The title compound (73 mg) was obtained by synthesis from N,N-dimethyl{6-cyclopropylmethoxy-3-{2-[1-(2,3-difluorobenzyl)piperidin-4-yl]ethyl}benz[d]isoxazol-7-ylmethyl}methylamine (88 mg) according to Example 220-(11).
1H-NMR (400 MHz, DMSO-d₆)
δ(ppm): 0.37-0.46 (m, 2H), 0.56-0.65 (m, 2H), 1.29-1.42 (m, 1H), 1.52-1.78 (m, 5H), 1.84-1.97 (m, 2H), 2.76 (s, 6H), 2.89-3.08 (m, 4H), 3.31-3.43 (m, 2H), 4.08 (d, J=7.2 Hz, 2H), 4.34 (s, 2H), 4.45 (s, 2H), 7.24 (d, J=8.8 Hz, 1H), 7.26-7.38 (m, 1H), 7.49-7.62 (m, 1H), 7.67-7.78 (m, 1H), 7.96 (d, J=8.8 Hz, 1H), 10.93 (bs, 1H), 11.39 (bs, 1H).
ESI-MS
m/z: 242 [M+2H] ²⁺, 484 [M+H]⁺.

### Example 230

### 1-{4-{2-[6-Cyclopropylmethoxy-7-(N,N-dimethylaminomethyl)benz[d]isoxazol-3-yl]ethyl}piperidin-1-ylmethyl}cyclopentanecarbonitrile dihydrochloride

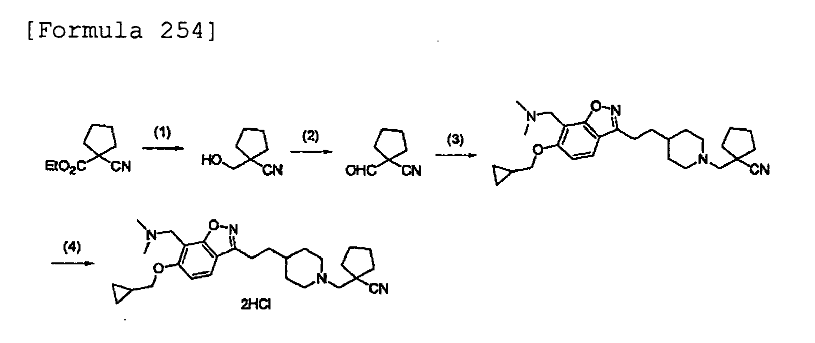

### (1) 1-(Hydroxymethyl)cyclopentanecarbonitrile

Ethyl 1-cyanocyclopentanecarboxylate [CAS Regsitry No. 28247-14-5] (500 mg) was dissolved in tetrahydrofuran (10 mL). A suspension of sodium borohydride (1.1 g) in tetrahydrofuran (6.8 mL) and water (0.75 mL) was added to the solution at room temperature, and then the mixture was stirred at 50°C for one hour. The reaction mixture was cooled to room temperature, and then water (10 mL) was added. Further, 5 N hydrochloric acid was added dropwise until bubbling was stopped, followed by extraction with ethyl acetate. The organic layer was washed with a saturated sodium chloride solution and dried over magnesium sulfate. The drying agent was removed by filtration and then the solvent was evaporated under reduced pressure. The residue was purified by silica gel column chromatography (heptane-ethyl acetate) to obtain the title compound (320 mg).
1H-NMR (400 MHz, CDCl₃)
δ(ppm): 1.67-1.92 (m, 6H), 2.03-2.18 (m, 2H), 3.64 (s, 2H) .

### (2)(3) 1-{4-{2-[6-Cyclopropylmethoxy-7-(N,N-dimethylaminomethyl)benz[d]isoxazol-3-yl]ethyl}piperidin-1-ylmethyl}cyclopentanecarbonitrile

1-(Hydroxymethyl)cyclopentanecarbonitrile (320 mg) and triethylamine (2.1 mL) were dissolved in dimethyl sulfoxide (6.5 mL). A solution of a sulfurtrioxide-pyridine complex (1.2 g) in dimethyl sulfoxide (6.5 mL) was slowly added to the solution, and then the mixture was stirred at room temperature for one hour. Water was added to the reaction mixture, followed by extraction with ethyl acetate. The organic layer was washed with 1 M hydrochloric acid twice and a saturated sodium chloride solution twice and dried over magnesium sulfate. The organic layer was filtered through a small amount of silica gel and then the solvent was evaporated under reduced pressure to obtain a crude product of 1-formylcyclopentanecarbonitrile (140 mg).
The title compound (63 mg) was obtained by synthesis from the crude product of 1-formylcyclopentanecarbonitrile (100 mg) and N,N-dimethyl{6-cyclopropylmethoxy-3-[2-(piperidin-4-yl)ethyl]benz[d]isoxazol-7-yl}methylamine obtained in Preparation Example 3-(3) (100 mg) according to Example 223- (1) .
1H-NMR (400 MHz, CDCl₃)
δ(ppm): 0.34-0.42 (m, 2H), 0.60-0.69 (m, 2H), 1.24-1.40 (m, 4H), 1.64-1.88 (m, 10H), 2.00-2.11 (m, 2H), 2.15-2.25 (m, 2H), 2.34 (s, 6H), 2.47 (s, 2H), 2.89-3.04 (m, 4H), 3.83 (s, 2H), 3.91-3.97 (m, 2H), 6.90 (d, J=8.4 Hz, 1H) , 7.45 (d, J=8.4 Hz, 1H).

### (4) 1-{4-{2-[6-Cyclopropylmethoxy-7-(N,N-dimethylaminomethyl)benz[d]isoxazol-3-yl]ethyl}piperidin-1-ylmethyl}cyclopentanecarbonitrile dihydrochloride

The title compound (53 mg) was obtained by synthesis from 1-{4-{2-[6-cyclopropylmethoxy-7-(N,N-dimethylaminomethyl)benz[d]isoxazol-3-yl]ethyl}piperidin-1-ylmethyl}cyclopentanecarbonitrile (60 mg) according to Example 220-(11).
1H-NMR (400 MHz, DMSO-d₆)
δ(ppm): 0.36-0.48 (m, 2H), 0.54-0.67 (m, 2H), 1.29-1.42 (m, 1H), 1.53-2.28 (s, 15H), 2.76 (s, 6H), 2.95-3.17 (m, 4H), 3.18-3.69 (m, 4H), 4.08 (d, J=6.8 Hz, 2H), 4.46 (s, 2H), 7.25 (d, J=8.4 Hz, 1H), 7.97 (d, J=8.4 Hz, 1H), 10.88 (bs, 2H).
ESI-MS
m/z: 465 [M+H]⁺.

### Example 231

### N,N-Dimethyl{6-cyclopropylmethoxy-3-{2-[1-(tetrahydrofuran-3-ylmethyl)piperidin-4-yl]ethyl}benz[d]isoxazol-7-yl}methylamine dihydrochloride

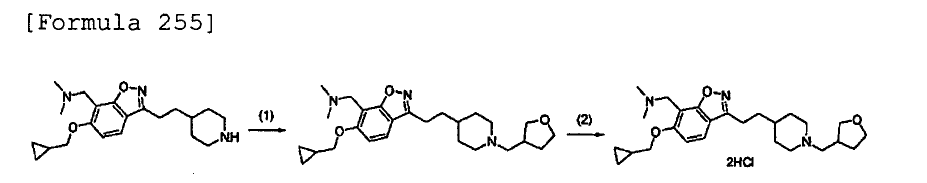

### (1) N,N-Dimethyl{6-cyclopropylmethoxy-3-{2-[1-(tetrahydrofuran-3-ylmethyl)piperidin-4-yl]ethyl}benz[d]isoxazol-7-ylmethyl}methylamine

N,N-Dimethyl{6-cyclopropylmethoxy-3-[2-(piperidin-4-yl)ethyl]benz[d]isoxazol-7-yl}methylamine obtained in Preparation Example 3-(3) (100 mg) and tetrahydrofuran-3-carbaldehyde (50% aqueous solution, 168 mg) were dissolved in 1,2-dichloroethane (3 mL). Sodium triacetoxyborohydride (119 mg) was added to the solution, and the mixture was stirred at room temperature for 22 hours. The reaction mixture was made alkaline with a saturated sodium bicarbonate aqueous solution and sodium carbonate, followed by extraction with chloroform. The organic layer was washed with a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (94 mg).
1H-NMR (400 MHz, CDCl₃)
δ(ppm): 0.33-0.42 (m, 2H), 0.60-0.69 (m, 2H), 1.21-1.41 (m, 4H), 1.52-1.66 (m, 1H), 1.69-1.81 (m, 4H), 1.83-2.07 (m, 3H), 2.24-2.52 (m, 3H), 2.34 (s, 6H), 2.82-2.98 (m, 4H), 3.44-3.50 (m, 1H) , 3.69-3.78 (m, 1H) , 3.79-3.90 (m, 2H), 3.82 (s, 2H), 3.92-3.97 (m, 2H), 6.90 (d, J=8.8 Hz, 1H), 7.45 (d, J=8.8 Hz, 1H).

### (2) N,N-Dimethyl{6-cyclopropylmethoxy-3-{2-[1-(tetrahydrofuran-3-ylmethyl)piperidin-4-yl]ethyl}benz[d]isoxazol-7-yl}methylamine dihydrochloride

The title compound (65 mg) was obtained by synthesis from N,N-dimethyl{6-cyclopropylmethoxy-3-{2-[1-(tetrahydrofuran-3-ylmethyl)piperidin-4-yl]ethyl}benz[d]isoxazol-7-yl}methylamine (94 mg) according to Example 220-(11).
1H-NMR (400 MHz, DMSO-d₆)
δ(ppm): 0.38-0.46 (m, 2H), 0.57-0.66 (m, 2H), 1.28-1.42 (m, 1H), 1.49-1.79 (s, 5H), 1.81-1.97 (m, 2H), 2.03-2.17 (m, 1H), 2.60-2.92 (m, 3H), 2.77 (s, 6H), 2.95-3.22 (m, 4H), 3.38-3.78 (m, 6H), 3.80-3.90 (m, 1H) , 4.08 (d, J=7.2 Hz, 2H), 4.46 (s, 2H), 7.25 (d, J=8.8 Hz, 1H), 7.97 (d, J=8.8 Hz, 1H) , 10.65 (bs, 1H) , 10.79 (bs, 1H) .
ESI-MS
m/z: 221 [M+2H]²⁺, 442 [M+H]⁺.

### Example 232

### 1-{4-{2-[6-Cyclopropylmethoxy-7-(N,N-dimethylaminomethyl)benz[d]isoxazol-3-yl]ethyl}piperidin-1-ylmethyl}cyclobutanecarbonitrile dihydrochloride

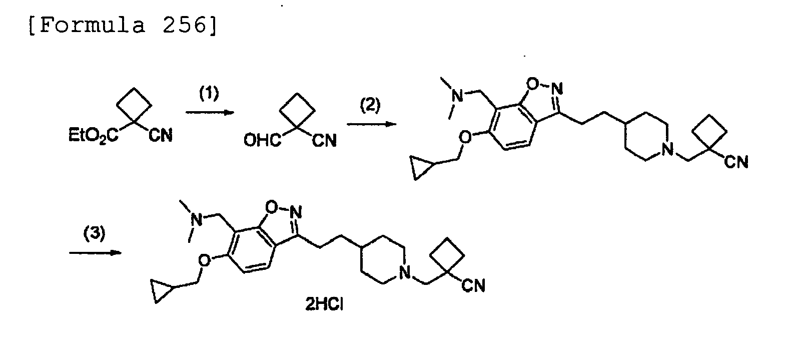

### (1)(2) 1-{4-{2-[6-Cyclopropylmethoxy-7-(N,N-dimethylaminomethyl)benz[d]isoxazol-3-yl]ethyl}piperidin-1-ylmethyl}cyclobutanecarbonitrile

1-(Hydroxymethyl)cyclobutanecarbonitrile (1.1 g) was obtained by synthesis from Ethyl 1-cyanocyclobutanecarboxylate [CAS Registry No. 28246-87-9] (1.8 g) according to Example 230-(1).
The title compound (91 mg) was obtained by synthesis from the 1-(hydroxymethyl)cyclobutanecarbonitrile (600 mg) according to Example 230-(2)(3).
1H-NMR (400 MHz, CDCl₃)
δ(ppm): 0.34-0.41 (m, 2H), 0.60-0.69 (m, 2H), 1.25-1.40 (m, 4H), 1.68-1.81 (m, 4H), 1.93-2.04 (m, 1H), 2.07-2.27 (m, 5H), 2.34 (s, 6H), 2.41-2.53 (m, 2H), 2.61 (s, 2H), 2.87-2.98 (m, 4H), 3.83 (s, 2H), 3.92-3.97 (m, 2H), 6.90 (d, J=8.8 Hz, 1H) , 7.45 (d, J=8.8 Hz, 1H) .

### (3) 1-{4-{2-[6-Cyclopropylmethoxy-7-(N,N-dimethylaminomethyl)benz[d]isoxazol-3-yl]ethyl}piperidin-1-ylmethyl}cyclobutanecarbonitrile dihydrochloride

The title compound (91 mg) was obtained by synthesis from 1-{4-{2-[6-cyclopropylmethoxy-7-(N,N-dimethylaminomethyl)benz[d]isoxazol-3-yl]ethyl}piperidin-1-ylmethyl}cyclobutanecarbonitrile (91 mg) according to Example 220-(11).
1H-NMR (400 MHz, DMSO-d₆)
δ(ppm): 0.35-0.47 (m, 2H), 0.56-0.67 (m, 2H), 1.28-1.41 (m, 1H) , 1.51-2.20 (s, 11H), 2.40-2.58 (m, 2H), 2.80 (s, 6H), 2.94-3.14 (m, 4H), 3.40-3.52 (m, 2H), 3.57 (s, 2H), 4.08 (d, J=7.2 Hz, 2H), 4.48 (s, 2H), 7.26 (d, J=8.8 Hz, 1H), 7.97 (d, J=8.8 Hz, 1H), 10.34 (bs, 1H), 10.57 (bs, 1H).
ESI-MS
m/z: 451 [M+H]⁺.

### Example 233

### 2-{4-{2-[6-Cyclopropylmethoxy-7-(N,N-dimethylaminomethyl)benz[d]isoxazol-3-yl]ethyl}piperidin-1-ylmethyl}nicotinonitrile dihydrochloride

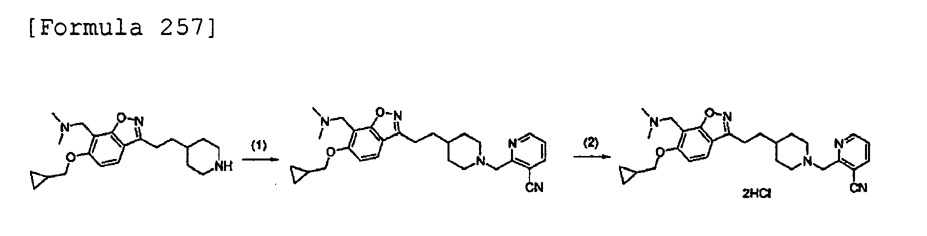

### (1) 2-{4-{2-[6-Cyclopropylmethoxy-7-(N,N-dimethylaminomethyl)benz[d]isoxazol-3-yl]ethyl}piperidin-1-ylmethyl}nicotinonitrile

The title compound (93 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (100 mg) and 2-formylnicotinonitrile [CAS Registry No. 405174-98-3] (74 mg) according to Example 223-(1).
1H-NMR (400 MHz, CDCl₃) δ(ppm): 0.33-0.43 (m, 2H), 0.60-0.70 (m, 2H), 1.22-1.46
(m, 4H), 1.68-1.82 (m, 4H), 2.12-2.25 (m, 2H), 2.33 (s, 6H), 2.87-3.00 (m, 4H), 3.818 (s, 2H), 3.822 (s, 2H), 3.90-4.00 (m, 2H), 6.90 (d, J=8.8 Hz, 1H), 7.32 (dd, J=5.2, 7.6 Hz, 1H), 7.44 (d, J=8.8 Hz, 1H), 7.96 (d, J=7.6Hz, 1H), 8.76 (d, J=5.2 Hz, 1H) .

### (2) 2-{4-{2-[6-Cyclopropylmethoxy-7-(N,N-dimethylaminomethyl)benz[d]isoxazol-3-yl]ethyl}piperidin-1-ylmethyl}nicotinonitrile dihydrochloride

The title compound (89 mg) was obtained by synthesis from 2-{4-{2-[6-cyclopropylmethoxy-7-(N,N-dimethylaminomethyl)benz[d]isoxazol-3-yl]ethyl}piperidin-1-ylmethyl}nicotinonitrile (93 mg) according to Example 220-(11).
1H-NMR (400 MHz, DMSO-d₆) δ(ppm): 0.37-0.46 (m, 2H), 0.55-0.67 (m, 2H), 1.29-1.42 (m, 1H) , 1.53-2.06 (m, 7H), 2.77 (s, 6H), 2.96-3.07 (m, 2H), 3.11-3.28 (m, 2H), 3.50-3.66 (m, 2H), 4.08 (d, J=6.8 Hz, 2H), 4.46 (s, 2H), 4.72 (s, 2H), 7.25 (d, J=8.8 Hz, 1H), 7.71 (dd, J=4.8, 8.0 Hz, 1H), 7.98 (d, J=8.8 Hz, 1H), 8.47 (d, J=8.0 Hz, 1H), 8.94 (d, J=4.8 Hz, 1H), 10.57 (bs, 1H), 10.88 (bs, 1H).
ESI-MS
m/z: 237 [M+2H]²⁺, 474 [M+H]⁺.

### Example 234

### 6-{4-{2-[6-Cyclopropylmethoxy-7-(N,N-dimethylaminomethyl)benz[d]isoxazol-3-yl]ethyl}piperidin-1-ylmethyl}nicotinonitrile dihydrochloride

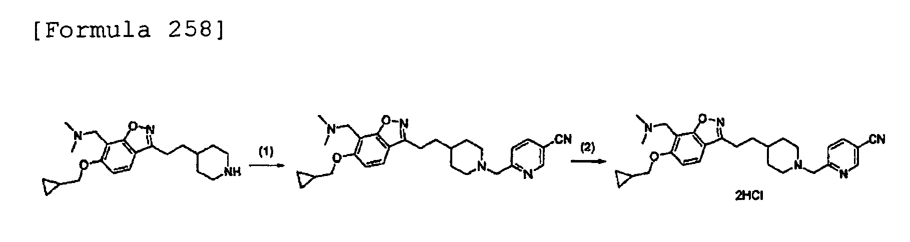

### (1) 6-{4-{2-[6-Cyclopropylmethoxy-7-(N,N-dimethylaminomethyl)benz[d]isoxazol-3-yl]ethyl}piperidin-1-ylmethyl}nicotinonitrile

The title compound (90 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (100 mg) and 6-formylnicotinonitrile [CAS Registry No. 206201-64-1] (74 mg) according to Example 223-(1).
1H-NMR (400 MHz, CDCl₃)
δ(ppm): 0.33-0.42 (m, 2H), 0.60-0.69 (m, 2H), 1.25-1.45 (m, 4H), 1.71-1.85 (m, 4H), 2.03-2.16 (m, 2H), 2.34 (s, 6H), 2.80-2.89 (m, 2H), 2.91-3.00 (m, 2H), 3.69 (s, 2H), 3.82 (s, 2H), 3.91-3.98 (m, 2H), 6.90 (d, J=8.8 Hz, 1H), 7.44 (d, J=8.8 Hz, 1H), 7.61 (d, J=8.0 Hz, 1H) , 7.92 (d, J=8.0 Hz, 1H) , 8.82 (s, 1H) .

### (2) 6-{4-{2-[6-Cyclopropylmethoxy-7-(N,N-dimethylaminomethyl)benz[d]isoxazol-3-yl]ethyl}piperidin-1-ylmethyl}nicotinonitrile dihydrochloride

The title compound (94 mg) was obtained by synthesis from 6-{4-{2-[6-cyclopropylmethoxy-7-(N,N-dimethylaminomethyl)benz[d]isoxazol-3-yl]ethyl}piperidin-1-ylmethyl}nicotinonitrile (90 mg) according to Example 220-(11).
1H-NMR (400 MHz, DMSO-d₆)
δ(ppm): 0.35-0.50 (m, 2H), 0.55-0.70 (m, 2H), 1.27-1.43 (m, 1H), 1.50-2.08 (m, 7H), 2.78 (s, 6H), 2.92-3.15 (m, 4H), 3.32-3.55 (m, 2H), 4.07 (d, J=6.8 Hz, 2H), 4.46 (s, 2H), 4.53 (s, 2H), 7.25 (d, J=8.8 Hz, 1H), 7.96 (d, J=8.8 Hz, 1H), 7.96 (d, J=8.4 Hz, 1H), 8.47 (d, J=8.4 Hz, 1H), 9.13 (s, 1H), 10.65 (bs, 1H), 10.95 (bs, 1H).
ESI-MS
m/z: 237 [M+2H]²⁺, 474 [M+H]⁺.

### Example 235

### 1-{2-{4-{2-[6-Cyclopropylmethoxy-7-(N,N-dimethylaminomethyl)benz[d]isoxazol-3-yl]ethyl}piperidin-1-yl}ethyl}-1H-pyridin-2-one dihydrochloride

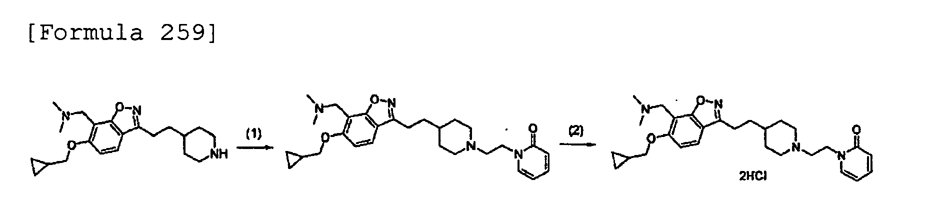

### (1) 1-{2-{4-{2-[6-Cyclopropylmethoxy-7-(N,N-dimethylaminomethyl)benz[d]isoxazol-3-yl]ethyl}piperidin-1-yl}ethyl}-1H-pyridin-2-one

The title compound (78 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (100 mg) and (2-oxo-2H-pyridin-1-yl)acetaldehyde [CAS Registry No. 408530-65-4] (97 mg) according to Example 223-(1).
1H-NMR (400 MHz, CDCl₃)
δ(ppm): 0.33-0.42 (m, 2H), 0.60-0.69 (m, 2H), 1.18-1.43 (m, 4H), 1.71-1.81 (m, 4H), 2.01-2.11 (m, 2H), 2.34 (s, 6H), 2.65 (t, J=6.4 Hz, 2H), 2.84-2.99 (m, 4H), 3.82 (s, 2H), 3.92-3.97 (m, 2H), 4.03 (t, J=6.4 Hz, 2H), 6.10-6.16 (m, 1H), 6.52-6.58 (m, 1H), 6.90 (d, J=8.8 Hz, 1H) , 7.27-7.35 (m, 2H), 7.44 (d, J=8.8 Hz, 1H).

### (2) 1-{2-{4-{2-[6-Cyclopropylmethoxy-7-(N,N-dimethylaminomethyl)benz[d]isoxazol-3-yl]ethyl}piperidin-1-yl}ethyl}-1H-pyridin-2-one dihydrochloride

The title compound (78 mg) was obtained by synthesis from 1-{2-{4-{2-[6-cyclopropylmethoxy-7-(N,N-dimethylaminomethyl)benz[d]isoxazol-3-yl]ethyl}piperidin-1-yl}ethyl}-1H-pyridin-2-one (78 mg) according to Example 220-(11).
1H-NMR (400 MHz, DMSO-d₆)
δ(ppm): 0.37-0.47 (m, 2H), 0.55-0.67 (m, 2H), 1.28-1.42 (m, 1H), 1.48-2.04 (m, 7H), 2.77 (s, 6H), 2.88-3.06 (m, 4H), 3.28-3.39 (m, 2H), 3.48-3.61 (m, 2H), 4.08 (d, J=7.2 Hz, 2H), 4.29-4.39 (m, 2H), 4.46 (s, 2H), 6.31 (dd, J=6. 4, 6. 8 Hz, 1H) , 6. 44 (d, J=8. 8 Hz, 1H) , 7.25 (d, J=8.8 Hz, 1H), 7.48 (dd, J=6.8, 8.8 Hz, 1H), 7.78 (d, J=6.4 Hz, 1H) , 7.98 (d, J=8.8 Hz, 1H), 10.73 (bs, 2H).
ESI-MS
m/z: 240 [M+2H]²⁺, 479 [M+H]⁺.

### Example 236

### 2-{4-{2-[6-Cyclopropylmethoxy-7-(N,N-dimethylaminomethyl)benz[d]isoxazol-3-yl]ethyl}piperidin-1-ylmethyl}cyclopent-1-enecarbonitrile dihydrochloride

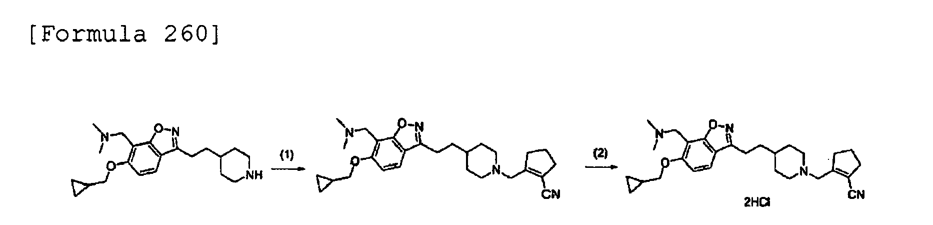

### (1) 2-{4-{2-[6-Cyclopropylmethoxy-7-(N,N-dimethylaminomethyl)benz[d]isoxazol-3-yl]ethyl}piperidin-1-ylmethyl}cyclopent-1-enecarbonitrile

The title compound (88 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (100 mg) and 2-formylcyclopent-1-enecarbonitrile [CAS Registry No. 261177-50-8] (113 mg) according to Example 223-(1).
1H-NMR (400 MHz, CDCl₃) δ(ppm): 0.33-0.42 (m, 2H), 0.60-0.68 (m, 2H), 1.23-1.41 (m, 4H), 1.70-1.82 (m, 4H), 1.91-2.04 (m, 4H), 2.34 (s, 6H), 2.48-2.67 (m, 4H), 2.72-2.82 (m, 2H), 2.88-2.98
(m, 2H), 3.21 (s, 2H), 3. 83 (s, 2H), 3. 91-3.98 (m, 2H), 6. 90 (d, J=8.8 Hz, 1H) , 7.45 (d, J=8. 8 Hz, 1H).

### (2) 2-{4-{2-[6-Cyclopropylmethoxy-7-(N,N-dimethylaminomethyl)benz[d]isoxazol-3-yl]ethyl}piperidin-1-ylmethyl}cyclopent-1-enecarbonitrile dihydrochloride

The title compound (95 mg) was obtained by synthesis from 2-{4-{2-[6-cyclopropylmethoxy-7-(N,N-dimethylaminomethyl)benz[d]isoxazol-3-yl]ethyl}piperidin-1-ylmethyl}cyclopent-1-enecarbonitrile (88 mg) according to Example 220-(11).
1H-NMR (400 MHz, DMSO-d₆)
δ(ppm): 0.37-0.47 (m, 2H), 0.57-0.67 (m, 2H), 1.28-1.42 (m, 1H), 1.53-2.04 (m, 9H), 2.58-2.70 (m, 2H), 2.72-2. 86 (m, 2H), 2.78 (s, 6H), 2.90-3.08 (m, 4H), 3.30-3.56 (m, 2H), 3.93 (s, 2H), 4.08 (d, J=6.8 Hz, 2H), 4.46 (s, 2H), 7.25 (d, J=8.8 Hz, 1H) , 7.97 (d, J=8.8 Hz, 1H), 10.65 (bs, 1H), 11.19 (bs, 1H).
ESI-MS
m/z: 232 [M+2H] ²⁺, 463 [M+H]⁺.

### Example 237

### 5-{4-{2-[6-Cyclopropylmethoxy-7-(N,N-dimethylaminomethyl)benz[d]isoxazol-3-yl]ethyl}piperidin-1-ylmethyl}pyridine-2-carbonitrile dihydrochloride

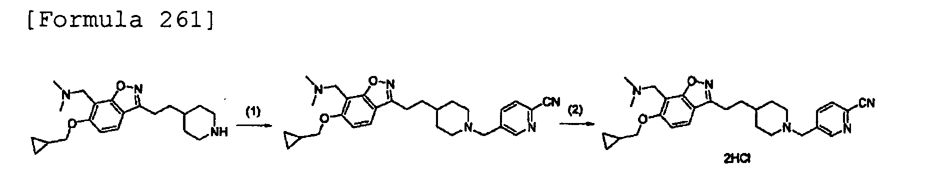

### (1) 5-{4-{2-[6-Cyclopropylmethoxy-7-(N,N-dimethylaminomethyl)benz[d]isoxazol-3-yl]ethyl}piperidin-1-ylmethyl}pyridine-2-carbonitrile

The title compound (81 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (100 mg) and 5-formylpyridine-2-carbonitrile [CAS Registry No. 70416-53-4] (74 mg) according to Example 223-(1).
1H-NMR (400 MHz, CDCl₃)
δ(ppm): 0.32-0.42 (m, 2H), 0.59-0.69 (m, 2H), 1.23-1.45 (m, 4H), 1.71-1.82 (m, 4H), 1.95-2.06 (m, 2H), 2.33 (s, 6H), 2.75-2.85 (m, 2H), 2.89-2.98 (m, 2H), 3.54 (s, 2H), 3. 81 (s, 2H), 3. 90-3. 97 (m, 2H), 6. 89 (d, J=8.8 Hz, 1H) , 7.42 (d, J=8.8 Hz, 1H) , 7.64 (d, J=7.6 Hz, 1H) , 7.81 (d, J=7.6 Hz, 1H) , 8.63 (s, 1H) .

### (2) 5-{4-{2-[6-Cyclopropylmethoxy-7-(N,N-dimethylaminomethyl)benz[d]isoxazol-3-yl]ethyl}piperidin-1-ylmethyl}pyridine-2-carbonitrile dihydrochloride

The title compound (81 mg) was obtained by synthesis from 5-{4-{2-[6-cyclopropylmethoxy-7-(N,N-dimethylaminomethyl)benz[d]isoxazol-3-yl]ethyl}piperidin-1-ylmethyl}pyridine-2-carbonitrile (81 mg) according to Example 220-(11).
1H-NMR (400 MHz, DMSO-d₆)
δ (ppm) : 0.33-0.49 (m, 2H), 0.54-0.69 (m, 2H), 1.27-1.42 (m, 1H), 1.50-2.00 (m, 7H), 2.79 (s, 6H), 2.81-3.06 (m, 4H), 3.22-3.45 (m, 2H), 4.07 (d, J=6.8 Hz, 2H), 4.42 (s, 2H), 4 . 47 (s, 2H), 7.25 (d, J=8. 8 Hz, 1H) , 7. 96 (d, J=8.8 Hz, 1H), 8.17 (d, J=8.0 Hz, 1H), 8.40 (d, J=8.0 Hz, 1H), 8.97 (s, 1H), 10.41 (bs, 1H), 11.41 (bs, 1H).
ESI-MS
m/z: 474 [M+H]⁺.

### Example 238

### 5-{4-{2-[6-Cyclopropylmethoxy-7-(N,N-dimethylaminomethyl)benz[d]isoxazol-3-yl]ethyl}piperidin-1-ylmethyl}furan-2-carbonitrile dihydrochloride

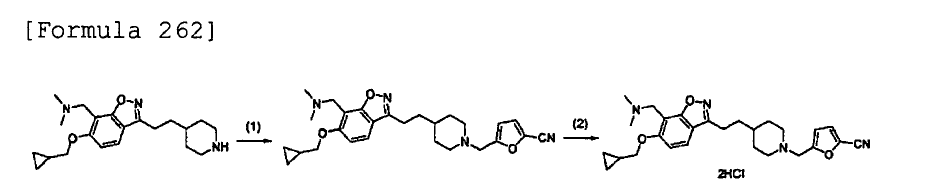

### (1) 5-{4-{2-[6-Cyclopropylmethoxy-7-(N,N-dimethylaminomethyl)benz[d]isoxazol-3-yl]ethyl}piperidin-1-ylmethyl}furan-2-carbonitrile

N,N-Dimethyl{6-cyclopropylmethoxy-3-[2-(piperidin-4-yl)ethyl]benz[d]isoxazol-7-yl}methylamine obtained in Preparation Example 3-(3) (100 mg) and 5-formylfuran-2-carbonitrile [CAS Registry No. 42061-89-2] (85 mg) were dissolved in methylene chloride (3 mL). Sodium triacetoxyborohydride (89 mg) was added to the solution, and the mixture was stirred at room temperature for 15.5 hours. The reaction mixture was made alkaline with a saturated sodium bicarbonate aqueous solution and sodium carbonate, followed by extraction with chloroform. The organic layer was washed with a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (84 mg).
1H-NMR (400 MHz, CDCl₃)
δ(ppm): 0.33-0.42 (m, 2H), 0.59-0.69 (m, 2H), 1.25-1.42 (m, 4H), 1.70-1.84 (m, 4H), 1.95-2.08 (m, 2H), 2.34 (s, 6H), 2.82-2.98 (m, 4H), 3.56 (s, 2H), 3.83 (s, 2H), 3.90-3.97 (m, 2H), 6. 32 (d, J=3.6 Hz, 1H), 6. 90 (d, J=8.8 Hz, 1H), 7.04 (d, J=3.6 Hz, 1H), 7.44 (d, J=8.8 Hz, 1H) .

### (2) 5-{4-{2-[6-Cyclopropylmethoxy-7-(N,N-dimethylaminomethyl)benz[d]isoxazol-3-yl]ethyl}piperidin-1-ylmethyl}furan-2-carbonitrile dihydrochloride

The title compound (88 mg) was obtained by synthesis from 5-{4-{2-[6-cyclopropylmethoxy-7-(N,N-dimethylaminomethyl)benz[d]isoxazol-3-yl]ethyl}piperidin-1-ylmethyl}furan-2-carbonitrile (84 mg) according to Example 220-(11).
1H-NMR (400 MHz, DMSO-d₆)
δ(ppm): 0.35-0.49 (m, 2H), 0.54-0.68 (m, 2H), 1.28-1.42 (m, 1H), 1.48-2.05 (m, 7H), 2.78 (s, 6H), 2.82-3.06 (m, 4H), 3.27-3.46 (m, 2H), 4.08 (d, J=7.2 Hz, 2H), 4.45 (s, 2H), 4.46 (s, 2H), 7.04 (d, J=3.2 Hz, 1H) , 7.25 (d, J=8.8 Hz, 1H), 7.68 (d, J=3.2 Hz, 1H), 7.96 (d, J=8.8 Hz, 1H), 10.66 (bs, 1H), 11.41 (bs, 1H).
ESI-MS
m/z: 232 [M+2H]²⁺, 463 [M+H]⁺

### Example 239

### 1-{2-{4-{2-[6-Cyclopropylmethoxy-7-(N,N-dimethylaminomethyl)benz[d]isoxazol-3-yl]ethyl}piperidin-1-yl}ethyl}piperidin-2-one dihydrochloride

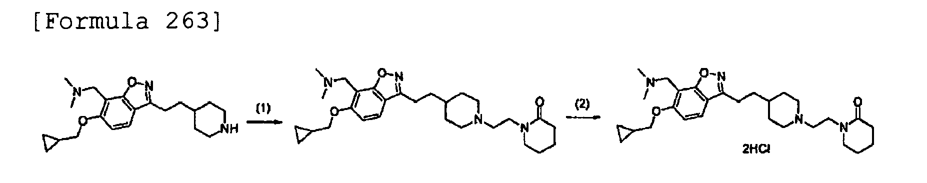

### (1) 1-{2-{4-{2-[6-Cyclopropylmethoxy-7-(N,N-dimethylaminomethyl)benz[d]isoxazol-3-yl]ethyl}piperidin-1-yl}ethyl}piperidin-2-one

The title compound (52 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (100 mg) and (2-oxo-piperidin-1-yl)acetaldehyde [CAS No. 376581-12-3] (127 mg) according to Example 238-(1).
1H-NMR (400 MHz, CDCl₃)
δ(ppm): 0.33-0.42 (m, 2H), 0.59-0.69 (m, 2H), 1.18-1.42 (m, 4H), 1.68-1.86 (m, 8H), 1.93-2.06 (m, 2H), 2.31-2.41 (m, 2H), 2.34 (s, 6H), 2.49 (t, J=7.6 Hz, 2H), 2.87-3.00 (m, 4H), 3.28-3.38 (m, 2H), 3.48 (t, J=7.6 Hz, 2H), 3.82 (s, 2H), 3. 91-3. 98 (m, 2H), 6. 90 (d, J=8.4 Hz, 1H), 7.45 (d, J=8.4 Hz, 1H).

### (2) 1-{2-{4-{2-[6-Cyclopropylmethoxy-7-(N,N-dimethylaminomethyl)benz[d]isoxazol-3-yl]ethyl}piperidin-1-yl}ethyl}piperidin-2-one dihydrochloride

The title compound (41 mg) was obtained by synthesis from 1-{2-{4-{2-[6-cyclopropylmethoxy-7-(N,N-dimethylaminomethyl)benz[d]isoxazol-3-yl]ethyl}piperidin-1-yl}ethyl}piperidin-2-one (52 mg) according to Example 220-(11).
1H-NMR (400 MHz, DMSO-d₆)
δ(ppm): 0.37-0.46 (m, 2H), 0.57-0.67 (m, 2H), 1.28-1.41 (m, 1H), 1.45-2.02 (m, 11H), 2.18-2.29 (m, 2H), 2.80 (s, 6H), 2.80-3.07 (m, 4H), 3.11-3.71 (m, 8H), 4.08 (d, J=6.8 Hz, 2H), 4.48 (s, 2H), 7.26 (d, J=8.8 Hz, 1H), 7.98 (d, J=8.8 Hz, 1H) , 10.00 (bs, 1H) , 10.18 (bs, 1H) .
ESI-MS
m/z: 242 [M+2H]²⁺, 483 [M+H]⁺.

### Example 240

### N,N-Dimethyl{3-{2-[1-(3-chlorobenzyl)piperidin-4-yl]ethyl}-6-cyclopropylmethoxybenz[d]isoxazol-7-yl}methylamine dihydrochloride

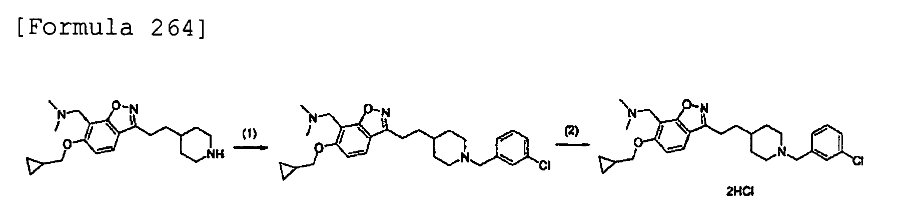

### (1) N,N-Dimethyl{3-{2-[1-(3-chlorobenzyl)piperidin-4-yl]ethyl}-6-cyclopropylmethoxybenz[d]isoxazol-7-yl}methylamine

The title compound (77 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (80 mg) and 3-chlorobenzaldehyde (63 mg) according to Example 223-(1) .
1H-NMR (400 MHz, CDCl₃)
δ(ppm): 0.33-0.42 (m, 2H), 0.60-0.70 (m, 2H), 1.24-1.43
(m, 4H), 1.70-1.84 (m, 4H), 1.88-2.01 (m, 2H), 2.34 (s, 6H), 2.80-2.98 (m, 4H), 3.45 (s, 2H), 3.83 (s, 2H), 3.91-3.97 (m, 2H), 6.90 (d, J=8.8 Hz, 1H), 7.16-7.25 (m, 3H), 7.33 (s, 1H) , 7.45 (d, J=8.8 Hz, 1H) .

### (2) N,N-Dimethyl{3-{2-[1-(3-chlorobenzyl)piperidin-4-yl]ethyl}-6-cyclopropylmethoxybenz[d]isoxazol-7-yl}methylamine dihydrochloride

The title compound (78 mg) was obtained by synthesis from N,N-dimethyl{3-{2-[1-(3-chlorobenzyl)piperidin-4-yl]ethyl}-6-cyclopropylmethoxybenz[d]isoxazol-7-yl}methylamine (77 mg) according to Example 220-(11).
1H-NMR (400 MHz, DMSO-d₆)
δ(ppm): 0.36-0.46 (m, 2H), 0.54-0.67 (m, 2H), 1.28-1.42 (m, 1H), 1.49-1.81 (m, 5H), 1.83-1.97 (m, 2H), 2.78 (s, 6H), 2.80-3.06 (m, 4H), 3.23-3.36 (m, 2H), 4.07 (d, J=6.8 Hz, 2H), 4.26 (s, 2H), 4.46 (s, 2H), 7.24 (d, J=8. 8 Hz, 1H), 7.48 (dd, J=8.0, 8.0 Hz, 1H) , 7.52 (d, J=8.0 Hz, 1H) , 7.62 (d, J=8.0 Hz, 1H), 7.80 (s, 1H) , 7.96 (d, J=8.8 Hz, 1H) , 10.64 (bs, 1H), 11.14 (bs, 1H) .
ESI-MS
m/z: 241 [M+2H]²⁺, 482 [M+H]⁺.

### Example 241

### N,N-Dimethyl{6-benzyloxy-3-[2-(1-benzylpiperidin-4-yl)ethyl]benz[d]isoxazol-7-yl}methylamine dihydrochloride

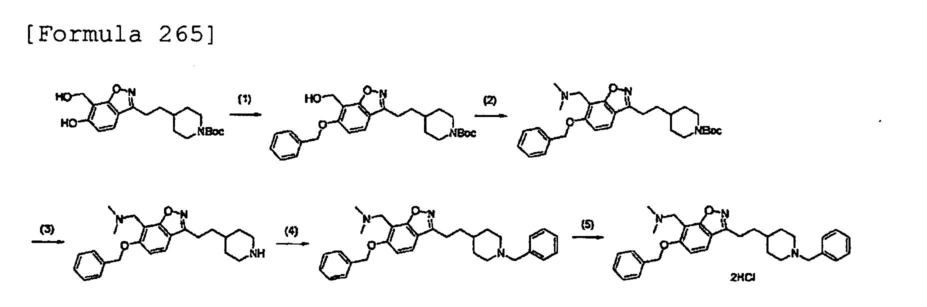

### (1) tert-Butyl 4-[2-(6-benzyloxy-7-hydroxymethylbenz[d]isoxazol-3-yl)ethyl]piperidine-1-carboxylate

tert-Butyl 4-[2-(6-hydroxy-7-hydroxymethylbenz[d]isoxazol-3-yl)ethyl]piperidine-1-carboxylate obtained in Preparation Example 2-(6) (220 mg) was dissolved in N,N-dimethylformamide (3 mL). Potassium carbonate (121 mg) and benzyl bromide (83 µL) were sequentially added to the solution at room temperature. The mixture was stirred at 70°C for 1.5 hours. The reaction mixture was cooled to room temperature and then water was added, followed by extraction with ethyl acetate. The organic layer was washed with a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (200 mg) .
¹H-NMR (400 MHz, CDCl₃)
δ (ppm): 1.08-1.22 (m, 2H), 1.42-1.56 (m, 1H), 1.46 (s, 9H), 1.68-1.82 (m, 4H), 2.37 (t, J=6.8 Hz, 1H), 2.59-2.76 (m, 2H), 2.92-3.02 (m, 2H), 3.98-4.21 (m, 2H), 5.05 (d, J=6.8 Hz, 2H), 5.24 (s, 2H), 7.02 (d, J=8.8 Hz, 1H) , 7.32-7.47 (m, 5H), 7.48 (d, J=8.8 Hz, 1H) .

### (2) tert-Butyl 4-{2-[6-benzyloxy-7-(N,N-dimethylaminomethyl)benz[d]isoxazol-3-yl]ethyl}piperidine-1-carboxylate

tert-Butyl 4-[2-(6-benzyloxy-7-hydroxymethylbenz[d]isoxazol-3-yl)ethyl]piperidine-1-carboxylate (200 mg) and triethylamine (180 µL) were dissolved in tetrahydrofuran (4 mL). Methanesulfonyl chloride (50 µL) was added to the solution under ice-cooling, and the mixture was stirred for 20 minutes. Dimethylamine (2 M solution in tetrahydrofuran, 2.2 mL) and sodium iodide (6 mg) were added to the reaction mixture under ice-cooling. The reaction mixture was stirred at room temperature for five hours. A saturated sodium chloride solution was added to the reaction mixture, followed by extraction with chloroform. The organic layer was washed with a saturated sodium chloride solution and dried over magnesium sulfate. The drying agent was removed by filtration and then the solvent was evaporated under reduced pressure. The residue was purified by NH silica gel column chromatography (heptane-ethyl acetate) to obtain the title compound (206 mg).
¹H-NMR (400 MHz, CDCl₃)
δ (ppm) : 1.08-1.23 (m, 2H), 1.42-1.60 (m, 1H), 1.46 (s, 9H), 1.70-1.83 (m, 4H), 2.33 (s, 6H), 2.60-2.77 (m, 2H), 2.92-3.01 (m, 2H), 3.83 (s, 2H), 4.00-4.20 (m, 2H), 5.22 (s, 2H), 7.00 (d, J=8.8 Hz, 1H), 7.30-7.50 (m, 5H), 7.45 (d, J=8.8 Hz, 1H) .

### (3) (4) N,N-Dimethyl {6-benzyloxy-3-[2-(1-benzylpiperidin-4-yl)ethyl]benz[d]isoxazol-7-yl}methylamine

The title compound (55 mg) was obtained by synthesis from tert-butyl 4-{2-[6-benzyloxy-7-(N,N-dimethylaminomethyl)benz[d]isoxazol-3-yl]ethyl}piperidine-1-carboxylate (206 mg) according to Example 221-(6)(7).
1H-NMR (400 MHz, CDCl₃)
δ (ppm) : 1.24-1.41 (m, 3H), 1.70-1.81 (m, 4H), 1.88-1.99 (m, 2H), 2.33 (s, 6H), 2.84-2.98 (m, 4H), 3.48 (s, 2H), 3.83 (s, 2H), 5.21 (s, 2H), 6.99 (d, J=8.8 Hz, 1H) , 7.21-7.49 (m, 10H), 7.45 (d, J=8.8 Hz, 1H).

### (5) N,N-Dimethyl{6-benzyloxy-3-[2-(1-benzylpiperidin-4-yl)ethyl]benz[d]isoxazol-7-yl}methylamine dihydrochloride

The title compound (57 mg) was obtained by synthesis from N,N-dimethyl{6-benzyloxy-3-[2-(1-benzylpiperidin-4-yl)ethyl]benz[d]isoxazol-7-yl}methylamine (55 mg) according to Example 220-(11). ¹H-NMR (400 MHz, DMSO-d₆)
δ(ppm): 1.48-1.81 (m, 5H), 1.83-1.97 (m, 2H), 2.77 (s, 6H), 2.78-2.93 (m, 2H), 2.95-3.04 (m, 2H), 3.22-3.35 (m, 2H), 4.24 (s, 2H), 4. 50 (s, 2H), 5.37 (s, 2H), 7.32-7.50 (m, 6H), 7.36 (d, J=8.8 Hz, 1H), 7.64-7.69 (m, 4H), 7.98 (d, J=8.8 Hz, 1H), 10.45 (bs, 1H) , 10.88 (bs, 1H) .
ESI-MS
m/z: 242 [M+2H]²⁺, 484 [M+H]⁺.

### Example 242

### N,N-Dimethyl{6-benzyloxy-3-{2-[1-(1,3-dioxolan-2-ylmethyl)piperidin-4-yl]ethyl}benz[d]isoxazol-7-yl}methylamine fumarate

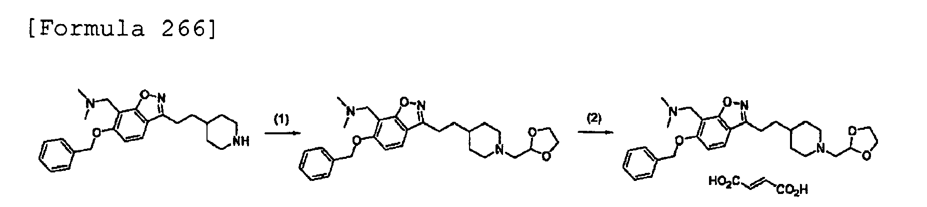

### (1) N,N-Dimethyl{6-benzyloxy-3-{2-[1-(1,3-dioxolan-2-ylmethyl)piperidin-4-yl]ethyl}benz[d]isoxazol-7-yl}methylamine

The title compound (52 mg) was obtained by synthesis from N,N-dimethyl{6-benzyloxy-3-[2-(piperidin-4-yl)ethyl]benz[d]isoxazol-7-yl}methylamine obtained in Example 241-(3) (90 mg) and 2-bromomethyl-1,3-dioxolane (59 µL) according to Example 227-(1).
1H-NMR (400 MHz, CDCl₃)
δ(ppm): 1.30-1.44 (m, 3H), 1.70-1.81 (m, 4H), 2.00-2.11 (m, 2H), 2.33 (s, 6H), 2.56 (d, J=4.8 Hz, 2H), 2.90-3.04 (m, 4H), 3.83 (s, 2H), 3.83-4.00 (m, 4H), 5.00 (t, J=4.8 Hz, 1H), 5.22 (s, 2H), 7.00 (d, J=8.4 Hz, 1H), 7.30-7.48 (m, 5H), 7.45 (d, J=8.4 Hz, 1H).

### (2) N,N-Dimethyl{6-benzyloxy-3-{2-[1-(1,3-dioxolan-2-ylmethyl)piperidin-4-yl]ethyl}benz[d]isoxazol-7-yl}methylamine fumarate

N,N-Dimethyl{6-benzyloxy-3-{2-[1-(1,3-dioxolan-2-ylmethyl)piperidin-4-yl]ethyl}benz[d]isoxazol-7-yl}methylamine (50 mg) and fumaric acid (13.3 mg) were dissolved in methanol. The solvent was evaporated under reduced pressure. The residue was dried under reduced pressure to obtain the title compound (50 mg).
¹H-NMR (400 MHz, DMSO-d₆)
δ (ppm) : 1.13-1.32 (m, 3H), 1.61-1.74 (m, 4H), 1.94-2.08 (m, 2H), 2.21 (s, 6H), 2.47 (d, J=4.4 Hz, 2H), 2.88-2.99 (m, 4H), 3.70-3.91 (m, 6H), 4.88 (t, J=4.4 Hz, 1H) , 5.27 (s, 2H), 6.59 (s, 2H), 7.24 (d, J=8. 8 Hz, 1H), 7.31-7.37 (m, 1H), 7.38-7.45 (m, 2H), 7.47-7.53 (m, 2H), 7.76 (d, J=8.8 Hz, 1H).
ESI-MS
m/z: 240 [M+2H]²⁺, 480 [M+H]⁺.

### Example 243

### N,N-Dimethyl{3-[2-(1-benzylpiperidin-4-yl)ethyl]-6-phenethyloxybenz[d]isoxazol-7-yl}methylamine dihydrochloride

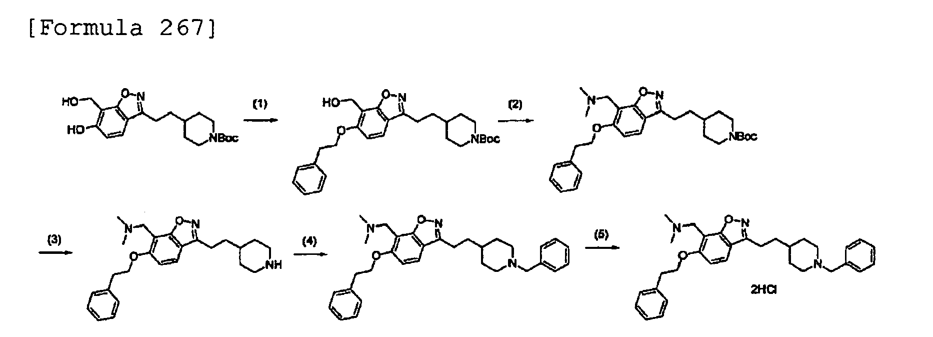

### (1)(2) tert-Butyl 4-[2-(7-dimethylaminomethyl-6-phenethyloxybenz[d]isoxazol-3-yl)ethyl]piperidine-1-carboxylate

The title compound (155 mg) was obtained by synthesis from tert-butyl 4-[2-(6-hydroxy-7-hydroxymethylbenz[d]isoxazol-3-yl)ethyl]piperidine-1-carboxylate obtained in Preparation Example 2-(6) (250 mg) and phenethyl bromide (155 µL) according to Example 241-(1) (2) .
1H-NMR (400 MHz, CDCl₃)
δ (ppm) : 1.07-1.22 (m, 2H), 1.43-1.57 (m, 1H) , 1.45 (s, 9H), 1.68-1.82 (m, 4H), 2.28 (s, 6H), 2.60-2.76 (m, 2H), 2.91-3.00 (m, 2H), 3.17 (t, J=6.8 Hz, 2H), 3.70 (s, 2H), 4.00-4.20 (m, 2H), 4.30 (t, J=6.8 Hz, 2H), 6.92 (d, J=8.4 Hz, 1H), 7.22-7.38 (m, 5H), 7.44 (d, J=8.4 Hz, 1H).

### (3) (4) N,N-Dimethyl {3-[2-(1-benzylpiperidin-4-yl)ethyl]-6-phenethyloxybenz[d]isoxazol-7-yl}methylamine

The title compound (32 mg) was obtained by synthesis from tert-butyl 4-[2-(7-dimethylaminomethyl-6-phenethyloxybenz[d]isoxazol-3-yl)ethyl]piperidine-1-carboxylate (155 mg) according to Example 221-(6)(7). 1H-NMR (400 MHz, CDCl₃)
δ(ppm): 1.23-1.41 (m, 3H), 1.68-1.81 (m, 4H), 1.88-1.99 (m, 2H), 2.28 (s, 6H), 2.83-2.98 (m, 4H), 3.17 (t, J=6.8 Hz, 2H), 3.48 (s, 2H), 3.69 (s, 2H), 4.30 (t, J=6.8 Hz, 2H), 6.91 (d, J=8.8 Hz, 1H), 7.21-7.37 (m, 10H), 7.44 (d, J=8.8 Hz, 1H) .

### (5) N,N-Dimethyl{3-[2-(1-benzylpiperidin-4-yl)ethyl]-6-phenethyloxybenz[d]isoxazol-7-yl}methylamine dihydrochloride

The title compound (31 mg) was obtained by synthesis from N,N-dimethyl{3-[2-(1-benzylpiperidin-4-yl)ethyl]-6-phenethyloxybenz[d]isoxazol-7-yl}methylamine (32 mg) according to Example 220-(11).
1H-NMR (400 MHz, DMSO-d₆) δ (ppm) : 1.47-1.80 (m, 5H), 1.82-1.98 (m, 2H), 2.65 (s, 6H), 2.78-2.92 (m, 2H), 2.94-3.05 (m, 2H), 3.18 (t, J=6.2 Hz, 2H), 3.22-3.35 (m, 2H), 4.23 (s, 2H), 4.34
(s, 2H), 4.43 (t, J=6.2 Hz, 2H), 7.21-7.49 (m, 8H), 7.30 (d, J=8.8 Hz, 1H), 7.59-7.70 (m, 2H), 7.96 (d, J=8.8 Hz, 1H) , 10.55 (bs, 1H), 10.93 (bs, 1H).
ESI-MS
m/z: 249 [M+2H]²⁺, 498 [M+H]⁺.

### Example 244

### N,N-Dimethyl{3-{2-[1-(1,3-dioxolan-2-ylmethyl)piperidin-4-yl]ethyl}-6-phenethyloxybenz[d]isoxazol-7-yl}methylamine fumarate

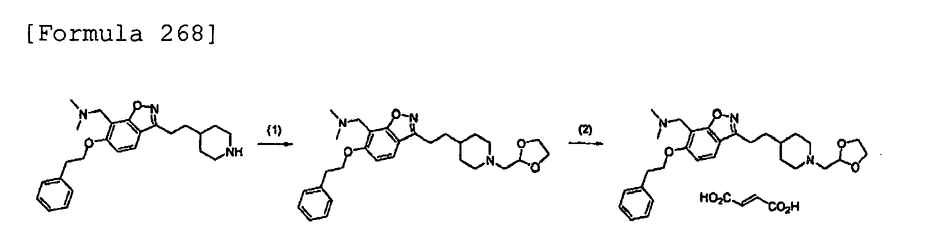

### (1) N,N-Dimethyl{3-{2-[1-(1,3-dioxolan-2-ylmethyl)piperidin-4-yl]ethyl}-6-phenethyloxybenz[d]isoxazol-7-yl}methylamine

The title compound (35 mg) was obtained by synthesis from N,N-dimethyl[6-phenethyloxy-3-(2-piperidin-4-ylethyl)benz[d]isoxazol-7-yl]methylamine obtained in Example 243-(3) (68 mg) according to Example 227-(1).
1H-NMR (400 MHz, CDCl₃)
δ (ppm) : 1.22-1.44 (m, 3H), 1.67-1.82 (m, 4H), 1.98-2.13 (m, 2H), 2.28 (s, 6H), 2.57 (d, J=4.4 Hz, 2H), 2.88-3.05 (m, 4H), 3.17 (t, J=6.8 Hz, 2H), 3.70 (s, 2H), 3.81-4.03 (m, 4H), 4.30 (t, J=6.8 Hz, 2H), 5.00 (t, J=4.4 Hz, 1H), 6.92 (d, J=8.8 Hz, 1H) , 7.22-7.37 (m, 5H), 7.44 (d, J=8.8 Hz, 1H).

### (2) N,N-Dimethyl{3-{2-[1-(1,3-dioxolan-2-ylmethyl)piperidin-4-yl]ethyl}-6-phenethyloxybenz[d]isoxazol-7-yl}methylamine fumarate

The title compound (31 mg) was obtained by synthesis from N,N-dimethyl{3-{2-[1-(1,3-dioxolan-2-ylmethyl)piperidin-4-yl]ethyl}-6-phenethyloxybenz[d]isoxazol-7-yl}methylamine (35 mg) according to Example 242-(2).
1H-NMR (400 MHz, DMSO-d₆)
δ(ppm): 1.12-1.32 (m, 3H), 1.60-1.74 (m, 4H), 1.95-2.07 (m, 2H), 2.14 (s, 6H), 2.48 (d, J=4.4 Hz, 2H), 2.87-2.98 (m, 4H), 3.08 (t, J=6.4 Hz, 2H), 3.66 (s, 2H), 3.70-3. 91 (m, 4H), 4.34 (t, J=6.4 Hz, 2H), 4 . 88 (t, J=4.4 Hz, 1H), 6.59 (s, 2H), 7.17 (d, J=8.8 Hz, 1H), 7.20-7.26 (m, 1H), 7.29-7.40 (m, 4H), 7.74 (d, J=8.8 Hz, 1H) .
ESI-MS
m/z: 247 [M+2H] ²⁺, 494 [M+H]⁺.

### Example 245

### N,N-Dimethyl{3-[2-(1-benzylpiperidin-4-yl)ethyl]-6-(pyridin-3-ylmethoxy)benz[d]isoxazol-7-yl}methylamine dihydrochloride

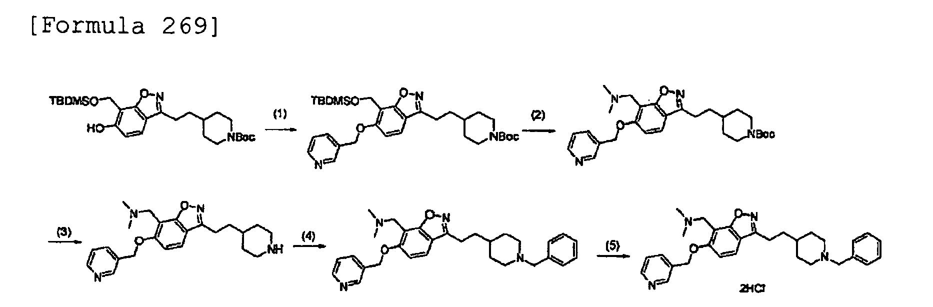

### (1) tert-Butyl 4-{2-[7-(tert-butyldimethylsilanyloxymethyl)-6-(pyridin-3-ylmethoxy)benz[d]isoxazol-3-yl]ethyl}piperidine-1-carboxylate

tert-Butyl 4-{2-[7-(tert-butyldimethylsilanyloxymethyl)-6-hydroxybenz[d]isoxazol-3-yl]ethyl}piperidine-1-carboxylate obtained in Example 79-(1) (200 mg), pyridine-3-methanol (52 µL) and triphenylphosphine (145 mg) were dissolved in tetrahydrofuran (2.7 mL). Diisopropyl azodicarboxylate (112 µL) was added dropwise to the solution under ice-cooling over five minutes. The reaction mixture was stirred at room temperature for 13 hours. The solvent was evaporated under reduced pressure. The residue was purified by NH silica gel column chromatography (heptane-ethyl acetate) and silica gel column chromatography (heptane-ethyl acetate) to obtain the title compound (237 mg). 1H-NMR (400 MHz, CDCl₃)
δ(ppm): 0.09 (s, 6H), 0.89 (s, 9H), 1.08-1.23 (m, 2H), 1.45-1.55 (m, 1H), 1.46 (s, 9H), 1.69-1.85 (m, 4H), 2.60-2.77 (m, 2H), 2.91-3.02 (m, 2H), 4.00-4.20 (m, 2H), 5.03 (s, 2H), 5.23 (s, 2H), 6.98 (d, J=8.4 Hz, 1H), 7.33 (dd, J=4.8, 8.0 Hz, 1H), 7.48 (d, J=8.4 Hz, 1H), 7.84 (d, J=8.0 Hz, 1H), 8.61 (d, J=4.8 Hz, 1H), 8.71 (s, 1H) .

### (2)(3)(4) N,N-Dimethyl{3-[2-(1-benzylpiperidin-4-yl)ethyl]-6-(pyridin-3-ylmethoxy)benz[d]isoxazol-7-yl}methylamine

The title compound (28 mg) was obtained by synthesis from tert-butyl 4-{2-[7-(tert-butyldimethylsilanyloxymethyl)-6-(pyridin-3-ylmethoxy)benz[d]isoxazol-3-yl]ethyl}piperidine-1-carboxylate (237 mg) according to Example 221-(5) (6) (7) .
1H-NMR (400 MHz, CDCl₃)
δ(ppm): 1.23-1.42 (m, 3H), 1.69-1.83 (m, 4H), 1.88-2.00 (m, 2H), 2.31 (s, 6H), 2.84-3.00 (m, 4H), 3.49 (s, 2H), 3.80 (s, 2H), 5.23 (s, 2H), 7.00 (d, J=8.8 Hz, 1H), 7.21-7.36 (m, 5H), 7.35 (dd, J=4.8, 8.0 Hz, 1H), 7.48 (d, J=8.8 Hz, 1H), 7.82 (d, J=8.0 Hz, 1H), 8.61 (d, J=4.8 Hz, 1H), 8.72 (s, 1H).

### (5) N,N-Dimethyl[3-[2-(1-benzylpiperidin-4-yl)ethyl]-6-(pyridin-3-ylmethoxy)benz[d]isoxazol-7-yl]methylamine dihydrochloride

The title compound (35 mg) was obtained by synthesis from N,N-dimethyl[3-[2-(1-benzylpiperidin-4-yl)ethyl]-6-(pyridin-3-ylmethoxy)benz[d]isoxazol-7-yl]methylamine (28 mg) according to Example 220-(11).
1H-NMR (400 MHz, DMSO-d₆)
δ(ppm): 1.47-2.00 (m, 7H), 2.76 (s, 6H), 2.80-3.07 (m, 4H), 3.20-3.40 (m, 2H), 4.24 (s, 2H), 4.51 (s, 2H), 5.44 (s, 2H), 7.39 (d, J=8.8 Hz, 1H), 7.41-7.51 (m, 3H), 7.56 (dd, J=5.2, 7.6 Hz, 1H), 7.57-7.68 (m, 2H), 8.01 (d, J=8.8 Hz, 1H) , 8.16 (d, J=7.6 Hz, 1H), 8.64 (d, J=5.2 Hz, 1H) , 8.88 (s, 1H) , 10.39 (bs, 1H) , 10.74 (bs, 1H).
ESI-MS
m/z: 243 [M+2H]²⁺, 485 [M+H]⁺.

### Example 246

### 5-{3-[2-(1-Benzylpiperidin-4-yl)ethyl]-7-(N,N-dimethylaminomethyl)benz[d]isoxazol-6-yloxymethyl}pyridine-2-carbonitrile dihydrochloride

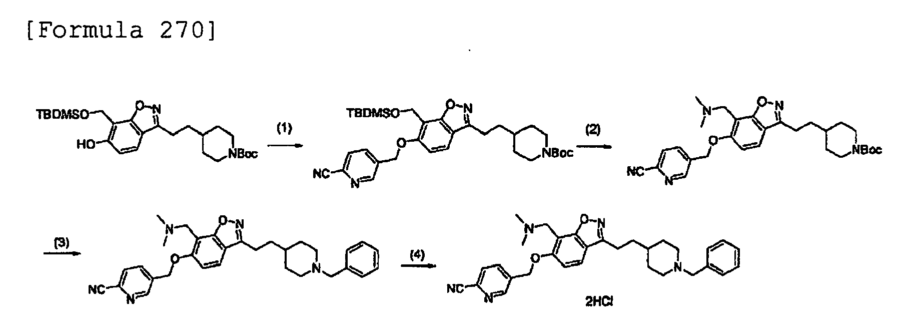

### (1) tert-Butyl 4-{2-[7-(tert-butyldimethylsilanyloxymethyl)-6-(6-cyanopyridin-3-ylmethoxy)benz[d]isoxazol-3-yl]ethyl}piperidine-1-carboxylate

The title compound (428 mg) was obtained by synthesis from tert-butyl 4-{2-[7-(tert-butyldimethylsilanyloxymethyl)-6-hydroxybenz[d]isoxazol-3-yl]ethyl}piperidine-1-carboxylate obtained in Example 79-(1) (300 mg) and 5-hydroxymethylpyridine-2-carbonitrile [CAS Registry No. 58553-48-3] (123 mg) according to Example 245-(1).
1H-NMR (400 MHz, CDCl₃)
δ(ppm): 0.10 (s, 6H), 0.89 (s, 9H), 1.08-1.26 (m, 2H), 1.43-1.58 (m, 1H), 1.46 (s, 9H), 1.68-1.85 (m, 4H), 2.59-2.78 (m, 2H), 2.92-3.03 (m, 2H), 4.00-4.20 (m, 2H), 5.04 (s, 2H), 5.31 (s, 2H), 6. 94 (d, J=8.4 Hz, 1H), 7.50 (d, J=8.4 Hz, 1H) , 7.74 (d, J=7.6 Hz, 1H) , 8.03 (d, J=7.6 Hz, 1H) , 8.82 (s, 1H).

### (2) tert-Butyl 4-{2-[6-(6-cyanopyridin-3-ylmethoxy)-7-(N,N-dimethylaminomethyl)benz[d]isoxazol-3-yl]ethyl}piperidine-1-carboxylate

The title compound (235 mg) was obtained by synthesis from tert-butyl 4-{2-[7-(tert-butyldimethylsilanyloxymethyl)-6-(6-cyanopyridin-3-ylmethoxy)benz[d]isoxazol-3-yl]ethyl}piperidine-1-carboxylate (532 mg) according to Example 221-(5).
1H-NMR (400 MHz, CDCl₃)
δ(ppm): 1.10-1.24 (m, 2H), 1.45-1.60 (m, 1H), 1.46 (s, 9H), 1.69-1.85 (m, 4H), 2.32 (s, 6H), 2.60-2.77 (m, 2H), 2.92-3.03 (m, 2H), 3.80 (s, 2H), 4.00-4.20 (m, 2H), 5. 31 (s, 2H), 6.97 (d, J=8.8 Hz, 1H), 7.51 (d, J=8.8 Hz, 1H), 7.76 (d, J=8.0 Hz, 1H), 8.01 (d, J=8.0 Hz, 1H), 8.85 (s, 1H).

### (3) 5-{3-[2-(1-Benzylpiperidin-4-yl)ethyl]-7-(N,N-dimethylaminomethyl)benz[d]isoxazol-6-yloxymethyl}pyridine-2-carbonitrile

tert-Butyl 4-{2-[6-(6-cyanopyridin-3-ylmethoxy)-7-(N,N-dimethylaminomethyl)benz[d]isoxazol-3-yl]ethyl}piperidine-1-carboxylate (200 mg) was dissolved in methylene chloride (4 mL). Hydrogen chloride (4 M solution in ethyl acetate, 1 mL) was added to the solution at room temperature, and the mixture was stirred for 2.5 hours. The supernatant was removed by decantation. The residue was made alkaline with chloroform and a saturated sodium bicarbonate solution. After extraction with chloroform, the organic layer was dried over magnesium sulfate. The drying agent was removed by filtration and the solvent was evaporated under reduced pressure. A crude product of 5-{7-(N,N-dimethylaminomethyl)-3-[2-(piperidin-4-yl)ethyl]benz[d]isoxazol-6-yloxymethyl}pyridine-2-carbonitrile (180 mg) was obtained as a residue.

The crude product of 5-{7-(N,N-dimethylaminomethyl)-3-[2-(piperidin-4-yl)ethyl]benz[d]isoxazol-6-yloxymethyl}pyridine-2-carbonitrile (80 mg) and benzaldehyde (27 µL) were dissolved in methylene chloride (1.9 mL). Acetic acid (16 µL) and sodium triacetoxyborohydride (61 mg) were sequentially added to the solution, and the reaction mixture was stirred at room temperature for 18.5 hours. The reaction mixture was made alkaline with a saturated sodium bicarbonate aqueous solution and sodium carbonate, followed by extraction with chloroform. The organic layer was washed with a saturated sodium chloride solution and dried over magnesium sulfate. The drying agent was removed by filtration and the solvent was evaporated under reduced pressure. The residue was purified by NH silica gel column chromatography (heptane-ethyl acetate) to obtain the title compound (47 mg).
1H-NMR (400 MHz, CDCl₃)
δ(ppm): 1.27-1.42 (m, 3H), 1.69-1.83 (m, 4H), 1.88-2.00 (m, 2H), 2.32 (s, 6H), 2.84-3.00 (m, 4H), 3.49 (s, 2H), 3.79 (s, 2H), 5.31 (s, 2H), 6.96 (d, J=8.4 Hz, 1H), 7.20-7.35 (m, 5H), 7.50 (d, J=8.4 Hz, 1H), 7.76 (d, J=8.0 Hz, 1H), 8.01 (d, J=8.0 Hz, 1H), 8.85 (s, 1H).

### (4) 5-{3-[2-(1-Benzylpiperidin-4-yl)ethyl]-7-(N,N-dimethylaminomethyl)benz[d]isoxazol-6-yloxymethyl}pyridine-2-carbonitrile dihydrochloride

The title compound (59 mg) was obtained by synthesis from 5-{3-[2-(1-benzylpiperidin-4-yl)ethyl]-7-(N,N-dimethylaminomethyl)benz[d]isoxazol-6-yloxymethyl}pyridine-2-carbonitrile (47 mg) according to Example 220-(11).
1H-NMR (400 MHz, DMSO-d₆) δ(ppm) : 1.47-1.81 (m, 5H), 1.83-1.98 (m, 2H), 2.77 (s, 6H), 2.78-2.93 (m, 2H), 2.95-3.07 (m, 2H), 3.21-3.38
(m, 2H), 4.24 (s, 2H), 4.54 (s, 2H), 5.52 (s, 2H), 7.36
(d, J=8.8 Hz, 1H), 7.41-7.53 (m, 3H), 7.57-7.69 (m, 2H), 8.02 (d, J=8.8 Hz, 1H), 8.13 (d, J=8.0 Hz, 1H), 8.31 (d, J=8.0 Hz, 1H), 9.00 (s, 1H), 10.37 (bs, 1H), 10.70 (bs, 1H).
ESI-MS
m/z: 255 [M+2H]²⁺, 510 [M+H]⁺.

### Example 247

### N,N-Dimethyl{3-[2-(1-benzylpiperidin-4-yl)ethyl]-6-(pyridin-4-ylmethoxy)benz[d]isoxazol-7-yl}methylamine dihydrochloride

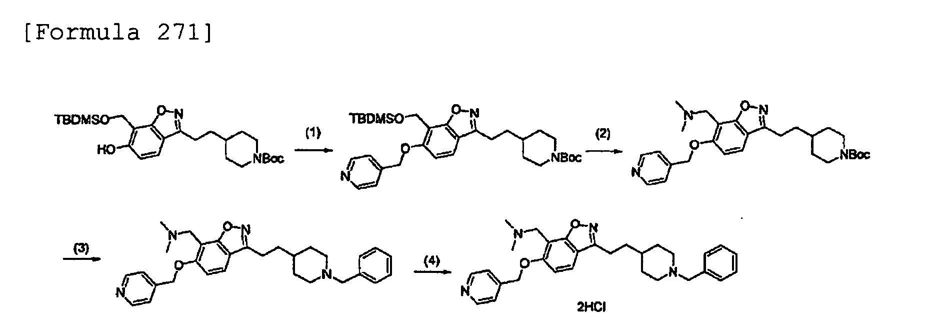

### (1)(2)(3) N,N-Dimethyl{3-[2-(1-benzylpiperidin-4-yl)ethyl]-6-(pyridin-4-ylmethoxy)benz[d]isoxazol-7-yl}methylamine

The title compound (39 mg) was obtained by synthesis from tert-butyl 4-{2-[7-(tert-butyldimethylsilanyloxymethyl)-6-hydroxybenz[d]isoxazol-3-yl]ethyl}piperidine-1-carboxylate obtained in Example 79-(1) (320 mg) and pyridine-4-methanol (107 µL) according to Example 245-(1) (2) (3) (4) .
1H-NMR (400 MHz, CDCl₃)
δ(ppm): 1.37-1.41 (m, 3H), 1.69-1.82 (m, 4H), 1.88-1.99 (m, 2H), 2.35 (s, 6H), 2.84-2.99 (m, 4H), 3.48 (s, 2H), 3.85 (s, 2H), 5.23 (s, 2H), 6.92 (d, J=8.8 Hz, 1H), 7.21-7.34 (m, 5H), 7.39 (d, J=6. Hz, 2H), 7.47 (d, J=8.8 Hz, 1H), 8.64 (d, J=6.4 Hz, 2H).

### (4) N,N-Dimethyl{3-[2-(1-benzylpiperidin-4-yl)ethyl]-6-(pyridin-4-ylmethoxy)benz[d]isoxazol-7-yl}methylamine dihydrochloride

The title compound (49 mg) was obtained by synthesis from N,N-dimethyl{3-[2-(1-benzylpiperidin-4-yl)ethyl]-6-(pyridin-4-ylmethoxy)benz[d]isoxazol-7-yl}methylamine (39 mg) according to Example 220-(11).
1H-NMR (400 MHz, DMSO-d₆)
5(ppm): 1.48-2.00 (m, 7H), 2.77-3.07 (m, 4H), 2.81 (s, 6H), 3.18-3.40 (m, 2H), 4.24 (s, 2H), 4.59 (s, 2H), 5.49 (s, 2H), 7.29 (d, J=8.8 Hz, 1H), 7.41-7.52 (m, 3H), 7.57-7.65 (m, 2H), 7.67 (d, J=5.6 Hz, 2H), 8.00 (d, J=8.8 Hz, 1H), 8.66 (d, J=5.6 Hz, 2H), 10.35 (bs, 1H), 10.68 (bs, 1H).
ESI-MS
m/z: 243 [M+2H]²⁺, 485 [M+H]⁺.

### Example 248

### N,N-Dimethyl{3-{2-[1-(1,3-dioxolan-2-ylmethyl)piperidin-4-yl]ethyl}-6-(pyridin-3-ylmethoxy)benz[d]isoxazol-7-yl}methylamine fumarate

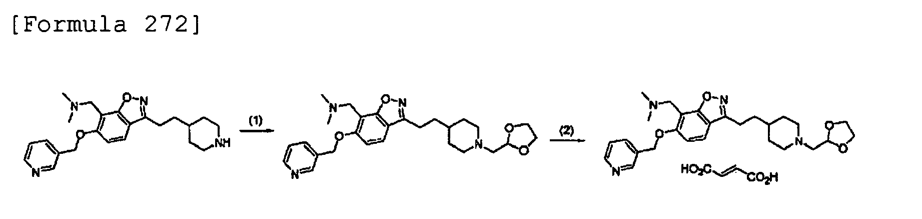

### (1) N,N-Dimethyl{3-{2-[1-(1,3-dioxolan-2-ylmethyl)piperidin-4-yl]ethyl}-6-(pyridin-3-ylmethoxy)benz[d]isoxazol-7-yl}methylamine

The title compound (18 mg) was obtained by synthesis from N,N-dimethyl{3-[2-(piperidin-4-yl)ethyl]-6-(pyridin-3-ylmethoxy)benz[d]isoxazol-7-yl}methylamine obtained in Example 245-(3) (60 mg) and 2-bromomethyl-1,3-dioxolane (39 µL) according to Example 227-(1).
1H-NMR (400 MHz, CDCl₃)
δ(ppm): 1.30-1.44 (m, 3H), 1.69-1.82 (m, 4H), 2.01-2.12 (m, 2H), 2.31 (s, 6H), 2.57 (d, J=4.4 Hz, 2H), 2.90-3.05 (m, 4H), 3.80 (s, 2H), 3.83-4.00 (m, 4H), 5.01 (t, J=4.4 Hz, 1H), 5.23 (s, 2H), 7.01 (d, J=8.8 Hz, 1H), 7.35 (dd, J=4.8, 7.6 Hz, 1H), 7.49 (d, J=8.8 Hz, 1H), 7.82 (d, J=7.6 Hz, 1H), 8.61 (d, J=4.8 Hz, 1H), 8.72 (s, 1H).

### (2) N,N-dimethyl{3-[2-(1-[1,3]dioxolan-2-ylmethylpiperidin-4-yl)ethyl]-6-(pyridin-3-ylmethoxy)benz[d]isoxazol-7-yl}methylamine fumarate

The title compound (17 mg) was obtained by synthesis from N,N-dimethyl{3-[2-(1-[1,3]dioxolan-2-ylmethylpiperidin-4-yl)ethyl]-6-(pyridin-3-ylmethoxy)benz[d]isoxazol-7-yl}methylamine (18 mg) according to Example 242-(2).
1H-NMR (400 MHz, DMSO-d₆) δ(ppm): 1.10-1.31 (m, 3H), 1.60-1.74 (m, 4H), 1.92-2.06 (m, 2H), 2.18 (s, 6H), 2.46 (d, J=4.4 Hz, 2H), 2.86-3.00 (m, 4H), 3.68-3.90 (m, 4H), 3.72 (s, 2H), 4.88 (t, J=4.4 Hz, 1H), 5.32 (s, 2H), 6.59 (s, 2H), 7.28 (d, J=8.8 Hz, 1H), 7.45 (dd, J=4.8, 8.0 Hz, 1H), 7.78 (d, J=8.8 Hz, 1H), 7.92 (d, J=8.0 Hz, 1H), 8.56 (d, J=4.8 Hz, 1H), 8.72 (s, 1H).
ESI-MS
m/z: 241 [M+2H]²⁺, 481 [M+H]⁺,

### Example 249

### N,N-Dimethyl{6-cyclopropylmethoxy-3-{2-[1-(5-fluoropyridin-2-ylmethyl)piperidin-4-yl]ethyl}benz[d]isoxazol-7-yl}methylamine dihydrochloride

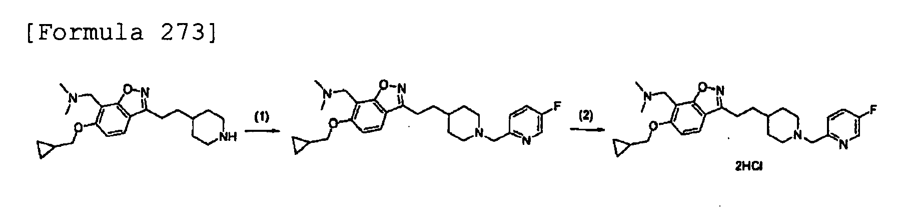

### (1) N,N-Dimethyl{6-cyclopropylmethoxy-3-{2-[1-(5-fluoropyridin-2-ylmethyl)piperidin-4-yl]ethyl}benz[d]isoxazol-7-yl}methylamine

The title compound (92 mg) was obtained by synthesis from N,N-dimethyl[6-cyclopropylmethoxy-3-(2-piperidin-4-ylethyl)benz[d]isoxazol-7-yl]methylamine obtained in Preparation Example 3-(3) (100 mg) and 5-fluoropyridine-2-carbaldehyde [CAS No. 31181-88-1] (70 mg) according to Example 223-(1).
1H-NMR (400 MHz, CDCl₃)
δ(ppm): 0.33-0.42 (m, 2H), 0.60-0.69 (m, 2H), 1.25-1.44 (m, 4H), 1.70-1.82 (m, 4H), 1.98-2.10 (m, 2H), 2.34 (s, 6H), 2.82-3.00 (m, 4H), 3.61 (s, 2H), 3.83 (s, 2H), 3.91-3.98 (m, 2H), 6.90 (d, J=8.8 Hz, 1H), 7.36 (ddd, J=2.6, 8.4, 8.4 Hz, 1H), 7.41 (dd, J=5.0, 8.4 Hz, 1H), 7.44 (d, J=8.8 Hz, 1H), 8.41 (d, J=2.6 Hz, 1H).

### (2) N,N-Dimethyl{6-cyclopropylmethoxy-3-{2-[1-(5-fluoropyridin-2-ylmethyl)piperidin-4-yl]ethyl}benz[d]isoxazol-7-yl}methylamine dihydrochloride

The title compound (98 mg) was obtained by synthesis from N,N-dimethyl{6-cyclopropylmethoxy-3-{2-[1-(5-fluoropyridin-2-ylmethyl)piperidin-4-yl]ethyl}benz[d]isoxazol-7-yl}methylamine (92 mg) according to Example 220-(11).
1H-NMR (400 MHz, DMSO-d₆)
δ(ppm): 0.36-0.45 (m, 2H), 0.57-0.67 (m, 2H), 1.28-1.40 (m, 1H), 1.45-1.80 (m, 5H), 1.83-1.98 (m, 2H), 2.81 (s, 6H), 2.91-3.07 (m, 4H), 3.33-3.48 (m, 2H), 4.07 (d, J=7.2 Hz, 2H), 4.43 (s, 2H), 4.49 (s, 2H), 7.26 (d, J=8.8 Hz, 1H), 7.70-7.78 (m, 1H), 7.85-7.94 (m, 1H), 7.97 (d, J=8.8 Hz, 1H), 8.70 (d, J=2.4 Hz, 1H), 10.09 (bs, 1H), 10.27 (bs, 1H).
ESI-MS
m/z: 234 [M+2H]²⁺, 467 [M+H]⁺.

### Example 250

### N,N-Dimethyl{6-cyclopropylmethoxy-3-{2-[1-(tetrahydrofuran-2-ylmethyl)piperidin-4-yl]ethyl}benz[d]isoxazol-7-yl}methylamine dihydrochloride

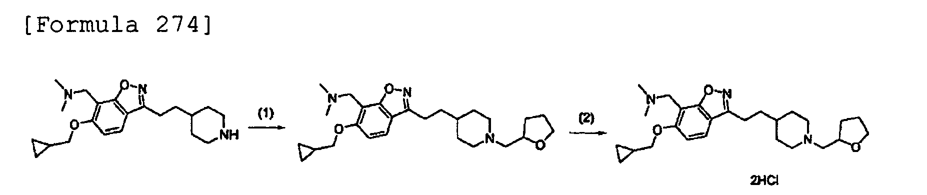

### (1) N,N-Dimethyl{6-cyclopropylmethoxy-3-{2-[1-(tetrahydrofuran-2-ylmethyl)piperidin-4-yl]ethyl}benz[d]isoxazol-7-yl}methylamine

The title compound (35 mg) was obtained by synthesis from N,N-dimethyl[6-cyclopropylmethoxy-3-(2-piperidin-4-ylethyl)benz[d]isoxazol-7-yl]methylamine obtained in Preparation Example 3-(3) (100 mg) and tetrahydrofurfuryl bromide (79 µL) according to Example 227-(1).
1H-NMR (400 MHz, CDCl₃) δ(ppm) : 0.33-0.42 (m, 2H), 0.60-0.70 (m, 2H), 1.22-1.53 (m, 5H), 1.68-2.05 (m, 9H), 2.32-2.53 (m, 2H), 2.34 (s, 6H), 2.88-3.06 (m, 4H), 3.70-3.78 (m, 1H), 3.82 (s, 2H), 3.83-4.08 (m, 4H), 6.90 (d, J=8.8 Hz, 1H), 7.44 (d, J=8.8 Hz, 1H).

### (2) N,N-Dimethyl{6-cyclopropylmethoxy-3-{2-[1-(tetrahydrofuran-2-ylmethyl)piperidin-4-yl]ethyl}benz[d]isoxazol-7-yl}methylamine dihydrochloride

The title compound (33 mg) was obtained by synthesis from N,N-dimethyl{6-cyclopropylmethoxy-3-{2-[1-(tetrahydrofuran-2-ylmethyl)piperidin-4-yl]ethyl}benz[d]isoxazol-7-yl}methylamine (35 mg) according to Example 220-(11).
1H-NMR (400 MHz, DMSO-d₆)
δ(ppm): 0.37-0.47 (m, 2H), 0.55-0.67 (m, 2H), 1.28-1.42 (m, 1H), 1.45-2.10 (m, 11H), 2.78 (s, 6H), 2.82-3.08 (m, 4H), 3.10-3.30 (m, 2H), 3.38-3.50 (m, 1H), 3.54-3.65 (m, 1H), 3.66-3.76 (m, 1H), 3.77-3.87 (m, 1H), 4.08 (d, J=7.2 Hz, 2H), 4.25-4.39 (m, 1H), 4.47 (s, 2H), 7.25 (d, J=8.8 Hz, 1H), 7.97 (d, J=8.8 Hz, 1H), 10.40 (bs, 1H), 10.64 (bs, 1H).
ESI-MS
m/z: 221 [M+2H]²⁺, 442 [M+H]⁺.

### Example 251

### 1-{2-{4-{2-[6-Cyclopropylmethoxy-7-(N,N-dimethylaminomethyl)benz[d]isoxazol-3-yl]ethyl}piperidin-1-yl}ethyl}pyrrolidin-2-one dihydrochloride

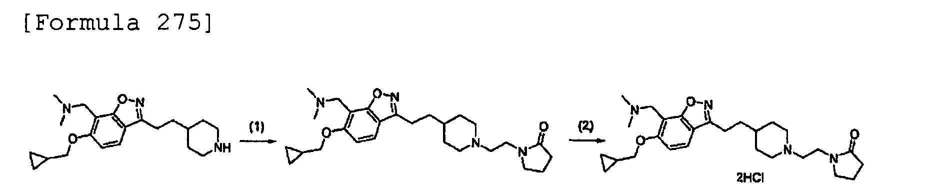

### (1) 1-{2-{4-{2-[6-Cyclopropylmethoxy-7-(N,N-dimethylaminomethyl)benz[d]isoxazol-3-yl]ethyl}piperidin-1-yl}ethyl}pyrrolidin-2-one

The title compound (20 mg) was obtained by synthesis from N,N-dimethyl{6-cyclopropylmethoxy-3-[2-(piperidin-4-yl)ethyl]benz[d]isoxazol-7-yl}methylamine obtained in Example 3-(3) (50 mg) and 2-(2-oxopyrrolidin-1-yl)ethyl 4-toluenesulfonate [CAS No. 136452-76-1] (44 mg) according to Example 227-(1).
1H-NMR (400 MHz, CDCl₃)
δ(ppm): 0.33-0.42 (m, 2H), 0.60-0.70 (m, 2H), 1.19-1.42 (m, 4H), 1.70-1.82 (m, 4H), 1.91-2.02 (m, 2H), 2.01 (quin, J=8.0 Hz, 2H), 2.34 (s, 6H), 2.37 (t, J=8.0 Hz, 2H), 2.47 (t, J=6.8 Hz, 2H), 2.87-2.99 (m, 4H), 3.41 (t, J=6.8 Hz, 2H), 3.44 (t, J=8.0 Hz, 2H), 3.82 (s, 2H), 6.90 (d, J=8.8 Hz, 1H), 7.45 (d, J=8.8 Hz, 1H).

### (2) 1-{2-{4-{2-[6-Cyclopropylmethoxy-7-(N,N-dimethylaminomethyl)benz[d]isoxazol-3-yl]ethyl}piperidin-1-yl}ethyl}pyrrolidin-2-one dihydrochloride

The title compound (20 mg) was obtained by synthesis from 1-{2-{4-{2-[6-cyclopropylmethoxy-7-(N,N-dimethylaminomethyl)benz[d]isoxazol-3-yl]ethyl}piperidin-1-yl}ethyl}pyrrolidin-2-one (20 mg) according to Example 220-(11).
1H-NMR (400 MHz, DMSO-d₆)
δ(ppm): 0.37-0.45 (m, 2H), 0.57-0.66 (m, 2H), 1.28-1.40 (m, 1H), 1.50-1.78 (m, 5H), 1.82-2.02 (m, 4H), 2.19-2.30 (m, 2H), 2.79 (s, 6H), 2.79-2.93 (m, 2H), 2.96-3.07 (m, 2H), 3,12-3.25 (m, 2H), 3.33-3.45 (m, 2H), 3.50-3.62 (m, 4H), 4.08(d, J=6.8 Hz, 2H), 4.47 (s, 2H), 7.26 (d, J=8.8 Hz, 1H), 7.98 (d, J=8.8 Hz, 1H), 10.37 (bs, 1H), 10.44 (bs, 1H).
ESI-MS
m/z: 235 [M+2H]²⁺, 469 [M+H]⁺.

### Example 252

### N,N-Dimethyl{6-cyclopropylmethoxy-3-{2-[1-(5-fluorothiophen-2-ylmethyl)piperidin-4-yl]ethyl}benz[d]isoxazol-7-yl}methylamine dihydrochloride

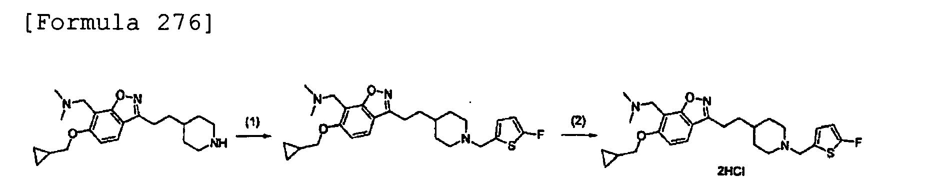

### (1) N,N-Dimethyl{6-cyclopropylmethoxy-3-{2-[1-(5-fluorothiophen-2-ylmethyl)piperidin-4-yl]ethyl}benz[d]isoxazol-7-yl}methylamine dihydrochloride

The title compound (26 mg) was obtained by synthesis from N,N-dimethyl{6-cyclopropylmethoxy-3-[2-(piperidin-4-yl)ethyl]benz[d]isoxazol-7-yl}methylamine obtained in Preparation Example 3-(3) (54 mg) and 5-fluorothiophene-2-carbaldehyde [CAS No. 29669-49-6] (40 mg) according to Example 223-(1).
1H-NMR (400 MHz, CDCl₃)
δ(ppm): 0.33-0.41 (m, 2H), 0.60-0.70 (m, 2H), 1.23-1.42 (m, 4H), 1.69-1.82 (m, 4H), 1.90-2.03 (m, 2H), 2.38 (bs, 6H), 2.86-2.99 (m, 4H), 3.54 (s, 2H), 3.89 (bs, 2H), 3.92-3.99 (m, 2H), 6.26 (dd, J=2.0, 4.0 Hz, 1H), 6.46 (dd, J=3.6, 4.0 Hz, 1H), 6.91 (d, J=8.8 Hz, 1H), 7.46 (d, J=8.8 Hz, 1H).

### (2) N,N-Dimethyl{6-cyclopropylmethoxy-3-{2-[1-(5-fluorothiophen-2-ylmethyl)piperidin-4-yl]ethyl}benz[d]isoxazol-7-yl}methylamine dihydrochloride

The title compound (22 mg) was obtained by synthesis from N,N-dimethyl{6-cyclopropylmethoxy-3-{2-[1-(5-fluorothiophen-2-ylmethyl)piperidin-4-yl]ethyl}benz[d]isoxazol-7-yl}methylamine (26 mg) according to Example 220-(11).
1H-NMR (400 MHz, DMSO-d₆)
δ(ppm): 0.35-0.46 (m, 2H), 0.53-0.67 (m, 2H), 1.26-1.40 (m, 1H), 1.55-2.04 (m, 7H), 2.70-3.07 (m, 4H), 2.78 (s, 6H), 3.25-3.47 (m, 2H), 4.07 (d, J=7.2 Hz, 2H), 4.37 (s, 2H), 4.46 (s, 2H), 6.75 (s, 1H), 7.08 (s, 1H), 7.24 (d, J=8.8 Hz, 1H), 7.95 (d, J=8.8 Hz, 1H), 10.37 (bs, 1H), 10.94 (bs, 1H).
ESI-MS
m/z: 472 [M+H]⁺.

### Test Example 1

### Acetylcholine esterase inhibitory activity

Esterase activity was measured according to the method of Ellman et al. (Biochem. Pharmacol., 1961, 7, 88-95) using a centrifugation supernatant of a male Wistar rat cerebral homogenate as an acetylcholine esterase source. First, a sample dissolved in dimethyl sulfoxide (DMSO) (10 mM) was dissolved in water to 30 µM, and eight serial dilutions were prepared at a common ratio of 3 with 30 µM as a highest concentration. Next, 18 times weight of a phosphate buffer [0.1 M phosphate buffer (0.1 M Na₂HPO₄, prepared from 0.1 M KH₂PO₄; pH 8.0) with 0.5% Triton X-100] was added to the rat cerebrum. The mixture was homogenized and centrifuged at 3000 rpm for five minutes. The supernatant was diluted 30-fold with a phosphate buffer (pH 8.0) and dispensed to a 96-well plate at 165 µL/well. 6 µL of the diluted sample was added, followed by preincubation at room temperature for about 10 minutes. Thereafter, 9 µL of a reaction mixture [10 mM 5,5'-dithiobis(2-nitrobenzoic acid) (DTNB)/0.1 M phosphate buffer (pH 7.0) with 6 µL of 0.15% NaHCO₃ + 90 mM acetylthiocholine iodide/1 µL of H₂O + 2 µL of 0.1 M phosphate buffer (pH 8.0)] was added and the absorbance (412 nm) of anions (yellow) of 5-thio-2-nitrobenzoic acid generated by reaction of the produced thiocholine with DTNB anions was measured at room temperature. The acetylcholine esterase activity was determined from the change in absorbance between two minutes and four minutes after the start of the reaction. The inhibition rate (%) of the test compound was determined based on the acetylcholine esterase activity when the compound was not added as 100% and the 50% inhibition concentration (IC50) was calculated.

### Test Example 2

### Serotonin transporter binding inhibitory activity

Affinity of the test substance to a serotonin transporter (SERT) was determined by a test of inhibition of binding of [³H]paroxetine selectively binding to SERT to a rat cerebral cortex membrane fraction by the test substance. Serotonin reuptake inhibitors are generally known to have SERT binding inhibitory activity. Based on the assumption that the intensity of SERT binding inhibitory activity of the test substance indicates the intensity of reuptake inhibitory activity, affinity of the test substance to SERT was determined and the intensity of the activity was estimated (according to the method described in Marcusson, J.; Eriksson, K; J. Neurochemistry, 1988, 51 (5), 1477).
The rat cerebral cortex was homogenized in ice-cold 0.32 M sucrose. The suspension was centrifuged at 1,000 × g for 10 minutes. The resulting supernatant was further centrifuged at 20,000 × g for 20 minutes. The resulting precipitate was suspended in 50 mM Tris-HCl (pH 7.4) containing 120 mM NaCl and 5 mM KCl (hereinafter called suspension A) and centrifuged at 20,000 × g for 20 minutes. The same operation was repeated once more. The finally obtained precipitate was suspended in a suspension A having a wet weight about 100 times that of the rat cerebral cortex to prepare a membrane fraction which was stored at -80°C until use.
An incubation mixture (0.5 mL) contained a solution obtained by further diluting the test substance dissolved in DMSO (dimethyl sulfoxide) to a desired concentration (control: only DMSO) 500-fold with purified water (25 µL); a [³H]paroxetine solution (final concentration: 0.05 nM) as a ligand (25 µL); and the membrane fraction prepared as described above (450 µL). The reaction was started by addition of the membrane fraction, followed by incubation at room temperature for 90 minutes. After the incubation, the mixture was filtered by suction through a GF/B filter using a cell harvester. The filter was washed with 50 mM Tris-HCl (pH 7.4) and then radioactivity of [³H]paroxetine binding to SERT was measured using a liquid scintillation counter. Binding detected in the presence of 1 or 10 µM fluoxetine was defined as nonspecific binding.

The results of the above Test Examples 1 and 2 are shown in Table 1.

**[Table 1]**

| Example No. | Acetylcholine esterase inhibition (IC50. nM) | Serotonin transporter binding inhibition (IC50. nM) |
|---|---|---|
| 22 | 4.1 | 1.6 |
| 39 | 0.4 | 13 |
| 51 | 3. 6 | 1.9 |
| 78 | 0.7 | 1.1 |
| 88 | 7. 7 | 5. 3 |
| 93 | 73 | 8.9 |
| 118 | 12 | 10 |
| 120 | 1.3 | 0.9 |
| 151 | 33 | 54 |
| 152 | 4. 9 | 10 |
| 153 | 0.4 | 0. 3 |
| 154 | 3.0 | 1.0 |
| 155 | 5. 0 | 5. 3 |
| 178 | 4. 1 | 62 |
| 210 | 2.4 | 12 |
| 213 | 3.0 | 19 |

### INDUSTRIAL APPLICABILITY

The compound (I) or salt thereof according to the present invention has excellent acetylcholine esterase inhibitory and serotonin reuptake inhibitory effects together, and is therefore useful for dementia, cognitive impairment, depression or a disease with depressive symptoms. In particular, the compound (I) or salt thereof according to the present invention can be a medicine useful as a therapeutic agent for Alzheimer's disease.

## Claims

1. A compound represented by the following general formula (I): 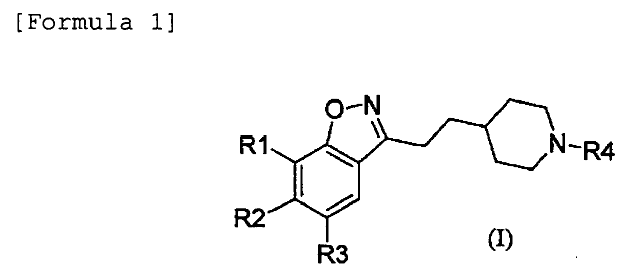 or a salt thereof,
wherein any one of R1, R2 and R3 is a group represented by the formula -(CH₂)m-NR11R12 (wherein m is 1 or 2; and R11 and R12 are the same or different and each represent a hydrogen atom or a C1-6 alkyl group, or R11 and R12, together with a nitrogen atom to which they are bonded, form a 4- or 5-membered cyclic group); the remaining two of R1, R2 and R3 are the same or different and each represent a group represented by the formula -(O)n-R21 [wherein n is 0 or 1; and R21 represents (1) a hydrogen atom, (2) a C1-6 alkyl group, (3) a C2-6 alkenyl group, (4) a C2-6 alkynyl group, (5) an amino group substituted with two C1-6 alkyl groups, (6) a C3-7 cycloalkyl group, (7) a C4-7 cycloalkenyl group, (8) a heterocycloalkyl group selected from the group consisting of a 1-pyrrolidinyl group, a piperidino group, a perhydro-2H-azepin-1-yl group and a morpholino group, (9) a 3,6-dihydro-2H-pyranyl group, (10) a C6-10 aryl group, (11) a heteroaryl group selected from the group consisting of a furyl group, a thienyl group, a thiazolyl group, an oxazolyl group, a pyridyl group and a pyridazinyl group or (12) a group represented by the formula - (CH₂)p-X-(CH₂)q-R22 (wherein X represents -O-, -NHCO- or -CONH-; R22 represents any substituent selected from Group A1 shown below, wherein the substituent may be substituted with 1 to 3 substituents which are the same or different and are selected from Group B1; and p and q are the same or different and each represent 0 to 2, provided that when X is a -O- group or a -NHCO- group and n is 1, p is 2), wherein R21 of (2) to (11) may be substituted with 1 to 3 substituents which are the same or different and are selected from Group A1 and Group A2, provided that when the substituents are selected from the Group A1, the substituents may each be substituted with 1 to 3 substituents which are the same or different and are selected from Group B1], provided that when the remaining two are R2 and R3, the R2 and R3, together with carbon atoms to which they are bonded, may form a benzene ring; and
R4 is (1) a C1-6 alkyl group, (2) a C2-6 alkenyl group, (3) a C2-6 alkynyl group, (4) a heteroaryl group selected from the group consisting of a pyridyl group, a pyridazinyl group, a pyrimidinyl group, a pyrazinyl group, a thiazolyl group, a furyl group, a thienyl group, an isoxazolyl group and an oxazolyl group or (5) a C6-10 aryl group, provided that when R4 is (1) a C1-6 alkyl group, (2) a C2-6 alkenyl group or (3) a C2-6 alkynyl group, the R4 may be substituted with 1 to 3 substituents which are the same or different and are selected from Group T1, provided that when the substituents are other than a formyl group and a hydroxyl group, the substituents may each be further substituted with 1 to 3 substituents which are the same or different and are selected from Group U1.
(Group A1)
<(1) a C3-7 cycloalkyl group, (2) a C6-10 aryl group, (3) a heteroaryl group selected from the group consisting of a pyridyl group, a pyridazinyl group, a pyrimidinyl group, a furyl group, a thiazolyl group and a thienyl group, (4) a 1,2-dihydro-2-oxopyridyl group, (5) a tetrahydrofuryl group and (6) a C3-7 cycloalkenyl group>
(Group A2)
<(1) a halogen atom, (2) a cyano group and (3) a C1-6 alkoxy group>
(Group B1)
<(1) a halogen atom, (2) a hydroxyl group, (3) a cyano group, (4) a C1-6 alkoxymethyl group, (5) a C1-6 alkyl group substituted with one hydroxyl group, (6) a C1-6 alkoxy group, (7) a C1-6 alkyl group and (8) a C2-6 alkynyl group which may be substituted with one C1-6 alkoxy group>
(Group T1)
<(1) a C3-7 cycloalkyl group, (2) a heterocycloalkyl group selected from the group consisting of a 1,3-dioxolanyl group, a 1,3-dioxanyl group, a 1,4-dioxanyl group, a tetrahydrofuryl group, a 3,4,5,6-tetrahydro-2H-pyranyl group, a 2-oxopyrrolidinyl group and a 2-oxopiperidyl group, (3) a C4-7 cycloalkenyl group, (4) a heterocycloalkenyl group selected from the group consisting of a 1,2-dihydro-2-oxopyrazinyl group, a 1,2-dihydro-2-oxopyridyl group and a 1,2-dihydro-2-oxopyrimidinyl group, (5) a C6-10 aryl group, (6) a heteroaryl group selected from the group consisting of a pyridyl group, a pyridazinyl group, a pyrimidinyl group, a pyrazinyl group, a pyrrolyl group, a pyrazolyl group, an imidazolyl group, a thiazolyl group, a furyl group, a thienyl group, an isoxazolyl group, an imidazo[1,2-a]pyridyl group, a pyrazolo[1,5-a]pyridyl group, a benzo[b]thienyl group and an indolyl group, (7) a formyl group, (8) a hydroxyl group and (9) a 4,5,6,7-tetrahydrobenzothienyl group>
(Group U1)
<(1) a halogen atom, (2) a hydroxyl group, (3) a cyano group, (4) a C1-6 alkyl group, (5) a C3-6 cycloalkyl group, (6) a C1-6 alkoxymethyl group, (7) a C1-6 alkyl group substituted with one hydroxyl group, (8) a C1-6 alkoxyimino group, (9) a C1-6 alkoxy group, (10) a methylenedioxy group, (11) a pyridyl group, (12) a C6-10 aryl group, (13) an N-(C1-6 alkyl)carbamoyl group, (14) a C2-6 alkynyl group, (15) a C2-6 alkenyl group, (16) a C1-6 alkylthio group and (17) an amino group which may be substituted with one or two C1-6 alkyl groups>

2. The compound or salt thereof according to claim 1, wherein any one of R1, R2 and R3 is a group represented by the formula -(CH₂)m-NR11R12 [wherein m is 1; and R11 and R12 are as defined in claim 1].

3. The compound or salt thereof according to claim 1 or 2, wherein R1 is a group represented by the formula -(CH₂)m-NR11R12 [wherein m is 1; and R11 and R12 are as defined in claim 1]; and R2 and R3 are the same or different and each represent a group represented by the formula -(O)n-R21 [wherein R21 and n are as defined in claim 1], or R2 and R3, together with the carbon atoms to which they are bonded, form a benzene ring.

4. The compound or salt thereof according to claim 1, wherein R1 is a group represented by the formula -(CH₂)m-NR11R12 [wherein m is 1; and R11 and R12 are as defined in claim 1]; R2 is a group represented by the formula -(O)n-R21 [wherein R21 and n are as defined in claim 1]; and R3 is a hydrogen atom.

5. The compound or salt thereof according to claim 4, wherein R1 is a group represented by the formula -(CH₂)m-NR11R12 [wherein m is 1; and R11 and R12 are the same or different and each represent (1) a hydrogen atom, (2) a methyl group or (3) an ethyl group].

6. The compound or salt thereof according to claim 4 or 5, wherein R2 is a group represented by the formula -(O)n-R21 [wherein n is 1; and R21 represents (1) a C1-6 alkyl group, (2) a C2-6 alkenyl group or (3) a C2-6 alkynyl group, wherein R21 may be substituted with 1 to 3 substituents which are the same or different and are selected from Group A1 and Group A2 according to claim 1, provided that when the substituents are selected from the Group A1, the substituents may each be substituted with 1 to 3 substituents which are the same or different and are selected from Group B1 according to claim 1].

7. The compound or salt thereof according to claim 4 or 5, wherein R2 is a group represented by the formula -(O)n-R21 [wherein n is 1; and R21 represents a C1-6 alkyl group, wherein R21 may be substituted with 1 to 3 substituents selected from Group A1 and Group A2 according to claim 1, provided that when the substituents are selected from the Group A1, the substituents may each be substituted with 1 to 3 substituents which are the same or different and are selected from Group B1 according to claim 1].

8. The compound or salt thereof according to claim 4 or 5, wherein R2 is a group represented by the formula -(O)n-R21 [wherein n is 1; and R21 is a group represented by the formula -(CH₂)t-R23 (wherein t is 1 to 2; and R23 represents (1) a C6-10 aryl group, (2) a heteroaryl group selected from the group consisting of a pyridyl group, a pyridazinyl group, a pyrimidinyl group, a furyl group, a thiazolyl group and a thienyl group, (3) a C3-7 cycloalkyl group or (4) a C3-7 cycloalkenyl group, wherein R23 may be substituted with 1 to 3 substituents which are the same or different and are selected from Group B1 according to claim 1)].

9. The compound or salt thereof according to claim 4 or 5, wherein R2 is a group represented by the formula -(O)n-R21 [wherein n is 1; and R21 is a group represented by the formula -(CH₂)t-R23 (wherein t is 1 or 2; and R23 represents (1) a C6-10 aryl group, (2) a heteroaryl group selected from the group consisting of a pyridyl group, a pyridazinyl group, a pyrimidinyl group, a furyl group, a thiazolyl group and a thienyl group or (3) a C3-7 cycloalkyl group, wherein R23 may be substituted with 1 to 3 substituents which are the same or different and are selected from Group B1 according to claim 1)].

10. The compound or salt thereof according to claim 4 or 5, wherein R2 is a group represented by the formula -(O)n-R21 [wherein n is 1; and R21 is a group represented by the formula -(CH₂)t-R23 (wherein t is 1 or 2; and R23 represents (1) a phenyl group, (2) a pyridyl group, (3) a furyl group, (4) a thienyl group, (5) a cyclopropyl group or (6) a cyclohexyl group, wherein R23 may be substituted with 1 to 3 substituents which are the same or different and are selected from Group B1 according to claim 1)].

11. The compound or salt thereof according to claim 4 or 5, wherein R2 is a group represented by the formula -(O)n-R21 [wherein n is 1; and R21 is a group represented by the formula -(CH₂)t-R23 (wherein t is 1 or 2; and R23 represents (1) a phenyl group or (2) a cyclopropyl group, wherein R23 may be substituted with 1 to 3 substituents which are the same or different and are selected from Group B1 according to claim 1)].

12. The compound or salt thereof according to claim 4 or 5, wherein R2 is a group represented by the formula -(O)n-R21 [wherein n is 0; and R21 represents (1) a C6-10 aryl group, (2) a heteroaryl group selected from the group consisting of a furyl group, a thienyl group, a thiazolyl group, an oxazolyl group, a pyridyl group and a pyridazinyl group, (3) a heterocycloalkyl group selected from the group consisting of a 1-pyrrolidinyl group, a piperidino group, a perhydro-2H-azepin-1-yl group and a morpholino group or (4) a 3,4,5,6-tetrahydro-2H-pyranyl group, which may be substituted with 1 to 3 cyano groups or/and halogen atoms].

13. The compound or salt thereof according to claim 4 or 5, wherein R2 is a group represented by the formula -(O)n-R21 [wherein n is 0; and R21 represents a phenyl group which may be substituted with 1 to 3 cyano groups or/and halogen atoms].

14. The compound or salt thereof according to claim 4 or 5, wherein R2 is a group represented by the formula -(O)n-R21 [wherein n is 0; and R21 represents (1) a pyridyl group, (2) a thienyl group or (3) a furyl group, which may be substituted with 1 to 3 cyano groups or/and halogen atoms].

15. The compound or salt thereof according to claim 4 or 5, wherein R2 is a group represented by the formula -(O)n-R21 [wherein n is 0; and R21 represents (1) a morpholino group or (2) a piperidino group].

16. The compound or salt thereof according to claim 4, 5, 6 or 12, wherein R4 represents (1) a C1-6 alkyl group, (2) a C2-6 alkenyl group or a (3) C2-6 alkynyl group, which is substituted with 1 to 3 substituents which are the same or different and are selected from Group T2 shown below, which substituents selected from Group T2 may be substituted with 1 to 3 substituents which are the same or different and are selected from Group U2 shown below:
(Group T2)
<(1) a C3-7 cycloalkyl group, (2) a heterocycloalkyl group selected from the group consisting of a 1,3-dioxolanyl group, a 1,3-dioxanyl group, a 1,4-dioxanyl group, a tetrahydrofuryl group, a 3,4,5,6-tetrahydro-2H-pyranyl group, a 2-oxopyrrolidinyl group and a 2-oxopiperidyl group, (3) a C4-7 cycloalkenyl group, (4) a heterocycloalkenyl group selected from the group consisting of a 1,2-dihydro-2-oxopyrazinyl group, a 1,2-dihydro-2-oxopyridyl group and a 1,2-dihydro-2-oxopyrimidinyl group, (5) a C6-10 aryl group, (6) a heteroaryl group selected from the group consisting of a pyridyl group, a pyridazinyl group, a pyrimidinyl group, a pyrazinyl group, a pyrrolyl group, a pyrazolyl group, an imidazolyl group, a thiazolyl group, a furyl group, a thienyl group, an isoxazolyl group, an imidazo[1,2-a]pyridyl group, a pyrazolo[1,5-a]pyridyl group, a benzo[b]thienyl group and an indolyl group and (7) a 4,5,6,7-tetrahydrobenzothienyl group>
(Group U2)
<(1) a halogen atom, (2) a hydroxyl group, (3) a cyano group, (4) a C1-6 alkyl group, (5) a C3-6 cycloalkyl group, (6) a C1-6 alkoxymethyl group, (7) a C1-6 alkyl group substituted with one hydroxyl group, (8) a C1-6 alkoxyimino group, (9) a C1-6 alkoxy group, (10) a methylenedioxy group, (11) an N-(C1-6 alkyl)carbamoyl group, (12) a C2-6 alkynyl group, (13) a C2-6 alkenyl group, (14) a C1-6 alkylthio group and (15) an amino group which may be substituted with one or two C1-6 alkyl groups>.

17. The compound or salt thereof according to claim 4, 5, 6 or 12, wherein R4 represents a C1-6 alkyl group which is substituted with 1 to 3 substituents which are the same or different and are selected from Group T2 according to claim 16, which substituents selected from Group T2 may be substituted with 1 to 3 substituents which are the same or different and are selected from Group U2 acocrding to Claim 16.

18. The compound or salt thereof according to claim 4, 5, 6 or 12, wherein R4 is a group represented by the formula -(CH₂)u-R41 [wherein u is 1 to 3; and R41 is a substituent selected from Group T2 according to claim 16, wherein the substituent selected from the Group T2 may be substituted with 1 to 3 substituents which are the same or different and are selected from Group U2 according to claim 16].

19. The compound or salt thereof according to claim 4, 5, 6 or 12, wherein R4 is a group represented by the formula -(CH₂)u-R41 [wherein u is 1; and R41 is a substituent selected from Group T2 according to claim 16, wherein the substituent selected from the Group T2 may be substituted with 1 to 3 substituents which are the same or different and are selected from Group U2 according to claim 16].

20. The compound or salt thereof according to claim 4, 5, 6 or 12, wherein R4 is a group represented by the formula -(CH₂)u-R41 [wherein u is 1; and R41 represents (1) a cyclopentyl group, (2) a 1,3-dioxolanyl group, (3) a phenyl group, (4) a pyridyl group or (5) a thienyl group, which may be substituted with 1 to 3 substituents which are the same or different and are selected from Group U2 according to claim 16].

21. The compound or salt thereof according to claim 4 or 5, wherein R2 is a group represented by the formula -(O)n-R21 [wherein n is 1; and R21 is a group represented by the formula -(CH₂)t-R23 (wherein t is 1 or 2; and R23 represents (1) a phenyl group or (2) a cyclopropyl group, wherein R23 may be substituted with 1 to 3 substituents which are the same or different and are selected from Group B1 according to claim 1)]; and R4 is a group represented by the formula -(CH₂)u-R41 [wherein u is 1; and R41 represents (1) a cyclopentyl group, (2) a 1,3-dioxolanyl group, (3) a phenyl group, (4) a pyridyl group or (5) a thienyl group, which may be substituted with 1 to 3 substituents which are the same or different and are selected from Group U2 according to claim 16].

22. The compound or salt thereof according to claim 4 or 5, wherein R2 is a group represented by the formula -(O)n-R21 [wherein n is 0; and R21 represents (1) a C6-10 aryl group, (2) a heteroaryl group selected from the group consisting of a furyl group, a thienyl group, a thiazolyl group, an oxazolyl group, a pyridyl group and a pyridazinyl group, (3) a heterocycloalkyl group selected from the group consisting of a 1-pyrrolidinyl group, a piperidino group, a perhydro-2H-azepin-1-yl group and a morpholino group or (4) a 3,4,5,6-tetrahydro-2H-pyranyl group, which may be substituted with 1 to 3 cyano groups or/and halogen atoms]; and
R4 is a group represented by the formula -(CH₂)u-R41 [wherein u is 1; and R41 represents (1) a cyclopentyl group, (2) a 1,3-dioxolanyl group, (3) a phenyl group, (4) a pyridyl group or (5) a thienyl group, which may be substituted with 1 to 3 substituents which are the same or different and are selected from Group U3 shown below].
(Group U3)
<(1) a halogen atom, (2) a hydroxyl group, (3) a cyano group, (4) a C1-6 alkyl group substituted with one hydroxyl group and (5) a C1-6 alkoxy group>

23. A compound selected from the group consisting of (1) N,N-dimethyl{3-[2-(1-benzylpiperidin-4-yl)ethyl]-7-cyclopropylmethoxybenz[d]isoxazol-6-yl}methylamine, (2) 5-{4-{2-[7-(azetidin-1-ylmethyl)-6-cyclopropylmethoxybenz[d]isoxazol-3-yl]ethyl}piperidinomethyl}thiophene-2-carbonitrile, (3) N,N-dimethyl{6-cyclopropylmethoxy-3-{2-[1-(4,5,6,7-tetrahydrobenzo[b]thiophen-4-ylmethyl)piperidin-4-yl]ethyl}benz[d]isoxazol-7-yl}methylamine, (4) N,N-dimethyl{3-[2-(1-benzylpiperidin-4-yl)ethyl]-6-cyclopropylmethoxybenz[d]isoxazol-5-yl}methylamine, (5) N,N-dimethyl{6-cyclopropylmethoxy-3-[2-(1-benzylpiperidin-4-yl)ethyl]benz[d]isoxazol-7-yl}methylamine, (6) 4-{3-[2-(1-benzylpiperidin-4-yl)ethyl]-7-(N,N-dimethylaminomethyl)benz[d]isoxazol-6-yloxymethyl}benzonitrile, (7) 4-{7-(N,N-dimethylaminomethyl)-3-{2-[1-(1,3-dioxolan-2-ylmethyl)piperidin-4-yl]ethyl}benz[d]isoxazol-6-yloxymethyl}benzonitrile, (8) N,N-dimethyl{3-[2-(1-benzylpiperidin-4-yl)ethyl]-6-phenylbenz[d]isoxazol-7-yl}methylamine, (9) 5-{4-{2-[6-cyclopropylmethoxy-7-(N,N-dimethylaminomethyl)benz[d]isoxazol-3-yl]ethyl}piperidinomethyl}thiophene-2-carbonitrile, (10) {1-{4-{2-[6-cyclopropylmethoxy-7-(N,N-dimethylaminomethyl)benz[d]isoxazol-3-yl]ethyl}piperidinomethyl}cyclopentyl}methanol, (11) N,N-dimethyl{6-cyclopropylmethoxy-3-{2-[1-(5-fluoro-1H-indol-3-ylmethyl)piperidin-4-yl]ethyl}benz[d]isoxazol-7-yl}methylamine, (12) 5-{4-{2-[7-(N,N-dimethylaminomethyl)-6-piperidinobenz[d]isoxazol-3-yl]ethyl}piperidinomethyl}thiophene-2-carbonitrile, (13) 5-{4-{2-[7-(N,N-dimethylaminomethyl)-6-morpholinobenz[d]isoxazol-3-yl]ethyl}piperidinomethyl}thiophene-2-carbonitrile, (14) N,N-dimethyl{3-{2-[1-(1,3-dioxolan-2-ylmethyl)piperidin-4-yl]ethyl}-6-benzyloxybenz[d]isoxazol-7-yl}methylamine, (15) 5-{4-{2-[9-(N,N-dimethylaminomethyl)naphtho[2,3-d]isoxazol-3-yl]ethyl}piperidinomethyl}thiophene-2-carbonitrile, (16) 5-{4-{2-[9-(N-methylaminomethyl)naphtho[2,3-d]isoxazol-3-yl]ethyl}piperidinomethyl}thiophene-2-carbonitrile, (17) N-methyl{3-{2-[1-(1,3-dioxolan-2-ylmethyl)piperidin-4-yl]ethyl}-6-(4-fluorobenzyloxy)benz[d]isoxazol-7-yl}methylamine and (18) N,N-dimethyl{3-{2-[1-(1,3-dioxolan-2-ylmethyl)piperidin-4-yl]ethyl}-6-(4-fluorobenzyloxy)benz[d]isoxazol-7-yl}methylamine, or a salt thereof.

24. A compound selected from the group consisting of (1) N,N-dimethyl{6-cyclopropylmethoxy-3-[2-(1-benzylpiperidin-4-yl)ethyl]benz[d]isoxazol-7-yl}methylamine, (2) 4-{3-[2-(1-benzylpiperidin-4-yl)ethyl]-7-(N,N-dimethylaminomethyl)benz[d]isoxazol-6-yloxymethyl}benzonitrile, (3) 4-{7-(N,N-dimethylaminomethyl)-3-{2-[1-(1,3-dioxolan-2-ylmethyl)piperidin-4-yl]ethyl}benz[d]isoxazol-6-yloxymethyl}benzonitrile, (4) N,N-dimethyl{3-[2-(1-benzylpiperidin-4-yl)ethyl]-6-phenylbenz[d]isoxazol-7-yl}methylamine, (5) 5-{4-{2-[6-cyclopropylmethoxy-7-(N,N-dimethylaminomethyl)benz[d]isoxazol-3-yl]ethyl}piperidinomethyl}thiophene-2-carbonitrile, (6) {1-{4-{2-[6-cyclopropylmethoxy-7-(N,N-dimethylaminomethyl)benz[d]isoxazol-3-yl]ethyl}piperidinomethyl}cyclopentyl}methanol, (7) N,N-dimethyl{6-cyclopropylmethoxy-3-{2-[1-(5-fluoro-1H-indol-3-ylmethyl)piperidin-4-yl]ethyl}benz[d]isoxazol-7-yl}methylamine, (8) 5-{4-{2-[7-(N,N-dimethylaminomethyl)-6-piperidinobenz[d]isoxazol-3-yl]ethyl}piperidinomethyl}thiophene-2-carbonitrile, (9) 5-{4-{2-[7-(N,N-dimethylaminomethyl)-6-morpholinobenz[d]isoxazol-3-yl]ethyl}piperidinomethyl}thiophene-2-carbonitrile, (10) N,N-dimethyl{3-{2-[1-(1,3-dioxolan-2-ylmethyl)piperidin-4-yl]ethyl}-6-benzyloxybenz[d]isoxazol-7-yl}methylamine, (11) 5-{4-{2-[9-(N,N-dimethylaminomethyl)naphtho[2,3-d]isoxazol-3-yl]ethyl}piperidinomethyl}thiophene-2-carbonitrile, (12) 5-{4-{2-[9-(N-methylaminomethyl)naphtho[2,3-d]isoxazol-3-yl]ethyl}piperidinomethyl}thiophene-2-carbonitrile, (13) N-methyl{3-{2-[1-(1,3-dioxolan-2-ylmethyl)piperidin-4-yl]ethyl}-6-(4-fluorobenzyloxy)benz[d]isoxazol-7-yl}methylamine and (14) N,N-dimethyl{3-{2-[1-(1,3-dioxolan-2-ylmethyl)piperidin-4-yl]ethyl}-6-(4-fluorobenzyloxy)benz[d]isoxazol-7-yl}methylamine, or a salt thereof.

25. A therapeutic agent for dementia or cognitive impairment, comprising the compound or salt thereof according to any one of claims 1 to 24 as an active ingredient.

26. A therapeutic agent for depression or a disease with depressive symptoms, anxiety, attention deficit hyperactivity disorder or eating disorder, comprising the compound or salt thereof according to any one of claims 1 to 24 as an active ingredient.

27. The therapeutic agent according to claim 25, wherein the dementia or cognitive impairment is cognitive impairment associated with Parkinson's disease, Huntington's chorea, head injury or Down's syndrome, or Alzheimer's disease, cerebrovascular dementia, Lewy body dementia, mild cognitive impairment or frontotemporal dementia.

28. The therapeutic agent according to claim 25, wherein the dementia or cognitive impairment is Alzheimer's disease or cerebrovascular dementia.

29. A pharmaceutical composition comprising the compound or salt thereof according to any one of claims 1 to 24.
